# EUROPEAN PATENT APPLICATION

(11) **EP 3 272 749 A1**
(43) Veröffentlichungstag der Anmeldung: **24.01.2018**
(21) Anmeldenummer: 17168254.5
(22) Anmeldetag: 04.11.2013
(51) Int. Cl.: C07D 471/04, C07D 471/08, C07D 519/00, A61K 31/437, A61P 9/00

(54) **AMINO-SUBSTITUIERTE IMIDAZO[1,2-A]PYRIDIN-3-CARBOXAMIDE UND IHRE VERWENDUNG ALS STIMULATOREN DER LÖSLISCHEN GUANYLATCYCLASE**

(30) Priorität: 05.11.2012 EP 12191201; 07.03.2013 US 201313789655; 26.07.2013 EP 13178248
(62) Teilanmeldung aus: 13785869.2
(71) Anmelder: Bayer Pharma Aktiengesellschaft, 13353 Berlin (DE)
(72) Erfinder: Vakalopoulos, Alexandros, 40721 Hilden (DE); Follmann, Markus, 50859 Berlin (DE); Hartung, Ingo, 13158 Berlin (DE); Buchgraber, Philipp, 10435 Berlin (DE); Jautelat, Rolf, 42781 Haan (DE); Haßfeld, Jorma, 40221 Düsseldorf (DE); Lindner, Niels, 42115 Wuppertal (DE); Gromov, Alexey, 40699 Erkrath (DE); Wunder, Frank, 42117 Wuppertal (DE); Stasch, Johannes-Peter, 00046 Grottaferrata (RM) (IT); Redlich, Gorden, 44799 Bochum (DE); Li, Volkhart Min-Jian, 42553 Velbert (DE)
(74) Vertreter: BIP Patents

(57) **Zusammenfassung**

Die vorliegende Anmeldung betrifft neue substituierte Imidazo[1,2-a]pyridin-3-carboxamide, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

## Beschreibung

Die vorliegende Anmeldung betrifft neue substituierte Imidazo[1,2-a]pyridin-3-carboxamide, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris,, Herzinsuffizienz, Myokardinfarkt, Thrombosen, Schlaganfall und sexueller Dysfunktion fuhren kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO bemht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorphosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Unter anderem in EP 0 266 890-A1, WO 89/03833-A1, TP 01258674-A [vgl. Chem. Abstr. 112:178986], WO 96/34866-A1, EP 1 277754-A1, WO 2006/015737-A1, WO 2008/008539-A2, WO 2008/082490-A2, WO 2008/134553-A1, WO 2010/030538-A2, WO 2011/113606-A1 und WO 2012/165399 Alsind verschiedene Imidazo[1,2-a]pyridin-Derivate beschrieben, die zur Behandlung von Erkrankungen verwendet werden können.

Aufgabe der vorliegenden Erfindung war die Bereitstellung neuer Substanzen, die als Stimulatoren der löslichen Guanylatcyclase wirken, und als solche zur Behandlung und/oder Prophylaxe von Krankheiten geeignet sind.

Gegenstand der vorliegenden Erfindung sind Verbindungen der allgemeinen Formel (I) 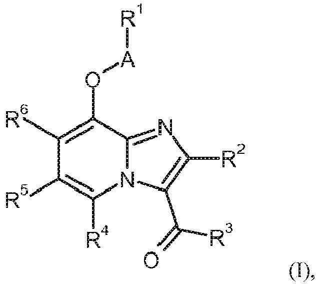 in welcher
- A: für CH₂, CD₂ oder CH(CH₃) steht,
- R¹: für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, Difluormethoxy und Trifluormethoxy substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 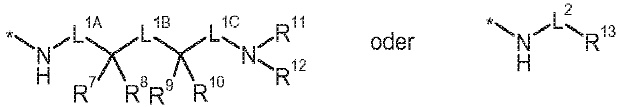 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkylsulfonyl und (C₁-C₄)-Alkoxy substituiert sein können,
- R⁸: für Wasserstoff oder (C₁-C₄)-Alkyl steht,
oder
- R⁷ und R⁸: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R⁹: für Wasserstoff, (C₁-C₆)-Alkyl, (C₂-C₆)-Alkenyl, (C₂-C₆)-Alkinyl, (C₃-C₇)-Cycloalkyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycaibonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R¹⁰: für Wasserstoff oder (C₁-C₄)-Alkyl steht,
oder
- R⁹ und R¹⁰: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass die Reste R⁷ und R⁹ nicht beide gleichzeitig für Phenyl stehen,
oder
- R⁷ und R⁹: zusammen mit den Kohlenstoffatomen, an die sie gebunden sind, sowie der Gruppe L^{1B} einen 5- bis 7-gliedrigen Carbocyclus oder einen 4- bis 7-gliedrigen Heterocyclus bilden,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
- R¹¹: für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
- R¹²: für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann, und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kein, der seinerseits mit 1 oder 2 Substituenten ausgewählt aus Halogen, (C₁-C₄)-Alkyl und Trifluormethyl substituiert sein kann,
- R⁴: für Wasserstoff steht,
- R⁵: für Wasserstoff, Halogen, Cyano, Difluormethyl, Trifluormethyl, (C₁-C₄)-Alkyl, (C₃-C₇)-Cycloalkyl, (C₂-C4)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Gegenstand der vorliegenden Erfindung sind Verbindungen der allgemeinen Formel (I) 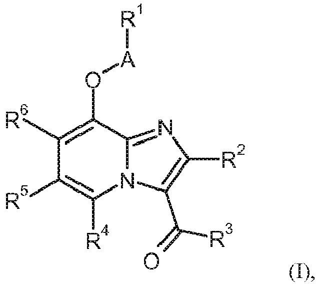 in welcher
- A: für CH₂, CD₂ oder CH(CH₃) steht,
- R¹: für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, Difluormethoxy und Trifluormethoxy substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 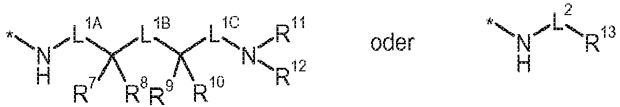 steht, wobei
* für die Anknüpfstelle an die Carbonylgruppe steht,
- L^{1A}: für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
- L^{1B}: für eine Bindung oder (C₁-C₄)-Alkandiyl steht,
- L^{1C}: für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
- R⁷: für Wasserstoff, (C₁-C₆)-Alkyl, (C₂-C₆)-Alkenyl, (C₂-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, (C₁-C₄)-Alkoxy, (C₁-C₄)-Alkoxycarbonyl, (C₁-C₄)-Allrylsulfonyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃, (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin das Phenyl an 2 benachbarten Kohlenstoffatomen mit einer Difluormethylendioxy-Brücke substituiert sein kann,
- R⁸: für Wasserstoff oder (C₁-C₄)-Alkyl steht,
oder
- R⁷ und R⁸: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R⁹: für Wasserstoff, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5-oder 6-gliedriges Heteroaryl substituiert sein kann,
   worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
- R¹⁰: für Wasserstoff oder (C₁-C₄)-Alkyl steht,
oder
- R⁹ und: R¹⁰ zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können,
mit der Maßgabe, dass die Reste R⁷ und R⁹ nicht beide gleichzeitig für Phenyl stehen,
oder
- R⁷ und R⁹: zusammen mit den Kohlenstoffatomen, an die sie gebunden sind, sowie der Gruppe L^{1B} einen 3- bis 7-gliedrigen Carbocyclus oder einen 4- bis 7-gliedrigen Heterocyclus bilden,
worin der 3- bis 7-gliedrigen Carbocyclus mit 1 oder 2 Substituenten unabhängig voneinander ausgewählt aus der Gruppe (C₁-C₄)-Alkyl, Fluor, Hydroxy und (C₁-C₄)-Alkoxy substituiert sein kann,
mit der Maßgabe, dass gleichzeitig nicht mehr als eines der Reste-Paare R⁷ und R⁸, R⁹ und
- R¹⁰ bzw. R⁷ und R⁹: einen Carbo- oder Heterocyclus bildet,
- R¹¹: für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
- R¹²: für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
- R¹¹ und R¹²: zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann,
und
- L²: für eine Bindung oder (C₁-C₄)-Alkandiyl steht,
- R¹³: für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
   und
worin 5- bis 9-gliedriges Aza-Heterocyclyl mit einem Phenyl-Ring anneliert sein kein, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,
- R⁴: für Wasserstoff steht,
- R⁵: für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl.
Cycloalkyl bzw. Carbocyclus steht in Rahmen der Erfindung für einen monocyclischen, gesättigten Alkylrest mit der jeweils angegebenen Anzahl an Ring-Kohlenstoffatomen. Beispielhaft und vorzugsweise seien genannt: Cyclopropyl, Cyclobutyl, Cyclopentyl, Cyclohexyl und Cycloheptyl.
Alkenyl steht im Rahmen der Erfindung für einen linearen oder verzweigten Alkenylrest mit 2 bis 6 Kohlenstoffatomen und einer oder zwei Doppelbindungen. Bevorzugt ist ein linearer oder verzweigter Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.
Alkinyl steht im Rahmen der Erfindung für einen linearen oder verzweigten Alkinylrest mit 2 bis 6 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.
Alkandiyl steht im Rahmen der Erfindung für einen linearen oder verzweigten divalenten Alkylrest mit 1 bis 4 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl und Butan-2,3-diyl.
Alkoxy steht im Rahmen der Erfindung für einen linearen oder verzweigten Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.
Alkoxycarbonyl stehen im Rahmen der Erfindung für einen linearen oder verzweigten Alkoxyrest mit 1 bis 4 Kohlenstoffatomen und einer am Sauerstoffatom angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.
Ein 4- bis 7-gliedriger Heterocyclus steht im Rahmen der Erfindung für einen monocyclischen, gesättigten Heterocyclus mit insgesamt 4 bis 7 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Azetidinyl, Oxetanyl, Pyrrolidinyl, Tetrahydrofuranyl, Piperidinyl, Piperazinyl, Tetrahydropyranyl und Morpholinyl.
Ein 4- bis 7-gliedriger Aza-Heterocyclus steht im Rahmen der Erfindung für einen monocyclischen, gesättigten Heterocyclus mit insgesamt 4 bis 7 Ringatomen, der ein Stickstoffatom enthält und darüberhinaus ein weiteres Ring-Heteroatom aus der Reihe N, O, S, SO oder SO₂ enthalten kann und über ein Ring-Stickstoffatom verknüpft ist. Beispielhaft seien genannt: Azetidinyl, Pyrrolidinyl, Pyrazolidinyl, Piperidinyl, Piperazinyl, Morpholinyl, Thiomorpholinyl, 1,1-Dioxothiomorpholinyl, Hexahydroazepinyl und Hexahydro-1,4-diazepinyl.
5- bis 9-gliedriges Aza-Heterocyclyl steht im Rahmen der Erfindung für einen monocyclischen oder bicyclischen, gesättigten oder teilweise ungesättigten Heterocyclus mit insgesamt 5 bis 9 Ringatomen, der ein Stickstoffatom enthält und darüberhinaus ein oder zwei weitere Ring-Heteroatome aus der Reihe N, O, S, SO und/oder SO₂ enthalten kann und über ein Ring-Kohlenstoffatom verknüpft ist. Beispielhaft seien genannt: Pyrrolidinyl, Pyrazolidinyl, Piperidinyl, Piperazinyl, Morpholinyl, Thiomorpholinyl, 1,1-Dioxothiomorpholinyl, Hexahydroazepinyl, Hexahydro-1,4₋diazepinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Arabicyclo[4.1.0]heptanyl und Quinuclidinyl.
Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt steht Heteroarvl im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl.
Halogen schließt im Rahmen der Erfindung Fluor, Chlor, Brom und Iod ein. Bevorzugt sind Chlor oder Fluor.

In der Formel der Gruppe, für die R³ bzw. R¹ stehen kann, steht der Endpunkt der Linie, an dem das Zeichen * und # steht, nicht für ein Kohlenstoffatom beziehungsweise eine CH₂-Gruppe, sondern ist Bestandteil der Bindung zu dem jeweils bezeichneten Atom, an das R³ bzw. R¹ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 4 Substituenten unabhängig voneinander ausgewählt aus der Gruppe Fluor, Chlor, Cyano, Methoxy, Difluormethyl, Trifluormethyl und Methyl substituiert sein kann,
- R²: für Wasserstoff, Trifluormethyl, (C₁-C₄)-Alkyl oder Cyclopropyl steht,
- R³: für eine Gruppe der Formel 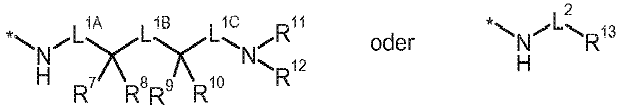 steht, wobei
* für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung oder Methylen steht,
worin Methylen mit 1 oder 2 Substituenten unabhängig voneinander ausgewählt aus der Gruppe Trifluormethyl, (C₁-C₄)-Alkyl, Cyclopropyl und Cyclobutyl substituiert sein kann,
R⁷ für Wasserstoff, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Fluor, Trifluormethyl, Difluormethoxy, Trifluor-methoxy, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Methyl und Trifluormethyl substituiert sein können,
- R⁸: für Wasserstoff, Methyl oder Ethyl steht,
oder
- R⁷ und R⁸: zusammen mit dem Kohlenstoffatom, an das sie gebunden sind, einen 3-bis 6-gliedrigen Carbocyclus oder einen Oxetanyl-, Tetrahydrofuranyl-, Tetrahydropyranyl-, Azetidinyl-, Pyrrolidinyl- oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl- und Piperidinyl-Ring mit 1 oder 2 Substituenten unabhängig voneinander ausgewählt aus Fluor und Methyl substituiert sein können,
- R⁹: für Wasserstoff, 1,1,2,2-Tetrafluorethyl, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Fluor, Trifluormethyl, Difluormethoxy, Trifluor-methoxy, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Methyl und Trifluormethyl substituiert sein können,
- R¹⁰: für Wasserstoff, Methyl oder Ethyl steht,
oder
- R⁹ und R¹⁰: zusammen mit dem Kohlenstoffatom, an das sie gebunden sind, einen 3-bis 6-gliedrigen Carbocyclus oder einen Oxetanyl-, Tetrahydrofuranyl-, Tetrahydropyranyl-, Azetidinyl-, Pyrrolidinyl- oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl- und Piperidinyl-Ring mit 1 oder 2 Substituenten unabhängig voneinander ausgewählt aus Fluor und Methyl substituiert sein können,
mit der Maßgabe, dass die Reste R⁷ und R⁹ nicht beide gleichzeitig für Phenyl stehen,
oder
- R⁷ und R⁹: zusammen mit den Kohlenstoffatomen, an die sie gebunden sind, sowie der Gruppe L^{1B} einen Cyclopentyl-, Cyclohexyl-, Azetidinyl-, Oxetanyl-, Pyrrolidinyl-, Tetrahydrofuranyl-, Piperidinyl- oder Tetrahydropyranyl-Ring bilden,
mit der Maßgabe, dass gleichzeitig nicht mehr als eines der Reste-Paare R⁷ und R⁸, R⁹ und R¹⁰ bzw. R⁷ und R⁹ einen der zuvor benannten Carbo- oder Heterocyclen bildet,
- R¹¹: für Wasserstoff oder (C₁-C₃)-Alkyl steht,
worin (C₁-C₃)-Alkyl mit 1 oder 2 Substituenten unabhängig voneinander ausgewählt aus der Gruppe Fluor, Trifluormethyl, Hydroxy, Methoxy und Ethoxy substituiert sein kann,
- R¹²: für Wasserstoff, (C₁-C₄)-Alkyl, Cyclopropyl, Cyclobutyl, Phenyl oder Benzyl steht,
worin (C₁-C₄)-Alkyl mit 1 oder 2 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Fluor, Chlor und Trifluormethyl substituiert sein können,
oder
- R¹¹ und R¹²: zusammen mit dem Stickstoffatom, an das sie gebunden sind, einen Azetidinyl-, Pyrrolidinyl-, Piperidinyl-, Morpholinyl-, Piperazinyl-, Thiomorpholinyl- oder 1,1-Dioxothiomorpholinyl-Ring bilden,
worin der Azetidinyl-, Pyrrolidinyl-, Piperidinyl-, Morpholinyl-, Piperazinyl-, Thiomorpholinyl- und 1,1-Dioxothiomorpholinyl-Ring mit 1 oder 2 Substituenten unabhängig voneinander ausgewählt aus der Gruppe Fluor, Trifluormethyl, Methyl, Ethyl, Cyclopropyl, Cyclobutyl, Azetidinyl, Pyrrolidinyl und Piperidinyl substituiert sein können,
und
- L²: für eine Bindung, Methylen oder 1,1-Ethandiyl steht,
- R¹³: für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Arabicyclo[3.3.1]nonanyl, 3-Azabicyclo-[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Aza-bicyclo[3.3.1]nonanyl, 3-Azabicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
- R⁴ für: Wasserstoff steht,
- R⁵: für Wasserstoff, Fluor, Chlor, Brom, Cyano, Methyl, Ethyl oder Cyclopropyl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 4 Substituenten unabhängig voneinander ausgewählt aus der Gruppe Fluor, Chlor, Difluormethyl, Trifluormethyl und Methyl substituiert sein kann,
- R²: für (C₁-C₃)-Alkyl, Trifluormethyl oder Cyclopropyl steht,
- R³: für eine Gruppe der Formel 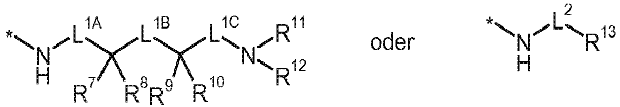 steht, wobei
* für die Anknüpfstelle an die Carbonylgruppe steht,
L^{1A} für eine Bindung steht,
L^{1B} für eine Bindung oder Methylen steht,
L^{1C} für eine Bindung oder Methylen steht,
worin Methylen mit 1 oder 2 Substituenten unabhängig voneinander ausgewählt aus der Gruppe Trifluormethyl, (C₁-C₄)-Alkyl, Cyclopropyl und Cyclobutyl substituiert sein kann,
- R⁷: für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano und Trifluormethyl substituiert sein kann,
- R⁸: für Wasserstoff, Methyl oder Ethyl steht,
oder
- R⁷ und R⁸: zusammen mit dem Kohlenstoffatom, an das sie gebunden sind, einen 3-bis 6-gliedrigen Carbocyclus oder einen Oxetanyl-, Tetrahydrofuranyl-, Tetrahydropyranyl-, Azetidinyl-, Pyrrolidinyl- oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl- und Piperidinyl-Ring mit 1 oder 2 Substituenten unabhängig voneinander ausgewählt aus Fluor und Methyl substituiert sein können,
- R⁹: für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano und Trifluormethyl substituiert sein kann,
- R¹⁰: für Wasserstoff, Methyl oder Ethyl steht,
oder
- R⁹ und R¹⁰: zusammen mit dem Kohlenstoffatom, an das sie gebunden sind, einen 3-bis 6-gliedrigen Carbocyclus oder einen Oxetanyl-, Tetrahydrofuranyl-, Tetrahydropyranyl-, Azetidinyl-, Pyrrolidinyl- oder Piperidinyl-Ring bilden,
worin der 3- bis 6-gliedrige Carbocyclus, sowie der Oxetanyl-, Tetrahydrofuranyl-, Tetrahydropyranyl-, Azetidinyl-, Pyrrolidinyl- und Piperidinyl-Ring mit 1 oder 2 Substituenten unabhängig voneinander ausgewählt aus Fluor und Methyl substituiert sein können,
mit der Maßgabe, dass die Reste R⁷ und R⁹ nicht beide gleichzeitig für Phenyl stehen,
oder
- R⁷ und R⁹: zusammen mit den Kohlenstoffatomen, an die sie gebunden sind, sowie der Gruppe L^{1B} einen Cyclopentyl-, Cyclohexyl-, Azetidinyl-, Oxetanyl-, Pyrrolidinyl-, Tetrahydrofuranyl-, Piperidinyl- oder Tetrahydropyranyl-Ring bilden,
mit der Maßgabe, dass gleichzeitig nicht mehr als eines der Reste-Paare R⁷ und R⁸, R⁹ und R¹⁰ bzw. R⁷ und R⁹ einen der zuvor benannten Carbo- oder Heterocyclen bildet,
- R¹¹: für Wasserstoff oder (C₁-C₃)-Alkyl steht,
worin (C₁-C₃)-Alkyl mit 1 oder 2 Substituenten unabhängig voneinander ausgewählt aus der Gruppe Fluor und Trifluormethyl substituiert sein kann,
- R¹²: für Wasserstoff, (C₁-C₄)-Alkyl, Cyclopropyl oder Cyclobutyl steht,
worin (C₁-C₄)-Alkyl mit 1 oder 2 Substituenten unabhängig voneinander ausgewählt aus der Gruppe Fluor und Trifluormethyl substituiert sein kann,
oder
- R¹¹ und R¹²: zusammen mit dem Stickstoffatom, an das sie gebunden sind, einen Azetidinyl-, Pyrrolidinyl-, Piperidinyl- oder Morpholinyl-Ring bilden,
worin der Azetidinyl-, Pyrrolidinyl-, Piperidinyl- und Morpholinyl-Ring mit 1 oder 2 Substituenten unabhängig voneinander ausgewählt aus der Gruppe Fluor, Trifluormethyl, Methyl, Ethyl, Cyclopropyl und Cyclobutyl substituiert sein können,
und
- L²: für eine Bindung oder Methylen steht,
- R¹³: für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Arabicyclo[3.3.1]nonanyl, 3-Aza-bicyclo[4.1.0]heptanyl und Chinuclidinyl steht,
oder
worin Pyrrolidinyl, Piperidinyl, 1,2,3,4-Tetrahydroisochinolinyl, 1,2,3,4-Tetrahydrochinolinyl, 8-Azabicyclo[3.2.1]octanyl, 9-Azabicyclo-[3.3.1]nonanyl, 3-Aza-bicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
- R⁴: für Wasserstoff steht,
- R⁵: für Wasserstoff, Fluor, Chlor, Methyl, Ethyl oder Cyclopropyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I), in welcher
- A: für CH₂ steht,
- R¹: für eine Phenyl-Gruppe der Formel 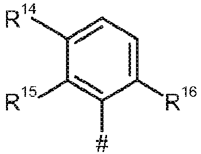 steht, wobei
# für die Anknüpfstelle an A steht, und
R¹⁴, R¹⁵ und R¹⁶ unabhängig voneinander für Wasserstoff, Fluor oder Chlor stehen, mit der Maßgabe, dass mindestens zwei der Reste R¹⁴, R¹⁵, R¹⁶ von Wasserstoff verschieden sind,
- R²: für Methyl steht,
- R³: für eine Gruppe der Formel 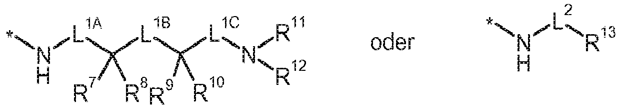 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor und Chlor substituiert sein kann,
R⁸ für Wasserstoff, Methyl oder Ethyl steht,
R⁹ für Wasserstoff, Trifluormethyl, (C₁-C₆)-Alkyl, Cyclopropyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor und Chlor substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3-bis 6-gliedrigen Carbocyclus oder einen Oxetanylring bilden, mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen Cyclopentyl- oder Cyclohexyl-Ring bilden, mit der Maßgabe, dass gleichzeitig nicht mehr als eines der Reste-Paare R⁹ und R¹⁰ bzw. R⁷ und R⁹ einen der zuvor benannten Carbo- oder Heterocyclen bildet,
R¹¹ für Wasserstoff steht,
R¹² für Wasserstoff steht, und
L² für eine Bindung steht,
R¹³ für Piperidin-2-yl, Piperidin-3-yl, Piperidin-4-yl oder 1,2,3,4-Tetrahydrochinolin-4-yl steht,
worin Piperidin-2-yl, Piperidin-3-yl und Piperidin-4-yl mit 1 bis 5 Substituenten unabhängig voneinander ausgewählt aus Trifluormethyl und Methyl substituiert sein kann,
und
worin 1,2,3,4-Tetrahydrochinolin-4-yl mit Fluor oder Trifluormethyl substituiert sein können,
- R⁴: für Wasserstoff steht,
- R⁵: für Wasserstoff, Fluor, Chlor oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für Phenyl steht, wobei Phenyl mit 1 bis 3 Substituenten unabhängig voneinander ausgewählt aus der Gruppe Halogen, Cyano, Monofluormethyl, Difluormethyl, Trifluormethyl, (C₁-C₄)-Alkyl substituiert sein kann,
und
wobei Phenyl mit 1 bis 2 Substituenten ausgewählt aus der Gruppe (C₃-C₆)-Cycloalkyl, (C₁-C₄)-Alkoxy, Difluormethoxy oder Trifluormethoxy substituiert ist,
- R²: für Wasserstoff, (C₁-C₄)-Alkyl, Cyclopropyl, Monofluormethyl, Difluormethyl oder Trifluormethyl steht,
- R³: für eine Gruppe der Formel 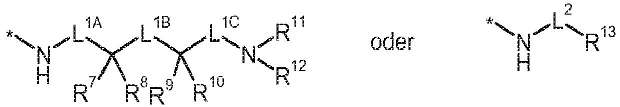
steht, wobei
- *: für die Anknüpfstelle an die Carbonylgruppe steht,
- L^{1A}: für eine Bindung oder (C₁-C₄)-Alkandiyl steht, worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
- L^{1B}: für eine Bindung oder (C₁-C₄)-Alkandiyl steht,
- L^{1C}: für eine Bindung oder (C₁-C₄)-Alkandiyl steht, worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
- R⁷: für Wasserstoff, (C₁-C₆)-Alkyl, (C₂-C₆)-Alkenyl, (C₂-C₆)-Alkinyl, (C₃-C₇)-Cycloalkyl, Cyano, 5- oder 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, (C₁-C₄)-Alkoxy, (C₁-C₄)-Sulfanyl, (C₁-C₄)-Alkoxy-carbonyl, (C₁-C₄)-Alkylsulfunyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, NH(CO)CH₃, (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-(₄)-Alkoxy substituiert sein können,
wobei (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
- R⁸: für Wasserstoff oder (C₁-C₄)-Alkyl steht,
oder
- R⁷ und R⁸: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R⁹: für Wasserstoff, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5-oder 6-gliedriges Heteroaryl substituiert sein kann,
   worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
- R¹⁰: für Wasserstoff oder (C₁-C₄)-Alkyl steht,
oder
- R⁹ und R¹⁰: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können,
mit der Maßgabe, dass die Reste R⁷ und R⁹ nicht beide gleichzeitig für Phenyl stehen,
oder
- R⁷ und: R⁹ zusammen mit den Kohlenstoffatomen, an die sie gebunden sind, sowie der Gruppe L^{1B} einen 3- bis 7-gliedrigen Carbocyclus oder einen 4- bis 7-gliedrigen Heterocyclus bilden,
worin der 3- bis 7-gliedrigen Carbocyclus mit 1 oder 2 Substituenten unabhängig voneinander ausgewählt aus der Gruppe (C₁-C₄)-Alkyl, Fluor, Hydroxy und (C₁-C₄)-Alkoxy substituiert sein kann,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
- R¹¹: für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
- R¹²: für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
- R¹¹ und R¹²: zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann,
und
- L²: für eine Bindung oder (C₁-C₄)-Alkandiyl steht,
- R¹³: für 5- bis 9-gliedriges, über ein Ring-Kohlenstoffatom gebundenes Asz-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heteroeyclyl mit einem Phenyl-Ring anneliert sein kein, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,

- R⁴: für Wasserstoff steht,
- R⁵: für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ oder CH(CH₃) steht,
- R¹: Phenyl steht, wobei Phenyl mit 1 bis 2 Substituenten unabhängig voneinander ausgewählt aus der Gruppe Fluor, Chlor, Cyano, Monofluormethyl, Difluormethyl, Trifluormethyl, (C₁-C₄)-Alkyl substituiert sein kann,
und
wobei Phenyl mit einem Substituenten ausgewählt aus der Gruppe (C₃-C₆)-Cycloalkyl, (C₁-C₂)-Alkoxy oder Trifluormethoxy substituiert ist,
- R²: für Wasserstoff, Trifluormethyl, (C₁-C₃)-Alkyl und Cyclopropyl steht,
- R³: für eine Gruppe der Formel 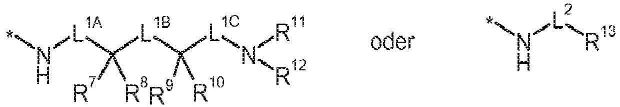 steht, wobei
* für die Anknüpfstelle an die Carbonylgruppe steht,
- L^{1A}: für eine Bindung steht,
- L^{1B}: für eine Bindung, Methylen oder 1,2-Ethandiyl steht,
- L^{1C}: für eine Bindung oder Methylen steht,
worin Methylen mit 1 oder 2 Substituenten unabhängig voneinander ausgewählt aus der Gruppe Fluor, Trifluormethyl, (C₁-C₄)-Alkyl, Cyclopropyl und Cyclobutyl substituiert sein kann,
- R⁷: für Wasserstoff, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Fluor, Trifluormethyl, Difluormethoxy, Trifluor-methoxy, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Nitro, Trifluormethyl, Difluormethoxy, Trifluormethoxy, -NH(CO)CH₃, Methyl, Ethenyl, (C₁-C₄)-Alkoxy, und Trifluormethyl substituiert sein können,
wobei (C₁-C₄)-Alkoxy mit Hydroxy substituiert sein kann,
- R⁸: für Wasserstoff, Methyl oder Ethyl steht,
oder
- R⁷ und R⁸: zusammen mit dem Kohlenstoffatom, an das sie gebunden sind, einen 3-bis 6-gliedrigen Carbocyclus oder einen Oxetanyl-, Tetrahydrofuranyl-, Tetrahydropyranyl-, Azetidinyl-, Pyrrolidinyl- oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl- und Piperidinyl-Ring mit 1 oder 2 Substituenten unabhängig voneinander ausgewählt aus Fluor und Methyl substituiert sein können,
- R⁹: für Wasserstoff, Cyano, 1,1,2,2-Tetrafluorethyl, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Cyano, Fluor, Trifluormethyl, Difluormethoxy, Trifluormethoxy, Hydroxy, (C₁-C₄)-Alkoxy, 5-gliedriges Heteroaryl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin 5- gliedriges Heteroaryl benzokondensiert oder mit einem 5-gliedrigen Heteroaryl substituiert sein kann,
worin 5- oder 6-gliedriges Heteroaryl mit (C₁-C₄)-Alkyl oder Trifluormethyl substituiert sein kann,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Methyl, Trifluormethoxy, Difluormethoxy, (C₁-C₄)-Alkoxy und Trifluormethyl substituiert sein können,
worin (C₁-C₄)-Alkoxy mit Hydroxy susbstituiert ist,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor, Benzyl und Methyl substituiert sein können, mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen Cyclopentyl-, Cyclohexyl-, Azetidinyl-, Oxetanyl-, Pyrrolidinyl-, Tetrahydrofuranyl-, Piperidinyl- oder Tetrahydropyranyl-Ring bilden, mit der Maßgabe, dass gleichzeitig nicht mehr als eines der Reste-Paare R⁷ und R⁸, R⁹ und R¹⁰ bzw. R⁷ und R⁹ einen der zuvor benannten Carbo- oder Heterocyclen bildet,
R¹¹ für Wasserstoff oder (C₁-C₃)-Alkyl steht,
worin (C₁-C₃)-Alkyl mit 1 oder 2 Substituenten unabhängig voneinander ausgewählt aus der Gruppe Fluor, Trifluormethyl, Hydroxy, Methoxy und Ethoxy substituiert sein kann,
R¹² für Wasserstoff, (C₁-C₄)-Alkyl, Cyclopropyl, Cyclobutyl, Phenyl oder Benzyl steht,
worin (C₁-C₄)-Alkyl mit 1 oder 2 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Fluor, Chlor und Trifluormethyl substituiert sein können, oder
R¹¹ und R¹² zusammen mit dem Stickstoffatom, an das sie gebunden sind, einen Azetidinyl-, Pyrrolidinyl-, Piperidinyl-, Morpholinyl-, Piperazinyl-, Thiomorpholinyl- oder 1, 1 -Dioxothiomorpholinyl-Ring bilden,
worin der Azetidinyl-, Pyrrolidinyl-, Piperidinyl-, Morpholinyl-, Piperazinyl-, Thiomorpholinyl- und 1,1-Dioxothiomorpholinyl-Ring mit 1 oder 2 Substituenten unabhängig voneinander ausgewählt aus der Gruppe Fluor, Trifluormethyl, Methyl, Ethyl, Cyclopropyl, Cyclobutyl, Azetidinyl, Pyrrolidinyl und Piperidinyl substituiert sein können, und
- L²: für eine Bindung, Methylen oder 1,1-Ethandiyl steht,
- R¹³: für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Arabicyclo[3.3.1]nonanyl, 3-Azabicyclo-[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Aza-bicyclo[3.3.1]nonanyl, 3-Azabicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
- R⁴: für Wasserstoff steht,
- R⁵: für Wasserstoff, Fluor, Chlor, Brom, Cyano, Methyl, Ethyl, Monofluormethyl, Ethinyl oder Cyclopropyl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: Phenyl steht,
wobei Phenyl mit 1 bis 2 Fluor substituiert ist,
und
wobei Phenyl mit einem Substituenten ausgewählt aus der Gruppe Cyclopropyl, oder Methoxy substituiert ist,
- R²: für Trifluormethyl, Methyl, Ethyl und Cyclopropyl steht,
- R³: für eine Gruppe der Formel 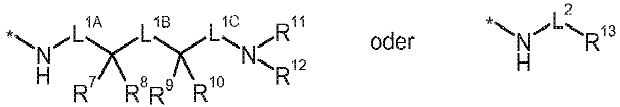 steht, wobei
* für die Anknüpfstelle an die Carbonylgruppe steht,
L^{1A} für eine Bindung steht,
L^{1B} für eine Bindung oder Methylen steht,
L^{1C} für eine Bindung oder Methylen steht,
worin Methylen mit 1 oder 2 Substituenten unabhängig voneinander ausgewählt aus der Gruppe Trifluormethyl, (C₁-C₄)-Alkyl, Cyclopropyl und Cyclobutyl substituiert sein kann,
R⁷ für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano, Nitro, Difluormehtoxy, Trifluormethoxy, NH(CO)CH₃ , Ethenyl, Ethoxy und Trifluormethyl substituiert sein kann,
wobei Ethoxy mit Hydroxy substituiert sein kann,
R⁸ für Wasserstoff, Methyl oder Ethyl steht, oder
R⁷ und R⁸ zusammen mit dem Kohlenstoffatom, an das sie gebunden sind, einen 3-bis 6-gliedrigen Carbocyclus oder einen Oxetanyl-, Tetrahydrofuranyl-, Tetrahydropyranyl-, Azetidinyl-, Pyrrolidinyl- oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl- und Piperidinyl-Ring mit 1 oder 2 Substituenten unabhängig voneinander ausgewählt aus Fluor und Methyl substituiert sein können,
R⁹ für Wasserstoff, Cyano, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Cyano, Fluor, Trifluormethyl, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano, Trifluormethoxy, Difluormethoxy, Ethoxy und Trifluormethyl substituiert sein kann,
worin Ethoxy mit Hydroxy susbstituiert sein kann,
R¹⁰ für Wasserstoff, Methyl oder Ethyl steht, oder
R⁹ und R¹⁰ zusammen mit dem Kohlenstoffatom, an das sie gebunden sind, einen 3-bis 6-gliedrigen Carbocyclus oder einen Oxetanyl-, Tetrahydrofuranyl-, Tetrahydropyranyl-, Azetidinyl-, Pyrrolidinyl- oder Piperidinyl-Ring bilden,
worin der 3- bis 6-gliedrige Carbocyclus, sowie der Oxetanyl-, Tetrahydrofuranyl-, Tetrahydropyranyl-, Azetidinyl-, Pyrrolidinyl- und Piperidinyl-Ring mit 1 oder 2 Substituenten unabhängig voneinander ausgewählt aus Fluor, Benzyl und Methyl substituiert sein können, mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen Cyclopentyl-, Cyclohexyl-. Azetidinyl-, Oxetanyl-, Pyrrolidinyl-, Tetrahydrofuranyl-, Piperidinyl- oder Tetrahydropyranyl-Ring bilden, mit der Maßgabe, dass gleichzeitig nicht mehr als eines der Reste-Paare R⁷ und R⁸, R⁹ und R¹⁰ bzw. R⁷ und R⁹ einen der zuvor benannten Carbo- oder Heterocyclen bildet,
R¹¹ für Wasserstoff oder (C₁-C₃)-Alkyl steht,
worin (C₁-C₃)-Alkyl mit 1 oder 2 Substituenten unabhängig voneinander ausgewählt aus der Gruppe Fluor und Trifluormethyl substituiert sein kann,
R¹² für Wasserstoff, (C₁-C₄)-Alkyl, Cyclopropyl oder Cyclobutyl steht,
worin (C₁-C₄)-Alkyl mit 1 oder 2 Substituenten unabhängig voneinander ausgewählt aus der Gruppe Fluor und Trifluormethyl substituiert sein kann, oder
R¹¹ und R¹² zusammen mit dem Stickstoffatom, an das sie gebunden sind, einen Azetidinyl-, Pyrrolidinyl-, Piperidinyl- oder Morpholinyl-Ring bilden,
worin der Azetidinyl-, Pyrrolidinyl-, Piperidinyl- und Morpholinyl-Ring mit 1 oder 2 Substituenten unabhängig voneinander ausgewählt aus der Gruppe Fluor, Trifluormethyl, Methyl, Ethyl, Cyclopropyl und Cyclobutyl substituiert sein können, und
L² für eine Bindung oder Methylen steht,
R¹³ für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Aza-bicyclo[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, 8-Azabicyclo[3.2.1]octanyl, 9-Azabicyclo-[3.3.1]nonanyl, 3-Aza-bicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
- R⁴: für Wasserstoff steht,
- R⁵: für Wasserstoff, Fluor, Chlor, Methyl, Ethyl, Monofluormethyl, Ethinyl oder Cyclopropyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: Phenyl steht,
wobei Phenyl mit 1 bis 2 Fluor substituiert ist,
und
wobei Phenyl mit einem Substituenten ausgewählt aus der Gruppe Cyclopropyl, oder Methoxy substituiert ist,
- R²: für Methyl steht,
- R³: für eine Gruppe der Formel 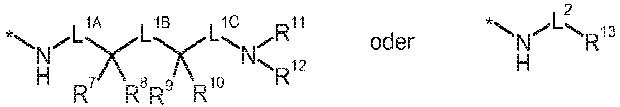 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor, Difluormehtoxy, Trifluormethoxy, Ethenyl, Ethoxy und Chlor substituiert sein kann,
wobei Ethoxy mit Hydroxy substituiert sein kann,
R⁸ für Wasserstoff, Methyl oder Ethyl steht,
R⁹ für Wasserstoff, Cyano, Trifluormethyl, (C₁-C₆)-Alkyl, Cyclopropyl oder Phenyl steht,
worin (C₁-C₆)-Alkyl mit 1 oder 2 Substituenten unabhängig voneinander ausgewählt aus der Gruppe Cyano, Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor, Trifluormethoxy, Difluormethoxy, Ethoxy und Chlor substituiert sein kann,
worin Ethoxy mit Hydroxy susbstituiert sein kann,
R¹⁰ für Wasserstoff, Methyl oder Ethyl steht, oder
R⁹ und R¹⁰ zusammen mit dem Kohlenstoffatom, an das sie gebunden sind, einen 3-bis 6-gliedrigen Carbocyclus oder einen Oxetanylring bilden, mit der Maßgabe, dass die Reste R⁷ und R⁹ nicht beide gleichzeitg für Phenyl stehen,
oder
R⁷ und R⁹ zusammen mit den Kohlenstoffatomen, an die sie gebunden sind, sowie der Gruppe L^{1B} einen Cyclopentyl- oder Cyclohexyl-Ring bilden, mit der Maßgabe, dass gleichzeitig nicht mehr als eines der Reste-Paare R⁹ und R¹⁰ bzw. R⁷ und R⁹ einen der zuvor benannten Carbo- oder Heterocyclen bildet,
R¹¹ für Wasserstoff steht,
R¹² für Wasserstoff steht, und
L² für eine Bindung steht,
R¹³ für Piperidin-2-yl, Piperidin-3-yl, Piperidin-4-yl oder 1,2,3,4-Tetrahydrochinolin-4-yl steht,
worin Piperidin-2-yl, Piperidin-3-yl und Piperidin-4-yl mit 1 bis 5 Substituenten unabhängig voneinander ausgewählt aus Trifluormethyl und Methyl substituiert sein können,
und
worin 1,2,3,4-Tetrahydrochinolin-4-yl mit Fluor oder Trifluormethyl substituiert sein kann,
- R⁴: für Wasserstoff steht,
- R⁵: für Wasserstoff, Fluor, Chlor, Monofluormethyl, Ethinyl oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, (C₃-C₆)-Cycloalkyl, Difluormethoxy und Trifluormethoxy substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 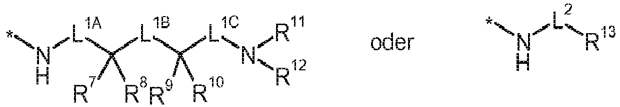 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppen Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy, substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃, (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R⁸ für Wasserstoff oder (C₁-C₄)-Alkyl steht, oder
R⁷ und R⁸ zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für Wasserstoff, Cyano, (C₁-C₆)-Alkyl (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, Cyano, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5-oder 6-gliedriges Heteroaryl substituiert sein kann,
   worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R¹⁰ für Wasserstoff oder (C₁-C₄)-Alkyl steht, oder
R⁹ und R¹⁰ zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können, mit der Maßgabe, dass die Reste R⁷ und R⁹ nicht beide gleichzeitig für Phenyl stehen,
oder
- R⁷ und R⁹: zusammen mit den Kohlenstoffatomen, an die sie gebunden sind, sowie der Gruppe L^{1B} einen 3- bis 7-gliedrigen Carbocyclus oder einen 4- bis 7-gliedrigen Heterocyclus bilden,
worin der 3- bis 7-gliedrigen Carbocyclus mit 1 oder 2 Substituenten unabhängig voneinander ausgewählt aus der Gruppe (C₁-C₄)-Alkyl, Fluor, Hydroxy und (C₁-C₄)-Alkoxy substituiert sein kann,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
- R¹¹: für Wasserstoff oder (C₁-C₄)-Alkyl steht,
worin Gruppe (C₁-C₄)-Alkyl mit 1 bis 3 Substituenten unabhängig voneinander ausgewählt aus der Fluor, Trifluormethyl, Hydroxy und (C₁-C₄)-Alkoxy substituiert sein kann,
- R¹²: für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
- R¹¹ und R¹²: zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann,
und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kein, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,
- R⁴: für Wasserstoff steht,
- R⁵: für Monofluormethyl, Difluormethyl, Trifluormethyl, (C₁-C₄))-Alkenyl, (C₁-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 4 Substituenten unabhängig voneinander ausgewählt aus der Gruppe Fluor, Chlor, Cyano, Methoxy, Ethoxy, Difluormethyl, Trifluormethyl, (C₃-C₆)-Cycloalkyl und Methyl substituiert sein kann,
- R²: für Wasserstoff, Trifluormethyl, (C₁-C₃)-Alkyl und Cyclopropyl steht,
- R³: für eine Gruppe der Formel 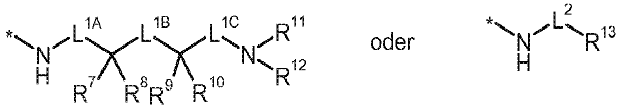 steht, wobei
* für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung oder Methylen steht,
worin Methylen mit 1 oder 2 Substituenten unabhängig voneinander ausgewählt aus der Gruppe Fluor, Trifluormethyl, (C₁-C₄)-Alkyl, Cyclopropyl und Cyclobutyl substituiert sein kann,
R⁷ für Wasserstoff, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Fluor, Trifluormethyl, Difluormethoxy, Trifluor-methoxy, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Nitro, Methyl, Ethenyl, Difluormehtoxy, Trifluormethoxy, -NH(CO)CH₃, (C₁-C₄)-Alkoxy und Trifluormethyl substituiert sein können,
wobei (C₁-C₄)-Alkoxy mit Hydroxy substituiert sein kann,
R⁸ für Wasserstoff, Methyl oder Ethyl steht, oder
R⁷ und R⁸ zusammen mit dem Kohlenstoffatom, an das sie gebunden sind, einen 3-bis 6-gliedrigen Carbocyclus oder einen Oxetanyl-, Tetrahydrofuranyl-, Tetrahydropyranyl-, Azetidinyl-, Pyrrolidinyl- oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl- und Piperidinyl-Ring mit 1 oder 2 Substituenten unabhängig voneinander ausgewählt aus Fluor und Methyl substituiert sein können,
R⁹ für Wasserstoff, Cyano, 1,1,2,2-Tetrafluorethyl, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Cyano, Fluor, Trifluormethyl, Difluormethoxy, Trifluormethoxy, Hydroxy, (C₁-C₄)-Alkoxy, 5-gliedriges Heteroaryl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin 5- gliedriges Heteroaryl benzokondensiert oder mit einem 5-gliedrigen Heteroaryl substituiert sein kann,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Methyl, Difluormethoxy, Trifluormethoxy, (C₁-C₄)-Alkoxy und Trifluormethyl substituiert sein können,
worin (C₁-C₄)-Alkoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3-bis 6-gliedrigen Carbocyclus oder einen Oxetanyl-, Tetrahydrofuranyl-, Tetrahydropyranyl-, Azetidinyl-, Pyrrolidinyl- oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl- und Piperidinyl-Ring mit 1 oder 2 Substituenten unabhängig voneinander ausgewählt aus Fluor, Benzyl und Methyl substituiert sein können, mit der Maßgabe, dass die Reste R⁷ und R⁹ nicht beide gleichzeitig für Phenyl stehen,
oder
R¹¹ und R⁹ zusammen mit den Kohlenstoffatomen, an die sie gebunden sind, sowie der Gruppe L^{1B} einen Cyclopentyl-, Cyclohexyl-, Azetidinyl-, Oxetanyl-, Pyrrolidinyl-, Tetrahydrofuranyl-, Piperidinyl- oder Tetrahydropyranyl-Ring bilden, mit der Maßgabe, dass gleichzeitig nicht mehr als eines der Reste-Paare R⁷ und R⁸, R⁹ und R¹⁰ bzw. R⁷ und R⁹ einen der zuvor benannten Carbo- oder Heterocyclen bildet,
R¹¹ für Wasserstoff oder (C₁-C₃)-Alkyl steht, worin (C₁-C₃)-Alkyl mit 1 oder 2 Substituenten unabhängig voneinander ausgewählt aus der Gruppe Fluor, Trifluormethyl, Hydroxy, Methoxy und Ethoxy substituiert sein kann,
R¹² für Wasserstoff, (C₁-C₄)-Alkyl, Cyclopropyl, Cyclobutyl, Phenyl oder Benzyl steht, worin (C₁-C₄)-Alkyl mit 1 oder 2 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Fluor, Chlor und Trifluormethyl substituiert sein können, oder
R¹¹ und R¹² zusammen mit dem Stickstoffatom, an das sie gebunden sind, einen Azetidinyl-, Pyrrolidinyl-, Piperidinyl-, Morpholinyl-, Piperazinyl-, Thiomorpholinyl- oder 1,1-Dioxothiomorpholinyl-Ring bilden,
worin der Azetidinyl-, Pyrrolidinyl,-, Piperidinyl-, Morpholinyl-, Piperazinyl-, Thiomorpholinyl- und 1,1-Dioxothiomorpholinyl-Ring mit 1 oder 2 Substituenten unabhängig voneinander ausgewählt aus der Gruppe Fluor, Trifluormethyl, Methyl, Ethyl, Cyclopropyl, Cyclobutyl, Azetidinyl, Pyrrolidinyl und Piperidinyl substituiert sein können, und
L² für eine Bindung, Methylen oder 1,1-Ethandiyl steht,
R¹³ für über ein Ring-Kohlenstoffatom gebundenes Pyrrolidinyl, Piperidinyl, 1,2,3,4-Tetrahydroisochinolinyl, 1,2,3,4-Tetrahydrochinolinyl, lndolinyl, 8-Azabicyclo[3.2.1]octanyl, 9-Arabicyclo[3.3.1]nonanyl, 3-Azabicyclo-[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, lndolinyl, 8-Azabicyclo[3.2.1]octanyl, 9-Aza-bicyclo[3.3.1]nonanyl, 3-Azabicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
- R⁴: für Wasserstoff steht,
- R⁵: für Monofluormethyl, Difluormethyl, Trifluormethyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, 5-bis 6-gliedriges Heterocyclyl oder 5- oder 6-gliedriges Heteroaryl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 3 Substituenten unabhängig voneinander ausgewählt aus der Gruppe Fluor, Chlor, Cyclopropyl, Methoxy und Methyl substituiert sein kann,
R² für Trifluormethyl, Methyl, Ethyl und Cyclopropyl steht,
R³ für eine Gruppe der Formel 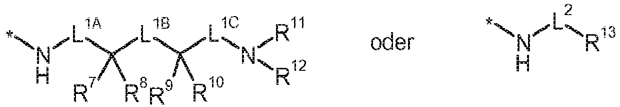 steht, wobei
* für die Anknüpfstelle an die Carbonylgruppe steht,
L^{1A} für eine Bindung steht,
L^{1B} für eine Bindung oder Methylen steht,
L^{1C} für eine Bindung oder Methylen steht,
worin Methylen mit 1 oder 2 Substituenten unabhängig voneinander ausgewählt aus der Gruppe Trifluormethyl, (C₁-C₄)-Alkyl, Cyclopropyl und Cyclobutyl substituiert sein kann,
R⁷ für Wasserstoff, Trifluormethyl, (C₁-C₆)-Alkyl, (C₃-C₆)-Cycloalkyl oder Phenyl steht,
worin (C₁-C₆)-Allryl mit 1 oder 2 Substituenten unabhängig voneinander ausgewählt aus der Gruppe Fluor, Trifluormethyl, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano, Nitro, Ethenyl, Difluormehtoxy, Trifluormethoxy, -NH(CO)CH₃, Ethoxy und Trifluormethyl substituiert sein kann,
wobei Ethoxy mit Hydroxy substituiert sein kann,
R⁸ für Wasserstoff, Methyl oder Ethyl steht, oder
R⁷ und R⁸ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor und Methyl substituiert sein können,
R⁹ für Wasserstoff, Cyano, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Cyano, Fluor, Trifluormethyl, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano, Difluormethoxy, Trifluormethoxy, Ethoxy und Trifluormethyl substituiert sein kann,
worin Ethoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus, sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor, Benzyl und Methyl substituiert sein können, mit der Maßgabe, dass die Reste R⁷ und R⁹ nicht beide gleichzeitig für Phenyl stehen,
oder
R¹¹ und R⁹ zusammen mit den Kohlenstoffatomen, an die sie gebunden sind, sowie der Gruppe L^{1B} einen Cyclopentyl-, Cyclohexyl-. Azetidinyl-, Oxetanyl-, Pyrrolidinyl-, Tetrahydrofuranyl-, Piperidinyl- oder Tetrahydropyranyl-Ring bilden, mit der Maßgabe, dass gleichzeitig nicht mehr als eines der Reste-Paare R⁷ und R⁸, R⁹ und R¹⁰ bzw. R⁷ und R⁹ einen der zuvor benannten Carbo- oder Heterocyclen bildet,
R¹¹ für Wasserstoff oder (C₁-C₃)-Alkyl steht,
worin (C₁-C₃)-Alkyl mit 1 oder 2 Substituenten unabhängig voneinander ausgewählt aus der Gruppe Fluor und Trifluormethyl substituiert sein kann,
R¹² für Wasserstoff, (C₁-C₄)-Alkyl, Cyclopropyl oder Cyclobutyl steht,
worin (C₁-C₄)-Alkyl mit 1 oder 2 Substituenten unabhängig voneinander ausgewählt aus der Gruppe Fluor und Trifluormethyl substituiert sein kann, oder
R¹¹ und R¹² zusammen mit dem Stickstoffatom, an das sie gebunden sind, einen Azetidinyl-, Pyrrolidinyl-, Piperidinyl- oder Morpholinyl-Ring bilden,
worin der Azetidinyl-, Pyrrolidinyl-, Piperidinyl- und Morpholinyl-Ring mit 1 oder 2 Substituenten unabhängig voneinander ausgewählt aus der Gruppe Fluor, Trifluormethyl, Methyl, Ethyl, Cyclopropyl und Cyclobutyl substituiert sein können,
und
- L²: für eine Bindung oder Methylen steht,
R¹³ für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Aza-bicyclo[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, 8-Azabicyclo[3.2.1]octanyl, 9-Azabicyclo-[3.3.1]nonanyl, 3-Aza-bicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
R⁴ für Wasserstoff steht,
R⁵ für Monofluormethyl, Difluormethyl, Trifluormethyl, Cyclopropyl, Ethinyl, Methoxy, Morpholino steht,
R⁶ für Wasserstoff steht, sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für eine Phenyl-Gruppe der Formel 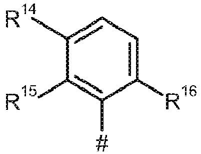 steht, wobei
# für die Anknüpfstelle an A steht,
und
R¹⁴, R¹⁵ und R¹⁶ unabhängig voneinander für Wasserstoff, Fluor, Methoxy, Cyclopropyl, oder Chlor stehen, mit der Maßgabe, dass mindestens zwei der Reste R¹⁴, R¹⁵, R¹⁶ von Wasserstoff verschieden sind,
- R²: für Methyl steht,
- R³: für eine Gruppe der Formel 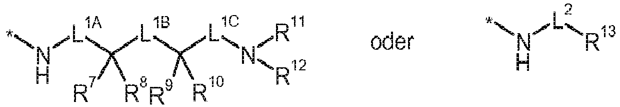 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor, Ethenyl, Difluormehtoxy, Trifluormethoxy, Ethoxy und Chlor substituiert sein kann,
wobei Ethoxy mit Hydroxy substituiert sein kann,
R⁸ für Wasserstoff, Methyl oder Ethyl steht,
R⁹ für Wasserstoff, Cyano, Trifluormethyl, (C₁-C₆)-Alkyl, Cyclopropyl oder Phenyl steht,
worin (C₁-C₆)-Alkyl mit 1 oder 2 Substituenten unabhängig voneinander ausgewählt aus der Gruppe Cyano, Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor, Difluormethoxy, Trifluormethoxy, Ethoxy und Chlor substituiert sein kann,
worin Ethoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanylring bilden, mit der Maßgabe, dass die Reste R⁷ und R⁹ nicht beide gleichzeitg für Phenyl stehen,
oder
R⁷ und R⁹ zusammen mit den Kohlenstoffatomen, an die sie gebunden sind, sowie der Gruppe L^{1B} einen Cyclopentyl- oder Cyclohexyl-Ring bilden, mit der Maßgabe, dass gleichzeitig nicht mehr als eines der Reste-Paare R⁹ und R¹⁰ bzw. R⁷ und R⁹ einen der zuvor benannten Carbo- oder Heterocyclen bildet,
R¹¹ für Wasserstoff steht,
R¹² für Wasserstoff steht, und
L² für eine Bindung steht,
R¹³ für Piperidin-2-yl, Piperidin-3-yl, Piperidin-4-yl oder 1,2,3,4-Tetrahydrochinolin-4-yl steht,
worin Piperidin-2-yl, Piperidin-3-yl und Piperidin-4-yl mit 1 bis 5 Substituenten unabhängig voneinander ausgewählt aus Trifluormethyl und Methyl substituiert sein können,
und
worin 1,2,3,4-Tetrahydrochinolin-4-yl mit Fluor oder Trifluormethyl substituiert sein kann,
- R⁴: für Wasserstoff steht,
- R⁵: für Monofluormethyl, Difluormethyl, Trifluormethyl, Cyclopropyl, Ethinyl, Methoxy, Morpholino steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, (C₃-C₆)-Cycloalkyl, Difluormethoxy und Trifluormethoxy substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 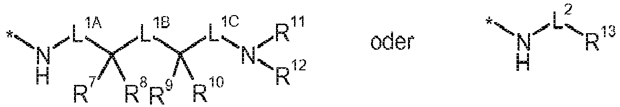 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppen Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für (C₁-C₆)-Alkyl, (C₂-C₆)-Alkinyl, Cyano oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy und Phenoxy substituiert ist,
worin Phenoxy mit 1 bis 3 Substituenten Halogen substituiert sein kann,
worin Phenyl mit 1 bis 2 Substituenten unabhängig voneinander ausgewählt aus der Gruppe Cyano, Nitro, Difluormethoxy, Trifluormethoxy, (C₁-C₄)-Alkoxy, -NH(CO)CH₃ und (C₁-C₄)-Alkenyl substituiert ist,
wobei (C₁-C₄)-Alkoxy mit Hydroxy substituiert ist,
R⁸ für Wasserstoff oder (C₁-C₄)-Alkyl steht,
R⁹ für Wasserstoff, Cyano, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, Cyano, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5- oder 6-gliedriges Heteroaryl substituiert sein kann,
worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R¹⁰ für Wasserstoff oder (C₁-C₄)-Alkyl steht, oder
R⁹ und R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können, mit der Maßgabe, dass die Reste R⁷ und R⁹ nicht beide gleichzeitig für Phenyl stehen,
R¹¹ für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
R¹² für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann,
und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kein, der seinerseits mit 1 oder 2 Substituenten ausgewählt aus Halogen, (C₁-C₄)-Alkyl, (C₁-C₄)-Alkoxy und Trifluormethyl substituiert sein kann, oder
für Adamantyl steht,
- R⁴: für Wasserstoff steht,
- R⁵: für Wasserstoff, Halogen, Cyano, Monofluormethyl, Difluormethyl, Trifluormethyl, (C₁-C₄)-Alkyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 4 Substituenten unabhängig voneinander ausgewählt aus der Gruppe Fluor, Chlor, Cyano, Methoxy, Ethoxy, Difluormethyl, Trifluormethyl, (C₃-C₆)-Cycloalkyl und Methyl substituiert sein kann,
- R²: für Wasserstoff, Trifluormethyl, (C₁-C₃)-Alkyl und Cyclopropyl steht,
- R³: für eine Gruppe der Formel 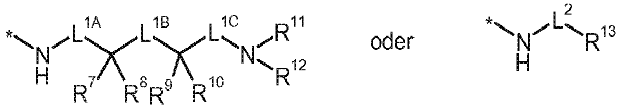 steht, wobei
* für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung oder Methylen steht,
worin Methylen mit 1 oder 2 Substituenten unabhängig voneinander ausgewählt aus der Gruppe Fluor, Trifluormethyl, (C₁-C₄)-Alkyl, Cyclopropyl und Cyclobutyl substituiert sein kann,
R⁷ für (C₁-C₆)-Alkyl, (C₂-C₆)-Alkinyl, Cyano oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy und Phenoxy substituiert ist,
worin Phenoxy mit 1 bis 3 Fluor substituiert sein kann,
worin Phenyl mit 1 bis 2 Substituenten unabhängig voneinander ausgewählt aus der Gruppe Cyano, Nitro, Difluormethoxy, Trifluormethoxy, (C₁-C₄)-Alkoxy, -NH(CO)CH₃ und (C₁-C₄)-Alkenyl substituiert ist,
wobei (C₁-C_{A})-Alkoxy mit Hydroxy substituiert ist,
R⁸ für Wasserstoff oder (C₁-C₄)-Alkyl steht,
R⁹ für Wasserstoff, Cyano, 1,1,2,2-Tetrafluorethyl, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Cyano, Fluor, Trifluormethyl, Difluormethoxy, Trifluormethoxy, Hydroxy, (C₁-C₄)-Alkoxy, 5-gliedriges Heteroaryl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin 5- gliedriges Heteroaryl benzokondensiert oder mit einem 5-gliedrigen Heteroaryl substituiert sein kann,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Methyl, Difluormethoxy, Trifluormethoxy, (C₁-C₄)-Alkoxy und Trifluormethyl substituiert sein können,
worin (C₁-C₄)-Alkoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor, Benzyl und Methyl substituiert sein können, mit der Maßgabe, dass die Reste R⁷ und R⁹ nicht beide gleichzeitig für Phenyl stehen,
oder
R¹¹ für Wasserstoff oder (C₁-C₃)-Alkyl steht,
worin (C₁-C₃)-Alkyl mit 1 oder 2 Substituenten unabhängig voneinander ausgewählt aus der Gruppe Fluor, Trifluormethyl, Hydroxy, Methoxy und Ethoxy substituiert sein kann,
R¹² für Wasserstoff, (C₁-C₄)-Alkyl, Cyclopropyl, Cyclobutyl, Phenyl oder Benzyl steht,
worin (C₁-C₄)-Alkyl mit 1 oder 2 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Fluor, Chlor und Trifluormethyl substituiert sein können,
oder
R¹¹ und R¹² zusammen mit dem Stickstoffatom, an das sie gebunden sind, einen Azetidinyl-, Pyrrolidinyl-, Piperidinyl-, Morpholinyl-, Piperazinyl-, Thiomorpholinyl- oder 1,1-Dioxothiomorpholinyl-Ring bilden,
worin der Azetidinyl-, Pyrrolidinyl-, Piperidinyl-, Morpholinyl-, Piperazinyl-, Thiomorpholinyl- und 1,1-Dioxothiomorpholinyl-Ring mit 1 oder 2 Substituenten unabhängig voneinander ausgewählt aus der Gruppe Fluor, Trifluormethyl, Methyl, Ethyl, Cyclopropyl, Cyclobutyl, Azetidinyl, Pyrrolidinyl und Piperidinyl substituiert sein können,
und
- L²: für eine Bindung, Methylen oder 1,1-Ethandiyl steht,
- R¹³: für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Azabicyclo-[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Aza-bicyclo[3.3.1]nonanyl, 3-Azabicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
- R⁴: für Wasserstoff steht,
- R⁵: für Wasserstoff, Fluor, Chlor, Brom, Cyano, Methyl, Ethyl, Monofluormethyl, ; Methoxy, Ethinyl oder Cyclopropyl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 3 Substituenten unabhängig voneinander ausgewählt aus der Gruppe Fluor, Chlor, Cyclopropyl, Methoxy und Methyl substituiert sein kann,
- R²: für Trifluormethyl, Methyl, Ethyl und Cyclopropyl steht,
- R³: für eine Gruppe der Formel 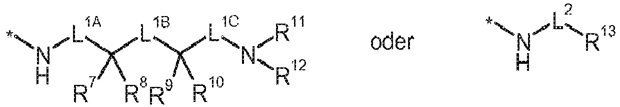 steht, wobei
* für die Anknüpfstelle an die Carbonylgruppe steht,
L^{1A} für eine Bindung steht,
L^{1B} für eine Bindung oder Methylen steht,
L^{1C} für eine Bindung oder Methylen steht,
worin Methylen mit 1 oder 2 Substituenten unabhängig voneinander ausgewählt aus der Gruppe Trifluormethyl, (C₁-C₄)-Alkyl, Cyclopropyl und Cyclobutyl substituiert sein kann,
R⁷ für (C₁-C₄)-Alkyl, Cyano oder Phenyl steht,
worin (C₁-C₄)-Alkyl mit 1 bis 3 Substituenten unabhängig voneinander ausgewählt aus der Gruppe Fluor, Trifluormethyl und Phenoxy substituiert ist,
worin Phenoxy mit 1 bis 3 Substituenten Fluor substituiert sein kann,
worin Phenyl mit 1 bis 2 Substituenten unabhängig voneinander ausgewählt aus der Gruppe Cyano, Difluormethoxy, Trifluormethoxy, Ethoxy, -NH(CO)CH₃ und Ethenyl substituiert ist,
wobei Ethoxy mit Hydroxy substituiert ist,
R⁸ für Wasserstoff steht,
R⁹ für Wasserstoff, Cyano, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Cyano, Fluor, Trifluormethyl, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Difluormethoxy, Trifluormethoxy, Ethoxy, Cyano und Trifluormethyl substituiert sein kann,
worin Ethoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus, sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor, Benzyl und Methyl substituiert sein können, mit der Maßgabe, dass die Reste R⁷ und R⁹ nicht beide gleichzeitig für Phenyl stehen,
oder
R¹¹ für Wasserstoff oder (C₁-C₃)-Alkyl steht,
worin (C₁-C₃)-Alkyl mit 1 oder 2 Substituenten unabhängig voneinander ausgewählt aus der Gruppe Fluor und Trifluormethyl substituiert sein kann,
R¹² für Wasserstoff, (C₁-C₄)-Alkyl, Cyclopropyl oder Cyclobutyl steht, worin (C₁-C₄)-Alkyl mit 1 oder 2 Substituenten unabhängig voneinander ausgewählt aus der Gruppe Fluor und Trifluormethyl substituiert sein kann,
oder
R¹¹ und R¹² zusammen mit dem Stickstoffatom, an das sie gebunden sind, einen Azetidinyl-, Pyrrolidinyl-, Piperidinyl- oder Morpholinyl-Ring bilden,
worin der Azetidinyl-, Pyrrolidinyl-, Piperidinyl- und Morpholinyl-Ring mit 1 oder 2 Substituenten unabhängig voneinander ausgewählt aus der Gruppe Fluor, Trifluormethyl, Methyl, Ethyl, Cyclopropyl und Cyclobutyl substituiert sein können, und
- L²: für eine Bindung oder Methylen steht,
R¹³ für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Aza-bicyclo[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, 8-Azabicyclo[3.2.1]octanyl, 9-Azabicyclo-[3.3.1]nonanyl, 3-Aza-bicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
R⁴ für Wasserstoff steht,
R⁵ für Wasserstoff, Fluor, Chlor, Methyl, Ethyl, Monofluormethyl, Methoxy, Ethinyl oder Cyclopropyl steht,
R⁶ für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für eine Phenyl-Gruppe der Formel 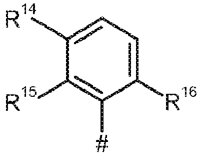 steht, wobei
# für die Anknüpfstelle an A steht,
und
R¹⁴, R¹⁵ und R¹⁶ unabhängig voneinander für Wasserstoff, Fluor, Methoxy, Cyclopropyl oder Chlor stehen, mit der Maßgabe, dass mindestens zwei der Reste R¹⁴, R'⁵, R¹⁶ von Wasserstoff verschieden sind,
- R²: für Methyl steht,
- R³: für eine Gruppe der Formel 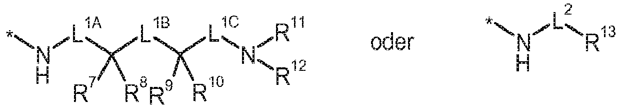 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für (C₁-C₄)-Alkyl, Cyano oder Phenyl steht,
worin (C₁-C₄)-Alkyl bis zu fünffach mit Fluor substituiert ist,
und
worin Phenyl mit Cyano, Difluormethoxy, Trifluormethoxy, Ethoxy, -NH(CO)CH₃ oder Ethenyl substituiert ist,
wobei Ethoxy mit Hydroxy substituiert ist,
R⁸ für Wasserstoff steht,
R⁹ für Wasserstoff, Cyano, Trifluormethyl, (C₁-C₆)-Alkyl, Cyclopropyl oder Phenyl steht,
worin (C₁-C₆)-Alkyl mit 1 oder 2 Substituenten unabhängig voneinander ausgewählt aus der Gruppe Cyano, Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor, Difluormethoxy, Trifluormethoxy, Ethoxy, und Chlor substituiert sein kann,
worin Ethoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanylring bilden, mit der Maßgabe, dass die Reste R⁷ und R⁹ nicht beide gleichzeitg für Phenyl stehen,
R¹¹ für Wasserstoff steht,
R¹² für Wasserstoff steht,
und
L² für eine Bindung steht,
R¹³ für Piperidin-2-yl, Piperidin-3-yl, Piperidin-4-yl oder 1,2,3,4-Tetrahydrochinolin-4-yl steht,
worin Piperidin-2-yl, Piperidin-3-yl und Piperidin-4-yl mit 1 bis 5 Substituenten unabhängig voneinander ausgewählt aus Trifluormethyl und Methyl substituiert sein können,
und
worin 1,2,3,4-Tetrahydrochinolin-4-yl mit Fluor oder Trifluormethyl substituiert sein kann,
- R⁴: für Wasserstoff steht,
- R⁵: für Wasserstoff, Fluor, Chlor, Monofluormethyl, Ethinyl oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, (C₃-C₆)-Cycloalkyl, Difluormethoxy und Trifluormethoxy substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 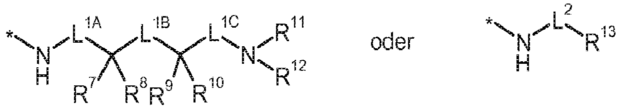 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy, substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃, (C₁-C_{A})-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R⁸ für Wasserstoff oder (C₁-C₄)-Alkyl steht, oder
R⁷ und R⁸ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für (C₁-C₆)-Alkyl, Cyano oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, 5- oder 6-gliedriges Heteroaryl, Phenoxy und Benzyloxy substituiert ist,
worin Phenoxy mit 1 bis 3 Substituenten Halogen substituiert ist,
worin Benzyloxy mit 1 bis 3 Substituenten Halogen substituiert sein kann,
worin 5- oder 6-gliedriges Heteroaryl mit einem 5- oder 6-gliedriges Heteroaryl, substituiert ist,
worin 5- oder 6-gliedriges Heteroaryl seinerseits mit (C₁-C₄)-Alkyl substituiert sein kann,
worin Phenyl mit 1 bis 2 Substituenten unabhängig voneinander ausgewählt aus der Gruppe Cyano, Difluormethoxy, Trifluormethoxy und (C₁-C₄)-Alkoxy substituiert ist,
wobei (C₁-C₄)-Alkoxy mit Hydroxy substituiert ist,
R¹⁰ für Wasserstoff oder (C₁-C₄)-Alkyl steht,
R¹¹ für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
R¹² für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können, oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann, und
L² für eine Bindung oder (C₁-C₄)-Alkandiyl steht,
R¹³ für 5- bis 9-gliedriges, über ein Ring-Kohlenstoffatom gebundenes Asa-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kein, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,
- R⁴: für Wasserstoff steht,
- R⁵: für Wasserstoff, Halogen, Cyano, Monofluormethyl, Difluormethyl, Trifluormethyl, (C₁-C₄)-Alkyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 4 Substituenten unabhängig voneinander ausgewählt aus der Gruppe Fluor, Chlor, Cyano, Methoxy, Ethoxy, Difluormethyl, Trifluormethyl, (C₃-C₆)-Cycloalkyl und Methyl substituiert sein kann,
- R²: für Wasserstoff, Trifluormethyl, (C₁-C₃)-Alkyl und Cyclopropyl steht,
- R³: für eine Gruppe der Formel 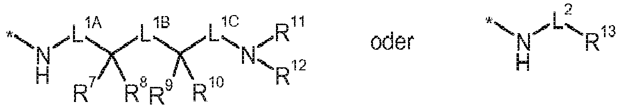 steht, wobei
* für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung oder Methylen steht,
worin Methylen mit 1 oder 2 Substituenten unabhängig voneinander ausgewählt aus der Gruppe Fluor, Trifluormethyl, (C₁-C₄)-Alkyl, Cyclopropyl und Cyclobutyl substituiert sein kann,
R⁷ für Wasserstoff, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Fluor, Trifluormethyl, Difluormethoxy, Trifluormethoxy, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Methyl, Ethenyl, Nitro, Difluormehtoxy, Trifluormethoxy, -NH(CO)CH₃ , (C₁-C₄)-Alkoxy, und Trifluormethyl substituiert sein können,
wobei (C₁-C₄)-Alkoxy mit Hydroxy substituiert sein kann,
R⁸ für Wasserstoff, Methyl oder Ethyl steht, oder
R⁷ und R⁸ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor und Methyl substituiert sein können,
R⁹ für (C₁-C₄)-Alkyl, Cyano oder Phenyl steht,
worin (C₁-C₄)-Alkyl mit 1 bis 3 Substituenten unabhängig voneinander ausgewählt aus der Gruppe Fluor, Difluormethyl, Trifluormethyl, Difluormethoxy, Trifluormethoxy, 5-gliedriges Heteroaryl und Benzyloxy substituiert ist,
worin Benzyloxy mit 1 bis 3 Substituenten Halogen substituiert sein kann,
worin 5-gliedriges Heteroaryl mit einem 5-gliedrigen Heteroaryl, substituiert ist,
worin 5-gliedriges Heteroaryl seinerseits mit (C₁-C₄)-Alkyl substituiert sein kann,
worin Phenyl mit 1 bis 2 Substituenten unabhängig voneinander ausgewählt aus der Gruppe Cyano, Difluormethoxy, Trifluormethoxy und (C₁-C₄)-Alkoxy substituiert ist,
wobei (C₁-C₄)-Alkoxy mit Hydroxy substituiert ist,
R¹⁰ für Wasserstoff oder Metyhl steht,
R¹¹ für Wasserstoff oder (C₁-C₃)-Alkyl steht,
worin (C₁-C₃)-Alkyl mit 1 oder 2 Substituenten unabhängig voneinander ausgewählt aus der Gruppe Fluor, Trifluormethyl, Hydroxy, Methoxy und Ethoxy substituiert sein kann,
R¹² für Wasserstoff, (C₁-C₄)-Alkyl, Cyclopropyl, Cyclobutyl, Phenyl oder Benzyl steht,
worin (C₁-C₄)-Alkyl mit 1 oder 2 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Fluor, Chlor und Trifluormethyl substituiert sein können,
oder
R¹¹ und R¹² zusammen mit dem Stickstoffatom, an das sie gebunden sind, einen Azetidinyl-, Pyrrolidinyl-, Piperidinyl-, Morpholinyl-, Piperazinyl-, Thiomorpholinyl- oder 1,1-Dioxothiomorpholinyl-Ring bilden, worin der Azetidinyl-, Pyrrolidinyl-, Piperidinyl-, Morpholinyl-, Piperazinyl-, Thiomorpholinyl- und 1,1-Dioxothiomorpholinyl-Ring mit 1 oder 2 Substituenten unabhängig voneinander ausgewählt aus der Gruppe Fluor, Trifluormethyl, Methyl, Ethyl, Cyclopropyl, Cyclobutyl, Azetidinyl, Pyrrolidinyl und Piperidinyl substituiert sein können,
und
- L²: für eine Bindung, Methylen oder 1,1-Ethandiyl steht,
- R¹³: für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Azabicyclo-[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Aza-bicyclo[3.3.1]nonanyl, 3-Azabicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
- R⁴: für Wasserstoff steht,
- R⁵: für Wasserstoff, Fluor, Chlor, Brom, Cyano, Methyl, Ethyl, Monofluormethyl, Methoxy, Ethinyl oder Cyclopropyl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 3 Substituenten unabhängig voneinander ausgewählt aus der Gruppe Fluor, Chlor, Cyclopropyl, Methoxy und Methyl substituiert sein kann,
- R²: für Trifluormethyl, Methyl, Ethyl und Cyclopropyl steht,
- R³: für eine Gruppe der Formel 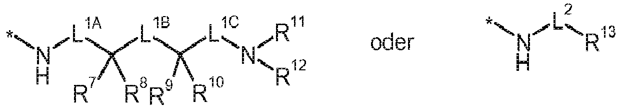 steht, wobei
* für die Anknüpfstelle an die Carbonylgruppe steht,
L^{1A} für eine Bindung steht,
L^{1B} für eine Bindung oder Methylen steht,
L^{1C} für eine Bindung oder Methylen steht,
worin Methylen mit 1 oder 2 Substituenten unabhängig voneinander ausgewählt aus der Gruppe Trifluormethyl, (C₁-C₄)-Alkyl, Cyclopropyl und Cyclobutyl substituiert sein kann,
R⁷ für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano, Nitro, Difluormehtoxy, Trifluormethoxy, -NH(CO)CH₃ , Ethenyl, Ethoxy und Trifluormethyl substituiert sein kann,
wobei Ethoxy mit Hydroxy substituiert sein kann,
R⁸ für Wasserstoff, Methyl oder Ethyl steht, oder
R⁷ und R⁸ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor und Methyl substituiert sein können,
R⁹ für Ethyl, Propyl, Cyano oder Phenyl steht,
worin Ethyl und Propyl mit 1 bis 3 Substituenten unabhängig voneinander ausgewählt aus der Gruppe Fluor, Trifluormethyl und Benzyloxy substituiert ist,
worin Benzyloxy mit 1 bis 3 Substituenten Halogen substituiert sein kann,
worin Phenyl mit Cyano, Difluormethoxy, Trifluormethoxy oder Ethoxy substituiert ist,
wobei Ethoxy mit Hydroxy substituiert ist,
R¹⁰ für Wasserstoff oder Methyl steht,
R¹¹ für Wasserstoff oder (C₁-C₃)-Alkyl steht,
worin (C₁-C₃)-Alkyl mit 1 oder 2 Substituenten unabhängig voneinander ausgewählt aus der Gruppe Fluor und Trifluormethyl substituiert sein kann,
R¹² für Wasserstoff, (C₁-C₄)-Alkyl, Cyclopropyl oder Cyclobutyl steht,
worin (C₁-C₄)-Alkyl mit 1 oder 2 Substituenten unabhängig voneinander ausgewählt aus der Gruppe Fluor und Trifluormethyl substituiert sein kann,
oder
R¹¹ und R¹² zusammen mit dem Stickstoffatom, an das sie gebunden sind, einen Azetidinyl-, Pyrrolidinyl-, Piperidinyl- oder Morpholinyl-Ring bilden,
worin der Azetidinyl-, Pyrrolidinyl-, Piperidinyl- und Morpholinyl-Ring mit 1 oder 2 Substituenten unabhängig voneinander ausgewählt aus der Gruppe Fluor, Trifluormethyl, Methyl, Ethyl, Cyclopropyl und Cyclobutyl substituiert sein können,
und
- L²: für eine Bindung oder Methylen steht,
R¹³ für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Aza-bicyclo[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, 8-Azabicyclo[3.2.1]octanyl, 9-Azabicyclo-[3.3.1]nonanyl, 3-Aza-bicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
- R⁴: für Wasserstoff steht,
- R⁵: für Wasserstoff, Fluor, Chlor, Methyl, Ethyl, Monofluormethyl, Methoxy, Ethinyl oder Cyclopropyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für eine Phenyl-Gruppe der Formel 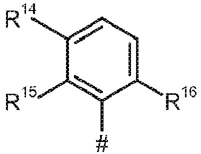 steht, wobei
# für die Anknüpfstelle an A steht, und
R¹⁴, R¹⁵ und R¹⁶ unabhängig voneinander für Wasserstoff, Fluor, Methoxy, Cyclopropyl oder Chlor stehen,
mit der Maßgabe, dass mindestens zwei der Reste R¹⁴, R¹⁵, R¹⁶ von Wasserstoff verschieden sind,
- R²: für Methyl steht,
- R³: für eine Gruppe der Formel 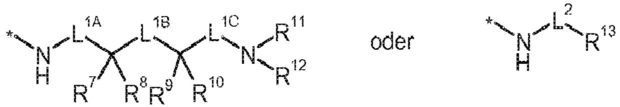 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor, Difluormethoxy, Trifluormethoxy, Ethenyl, Ethoxy und Chlor substituiert sein kann,
wobei Ethoxy mit Hydroxy substituiert sein kann,
R⁸ für Wasserstoff, Methyl oder Ethyl steht,
R⁹ für Ethyl, Cyano oder Phenyl steht,
worin Ethyl bis zu fünffach mit Fluor substituiert ist,
worin Phenyl mit Cyano, Difluormethoxy, Trifluormethoxy oder Ethoxy substituiert ist,
wobei Ethoxy mit Hydroxy substituiert ist,
R¹⁰ für Wasserstoff oder Methyl steht,
R¹¹ für Wasserstoff steht,
R¹² für Wasserstoff steht, und
L² für eine Bindung steht,
R¹³ für Piperidin-2-yl, Piperidin-3-yl, Piperidin-4-yl oder 1,2,3,4-Tetrahydrochinolin-4-yl steht,
worin Piperidin-2-yl, Piperidin-3-yl und Piperidin-4-yl mit 1 bis 5 Substituenten unabhängig voneinander ausgewählt aus Trifluormethyl und Methyl substituiert sein können,
und
worin 1,2,3,4-Tetrahydrochinolin-4-yl mit Fluor oder Trifluormethyl substituiert sein kann,
- R⁴: für Wasserstoff steht,
- R⁵: für Wasserstoff, Fluor, Chlor, Monofluormethyl, Methoxy, Ethinyl oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, (C₃-C₆)-Cycloalkyl, Difluormethoxy und Trifluormethoxy substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 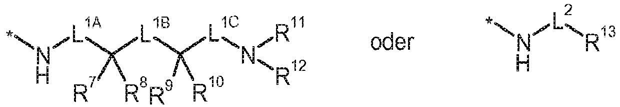 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann, worin Phenyl, Phenoxy und Benzyloxy ihrerseits mit 1 bis 3 Substituenten Halogen oder (C₁-C₄)-Alkoxy, substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃, (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin an 2 benachbarten Kohlenstoffatomen des Phenyls mit einer Difluormthylendioxy-Brücke substituiert sein kann,
R⁸ für Wasserstoff oder (C₁-C₄)-Alkyl steht, oder
R⁷ und R⁸ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für Wasserstoff, Cyano, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, Cyano, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5- oder 6-gliedriges Heteroaryl substituiert sein kann,
worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R¹⁰ für Wasserstoff oder (C₁-C₄)-Alkyl steht, oder
R⁹ und R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können, mit der Maßgabe, dass die Reste R⁷ und R⁹ nicht beide gleichzeitig für Phenyl stehen, oder
R⁷ und R⁹ zusammen mit den Kohlenstoffatomen, an die sie gebunden sind, sowie der Gruppe L^{1B} einen 3- bis 7-gliedrigen Carbocyclus oder einen 4- bis 7-gliedrigen Heterocyclus bilden,
worin der 3- bis 7-gliedrige Carbocyclus mit 1 oder 2 Substituenten unabhängig voneinander ausgewählt aus der Gruppe (C₁-C₄)-Alkyl, Fluor, Hydroxy und (C₁-C₄)-Alkoxy substituiert sein kann, mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
R¹¹ für (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert ist,
R¹² für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können, oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe (C₃-C₇)-Cycloalkyl und 4- bis 7-gliedriges Heterocyclyl substituiert ist, und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kann, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,
- R⁴: für Wasserstoff steht,
- R⁵: für Wasserstoff, Halogen, Cyano, Monofluormethyl, Difluormethyl, Trifluormethyl, (C₁-C₄)-Alkyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 4 Substituenten unabhängig voneinander ausgewählt aus der Gruppe Fluor, Chlor, Cyano, Methoxy, Ethoxy, Difluormethyl, Trifluormethyl, (C₃-C₆)-Cycloalkyl und Methyl substituiert sein kann,
- R²: für Wasserstoff, Trifluormethyl, (C₁-C₃)-Alkyl und Cyclopropyl steht,
- R³: für eine Gruppe der Formel 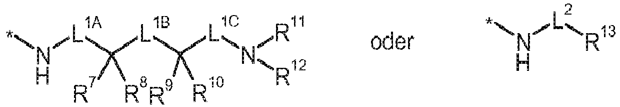 steht, wobei
* für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung oder Methylen steht,
worin Methylen mit 1 oder 2 Substituenten unabhängig voneinander ausgewählt aus der Gruppe Fluor, Trifluormethyl, (C₁-C₄)-Alkyl, Cyclopropyl und Cyclobutyl substituiert sein kann,
R⁷ für Wasserstoff, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Fluor, Trifluormethyl, Difluormethoxy, Trifluormethoxy, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Nitro, Methyl, Ethenyl, Difluormehtoxy, Trifluormethoxy, -NH(CO)CH₃, (C₁-C₄)-Alkoxy und Trifluormethyl substituiert sein können,
wobei (C₁-C₄)-Alkoxy mit Hydroxy substituiert sein kann,
R⁸ für Wasserstoff, Methyl oder Ethyl steht, oder
R⁷ und R⁸ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor und Methyl substituiert sein können,
R⁹ für Wasserstoff, Cyano, 1,1,2,2-Tetrafluorethyl, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Cyano, Fluor, Trifluormethyl, Difluormethoxy, Trifluormethoxy, Hydroxy, (C₁-C₄)-Alkoxy, 5-gliedriges Heteroaryl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin 5- gliedriges Heteroaryl benzokondensiert oder mit einem 5-gliedrigen Heteroaryl substituiert sein kann,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Methyl, Difluormethoxy, Trifluormethoxy, (C₁-C₄)-Alkoxy und Trifluormethyl substituiert sein können,
worin (C₁-C₄)-Alkoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor, Benzyl und Methyl substituiert sein können,
mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen Cyclopentyl-, Cyclohexyl-, Azetidinyl-, Oxetanyl-, Pyrrolidinyl-, Tetrahydrofuranyl-, Piperidinyl- oder Tetrahydropyranyl-Ring bilden, mit der Maßgabe, dass gleichzeitig nicht mehr als eines der Reste-Paare R⁷ und R⁸, R⁹ und R¹⁰ bzw. R⁷ und R⁹ einen der zuvor benannten Carbo- oder Heterocyclen bildet,
- L²: für eine Bindung, Methylen oder 1,1-Ethandiyl steht,
R¹¹ für Methyl und Ethyl steht,
worin Methyl und Ethyl mit 1 bis 3 Substituenten unabhängig voneinander ausgewählt aus der Gruppe Fluor, Trifluormethyl, Hydroxy und Methoxy substituiert ist,
R¹² für Wasserstoff, (C₁-C₄)-Alkyl, (C₃-C₆)-Cycloalkyl, Phenyl oder Benzyl steht,
worin (C₁-C₄)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können, oder
R¹¹ und R¹² zusammen mit dem Sticktoffatom, an das sie gebunden sind, einen 4- bis 6-gliedrigen Aza-Heterocyclus bilden,
worin der 4- bis 6-gliedrige Aza-Heterocyclus mit 1 oder 2 Substituenten unabhängig voneinander ausgewählt aus der Gruppe (C₃-C₆)-Cycloalkyl und 4- bis 6-gliedriges Heterocyclyl substituiert sein kann,
- R¹³: für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Azabicyclo-[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Aza-bicyclo[3.3.1]nonanyl, 3-Azabicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
- R⁴: für Wasserstoff steht,
- R⁵: für Wasserstoff, Fluor, Chlor, Brom, Cyano, Methyl, Ethyl, Monofluormethyl, Methoxy, Ethinyl oder Cyclopropyl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 3 Substituenten unabhängig voneinander ausgewählt aus der Gruppe Fluor, Chlor, Cyclopropyl, Methoxy und Methyl substituiert sein kann,
- R²: für Trifluormethyl, Methyl, Ethyl und Cyclopropyl steht,
- R³: für eine Gruppe der Formel 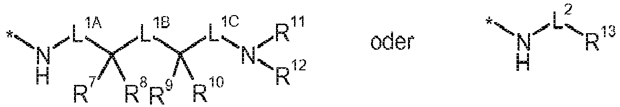 steht, wobei
* für die Anknüpfstelle an die Carbonylgruppe steht,
L^{1A} für eine Bindung steht,
L^{1B} für eine Bindung oder Methylen steht,
L^{1C} für eine Bindung oder Methylen steht,
worin Methylen mit 1 oder 2 Substituenten unabhängig voneinander ausgewählt aus der Gruppe Trifluormethyl, (C₁-C₄)-Alkyl, Cyclopropyl und Cyclobutyl substituiert sein kann,
R⁷ für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano, Nitro, Ethenyl, Difluormehtoxy, Trifluormethoxy, -NH(CO)CH₃, Ethoxy und Trifluormethyl substituiert sein kann,
wobei Ethoxy mit Hydroxy substituiert sein kann,
R⁸ für Wasserstoff, Methyl oder Ethyl steht, oder
R⁷ und R⁸ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor und Methyl substituiert sein können,
R⁹ für Wasserstoff, Cyano, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Cyano, Fluor, Trifluormethyl, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano, Difluormethoxy, Trifluormethoxy, Ethoxy und Trifluormethyl substituiert sein kann,
worin Ethoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus, sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor, Benzyl und Methyl substituiert sein können, mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen Cyclopentyl-, Cyclohexyl-. Azetidinyl-, Oxetanyl-, Pyrrolidinyl-, Tetrahydrofuranyl-, Piperidinyl- oder Tetrahydropyranyl-Ring bilden, mit der Maßgabe, dass gleichzeitig nicht mehr als eines der Reste-Paare R⁷ und R⁸, R⁹ und R¹⁰ bzw. R⁷ und R⁹ einen der zuvor benannten Carbo- oder Heterocyclen bildet,
R¹¹ für Methyl und Ethyl steht,
worin Methyl und Ethyl mit 1 bis 3 Substituenten unabhängig voneinander ausgewählt aus der Gruppe Fluor, Trifluormethyl, Hydroxy und Methoxy substituiert ist,
R¹² für Wasserstoff, (C₁-C₄)-Alkyl, (C₃-C₆)-Cycloalkyl, Phenyl oder Benzyl steht,
worin (C₁-C₄)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
R¹¹ und R¹² zusammen mit dem Sticktoffatom, an das sie gebunden sind, einen 4- bis 6-gliedrigen Aza-Heterocyclus bilden,
worin der 4- bis 6-gliedrige Aza-Heterocyclus mit 1 oder 2 Substituenten unabhängig voneinander ausgewählt aus der Gruppe (C₃-C₆)-Cycloalkyl und 4- bis 6-gliedriges Heterocyclyl substituiert sein kann, und
- L²: für eine Bindung oder Methylen steht,
R¹³ für über ein Ring-Kohlenstoffatom gebundenes Pyrrolidinyl, Piperidinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Aza-bicyclo[4.1.0]heptanyl und Chinuclidinyl steht,
worin Pyrrolidinyl, Piperidinyl, 1,2,3,4-Tetrahydroisochinolinyl, 1,2,3,4-Tetrahydrochinolinyl, 8-Azabicyclo[3.2.1]octanyl, 9-Azabicyclo-[3.3.1]nonanyl, 3-Aza-bicyclo[4.1.0]heptanyl und Chinuclidinyl mit 1 bis 5 Substituenten unabhängig voneinander ausgewählt aus der Gruppe Fluor, Trifluormethyl, Methyl, Ethyl, Cyclopropyl, Cyclobutyl und Benzyl substituiert sein können,
R⁴ für Wasserstoff steht,
R⁵ für Wasserstoff, Fluor, Chlor, Methyl, Ethyl, Monofluormethyl, Methoxy, Ethinyl oder Cyclopropyl steht,
R⁶ für Wasserstoff steht, sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für eine Phenyl-Gruppe der Formel 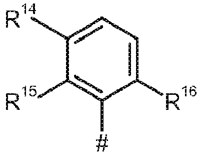 steht, wobei
# für die Anknüpfstelle an A steht,
und
R¹⁴, R¹⁵ und R¹⁶ unabhängig voneinander für Wasserstoff, Fluor, Methoxy, Cyclopropyl oder Chlor stehen,
mit der Maßgabe, dass mindestens zwei der Reste R¹⁴, R¹⁵, R¹⁶ von Wasserstoff verschieden sind,
- R²: für Methyl steht,
- R³: für eine Gruppe der Formel 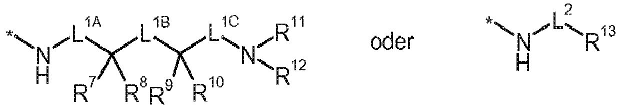 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor, Ethenyl, Difluormehtoxy, Trifluormethoxy, Ethoxy und Chlor substituiert sein kann,
wobei Ethoxy mit Hydroxy substituiert sein kann,
R⁸ für Wasserstoff, Methyl oder Ethyl steht,
R⁹ für Wasserstoff, Cyano, Trifluormethyl, (C₁-C₆)-Alkyl, Cyclopropyl oder Phenyl steht,
worin (C₁-C₆)-Alkyl mit 1 oder 2 Substituenten unabhängig voneinander ausgewählt aus der Gruppe Cyano, Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor, Difluormethoxy, Trifluormethoxy, Ethoxy und Chlor substituiert sein kann,
worin Ethoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanylring bilden, mit der Maßgabe, dass die Reste R⁷ und R⁹ nicht beide gleichzeitg für Phenyl stehen,
R¹¹ für Methyl und Ethyl steht,
worin Methyl und Ethyl mit 1 bis 3 Substituenten unabhängig voneinander ausgewählt aus der Gruppe Fluor, Trifluormethyl, Hydroxy und Methoxy substituiert ist,
R¹² für Wasserstoff, (C₁-C₄)-Alkyl, (C₃-C₆)-Cycloalkyl, Phenyl oder Benzyl steht,
worin (C₁-C₄)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können, oder
R¹¹ und R¹² zusammen mit dem Sticktoffatom, an das sie gebunden sind, einen 4- bis 6-gliedrigen Aza-Heterocyclus bilden,
worin der 4- bis 6-gliedrige Aza-Heterocyclus mit 1 oder 2 Substituenten unabhängig voneinander ausgewählt aus der Gruppe (C₃-C₆)-Cycloalkyl und 4- bis 6-gliedriges Heterocyclyl substituiert sein kann, und
L² für eine Bindung steht,
R¹³ für Piperidin-2-yl, Piperidin-3-yl, Piperidin-4-yl oder 1,2,3,4-Tetrahydrochinolin-4-yl steht,
worin Piperidin-2-yl, Piperidin-3-yl und Piperidin-4-yl mit 1 bis 5 Substituenten unabhängig voneinander ausgewählt aus Trifluormethyl und Methyl substituiert sein können,
und
worin 1,2,3,4-Tetrahydrochinolin-4-yl mit Fluor oder Trifluormethyl substituiert sein kann,
- R⁴: für Wasserstoff steht,
- R⁵: für Wasserstoff, Fluor, Chlor, Monofluormethyl, Methoxy, Ethinyl oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, (C₃-C₆)-Cycloalkyl, Difluormethoxy und Trifluormethoxy substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 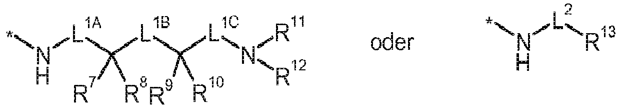
steht, wobei
- *: für die Anknüpfstelle an die Carbonylgruppe steht,
- L^{1A}: für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
- L^{1B}: für eine Bindung oder (C₁-C₄)-Alkandiyl steht,
- L^{1C}: für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
- R⁷: für Wasserstoff, (C₁-C₆)-Alkyl, (C₂-C₆)-Alkenyl, (C₂-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy, substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃, (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
- R⁸: für Wasserstoff oder (C₁-C₄)-Alkyl steht,
oder
- R⁷ und R⁸: zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R⁹ für: Wasserstoff, Cyano, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl, oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, Cyano, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5- oder 6-gliedriges Heteroaryl substituiert sein kann,
worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
- R¹⁰: für Wasserstoff oder (C₁-C₄)-Alkyl steht,
oder
- R⁹ und: R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können,
mit der Maßgabe, dass die Reste R⁷ und R⁹ nicht beide gleichzeitig für Phenyl stehen,
oder
- R⁷ und: R⁹ zusammen mit den Kohlenstoffatomen, an die sie gebunden sind, sowie der Gruppe L^{1B} einen 3- bis 7-gliedrigen Carbocyclus oder einen 4- bis 7-gliedrigen Heterocyclus bilden,
worin der 3- bis 7-gliedrigen Carbocyclus mit 1 oder 2 Substituenten unabhängig voneinander ausgewählt aus der Gruppe (C₁-C₄)-Alkyl, Fluor, Hydroxy und (C₁-C₄)-Alkoxy substituiert sein kann,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
- R¹¹: für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
- R¹²: für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
- R¹¹ und R¹²: zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann,
und
- L²: für eine Bindung oder (C₁-C₄)-Alkandiyl steht,
- R¹³: für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe (C₃-C₇)-Cycloalkyl und Benzyl substituiert ist,
- R⁴: für Wasserstoff steht,
- R⁵: für Wasserstoff, Halogen, Cyano, Monofluormethyl, Difluormethyl, Trifluormethyl, (C₁-C₄)-Alkyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂, CD₂ oder CH(CH₃) steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 4 Substituenten unabhängig voneinander ausgewählt aus der Gruppe Fluor, Chlor, Cyano, Methoxy, Ethoxy, Difluormethyl, Trifluormethyl, (C₃-C₆)-Cycloalkyl und Methyl substituiert sein kann,
- R²: für Wasserstoff, Trifluormethyl, (C₁-C₃)-Alkyl und Cyclopropyl steht,
- R³: für eine Gruppe der Formel 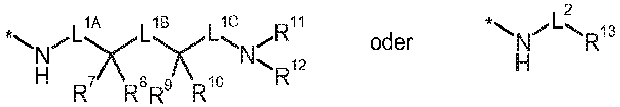 steht, wobei
* für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung oder Methylen steht,
worin Methylen mit 1 oder 2 Substituenten unabhängig voneinander ausgewählt aus der Gruppe Fluor, Trifluormethyl, (C₁-C₄)-Alkyl, Cyclopropyl und Cyclobutyl substituiert sein kann,
R⁷ für Wasserstoff, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Fluor, Trifluormethyl, Difluormethoxy, Trifluormethoxy, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Nitro, Methyl, Ethenyl, Difluormehtoxy, Trifluormethoxy, -NH(CO)CH₃, (C₁-C₄)-Alkoxy und Trifluormethyl substituiert sein können,
wobei (C₁-C₄)-Alkoxy mit Hydroxy substituiert sein kann,
R⁸ für Wasserstoff, Methyl oder Ethyl steht, oder
R⁷ und R⁸ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor und Methyl substituiert sein können,
R⁹ für Wasserstoff, Cyano, 1,1,2,2-Tetrafluorethyl, Trifluormethyl, (C₁-C₆)-Alkyl, (C₃-C₆)-Cycloalkyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Cyano, Fluor, Trifluormethyl, Difluormethoxy, Trifluormethoxy, Hydroxy, (C₁-C₄)-Alkoxy, 5-gliedriges Heteroaryl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin 5- gliedriges Heteroaryl benzokondensiert oder mit einem 5-gliedrigen Heteroaryl substituiert sein kann,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 oder 2 Substituenten unabhängig voneinander ausgewählt aus der Gruppe Fluor, Chlor, Cyano, Methyl, Difluormethoxy, Trifluormethoxy, (C₁-C₄)-Alkoxy und Trifluormethyl substituiert sein können,
worin (C₁-C₄)-Alkoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor, Benzyl und Methyl substituiert sein können, mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen Cyclopentyl-, Cyclohexyl-, Azetidinyl-, Oxetanyl-, Pyrrolidinyl-, Tetrahydrofuranyl-, Piperidinyl- oder Tetrahydropyranyl-Ring bilden, mit der Maßgabe, dass gleichzeitig nicht mehr als eines der Reste-Paare R⁷ und R⁸, R⁹ und R¹⁰ bzw. R⁷ und R⁹ einen der zuvor benannten Carbo- oder Heterocyclen bildet,
R¹¹ für Wasserstoff oder (C₁-C₃)-Alkyl steht,
worin (C₁-C₃)-Alkyl mit 1 oder 2 Substituenten unabhängig voneinander ausgewählt aus der Gruppe Fluor, Trifluormethyl, Hydroxy, Methoxy und Ethoxy substituiert sein kann,
R¹² für Wasserstoff, (C₁-C₄)-Alkyl, Cyclopropyl, Cyclobutyl, Phenyl oder Benzyl steht,
worin (C₁-C₄)-Alkyl mit 1 oder 2 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Fluor, Chlor und Trifluormethyl substituiert sein können, oder
R¹¹ und R¹² zusammen mit dem Stickstoffatom, an das sie gebunden sind, einen Azetidinyl-, Pyrrolidinyl-, Piperidinyl-, Morpholinyl-, Piperazinyl-, Thiomorpholinyl- oder 1,1-Dioxothiomorpholinyl-Ring bilden,
worin der Azetidinyl-, Pyrrolidinyl-, Piperidinyl-, Morpholinyl-, Piperazinyl-, Thiomorpholinyl- und 1,1-Dioxothiomorpholinyl-Ring mit 1 oder 2 Substituenten unabhängig voneinander ausgewählt aus der Gruppe Fluor, Trifluormethyl, Methyl, Ethyl, Cyclopropyl, Cyclobutyl, Azetidinyl, Pyrolidinyl und Piperidinyl substituiert sein können, und
- L²: für eine Bindung, Methylen oder 1,1-Ethandiyl steht,
R¹³ für 5- bis 6-gliedriges, über ein Ring-Kohlenstoffatom gebundenes Aza-Heterocyclyl steht,
worin 5- bis 6-gliedriges Aza-Heterocyclyl mit 1 bis 3 Substituenten unabhängig voneinander ausgewählt aus der Gruppe (C₃-C₇)-Cycloalkyl und Benzyl substituiert ist,
- R⁴: für Wasserstoff steht,
- R⁵: für Wasserstoff, Fluor, Chlor, Brom, Cyano, Methyl, Ethyl, Monofluormethyl, Methoxy, Ethinyl oder Cyclopropyl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 oder 2 Substituenten unabhängig voneinander ausgewählt aus der Gruppe Fluor, Trifluormethyl und Methyl substituiert sein kann,
und
wobei Phenyl mit 1 bis 3 Substituenten unabhängig voneinander ausgewählt aus der Gruppe Fluor, Chlor, Cyclopropyl, Methoxy und Methyl substituiert sein kann,

- R²: für Trifluormethyl, Methyl, Ethyl und Cyclopropyl steht,
- R³: für eine Gruppe der Formel 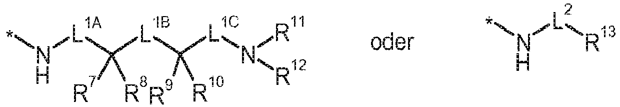 steht, wobei
* für die Anknüpfstelle an die Carbonylgruppe steht,
L^{1A} für eine Bindung steht,
L^{1B} für eine Bindung oder Methylen steht,
L^{1C} für eine Bindung oder Methylen steht,
worin Methylen mit 1 oder 2 Substituenten unabhängig voneinander ausgewählt aus der Gruppe Trifluormethyl, (C₁-C₄)-Alkyl, Cyclopropyl und Cyclobutyl substituiert sein kann,
R⁷ für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano Nitro, Ethenyl, Difluormehtoxy, Trifluormethoxy, --NH(CO)CH₃, Ethoxy und Trifluormethyl substituiert sein kann,
wobei Ethoxy mit Hydroxy substituiert sein kann,
R⁸ für Wasserstoff, Methyl oder Ethyl steht, oder
- R⁷ und R⁸: zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor und Methyl substituiert sein können,
- R⁹: für Wasserstoff, Cyano, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Cyano, Fluor, Trifluormethyl, Hydroxy, (C₁-C₄)-Alkoxy, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl mit 1 oder 2 Substituenten unabhängig voneinander ausgewählt aus der Gruppe Fluor, Chlor, Cyano, Difluormethoxy, Trifluormethoxy, Ethoxy und Trifluormethyl substituiert sein kann,
worin Ethoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanyl-, Tetrahydrofuranyl-, Tetrahydropyranyl-, Azetidinyl-, Pyrrolidinyl-oder Piperidinyl-Ring bilden,
worin der 3- bis 6-gliedrige Carbocyclus, sowie der Oxetanyl-, Tetrahydrofuranyl-, Tetrahydropyranyl-, Azetidinyl-, Pyrrolidinyl-und Piperidinyl-Ring mit 1 oder 2 Substituenten unabhängig voneinander ausgewählt aus Fluor, Benzyl und Methyl substituiert sein können, mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen Cyclopentyl-, Cyclohexyl-. Azetidinyl-, Oxetanyl-, Pyrrolidinyl-, Tetrahydrofuranyl-, Piperidinyl- oder Tetrahydropyranyl-Ring bilden, mit der Maßgabe, dass gleichzeitig nicht mehr als eines der Reste-Paare R⁷ und R⁸, R⁹ und R¹⁰ bzw. R⁷ und R⁹ einen der zuvor benannten Carbo- oder Heterocyclen bildet,
R¹¹ für Wasserstoff oder (C₁-C₃)-Alkyl steht,
worin (C₁-C₃)-Alkyl mit 1 oder 2 Substituenten unabhängig voneinander ausgewählt aus der Gruppe Fluor und Trifluormethyl substituiert sein kann,
R¹² für Wasserstoff, (C₁-C₄)₋Alkyl, Cyclopropyl oder Cyclobutyl steht,
worin (C₁-C₄)-Alkyl mit 1 oder 2 Substituenten unabhängig voneinander ausgewählt aus der Gruppe Fluor und Trifluormethyl substituiert sein kann, oder
R¹¹ und R¹² zusammen mit dem Stickstoffatom, an das sie gebunden sind, einen Azetidinyl-, Pyrrolidinyl-, Piperidinyl- oder Morpholinyl-Ring bilden,
worin der Azetidinyl-, Pyrrolidinyl-, Piperidinyl- und Morpholinyl-Ring mit 1 oder 2 Substituenten unabhängig voneinander ausgewählt aus der Gruppe Fluor, Trifluormethyl, Methyl, Ethyl, Cyclopropyl und Cyclobutyl substituiert sein können, und
L² für eine Bindung oder Methylen steht,
R¹³ für 5- bis 6-gliedriges, über ein Ring-Kohlenstoffatom gebundenes Aza-Heterocyclyl steht,
worin 5- bis 6-gliedriges Aza-Heterocyclyl mit 1 bis 3 Substituenten unabhängig voneinander ausgewählt aus der Gruppe (C₃-C7)-Cycloalkyl und Benzyl substituiert ist,
- R⁴: für Wasserstoff steht,
- R⁵: für Wasserstoff, Fluor, Chlor, Methyl, Ethyl, Monofluormethyl, Methoxy, Ethinyl oder Cyclopropyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für eine Phenyl-Gruppe der Formel 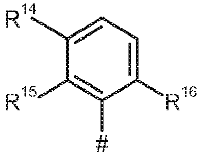 steht, wobei
# für die Anknüpfstelle an A steht, und
R¹⁴, R¹⁵ und R¹⁶ unabhängig voneinander für Wasserstoff, Fluor, Methoxy, Cyclopropyl oder Chlor stehen, mit der Maßgabe, dass mindestens zwei der Reste R¹⁴, R¹⁵, R¹⁶ von Wasserstoff verschieden sind,
- R²: für Methyl steht,
- R³: für eine Gruppe der Formel 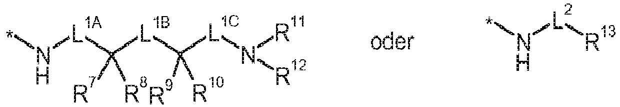 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff, Trifluormethyl, (C₁-C₆)-Alkyl, (C₃-C₆)-Cycloalkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor, Ethenyl, Difluormehtoxy, Trifluormethoxy, Ethoxy und Chlor substituiert sein kann,
wobei Ethoxy mit Hydroxy substituiert sein kann,
R⁸ für Wasserstoff, Methyl oder Ethyl steht,
R⁹ für Wasserstoff, Cyano, Trifluormethyl, (C₁-C₆)-Alkyl, Cyclopropyl oder Phenyl steht,
worin (C₁-C₆)-Alkyl mit 1 oder 2 Substituenten unabhängig voneinander ausgewählt aus der Gruppe Cyano, Fluor, Trifluormethyl, Hydroxy, (C₁-C₄)-Alkoxy und Phenyl substituiert sein kann,
und
worin Phenyl mit 1 oder 2 Substituenten unabhängig voneinander ausgewählt aus Fluor, Difluormethoxy, Trifluormethoxy, Ethoxy und Chlor substituiert sein kann,
worin Ethoxy mit Hydroxy substituiert sein kann,
R¹⁰ für Wasserstoff, Methyl oder Ethyl steht, oder
R⁹ und R¹⁰ zusammen mit dem Kohlenstoffatom, an das sie gebunden sind, einen 3- bis 6-gliedrigen Carbocyclus oder einen Oxetanylring bilden, mit der Maßgabe, dass die Reste R⁷ und R⁹ nicht beide gleichzeitg für Phenyl stehen,
R¹¹ für Wasserstoff steht,
R¹² für Wasserstoff steht, und
L² für eine Bindung steht,
R¹³ für 5- bis 6-gliedriges, über ein Ring-Kohlenstoffatom gebundenes Aza-Heterocyclyl steht,
worin 5- bis 6-gliedriges Aza-Heterocyclyl mit 1 bis 3 Substituenten unabhängig voneinander ausgewählt aus der Gruppe (C₃-C₇)-Cycloalkyl und Benzyl substituiert ist,
- R⁴: für Wasserstoff steht,
- R⁵: für Wasserstoff, Fluor, Chlor, Monofluormethyl, Methoxy, Ethinyl oder Methyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I), in welcher
- A: für CH₂ steht,
- R¹: für Phenyl steht,
wobei Phenyl mit 2 bis 3 Fluor substituiert ist,
- R²: für Methyl steht,
- R³: für eine Gruppe der Formel 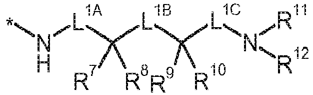 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff steht,
R⁸ für Wasserstoff steht,
R⁹ für Wasserstoff oder (C₁-C₄)-Alkyl steht,
R¹⁰ für Methyl oder Ethyl steht,
R¹¹ für Wasserstoff steht,
R¹² für Wasserstoff steht,
- R⁴: für Wasserstoff steht,
- R⁵: für Wasserstoff oder Methyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I), in welcher
- A: für CH₂ steht,
- R¹: für eine Phenyl-Gruppe der Formel 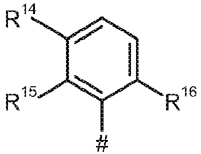 steht, wobei
# für die Anknüpfstelle an A steht, und
R¹⁴, R¹⁵ und R¹⁶ unabhängig voneinander für Wasserstoff oder Fluor stehen, mit der Maßgabe, dass mindestens zwei der Reste R¹⁴, R¹⁵, R¹⁶ von Wasserstoff verschieden sind,
- R²: für Methyl steht,
- R³: für eine Gruppe der Formel 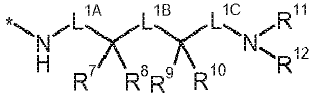 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff steht,
R⁸ für Wasserstoff steht,
R⁹ für Wasserstoff oder (C₁-C₄)-Alkyl steht,
R¹⁰ für Methyl oder Ethyl steht,
R¹¹ für Wasserstoff steht,
R¹² für Wasserstoff steht,
- R⁴: für Wasserstoff steht,
- R⁵: für Wasserstoff oder Methyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I), in welcher
- A: für CH₂ steht,
- R¹: für Phenyl steht,
wobei Phenyl mit 2 bis 3 Fluor substituiert ist,
- R²: für Methyl steht,
- R³: für eine Gruppe der Formel 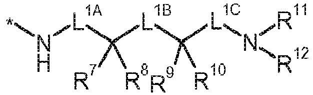 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff steht,
R⁸ für Wasserstoff steht,
R⁹ für (C₁-C₄)-Alkyl steht,
worin (C₁-C₄-Alkyl bis zu fünffach mit Fluor substituiert ist,
R¹⁰ für Methyl oder Ethyl steht,
R¹¹ für Wasserstoff steht,
R¹² für Wasserstoff steht,
- R⁴: für Wasserstoff steht,
- R⁵: für Wasserstoff oder Methyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I), in welcher
- A: für CH₂ steht,
- R¹: für eine Phenyl-Gruppe der Formel 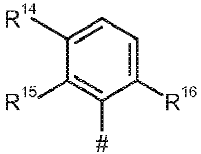 steht, wobei
# für die Anknüpfstelle an A steht,
und
R¹⁴, R¹⁵ und R¹⁶ unabhängig voneinander für Wasserstoff oder Fluor stehen, mit der Maßgabe, dass mindestens zwei der Reste R¹⁴, R¹⁵, R¹⁶ von Wasserstoff verschieden sind,
- R²: für Methyl steht,
- R³: für eine Gruppe der Formel 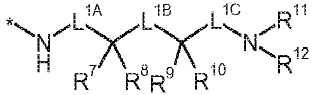 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff steht,
R⁸ für Wasserstoff steht,
R⁹ für (C₁-C₄)-Alkyl steht,
worin (C₁-C₄)-Alkyl bis zu fünffach mit Fluor substituiert ist,
R¹⁰ für Methyl oder Ethyl steht,
R¹¹ für Wasserstoff steht,
R¹² für Wasserstoff steht,
- R⁴: für Wasserstoff steht,
- R⁵: für Wasserstoff oder Methyl steht,
- R⁶: für Wasserstoff steht,
sowie ihre *N*-Oxide, Salze, Solvate, Salze der *N*-Oxide und Solvate der *N*-Oxide und Salze

Besonders bevorzugt sind auch folgende Verbindungen 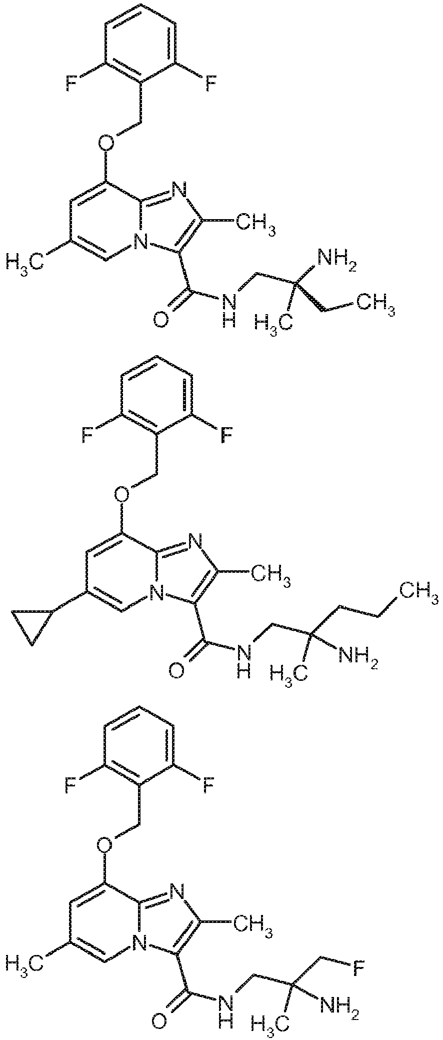 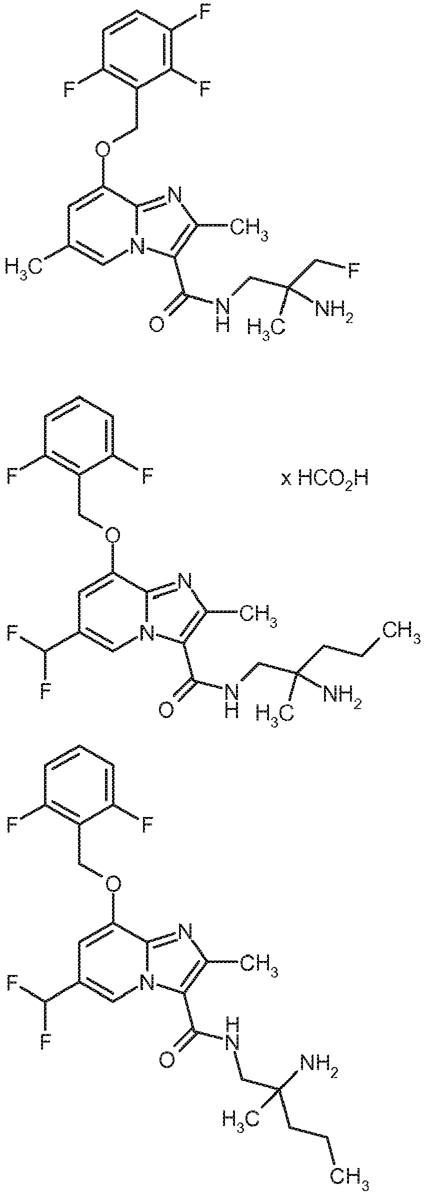 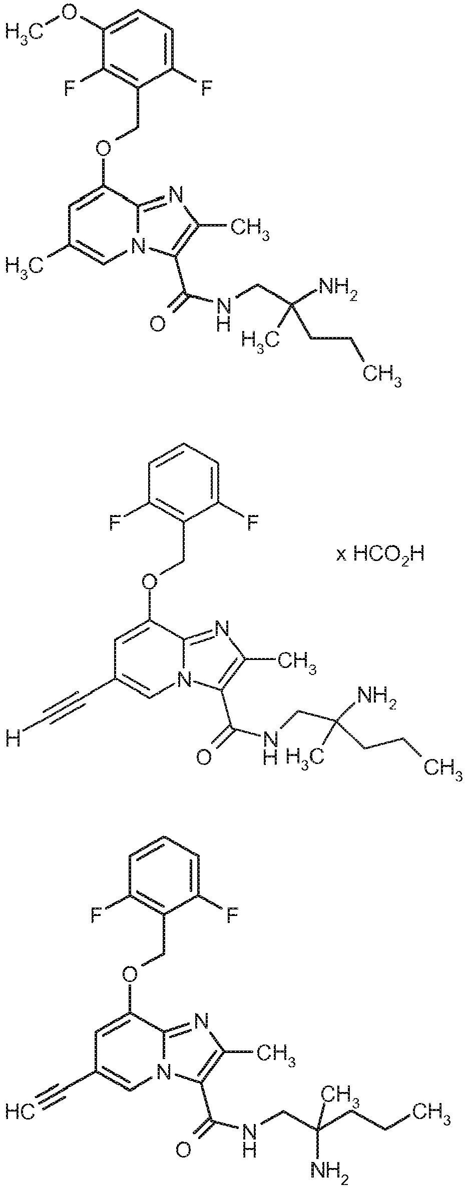 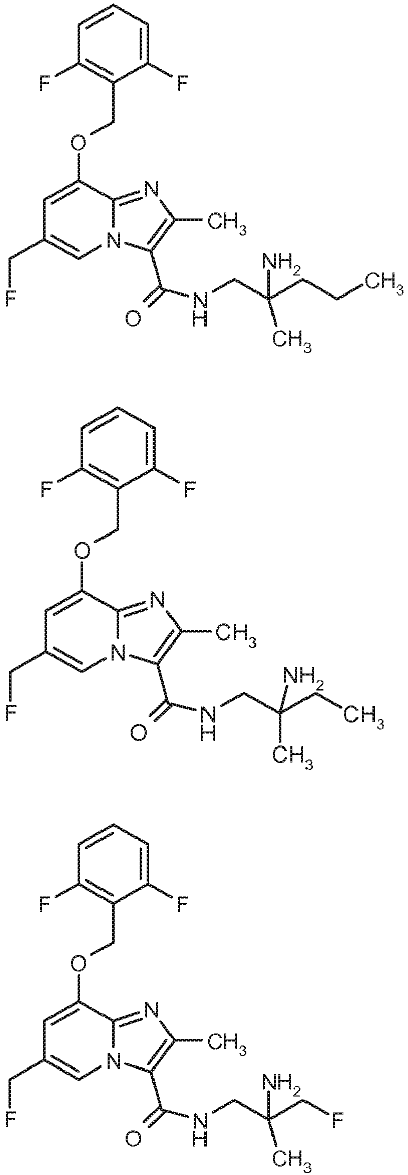 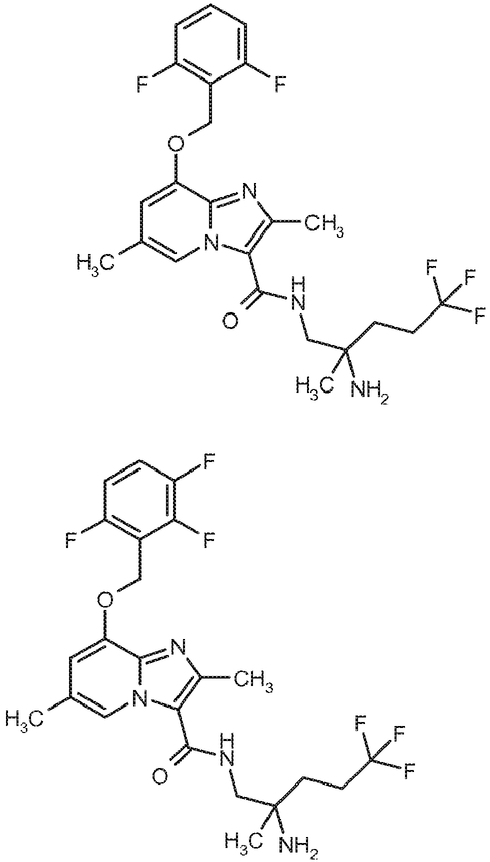 sowie ihre *N*-Oxide, Salze, Solvate, Salze der *N*-Oxide und Solvate der *N*-Oxide und Salze.

Besonders bevorzugt sind auch folgende Verbindungen, 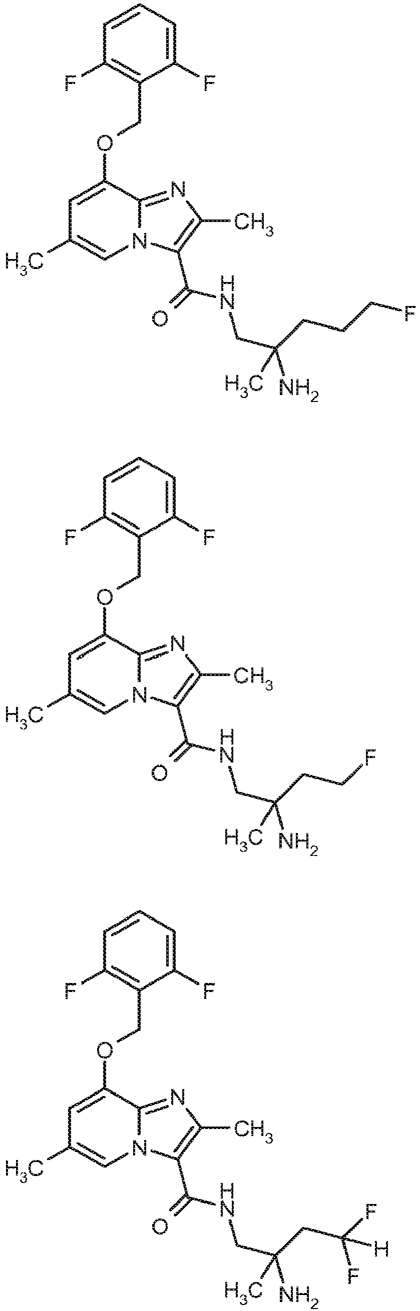 sowie ihre *N*-Oxide, Salze, Solvate, Salze der *N*-Oxide und Solvate der *N*-Oxide und Salze.

Die zuletzt genannten drei Verbindungen können nach dem Fachmann bekannten Synthesemethoden hergestellt werden (Vergleiche weiter unten aufgeführte Schemata 6-17).

Besonders bevorzugt sind auch folgende Verbindungen 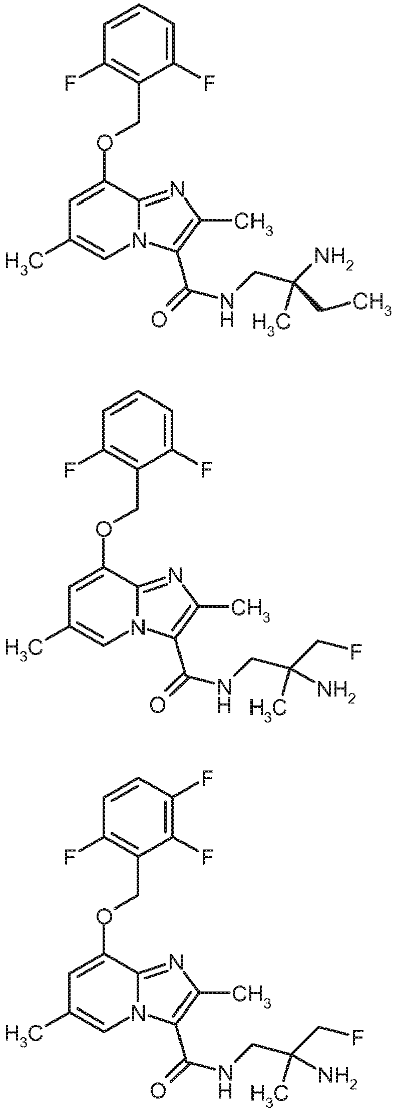 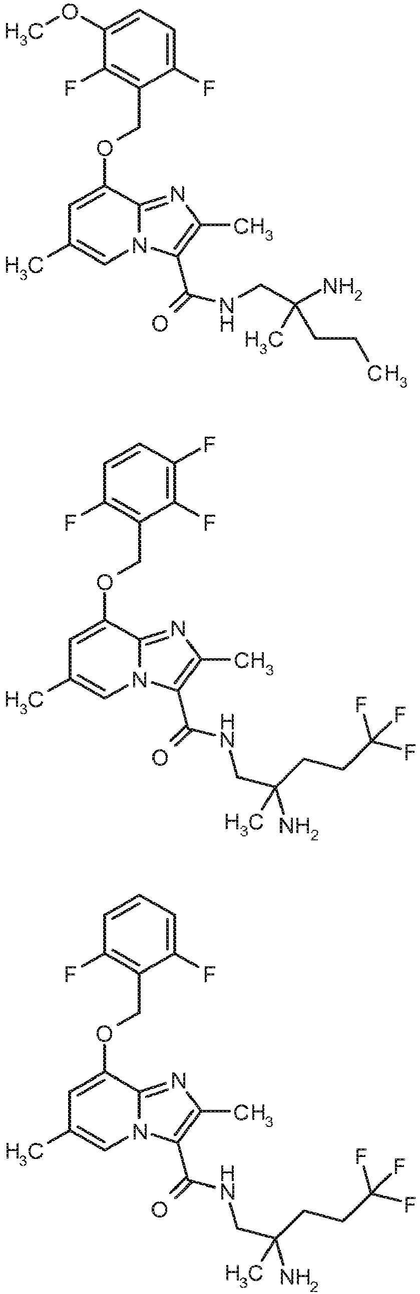 sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹: für eine Phenyl-Gruppe der Formel 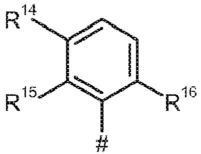 steht, wobei
# für die Anknüpfstelle an A steht, und
R¹⁴, R¹⁵ und R¹⁶ unabhängig voneinander für Wasserstoff, Fluor oder Chlor stehen, mit der Maßgabe, dass mindestens zwei der Reste R¹⁴, R¹⁵, R¹⁶ von Wasserstoff verschieden sind,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R²: für Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 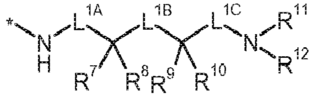
steht, wobei
- *: für die Anknüpfstelle an die Carbonylgruppe steht,
- L^{1A}: für eine Bindung steht,
- L^{1B}: für eine Bindung oder Methylen steht,
- V^{1C}: für eine Bindung oder Methylen steht,
worin Methylen mit 1 oder 2 Substituenten unabhängig voneinander ausgewählt aus der Gruppe Trifluormethyl, (C₁-C₄)-Alkyl, Cyclopropyl und Cyclobutyl substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 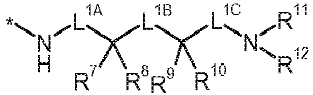 steht, wobei
* für die Anknüpfstelle an die Carbonylgruppe steht,
R⁸ für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 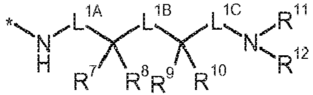
steht, wobei
- *: für die Anknüpfstelle an die Carbonylgruppe steht,
und
- R¹⁰: für Wasserstoff oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁵: für Wasserstoff, Fluor, Chlor oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹: Phenyl steht,
wobei Phenyl mit 1 bis 3 Substituenten unabhängig voneinander ausgewählt aus der Gruppe Halogen, Cyano, Monofluormethyl, Difluormethyl, Trifluormethyl, (C₁-C₄)-Alkyl substituiert sein kann,
und
wobei Phenyl mit 1 bis 2 Substituenten ausgewählt aus der Gruppe (C₃-C₇)-Cycloalkyl, (C₁-C₄)-Alkoxy, Monofluormethoxy, Difluormethoxy oder Trifluormethoxy substituiert ist,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹: Phenyl steht,
wobei Phenyl mit 1 bis 2 Substituenten unabhängig voneinander ausgewählt aus der Gruppe Fluor, Chlor, Cyano, Monofluormethyl, Difluormethyl, Trifluormethyl, (C₁-C₄)-Alkyl substituiert ist,

und
wobei Phenyl mit einem Substituenten ausgewählt aus der Gruppe (C₃-C₆)-Cycloalkyl, (C₁-C₂)-Alkoxy oder Trifluormethoxy substituiert ist,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹: Phenyl steht,
wobei Phenyl mit 1 bis 2 Fluor substituiert ist,
und
wobei Phenyl mit einem Substituenten ausgewählt aus der Gruppe Cyclopropyl, oder Methoxy substituiert ist,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁵: für Monofluormethyl, Difluormethyl, Trifluormethyl, (C₃-C₆)-Cycloalkyl, (C₂-C₄)-Alkenyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁵: für Monofluormethyl, Difluormethyl, Trifluormethyl, (C₃-C₆)-Cycloalkyl, (C₂-C₄)-Alkinyl, Difluormethoxy, Trifluormethoxy, (C₁-C₄)-Alkoxy, 5- bis 6-gliedriges Heterocyclyl oder 5- oder 6-gliedriges Heteroaryl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁵: für Monofluormethyl, Difluormethyl, Trifluormethyl, Cyclopropyl, (C₂-C₄)-Alkinyl, Methoxy, Morpholino steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁷: für (C₁-C₆)-Alkyl, (C₂-C₆)-Alkinyl, Cyano oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy und Phenoxy substituiert ist,
worin Phenoxy mit 1 bis 3 Fluor substituiert sein kann,
worin Phenyl mit 1 bis 2 Substituenten unabhängig voneinander ausgewählt aus der Gruppe Cyano, Nitro, Difluormethoxy, Trifluormethoxy, (C₁-C₄)-Alkoxy, -NH(CO)CH₃ und (C₁-C₄)-Alkenyl substituiert ist,
wobei (C₁-C₄)-Alkoxy mit Hydroxy substituiert ist,
- R⁸: für Wasserstoff oder (C₁-C₄)-Alkyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁷: für (C₁-C₄-Alkyl, Cyano oder Phenyl steht,
worin (C₁-C₄)-Alkyl bis zu fünffach mit Fluor substituiert ist,
und
worin Phenyl mit Cyano, Difluormethoxy, Trifluormethoxy, Ethoxy, -NH(CO)CH₃ oder Ethenyl substituiert ist,
wobei Ethoxy mit Hydroxy substituiert ist,
- R⁸: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁷: für (C₁-C₄)-Alkyl, Cyano oder Phenyl steht,
worin (C₁-C₄)-Alkyl bis zu fünffach mit Fluor substituiert ist,
und
worin Phenyl mit Cyano, Difluormethoxy, Trifluormethoxy, Ethoxy, -NH(CO)CH₃ oder Ethenyl substituiert ist,
wobei Ethoxy mit Hydroxy substituiert ist,
- R⁸: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁹: für (C₁-C₄)-Alkyl, Cyano oder Phenyl steht,
worin (C₁-C₄)-Alkyl mit 1 bis 3 Substituenten unabhängig voneinander ausgewählt aus der Gruppe Fluor, Difluormethyl, Trifluormethyl, Difluormethoxy, Trifluormethoxy, 5-gliedriges Heteroaryl und Benzyloxy substituiert ist,
worin Benzyloxy mit 1 bis 3 Substituenten Halogen substituiert sein kann,
worin 5-gliedriges Heteroaryl mit einem 5-gliedrigen Heteroaryl, substituiert ist,
worin 5-gliedriges Heteroaryl seinerseits mit (C₁-C₄)-Alkyl substituiert sein kann,
worin Phenyl mit 1 bis 2 Substituenten unabhängig voneinander ausgewählt aus der Gruppe Cyano, Difluormethoxy, Trifluormethoxy und (C₁-C₄)-Alkoxy substituiert ist,
wobei (C₁-C₄)-Alkoxy mit Hydroxy substituiert ist,
- R¹⁰: für Wasserstoff oder Metyhl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁹: für Ethyl, Propyl, Cyano oder Phenyl steht,
worin Ethyl und Propyl mit 1 bis 3 Substituenten unabhängig voneinander ausgewählt aus der Gruppe Fluor, Trifluormethyl und Benzyloxy substituiert ist,
worin Benzyloxy mit 1 bis 3 Substituenten Halogen substituiert sein kann,
worin Phenyl mit Cyano, Difluormethoxy, Trifluormethoxy oder Ethoxy substituiert ist,
wobei Ethoxy mit Hydroxy substituiert ist,
- R¹⁰: für Wasserstoff oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁹: für Ethyl, Cyano oder Phenyl steht,
worin Ethyl bis zu fünffach mit Fluor substituiert ist,
worin Phenyl mit Cyano, Difluormethoxy, Trifluormethoxy oder Ethoxy substituiert ist,
wobei Ethoxy mit Hydroxy substituiert ist,
- R¹⁰: für Wasserstoff oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹¹: für (C₁-C₄)-Alkyl steht,
worin (C₁-C₄-Alkyl mit 1 bis 3 Substituenten unabhängig voneinander ausgewählt aus der Gruppe Fluor, Trifluormethyl, Hydroxy und (C₁-C₄)-Alkoxy substituiert ist,
- R¹²: für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
- R¹¹ und R¹²: zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe (C₃-C₇)-Cycloalkyl und 4- bis 7-gliedriges Heterocyclyl substituiert ist,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹¹: für Methyl und Ethyl steht,
worin Methyl und Ethyl mit 1 bis 3 Substituenten unabhängig voneinander ausgewählt aus der Gruppe Fluor, Trifluormethyl, Hydroxy und Methoxy substituiert ist,
- R¹²: für Wasserstoff, (C₁-C₄)-Alkyl, (C₃-C₆)-Cycloalkyl, Phenyl oder Benzyl steht,
worin (C₁-C₄)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
- R¹¹ und R¹²: zusammen mit dem Sticktoffatom, an das sie gebunden sind, einen 4- bis 6-gliedrigen Aza-Heterocyclus bilden,
worin der 4- bis 6-gliedrige Aza-Heterocyclus mit 1 oder 2 Substituenten unabhängig voneinander ausgewählt aus der Gruppe (C₃-C₆)-Cycloalkyl und 4- bis 6-gliedriges Heterocyclyl substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹³: für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe (C₃-C₇)-Cycloalkyl und Benzyl substituiert ist,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹³: für 5- bis 6-gliedriges, über ein Ring-Kohlenstoffatom gebundenes Aza-Heterocyclyl steht,
worin 5- bis 6-gliedriges Aza-Heterocyclyl mit 1 bis 3 Substituenten unabhängig voneinander ausgewählt aus der Gruppe (C₃-C₇)-Cycloalkyl und Benzyl substituiert ist,
sowie ihre *N*-Oxide, Salze, Solvate, Salze der *N*-Oxide und Solvate der *N*-Oxide und Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 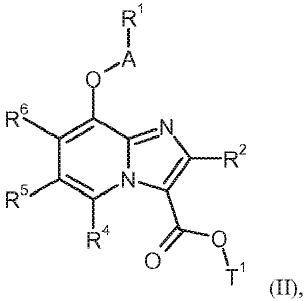 in welcher A, R¹, R², R⁴, R⁵ und R⁶ jeweils die oben angegebenen Bedeutungen haben und
   - T¹: für (C₁-C₄)-Alkyl oder Benzyl steht,
   in einem inerten Lösungsmittel in Gegenwart einer geeigneten Base oder Säure zu einer Carbonsäure der Formel (III) 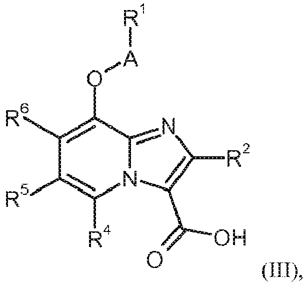 in welcher A, R¹, R², R⁴, R⁵ und R⁶ jeweils die oben angegebenen Bedeutungen haben,
   umsetzt und diese in der Folge in einen inerten Lösungsmittel unter Amidkupplungsbedingungen mit einem Amin der Formel (IV-A) oder (IV-B) 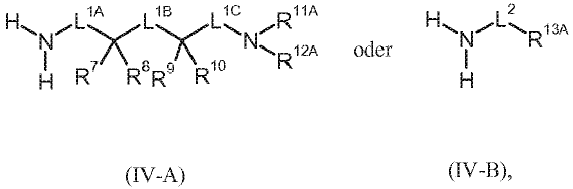 in welchem L^{1A}, L^{1B}, L^{1C}, L², R⁷, R⁸, R⁹, und R¹⁰ jeweils die oben angegebenen Bedeutungen haben
   und
   R^{11A}, R^{12A} und R^{13A} die oben für R¹¹, R¹² bzw. R¹³ angegebenen Bedeutungen haben oder für eine Amino-Schutzgruppe, wie beispielsweise tert.-Butoxycarbonyl, Benzyloxycarbonyl oder Benzyl stehen, umsetzt,
   oder
[B] eine Verbindung der Formel (III-B) 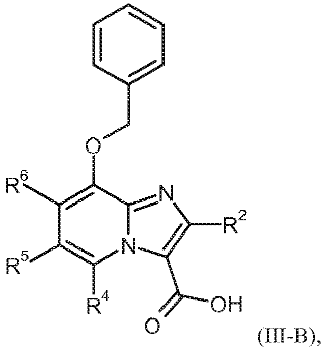 in welcher R², R⁴, R⁵ und R⁶ jeweils die oben angegebenen Bedeutungen haben,
   in einem inerten Lösungsmittel unter Amidkupplungsbedingungen mit einem Amin der Formel (IV) zu einer Verbindung der Formel (I-A) und (I-B), 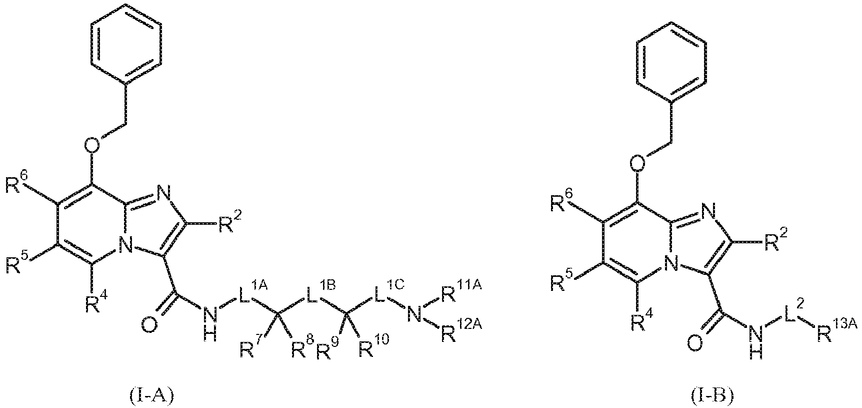 in welcher R², R⁴, R⁵, R⁶, L^{1A} , L^{1B}, L^{1C}, L², R⁷, R⁸, R⁹, R¹⁰, R^{11A}, R^{12A} und R^{13A} jeweils die oben angegebenen Bedeutungen haben,
   umsetzt, von dieser im Folgenden nach den dem Fachmann bekannten Methoden die Benzylgruppe abspaltet und die resultierende Verbindung der Formel (V-A) oder (V-B) 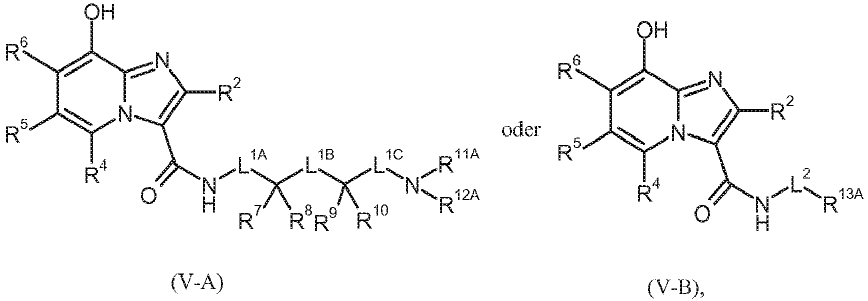 in welcher R², R⁴, R⁵, R⁶, L^{1A}, L^{1B}, L^{1C}, L², R⁷, R⁸, R⁹, R¹⁰, R^{11A}, R^{12A} und R^{13A} jeweils die oben angegebenen Bedeutungen haben,
   in einem inerten Lösungsmittel in Gegenwart einer geeigneten Base mit einer Verbindung der Formel (VI) 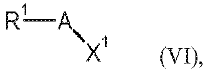 in welcher A und R¹ die oben angegebene Bedeutung hat und
   - X¹: für eine geeignete Abgangsgruppe, insbesondere Chlor, Brom, Iod, Mesylat, Triflat oder Tosylat, steht,
   umsetzt,
   anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (I-A) und (I-B) bilden eine Teilmenge der erfindungsgemäßen Verbindungen der Formel (I).

Die beschriebenen Herstellverfahren können durch die folgenden Syntheseschemata (Schema 1 und 2) beispielhaft verdeutlicht werden:

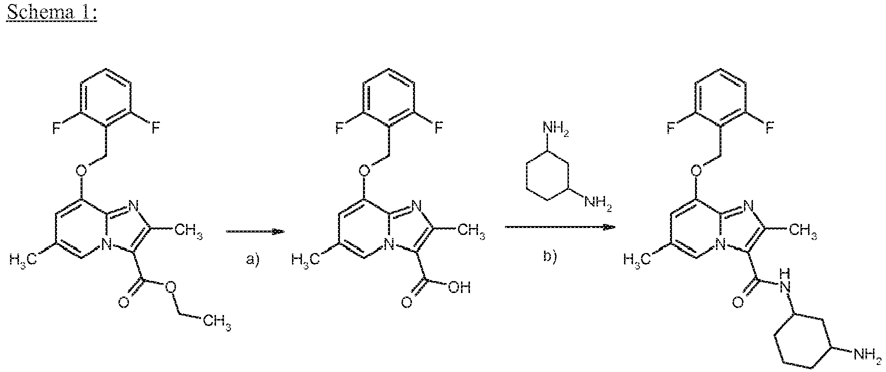

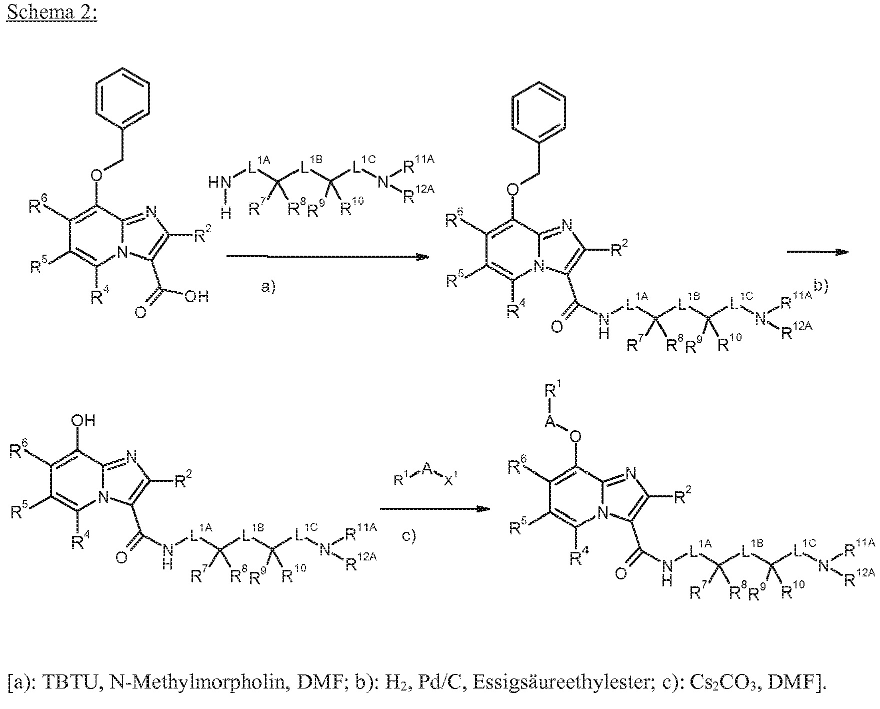

Die Verbindungen der Formeln (IV-A), (IV-B) und (VI) sind kommerziell erhältlich, literaturbekannt oder können in Analogie zu literaturbekannten Verfahren hergestellt werden.

Die freien Basen von (IV-A) können aus den gegebenenfalls mit einer Amino-Schutzgruppe versehenden Verbindungen (IV-A) freigesetzt werden, z.B. durch Verwendung von Säuren wie Chlorwasserstoff und Trifluoressigsäure in geeigneten Lösungsmitteln wie Diethylether, Dichlormethan, 1,4-Dioxan, Wasser, Methanol, Ethanol und deren Mischungen.

Inerte Lösungsmittel für die Verfahrensschritte (III) + (IV) → (I) und (III-B) + (IV) → (I-B) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N,N*-Dimethylformamid, *N,N-*Dimethylacetamid, *N*,*N'*-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für die Amidbildung in den Verfahrensschritte (III) + (IV) → (I) und (III-B) + (IV) → (I-B) eignen sich beispielsweise Carbodiimide wie *N,N'*-Diethyl-, *N,N'*-Dipropyl-, *N,N'*-Diisopropyl-, *N,N'*-Dicyclohexylcarbodiimid (DCC) oder *N*-(3-Dimethylaminopropyl)-*N'-*ethylcarbodiimid-Hydrochlorid (EDC), Phosgen-Derivate wie *N,N'*-Carbonyldiimidazol (CDI), 1,2-Oxazoliumverbindungen wie 2-Ethyl-5-phenyl-1,2-oxazolium-3-sulfat oder 2-*tert.*-Butyl-5-methyl-isoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid (T3P), 1-Chlor-*N,N*,2-trimethylpropl-en-1-amin, Cyanophosphonsäurediethylester, Bis-(2-oxo-3-oxazolidinyl)-phosphorylchlorid, Benzotriazol-1-yloxy-tris(dimethylamino)phosphonium-hexafluorphosphat, Benzotriazol-1-yloxy-tris(pyrrolidino)phosphonium-hexafluorphosphat (PyBOP), *O*-(Benzotriazol-1-yl)-*N,N,N',N'*-tetramethyluronium-tetrafluorborat (TBTU), *O-*(Benzotriazol-1-yl)-*N,N,N',N*'-tetramethyluronium-hexafluorphosphat (HBTU), 2-(2-Oxo-1-(2*H*)-pyridyl)-1,1,3,3-tetramethyluronium-tetrafluorborat (TPTU), *O*-(7-Arabenzotriazol-1-yl)-*N,N,N',N'*-tetramethyluronium-hexafluorphosphat (HATU) oder *O*-(1*H*-6-Chlorbenzotriazol-1-yl)-1,1,3,3-tetramethyluronium-tetrafluorborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N,N*-Diisopropylethylamin. Bevorzugt wird TBTU in Verbindung mit N-Methylmorpholin, HATU in Verbindung mit *N,N-*Diisopropylethylamin oder 1-Chlor-*N,N*,2-trimethylprop-1-en-1amin verwendet.

Die Kondensationen (III) + (IV) → (I) und (III-B) + (IV) → (I-B) wird im Allgemeinen in einem Temperaturbereich von -20°C bis +100°C, bevorzugt bei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Alternativ kann die Carbonsäure der Formel (III) auch zunächst in das entsprechende Carbonsäurechlorid überführt werden und dieses dann direkt oder in einer separaten Umsetzung mit einem Amin der Formel (IV) zu den erfindungsgemäßen Verbindungen umgesetzt werden. Die Bildung von Carbonsäurechloriden aus Carbonsäuren erfolgt nach den dem Fachmann bekannten Methoden, beispielsweise durch Behandlung mit Thionylchlorid, Sulfurylchlorid oder Oxalylchlorid in Gegenwart einer geeigneten Base, beispielsweise in Gegenwart von Pyridin, sowie optional unter Zusatz von Dimethylformamid, optional in einem geeigneten inerten Lösemittel.

Die Hydrolyse der Ester-Gruppe T¹ der Verbindungen der Formel (II) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren. Im Falle der Benzylester erfolgt die Esterspaltung bevorzugt hydrogenolytisch mit Palladium auf Aktivkohle oder Raney-Nickel. Als inerte Lösungsmittel eignen sich für diese Reaktion Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt

Als Basen für die Ester-Hydrolyse sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Ethylester.

Die Esterspaltung erfolgt im Allgemeinen in einem Temperaturbereich von 0°C bis +100°C, bevorzugt bei +0°C bis +50°C.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Inerte Lösungsmittel für den Verfahrensschritt (V) + (VI) → (I) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N,N*-Dimethylformamid, *N,N*-Dimethylacetamid, Dimethylsulfoxid, *N,N'*-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Dimethylformamid oder Dimethylsulfoxid verwendet.

Als Basen für den Verfahrensschritt (V) + (VI) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)-amid oder Lithiumdiisopropylamid, oder organische Amine wie Triethylamin, *N*-Methylmorpholin, *N*-Methylpiperidin, *N,N*-Diisopropylethylamin, Pyridin, 4-(*N,N*-Dimethylamino)-pyridin (DMAP), 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Kaliumcarbonat, Cäsiumcarbonat oder Natriummethanolat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Als Amino- Schutzgruppe wird bevorzugt *tert*.-Butoxycarbonyl (Boc) oder Benzyloxycarbonyl (Z) verwendet. Als Schutzgruppe für eine Hydroxy- oder Carboxyl-Funktion wird vorzugsweise *tert.-*Butyl oder Benzyl eingesetzt. Die Abspaltung dieser Schutzgruppen wird nach üblichen Methoden, vorzugsweise durch Reaktion mit einer starken Säure wie Chlorwasserstoff, Bromwasserstoff oder Trifluoressigsäure in einem inerten Lösungsmittel wie Dioxan, Diethylether, Dichlormethan oder Essigsäure durchgeführt; gegebenenfalls kann die Abspaltung auch ohne ein zusätzliches inertes Lösungsmittel erfolgen. Im Falle von Benzyl und Benzyloxycarbonyl als Schutzgruppe können diese auch durch Hydrogenolyse in Gegenwart eines Palladium-Katalysators entfernt werden. Die Abspaltung der genannten Schutzgruppen kann gegebenenfalls simultan in einer Eintopf-Reaktion oder in separaten Reaktionschritten vorgenommen werden.

Die Abspaltung der Benzylgruppe im Reaktionsschritt (I-B) → (V) erfolgt hierbei nach üblichen, aus der Schutzgruppenchemie bekannten Methoden, vorzugsweise durch Hydrogenolyse in Gegenwart von eines Palladiumkatalysators, wie beispielsweise Palladium auf Aktivkohle, in einem inerten Lösungsmittel, wie beispielsweise Ethanol oder Essigsäureethylester [siehe auch z.B. T.W. Greene und P.G.M. Wuts, Protective Groups in Organic Synthesis, Wiley, New York, 1999].

Die Verbindungen der Formel (II) sind literaturbekannt oder können hergestellt werden, indem eine Verbindung der Formel (VII) 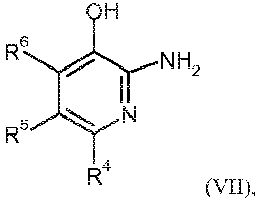 in welcher R⁴, R⁵ und R⁶ die oben angegebene Bedeutung hat,

in einem inerten Lösungsmittel in Gegenwart einer geeigneten Base mit einer Verbindung der Formel (VI) zu einer Verbindung der Formel (VIII) 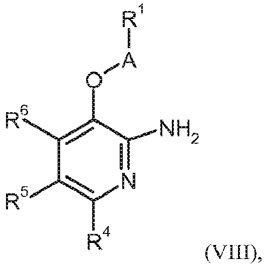 in welcher R¹, R⁴, R⁵ und R⁶ jeweils die oben angegebenen Bedeutungen haben,

umgesetzt wird, und diese anschliessend in einem inerten Lösungsmittel mit einer Verbindung der Formel (IX) 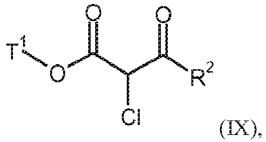 in welcher R² und T¹ jeweils die oben angegebenen Bedeutungen haben,
umgesetzt wird.

Das beschriebene Verfahren wird durch das nachfolgende Schema (Schema 3) beispielhaft verdeutlicht:

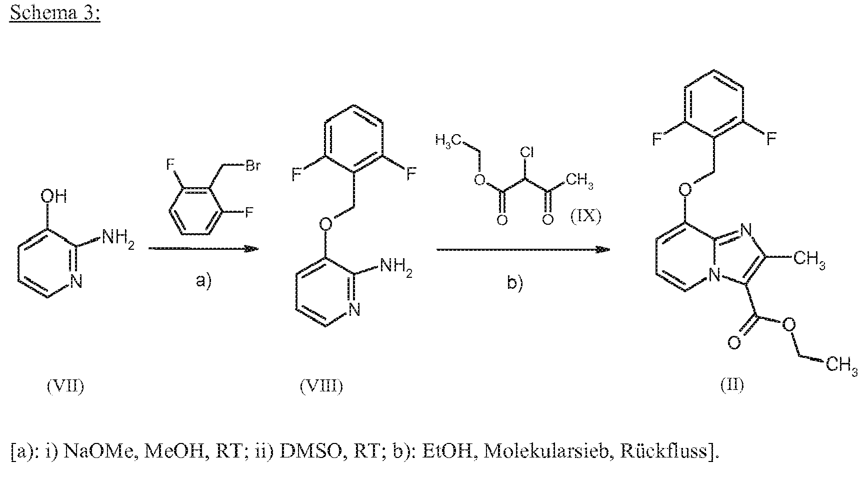

Die gezeigte Synthesesequenz kann dahingehend modifiziert werden, dass die jeweiligen Reaktionsschritte in einer veränderten Reihenfolge durchlaufen werden. Ein Beispiel für eine solche modifizierte Synthesesequenz ist in Schema 4 gezeigt.

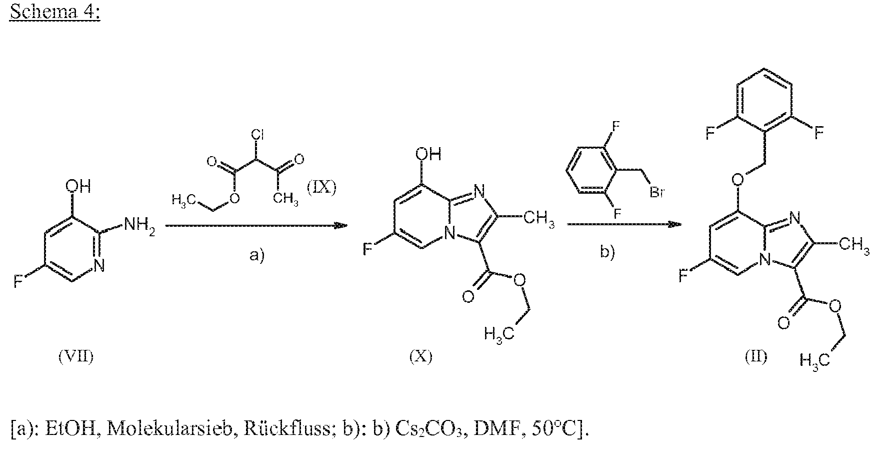

Inerte Lösungsmittel für den Ringschluss zum Imidazo[1,2-a]pyridin-Grundgerüst (VIII) + (IX) → (II) bzw. (VII) + (IX) → (X) sind die üblichen organischen Lösungsmittel. Hierzu gehören bevorzugt Alkohole wie Methanol, Ethanol, n-Propanol, Isopropanol, n-Butanol, n-Pentanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, 1,2-Dichlorethan, Acetonitril, Dimethylformamid oder Dimethylsulfoxid. Ebenso ist es möglich, Gemische der genannten Lösungsmittel einzusetzen. Bevorzugt wird Ethanol verwendet.

Der Ringschluss erfolgt im Allgemeinen in einem Temperaturbereich von +50°C bis +150°C, bevorzugt bei +50°C bis +100°C, gegebenenfalls in einer Mikrowelle.

Der Ringschluss (VIII) + (IX) → (II) bzw. (VII) + (IX) → (X) erfolgt optional in Gegenwart wasserziehender Reaktionszusätze, beispielsweise in Gegenwart von Molekularsieb (4Å Porengröße) oder mittels Wasserabscheider. Die Umsetzung (VIII) + (IX) → (II) bzw. (VII) + (IX) → (X) erfolgt unter Verwendung eines Überschusses des Reagenzes der Formel (IX), beispielsweise mit 1 bis 20 Äquivalenten des Reagenzes (IX), gegebenenfalls unter Zusatz von Basen (wie z.B. Natriumhydrogencarbonat) wobei die Zugabe dieses Reagenzes einmalig oder in mehreren Portionen erfolgen kann.

Alternativ zu den in den Schemata 1 bis 4 gezeigten Einführungen von R¹ durch Umsetzung der Verbindungen (V), (VII) oder (X) mit Verbindungen der Formel (VI), ist es ebenso möglich - wie in Schema 5 gezeigt - diese Zwischenverbindungen mit Alkoholen der Formel (XI) unter Bedingungen der Mitsunobu-Reaktion umzusetzen.

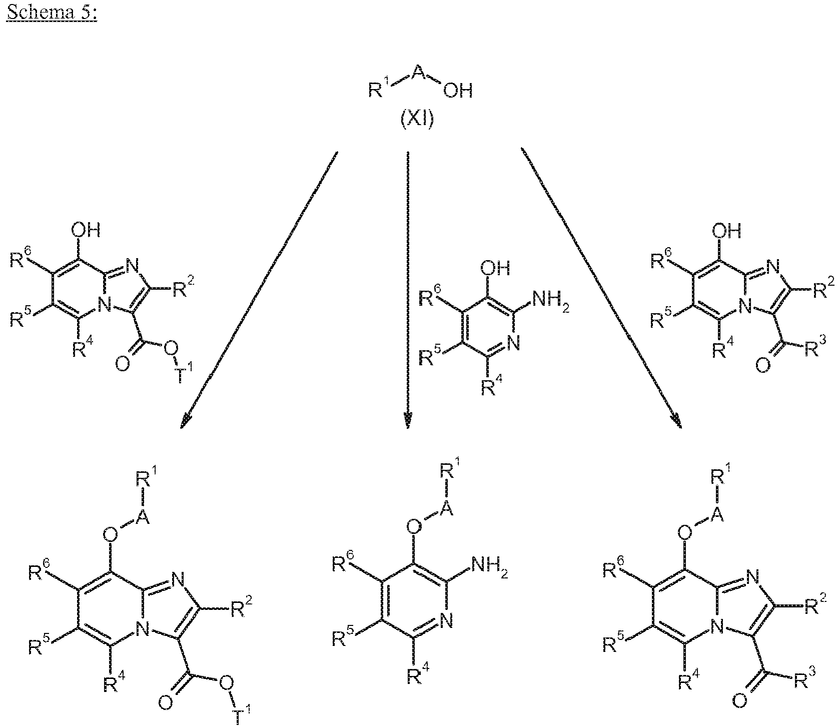

Typische Reaktionsbedingungen für derartige Mitsunobu-Kondensationen von Phenolen mit Alkoholen finden sich in der Fachliteratur, z.B. Hughes, D.L. Org. React. 1992, 42, 335; Dembinski, R. Eur. J. Org. Chem. 2004, 2763. Typischerweise wird mit einem Aktivierungsreagenz, z.B. Diethylazodicarboxylat (DEAD) oder Diisopropylazodicarboxylat (DIAD), sowie einem Phosphinreagenz, z.B. Triphenylphosphin oder Tributylphosphin, in einem inerten Lösemittel, z.B. THF, Dichlormethan, Toluol oder DMF, bei einer Temperatur zwischen 0 °C und dem Siedepunkt des verwendeten Lösemittels umgesetzt.

Weitere Ausführungsbeispiele können nach dem Fachmann bekannten Synthesemethoden hergestellt werden, wie beispielhaft in den nachfolgenden Schemata 6 -17 veranschaulicht:

Herstellung der Amine:

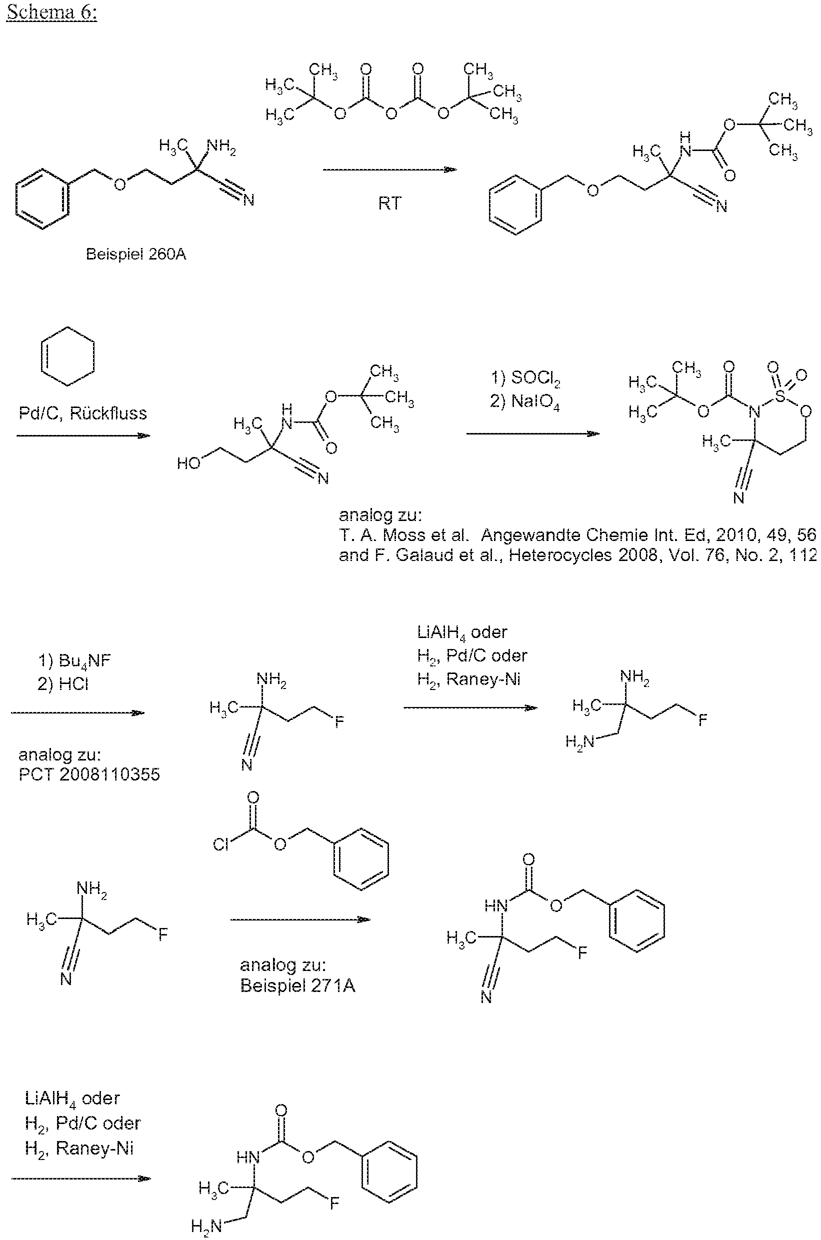

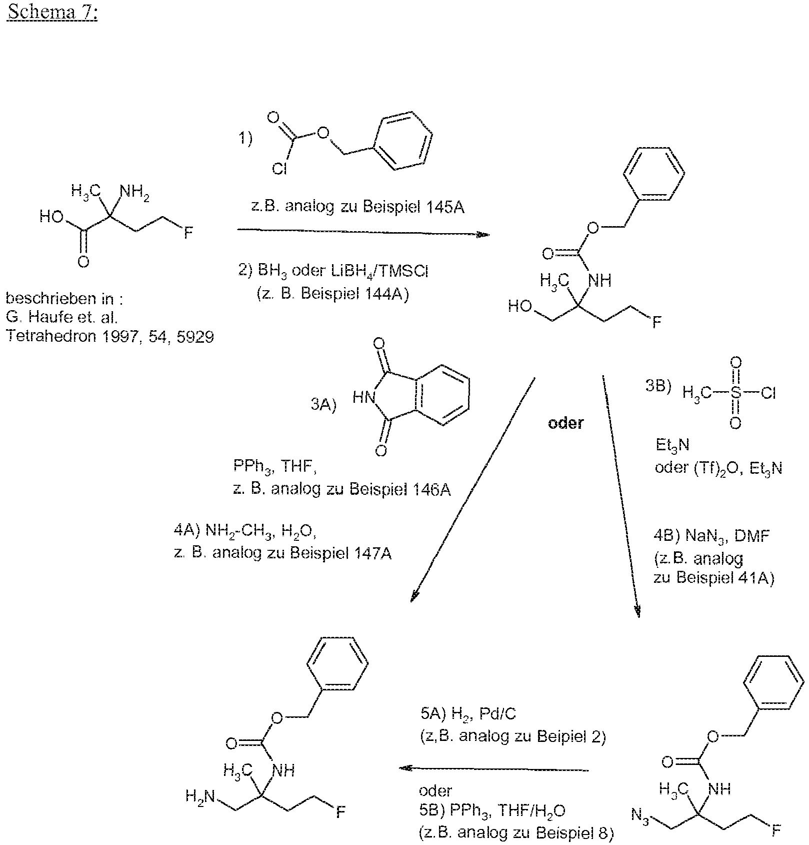

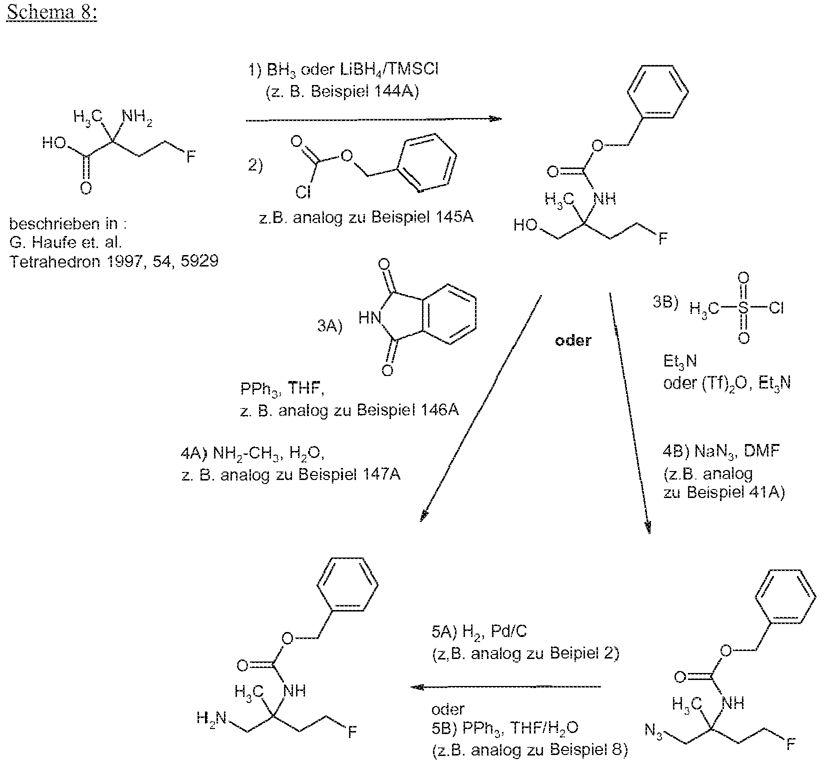

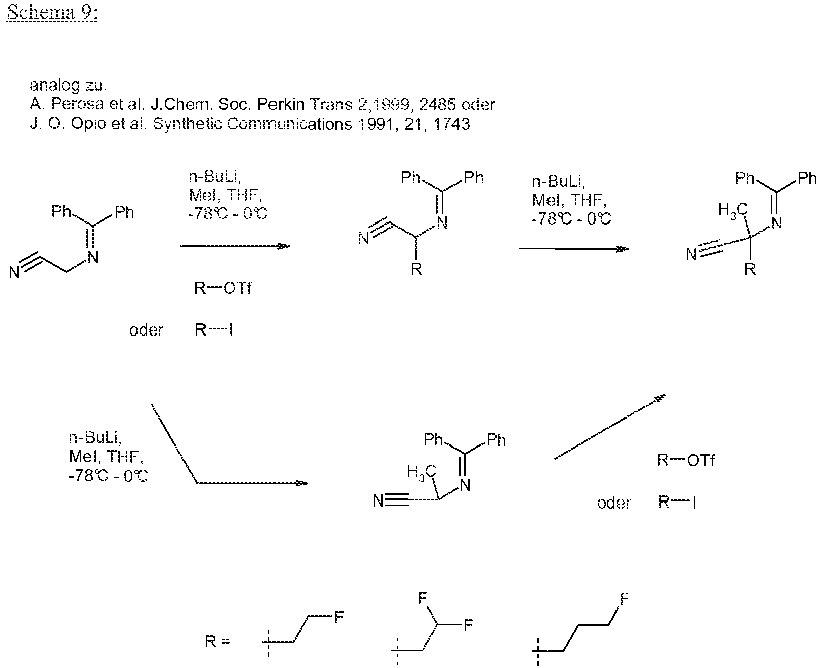

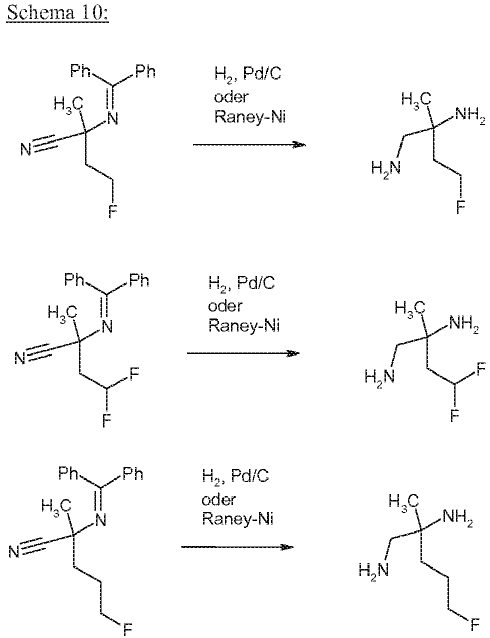

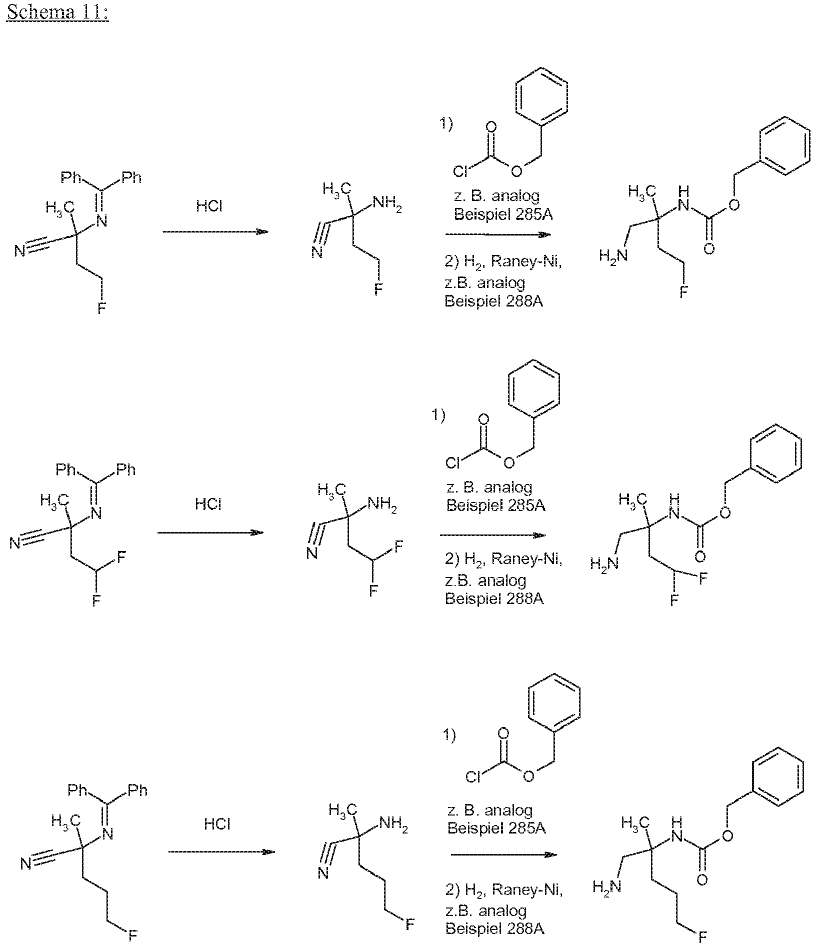

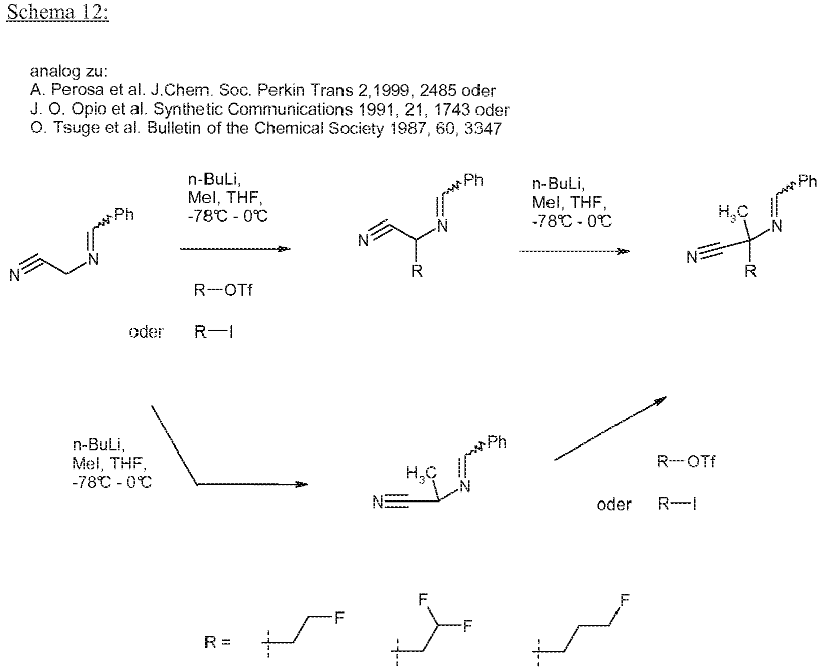

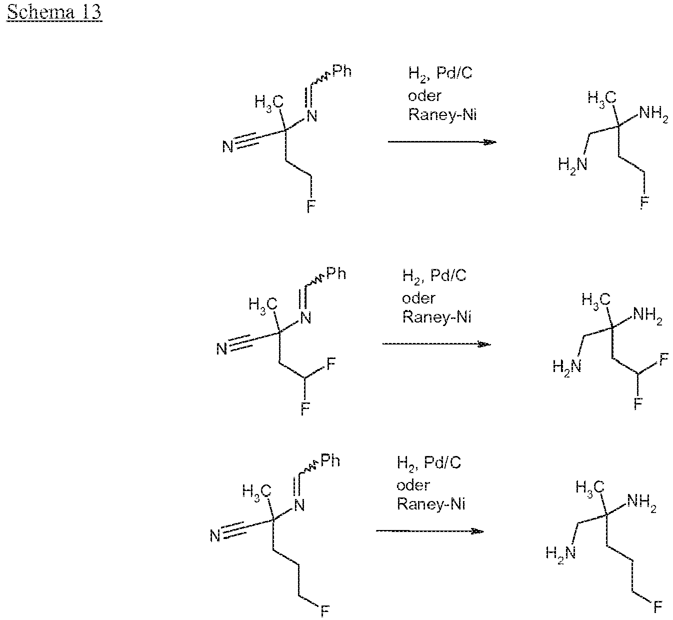

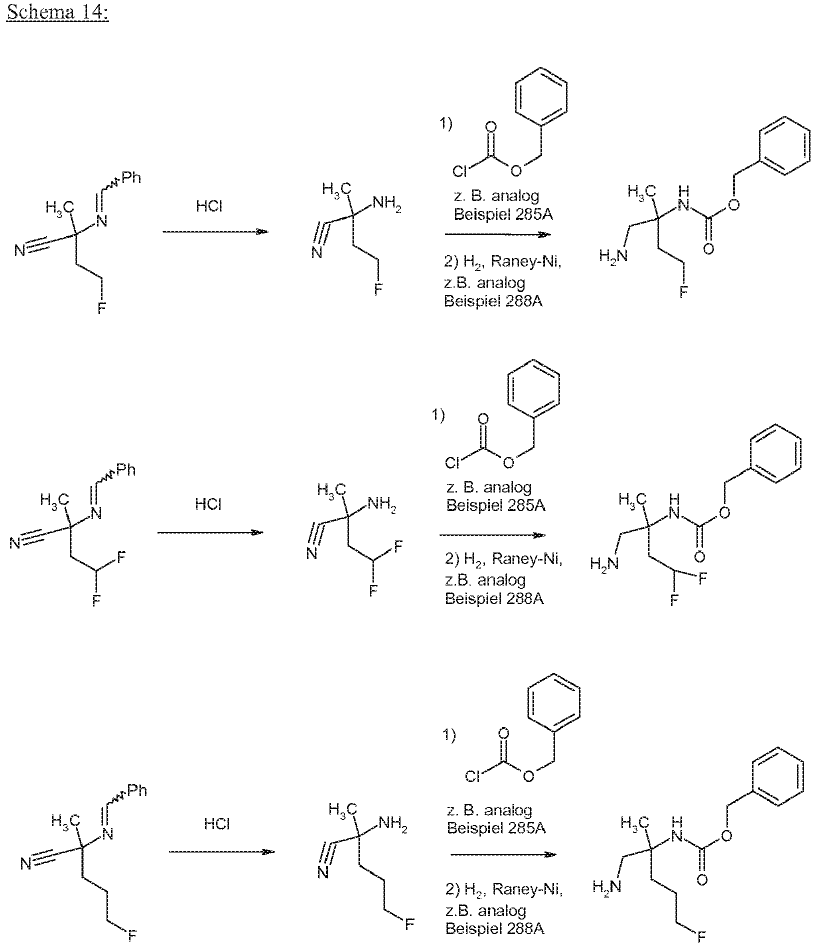

Herstellung der Ausführungsbeispiele:

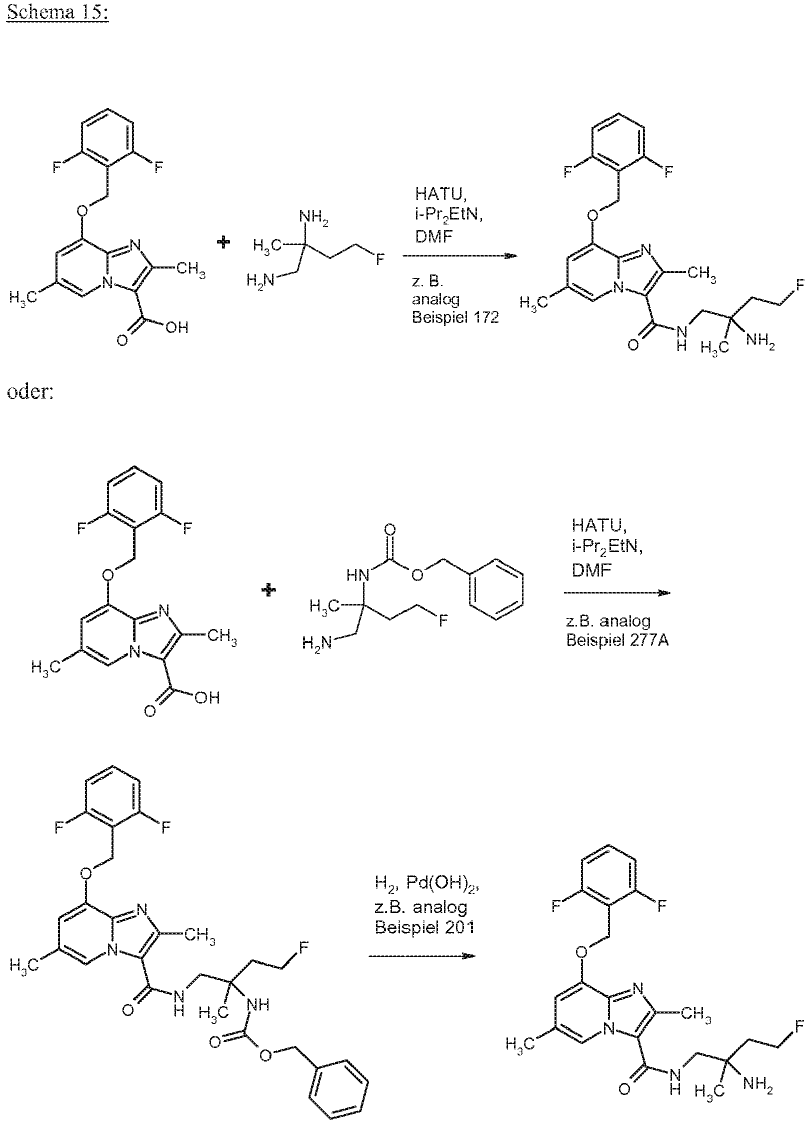

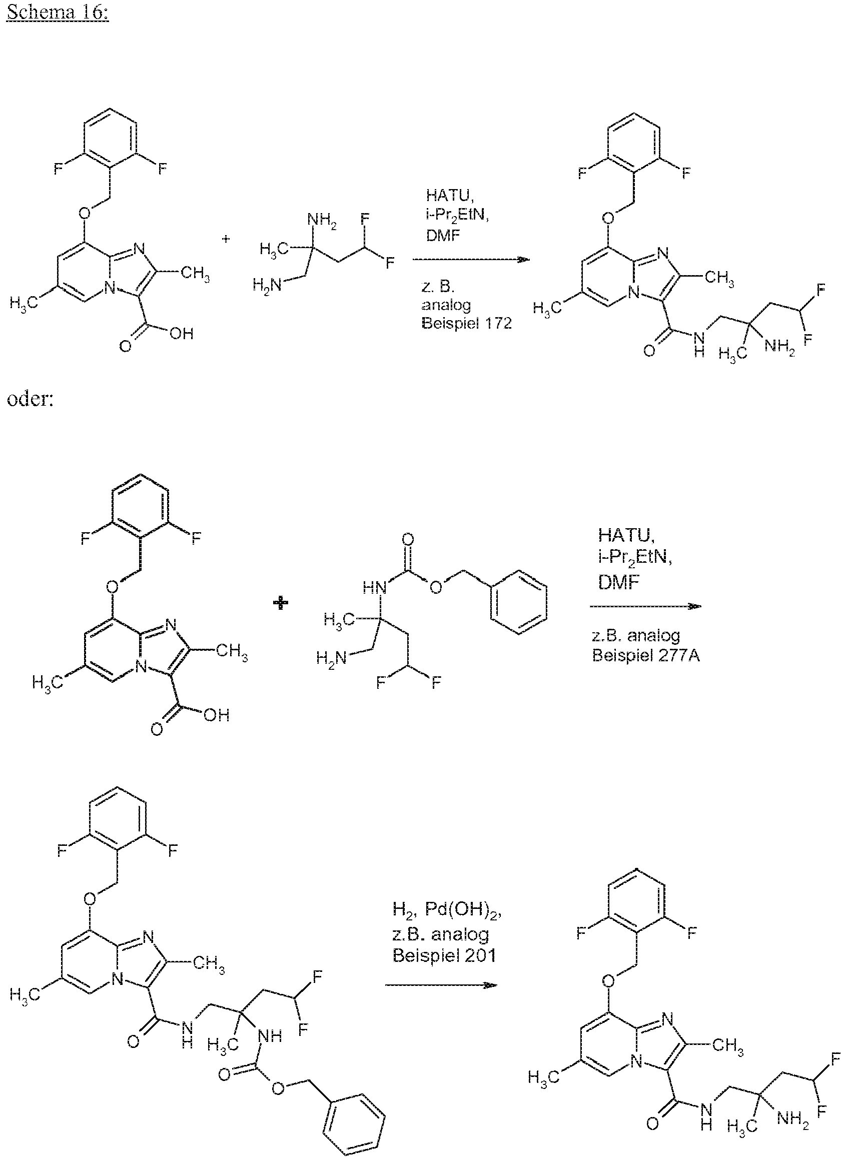

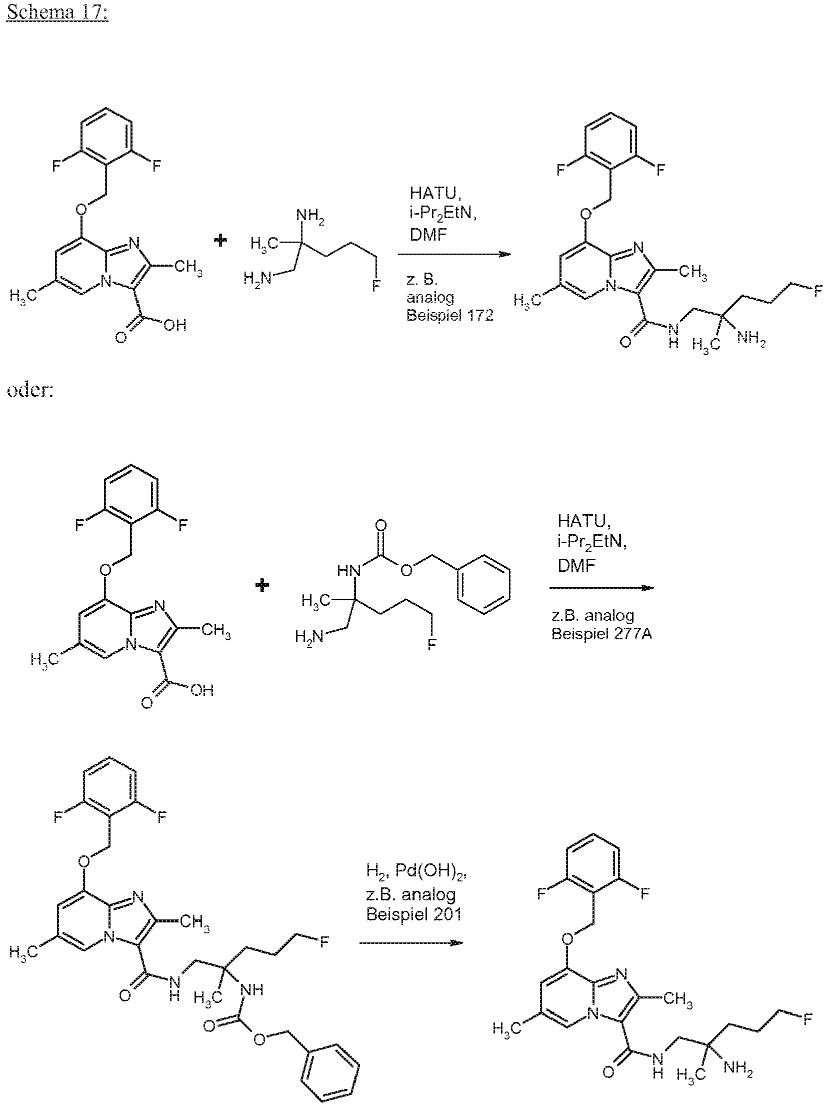

Weitere erfindungsgemäße Verbindungen können gegebenenfalls auch hergestellt werden durch Umwandlungen von funktionellen Gruppen einzelner Substituenten, insbesondere den unter R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung Etherspaltung, Bildung von Carbonamiden, sowie Einführung und Entfernung temporärer Schutzgruppen.

Die erfindungsgemäßen Verbindungen besitzen wertvolle pharmakologische Eigenschaften und können zur Vorbeugung und Behandlung von Erkrankungen bei Menschen und Tieren verwendet werden. Die erfindungsgemäßen Verbindungen eröffnen eine weitere Behandlungsalternative und stellen somit eine Bereicherung der Pharmazie dar.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfmdungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung ist ein Verfahren zur Behandlung und/oder Prophylaxe von Herzinsuffizienz, Angina pectoris, Hypertonie, pulmonaler Hypertonie, Ischämien, Gefaßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithxombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPARdelta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

**Abkürzungen und Akronyme:**

| | |
|---|---|
| abs. | absolutiert (= getrocknet) |
| aq. | wässrige Lösung |
| br | Verbreitertes Signal (NMR Kopplungsmuster) |
| δ | Verschiebung im NMR Spektrum (Angabe in ppm) |
| d | Dublett (NMR-Kopplungsmuster) |
| DCI | direkte chemische Ionisation (bei MS) |
| DMAP | 4-*N,N-*Dimethylaminopyridin |
| DMF | Dimethylformamid |
| DMSO | Dimethylsulfoxid |
| d. Th. | der Theorie (bei Ausbeute) |
| ent | Enantiomerenrein |
| eq. | Äquivalent(e) |
| ESI | Elektrospray-Ionisation (bei MS) |
| Et | Ethyl |
| h | Stunde(n) |
| HATU | (1-[Bis(dimethylamino)methylen]-1H-1,2,3-triazolo[4,5-b]pyridinium 3-oxid hexafluorophosphat) |
| HPLC | Hochdruck-, Hochleistungsflüssigchromatographie |
| HRMS | hochaufgelöste Massenspektrometrie |
| konz. | Konzentriert |
| LC/MS | Flüssigchromatographie-gekoppelte Massenspektrometrie |
| LiHMDS | Lithiumhexamethyldisilazid |
| m | Multiplett |
| Me | Methyl |
| min | Minute(n) |
| MS | Massenspektrometrie |
| NMR | Kernresonanzspektrometrie |
| Ph | Phenyl |
| q | Quartett (NMR Kopplungsmuster) |
| quint. | Quintett (NMR Kopplungsmuster) |
| rac | racemisch |
| RT | Raumtemperatur |
| Rt | Retentionszeit (bei HPLC) |
| s | Singulett (NMR Kopplungsmuster) |
| t | Triplett (NMR Kopplungsmuster) |
| TFA | Trifluoressigsäure |
| THF | Tetrahydrofuran |
| TBTU | (Benzotriazol-1-yloxy)bisdimethylaminomethyliumfluorborat |
| UV | Ultraviolett-Spektrometrie |
| v/v | Volumen zu Volumen-Verhältnis (einer Lösung) |
| XPHOS | Dicyclohexyl-(2',4',6'-triisopropylbiphenyl-2-yl)-phosphin |

### LC/MS- und HPLC-Methoden:

### Methode 1 (LC-MS):

Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 11 Acetonitril + 0.5 ml 50%ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm.

### Methode 2 (LC-MS):

Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure, Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 210 - 400 nm.

### Methode 3 (LC-MS):

Gerätetyp MS: Waters Micromass Quattro Micro; Gerätetyp HPLC: Agilent 1100 Serie; Säule: Thermo Hypersil GOLD 3 µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (DCI-MS):

Instrument: Thermo Fisher-Scientific DSQ; chemische Ionisierung; Reaktantgas NH3; Quellentemperatur: 200°C; Ionisierungsenergie 70eV.

### Methode 5 (LC-MS):

Gerätetyp MS: Waters (Micromass) Quattro Micro; Gerätetyp HPLC: Agilent 1100 Serie; Säule : Thermo Hypersil GOLD 3 µ 20 x 4 mm; Eluent A: 11 Wasser + 0.5 ml 50%ige Ameisensäure, Eluent B: 11 Acetonitril + 0.5 ml 50%ige Ameisensäure; Gradient: 0.0 min 100% A → 3.0 min 10% A → 4.0 min 10% A; Ofen: 50°C; Fluss: 2 ml/min; UV-Detektion: 210 nm

### Methode 6 (GC-MS):

Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 7 (LC-MS):

Instrument: Waters ACQUITY SQD UPLC System; Säule: Waters Acquity UPLC HSS T3 1.8 µ 30 x 2 mm; Eluent A: 11 Wasser + 0.25 ml 99%ige Ameisensäure, Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 8 (LC-MS):

Instrument MS: Waters SQD; Instrument HPLC: Waters UPLC; Saeule: Zorbax SB-Aq (Agilent), 50 mm x 2.1 mm, 1.8 µm; Eluent A: Wasser + 0.025% Ameisensaeure, Eluent B: Acetonitril (ULC) + 0.025% Ameisensaeure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode 9 (präparative HPLC):

Säule: Macherey-Nagel VP 50/21 Nucleosil 100-5 C18 Nautilus. Flussrate: 25 ml/min. Gradient: A = Wasser + 0.1 % konz. wässriger Ammoniak, B = Methanol, 0 min = 30 % B, 2 min = 30% B, 6 min = 100% B, 7 min = 100% B, 7.1 min = 30% B, 8 min = 30% B, Flussrate 25 ml/min, UV-Detektion 220 nm.

### Methode 10 (FIA/MS, ES):

Instrument: Waters ZQ 2000; Elektrospray-Ionisierung; Eluent A: 11 Wasser + 0.25 ml 99%ige Ameisensäure , Eluent B: 11 Acetanitril + 0.25 ml 99%ige Ameisensäure; 25% A, 75% B; Fluss: 0.25 ml/min

### Methode 11:

Instrument MS: Waters (Micromass) Quattro Micro; Instrument HPLC: Agilent 1100 Serie; Säule: YMC-Triart C18 3 µ 50 x 3 mm; Eluent A: 11 Wasser + 0.01 mol Ammoniumcarbonat, Eluent B: 1 1 Acetonitril; Gradient: 0.0 min 100% A → 2.75 min 5% A → 4.5 min 5% A; Ofen: 40°C; Fluss: 1.25 ml/min; UV-Detektion: 210 nm

### Methode 12 (präparative LC-MS):

Instrument MS: Waters, Instrument HPLC: Waters (Säule Waters X-Bridge C18, 18 mm x 50 mm, 5 µm, Eluent A: Wasser + 0.05% Triethylamin, Eluent B: Acetonitril (ULC) + 0.05% Triethylamin, Gradient: 0.0 min 95%A - 0.15 min 95%A - 8.0 min 5%A - 9.0 min 5%A; Fluss: 40 ml/min; UV-Detektion: DAD; 210 - 400 nm).

### bzw.

Instrument MS: Waters, Instrument HPLC: Waters (Säule Phenomenex Luna 5µ C18(2) 100A, AXIA Tech. 50 x 21.2 mm, Eluent A: Wasser + 0.05% Ameisensäuren, Eluent B: Acetonitril (ULC) + 0.05% Ameisensäure, Gradient: 0.0 min 95%A - 0.15 min 95%A - 8.0 min 5%A - 9.0 min 5%A; Fluss: 40 ml/min; UV-Detektion: DAD; 210 - 400 nm).

### Methode 13

Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 11 Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 14:

Instrument: Thermo Scientific DSQII, Thermo Scientific Trace GC Ultra; Säule: Restek RTX-35MS, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

### Methode 15:

Instrument MS: Waters (Micromass) QM; Instrument HPLC: Agilent 1100 Serie; Säule : Agilent ZORBAX Extend-C18 3.0x50mm 3.5-Micron; Eluent A: 11 Wasser + 0.01 mol Ammoniumcarbonat, Eluent B: 11 Acetonitril; Gradient: 0.0 min 98% A → 0.2 min 98% A → 3.0 min 5% A → 4.5 min 5% A; Ofen: 40°C; Fluss: 1.75 ml/min; UV-Detektion: 210 nm.

### Methode 16 (LC-MS):

Instrument: Agilent MS Quad 6150; HPLC: Agilent 1290; Säule: Waters Acquity UPLC HSS T3 1.8 µ 50 x 2.1 mm; Eluent A: 11 Wasser + 0.25 ml 99%ige Ameisensäure, Eluent B: 11 Acetonitril + 0.25 ml 99%ige Ameisensäure; Gradient: 0.0 min 90% A → 0.3 min 90% A → 1.7 min 5% A → 3.0 min 5% A Ofen: 50°C; Fluss: 1.20 ml/min; UV-Detektion: 205 - 305 nm.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische bzw. saure Funktionalität enthalten. Ein solches Salz kann durch verschiedene dem Fachmann bekannte Methoden in die entsprechende freie Base bzw. Säure überführt werden.

Salze können unter- oder überstöchiometrisch vorliegen, insbesondere bei Vorliegen eines Amins oder einer Carbonsäure. Zusätzlich können bei den vorliegenden Imidazopyridinen unter sauren Bedingungen stets Salze, auch unterstöchiometrisch, vorliegen, ohne dass diese im ¹H-NMR erkenntlich sind und ohne besondere Angabe und Kennzeichnung dieser in den jeweiligen IUPAC-Namen und Strukturformeln.

Alle Angaben in ¹H-NMR-Spektren geben die Chemischen Verschiebungen δ in ppm an.

Die in den folgenden Paragraphen angegebenen Multiplizitäten von Protonensignalen in ¹H-NMR-Spektren geben die jeweils beobachtete Signalform wieder und berücksichtigen keine Signalphänomene höherer Ordnung.

Die Methyl-Gruppe des chemischen Systems "2-methylimidazo[1,2-a]pyridin" erscheint in ¹H-NMR-Spektren als Singulett (oftmals in DMSO-d₆ und im Bereich von 2.40 - 2.60 ppm) und ist etweder klar als solches erkennbar, ist mit den Lösungsmittelsignalen überlagert oder liegt vollständig unter den Signalen der Lösungsmittel. Die Angabe dieses Signals in den ¹H-NMR-Spektren kann antizipierend erfolgen.

### Röntgenstrukturanalyse:

| | |
|---|---|
| Transmissionsdiffraktometer: | Bruker Diffraktometer mit Apex-II-CCD Detektor |
| Strahlung: | Kupfer, K alpha |
| Primär-Monochromator: | fokussierender Röntgenspiegel |
| Messbereich: | 4.73-67.08° |
| Raumbedingungen: | 20°C |

### Allgemeine Arbeitsvorschriften

### Repräsentative Arbeitsvorschrift 1

### Reduktion von Aminosäuren unter Verwendung von Lithiumborhydrid und Chlortrimethylsilan.

1.7-2.5 Äquivalente Lithiumborhydrid wurden in THF vorgelegt (ca. 0.1-0.5 M bezogen auf die Aminosäure) und mit 3.4-5.0 Äquivalente Chlortrimethylsilan (bei 0°C oder RT) versetzt und fünf bis 30 min bei RT gerührt. Anschließend wurde 1 Äquivalent der Aminosäure vorsichtig portionsweise bei 0°C oder RT zugegeben und das Reaktionsgemisch wurde über Nacht bei RT gerührt.

Beispielhafte Aufarbeitung der Reaktionsmischung: Es wurde Methanol zugegeben und das Gemisch wurde eingeengt. Der Rückstand wurde mit einer 20%igen Kaliumhydroxid-Lösung versetzt und dreimal mit Dichlormethan extrahiert. Die vereinigten organischen Phasen wurden über Natrimsulfat getrocknet, filtriert und eingeengt.

### Repräsentative Arbeitsvorschrift 2

### Amidbildung unter Verwendung von TBTU als Kupplungsreagenz.

1 Äquivalent der zu kuppelnden Carbonsäure (z.B. Beispiel 3A), 1.1 - 1.5 Äquivalente (Benzotriazol-1-yloxy)bisdimethylammomethyliumfluorborat (TBTU) und 3-6 Äquivalente 4-Methylmorpholin wurden in DMF oder Dichlormethan (ca. 0.1-0.2 M bezogen auf die zu kuppelnde Carbonsäure) vorgelegt. Anschließend wurden 1.1 bis 1.5 Äquivalente des zu kuppelnden Amins zugesetzt und es wurde über Nacht bei RT gerührt.

Beispielhafte Aufarbeitung der Reaktionsmischung: Die Reaktionslösung wurde mit Wasser versetzt, der entstandene Niederschlag noch 0.5-1.0 h ausgerührt, abfiltriert, gut mit Wasser gewaschen und über Nacht im Hochvakuum getrocknet. Alternativ wurde das Reaktionsgemisch direkt eingeengt, per präparativer HPLC weiter aufgereinigt und über Nacht im Hochvakuum getrocknet.

Gegebenenfalls wurde das Reaktionsgemisch vom Niederschlag abfiltriert, mit Diethylether gewaschen und im Hochvakuum getrocknet.

Gegebenenfalls wurde das Reaktionsgemisch mit Diethylether verdünnt, der Niederschlag abfiltriert und zwischen Essigsäureethylester oder Dichlormethan und gesättigter, wässriger Natriumhydrogencarbonatlösung verteilt. Die organische Phase wurde mit gesättigter, wässriger Natriumchlorid-Lösung gewaschen, über Natriumsulfat getrocknet, filtriert, das Filtrat eingeengt und im Hochvakuum getrocknet.

### Repräsentative Arbeitsvorschrift 3

### Amidbildung unter Verwendung von HATU als Kupplungsreagenz.

1 Äquivalent der zu kuppelnden Carbonsäure (z.B. Beispiel 3A, 6A, 11A, 16A, 19A, 21A, 25A, 28A oder 30A), 1.1 bis 2.5 Äquivalente *O*-(7-Azabenzotriazol-1-yl)-*N,N,N'N'-*tetramethyluroniumhexafluorphosphat (HATU) und 3 bis 4 Äquivalente *N,N*-Diisopropylethylamin wurden in DMF (ca. 0.2 M bezogen auf die zu kuppelnde Carbonsäure) vorgelegt und mit 1.2 bis 2.0 Äquivalenten des zu kuppelnden Amins versetzt und über Nacht bei RT gerührt.

Beispielhafte Aufarbeitung der Reaktionsmischung: Die Reaktionslösung wurde mit Wasser versetzt, der entstandene Niederschlag noch 30 min ausgerührt, abfiltriert, gut mit Wasser gewaschen und über Nacht im Hochvakuum getrocknet. Alternativ wurde das Reaktionsgemisch entweder direkt nach Aufkonzentration im Vakuum oder nach extraktiver Aufarbeitung per präparativer HPLC weiter aufgereinigt.

### Ausgangsverbindungen und Intermediate:

### Beispiel 1A

### 3-[(2,6-Difluorbenzyl)oxy]pyridin-2-amin

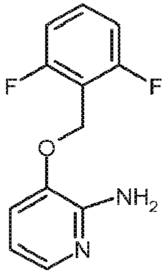

51 g Natriummethanolat (953 mmol, 1.05 Äquivalente) wurden in 1000 ml Methanol bei RT vorgelegt, mit 100 g 2-Amino-3-hydroxypyridin (908 mmol, 1 Äquivalent) versetzt und 15 min bei RT weitergerührt. Die Reaktionsmischung wurde am Vakuum eingeengt, der Rückstand in 2500 ml DMSO aufgenommen und mit 197 g 2,6-Difluorbenzylbromid (953 mmol, 1.05 Äquivalente) versetzt. Nach 4 h bei RT wurde das Reaktionsgemisch auf 20 L Wasser gegossen, für 15 min nachgerührt und der Feststoff abfiltriert. Der Feststoff wurde mit 1 L Wasser sowie 100 ml Iso-Propanol und 500 ml Petrolether nachgewaschen und im Hochvakuum getrocknet. Es wurden 171 g der Titelverbindung (78% d. Th) erhalten.

¹H-NMR (400 MHz, DMSO-d₆): δ = 5.10 (s, 2 H); 5.52 (br. s, 2 H), 6.52 (dd, 1 H); 7.16 - 7.21 (m, 3 H); 7.49 - 7.56 (m, 2 H).

### Beispiel 2A

### Ethyl-8-[(2,6-difluorbenzyl)oxy]-2-methylimidazo[1,2-a]pyridin-3-carboxylat

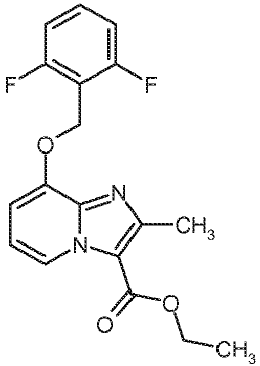

170 g 3-[(2,6-Difluorbenzyl)oxy]pyridin-2-amin (Beispiel 1A; 719 mmol, 1 Äquivalent) wurden in 3800 ml Ethanol vorgelegt, mit 151 g gepulvertem Molekularsieb 3Å und 623 g Ethyl-2-chloracetoacetat (3.6 mol, 5 Äquivalente) versetzt. Die Reaktionsmischung wurde für 24 h zum Rückfluß erhitzt, anschließend über Kieselgel abfiltriert und am Vakuum aufkonzentriert. Es wurde 48 h bei RT belassen und der enstandene Feststoff filtriert. Dann wurde dreimal mit wenig Iso-Propanol gerührt und anschließend abfiltriert und mit Diethylether gewaschen. Es wurden 60.8 g (23% d. Th.) der Titelverbindung erhalten. Die vereinigten Filtrate der Filtrationsschritte wurde eingeengt und der Rückstand an Kieselgel mit Cyclohexan/Diethylether als Eluent chromatographiert. Man erhielt weitere 46.5 g (18% d. Th.; Gesamtausbeute: 41% d. Th.) der Titelverbindung.
LC-MS (Methode 2): Rₜ = 1.01 min
MS (ESpos): m/z = 347 (M+H)⁺
¹H-NMR (400 MHz, DMSO-d₆): δ = 1.36 (t, 3 H); 2.54 (s, 3 H; verdeckt durch DMSO-Signal); 4.36 (q, 2 H); 5.33 (s, 2 H); 7.11 (t, 1 H); 7.18 - 7.27 (m, 3 H); 7.59 (quint, 1 H); 8.88 (d, 1 H).

### Beispiel 3A

### 8-[(2,6-Difluorbenzyl)oxy]-2-methylimidazo[1,2-a]pyridin-3-carbonsäure

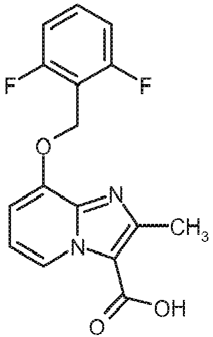

107 g Ethyl-8-[(2,6-difluorbenzyl)oxy]-2-methylimidazo[1,2-a]pyridin-3-carboxylat (Beispiel 2A; 300 mmol, 1 Äquivalent) wurde in 2.8 L THF/Methanol (1:1) gelöst, mit 1.5 L 1 N wässriger Lithiumhydroxid-Lösung (1.5 mol, 5 Äquivalente) versetzt und 16 h bei RT gerührt. Die organischen Lösemittel wurden am Vakuum entfernt und die resultierende wässrige Lösung im Eisbad mit 1 N wässriger Salzsäure auf pH 3-4 eingestellt. Der resultierende Feststoff wurde abfiltriert, mit Wasser und Iso-Propanol nachgewaschen und im Vakuum getrocknet. Es wurden 92 g (95% d. Th.) der Titelverbindung erhalten.
LC-MS (Methode 2): Rₜ = 0.62 min
MS (ESpos): m/z = 319.1 (M+H)⁺
¹H-NMR (400 MHz, DMSO-d₆): δ = 2.55 (s, 3 H; überlagert durch DMSO-Signal); 5.32 (s, 2 H); 7.01 (t, 1 H); 7.09 (d, 1 H); 7.23 (t, 2 H); 7.59 (quint, 1 H); 9.01 (d, 1 H).

### Beispiel 4A

### 3-(Cyclohexylmethoxy)pyridin-2-amin

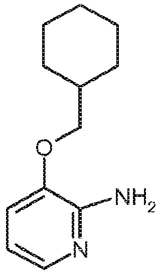

96 g Natriumhydroxid 45%ig in Wasser (1081 mmol, 1 Äquivalente) wurden in 1170 ml Methanol bei RT vorgelegt, mit 119 g 2-Amino-3-hydroxypyridin (1080 mmol, 1 Äquivalent) versetzt und 10 min bei RT weitergerührt. Die Reaktionsmischung wurde am Vakuum eingeengt, der Rückstand in 2900 ml DMSO aufgenommen und mit 101 g Cyclohexylmethylbromid (1135 mmol, 1.05 Äquivalente) versetzt. Nach 16 h bei RT wurde das Reaktionsgemisch langsam zu 6 L Wasser gegeben, die wässrige Lösung zweimal mit je 2 L Essigsäureethylester extrahiert. Die vereinigten organischen Phasen wurden mit je 1 L gesättigter, wässriger Natriumhydrogencarbonat-Lösung und Wasser gewaschen, getrocknet, filtriert und eingeengt. Der Rückstand wurde mit 500 ml n-Pentan verrührt, filtriert und am Vakuum getrocknet. Es wurden 130 g (58% d. Th.) der Titelverbindung erhalten.
LC-MS (Methode 3): Rₜ = 1.41 min
MS (ESpos): m/z = 207.1 (M+H)⁺

### Beispiel 5A

### Ethyl-8-(cyclohexylmethoxy)-2-methylimidazo[1,2-a]pyridin-3-carboxylat

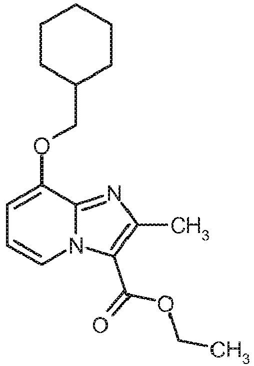

130 g 3-(Cyclohexylmethoxy)pyridin-2-amin (Beispiel 4A; 630 mmol, 1 Äquivalent) wurden in 3950 ml Ethanol vorgelegt und mit 436 ml Ethyl-2-chloracetoacetat (3.2 mol, 5 Äquivalente) versetzt. Es wurde 24 h am Rückfluß erhitzt und anschließend im Vakuum eingeengt. Das so erhaltene Rohprodukt wurde an Kieselgel mit Cyclohexan/Diethylether als Eluent chromatographiert und lieferte 66.2 g (33% d. Th.) der Titelverbindung.
LC-MS (Methode 2): Rₜ = 1.17 min
MS (ESpos): m/z = 317.1 (M+H)⁺
¹H-NMR (400 MHz, DMSO-d₆): δ =1.02-1.31 (m, 5 H); 1.36 (t, 3 H); 1.64 -1.77 (m, 3 H); 1.79 - 1.90 (m, 3 H); 2.60 (s, 3 H); 3.97 (d, 2 H); 4.35 (q, 2 H); 6.95 (d, 1 H); 7.03 (t, 1 H); 8.81 (d, 1 H).

### Beispiel 6A

### 8-(Cyclohexy]methoxy)-2-methylimidazo[1,2-a]pyridin-3-carbonsäure

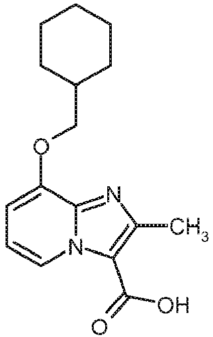

50 g Ethyl-8-(cyclohexy]methoxy)-2-methylimidazo[1,2-a]pyridin-3-carboxylat (Beispiel 5A; 158 mmol, 1 Äquivalent) wurde in 600 ml 1,4-Dioxan gelöst, mit 790 ml 2 N Natronlauge (1.58 mol, 10 Äquivalente) versetzt und 16 h bei RT gerührt. Es wurde mit 316 ml 6 N Salzsäure versetzt und auf ca. 1/5 des Gesamtvolumens eingeengt. Der resultierende Feststoff wurde abfiltriert, mit Wasser und tert.-Butylmethylether nachgewaschen und im Vakuum getrocknet. Es wurden 35 g (74% d. Th.) der Titelverbindung erhalten.
LC-MS (Methode 2): Rₜ = 0.81 min
MS (ESpos): m/z = 289.0 (M+H)⁺
¹H-NMR (400 MHz, DMSO-d₆): δ = 1.03-1.44 (m, 5 H); 1.64 - 1.78 (m, 3 H); 1.81 - 1.92 (m, 3 H); 2.69 (s, 3 H); 4.07 (d, 2 H); 7.30 - 7.36 (m, 2 H); 9.01 (d, 1 H).

### Beispiel 7A

### 5-Fluor-2-nitropyridin-3-ol

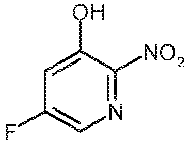

5 g 5-Fluorpyzidin-3-ol (44 mmol, 1 Äquivalent) wurden unter Eiskühlung in 43 ml konzentrierter Schwefelsäure gelöst und bei 0 °C innerhalb von 5 min mit 2.8 ml konzentrierter Salpetersäure versetzt. Die Reaktion wurde auf RT erwärmt und über Nacht weitergerührt. Es wurde auf 100 g Eis gegeben und für 30 min nachgerührt. Der erhaltene Feststoff wurde abfiltriert und im Vakuum getrocknet. Es wurden 5.6 g (81% d. Th.) der Titelverbindung erhalten und ohne weitere Aufreinigung in die Folgereaktion eingesetzt.
LC-MS (Methode 2): Rₜ = 0.45 min
MS (ESneg): m/z = 156.9 (M-H)-
¹H-NMR (400 MHz, DMSO-d₆): δ = 7.5 (dd, 1 H); 8.08 (d, 1 H); 12.2 (br. s, 1 H).

### Beispiel 8A

### 2-Amino-5-fluorpyridin-3-ol

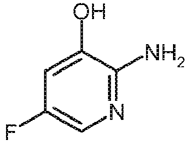

5.6 g 5-Fluor-2-nitropyridin-3-ol (Beispiel 7A; 36 mmol) wurden in 2 L Ethanol gelöst, mit einer katalytischen Menge Palladium auf Aktivkohle (10%-ig) versetzt und 16 h unter Wasserstoff-Normaldruck hydriert. Es wurde über Kieselgel abfiltriert und das Filtrat eingeengt (Produktcharge 1). Der Filterkuchen wurde mit Methanol solange nachgespült bis das Filtrat keine gelbliche Färbung mehr aufwies. Das Filtrat wurde eingeengt und ergab eine zweite Produktcharge. Es wurden 4.26 g (85% d. Th.) der Titelverbindung erhalten.
LC-MS (Methode 2): Rₜ = 0.17 min
MS (ESpos): m/z = 128.9 (M+H)⁺
¹H-NMR (400 MHz, DMSO-d₆): δ = 5.4 (br. s, 2 H); 6.8 (dd, 1 H); 7.4 (d, 1 H).

### Beispiel 9A

### Ethyl-6-fluor-8-hydroxy-2-methylimidazo[1,2-a]pyridin-3-carboxylat

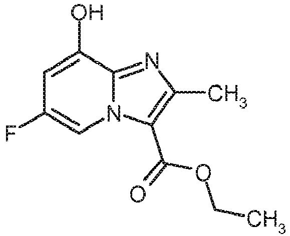

3.2 g 2-Amino-5-fluorpyridin-3-ol (Beispiel 8A; 25 mmol, 1 Äquivalent) wurden in 155 ml Ethanol vorgelegt, mit 1.5 g gepulvertem Molekularsieb 3Å und 20.6 g Ethyl-2-chloracetoacetat (125 mmol, 5 Äquivalente) versetzt und über Nacht am Rückfluß gekocht. Die Reaktionslösung wurde eingeengt und chromatographiert (Biotage Isolera Four, SNAP Cartridge KP-Sil 50 g; Cyclohexan-Essigsäureethylester Gradient; nachfolgend Dichlormethan-Methanol-Gradient). Das Rohprodukt wurde in wenig Methanol angelöst und mit tert.-Butylmethylether versetzt. Der Feststoff wurde abfiltriert und mit tert.-Butylmethylether gewaschen. Es wurden 570 mg (10% d. Th.) der Titelverbindung erhalten.
LC-MS (Methode 2): Rₜ = 0.77 min
MS (ESpos): m/z = 239.2 (M+H)⁺
¹H-NMR (400 MHz, DMSO-d₆): δ = 1.39 (t, 3 H); 2.64 (s, 3 H); 4.40 (q, 2 H); 7.20 (br. d, 1 H); 8.9 (dd, 1 H); 12.5 (br. s, 1 H).

### Beispiel 10A

### Ethyl-8-[(2,6-difluorbenzyl)oxy]-6-fluor-2-methylimidazo[1,2-a]pyridin-3-carboxylat

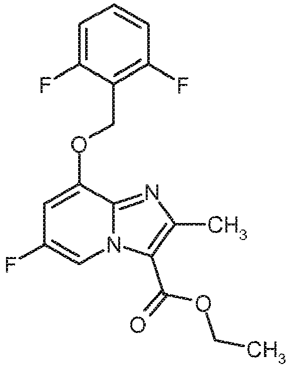

560 mg Ethyl-6-fluor-8-hydroxy-2-methylimidazo[1,2-a]pyridin-3-carboxylat (Beispiel 9A; 2.4 mmol, 1.0 Äquivalent), 1.7 g Cäsiumcarbonat (5.17 mmol, 2.2 Äquivalente) und 535 mg 2,6-Difluorbenzylbromid (2.6 mmol, 1.1 Äquivalente) wurden in 34 ml trockenem DMF vorgelegt und für 15 min auf 50°C erwärmt. Es wurde mit Wasser versetzt, 30 min nachgerührt, der Feststoff abfiltriert und mit Wasser gewaschen. Es wurden 560 mg der Titelverbindung (65% d. Th.) erhalten.
LC-MS (Methode 2): Rₜ = 1.18 min
MS (ESpos): m/z = 365.1 (M+H)⁺
¹H-NMR (400 MHz, DMSO-d₆): δ = 1.37 (t, 3 H); 2.55 (s, 3 H; überlagert durch DMSO-Signal); 4.38 (q, 2 H); 5.89 (s, 2 H); 7.23 (t, 2 H); 7.44 (dd, 1 H); 7.60 (q, 1 H); 8.90 (dd, 1 H).

### Beispiel 11A

### 8-[(2,6-Difluorbenzyl)oxy]-6-fluor-2-methylimidazo[1,2-a]pyridin-3-carbonsäure

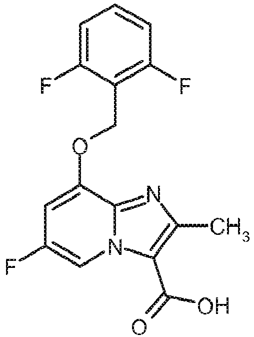

550 mg Ethyl-8-[(2,6-difluorbenzyl)oxy]-6-fluor-2-methylimidazo[1,2-a]pyridin-3-carboxylat (Beispiel 10A; 1.5 mmol, 1 Äquivalent) wurden in 64 ml THF und 12 ml Methanol gelöst, mit 7.5 ml 1 N wässriger Lithiumhydroxid-Lösung versetzt und über Nacht bei RT gerührt. Dann wurde mit 8 ml 1 N wässriger Salzsäure versetzt und im Vakuum eingeengt. Der entstandene Feststoff wurde abfiltriert und mit Wasser nachgewaschen. Es wurden 429 mg der Titelverbindung (80% d. Th.) erhalten.
LC-MS (Methode 1): Rₜ = 0.90 min
MS (ESpos): m/z = 337.1 (M+H)⁺
¹H NMR (400 MHz, DMSO-d₆): δ = 2.54 (s, 3 H; überlagert durch DMSO-Signal); 5.84 (s, 2 H); 7.23 (t, 2 H); 7.40 (dd, 1 H); 7.51 (q, 1 H); 8.92 (dd, 1 H); 13.28 (br. s, 1 H).

### Beispiel 12A

### 5-Chlor-2-nitropyridin-3-ol

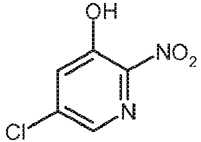

30 g 5-Chlorpyridin-3-ol (232 mmol, 1 Äquivalent) wurden unter Eiskühlung in 228 ml konzentrierter Schwefelsäure gelöst und bei 0 °C langsam mit 24 ml konzentrierter Salpetersäure versetzt. Die Reaktion wurde auf RT erwärmt, über Nacht gerührt und anschließend in ein Eis/Wasser-Gemisch eingerührt und für 30 min nachgerührt. Der Feststoff wurde abfiltriert, mit kaltem Wasser nachgewaschen und an der Luft getrocknet. Es wurden 33 g (82% d. Th.) der Titelverbindung erhalten und ohne weitere Aufreinigung in die Folgereaktion eingesetzt.
LC-MS (Methode 2): Rₜ = 0.60 min
MS (ESneg): m/z = 172.9/174.9 (M-H)⁻
¹H-NMR (400 MHz, DMSO-d₆): δ = 7.71 (d, 1 H); 8.10 (d, 1 H); 12.14 (br. 1 H).

### Beispiel 13A

### 5-Chlor-3-[(2,6-difluorbenzyl)oxy]-2-nitropyridin

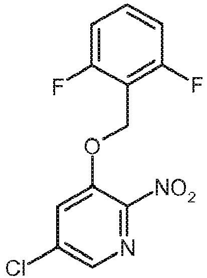

33 g 5-Chlor-2-nitropyridin-3-ol (Beispiel 12A; 189 mmol, 1 Äquivalent) und 61.6 g Cäsiumcarbonat (189 mmol, 1 Äquivalent) wurden in 528 ml DMF vorgelegt, mit 40.4 g 2,6-Difluorbenzylbromid (189 mmol, 1 Äquivalent) versetzt und bei RT über Nacht gerührt. Das Reaktionsgemisch wurde in Wasser/1N wässrige Salzsäure eingerührt. Der Feststoff wurde abfiltriert, mit Wasser nachgewaschen und an der Luft getrocknet. Es wurden 54.9 g (97% d. Th.) der Titelverbindung erhalten.
¹H-NMR (400 MHz, DMSO-d₆): δ = 5.46 (s, 2 H); 7.22 (t, 2 H); 7.58 (q, 1 H); 8.28 (d, 1 H); 8.47 (d, 1 H).

### Beispiel 14A

### 5-Chlor-3-[(2,6-difluorbenzyl)oxy]pyridin-2-amin

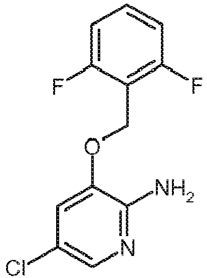

59.7 g 5-Chlor-3-[(2,6-difluoibenzyl)oxy]-2-nitropyridin (Beispiel 13A; 199 mmol, 1 Äquivalent) wurden in 600 ml Ethanol vorgelegt, mit 34.4 g Eisenpulver (616 mmol, 3.1 Äquivalente) versetzt und zum Rückfluß erhitzt. Es wurden langsam 152 ml konzentrierte Salzsäure zugetropft und weitere 30 min am Rückfluß gekocht. Das Reaktionsgemisch wurde abgekühlt und in ein Eis-Wassergemisch eingerührt. Das resultierende Gemisch wurde mit Natriumacetat auf pH 5 eingestellt. Der Feststoff wurde abfiltriert, mit Wasser nachgewaschen und an der Luft und anschließend im Vakuum bei 50°C getrocknet. Es wurden 52.7 g (98% d. Th.) der Titelverbindung erhalten.
LC-MS (Methode 2): Rₜ = 0.93 min
MS (ESpos): m/z = 271.1/273.1 (M+H)⁺
¹H-NMR (400 MHz, DMSO-d₆): δ = 5.14 (s, 2 H); 5.82 (br. s, 2 H); 7.20 (t, 2 H); 7.35 (d, 1 H); 7.55 (q, 1 H); 7.56 (d, 1 H).

### Beispiel 15A

### Ethyl-6-chlor-8-[(2,6-difluorbenzyl)oxy]-2-methylimidazo[1,2-a]pyridin-3-carboxylat

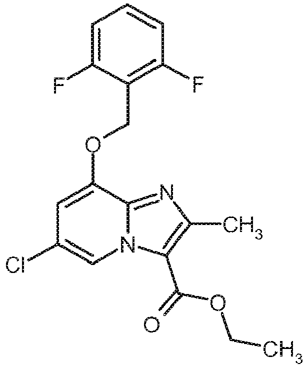

40 g 5-Chlor-3-[(2,6-difluorbenzyl)oxy]pyridin-2-amin (Beispiel 14A; 147.8 mmol; 1 Äquivalent) wurden in 800 ml Ethanol vorgelegt, mit 30 g gepulvertem Molekularsieb 3Å und 128 g Ethyl-2-chloracetoacetat (739 mmol, 5 Äquivalente) versetzt und über Nacht zum Rückfluß erhitzt. Das Reaktionsgemisch wurde eingeengt, der Rückstand in Essigsäureethylester aufgenommen und filtriert. Die Essigsäureethylester-Phase wurde mit Wasser gewaschen, getrocknet, filtriert und eingeengt. Es wurden 44 g (78% d. Th.) der Titelverbindung erhalten.
LC-MS (Methode 2): Rₜ = 1.27 min
MS (ESpos): m/z = 381.2/383.2 (M+H)⁺
¹H-NMR (400 MHz, DMSO-d₆): δ = 1.36 (t, 3 H); 2.54 (s, 3 H; verdeckt durch DMSO-Signal); 4.37 (q, 2 H); 5.36 (s, 2 H); 7.26 (t, 2 H); 7.38 (d, 1 H); 7.62 (q, 1 H); 8.92 (d, 1 H).

### Beispiel 16A

### 6-Chlor-8-[(2,6-difluorbenzyl)oxy]-2-methylimidazo[1,2-a]pyhdin-3-carbonsäure

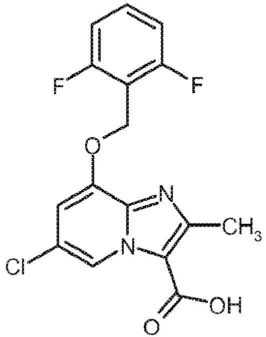

44 g Ethyl-6-chlor-8-[(2,6-difluorbenzyl)oxy]-2-methylimidazo[1,2-a]pyridin-3-carboxylat (Beispiel 15A; 115 mmol, 1 Äquivalent) wurden in 550 ml THF und 700 ml Methanol gelöst, mit 13.8 g Lithiumhydroxid (gelöst in 150 ml Wasser; 577 mmol, 5 Äquivalente) versetzt und über Nacht bei RT gerührt. Es wurde mit 1 N wässriger Salzsäure versetzt und im Vakuum eingeengt. Der erhaltene Feststoff wurde abfiltriert und mit Wasser nachgewaschen. Es wurden 34 g der Titelverbindung (84% d. Th.) erhalten.
LC-MS (Methode 1): Rₜ = 1.03 min
MS (ESpos): m/z = 353.0/355.0 (M+H)⁺
¹H-NMR (400 MHz, DMSO-d₆): δ = 2.54 (s, 3 H; überlagert von DMSO-Signal); 5.36 (s, 2 H); 7.26 (t, 2 H); 7.34 (d, 1 H); 7.61 (q, 1 H); 8.99 (d, 1 H); 13.36 (br. s, 1 H).

### Beispiel 17A

### 5-Brom-3-[(2,6-difluorbenzyl)oxy]pyridin-2-amin

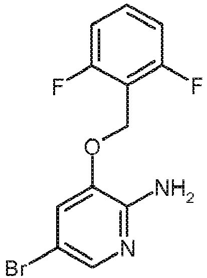

32.6 g 3-[(2,6-Difluorbenzyl)oxy]pyridin-2-amin (Beispiel 1A; 138 mmol, 1 Äquivalent) wurden in 552 ml 10%iger Schwefelsäure suspendiert und auf 0°C gekühlt. 8.5 ml Brom (165 mmol, 1.2 Äquivalente) wurde in 85 ml Essigsäure gelöst und dann innerhalb von 90 min zur eisgekühlten, Reaktionslösung getropft. Nach erfolgter Zugabe wurde 90 min bei 0°C nachgerührt, anschließend mit 600 ml Essigsäureethylester verdünnt und die wässrige Phase abgetrennt. Die wässrige Phase wurde mit Essigsäureethylester extrahiert. Die organischen Phasen wurden vereinigt, mit gesättigter, wässriger Natriumhydrogencarbonat-Lösung gewaschen, getrocknet und eingeengt. Der Rückstand wurde in Dichlormethan gelöst und an Kieselgel chromatographiert (Petrolether/Essigsäureethylester Gradient als Eluent). Es wurden 24 g (55% d. Th.) der Titelverbindung erhalten.
LC-MS (Methode 2): Rₜ = 0.96 min
MS (ESpos): m/z = 315.1/317.1 (M+H)⁺
¹H-NMR (400 MHz, DMSO-d₆): δ = 5.14 (s, 2 H); 5.83 (br. s, 2 H); 7.20 (t, 2 H); 7.42 (d, 1 H); 7.54 (q, 1 H); 7.62 (d, 1 H).

### Beispiel 18A

### Ethyl-6-brom-8-[(2,6-difluorbenzyl)oxy]-2-methylimidazo[1,2-a]pyridin-3-carboxylat

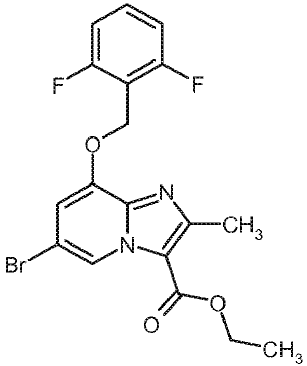

24 g 5-Brom-3-[(2,6-difluorbenzyl)oxy]pyridin-2-amin (Beispiel 17A; 76.2 mmol; 1 Äquivalent) in 400 ml Ethanol wurden mit 16 g gepulvertem Molekularsieb 3Å und 52.7 ml Ethyl-2-chloracetoacetat (380.8 mmol; 5 Äquivalente) versetzt und über Nacht zum Rückfluß erhitzt. Es wurden weitere 8 g Molekularsieb zugegeben und für weitere 24 h zum Rückfluß erhitzt. Das Reaktionsgemisch wurde im Vakuum eingeengt, der Rückstand in Dichlormethan aufgenommen und an Kieselgel chromatographiert (Dichlormethan/Methanol 20:1 als Eluent). Die produkthaltigen Fraktionen wurden eingeengt, der Rückstand mit 100 ml Diethylether 30 min verrührt. Dann wurde abfiltriert, mit wenig Diethylether gewaschen und getrocknet. Es wurden 15 g (45% d. Th.) der Titelverbindung erhalten.
LC-MS (Methode 1): Rₜ = 1.43 min
MS (ESpos): m/z = 414.9/416.8 (M+H)⁺
¹H-NMR (400 MHz, DMSO-d₆): δ = 1.36 (t, 3 H); 2.54 (s, 3 H; verdeckt durch DMSO-Signal); 4.37 (q, 2 H); 5.36 (s, 2 H); 7.25 (t, 2 H); 7.42 (d, 1 H); 7.61 (q, 1 H); 9.00 (d, 1 H).

### Beispiel 19A

### 6-Brom-8-[(2,6-difluorbenzyl)oxy]-2-methylimidazo[1,2-a]pyridin-3-carbonsäure

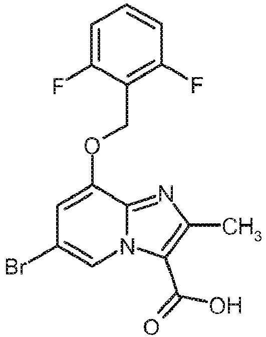

1.5 g Ethyl-6-brom-8-[(2,6-difluorbenzyl)oxy]-2-methylimidazo[1,2-a]pyridin-3-carboxylat (Beispiel 18A; 3.5 mmol, 1 Äquivalent) wurden in 72 ml THF/Methanol 5:1 gelöst, mit 17.6 ml 1N wässriger Lithiumhydroxid-Lösung (17.6 mmol, 5 Äquivalente) versetzt, auf 40°C erwärmt und für 6 h bei dieser Temperatur gerührt. Dann wurde mit 6 N wässriger Salzsäure auf pH 4 gestellt und im Vakuum eingeengt. Der enstandene Feststoff wurde mit Wasser versetzt, ausgerührt, abfiltriert, mit Wasser nachgewaschen und im Vakuum getrocknet. Es wurden 1.24 g der Titelverbindung (88% d. Th.) erhalten.
LC-MS (Methode 2): Rₜ = 0.93 min
MS (ESpos): m/z = 397.0/399.1 (M+H)⁺
¹H-NMR (400 MHz, DMSO-d₆): δ = 2.54 (s, 3 H; überlagert von DMSO-Signal); 5.36 (s, 2 H); 7.25 (t, 2 H); 7.40 (d, 1 H); 7.61 (q, 1 H); 9.06 (d, 1 H); 13.35 (br. s, 1 H).

### Beispiel 20A

### Ethyl-8-[(2,6difluorbenzyl)oxy]-2,6dimethylimidazo[1,2-a]pyridin-3-carboxylat

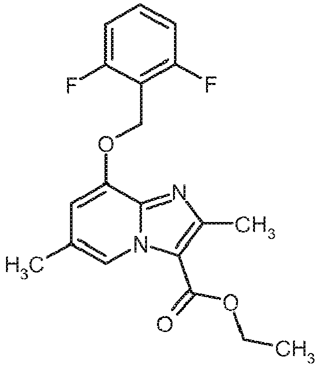

20.00 g (85.38 mmol) Ethyl-8-hydroxy-2,6-dimethylimidazo[1,2-a]pyridin-3-carboxylat aus Beispiel 239A, 19.44 g (93.91 mmol) 2,6-Difluorbenzylbromid und 61.20 g (187.83 mmol) Cäsiumcarbonat in 1.18 L DMF wurden 5 h bei 60°C gerührt. Dann wurde das Reaktionsgemisch auf 6.4 L 10%ige wässrige Natriumchlorid-Lösung gegossen und anschließend zweimal mit Essigsäureethylester extrahiert. Die vereinigten organischen Phasen wurden mit 854 ml 10%iger wässriger Natriumchlorid-Lösung gewaschen, getrocknet, eingeengt und über Nacht im Hochvakuum bei RT getrocknet. Es wurden 28.2 g (92% d. Th.; Reinheit ca. 90%) der Titelverbindung erhalten.
LC-MS (Methode 2): Rₜ = 1.05 min
MS (ESpos): m/z = 361.1 (M+H)⁺
¹H-NMR (400 MHz, DMSO-d₆): δ = 1.38 (t, 3 H); 2.36 (s, 3 H); 4.35 (q, 2 H); 5.30 (s, 2 H); 7.10 (s, 1 H); 7.23 (t, 2 H); 7.59 (q, 1 H); 8.70 (s, 1 H).

### Beispiel 21A

### 8-[(2,6-Difluorbenzyl)oxy]-2,6-dimethylimidazo[1,2,a]pyridin-3-carbonsäure

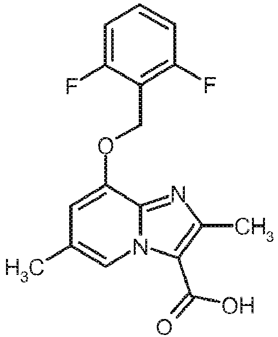

220 mg Ethyl-8-[(2,6-difluorbenzyl)oxy]-2,6-methylimidazo[1,2,a]pyridin-3-carboxylat (Beispiel 20A; 0.524 mmol, 1 Äquivalent) wurden in 7 ml THF/Methanol 1:1 gelöst, mit 2.6 ml 1 N wässriger Lithiumhydroxid-Lösung (2.6 mmol, 5 Äquivalente) versetzt und für 16 h bei RT gerührt. Es wurde im Vakuum eingeengt und der Rückstand mit 1N wässriger Salzsäure sauer gestellt und 15 min gerührt. Der Feststoff wurde abfiltriert, mit Wasser nachgewaschen und im Vakuum getrocknet. Es wurden 120 mg der Titelverbindung (60% d. Th.) erhalten.
LC-MS (Methode 2): Rₜ = 0.68 min
MS (ESpos): m/z = 333.1 (M+H)⁺
¹H-NMR (400 MHz, DMSO-d₆): δ = 2.34 (s, 3 H); 5.28 (s, 2 H); 7.09 (s, 1 H); 7.23 (t, 2 H); 7.58 (q, 1 H); 8.76 (s, 1 H); 13.1 (br. s, 1 H), [weiteres Signal unter DMSO-Signal verborgen].

### Beispiel 22A

### 8-[(2,6-Difluorbenzyl)oxy]-2-methylimidazo[1,2-a]pyridin-3-carbonylchlorid-Hydrochlorid

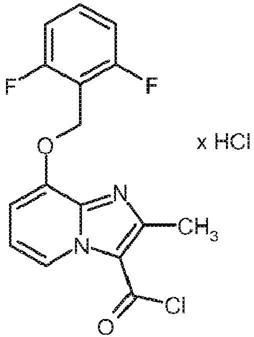

Zu 2.0 g 8-[(2,6-Difluorbenzyl)oxy]-2-methylimidazo[1,2-a]pyridin-3-carbonsäure (6.28 mmol, 1 Äquivalent) in 25 ml trockenem THF wurden 4 Tropfen DMF und anschließend 3.19 g Oxalsäuredichlorid (25.14 mmol, 4 Äquivalente) getropft. Das Reaktionsgemisch wurde 3 h bei RT gerührt. Es wurden nochmals 0.80 g Oxalsäuredichlorid (6.28 mmol, 1 Äquivalent) zugegeben und die Reaktion wurde weitere 4 h bei RT gerührt. Das Reaktionsgemisch wurde eingeengt, dreimal mit Toluol abgedampft und der Rückstand wurde im Hochvakuum getrocknet. Es wurden 2.43 g der Titelverbindung (103% d. Th.) erhalten.
DCI-MS (Methode 4): MS (ESpos): m/z = 437 (M-HCl+H)⁺

### Beispiel 23A

### Ethyl-2-chlor-3-cyclopropyl-3-oxopropanoat

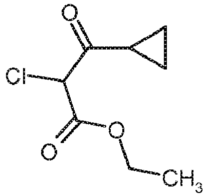

3.1 ml Sulfurylchlorid (38.2 mmol, 1.05 Äquivalente) wurden in 21 ml Dichlormethan vorgelegt und auf dem Wasserbad tropfenweise mit 5.68 g Ethyl-3-cyclopropyl-3-oxopropanoat (36.4 mmol) versetzt. Die Reaktionsmischung wurde 2 h bei RT gerührt. Anschließend wurde mit Wasser, 5%-iger, wässriger Natriumhydrogencarbonat-Lösung und gesättigter, wässriger Natriumchlorid-Lösung gewaschen, über Magnesiumsulfat getrocknet und eingeengt. Das Rohprodukt (6.8 g) wurde ohne weitere Aufreinigung in die Folgeumsetzung eingesetzt.

### Beispiel 24A

### Ethyl-2-cyclopropyl-8-[(2,6-difluorbenzyl)oxy]imidazo[1,2-a]pyridin-3-carboxylat

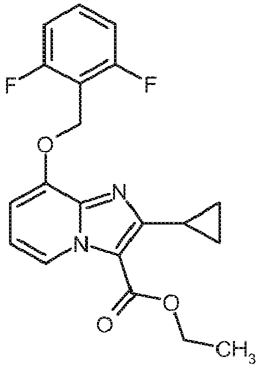

1.69 g 3-[(2,6-Difluorbenzyl)oxy]pyridin-2-amin (Beispiel 1A; 7.13 mmol, 1 Äquivalent) wurden in 44.4 ml Ethanol vorgelegt, mit 425 mg gepulvertem Molekularsieb 3Å und 6.8 g Ethyl-2-chlor-3-cyclopropyl-3-oxopropanoat (Rohprodukt aus Beispiel 23A) versetzt. Die Reaktionsmischung wurde 48 h zum Rückfluß erhitzt, anschließend im Vakuum eingeengt und der Rückstand chromatographiert (Cyclohexan/Essigsäureethylester als Eluens). Die produkthaltigen Fraktionen wurden vereinigt und im Vakuum eingeengt. Der so erhaltene Rückstand wurde in Methanol, DMSO und Wasser aufgenommen. Der erhaltene Feststoff wurde abfiltiert und im Hochvakuum getrocknet. Es wurden 410 mg (15.4% d. Th.) Titelverbindung erhalten.
LC-MS (Methode 2): Rₜ = 1.22 min
MS (ESpos): m/z = 373.2 (M+H)⁺
¹H-NMR (400 MHz, DMSO-d₆): δ = 0.95 -1.05 (m, 4 H); 1.39 (t, 3 H); 2.36 (s, 3 H); 2.70 - 2.80 (m, 1 H); 4.39 (q, 2 H); 5.30 (s, 2 H); 7.08 (t, 1 H); 7.15 (d, 1 H); 7.20 (t, 2 H); 7.59 (q, 1 H); 8.88 (d, 1 H).

### Beispiel 25A

### 2-Cyclopropyl-8-[(2,6-difluorbenzyl)oxy]imidazo[1,2-a]pyridin-3-carbonsäure

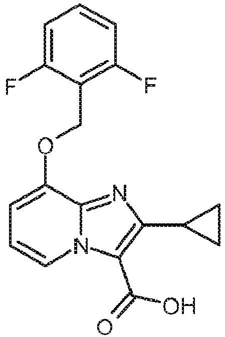

410 mg Ethyl-2-cyclopropyl-8-[(2,6difluorbenzyl)oxy]imidazo[1,2-a]pyridin-3-carboxylat (Beispiel 24A, 1.1 mmol, 1 Äquivalent) wurden in 15 ml Methanol/THF (1:1) vorgelegt und mit 5.5 ml einer 1 N wässrigen Lithiumhydroxid-Lösung (5.5 mmol, 5 Äquivalente) versetzt. Die Reaktionsmischung wurde über Nacht bei RT gerührt. Es wurden erneut 5.5 ml 1 N wässrige Lithiumhydroxid-Lösung zugegeben und es wurde eine weitere Nacht bei RT gerührt. Dann wurde im Vakuum eingeengt, der Rückstand in Wasser aufgenommen und mit 1 N wässriger Salzsäure sauer gestellt. Das ausgefallene Produkt wurde abfiltriert und am Hochvakuum getrocknet. Es wurden 293 mg (77% d. Th.) der Titelverbindung erhalten.
LC-MS (Methode 2): Rₜ = 0.83 min
MS (ESpos): m/z = 345.2 (M+H)⁺
¹H-NMR (400 MHz, DMSO-d₆): δ = 0.95 - 1.02 (m, 4 H); 2.80 (q, 1 H); 5.30 (s, 2 H); 7.02 (t, 1 H); 7.15 (d, 1 H); 7.22(t, 2 H); 7.59 (q, 1 H); 8.92 (s, 1 H); 13.3 (br. s, 1 H).

### Beispiel 26A

### Ethyl-2-chlor-3-oxopropanoat

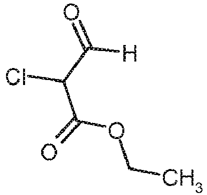

139 ml einer 21%igen Natriumethylat-Lösung in Ethanol (371 mmol, 0.91 Äquivalente) wurden in 200 ml Diethylether vorgelegt und bei RT mit einer Lösung aus 43.7 ml Chloressigsäureethylester (408 mmol, 1 Äquivalent) und 32.9 ml Ameisensäureethylester (408 mmol, 1 Äquivalent) in 150 ml Diethylether tropfenweise versetzt. Die Reaktionsmischung wurde über Nacht gerührt, der entstandene Feststoff abfiltriert und mit Diethylether nachgewaschen. Der Feststoff wurde in Wasser gelöst und die wässrige Phase unter Eisbadkühlung mit konzentrierter Salzsäure auf pH 4 eingestellt. Es wurde mehrfach mit Diethylether extrahiert, die vereinigten organischen Phasen wurden mit gesättigter, wässriger Natriumchlorid-Lösung gewaschen, mit Magnesiumsulfat getrocknet, filtriert und eingeengt. Das erhaltene Rohprodukt (8.2 g) wurde am Hochvakuum von Lösungsmittelresten befreit und ohne weitere Aufreinigung in die Folgereaktion eingesetzt.

### Beispiel 27A

### Ethyl-8-[(2,6-difluorbenzyl)oxy]imidazo[1,2-a]pyridin-3-carboxylat

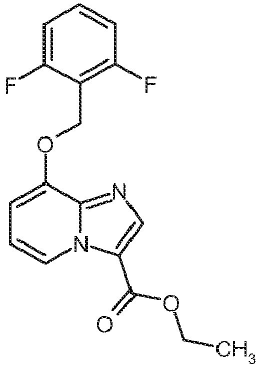

1.93 g 3-[(2,6-difluorbenzyl)oxy]pyridin-2-amin (Beispiel 1A; 8.2 mmol, 1 Äquivalent) wurden in 50 ml Ethanol vorgelegt und mit 8.2 g Ethyl-2-chlor-3-oxopropanoat (75% Reinheit, Rohprodukt aus Beispiel 26A, 40.8 mmol, 5 Äquivalente) versetzt. Die Reaktionsmischung wurde über Nacht am Rückfluß erhitzt. Dann wurde im Vakuum eingeengt und das erhaltene Rohprodukt über 340 g Kieselgel (Biotage Isolera) chromatographiert (Laufmittel: Cyclohexan:Essigsäureethylester Gradient; R_{f}-Wert Produkt in Cyclohexan:Essigsäureethylester 2:1 = 0.36). Die Produktfraktionen wurden vereinigt, eingeengt und der erhaltene Rückstand mit Diisopropylether ausgerührt. Der Feststoff wurde abfiltriert und im Hochvakuum getrocknet. Es wurden 2.02 g der Titelverbindung (71 % d. Th.) erhalten
LC-MS (Methode 2): Rₜ = 1.08 min
MS (ESpos): m/z = 333.1 (M+H)⁺
¹H-NMR (400 MHz, DMSO-d₆): δ = 1.35 (t, 3 H); 4.39 (q, 2 H); 5.35 (s, 2 H); 7.15 - 7.28 (m, 4 H); 7.58 (q, 1 H); 8.18 (s, 1 H); 8.90 (d, 1 H).

### Beispiel 28A

### 8-[(2,6-Difluorbenzyl)oxy]imidazo[1,2-a]pyridin-3-carbonsäure

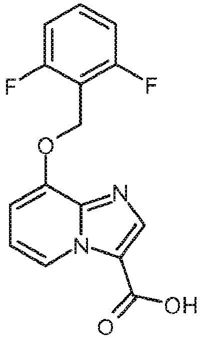

1 g Ethyl-8-[(2,6-difluorbenzyl)oxy]imidazo[1,2-a]pyridin-3-carboxylat (Beispiel 27A, 3 mmol, 1 Äquivalent) wurden in 60 ml Methanol/THF (5:1) vorgelegt, mit 15 ml einer 1 N wässrigen Lithiumhydroxid-Lösung (15 mmol, 5 Äquivalente) versetzt, auf 40 °C erwärmt und für 4 h bei dieser Temperatur gerührt. Dann wurde abgekühlt und unter Eiskühlung mit 6 N wässriger Salzsäure auf pH 4 eingestellt. Die organischen Lösungsmittel wurden am Rotationsverdampfer entfernt, das ausgefallende Produkt wurde mit Wasser versetzt, filtriert, mit Wasser nachgewaschen und im Hochvakuum getrocknet. Es wurden 797 mg (87% d. Th.) der Titelverbindung erhalten.
LC-MS (Methode 2): Rₜ = 0.66 min
MS (ESpos): m/z = 305.1 (M+H)⁺
¹H-NMR (400 MHz, DMSO-d6): δ = 5.38 (s, 2 H); 7.10 - 7.28 (m, 4 H); 7.59 (q, 1 H); 8.12 (s, 1 H); 8.92 (s, 1 H); 13.1 (br. s, 1 H).

### Beispiel 29A

### Ethyl-8-(benzyloxy)-2-methylimidazo[1,2-a]pyridin-3-carboxylat

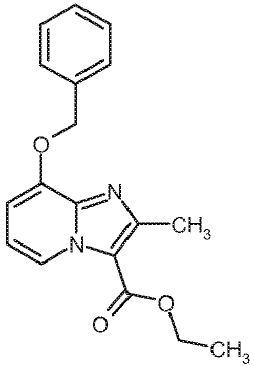

25 g 2-Amino-3-benzyloxypyridin (124.8 mmol, 1 Äquivalent) wurde in 781 ml Ethanol gelöst, mit 102.7 g Ethyl-2-chloracetoacetat (624.2 mmol, 5 Äquivalente) und mit 15 g 4Å Molekularsieb versetzt. Es wurde 2 d zum Rückfluß erhitzt (Badtemperatur 100°C). Dann wurde eingeengt und am Rotationsverdampfer mit Trockeneiskühlung das überschüssige Ethyl-2-chloracetoacetat abdestilliert. Der Rückstand wurde über eine Kieselgelchromatographie gereinigt (Laufmittel Cyclohexan:Essigsäureethylester 9:1, 4:1). Es wurden 20.81 g der Titelverbindung (54% d. Th.) erhalten.
LC-MS (Methode 1): Rₜ = 1.12 min
MS (ESpos): m/z = 311 (M+H)⁺
¹H-NMR (400 MHz, DMSO-d₆): δ = 1.35 (t, 3H), 2.59 (s, 3H), 4.34 (q, 2H), 5.32 (s, 2H), 7.01-7.09 (m, 2H), 7.33-7.48 (m, 3H), 7.52 (d, 2H), 8.81-8.86 (m, 1H).

### Beispiel 30A

### 8-(Benzyloxy)-2-methylimidazo[1,2-a]pyridin-3-carbonsäure

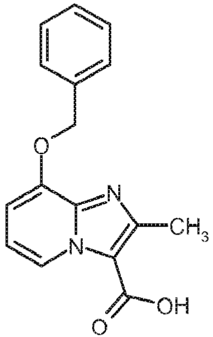

Eine Lösung von 15.7 g Ethyl-8-(benzyloxy)-2-methylimidazo[1,2-a]pyridin-3-carboxylat (50.59 mmol) in 253 ml 1,4-Dioxan wurde mit 253 ml 2 N wässriger Natronlauge versetzt und 14 h bei RT gerührt. Dann wurde mit 101 ml 6 N wässriger Salzsäure versetzt. Der enstandene Feststoff wurde abfiltriert, mit Wasser und Methyl-tert.-butylether nachgewaschen und dann über Nacht im Vakuum bei 40°C getrocknet. Man erhielt 15.49 g (108% d. Th.) 8-(Benzyloxy)-2-methylimidazo[1,2,a]pyridin-3-carbonsäure.
LC-MS (Methode 1): Rₜ = 0.66 min
MS (ESpos): m/z = 283.0 (M+H)
¹H-NMR (400 MHz, DMSO-d₆): δ = 2.67 (s, 3H), 3.2 - 3.8 (sehr breiter Wasserpeak), 5.41 (s, 2H), 7.30 (m, 1H), 7.35 - 7.48 (m, 4H), 7.57 (d, 2H), 9.02 (d, 1H).

### Beispiel 31A

### rac-2-Amino-4,4,4-trifluorbutan-1-ol

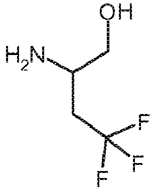

0.32 ml Lithiumborhydrid (2 M in THF, 0.65 mmol, 2.5 Äquivalente) wurden in 0.5 ml abs. THF vorgelegt, mit 0.16 ml Chlortrimethylsilan (1.28 mmol, 5 Äquivalente) bei RT versetzt und 5 min bei RT gerührt. Anschließend wurden 50 mg 2-Amino-4,4,4-trifluorbutansäurehydrochlorid (0.26 mmol, 1 Äquivalent) portionsweise zugegeben und das Reaktionsgemisch wurde über Nacht bei RT gerührt. Es wurden 0.5 ml Methanol zugegeben und das Gemisch anschließend eingeengt. Der Rückstand wurde mit 0.6 ml einer 20%igen Kaliumhydroxid-Lösung versetzt und dreimal mit Dichlormethan extrahiert. Die vereinigten organischen Phasen wurden über Natrimsulfat getrocknet, filtriert und eingeengt. Es wurden 33 mg der Titelverbindung (88% d. Th.) erhalten.
DCI-MS (Methode 4): MS (ESpos): m/z = 144 (M+H)+
¹H-NMR (400 MHz, DMSO-d₆): δ = 2.08-2.20 (m, 1H), 2.22-2.38 (m, 1H), 3.25-3.32 (m, 1H), 3.39-3.44 (m, 1H), 3.59-3.65 (m, 1H).

In Analogie zu Beispiel 31A wurden die in Tabelle 1A gezeigten Beispiele hergestellt, indem Lithiumborhydrid (1.7-2.5 Äquivalente) und Chlortrimethylsilan (3.4-5 Äquivalente) mit den entsprechenden, kommerziell erhältlichen Aminosäuren nach der allgemeinen Arbeitsvorschrift 1 umgesetzt wurden:

**Tabelle 1A:**

| **Beispiel** | **IUPAC-Name / Struktur (Ausbeute)** | **Analytische Daten** |
|---|---|---|
| **32A** | *rac*-2-Amino-6,6,6-trifluorhexan-1-ol | DCI-MS (Methode 4): |
| | 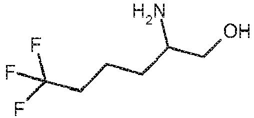 | MS (ESpos): m/z = 172 (M+H)⁺ |
| | (75% d. Th.) | |
| **33A** | *rac*-2-Amino-5,5,5-trifluor-4-(trifluormethyl)pentan-1-ol-hydrochlorid | DCI-MS (Methode 4): |
| | 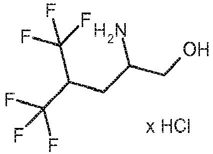 | MS (ESpos): m/z = 226 (M+H)⁺ |
| | (55% d. Th.) | |

### Beispiel 34A

### rac-8-[(2,6-Difluorbenzyl)oxy]-2-methyl-N-(4,4,4-trifluor-1-hydroxybutan-2-yl)imidazo[1,2-a]pyridin-3-carboxamid

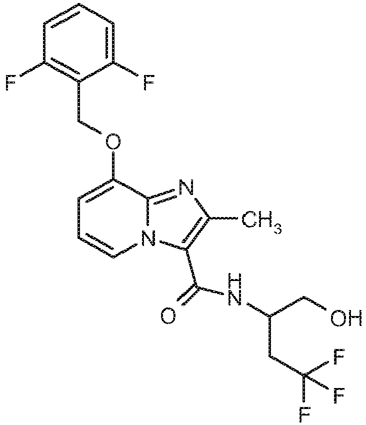

330 mg 8-[(2,6-Difluorbenzyl)oxy]-2-methylimidazo[1,2-a]pyridin-3-carbonsäure (1.04 mmol), 399 mg (Benzotriazol-1-yloxy)bisdimethylaminomethyliumfluorborat (TBTU, 1.24 mmol) und 524 mg 4-Methylmorpholin (5.18 mmol) wurden in 6.6 ml DMF vorgelegt. Nach 10 min bei RT wurden 371 mg (1.56 mmol, Reinheit ca. 60%) 2-Amino-4,4,4-trifluorbutan-1-ol (Beispiel 31A) zugesetzt und über Nacht bei RT gerührt. Die Reaktionslösung wurde mit ca. 200 ml Wasser versetzt, noch 30 min gerührt, der entstandene Niederschlag abfiltriert, mit Wasser gewaschen und über Nacht im Hochvakuum getrocknet. Es wurden 439 mg der Titelverbindung (96% d. Th.) erhalten.
LC-MS (Methode 5): Rₜ = 1.62 min
MS (ESpos): m/z = 444 (M+H)⁺
¹H-NMR (400 MHz, DMSO-d₆): δ = 2.50 (s, 3H), 2.55-2.72 (m, 2H), 3.38-3.47 (m, 1H), 3.51-3.62 (m, 1H), 4.29-4.40 (m, 1H), 5.12 (t, 1H), 5.30 (s, 2H), 6.92 (t, 1H), 7.02 (d, 1H), 7.23 (t, 2H), 7.59 (quint, 1H), 7.80 (d, 1H), 8.56 (d, 1H).

In Analogie zu Beispiel 34A wurden die in Tabelle 2A gezeigten Beispiele hergestellt, indem 8-[(2,6-Difluorbenzyl)oxy]-2-methylimidazo[1,2-a]pyridin-3-carbonsäure mit den entsprechenden, kommerziell erhältlichen Aminen unter den in der allgemeinen Arbeitsvorschrift 2 beschriebenen Reaktionsbedingungen umgesetzt wurde:

**Tabelle 2A:**

| **Beispiel** | **IUPAC-Name / Struktur (Ausbeute)** | **Analytische Daten** |
|---|---|---|
| **35A** | *rac*-8-[(2,6-Difluorbenzyl)oxy]-2-methyl-*N-*(6,6,6-trifluor-1-hydroxyhexan-2-yl)imidazo[1,2-a]pyridin-3-carboxamid | LC-MS (Methode 2): Rₜ = 0.88 min |
| | | MS (ESpos): m/z = 472 (M+H)⁺ |
| | 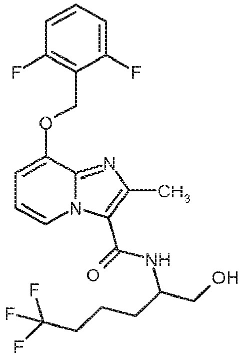 | ¹H-NMR (400 MHz, DMSO-d₆): δ = 1.51-1.79 (m, 4H), 2.19-2.40 (m, 2H), 2.50 (s, 3H), 3.41-3.57 (m, 2H), 3.96-4.08 (m, 1H), 4.82 (t, 1H), 5.30 (s, 2H), 6.91 (t, 1H), 6.99 (d, 1H), 7.22 (t, 2H), 7.56-7.62 (m, 2H), 8.52 (d, 1H). |
| | (76% d. Th.) | |
| **36A** | *rac*-8-[(2,6-difluorbenzyl)oxy]-2-methyl-*N-*[5,5,5-trifluor-1-hydroxy-4-(trifluormethyl)pentan-2-yl]imidazo[1,2-a]pyridin-3-carboxamid-Trifluoracetat ^{a)} | LC-MS (Methode 2): Rₜ = 0.90 min |
| | | MS (ESpos): m/z = 526 (M-TFA+H)⁺ |
| | 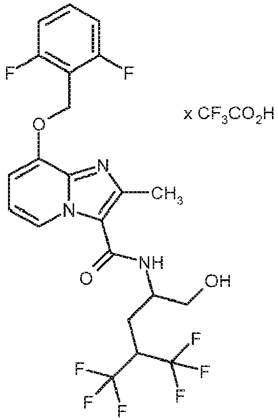 | |
| | (52% d. Th.) | |

| | | |
|---|---|---|
| a) Alternative Aufarbeitung: Das Reaktionsrohgemisch wurde direkt per präparativer HPLC (RP18 Säule, Laufmittel: Acetonitril/Wasser-Gradient unter Zusatz von 0.1 % TFA) aufgereinigt. | | |

### Beispiel 37A

### rac-N-(1-Chlor-4,4,4-trifluorbutan-2-yl)-8-[(2,6-difluorbenzyl)oxy]-2-methylimidazo[1,2-a]pyridin-3-carboxamid-Hydrochlorid

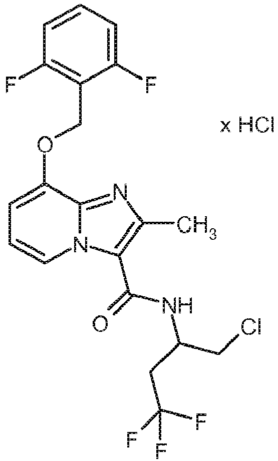

2.48 g *rac*-8-[(2,6-Difluorbenzyl)oxy]-2-methyl-N-(4,4,4-trifluor-1-hydroxybutan-2-yl)imidazo-[1,2-a]pyridin-3-carboxamid (Beispiel 34A, 5.59 mmol) wurden in Dichlormethan vorgelegt. Bei 0°C wurden 1.22 ml Thionylchlorid (16.77 mmol) zugetropft und es wurde über Nacht bei RT gerührt. Die Reaktionslösung wurde eingeengt und im Hochvakuum getrocknet. Es wurden 2.78 g der Titelverbindung (99.8% d. Th.) erhalten.
LC-MS (Methode 2): Rₜ = 0.94 min
MS (ESpos): m/z = 462 (M-HCl+H)⁺
¹H-NMR (400 MHz, DMSO-d₆): δ = 2.60 (s, 3H), 2.70-2.84 (m, 2H), 3.82-3.92 (m, 2H), 4.55-4.67 (m, 1H), 5.43 (s, 2H), 7.23 (t, 2H), 7.31-7.43 (m, 1H), 7.51-7.66 (m, 2H), 8.63 (d, 1H), 8.82 (br s, 1H).

In Analogie zu Beispiel 37A wurden die in Tabelle 3A gezeigten Beispiele hergestellt.

**Tabelle 3A:**

| **Beispiel** | **IUPAC-Name / Struktur (Ausbeute)** | **Analytische Daten** |
|---|---|---|
| **38A** | *rac*-*N*-(1-Chlor-6,6,6-trifluorhexan-2-yl)-8-[(2,6-difluorbenzyl)oxy]-2-methylimidazo[1,2-a]pyridin-3-carboxamid-Hydrochlorid | LC-MS (Methode 2): Rₜ = 1.05 min |
| | | MS (ESpos): m/z = 490 (M-HCl+H)⁺ |
| | 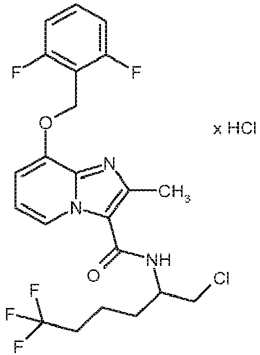 | ¹H-NMR (400 MHz, DMSO-d₆): δ = 1.52-1.81 (m, 4H), 2.20-2.42 (m, 2H), 2.60 (s, 3H), 3.72-3.87 (m, 2H), 4.21-4.33 (m, 1H), 5.45 (s, 2H), 7.23 (t, 2H), 7.40 (br s, 1H), 7.52-7.68 (m, 2H), 8.54 (br s, 1H), 8.61 (d, 1H). |
| | (99% d. Th.) | |
| **39A** | *rac-N-*[1-Chlor-5,5,5-trifluor-4-(trifluormethyl)pentan-2-yl]-8-[(2,6-difluorbenzyl)oxy]-2-methylimidazo[1,2-a]pyridin-3-carboxamid-Hydrochlorid | LC-MS (Methode 2): Rₜ = 1.15 min |
| | | MS (ESpos): m/z = 544 (M-HCl+H)⁺ |
| | 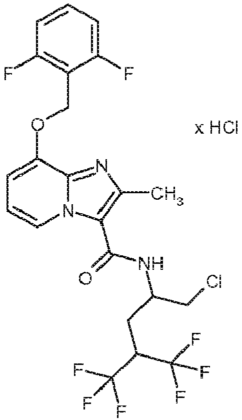 | |
| | (98% d. Th.) | |
| **40A** | *N*-[(2*R*)-1-Chlorhexan-2-yl]-8-[(2,6-difluor-benzyl)oxy]-2-methylimidazo[1,2-a]pyridin-3-carboxamidhydrochlorid | LC-MS (Methode 2): Rₜ = 1.06 min |
| | | MS (ESpos): m/z = 436 (M-HCl+H)⁺ |
| | 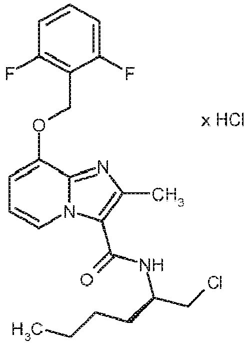 | |
| | (98% d. Th.) | |

### Beisniel 41A

### rac-N-(1-Azido-4,4,4-trifluorbutan-2-yl)-8-[(2,6-difluorbenzyl)oxy]-2-methylimidazo[1,2-a]pyridin-3-carboxamid

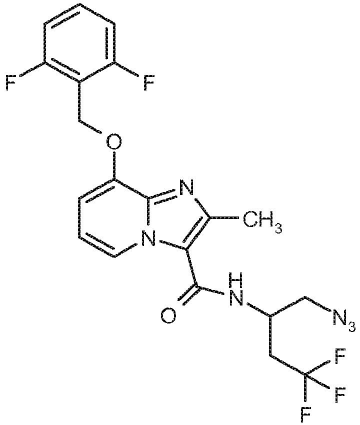

195 mg *rac*-N-(1-Chlor-4,4,4-trifluorbutan-2-yl)-8-[(2,6-difluorbenzyl)oxy]-2-methylimidazo[1,2-a]pyridin-3-carboxamidhydrochlorid (Beispiel 37A, 0.39 mmol) wurden in 3.4 ml DMF vorgelegt, mit 254 mg Natriumazid (3.91 mmol) versetzt und 4 h bei 40°C gerührt. Anschließend wurde 5 h bei 60°C gerührt. Das Reaktionsgemisch wurde mit Wasser versetzt und dreimal mit Essigsäureethylester extrahiert. Die vereinigten organischen Phasen wurden über Natriumsulfat getrocknet, filtriert, das Filtrat eingeengt und der Rückstand mittels Kieselgel-Chromatographie gereinigt (Laufmittel: Cyclohexan:Essigsäureethylester 7:3, isokratisch). Es wurden 50 mg der Titelverbindung (27% d. Th.) erhalten.
LC-MS (Methode 2): Rₜ = 0.97 min
MS (ESpos): m/z = 469 (M+H)⁺
¹H-NMR (400 MHz, DMSO-d₆): δ = 2.50 (s, 3H), 2.58-2.78 (m, 2H), 3.52-3.63 (m, 2H), 4.47-4.58 (m, 1H), 5.30 (s, 2H), 6.93 (t, 1H), 7.02 (d, 1H), 7.22 (t, 2H), 7.59 (quint, 1H), 8.09 (d, 1H), 8.55 (d, 1H).

In Analogie zu Beispiel 41A wurden die in Tabelle 4A gezeigten Beispiele hergestellt, indem Natriumazid (5-20 Äquivalente) mit den entsprechenden Chloriden umgesetzt wurde. Die Rohprodukte wurden mittels Kieselgel-Chromatographie gereinigt (Laufmittel: Cyclohexan:Essigsäureethylester-Gradient oder isokratisch).

**Tabelle 4A:**

| **Beispiel** | **IUPAC-Name / Struktur (Ausbeute)** | **Analytische Daten** |
|---|---|---|
| **42A** | *rac-N*-(1-Azido-6,6,6-trifluorhexan-2-yl)-8-[(2,6-difluorbenzyl)oxy]-2-methylimidazo[1,2-a]pyridin-3-carboxamid ^{a)} | LC-MS (Methode 2): Rₜ = 1.03 min |
| | | MS (ESpos): m/z = 497 (M+H)⁺ |
| | 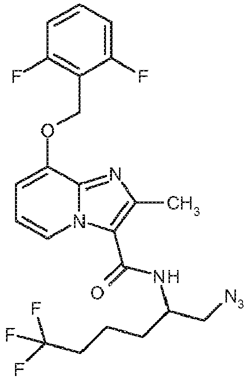 | ¹H-NMR (400 MHz, DMSO-d₆): δ = 1.52-1.70 (m, 4H), 2.20-2.41 (m, 2H), 2.50 (s, 3H), 3.52 (d, 2H), 4.18-4.26 (m, 1H), 5.30 (s, 2H), 6.93 (t, 1H), 7.01 (d, 1H), 7.23 (t, 2H), 7.59 (quint, 1H), 7.91 (d, 1H), 8.50 (d, 1H). |
| | (25% d. Th.) | |
| **43A** | *rac-N-*[1-Azido-5,5,5-trifluor-4-(trifluormethyl)pentan-2-yl]-8-[(2,6-difluorbenyl)oxy]-2-methylimidazo[1,2-a]pyridin-3-carboxamid | LC-MS (Methode 2): Rₜ = 1.08 min |
| | | MS (ESpos): m/z = 551 (M+H)⁺ |
| | 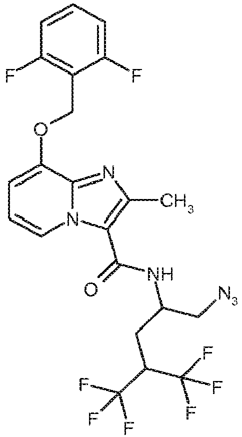 | ¹H-NMR (400 MHz, DMSO-d₆): δ = 2.06-2.19 (m, 2H), 2.52 (s, 3H), 3.61 (d, 2H), 3.98-4.13 (m, 1H), 4.26-4.38 (m, 1H), 5.30 (s, 2H), 6.94 (t, 1H), 7.03 (d, 1H), 7.23 (t, 2H), 7.59 (quint, 1H), 7.89 (d, 1H), 8.59 (d, 1H). |
| | (45% d. Th.) | |
| **44A** | *ent*-*N*-[(2*R*)-1-Azidohexan-2-yl]-8-[(2,6-difluorbenzyl)oxy]-2-methylimidazo[1,2-a]pyridin-3-carboxamid | LC-MS (Methode 2): Rₜ = 1.04 min |
| | | MS (ESpos): m/z = 443 (M+H)⁺ |
| | 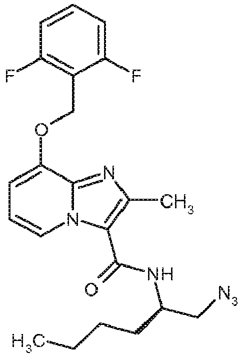 | ¹H-NMR (400 MHz, DMSO-d₆): δ = 0.88 (t, 3H), 1.22-1.42 (m, 4H), 1.49-1.61 (m, 2H), 2.50 (s, 3H), 3.48 (d, 2H), 4.10-4.21 (m, 1H), 5.30 (s, 2H), 6.93 (t, 1H), 6.99 (d, 1H), 7.23 (t, 2H), 7.59 (quint, 1H), 7.88 (d, 1H), 8.50 (d, 1H). |
| | (37% d. Th.) | |

| | | |
|---|---|---|
| a) Es wurden 20 Äquivalente Natriumazid verwendet. | | |

### Beispiel 45A

### rac-tert.-Butyl-[2-(4-chlorphenyl)-2-hydroxyethyl]carbamat

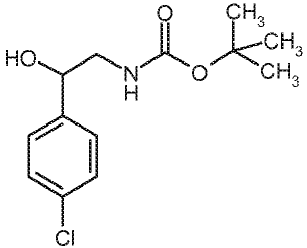

2.0 g *rac*-2-Amino-1-(4-chlorphenyl)ethanol-Hydrochlorid (9.61 mmol) in 14 ml Dichlormethan wurden zunächst mit 2.43 g Triethylamin (24.03 mmol) und anschließend mit 2.35 g Di-tert.-butyldicarbonat (10.76 mmol) versetzt. Das Reaktionsgemisch wurde 2 h bei RT gerührt. Es wurde mit Dichlormethan verdünnt, mit gesättigter, wässriger Natriumhydrogencarbonat-Lösung und mit gesättigter, wässriger Natriumchlorid-Lösung gewaschen, über Natriumsulfat getrocknet, filtriert, im Vakuum eingeengt und im Hochvakuum getrocknet. Es wurden 2.72 g der Titelverbindung (104% d. Th.) erhalten.
LC-MS (Methode 2): Rₜ = 0.97 min
MS (ESneg): m/z = 272 (M+H)⁺
¹H-NMR (400 MHz, DMSO-d₆): δ = 1.33 (s, 9H), 2.97-3.13 (m, 2H), 4.54-4.62 (m, 1H), 5.44 (d, 1H), 6.73 (t, 1H), 7.31 (d, 2H), 7.38 (d, 2H).

In Analogie zu Beispiel 45A wurde das in Tabelle 5A gezeigten Beispiel hergestellt, indem Di-tert.-butyldicarbonat in Dichlormethan mit dem entsprechenden, kommerziell erhältlichen Amin umgesetzt wurde.

**Tabelle 5A:**

| **Beispiel** | **IUPAC-Name / Struktur (Ausbeute)** | **Analytische Daten** |
|---|---|---|
| **46A** | *rac*-tert.-Butyl-(2-hydroxy-2-phenylethyl)carbamat | LC-MS (Methode 4): |
| | 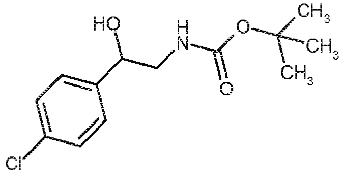 | MS (ESpos): m/z = 272 (M+H)⁺ |
| | | ¹H-NMR (400 MHz, DMSO-d₆): |
| | | δ = 1.33 (s, 9H), 2.97-3.13 (m, 2H), 4.54-4.62 (m, 1H), 5.44 (d, 1H), 6.73 (t, 1H), 7.19-7.38 (d, 2H). |
| | (104% d. Th.) | |

### Beispiel 47A

### rac-3-(1,3-Dioxo-1,3-dihydro-2H-isoindol-2-yl)-3-(4-fluorphenyl)propansäure

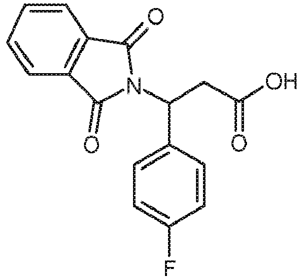

2.0 g *rac*-3-Amino-3-(4-fluorphenyl)propansäure (10.92 mmol) und 1.62 g Phthalsäureanhydrid (10.92 mmol) wurden in 9 ml DMF gelöst und bei 135°C über Nacht zum Rückfluss erhitzt. Die Reaktionslösung wurde auf ca. 200 ml Wasser gegeben. Der entstandene Feststoff wurde ca. 30 min bei RT gerührt, anschließend abfiltriert, mit Wasser gewaschen und im Hochvakuum getrocknet. Es wurden 3.43 g der Titelverbindung (86% d. Th., Reinheit ca. 86%) erhalten.
LC-MS (Methode 1): Rₜ = 1.09 min
MS (ESpos): m/z = 314 (M+H)⁺
¹H-NMR (400 MHz, DMSO-d₆): δ = 3.24-3.3.34 (m, 1H), 3.44-3.53 (m, 1H), 5.63-5.70 (m, 1H), 7.18 (t, 2H), 7.48 (dd, 1H), 7.82-7.90 (m, 4H), 12.48 (br s, 1H).

### Beisniel 48A

### rac-3-(3,4-Difluorphenyl)-3-(1,3-dioxo-1,3-dihydro-2H-isoindol-2-yl)propansäure

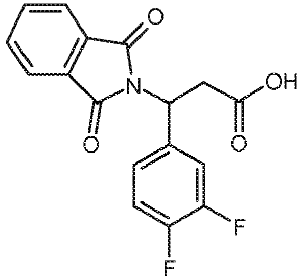

### 1.Stufe:

697 g 3,4-Difluorbenzaldehyd (4.76 mol, 1 Äquivalent) wurden mit 495 g Malonsäure (4.76 mol, 1 Äquivalent) und 733 g Ammoniumacetat (9.52 mol, 2 Äquivalente) in 2788 ml Ethanol 20 h bei Rückfluss unter Argon gerührt. Dann wurde auf RT abgekühlt und über Nacht bei RT gerührt. Die ausgefallenen Kristalle wurden abgesaut, mit Ethanol und Diethylether gewaschen und im Vakuum getrocknet. Es wurden 590 g (62% d. Th.) *rac*-3-Amino-3-(3,4-difluorphenyl)propansäure erhalten.
*ra*c-3-Amino-3-(3,4-difluorphenyl)propansäure:
LC-MS (Methode 1): Rₜ = 0.27 min
MS (ESpos): m/z = 202.0 (M+H)⁺

### 2. Stufe:

0.20 g (0.99 mmol) *rac*-3-Amino-3-(3,4-difluorphenyl)propansäure und 0.15 g (0.99 mmol) Phthalsäureanhydrid wurden in 0.8 ml DMF gelöst und bei 135°C über Nacht zum Rückfluss erhitzt. Die Reaktionslösung wurde auf ca. 9 ml Wasser gegeben. Die resultierende Suspension wurde zweimal mit Essigsäureethylester extrahiert. Die vereinigten organischen Phasen wurden mit Wasser gewaschen, über Natriumsulfat getrocknet, filtriert und eingeengt. Das Rohprodukt wurde mittels präparativer HPLC gereinigt (RP18 Säule, Laufmittel: Acetonitril/Wasser-Gradient unter Zusatz von 0.1 % TFA). Es wurden 0.2 g der Titelverbindung (61% d. Th.) erhalten.
LC-MS (Methode 2): Rₜ = 0.97 min
MS (ESpos): m/z = 332 (M+H)⁺
¹H-NMR (400 MHz, DMSO-d₆): δ = 3.24-3.3.33 (m, 1H), 3.44-3.52 (m, 1H), 5.63-5.70 (m, 1H), 7.23-7.28 (m, 1H), 7.36-7.47 (m, 1H), 7.49-7.57 (m, 1H), 7.82-7.90 (m, 4H), 12.51 (br s, 1H).

### Beispiel 49A

### rac-3-(2,4-Difluorphenyl)-3-(1,3-dioxo-1,3-dihydro-2H-isoindol-2-yl)propansäure

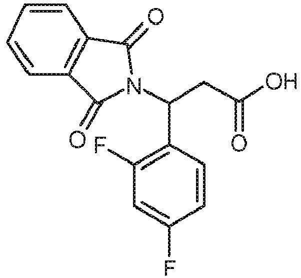

5.0 g *rac*-3-Amino-3-(2,4-difluorphenyl)propansäure (24.85 mmol) und 3.68 g Phthalsäureanhydrid (24.85 mmol) wurden in 20 ml DMF gelöst und bei 135°C über Nacht zum Rückfluss erhitzt. Die Reaktionslösung wurde auf ca. 160 ml Wasser gegeben und es wurde zweimal mit Essigsäureethylester extrahiert. Die vereinten organischen Phasen wurden mit Wasser gewaschen, über Natriumsulfat getrocknet, filtriert und eingeengt. Das Rohprodukt wurde mittels Kieselgel-Chromatographie (Laufmittel: Dichlormethan/Methanol 80:1, isokratisch) und anschließend mittels präparativer HPLC (RP18 Säule, Laufmittel: Acetonitril/Wasser-Gradient unter Zusatz von 0.1% TFA) gereinigt. Es wurden 3.43 g der Titelverbindung (27% d. Th.) erhalten.
LC-MS (Methode 1): Rₜ = 1.11 min
MS (ESpos): m/z = 332 (M+H)⁺
¹H-NMR (400 MHz, DMSO-d₆): δ = 3.24-3.3.34 (m, 1H), 3.40-3.49 (m, 1H), 5.89 (t, 1H), 7.09-7.15 (m, 1H), 7.19-7.28 (m, 1H), 7.70 (q, 1H), 7.82-7.89 (m, 4H), 12.55 (br s, 1H).

### Beispiel 50A

### rac-tert.-Butyl-[2-(4-chlorphenyl)-2-(1,3-dioxo-1,3-dihydro-2H-isoindol-2-yl)ethyl]carbamat

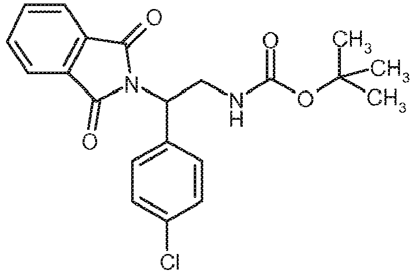

2.61 g *rac*-tert.-Butyl-[2-(4-chlorphenyl)-2-hydroxyethyl]carbamat (Beispiel 45A, 9.62 mmol), 1.42 g Phthalimid (9.62 mmol) und 3.78 g Triphenylphosphin (14.43 mmol) wurden bei RT in abs. THF vorgelegt. Anschließend wurden 4.03 g (14.43 mmol) Azodicarbonsäurediisopropylester zugetropft und es wurde 30 min bei RT gerührt. Das Reaktionsgemisch wurde eingeengt und mittels Kieselgel-Chromatographie gereinigt (Laufmittel: Cyclohexan:Essigsäureethylester 10:1). Es wurden 2.92 g der Titelverbindung (55% d. Th., Reinheit ca. 73%) erhalten.
LC-MS (Methode 2): Rₜ = 1.22 min
MS (ESpos): m/z = 401 (M+H)⁺
¹H-NMR (400 MHz, DMSO-d₆): δ = 1.26 (s, 9H), 3.70-3.79 (m, 1H), 3.82-3.93 (m, 1H), 5.32-5.38 (m, 1 H), 7.22 (t, 1H), 7.38-7.44 (m, 4H), 7.80-7.85 (m, 4H).

In Analogie zu Beispiel 50A wurde das in Tabelle 6A gezeigte Beispiel hergestellt, indem Phthalimid, Triphenylphosphin und Azodicarbonsäurediisopropylester in THF mit dem entsprechenden Alkohol umgesetzt wurde.

**Tabelle 6A:**

| **Beispiel** | **IUPAC-Name / Struktur (Ausbeute)** | **Analytische Daten** |
|---|---|---|
| **51A** | *rac*-tert.-Butyl-[2-(1,3-dioxo-1,3-dihydro-2H-isoindol-2-yl)ethyl]carbamat | LC-MS (Methode 2): Rₜ = 1.22 min |
| | 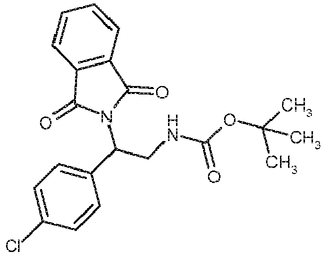 | MS (ESpos): m/z = 401 (M+H)⁺ |
| | | ¹H-NMR (400 MHz, DMSO-d₆): |
| | | δ = 1.26 (s, 9H), 3.70-3.79 (m, 1H), 3.82-3.93 (m, 1H), 5.32-5.38 (m, 1H), 7.22 (t, 1H), 7.38-7.44 (m, 4H), 7.80-7.85 (m, 4H). |
| | (55% d. Th., Reinheit ca. 73%) | |

### Beispiel 52A

### rac-tert.-Butyl-[2-(1,3-dioxo-1,3-dihydro-2H-isoindol-2-yl)-2-(4-fluorphenyl)ethyl]carbamat

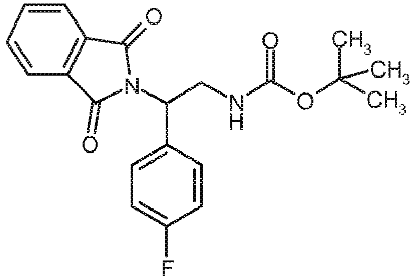

Unter Argon wurde eine Lösung aus 3.2 g *rac*-3-(1,3-Dioxo-1,3-dihydro-2H-isoindol-2-yl)-3-(4-fluorphenyl)propansäure (Beispiel 47A, 8.78 mmol) in 32 ml Toluol vorgelegt und mit 1.33 g Triethylamin (13.18 mmol), 98 mg 1,4-Diazabicyclo[2.2.2]octan (0.88 mmol), 3.14 g Diphenylphosphorazidat (11.42 mmol) sowie 6.51 g tert.-Butanol (87.84 mmol) versetzt. Das Reaktionsgemisch wurde über Nacht zum Rückfluss erhitzt und dann mit Essigsäureethylester verdünnt und mit Wasser gewaschen. Die organische Phase wurde über Natriumsulfat getrocknet, filtriert und eingeengt. Das Rohprodukt wurde mittels Kieselgel-Chromatographie (Laufmittel: Cyclohexan/Ethylacetat 8:1; 6:1) gereinigt. Es wurden 959 mg der Titelverbindung (28% d. Th.) erhalten.
LC-MS (Methode 2): Rₜ = 1.11 min
MS (ESpos): m/z = 385 (M+H)
¹H-NMR (400 MHz, DMSO-d₆): δ = 1.26 (s, 9H), 3.69-3.78 (m, 1H), 3.84-3.95 (m, 1H), 5.32-5.39 (m, 1H), 7.15-7.26 (m, 3H), 7.41-7.48 (m, 2H), 7.80-7.89 (m, 4H).

### Beispiel 53A

### rac-tert.-Butyl-[2-(3,4-difluorphenyl)-2-(1,3-dioxo-1,3-dihydro-2H-isoindol-2-yl)ethyl]carbamat

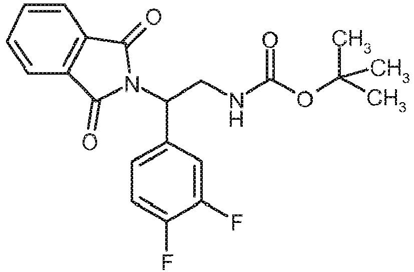

Unter Argon wurde eine Lösung aus 5.0 g *rac*-3-(3,4-Difluorphenyl)-3-(1,3-dioxo-1,3-dihydro-2H-isoindol-2-yl)propansäure (Beispiel 48A, 15.09 mmol) und 3.06 g Triethylamin (30.19 mmol) in 65 ml Toluol vorgelegt, mit 4.36 g Diphenylphosphorazidat (15.85 mmol) versetzt und 3.5 h bei RT gerührt. Anschließend wurden 65 ml tert.-Butanol zugegeben und über Nacht unter Rückfluss gerührt. Nach dem Abkühlen wurde die Reaktionslösung eingeengt und mittels Flashchromatographie gereinigt (Laufmittel: Petrolether/Essigsäureethylester 2:1, isokratisch). Es wurden 3.1 g der Titelverbindung (45% d. Th.) erhalten.
LC-MS (Methode 2): Rₜ = 1.19 min
MS (ESpos): m/z = 403 (M+H)⁺
¹H-NMR (400 MHz, DMSO-d₆): δ = 1.26 (s, 9H), 3.73-3.90 (m, 2H), 5.32-5.39 (m, 1H), 7.20-7.27 (m, 2H), 7.36-7.46 (m, 1H), 7.48-7.56 (m, 1H), 7.81-7.91 (m, 4H).

### Beispiel 54A

### rac-tert.-Butyl-[2-(2,4-difluorphenyl)-2-(1,3-dioxo-1,3-dihydro-2H-isoindol-2-yl)ethyl]carbamat

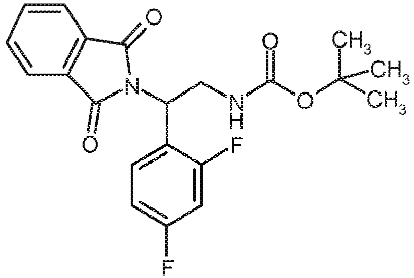

2.17 g *rac*-3-(2,4-Difluorphenyl)-3-(1,3-dioxo-1,3-dihydro-2H-isoindol-2-yl)propansäure (Beispiel 49A, 6.54 mmol) und 1.32 g Triethylamin (13.07 mmol) wurden unter Argon in 23.8 ml abs. Toluol vorgelegt. 1.89 g Diphenylphosphorazidat (6.86 mmol) wurden bei RT zugegeben und 3.5 h bei RT unter Wasserkühlung gerührt und anschließend wurden 23.8 ml tert.-Butanol zugegeben und über Nacht unter Rückfluss gerührt. Nach dem Abkühlen wurde die Reaktionslösung eingeengt und mittels Flashchromatographie gereinigt (Laufmittel: Cyclohexan/Essigsäureethylester 2:1). Es wurden 650 mg der Titelverbindung (24% d. Th.) erhalten.
LC-MS (Methode 2): Rₜ = 1.11 min
MS (ESpos): m/z = 403 (M+H)⁺

### Beispiel 55A

### rac-tert.-Butyl-[2-amino-2-(3,4-difluorphenyl)ethyl]carbamat

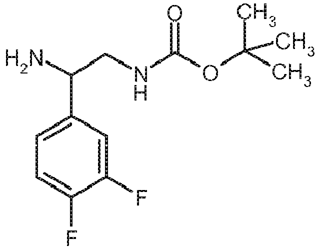

6.13 g *rac*-tert.-Butyl-[2-(3,4-difluorphenyl)-2-(1,3-dioxo-1,3-dihydro-2H-isoindol-2-yl)ethyl]-carbamat (Beispiel 53A, Reinheit ca. 60%, ca. 9.14 mmol) wurde in 13.1 ml 40%iger, wässriger Methylamin-Lösung vorgelegt und über Nacht bei 60°C in einem verschlossenen Gefäß gerührt Das Reaktionsgemisch wurde eingeengt und der Rückstand mittels Kieselgel-Chromatographie gereinigt (Laufmittel: Dichlormethan:Methanol:Diethylamin 30:1:0.1; 20:1:0.1). Es wurden 1.83 g der Titelverbindung (74% d. Th.) erhalten.
LC-MS (Methode 1): Rₜ = 0.65 min
MS (ESpos): m/z = 273 (M+H)⁺
¹H-NMR (400 MHz, DMSO-d₆): δ = 1.33 (s, 9H), 1.96 (br s, 2H), 2.92-3.10 (m, 2H), 3.81-3.88 (m, 1H), 6.76-6.82 (m, 1H), 7.11-7.17 (m, 1H), 7.27-7.40 (m, 2H).

In Analogie zu Beispiel 55A wurden die in Tabelle 7A gezeigten Beispiele hergestellt, indem eine Lösung von Methylamin mit den entsprechenden Phthalimiden umgesetzt wurde

**Tabelle 7A:**

| **Beispiel** | **IUPAC-Name / Struktur (Ausbeute)** | **Analytische Daten** |
|---|---|---|
| **56A** | *rac*-tert.-Butyl-[2,amino-2-(4-chlorphenyl)ethyl]carbamat-Trifluoracetat ¹⁾ | LC-MS (Methode 2): Rₜ = 0.68 min |
| | | MS (ESpos): m/z = 271 (M+H-TFA)⁺ |
| | 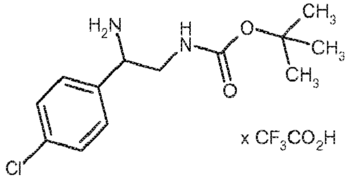 | ¹H-NMR (400 MHz, DMSO-d₆): |
| | | δ = 1.31 (s, 9H), 3.28-3.44 (m, 2H), 4.31 (br s, 1H), 7.00 (t, 1H), 7.43 (d, 2H), 7.52 (d, 2H), 8.42 (br s, 3H). |
| | (41% d. Th.) | |
| **57A** | *rac*-tert.-Butyl-(2-amino-2-phenylethyl)carbamat-Trifluoracetat ¹⁾ | LC-MS (Methode 2): Rₜ = 0.59 min |
| | | MS (ESpos): m/z = 237 (M+H-TFA)⁺ |
| | 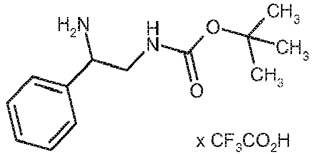 | ¹H-NMR (400 MHz, DMSO-d₆): |
| | | δ = 1.34 (s, 9H), 3.28-3.46 (m, 2H), 4.29 (br s, 1H), 7.01 (t, 1H), 7.35-7.48 (m, 5H), 8.43 (br s, 3H). |
| | (45% d. Th.) | |
| **58A** | *rac*-tert.-Butyl-[2-amino-2-(4-fluorphenyl)ethyl]carbamat ²⁾ | LC-MS (Methode 2): Rₜ = 0.60 min |
| | | MS (ESpos): m/z = 255 (M+H)⁺ |
| | 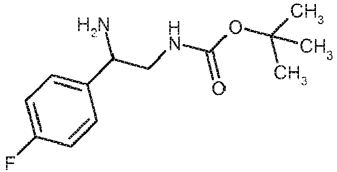 | ¹H-NMR (400 MHz, DMSO-d₆): |
| | | δ = 1.33 (s, 9H), 1.89 (br s, 2H), 2.88-2.97 (m, 1H), 3.04-3.11 (m, 1H), 3.84-3.90 (m, 1H), 6.80 (t, 1H), 7.11 (t, 2H), 7.36 (dd, 2H). |
| | (85% d. Th.) | |
| **59A** | *rac*-tert.-Butyl-[2-amino-2-(2,4-difluorphenyl)ethyl]carbamat | LC-MS (Methode 2): Rₜ = 0.65 min |
| | | MS (ESpos): m/z = 273 (M+H)⁺ |
| | 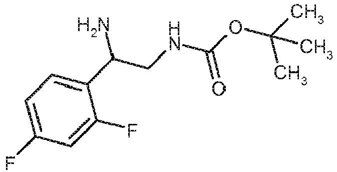 | ¹H-NMR (400 MHz, DMSO-d₆): |
| | | δ = 1.31 (s, 9H), 1.91 (br s, 2H), 2.97-3.14 (m, 2H), 4.12 (t, 1H), 6.81 (t, 1H), 6.99-7.17 (m, 2H), 7.54 (q,1H). |
| | (ca. 86% d. Th.) | |

| | | |
|---|---|---|
| 1) Das erhaltene Rohprodukt wurde eingeengt und mittels präparativer HPLC ein weiteres Mal gereinigt (RP18 Säule, Laufmittel: Acetonitril/Wasser-Gradient unter Zusatz von 0.1% TFA). 2) Reaktionsbedingungen: 20 eq. Methylamin [40%ige Lösung in Wasser]; 7 h bei 60°C. | | |

### Beispiel 60A

### ent- tert.-Butyl-[2-amino-2-(3,4-difluorphenyl)ethyl]carbamat (Enantiomer A)

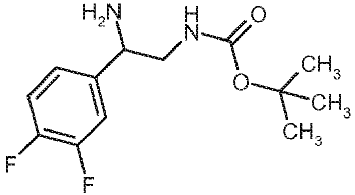

100 mg *rac*-tert.-Butyl-[2-amino-2-(3,4-difluorphenyl)ethyl]carbamat (Beispiel 55A) wurden an chiraler Phase in die Enantiomere getrennt [Säule: Daicel Chiralpak AY-H, 5 µm, 250 x 20 mm, Eluent: 80% iso-Hexan, 20% Ethanol + 0.2% Diethylamin, Fluß 15 ml/min; 30°C, Detektion: 220 nm].
Ausbeute: 43 mg Enantiomer A (99% Reinheit, >99% ee)
Rₜ = 4.58 min [Daicel Chiralpak AY-H, 5µm, 250 x 4.6 mm; Eluent: 80% iso-Hexan, 20% Ethanol + 0.2% Diethylamin; Fluss 1.0 ml/min; 30°C; Detektion: 220 nm].

### Beispiel 61A

### ent- tert.-Butyl-[2-amino-2-(3,4-difluorphenyl)ethyl]carbamat (Enantiomer B):

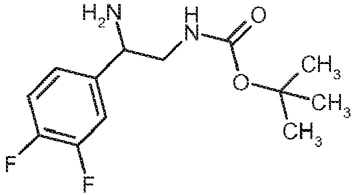

100 mg *rac*-tert.-Butyl-[2-amino-2-(3,4-difluorphenyl)ethyl]carbamat (Beispiel 55A) wurden an chiraler Phase in die Enantiomere getrennt [Säule: Daicel Chiralpak AY-H, 5 µm, 250 x 20 mm, Eluent: 80% iso-Hexan, 20% Ethanol + 0.2% Diethylamin, Fluß 15 ml/min; 30°C, Detektion: 220 nm].

Ausbeute: 44 mg Enantiomer B (99% Reinheit, >99% ee) Rₜ = 5.61 min [Daicel Chiralpak AY-H, 5µm, 250 x 4.6 mm; Eluent: 80% iso-Hexan, 20% Ethanol + 0.2% Diethylamin; Fluss 1.0 ml/min; 30°C; Detektion: 220 nm].

### Beispiel 62A

### ent- tert.-Butyl-[2-amino-2-(2,4-difluorphenyl)ethyl]carbamat (Enantiomer A)

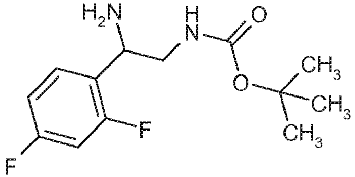

435 mg von Beispiel 59A wurden an chiraler Phase in die Enantiomere getrennt [Säule: Daicel Chiralpak AY-H, 5 µm, 250 x 20 mm, Eluent: 80% iso-Hexan, 20% Ethanol + 0.2% Diethylamin, Fluß 15 ml/min; 30°C, Detektion: 220 nm]. Zur Entfernung von Lösungsmittelresten wurde das Produkt in Acetonitril/Wasser gelöst und lyophilisiert.
Ausbeute: 182 mg (97% Reinheit, >99% ee)
Enantiomer B: Rₜ = 5.25 min [Daicel Chiralpak AY-H, 5µm, 250 x 4.6 mm; Eluent: 80% iso-Hexan, 20% Ethanol + 0.2% Diethylamin; Fluss 1.0 ml/min; 30°C; Detektion: 220 nm].

### Beispiel 63A

### ent-Benzyl-tert.-butyl-[1-(3,4-difluorphenyl)ethan-1,2-diyl]biscarbamat

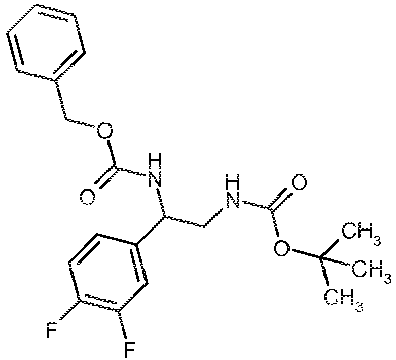

300 mg *ent*-tert.-Butyl-[2-amino-2-(3,4-difluorphenyl)ethyl]carbamat (Enantiomer A) (Beispiel 60A; 1.10 mmol) wurden in 5 ml trockenem THF vorgelegt und mit 1.15 ml Diisopropylethylamin (6.6 mmol, 6 Äquivalente), 26 mg *N*,*N-*Dimethylaminopyridin (0.22 mmol, 0.2 Äquivalente) und anschließend tropfenweise mit 0.31 ml Chlorameisensäurebenzylester (2.2 mmol, 2 Äquivalente) versetzt. Das Reaktionsgemisch wurde 48 h bei RT gerührt, anschließend eingeengt, in Essigsäureethylester aufgenommen und mit Wasser gewaschen. Die vereinigten organischen Phasen wurden mit gesättigter, wässriger Natriumchlorid-Lösung gewaschen, über Magnesiumsulfat getrocknet, filtriert und eingeengt. Der Rückstand wurde an Silicagel chromatographiert (Laufmittel: Cyclohexan/Essigsäureethylester 1:1). Es wurden 336 mg (75% d. Th.) der Titelverbindung erhalten.
LC-MS (Methode 2): Rₜ = 1.14 min
MS (ESpos): m/z = 407.3 (M+H)⁺
¹H-NMR (400 MHz, DMSO-d₆): δ = 1.3 (s, 9 H); 3.13 (t, 2 H); 4.65 (q, 1 H); 5.00 (q, 2 H); 6.88 (t, 1 H); 7.1 (br. s., 1H); 7.21- 7.40 (m, 7 H); 7.80 (d, 1H).

### Beispiel 64A

### ent-Benzyl-[2-amino-1-(3,4-difluorphenyl)ethyl]carbamat

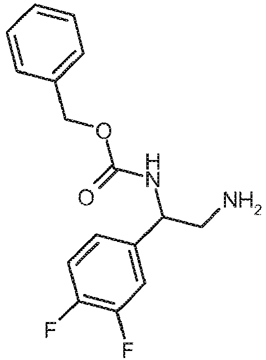

335 mg *ent*-Benzyl-tert.-butyl-[1-(3,4-difluorphenyl)ethan-1,2-diyl]biscarbamat (Beispiel 63A; 0.824 mmol) wurden mit 16.5 ml einer 2 N Lösung von Salzsäure in Diethylether versetzt und über Nacht bei RT gerührt. Es wurden zusätzliche 16.5 ml einer 4 N Lösung von Salzsäure in 1,4-Dioxan zugesetzt und für weitere 3 h bei RT gerührt. Das Reaktionsgemisch wurde eingeengt, mit gesättigter, wässriger Natriumhydrogencarbonat-Lösung versetzt und dreimal mit Essigsäureethylester extrahiert. Die vereinigten organischen Phasen wurden mit gesättigter, wässriger Natriumchlorid-Lösung gewaschen, über Magnesiumsulfat getrocknet, filtiert und eingeengt. Es wurden 252.4 mg (84% d. Th.) der Titelverbindung erhalten.
LC-MS (Methode 2): Rₜ = 0.74 min
MS (ESpos): m/z = 307.2 (M+H)⁺
¹H-NMR (400 MHz, DMSO-d₆): δ = 1.82 - 1.95 (br. s, 2 H); 2.70 (d, 2 H); 4.49 (q, 1H); 5.00 (m, 2 H); 7.1 (br. s., 1H); 7.21 - 7.40 (m, 7 H); 7.80 (d, 1H).

### Beispiel 65A

### rac-tert.-Butyl-(3-ethoxy-2-hydroxypropyl)carbamat

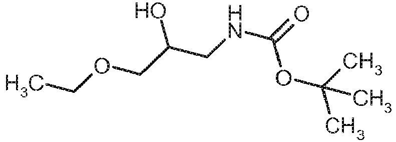

3 g *rac*-1-Amino-3-ethoxy-2-propanol-Hydrochlorid (19.28 mmol, 1 Äquivalent) wurden in 40 ml Dichlormethan vorgelegt, mit 5.7 ml Triethylamin (40.9 mmol, 2.1 Äquivalente) und anschließend mit 4.96 ml Di-tert.-butyldicarbonat (21.6 mmol, 1.12 Äquivalente) versetzt. Das Reaktionsgemisch wurde 2 h bei RT gerührt, mit Dichlormethan verdünnt und mit gesättigter, wässriger Natriumhydrogencarbonat-Lösung und mit gesättigter, wässriger Natriumchlorid-Lösung gewaschen. Die organische Phase wurde mittels Magnesiumsulfat getrocknet, filtriert und eingeengt. Der Rückstand wurde am Hochvakuum getrocknet. Es wurden 3.73 g Rohprodukt (88% d. Th.) erhalten, die ohne weitere Aufreinigung weiter umgesetzt wurden.
¹H-NMR (400 MHz, DMSO-d₆): δ = 1.10 (t, 3H); 1.39 (s, 9 H); 2.85 (dt, 1 H); 3.02 (dt, 1 H); 3.20 - 3.30 (m, 2 H); 3.40 (q, 2 H); 3.55 - 3.60 (m, 1 H); 4.75 (d, 1 H); 6.61 (t, 1 H).

### Beispiel 66A

### rac-tert.-Butyl-[2-(1,3-dioxo-1,3-dihydro-2H-isoindol-2-yl)-3-ethoxypropyl]carbamat

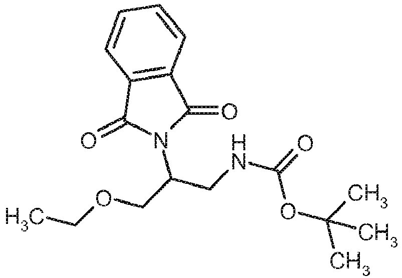

3.73 g *rac*-tert.-Butyl-(3-ethoxy-2-hydroxypropyl)carbamat (17 mmol, 1 Äquivalent), 2.50 g Phthalimid (17 mmol, 1 Äquivalente) und 6.69 g Triphenylphosphin (25.52 mmol, 1.5 Äquivalente) wurden bei RT in 70 ml trockenem THF vorgelegt. 5.06 ml Diisopropylazodicarboxylat (25.5 mmol, 1.5 Äquivalente) wurden zugetropft und 2 h bei RT gerührt. Das Reaktionsgemisch wurde im Vakuum eingeengt. Das Rohprodukt wurde mit Methanol, Acetonitril und Wasser auf 90 ml verdünnt und per präparativer HPLC (Säulenmaterial: Sunfire C18 5 µm 75x30 mm; Fluß 56 ml/min; Eluent: 45% Milli-Q-Wasser/50% Acetonitril/5% 1% wässrige Ameisensäure; Injektionsvolumen: 0.5 ml; Detektions-Wellenlänge: 210 nm) aufgereinigt. Es wurden 4.88 g der Titelverbindung (82% d. Th.) erhalten.
LC-MS (Methode 2): Rₜ = 1.04 min
MS (ESpos): m/z = 349.3 (M+H)⁺
¹H-NMR (400 MHz, DMSO-d₆): δ = 1.02 (t, 3H); 1.25 (s, 9 H); 3.34 - 3.48 (m, 4 H); 3.65 (dd, 1 H); 3.81 (dd, 1H); 4.32 - 4.38 (m, 1 H); 7.10 (t, 1H); 7.80 - 7.90 (m, 4 H).

### Beispiel 67A

### rac-tert.-Butyl-(2-amino-3-ethoxypropyl)carbamat

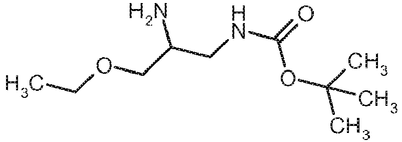

4.88 g *rac*-tert.-Butyl-[2-(1,3-dioxo-1,3-dihydro-2H-isoindol-2-yl)-3-ethoxypropyl]carbamat (14.0 mmol, 1 Äquivalent) wurden in 12 ml 40%iger, wässriger Methylamin-Lösung vorgelegt und für 1.5 h bei 100°C in der Mikrowelle umgesetzt. Die Reaktionsmischung wurde eingeengt, der Rückstand in 10 ml Toluol aufgenommen und wieder eingeengt. Dieser Schritt wurde mehrfach wiederholt. Der Rückstand wurde anschließend an Kieselgel chromatographiert (Eluent: Dichlormethan/Methanol 10:1). Es wurden 470 mg (15% d. Th.) der Titelverbindung erhalten.
¹H-NMR (400 MHz, DMSO-d₆): δ = 1.10 (t, 3H); 1.38 (s, 9 H); 3.03 - 3.20 (m, 3 H); 3.34 - 3.48 (m, 4 H); 6.95 (t, 1 H).

### Beispiel 68A

### rac-tert.-Butyl-2-hydroxy-3-phenoxypropyl)carbamat

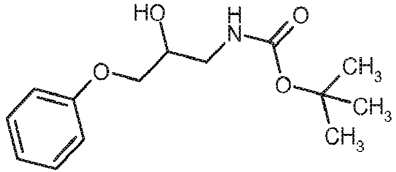

1.13 g *rac*-1-Amino-3-phenoxypropan-2-ol-Hydrochlorid (5.5 mmol, 1 Äquivalent) wurden in 11.5 ml Dichlormethan vorgelegt, mit 1.64 ml Triethylamin (11.7 mmol, 2.1 Äquivalente) und anschließend mit 1.43 ml Di-tert.-butyldicarbonat (6.21 mmol, 1.12 Äquivalente) versetzt. Das Reaktionsgemisch wurde 2 h bei RT gerührt, mit Dichlormethan verdünnt und mit gesättigter, wässriger Natriumhydrogencarbonat-Lösung und mit gesättigter, wässriger Natriumchlorid-Lösung gewaschen. Die organische Phase wurde mittels Magnesiumsulfat getrocknet, filtriert und eingeengt. Der Rückstand wurde am Hochvakuum getrocknet. Es wurden 1.5 g Rohprodukt (quantitative Ausbeute) erhalten, die ohne weitere Aufreinigung weiter umgesetzt wurden.
LC-MS (Methode 2): Rₜ = 0.88 min
MS (ESpos): m/z = 268.2 (M+H)⁺

### Beispiel 69A

### rac-tert.-Butyl-[2-(1,3-dioxo-1,3-dihydro-2H-isoindol-2-yl)-3-phenoxypropyl]carbamat

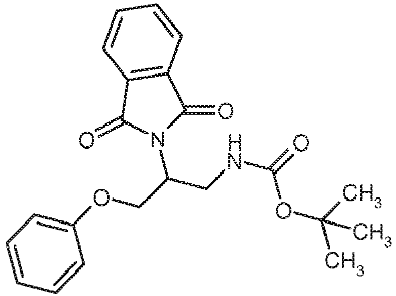

1.5 g *rac*-tert.-Butyl-(2-hydroxy-3-phenoxypropyl)carbamat (5.6 mmol, 1 Äquivalent), 0.99 g Phthalimid (6.73 mmol, 1.2 Äquivalente) und 2.21 g Triphenylphosphin (8.4 mmol, 1.5 Äquivalente) wurden bei RT in 23 ml trockenem Tetrahydrofuran vorgelegt. 1.70 ml Diisopropylazodicarboxylat (8.4 mmol, 1.5 Äquivalente) wurden zugetropft und 2 h bei RT gerührt. LC/MS zeigte vollständigen Reaktionsumsatz. Das Reaktionsgemisch wurde eingeengt und per Chromatographie an Kieselgel (Biotage Isolera; Cyclohexan-Essigsäureethylester-Gradient als Laufmittel) aufgereinigt. Es wurden 1.08 g der Titelverbindung (48% d. Th.) erhalten.
LC-MS (Methode 2): Rₜ = 1.13 min
MS (ESpos): m/z = 397.3 (M+H)⁺

### Beispiel 70A

### rac-tert.-Butyl-2-amino-3-phenoxypropyl)carbamat

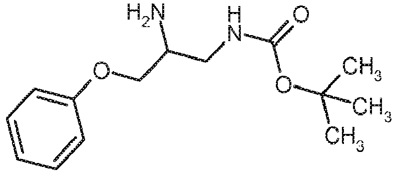

1.08 g *rac-*tert.-Butyl-[2-(1,3-dioxo-1,3-dihydro-2H-isoindol-2-yl)-3-phenoxypropyl]carbamat (2.72 mmol, 1 Äquivalent) wurden in 5 ml 40%iger wässriger Methylamin-Lösung vorgelegt und für 2 h bei 100°C in der Mikrowelle umgesetzt. Die Reaktionsmischung wurde eingeengt und der Rückstand wurde anschließend an Kieselgel chromatographiert (Biotage Isolera; Laufmittel: Dichlomethan/Methanol Gradient). Es wurden 200 mg (27% d. Th.) der Titelverbindung erhalten.
LC-MS (Methode 2): Rₜ = 0.57 min
MS (ESpos): m/z = 267.1 (M+H)⁺

### Beispiel 71A

### rac-tert.-Butyl-[3-(4-fluorphenoxy)-2-hydroxypropyl]carbamat

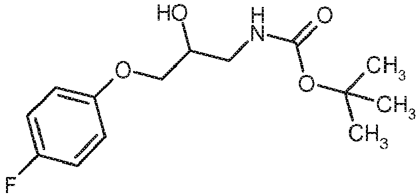

0.93 g *rac*-1-Amino-3-(4-fluorphenoxy)-propan-2-ol (5.07 mmol, 1 Äquivalent) wurden in 10.5 ml Dichlormethan vorgelegt und zunächst mit 1.5 ml Triethylamin (10.7 mmol, 2.1 Äquivalente) und anschließend mit 1.31 ml Di-tert.-butyldicarbonat (5.68 mmol, 1.12 Äquivalente) versetzt. Das Reaktionsgemisch wurde 2 h bei RT gerührt. Dann wurde mit Dichlormethan verdünnt und mit gesättigter, wässriger Natriumhydrogencarbonat-Lösung und mit gesättigter, wässriger Natriumchlorid-Lösung gewaschen. Die organische Phase wurde über Magnesiumsulfat getrocknet, filtriert und eingeengt. Der Rückstand wurde ohne weitere Aufreinigung in die nächste Umsetzung eingesetzt.
LC-MS (Methode 2): Rₜ = 0.89 min
MS (ESpos): m/z = 286.2 (M+H)⁺

### Beispiel 72A

### rac-tert.-Butyl-[2-(1,3-dioxo-1,3-dihydro-2H-isoindol-2-yl)-3-(4-fluorphenoxy)propyl]carbamat

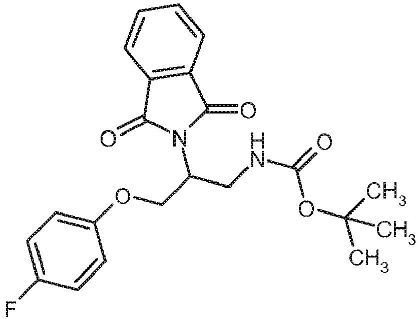

1.5 g *rac*-tert.-Butyl-[3-(4-fluorphenoxy)-2-hydroxypropyl]carbamat (5.26 mmol, 1 Äquivalent), 0.93 g Phthalimid (6.31 mmol, 1.2 Äquivalente) und 2.07 g Triphenylphosphin (7.9 mmol, 1.5 Äquivalente) wurden bei RT in 22 ml trockenem THF vorgelegt. 1.56 ml Diisopropylazodicarboxylat (7.9 mmol, 1.5 Äquivalente) wurden zugetropft und 2 h bei RT gerührt. Das Reaktionsgemisch wurde im Vakuum eingeengt und per Chromatographie an Kieselgel (Biotage Isolera; Cyclohexan-Essigsäureethylester-Gradient als Laufmittel) aufgereinigt. Es wurden 1.97 g der Titelverbindung (90% d. Th.) erhalten.
LC-MS (Methode 2): Rₜ = 1.14 min
MS (ESpos): m/z = 415.3 (M+H)⁺

### Beispiel 73A

### rac-tert.-Butyl-[2-amino-3-(4-fluorphenoxy)propyl]carbamat

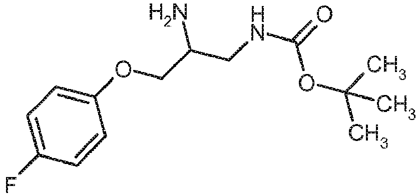

1.97 g *rac*-tert.-Butyl-[2-(1,3-dioxo-1,3-dihydro-2H-isoindol-2-yl)-3-(4-fluorphenoxy)propyl]-carbamat (4.75 mmol, 1 Äquivalent) wurden in 5 ml 40%iger wässriger Methylamin-Lösung vorgelegt und für 2 h bei 100°C in der Mikrowelle umgesetzt. Die Reaktionsmischung wurde eingeengt und der Rückstand wurde anschließend an Kieselgel chromatographiert (Biotage Isolera; Laufmittel: Dichlomethan/Methanol Gradient). Es wurden 900 mg (67% d. Th.) der Titelverbindung erhalten.
LC-MS (Methode 2): Rₜ = 0.58 min
MS (ESpos): m/z = 285.1 (M+H)

### Beispiel 74A

### rac-Benzyl-(2-hydroxy-3-isopropoxypropyl)carbamat

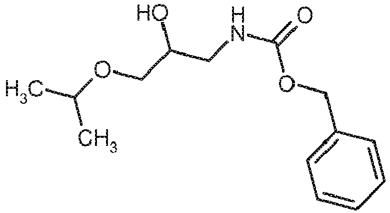

1 g *rac*-1-Amino-3-isopropoxypropan-2-ol (7.5 mmol, 1 Äquivalent) wurden in 25 ml THF vorgelegt, mit 1.16 ml Chlorameisensäurebenzylester (8.3 mmol, 1.1 Äquivalente), 3.9 ml Diisopropylethylamin (22.5 mmol, 3 Äquivalente) und 183 mg *N,N-*Dimethylaminopyridin (1.5 mmol, 0.2 Äquivalente) versetzt. Das Reaktionsgemisch wurde bei RT gerührt und nach ca. 30 Min mit 5 ml DMF versetzt. Nach weiteren 2.5 h bei RT wurde zur Trockene eingeengt. Der Rückstand wurde mit Essigsäureethylester aufgenommen, mit gesättigter, wässriger Natriumhydrogencarbonat-Lösung und anschließend mit gesättigter, wässriger Natriumchlorid-Lösung gewaschen. Die organische Phase wurde über Magnesiumsulfat getrocknet, filtriert und eingeengt. Der Rückstand wurde ohne weitere Aufreinigung in die nächste Umsetzung eingesetzt.
LC-MS (Methode 2): Rₜ = 0.80 min
MS (ESpos): m/z = 268.2 (M+H)⁺

### Beispiel 75A

### rac-Benzyl-[2-(1,3-dioxo-1,3-dihydro-2H-isoindol-2-yl)-3-isopropoxypropyl]carbamat

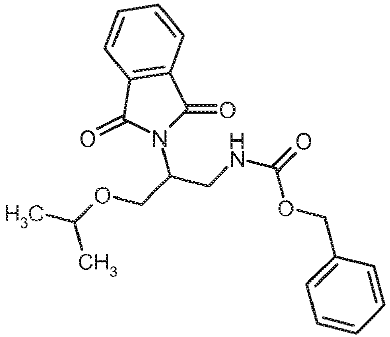

1.28 g racemisches Benzyl-(2-hydroxy-3-isopropoxypropyl)carbamat (4.79 mmol, 1 Äquivalent), 0.85 g Phthalimid (5.75 mmol, 1.2 Äquivalent) und 1.88 g Triphenylphosphin (7.2 mmol, 1.5 Äquivalent) wurden bei RT in 20 ml trockenem Tetrahydrofuran vorgelegt. 1.42 ml Düsopropylazodicarboxylat (7.2 mmol, 1.5 Äquivalent) wurden zugetropft und es wurde 2 h bei RT gerührt. Das Reaktionsgemisch wurde im Vakuum eingeengt und per Chromatographie an Kieselgel (Biotage Isolera; Cyclohexan-Essigsäureethylester-Gradient als Laufmittel) aufgereinigt. Es wurden 2.3 g (66% Reinheit; 78% d. Th.) der Titelverbindung (verunreinigt mit Diisopropylhydrazin-1,2-dicarboxylat) erhalten.
LC-MS (Methode 2): Rₜ = 1.12 min
MS (ESpos): m/z = 397.2 (M+H)⁺
¹H-NMR (400 MHz, DMSO-d₆): δ = 0.91 (d, 3 H), 1.00 (d, 3 H), 3.42 - 3.48 (m, 2 H), 3.50 (hept, 1 H), 3.69 (dd, 1 H), 3.79 (t, 1 H), 4.31 (q, 1 H), 4.91 (s, 2 H), 7.20 - 7.30 (m, 5 H), 7.52 (t, 1 H), 7.80 - 7.86 (m, 4 H).

### Beispiel 76A

### rac-Benzyl-(2-amino-3-isopropoxypropyl)carbamat

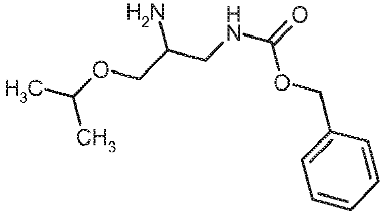

2.3 g *rac*-Benzyl-[2-(1,3-dioxo-1,3-dihydro-2H-isoindol-2-yl)-3-isopropoxypropyl]carbamat (5.6 mmol, 1 Äquivalent) wurden in 30 ml Ethanol gelöst, mit 7.3 ml 40%iger wässriger Methylamin-Lösung (84.4 mmol, 15 Äquivalent) versetzt und über Nacht bei 60 °C gerührt. Die Reaktionsmischung wurde eingeengt und der Rückstand wurde anschließend an Kieselgel chromatographiert (Biotage Isolera; Laufmittel: Dichlomethan/Methanol Gradient). Es wurden 730 mg (49% d. Th.) der Titelverbindung erhalten.
LC-MS (Methode 2): Rₜ = 0.59 min
MS (ESpos): m/z = 267.2 (M+H)⁺
¹H-NMR (400 MHz, DMSO-d₆): δ = 1.05 (d, 6 H), 1.74 (br. s, 2 H), 2.79 - 2.90 (m, 2 H), 3.02 - 3.12 (m, 1 H), 3.18 (dd, 1 H), 3.21 (dd, 1 H), 3.50 (q, 1 H), 5.00 (s, 2 H), 7.18 (t, 1 H), 7.28 - 7.39 (m, 5 H).

### Beispiel 77A

### rac-tert.-Butyl-{2-[({8-[(2,6-difluorbenzyl)oxy]-2-methylimidazo[1,2-a]pyridin-3-yl}carbonyl)amino]-2-(3,4-difluorphenyl)ethyl}carbamat

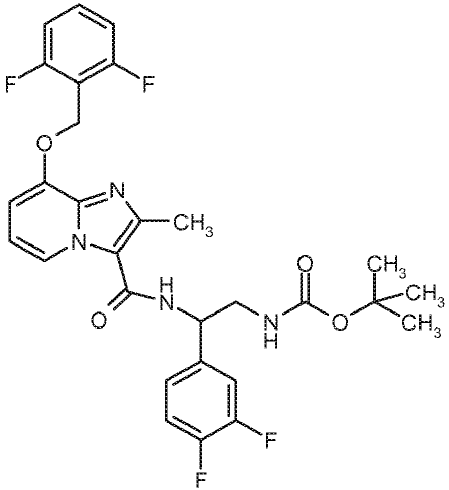

200 mg 8-[(2,6-Difluorbenzyl)oxy]-2-methylimidazo[1,2,a]pyridin-3-carbonsäure (0.63 mmol), 242 mg *rac*-(Benzotriazol-1-yloxy)bisdimethylaminomethyliumfluorborat (TBTU, 0.75 mmol) und 318 mg 4-Methylmorpholin (3.14 mmol) wurden in 4.3 ml DMF vorgelegt. Bei RT wurden 242 mg *rac*-tert.-Butyl-[2-amino-2-(3,4-(difluorphenyl)ethyl]carbamat (Beispiel 55A, 0.75 mmol) zugesetzt und es wurde über Nacht bei RT gerührt. Die Reaktionslösung wurde mit ca. 16 ml Wasser versetzt, noch 30 min gerührt, der entstandene Niederschlag abfiltriert und mit Wasser gewaschen. Der Feststoff wurde mit ca. 4 ml Acetonitril für 10 min im Ultraschallbad behandelt, abfiltriert und über Nacht im Hochvakuum getrocknet. Es wurden 355 mg der Titelverbindung (96% d. Th) erhalten.
LC-MS (Methode 2): Rₜ = 1.10 min
MS (ESpos): m/z = 573 (M+H)⁺
¹H-NMR (400 MHz, DMSO-d₆): δ = 1.32 (s, 9H), 2.59 (s, 3H), 3.29-3.46 (m, 2H), 5.15 (q, 1H), 5.31 (s, 2H), 6.91 (t, 1H), 7.01 (d, 1H), 7.08 (t, 1H), 7.19-7.27 (m, 3H), 7.36-7.51 (m, 2H), 7.59 (q, 1H), 8.21 (d, 1H), 8.56 (d, 1H).

Die in Tabelle 8A gezeigten Beispiele wurden hergestellt, indem 8-[(2,6-Difluorbenzyl)oxy]-2-methylimidazo[1,2-a]pyridin-3-carbonsäure mit den entsprechenden wie zuvor beschrieben hergestellten oder kommerziell erhältlichen Aminen (1.1-1.5 Äquivalente) und 4-Methylmorpholin (4-6 Äquivalente) unter den in der allgemeinen Arbeitsvorschrift 3 beschriebenen Reaktionsbedingungen umgesetzt wurde.

**Tabelle 8A:**

| **Beispiel** | **IUPAC-Name / Struktur (Ausbeute)** | **Analytische Daten** |
|---|---|---|
| **78A** | *rac*-tert.-Butyl-{2-(4-chlorphenyl)-2-[({8-[(2,6-difluorbenzyl)oxy]-2-methylimidazo[1,2-a]pyridin-3-yl}carbonyl)amino]ethyl}carbamat | LC-MS (Methode 2): Rₜ = 1.13 min |
| | | MS (ESpos): m/z = 571 (M+H)⁺ |
| | 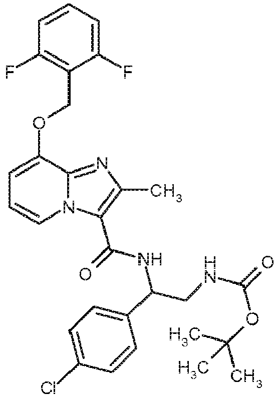 | ¹H-NMR (400 MHz, DMSO-d₆): |
| | | δ = 1.32 (s, 9H), 2.58 (s, 3H), 3.29-3.46 (m, 2H), 5.16 (q, 1H), 5.31 (s, 2H), 6.91 (t, 1H), 7.02 (d, 1H), 7.09 (t, 1H), 7.23 (t, 2H), 7.38-7.45 (m, 4H), 7.59 (quint, 1H), 8.21 (d, 1H), 8.56 (d, 1H). |
| | (88% d. Th.) | |
| **79A** | *rac*-tert.-Butyl-{2-[({8-[(2,6-difluorbenzyl)oxy]-2-methylimidazo[1,2-a]pyridin-3-yl}carbonyl)amino]-2-(4-fluorphenyl)ethyl} carbamat | LC-MS (Methode 2): Rₜ = 1.03 min |
| | | MS (ESpos): m/z = 555 (M+H)⁺ |
| | 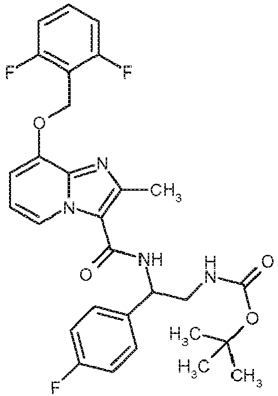 | ¹H-NMR (400 MHz, DMSO-d₆): |
| | | δ = 1.32 (s, 9H), 2.58 (s, 3H), 3.29-3.46 (m, 2H), 5.18 (q, 1H), 5.31 (s, 2H), 6.91 (t, 1H), 7.02 (d, 1H), 7.08 (t, 1H), 7.13-7.26 (m, 4H), 7.43 (dd, 2H), 7.59 (quint, 1H), 8.19 (d, 1H), 8.56 (d, 1H). |
| | (99% d. Th.) | |
| **80A** | *rac*-tert.-Butyl-{2-[({8-[(2,6-difluorbenzyl)oxy]-2-methylimidazo[1,2-a]pyridin-3-yl}carbonyl)amino]-2-phenylethyl} carbamat | LC-MS (Methode 2): Rₜ = 1.06 min |
| | | MS (ESpos): m/z = 537 (M+H)⁺ |
| | 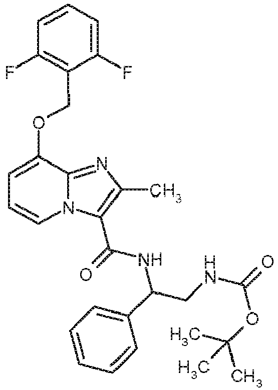 | ¹H-NMR (400 MHz, DMSO-d₆): |
| | | δ = 1.32 (s, 9H), 2.59 (s, 3H), 3.28-3.46 (m, 2H), 5.19 (q, 1H), 5.31 (s, 2H), 6.91 (t, 1H), 7.01 (d, 1H), 7.08 (t, 1H), 7.20-7.28 (m, 3H), 7.38 (t, 2H), 7.40 (d, 2H), 7.59 (quint, 1H), 8.19 (d, 1H), 8.57 (d, 1H). |
| | (63% d. Th.) | |
| **81A** | *ent*-tert.-Butyl-{2-[({8-[(2,6-difluorbenzyl)oxy]-2-methylimidazo[1,2-a]pyridin-3-yl}carbonyl)amino]-2-(2,4-difluorphenyl)ethyl}carbamat | LC-MS (Methode 2): Rₜ = 1.07 min |
| | | MS (ESpos): m/z = 573 (M+H)⁺ |
| | 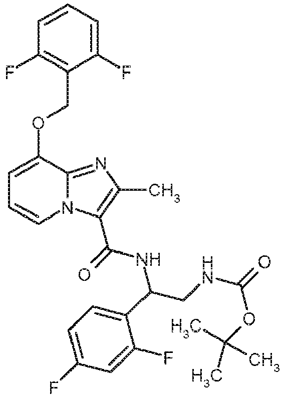 | |
| | (64% d. Th.) | |
| **82A** | *rac*-tert.-Butyl-[2-({[8-(cyclohexylmethoxy)-2-methylimidazo[1,2-a]pyridin-3-yl]carbonyl}amino)-2-phenylethyl]carbamat-Trifluoracetat | LC-MS (Methode 2): Rₜ = 1.09 min |
| | | MS (ESpos): m/z = 507 (M-TFA+H)⁺ |
| | 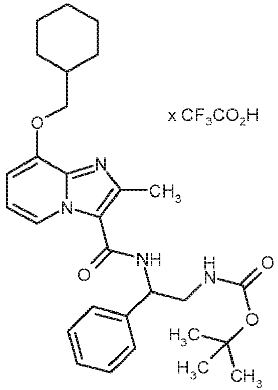 | |
| | (41% d. Th.) | |
| **83A** | *rac*-tert.-Butyl-[2-(4-chlorphenyl)-2-({[8-(cyclohexylmethoxy)-2-methylimidazo[1,2-a]pyridin-3-yl]carbonyl}amino)ethyl]carbamat-Trifluoracetat | LC-MS (Methode 2): Rₜ = 1.17 min |
| | | MS (ESpos): m/z = 541 (M-TFA+H)⁺ |
| | 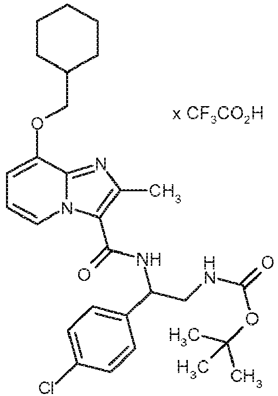 | |
| | (67% d. Th.) | |
| **84A** | *rac*-tert.-Butyl-[2-({[8-(cyclohexylmethoxy)-2-methylimidazo[1,2,a]pyridin-3-yl]carbonyl}amino)-2-(3,4-difluorphenyl)ethyl]carbamat-Trifluoracetat | LC-MS (Methode 2): Rₜ = 1.17 min |
| | | MS (ESpos): m/z = 543 (M-TFA+H)⁺ |
| | 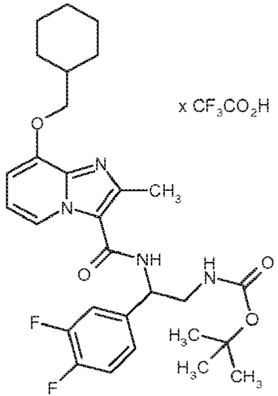 | |
| | (58% d. Th.) | |
| **85A** | *ent*-tert.-Butyl-[2-({[8-(cyclohexylmethoxy)-2-methylimidazo[1,2-a]pyridin-3-yl]carbonyl}amino)-2-(2,4-difluorphenyl)ethyl]carbamat-Trifluoracetat | LC-MS (Methode 2): Rₜ = 1.17 min |
| | | MS (ESpos): m/z = 543 (M-TFA+H)⁺ |
| | 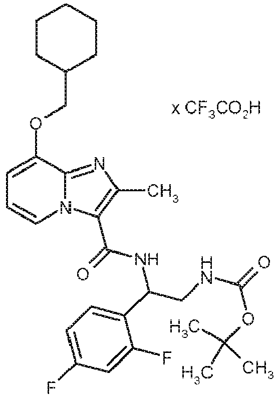 | |
| | (42% d. Th.) | |
| **86A** | *rac*-tert.-Butyl-{2-[({8-[(2,6-difluorbenzyl)oxy]-2-methylimidazo[1,2-a]pyridin-3-yl}carbonyl)amino]propyl} carbamat | LC-MS (Methode 1): Rₜ = 1.04 min |
| | | MS (ESpos): m/z = 475 (M+H)⁺ |
| | 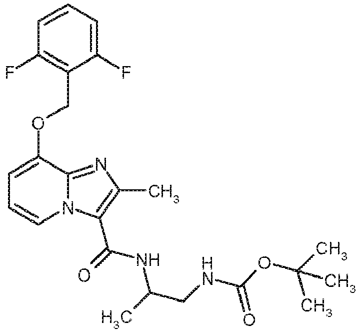 | ¹H-NMR (400 MHz, DMSO-d₆): |
| | | δ = 1.14 (d, 3H), 1.36 (s, 9H), 2.50 (s, 3H), 3.04-3.20 (m, 2H), 4.06-4.16 (m, 1H), 5.30 (s, 2H), 6.89-7.03 (m, 3H), 7.22 (t, 2H), 7.56-7.64 (m, 2H), 8.61 (d, 1H). |
| | (57% d. Th.) | |
| **87A** | *rac*-tert.-Butyl-{2-[({6-chlor-8-[(2,6-difluorbenzyl)oxy]-2-methylimidazo[1,2-a]pyridin-3-yl}carbonyl)amino]propyl}carbamat-Trifluoracetat | LC-MS (Methode 2): Rₜ = 1.11 min |
| | | MS (ESpos): m/z = 509 (M-TFA+H)⁺ |
| | | ¹H-NMR (400 MHz, DMSO-d₆): |
| | 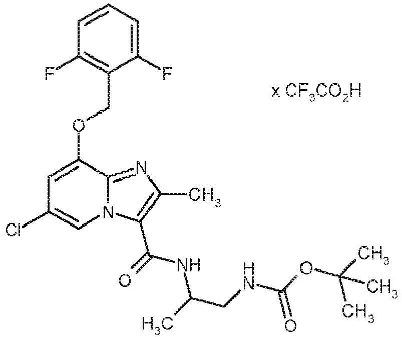 | δ = 1.15 (d, 3H), 1.37 (s, 9H), 2.50 (s, 3H), 3.05-3.19 (m, 2H), 4.05-4.17 (m, 1H), 5.38 (s, 2H), 6.98 (t, 1H), 7.22 (t, 2H), 7.31 (s, 1H), 7.60 (quintett, 1H), 7.80 (d, 1H), 8.72 (s, 1H). |
| | (74% d. Th., Reinheit ca. 80%) | |
| **88A** | *rac*-tert.-Butyl-{2-[({8-[(2,6-difluorbenzyl)oxy]-2,6-dimethylimidazo[1,2-a]pyridin-3-yl}carbonyl)amino]propyl carbamat | LC-MS (Methode 2): Rₜ = 0.95 min |
| | | MS (ESpos): m/z = 489 (M+H)⁺ |
| | | ¹H-NMR (400 MHz, DMSO-d₆): |
| | 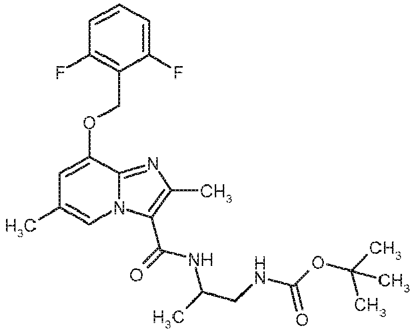 | δ = 1.13 (d, 3H), 1.37 (s, 9H), 2.31 (s, 3H), 2.46 (s, 3H), 3.01-3.19 (m, 2H), 4.05-4.17 (m, 1H), 5.29 (s, 2H), 6.90 (s, 1H), 6.96 (t, 1H), 7.22 (t, 2H), 7.53-7.63 (m, 2H), 8.43 (s, 1H). |
| | (78% d. Th., Reinheit ca. 93%) | |
| **89A** | *rac*-tert.-Butyl-{1-[({6-chlor-8-[(2,6-(difluorbenzyl)oxy]-2-methylimidazo[1,2-a]pyridin-3-yl}carbonyl)amino]propan-2-yl}carbamat-Trifluoracetat ¹⁾ | LC-MS (Methode 2): Rₜ = 1.15 min |
| | | MS (ESpos): m/z = 509 (M-TFA+H)⁺ |
| | | ¹H-NMR (400 MHz, DMSO-d₆): |
| | 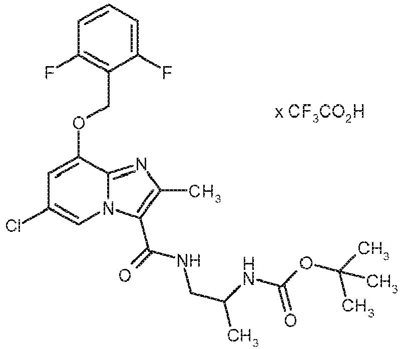 | δ = 1.07 (d, 3H), 1.36 (s, 9H), 2.50 (s, 3H), 3.20-3.37 (m, 2H), 3.69-3.82 (m, 1H), 5.37 (s, 2H), 6.69 (d, 1H), 7.24 (t, 2H), 7.33 (s, 1H), 7.61 (quintett, 1H), 8.02 (br s, 1 H), 8.78 (s, 1H). |
| | (74% d. Th.) | |
| **90A** | tert.-Butyl-{2-[({6-chlor-8-[(2,6-difluorbenzyl)oxy]-2-methylimidazo[1,2-a]pyridin-3-yl}carbonyl)amino]-2-methylpropyl}carbamat-Trifluoracetat ¹⁾ | LC-MS (Methode 2): Rₜ = 1.23 min |
| | | MS (ESpos): m/z = 523 (M-TFA+H)⁺ |
| | | ¹H-NMR (400 MHz, DMSO-d₆): |
| | 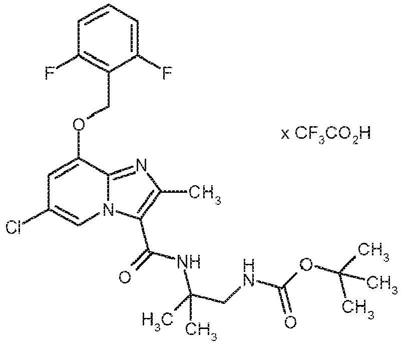 | δ = 1.31 (s, 6H), 1.34 (s, 9H), 2.48 (s, 3H), 3.31 (d, 2H), 5.38 (s, 2H), 7.08 (t, 1H), 7.24 (t, 2H), 7.33 (br s, 1H), 7.58-7.67 (m, 2H), 8.69 (s, 1H). |
| | (72% d. Th.) | |
| **91A** | *rac*-tert.-Butyl-{1-[({8-[(2,6-difluorbenzyl)oxy]-2,6-dimethylimidazo[1,2-a]pyridin-3-yl}carbonyl)amino]propan-2-yl}carbamat-Trifluoracetat ¹⁾ | LC-MS (Methode 2): Rₜ = 0.95 min |
| | | MS (ESpos): m/z = 489 (M-TFA+H)⁺ |
| | | ¹H-NMR (400 MHz, DMSO-d₆): |
| | 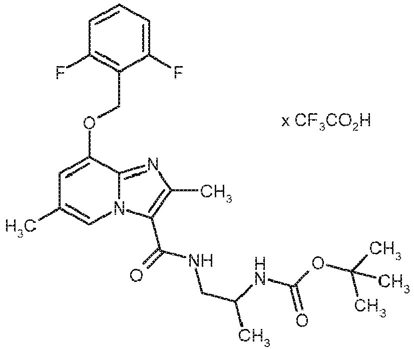 | δ = 1.08 (d, 3H), 1.36 (s, 9H), 2.39 (s, 3H), 2.50 (s, 3H), 3.20-3.38 (m, 2H), 3.69-3.82 (m, 1H), 5.39 (s, 2H), 6.70 (d, 1H), 7.24 (t, 2H), 7.41 (br s, 1H), 7.61 (quintett, 1H), 8.28 (br s, 1H), 8.53 (s, 1H). |
| | (70% d. Th.) | |
| **92A** | tert.-Butyl-{2-[({8-[(2,6-difluorbenzyl)oxy]-2,6-dimethylimidazo[1,2-a]pyridin-3-yl}carbonyl)amino]-2-methylpropyl}carbamat-Trifluoracetat ¹⁾ | LC-MS (Methode 2): Rₜ = 1.00 min |
| | | MS (ESpos): m/z = 503 (M-TFA+H)⁺ |
| | | ¹H-NMR (400 MHz, DMSO-d₆): |
| | 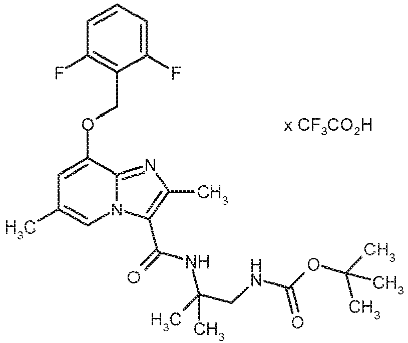 | δ = 1.32 (s, 6H), 1.34 (s, 9H), 2.42 (s, 3H), 2.50 (s, 3H), 3.32 (d, 2H), 5.41 (s, 2H), 7.09 (t, 1H), 7.24 (t, 2H), 7.50 (br s, 1H), 7.61 (quintett, 1H), 7.88 (br s, 1H), 8.49 (s, 1H). |
| | (66% d. Th.) | |
| **93A** | tert.-Butyl-(1-{2-[({6-chlor-8-[(2,6-difluorbenzyl)oxy]-2-methylimidazo[1,2-a]pyridin-3-yl}carbonyl)amino]ethyl}cyclohexyl)carbamat-Trifluoracetat | LC-MS (Methode 2): Rₜ = 1.36 min |
| | | MS (ESpos): m/z = 577 (M-TFA+H)⁺ |
| | 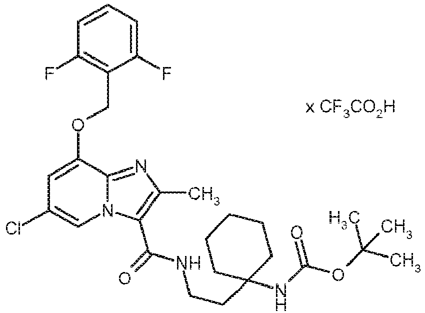 | |
| | (91% d. Th.) | |
| **94A** | *rac*-tert.-Butyl-{1-[({8-[(2,6-difluorbenzyl)oxy]-2-methylimidazo[1,2-a]pyridin-3-yl}carbonyl)amino]propan-2-yl}carbamat-Trifluoracetat | LC-MS (Methode 7): Rₜ = 0.88 min |
| | | MS (ESpos): m/z = 475 (M-TFA+H)⁺ |
| | 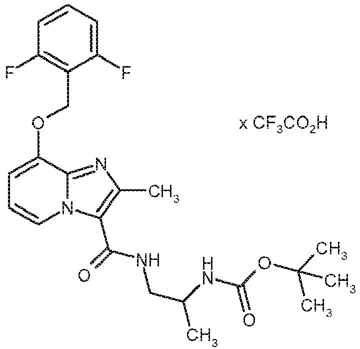 | |
| | (71% d. Th.) | |
| **95A** | *ent*-tert.-Butyl-{(2R)-1-[({6-chlor-8-[(2,6-difluorbenzyl)oxy]-2-methylimidazo[1,2-a]pyridin-3-yl}carbonyl)amino]butan-2-yl}carbamat-Trifluoracetat | LC-MS (Methode 2): Rₜ = 1.17 min |
| | | MS (ESpos): m/z = 523 (M-TFA+H)⁺ |
| | 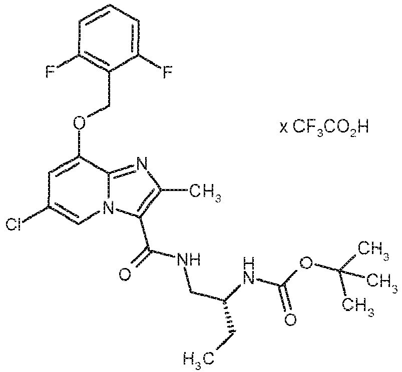 | |
| | (81% d. Th.) | |
| **96A** | tert.-Butyl-{3-[({6-chlor-8-[(2,6-difluorbenzyl)oxy]-2-methylimidzo[1,2-a]pyridin-3-yl}carbonyl)amino]propyl}carbamat-Trifluoracetat | LC-MS (Methode 2): Rₜ = 1.10 min |
| | | MS (ESpos): m/z = 509 (M-TFA+H)⁺ |
| | 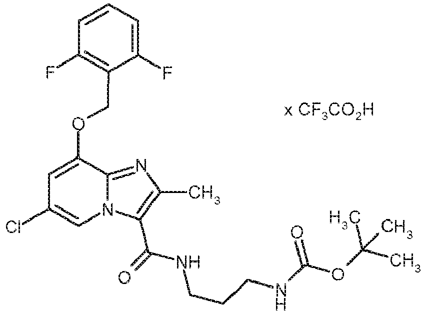 | |
| | (80% d. Th.) | |
| **97A** | tert.-Butyl-{2-[({chlor-8-[(2,6-difluorbenzyl)oxy]-2-methylimidazo[1,2-a]pyridin-3-yl}carbonyl)amino]ethyl}carbamat-Trifluoracetat | LC-MS (Methode 7): Rₜ = 1.11 min |
| | | MS (ESpos): m/z = 495 (M-TFA+H)⁺ |
| | 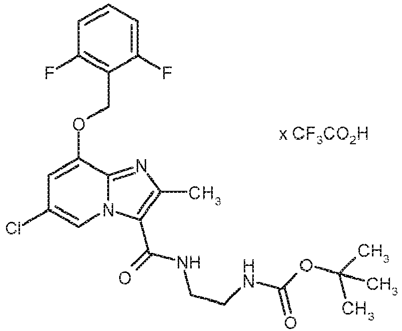 | |
| | (76% d. Th.) | |
| **98A** | tert.-Butyl-(1{[({8-[(2,6-difluorbenzyl)oxy]-2-methylimidazo[1,2-a]pyridin-3-yl}carbonyl)amino]methyl}cyclopropyl)carbamat-Trifluoracetat | LC-MS (Methode 7): Rₜ = 0.90 min |
| | | MS (ESpos): m/z = 487 (M-TFA+H)⁺ |
| | 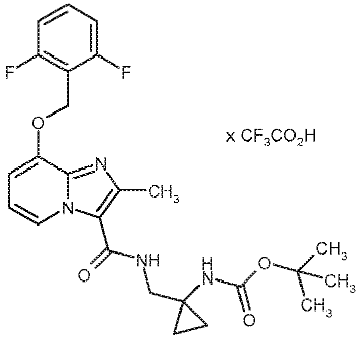 | |
| | (80% d. Th.) | |
| **99A** | tert.-Butyl-(1-{[({6-chlor-8-[(2,6-difluor-benzyl)oxy]-2-methylimidazo[1,2-a]pyridin-3-yl}carbonyl)amino]methyl}cyclopropyl)carbamat-Trifluoracetat | LC-MS (Methode 7): Rₜ = 1.20 min |
| | | MS (ESpos): m/z = 521 (M-TFA+H)⁺ |
| | 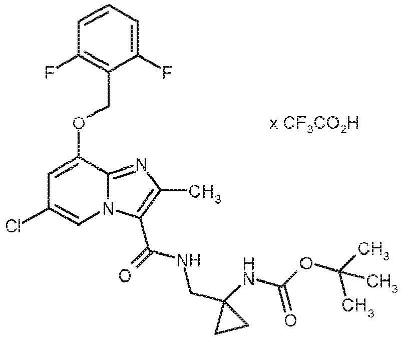 | |
| | (66% d. Th.) | |
| **100A** | tert.-Butyl-(1-{[({8-[(2,6-difluorbenzyl)oxy]-2,6-dimethylimidazo[1,2-a]pyridin-3-yl}carbonyl)amino]methyl}cyclopropyl)carbamat-Trifluoracetat | LC-MS (Methode 7): Rₜ = 0.93 min |
| | | MS (ESpos): m/z = 501 (M-TFA+H)⁺ |
| | 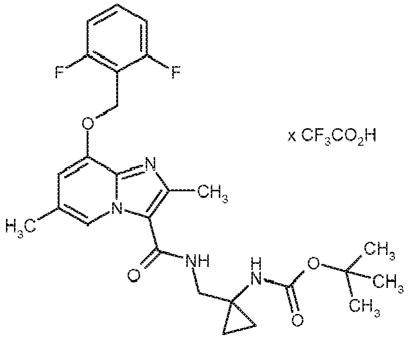 | |
| | (86% d. Th.) | |
| **101A** | tert.-Butyl-(1-{[({8-[(2,6-difluorbenzyl)oxy]-2-methylimidazo[1,2-a]pyridin-3-yl}carbonyl)amino]methyl}cyclopentyl)carbamat-Trifluoracetat | LC-MS (Methode 7): Rₜ = 1.04 min |
| | | MS (ESpos): m/z = 515 (M-TFA+H)⁺ |
| | 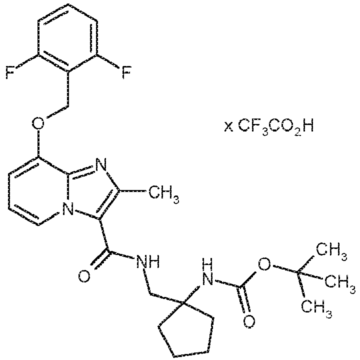 | |
| | (73% d. Th.) | |
| **102A** | tert.-Butyl-{1-{[({6-chlor-8-[(2,6-difluorbenzyl)oxy]-2-methylimidazo[1,2-a]pyridin-3-yl}carbonyl)amino]methyl}cyclopentyl)carbamat-Trifluoracetat | LC-MS (Methode 7): Rₜ = 1.36 min |
| | | MS (ESpos): m/z = 549 (M-TFA+H)⁺ |
| | 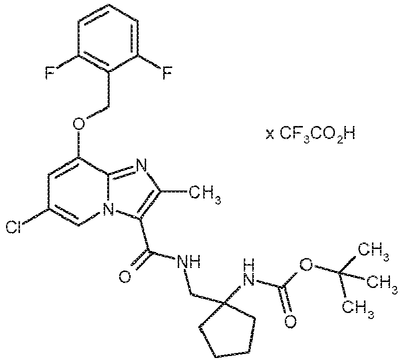 | |
| | (85% d. Th.) | |
| **103A** | *rac*-tert.-Butyl-3-[({8-[(2,6-difluorbenzyl)oxy]-2-methylimidazo[1,2-a]pyridin-3-yl}carbonyl)amino]indolin-1-carboxylat | LC-MS (Methode 2): Rₜ = 1.16 min |
| | | MS (ESpos): m/z = 535 (M+H)⁺ |
| | | ¹H-NMR (400 MHz, DMSO-d₆): |
| | 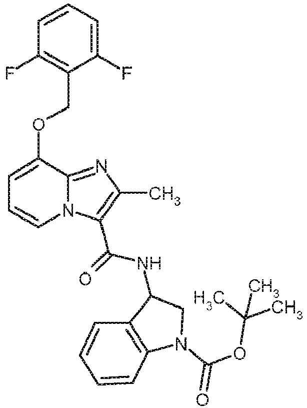 | δ = 1.52 (s, 9H), 2.49 (s, 3H), 3.69 (dd, 1H), 4.29 (t, 1H), 5.30 (s, 2H), 5.64-5.73 (m, 1H), 6.94-7.06 (m, 3H), 7.19-7.30 (m, 3H), 7.40 (d, 1H), 7.60 (quintett, 1H), 7.79 (br s, 1H), 8.51 (d, 1H), 8.67 (d, 1H). |
| | (83% d. Th.) | |
| **104A** | *rac*-tert.-Butyl-3-[({6-chlor-8-[(2,6-difluorbenzyl)oxy]-2-methylimidazo[1,2-a]pyridin-3-yl}carbonyl)amino]indolin-1-carboxylat ²⁾ | LC-MS (Methode 2): Rₜ = 1.39 min |
| | | MS (ESpos): m/z = 569 (M+H)⁺ |
| | 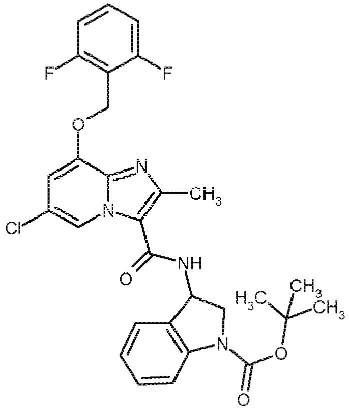 | |
| | (87% d. Th.) | |
| **105A** | *rac*-tert.-Butyl-[({8-[(2,6-difluorbenzyl)oxy]-2-methylimidazo[1,2-a]pyridin-3-yl}carbonyl)amino]-3,4-dihydrochinolin-1(2H)-carboxylat ³⁾ | LC-MS (Methode 2): Rₜ = 1.14 min |
| | | MS (ESpos): m/z = 549 (M+H)⁺ |
| | | ¹H-NMR (400 MHz, DMSO-d₆): |
| | 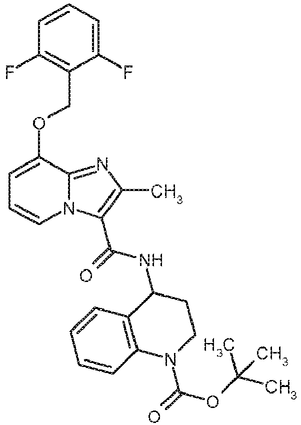 | δ = 1.49 (s, 9H), 1.97-2.08 (m, 1H), 2.12-2.21 (m, 1H), 2.50 (s, 3H), 3.72-3.79 (m, 1H), 3.82-3.90 (m, 1H), 5.27 (q, 1H), 5.31 (s, 2H), 6.94-7.11 (m, 3H), 7.18-7.28 (m, 3H), 7.36 (d, 1H), 7.59 (quintett, 1H), 7.62 (d, 1H), 8.42 (d, 1H), 8.60 (d, 1H). |
| | (75% d. Th.) | |

| | | |
|---|---|---|
| ¹⁾ Aufarbeitung: Das Reaktionsrohgemisch wurde eingeengt und mittels präparativer HPLC gereinigt (RP18 Säule, Laufmittel: Acetonitril/Wasser-Gradient unter Zusatz von 0.1% TFA). ²⁾ Aufarbeitung: Der Niederschlag wurde mittels Kieselgel-Chromatographie gereinigt (Laufmittel: Dichlormethan:Methanol 200:1). ³⁾ Aufarbeitung: Der Niederschlag wurde mittels Kieselgel-Chromatographie gereinigt (Laufmittel: Cyclohexan:Essigsäureethylester 2:1). | | |

### Beispiel 106A

### ent-tert-Butyl-{2-[({8-[(2,6-difluorbenzyl)oxy]-6-fluor-2-methylimidazo[1,2-a]pyridin-3-yl}carbonyl)amino]-2-(3,4-difluorphenyl)ethyl}carbamat

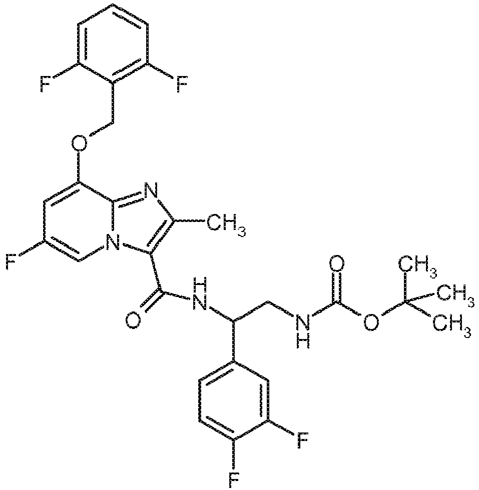

90 mg 8-[(2,6-Difluorbenzyl)oxy]-6-fluor-2-methylimidazo[1,2-a]pyridin-3-carbonsäure (Beispiel 11A; 0.268 mmol, 1 Äquivalent), 132 mg *O*-(7-Azabenzotriazol-1-yl)-*N*,*N*,*N'N'-*tetramethyluroniumhexafluorphosphat (0.348 mmol, 1.4 Äquivalente) und 0.132 ml *N*,*N-*Diisopropylethylamin (0.803 mmol, 3 Äquivalente) wurden in 0.85 ml DMF vorgelegt, bei RT mit 102 mg *ent*-tert.-Butyl-[2-amino-2-(3,4-difluorphenyl)ethyl]carbamat (Beispiel 60A) versetzt und über Nacht bei RT gerührt. Die Reaktionslösung wurde mit Wasser versetzt, noch 30 min gerührt und der entstandene Niederschlag abfiltriert und mit Wasser, wenig Acetonitril und Methanol gewaschen. Es wurden 148 mg (85% d. Th.) der Titelverbindung erhalten.
LC-MS (Methode 2): Rₜ = 1.24 min
MS (ESpos): m/z = 591.4 (M+H)⁺
¹H-NMR (400 MHz, DMSO-d₆): δ = 1.34 (s, 9H), 2.58 (s, 3H; teilweise verdeckt durch DMSO-Signal), 3.35-3.45 (m, 2H; teilweise überlagert durch H₂O-Signal), 5.15 (q, 1H), 5.31 (s, 2H), 7.10 (t, 1H), 7.19-7.27 (m, 4H), 7.36-7.50 (m, 2H), 7.59 (quint, 1H), 8.21 (d, 1H), 8.62 (d, 1H).

Die in Tabelle 9A gezeigten Beispiele wurden hergestellt, indem Beispiele 3A, 16A, 21A, 25A bzw. 28A mit den entsprechenden, kommerziell erhältlichen oder entsprechend der beschriebenen Methoden hergestellen Aminen und *N,N-*Diisopropylethylamin unter den in der repräsentativen Arbeitsvorschrift 3 beschriebenen Reaktionsbedingungen umgesetzt wurden.

**Tabelle 9A:**

| **Beispiel** | **IUPAC-Name / Struktur (Ausbeute)** | **Analytische Daten** |
|---|---|---|
| 107A | *ent*-tert.-Butyl-{2-[({8-[(2,6difluorbenzyl)oxy]-2,6-dimethylimidazo[1,2-a]pyridin-3-yl}carbonyl)amino]-2-(3,4-difluorphenyl)ethyl}carbamat | LC-MS (Methode 2): Rₜ = 1.13 min |
| | | MS (ESpos): m/z = 587 (M+H)⁺ |
| | | ¹H-NMR (400 MHz, DMSO-d₆): |
| | 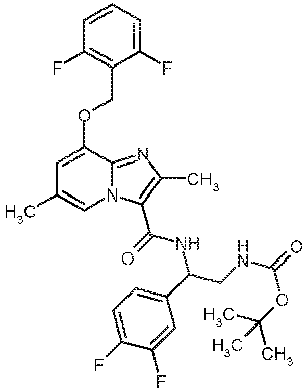 | δ = 1.32 (s, 9H), 2.28 (s, 3H); 2.52 (s, 3H), 3.33-3.46 (m, 2H; überlagert durch H₂O-Signal), 5.13 (q, 1H), 5.28 (s, 2H), 6.92 (s, 1H), 7.08 (t, 1H), 7.20-7.26 (m, 3H), 7.36-7.51 (m, 2H); 7.60 (quint, 1H), 8.19 (d, 1 H); 8.38 (s, 1H). |
| | (94% d. Th.) | |
| **108A** | *ent*-tert.-Butyl-{2-[({2-cyclopropyl-8-[(2,6-difluorbenzyl)oxy]imidazo[1,2-a]pyridin-3-yl}carbonyl)amino]-2-(3,4-difluorphenyl)ethyl}carbamat | LC-MS (Methode 2): Rₜ = 1.23 min |
| | | MS (ESpos): m/z = 599.4 (M+H)⁺ |
| | | ¹H-NMR (400 MHz, DMSO-d₆): |
| | 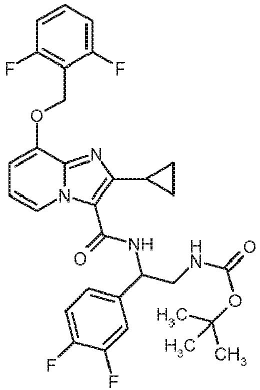 | δ = 0.9 -1.05 (m, 4 H); 1.35 (s, 9H), 2.40 - 2.48 (m, 1 H); 3.31-3.48 (m, 2H; überlagert durch H₂O-Signal), 5.13 (q, 1H), 5.30 (s, 2H), 6.89 (t, 1H), 7.01 (d, 1H), 7.10 (t, 1H), 7.21 (t, 2H), 7.22 (m, 1 H); 7.35-7.48 (m, 2H), 7.59 (quint, 1H), 8.41 (d, 1 H); 8.51 (d, 1H). |
| | (79% d. Th.) | |
| **109A** | *ent*-tert.-Butyl-{2-[({8-[(2,6-difluorbenzyl)oxy]imidazo[1,2,a]pyridin-3-yl}carbonyl)amino]-2-(3,4-difluorphenyl)ethyl} carbamat | LC-MS (Methode 2): Rₜ = 1.13 min |
| | | MS (ESpos): m/z = 559.4 (M+H)⁺ |
| | | ¹H-NMR (400 MHz, DMSO-d₆): |
| | 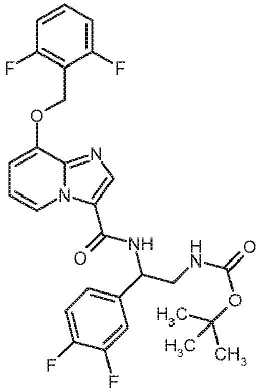 | δ = 1.30 (s, 9H), 3.29-3.35 (m, 2H, überlagert durch H₂O-Signal), 5.13 (q, 1H), 5.31 (s, 2H), 7.00 - 7.12 (m, 3 H); 7.21 (m, 1 H); 7.23 (t, 2H), 7.35-7.48 (m, 2H), 7.59 (quint, 1H), 8.30 (s, 1 H); 8.71 (d, 1H), 8.99 (d, 1H). |
| | (64 % d. Th.) | |
| **110A** | *rac-*tert.-Butyl-{2-[({8-[(2,6-difluorbenzyl)oxy]-2-methylimidazo[1,2-a]pyridin-3-yl}carbonyl)amino]-3-ethoxypropyl}carbamat | LC-MS (Methode 2): Rₜ = 0.93 min |
| | | MS (ESpos): m/z = 519.4 (M+H)⁺ |
| | | ¹H-NMR (400 MHz, DMSO-d₆): |
| | 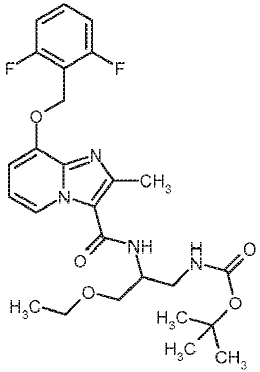 | δ = 1.10 (t, 3H); 1.35 (s, 9H), 2.55 (s, 3H; verdeckt durch DMSO-Signal), 3.23 (t, 2H), 3.45 - 3.52 (m, 4H); 4.20 (m, 1H); 5.31 (s, 2H), 6.90 - 7.08 (m, 3 H); 7.20 (t, 2H); 7.52 - 7.61 (m, 2H); 8.62 (d, 1H). |
| | (65% d. Th.) | |
| **111A** | *rac*-tert.-Butyl-{2-[({6-chlor-8-[(2,6-difluorbenzyl)oxy]-2-methylimidazo[1,2-a]pyridin-3-yl}carbonyl)amino]-3-ethoxypropyl}carbamat | LC-MS (Methode 2): Rₜ = 1.18 min |
| | | MS (ESpos): m/z = 553.4 (M+H)⁺ |
| | | ¹H-NMR (400 MHz, DMSO-d₆): |
| | 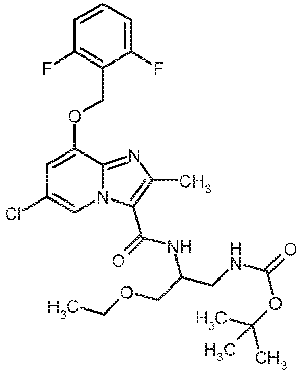 | δ = 1.10 (t, 3H); 1.37 (s, 9H), 2.55 (s, 3H; überlagert durch DMSO-Signal), 3.18-3.26 (m, 2H), 3.47 - 3.51 (m, 4H); 4.21 (q, 1H), 5.32 (s, 2H), 6.92 (t, 1H), 7.20 (s, 1H); 7.23 (t, 2H), 7.59 (quint, 1H), 7.61 (d, 1H); 8.70 (s, 1 H). |
| | (86% d. Th.) | |
| **112A** | *rac-tert*.-Butyl-{2-[({8-[(2,6-difluorbenzyl)oxy]-2-methylimidazo[1,2-a]pyridin-3-yl}carbonyl)amino]-3-(4-fluorphenoxy)propyl}carbamat | LC-MS (Methode 2): Rₜ = 1.09 min |
| | | MS (ESpos): m/z = 585.2 (M+H)⁺ |
| | | ¹H-NMR (400 MHz, DMSO-d₆): |
| | 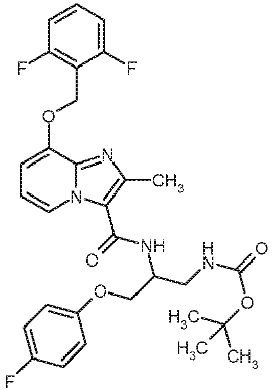 | δ = 1.32 (s, 9H), 2.55 (s, 3H; verdeckt durch DMSO-Signal), 3.30 (überlagert durch Wasser-Signal; 2H), 4.04 (q, 2H), 4.40 (m, 1H), 5.30 (s, 2H), 6.90 - 7.05 (m, 4H), 7.09 (t, 1H); 7.11 (t, 2H), 7.21 (t, 2H), 7.60 (q, 1H), 7.75 (d, 1H), 8.60 (d, 1H). |
| | (14% d. Th.) | |
| **113A** | *rac*-*tert.*-Butyl-{2-[{8-[(2,6-difluorbenzyl)oxy]-2-methylimidazo[1,2-a]pyridin-3-yl}carbonyl)amino]-3-phenoxypropyl}carbamat | LC-MS (Methode 2): Rₜ = 1.09 min |
| | | MS (ESpos): m/z = 557.2 (M+H)⁺ |
| | | ¹H-NMR (400 MHz, DMSO-d₆): |
| | 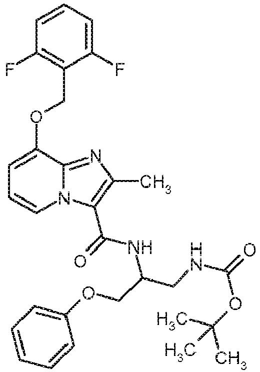 | δ = 1.34 (s, 9H), 2.55 (s, 3H; verdeckt durch DMSO-Signal), 3.30 (überlagert durch Wasser-Signal; 2H), 4.08 (m, 2H), 4.42 (m, 1H), 5.30 (s, 2H), 6.90 - 7.05 (m, 5H), 7.09 (t, 1H); 7.21 (t, 2 H), 7.27 (t, 2H), 7.58 (q, 1H), 7.72 (d, 1H), 8.60 (d, 1H). |
| | (41% d. Th.) | |
| **114A** | *ent-tert*.-Butyl-(3*S*)-3-[({8-[(2,6-difluorbenzyl)oxy]-2-methylimidazo[1,2,a]pyridin-3-yl}carbonyl)amino]piperidin-1-carboxylat | LC-MS (Methode 2): Rₜ = 0.95 min |
| | | MS (ESpos): m/z = 501.4 (M+H)⁺ |
| | | ¹H-NMR (400 MHz, DMSO-d₆): |
| | 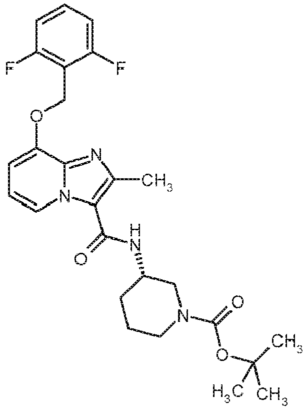 | δ = 1.37 (s, 9H), 1.40 - 1.48 (m, 1H); 1.60 - 1.70 (m, 2H); 1.86 - 1.93 (m, 1 H); 2.55 (s, 3H; überlagert durch DMSO-Signal); 2.85 - 3.10 (m, 2H); 3.51- 3.91 (m, 3H); 5.30 (s, 2H); 6.92 (t, 1H); 7.05 (d, 1H); 7.23 (t, 2H); 7.59 (quint, 1H), 7.71 (br. s, 1H); 8.60 (d, 1H). |
| | (68% d. Th.) | |
| **115A** | *ent*-Benzyl-(3*S*)-3-[({8-[(2,6-difluorbenzyl)oxy]-2-methylimidazo[1,2-a]pyridin-3-yl}carbonyl)mino]pyrrolidin-1-carboxylat | LC-MS (Methode 2): Rt = 0.97 min |
| | | MS (ESpos): m/z = 521.3 (M+H)+ |
| | | ¹H-NMR (400 MHz, DMSO-d₆): |
| | 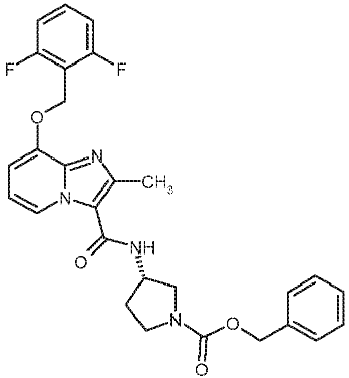 | δ = 1.90 - 2.02 (m, 1H); 2.11 - 2.21 (m, 1 H); 2.49 (s, 3H); 3.31 - 3.69 (m, 4 H); 4.50 (m, 1H); 5.08 (s, 2H); 5.30 (s, 2 H); 6.91 (t, 1H); 7.02 (d, 1 H); 7.23 (t, 2H); 7.25 - 7.37 (m, 5H); 7.59 (quint, 1H), 8.11 (d, 1H); 8.51 (d, 1H). |
| | (66% d. Th.) | |
| **116A** | *rac*-Benzyl-{2-[({8-[(2,6-difluorbenzyl)oxy]-2-methylimidazo[1,2-a]pyridin-3-yl}carbonyl)amino]-3-isopropoxypropyl}carbamat | LC-MS (Methode 1): Rₜ = 1.21 min |
| | | MS (ESpos): m/z = 567.1 (M+H)⁺ |
| | | ¹H-NMR (400 MHz, DMSO-d₆): |
| | 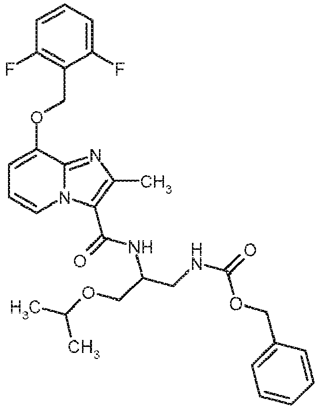 | δ = 1.08 (d, 6H), 2.55 (s, 3H; verdeckt durch DMSO-Signal), 3.20 - 3.35 (m, 2H), 3.45 (q, 2H), 3.55 (q, 1 H), 4.20 (m, 1H), 5.00 (s, 2 H), 5.31 (s, 2H), 6.90 (t, 1H), 7.00 (d, 1 H), 7.20 - 7.30 (m, 7H), 7.38 (t, 1H), 7.50 (d, 1H), 7.60 (q, 1H), 8.60 (d, 1H). |
| | (42% d. Th.) | |
| **117A** | *ent*-Benzyl-{2-[({8-[(2,6-difluorbenzyl)oxy]-2-methylimidazo[1-a]pyridin-3-yl}carbonyl)amino]-1-(3,4-difluorphenyl)ethyl}carbamat | LC-MS (Methode 2): Rt = 1.08 min MS (ESpos): m/z = 607.3 (M+H)+ |
| | | ¹H-NMR (400 MHz, DMSO-d₆): |
| | 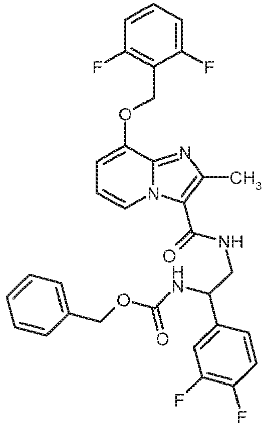 | δ = 2.30 (s, 3H); 3.46 - 3.63 (m, 2H); 4.88 - 5.05 (m, 3H); 5.30 (s, 2H); 6.90 (t, 1H); 7.00 (d, 1H); 7.15 - 7.48 (m, 10H); 7.58 (q, 1H); 7.88 (t, 1H); 7.96 (d, 1H); 8.51 (d, 1H). |
| | (55% d. Th.) | |
| | Ausgehend von Beispiel 64A | |

### Beispiel 118A

### ent-tert.-Butyl-(2-[({8-[(2,6-difluorbenzyl)oxy]-2-methylimidazo[1,2-a]pyridin-3-yl}carbonyl)amino]-2-(3,4-difluorphenyl)ethyl}carbamat

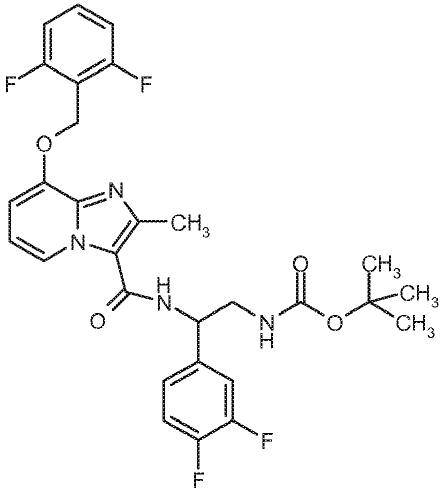

1.50 g 8-[(2,6-Diftuorbenzyl)oxy]-2-methylimidazo[1,2-a]pyridin-3-carbonsäure (4.71 mmol), 1.67 g (Benzotriazol-1-yloxy)bisdimethylaminomethyliumfluorborat (TBTU, 5.18 mmol) und 2.38 g 4-Methylmorpholin (23.5 mmol) wurden in 30 ml DMF vorgelegt. Bei RT wurden 1.48 g *ent-*tert.-Butyl-[2-amino-2-(3,4-difluorphenyl)ethyl]carbamat (Beispiel 60A, 5.42 mmol) zugesetzt und es wurde 2 h bei RT gerührt. Das Reaktionsgemisch wurde auf ca. 250 ml Wasser gegossen, der ausgefallene Feststoff ca. 30 min bei RTverrührt. Dann wurde abfiltriert, mit Wasser gewaschen und er Feststoff anschließend im Hochvakuum getrocknet. Das Rohprodukt wurde mittels Kieselgel-Chromatographie gereinigt (Laufmittel: Dichlormethan/Methanol = 100/1). Es wurden 2.18 g der Titelverbindung (81% d. Th) erhalten.
LC-MS (Methode 2): Rₜ = 1.12 min
MS (ESpos): m/z = 573 (M+H)⁺
¹H-NMR (400 MHz, DMSO-d₆): δ = 1.32 (s, 9H), 2.59 (s, 3H), 3.29-3.46 (m, 2H), 5.15 (q, 1H), 5.31 (s, 2H), 6.91 (t, 1H), 7.01 (d, 1H), 7.08 (t, 1H), 7.19-7.27 (m, 3H), 7.36-7.51 (m, 2H), 7.59 (quint, 1H), 8.21 (d, 1H), 8.56 (d, 1H).

### Beispiel 119A

### ent-tert.-Butyl-{2-[({6-chlor-8-[(2,6-difluorbenzyl)oxy]-2-methylimidazo[1,2-a]pyridin-3-yl}carbonyl)amino]propyl}carbamat (Enantiomer A)

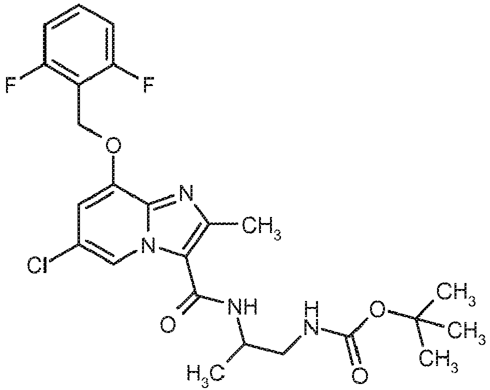

227 mg Beispiel 87A wurden an chiraler Phase in die Enantiomere getrennt [Säule: Daicel Chiralpak AD-H, 5 µm, 250 x 20 mm, Eluent: 70% iso-Hexan, 30% Ethanol, Fluß 20 ml/min; 25°C, Detektion: 230 nm].
Ausbeute Enantiomer A: 74 mg (>99% ee)
Enantiomer A: Rₜ = 4.66 min [Daicel Chiralpak AD-H, 5µm, 250 x 4.6 mm; Eluent: 50% iso-Hexan, 50% Ethanol; Fluss: 1.0 ml/min; 25°C; Detektion: 230 nm].

### Beispiel 120A

### ent-tert.-Butyl-{2-[({6-chlor-8-[(2,6-difluorbenzyl)oxy]-2-methylimidazo[1,2-a]pyridin-3-yl}carbonyl)amino]propyl}carbamat (Enantiomer B)

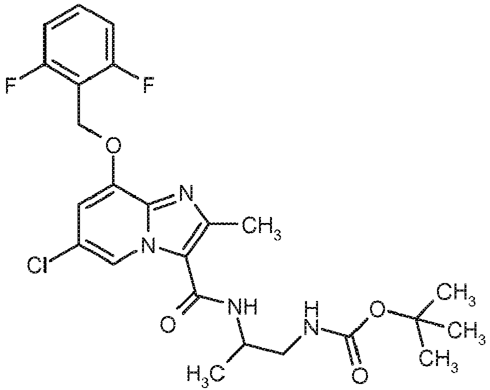

227 mg Beispiel 87A wurden an chiraler Phase in die Enantiomere getrennt [Säule: Daicel Chiralpak AD-H, 5 µm, 250 x 20 mm, Eluent: 70% iso-Hexan, 30% Ethanol, Fluß 20 ml/min; 25°C, Detektion: 230 nm].
Ausbeute Enantiomer B: 58 mg (>99% ee)
Enantiomer B: Rₜ = 5.90 min [Daicel Chiralpak AD-H, 5µm, 250 x 4.6 mm; Eluent: 50% iso-Hexan, 50% Ethanol; Fluss 1.0 ml/min; 25°C; Detektion: 230 nm].
¹H-NMR (400 MHz, DMSO-d₆): δ = 1.13 (d, 3H), 1.37 (s, 9H), 2.50 (s, 3H), 3.05-3.19 (m, 2H), 4.05-4.17 (m, 1H), 5.33 (s, 2H), 6.95 (t, 1H), 7.19 (s, 1H), 7.22 (t, 2H), 7.60 (quintett, 1H), 7.68 (d, 1H), 8.71 (s, 1H).

### Beispiel 121A

### ent-tert.-Butyl-{2-[({8-[(2,6-difluorbenzyl)oxy]-2,6-dimethylimidazo[1,2-a]pyridin-3-yl}carbonyl)amino]propyl}carbamat (Enantiomer A)

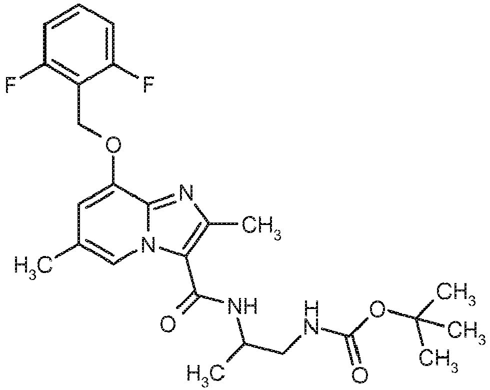

215 mg Beispiel 88A wurden an chiraler Phase in die Enantiomere getrennt [Säule: Daicel Chiralpak AD-H, 5 µm, 250 x 20 mm, Eluent: 70% iso-Hexan, 30% Ethanol + 0.2% Diethylamin, Fluß: 15 ml/min; 30°C, Detektion: 220 nm].

Enantiomer A (noch verunreinigt) wurde in 0.5 ml Methanol und 2.5 ml tert.-Butyl-Methylether gelöst und erneut gereinigt [Säule: Daicel Chiralpak IA, 5 µm, 250 x 20 mm, Eluent: 70% iso-Hexan, 30% Ethanol + 0.2% Diethylamin, Fluß 25 ml/min; 25°C, Detektion: 220 nm]
Ausbeute Enantiomer A: 48 mg (>99% ee)
Enantiomer A: Rₜ = 7.48 min [Daicel Chiralpak AD-H, 5µm, 250 x 4.6 mm; Eluent: 70% iso-Hexan, 30% Ethanol + 0.2% Diethylamin; Fluss 1.0 ml/min; 30°C; Detektion: 220 nm].

### Beispiel 122A

### ent-tert.-Butyl-{2-[({8-[(2,6-difluorbenzyl)oxy]-2,6-dimethylimidazo[1,2-a]pyridin-3-yl}carbonyl)amino]propyl}carbamat (Enantiomer B)

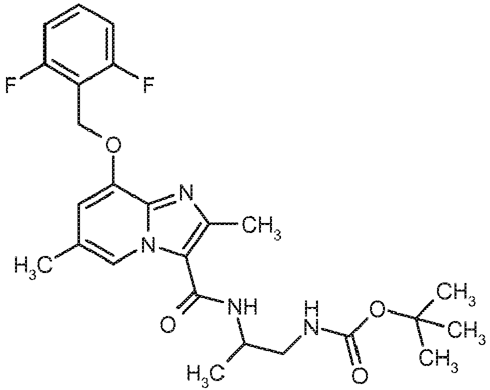

215 mg Beispiel 88A wurden an chiraler Phase in die Enantiomere getrennt [Säule: Daicel Chiralpak AD-H, 5 µm, 250 x 20 mm, Eluent: 70% iso-Hexan, 30% Ethanol + 0.2% Diethylamin, Fluß: 15 ml/min; 30°C, Detektion: 220 nm].
Ausbeute Enantiomer B: 67 mg (>99% ee)
Enantiomer B: 11.28 min [Daicel Chiralpak AD-H, 5µm, 250 x 4.6 mm; Eluent: 70% iso-Hexan, 30% Ethanol + 0.2% Diethylamin; Fluss 1.0 ml/min; 30°C; Detektion: 220 nm].

### Beispiel 123A

### ent-tert.-Butyl-{2-[({8-[(2,6difluorbenzyl)oxy]-2-methylimidazo[1,2,a]pyridin-3-yl}carbonyl)amino]-3-ethoxypropyl)carbamat (Enantiomer A)

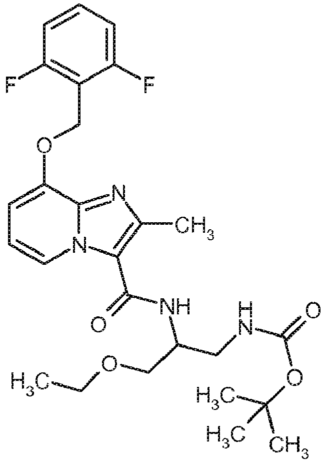

211 mg Beispiel 110A wurden an chiraler Phase in die Enantiomere getrennt [Säule: Daicel Chiralpak AD-H, 5 µm, 250 x 20 mm, Eluent: 50% iso-Hexan, 50% Ethanol, Fluß: 15 ml/min; 30°C, Detektion: 220 nm].
Ausbeute Enantiomer A: 84 mg (99% ee)
Enantiomer A: Rₜ = 7.56 min [Daicel Chiralpak AD-H, 5µm, 250 x 4.6 mm; Eluent: 50% iso-Hexan, 50% Ethanol; Fluss 1.0 ml/min; 30°C; Detektion: 220 nm].

### Beispiel 124A

### ent-tert.-Butyl-{2-[({8-[(2,6-difluorbenzyl)oxy]-2-methylimidazo[1,2-a]pyridin-3-yl}carbonyl)amino]-3-ethoxypropyl}carbamat (Enantiomer B)

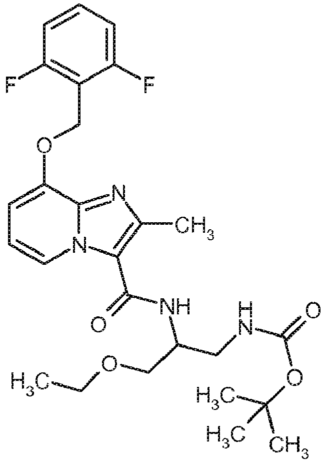

211 mg Beispiel 110A wurden an chiraler Phase in die Enantiomere getrennt [Säule: Daicel Chiralpak AD-H, 5 µm, 250 x 20 mm, Eluent: 50% iso-Hexan, 50% Ethanol, Fluß: 15 ml/min; 30°C, Detektion: 220 nm].
Ausbeute Enantiomer B: 82 mg (99% ee)
Enantiomer B: Rₜ = 9.71 min [Daicel Chiralpak AD-H, 5µm, 250 x 4.6 mm; Eluent: 50% iso-Hexan, 50% Ethanol; Fluss 1.0 ml/min; 30°C; Detektion: 220 nm].

### Beispiel 125A

### ent-tert.-Butyl-{2-[({6-chlor-8-[(2,6-difluorbenzyl)oxy]-2-methylimidazo[1,2-a]pyridin-3-yl}carbonyl)amino]-3-ethoxypropyl}carbamat (Enantiomer A)

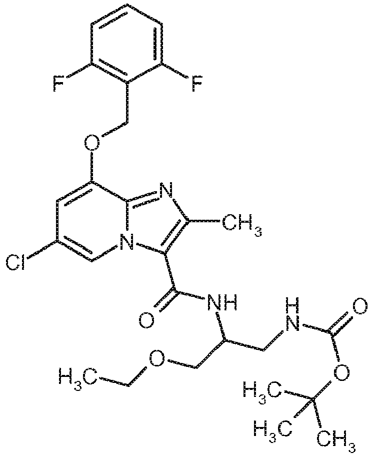

270 mg Beispiel 111 A wurden an chiraler Phase in die Enantiomere getrennt [Säule: Daicel Chiralpak AD-H, 5 µm, 250 x 20 mm, Eluent: 50% iso-Hexan, 50% Iso-Propanol, Fluß: 15 ml/min; 30°C, Detektion: 220 nm].
Ausbeute Enantiomer A: 114 mg (99% ee)
Enantiomer A: Rₜ = 4.66 min [Daicel Chiralpak AD-H, 5µm, 250 x 4.6 mm; Eluent: 50% iso-Hexan, 50% Iso-Propanol; Fluss 1.0 ml/min; 30°C; Detektion: 220 nm].

### Beispiel 126A

### ent-tert.-Butyl-{2-[({6-chlor-8-[(2,6-difluorbenzyl)oxy]-2-methylimidazo[1,2-a]pyridin-3-yl}carbonyl)amino]-3-ethoxypropyl}carbamat (Enantiomer B)

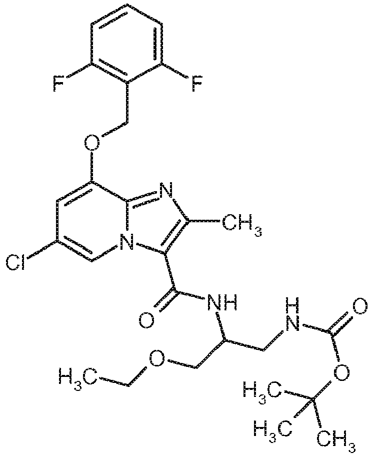

270 mg Beispiel 111 A wurden an chiraler Phase in die Enantiomere getrennt [Säule: Daicel Chiralpak AD-H, 5 µm, 250 x 20 mm, Eluent: 50% iso-Hexan, 50% Iso-Propanol, Fluß: 15 ml/min; 30°C, Detektion: 220 nm].
Ausbeute Enantiomer B: 118 mg (99% ee)
Enantiomer B: Rₜ = 6.75 min [Daicel Chiralpak AD-H, 5µm, 250 x 4.6 mm; Eluent: 50% iso-Hexan, 50% Ethanol; Fluss 1.0 ml/min; 30°C; Detektion: 220 nm].

### Beispiel 127A

### tert.-Butyl-3-[({8-[(2,6-difluorbenzyl)oxy]-2-methylimidazo[1,2-a]pyridin-3-yl)carbonyl)amino]-9-azabicyclo[3.3.1]nonan-9-carboxylat-Trifluoracetat

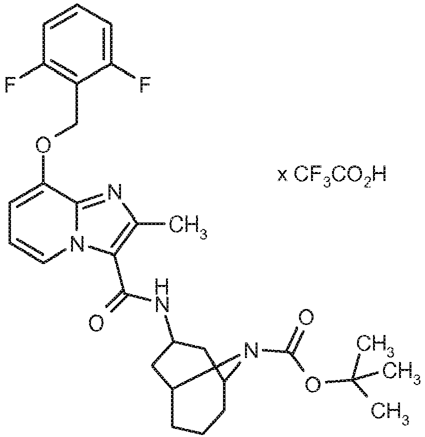

400 mg (1.07 mmol) (8-[(2,6-Difluorbenzyl)oxy]-2-methylimidazo[1,2-a]pyridin-3-carbonyl-chlorid-Hydrochlorid (Beispiel 22A) wurden in 40 ml trockenem THF vorgelegt. Anschließend wurden 309 mg (1.29 mmol) tert.-Butyl-3-amino-9-azabicyclo[3.3.1]nonan-9-carboxylat und 554 mg (4.29 mmol) *N*,*N*-Diisopropylethylamin zugegeben und es wurde 1 h bei RT gerührt. Die Reaktionslösung wurde eingeengt und mittels präparativer HPLC gereinigt (RP18 Säule, Laufmittel: Acetonitril/Wasser-Gradient unter Zusatz von 0.1% TFA). Es wurden 620 mg der Titelverbindung (88% d. Th.) erhalten.
LC-MS (Methode 2): Rₜ = 1.07 min
MS (ESpos): m/z = 541 (M-TFA+H)⁺
¹H-NMR (400 MHz, DMSO-d₆): δ = 1.40 (s, 9H), 1.62-1.79 (m, 7H), 1.81-2.06 (m, 3H), 2.50 (s, 3H), 4.22 (d, 2H), 4.78-4.90 (m, 1H), 5.40 (s, 2H), 7.25 (t, 3H), 7.41 (br s, 1H), 7.60 (quint, 1H), 8.09 (br s, 1H), 8.63 (d, 1H).

### Beispiel 128A

### 8-[(2,6-Diftuorbenzyl)oxy]-N-[(2R)-1-hydmxyhexan-2-yl]-2-methylimidazo[1,2-a]pyridin-3-carboxamid

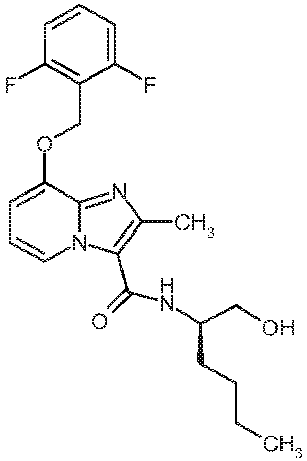

2 g 8-[(2,6-Difluorbenzyl)oxy]-2-methylimidazo[1,2-a]pyridin-3-carbonsäure (Beispiel 3A, 6.28 mmol, 1 Äquivalent), 2.2 g (Benzotriazol-1-yloxy)bisdimethylaminomethyliumfluorborat (TBTU; 6.9 mmol, 1.1 Äquivalente) und 3.45 ml 4-Methylmorpholin (31.4 mmol, 5 Äquivalente) wurden in 15 ml Dichlormethan und 10 ml DMF vorgelegt. Bei RT wurden 810 mg (*R*)-(-)-2-Amino-1-hexanol (6.9 mmol, 1.1 Äquivalente) zugesetzt und es wurde 3 h gerührt. Dann wurde mit Dichlormethan verdünnt und mit Wasser und 1 N wässriger Salzsäure auf pH 4 eingestellt. Die organische Phase wurde abgetrennt und die wäßrige Phase zweimal mit Dichlormethan extrahiert. Die vereinigten organischen Phasen wurden zweimal mit Wasser gewaschen, über Natriumsulfat getrocknet, filtriert und eingeengt. Das Rohgemisch wurde über präparative HPLC (Methode 9) aufgereinigt. Es wurden 1066 mg (41% d. Th.) der Titelverbindung erhalten.
LC-MS (Methode 2): Rₜ = 0.87 min
MS (ESpos): m/z = 418.2 (M+H)⁺
¹H-NMR (400 MHz, DMSO-d₆): δ = 0.87 (t, 3H), 1.23 - 1.70 (m, 6H), 2.54 (s, 3H), 3.39 - 3.54 (m, 2H), 3.92 - 4.02 (m, 1H), 4.74 (t, 1H), 5.31 (s, 2H), 6.92 (t, 1H), 6.99 (d, 1H), 7.23 (t, 2H), 7.52 (d, 1H), 7.59 (m, 1H), 8.53 (d, 1H).

### Beispiel 129A

### 8-[(2,6-Difluorbenzyl)oxy]-2-methyl-N-[(2R)-1-oxohexan-2-yl]imidazo[1,2-a]pyridin-3-carboxamid

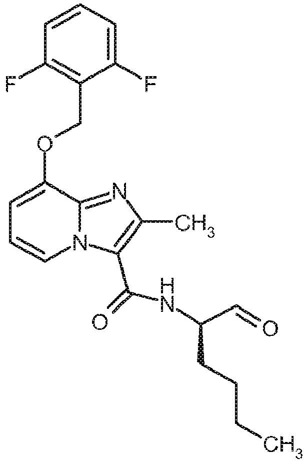

1.14 g Dess-Martin Periodinan (2.7 mmol, 1.5 Äquivalente) wurden in 25 ml Dichlormethan vorgelegt und bei -20°C mit 0.145 ml Pyridin (1.8 mmol, 1 Äquivalent) und 750 mg 8-[(2,6-Difluorbenzyl)oxy]-*N*-[(2*R*)-1-hydroxyhexan-2-yl]-2-methylimidazo[1,2-a]pyridin-3-carboxamid (Beispiel 3A, 1.8 mmol) versetzt. Das Reaktionsgemisch wurde für 2 h bei dieser Temperatur gerührt. Dann wurde unter Eiskühlung mit 1 N wässriger Natronlauge versetzt und dreimal mit Essigsäureethylester extrahiert. Die vereinigten organischen Phasen wurden mit gesättigter, wässriger Natriumchlorid-Lösung gewaschen, über Magnesiumsulfat getrocknet, filtriert und eingeengt. Es wurden 899 mg (ca. 70% Reinheit; 90% d. Th.) der Zielverbindung erhalten, die nicht weiter aufgereinigt wurden.
LC-MS (Methode 2): Rₜ =1.00 min [Hydrat: 0.83 min]
MS (ESpos): m/z = 416.3 (M+H)⁺ [Hydrat: 434.3 (M+H⁺)]
¹H-NMR (400 MHz, DMSO-d₆): δ = 0.87 (t, 3H), 1.20 - 1.95 (m, 6H), 2.54 (s, 3H), 4.30 - 4.38 (m, 1H), 5.31 (s, 2H), 6.92 (t, 1H), 7.01 (d, 1H), 7.23 (t, 2H), 7.59 (m, 1H), 8.20 (d, 1 H), 8.57 (d, 1 H), 9.10 (s, 1 H).

### Beispiel 130A

### ent-tert.-Butyl-[2-({[8-(benzyloxy)-2-methylimidazo[1,2-a]pyridin-3-yl]carbonyl}amino)-2-(3,4-difluorphenyl)ethyl]carbamat

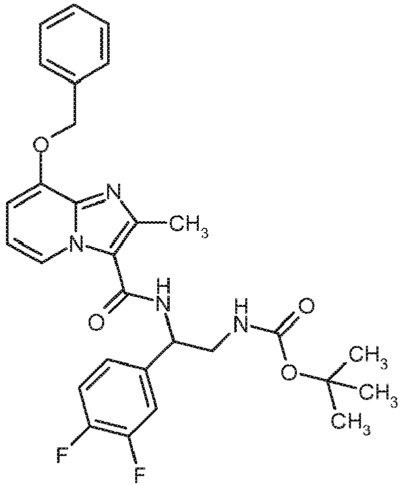

4.90 g 8-(Benzyloxy)-2-methylimidazo[1,2-a]pyridin-3-carbonsäure (Beispiel 30A, 17.36 mmol, 1 Äquivalent), 6.13 g (Benzotriazol-1-yloxy)bisdimethylaminomethyliumfluorborat (TBTU, 19 mmol, 1.1 Äquivalente) und 9.5 ml *N*-Methylmorpholin (8.78 g, 86.8 mmol, 5 Äquivalente) in 110 ml DMF wurden mit *ent*-tert.-Butyl-[2-amino-2-(3,4-difluorphenyl)ethyl]carbamat (Edukt 60A, 19.96 mmol, 1.15 Äquivalente) versetzt. Das Reaktionsgemisch wurde 2 h bei Raumtemperatur gerührt, dann auf 800 ml Wasser gegossen und mit 800 ml und 300 ml Essigsäureethylester extrahiert. Die organische Phase wurde mit 300 ml gesättigter, wässriger Natriumchlorid-Lösung gewaschen, getrocknet und bis auf ca. 15 ml eingeengt. Der Rückstand wurde in 90 ml tert.-Butylmethylether verrührt, abfiltriert, mit 30 ml tert.-Butylmethylether und dann mit n-Pentan gewaschen und im Vakuum getrocknet. Es wurden 7.30 g (75% d. Th.) der Titelverbindung erhalten.
LC-MS (Methode 7): Rₜ = 1.06 min
MS (ESpos): m/z = 537.2 (M+H)⁺
¹H-NMR (400 MHz, DMSO-d₆): δ = ppm 1.33 (s, 9 H), 2.61 (s, 3 H), 5.11 - 5.21 (m, 1 H), 5.28 (s, 2 H), 6.84 - 6.95 (m, 2 H), 7.08 (t, 1 H), 7.21 - 7.28 (m, 1 H), 7.43 (m, 7 H), 8.20 (d, 2 H), 8.52 (d, 2 H).

### Beispiel 131A

### ent-tert.-Butyl-[2-(3,4-difluorphenyl)-2-{[(8-hydroxy-2-methylimidazo[1,2-a]pyridin-3-yl)carbonyl]amino}ethyl]carbamat

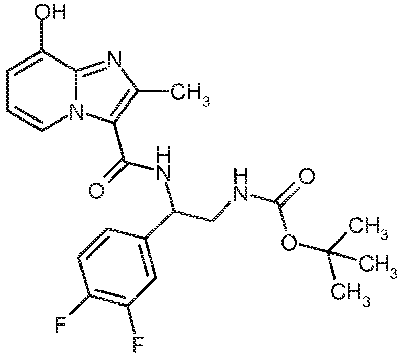

6.90 g *ent*-tert.-Butyl-[2-({[8-(benzyloxy)-2-methylimidazo[1,2-a]pyridin-3-yl]carbonyl}amino)-2-(3,4-difluorphenyl)ethyl]carbamat (Beispiel 130A, 12.8 mmol, 1 Äquivalent) wurden in 207 ml einer 1:1:1 Mischung aus Ethanol/THF/Dichlormethan vorgelegt und mit 0.69 g 10%igem Palladium auf Aktivkohle versetzt. Es wurde über Nacht bei RT und Normaldruck hydriert. Dann wurden 500 ml Dichlormethan zugegeben und das Reaktionsgemisch wurde bei 40°C über Kieselgel abfiltriert, mit Dichlormethan gewaschen und im Vakuum getrocknet. Es wurden 5.20 g (83% d. Th.; Reinheit 92%) der Titelverbindung erhalten.
LC-MS (Methode 2): Rₜ = 0.84 min
MS (ESpos): m/z = 447.3 (M+H)⁺
¹H-NMR (400 MHz, DMSO-d₆): 8 = 1.33 (s, 9 H), 2.63 (s, 3 H), 5.10 - 5.22 (m, 1 H), 6.65 (d, 1 H), 6.80 (t, 1 H), 7.08 (t, 1 H), 7.20 - 7.27 (m, 1 H), 7.36 - 7.54 (m, 2 H), 8.18 (d, 1 H), 8.43 (d, 1 H), [weitere Signale unter den Lösungsmittel-Peaks verborgen].

### Beispiel 132A

### rac-Benzyl-(2-hydroxy-3-metboxypropyl)carbamat

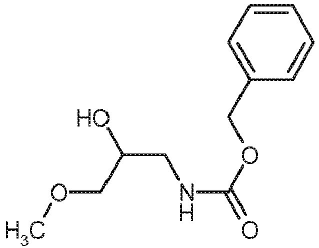

5.0 g (47.6 mmol) *rac*-1-Amino-3-methoxypropan-2-ol wurde in THF (158 ml) vorgelegt, mit 7.36 ml (52.3 mmol) Benzylchlorocarbonat, 24.85 ml (142.7 mmol) *N,N-*Diisoprnpylethylamin und 1.16 g (9.5 mmol) 4-Dimethylaminopyridin versetzt. Die Reaktionsmischung wurde 16 h bei RT gerührt. 3.7 ml (26.2 mmol) Benzylchlorocarbonat und anschließend 15 ml DMF wurden zur Reaktionslösung gegeben und über Nacht bei RT gerührt. Das Gemisch wurde eingeengt und mit Essigsäureethylester verdünnt. Dann wurde mit Wasser, gesättigter, wässriger Natriumhydrogencarbonat-Lösung und mit gesättigter, wässriger Natriumchlorid-Lösung gewaschen. Die organische Phase wurde über Natriumsulfat getrocknet, abfiltriert und eingeengt. Die wässrige Phase wurde eingeengt, der Rückstand in Essigsäureethylester aufgenommen, mit gesättigter, wässriger Natriumhydrogen-carbonat-Lösung und mit gesättigter, wässriger Natriumchlorid-Lösung gewaschen, über Natriumsulfat getrocknet, abfiltriert und eingeengt. Beide Fraktionen wurden zusammen gegeben und mittels Kieselgelchromatographie (Dichlormethan/Methanol-Gradient: 100:0 nach 10:1) gereinigt. Man erhielt 12.16 g (81% d. Th., Reinheit 76%) der Zielverbindung.
LC-MS (Methode 7): Rₜ = 0.68 min
MS (ESIpos): m/z = 240 (M+H)⁺.
¹H-NMR (400 MHz, DMSO-d₆): δ = 2.90 - 2.98 (m, 1H), 3.03 - 3.11 (m, 1H), 3.18 - 3.28 (m, 5H), 3.58 - 3.66 (m, 1H), 4.85 (d, 1H), 5.01 (s, 2H), 7.17 (t, 1H), 7.28 - 7.40 (m, 5H).

### Beispiel 133A

### Benzyl-[(2S)-3-hydroxybutan-2-yl]carbamat

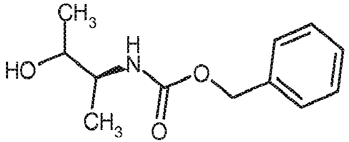

Unter Argon wurden 810 mg Benzyl-[(2*S*)-3-oxobutan-2-yl]carbamat (Beispiel 150A, 3.7 mmol, 1 Äquivalent) in 12 ml trockenem THF und 12 ml Methanol vorgelegt und bei 0°C mit 208 mg Natriumborhydrid (5.5 mmol, 1.5 Äquivalente) versetzt. Nach 2 h bei 0°C wurde eingeengt, der Rückstand mit Wasser und gesättigter, wässriger Natriumhydrogencarbonat-Lösung versetzt und viermal mit Essigsäureethylester extrahiert. Die vereinigten organischen Phasen wurden mit gesättigter, wässriger Natriumchlorid-Lösung gewaschen, über Magnesiumsulfat getrocknet und eingeengt. Es wurden 777 mg (90% d. Th.) der Titelverbindung erhalten.
Diastereomerengemisch im Verhältnis 2:1
Hauptdiastereomer:
LC-MS (Methode 2): Rt = 0.78 min
MS (ESpos): m/z = 224.1 (M+H)⁺
¹H-NMR (400 MHz, DMSO-d₆): δ = 1.01 (m, 6 H), 3.43 - 3.51 (m, 1 H), 4.48 (d, 1 H), 5.00 (s, 2 H), 7.34 - 7.38 (m, 5 H), [weitere Signale unter Lösungsmittel-Peaks verborgen].

### Beispiel 134A

### Benzyl-[(2R)-3-hydroxybulan-2-yl]carbamat

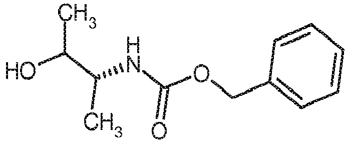

Unter Argon wurden 885 mg Benzyl-[(2*R*)-3-oxobutan-2-yl]carbamat (Beispiel 151A, 4 mmol, 1 Äquivalent) in 13 ml THF und 13 ml Methanol vorgelegt und mit 227 mg Natriumborhydrid (6 mmol, 1.5 Äquivalente) in Analogie zu Beispiel 133A umgesetzt und aufgearbeitet. Es wurden 830 mg (92% d. Th.) der Titelverbindung erhalten.
Diastereomerengemisch im Verhältnis 2:1
Hauptdiastereomer:
   LC-MS (Methode 2): Rₜ = 0.78 min
   MS (ESpos): m/z = 224.1 (M+H)⁺
   ¹H-NMR (400 MHz, DMSO-d₆): δ = 1.01 (m, 6 H), 3.43 - 3.51 (m, 1 H), 4.48 (d, 1 H), 5.00 (s, 2 H), 7.34 - 7.38 (m, 5 H), [weitere Signale unter Lösungsmittel-Peaks verborgen].

### Beispiel 135A

### rac-Benzyl-[3-(3,4-difluorphenoxy)-2-hydroxypropyl]carbamat

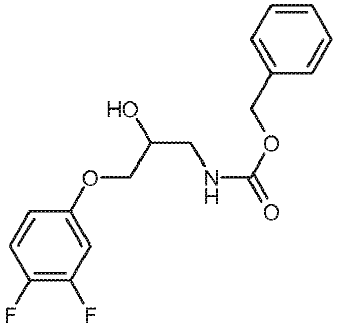

1 g *rac*-1-Amino-3-(3,4-difluorphenoxy)propan-2-ol (4.9 mmol, 1 Äquivalent) wurde in 16 ml THF gelöst und mit 0.76 ml Chlorameisensäurebenzylester (0.9 g, 5.4 mmol, 1.1 Äquivalente) und anschließend mit 2.6 ml *N*,*N*-Diisopropylethylamin (1.9 g, 14.8 mmol, 3 Äquivalente) und 0.12 g 4-Dimethylaminopyridin versetzt. Es wurde 30 min bei RT gerührt. Dann wurden 2 ml DMF hinzugefügt und über Nacht bei RT gerührt Es wurde eingeengt und der Rückstand anschließend in Essigsäureethylester aufgenommen. Die organische Phase wurde mit gesättigter, wässriger Natriumhydrogencarbonat-Lösung und gesättigter, wässriger Natriumchlorid-Lösung gewaschen, über Magnesiumsulfat getrocknet, filtriert und eingeengt. Es wurden 2.2 g (96% d. Th., Reinheit 73%) als Rohprodukt erhalten und ohne Reinigung weiter umgesetzt.
LC-MS (Methode 2): Rt = 1.00 min
MS (ESpos): m/z = 338.2 (M+H)⁺

### Beispiel 136A

### rac-Benzyl-[2-(1,3-dioxo-1,3-dihydro-2H-isoindol-2-yl)-3-methoxypropyl]carbamat

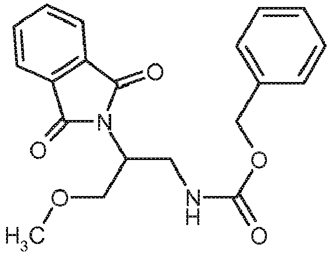

12.10 g (38.4 mmol, 76%-ig) *rac*-Benzyl-(2-hydroxy-3-methoxypropyl)carbamat (Beispiel 132A), 6.79 g (46.1 mmol) 1H-Isoindol-1,3(2H)-dion und 15.12 g (57.7 mmol) Triphenylphosphin wurden in THF (158 ml) vorgelegt. Es wurden 11.4 ml (57.7 mmol) Azodicarbonsäurediisopropylester zugetropft und anschließend 10 min bei RT gerührt. Dann wurde eingeengt und der Rückstand mittels Kieselgelchromatographie (Cyclohexan/Essigsäureethylester -Gradient: 3:0 nach 2:1) gereinigt. Die Produktfraktionen wurden eingeengt. Man erhielt 14.84 g (97% d. Th., Reinheit 93%) der Zielverbindung.
LC-MS (Methode 2): Rₜ = 0.95 min.
MS (ESIpos): m/z = 369 (M+H)⁺.
¹H-NMR (400 MHz, DMSO-d₆): δ = 3.22 (s, 3H), 3.46 (t, 2H), 3.63 (dd, 1H), 3.80 (t, 1H), 4.37 - 4.47 (m, 1H), 4.94 - 4.98 (m, 2H), 7.20 - 7.35 (m, 5H), 7.56 (t, 1H), 7.81 - 7.90 (m, 4H).

### Beispiel 137A

### Benzyl-[(2S)-3-(1,3-dioxo-1,3-dihydro-2H-isoindol-2-yl)butan-2-yl]carbamat

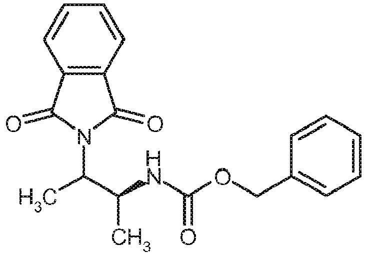

Unter Argon wurden 0.72 g Benzyl-[(2*S*)-3-hydroxybutan-2-yl]carbamat (Beispiel 133A, 3.3 mmol, 1 Äquivalent), 0.57 g 1H-Isoindol-1,3(2H)-dion (3.9 mmol, 1.2 Äquivalente) und 1.3 g Triphenylphosphin (4.9 mmol, 1.5 Äquivalente) in 15 ml THF gelöst und bei RT mit 1.1 g Di-tert.-butyl-(*E*)-diazen-1,2-dicarboxylat (DIAD, 4.9 mmol, 1.5 Äquivalente) versetzt. Es wurde über Nacht bei RT gerührt, mit 2 Tropfen Wasser und Extrelut^{®} versetzt und eingeengt. Der Rückstand wurde über eine Kieselgel-Kartusche (Biotage SNAP Cartrige KP-Sil 25 g) gereinigt (Gradient Cyclohexan/Essigsäureethylester von 5% bis 100%). Das erhaltene Rohprodukt wurde über Nacht in einem Gemisch aus 5 ml Methanol und 1 ml Wasser belassen. Der erhaltene Feststoff wurde abfiltriert, mit wenig Methanol/Wasser-Gemisch gewaschen und im Vakuum getrocknet. Das Filtrat wurde über eine präparative HPLC (Methode 9) gereinigt. Es wurden insgesamt 692 mg (53% d. Th., Reinheit 87%) der Titelverbindung erhalten (Diastereomerengemisch im Verhältnis ca. 4:1).
LC-MS (Methode 2): Rₜ = 1.02 min
MS (ESpos): m/z = 353.2 (M+H)⁺

### Beispiel 138A

### Benzyl-[(2R)-3-(1,3-dioxo-1,3-dihydro-2H-isoindol-2-yl)butan-2-yl]carbamat

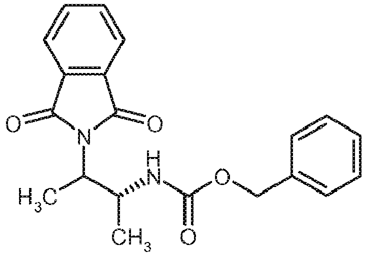

Unter Argon wurden 0.82 g Benzyl-[(2*R*)-3-hydroxybutan-2-yl]carbamat (Beispiel 134A, 3.3 mmol, 1 Äquivalent) mit 0.57 g 1H-Isoindol-1,3(2H)-dion (Phthalimid, 3.9 mmol, 1.1 Äquivalente) in Analogie zu Beispiel 137A umgesetzt und aufgearbeitet. Es wurden 1.25 g (72% d. Th.; Reinheit 75%) der Titelverbindung erhalten (Diastereomerenverhältnis ca. 3:1).
LC-MS (Methode 2): Rₜ = 1.02 min
MS (ESpos): m/z = 353.2 (M+H)⁺

### Beispiel 139A

### rac-Benzyl-[3-(3,4-difluorphenoxy)-2-(1,3-dioxo-1,3-dihydro-2H-isoindol-2-yl)propyl]carbamat

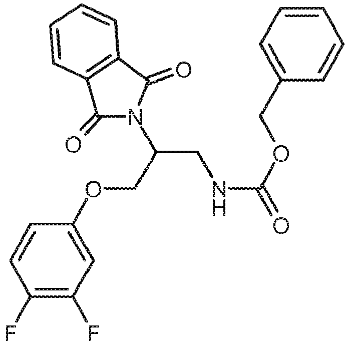

2.2 g *rac*-Benzyl-[3-(3,4-difluorphenoxy)-2-hydroxypropyl]carbamat (Beispiel 135A, 6.5 mmol, 1 Äquivalent) wurden mit 1.2 g 1H-Isoindol-1,3(2H)-dion (Phthalimid, 7.8 mmol, 1.2 Äquivalente) in Analogie zu Beispiel 137A umgesetzt und aufgearbeitet. Es wurden 1.78 g (54% d. Th.; Reinheit 93%) der Titelverbindung erhalten.
LC-MS (Methode 2): Rₜ = 1.17 min
MS (ESpos): m/z = 467.2 (M+H)⁺

### Beispiel 140A

### rac-Benzyl-(2-amino-3-methoxypropyl)carbamat Trifluoracetat

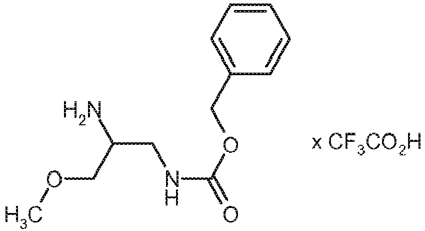

14.13 g (35.7 mmol, 93% rein) *rac*-Benzyl-[2-(1,3-dioxo-1,3-dihydro-2H-isoindol-2-yl)-3-methoxypropyl]carbamat (Beispiel 136A) wurden mit 123 ml (1.43 mol) Methylamin (40%ig in Wasser) versetzt. Die Reaktionsmischung wurde 35 min bei 60°C im geschlossenen Gefäß gerührt. Dann wurde eingeengt, dreimal in Methanol aufgenommen und wieder eingeengt und der Rückstand mittels präparativer RP-HPLC (Acetonitril/Wasser-Gradient unter Zusatz von 0.1% TFA) gereinigt. Die noch verunreinigte Produktfraktion wurde mittels RP-HPLC (Acetonitril/Wasser-Gradient unter Zusatz von 0.1% TFA) gereinigt. Man erhielt 9.7 g (69.5% d. Th., Reinheit 90%) der Zielverbindung.
LC-MS (Methode 7): Rₜ = 0.46 min.
MS (ESIpos): m/z = 239 (M+H)⁺.
¹H-NMR (400 MHz, DMSO-d₆): δ = 3.18 - 3.58 (m, 8H, überlagert durch Wasser-Signal), 5.05 (s, 2H), 7.29 - 7.40 (m, 5H), 7.48 (t, 1H), 7.82 - 8.01 (br. s, 2H).

### Beispiel 141A

### Benzyl-[(2S)-3-aminobutan-2-yl]carbamat

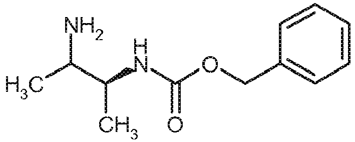

600 mg Benzyl-[(2*S*)-3-(1,3-dioxo-1,3-dihydro-2H-isoindol-2-yl)butan-2-yl]carbamat (Beispiel 137A, 1.7 mmol, 1 Äquivalent) wurden in 10 ml Methanol suspendiert und bei RT mit 2.9 ml 40%iger wässriger Methylamin-Lösung (2.6 g, 34 mmol, 20 Äquivalent) versetzt. Es wurde über Nacht bei RT stehen gelassen und dann mit Extrelut^{®} versetzt und eingeengt. Der Rückstand wurde über eine Kieselgel-Kartusche (Biotage Isolera SNAP Cartrige KP-Sil 25 g, Eluent: Dichlormethan/Methanol 95:5 bis 0:100) gereinigt. Es wurden 293 mg (43% d. Th.) der Titelverbindung.
LC-MS (Methode 2): Rₜ = 0.49 min
MS (ESpos): m/z = 223.2 (M+H)⁺

### Beispiel 142A

### Benzyl-[(2R)-3-aminobutan-2-yl]carbamat

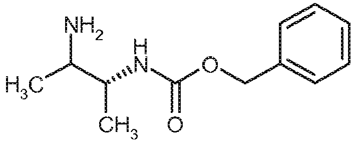

1.2 g Benzyl-[(2*R*)-3-(1,3-dioxo-1,3-dihydm-2H-isoindol-2-yl)butan-2-yl]carbamat (Beispiel 138A, 3.4 mmol, 1 Äquivalent) wurden in Analogie zu Beispiel 141A umgesetzt und aufgearbeitet. Das erhaltene Rohprodukt wurde nach der Kieselgelchromatographie über die präparative HPLC (Methode 9) nachgereinigt. Es wurden 147 mg (19% d. Th.) der Titelverbindung erhalten (Diastereomerenverhältnis ca. 3:1).
LC-MS (Methode 2): Rₜ = 0.48 min
MS (ESpos): m/z = 223.1 (M+H)⁺
¹H-NMR (400 MHz, DMSO-d₆): δ = 0.90 (d, 3 H), 0.99 (d, 3 H), 1.4 (br. s, 2 H); 2.63 - 2.80 (m, 1 H), 3.30 - 3.40 (m, 1 H; teilweise überlagert durch Wasser-Signal); 5.01 (s, 2 H), 6.95 (d, 1 H), 7.25 - 7.44 (m, 5 H).

### Beispiel 143A

### rac-Benzyl-[2-amino-3-(3,4-difluorphenoxy)propyl]carbamat

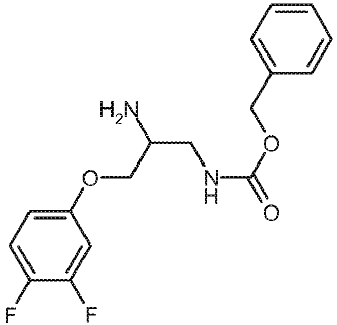

1.8 g *rac*-Benzyl-[3-(3,4-difluorphenoxy)-2-(1,3-dioxo-1,3-dihydro-2H-isoindol-2-yl)propyl]-carbamat (Beispiel 139A) wurden in 25 ml Ethanol gelöst, mit 4.8 ml 40%iger wässriger Methylamin-Lösung (56 mmol, 15 Äquivalente) versetzt und für 4.5 h bei 60°C gerührt. Die Reaktionsmischung wurde eingeengt und der Rückstand anschließend über Kieselgel chromatographiert (Biotage Isolera; Laufmittel: Dichlormethan/Methanol Gradient). Es wurden 600 mg (45% d. Th.; Reinheit 93%) der Titelverbindung erhalten.
LC-MS (Methode 2): Rₜ = 0.66 min
MS (ESpos): m/z = 337.2 (M+H)⁺

### Edukt 144A

### rac-2-Amino-4,4,4-trifluorbutan-1-ol

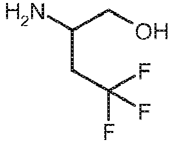

12.9 ml 2 M Lithiumborhydrid-Lösung in THF (25.8 mmol, 2.5 Äquivalente) wurden in 20 ml THF vorgelegt, mit 6.5 ml Chlortrimethylsilan (51.1 mmol, 5 Äquivalente) versetzt und es wurde 5 min bei RT gerührt. Dann wurden portionsweise 2.0 g *rac*-2-Amino-4,4,4-trifluorbutansäurehydrochlorid (10.3 mmol, 1 Äquivalent) zugegeben und es wurde über Nacht bei RT gerührt. Dann wurde tropfenweise mit 20.4 ml Methanol versetzt und nach beendeter Zugabe eingeengt. Der Rückstand wurde mit 12 ml einer 20%-igen wässrigen Kaliumhydroxid-Lösung versetzt und dreimal mit Dichlormethan extrahiert. Die vereinigten organischen Lösungen wurden über Natriumsulfat getrocknet, filtiert und eingeengt. Es wurden 1.58 g (96% d. Th.; Reinheit 90%) der Titelverbindung erhalten.
MS (Methode 10): m/z = 144.0 (M+H)⁺
¹H-NMR (400 MHz, DMSO-d₆): δ = 1.54 (br. s, 2 H), 1.97 - 2.15 (m, 1 H), 2.28 - 2.45 (m, 1 H), 2.84 - 2.98 (m, 1 H), 3.24 (d, 2 H), 4.78 (br. s, 1H).

### Edukt 145A

### rac-Benzyl-(4,4,4-trifluor-1-hydroxybutan-2-yl)carbamat

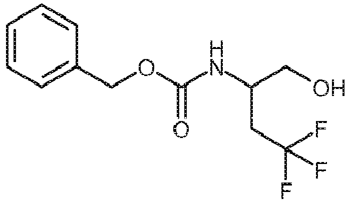

400 mg *rac*-2-Amino-4,4,4-trifluorbutan-1-ol (Beispiel 144A, 2.5 mmol, Reinheit ca. 90%, 1 Äquivalent) in 36 ml 1,4-Dioxan wurden bei RT mit 0.38 ml 50%-iger wässriger Kaliumcarbonat-Lösung (1.7 mmol, 0.68 Äquivalente) und 0.54 ml Chlorameisensäurebenzylester (3.8 mmol, 1.5 Äquivalente) versetzt und es wurde 2 h bei RT gerührt. Dann wurden 0.11 ml Chlorameisensäurebenzylester (0.76 mmol, 0.3 Äquivalente) und 0.08 ml 50%-iger wässriger Kaliumcarbonat-Lösung (0.35 mmol, 0.14 Äquivalente) nachgegeben und weitere 30 min bei RT gerührt. Nach Einengen im Vakuum wurde mittels präparative HPLC (RP18 Säule, Laufmittel: Acetonitril/Wasser-Gradient unter Zusatz von 0.1% TFA) gereinigt. Das erhaltene Rohprodukt wurde in Essigsäureethylester aufgenommen und zweimal mit gesättigter, wässriger Natriumhydrogencarbonat-Lösung gewaschen. Die wässrige Phase wurde mit Essigsäureethylester extrahiert und die vereinigten organischen Phasen wurden über Natriumsulfat getrocknet, filtriert und eingeengte. Es wurden 490 mg (70% d. Th.) der Titelverbindung erhalten.
LC-MS (Methode 2): Rₜ = 0.81 min
MS (ESpos): m/z = 278.1 (M+H)⁺
¹H-NMR (400 MHz, DMSO-d₆): δ = 2.20 - 2.38 (m, 1 H), 3.19 - 3.27 (m, 1 H), 3.28 - 3.42 (m, 2 H), 3.72 - 3.83 (m, 1 H), 4.96 - 5.08 (m, 3 H), 7.26 - 7.39 (m, 5 H).

### Edukt 146A

### rac-Benzyl-[1-(1,3-dioxo-1,3-dihydro-2H-isoindol-2-yl)-4,4,4-trifluorbutan-2-yl]carbamat

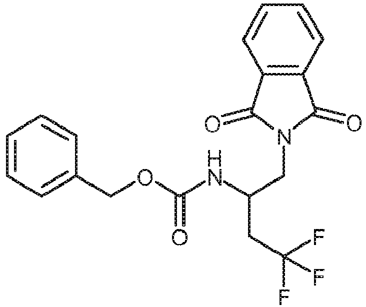

Unter Argon wurden 1.58 g *rac*-Benzyl-(4,4,4-trifluor-1-hydroxybutan-2-yl)carbamat (Beispiel 145A, 5.70 mmol, 1 Äquivalent), 0.84 g 1H-Isoindol-1,3(2H)-dion (Phthalimid, 5.70 mmol, 1 Äquivalente) und 2.24 g Triphenylphosphin (8.54 mmol, 1.5 Äquivalente) in 28 ml THF gelöst und bei RT mit 1.84 g Di-tert.-butyl-(*E*)-diazen-1,2-dicarboxylat (DIAD, 8.54 mmol, 1.5 Äquivalente) versetzt. Es wurde 30 min bei RT gerührt und dann mit einem Wasser/Acetonitril Gemisch versetzt. Reinigung mittels präparativer HPLC (RP18 Säule, Laufmittel: Acetonitril/Wasser-Gradient unter Zusatz von 0.1% TFA) lieferte 1.92 g (79% d. Th.) der Titelverbindung.
LC-MS (Methode 2): Rₜ = 1.08 min
MS (ESpos): m/z = 407.2 (M+H)⁺

### Edukt 147A

### rac-Benzyl-(1-amino-4,4,4-trifluorbutan-2-yl)carbamat Trifluoracetat

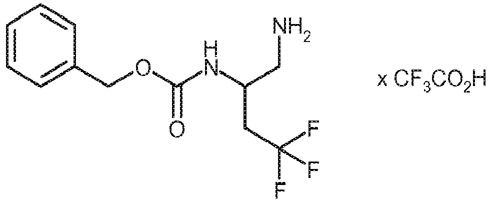

1.86 g *rac*-Benzyl-[1-(1,3-dioxo-1,3-dihydro-2H-isoindol-2-yl)-4,4,4-trifluorbutan-2-yl]carbamat (Beispiel 146A, 4.35 mmol, 1 Äquivalent) wurden in 15.0 ml 40%iger wässriger Methylamin-Lösung (174 mmol, 40 Äquivalente) gelöst und 30 min bei 60°C in einem geschlossenen Kolben gerührt. Die Reaktionsmischung wurde eingeengt und der Rückstand wurde anschließend über die präparative HPLC (RP18 Säule, Laufmittel: Acetonitril/Wasser-Gradient unter Zusatz von 0.1% TFA) gereinigt. Es wurden 1.25 g (74% d. Th.) der Titelverbindung erhalten.
LC-MS (Methode 2): Rₜ = 0.64 min
MS (ESpos): m/z = 277.2 (M+H)⁺
¹H-NMR (400 MHz, DMSO-d₆): δ = 2.57 - 2.71 (m, 1 H), 2.76 - 2.89 (m, 1 H), 2.93 - 3.04 (m, 1 H), 4.00 - 4.14 (m, 1 H), 5.02 (d, 1 H), 5.09 (d, 1 H), 7.27 - 7.42 (m, 5 H), 7.47 - 7.53 (m, 1 H), 7.88 - 8.08 (br. s, 3 H), [weiteres Signal unter DMSO Peak verborgen].

### Beispiel 148A

### ent-Benzyl-{(2S)-1-[methoxy(methyl)amino]-1-oxopropan-2-yl}carbamat

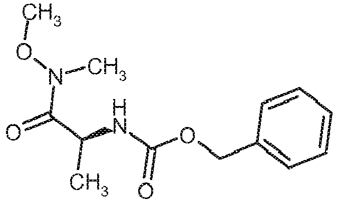

Unter Argon wurden 2 g *N*-[(Benzyloxy)carbonyl]-L-alanin (9 mmol, 1 Äquivalent) in 30 ml THF gelöst und nach der Zugabe von 1.9 g (11 mmol, 1.2 Äquivalente) 2-Chlor-4,6-dimethoxy-1,3,5-triazin und 3 ml 4-Methylmorpholin (2.7 g, 27 mmol, 3 Äquivalente) wurde 1 h bei RT gerührt. Es wurden 0.96 g (9.8 mmol, 1.1 Äquivalente) N,O-Dimethylhydroxylamin-Hydrochlorid hinzugegeben und über Nacht bei RT gerührt. Dann wurde eingeengt, der Rückstand mit Essigsäureethylester versetzt und mit 1 N wässriger Salzsäure, gesättigter, wässriger Natriumhydrogencarbonat-Lösung und gesättigter, wässriger Natriumchlorid-Lösung gewaschen, über Magnesiumsulfat getrocknet, eingeengt und der Rückstand im Vakuum getrocknet. Es wurden 2.25 g (61% d. Th.; Reinheit 65%) der Titelverbindung erhalten.
LC-MS (Methode 7): Rₜ = 0.82 min
MS (ESpos): m/z = 267.1 (M+H)⁺

### Beispiel 149A

### ent-Benzyl-{(2R)-1-[methoxy(methyl)amino]-1-oxopropan-2-yl}carbamat

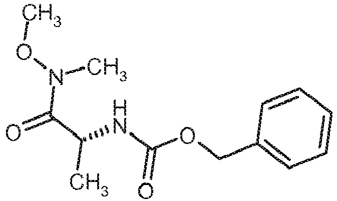

Unter Argon wurden 2 g *N*-[(Benzyloxy)carbonyl]-D-alanin (9 mmol, 1 Äquivalent) in 30 ml THF gelöst und nach der Zugabe von 1.9 g (11 mmol, 1.2 Äquivalente) 2-Chlor-4,6-dimethoxy-1,3,5-triazin, 3 ml 4-Methylmorpholin (2.7 g, 27 mmol, 3 Äquivalente) und 0.96 g (9.8 mmol, 1.1 Äquivalente) N,O-Dimethylhydroxylamin-Hydrochlorid wurde über Nacht bei RT gerührt. Dann wurde eingeengt, der Rückstand mit Essigsäureethylester versetzt und mit 1 N wässriger Salzsäure, gesättigter, wässriger Natriumhydrogencarbonat-Lösung und gesättigter, wässriger Natriumchlorid-Lösung gewaschen, über Magnesiumsulfat getrocknet, eingeengt und der Rückstand im Vakuum getrocknet. Es wurden 2.11 g (64% d. Th.; Reinheit 61%) der Titelverbindung als farbloses Öl erhalten.
LC-MS (Methode 7): Rₜ = 0.82 min
MS (ESpos): m/z = 267.1 (M+H)⁺

### Beispiel 150A

### Benzyl-[(2S)-3-oxobutan-2-yl]carbamat

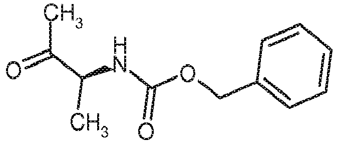

Unter Argon wurden 1000 mg Benzyl-{(2*S*)-1-[methoxy(methyl)amino]-1-oxopropan-2-yl}carbamat (Beispiel 148A, 3.8 mmol, 1 Äquivalent) in 19.5 ml THF gelöst und auf -78°C abgekühlt. Bei dieser Temperatur wurden langsam 4 ml 1.4 M Methylmagnesiumbromid-Lösung in 1:3 THF/Toluol (6 mmol, 1.5 Äquivalente) zugegeben. Es wurde 5 min bei -78°C und 1 h bei RT gerührt und über Nacht bei RT stehen gelassen. Dann wurden unter Eiskühlung 0.27 ml Wasser zugegeben und eingeengt. Der Rückstand wurde mit Wasser und 1 N wässriger Salzsäure-Lösung verdünnt und viermal mit Essigsäureethylester extrahiert. Die organische Phase wurde mit gesättigter, wässriger Natriumhydrogencarbonat-Lösung und mit gesättigter, wässriger Natriumchlorid-Lösung gewaschen, über Magnesiumsulfat getrocknet, filtriert und eingeengt. Es wurden 813 mg (88% d. Th.; Reinheit 90%) der Titelverbindung erhalten.
LC-MS (Methode 2): Rₜ = 0.77 min
MS (ESpos): m/z = 222.1 (M+H)⁺
¹H-NMR (400 MHz, DMSO-d₆): δ = 1.17 (d, 3 H), 2.08 (s, 3 H), 4.04 (m, 1 H), 5.04 (s, 2 H), 7.29 - 7.39 (m, 5 H), 7.67 (d, 1 H).

### Beispiel 151A

### Benzyl-[(2R)-3-oxobutan-2-yl]carbamat

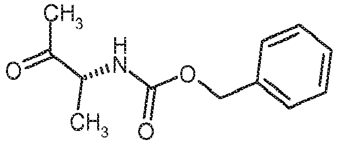

Unter Argon wurden 1000 mg Benzyl-{(2*R*)-1-[methoxy(methyl)amino]-1-oxopropan-2-yl}carbamat (Beispiel 149A, 3.8 mmol, 1 Äquivalent) in 19.5 ml THF gelöst und auf -78°C abgekühlt. Bei dieser Temperatur wurden langsam 4 ml 1.4 M Methylmagnesiumbromid-Lösung in 1:3 THF/Toluol (6 mmol, 1.5 Äquivalente) zugegeben. Es wurde dann 5 min bei -78°C und 1 h bei RT gerührt und über Nacht bei RT stehen gelassen. Dann wurden unter Eiskühlung 0.27 ml Wasser zugegeben und anschließend eingeengt. Den Rückstand verdünnte man mit Wasser und 1 N wässriger Salzsäure-Lösung und extrahierte ihn viermal mit Essigsäureethylester. Die organische Phase wurde mit gesättigter, wässriger Natriumhydrogencarbonat-Lösung und mit gesättigter, wässriger Natriumchlorid-Lösung gewaschen, über Magnesiumsulfat getrocknet, filtriert und eingeengt. Es wurden 888 mg (97% d. Th.) der Titelverbindung als farbloses Öl erhalten.
LC-MS (Methode 2): Rₜ = 0.77 min
MS (ESpos): m/z = 222.1 (M+H)⁺
¹H-NMR (400 MHz, DMSO-d₆): δ = 1.17 (d, 3 H), 2.08 (s, 3 H), 3.99 - 4.10 (m, 1 H), 5.03 (s, 2 H), 7.27 - 7.41 (m, 5 H), 7.68 (d, 1 H).

### Beispiel 152A

### 3-Brom-N-(4-fluorphenyl)propanamid

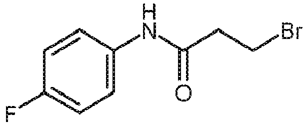

5.75 ml 4-Fluoranilin (6.65 g, 59.88 mmol, 1 Äquivalent) wurden in 65 ml THF vorgelegt, bei - 78°C mit 33 ml 2 M Trimethylaluminium-Lösung in Hexan (65.87 mmol, 1.1 Äquivalente) versetzt und langsam auf RT kommend 20 min gerührt. Diese Lösung wurde bei -20°C zu einer Lösung von 6.54 ml (10 g, 59.8 mmol, 1 Äquivalent) Methyl-3-brompropanoat in 65 ml THF getropft und anschließend wurde 3 h bei RT gerührt. Die Reaktionslösung wurde bei 0°C vorsichtig mit 1 N wässriger Salzsäure-Lösung angesäuert und zweimal mit Essigsäureethylester extrahiert. Die vereinten organischen Phasen wurden über Natriumsulfat getrocknet, filtriert und eingeengt. Es wurden 12.6 g (67% d. Th.; Reinheit 78%) der Titelverbindung erhalten und ohne Reinigung weiter umgesetzt.
LC-MS (Methode 7): Rₜ = 0.83 min
MS (ESpos): m/z = 248.0 (M+H)⁺

### Beispiel 153A

### 3-Brom-N-[4-{trifluormethyl)phenyl]propanamid

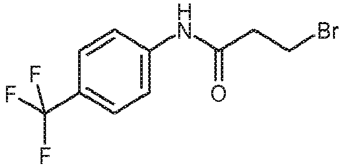

22.3 ml 4-(Trifluormethyl)anilin (29 g, 179.6 mmol, 1 Äquivalent) wurden mit 19.6 ml (30 g, 179.6 mmol, 1 Äquivalent) Methyl-3-brompropanoat in Analogie zu Beispiel 152A umgesetzt und aufgearbeitet. Das erhaltene Rohprodukt wurde dann über eine Kieselgelsäule gereinigt (Eluent: Dichlormethan). Es wurden 20.3 g (37% d. Th.) der Titelverbindung erhalten.
LC-MS (Methode 7): Rₜ = 1.03 min
MS (ESpos): m/z = 296.0 (M+H)⁺

### Beispiel 154A

### 1-(4-Fluorphenyl)azetidin-2-on

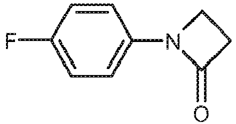

12.60 g 3-Brom-*N*-(4-fluorphenyl)propanamid (Beispiel 152A, 39.94 mmol, 1 Äquivalent) wurden in 97.1 ml Dichlormethan vorgelegt, mit 11.19 g 1,4,7,10,13,16-Hexaoxacyclooctadecan (18-Krone-6, 42.33 mmol, 1.06 Äquivalente) und 2.33 g Kaliumhydroxid (41.54 mmol, 1.04 Äquivalente) versetzt und über Nacht bei RT gerührt. Dann wurden 0.35 Äquivalente 1,4,7,10,13,16-Hexaoxacyclooctadecan und 0.35 Äquivalente Kaliumhydroxid zugegeben und über Nacht bei RT gerührt. Anschließend wurde mit gesättigter, wässriger Ammoniumchlorid-Lösung versetzt und dreimal mit Essigsäureethylester extrahiert. Die vereinten organischen Phasen wurden über Natriumsulfat getrocknet, filtriert, das Filtrat eingeengt und gereinigt [Säule: Reprosil C18, 10 µm, Spring Column 470 x 50 mm; Acetonitril/Wasser-Gradient; Fluss: 200 ml/min; 22°C; Wellenlänge: 210 nM]. Es wurden 3.46 g (38% d. Th.) der Titelverbindung erhalten.
LC-MS (Methode 7): Rₜ = 0.77 min
MS (ESpos): m/z = 166.1 (M+H)⁺
¹H-NMR (400 MHz, DMSO-d₆): δ = 3.08 (t, 2 H), 3.62 (t, 2 H), 7.18 - 7.28 (m, 2 H), 7.33 - 7.40 (m, 2 H).

### Beispiel 155A

### 1-[4-(Trifluormethyl)phenyl]azetidin-2-on

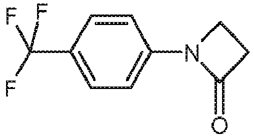

20.30 g 3-Brom-*N*-[4-(trifluormethyl)phenyl]propanamid (Beispiel 153A, 66.16 mmol, 1 Äquivalent) wurden in 161 ml Dichlormethan vorgelegt, mit 18.54 g 1,4,7,10,13,16-Hexaoxacyclooctadecan (18-Krone-6, 70.13 mmol, 1.06 Äquivalente) und 3.86 g Kaliumhydroxid (68.81 mmol, 1.04 Äquivalente) versetzt und über Nacht bei RT gerührt. Es wurde mit gesättigter, wässriger Ammoniumchlorid-Lösung versetzt und dreimal mit Essigsäureethylester extrahiert. Die vereinten organischen Phasen wurden über Natriumsulfat getrocknet, filtriert und das Filtrat eingeengt. Es wurden 25.0 g (86% d. Th.; Reinheit ca. 49%) der Titelverbindung erhalten und ohne Reinigung weiter umgesetzt.
LC-MS (Methode 7): Rₜ = 0.94 min
MS (ESpos): m/z = 216.1 (M+H)⁺
¹H-NMR (400 MHz, DMSO-d₆): δ = 3.14 (t, 2 H), 3.70 (t, 2 H), 7.51 (d, 2 H), 7.73 (d, 2 H).

### Beispiel 156A

### 6-Fluor-2,3-dihydrochinolin-4(1H)-on

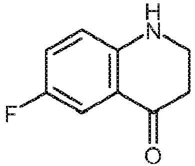

500 mg 1-(4-Fluorphenyl)azetidin-2-on (Beispiel 154A, 3.03 mmol, 1 Äquivalent) wurden in 24 ml Dichlormethan vorgelegt, bei 0°C mit 0.5 ml Trifluormethansulfonsäure (909 mg, 6.05 mmol, 2 Äquivalente) versetzt und 45 min bei RT gerührt. Anschließend wurden noch zweimal 0.3 Äquivalente Trifluormethansulfonsäure nachgegeben und je 30 min gerührt. Das Reaktionsgemisch wurde vorsichtig unter Eiskühlung mit gesättigter, wässriger Natriumhydrogen-carbonat-Lösung versetzt und dreimal mit Dichlormethan extrahiert. Die vereinten organischen Phasen wurden über Natriumsulfat getrocknet, filtriert und eingeengt. Es wurden 284 mg (57% d. Th.) der Titelverbindung erhalten.
LC-MS (Methode 7): Rₜ = 0.69 min
MS (ESpos): m/z = 166.1 (M+H)⁺

### Beispiel 157A

### 6-(Trifluormethyl)-2,3-dihydrochinolin-4(1H)-on

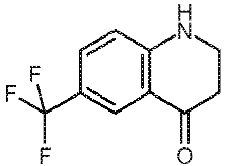

18.77 g 1-[4-(Trifluormethyl)phenyl]azetidin-2-on (Beispiel 155A, 42.74 mmol, 1 Äquivalent) wurden in Analogie zu Beispiel 156A umgesetzt und aufgearbeitet. Das erhaltene Rohprodukt wurde über eine Kieselgelsäule (Eluent: Cyclohexan/Essigsäureethylester-Gradient: 7:3 nach 5:1) gereinigt. Es wurden 1.75 g (16% d. Th.; Reinheit 86%) der Titelverbindung erhalten.
LC-MS (Methode 2): Rₜ = 0.89 min
MS (ESpos): m/z = 216.1 (M+H)⁺
¹H-NMR (400 MHz, DMSO-d₆): δ = 2.59 (t, 2 H), 3.46 - 3.55 (m, 2 H), 6.90 (d, 1 H), 7.53 (dd, 2 H), 7.80 (s, 1 H).

### Beispiel 158A

### tert.-Butyl-6-fluor-4-oxo-3,4-dihydrochinolin-1(2H)-carboxylat

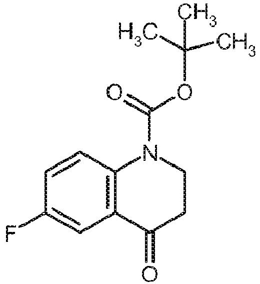

294 mg 6-Fluor-2,3-dihydrochinolin-4(1H)-on (Beispiel 156A, 1.78 mmol, 1 Äquivalent) wurden in 12.8 ml THF vorgelegt, mit 466 mg Di-tert.-butyldicarbonat (2.14 mmol, 1.2 Äquivalente) und 44 mg 4-Dimethylaminopyridin (0.36 mmol, 0.2 Äquivalente) versetzt und 1 h und 15 min bei RT gerührt. Dann wurden 0.2 Äquivalente Di-tert.-butyldicarbonat nachgegeben und über Nacht bei RT gerührt. Die Reaktionslösung wurde mit Wasser verdünnt und dreimal mit Essigsäureethylester extrahiert. Die vereinigten organischen Phasen wurden mit Wasser und mit gesättigter, wässriger Natriumhydrogencarbonat-Lösung gewaschen, über Natriumsulfat getrocknet, filtriert und eingeengt. Das erhaltene Rohprodukt wurde über eine Kieselgelsäule (Eluent: Cyclohexan/Essigsäureethylester 7:3) gereinigt. Es wurden 394 mg (81% d. Th.) der Titelverbindung erhalten.
LC-MS (Methode 5): Rₜ = 2.30 min
MS (ESpos): m/z = 266.2 (M+H)⁺
¹H-NMR (400 MHz, DMSO-d₆): δ = 1.50 (s, 9 H), 2.77 (t, 2 H), 4.08 (t, 2 H), 7.44 - 7.54 (m, 2 H), 7.79 (dd, 1H).

### Beispiel 159A

### tert.-Butyl-4-oxo-(trifluormethyl)-3,4-dihydrochinolin-1(2H)-carboxylat

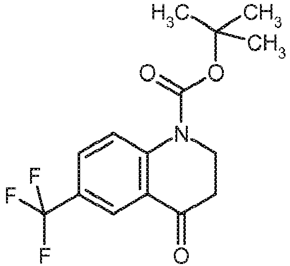

1.75 g 6-(Trifluormethyl)-2,3-dihydrochinolin-4(1H)-on (Beispiel 157A, 7.0 mmol, 1 Äquivalent) wurden in Analogie zu Beispiel 158A, umgesetzt und aufgearbeitet. Das Rohprodukt wurde über eine Kieselgelsäule (Eluent: Cyclohexan/Essigsäureethylester 4:1) gereinigt. Es wurden 1.72 g (74% d. Th.) der Titelverbindung erhalten.
LC-MS (Methode 7): Rt = 1.27 min
MS (ESpos): m/z = 360.0 (M-H+HCOOH)⁻
¹H-NMR (400 MHz, DMSO-d₆): δ =1.52 (s, 9 H), 2.83 (t, 2 H), 4.15 (t, 2 H), 7.89 - 7.95 (m, 1 H), 8.01 - 8.09 (m, 2 H).

### Bespiel 160A

### rac-tert.-Butyl-4-amino-6-fluor-3,4-dihydrochinolin-1(2H)-carboxylat Trifluoracetat

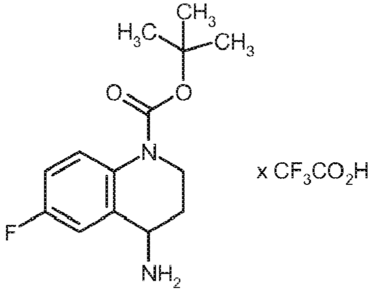

295 mg tert.-Butyl-6-fluor-4-oxo-3,4-dihydrochinolin-1(2H)-carboxylat (Beispiel 158A, 1.11 mmol, 1 Äquivalent) wurden in 3.5 ml Ethanol vorgelegt, mit 1285 mg Ammoniumacetat (16.68 mmol, 15 Äquivalente) und 84 mg Natriumcyanoborhydrid (1.33 mmol, 1.2 Äquivalente) versetzt und 2 min bei 130°C in der Mikrowelle umgesetzt. Die Reaktionslösung wurde eingeengt, der Rückstand in Essigsäureethylester aufgenommen und mit Wasser gewaschen. Die organische Phase wurde über Natriumsulfat getrocknet, filtriert und eingeengt. Das Rohprodukt reinigte man mittels präparativer HPLC (RP18 Säule, Laufmittel: Methanol/Wasser-Gradient unter Zusatz von 0.1 % TFA). Es wurden 185 mg (43% d. Th.) der Titelverbindung erhalten.
LC-MS (Methode 7): Rₜ = 0.56 min
MS (ESpos): m/z = 267.2 (M+H)⁺

### Bespiel 161A

### rac-tert.-Butyl-4-amino-6-(trifluormethyl)-3,4-dihydrochinolin-1(2H)-carboxylat

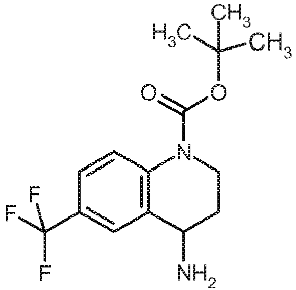

1.3 g *tert*.-Butyl-4-oxo-6-(trifluormethyl)-3,4-dihydrochinolin-1(2H)-carboxylat (Beispiel 159A, 4 mmol, 1 Äquivalent) wurden in 12.6 ml Ethanol vorgelegt und mit 4.6 g Ammoniumacetat (59.5 mmol, 15 Äquivalente) und 0.3 g Natriumcyanoborhydrid (4.8 mmol, 1.2 Äquivalente) versetzt. Die Reaktionsmischung wurde aufgeteilt und jeder Teil jeweils 1 min bei 130°C in der Mikrowelle bestrahlt. Dann wurde eingeengt, der Rückstand in Essigsäureethylester aufgenommen und mit Wasser gewaschen. Die organische Phase wurde über Natriumsulfat getrocknet, filtriert und eingeengt. Das erhaltene Rohprodukt wurde über Kieselgel (Eluent: Dichlormethan/Essigsäureethylester: 5/1, anschließend Dichlormethan/Methanol: 10/1) gereinigt. Es wurden 0.84 g (78% d. Th.) der Titelverbindung erhalten.
LC-MS (Methode 7): Rₜ = 0.69 min
MS (ESpos): m/z = 317.1 (M+H)⁺

### Beispiel 162A

### (2,6-Difluorphenyl)(²H₂)methanol

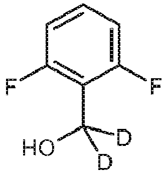

2.0 g 2,6-Difluorbenzoesäuremethylester (11.6 mmol, 1 Äquivalent) wurden bei 0°C in 40 ml THF vorgelegt und bei dieser Temperatur langsam mit 23.2 ml 1 M Lithiumaluminiumdeuterid-Lösung in THF (23.2 mmol, 2 Äquivalente) versetzt. Es wurde 45 min nachgerührt und dann nacheinander mit 1.2 ml Wasser, 1.2 ml 2 N wässriger Natriumhydroxid-Lösung und 2.3 ml Wasser versetzt. Der ausgefallene Niederschlag wurde abfiltriert und gut mit THF nachgewaschen. Das Filtrat wurde eingeengt und der Rückstand ohne Reinigung weiter umgesetzt. Es wurden 1.77 g (104% d. Th.) der Titelverbindung erhalten.
GC-MS (Methode 6): Rₜ = 2.38 min
MS (EIpos): m/z = 146.1 (M)⁺
¹H-NMR (400 MHz, DMSO-d₆): δ = 5.20 (s, 1H), 7.02 - 7.12 (m, 2 H), 7.24 - 7.44 (m, 1 H).

### Beispiel 163A

### rac-Benzyl-1-amino-3-azabicyclo[4.1.0]heptan-3-carboxylat Trifluoracetat

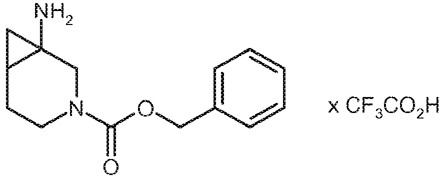

530 mg *rac*-3-Azabicyclo[4.1.0]heptan-1-amindihydrochlorid (2.86 mmol, 1 Äquivalent; Darstellung nach Gensini, M.; Kozhushkov, S. L; Yufit, D. S.; Howard, J. A. K.; Es-Sayed, M.; De Meijere, A.; Eur. J. Org. Chem., 2002, p. 2499 - 2507) wurden in 11.8 ml 1,4-Dioxan und 14.3 ml 1 N wässriger Natriumhydroxid-Lösung gelöst. Bei 0°C wurde eine Lösung von 0.3 ml Chlorameisensäurebenzylester (366 mg, 2.15 mmol, 0.75 Äquivalente) in 8.8 ml 1,4-Dioxan innerhalb von 30 min zugetropft. Dann wurde die Lösung langsam auf RT erwärmt und 30 min bei RT gerührt. Im Vakuum wurde vom 1,4-Dioxan befreit und die wässrige Phase dreimal mit Essigsäureethylester extrahiert. Die vereinigten organischen Phasen wurden über Natriumsulfat getrocknet, filtriert und eingeengt. Das Rohprodukt reinigte man über präparative HPLC (RP18 Säule, Laufmittel: Methanol/Wasser-Gradient unter Zusatz von 0.1% TFA/Ameisensäure). Es wurden 428 mg (41% d. Th.) der Titelverbindung erhalten.
LC-MS (Methode 2): Rₜ = 0.55 min
MS (ESpos): m/z = 247.2 (M+H)⁺
¹H-NMR (400 MHz, DMSO-d₆): δ = 0.58 (t, 1 H), 1.01 -1.13 (m, 1 H), 1.38 -1.47 (m, 1 H), 1.49 - 1.63 (m, 1 H), 1.93 - 2.07 (m, 1 H), 2.89 - 3.10 (m, 1 H), 3.91 - 4.11 (m, 1 H), 5.08 (s, 2 H), 7.24 - 7.43 (m, 5 H), 8.28 (br. s, 3 H), [weitere Signale unter Lösungsmittel-Peaks verborgen].

### Beispiel 164A

### tert.-Butyl-[2-(3,3-difluorpyrrolidin-1-yl)ethyl]carbamat

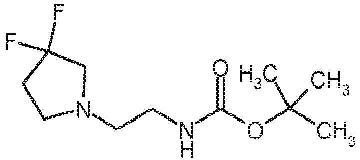

900 mg 3,3-Difluorpyrrolidinhydrochlorid (6.27 mmol, 1 Äquivalent) wurden in 62 ml Acetonitril vorgelegt, mit 1475 mg tert.-Butyl-(2-bromethyl)carbamat (6.58 mmol, 1.05 Äquivalent) und anschließend mit 3.28 ml *N*,*N*-Diisopropylethylamin (2.43 g, 18.81 mmol, 3 Äquivalente) versetzt und über Nacht bei 80°C gerührt. Dann wurden 0.5 Äquivalente 3,3-Difluorpyrrolidinhydrochlorid (450 mg, 3.14 mmol) zur Reaktionslösung gegeben und weiter über Nacht bei 80°C gerührt. Die Reaktionslösung wurde eingeengt und über eine Kieselgelsäule (Eluent: Cyclohexan/Essigsäureethylester-Gradient: 5/1 nach Essigsäureethylester: pur) gereinigt. Es wurden 431 mg (28% d. Th) der Titelverbindung erhalten.
GC-MS (Methode 6): Rₜ = 4.81 min
MS (ESpos): m/z = 251.0 (M+H)⁺
¹H-NMR (400 MHz, DMSO-d₆): δ = 1.37 (s, 9 H), 2.13 - 2.27 (m, 2 H), 2.44 (t, 2 H), 2.68 (t, 2 H), 2.87 (t, 2 H), 2.97 - 3.05 (m, 2 H), 6.76 (t, 1 H).

### Beispiel 165A

### tert.-Butyl-{2-[(2-methoxyethyl)amino]ethyl}carbamat

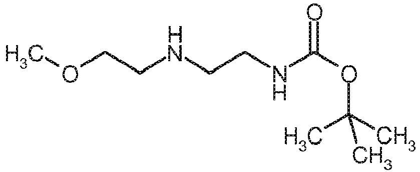

1.57 g tert.-Butyl-(2-bromethyl)carbamat (7.0 mmol, 0.7 Äquivalente) wurden in 98.5 ml Acetonitril vorgelegt, mit 0.87 ml 2-Methoxyethylamin (750 mg, 10.0 mmol, 1 Äquivalent) und 3.5 ml *N,N-*Diisopropylethylamin (2.58 g, 20 mmol, 2 Äquivalente) versetzt und über Nacht bei 80°C gerührt. Es wurde eingeengt und der Rückstand über eine Kieselgelsäule (Eluent: Dichlormethan/Methanol-Gradient: 50:1 nach 30:1 nach 10:1, dann Dichlormethan/2 N Ammoniak in Methanol: 10:1) gereinigt. Es wurden 1.69 g (99% d. Th.; Reinheit ca. 90%) der Titelverbindung erhalten.
MS (Methode 10, ESpos): m/z = 219.1 (M+H)⁺

### Beispiel 166A

### tert-Butyl-[2-(4,4-difluorpiperidin-1-yl)ethyl]carbamat

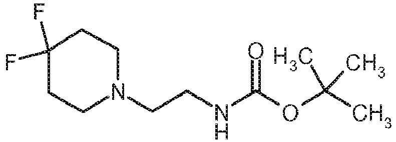

0.90 g 4,4-Difluorpiperidinhydrochlorid (5.71 mmol, 1 Äquivalente) wurden in 56 ml Acetonitril vorgelegt, mit 1.41 g tert-Butyl-(2-bromethyl)carbamat (6.28 mmol, 1.1 Äquivalente) und 3 ml *N,N-*Diisopropylethylamin (2.21 g, 17.13 mmol, 3 Äquivalente) versetzt und über Nacht bei 80°C gerührt. Dann wurden zweimal 0.4 Äquivalente tert-Butyl-(2-bromethyl)carbamat zur Reaktionslöung gegeben und jeweils weiter über Nacht bei 80°C gerührt. Es wurde eingeengt und über eine Kieselgelsäule (Gradient: Cyclohexan/Essigsäureethylester: 3:1 bis Essigsäureethylester) aufgereinigt. Es wurden 1.28 g (62% d. Th.; Reinheit 73%) der Titelverbindung erhalten.
GC-MS (Methode 6): Rₜ = 4.87 min
MS (EIpos): m/z = 191.1 (M-73)⁺
MS (Methode 10, ESpos): m/z = 265.2 (M+H)⁺

### Beispiel 167A

### 2-(3,3-Difluorpyrrolidin-1-yl)ethanamindihydrochlorid

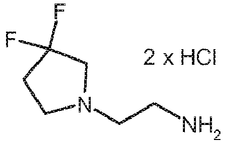

431 mg tert.-Butyl-[2-(3,3-difluorpyrrolidin-1-yl)ethyl]carbamat (Beispiel 164A, 1.72 mmol, 1 Äquivalent) wurden mit 8.6 ml 2 N Chlorwassentoff-Lösung in Diethylether (17.22 mmol, 10 Äquivalente) versetzt und die Reaktionsmischung wurde über Nacht bei RT gerührt. Dann wurde das Lösungsmittel abdekantiert, der Rückstand dreimal mit Diethylether verrührt und der so erhaltene Rückstand im Vakuum getrocknet. Es wurden 410 mg (99% d. Th.; Reinheit 93%) der Titelverbindung erhalten.
MS (Methode 10, ESpos): m/z = 151.1 (M-2HCl+H)⁺
¹H-NMR (400 MHz, DMSO-d₆): δ = 3.07 - 3.23 (m, 2 H), 3.28 - 4.13 (m, 8 H), 8.34 (br. s, 4 H).

### Beispiel 168A

### N-(2-Methoxyethyl)ethan-1,2-diamindihydrochlorid

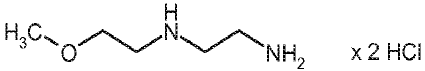

750 mg tert.-Butyl-{2-[(2-methoxyethyl)amino]ethyl}carbamat (Beispiel 165A, Reinheit ca. 90%, 3.1 mmol, 1 Äquivalent) wurden in 15.5 ml 2 N Clorwasserstoff-Lösung in Diethylether (31 mmol, 10 Äquivalente) versetzt und über Nacht bei RT gerührt. Das Lösungsmittel wurde abdekantiert, der Rückstand dreimal mit Diethylether verrührt und das so erhaltene Produkt wurde im Vakuum getrocknet. Es wurden 482 mg (82% d. Th.) der Titelverbindung erhalten.
MS (Methode 10, ESpos): m/z = 119 (M-2HCl+H)⁺
¹H-NMR (400 MHz, DMSO-d₆): δ = 3.11 - 3.24 (m, 6 H), 3.30 (s, 3 H), 3.61 (t, 2 H), 8.19 (br. s, 3 H), 9.16 (br. s, 2 H).

### Beispiel 169A

### 2-(4,4-Difluorpiperidin-1-yl)ethanamindihydrochlorid

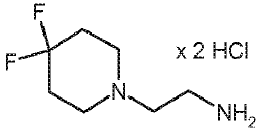

500 mg tert.-Butyl-[2-(4,4-difluorpiperidin-1-yl)ethyl]carbamat (Beispiel 166A, 1.38 mmol, 1 Äquivalent) wurden mit 6.9 ml 2 N Chlorwasserstoff-Lösung in Diethylether (13.8 mmol, 10 Äquivalente) versetzt und über Nacht bei RT gerührt. Das Lösungsmittel wurde abdekantiert, der Rückstand dreimal mit Diethylether verrührt, der Diethylether abdekantiert und das so erhaltene Produkt im Vakuum getrocknet. Es wurden 448 mg (100% d. Th.; Reinheit 73%) der Titelverbindung erhalten.
MS (Methode 10, ESpos): m/z =165.1 (M-2HCl+H)⁺

### Beispiel 170A

### ent-tert.-Butyl-[2-(3,4-difluorphenyl)-2-{[(8-{[(2,6-difluorphenyl)(²H₂)methyl]oxy}-2-methylimidazo[1,2-a]pyridin-3-yl)carbonyl]amino}ethyl]carbamat

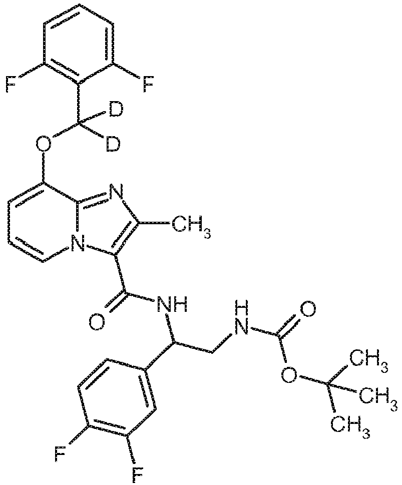

1222 mg *ent*-tert.-Butyl-[2-(3,4-difluorphenyl)-2-{[(8-hydroxy-2-methylimidazo[1,2-a]pyridin-3-yl)carbonyl]amino}ethyl]carbamat (Beispiel 131A, 2.7 mmol, 1 Äquivalent) wurden in 16.5 ml Toluol suspendiert und bei RT mit 600 mg (2,6-Difluorphenyl)(²H₂)methanol (Beispiel 162A, 4.1 mmol, 1.5 Äquivalente) und 1148 mg Triphenylphosphin (4.4 mmol, 1.6 Äquivalente) versetzt. Dann wurden unter Eisbadkühlung 12 ml THF und 0.9 ml Azodicarbonsäure-diisopropylester (942 mg, 4.4 mmol, 1.6 Äquivalente) zugegeben und es wurde 2 Tage bei RT gerührt und 2 Tage bei RT stehen gelassen. Die Lösung wurde dreimal mit Essigsäureethylester extrahiert und die vereinten organische Phasen mit gesättigter, wässriger Natriumchlorid-Lösung gewaschen, über Magnesiumsulfat getrocknet, filtriert und eingeengt. Das erhaltene Rohprodukt wurde über eine Kieselgelsäule (Biotage Isolera SNAP-Cartrige KP-Sil 100 g; Eluent: Cyclohexan/Essigsäureethylester: 12 % bis 100 %) vorgetrennt und dann über die präparative HPLC (Methode 9) nachgereinigt. Es wurden 255 mg (16% d. Th.) der Titelverbindung erhalten.
LC-MS (Methode 2): Rₜ = 1.11 min
MS (ESpos): m/z = 575.3 (M+H)⁺
¹H-NMR (400 MHz, DMSO-d₆): δ = 1.32 (s, 9 H), 2.58 (s, 3 H), 3.37 - 3.46 (m, 1 H), 5.12 - 5.19 (m, 1 H), 6.91 (t, 1 H), 7.02 (d, 1 H), 7.09 (t, 1 H), 7.19 - 7.27 (m, 3 H), 7.36 - 7.51 (m, 2 H), 7.54 - 7.65 (m, 1 H), 8.22 (d, 1 H), 8.55 (d, 1 H), [weiteres Signal unter Lösungsmittel-Peak verborgen].

In Analogie zu Beispiel 34A wurden die in Tabelle 10A gezeigten Beispiele hergestellt, indem 8-[(2,6-Difluorbenzyl)oxy]-2-methylimidazo[1,2-a]pyridin-3-carbonsäure aus Beispiel 3A mit den entsprechenden, kommerziell erhältlichen Aminen unter den in der allgemeinen Arbeitsvorschrift 2 beschriebenen Reaktionsbedingungen umgesetzt wurde.

Beispielhafte Aufarbeitung der Reaktionsmischung: Die Reaktionslösung wurde mit Wasser versetzt, der entstandene Niederschlag noch 0.5-1.0 h gerührt, abfiltriert, mit Wasser gewaschen und über Nacht im Hochvakuum getrocknet. Alternativ wurde der Niederschlag oder das Reaktionsgemisch direkt per präparativer HPLC (RP18 Säule, Laufmittel: Acetonitril/Wasser-Gradient unter Zusatz von 0.1% TFA) weiter aufgereinigt und über Nacht im Hochvakuum getrocknet. Gegebenenfalls wurden die Produktfraktionen in Essigsäureethylester oder Dichlormethan aufgenommen und zweimal mit gesättigter, wässriger Natriumhydrogencarbonatlösung gewaschen. Die wässrige Phase wurde zweimal mit Essigsäureethylester oder Dichlormethan extrahiert. Die vereinigten organischen Phasen wurden über Natriumsulfat getrocknet, filtriert und eingeengt.

**Tabelle 10A:**

| **Beispiel** | **IUPAC-Name / Struktur (Ausbeute)** | **Analytische Daten** |
|---|---|---|
| **171A** | tert.-Butyl-{4-[({8-[(2,6-difluorbenzyl)oxy]-2-methylimidazo[1,2-a]pyridin-3-yl}carbonyl)-amino]-2-methylbutan-2-yl}carbamat Trifluoracetat | LC-MS (Methode 1): Rₜ = 1.16 min |
| | | MS (ESpos): m/z = 503.1 (M+H)⁺ |
| | 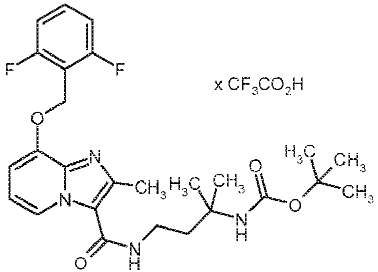 | |
| | (89% d. Th.) | |
| **172A** | *rac*-tert.-Butyl-4-[({8-[(2,6-difluorbenzyl)oxy]-2-methylimidazo[1,2-a]pyridin-3-yl)carbonyl)amino]-6-fluor-3,4-dihydrochinolin-1(2H)-carboxylat | LC-MS (Methode 7): Rₜ = 1.16 min |
| | | MS (ESpos): m/z = 567.2 (M+H)⁺ |
| | 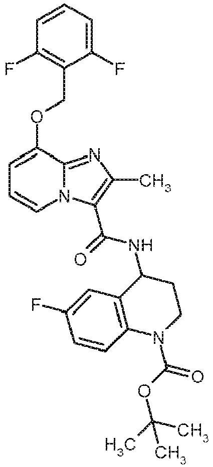 | |
| | (75% d. Th.) | |
| **173A** | *rac*-tert.-Butyl-4-[({8-[(2,6-difluorbenzyl)oxy]-2-methylimidazo[1,2-a]pyridin-3-yl}carbonyl)amino]-3,4-dihydroisochinolin-2(1H)-carboxylat | LC-MS (Methode 2): Rₜ = 1.07 min |
| | | MS (ESpos): m/z = 549.3 (M+H)⁺ |
| | 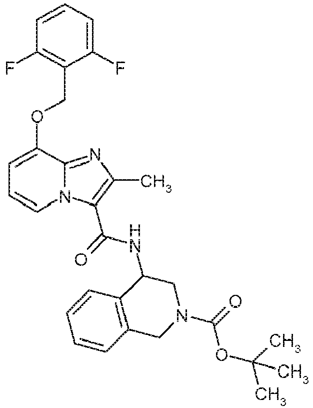 | |
| | (72% d. Th.) | |
| **174A** | *rac*-tert.-Butyl-4-[({8-[(2,6-difluorbenzyl)oxy]-2-methylimidazo[1,2-a]pyridin-3-yl}carbonyl)amino]-6-(trifluormethyl)-3,4-dihydrochinolin-1(2H)-carboxylat | LC-MS (Methode 7): Rₜ = 1.28 min |
| | | MS (ESpos): m/z = 617.2 (M+H)⁺ |
| | 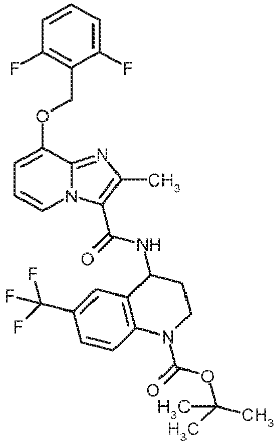 | |
| | (54% d. Th.) | |
| **175A** | 8-[(2,6-Difluorbenzyl)oxy]-*N*-(2-hydroxyethyl)-2-methylimidazo[1,2-a]pyridin-3-carboxamid | LC-MS (Methode 7): Rₜ = 0.57 min |
| | | MS (ESpos): m/z = 362.1 (M+H)⁺ |
| | 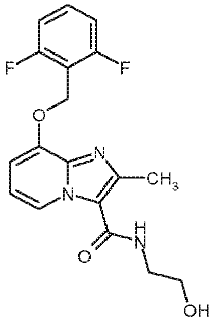 | ¹H-NMR (400 MHz, DMSO-d₆): 8 = 3.35 - 3.41 (m, 2 H), 3.50 - 3.59 (m, 2 H), 4.77 (t, 1 H), 5.30 (s, 2 H), 6.93 (t, 1 H), 7.01 (d, 1 H), 7.24 (t, 2 H), 7.54 - 7.65 (m, 1 H), 7.78 (t, 1 H), 8.63 (d, 1 H), [weiteres Signal unter DMSO-Peak verborgen]. |
| | (79% d. Th.) | |
| **176A** | *rac*-Benzyl-1-[({8-[(2,6-difluorbenzyl)oxy]-2-methylimidazo[1,2-a]pyridin-3-yl}carbonyl)amino]-3-azabicyclo[4.1.0]heptan-3-carboxylat | LC-MS (Methode 2): Rₜ = 1.00 min |
| | | MS (ESpos): m/z = 547.3 (M+H)⁺ |
| | 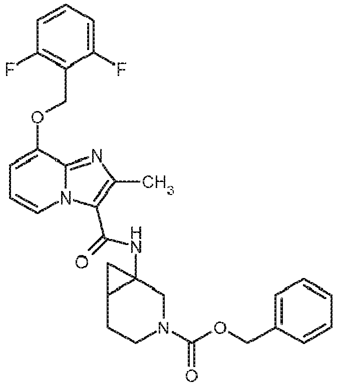 | ¹H-NMR (400 MHz, DMSO-d₆): δ = 0.53 - 0.64 (m, 1 H), 0.97 (dd, 1 H), 1.29 -1.38 (m, 1 H), 1.56 -1.67 (m, 1 H), 2.04 - 2.16 (m, 1 H), 2.45 (s, 3 H), 3.05 - 3.21 (m, 1 H), 3.42 - 3.68 (m, 2 H), 4.11 (d, 1 H), 5.08 (s, 2 H), 5.30 (s, 2 H), 6.90 - 6.97 (m, 1 H), 7.02 (d, 1 H), 7.23 (t, 2 H), 7.27 - 7.43 (m, 5 H), 7.51 - 7.64 (m, 1 H), 8.26 (br. s, 1 H), 8.62 (d, 1 H). |
| | (84% d. Th.) | |
| **177A** | 8-[(2,6-Difluorbenzyl)oxy]-*N*-[(2*R*)-1-hydroxypropan-2-yl]-2-methylimidazo[1,2-a]pyridin-3-carboxamid | LC-MS (Methode 1): Rₜ = 0.74 min |
| | | MS (ESpos): m/z = 376.1 (M+H)⁺ |
| | 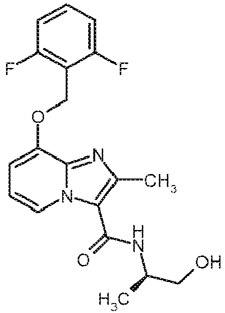 | |
| | (93% d. Th.) | |
| **178A** | 8-[(2,6-Difluorbenzyl)oxy]-*N*-[(2*S*)-1-hydroxypropan-2-yl]-2-methylimidazo[1,2-a]pyridin-3-carboxamid | LC-MS (Methode 1): Rₜ = 0.74 min |
| | | MS (ESpos): m/z = 376.1 (M+H)⁺ |
| | 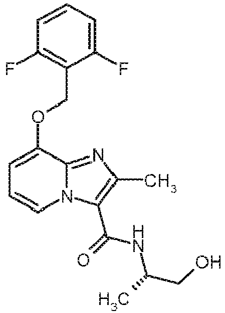 | |
| | (91% d. Th.) | |

### Beispiel 179A

### tert.-Butyl-{4-[({6-chlor-8-[(2,6-difluorbenzyl)oxy]-2-methylimidazo[1,2-a]pyridin-3-yl}carbonyl)amino]-2-methylbutan-2-yl}carbamat Trifluoracetat

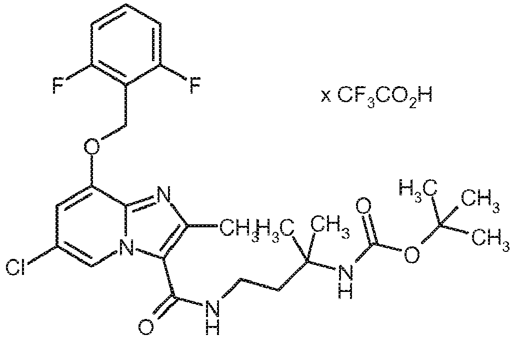

75 mg 6-Chlor-8-[(2,6-difluorbenzyl)oxy]-2-methylimidazo[1,2-a]pyridin-3-carbonsäure (Beispiel 16A, 0.21 mmol, 1 Äquivalent), 82 mg (Benzotriazol-1-yloxy)bisdimethylamino-methyliumfluorborat (TBTU, 0.26 mmol, 1.2 Äquivalente) und 108 mg 4-Methylmorpholin (1.06 mmol, 5 Äquivalente) wurden in 1.45 ml DMF vorgelegt. Nach 10 min bei RT wurden 56 mg (0.23 mmol, 1.1 Äquivalente) tert.-Butyl-(4-amino-2-methylbutan-2-yl)carbamathydrochlorid zugesetzt und es wurde über Nacht bei RT gerührt. Die Reaktionslösung wurde mit Wasser und Trifluoressigsäure versetzt und über präparative HPLC (Methode: RP18 Säule, Laufmittel: Acetonitril/Wasser-Gradient unter Zusatz von 0.1 % TFA) gereinigt. Es wurden 118 mg (84% d. Th.) der Titelverbindung erhalten.
LC-MS (Methode 1): Rₜ = 1.43 min
MS (ESpos): m/z = 537.0 (M-TFA+H)⁺

In Analogie zu Beispiel 179A wurden die in Tabelle 11A gezeigten Beispiele hergestellt, indem 6-Chlor-8-[(2,6-difluorbenzyl)oxy]-2-methylimidazo[1,2-a]pyridin-3-carbonsäure (Beispiel 16A) mit den entsprechenden, kommerziell erhältlichen oder selbst hergestellten Aminen unter den in der allgemeinen Arbeitsvorschrift 2 beschriebenen Reaktionsbedingungen umgesetzt wurde:

**Tabelle 11A:**

| **Beispiel** | **IUPAC-Name / Struktur (Ausbeute)** | **Analytische Daten** |
|---|---|---|
| **180A** | *rac*-tert.-Butyl-4-[({6-chlor-8-[(2,6-difluorbenzyl)oxy]-2-methylimidazo[1,2-a]pyridin-3-yl}carbonyl)amino]-3,4-dihydroisochinolin-2(1H)-carboxylat | LC-MS (Methode 7): Rₜ = 1.38 min |
| | | MS (ESpos): m/z = 583.1 (M+H)⁺ |
| | 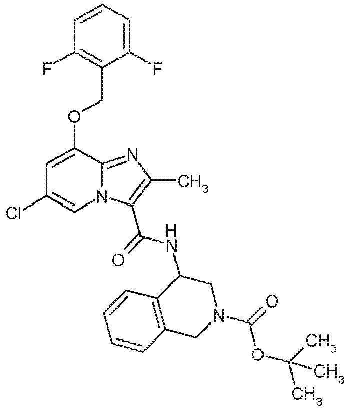 | |
| | (70% d. Th.; Reinheit 90%) | |

### Beispiel 181A:

### tert.-Butyl-{2-[({8-[(2,6-difluorbenzyl)oxy]-2,6-dimethylimidazo[1,2-a]pyridin-3-yl}carbonyl)amino]ethyl}carbamat Trifluoracetat

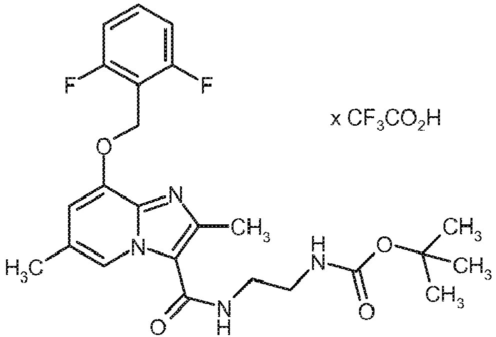

60 mg 8-[(2,6-Difluorbenzyl)oxy]-2,6-dimethylimidazo[1,2-a]pyridin-3-carbonsäure (Beispiel 21A, 0.18 mmol, 1 Äquivalent), 70 mg (Benzotriazol-1-yloxy)bisdimethylamino-methyliumfluorborat (TBTU, 0.22 mmol, 1.2 Äquivalente) und 73 mg 4-Methylmorpholin (0.72 mmol, 4 Äquivalente) wurden in 1.3 ml DMF vorgelegt. Nach 10 min bei RT wurden 32 mg tert.-Butyl-(2-aminoethyl)carbamat (0.2 mmol, 1.1 Äquivalente) zugesetzt und es wurde über Nacht bei RT gerührt. Die Reaktionslösung wurde mit Wasser und Trifluoressigsäure versetzt und über präparative HPLC (Methode: RP18 Säule, Laufmittel: Acetonitril/Wasser-Gradient unter Zusatz von 0.1% TFA) gereinigt. Es wurden 93 mg (87% d. Th.) der Titelverbindung erhalten.
LC-MS (Methode 7): Rₜ = 0.88 min
MS (ESpos): m/z = 475.2 (M+H)⁺

In Analogie zu Beispiel 181A wurden die in Tabelle 12A gezeigten Beispiele hergestellt, indem 8-[(2,6-Difluorbenzyl)oxy]-2,6-dimethylimidazo[1,2-a]pyridin-3-carbonsäure (Beispiel 21A) mit den entsprechenden, kommerziell erhältlichen oder selbst hergestellten Aminen unter den in der allgemeinen Arbeitsvorschrift 2 beschriebenen Reaktionsbedingungen umgesetzt wurde:

**Tabelle 12A:**

| **Beispiel** | **IUPAC-Name / Struktur (Ausbeute)** | **Analytische Daten** |
|---|---|---|
| **182A** | tert.-Butyl-{4-[({8-[(2,6-difluorbenzyl)oxy]-2,6-dimethylimidazo[1,2-a]pyridin-3-yl}carbonyl)-amino]-2-methylbutan-2-yl}carbamat Trifluoracetat | LC-MS (Methode 2): Rₜ = 1.01 min |
| | | MS (ESpos): m/z = 517.3 (M+H)⁺ |
| | 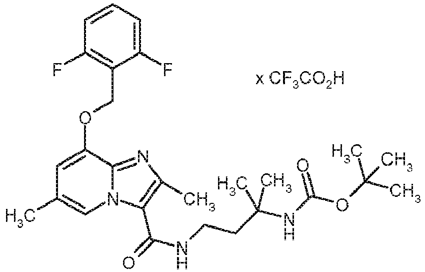 | ¹H-NMR (400 MHz, DMSO-d₆): δ = 1.23 (s, 6 H), 1.37 (s, 9 H), 1.84 -1.96 (m, 2 H), 2.39 (s, 3 H), 3.24 - 3.33 (m, 2 H), 5.39 (s, 2 H), 6.51 (br. s, 1 H), 7.26 (t, 2 H), 7.41 (br. s, 1 H), 7.55 - 7.68 (m, 1 H), 8.20 (br. s, 1 H), 8.53 (s, 1 H), [weiteres Signal unter Lösungsmittel-Peaks verborgen]. |
| | (53% d. Th.) | |
| **183A** | tert.-Butyl-(2*R*)-2-{[({8-[(2,6-difluorbenzyl)oxy]-2,6-dimethylimidazo[1,2-a]pyridin-3-yl}carbonyl)amino]methyl}piperidin-1-carboxylat Trifluoracetat ¹⁾ | LC-MS (Methode 7): Rₜ = 1.07 min |
| | | MS (ESpos): 529.3 m/z = (M+H)⁺ |
| | 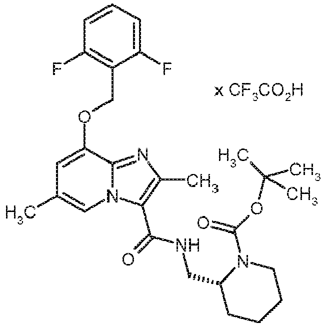 | |
| | (39% d. Th.) | |

| | | |
|---|---|---|
| ¹⁾ Kupplungsreagenz : HATU | | |

In Analogie zu Beispiel 34A wurden die in Tabelle 13A gezeigten Beispiele hergestellt, indem die entsprechenden Carbonsäuren mit den entsprechenden, kommerziell erhältlichen Aminen unter den in der allgemeinen Arbeitsvorschrift 2 beschriebenen Reaktionsbedingungen umgesetzt wurden. Beispielhafte Aufarbeitung der Reaktionsmischung: Die Reaktionslösung wurde mit Wasser versetzt, der entstandene Niederschlag noch 0.5-1.0 h gerührt, abfiltriert, mit Wasser gewaschen und über Nacht im Hochvakuum getrocknet. Alternativ wurde der Niederschlag oder das Reaktionsgemisch direkt per präparativer HPLC (RP18 Säule, Laufmittel: Acetonitril/Wasser-Gradient unter Zusatz von 0.1% TFA) weiter aufgereinigt und über Nacht im Hochvakuum getrocknet. Gegebenenfalls wurden die Produktfraktionen in Essigsäureethylester oder Dichlormethan aufgenommen und zweimal mit gesättigter, wässriger Natriumhydrogencarbonatlösung gewaschen. Die wässrige Phase wurde zweimal mit Essigsäureethylester oder Dichlormethan extrahiert. Die vereinigten organischen Phasen wurden über Natriumsulfat getrocknet, filtriert und eingeengt.

**Tabelle 13A:**

| **Beispiel** | **IUPAC-Name / Struktur (Ausbeute)** | **Analytische Daten** |
|---|---|---|
| **184A** | Benzyl-{(2*R*)-3-[({8-[(2,6-difluorbenzyl)oxy]-2-methylimidazo[1,2-a]pyridin-3-yl}carbonyl)-amino]butan-2-yl}carbamat | LC-MS (Methode 7): Rₜ = 0.94 min |
| | | MS (ESpos): m/z = 523.2 (M+H)⁺ |
| | 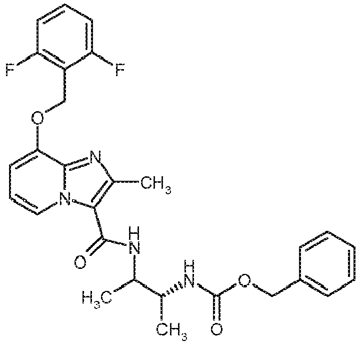 | ¹H-NMR (400 MHz, DMSO-d₆): 8 = 1.11 (d, 3 H), 1.16 (d, 3 H), 2.47 (s, 3 H), 3.63 - 3.87 (m, 1 H), 4.06 - 4.15 (m, 1 H), 4.94 - 5.05 (m, 2 H), 5.30 (s, 2 H), 6.88 - 6.94 (m, 1 H), 7.00 (d, 1 H), 7.19 - 7.38 (m, 8 H), 7.54 - 7.64 (m, 2 H), 8.51 - 8.58 (m, 1 H). |
| | (68% d. Th.; Reinheit 96% Diastereomerengemisch ca. 3:1) | |
| **185A** | *rac*-tert.-Butyl-{3-[({8-[(2,6-difluorbenzyl)oxy]-2-methylimidazo[1,2-a]pyridin-3-yl}carbonyl)-amino]-2-methylpropyl}carbamat | LC-MS (Methode 2): Rₜ = 0.96 min |
| | | MS (ESpos): m/z = 489.3 (M+H)⁺ |
| | 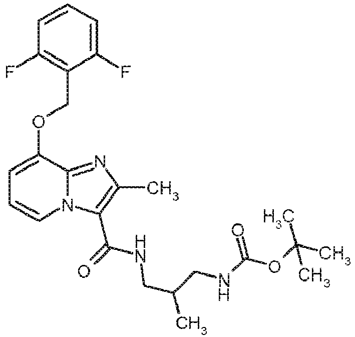 | ¹H-NMR (400 MHz, DMSO-d₆): 8 = 0.86 (d, 3 H), 1.38 (s, 9 H), 1.78 -1.91 (m, 1 H), 2.82 - 3.00 (m, 2 H), 3.13 - 3.26 (m, 2 H), 5.30 (s, 2 H), 6.87 - 6.96 (m, 2 H), 7.01 (d, 1 H), 7.24 (t, 2 H), 7.54 - 7.64 (m, 1 H), 7.83 (t, 1 H), 8.63 (d, 1 H), [weiteres Signal unter dem DMSO-Peak verborgen]. |
| | (61% d. Th.; Reinheit 96%) | |
| **186A** | *rac*-Benzyl-{2-[({8-[(2,6difluorbenzyl)oxy]-2-methylimidazo[1,2-a]pyridin-3-yl}carbonyl)amino]-3-methoxypropyl}carbamat ¹⁾ | LC-MS (Methode 7): Rₜ = 0.90 min |
| | | MS (ESpos): m/z = 539.2 (M+H)⁺ |
| | 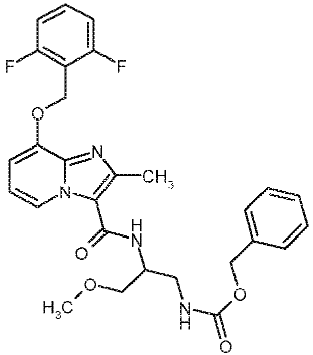 | |
| | (56% d. Th.; Reinheit 65%) | |
| **187A** | *rac*-Benzyl-{2-[({8-[(2,6-difluorbenzyl)oxy]-2-methylimidazo[1,2-a]pyridin-3-yl}carbonyl)amino]-3-(3,4-difluorphenoxy)propyl}carbamat ¹⁾ | LC-MS (Methode 7): Rₜ = 1.13 min |
| | | MS (ESpos): m/z = 637.2 (M+H)⁺ |
| | 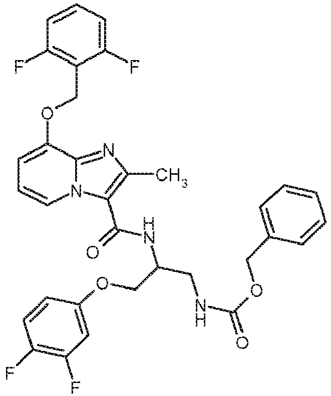 | |
| | (37% d. Th.; Reinheit 77%) | |
| **188A** | tert.-Butyl-{3-[({8-[(2,6-difluorbenzyl)oxy]-2-methylimidazo[1,2-a]pyridin-3-yl}carbonyl)amino]-2,2-dimethylpropyl}carbamat | LC-MS (Methode 7): Rₜ = 1.05 min |
| | | MS (ESpos): m/z = 503.2 (M+H)⁺ |
| | 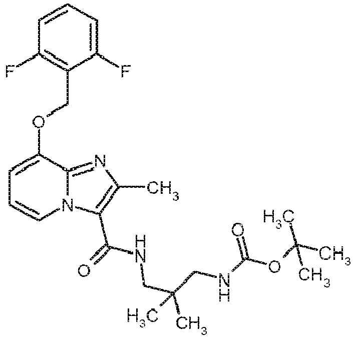 | ¹H-NMR (400 MHz, DMSO-d₆): 8 = 0.84 (s, 6 H), 1.39 (s, 9 H), 2.58 (s, 3 H), 2.80 - 2.86 (m, 2 H), 3.13 (d, 2 H), 5.31 (s, 2 H), 6.89 - 7.05 (m, 3 H), 7.17 - 7.32 (m, 2 H), 7.54 - 7.64 (m, 1 H), 7.81 (t, 1 H), 8.64 (d, 1 H). |
| | (72% d. Th.; Reinheit 84%) | |
| **189A** | *rac*-Benzyl-{1-[({8-[(2,6-difluorbenzyl)oxy]-2-methylimidazo[1,2-a]pyridin-3-yl)carbonyl)amino]-4,4,4-trifluorbutan-2-yl}carbamat Trifluoracetat | LC-MS (Methode 2): Rₜ = 0.96 min |
| | | MS (ESpos): m/z = 577.2 (M+H)⁺ |
| | 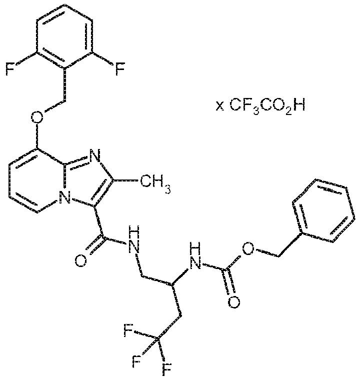 | |
| | (96% d. Th.) | |
| **190A** | *rac*-Benzyl-{1-[({8-[(2,6-difluorbenzyl)-oxy]-2,6-dimethylimidazo[1,2-a]pyridin-3-yl}carbonyl)amino]-4,4,4-trifluorbutan-2-yl}carbamat Trifluoracetat | LC-MS (Methode 2): Rₜ = 0.97 min |
| | | MS (ESpos): m/z = 591.3 (M+H)⁺ |
| | 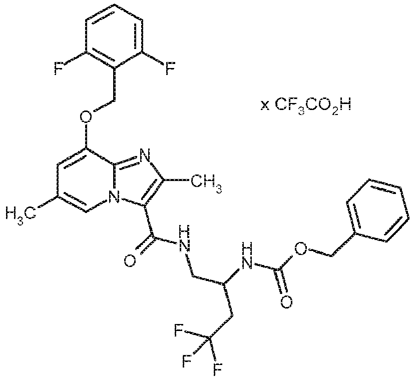 | |
| | (89% d. Th.; Reinheit 100%) | |

| | | |
|---|---|---|
| ¹⁾ Das Amin wurde im Überschuss eingesetzt, da die genaue Reinheit nicht bekannt war. | | |

### Beispiel 191A

### Benzyl-{(2S)-3-[({6-chlor-8-[(2,6-difluorbenzyl)oxy]-2-methylimidazo[1,2-a]pyridin-3-yl}carbonyl)amino]butan-2-yl}carbamat

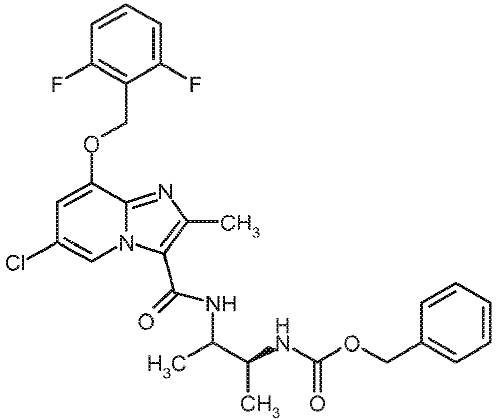

164 mg 6-Chlor-8-[(2,6-difluorbenzyl)oxy]-2-methylimidazo[1,2-a]pyridin-3-carbonsäure (Beispiel 16A, 0.47 mmol, 1 Äquivalent) wurden in 1.4 ml DMF gelöst und mit 145 mg Benzyl-[(2*S*)-3-aminobutan-2-yl]carbamat (Beispiel 141A, 0.65 mmol, 1.4 Äquivalente), 230 mg *O*-(7-Azabenzotriazol-1-yl)-*N*,*N*,*N*'*N*'-tetramethyluroniumhexafluorphosphat (HATU, 0.6 mmol, 1.3 Äquivalente) und 180.7 mg *N,N*-Diisopropylethylamin (0.23 ml, 1.4 mmol, 3 Äquivalente) versetzt und es wurde über Nacht bei RT gerührt. Anschließend wurde mit einem Tropfen Wasser versetzt und der ausgefallene Feststoff abfiltriert und im Vakuum getrocknet. Es wurden 160 mg (53% d. Th.; Reinheit 86%) der Titelverbindung als farblose Kristalle erhalten.
LC-MS (Methode 1): Rₜ = 2.50 min
MS (ESpos): m/z = 557.1 (M+H)⁺

### Beispiel 192A

### tert.-Butyl-(2S)-2-{[({8-[(2,6-difluorbenzyl)oxy]-2,6-dimethylimidazo[1,2-a]pyridin-3-yl}carbonyl)amino]methyl}pyrrolidin-1-carboxylat Trifluoracetat

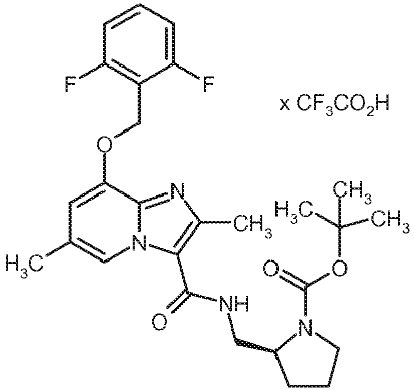

75 mg 8-[(2,6-Difluorbenzyl)oxy]-2,6-dimethylimidazo[1,2-a]pyridin-3-carbonsäure (Beispiel 21A, 0.23 mmol, 1 Äquivalent) wurden mit 90 mg *O*-(7-Azabenzotriazol-1-yl)-*N*,*N*,*N*'*N*'-tetramethyluroniumhexafluorphosphat (HATU, 0.24 mmol, 1.05 Äquivalente) und 88 mg *N,N-*Diisopropylethylamin (0.12 ml, 0.68 mmol, 3 Äquivalente) in 1.4 ml DMF vorgelegt und 20 min bei RT gerührt. Dann wurden 50 mg (0.25 mmol, 1.1 Äquivalente) tert.-Butyl-(2*S*)-2-(aminomethyl)pyrrolidin-1-carboxylat zugegeben und Es rührte über Nacht bei RT. Die Reaktionslösung wurde dann mit Wasser/TFA verdünnt und über die präparative HPLC (Methode: RP18 Säule, Laufmittel: Acetonitril/Wasser-Gradient unter Zusatz von 0.1% TFA) gereinigt. Es wurden 143 mg (99% d. Th.; Reinheit 98%) der Titelverbindung erhalten.
LC-MS (Methode 2): Rₜ = 1.02 min
MS (ESpos): m/z = 515.3 (M-TFA+H)⁺

### Beispiel 193A

### tert.-butyl-(2R)-2-{[({8-[(2,6-difluorbenzyl)oxy]-2,6-dimethylimidazo[1,2-a]pyridin-3-yl}carbonyl)amino]methyl}pyrrolidin-1-carboxylat Trifluoracetat

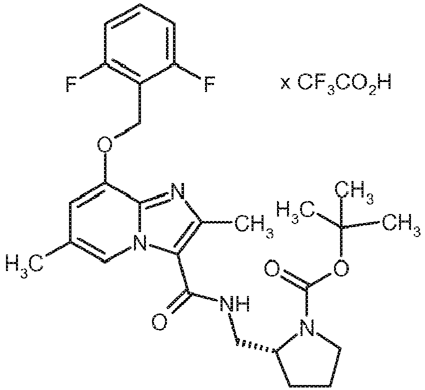

75 mg 8-[(2,6-Difluorbenzyl)oxy]-2,6-dimethylimidazo[1,2-a]pyridin-3-carbonsäure (Beispiel 21A, 0.23 mmol, 1 Äquivalent) wurden mit 90 mg *O*-(7-Azabenzotriazol-1-yl)-*N*,*N*,*N*',*N*'-tetramethyluroniumhexafluorphosphat (HATU, 0.24 mmol, 1.05 Äquivalente) und 88 mg *N,N-*Diisopropylethylamin (0.12 ml, 0.68 mmol, 3 Äquivalente) in 1.4 ml DMF vorgelegt und 20 min bei RT gerührt. Dann wurden 50 mg (0.25 mmol, 1.1 Äquivalente) tert.-Butyl-(2*R*)-2-(aminomethyl)pyrrolidin-1-carboxylat zugegeben und über Nacht bei RT gerührt. Die Reaktionslösung wurde mit Wasser/TFA verdünnt und über präparative HPLC (Methode: RP18 Säule, Laufmittel: Acetonitril/Wasser-Gradient unter Zusatz von 0.1% TFA) gereinigt. Es wurden 108 mg (76% d. Th.; Reinheit 100%) der Titelverbindung erhalten.
LC-MS (Methode 2): Rt = 1.02 min
MS (ESpos): m/z = 515.3 (M-TFA+H)⁺

### Beispiel 194A

### 8-[(2,6-Difluorbenzyl)oxy]-2-methyl-N-(2-oxoethyl)imidazo[1,2-a]pyridin-3-carboxamid

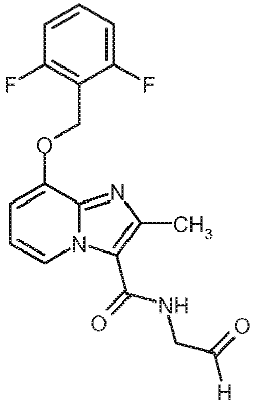

Unter Argon wurden 1.65 g 1,1,1-Triacetoxy-1lambda⁵,2-benziodoxol-3(1H)-on (Dess-Martin-Periodinan, 3.9 mmol, 1.5 Äquivalente) in 20 ml Dichlormethan vorgelegt. Bei -25°C wurde langsam eine Lösung von 1 g 8-[(2,6-Difluorbenzyl)oxy]-*N*-(2-hydroxyethyl)-2-methylimidazo-[1,2-a]pyridin-3-carboxamid (Beispiel 175A, 2.6 mmol, 1 Äquivalent) in 32 ml Dichlormethan zugetropft. Das Reaktionsgemisch wurde, auf RT kommend, über Nacht gerührt. Dann wurde die Reaktionslösung mit ca. 160 ml Essigsäureethylester verdünnt und dreimal mit 1 N wässriger Natronlauge gewaschen. Die organische Phase wurde mit gesättigter, wässriger Natriumchlorid-Lösung neutral gewaschen, über Natriumsulfat getrocknet, filtriert und eingeengt. Es wurden 879 mg (47% d. Th.; Reinheit ca. 50%) der Titelverbindung erhalten und ohne Reinigung weiter umgesetzt.
LC-MS (Methode 2): Rₜ = 0.56 min
MS (ESneg): m/z = 358 (M-H)⁻

### Beispiel 195A

### 8-[(2,6-Difluorbenzyl)oxy]-2-methyl-N-[(2R)-1-oxopropan-2-yl]imidazo[1,2-a]pyridin-3-carboxamid

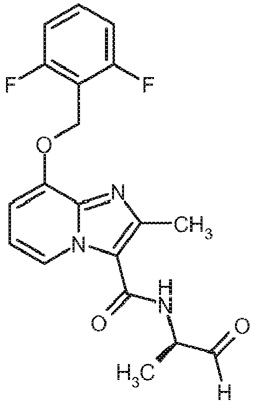

500 mg 8-[(2,6-Difluorbenzyl)oxy]-*N*-[(2*R*)-1-hydroxypropan-2-yl]-2-methylimidazo[1,2-a]pyridin-3-carboxamid (Beispiel 177A, 1.33 mmol) wurden in Analogie zu Beispiel 194A mit 847 mg (2.0 mmol) Dess-Martin-Periodinan unter Zusatz von 0.11 ml Pyridin (105 mg, 1.33 mmol) oxidiert und aufgearbeitet. Es wurden 440 mg (53% d. Th.; Reinheit 60%) der Titelverbindung erhalten und ohne Reinigung weiter umgesetzt.
LC-MS (Methode 2): Rₜ = 0.64 min
MS (ESneg): m/z = 372.1 (M-H)⁺

### Beispiel 196A

### 8-[(2,6-Difluorbenzyl)oxy]-2-methyl-N-[(2S)-1-oxopropan-2-yl]imidazo[1,2-a]pyridin-3-carboxamid

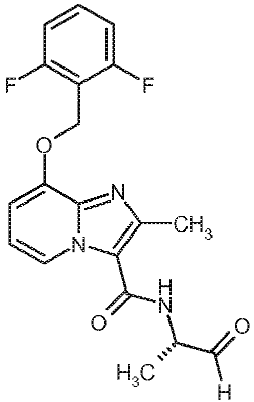

500 mg 8-[(2,6-Difluorbenzyl)oxy]-*N*-[(2*S*)-1-hydroxypropan-2-yl]-2-methylimidazo[1,2-a]pyridin-3-carboxamid (Beispiel 178A, 1.33 mmol, 1 Äquivalent) wurden in Analogie zu Beispiel 194A mit Dess-Martin-Periodinan unter Zusatz von 0.11 ml Pyridin (105 mg, 1.33 mmol, 1 Äquivalent) oxidiert und aufgearbeitet. Es wurden 439 mg (53% d. Th.; Reinheit 60%) der Titelverbindung erhalten und ohne Reinigung weiter umgesetzt.
LC-MS (Methode 5): Rₜ = 1.49 min
MS (ESneg): m/z = 372.1 (M-H)⁺

### Beispiel 197A

### ent-tert.-Butyl-{2-(3,4-difluorphenyl)-2-[({8-[(2-fluorbenzyl)oxy]-2-methylimidazo[1,2-a]pyridin-3-yl}carbonyl)amino]ethyl}carbamat

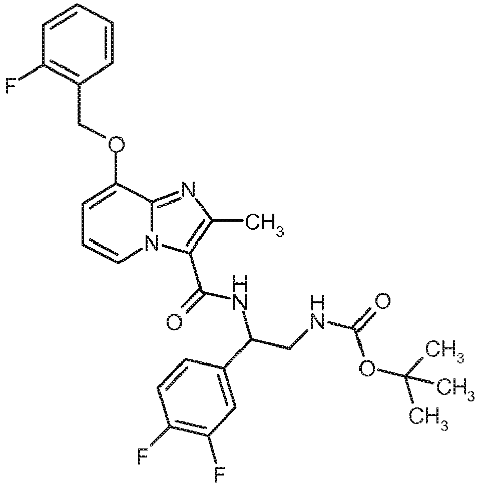

200 mg *ent*-tert.-Butyl-[2-(3,4-difluorphenyl)-2-{[(8-hydroxy-2-methylimidazo[1,2-a]pyridin-3-yl)carbonyl]amino}ethyl]carbamat (Beispiel 131A, 0.45 mmol, 1 Äquivalent) wurden mit 93 mg 2-Fluorbenzylbromid (0.49 mmol, 1.1 Äquivalente) und 321 mg Cäsiumcarbonat (0.99 mmol, 2.2 Äquivalente) in 10 ml DMF über Nacht bei RT gerührt. Dann wurden 150 ml Wasser zugefügt und 15 min bei RT nachgerührt. Das ausgefallene Produkt filtrierte man ab und trocknete es im Vakuum. Man erhielt 190 mg (79% d. Th.; Reinheit 91%) der Titelverbindung.
LC-MS (Methode 1): Rₜ = 1.06 min
MS (ESpos): m/z = 555.3 (M+H)⁺
¹H-NMR (400 MHz, DMSO-d₆): δ = 1.33 (s, 9 H), 2.58 (s, 3 H), 3.37 - 3.47 (m, 2 H), 5.10 - 5.22 (m, 1 H), 5.32 (s, 2 H), 6.89 (t, 1 H), 6.97 (d, 1 H), 7.08 (t, 1 H), 7.20 - 7.34 (m, 3 H), 7.37 - 7.52 (m, 3 H), 7.61 (t, 1 H), 8.22 (d, 1 H), 8.53 (d, 1 H).

In Analogie zu Beispiel 197A wurden die in Tabelle 14A gezeigten Beispiele hergestellt, indem *ent*-tert.-Butyl-[2-(3,4-difluorphenyl)-2-{[(8-hydroxy-2-methylimidazo[1,2a]pyridin-3-yl)carbonyl]amino}ethyl]carbamat (Beispiel 131A) mit den entsprechenden, kommerziell erhältlichen Benzylbromiden umgesetzt wurde:

**Tabelle 14A:**

| **Beispiel** | **IUPAC-Name / Struktur (Ausbeute)** | **Analytische Daten** |
|---|---|---|
| **198A** | *ent*-tert.-Butyl-{2-[({8-[(2,3-difluorbenzyl)oxy]-2-methylimidazo[1,2-a]pyridin-3-yl}carbonyl)amino]-2-(3,4-difluorphenyl)ethyl}carbamat | LC-MS (Methode 1): Rₜ = 1.33 min |
| | | MS (ESpos): m/z = 573.1 (M+H)⁺ |
| | | ¹H-NMR (400 MHz, DMSO-d₆): |
| | 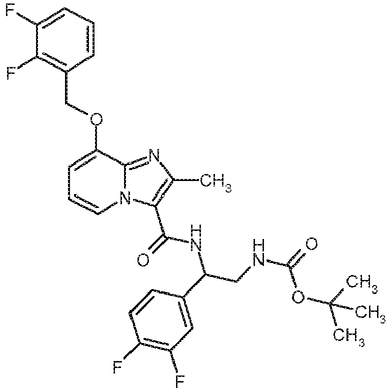 | 8 = 1.33 (s, 9 H), 2.60 (s, 3 H), 3.37 - 3.47 (m, 2 H), 5.11 - 5.20 (m, 1 H), 5.39 (s, 2 H), 6.90 (t, 1 H), 6.98 (d, 1 H), 7.09 (t, 1 H), 7.21 - 7.33 (m, 2 H), 7.37 - 7.55 (m, 4 H), 8.23 (d, 1 H), 8.54 (d, 1 H). |
| | (86% d. Th.; Reinheit 85%) | |
| **199A** | *ent*-tert.-Butyl-{2-(3,4-difluorphenyl)-2-[({2-methyl-8-[(2,3,6-trifluorbenzyl)oxy]imidazo[1,2-a]pyridin-3-yl}carbonyl)amino]ethyl}carbamat | LC-MS (Methode 1): Rₜ = 1.33 min |
| | | MS (ESpos): m/z = 591.1 (M+H)⁺ |
| | | ¹H-NMR (400 MHz, DMSO-d₆): |
| | 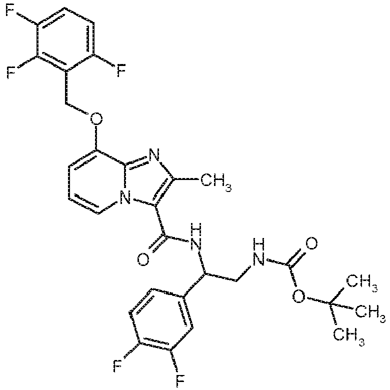 | δ = 1.32 (s, 9 H), 2.58 (s, 3 H), 3.37 - 3.46 (m, 2 H), 5.11 - 5.20 (m, 1 H), 5.35 (s, 2 H), 6.92 (t, 1 H), 7.02 (d, 1 H), 7.08 (t, 1 H), 7.19 - 7.33 (m, 2 H), 7.35 - 7.52 (m, 2 H), 7.60 - 7.72 (m, 1 H), 8.22 (d, 1 H), 8.56 (d, 1 H). |
| | (94% d. Th.; Reinheit 100%) | |
| **200A** | *ent*-tert.-Butyl-{2-(3,4-difluorphenyl)-2-[({2-methyl-8-[(2,4,6-trifluorbenzyl)oxy]imidazo[1,2-a]pyridin-3-yl}carbonyl)amino]ethyl}carbamat | LC-MS (Methode 1): Rₜ = 1.33 min |
| | | MS (ESpos): m/z = 591.1 (M+H)⁺ |
| | | ¹H-NMR (400 MHz, DMSO-d₆): |
| | 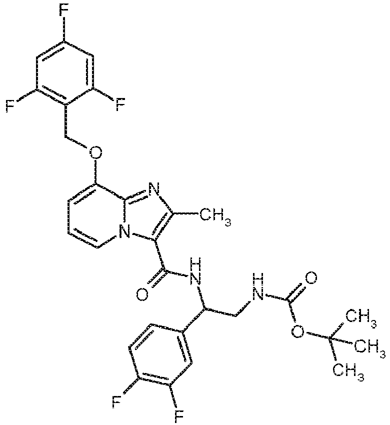 | δ = 1.32 (s, 9 H), 2.58 (s, 3 H), 3.37 - 3.46 (m, 2 H), 5.11 - 5.20 (m, 1 H), 5.27 (s, 2 H), 6.92 (t, 1 H), 7.01 (d, 1 H), 7.09 (t, 1 H), 7.21 - 7.27 (m, 1 H), 7.30 - 7.50 (m, 4 H), 8.21 (d, 1 H), 8.55 (d, 1 H). |
| | (76% d. Th.; Reinheit 90%) | |
| **201A** | *ent*-tert.-Butyl-{2-[({8-[(2-chlor-6-fluorbenzyl)oxy]-2-methylimidazo[1,2-a]pyridin-3-yl}carbonyl)-amino]-2-(3,4-difluorphenyl)ethyl}carbamat | LC-MS (Methode 1): Rₜ = 1.36 min |
| | | MS (ESpos): m/z = 589.1 (M+H)⁺ |
| | 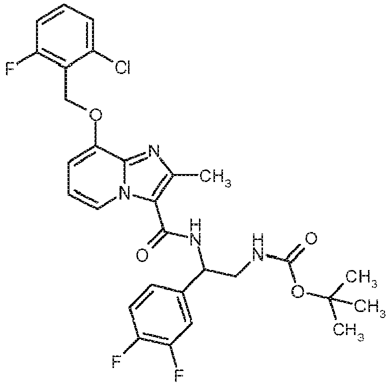 | ¹H-NMR (400 MHz, DMSO-d₆): δ = 1.33 (s, 9 H), 2.58 (s, 3 H), 3.36 - 3.46 (m, 2 H), 5.11 - 5.20 (m, 1 H), 5.33 (s, 2 H), 6.92 (t, 1 H), 7.01 - 7.12 (m, 2 H), 7.21 - 7.27 (m, 1 H), 7.33 - 7.51 (m, 4 H), 7.54 - 7.62 (m, 1 H), 8.21 (d, 1 H), 8.54 (d, 1 H). |
| | (85% d. Th.; Reinheit 90%) | |

### Beispiel 202A:

### ent-tert.-Butyl-4-[({8-[(2,6-difluorbenzyl)oxy]-2-methylimidazo[1,2-a]pyridin-3-yl}carbonyl)amino]-6-fluor-3,4-dihydrochinolin-1(2H)-carboxylat (Enantiomer A)

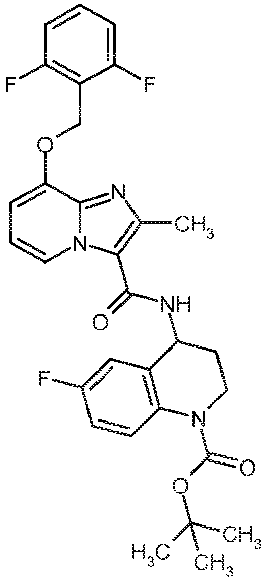

198 mg Beispiel 172A wurden an chiraler Phase in die Enantiomere getrennt [Säule: Daicel Chiralpak AD-H, 5 µm, 250 x 20 mm, Eluent: 25% iso-Hexan, 75% Ethanol, Fluß: 15 ml/min; 40°C, Detektion: 220 nm].
Ausbeute: Enantiomer A: 76 mg (99% ee)
Enantiomer A: Rₜ = 4.20 min [Daicel Chiralpak AD-H, 5 µm, 250 x 4.6 mm, Eluent: 25% iso-Hexan, 75% Ethanol, Fluß: 1 ml/min; 40°C, Detektion: 220 nm].

### Beispiel 203A:

### ent-tert.-Butyl-4-[(18-[(2,6-difluorbenzyl)oxy]-2-methylimidazo[1,2-a]pyridin-3-yl}carbonyl)amino]-6-fluor-3,4-dihydrochinolin-1(2H)-carboxylat (Enantiomer B)

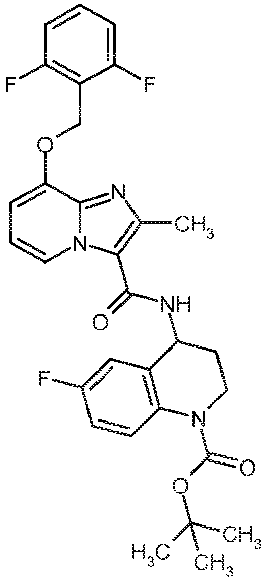

198 mg Beispiel 172A wurden an chiraler Phase in die Enantiomere getrennt [Säule: Daicel Chiralpak AD-H, 5 µm, 250 x 20 mm, Eluent: 25% iso-Hexan, 75% Ethanol, Fluß: 15 ml/min; 40°C, Detektion: 220 nm].
Ausbeute Enantiomer B: 76 mg (99% ee)
Enantiomer B: Rₜ = 9.13 min [Daicel Chiralpak AD-H, 5 µm, 250 x 4.6 mm, Eluent: 25% iso-Hexan, 75% Ethanol, Fluß: 1 ml/min; 40°C, Detektion: 220 nm].

### Beispiel 204A:

### ent-tert.-Butyl-4-[({6-chlor-8-[(2,6-difluorbenzyl)oxy]-2-methylimidazo[1,2-a]pyridin-3-yl}carbonyl)amino]-3,4-dihydrochinolin-1(2H)carboxylat (Enantiomer A)

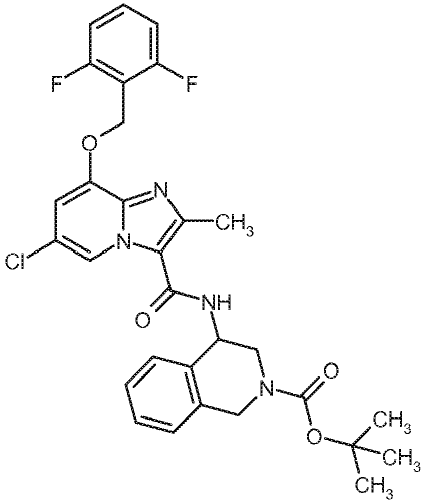

190 mg Beispiel 180A wurden an chiraler Phase in die Enantiomere getrennt [Säule: Daicel Chiralpak OZ-H, 5 µm, 250 x 20 mm, Eluent: 30% iso-Hexan, 70% Ethanol + 0.2% Diethylamin, Fluß: 15 ml/min; 40°C, Detektion: 220 nm].
Ausbeute: Enantiomer A: 64 mg (99% ee)
Enantiomer A: Rₜ = 4.84 min [Daicel Chiralpak OZ-H, 5 µm, 250 x 4.6 mm, Eluent: 30% iso-Hexan, 70% Ethanol + 0.2% Diethylamin, Fluß: 1 ml/min; 40°C, Detektion: 220 nm].

### Beispiel 205A:

### ent-tert.-Butyl-4-[({6-chlor-8-[(2,6-difluorbenzyl)oxy]-2-methylimidazo[1,2-a]pyridin-3-yl}carbonyl)amino]-3,4-dihydrochinolin-1(2H)carboxylat (Enantiomer B)

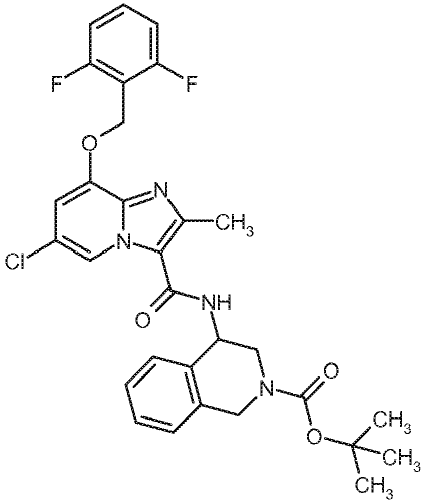

190 mg Beispiel 180A wurden an chiraler Phase in die Enantiomere getrennt [Säule: Daicel Chiralpak OZ-H, 5 µm, 250 x 20 mm, Eluent: 30% iso-Hexan, 70% Ethanol + 0.2% Diethylamin, Fluß: 15 ml/min; 40°C, Detektion: 220 nm].
Ausbeute: Enantiomer B: 65 mg (99% ee)
Enantiomer B: Rₜ = 5.62 min [Daicel Chiralpak OZ-H, 5 µm, 250 x 4.6 mm, Eluent: 30% iso-Hexan, 70% Ethanol + 0.2% Diethylamin, Fluß: 1 ml/min; 40°C, Detektion: 220 nm].

### Beispiel 206A:

### ent-tert.-Butyl-4-[({8-[(2,6-difluorbenzyl)oxy]-2-methylimidazo[1,2-a]pyridin-3-yl}carbonyl)amino]-3,4-dihydrochinolin-1(2H)-carboxylat (Enantiomer A)

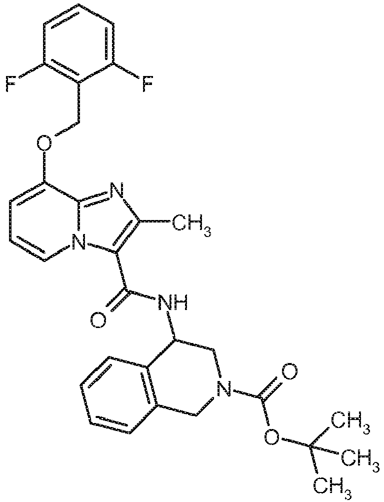

188 mg Beispiel 173A wurden an chiraler Phase in die Enantiomere getrennt [Säule: Daicel Chiralpak OZ-H, 5 µm, 250 x 20 mm, Eluent: 25% iso-Hexan, 75% Ethanol + 0.2% Diethylamin, Fluß: 15 ml/min; 40°C, Detektion: 220 nm].
Ausbeute Enantiomer A: 80 mg (99% ee)
Enantiomer A: Rₜ = 7.44 min [Daicel Chiralpak OZ-H, 5 µm, 250 x 4.6 mm, Eluent: 25% iso-Hexan, 75% Ethanol + 0.2% Diethylamin, Fluß: 1.0 ml/min; 40°C, Detektion: 220 nm].

### Beispiel 207A:

### ent-tert.-Butyl-4-[({8-[(2,6-difluorbenzyl)oxy]-2-methylimidazo[1,2-a]pyridin-3-yl}carbonyl)amino]-3,4-dihydrochinolin-1(2H)-carboxylat (Enantiomer B)

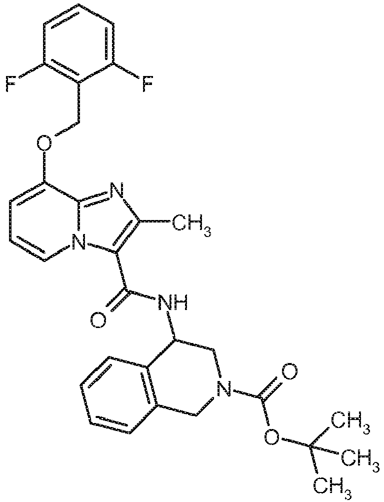

188 mg Beispiel 173A wurden an chiraler Phase in die Enantiomere getrennt [Säule: Daicel Chiralpak OZ-H, 5 µm, 250 x 20 mm, Eluent: 25% iso-Hexan, 75% Ethanol + 0.2% Diethylamin, Fluß: 15 ml/min; 40°C, Detektion: 220 nm].
Ausbeute Enantiomer B: 81 mg (99% ee)
Enantiomer B: 10.05 min [Daicel Chiralpak OZ-H, 5 µm, 250 x 4.6 mm, Eluent: 25% iso-Hexan, 75% Ethanol + 0.2% Diethylamin, Fluß: 1.0 ml/min; 40°C, Detektion: 220 nm].

### Beispiel 208A:

### ent-tert.-Butyl-4-[({8-[(2,6-difluorbenzyl)oxy]-2-methylimidazo[1,2-a]pyridin-3-yl}carbonyl)amino]-6-(trifluormethyl)-3,4-dihydrochinolin-1(2H)-carboxylat (Enantiomer A)

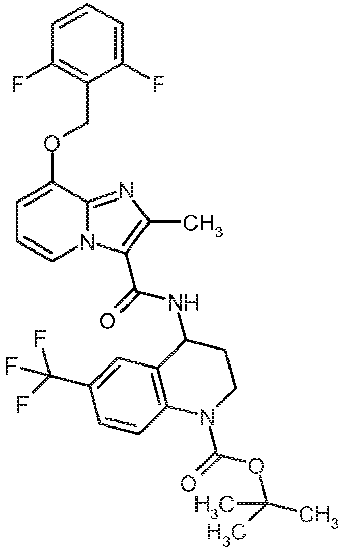

149 mg Beispiel 174A wurden an chiraler Phase in die Enantiomere getrennt [Säule: Daicel Chiralpak AD-H, 5 µm, 250 x 20 mm, Eluent: 50% iso-Hexan, 50% Isopropanol, Fluß: 20 ml/min; 25°C, Detektion: 230 nm].
Ausbeute Enantiomer A: 56 mg (100% ee)
Enantiomer A: Rt = 3.90 min [Daicel Chiralpak AD-H, 5µm, 250 x 4.6 mm; Eluent: 50% iso-Hexan, 50% Isopropanol; Fluss 1.0 ml/min; 25°C; Detektion: 220 nm].

### Beispiel 209A:

### ent-tert.-Butyl-4-[({8-[(2,6-difluorbenzyl)oxy]-2-methylimidazo[1,2-a]pyridin-3-yl}carbonyl)amino]-6-(trifluormethyl)-3,4-dihydrochinolin-1(2H)-carboxylat (Enantiomer B)

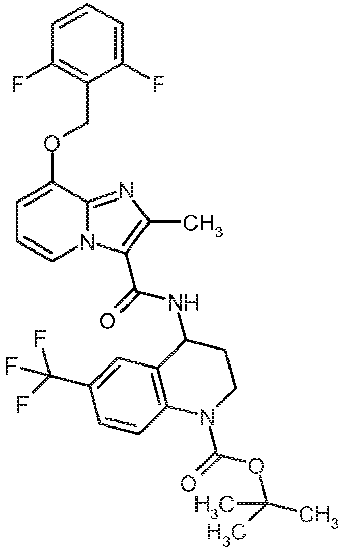

149 mg Beispiel 174A wurden an chiraler Phase in die Enantiomere getrennt [Säule: Daicel Chiralpak AD-H, 5 µm, 250 x 20 mm, Eluent: 50% iso-Hexan, 50% Isopropanol, Fluß: 20 ml/min; 25°C, Detektion: 230 nm].
Ausbeute Enantiomer B: 55 mg (100% ee)
Enantiomer B: Rₜ = 7.60 min [Daicel Chiralpak AD-H, 5µm, 250 x 4.6 mm; Eluent: 50% iso-Hexan, 50% Isopropanol; Fluss 1.0 ml/min; 25°C; Detektion: 220 nm].

### Beispiel 210A:

### ent-Benzyl-{2-[({8-[(2,6-difluorbenzyl)oxy]-2-methylimidazo[1,2-a]pyridin-3-yl}carbonyl)amino]-3-methoxypropyl}carbamat (Enantiomer A)

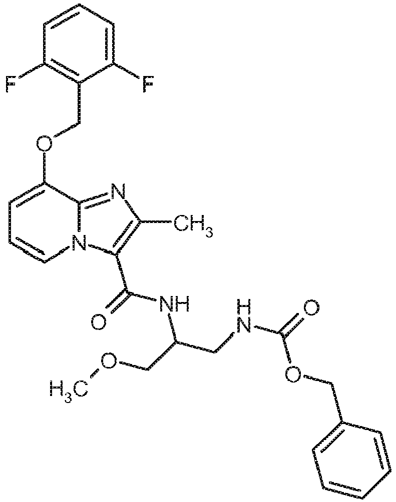

290 mg Beispiel 186A wurden in 3.5 ml Ethanol aufgelöst und an chiraler Phase in die Enantiomere getrennt [Säule: Daicel Chiralpak OD-H, 5 µm, 250 x 20 mm, Eluent: 50% iso-Hexan, 50% Ethanol, Fluß: 20 ml/min; 25°C, Detektion: 220 nm].
Ausbeute Enantiomer A: 58 mg (100% ee)
Enantiomer A: Rₜ = 5.47 min [Daicel Chiralpak OD-H, 5µm, 250 x 4.6 mm; Eluent: 50% iso-Hexan, 50% Ethanol; Fluss 1.0 ml/min; 30°C; Detektion: 220 nm].
¹H-NMR (400 MHz, DMSO-d₆): 8 = 2.55 (s, 3H; verdeckt durch DMSO-Signal), 3.20 - 3.35 (m, 2H), 3.25 (s, 3H), 3.39 - 3.49 (m, 2H), 4.20 - 4.31 (m, 1 H), 5.00 (s, 2H), 5.31 (s, 2H), 6.90 (t, 1H), 7.00 (d, 1 H), 7.20 - 7.30 (m, 7H), 7.40 (t, 1H), 7.52 (d, 1H), 7.54 - 7.64 (m, 1H), 8.60 (d, 1H).

### Beispiel 211A:

### ent-Benzyl-{2-[({8-[(2,6-difluorbenzyl)oxy]-2-methylimidazo[1,2-a]pyridin-3-yl}carbonyl)amino]-3-methoxypropyl}carbamat (Enantiomer B)

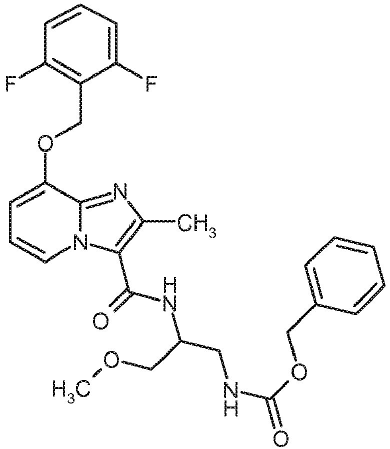

290 mg Beispiel 186A wurden in 3.5 ml Ethanol gelöst und an chiraler Phase in die Enantiomere getrennt [Säule: Daicel Chiralpak OD-H, 5 µm, 250 x 20 mm, Eluent: 50% iso-Hexan, 50% Ethanol, Fluß: 20 ml/min; 25°C, Detektion: 220 nm].
Ausbeute Enantiomer B: 53 mg (95% ee)
Enantiomer B: Rₜ = 6.998 min [Daicel Chiralpak OD-H, 5µm, 250 x 4.6 mm; Eluent: 50% iso-Hexan, 50% Ethanol; Fluss 1.0 ml/min; 30°C; Detektion: 220 nm].

### Beispiel 212A:

### ent-Benzyl-{2-[({8-[(2,6-difluorbenzyl)oxy]-2-methylimidazo[1,2-a]pyridin-3-yl}carbonyl)amino]-3-(3,4-difluorphenoxy)propyl}carbamat (Enantiomer B)

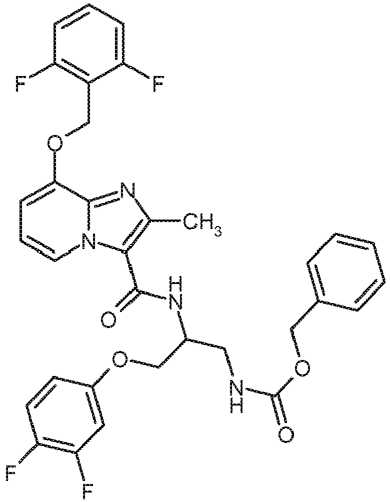

350 mg Beispiel 187A wurden an chiraler Phase in die Enantiomere getrennt [Säule: Daicel Chiralpak AH-H, 5 µm, 250 x 20 mm, Eluent: 25% iso-Hexan, 75% Ethanol, Fluß: 15 ml/min; 45°C, Detektion: 220 nm].
Ausbeute Enantiomer B: 110 mg (99% ee)
Enantiomer B: Rₜ = 7.66 min [Daicel Chiralcel OD-H, 5µm, 250 x 4.6 mm; Eluent: 50% iso-Hexan, 50% Ethanol; Fluss 1.0 ml/min; 40°C; Detektion: 220 nm].

### Beispiel 213A:

### ent-tert.-Butyl-{3-[({8-[(2,6-difluorbenzyl)oxy]-2-methylimidazo[1,2-a]pyridin-3-yl}carbonyl)amino]-2-methylpropyl}carbamat (Enantiomer A)

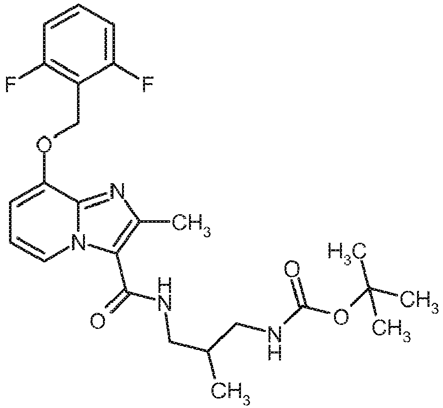

192 mg Beispiel 185A wurden an chiraler Phase in die Enantiomere getrennt [Säule: Daicel Chiralpak AD-H, 5 µm, 250 x 20 mm, Eluent: 70% iso-Hexan, 15% Ethanol, 15% Isopropanol, Fluß: 25 ml/min; 40°C, Detektion: 210 nm].
Ausbeute Enantiomer A: 68 mg (100 % ee)
Enantiomer A: Rₜ = 8.65 min [Daicel Chiralpak AD-H, 5 µm, 250 x 4.6 mm, Eluent: 70% iso-Hexan, 15% Ethanol, 15% Isopropanol, Fluß: 1.5 ml/min; 30°C, Detektion: 220 nm].

### Beispiel 214A:

### ent-tert.-Butyl-(3-[({8-[(2,6-difluorbenzyl)oxy]-2-methylimidazo[1,2-a]pyridin-3-yl)carbonyl)amino]-2-methylpropyl)carbamat (Enantiomer B)

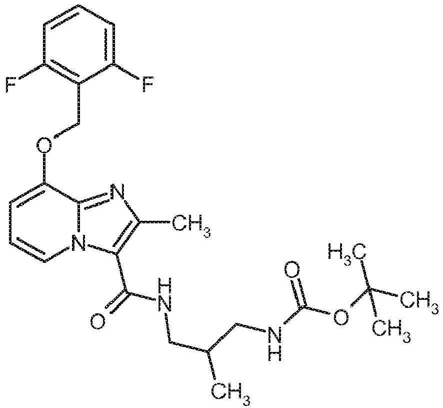

195 mg Beispiel 185A wurden an chiraler Phase in die Enantiomere getrennt [Säule: Daicel Chiralpak AD-H, 5 µm, 250 x 20 mm, Eluent: 70% iso-Hexan, 15% Ethanol, 15% Isopropanol, Fluß: 25 ml/min; 40°C, Detektion: 210 nm].
Ausbeute Enantiomer B: 79 mg (87 % ee)
Enantiomer B: 9.24 min [Daicel Chiralpak AD-H, 5 µm, 250 x 4.6 mm, Eluent: 70% iso-Hexan, 15% Ethanol, 15% Isopropanol, Fluß: 1.5 ml/min; 30°C, Detektion: 220 nm].

### Beispiel 215A

### ent-Benzyl-(1-[({8-[(2,6-difluorbenzyl)oxy]-2-methylimidazo[1,2-a]pyridin-3-yl}carbonyl)amino]-4,4,4-trifluorbutan-2-yl}carbamat (Enantiomer A)

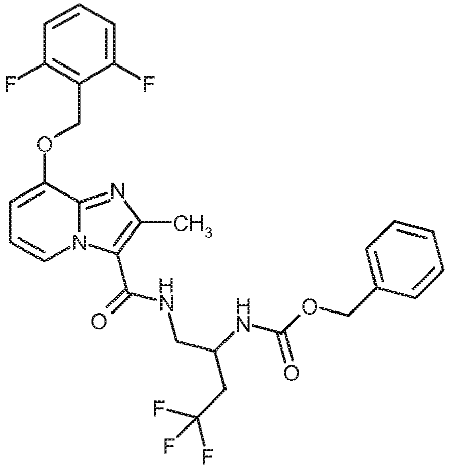

280 mg Beispiel 189A wurden an chiraler Phase in die Enantiomere getrennt [Säule: Daicel Chiralpak AZ-H, 5 µm, 250 x 20 mm, Eluent: 50% iso-Hexan, 50% Ethanol, Fluß: 18 ml/min; 40°C, Detektion: 210 nm].
Ausbeute Enantiomer A: 80 mg (100% ee)
Enantiomer A: Rt = 8.79 min [Daicel Chiralpak AZ-H, 5 µm, 250 x 4.6 mm, Eluent: 50% iso-Hexan, 50% Ethanol, Fluß: 1.0 ml/min; 30°C, Detektion: 220 nm].

### Beispiel 216A

### ent-Benzyl-{1-[({8-[(2,6-difluorbenzyl)oxy]-2-methylimidazo[1,2-a]pyridin-3-yl}carbonyl)amino]-4,4,4-trifluorbutan-2-yl}carbamat (Enantiomer B)

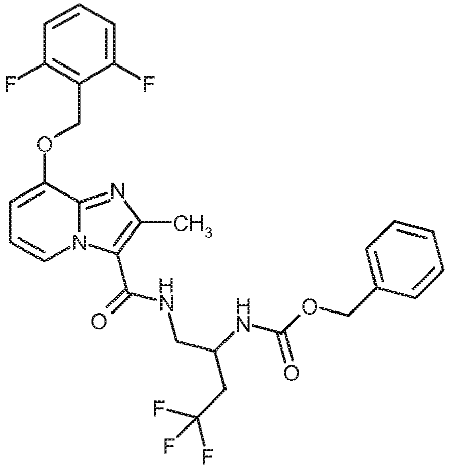

280 mg Beispiel 189A wurden an chiraler Phase in die Enantiomere getrennt [Säule: Daicel Chiralpak AZ-H, 5 µm, 250 x 20 mm, Eluent: 50% iso-Hexan, 50% Ethanol, Fluß: 18 ml/min; 40°C, Detektion: 210 nm].
Ausbeute Enantiomer B: 94 mg (99% ee)
Enantiomer B: Rₜ = 11.63 min [Daicel Chiralpak AZ-H, 5 µm, 250 x 4.6 mm, Eluent: 50% iso-Hexan, 50% Ethanol, Fluß: 1.0 ml/min; 30°C, Detektion: 220 nm].

### Beispiel 217A

### ent-Benzyl-{1-[({8-[(2,6-difluorbenzyl)oxy]-2,6-dimethylimidazo[1,2-a]pyridin-3-yl}carbonyl)amino]-4,4,4-trifluorbutan-2-yl} carbamat (Enantiomer A)

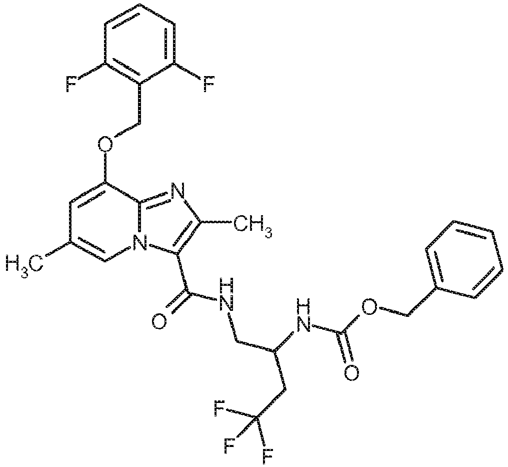

260 mg Beispiel 190A wurden an chiraler Phase in die Enantiomere getrennt [Säule: Daicel Chiralpak AD-H, 5 µm, 250 x 20 mm, Eluent: 50% iso-Hexan, 50% Isopropanol, Fluß: 15 ml/min; 40°C, Detektion: 220 nm].
Ausbeute Enantiomer A: 85 mg (>99% ee)
Enantiomer A: Rₜ = 4.81 min [Daicel Chiralpak AD-H, 5 µm, 250 x 4.6 mm, Eluent: 50% iso-Hexan, 50% Isopropanol + 0.2% TFA + 1% Wasser, Fluß: 1.0 ml/min; 45°C, Detektion: 220 nm].

### Beispiel 218A

### ent-Benzyl-{1-[({8-[(2,6-difluorbenzyl)oxy]-2,6-dimethylimidazo[1,2-a]pyridin-3-yl}carbonyl)amino]-4,4,4-trifluorbutan-2-yl}carbamat (Enantiomer B)

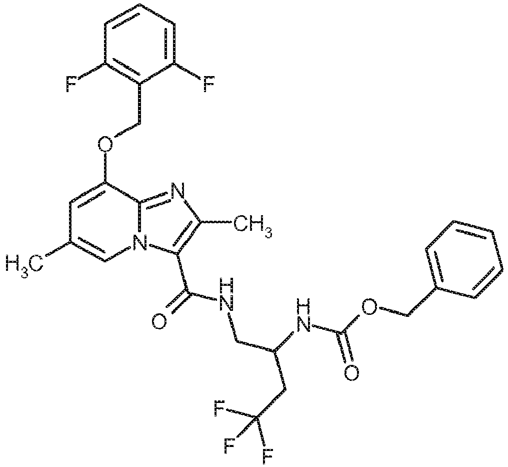

260 mg Beispiel 190A wurden an chiraler Phase in die Enantiomere getrennt [Säule: Daicel Chiralpak AD-H, 5 µm, 250 x 20 mm, Eluent: 50% iso-Hexan, 50% Isopropanol, Fluß: 15 ml/min; 40°C, Detektion: 220 nm].
Ausbeute Enantiomer B: 92 mg (99% ee)
Enantiomer B: Rₜ = 6.59 min [Daicel Chiralpak AD-H, 5 µm, 250 x 4.6 mm, Eluent: 50% iso-Hexan, 50% Isopropanol + 0.2% TFA + 1% Wasser, Fluß: 1.0 ml/min; 45°C, Detektion: 220 nm].

### Beispiel 219A

### 1-(Azetidin-3-yl)pyrrolidindihydrochlorid

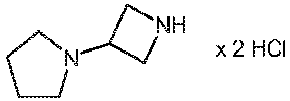

1.00 g (4.42 mmol) tert.-Butyl-3-(pyrrolidin-1-yl)azetidin-1-carboxylat wurde in 5.3 ml 1,4-Dioxan vorgelegt, mit 5.3 ml 4 N Salzsäure in 1,4-Dioxan versetzt und über Nacht bei RT gerührt. Dann wurde das Reaktionsgemisch eingeengt, mit Dichlormethan aufgeschäumt und am Hochvakuum getrocknet. Es wurden 950 mg (99% d. Th.; Reinheit 92%) der Titelverbindung erhalten.
¹H-NMR (400 MHz, DMSO-d₆): δ = 1.82 - 1.94 (m, 2 H), 1.94 - 2.05 (m, 2 H), 2.89 - 3.00 (m, 2 H), 4.07 - 4.19 (m, 2 H), 4.28 - 4.42 (m, 3 H), 9.11 (br. s, 1 H), 9.49 (br. s, 1 H), 12.33 (br. s, 1 H).

### Beispiel 220A

### tert.-Butyl-{2-[3-(pyrrolidin-1-yl)azetidin-1-yl]ethyl}carbamat

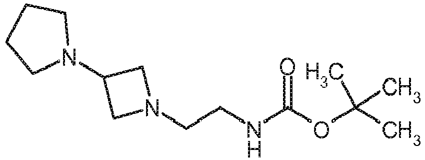

595 mg 1-(Azetidin-3-yl)pyrrolidindihydrochlorid (Beispiel 219A, 2.75 mmol, Reinheit ca. 92%, 1 Äquivalent) wurden in 3 Chargen umgesetzt. Um die freie Base zu erhalten wurde das 1-(Azetidin-3-yl)pyrrolidindihydrochlorid über eine StratoSpheres TM SPE Säule gegeben. Hierzu wurde die Säule zunächst mit 1 ml Methanol befeuchtet. Dann wurde 1-(Azetidin-3-yl)pyrrolidindihydrochlorid, in 3 ml Methanol gelöst, über die Säule gegeben und es wurde mit 3 ml Methanol nachgespült. Die erhaltene Lösung wurde eingeengt (die Säule fängt ca. 0.9 mmol Salz ab). Anschließend wurde die freie Base in 26.8 ml Acetonitril vorgelegt, mit 656 mg 2-(Boc-amino)ethylbromid (2.9 mmol, 1.05 Äquivalente) und 1.45 ml N,N-Diisopropylethylamin (8.25 mmol, 3 Äquivalente) versetzt und über Nacht bei 80°C gerührt. Das Reaktionsgemisch wurde eingeengt und durch Kieselgelchromatographie gereinigt (Eluent: Cyclohexan/Essigsäureethyester 5:1; Dichlormethan/Methanol 10:1; Dichlormethan/Methanol 4:1 und Dichlormethan/1 N Ammoniak in Methanol 4:1). Es wurden 463 mg (67% d. Th.) der Titelverbindung erhalten.
DCI-MS (Methode 4): m/z = 270.2 (M+H)⁺

### Beispiel 221A

### 2-[3-(Pyrrolidin-1-yl)azetidin-1-yl]ethanamindihydrochlorid

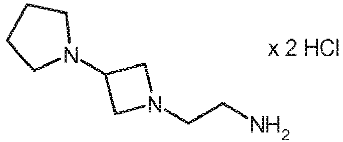

460 mg tert-Butyl-{2-[3-(pyrrolidin-1-yl)azetidin-1-yl]ethyl}carbamat (Beispiel 220A, 1.7 mmol, 1 Äquivalent) wurden in 8.54 ml 2 N Salzsäure in Diethylether und 1 ml Dioxan vorgelegt und über Nacht bei RT gerührt. Der ausgefalle Feststoff wurde abfiltriert, mit Diethylether gewaschen und im Vakuum getrocknet. Es wurden 390 mg (94% d. Th.) der Titelverbindung erhalten.
DCI-MS (Methode 4): m/z = 170.2 (M+H)⁺

### Beispiel 222A

### 1-(Aminomethyl)cyclobutanamin

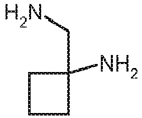

750 mg 1-Aminocyclobutancarbonitril (7.8 mmol, 1 Äquivalent) wurden in 16.5 ml THF vorgelegt, unter Argon bei 0°C tropfenweise mit 23.4 ml 1 N Lithiumaluminiumhydrid-Lösung in THF (23.4 mmol, 3 Äquivalente) versetzt und 3.5 h bei RT gerührt. Anschließend wurde die Reaktionslösung nacheinander mit 0.8 ml Wasser, 0.8 ml 2 N wässriger Natriumhydroxid-Lösung und 1.6 ml Wasser versetzt. Der entstandene Feststoff wurde abfiltiert und mit Methanol gewaschen. Das Filtrat wurde eingeengt. Es wurden 1980 mg (76% d. Th.; Annahme der Reinheit 30%) der Titelverbindung erhalten und ohne Reinigung weiter umgesetzt.
FIA-MS (Methode 10, ESpos): m/z = 101 (M+H)⁺

### Beispiel 223A

### rac-4,4,4-Trifluorbutan-1,2-diamindihydrochlorid

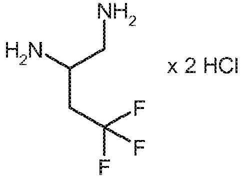

130 mg *rac*-Benzyl-(1-amino-4,4,4-trifluorbutan-2-yl)carbamat Trilfluoracetat (Beispiel 147 A, 0.33 mmol, 1 Äquivalent) wurden in 8.4 ml Methanol vorgelegt, unter Argon mit 35 mg (0.03 mmol) 10%igem Palladium auf Aktivkohle versetzt und über Nacht bei RT und Normaldruck hydriert. Dann wurde über einen Millipore^{®}-Filter filtiert, mit 0.33 ml 2 N Salzsäure in Diethylether (0.66 mmol, 2 Äquivalente) versetzt und eingeengt. Es wurden 72 mg (100% d. Th.) der Titelverbindung erhalten.
FIA-MS (Methode 10, ESpos): m/z = 143.0 (M-2HCl+H)⁺

### Beispiel 224A

### rac-3-Isopropoxypropan-1,2-diamin Dihydrochlorid

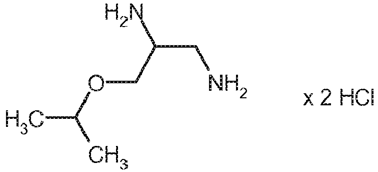

218 mg (0.71 mmol, 87% rein) *rac*-Benzyl-(2-amino-3-isopropoxypropyl)carbamat aus Beispiel 76A wurde unter Argon in Ethanol (5.0 ml) vorgelegt und mit 76 mg (0.07 mmol) 10%igem Palladium auf Aktivkohle und 2.2 ml (21.36 mmol) Cyclohexen versetzt. Die Reaktionsmischung wurde über Nacht unter Rückfluss gerührt. Anschließend wurde über einen Millipore^{®}-Filter filtriert, mit Ethanol gewaschen, das Filtrat mit 0.7 ml (1.42 mmol) 2 M Chlorwasserstoff in Diethylether versetzt, eingeengt und im Hochvakuum getrocknet. Man erhielt 190 mg (99% d. Th., Reinheit 76%) der Zielverbindung.
DCI-MS (Methode 4): m/z = 133 (M-2HCl+H)⁺

### Beispiel 225A

### 3-(Dibenzylamino)oxetan-3-carbonitril

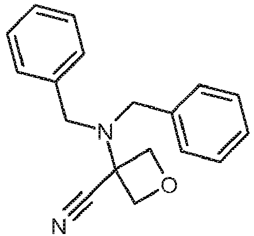

10.4 ml (54.1 mmol) Dibenzylamin in 72 ml Essigsäure wurden mit 0.63 ml (10.8 mmol) Oxetan-3-on und 3.6 ml (27.1 mmol) Trimethylsilylcyanid versetzt. Die Reaktionsmischung wurde anschließend über Nacht bei RT gerührt. Dann wurde eingeengt, der Rückstand in Wasser/Diethylether gelöst und die wässrige Phase zweimal mit Diethylether extrahiert. Die vereinigten organischen Phasen wurden zweimal mit gesättigter, wässriger Natriumhydrogencarbonat-Lösung gewaschen, über Natriumsulfat getrocknet, filtriert und eingeengt. Der Rückstand wurde mittels Kieselgel-Chromatographie (Cyclohexan/Essigsäureethylester 40:1 bis 30:1) gereinigt. Man erhielt 2.39 g (77% d. Th., Reinheit 97%) der Zielverbindung.
LC-MS (Methode 13): Rₜ = 2.53 min.
MS (ESIpos): m/z = 279 (M+H)⁺.
¹H-NMR (400 MHz, DMSO-d₆): 8 = 3.49 (s, 4H), 4.29 (d, 2H), 4.37 (d, 2H), 7.26 - 7.39 (m, 10H).

### Beispiel 226A

### 3-(Aminomethyl)oxetan-3-amin Dihydrochlorid

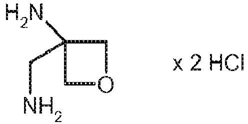

585 mg (2.07 mmol) 3-(Aminomethyl)-*N*,*N*-dibenzyloxetan-3-amin [Synthese beschrieben in: US2008/103183 A1, 2008; p. 48] wurde in Ethanol (29.2 ml) vorgelegt, mit 441 mg (0.41 mmol) 10%igem Palladium auf Aktivkohle und 6.3 ml (62.2 mmol) Cyclohexen versetzt. Die Reaktionsmischung wurde 8 h unter Rückfluss gerührt. Anschließend wurde über einen Millipore^{®}-Filter filtriert, mit Methanol gewaschen, das Filtrat mit 2.6 ml (5.2 mmol) 2 M Chlorwasserstoff in Diethylether versetzt, eingeengt und im Hochvakuum getrocknet. Man erhielt 423 mg (87% d. Th., Reinheit 75%) der Zielverbindung.
DCI-MS (Methode 4): m/z = 103 (M-2HCl+H)⁺

### Beispiel 227A

### rac-tert.-Butyl-{1-[({6-chlor-8-[(2,6-difluorbenzyl)oxy]-2-methylimidazo[1,2-a]pyridin-3-yl}carbanyl)amino]-3-methylbutan-2-yl}carbamat

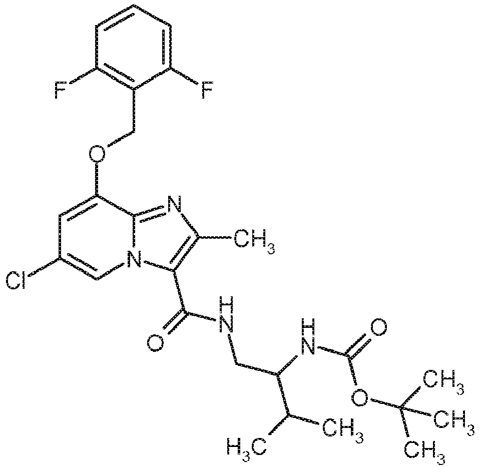

200 mg (0.57 mmol) 6-Chlor-8-[(2,6-difluorbenzyl)oxy]-2-methylimidazo[1,2-a]pyridin-3-carbonsäure Beispiel 16A, 191 mg (0.60 mmol) HATU und 0.19 ml (1.70 mmol) 4-Methylmorpholin in DMF (3.6 ml) wurden 20 min bei RT gerührt. Dann wurde mit 126 mg (0.62 mmol) *rac*-tert.-Butyl-(1-amino-3-methylbutan-2-yl)carbamat versetzt und die Reaktionsmischung über Nacht bei RT gerührt. Dann wurde mit Wasser/TFA versetzt und mittels präparativer RP-HPLC (Acetonitril/Wasser-Gradient unter Zusatz von 0.1% TFA) gereinigt. Die Produktfraktionen wurden eingeengt, der Rückstand in Dichlormethan aufgenommen und zweimal mit gesättigter, wässriger Natriumhydrogencarbonat-Lösung gewaschen. Die wässrige Phase wurde zweimal mit Dichlormethan extrahiert, die vereinigten organischen Phasen wurden über Natriumsulfat getrocknet, abfiltriert und eingeengt. Man erhielt 264 mg (87% d. Th., Reinheit 100%) der Zielverbindung.
LC-MS (Methode 2): Rₜ = 1.23 min.
MS (ESIpos): m/z = 537 (M+H)⁺.
¹H-NMR (400 MHz, DMSO-d₆): δ = 0.86 (d, 3H), 0.91 (d, 3H), 1.32 (s, 9H), 1.66 - 1.78 (m, 1H), 2.47 (s, 3H, Signal teilweise verdeckt durch DMSO-Peak), 3.20 - 3.29 (m, 1H), 3.38 - 3.46 (m, 1H), 3.49 - 3.60 (m, 1H), 5.34 (s, 2H), 6.62 (d, 1H), 7.19 (d, 1H), 7.22 - 7.29 (m, 2H), 7.56 - 7.66 (m, 1H), 7.81 (t, 1H), 8.74 (d, 1H).

### Beispiel 228A

### ent-tert.-Butyl-{1-[({6-chlor-8-[(2,6-difluorbenzyl)oxy]-2-methylimidazo[1,2-a]pyridin-3-yl)carbonyl)amino]-3-methylbutan-2-yl}carbamat (Enantiomer A)

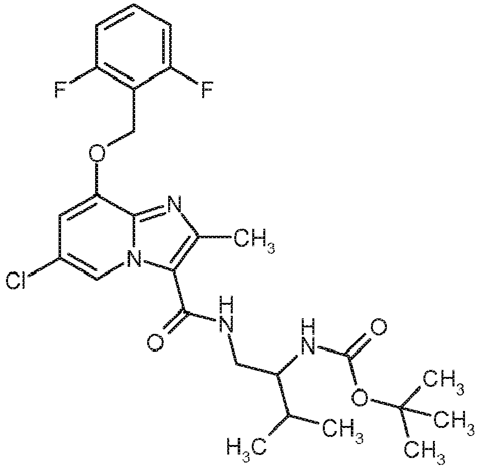

260 mg Beispiel 227A wurden durch präparative Trennung an chiraler Phase in die Enantiomere getrennt [Säule: Daicel Chiralpak AD-H, 5 µm, 250 x 20 mm, Eluent: 50% iso-Hexan, 50% Ethanol, Fluß: 20 ml/min; 40°C, Detektion: 220 nm].
Enantiomer A:
Ausbeute: 124 mg (99% ee)
Rₜ = 3.84 min [Daicel Chiralpak AD-H, 5µm, 250 x 4.6 mm; Eluent: 50% iso-Hexan, 50% Isopropanol; Fluss 1.0 ml/min; 40°C; Detektion: 220 nm].

### Beispiel 229A

### ent-tert.-Butyl-{1-[({6-chlor-8-[(2,6-difluorbenzyl)oxy]-2-methylimidazo[1,2,a]pyridin-3-yl}carbonyl)amino]-3-methylbutan-2-yl}carbamat (Enantiomer B)

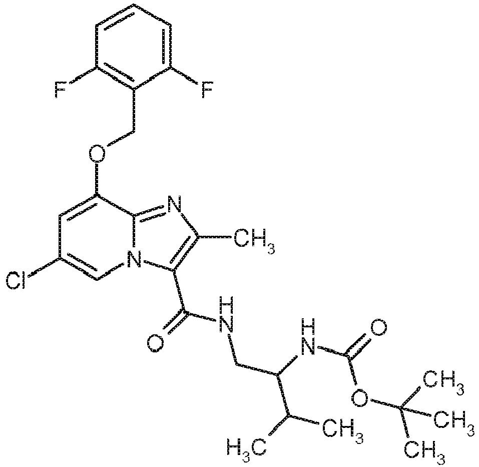

260 mg Beispiel 227A wurden durch präparative Trennung an chiraler Phase in die Enantiomere getrennt [Säule: Daicel Chiralpak AD-H, 5 µm, 250 x 20 mm, Eluent: 50% iso-Hexan, 50% Ethanol, Fluß: 20 ml/min; 40°C, Detektion: 220 nm].
Enantiomer B:
Ausbeute: 122 mg (99% ee)
Rₜ = 5.97 min [Daicel Chiralpak AD-H, 5µm, 250 x 4.6 mm; Eluent: 50% iso-Hexan, 50% Isopropanol; Fluss 1.0 ml/min; 40°C; Detektion: 220 nm].

### Beispiel 230A

### rac-tert.-Butyl-{1-[({8-[(2,6-difluorbenzyl)oxy]-2,6-dimethylimidazo[1,2-a]pyridin-3-yl}carbonyl)amino]-3-methylbutan-2-yl}carbamat

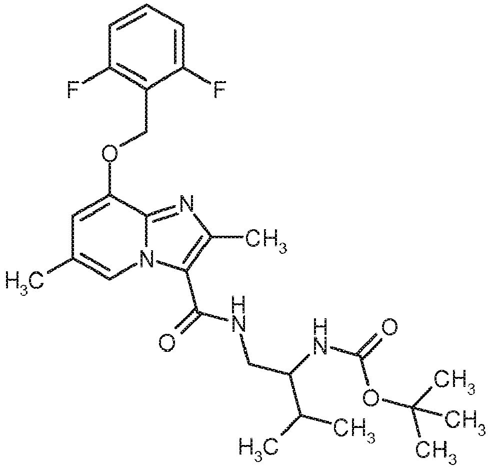

200 mg (0.60 mmol) 8-[(2,6-Difluorbenzyl)oxy]-2,6-dimethylimidazo[1,2-a]pyridin-3-carbonsäure aus Beispiel 21 A wurde mit 203 mg (0.63 mmol) HATU und 0.20 ml (1.81 mmol) 4-Methylmorpholin in DMF (3.8 ml) vorgelegt, 20 min bei RT gerührt, mit 134 mg (0.66 mmol) *rac-*tert.-Butyl-(1-amino-3-methylbutan-2-yl)carbamat versetzt und die Reaktionsmischung über Nacht bei RT gerührt. Das Gemisch wurde mit Wasser/TFA versetzt und mittels präparativer RP-HPLC (Acetonitril/Wasser-Gradient unter Zusatz von 0.1% TFA) gereinigt. Die Produktfraktionen wurden eingeengt, der Rückstand in Dichlormethan aufgenommen und zweimal mit gesättigter, wässriger Natriumhydrogencarbonat-Lösung gewaschen. Die wässrige Phase wurde zweimal mit Dichlormethan extrahiert, die vereinigten organischen Phasen wurden über Natriumsulfat getrocknet, abfiltriert und eingeengt. Man erhielt 262 mg (84% d. Th., Reinheit 100%) der Zielverbindung.
LC-MS (Methode 2): Rₜ = 1.03 min.
MS (ESIpos): m/z = 517 (M+H)⁺.
¹H-NMR (400 MHz, DMSO-d₆): δ = 0.86 (d, 3H), 0.90 (d, 3H), 1.33 (s, 9H), 1.68 - 1.78 (m, 1H), 2.31 (s, 3H), 2.46 (s, 3H), 3.18 - 3.28 (m, 1H), 3.38 - 3.46 (m, 1H), 3.50 - 3.59 (m, 1H), 5.28 (s, 2H), 6.63 (d, 1H), 6.91 (s, 1H), 7.20 - 7.28 (m, 2H), 7.55 - 7.64 (m, 1H), 7.67 (t, 1H), 8.47 (s, 1H).

### Beispiel 231A

### ent-tert.-Butyl-{1-[({8-[(2,6-difluorbenzyl)oxy]-2,6-dimethylimidazo[1,2-a]pyridin-3-yl}carbonyl)amino]-3-methylbutan-2-yl}carbamat (Enantiomer A)

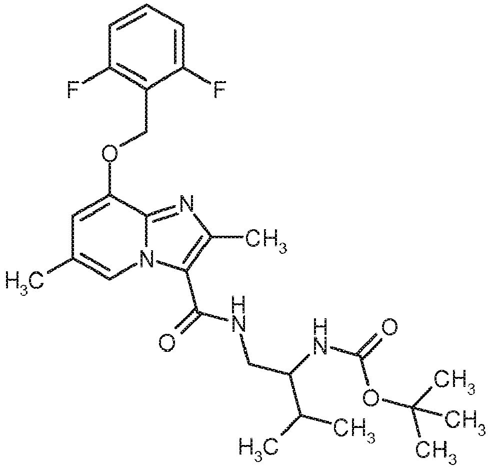

260 mg Beispiel 230A wurden durch präparative Trennung an chiraler Phase in die Enantiomere getrennt [Säule: Daicel Chiralpak IA, 5 µm, 250 x 20 mm, Eluent: 50% Acetonitril, 50% tert.-butyl-methylether, Fluß: 20 ml/min; 25°C, Detektion: 220 nm].
Enantiomer A:
Ausbeute: 89 mg (100% ee)
Rₜ = 4.04 min [Daicel Chiralpak IA, 5µm, 250 x 4.6 mm; Eluent: 50% Acetonitril, 50% tert.-butylmethylether; Fluss 1.0 ml/min; 30°C; Detektion: 220 nm].

### Beispiel 232A

### ent-tert.-Butyl-{1-[({8-[(2,6-difluorbenzyl)oxy]-2,6-dimethylimidazo[1,2-a]pyridin-3-yl}carbonyl)amino]-3-methylbutan-2-yl}carbamat (Enantiomer B)

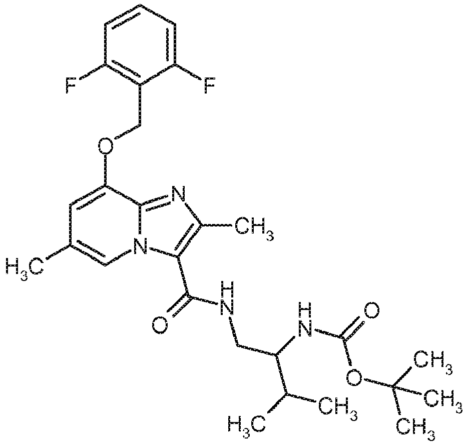

260 mg Beispiel 230A wurden durch präparative Trennung an chiraler Phase in die Enantiomere getrennt [Säule: Daicel Chiralpak IA, 5 µm, 250 x 20 mm, Eluent: 50% Acetonitril, 50% tert.-butyl-methylether, Fluß: 20 ml/min; 25°C, Detektion: 220 nm].
Enantiomer B:
Ausbeute: 93 mg (100% ee)
Rₜ = 6.02 min [Daicel Chiralpak IA, 5µm, 250 x 4.6 mm; Eluent: 50% Acetonitril, 50% tert.-butylmethylether; Fluss 1.0 ml/min; 30°C; Detektion: 220 nm].

### Beispiel 233A

### rac-tert.-Butyl-{1-[({8-[(2,6-difluorbenzyl)oxy]-2-methylimidazo[1,2-a]pyridin-3-yl}carbonyl)amino]-3-methylbutan-2-yl}carbamat

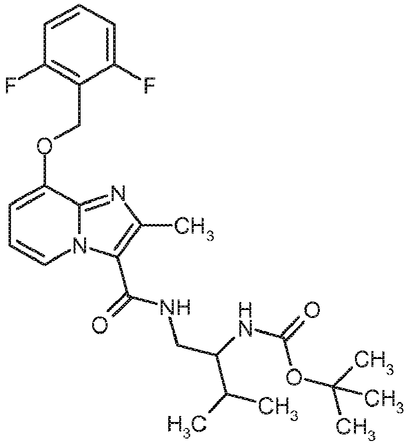

Darstellung und Reinigung der Titelverbindung erfolgte analog zum Beispiel 230A. Ausgehend von 200 mg (0.63 mmol) 8-[(2,6-Difluorbenzyl)oxy]-2-methylimidazo[1,2-a]pyridin-3-carbonsäure aus Beispiel 3A und 140 mg (0.69 mmol) *rac*-tert.-Butyl-(1-amino-3-methylbutan-2-yl)carbamat erhielt man 215 mg (68% d. Th., Reinheit 100%) der Zielverbindung.
LC-MS (Methode 2): Rₜ = 1.02 min.
MS (ESIpos): m/z = 503 (M+H)⁺.
¹H-NMR (400 MHz, DMSO-d₆): δ = 0.86 (d, 3H), 0.90 (d, 3H), 1.33 (s, 9H), 1.68 - 1.78 (m, 1H), 2.54 (s, 3H, verdeckt durch DMSO-Signal), 3.19 - 3.28 (m, 1H), 3.39 - 3.46 (m, 1H), 3.50 - 3.59 (m, 1H), 5.30 (s, 2H), 6.64 (d, 1H), 6.93 (t, 1H), 7.01 (d, 1H), 7.20 - 7.28 (m, 2H), 7.54 - 7.63 (m, 1H), 7.70 (t, 1H), 8.63 (d, 1H).

### Beispiel 234A

### ent-tert.-Butyl-{1-[({8-[(2,6-difluorbenzyl)oxy]-2-methylimidazo[1,2,a]pyridin-3-yl}carbonyl)amino]-3-methylbutan-2-yl}carbamat (Enantiomer A)

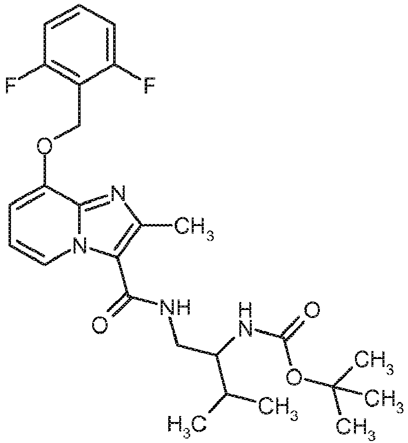

210 mg Beispiel 233A wurden durch präparative Trennung an chiraler Phase in die Enantiomere getrennt [Säule: Daicel Chiralpak IA, 5 µm, 250 x 20 mm, Eluent: 20% Acetonitril, 80% tert.-Butyl-methylether, Fluß: 20 ml/min; 25°C, Detektion: 230 nm].
Enantiomer A:
Ausbeute: 89 mg (100% ee)
Rₜ = 4.69 min [Daicel Chiralpak IA, 5µm, 250 x 4.6 mm; Eluent: 20% Acetonitril, 80% tert.-Butylmethylether; Fluss 1.0 ml/min; 30°C; Detektion: 220 nm].

### Beispiel 235A

### ent-tert.-Butyl-{1-[({8-[(2,6-difluorbenzyl)oxy]-2-methylimidazo[1,2-a]pyridin-3-yl}carbonyl)amino]-3-methylbutan-2-yl}carbamat (Enantiomer B)

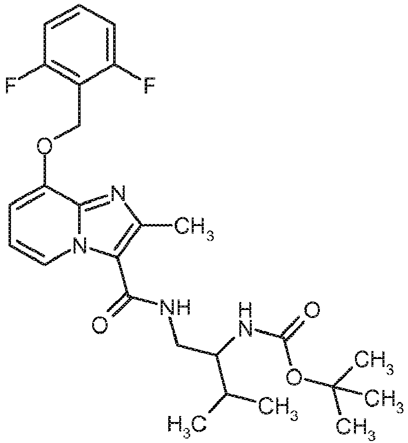

210 mg Beispiel 233A wurden durch präparative Trennung an chiraler Phase in die Enantiomere getrennt [Säule: Daicel Chiralpak IA, 5 µm, 250 x 20 mm, Eluent: 20% Acetonitril, 80% tert.-Butyl-methylether, Fluß: 20 ml/min; 25°C, Detektion: 230 nm].
Enantiomer B:
Ausbeute: 73 mg (100% ee)
Rₜ = 7.29 min [Daicel Chiralpak IA, 5µm, 250 x 4.6 mm; Eluent: 20% Acetonitril, 80% tert.-Butylmethylether; Fluss 1.0 ml/min; 30°C; Detektion: 220 nm].

### Beispiel 236A

### 3-(Benzyloxy)-5-brompyridin-2-amin

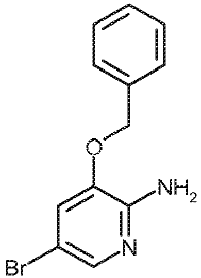

200 g (1 mol) 2-Amino-3-benzyloxypyridin wurden in 4 1 Dichlormethan vorgelegt und bei 0°C innerhalb von 30 min mit einer Lösung aus 62 ml (1.2 mol) Brom in 620 ml Dichlormethan versetzt. Nach beendeter Zugabe wurde die Reaktionslösung 60 min bei 0°C gerührt. Dann wurde das Gemisch mit ca. 4 1 gesättigter, wässriger Natriumhydrogencarbonat-Lösung versetzt. Die organische Phase wurde abgetrennt und eingeengt. Der Rückstand wurde mittels Kieselgelsäulechromatographie (Petrolether:Essigsäurethylester 6:4) gereinigt und die Produktfraktionen wurden eingeengt. Man erhielt 214 g (77% d. Th.) der Titelverbindung.
LC-MS (Methode 2): Rₜ = 0.92 min
MS (ESpos): m/z = 279 (M+H)⁺
¹H-NMR (400 MHz, DMSO-d₆): δ = 5.16 (s, 2H), 5.94 - 6.00 (m, 2H), 7.26 - 7.29 (m, 1H), 7.31 - 7.36 (m, 1H), 7.37 - 7.43 (m, 2H), 7.47-7.52 (m, 2H), 7.57 - 7.59 (m, 1H).

### Beispiel 237A

### Ethyl-8-(benzyloxy)-6-brom-2-methylimidazo[1,2-a]pyridin-3-carboxylat

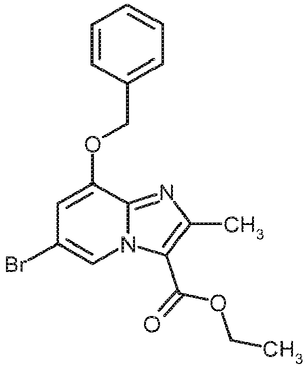

Unter Argon wurden 200 g (0.72 mol) 3-(Benzyloxy)-5-brompyridin-2-amin, 590 g (3.58 mol) Ethyl-2-chloracetoacetat und 436 g 3A Molekularsieb in 6 1 Ethanol suspendiert und 72 h bei Rückfluß gerührt. Die Reaktionsmischung wurde über Kieselgel abfiltriert und eingeengt. Der Rückstand wurde mittels Kieselgelchromatographie (Petrolether:Essigsäureethylester 9:1, anschließend 6:4) gereinigt und die Produktfraktionen wurden eingeengt. Man erhielt 221 g (79% d. Th.) der Zielverbindung.
LC-MS (Methode 7): Rₜ = 1.31 min
MS (ESpos): m/z = 389 (M+H)⁺
¹H-NMR (400 MHz, DMSO-d₆): δ = 1.36 (t, 3 H), 2.58 (s, 3 H), 4.32 - 4.41 (m, 2 H), 5.33 (s, 2 H), 7.28 - 7.32 (m, 1 H), 7.36 - 7.47 (m, 3 H), 7.49 - 7.54 (m, 2 H), 8.98 (d, 1 H).

### Beispiel 238A

### Ethyl-8-(benzyloxy)-2,6-dimethylimidazo[1,2-a]pyridin-3-carboxylat

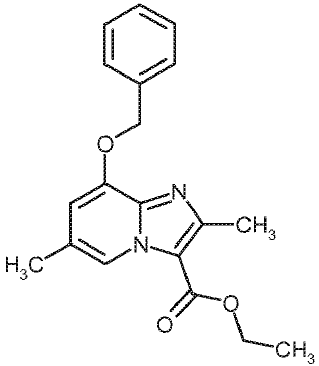

105 g (270 mmol) Ethyl-8-(benzyloxy)-6-brom-2-methylimidazo[1,2-a]pyridin-3-carboxylat aus Beispiel 237A wurden unter Argon in 4.2 11,4-Dioxan suspendiert und nacheinander mit 135.4 g (539 mmol, Reinheit 50%) Trimethylboroxin, 31.2 g (27 mmol) Tetrakis(triphenylphosphin)-palladium(0) und 78.3 g (566 mmol) Kaliumcarbonat versetzt und 8 h unter Rückfluss gerührt. Die auf RT abgekühlte Reaktionsmischung wurde über Kieselgel vom Niederschlag abfiltriert und das Filtrat eingeengt. Der Rückstand wurde in Dichlormethan gelöst und mittels Kieselgelchromatographie (Dichlormethan:Essigsäureethylester = 9:1) gereinigt. Man erhielt 74 g (84.6% d. Th.; Reinheit 100%) der Zielverbindung.
LC-MS (Methode 7): Rₜ =1.06 min
MS (ESpos): m/z = 325 (M+H)⁺
¹H-NMR (400 MHz, DMSO-d₆): δ = 1.35 (t, 3 H), 2.34 (br. s, 3 H), 2.56 (s, 3 H), 4.31 - 4.38 (m, 2 H), 5.28 (br. s, 2 H), 6.99 - 7.01 (m, 1 H), 7.35 - 7.47 (m, 3 H), 7.49 - 7.54 (m, 2 H), 8.68 - 8.70 (m, 1 H).

### Beispiel 239A

### Ethyl-8-hydroxy-2,6-dimethylimidazo[1,2-a]pyridin-3-carboxylat

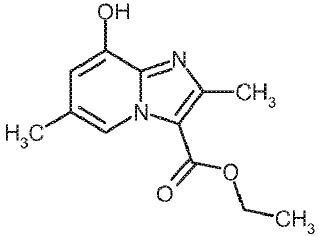

74 g (228 mmol) Ethyl-8-(benzyloxy)-2,6-dimethylimidazo[1,2-a]pyridin-3-carboxylat aus Beispiel 238A wurden in 1254 ml Dichlormethan und 251 ml Ethanol vorgelegt und unter Argon mit 20.1 g 10%igem Palladium auf Aktivkohle (wasserfeucht 50%) versetzt. Das Reaktionsgemisch wurde über Nacht bei RT und Normaldruck hydriert. Die Reaktionsmischung wurde über Kieselgel abfiltriert und eingeengt. Der Rohprodukt wurde mittels Kieselgelchromatographie (Dichlormethan:Methanol = 95:5) gereinigt. Man erhielt 50.4 g (94% d. Th.) der Zielverbindung.
DCI-MS: (Methode 4) (ESpos): m/z = 235.2 (M+H)⁺
¹H-NMR (400 MHz, DMSO-d₆): δ = 1.35 (t, 3 H), 2.27 (s, 3 H), 2.58 (s, 3 H), 4.30 - 4.38 (m, 2 H), 6.65 (d, 1 H), 8.59 (s, 1 H), 10.57 (br. s, 1H).

### Beispiel 240A

### rac-tert.-Butyl-2-{[({8-[(2,6-difluorbenzyl)oxy]-2,6-dimethylimidazo[1,2-a]pyridin-3-yl}carbonyl)amino]methyl}-3,4-dihydrochinolin-1(2H)-carboxylat

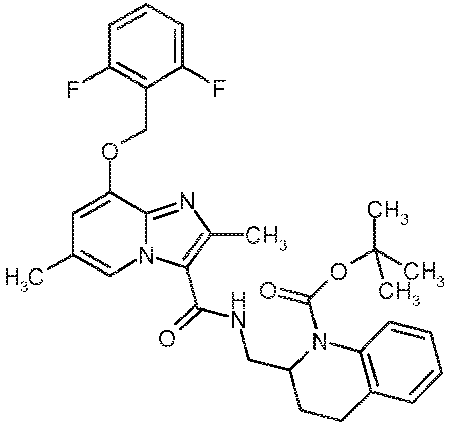

120 mg (0.36 mmol) 8-[(2,6-Difluorbenzyl)oxy]-2,6-dimethylimidazo[1,2-a]pyridin-3-carbonsäure Beispiel 21A, 139 mg (0.43 mmol) TBTU und 0.20 ml (1.81 mmol) 4-Methylmorpholin in DMF (2.3 ml) wurden 10 min bei RT gerührt, dann mit 119 mg (0.40 mmol) rac-tert.-Butyl-2-(aminomethyl)-3,4-dihydrochinolin-1(2H)-carboxylathydrochlorid versetzt und die Reaktionsmischung über Nacht bei RT gerührt. Das Gemisch wurde mit Wasser versetzt, 30 min bei RT gerührt. Der enstandenen Feststoff wurde abfiltriert, mit Wasser gewaschen und im Hochvakuum getrocknet. Man erhielt 182 mg (85% d. Th., Reinheit 98%) der Zielverbindung.
LC-MS (Methode 7): Rₜ = 1.13 min.
MS (ESIpos): m/z = 577 (M+H)⁺.
¹H-NMR (400 MHz, DMSO-d₆): δ = 1.38 (s, 9H), 1.64 -1.75 (m, 1H), 2.10 - 2.20 (m, 1H), 2.22 (s, 3H), 2.30 (s, 3H), 2.56 - 2.62 (m, 1H), 2.66 - 2.76 (m, 1H), 3.09 - 3.19 (m, 1H), 3.55 - 3.64 (m, 1H), 4.63 - 4.72 (m, 1H), 5.27 (s, 2H), 6.90 (s, 1H), 6.99 (t, 1H), 7.05 - 7.15 (m, 2H), 7.19 - 7.28 (m, 2H), 7.37 (d, 1H), 7.54 - 7.64 (m, 1H), 7.80 (t, 1H), 8.40 (s, 1H).

### Beispiel 241A

### 2-Methyl-4 phenyl-2-(trifluormethyl)-1,3-oxazolidin (Diastereomere)

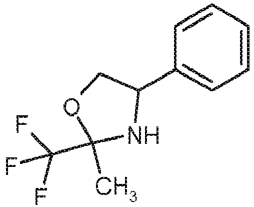

55.13 g (492.0 mmol) 1,1,1-Trifluoraceton in Toluol (1.351) wurden mit 45 g (328.0 mmol) *rac*-2-Amino-2-phenylethanol und 8.24 g (32.8 mmol) Pyridinium-p-toluolsulfonat versetzt. Die Reaktionsmischung wurde 16 h unter Rückfluss am Wasserabscheider gekocht. Das Gemisch wurde auf 0°C abgekühlt, der entstandene Feststoff abfiltriert und im Hochvakuum getrocknet. Man erhielt 68.6 g (77% d. Th., Reinheit 85%) der Zielverbindung.
LC-MS (Methode 2): Rₜ = 0.99 min
MS (ESIpos): m/z = 232 (M+H)⁺.
¹H-NMR (400 MHz, DMSO-d₆): δ = 1.54 (s, 3H), 3.56 (t, 1H), 3.81 (d, 1H), 4.28 (t, 1H), 4.35 - 4.43 (m, 1H), 7.29 - 7.47 (m, 5H).

### Beispiel 242A

### 3,3,3-Tnfluor-2-[(2-hydroxy-l -phenylethyl)amino]-2-methylpropanomtril (Diastereomere)

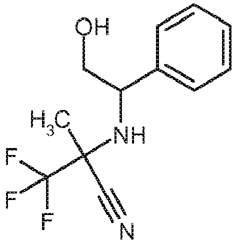

52.8 g (228.3 mmol) 2-Methyl-4-phenyl-2-(trifluormethyl)-1,3-oxazolidin (Diastereomere) Beispiel 241A wurden unter Argon in Dichlormethan (21) vorgelegt und suf 0°C abgekühlt. 42.85 ml (342.5 mmol) Trimetylsilylcyanid und 42.1 ml (342.5 mmol) Bortrifluorid-diethylether-Komplex wurden langsam zugegeben und es wurde 16 h bei RT gerührt. Anschließend wurde die Reaktionslösung in 1.5 1 gesättigte Natriumhydrogencarbonat-Lösung gegossen. Anschließend wurde noch mit 400 g Natriumhydrogencarbonat versetzt und die Lösung mit konz. Natronlauge auf pH 10 gestellt. Die wässrige Lösung wurde dreimal mit 500 ml Dichlormethan extrahiert, die vereinigten organischen Phasen über Natriumsulfat getrocknet, abfiltriert und eingeengt. Man erhielt 56.8 g (96% d. Th., 2 Diastereomere) der Zielverbindung.
LC-MS (Methode 2): Rₜ = 0.89 min und 0.93 min.
MS (ESIneg): m/z = 303 (M-H+HCOOH)-.

### Beispiel 243A

### 2-[(3-Amino-1,1,1-trifluor-2-methylpropan-2-yl)amino]-2-phenylethanol (Diastereomere)

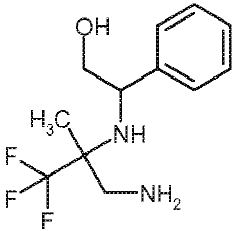

31 g (120.0 mmol) 3,3,3-Trifluor-2-[(2-hydroxy-1-phenylethyl)amino]-2-methylpropanonitril Beispiel 242A wurde in tert.-Butylmethylether (3.1 1) vorgelegt, auf 0°C abgekühlt, mit 18.25 g (480.2 mmol) Lithiumaluminiumhydrid versetzt und die Reaktionsmischung 16 h bei RT gerührt. Anschließend wurde auf 0°C abgekühlt, erst mit 24 ml Wasser gequencht, dann mit 24 ml 15%iger wässriger Kaliumhydroxid-Lösung und 48 ml Wasser versetzt. Die enstandene Mischung wurde über Kieselgel filtriert und mit tert.-Butylmethylether gewaschen. Die organische Phase wurde abgetrennt, über Natriumsulfat getrocknet, filtriert und eingeengt. Man erhielt 29.2 g (83% d. Th., Reinheit 89%) der Zielverbindung.
LC-MS (Methode 2): Rₜ = 0.52 min
MS (ESIpos): m/z = 263 (M+H)⁺.

### Beispiel 244A

### tert.-Butyl-{3,3,3-trifluor-2-[(2-hydroxy-1-phenylethyl)amino]-2-methylpropyl}carbamat (Diastereomere)

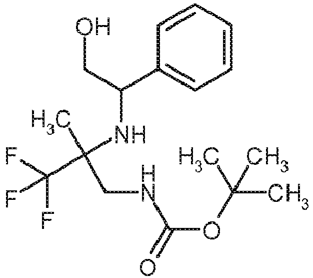

26.2 g (99.9 mmol) 2-[(3-Amino-1,1,1-trifluor-2-methylpropan-2-yl)amino]-2-phenylethanol (Diastereomere) Beispiel 243A in THF (500 ml) wurden mit 29.1 ml (209.8 mmol) Triethylamin und 23.98 g (109.9 mmol) Di-tert.-butyldicarbonat (gelöst in 286 ml THF) versetzt. Die Reaktionsmischung wurde 16 h bei RT gerührt. Anschließend wurde eingeengt und in jeweils 500 ml gesättigter, wässriger Natriumhydrogencarbonat-Lösung und Essigsäureethylester aufgenommen. Die Phasen wurden getrennt, die organische Phase über Natriumsulfat getrocknet, abfiltriert und eingeengt. Man erhielt 39.80 g (110% d. Th.) der Zielverbindung, welche ohne weitere Reinigung in die nächste Stufe eingesetzt wurde.
FIA-MS (Methode 10, ESpos): m/z = 363 (M+H)⁺

### Beispiel 245A

### rac-tert.-Butyl-(2-amino-3,3,3-trifluor-2-methylpropyl)carbamat

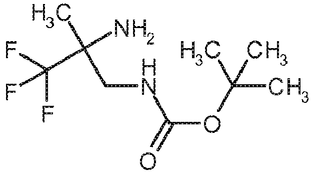

39 g (107.6 mmol) tert.-Butyl-{3,3,3-trifluor-2-[(2-hydroxy-1-phenylethyl)amino]-2-methylpropyl}carbamat Beispiel 244A wurden unter Argon in Ethanol (700 ml) vorgelegt und mit 5.44 g (53.8 mmol) Palladium(II)hydroxid (20% auf Aktivkohle, wasserfeucht ca. 60%ig) versetzt. Die Reaktionsmischung wurde 16 h bei Normaldruck hydriert. Dann wurde über Kieselgel filtriert und eingeengt. Der Rückstand wurde mittels Kieselgelchromatographie (Cyclohexan/Essigsäureethylester-Gradient: 9/1 nach 6/4) gereinigt. Man erhielt 15.8 g (61 % d. Th.) der Zielverbindung.
FIA-MS (Methode 10, ESpos): m/z = 243 (M+H)⁺
¹H-NMR (400 MHz, CDCl₃): δ = 1.22 (s, 3H), 1.45 (s, 9H), 3.13 - 3.23 (m, 1H), 3.37 - 3.48 (m, 1H), 4.89 (br. s, 1H).

### Beispiel 246A

### rac-3,3,3-Trifluor-2-methylpropan-1,2diamindihydrochlorid

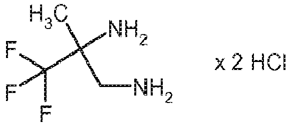

15 g (61.9 mmol) *rac*-tert.-Butyl-(2-amino-3,3,3-triftuor-2-methylpropyl)carbamat aus Beispiel 245A in Dioxan (188 ml) wurden mit 188 ml 4 M Chlorwasserstoff in Dioxan versetzt. Die Reaktionsmischung wurde 16 h bei RT gerührt, dann eingeengt und unter Argon aufbewahrt. Man erhielt 14.4 g (108% d. Th.) der Zielverbindung, die nicht weiter gereinigt wurde.
FIA-MS (Methode 10, ESpos): m/z = 143 (M-2HCl+H)⁺
¹H-NMR (400 MHz, D₂O): δ = 1.40 (s, 3H), 3.21 - 3.31 (m, 2H).

### Beispiel 247A

### ent-Benzyl-(2-amino-3-methoxypropyl)carbamat (Enantiomer A)

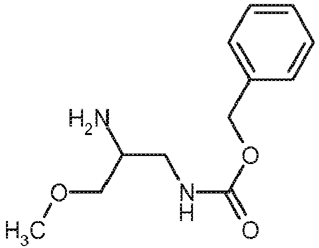

10 g Beispiel 140A wurden durch präparative Trennung an chiraler Phase in die Enantiomere getrennt, nachdem die TFA der Probe durch das kieselgel-basierte Sorbens "Bond Elut PSA" (Hersteller: Agilent) entfernt wurde [Säule: Daicel Chiralpak AY-H, 5 µm, 250 x 20 mm, Eluent: 50% Iso-Hexan, 50% Isopropanol + 0.2% Diethylamin, Fluß: 15 ml/min; 40°C, Detektion: 220 nm].
Enantiomer A:
Ausbeute: 2.17 g (96% ee)
Rₜ = 5.79 min [Daicel Chiralpak AY-H, 5µm, 250 x 4.6 mm; Eluent: 50% Iso-Hexan, 50% Isopropanol + 0.2% Diethylamin; Fluss 1.0 ml/min; 40°C; Detektion: 220 nm].

### Beispiel 248A

### ent-Benzyl-(2-amino-3-methoxypropyl)carbamat (Enantiomer B)

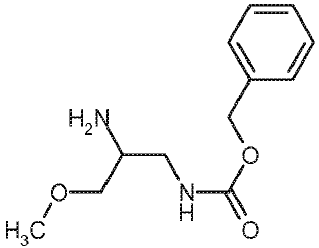

10 g Beispiel 140A wurden durch präparative Trennung an chiraler Phase in die Enantiomere getrennt, nachdem die TFA der Probe durch das kieselgel-basierte Sorbens "Bond Elut PSA" (Hersteller: Agilent) entfernt wurde [Säule: Daicel Chiralpak AY-H, 5 µm, 250 x 20 mm, Eluent: 50% Iso-Hexan, 50% Isopropanol + 0.2% Dimethylamin, Fluß: 15 ml/min; 40°C, Detektion: 220 nm].
Enantiomer B:
Ausbeute: 2.07 g (94% ee)
Rₜ = 7.26 min [Daicel Chiralpak AY-H, 5µm, 250 x 4.6 mm; Eluent: 50% Iso-Hexan, 50% Isopropanol + 0.2% Diethylamin; Fluss 1.0 ml/min; 40°C; Detektion: 220 nm].

### Beispiel 249A

### ent-3-Methoxypropan-1,2-diamindihydrochlorid (Enantiomer A)

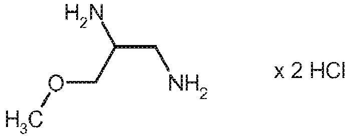

750 mg (3.15 mmol) *ent*-Benzyl-(2-amino-3-methoxypropyl)carbamat (Enantiomer A) Beispiel 247A wurden unter Argon in Ethanol (22 ml) vorgelegt, mit 335 mg (0.32 mmol) 10%igem Palladium auf Aktivkohle und 9.6 ml (94.42 mmol) Cyclohexen versetzt. Die Reaktionsmischung wurde 7 h unter Rückfluss gerührt. Anschließend wurde mit 335 mg (0.32 mmol) 10%igem Palladium auf Aktivkohle versetzt und weitere zwei Tage unter Rückfluss gerührt. Die auf RT abgekühlte Reaktionsmischung wurde über einen Milipore-Filter filtriert und mit Ethanol gewaschen. Das Filtrat wurde mit 3.2 ml (6.3 mmol) 2 N Chlorwasserstoff in Diethylether versetzt, eingeengt und im Hochvakuum getrocknet. Man erhielt 440 mg (79% d. Th.) der Zielverbindung.
¹H-NMR (400 MHz, DMSO-d₆): δ = 3.03 - 3.11 (m, 2H), 3.32 (s, 3H), 3.55 - 3.64 (m, 3H), 8.31 - 8.69 (m, 4H).

### Beispiel 250A

### ent-3-Methoxypropan-1,2-diamindihydrochlorid (Enantiomer B)

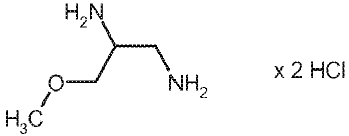

Darstellung und Reinigung der Titelverbindung erfolgte analog zum Beispiel 249A. Ausgehend von 750 mg (3.15 mmol) ent-Benzyl-(2-amino-3-methoxypropyl)carbamat (Enantiomer B) aus Beispiel 248A erhielt man 454 mg (81% d. Th.) der Zielverbindung.
¹H-NMR (400 MHz, DMSO-d₆): δ = 3.03 - 3.11 (m, 2H), 3.32 (s, 3H), 3.55 - 3.64 (m, 3H), 8.31 - 8.69 (m, 4H).

### Beispiel 251A

### Ethyl-2,6-dimethyl-8-(3-methylbutoxy)imidazo[1,2-a]pyridin-3-carboxylat

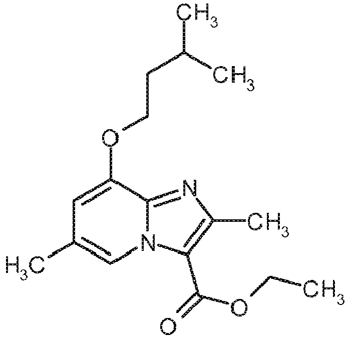

2.0 g (8.5 mmol) Ethyl-8-hydroxy-2,6-dimethylimidazo[1,2-a]pyridin-3-carboxylat Beispiel 239A in 122.3 ml DMF wurden mit 1.23 ml (9.4 mmol) 1-Iod-3-methyl-butan und 6.12 g (18.8 mmol) Cäsiumcarbonat versetzt und es wurde 40 min bei 60°C gerührt. Das auf RT abgekühlte Reaktionsgemisch wurde mit 900 ml Wasser versetzt, 1 h bei RT gerührt, der ausgefallenen Feststoff abfiltriert, mit Wasser gewaschen und am Hochvakuum getrocknet. Man erhielt 2.25 g (84% d. Th.; Reinheit 97%) der Titelverbindung.
LC-MS (Methode 7): Rₜ =1.12 min
MS (ESpos): m/z = 305 (M+H)⁺
¹H-NMR (400 MHz, DMSO-d₆): δ = 0.96 (d, 6H), 1.35 (t, 3H), 1.70 (q, 2H), 1.77 - 1.89 (m, 1H), 2.33 (s, 3H), 2.56 (s, 3H), 4.17 (t, 2H), 4.34 (q, 2H), 6.88 (s,1H), 8.64 (s, 1H).

### Beispiel 252A

### 2,6-Dimethyl-8-(3-methylbutoxy)imidazo[1,2-a]pyridin-3-carbonsäure

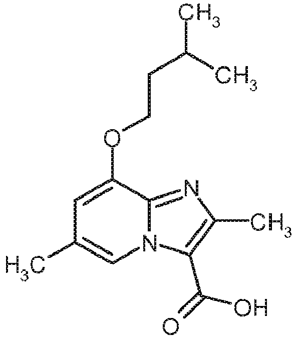

2.25 g (7.4 mmol) Ethyl-2,6-dimefhyl-8-(3-methylbutoxy)imidazo[l,2-a]pyridin-3-carboxylat Beispiel 251A wurden in 157 ml THF/Methanol 5:1 vorgelegt, mit 37 ml (37 mmol) 1 N Lithiumhydroxid-Lösung versetzt und die Reaktionsmischung über das Wochenende bei RT gerührt. Anschließend wurde auf 0°C abgekühlt, mit 6 N Salzsäure auf pH 4 angesäuert und im Vakuum vom organischen Lösungsmittel befreit. Der ausgefallene Feststoff wurde abfiltriert, mit Wasser gewaschen und am Hochvakuum getrocknet. Man erhielt 1.64 g (80% d. Th.; Reinheit 100%) der Titelverbindung.
LC-MS (Methode 2): Rₜ = 0.71 min
MS (ESpos): m/z = 277 (M+H)⁺
¹H-NMR (400 MHz, DMSO-d₆): δ = 0.96 (d, 6H), 1.70 (q, 2H), 1.78 - 1.89 (m, 1H), 2.32 (s, 3H), 2.56 (s, 3H), 4.17 (t, 2H), 6.85 (s,1H), 8.69 (s, 1H), 12.86 -13.08 (m, 1H).

### Beispiel 253A

### rac-tert.-Butyl-2-{[({8-[(2,6-difluorbenzyl)oxy]-2-methylimidazo[1,2-a]pyridin-3-yl}carbonyl)amino]methyl}-3,4-dihydrochinolin-1(2H)-carboxylat

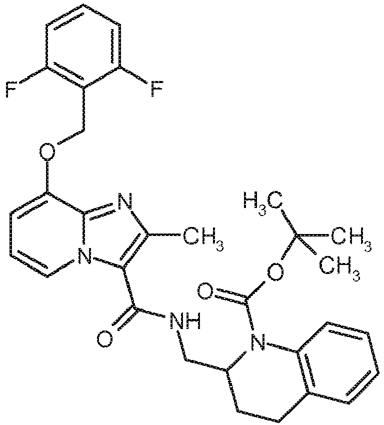

120 mg (0.38 mmol) 8-[(2,6-Difluorbenzyl)oxy]-2-methylimidazo[1,2-a]pyridin-3-carbonsäure Beispiel 3A in 2.4 ml DMF wurden mit 145 mg (0.45 mmol) TBTU und 0.2 ml (1.89 mmol) 4-Methylmorpholin versetzt und 10 min bei RT gerührt. 124 mg (0.42 mmol) *rac*-tert.-Butyl-2-(aminomethyl)-3,4-dihydrochinolin-1(2H)-carboxylathydrochlorid wurden zugegeben und die Reaktionsmischung über Nacht bei RT gerührt. Das Reaktionsgemisch wurde mit Wasser versetzt und 30 min bei RT gerührt. Der ausgefallene Feststoff wurde abfiltriert, mit Wasser gewaschen und am Hochvakuum getrocknet. Man erhielt 190 mg (90% d. Th.; Reinheit 100%) der Titelverbindung.
LC-MS (Methode 7): Rₜ = 1.13 min
MS (ESpos): m/z = 563 (M+H)⁺
¹H-NMR (400 MHz, DMSO-d₆): δ = 1.38 (s, 9H), 1.64 -1.75 (m, 1H), 2.11 - 2.21 (m, 1H), 2.25 (s, 3H), 2.65 - 2.76 (m, 2H), 3.10 - 3.20 (m, 1H), 3.57 - 3.66 (m, 1H), 4.68 (quintett, 1H), 5.29 (s, 2H), 6.91 (t, 1H), 6.96 - 7.03 (m, 2H), 7.08 (t, 1H), 7.13 (d, 1H), 7.19 - 7.27 (m, 2H), 7.37 (d, 1H), 7.54 - 7.63 (m, 1H), 7.83 (t, 1H), 8.58 (d, 1H).

### Beispiel 254A

### ent-tert.-Butyl-{(1R,2R)-2-[({8-[(2,6₋difluorbenzyl)oxy]-2,6-dimethylimidazo[1,2-a]pyridin-3-yl} carbonyl)amino]cyclohexyl} carbamat

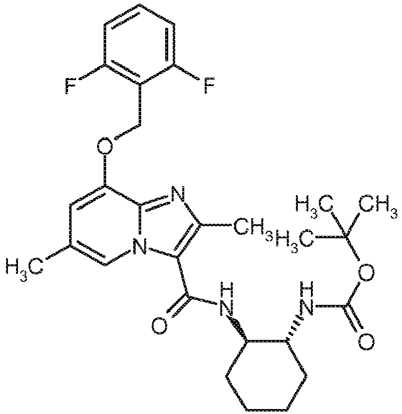

Darstellung und Reinigung der Titelverbindung erfolgte analog zum Beispiel 253A. Ausgehend von 80 mg (0.24 mmol) 8-[(2,6-Difluorbenzyl)oxy]-2,6-dimethylimidazo[1,2-a]pyridin-3-carbonsäure Beispiel 21A und 57 mg (0.27 mmol) *ent*-(1*R*,2*R*)-trans-N-Boc-1,2-cyclohexandiamin erhielt man 107 mg (83% d. Th.; Reinheit 98%) der Zielverbindung.
LC-MS (Methode 7): Rₜ = 1.02 min
MS (ESpos): m/z = 529 (M+H)⁺
¹H-NMR (400 MHz, DMSO-d₆): δ = 1.16 - 1.45 (m, 13H), 1.61 - 1.74 (m, 2H), 1.76 - 1.86 (m, 1H), 1.89 - 1.99 (m, 1H), 2.30 (s, 3H), 2.46 (s, 3H), 3.37 - 3.46 (m, 1H), 3.67 - 3.78 (m, 1H), 5.28 (s, 2H), 6.81 (d, 1H), 6.91 (s,1H), 7.20 - 7.28 (m, 2H), 7.54 - 7.64 (m, 2H), 8.46 (s, 1H).

### Beispiel 255A

### rac-Ethyl-8-[1-(2,6-difluorphenyl)ethoxy]-2,6-dimethylimidazo[1,2-a]pyridin-3-carboxylat

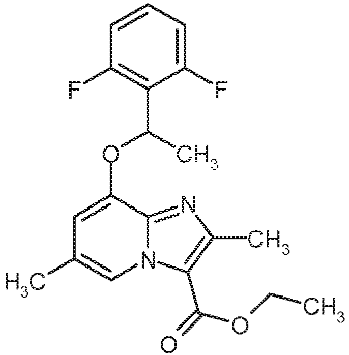

5.50 g (23.5 mmol) Ethyl-8-hydroxy-2,6-dimethylimidazo[1,2-a]pyridin-3-carboxylat Beispiel 239A wurden mit 4.46 g (28.2 mmol) 1-(2,6-Difluorphenyl)ethanol, 5.35 ml (27.0 mmol) Azodicarbonsäurediisopropylester und 7.08 g (27.0 mmol) Triphenylphosphin in 141 ml THF gelöst und 2 h bei RT gerührt. Die Reaktionsmischung wurde mit 0.70 ml (3.5 mmol) Azodicarbonsäurediisopropylester und 0.62 g (2.3 mmol) Triphenylphosphin versetzt und die Reaktionslösung wurde 1 h bei RT gerührt. Der ausgefallene Feststoff wurde abfiltriert und am Hochvakuum getrocknet. Man erhielt 4.6 g (52.8% d. Th.; Reinheit 100%) der Titelverbindung. Das Fitrat wurde eingeengt und zweimal mittels Kieselgelchromatographie (Cyclohexan:Essigsäureethylester -Gradient = 8:1 nach 4:1) gereinigt. Alle produkthaltigen Fraktionen wurden nochmals mittels präparativer HPLC gereinigt (RP18 Säule, Laufmittel: Acetonitril/Wasser-Gradient unter Zusatz von 0.1% TFA). Man erhielt nochmals 2.16 g (25% d. Th.) der Zielverbindung.
LC-MS (Methode 2): Rₜ =1.08 min
MS (ESpos): m/z = 375 (M+H)⁺
¹H-NMR (400MHz, DMSO-d₆): δ = 1.34 (t, 3H), 1.79 (d, 3H), 2.25 (s, 3H), 2.58 (s, 3H), 4.33 (q, 2H), 6.17 (q, 1H), 6.73 (s,1H), 7.06 - 7.16 (m, 2H), 7.37 - 7.48 (m, 1H), 8.67 (s, 1H).

### Beispiel 256A

### ent-Ethyl-8-[1-(2,6-difluorphenyl)ethoxy]-2,6-dimethylimidazo[1,2-a]pyridin-3-carboxylat (Enantiomer B)

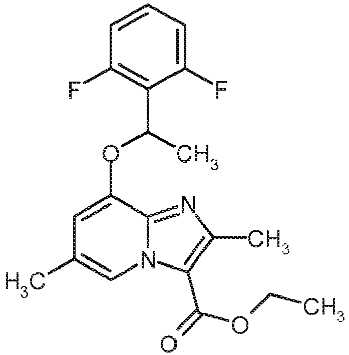

6.8 g Beispiel 255A wurden durch präparative Trennung an chiraler Phase in die Enantiomere getrennt [Säule: Daicel Chiralpak AD-H, 5 µm, 250 x 30 mm, Eluent: 70% Iso-Hexan, 30% Ethanol, Fluß: 50 ml/min; 40°C, Detektion: 210 nm].
Enantiomer B:
Ausbeute: 2.7 g (98.4% ee)
Rₜ= 5.18 min [Daicel Chiralpak AD-H, 5µm, 250 x 4.6 mm; Eluent: 70% Iso-Hexan, 30% Ethanol; Fluss 1.0 ml/min; 30°C; Detektion: 220 nm].

### Beispiel 257A

### ent-8-[1-(2,6-Difluorphenyl)ethoxy]-2,6-dimethylimidazo[1,2-a]pyridin-3-carbonsäure (Enantiomer B)

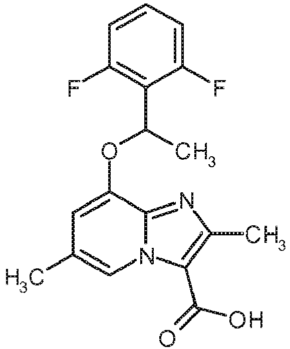

2.58 g (6.9 mmol) *ent*-Ethyl-8-[1-(2,6-difluorphenyl)ethoxy]-2,6-dimethylimidazo[1,2-a]pyridin-3-carboxylat Beispiel 256A (Enantiomer B) wurde in 154 ml THF/Methanol 5:1 gelöst, mit 34.5 ml (34.5 mmol) 1 N wässriger Lithiumhydroxid-Lösung versetzt und 5 h bei 40°C gerührt. Die auf RT abgekühlte Reaktionsmischung wurde mit 6 N Salzsäure-Lösung angesäuert und eingeengt. Der Feststoff wurde abfiltriert, mit Wasser gewaschen und am Hochvakuum getrocknet. Man erhielt 2.26 g (95% d. Th.; Reinheit 100%) der Titelverbindung.
LC-MS (Methode 2): Rₜ = 0.74 min
MS (ESpos): m/z = 347 (M+H)⁺
¹H-NMR (400 MHz, DMSO-d₆): δ = 1.79 (d, 3H), 2.24 (s, 3H), 2.57 (s, 3H), 6.16 (q, 1H), 6.67 (s, 1H), 7.06 - 7.16 (m, 2H), 7.38 - 7.48 (m, 1H), 8.74 (s,1H), 12.24 -13.90 (br. s, 1H).

### Beispiel 258A

### Ethyl-2,6-dimethyl-8-[4,4,4-trifluor-3-(trifluormethyl)butoxy]imidazo[1,2-a]pyridin-3-carboxylat

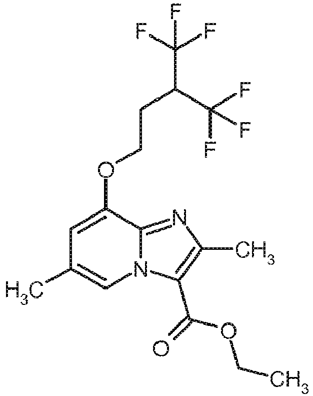

1.89 g (8.07 mmol) Ethyl-8-hydroxy-2,6-dimethylimidazo[1,2-a]pyridin-3-carboxylat Beispiel 239A in 60 ml DMF wurden mit 7.89 g (24.2 mmol) Cäsiumcarbonat und 2.30 g (8.88 mmol) 4,4,-Trifluoro-3-(trifluoromethyl)butylbromid versetzt und das Reaktionsgemisch 90 min bei RT gerührt. Anschließend wurde mit 60 ml Wasser versetzt, der ausgefallene Feststoff abfiltriert und der Filterrückstand mit 100 ml Wasser und zweimal mit 20 ml tert.-Butylmethylether nachgewaschen. Der aus dem Filtrat ausgefallene Niederschlag wurde abfiltriert und mit Filtrat nachgewaschen. Beide Filterrückstände wurden mit 50 ml Essigsäureethylester aufgenommen. Die Lösung wurde im Vakuum eingeengt und der Rückstand über Nacht im Vakuum getrocknet. Es wurden 2.25 g der Zielverbindung (95% Reinheit, 64% d. Th.) erhalten.
LC-MS (Methode 2): Rₜ = 1.16 min
MS (ESpos): m/z = 413 (M+H)⁺
¹H-NMR (400 MHz, DMSO-d₆): δ = 1.36 (t, 3H), 2.34 (s, 3H), 2.32-2.38 (m, 2H), 2.58 (s, 3H), 4.18-4.30 (m, 1H), 4.31-4.38 (m, 4H), 6.93 (s, 1H), 8.71 (s, 1H).

### Beispiel 259A

### 2,6-Dimethyl-8-[4,4,4-trifluor-3-(trifluormethyl)butoxy]imidazo[1,2-a]pyridin-3-carbonsäure

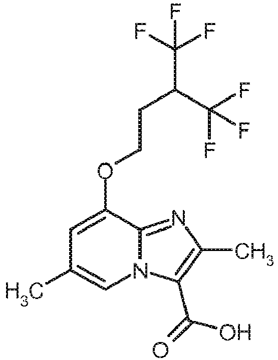

1.95 g (4.73 mmol) Ethyl-2,6-dimethyl-8-[4,4,4-trifluor-3-(trifluormethyl)butoxy]imidazo[1-2-a]pyridin-3-carboxylat Beispiel 258A in 30 ml Methanol wurden mit 3.28 g (10.4 mmol) Bariumhydroxid-Octahydrat versetzt und es wurde 3 Tage bei RT gerührt. Die Suspension wurde mit 30 ml Wasser verdünnt und mit 1 M Salzsäure auf pH 6 gestellt. Der Feststoff wurde abfiltriert, mit 50 ml Wasser gewaschen und bei 70°C 2 h im Vakuum getrocknet. Es wurden 1.64 g der Zielverbindung (90% Reinheit, 81% d. Th.) erhalten.
LC-MS (Methode 2): Rₜ = 0.78 min
MS (ESpos): m/z = 385 (M+H)⁺
¹H-NMR (400 MHz, DMSO-d₆): δ = 2.29 (s, 3H), 2.28-2.37 (m, 2H), 2.56 (s, 3H), 4.22-4.35 (m, 3H), 6.74 (s, 1H), 8.99 (s, 1H).

### Beispiel 260A

### rac-2-Amino-4-(benzyloxy)-2-methylbutanonitril

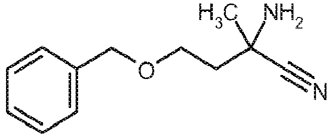

5.31 g (108.3 mmol) Natriumcyanid in 10 ml Wasser wurden mit 6.37 g (119.1 mmol) Ammoniumchlorid (gelöst in 15 ml warmem Wasser) und 9 ml (216.6 mmol) konz. Ammoniak in Wasser versetzt. Anschließend wurden 19.3 g (108.3 mmol) 4-(Benzyloxy)butan-2-on, gelöst in 3 ml Ethanol, zugegeben. Es wurde 15 min bei RT und 2 h bei 60°C gerührt. Erneut wurden 4 g (81.6 mmol) Natriumcyanid, 4.8g (89.7 mmol) Ammoniumchlorid und 6.5 ml (156.4 mmol) konz. Ammoniak in Wasser zugegeben und weitere 2 h bei 60°C gerührt. Dann wurde abgekühlt und die Reaktionslösung mit je 300 ml Methylenchlorid und Wasser versetzt. Nach Phasentrennung wurde die wässrige Phase mit 300 ml Methylenchlorid extrahiert. Die vereinigten organischen Phasen wurden getrocknet und eingeengt. Das Rohprodukt wurde über Kieselgel gereinigt (Cyclohexan/Essigsäureethylester -Gradient 6/4 - 1/1). Es wurden 19.9 g der Zielverbindung (77% Reinheit, 69% d. Th.) erhalten.
LC-MS (Methode 11): Rₜ = 2.31 min
MS (ESpos): m/z = 205 (M+H)⁺
¹H-NMR (400 MHz, DMSO-d₆): δ =1.37 (s, 3H), 1.81 - 1.94 (m, 2H), 2.57 (br. s, 2H), 3.58 - 3.69 (m, 2H), 4.48 (s, 2H), 7.25 - 7.38 (m, 5H).

### Beispiel 261A

### rac-4-(Benzyloxy)-2-methylbutan-1,2-diamin

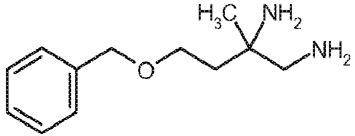

0.5 g (2.45 mmol) 2-Amino-4-(benzyloxy)-2-methylbutanonitril Beispiel 260A in 25 ml trockenem THF wurden unter Argon bei 0°C mit 1.59 ml (1.59 mmol) Lithiumaluminiumhydrid (1 N Lösung in Diethylether) versetzt. Die Reaktionslösung wurde erst 30 min bei 0°C gerührt und anschließend langsam auf Raumtemperatur kommend 1 h nachgerührt. Dann wurde vorsichtig mit 245 µl Wasser, 245 µl 2 N wässriger Natronlauge und 490 µl Wasser versetzt. Der Niederschlag wurde abfiltriert, mit THF und Methanol gewaschen, das Filtrat eingeengt und der Rückstand mittels Kieselgel-Chromatographie gereinigt (Laufmittel: Dichlormethan/2 N Ammoniak in Methanol = 20/1, isohatisch). Es wurden 0.30 g der Zielverbindung (96% Reinheit, 57% d. Th.) erhalten.
LC-MS (Methode 11): Rₜ =1.94 min
MS (ESpos): m/z = 209 (M+H)⁺
¹H-NMR (400 MHz, DMSO-d₆): δ = 0.90 (s, 3H), 1.56 (t, 2H), 2.27-2.38 (m, 2H), 3.45 - 3.60 (m, 2H), 4.42 (s, 2H), 7.22 - 7.36 (m, 5H).

### Beispiel 262A

### rac-2-Amino-3-(benzyloxy)-2-methylpropanonitril

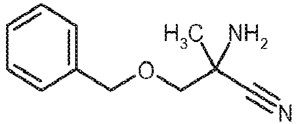

5.07 g (27.79 mmol) 1-(Benzyloxy)aceton wurden in 55.6 ml 2 N wässrigem Ammoniak in Methanol vorgelegt, mit 1.53 g (31.12 mmol) Natriumcyanid und 3.71 g (31.12 mmol) Ammoniumchlorid versetzt und 2 h unter Rückfluss gerührt. Anschließend wurden nochmals 27.4 ml 2 N wässriger Ammoniak in Methanol zu dem Reaktionsgemisch gegeben und es wurde 2 h unter Rückfluss gerührt. Die Reaktionslösung wurde abgekühlt und mit 90 ml Dichlormethan verdünnt. Der enthaltene Feststoff wurde abfiltriert und das Filtrat eingeengt. Der Rückstand wurde mittels Kieselgel gereinigt (Laufmittel: Cyclohexan/Etlyacetat-Gradient: 4/1 bis 1/1). Es wurden 4.94 g der Zielverbindung (90% Reinheit, 84% d. Th.) erhalten.
LC-MS (Methode 2): Rₜ = 0.60 min
MS (ESpos): m/z = 191 (M+H)⁺
¹H-NMR (400 MHz, DMSO-d₆): δ = 1.34 (s, 3H), 3.29 - 3.44 (m, 2H), 4.59 (s, 2H), 7.26 - 7.39 (m, 5H).

### Beispiel 263A

### rac-3-(Benzyloxy)-2-methylpropan-1,2-diamin

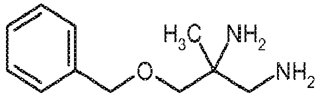

6.8 g (32.17 mmol, Reinheit ca. 90%) 2-Amino-3-(benzyloxy)-2-methylpropanonitril Beispiel 262A in 329 ml trockenem THF wurden unter Argon bei -78°C mit 20.9 ml (20.9 mmol) Lithiumaluminiumhydrid (1 N Lösung in Diethylether) versetzt. Die Reaktionslösung wurde 1 h bei -78°C, 2 h bei -20°C und 2 h bei 0°C gerührt. Dann wurde vorsichtig mit 3.22 ml Wasser, 3.22 ml 2 N Natronlauge und 6.44 ml Wasser versetzt. Der Niederschlag wurde abfiltriert, mit THF und Methanol gewaschen und das Filtrat eingeengt. Es wurden 8 g des Rohproduktes erhalten. 7 g dieses Rohprodukktes wurden mittels Kieselgel-Chromatographie gereinigt (Laufmittel: Dichlormethan/2 N Ammoniak in Methanol = 20/1 nach 10/1). Es wurden 1.52 g der Zielverbindung (ca. 28% d. Th.) erhalten.
LC-MS (Methode 11): Rₜ = 2.05 min
MS (ESpos): m/z = 195 (M+H)⁺
¹H-NMR (400 MHz, DMSO-d₆): δ = 0.88 (s, 3H), 1.39 (br. s, 2H), 2.30-2.47 (m, 2H), 3.12 - 3.22 (m, 2H), 4.44 (s, 2H), 7.24 - 7.38 (m, 5H).

### Beispiel 264A

### Ethyl-2,6-dimethyl-8-[(2,3,6-trifluorbenzyl)oxy]imidazo[1,2-a]pyridin-3-carboxylat

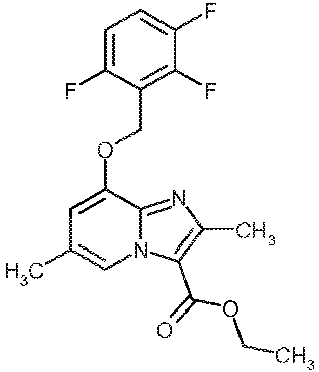

3.00 g (12.81 mmol) Ethyl-8-hydroxy-2,6-dimethylimidazo[1,2-a]pyridin-3-carboxylat Beispiel 239A, 3.27 g (14.1 mmol) 2-(Brommethyl)-1,3,4-trifluorbenzol und 9.18 g (28.17 mmol) Cäsiumcarbonat wurden in 183 ml trockenem DMF vorgelegt und für 30 min in einem auf 60°C erwärmten Ölbad erhitzt. Dann wurde mit ca. 1.8 1 Wasser versetzt und 30 min gerührt. Der Feststoff wurde abfiltriert, mit Wasser nachgewaschen und im Vakuum getrocknet. Es wurden 5.07 g der Titelverbindung (99% d. Th.; Reinheit ca. 96%) erhalten.
LC-MS (Methode 2): Rₜ =1.14 min
MS (ESpos): m/z = 379 (M+H)⁺
¹H-NMR (400 MHz, DMSO-d₆): δ = 1.35 (t, 3 H); 2.36 (s, 3 H); 2.55 (s, 3 H; überlagert durch DMSO-Signal); 4.36 (q, 2 H); 5.35 (s, 2 H); 7.09 (s, 1 H); 7.22 - 7.32 (m, 1 H); 7.60 - 7.73 (m, 1 H); 8.72 (s, 1 H).

### Beispiel 265A

### 2,6-Dimethyl-8-[(2,3,6-trifluorbenzyl)oxy]imidazo[1,2,a]pyridin-3-carbonsäure

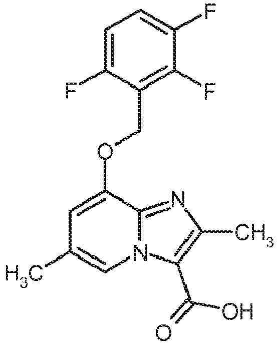

5.07 g (12.87 mmol) Ethyl-2,6-dimethyl-8-[(2,3,6-trifluorbenzyl)oxy]imidazo[1,2-a]pyridin-3-carboxylat Beispiel 264A wurden in 275 ml THF/Methanol (5/1) gelöst, mit 64.4 ml 1 N wässriger Lithiumhydroxid-Lösung versetzt und 3.5 h bei 40°C gerührt. Es wurde bei 0°C mit 6 N wässriger Salzsäure auf ca. pH 4 angesäuert und eingeengt. Der entstandene Feststoff wurde abfiltriert, mit Wasser nachgewaschen und im Vakuum getrocknet. Es wurden 4.77 g (98% d. Th.; Reinheit ca. 93%) der Titelverbindung erhalten.
LC-MS (Methode 2): Rₜ = 0.72 min
MS (ESpos): m/z = 351 (M+H)⁺
¹H-NMR (400 MHz, DMSO-d₆): δ = 2.37 (s, 3 H); 2.54 (s, 3 H; überlagert durch DMSO-Signal); 5.36 (s, 2 H); 7.11 (s, 1 H); 7.25 - 7.33 (m, 1 H); 7.61- 7.73 (m, 1 H); 8.78 (s, 1 H); 13.10 (br. s, 1 H).

### Beispiel 266A

### 4-Fluor-2-nitropyridin-3-ol

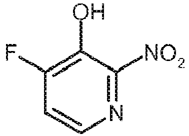

500 mg (3.43 mmol) 4-Fluorpyridin-3-olhydrochlorid wurden unter Eiskühlung vorsichtig in 3.2 ml konzentrierter Schwefelsäure gelöst und bei 0°C langsam mit 0.21 ml konzentrierter Salpetersäure versetzt. Die Reaktion wurde auf RT erwärmt und über Nacht bei RT gerührt. Dann wurde auf 10 g Eis gegeben und unter Eiskühlung wurden 6 ml 45%ige Natronlauge zugetropft. Der entstandene Niederschlag wurde abfiltriert und anschließend über Nacht im Vakuum getrocknet wurden. Es wurden 191 mg (36% d. Th.) der Titelverbindung erhalten.
LC-MS (Methode 2): Rₜ = 0.36 min
MS (ESneg): m/z = 157 (M-H)⁻
¹H-NMR (400 MHz, DMSO-d₆): δ = 7.69 (dd, 1 H); 7.95 - 8.01 (m, 1 H); 11.97 (br. s, 1 H).

### Beispiel 267A

### 2-Amino-4-fluoipyridin-3-ol

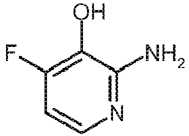

90 mg (0.57 mmol) 4-Fluor-2-nitropyridin-3-ol Beispiel 266A wurden unter Argon in 30 ml Ethanol gelöst, mit einer Spatelspitze 10%igem Palladium auf Aktivkohle versetzt und 1.5 h bei RT unter Normaldruck hydriert. Anschließend wurde über Kieselgel abfiltriert und mit viel Ethanol nachgewaschen. Die Lösung wurde eingeengt und getrocknet. Es wurden 56 mg (77% d. Th.) der Titelverbindung erhalten.
LC-MS (Methode 2): Rₜ = 0.16 min
MS (ESpos): m/z = 129 (M+H)⁺
¹H-NMR (400 MHz, DMSO-d₆): δ = 5.78 (br. s, 2 H); 6.42 (dd, 1 H); 7.37 - 7.43 (m, 1 H); 9.47 (br. s, 1 H).

### Beispiel 268A

### 3-[(2,6-Difluorbenzyl)oxy]-4-fluorpyridin-2-amin

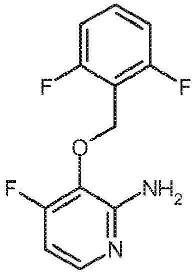

55 mg (0.43 mmol) 2-Amino-4-fluorpyridin-3-ol Beispiel 267A, 98 mg (0.47 mmol) 2-(Brommethyl)-1,3-difluorbenzol und 308 mg (0.95 mmol) Cäsiumcarbonat wurden in 1 ml trockenem DMF vorgelegt und für 15 min in einem auf 50°C erwärmten Ölbad erhitzt. Dann wurde abfiltriert und mittels präparativen HPLC (Methode 9) gereinigt. Es wurden 70 mg der Titelverbindung (64% d. Th.) erhalten.
LC-MS (Methode 2): Rₜ = 0.70 min
MS (ESpos): m/z = 255 (M+H)⁺
¹H-NMR (400 MHz, DMSO-d₆): δ = 5.06 (s, 2 H); 6.04 (br. s, 2 H); 6.42 (dd, 1 H); 7.08 - 7.16 (m, 2 H); 7.45 - 7.54 (m, 1 H); 7.62 - 7.69 (m, 1 H).

### Beispiel 269A

### Ethyl-8-[(2,6-difluorbenzyl)oxy]-7-fluor-2-methylimidazo[1,2-a]pyridin-3-carboxylat

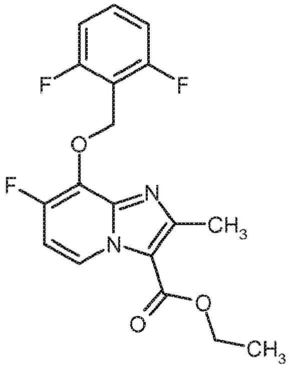

500 mg (1.97 mmol) 3-[(2,6-Difluorbenzyl)oxy]-4-fluorpyridin-2,amin Beispiel 268A wurden unter Argon in 10 ml Ethanol vorgelegt und mit 500 mg gepulvertem Molekularsieb 4Å und 3.24 g (19.67 mmol) Ethyl-2-chloracetoacetat versetzt und dann wurde 48 h zum Rückfluß erhitzt. Alle flüchtigen Komponeneten wurden im Vakuum bei 85°C eingeengt. Das Rohprodukt wurde mittels Kieselgel-Chromatographie gereinigt (Laufmittel: Cyclohexan/Essigsäureethylester = 9/1 isokratisch). Es wurden 368 mg (39% d. Th.; Reinheit ca. 76%) der Titelverbindung erhalten.
LC-MS (Methode 2): Rₜ = 1.19 min
MS (ESpos): m/z = 365 (M+H)⁺
¹H-NMR (400 MHz, DMSO-d₆): δ = 1.37 (t, 3 H); 2.62 (s, 3 H); 4.38 (q, 2 H); 5.60 (s, 2 H); 7.09 - 7.22 (m, 3 H); 7.47 - 7.56 (m, 1 H); 8.98 (dd, 1 H).

### Beispiel 270A

### 8-[(2,6-Difluorbenzyl)oxy]-7-fluor-2-methylimidazo[1,2-a]pyridin-3-earbonsäure

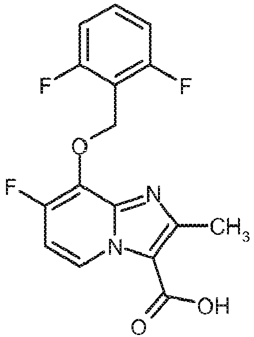

365 mg (0.76 mmol; Reinheit ca. 76%) Ethyl-8-[(2,6-difluorbenzyl)oxy]-7-fluor-2-methylimidazo[1,2-a]pyridin-3-carboxylat Beispiel 269A in 16.6 ml THF/Ethanol (5/1) wurden mit 1.14 ml (1.14 mmol) 1 N Lithiumhydroxid-Lösung versetzt und über Nacht bei RT gerührt. Es wurden nochmals 2.67 ml (2.67 mmol) 1 N Lithiumhydroxid-Lösung hinzugegeben und über Nacht bei RT gerührt. Das Reaktionsgemisch wurde im Vakuum vom organischen Lösungsmittel befreit und die wässrige Phase wurde mit 6 N Salzsäure auf pH 4 angesäuert, dabei wurde mit Eiswasser gekühlt. Der entstandene Feststoff wurde abfiltriert und im Hochvakuum getrocknet. Es wurden 236 mg der Zielverbindung (87% d. Th., Reinheit 94%) erhalten.
LC-MS (Methode 2): Rₜ = 0.83 min
MS (ESpos): m/z = 337 (M+H)⁺
¹H-NMR (400 MHz, DMSO-d₆): δ = 2.62 (s, 3 H); 5.60 (s, 2 H); 7.09 - 7.18 (m, 3 H); 7.47 - 7.55 (m, 1 H); 9.04 (dd, 1 H); 13.22 (br. s, 1 H).

### Beispiel 271A

### rac-Benzyl-(2-cyanbutan-2-yl)carbamat

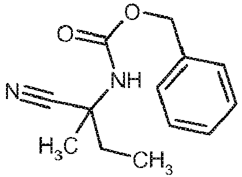

5.00 g (50.94 mmol) 2-Amino-2-methylbutanonitril [Synthese beschrieben in: Lonza AG, US 5698704 (1997); Deng, S. L. et al. Synthesis 2001, 2445; Hjorringgaard, C. U. et al. J. Org. Chem. 2009, 74, 1329; Ogrel, A. et al. Eur. J. Org. Chem. 2000, 857] wurden in 50 ml THF und 6.5 ml Wasser vorgelegt, mit 21.83 g (157.92 mmol) Kaliumcarbonat versetzt und bei 0°C langsam mit 7.9 ml (56.04 mmol) Benzylchlorocarbonat (Chlorameisensäurebenzylester) versetzt. Nach Zugabe von 8 ml THF und 3 ml Wasser wurde das Reaktionsgemisch langsam auf RT kommend über Nacht gerührt. Dann wurde mit Wasser versetzt und dreimal mit Essigsäureethylester extrahiert. Die vereinten organischen Phasen wurden über Natriumsulfat getrocknet und eingeengt. Der Rückstand wurde in Diethylether gelöst und mit Petrolether ausgefällt. Das Produkt wurde abfiltriert, der Feststoff mit etwas Petrolether gewaschen und im Hochvakuum getrocknet. Es wurden 11.35 g der Zielverbindung (93% d. Th., Reinheit 97%) erhalten.
LC-MS (Methode 2): Rₜ = 0.97 min
MS (ESpos): m/z = 233 (M+H)⁺
¹H-NMR (400 MHz, DMSO-d₆): δ = 0.95 (t, 3H), 1.51 (s, 3H), 1.75 - 1.95 (m, 2H), 5.07 (s, 2H), 7.30 - 7.43 (m, 4H), 7.88 - 8.03 (m, 1 H).

### Beispiel 272A

### ent-Benzyl-(2-cyanbutan-2-yl)carbamat (Enantiomer A)

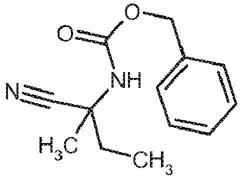

8 g *rac*-Benzyl-(2-cyanbutan-2-yl)carbamat Beispiel 271A wurden durch präparative Trennung an chiraler Phase in die Enantiomere getrennt [Säule: Daicel Chiralcel OJ-H, 5 µm, 250 x 20 mm, Eluent: 50% Iso-Hexan, 50% Isopropanol, Fluß: 20 ml/min; 40°C, Detektion: 220 nm].
Enantiomer A: Ausbeute: 3.23 g (>99% ee)
Rₜ = 6.69 min [Daicel Chiralcel OJ-H, 5 µm, 250 x 4.6 mm; Eluent: 50% Iso-Hexan, 50% Isopropanol; Fluss 1.0 ml/min; 30°C; Detektion: 220 nm].

### Beispiel 273A

### ent-Benzyl-(2-cyanbutan-2-yl)carbamat (Enantiomer B)

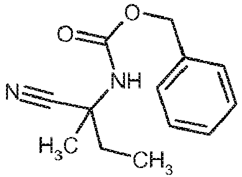

8 g *rac*-Benzyl-(2-cyanbutan-2-yl)carbamat Beipielverbindung 271A wurden durch präparative Trennung an chiraler Phase in die Enantiomere getrennt [Säule: Daicel Chiralcel OJ-H, 5 µm, 250 x 20 mm, Eluent: 50% Iso-Hexan, 50% Isopropanol, Fluß: 20 ml/min; 40°C, Detektion: 220 nm].
Enantiomer B: Ausbeute: 3.18 g (>99% ee)
Rₜ = 8.29 min [Daicel Chiralcel OJ-H, 5 µm, 250 x 4.6 mm; Eluent: 50% Iso-Hexan, 50% Isopropanol; Fluss 1.0 ml/min; 30°C; Detektion: 220 nm].

### Beispiel 274A

### ent-Benzyl-(1-amino-2-methylbutan-2-yl)carbamat (Enantiomer A)

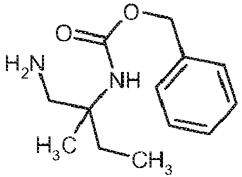

4.00 g (17.22 mmol) *ent*-Benzyl-(2-cyanbutan-2-yl)carbamat Beipiel 272A wurden in 50 ml einer 7 N Lösung von Ammoniak in Methanol gelöst, mit 5.33 g Raney-Nickel versetzt und 24 h bei ca. 25 bar bei RT hydriert. Es wurde über Celite abfiltriert, mit Methanol gewaschen und eingeengt. Das Rohprodukt wurde mittels Kieselgelchromatographie gereinigt (Laufmittel: Dichlormethan/2N Ammoniak in Methanol =10/0.5). Es wurden 2.20 g der Zielverbindung (54% d. Th.) erhalten.
LC-MS (Methode 2): Rₜ = 0.56 min
MS (ESpos): m/z = 237 (M+H)⁺

### Beispiel 275A

### ent-Benzyl-(1-amino-2-methylbutan-2-yl)carbamat (Enantiomer B)

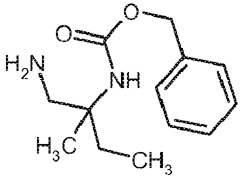

4.00 g (17.22 mmol) *ent*-Benzyl-(2-cyanbutan-2-yl)carbamat Beipiel 273A wurden in 50 ml 7 N ammoniakalische Methanol-Lösung gelöst, mit 5.33 g Raney-Nickel versetzt und 24 h bei ca. 25 bar bei RT hydriert. Das Reaktionsgemisch wurde über Celtite abfiltriert, gut mit Methanol gespült und eingeengt. Das Rohprodukt wurde mittels Kieselgelchromatographie gereinigt (Laufmittel: Dichlormethan/2N Ammoniak in Methanol =10/0.5).
Es wurden 3.56 g der Zielverbindung (87% d. Th.) erhalten.
LC-MS (Methode 13): Rₜ =1.40 min
MS (ESpos): m/z = 237 (M+H)⁺

### Beisipiel 276A

### ent-Benzyl-{1-[({8-[(2,6-difluorbenzyl)oxy]-2,6-dimethylimidazo[1,2-a]pyridin-3-yl}carbonyl)amino]-2-methylbutan-2-yl}carbamat (Enantiomer A)

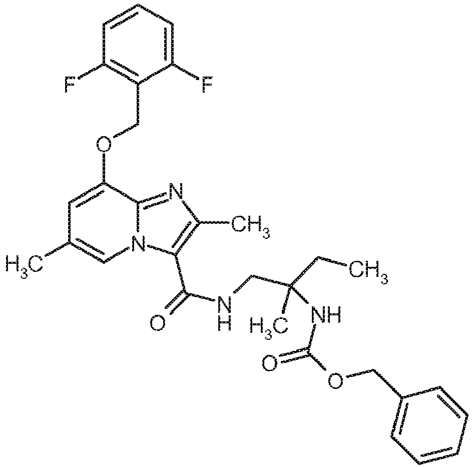

2.34 g (6.75 mmol; Reinheit ca. 96%) 8-[(2,6-Difluorbenzyl)oxy]-2,6-dimethylünidazo[1,2-a]pyridin-3-carbonsäure Beispiel 21A, 2.82 g (7.43 mmol) *O*-(7-Azabenzotriazol-1-yl)-*N,N,N'N'-*tetramethyluroniumhexafluorphosphat (HATU) und 2.62 g (20.25 mmol) *N,N-*Diisopropyl-ethylamin wurden in 43 ml DMF vorgelegt und 20 min bei RT gerührt. Anschließend wurden 1.80 g (7.43 mmol) *ent*-Benzyl-(1-amino-2-methylbutan-2-yl)carbamat (Enantiomer A) Beispiel 274A hinzugegeben und über Nacht bei RT gerührt. Die Reaktionslösung wurde mit ca. 200 ml Wasser versetzt und 45 min bei RT gerührt. Der entstandene Feststoff wurde abfiltriert, in Essigsäureethylester gelöst und zweimal mit Wasser gewaschen. Die organische Phase wurde über Natriumsulfat getrocknet, filtriert und eingeengt. Der Rückstand wurde in Essigsäureethylester gelöst und mit 0.1 N wässriger Salzsäure und Wasser gewaschen. Die organische Phase wurde über Natriumsulfat getrocknet, filtriert und eingeengt. Es wurden 3.55 g der Zielverbindung (96% d. Th.) erhalten.
LC-MS (Methode 2): Rₜ =1.08 min
MS (ESpos): m/z = 551 (M+H)⁺
¹H-NMR (400 MHz, DMSO-d₆): δ = 0.82 (t, 3H), 1.19 (s, 3H), 1.52 -1.63 (m, 1H), 1.75 -1.87 (m, 1H), 2.31 (s, 3H), 3.46 - 3.58 (m, 2H), 5.00 (s, 2H), 5.30 (s, 2H), 6.98 (br. s, 1H), 7.05 (s, 1H), 7.19 - 7.39 (m, 7H), 7.54 - 7.64 (m, 1H), 7.75 (br. s, 1H), 8.48 (s, 1H), [weiteres Signal unter DMSO-Signal verborgen].

### Beispiel 277A

### ent-Benzyl-{1-[({8-[(2,6-difluorbenzyl)oxy]-2,6-dimethylimidazo[1,2-a]pyridin-3-yl}carbonyl)amino]-2-methylbutan-2-yl}carbamat (Enantiomer B)

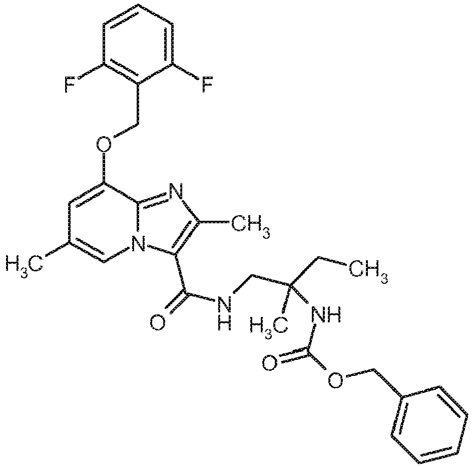

1.40 g (4.03 mmol; Reinheit ca. 96%) 8-[(2,6-Difluorbenzyl)oxy]-2,6-dimethylimidazo[1,2-a]pyridin-3-carbonsäure Beispiel 21A, 1.69 g (4.43 mmol) *O*-(7-Azabenzotriazol-1-yl)-*N,N,N'N*'-tetramethyluroniumhexafluorphosphat (HATU) und 1.56 g (12.09 mmol) N,N-Diisopropylethylamin wurden in 26 ml DMF vorgelegt und 20 min bei RT gerührt. Anschließend wurden 1.00 g (4.23 mmol) *ent*-Benzyl-(1-amino-2-methylbutan-2-yl)carbamat (Enantiomer B) Beispiel 275A hinzugegeben und über Nacht bei RT gerührt. Es wurden nochmals 48 mg (0.20 mmol) *ent*-Benzyl-(1-amino-2-methylbutan-2-yl)carbamat (Enantiomer B) hinzugegeben und 30 min bei RT gerührt. Die Reaktionslösung wurde mit ca. 200 ml Wasser versetzt und 45 min bei Raumtemperatur gerührt. Der entstandene Feststoff wurde abfiltriert und zweimal mit Wasser gewaschen. Es wurden 2.06 g der Zielverbindung (89% d. Th.; Reinheit ca. 96%) erhalten. Der verwendete Filter wurde mit Acetonitril ausgespült und das Lösungsmittel wurde eingeengt. Es wurden nochmals 0.12 g der Zielverbindung (5% d. Th.; Reinheit ca. 96%) erhalten.
LC-MS (Methode 2): Rₜ =1.08 min
MS (ESpos): m/z = 551 (M+H)⁺
¹H-NMR (400 MHz, DMSO-d₆): δ = 0.82 (t, 3H), 1.19 (s, 3H), 1.51 -1.63 (m, 1H), 1.75 -1.87 (m, 1H), 2.31 (s, 3H), 3.46 - 3.58 (m, 2H), 5.00 (s, 2H), 5.29 (s, 2H), 6.92 (s, 1H), 7.05 (s, 1H), 7.19 - 7.39 (m, 7H), 7.54 - 7.64 (m, 1H), 7.69 (t, 1H), 8.48 (s, 1H), [weiteres Signal unter DMSO-Signal verborgen].

### Beispiel 278A

### ent-Benzyl-{1-[({2,6-dimethyl-8-[(2,3,6-trifluorbenzyl)oxy]imidazo[1,2-a]pyridin-3-yl}carbonyl)amino]-2-methylbutan-2-yl}carbamat-Trifluoracetat (Enantiomer A)

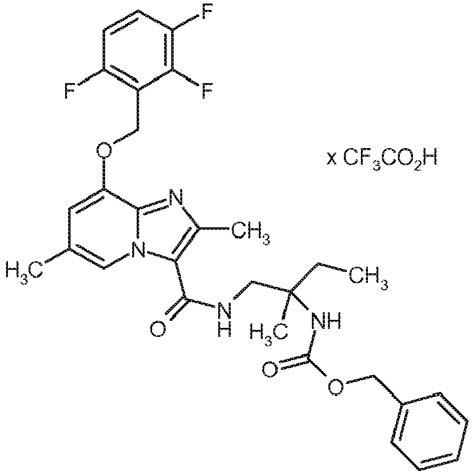

282 mg (0.81 mmol) 2,6-Dimethyl-8-[(2,3,6-trifluorbenzyl)oxy]imidazo[1,2-a]pyridin-3-carbonsäure Beispiel 265A, 337 mg (0.89 mmol) *O*-(7-Azabenzotriazol-1-yl)-*N,N,N'N'-*tetramethyluroniumhexafluorphosphat (HATU) und 313 mg (2.42 mmol) *N,N-*Diisopropylethylamin wurden in 5.1 ml DMF vorgelegt und 20 min bei RT gerührt. Anschließend wurden 219 mg (0.93 mmol) *ent*-Benzyl-(1-amino-2-methylbutan-2-yl)carbamat (Enantiomer A) Beispiel 274A hinzugegeben und über Nacht bei RT gerührt. Die Reaktionslösung wurde mit wenig Wasser/Acetonitril versetzt und mittels präparativer HPLC gereinigt (RP18 Säule, Laufmittel: Acetonitril/Wasser-Gradient unter Zusatz von 0.1% TFA). Es wurden 389 mg der Zielverbindung (71% d. Th.) erhalten.
LC-MS (Methode 2): Rₜ = 1.14 min
MS (ESpos): m/z = 569 (M-TFA+H)⁺
¹H-NMR (400 MHz, DMSO-d₆): δ = 0.82 (t, 3H), 1.19 (s, 3H), 1.49 -1.62 (m, 1H), 1.76 -1.89 (m, 1H), 2.38 (s, 3H), 3.48 - 3.60 (m, 2H; überlagrt mit Lösungsmittel-Signal), 4.95 - 5.05 (m, 2H), 5.43 (s, 2H), 7.08 (s, 1H), 7.22 - 7.43 (m, 7H), 7.64 - 7.74 (m, 1H), 8.18 (br. s, 1H), 8.53 (s, 1H), [weiteres Signal unter DMSO-Signal verborgen].

### Beispiel 279A

### ent-Benzyl-{l-[({2,6-dimethyl-8-[(2,3,6-trifluorbenzyl)oxy]imidazo[1,2-a]pyridin-3-yl}carbonyl)amino]-2-methylbutan-2-yl}carbamat-Trifluoracetat (Enantiomer B)

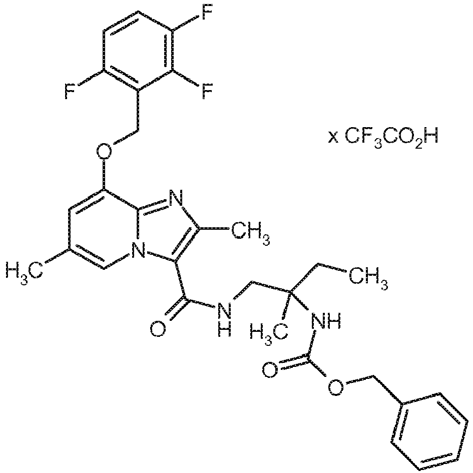

282 mg (0.81 mmol) 2,6-Dimethyl-8-[(2,3,6-trifluorbenzyl)oxy]imidazo[1,2-a]pyridin-3-carbonsäure Beispiel 265A, 337 mg (0.89 mmol) *O*-(7-Azabenzotriazol-1-yl)-*N,N,N'N'-*tetramethyluroniumhexafluorphosphat (HATU) und 313 mg (2.42 mmol) *N*,*N-*Diisopropylethylamin wurden in 5.1 ml DMF vorgelegt und 20 min bei RT gerührt. Anschließend wurden 200 mg (0.85 mmol) *ent*-Benzyl-(1-amino-2-methylbutan-2-yl)carbamat (Enantiomer B) Beispiel 275A hinzugegeben und über Nacht bei RT gerührt. Die Reaktionslösung wurde mit wenig Wasser/Acetonitril versetzt und mittels präparativer HPLC gereinigt (RP18 Säule, Laufmittel: Acetonitril/Wasser-Gradient unter Zusatz von 0.1% TFA). Es wurden 206 mg der Zielverbindung (36% d. Th.; Reinheit 97%) erhalten.
LC-MS (Methode 2): Rₜ = 1.13 min
MS (ESpos): m/z = 569 (M-TFA+H)⁺
¹H-NMR (400 MHz, DMSO-d₆): δ = 0.82 (t, 3H), 1.19 (s, 3H), 1.49 -1.62 (m, 1H), 1.76 -1.89 (m, 1 H), 2.39 (s, 3H), 3.49 - 3.61 (m, 2H; überlagrt mit Lösungsmittel-Signal), 4.95 - 5.05 (m, 2H), 5.44 (s, 2H), 7.08 (s, 1H), 7.23 - 7.46 (m, 7H), 7.64 - 7.74 (m, 1H), 8.20 (br. s, 1H), 8.53 (s, 1H), [weiteres Signal unter DMSO-Signal verborgen].

### Beispiel 280A

### 5-Methoxy-2-nitropyridin-3₋ol

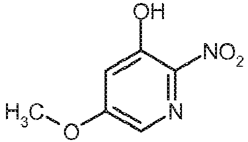

1) Unter Argon wurden 1.46 g (4.8 mmol) Tetra-n-butylammoniumnitrat in 10 ml Dichlormethan bei 0°C langsam mit 0.68 ml (4.8 mmol) Trifluoressigsäureanhydrid versetzt und 10 min bei 0°C gerührt.
2) 500 mg (4 mmol) 5-Methoxypyridin-3-ol wurden in einem separaten Reaktionskolben unter Argon in 10 ml Dichlormethan gelöst und bei -30°C wurde die Lösung aus Schritt 1) zugetropft. Das Reaktionsgemisch wurde im auftauendem Eisbad (nicht wärmer als 0°C) 4 h gerührt. Die Reaktionslösung wurde mit Kieselgur versetzt, bei niedriger Temperatur eingeengt und mittels Kieselgelchromatographie gereinigt (Laufmittel: Cyclohexan/Essigester: 9/1). Es wurden 637 mg der Zielverbindung (94% d. Th.) erhalten.
   LC-MS (Methode 2): Rₜ = 0.58 min
   MS (ESpos): m/z = 171 (M+H)⁺
   ¹H-NMR (400 MHz, DMSO-d₆): δ = 3.90 (s, 3 H), 7.11 (d, 1 H), 7.78 (d, 1 H), 11.35 (br. 1 H).

### Beispiel 281A

### 3-[(2,6-Difluorbenzyl)oxy]-5-methoxy-2-nitropyridin

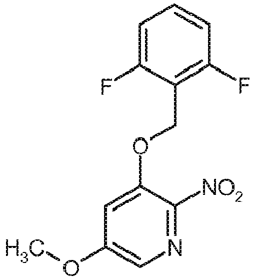

0.76 g (4.47 mmol) 5-Methoxy-2-nitropyridin-3-ol aus Beispiel 280A und 2.18 g (6.70 mmol) Cäsiumcarbonat wurden in 12.5 ml DMF mit 0.93 g (4.47 mmol) 2,6-Difluorbenzylbromid versetzt und bei RT über Nacht gerührt. Das Reaktionsgemisch wurde in 100 ml 1 N wässrige Salzsäure gegeben und 30 min bei RT gerührt. Der Feststoff wurde abfiltriert, mit Wasser gewaschen und im Hochvakuum getrocknet. Es wurden 1.28 g (97% d. Th.) der Titelverbindung erhalten.
LC-MS (Methode 2): Rₜ = 1.02 min
MS (ESpos): m/z = 297 (M+H)⁺
¹H-NMR (400 MHz, DMSO-d₆): δ = 4.00 (s, 3 H), 5.42 (s, 2 H), 7.21 (t, 2 H), 7.58 (quintett, 1 H), 7.70 (d, 1 H), 7.88 (d, 1 H).

### Beispiel 282A

### 3-[(2,6-Difluorbenzyl)oxy]-5-methoxypyridin-2-amin

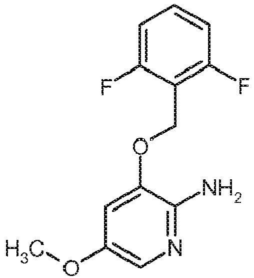

1.25 g (4.22 mmol) 3-[(2,6-Difluorbenzyl)oxy]-5-methoxy-2-nitropyridin aus Beispiel 281A wurden in 12.7 ml Ethanol mit 0.73 g (13.1 mmol) Eisenpulver versetzt und zum Rückfluß erhitzt. 3.23 ml (38.8 mmol) konzentrierte wässrige Salzsäure wurden langsam zugetropft und es wurde weitere 30 min am Rückfluß gerührt. Das Reaktionsgemisch wurde abgekühlt und in ein Eis-Wassergemisch eingerührt und 30 min gerührt. Das organische Lösungsmittel wurde im Vakuum entfernt, die wässrige Phase mit 1 N wässriger Natronlauge alkalisch gestellt, mit Dichlormethan, gerührt und die Mischung über Celite abfiltriert. Es wurde mit Dichlormethan gewaschen und die wässrige Phase zweimal mit Dichlormethan extrahiert. Die vereinten organische Phasen wurden über Natriumsulfat getrocknet, filtriert, das Filtrat eingeengt und der Rückstand im Hochvakuum getrocknet. Es wurden 974 mg der Zielverbindung (85% d. Th.) erhalten.
LC-MS (Methode 2): Rₜ = 0.61 min
MS (ESpos): m/z = 267 (M+H)⁺
¹H-NMR (400 MHz, DMSO-d₆): δ = 3.72 (s, 3 H), 5.10 (s, 2 H), 5.14 (s, 2 H), 7.04 (d, 1 H), 7.20 (t, 2 H), 7.32 (d, 1 H), 7.55 (quintett, 1 H).

### Beispiel 283A

### Ethyl-8-[(2,6₋difluorbenzyl)oxy]-6-methoxy-2-methylimidazo[1,2-a]pyridin-3-carboxylat

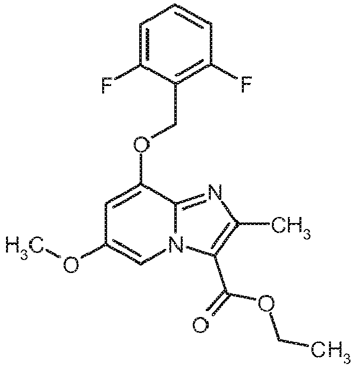

0.97 g (3.64 mmol) 3-[(2,6-Difluorbenzyl)oxy]-5-methoxypyridin-2-amin aus Beispiel 282A wurden in 18.5 ml Ethanol mit 0.93 g gepulvertem Molekularsieb 3Å und 6.0 g (36.43 mmol) Ethyl-2-chloracetoacetat versetzt und über Nacht zum Rückfluß erhitzt. Das Reaktionsgemisch wurde am Trockeneisrotationsverdampfer bei einer Wasserbadtemperatur von 85°C eingeengt. Das Rohprodukt wurde mittels Kieselgel-Chromatographie gereinigt (Laufmittel: Cyclohexan/Essigsäureethylester: 9/1 isokratisch). Es wurden 583mg der Zielverbindung (41% d. Th.) erhalten.
LC-MS (Methode 2): Rₜ =1.09 min
MS (ESpos): m/z = 377 (M+H)⁺
¹H-NMR (400 MHz, DMSO-d₆): δ = 1.36 (t, 3 H), 2.54 (s, 3 H; verdeckt durch DMSO-Signal), 3.83 (s, 3 H), 4.37 (q, 2 H), 5.32 (s, 2 H), 7.05 (d, 1 H), 7.23 (t, 2 H), 7.60 (quintett, 1 H), 8.58 (d, 1 H).

### Beispiel 284A

### 8-[(2,6-Difluorbenzyl)oxy]-6-methoxy-2-methylimidazo[1,2-a]pyridin-3-carbonsäure

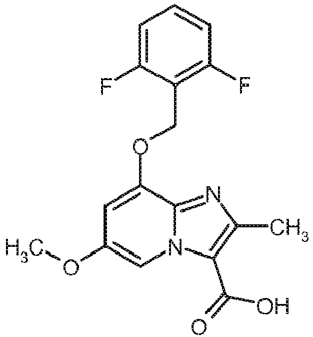

580 mg (1.54 mmol) Ethyl-8-[(2,6-difluorbenzyl)oxy]-6-methoxy-2-methylimidazo[1,2,a]pyridin-3-carboxylat aus Beispiel 283A wurden in 33 ml THF/Methanol (5/1) mit 7.7 ml 1 M wässriger Lithiumhydroxid-Lösung versetzt und über Nacht bei 40°C gerührt. Die Reaktionsmischung wurde abgekühlt, unter Eiskühlung mit 6 N wässriger Salzsäure auf pH4 gestellt und anschließend am Rotationsverdampfer von den organischen Lösemitteln befreit. Der entstandene Feststoff wurde abfiltriert, mit Wasser gewaschen und anschließend im Hochvakuum getrocknet. Es wurden 507 mg der Zielverbindung (94% d. Th.) erhalten.
LC-MS (Methode 2): Rₜ = 0.74 min
MS (ESpos): m/z = 349 (M+H)⁺
¹H-NMR (400 MHz, DMSO-d₆): δ = 2.54 (s, 3 H; überlagert von DMSO-Signal), 3.85 (s, 3 H), 5.38 (s, 2 H), 7.20 - 7.32 (m, 3 H), 7.61 (quintett, 1 H), 8.68 (d, 1 H), 13.40 (br. s, 1 H).

### Beisipiel 285A

### rac-Benzyl-(2-cyanpentan-2-yl)carbamat

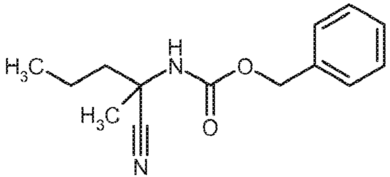

20 g (178.3 mmol) *rac*-2-Amino-2-methylpentanonitril (beschrieben in: Deng, S L. et al., Synthesis 2001, 2445-2449; Freifelder, M. et al., J. Am. Chem. Soc. 1960, 696-698) wurden in 2.63 1 THF/Wasser (8/1) mit 76.4 g (552.7 mmol) Kaliumcarbonat versetzt. Bei 0°C wurden 27.6 ml (196.1 mmol) Chlorameisensäurebenzylester langsam zugetropft und es wurde über Nacht bei RT gerührt. Die Reaktionsmischung wurde eingeengt, der Rückstand mit Wasser versetzt und zweimal mit Essigsäureethylester extrahiert. Die vereinigten organischen Phasen wurden über Natriumsulfat getrocknet, filtriert und eingeengt. Der Rückstand wurde mittels Kieselgel-Chromatographie (Laufmittel: Cyclohexan/Essigsäureethylester 4/1) gereinigt. Es wurden 43.84 g der Zielverbindung (76% d. Th., Reinheit 76%) erhalten.
LC-MS (Methode 2): Rₜ = 1.02 min
MS (ESpos): m/z = 247 (M+H)⁺
¹H-NMR (400 MHz, DMSO-d₆): δ = 0.90 (t, 3H), 1.31-1.48 (m, 2H), 1.52 (s, 3H), 1.70 -1.88 (m, 2H), 5.07 (s, 2H), 7.30 - 7.42 (m, 5H), 8.00 (br. s, 1H).

### Beispiel 286A

### ent-Benzyl-(2-cyanpentan-2-yl)carbamat (Enantiomer A) aus 285A

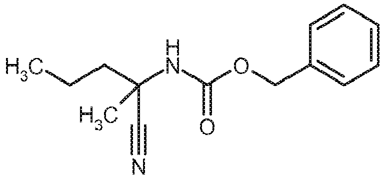

43.8 g (135.3 mmol) rac-Benzyl-(2-cyanpentan-2-yl)carbamat aus Beispiel 285A wurden durch präparative Trennung an chiraler Phase in die Enantiomere getrennt [Säule: SFC Chiralpak AZ-H, 5 µm, 250 x 50 mm, Eluent: 85% CO₂, 15% Methanol, Fluß: 250 ml/min; Temperatur: 28°C, backpressure: 100 bar, Detektion: 220 nm].
Enantiomer A: Ausbeute: 13.13 g (>99% ee)
Rₜ = 2.76 min [SFC Chiralpak AZ-H, 5 µm, 250 x 4.6 mm; Eluent: 90% CO₂, 10% Methanol; Fluß: 3 ml/min; Detektion: 220 nm].

### Beispiel 287A

### ent-Benzyl-(2-cyanpentan-2-yl)carbamat (Enantiomer B) aus 285A

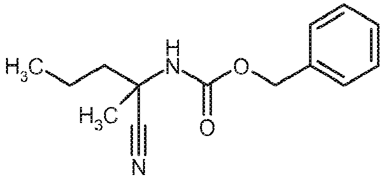

43.8 g (135.3 mmol) rac-Benzyl-(2-cyanpentan-2-yl)carbamat aus Beispiel 285A wurden durch präparative Trennung an chiraler Phase in die Enantiomere getrennt [Säule: SFC Chiralpak AZ-H, 5 µm, 250 x 50 mm, Eluent: 85% CO₂, 15% Methanol, Fluß: 250 ml/min; Temperatur: 28°C, backpressure: 100 bar, Detektion: 220 nm].
Enantiomer B: Ausbeute: 13.48 g (ca. 90.4% ee)
Rₜ = 3.93 min [SFC Chiralpak AZ-H, 5 µm, 250 x 4.6 mm; Eluent: 90% CO₂,10% Methanol; Fluß: 3 ml/min; Detektion: 220 nm].

### Beispiel 288A

### ent-Benzyl-(1-amino-2-methylpentan-2-yl)carbamat (Enantiomer A)

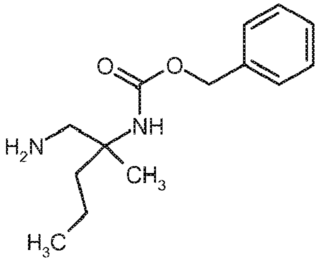

13.1 g (53.31 mmol) *ent*-Benzyl-(2-cyanpentan-2-yl)carbamat (Enantiomer A) aus Beispiel 286A VAK5346-1-3 wurden in 155 ml 7 N Ammoniak-Lösung in Methanol gelöst und unter Argon mit 16.5 g Raney-Nickel (50%ige wässrige Aufschlämmung) versetzt. Das Reaktionsgemisch wurde über Nacht im Autoklaven bei 20-30 bar hydriert. Es wurde über Celite abfiltriert, mit Methanol, Dichlormethan/2 N Ammoniak in Methanol (20/1) gespült und eingeengt. Der Rückstand wurde mittels Kieselgel-Chromatographie (Laufmittel: Dichlormethan/Methanol 40/1nach 20/1) gereinigt. Es wurden 9.85 g der Zielverbindung (63% d. Th., Reinheit 86%) erhalten.
LC-MS (Methode 2): Rₜ = 0.58 min
MS (ESpos): m/z = 251 (M+H)⁺
¹H-NMR (400 MHz, DMSO-d₆): δ = 0.83 (t, 3H), 1.11 (s, 3H), 1.15 - 1.24 (m, 2H), 1.37 (br. s, 2H), 1.42 - 1.51 (m, 1H), 1.53 - 1.63 (m, 1H), 2.46 (d, 1H), 2.66 (d, 1H), 4.97 (s, 2H), 6.69 (br. s, 1H), 7.26 - 7.40 (m, 5H).

### Beispiel 289A

### ent-Benzyl-(1-amino-2-methylpentan-2-yl)carbamat (Enantiomer B)

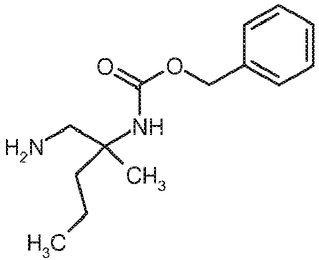

13.5 g (54.73 mmol) ent-Benzyl-(2-cyanpentan-2-yl)carbamat (Enantiomer B) aus Beispiel 287A VAK5347-1-4 wurden in 159 ml 7 N Ammoniak-Lösung in Methanol gelöst und unter Argon mit 16.95 g Raney-Nickel (50%ige wässrige Aufschlämmung) versetzt. Das Reaktionsgemisch wurde über Nacht im Autoklaven bei 20-30 bar hydriert. Die Reaktionsmischung wurde über Celite abfiltriert, mit Methanol, Dichlormethan/2 N Ammoniak in Methanol (10/1) gewaschen und eingeengt. Der Rückstand wurde mittels Kieselgel-Chromatographie (Laufmittel: Dichlormethan/Methanol 40/1 nach 20/1) gereinigt. Es wurden 9.46 g der Zielverbindung (61% d. Th., Reinheit 88%) erhalten.
LC-MS (Methode 2): Rₜ = 0.58 min
MS (ESpos): m/z = 251 (M+H)⁺
¹H-NMR (400 MHz, DMSO-d₆): δ = 0.83 (t, 3H), 1.11 (s, 3H), 1.15 - 1.24 (m, 2H), 1.37 (br. s, 2H), 1.42 - 1.51 (m, 1H), 1.53 - 1.63 (m, 1H), 2.46 (d, 1H), 2.66 (d, 1H), 4.97 (s, 2H), 6.69 (br. s., 1H), 7.26 - 7.40 (m, 5H).

### Beispiel 290A

### ent-Benzyl-{1-[({8-[(2,6-difluorbenzyl)oxy]-6-methoxy-2-methylimidazo[1,2-a]pyridin-3-yl}carbonyl)amino]-2-methylpentan-2-yl}carbamat Trifluoracetat (Enantiomer B)

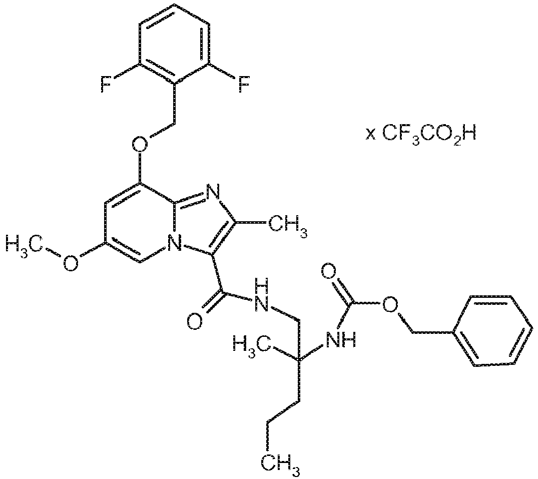

110 mg (0.32 mmol) 8-[(2,6-Difluorbenzyl)oxy]-6-methoxy-2-methylimidazo[1,2-a]pyridin-3-carbonsäure aus Beispiel 284A wurden mit 156 mg (0.41 mmol) HATU und 0.28 ml (1.58 mmol) N,N-Diisopropylethylamin versetzt. Das Reaktionsgemisch wurde 10 min bei RT gerührt, anschließend mit 103 mg (0.41 mmol) *ent*-Benzyl-(1-amino-2-methylpentan-2-yl)carbamat (Enantiomer B) aus Beispiel 289A versetzt und 1 Stunde bei RT gerührt. Die Reaktionsmischung wurde mit TFA versetzt und mittels präparativer HPLC (RP18 Säule, Laufmittel: Acetonitril/Wasser-Gradient unter Zusatz von 0.1% TFA) getrennt. Die Produktfraktionen wurden am Rotationsverdampfer eingedampft. Es wurden 180 mg der Zielverbindung (82% d. Th.) erhalten.
LC-MS (Methode 2): Rₜ =1.15 min
MS (ESpos): m/z = 581 (M-TFA+H)⁺

### Beispiel 291A

### ent-Benzyl-{1-[({8-[(2,6-difluorbenzyl)oxy]-6-methoxy-2-methylimidazo[1,2-a]pyridin-3-yl}carbonyl)amino]-2-methylbutan-2-yl}carbamat Trifluoracetat (Enantiomer A)

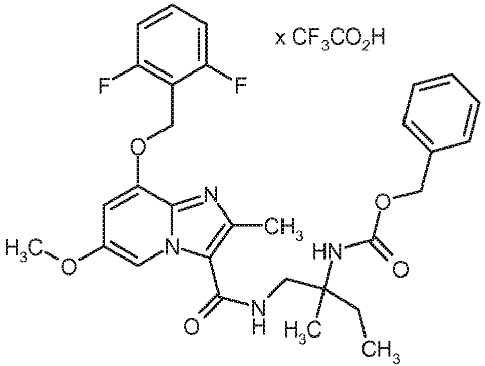

110 mg (0.32 mmol) 8-[(2,6-Difluorbenzyl)oxy]-6-methoxy-2-methylimidazo[1,2-a]pyridin-3-carbonsäure aus Beispiel 284A wurden mit 156 mg (0.41 mmol) HATU und 0.28 ml (1.58 mmol) N,N-Diisopropylethylamin versetzt. Das Reaktionsgemisch wurde 10 min bei RT gerührt, mit 97 mg (0.41 mmol) *ent-* Benzyl-(1-amino-2-methylbutan-2-yl)carbamat (Enantiomer A) aus Beispiel 274A versetzt und 45 min bei RT gerührt. Die Reaktionsmischung wurde mit TFA versetzt und mittels präparativer HPLC (RP18 Säule, Laufmittel: Acetonitril/Wasser-Gradient unter Zusatz von 0.1% TFA) getrennt. Die Produktfraktionen wurden am Rotationsverdampfer eingeengt. Es wurden 157 mg der Zielverbindung (73% d. Th.) erhalten.
LC-MS (Methode 2): Rₜ =1.09 min
MS (ESpos): m/z = 567 (M-TFA+H)⁺

### Beispiel 292A

### ent-Benzyl-{1-[({6-brom-8-[(2,6-difluorbenzyl)oxy]-2-methylimidazo[1,2-a]pyridin-3-yl}carbonyl)amino]-2-methylpentan-2yl}carbamat Trifluoracetat (Enantiomer B)

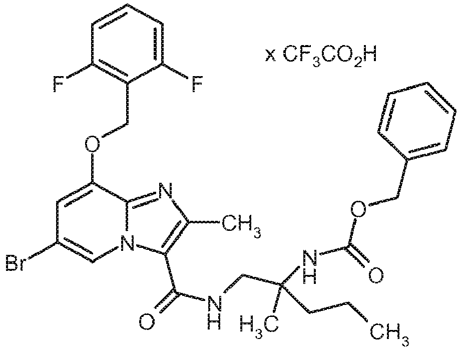

2.88 g (7.26 mmol) 6-Brom-8-[(2,6-difluorbenzyl)oxy]-2-methylimidazo[1,2,a]pyridin-3-carbonsäure aus Beispiel 19A wurden in 24.1 ml DMF mit 2.57 g (7.99 mmol) (Benzotriazol-1-yloxy)bisdimethylaminomethyliumfluoroborat und 4 ml (36.31 mmol) 4-Methylmorpholin versetzt. Anschließend wurden 2.0 g (7.99 mmol) *ent*-Benzyl-(1-amino-2-methylpentan-2-yl)carbamat (Enantiomer B) aus Beispiel 289A zugegeben und das Reaktionsgemisch 1 Stunde bei RT gerührt. Die Reaktionslösung wurde mit 200 ml Wasser versetzt, der enstandene Feststoff ca. 30 min gerührt, abfiltriert, mit Wasser gewaschen und im Hochvakuum getrocknet. Es wurden 4.41 g der Zielverbindung (73% d. Th., Reinheit 76%) erhalten.
LC-MS (Methode 2): Rₜ = 1.38 min
MS (ESpos): m/z = 629 (M-TFA+H)⁺
¹H-NMR (400 MHz, DMSO-d₆): δ = 0.85 (t, 3H), 1.19 (s, 3H), 1.23 -1.34 (m, 2H), 1.44 -1.54 (m, 1H), 1.71 - 1.80 (m, 1H), 2.53 (s, 3H), 3.47 - 3.59 (m, 2H), 5.00 (s, 2H), 5.38 (s, 2H), 7.08 (br. s., 1H), 7.21 - 7.37 (m, 7H), 7.40 (s,1H), 7.56 - 7.66 (m, 1H), 7.91 - 7.98 (m, 1H), 8.83 (s, 1H).

### Beispiel 293A

### ent-Benzyl-{1-[({8-[(2,6-difluorbenzyl)oxy]-2-methyl-6-(morpholin-4-yl)imidazo[1,2-a]pyridin-3-yl}carbonyl)amino]-2-methylpentan-2-yl}carbamat (Enantiomer B)

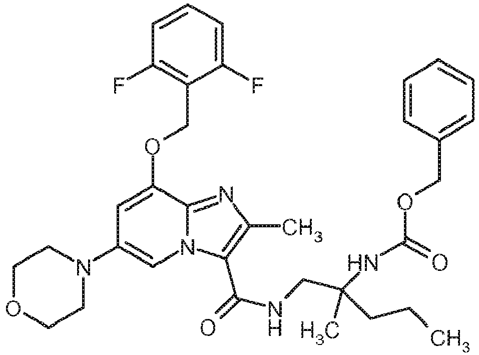

50 mg (0.07 mmol) *ent*-Benzyl-{1-[({6-brom-8-[(2,6-difluorbenzyl)oxy]-2-methylimidazo[1,2-a]pyridin-3-yl}carbonyl)amino]-2-methylpentan-2-yl}carbamat Trifluoracetat (Enantiomer B) aus Beispiel 292A, 0.02 ml (0.20 mmol) Morpholin, 9 mg (0.09 mmol) Natrium-tert.-butylat, 2.5 mg (0.003 mmol) Tris(dibenzylidenaceton)dipalladium und 3.8 mg (0.008 mmol) Dicyclohexyl[2',4',6'-tri(propan-2-yl)biphenyl-2-yl]phosphan [X-PHOS] wurden unter Argon mit 1.7 ml Toluol (abs.) versetzt und über Nacht bei 100°C gerührt. Die Reaktionslösung wurde eingeengt, der Rückstand in Dichlormethan aufgenommen und zweimal mit Wasser gewaschen.

Die organische Phase wurde eingeengt und der Rückstand mittels präparativer HPLC gereinigt (RP18 Säule, Laufmittel: Acetonitril/Wasser-Gradient unter Zusatz von 0.1% TFA). Das Produkt wurde erneut in Dichlormethan aufgenommen, einmal mit gesättigter, wässriger Natriumhydrogencarbonat-Lösung gewaschen, die wässrige Phase zweimal mit Dichlormethan extrahiert, die vereinten organischen Phasen über Natriumsulfat getrocknet, filtriert und eingeengt. Die Produktfraktion wurde anschließend nochmal mittels Dünnschichtchromatographie (Laufmittel: Dichlormethan/Methanol = 20/1) gereinigt. Es wurden 11 mg der Zielverbindung (26% d. Th.) erhalten.
LC-MS (Methode 2): Rₜ = 1.09 min
MS (ESpos): m/z = 636 (M+H)⁺
¹H-NMR (400 MHz, DMSO-d₆): δ = 0.85 (t, 3H), 1.20 (s, 3H), 1.23 -1.34 (m, 2H), 1.45 -1.58 (m, 1H), 1.69 - 1.79 (m, 1H), 2.50 (s, 3H unter Lösungsmittel-Peak), 3.05 (t, 4H), 3.48 - 3.54 (m, 2H), 3.76 (t, 4H), 4.99 (s, 2H), 5.32 (s, 2H), 7.02 - 7.08 (m, 2H), 7.20 - 7.27 (m, 2H), 7.28 - 7.37 (m, 5H), 7.54 - 7.66 (m, 2H), 8.16 - 8.21 (m, 1H).

### Beispiel 294A

### ent-Benzyl-{1-[({6-cyclopropyl-8-[(2,6-difluorbenzyl)oxy]-2-methylimidazo[1,2-a]pyridin-3-yl}carbonyl)amino]-2-methylpentan-2-yl}carbamat Trifluoracetat (Enantiomer B)

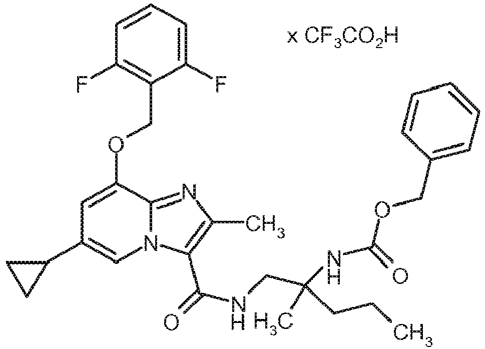

50 mg (0.07 mmol) *ent*-Benzyl-{1-[({6-brom-8-[(2,6-difluorbenzyl)oxy]-2-methylimidazo[1,2-a]pyridin-3-yl}carbonyl)amino]-2-methylpentan-2-yl}carbamat Trifluoracetat (Enantiomer B) aus Beispiel 292A, 7.5 mg (0.09 mmol) Cyclopropylboronsäure, 57 mg (0.27 mmol) Kaliumphosphat, 3 mg (0.01 mmol) Tricyclohexylphosphin und 1.2 mg (0.005 mmol) Palladium(II)acetat wurden unter Argon vorgelegt und mit 0.6 ml Toluol/Wasser 20/1 versetzt. Es wurde 5 min Argon durch das Reaktionsgemisch durchgeleitet und das Reaktionsgemisch wurde über Nacht bei 100°C gerührt. Das Reaktionsgemisch wurde eingeengt und der Rückstand mittels präparativer HPLC gereinigt (RP18 Säule, Laufmittel: Acetonitril/Wasser-Gradient unter Zusatz von 0.1% TFA). Es wurden 27 mg der Zielverbindung (56% d. Th.) erhalten.
LC-MS (Methode 2): Rₜ = 1.20 min
MS (ESpos): m/z = 591 (M-TFA+H)⁺
¹H-NMR (400 MHz, DMSO-d₆): δ = 0.80 - 0.92 (m, 6H), 1.02 (d, 2H), 1.22 (s, 3H), 1.24 -1.36 (m, 3H), 1.44 -1.58 (m, 1H), 1.73 -1.82 (m, 1H), 2.03 - 2.15 (m, 1H), 2.55 (br. s., 3H), 3.50 - 3.62 (m, 2H), 5.01 (s, 2H), 5.44 (br. s., 2H), 7.08 (br. s., 1H), 7.22 - 7.41 (m, 9H), 7.62 (quin, 1H), 8.54 (s, 1H).

### Beispiel 295A

### rac-2-Amino-3-fluor-2-methylpropanonitril

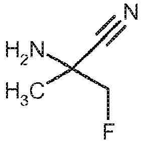

Die Titelverbindung ist literaturbekannt:
1) McConathy, J. et al., Journal of Medicinal Chemistry 2002, 45, 2240-2249.
2) Bergmann, E.D. et al., Journal of the Chemical Society 1963,3462-3463.

### Weitere Methode:

1.0 g (0.94 ml; 13.15 mmol) Fluoraceton wurden in 11 ml 2 N Ammoniak in Methanol vorgelegt. Bei RT wurden nacheinander 721 mg (14.72 mmol) Natriumcyanid und 788 mg (14.72 mmol) Ammoniumchlorid zugeben und das Gemisch wurde 2 Stunden bei Rückfluss gerührt. Die Reaktionslösung wurde abgekühlt, filtriert und mit Methylenchlorid gewaschen. Aus der Mutterlauge fiel ein Feststoff aus, der abfiltiert wurde. Aus der Mutterlauge wurden Methylenchlorid und Methanol bei Normaldruck abdestilliert. Es wurden 1.32 g der Zielverbindung (89% d. Th., Reinheit ca. 90%) erhalten. Das Produkt wurde ohne weitere Reinigung in die nächste Reaktion eingesetzt.
GC-MS (Methode 14): Rₜ = 1.64 min
MS (EIpos): m/z = 87 (M-CH₃)⁺

### Beispiel 296A

### rac-Benzyl-(2-cyan-1-fluorpropan-2-yl)carbamat

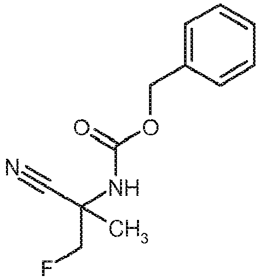

1.34 g (11.83 mmol, ca. 90%ig) rac-2-Amino-3-fluor-2-methylpropanonitril aus Beispiel 295A wurden in 29 ml THF/Wasser (9/1) mit 5.07 g (36.67 mmol) Kaliumcarbonat versetzt. Bei 0°C wurden langsam 1.69 ml (11.83 mmol) Chlorameisensäurebenzylester zugetropft und die Reaktionsmischung wurde über Nacht bei RT gerührt. Das Lösungsmittel wurde abdekantiert und die wässrige Phase zweimal mit THF ausgeschüttelt und das THF anschließend abdekantiert. Die vereinigten organischen Phasen wurden mit Natriumsulfat getrocknet, abfiltriert und eingeengt. Der Rückstand wurde mittels Kieselgelchromatographie getrennt (Laufmittel: Cyclohexan/ Essigsäureethylester 7/3- 9/1) und die Produktfraktionen wurden am Rotationsverdampfer eingedampft. Es wurden 1.89 g der Zielverbindung (66% d. Th.; Reinheit 97%) erhalten.
LC-MS (Methode 2): Rₜ = 0.89 min
MS (ESpos): m/z = 237 (M+H)⁺
¹H-NMR (400 MHz, DMSO-d₆): δ = 1.58 (d, 3H), 4.47 - 4.78 (m, 2H), 5.10 (s, 2H), 7.30 - 7.43 (m, 5H), 8.34 (br. s, 1H).

### Beispiel 297A

### ent-Benzyl-(2-cyan-1-fluorpropan-2-yl)carbamat (Enantiomer A)

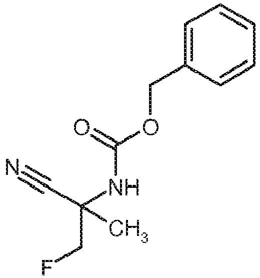

3.0 g (12.69 mmol) *rac*-Benzyl-(2-cyan-1-fluorpropan-2-yl)carbamat aus Beispiel 296A wurden durch präparative Trennung an chiraler Phase in die Enantiomere getrennt [Säule: Daicel Chiralpak AY-H, 5 µm, 250 x 20 mm, Eluent: 80% Iso-Hexan, 20% Isopropanol, Fluß: 15 ml/min; 40°C, Detektion: 220 nm].
Enantiomer A: Ausbeute: 1.18 g (>99% ee)
Rₜ = 5.37 min [Daicel Chiralcel AY-H, 5 µm, 250 x 4.6 mm; Eluent: 70% iso-Hexan, 30% 2-Propanol; Fluss 1.0 ml/min; 40°C; Detektion: 220 nm].

### Beispiel 298A

### ent-Benzyl-(2-cyan-1-fluorpropan-2-yl)carbamat (Enantiomer B)

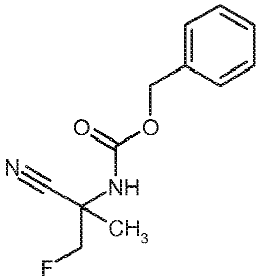

3.0 g (12.69 mmol) *rac*-Benzyl-(2-cyan-1-fluorpropan-2-yl)carbamat aus Beispiel 296A wurden durch präparative Trennung an chiraler Phase in die Enantiomere getrennt [Säule: Daicel Chiralpak AY-H, 5 µm, 250 x 20 mm, Eluent: 80% Iso-Hexan, 20% Isopropanol, Fluß: 15 ml/min; 40°C, Detektion: 220 nm].
Enantiomer B: Ausbeute: 1.18 g (>99% ee)
Rₜ = 6.25 min [Daicel Chiralcel AY-H, 5 µm, 250 x 4.6 mm; Eluent: 70% iso-Hexan, 30% 2-Propanol; Fluss 1.0 ml/min; 40°C; Detektion: 220 nm].

### Beispiel 299A

### rac-Benzyl-(1-amino-3-fluor-2-methylpropan-2-yl)carbamat

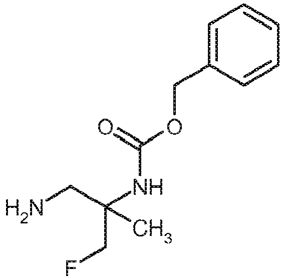

Unter Argon wurden 1.2 g (5.08 mmol) rac-Benzyl-(2-cyan-l-fluorpropan-2-yl)carbamat aus Beispiel 296A in 14.9 ml 7 N Ammoniak in Methanol mit 1.55 g Raney-Nickel (wässrige Aufschlämmung) versetzt und 24 Stunden bei ca. 25 bar Wasserstoff-Druck und RT hydriert. Das Reaktionsgemisch wurde über Kieselgur filtriert, mit Methanol gewaschen und eingeengt. Es wurden 1.2 g der Zielverbindung (98% d. Th.) erhalten.
LC-MS (Methode 2): Rₜ = 0.49 min
MS (ESpos): m/z = 241 (M+H)⁺

### Beispiel 300A

### ent-Benzyl-(1-amino-3-fluor-2-methylpropan-2-yl)carbamat (Enantiomer A)

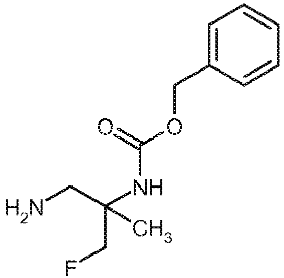

Unter Argon wurden 1.2 g (5.08 mmol) *ent*-Benzyl-(2-cyan-1-fluorpropan-2-yl)carbamat (Enantiomer A) aus Beispiel 297A in 14.9 ml 7 N Ammoniak in Methanol mit 1.55 g Raney-Nickel (wässrige Aufschlämmung) versetzt und 24 Stunden bei ca. 25 bar Wasserstoff-Druck und RT hydriert. Das Reaktionsgemisch wurde über Kieselgur filtriert, mit Methanol gewaschen und eingeengt. Es wurden 700 mg der Zielverbindung (57% d. Th.; Reinheit ca. 85%) erhalten.
LC-MS (Methode 2): Rₜ = 0.52 min
MS (ESpos): m/z = 241 (M+H)⁺

### Beispiel 301A

### ent-Benzyl-(1-amino-3-fluor-2-methylpropan-2-yl)carbamat (Enantiomer B)

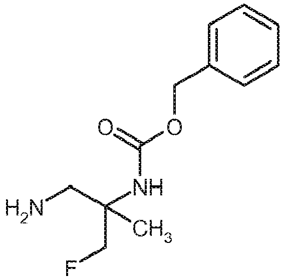

Unter Argon wurden 1.2 g (5.08 mmol) *ent*-Benzyl-(2-cyan-1-fluorpropan-2-yl)carbamat (Enantiomer B) aus Beispiel 298A in 14.9 ml 7 N Ammoniak in Methanol mit 1.55 g Raney-Nickel (wässrige Aufschlämmung) versetzt und 24 Stunden bei ca. 25 bar Wasserstoff-Druck und RT hydriert. Das Reaktionsgemisch wurde über Kieselgur filtriert, mit Methanol gewaschen und eingeengt. Es wurden 1.2 g der Zielverbindung (98% d. Th.; Reinheit ca. 85%) erhalten.
LC-MS (Methode 2): Rₜ = 0.50 min
MS (ESpos): m/z = 241 (M+H)⁺

### Beispiel 302A

### rac-Benzyl-{1-[({8-[(2,6-difluorbenzyl)oxy]-2,6-dimethylimidazo[1,2-a]pyridin-3-yl}carbonyl)amino]-3-fluor-2-methylpropan-2-yl}carbamat

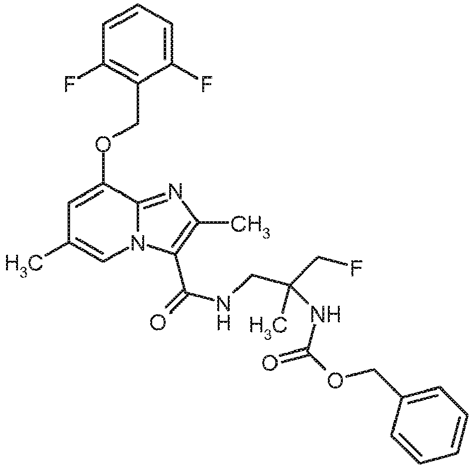

147 mg (0.44 mmol) 8-[(2,6-Difluorbenzyl)oxy]-2,6-dimethylimidazo[1,2-a]pyridin-3-carbonsäure aus Beispiel 21A, 185 mg (0.49 mmol) HATU und 0.31 ml (1.77 mmol) N,N-Diisopropylethylamin wurden unter Argon in 7 ml DMF 20 min gerührt, dann mit 112 mg (0.39 mmol) *rac*-Benzyl-(1-amino-3-fluor-2-methylpropan-2-yl)carbamat aus Beispiel 299A versetzt und über Nacht bei RT gerührt. Es wurde mit Wasser/TFA versetzt und mittels präparativer HPLC gereinigt (RP18 Säule, Laufmittel: Acetonitril/Wasser-Gradient unter Zusatz von 0.05% Ameisensäure). Die Produktfraktionen wurden am Rotationsverdampfer eingeengt. Es wurden 56 mg der Zielverbindung (23% d. Th.) erhalten.
LC-MS (Methode 2): Rₜ = 1.02 min
MS (ESpos): m/z = 555 (M+H)⁺
¹H-NMR (400 MHz, DMSO-d₆): δ = 1.25 - 1.29 (m, 3H), 2.32 (s, 3H), 3.53 - 3.65 (m, 2H), 4.45 - 4.56 (m, 1H), 4.57 - 4.69 (m, 1H), 5.02 (s, 2H), 5.31 (s, 2H), 7.02 (br. s, 1H), 7.20 - 7.27 (m, 2H), 7.28 - 7.38 (m, 5H), 7.55 - 7.65 (m, 1H), 7.89 (br. s, 1H), 8.46 (s, 1H), [weiteres Signal unter Lösungsmittel-Peak].

### Beispiel 303A

### ent-Benzyl-{1-[({8-[(2,6-difluorbenzyl)oxy]-2,6-dimethylimidazo[1,2-a]pyridin-3-yl}carbonyl)amino]-3-fluor-2-methylpropan-2-yl}carbamat Trifluoroacetat (Enantiomer A)

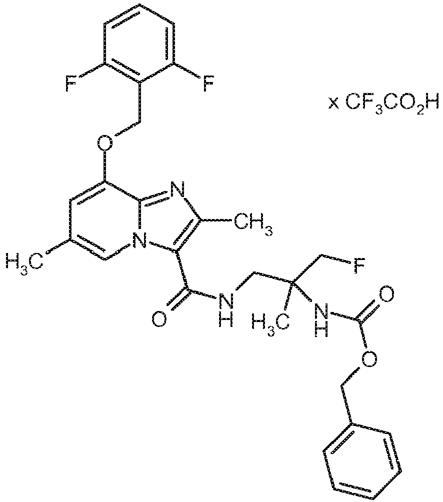

150 mg (0.45 mmol) 8-[(2,6-Difluorbenzyl)oxy]-2,6-dimethylimidazo[1,2-a]pyridin-3-carbonsäure aus Beispiel 21A, 180 mg (0.47 mmol) HATU und 0.24 ml (1.35 mmol) N,N-Diisopropylethylamin wurden in 2.9 ml DMF 20 min gerührt, dann mit 134 mg (0.47 mmol, 85 %ig) *ent*-Benzyl-(1-amino-3-fluor-2-methylpropan-2-yl)carbamat (Enantiomer A) aus Beispiel 300A versetzt und über Nacht bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingeengt. Es wurden 148 mg der Zielverbindung (47% d. Th.) erhalten.
LC-MS (Methode 2): Rₜ = 1.03 min
MS (ESpos): m/z = 555 (M-TFA+H)⁺

### Beispiel 304A

### ent-Benzyl-{1-[({8-[(2,6-difluorbenzyl)oxy]-2,6-dimethylimidazo[1,2-a]pyridin-3-yl}carbonyl)amino]-3-fluor-2-methylpropan-2-yl}carbamat Trifluoroacetat (Enantiomer B)

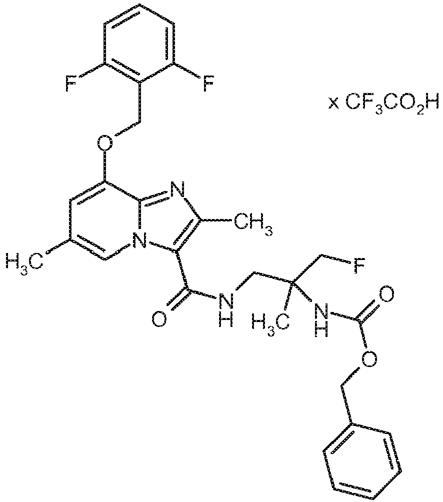

150 mg (0.45 mmol) 8-[(2,6-Difluorbenzyl)oxy]-2,6-dimethylimidazo[1,2-a]pyridin-3-carbonsäure aus Beispiel 21A, 180 mg (0.47 mmol) HATU und 0.24 ml (1.35 mmol) N,N-Diisopropylethylamin wurden in 2.9 ml DMF 20 min gerührt, dann mit 134 mg (0.47 mmol, 85 %ig) *ent*-Benzyl-(1-amino-3-fluor-2-methylpropan-2-yl)carbamat (Enantiomer B) aus Beispiel 301A versetzt und über Nacht bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingengt. Es wurden 201 mg der Zielverbindung (67% d. Th.) erhalten.
LC-MS (Methode 2): Rₜ = 1.04 min
MS (ESpos): m/z = 555 (M-TFA+H)⁺

### Beispiel 305A

### ent-Benzyl-{1-[({2,6-dimethyl-8-[(2,3,6-trifluorbenzyl)oxy]imidazo[1,2-a]pyridin-3-yl}carbonyl)amino]-3-fluor-2-methylpropan-2-yl}carbamat Trifluoroacetat (Enantiomer A)

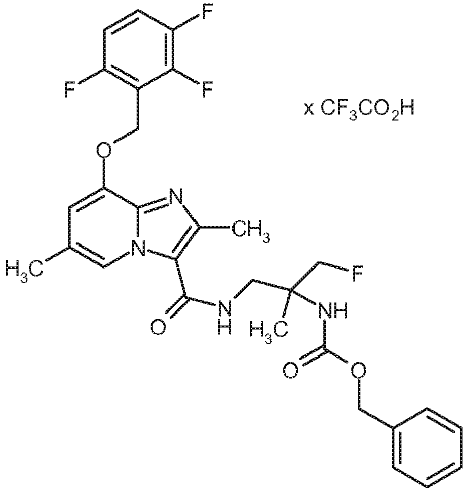

150 mg (0.43 mmol) 2,6-Dimethyl-8-[(2,3,6-trifluorbenzyl)oxy]imidazo[1,2-a]pyridin-3-carbonsäure aus Beispiel 265A , 171 mg (0.45 mmol) HATU und 0.22 ml (1.29 mmol) N,N-Diisopropylethylamin wurden in 2.7 ml DMF 20 min gerührt, dann mit 127 mg (0.45 mmol, 85 %ig) *ent*-Benzyl-(1,amino-3-fluor-2-methylpropan-2-yl)carbamat (Enantiomer A) aus Beispiel 300A versetzt und über Nacht bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingedampft. Es wurden 170 mg der Zielverbindung (50% d. Th., Reinheit 87%) erhalten.
LC-MS (Methode 2): Rₜ = 1.05 min
MS (ESpos): m/z = 573 (M-TFA+H)⁺

### Beispiel 306A

### ent-Benzyl-{1-[({2,6-dimethyl-8-[(2,3,6-trifluorbenzyl)oxy]imidazo[1,2,a]pyridin-3-yl}carbonyl)amino]-3-fluor-2-methylpropan-2-yl}carbamat Trifluoroacetat (Enantiomer B)

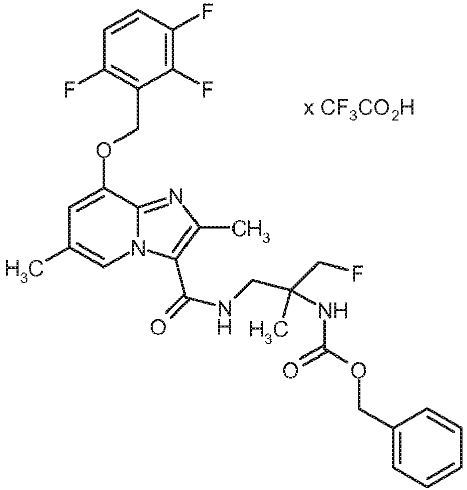

150 mg (0.43 mmol) 2,6-Dimethyl-8-[(2,3,6-trifluorbenzyl)oxy]imidazo[1,2-a]pyridin-3-carbonsäure aus Beispiel 265A, 171 mg (0.45 mmol) HATU und 0.22 ml (1.29 mmol) N,N-Diisopropylethylamin wurden in 2.7 ml DMF 20 min gerührt, dann mit 127 mg (0.45 mmol, 85 %ig) *ent*-Benzyl-(1,amino-3-fluor-2-methylpropan-2-yl)carbamat (Enantiomer B) aus Beispiel 301A versetzt und über Nacht bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingeengt. Es wurden 202 mg der Zielverbindung (69% d. Th., Reinheit 96%) erhalten.
LC-MS (Methode 2): Rₜ =1.06 min
MS (ESpos): m/z = 573 (M-TFA+H)⁺

### Beispiel 307A

### Ethyl-8-[(2,6-difluorbenzyl)oxy]-2-methyl-6-vinylimidazo[1,2-a]pyridin-3-carboxylat

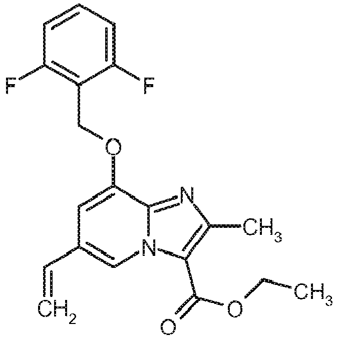

Unter Argon wurden 1.0 g (2.35 mmol) Ethyl-6-brom-8-[(2,6-difluorbenzyl)oxy]-2-methylimidazo[1,2-a]pyridin-3-carboxylat aus Beispiel 18A, 945 mg (7.06 mmol) Kaliumvinyltrifluoroborat, 1.64 ml (11.76 mmol) Triethylamin und 388 mg (0.48 mmol) Dichloro[1,1'-ferrocenylbis(diphenylphosphan)]palladium(II)-Dichlormethan in 50 ml 2-Propanol vorgelegt und 30 min bei 90°C gerührt. Die Reaktionsmischung wurde abgekühlt, mit Essigsäureethylester versetzt, dreimal mit Wasser und einmal mit gesättigter wässriger Natriumchloridlösung gewaschen. Die organische Phase wurde über Natriumsulfat getrocknet, abfiltriert, eingeengt und am Hochvakuum getrocknet. Es wurden 876 mg der Zielverbindung (quantitative Ausbeute) erhalten. Das Produkt wurde ohne weitere Reinigung für die Folgereaktion eingesetzt.
LC-MS (Methode 2): Rₜ = 1.20 min
MS (ESpos): m/z = 373 (M+H)⁺

### Beispiel 308A

### Ethyl-8-[(2,6-difluorbenzyl)oxy]-6-formyl-2-methylimidazo[1,2-a]pyridin-3-carboxylat

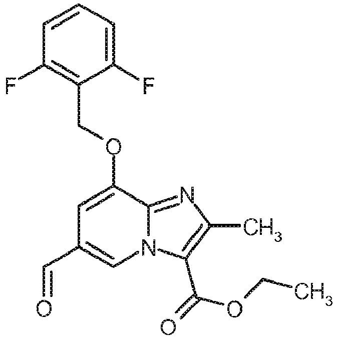

876 mg (2.35 mmol) Ethyl-8-[(2,6-difluorbenzyl)oxy]-2-mefhyl-6-vinylimidazo[1,2-a]pyridin-3-carboxylat aus Beispiel 307A wurden in 20 ml THF/Wasser (1/1) mit 1.45 ml (0.24 mmol, 4%ige Lösung in Wasser) Osmiumtetroxid und 1.52 g (7.13 mmol) Natriumperiodat versetzt und es wurde 1h bei RT gerührt. Die Reaktionsmischung wurde mit Essigsäureethylester versetzt, dreimal mit Wasser und einmal mit gesättigter wässriger Natriumchlorid-Lösung gewaschen. Die organische Phase wurde über Natriumsulfat getrocknet, abfiltriert und eingeengt. Der Rückstand wurde mittels Kieselgel-Chromatographie (Laufmittel: Cyclohexan/Essigsäureethylester-Gradient) gereinigt. Es wurden 478 mg der Zielverbindung (54% d. Th.) erhalten.
LC-MS (Methode 2): Rₜ = 1.12 min
MS (ESpos): m/z = 375 (M+H)⁺
¹H-NMR (400 MHz, DMSO-d₆): δ = 1.39 (t, 3H), 2.60 (s, 3H), 4.41 (q, 2H), 5.40 (s, 2H), 7.20 - 7.28 (m, 2H), 7.42 (s, 1H), 7.55 - 7.65 (m,1H), 9.54 (s, 1H), 10.06 (s, 1H).

### Beispiel 309A

### Ethyl-8-[(2,6-difluorbenzyl)oxy]-6-(difluormethyl)-2-methylimidazo[1,2-a]pyridin-3-carboxylat

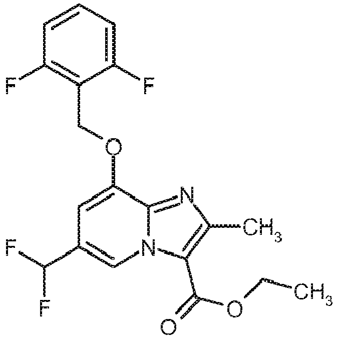

Unter Argon wurden zu 235 mg (0.63 mmol) Ethyl-8-[(2,6-difluorbenzyl)oxy]-6-formyl-2-methylimidazo[1,2-a]pyridin-3-carboxylat aus Beispiel 308A in 4 ml abs. Dichlormethan 0.50 ml (3.77 mmol) Diethylaminoschwefeltrifluorid getropft und es wurde über Nacht bei RT gerührt. Die Reaktionsmischung wurde im Eisbad abgekühlt, vorsichtig mit gesättigter wässriger Natriumhydrogencarbonat-Lösung versetzt und die entstandenen Phasen wurden getrennt. Die wässrige Phase wurde zweimal mit Dichlormethan extrahiert. Die vereinigten organischen Phasen wurden über Natriumsulfat getrocknet, filtriert und eingeengt. Es wurden 249 mg der Zielverbindung (quantitative Ausbeute) erhalten.
LC-MS (Methode 2): Rₜ = 1.19 min
MS (ESpos): m/z = 397 (M+H)⁺
¹H-NMR (400 MHz, DMSO-d₆): δ = 1.37 (t, 3H), 2.58 (s, 3H), 4.38 (q, 2H), 5.39 (s, 2H), 7.11 - 7.40 (m, 4H), 7.55 - 7.66 (m, 1H), 9.17 - 9.21 (m, 1H).

### Beispiel 310A

### 8-[(2,6-Difluorbenzyl)oxy]-6-(difluormethyl)-2-methylimidazo[1,2-a]pyridin-3-arbonsäure

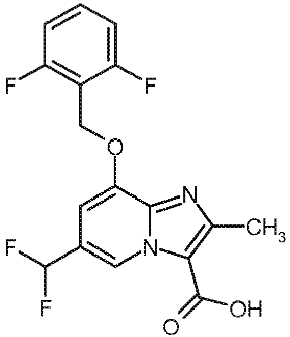

247 mg (0.62 mmol) Ethyl-8-[(2,6-difluorbenzyl)oxy]-6-(difluormethyl)-2-methylimidazo[1,2-a]pyridin-3-carboxylat aus Beispiel 309A wurden in 6 ml THF/Methanol (5/1) mit 45 mg (1.87 mmol) Lithiumhydroxid versetzt und das Reaktionsgemisch wurde anschließend über Nacht bei RT gerührt. Die Reaktionsmischung wurde mit Dichlormethan und Wasser versetzt, die Phasen wurden getrennt, die wässrige Phase mit 1 N Salzsäure angesäuert und im Eisbad abgekühlt. Der Niederschlag wurde abfiltriert, mit Wasser gewaschen und über Nacht im Hochvakuum getrocknet. Es wurden 202 mg der Zielverbindung (88% d. Th.) erhalten.
LC-MS (Methode 2): Rₜ = 0.89 min
MS (ESpos): m/z = 369 (M+H)⁺
¹H-NMR (400 MHz, DMSO-d₆): δ = 2.57 (s, 3H), 5.38 (s, 2H), 7.10 - 7.41 (m, 4H), 7.55 - 7.65 (m, 1H), 9.24 (s, 1H), 13.38 (s, 1H).

### Beispiel 311A

### ent-Benzyl-{1-[({8-[(2,6-difluorbenzyl)oxy]-6-(difluormethyl)-2-methylimidazo[1,2-a]pyridin-3-yl}carbonyl)amino]-2-methylbutan-2-yl}carbamat (Enantiomer A)

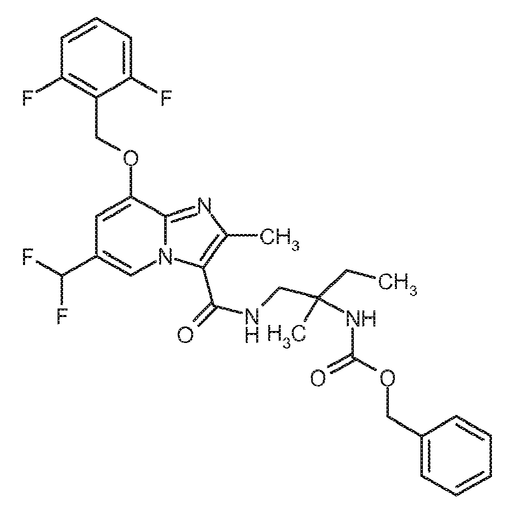

102 mg (0.28 mmol) 8-[(2,6-Difluorbenzyl)oxy]-6-(difluormethyl)-2-methylimidazo[1,2-a]pyridin-3-carbonsäure aus Beispiel 310A wurden in 2 ml DMF mit 126 mg (0.33 mmol) HATU und 0.24 ml (1.38 mmol) N,N-Diisopropylethylamin versetzt und 10 min bei Raumtemperatur gerührt. Anschließend wurden 98 mg (0.41 mmol) *ent*-Benzyl-(1-amino-2-methylbutan-2-yl)carbamat (Enantiomer A) aus Beispiel 274A zur Reaktionslösung gegeben, über Nacht bei RT gerührt und dann mittels präparativer HPLC gereinigt (RP18 Säule, Laufmittel: Acetonitril/Wasser-Gradient unter Zusatz von 0.05% Ameisensäure). Es wurden 124 mg der Zielverbindung (77% d. Th.) erhalten.
LC-MS (Methode 2): Rₜ = 1.23 min
MS (ESpos): m/z = 587 (M+H)⁺
¹H-NMR (400 MHz, DMSO-d₆): δ = 0.82 (t, 3H), 1.19 (s, 3H), 1.49 -1.61 (m, 1H), 1.77 -1.87 (m, 1H), 3.49 - 3.60 (m, 2H), 5.00 (s, 2H), 5.37 (s, 2H), 7.01 - 7.10 (m, 1H), 7.13 - 7.38 (m, 9H), 7.55 - 7.65 (m, 1H), 7.89 (t, 1H), 8.98 (s, 1H).

### Beispiel 312A

### ent-Benzyl-{1-[({8-[(2,6-difluorbenzyl)oxy]-6-(difluormethyl)-2-methylimidazo[1,2-a]pyridin-3-yl}carbonyl)amino]-2-methylpentan-2-yl}carbamat (Enantiomer B)

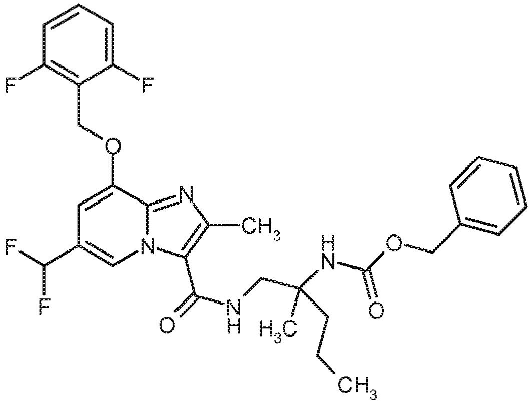

102 mg (0.28 mmol) 8-[(2,6-Difluorbenzyl)oxy]-6-(difluormethyl)-2-methylimidazo[1,2-a]pyridin-3-carbonsäure aus Beispiel 310A wurden in 2 ml DMF mit 126 mg (0.33 mmol) HATU und 0.24 ml (1.38 mmol) N,N-Diisopropylethylamin versetzt und 10 min bei Raumtemperatur gerührt. Anschließend wurden 103 mg (0.41 mmol) *ent*-Benzyl-(1-amino-2-methylpentan-2-yl)carbamat (Enantiomer B) aus Beispiel 289A zur Reaktionslösung gegeben, über Nacht bei RT gerührt und dann mittels präparativer HPLC gereinigt (RP18 Säule, Laufmittel: Acetonitril/Wasser-Gradient unter Zusatz von 0.05% Ameisensäure). Es wurden 123 mg der Zielverbindung (74% d. Th.) erhalten.
LC-MS (Methode 2): Rₜ = 1.31 min
MS (ESpos): m/z = 601 (M+H)⁺
¹H-NMR (400 MHz, DMSO-d₆): δ = 0.85 (t, 3H), 1.20 (s, 3H), 1.23 -1.34 (m, 2H), 1.44 -1.57 (m, 1H), 1.68 -1.82 (m, 1H), 3.49 - 3.58 (m, 2H), 4.99 (s, 2H), 5.37 (s, 2H), 7.01 - 7.38 (m, 10H), 7.55 - 7.65 (m, 1H), 7.90 (t, 1H), 8.98 (s, 1H).

### Beispiel 313A

### ent-Benzyl-{1-[({8-[(2,6-difluorbenzyl)oxy]-6-(difluormethyl)-2-methylimidazo[1,2-a]pyridin-3-yl}carbonyl)amino]-3-fluor-2-methylpropan-2-yl}carbamat Trifluoroacetat (Enantiomer A)

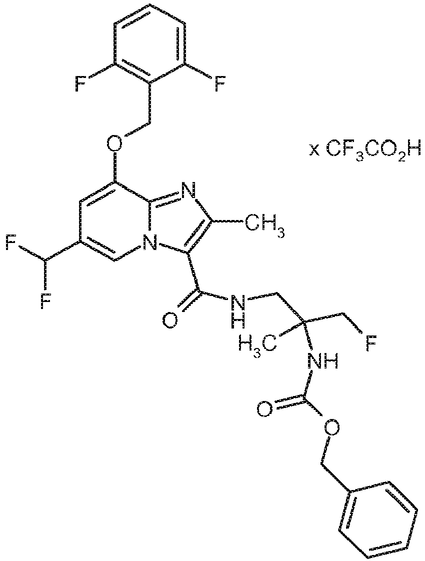

50 mg (0.14 mmol) 8-[(2,6-Difluorbenzyl)oxy]-6-(difluormethyl)-2-methylimidazo[1,2-a]pyridin-3-carbonsäure aus Beispiel 310A, 54 mg (0.14 mmol) HATU und 0.07 ml (0.41 mmol) N,N-Diisopropylethylamin wurden in 0.9 ml DMF 20 min gerührt, dann mit 40 mg (0.14 mmol, 85%) ent-Benzyl-(1-amino-3-fluor-2-methylpropan-2-yl)carbamat (Enantiomer A) aus Beispiel 300A versetzt und über Nacht bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1 % TFA). Es wurden 75 mg der Zielverbindung (78% d. Th.) erhalten.
LC-MS (Methode 2): Rₜ = 1.19 min
MS (ESpos): m/z = 591 (M-TFA+H)⁺

### Beispiel 314A

### ent-Benzyl-{1-[({8-[(2,6-difluorbenzyl)oxy]-6-(difluormethyl)-2-methylimidazo[1,2-a]pyridin-3-yl}carbonyl)amino]-3-fluor-2-methylpropan-2-yl}carbamat Trifluoroacetat (Enantiomer B)

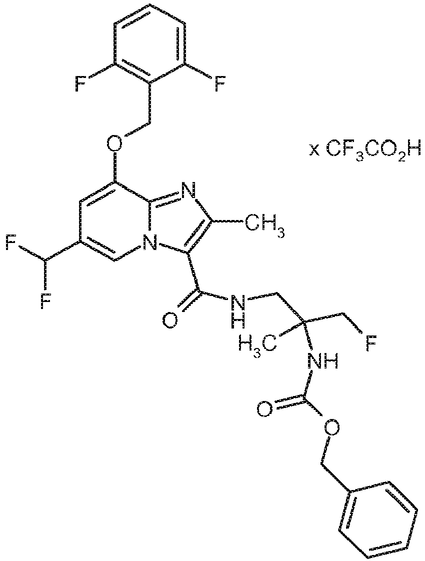

50 mg (0.14 mmol) 8-[(2,6-Difluorbenzyl)oxy]-6-(difluormethyl)-2-methylimidazo[1,2-a]pyridin-3-carbonsäure aus Beispiel 310A, 54 mg (0.14 mmol) HATU und 0.07 ml (0.41 mmol) N,N-Diisopropylethylamin wurden in 0.9 ml DMF 20 min gerührt, dann mit 40 mg (0.14 mmol, 85%ig) *ent*-Benzyl-(1-amino-3-fluor-2-methylpropan-2-yl)carbamat (Enantiomer B) aus Beispiel 301A versetzt und über Nacht bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Es wurden 47 mg der Zielverbindung (44% d. Th., Reinheit ca. 89%) erhalten.
LC-MS (Methode 2): Rₜ = 1.20 min
MS (ESpos): m/z = 591 (M-TFA+H)⁺

### Beispiel 315A

### Methyl-N²-(tert-butoxycarbonyl)-N⁶-({8-[(2,6-difluorbenzyl)oxy]-2,6-dimethylimidazo[1,2-a]pyridin-3-yl}carbonyl)-L-lysinat Trifluoracetat

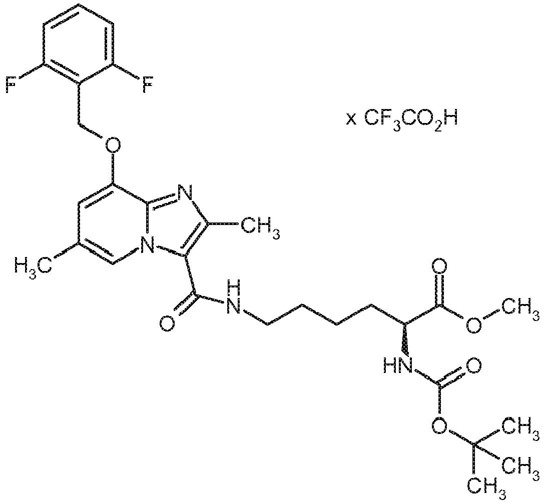

300 mg (0.90 mmol) 8-[(2,6-Difluorbenzyl)oxy]-2,6-dimethylimidazo[1,2-a]pyridin-3-carbonsäure aus Beispiel 21A, 377 mg (1.17 mmol) (Benzotriazol-1-yloxy)bisdimethylamino-methyliumfluoroborat und 0.5 ml (4.51 mmol) 4-Methylmorpholin wurden in 5.75 ml DMF vorgelegt, 10 min bei Raumtemperatur gerührt, dann mit 318 mg (0.99 mmol) Methyl-N²-(tert-butoxycarbonyl)-L-lysinatacetat versetzt und über Nacht bei RT gerührt. Es wurden nochmals 188 mg (0.59 mmol) (Benzotriazol-1-yloxy)bisdimethylaminomethyliumfluoroborat, 0.4 ml (3.61 mmol) 4-Methylmorpholin und 376 mg (1.17 mmol) Methyl-N²-(tert-butoxycarbonyl)-L-lysinatacetat zur Reaktionslösung gegeben und über Nacht bei Raumtemperatur gerührt. Es wurde mit Wasser/TFA versetzt und mittels präparativer HPLC gereinigt (RP18 Säule, Laufmittel: Acetonitril/Wasser-Gradient unter Zusatz von 0.1% TFA). Es wurden 205 mg der Zielverbindung (33% d. Th.) erhalten.
LC-MS (Methode 2): Rₜ = 0.95 min
MS (ESpos): m/z = 575 (M-TFA+H)⁺
¹H-NMR (400 MHz, DMSO-d₆): δ = 1.30 -1.43 (m, 13H), 1.48 - 1.72 (m, 2H), 2.41 (s, 3H), 2.54 (s, 3 H; verdeckt durch DMSO-Signal), 3.30 (q, 2H), 3.61 (s, 3H), 3.91 - 3.98 (m, 1H), 5.40 (s, 2H), 7.01 (s, 1H), 7.13 (s, 1H), 7.22 - 7.30 (m, 2H), 7.57 - 7.66 (m,1H), 8.50 - 8.55 (m, 1H).

### Beispiel 316A

### rac-2-Amino-3-(4-fluorphenyl)-2-methylpropanonitril

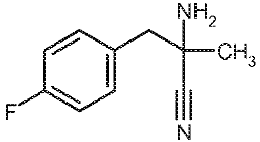

1.0 g (6.57 mmol) 4-Fluorphenylaceton wurden in 13.1 ml 2 N Ammoniak in Methanol vorgelegt, mit 361 mg (7.36 mmol) Natriumcyanid und 877 mg (7.36 mmol) Ammoniumchlorid versetzt und 2 Stunden unter Rückfluss gerührt. Die Reaktionslösung wurde abgekühlt, mit 20 ml Dichlormethan verdünnt und der ausgefallene Feststoff abfiltriert. Das Filtrat wurde eingeengt und der Rückstand mittels Kieselgel-Chromatographie gereinigt (RP18 Säule, Laufmittel: Cyclohexan/Essigsäureethylester 4/1 nach 1/1). Es wurden 340 mg der Zielverbindung (23% d. Th., Reinheit 81 %) erhalten.
LC-MS (Methode 15): Rₜ = 1.87 min
MS (ESpos): m/z = 179 (M+H)⁺
¹H-NMR (400 MHz, DMSO-d₆): δ = 1.30 (s, 3H), 2.87 (q, 2H), 7.13 - 7.20 (m, 2H), 7.28 - 7.36 (m, 2H).

### Beispiel 317A

### rac-3-(4-Fluorphenyl)-2-methylpropan-1,2-diamin

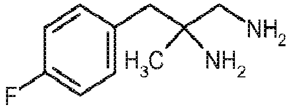

340 mg (1.54 mmol, Reinheit ca. 81%) *rac*-2-Amino-3-(4-fluorphenyl)-2-methylpropanonitril aus Beispiel 316A wurden in 2 ml Ethanol gelöst und mit 4.6 ml (4.64 mmol) 1 N Salzsäure in Ethanol versetzt. 5.2 mg (0.02 mmol) Platin(IV)oxid wurden zugegeben und die Reaktionsmischung 5 Stunden bei 3 bar hydriert. Es wurden nochmals 5 mg (0.02 mmol) Platin(IV)oxid zur Reaktionslösung gegeben und für 5 h bei 3 bar hydriert. Dann wurde über Celite abfiltriert, das Filtrat mit 1.5 ml (3.09 mmol) 2 N Salzsäure in Diethylether versetzt, eingeengt und im Hochvakuum getrocknet. Das Produkt wurde mittels Kieselgel-Chromatographie gereinigt (Laufmittel: Dichlormethan/2N Ammoniak in Methanol 60/1 nach 20/1). Es wurden 145 mg der Zielverbindung (51% d. Th.) erhalten.
LC-MS (Methode 13): Rₜ = 0.20 min
MS (ESpos): m/z = 183 (M+H)⁺
¹H-NMR (400 MHz, DMSO-d₆): δ = 0.80 (s, 3H), 1.34 (br. s., 4H), 2.24 - 2.34 (m, 2H), 2.53 (d, 2H), 7.03 - 7.12 (m, 2H), 7.18 - 7.27 (m, 2H).

### Beispiel 318A

### rac-2-Amino-2-methyl-3-pyridin-2-yl)ropanonitril

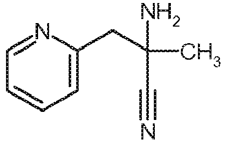

4.88 ml (14.80 mmol) 1-(Pyridin-2-yl)aceton wurden in 29.6 ml 2 N Ammoniak in Methanol vorgelegt, mit 812 mg (16.57 mmol) Natriumcyanid und 1.97 g (16.57 mmol) Ammoniumchlorid versetzt und 2 Stunden unter Rückfluss gerührt. Das Reaktionsgemisch wurde abgekühlt, mit Dichlormethan verdünnt und vom enthaltenen Feststoff abfiltriert. Das Filtrat wurde eingeengt und der Rückstand wurde mittels Kieselgel-Chromatographie gereinigt (Laufmittel: Cyclohexan/Essigsäureethylester = 1/1). Die erhaltenen verunreinigten Produktfraktionen wurden erneut mittels Kieselgel-Chromatographie gereinigt (Laufmittel: Cyclohexan/Essigsäureethylester 1/1). Es wurden insgesamt 1.1 g der Zielverbindung (45% d. Th.) erhalten.
LC-MS (Methode 15): Rₜ = 1.30 min
MS (ESpos): m/z = 162 (M+H)⁺

### Beispiel 319A

### rac-2-Methyl-3-(pyridin-2-yl)propan-1,2-diamin Trihydrochlorid

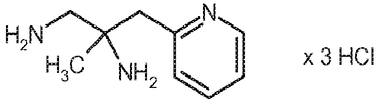

666 mg (4.01 mmol) 2-Amino-2-methyl-3-(pyridin-2-yl)propanonitril aus Beispiel 318A wurden in 41 ml THF vorgelegt und unter Argon bei 0°C langsam mit 2.61 ml (2.61 mmol) 1 N Lithiumaluminiumhydridlösung in THF versetzt. Die Reaktionsmischung wurde 30 min bei 0°C und über Nacht bei RT gerührt. Dann wurde nochmal mit 0.7 ml (0.70 mmol) 1 N Lithiumaluminiumhydridlösung in THF versetzt und 2 Stunden bei RT gerührt. Das Reaktionsgemisch wurde auf 0°C abgekühlt und mit 0.4 ml Wasser, 0.4 ml 2 N wässriger Natriumhydroxidlösung und 0.8 ml Wasser versetzt. Der Niederschlag wurde abfiltriert und mit Dichlormethan/Methanol (10/1) gewaschen. Das Filtrat wurde mit 2 N Chlorwasserstoff in Diethylether versetzt und im Hochvakuum getrocknet. Es wurden 1.15 g der Zielverbindung (104% d. Th.) erhalten.
LC-MS (Methode 15): Rₜ = 1.26 min
MS (ESpos): m/z = 166 (M-3HCl+H)⁺

### Beispiel 320A

### rac-tert-Butyl-4-[({8-[(2,6-difluorbenzyl)oxy]-2,6-dimethylimidazo[1,2-a]pyridin-3-yl}carbonyl)amino]-3,3-difluorpiperidin-1-carboxylat

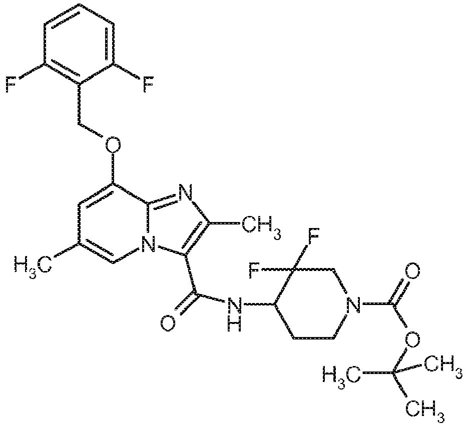

75 mg (0.23 mmol) 8-[(2,6-Difluorbenzyl)oxy]-2,6-dimethylimidazo[1,2-a]pyridin-3-carbonsäure aus Beispiel 21A, 94 mg (0.25 mmol) HATU und 0.2 ml (1.13 mmol) N,N-Diisopropylethylamin in 1.4 ml DMF wurden 20 min gerührt, anschließend mit 64 mg (0.27 mmol) *tert*-Butyl-4-amino-3,3-difluorpiperidin-1-carboxylat versetzt und dann wurde über Nacht bei Raumtemperatur gerührt. Die Reaktionslösung wurde mit Wasser versetzt, der ausgefallene Feststoff wurde 30 min bei Raumtemperatur gerührt, abfiltriert, mit Wasser gewaschen und im Hochvakuum getrocknet. Es wurden 81 mg der Zielverbindung (65% d. Th.) erhalten.
LC-MS (Methode 2): Rₜ = 1.06 min
MS (ESpos): m/z = 551 (M+H)⁺

### Beispiel 321A

### 5-Methyl-2-nitropyridin-3-ol

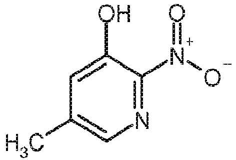

25 g (0.23 mol) 5-Methylpyridin-3-ol wurden unter Eiskühlung in 226 ml (4.12 mol) konzentrierter Schwefelsäure vorgelegt und anschließend auf RT erwärmt. Nachdem das Edukt komplett gelöst war, wurde das Reaktionsgemisch wieder auf 0°C abgekühlt. Anschließend wurden bei 0°C bis 10°C langsam 14.25 ml (0.34 mol) rauchende Salpetersäure zugetropft, innerhalb von 3.5 Stunden auf 15°C erwärmt und dann über Nacht bei RT gerührt. Die Reaktionslösung wurde auf 1000 g Eis geschüttet und zweimal mit je 500 ml Essigsäureethylester extrahiert. Die vereinigten organischen Phasen wurden getrocknet und eingeengt. Es wurden 31.5 g der Zielverbindung (89% d. Th.) erhalten.
LC-MS (Methode 13): Rₜ = 1.21 min
MS (ESpos): m/z = 155 (M+H)⁺

### Beispiel 322A

### 3-[(2,6-Difluorbenzyl)oxy]-5-mefhyl-2-nitropyridin

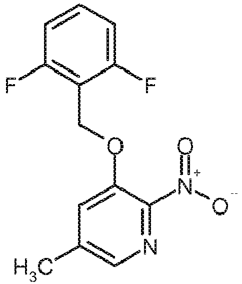

31.5 g (0.155 mol) 5-Methyl-2-nitropyridin-3-ol aus Beispiel 321A und 75.78 g (0.23 mol) Cäsiumcarbonat wurden in 432 ml DMF vorgelegt, mit 33.7 g (0.163 mol) 2,6-Difluorbenzylbromid versetzt und über Nacht bei RT gerührt. Die Reaktionslösung wurde in 3600 ml 0.5 N wässrige Salzsäure eingerührt. Der entstandene Niederschlag wurde noch 30 min gerührt, abgesaugt, mit Wasser gewaschen und bei RT und Normaldruck an der Luft getrocknet. Es wurden 45.8 g der Zielverbindung (105% d. Th.) erhalten.
LC-MS (Methode 2): Rₜ = 0.98 min
MS (ESpos): m/z = 281 (M+H)⁺
¹H-NMR (400 MHz, DMSO-d₆): δ = 2.44 (s, 3H), 5.37 (s, 2H), 7.21 (quint., 2H), 7.52 - 7.61 (m, 1H), 8.01 (s, 1H), 8.06 (s, 1H).

### Beispiel 323A

### 3-[(2,6-Difluorbenzyl)oxy]-5-methylpyridin-2-amin

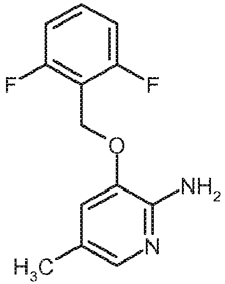

Unter Argon wurde 91 g (324.7 mmol) 3-[(2,6-Difluorbenzyl)oxy]-5-methyl-2-nitropyridin aus Beispiel 322A in 980 ml Ethanol vorgelegt, mit 56.2 g (1.0 mol) Eisenpulver versetzt und zum Rückfluss erhitzt. Es wurden langsam 248 ml konzentrierte, wässrige Salzsäure zugetropft und 30 min unter Rückfluss weiter gerührt. Nach dem Abkühlen wurde ca. 2000 ml Wasser/Eis (1/1) zum Reaktionsgemisch gegeben und 30 min bei RT gerührt. Die Lösung wurde soweit eingeengt, dass das Lösungsmittel zum größten Teil entfernt wurde. Die wässrige Phase wurde mit konzentrierter wässriger Natriumhydroxid-Lösung alkalisch gestellt und mit 1200 ml Dichlormethan versetzt und 1 h kräftig gerührt. Die Mischung wurde über Kieselgur abgesaugt und mehrfach gut mit insgesamt ca. 2800 ml Dichlormethan gewaschen. Die Mutterlauge wurde getrennt, die organische Phase getrocknet und eingeengt. Es wurden 77.8 g der Zielverbindung (96% d. Th.) erhalten.
LC-MS (Methode 2): Rₜ = 0.57 min
MS (ESpos): m/z = 251 (M+H)⁺
¹H-NMR (400 MHz, DMSO-d₆): δ = 2.13 (s, 3H), 5.08 (s, 2H), 5.25 (s, 2H), 7.09 (d, 1H), 7.14 - 7.22 (m, 2H), 7.37 - 7.41 (m, 1H), 7.49 - 7.57 (m, 1H).

### Beispiel 324A

### Ethyl-8-[(2,6-difluorbenzyl)oxy]-2-ethyl-6-methylimidazo[1,2-a]pyridin-3-carboxylat

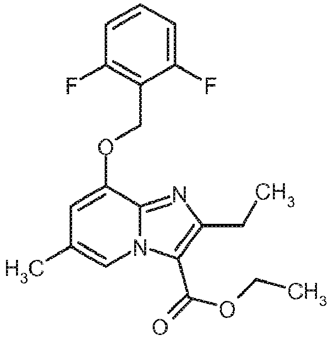

Unter Argon wurden 3.5 g (13.99 mmol) 3-[(2,6-Difluorbenzyl)oxy]-5-methylpyridin-2-amin aus Beispiel 323A und 9.6 ml (69.93 mmol) 2-Chlor-2-propionyl-essigsäure-methylester in 140 ml Ethanol gelöst und über Nacht mit 500 mg 3 Å Molsieb unter Rückfluss gerührt. 500 mg 3 Å Molsieb wurden zugegeben und das Gemisch wurde weitere 16 Stunden unter Rückfluss gerührt. Das Reaktionsgemisch wurde 8 Tage unter Rückfluss gerührt, wobei an jedem Tag 3 Å Molsieb zugegeben wurde. Das Gemisch wurde abgekühlt, abgesaugt und die Mutterlauge weitgehend eingeengt. Der erhaltene Rückstand wurde mittels Kieselgel-Chromatographie (Laufmittel: Cyclohexan/Essigsäureethylester 9/1 nach 7/3) gereinigt. Es wurden 3.8 g der Zielverbindung (68% d. Th., als 1:1 Gemisch mit Methyl -8-[(2,6-difluorbenzyl)oxy]-2-ethyl-6-methylimidazo[1,2-a]pyridin-3-carboxylat) erhalten.
LC-MS (Methode 2): Rₜ = 1.18 min
MS (ESpos): m/z = 361 (M+H)⁺

### Beispiel 325A

### 8-[(2,6-Difluorbenzyl)oxy]-2-ethyl-6-methylimidazo[1,2-a]pyridin-3-carbonsäure

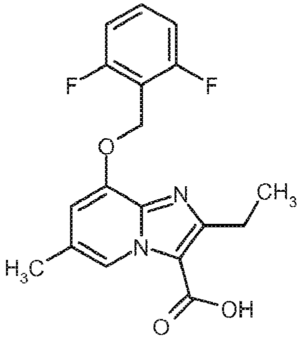

2.0 g (5.34 mmol) Ethyl-8-[(2,6-difluorbenzyl)oxy]-2-ethyl-6-methylimidazo[1,2-a]pyridin-3-carboxylat aus Beispiel 324A (1:1 Gemisch von Methyl- und Ethylester) wurden in 114 ml THF/Methanol (5/1) gelöst, mit 5.34 ml (5.34 mmol) 1 N wässriger Lithiumhydroxid-Lösung versetzt und über Nacht bei RT gerührt. Das Reaktionsgemisch wurde 4 Tage bei 40°C gerührt wobei nach 3 Tagen nochmals 5.34 ml (5.34 mmol) 1 N wässrige Lithiumhydroxid-Lösung zugegeben wurde. Nach dem Abkühlen wurde das Gemisch unter Eiskühlung mit 6 N wässriger Salzsäure auf pH 4 angesäuert und anschließend am Rotationsverdampfer vom organischen Lösungsmittel befreit. Der ausgefallene Feststoff wurde abgesaugt, mit Wasser nachgewaschen und anschließend im Hochvakuum getrocknet. Es wurden 1.94 g der Zielverbindung (99% d. Th.) erhalten.
LC-MS (Methode 2): Rₜ = 0.79 min
MS (ESpos): m/z = 347 (M+H)⁺
¹H-NMR (400 MHz, DMSO-d₆): δ = 1.19 (t, 3H), 2.36 (s, 3H), 2.95 (q, 2H), 5.31 (s, 2H), 7.08 (s, 1H), 7.26 (quin, 2H), 7.55 - 7.65 (m, 1H), 8.78 (s, 1H), 13.02 - 13.06 (m, 1H).

### Beispiel 326A

### rac-2-Methylpentan-1,2-diamin Dihydrochlorid

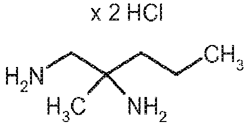

1.0 g (8.91 mmol) *rac*-2-Amino-2-methylpentanonitril wurden unter Eiskühlung in 26.7 ml (26.74 mmol) 1 M Salzsäure in Ethanol vorgelegt und zusammen mit 30 mg (0.13 mmol) Platin(IV)oxid bei Normaldruck über Nacht hydriert. Es wurden nochmals 30 mg (0.13 mmol) Platin(IV)oxid zugegeben und das Reaktionsgemisch 6 Stunden bei 3 bar hydriert. Das Reaktionsgemisch wurde über Celite abfiltriert, das Filtrat mit 9 ml (17.83 mmol) 2 N Salzsäure in Diethylether versetzt, eingeengt und im Hochvakuum getrocknet. Es wurden 1.56 g der Zielverbindung (92% d. Th.) erhalten.
MS (ESpos): m/z = 117 (M+H)⁺
¹H-NMR (400 MHz, DMSO-d₆): δ = 0.89 (t, 3H), 1.26 - 1.41 (m, 5H), 1.58 - 1.68 (m, 2H), 3.03 - 3.16 (m, 2H), 8.56 - 8.74 (m, 4H).

### Beispiel 327A

### rac-2-(Trifluormethyl)piperidin-4-amin Hydrochlorid

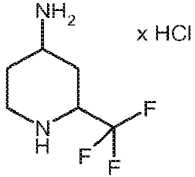

115 mg (0.43 mmol) *rac*-*tert*-Butyl-[2-(trifluormethyl)piperidin-4-yl]carbamat wurde in 2.2 ml Diethylether vorgelegt, mit 2.14 ml (4.28 mmol) 2 N Salzsäure in Diethylether versetzt und über Nacht bei RT gerührt. Das Reaktionsgemisch wurde eingeengt und der Rückstand im Hochvakuum getrocknet. Es wurden 89 mg der Zielverbindung (101% d. Th.) erhalten.

### Beispiel 328A

### 8-(Benzyloxy)-2,6-dimethylimidazo[1,2-a]pyridin-3-carbonsäure

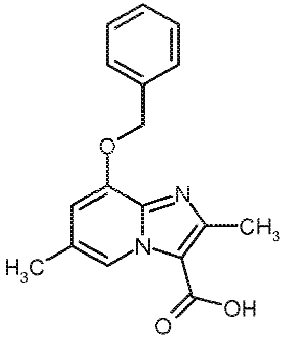

8.0 g (24.66 mmol) Ethyl-8-(benzyloxy)-2,6-dimethylimidazo[1,2-a]pyridin-3-carboxylat aus Beispiel 238A wurden in 526 ml THF/Methanol (5/1) gelöst, mit 123 ml (123.31 mmol) 1 N wässriger Lithiumhydroxidlösung versetzt und bei 40°C für 6 Stunden gerührt. Die Reaktionsmischung wurde unter Eiskühlung mit 6 N wässriger Salzsäure auf pH 5 angesäuert und anschließend wurde im Vakuum vom organischen Lösemittel befreit. Der ausgefallene Feststoff wurde abgesaugt, mit Wasser nachgewaschen und anschließend im Hochvakuum getrocknet. Es wurden 7.47 g der Zielverbindung (96% d. Th., Reinheit 94%) erhalten.
LC-MS (Methode 2): Rₜ = 0.67 min
MS (ESpos): m/z = 297 (M+H)⁺

### Beispiel 329A

### rac-tert-Butyl-[1-({[8-benzyloxy)-2,6-dimethylimidazo[1,2-a]pyridin-3-yl]carbonyl}amino)-2-methylpentan-2-yl]carbamat

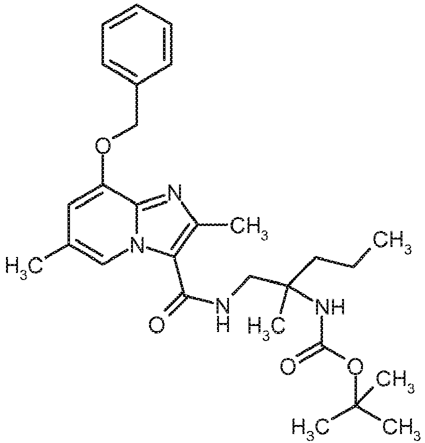

7.0 g (22.20 mmol, Reinheit 94%) 8-(Benzyloxy)-2,6-dimethylimidazo[1,2-a]pyridin-3-carbonsäure aus Beispiel 328A, 10.13 g (26.65 mmol) HATU und 11.6 ml (66.61 mmol) *N,N-*Diisopropylethylamin wurden in 141 ml DMF vorgelegt, 20 min bei Raumtemperatur gerührt und mit 17.4 g (44.41 mmol, Reinheit 74%) *rac*-*tert*-Butyl-(1-amino-2-methylpentan-2-yl)carbamat aus Beispiel 390A versetzt. Es wurde 1 Stunde bei RT gerührt, dann mit ca. 1.21 Wasser versetzt und 1 Stunde bei RT gerührt. Der entstandene Feststoff wurde abfiltriert und im Hochvakuum getrocknet. Es wurden 9.98 g der Zielverbindung (88% d. Th.) erhalten.
LC-MS (Methode 2): Rₜ = 1.09 min
MS (ESpos): m/z = 495 (M+H)⁺
¹H-NMR (400 MHz, DMSO-d₆): δ = 0.86 (t, 3H), 1.18 (s, 3H), 1.21 - 1.33 (m, 2H), 1.38 (s, 9H), 1.46 - 1.59 (m, 1H), 1.65 - 1.76 (m, 1H), 2.28 (s, 3H), 2.55 (s, 3H), 3.41 - 3.54 (m, 2H), 5.27 (s, 2H), 6.50 - 6.60 (m, 1H), 6.80 - 6.85 (m, 1H), 7.34 - 7.40 (m, 1H), 7.40 - 7.46 (m, 2H), 7.48 - 7.54 (m, 2H), 7.67 (t, 1H), 8.44 (s, 1 H).

### Beispiel 330A

### rac-tert-Butyl-(1-{[(8-hydroxy-2,6-dimethylimidazo[1,2-a]pyridin-3-yl)carbonyl]amino}-2-methylpentan-2-yl)carbamat

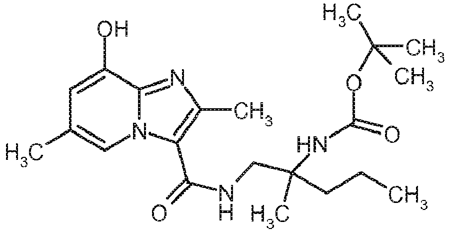

9.06 g (18.31 mmol) *rac*-*tert*-Butyl-[1-({[8-(benzyloxy)-2,6-dimethylimidazo[1,2-a]pyridin-3-yl]carbonyl}amino)-2-methylpentan-2-yl]carbamat aus Beispiel 329A wurden unter Argon in 189 ml Ethanol vorgelegt, mit 1.95 g (1.83 mmol) 10%-igem Palladium auf Kohle versetzt und 90 min unter Normaldruck bei RT hydriert. Das Reaktionsgemisch wurde über Celite filtriert, mit Ethanol gewaschen und eingeengt. Es wurden 7.2 g der Zielverbindung (92% d. Th.) erhalten.
LC-MS (Methode 2): Rₜ = 0.86 min
MS (ESpos): m/z = 405 (M+H)⁺
¹H-NMR (400 MHz, DMSO-d₆): δ = 0.86 (t, 3H), 1.18 (s, 3H), 1.21 - 1.33 (m, 2H), 1.35 - 1.41 (s, 9H), 1.48 - 1.59 (m, 1H), 1.65 - 1.77 (m, 1H), 2.22 (s, 3H), 2.56 (s, 3H), 3.41 - 3.53 (m, 2H), 6.49 (d, 1H), 6.53 - 6.59 (m, 1H), 7.63 (t, 1H), 8.34 (s, 1H).

### Beispiel 331A

### Ethyl-8-[(2,6-difluorbenzyl)oxy]-6-methyl-2-(trifluormethyl)imidazo[1,2-a]pyridin-3-carboxylat

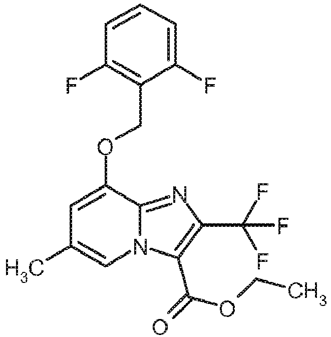

Unter Argon wurden 1.1 g (4.40 mmol) 3-[(2,6-Difluorbenzyl)oxy]-5-methylpyridin-2-amin aus Beispiel 323A und 4.8 g (21.98 mmol) Ethyl-2-chlor-4,4,4-trifluor-3-oxobutanoat in 44 ml Ethanol gelöst und über Nacht mit ca. 200 mg 3 Å Molsieb unter Rückfluss gerührt. Es wurden ca. 200 mg 3 Å Molsieb zugegeben und das Gemisch wurde weitere 16 Stunden unter Rückfluss gerührt. Dann wurde 8 Tage unter Rückfluss gerührt, wobei an jedem Tag 3 Å Molsieb zugegeben wurde. Das Gemisch wurde abgekühlt, abgesaugt, die Mutterlauge nahezu vollständig eingedampft und der erhaltene Rückstand mittels Kieselgel-Chromatographie gereinigt (Laufmittel: Cyclohexan/Essigester 9/1 nach 7/3). Es wurden 600 mg der Zielverbindung (33% d. Th.) erhalten.
LC-MS (Methode 2): Rₜ = 1.33 min
MS (ESpos): m/z = 415 (M+H)⁺

### Beispiel 332A

### 8-[(2,6-Difluorbenzyl)oxy]-6-methyl-2-(trifluormethyl)imidazo[1,2-a]pyridin-3-carbonsäure

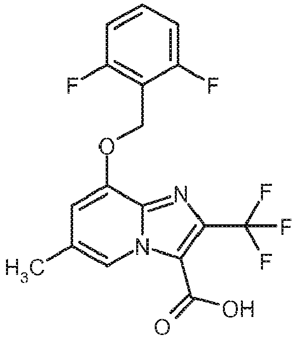

491 mg (1.19 mmol) Ethyl-8-[(2,6-difluorbenzyl)oxy]-6-methyl-2-(trifluormethyl)imidazo[1,2-a]pyridin-3-carboxylat aus Beispiel 331A wurden in 26 ml THF/Methanol (5/1) gelöst, mit 6 ml 1 N wässriger Lithiumhydroxidlösung versetzt und 2 Stunden bei RT gerührt. Die Reaktionslösung wurde mit 1 N wässriger Salzsäure auf pH 6 eingestellt und das organische Lösungsmittel wurde abdestilliert. Der entstandene Niederschlag wurde abfiltriert, mit Wasser gewaschen und im Hochvakuum getrocknet. Es wurden 336 mg der Zielverbindung (73% d. Th.) erhalten.
LC-MS (Methode 2): Rₜ = 1.04 min
MS (ESpos): m/z = 387 (M+H)⁺
¹H-NMR (400 MHz, DMSO-d₆): δ = 2.42 (s, 3H), 5.35 (s, 2H), 7.20 - 7.30 (m, 3H), 7.56 - 7.66 (m, 1H), 8.84 (s, 1H).

### Beispiel 333A

### ent-Benzyl-{1-[({8-[(2,6-difluorbenzyl)oxy]-6-methyl-2-(trifluormethyl)imidazo[1,2-a]pyridin-3-yl}carbonyl)amino]-2-methylpentan-2yl}carbamat Trifluoracetat

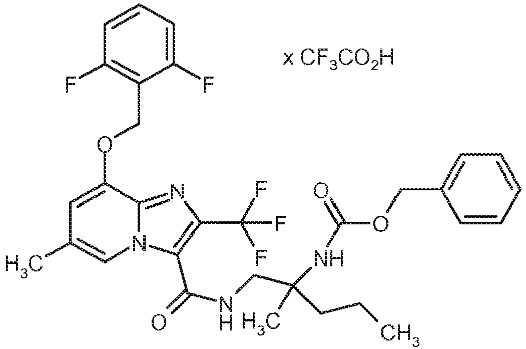

50 mg (0.13 mmol) 8-[(2,6-Difluorbenzyl)oxy]-6-methyl-2-(trifluormethyl)midazo[1,2-a]pyridin-3-carbonsäure aus Beispiel 332A, 45.7 mg (0.14 mmol) (Benzotriazol-1-yloxy)bisdimethylaminomethyliumfluoroborat und 0.07 ml (0.65 mmol) 4-Methylmorpholin wurden in 0.43 ml DMF vorgelegt, mit 35.6 mg (0.14 mmol) *ent*-Benzyl-(1-amino-2-methylpentan-2-yl)carbamat aus Beispiel 289A versetzt und 1 Stunde bei RT gerührt. Die Reaktionslösung wurde mit etwas Wasser/TFA versetzt und mittels präparativer HPLC gereinigt (RP18 Säule, Laufmittel: Acetonitril/Wasser-Gradient unter Zusatz von 0.1% TFA). Es wurden 46 mg der Zielverbindung (48% d. Th.) erhalten.
LC-MS (Methode 2): Rₜ = 1.40 min
MS (ESpos): m/z = 619 (M-TFA+H)⁺
¹H-NMR (400 MHz, DMSO-d₆): δ = 0.85 (t, 3H), 1.20 (s, 3H), 1.23 -1.33 (m, 2H), 1.44 -1.55 (m, 1H), 1.68 -1.78 (m, 1H), 2.32 (s, 3H), 3.50 - 3.64 (m, 2H), 4.94 - 5.03 (m, 2H), 5.33 (s, 2H), 6.93 - 7.00 (m, 1H), 7.06 (s, 1H), 7.20 - 7.36 (m, 7H), 7.56 - 7.65 (m, 1H), 7.93 (s, 1H), 8.60 - 8.67 (m, 1H).

### Beispiel 334A

### tert-Butyl-(1-{[({8-[(2,6-difluorbenzyl)oxy]-2,6-dimethylimidazo[1,2-a]pyridin-3-yl}carbonyl)amino]methyl}cyclohexyl)carbamat Trifluoracetat

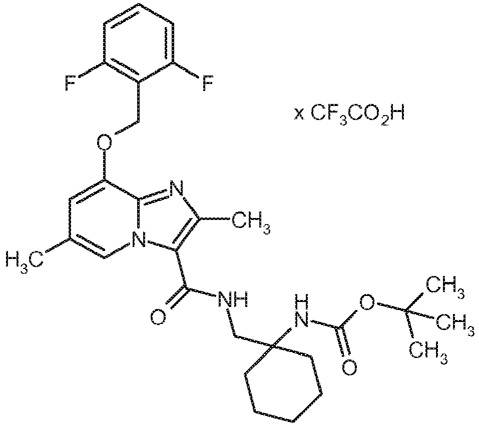

100 mg (0.30 mmol) 8-[(2,6-Difluorbenzyl)oxy]-2,6-dimethylimidazo[1,2-a]pyridin-3-carbonsäure aus Beispiel 21A, 145 mg (0.45 mmol) (Benzotriazol-1-yloxy)bisdimethylamino-methyliumfluoroborat und 0.17 ml (1.51 mmol) 4-Methylmorpholin wurden in 2 ml DMF vorgelegt, mit 82.5 mg (0.36 mmol) *tert*-Butyl-[1-(aminomethyl)cyclohexyl]carbamat versetzt und über Nacht bei RT gerührt. Die Reaktionslösung wurde mit etwas Wasser/TFA versetzt und mittels präparativer HPLC gereinigt (RP18 Säule, Laufmittel: Acetonitril/Wasser-Gradient unter Zusatz von 0.1% TFA). Es wurden 163 mg der Zielverbindung (82% d. Th..) erhalten.
LC-MS (Methode 2): Rₜ = 1.15 min
MS (ESpos): m/z = 543 (M-TFA+H)⁺

### Beispiel 335A

### tert-Butyl-{3-[({8-[(2,6-difluorbenzyl)oxy]-2,6-dimethylimidazo[1,2-a]pyridin-3-yl}carbonyl)amino]cyclopentyl}carbamat (Stereoisomerengemisch)

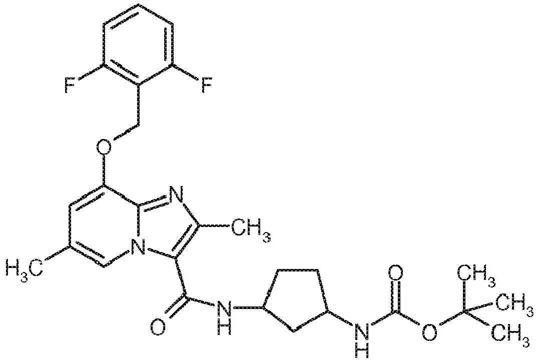

100 mg (0.30 mmol) 8-[(2,6-Difluorbenzyl)oxy]-2,6-dimethylimidazo[1,2-a]pyridin-3-carbonsäure aus Beispiel 21A, 106 mg (0.33 mmol) (Benzotriazol-1-yloxy)bisdimethylaminomethyliumfluoroborat und 0.17 ml (1.51 mmol) 4-Methylmorpholin wurden in 1 ml DMF vorgelegt, mit 78.4 mg (0.33 mmol) *tert*-Butyl-(3-aminocyclopentyl)carbamat Hydrochlorid versetzt und über Nacht bei RT gerührt. Die Reaktionslösung wurde mit Wasser versetzt, der entstandene Feststoff 30 min bei RT gerührt, abfiltriert, mit Wasser gewaschen und im Hochvakuum getrocknet. Es wurden 120 mg der Zielverbindung (77% d. Th.) erhalten.
LC-MS (Methode 2): Rₜ = 0.94 min
MS (ESpos): m/z = 515 (M+H)⁺

### Beispiel 336A

### rac-2-Amino-3-(1,3-benzothiazol-2-yl)-2-methylpropanonitril

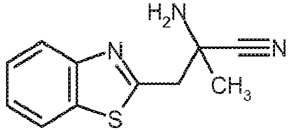

3.0 g (14.12 mmol, Reinheit 90%) 1-(1,3-Benzothiazol-2-yl)aceton wurden in 28.2 ml (56.47 mmol) 2 N Ammoniak in Methanol vorgelegt, mit 775 mg (15.81 mmol) Natriumcyanid und 1.8 g (15.81 mmol) Ammoniumchlorid versetzt und 3 Stunden unter Rückfluss gerührt. Die Reaktionslösung wurde abgekühlt, mit 90 ml Dichlormethan verdünnt, der ausgefallene Feststoff abfiltriert und das Filtrat wurde eingeengt. Der Rückstand wurde mittels Kieselgelchromatographie gereinigt (Laufmittel: Cyclohexan/Essigsäureethylester 5/1 nach 1/1). Es wurden 1.16 g der Zielverbindung (29% d. Th., Reinheit 77%) erhalten.
LC-MS (Methode 2): Rₜ = 0.70 min
MS (ESpos): m/z = 218 (M+H)⁺

### Beispiel 337A

### rac-3-(1,3-Benzothiazol-2-yl)-2-methylpropan-1,2-diamin

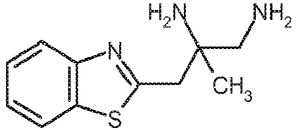

100 mg (0.35 mmol, 77%ig) *rac*-2-Amino-3-(1,3-benzothiazol-2-yl)-2-methylpropanonitril aus Beispiel 336A wurden in 3.6 ml THF vorgelegt und unter Argon bei 0°C mit 0.23 ml (0.23 mmol) 1 N Lithiumaluminiumhydrid in Diethylether versetzt. Es wurde 30 min bei 0°C und anschließend 1 h bei RT gerührt. Das Reaktionsgemisch wurde vorsichtig mit 0.04 ml Wasser, 0.04 ml 2 N wässriger Natriumhydroxidlösung und 0.07 ml Wasser versetzt. Der Niederschlag wurde abfiltriert, mit THF und wenig Methanol gewaschen, das Filtrat eingeengt und der Rückstand mittels Kieselgel-Chromatographie gereinigt (Laufmittel: Dichlormethan/Methanol 10/1; Dichlormethan/2 N Ammoniak in Methanol 20/1). Es wurden 17.6 mg der Zielverbindung (21% d. Th., Reinheit 94%) erhalten.
LC-MS (Methode 15): Rₜ = 1.73 min
MS (ESpos): m/z = 222 (M+H)⁺

### Beispiel 338A

### rac-tert-Butyl-5-[({8-[(2,6-difluorbenzyl)oxy]-2,6-dimethylimidazo[1,2-a]pyridin-3-yl}carbonyl)amino]-3,3-difluorpiperidin-1-carboxylat

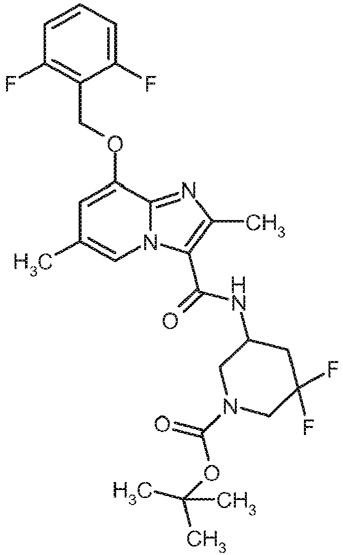

75 mg (0.23 mmol) 8-[(2,6-Difluorbenzyl)oxy]-2,6-dimethylimidazo[1,2-a]pyridin-3-carbonsäure aus Beispiel 21A wurden mit 94 mg (0.25 mmol) HATU und 0.2 ml (1.13 mmol) *N,N-*Diisopropylethylamin in 1.44 ml DMF vorgelegt, 20 min gerührt, anschließend mit 64 mg (0.27 mmol) *rac*-*tert*-butyl-5-amino-3,3-difluoropiperidin-1-carboxylat versetzt und über Nacht bei RT gerührt. Die Reaktionslösung wurde mit Wasser versetzt, der ausgefallene Feststoff 30 min bei RT gerührt, abfiltriert, mit Wasser gewaschen und im Hochvakuum getrocknet. Es wurden 95 mg der Zielverbindung (73% d. Th.) erhalten.
LC-MS (Methode 15): Rₜ = 1.07 min
MS (ESpos): m/z = 551 (M+H)⁺
¹H-NMR (400 MHz, DMSO-d₆): δ = 0.89 - 0.99 (m, 1H), 1.27 - 1.46 (m, 10H), 2.32 (s, 3H), 2.47 (s, 3H), 2.91 - 3.07 (m, 1H), 3.37 - 3.67 (m, 1H), 3.68 - 4.04 (m, 2H), 4.06 - 4.15 (m, 1H), 5.29 (s, 2H), 6.95 (s, 1H), 7.20 - 7.28 (m, 2H), 7.55 - 7.64 (m, 1H), 7.65 - 7.82 (m, 1H), 8.42 - 8.58 (m, 1H).

### Beispiel 339A

### tert-Butyl-3-[({8-[(2,6-difluorbenzyl)oxy]-2,6-dimethylimidazo[1,2-a]pyridin-3-yl}carbonyl)amino]-4-(trifluormethyl)pyrrolidin-1-carboxylat Trifluoracetat (Mischung von Stereoisomeren)

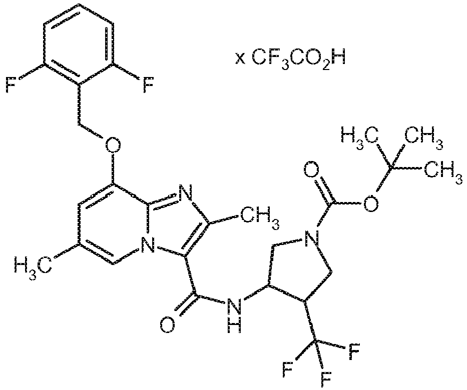

75 mg (0.23 mmol) 8-[(2,6-Difluorbenzyl)oxy]-2,6-dimethylimidazo[1,2-a]pyridin-3-carbonsäure aus Beispiel 21A wurden mit 94 mg (0.25 mmol) HATU und 0.12 ml (0.68 mmol) N,N-Diisopropylethylamin in 1.44 ml DMF vorgelegt, 20 min gerührt, mit 69 mg (0.27 mmol) *rac-tert-*Butyl-3-amino-4-(trifluormethyl)pyrrolidin-1-carboxylat (Mischung von Stereoisomeren) versetzt und über Nacht bei RT gerührt. Es wurden nochmals 47 mg (0.13 mmol) HATU und 0.05 ml (0.34 mmol) N,N-Diisopropylethylamin zugegeben und 15 min bei RT gerührt. Anschließend wurden 34 mg (0.13 mmol) *tert-*Butyl-3-amino-4-(trifluormethyl)pyrrolidin-1-carboxylat (Mischung von Stereoisomeren) zugegeben und über Nacht in einem auf 60°C erwärmten Ölbad gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Es wurden 11 mg der Zielverbindung (6% d. Th., Reinheit 80%) erhalten.
LC-MS (Methode 15): Rₜ = 1.12 min
MS (ESpos): m/z = 569 (M-TFA+H)⁺

### Beispiel 340A

### ent-Benzyl-{2-[({2,6-dimethyl-8-[(2,3,6-trifluorbenzyl)oxy]imidazo[1,2-a]pyridin-3-yl}carbonyl)amino]-2-methylbutyl}carbamat Trifluoracetat

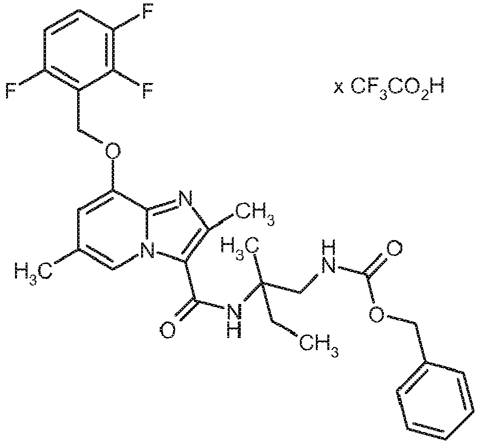

282 mg (0.81 mmol) 2,6-Dimethyl-8-[(2,3,6-trifluorbenzyl)oxy]imidazo[1,2-a]pyridin-3-carbonsäure aus Beispiel 265A wurden mit 337 mg (0.89 mmol) HATU und 0.4 ml (2.42 mmol) N,N-Diisopropylethylamin in 5 ml DMF vorgelegt, 20 min gerührt, dann mit 200 mg (0.85 mmol) *ent*-Benzyl-(2-amino-2-methylbutyl)carbamat aus Beispiel 275A versetzt und über Nacht bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Es wurden 165 mg der Zielverbindung (30% d. Th.) erhalten.
LC-MS (Methode 2): Rₜ = 1.07 min
MS (ESpos): m/z = 569 (M-TFA+H)⁺
¹H-NMR (400 MHz, DMSO-d₆): δ = 0.87 (t, 3H), 1.26 (s, 3H), 1.49 -1.60 (m, 1H), 1.93 - 2.06 (m, 1H), 2.37 (s, 3H), 2.71 (s, 3H), 3.29 - 3.37 (m, 1H), 3.51 - 3.59 (m, 1H), 4.99 (s, 2H), 5.44 (s, 2H), 7.18 - 7.37 (m, 6H), 7.50 (t, 1H), 7.65 - 7.75 (m, 2H), 8.41 (s, 1H).

### Beispiel 341A

### rac-tert-Butyl-3-[({8-[(2,6-difluorbenzyl)oxy]-2,6-dimethylimidazo[1,2-a]pyridin-3-yl}carbonyl)amino]azepan-1-carboxylat Trifluoracetat

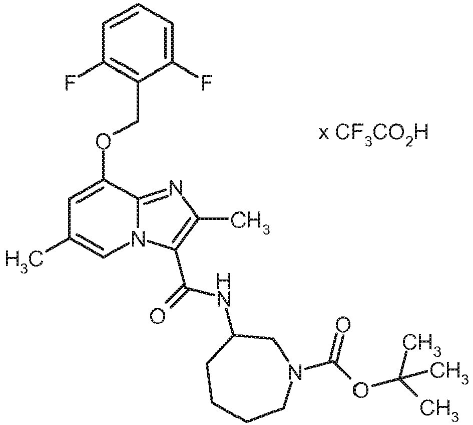

125 mg (0.38 mmol) 8-[(2,6-Difluorbenzyl)oxy]-2,6-dimethylimidazo[1,2-a]pyridin-3-carbonsäure aus Beispiel 21A, 157 mg (0.49 mmol) (Benzotriazol-1-yloxy)bisdimethylamino-methyliumfluoroborat und 0.12 ml (1.13 mmol) N-Methylmorpholin wurden in 2.4 ml DMF vorgelegt, 10 min bei RT gerührt, dann mit 105 mg (0.49 mmol) *rac*-*tert*-Butyl-3-aminoazepan-1-carboxylat versetzt und über Nacht bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Es wurden 194 mg der Zielverbindung (80% d. Th.) erhalten.
LC-MS (Methode 2): Rₜ = 1.11 min
MS (ESpos): m/z = 529 (M-TFA+H)⁺
¹H-NMR (400 MHz, DMSO-d₆): δ = 1.42 (s, 9H), 1.47 - 1.67 (m, 2H), 1.69 - 1.96 (m, 3H), 2.41 (br. s., 3H), 2.98 - 3.15 (m, 1H), 3.26 - 3.41 (m, 2H), 3.61 - 3.79 (m, 2H), 4.13 - 4.25 (m, 1H), 5.39 (s, 2H), 7.22 - 7.30 (m, 2H), 7.33 - 7.51 (m, 1H), 7.57 - 7.67 (m, 1H), 8.06 - 8.26 (m, 1H), 8.42 - 8.61 (m, 1H).

### Beispiel 351A

### Methyl-N-(tert-butoxycarbonyl)-3-[({8-[(2,6-difluorbenzyl)oxy]-2,6-dimethylimidazo[1,2-a]pyridin-3-yl}carbonyl)amino]-L-alaninat

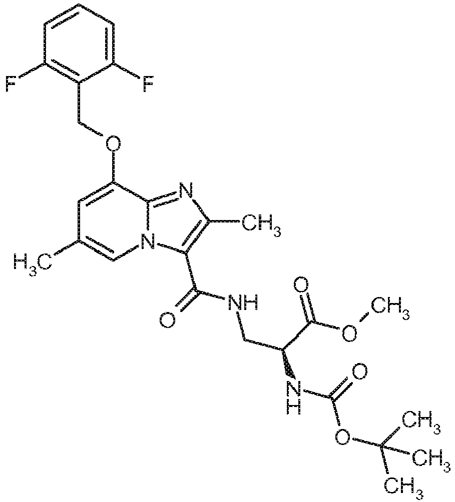

300 mg (0.90 mmol) 8-[(2,6-Difluorbenzyl)oxy]-2,6-dimethylimidazo[1,2-a]pyridin-3-carbonsäure aus Beispiel 21A, 377 mg (1.17 mmol) (Benzotriazol-1-yloxy)bisdimethylamino-methyliumfluoroborat und 0.5 ml (4.51 mmol) *N*-Methylmorpholin wurden in 5.7 ml DMF vorgelegt, 10 min bei RT gerührt, dann mit 253 mg (0.99 mmol) Methyl-3-amino-N-(tert-butoxycarbonyl)-L-alaninathydrochlorid versetzt und über Nacht bei RT gerührt. Die Reaktionslösung wurde mit ca. 40 ml Wasser versetzt und ca. 30 min bei RT gerührt. Der Feststoff wurde abgesaugt, mit Wasser gewaschen und im Hochvakuum getrocknet. Es wurden 377 mg der Zielverbindung (75% d. Th.) erhalten.
LC-MS (Methode 2): Rₜ = 0.96 min
MS (ESpos): m/z = 533 (M+H)⁺
¹H-NMR (400 MHz, DMSO-d₆): δ = 1.37 (s, 9H), 2.31 (s, 3H), 2.47 (s, 3H), 3.55 - 3.69 (m, 5H), 4.28 (q, 1H), 5.28 (s, 2H), 6.91 - 6.98 (m, 1H), 7.19 - 7.28 (m, 2H), 7.33 (d, 1H), 7.54 - 7.64 (m, 1H), 7.86 (t, 1H), 8.40 - 8.47 (m, 1H).

### Beispiel 352A

### rac-2-Amino-2-methyl-3-[1-(5-methyl-1,2-oxazol-3-yl)-1H-1,2,4-triazol-3-yl]propanonitril

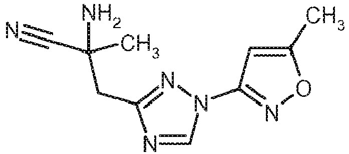

860 mg (4.17 mmol) 1-[1-(5-Methyl-1,2-oxazol-3-yl)-1H-1,2,4-triazol-3-yl]aceton wurden in 8.3 ml 2 N Ammoniak in Methanol vorgelegt, mit 229 mg (4.67 mmol) Natriumcyanid und 556 mg (4.67 mmol) Ammoniumchlorid versetzt und 4 Stunden unter Rückfluss gerührt. Die Reaktionslösung wurde abgekühlt, mit 90 ml Dichlormethan verdünnt, der enthaltene Feststoff abfiltriert, das Filtrat eingeengt und der Rückstand mittels Kieselgel-Chromatographie gereinigt (Laufmittel: Cyclohexan/Ethylacetat: 4/1 nach 1/1). Es wurden 761 mg der Zielverbindung (78% d. Th., Reinheit 93%) erhalten.
LC-MS (Methode 15): Rₜ = 1.48 min
MS (ESpos): m/z = 233 (M+H)⁺
¹H-NMR (400 MHz, DMSO-d₆): δ = 1.51 (s, 3H), 2.82 (s, 2H), 3.08 (q, 2H), 3.33 (s, 3H), 6.81 (d, 1H), 9.25 (s, 1H).

### Beispiel 353A

### rac-2-Methyl-3-[1-(5-methyl-1,2-oxazol-3-yl)-1H-1,2,4-triazol-3-yl]propan-1,2-diamin

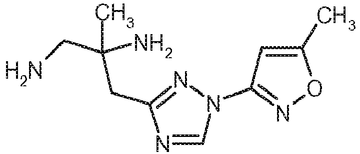

250 mg (1.00 mmol, Reinheit 93%) *rac*-2-Amino-2-methyl-3-[1-(5-methyl-1,2-oxazol-3-yl)-1H-1,2,4-triazol-3-yl]propanonitril aus Beispiel 352A wurden in 10.1 ml THF vorgelegt und unter Argon bei 0°C langsam mit 0.65 ml (0.65 mmol) 1 M Lithiumaluminiumhydrid-Lösung in THF versetzt. Es wurde 30 min bei 0°C und anschließend 2 Stunden bei RT gerührt. Bei 0°C wurden 0.20 ml (0.20 mmol) 1 M Lithiumaluminiumhydrid-Lösung in THF zugetropft und dann 2 Stunden bei RT gerührt. Bei 0°C wurden nochmals 0.40 ml (0.40 mmol) 1 M Lithiumaluminiumhydrid-Lösung in THF zugetropft und 2 Stunden bei RT gerührt. Das Reaktionsgemisch wurde vorsichtig mit 0.1 ml Wasser, 0.1 ml 2 N wässriger Natronlauge und 0.2 ml Wasser versetzt. Der entstehende Niederschlag wurde abfiltriert, mit Dichlormethan/Methanol (10/1) gewaschen, das Filtrat eingeengt und im Hochvakuum getrocknet. Es wurden 109 mg der Zielverbindung (46% d. Th.) erhalten.
LC-MS (Methode 2): Rₜ = 0.17 min
MS (ESpos): m/z = 237 (M+H)⁺

### Beispiel 354A

### Ethyl-2-chlor-3-cyclopropyl-3-oxopropanoat

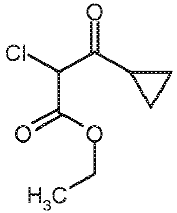

13.5 ml (168.07 mmol) Sulfuryldichlorid wurden in 100 ml Dichlormethan vorgelegt. Bei 15°C wurden langsam 25 g (160.07 mmol) Ethyl-3-cyclopropyl-3-oxopropanoat zugetropft und es wurde 2 Stunden bei RT gerührt. Das Reaktionsgemisch wurde mit je 100 ml Wasser, 5%iger wässriger Natriumhydrogencarbonat-Lösung und gesättigter wässriger Natriumchlorid-Lösung gewaschen. Die organische Phase wurde über Natriumsulfat getrocknet, abfiltriert und am Rotationsverdampfer (25°C Badtemperatur, 200 mbar) vorsichtig eingeengt. Es wurden 38 g der Zielverbindung (109% d. Th., Reinheit ca. 88%) erhalten.
LC-MS (Methode 2): Rₜ = 0.88 min
MS (ESpos): m/z = 191 (M+H)⁺
¹H-NMR (400 MHz, DMSO-d₆): δ = 0.92 - 1.03 (m, 2H), 1.03 - 1.14 (m, 2H), 1.22 (t, 3H), 2.22 - 2.30 (m, 1H), 4.19 - 4.28 (m, 2H), 5.79 (s, 1H).

### Beispiel 355A

### Ethyl-2-cyclopropyl-8-[(2,6-difluorbenzyl)oxy]-6-methylimidazo[1,2-a]pyridin-3-carboxylat

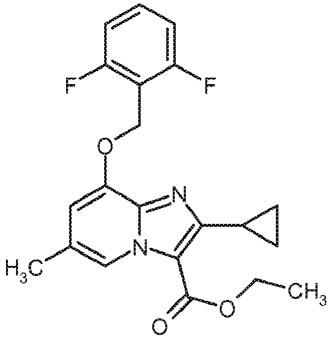

4.00 g (15.98 mmol) 3-[(2,6-Difluorbenzyl)oxy]-5-methylpyridin-2-amin aus Beispiel 323A und 17.31 g (79.92 mmol, Reinheit 88%) Ethyl-2-chlor-3-cyclopropyl-3-oxopropanoat aus Beispiel 354A wurden unter Argon in 160 ml Ethanol gelöst und 8 Tage mit ca. 2 g 3 Å Molsieb unter Rückfluß gerührt (es wurden jeden Tag ca. 0.5 g 3 Å Molsieb hinzugegeben). Die Reaktionsmischung wurde abgekühlt, abgesaugt und die Mutterlauge eingeengt. Der Rückstand wurde mittels Kieselgel-Chromatographie (Laufmittel: Cyclohexan/Essigsäureethylester-Gradient: 95/5 nach 7/3) gereinigt. Es wurden 0.6 g der Zielverbindung (10% d. Th.) erhalten.
¹H-NMR (400 MHz, DMSO-d₆): δ = 0.93 - 1.01 (m, 4H), 1.36 (t, 3H), 2.08 - 2.17 (m, 1H), 2.35 (s, 3H), 4.38 (q, 3H), 5.29 (s, 2H), 7.08 (s, 1H), 7.19 - 7.28 (m, 2H), 7.54 - 7.64 (m, 1H), 8.73 (s, 1H).

### Beispiel 356A

### 2-Cyclopropyl-8-[(2,6-difluorbenzyl)oxy]-6-methylimidazo[1,2-a]pyridin-3-carbonsäure

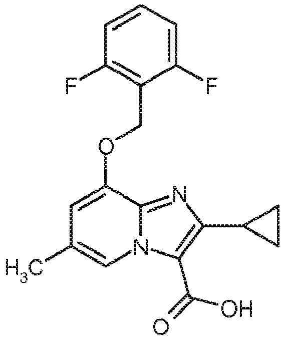

100 mg (0.26 mmol) Ethyl-2-cyclopropyl-8-[(2,6-difluorbenzyl)oxy]-6-methylimidazo[1,2-a]pyridin-3-carboxylat aus Beispiel 355A wurde in 5.6 ml THF/Methanol (5/1) gelöst, mit 1.3 ml (1.29 mmol) 1 N wässriger Lithiumhydroxidlösung versetzt und 2 Tage bei RT gerührt. Die Reaktionslösung wurde mit 1 N wässriger Salzsäure auf pH 3 eingestellt und das organische Lösungsmittel wurde abdestilliert. Der entstandene Niederschlag wurde abfiltriert, mit Wasser gewaschen und im Hochvakuum getrocknet. Es wurden 64 mg der Zielverbindung (63% d. Th., Reinheit 91%) erhalten.
LC-MS (Methode 2): Rₜ = 0.92 min
MS (ESpos): m/z = 359 (M+H)⁺

### Beispiel 357A

### ent-Benzyl-{1-[({2-cyclopropyl-8-[(2,6-difuorbenzyl)oxy]-6-methylimidazo[1,2-a]pyridin-3-yl}carbonyl)amino]-2-methylpentan-2-yl}carbamat Trifluoracetat

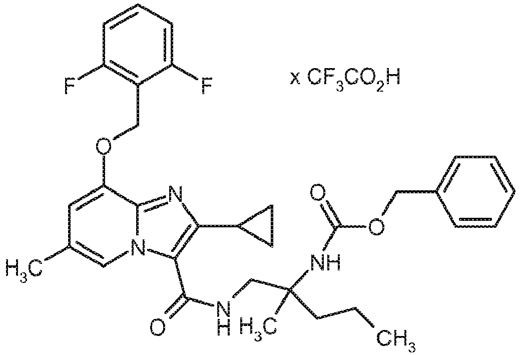

50 mg (0.13 mmol, Reinheit 91%) 2-Cyclopropyl-8-[(2,6-difluorbenzyl)oxy]-6-methylimidazo[1,2-a]pyridin-3-carbonsäure aus Beispiel 356A, 45 mg (0.14 mmol) (Benzotriazol-1-yloxy)bisdimethylaminomethyliumfluoroborat (TBTU) und 0.07 ml (0.64 mmol) 4-Methylmorpholin wurden in 0.5 ml DMF vorgelegt, mit 35 mg (0.14 mmol) *ent*-Benzyl-(1-amino-2-methylpentan-2-yl)carbamat aus Beispiel 289A versetzt und über Nacht bei RT gerührt. Die Reaktionslösung wurde mit etwas Wasser/TFA versetzt und mittels präparativer HPLC gereinigt (RP18 Säule, Laufmittel: Acetonitril/Wasser-Gradient unter Zusatz von 0.1% TFA). Es wurden 75 mg der Zielverbindung (82% d. Th.) erhalten.
LC-MS (Methode 2): Rₜ = 1.32 min
MS (ESpos): m/z = 591 (M-TFA+H)⁺

### Beispiel 358A

### ent-Benzyl-{1-[({8-[(2,6-difluorbenzyl)oxy]-2-methyl-6-[(trimethylsilyl)ethinyl]imidazo[1,2-a]pyridin-3-yl}carbonyl)amino]-2-methylpentan-2-yl}carbamat

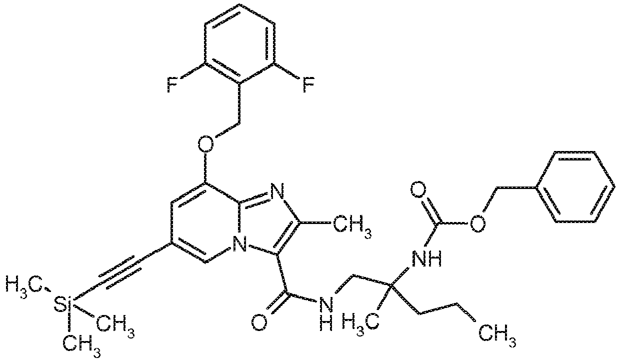

514 mg (0.691 mmol) *ent*-Benzyl-{1-[({6-brom-8-[(2,6-difluorbenzyl)oxy]-2-methylimidazo[1,2-a]pyridin-3-yl}carbonyl)amino]-2-methylpentan-2-yl}carbamat Trifluoracetat aus Beispiel 292A, 97 mg (0.138 mmol) Dichlor[bis(triphenylphosphoranyl)]palladium und 26 mg (0.138 mmol) Kupfer(I)iodid wurden in 7.5 ml Dioxan und 7.5 ml Diisopropylethylamin bei RT vorgelegt. Anschließend wurden 407 mg (4.15 mmol) Ethinyl(trimethyl)silan zugetropft und über Nacht bei 50°C gerührt. Die Reaktionsmischung wurde am Rotationsverdampfer eingeengt. Der Rückstand wurde mittels präparativer HPLC gereinigt (RP-C18, Laufmittel: Acetonitril/Wasser-Gradient unter Zusatz von 0.05% Ameisensäure). Es wurden 401 mg (90% d. Th.) der Titelverbindung erhalten.
LC-MS (Methode 2): Rₜ = 1.53 min
MS (ESpos): m/z = 647 (M+H)⁺
¹H-NMR (400 MHz, DMSO-d₆): δ = 0.26 (s, 9H), 0.85 (t, 3H), 1.20 (s, 3H), 1.23 - 1.34 (m, 2H), 1.45 - 1.56 (m, 1H), 1.69 - 1.82 (m, 1H), 2.52 (s, 3H), 3.45 - 3.59 (m, 2H), 5.00 (s, 2H), 5.33 (s, 2H), 7.02 - 7.12 (m, 2H), 7.20 - 7.37 (m, 7H), 7.53 - 7.66 (m, 1H), 7.84 (t, 1H), 8.78 (s, 1H).

### Beispiel 359A

### ent-Benzyl-{1-[({8-[(2,6-difluorbenzyl)oxy]-6-ethinyl-2-methylimidazo[1,2-a]pyridin-3-yl}carbonyl)amino]-2-methylpentan-2yl}carbamat

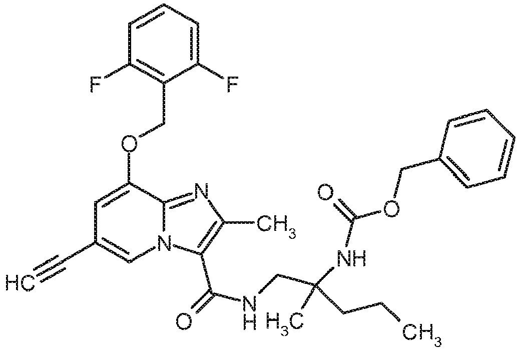

400 mg (0.62 mmol) *ent*-Benzyl-{1-[({8-[(2,6-difluorbenzyl)oxy]-2-methyl-6-[(trimethylsilyl)ethinyl]imidazo[1,2-a]pyridin-3-yl}carbonyl)amino]-2-methylpentan-2-yl}carbamat aus Beispiel 358A wurden in 6.2 ml Methanol vorgelegt, mit 256 mg (1.86 mmol) Kaliumcarbonat bei RT versetzt und 1.5 h bei RT gerührt. Die Reaktionsmischung wurde am Rotationsverdampfer eingeengt. Der Rückstand wurde mit Eiswasser versetzt und dreimal mit Essigsäureethylester extrahiert. Die vereinigten organischen Phasen wurden über Natriumsulfat getrocknet und im Vakuum eingeengt. Es wurden 312 mg (89% d. Th.) der Titelverbindung erhalten.
LC-MS (Methode 2): Rₜ = 1.31 min
MS (ESpos): m/z = 575 (M+H)⁺

### Beispiel 360A

### ent-N-[2-Ethyl-6-(trifluormethyl)-1,2,3,4-tetrahydro-1,5-naphthyridin-4-yl]acetamid (Enantiomer A)

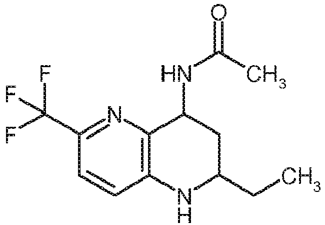

5.80 g (20.19 mmol) *rac*-N-[2-Ethyl-6-(trifluormethyl)-1,2,3,4-tetrahydro-1,5-naphthyridin-4-yl]acetamid (beschrieben in: M.-C. Fernandez et al. Bioorg. Med. Chem. Lett. 2012, 22, 3056-3062) wurden durch präparative Trennung an der chiralen Phase in die Enantiomere getrennt [Säule: SFC Chiralpak AY-H, 20 µm, 360 x 50 mm, Eluent: 85% Kohlendioxid, 15% Isopropanol, Fluß: 400 ml/min; Temperatur: 38°C; backpressure: 80 bar; Detektion: 220 nm].
Enantiomer A: Ausbeute: 2.20 g (>99% ee)
Rₜ = 1.30 min [Daicel Chiralpak AY-H, 5 µm, 250 x 4.6 mm; Eluent: 70% Kohlendioxid, 30% Isopropanol; Fluß: 3 ml/min; Detektion: 210 nm].

### Beispiel 361A

### ent-2-Ethyl-6-(trifluormethyl)-1,2,3,4-tetrahydro-1,5-naphthyridin-4-amin Hydrochlorid

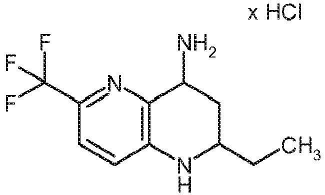

150 mg (0.52 mmol) *ent*-N-[2-Ethyl-6-(trifluormethyl)-1,2,3,4-tetrahydro-1,5-naphthyridin-4-yl]acetamid (Enantiomer A) aus Beispiel 360A wurden mit 2.8 ml gesättigter Chlorwasserstofflösung in Methanol versetzt und in der Mikrowelle für 1 Stunde bei 80°C gerührt. Das Reaktionsgemisch wurde eingeengt, in Acetonitril/Wasser (1:1) gelöst und lyophilisiert. Es wurden 124 mg der Zielverbindung (80% d. Th.) erhalten.
LC-MS (Methode 15): Rₜ = 2.05 min
MS (ESpos): m/z = 246 (M-HCl+H)⁺
¹H-NMR (400 MHz, DMSO-d₆): δ = 0.96 (t, 3H), 1.47 - 1.69 (m, 3H), 2.29 - 2.39 (m, 1H), 3.41 - 3.52 (m, 1H), 4.50 - 4.62 (m, 1H), 6.93 (br. s., 1H), 7.09 (d, 1H), 7.52 (d, 1H), 8.45 (br. s., 3H).

### Beispiel 362A

### ent-N-[2-Methyl-6-(trifluormethyl)-1,2,3,4-tetrahydro-1,5-naphthyridin-4-yl]acetamid (Enantiomer A)

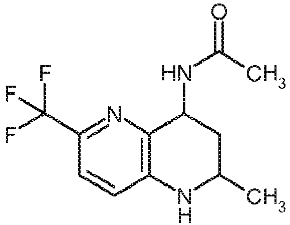

6.00 g (21.96 mmol) *rac*-N-[2-Methyl-6-(trifluormethyl)-1,2,3,4-tetrahydro-1,5-naphthyridin-4-yl]acetamid (beschrieben in: M.-C. Fernandez et al. Bioorg. Med. Chem. Lett. 2012, 22, 3056-3062) wurden durch präparative Trennung an der chiralen Phase in die Enantiomere getrennt [Säule: SFC Chiralpak AY-H, 20 µm, 360 x 50 mm, Eluent: 90% Kohlendioxid, 10% Methanol, Fluß: 400 ml/min; Temperatur: 38°C; backpressure: 80 bar; Detektion: 220 nm].
Enantiomer A: Ausbeute: 2.41 g (>99% ee)
Rₜ = 2.66 min [Daicel Chiralpak AY-H, 5 µm, 250 x 4.6 mm; Eluent: 90% Kohlendioxid, 10% Isopropanol; Fluß: 3 ml/min; Detektion: 210 nm].

### Beispiel 363A

### ent-2-Methyl-6-(trifluormethyl)-1,2,3,4-tetrahydro-1,5-naphthyridin-4-amin Hydrochlorid

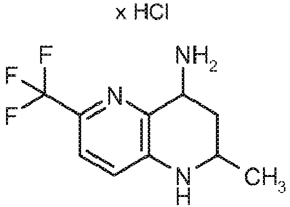

152 mg (0.56 mmol) *ent*-N-[2-Methyl-6-(trifluormethyl)-1,2,3,4-tetrahydro-1,5-naphthyridin-4-yl]acetamid (Enantiomer A) aus Beispiel 362A (beschrieben in: M.-C. Fernandez et al. Bioorg. Med. Chem. Lett. 2012, 22, 3056-3062) wurden mit 2.8 ml gesättigter Chlorwasserstofflösung in Methanol versetzt und in der Mikrowelle für 1 Stunde bei 80°C gerührt. Das Reaktionsgemisch wurde eingeengt und lyophilisiert. Es wurden 147 mg der Zielverbindung (97% d. Th.) erhalten.
LC-MS (Methode 2): Rₜ = 0.40 min
MS (ESpos): m/z = 232 (M-HCl+H)⁺
¹H-NMR (400 MHz, DMSO-d₆): δ = 1.23 (d, 3H), 1.59 (q, 1H), 2.25 - 2.35 (m, 1H), 3.58 - 3.69 (m, 1H), 4.50 - 4.61 (m, 1H), 6.97 - 7.05 (m, 2H), 7.52 (d, 1H), 8.40 (br. s., 3H).

### Beispiel 364A

### Ethyl-8-[(2,6-difluorbenzyl)oxy]-6-methyl-2-propylimidazo[1,2-a]pyridin-3-carboxylat

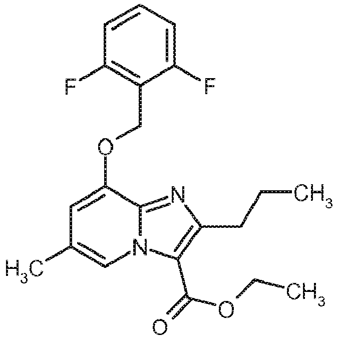

Unter Argon wurden 3.0 g (11.99 mmol) 3-[(2,6-Difluorbenzyl)oxy]-5-methylpyridin-2-amin aus Beispiel 323A in 60 ml Ethanol vorgelegt. Dann wurden 18.48 g (95.90 mmol) Ethyl-2-chlor-3-oxohexanoat (beschrieben in: M. Altuna-Urquijo et al. Tetrahedron 2009, 65, 975-984) und 600 mg 3 Å Molsieb hinzugegeben und 5 Tage unter Rückfluss gerührt. Die Reaktionslösung wurde eingeengt und zwischen Wasser und Essigsäureethylester verteilt. Die Phasen wurden getrennt und die wässrige Phase mit Essigsäureethylester extrahiert. Die organischen Phasen wurden vereinigt, mit Natriumsulfat getrocknet, abfiltriert und eingeengt. Der Rückstand wurde mittels Kieselgel-Chromatographie gereinigt (Laufmittel: Cyclohexan/Essigester = 95/5 nach 8/2). Es wurden 2.4 g der Zielverbindung (47% d. Th., Reinheit ca. 92%) erhalten.
LC-MS (Methode 2): Rₜ = 1.23 min
MS (ESpos): m/z = 389 (M+H)⁺
¹H-NMR (400 MHz, DMSO-d₆): δ = 0.90 (t, 3H), 1.35 (t, 3H), 1.60 - 1.70 (m, 2H), 2.37 (s, 3H), 2.87 - 2.94 (m, 2H), 4.35 (q, 2H), 5.31 (s, 2H), 7.10 (s, 1H), 7.21 - 7.29 (m, 2H), 7.55 - 7.65 (m, 1H), 8.74 (s, 1H).

### Beispiel 365A

### 8-[(2,6-Difluorbenzyl)oxy]-6-methyl-2-propylimidazo[1,2-a]pyridin-3-carbonsäure

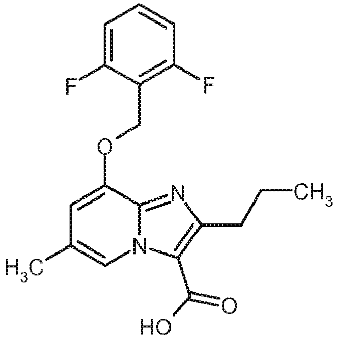

2.30 g (5.92 mmol) Ethyl-8-[(2,6-difluorbenzyl)oxy]-6-methyl-2-propylimidazo[1,2-a]pyridin-3-carboxylat aus Beispiel 364A wurden in 108 ml THF, 29 ml Wasser und 21.6 ml Methanol bei RT vorgelegt. Es wurden 1.24 g (29.61 mmol) Lithiumhydroxid Monohydrat zugeben und 16 Stunden bei RT gerührt. Das Reaktionsgemisch wurde von den organischen Lösungsmitteln befreit und die erhaltene wässrige Lösung wurde mit halbkonzentrierter Salzsäure angesäuert. Die wässrige Phase wurde zweimal mit Dichlormethan extrahiert. Die organischen Phasen wurden vereinigt, mit Natriumsulfat getrocknet, abfiltriert und eingeengt. Es wurden 2.50 g der Zielverbindung (115% d. Th.) erhalten.
LC-MS (Methode 2): Rₜ = 0.83 min
MS (ESpos): m/z = 361 (M+H)⁺
¹H-NMR (400 MHz, DMSO-d₆): δ = 0.89 (t, 3H), 1.61 - 1.72 (m, 2H), 2.41 (s, 3H), 2.95 (t, 2H), 5.35 (s, 2H), 7.19 - 7.35 (m, 3H), 7.56 - 7.66 (m, 1H), 8.85 (s, 1H), 12.94 -13.92 (br. s, 1H).

### Beispiel 366A

### ent-Benzyl-{1-[({8-[(2,6-difluorbenzyl)oxy]-6-methyl-2-propylimidazo[1,2-a]pyridin-3-yl}carbonyl)amino]-2-methylbutan-2-yl}carbamat

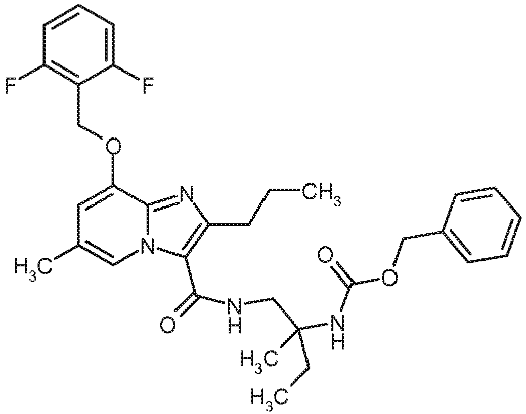

100 mg (0.28 mmol) 8-[(2,6-Difluorbenzyl)oxy]-6-methyl-2-propylimidazo[1,2-a]pyridin-3-carbonsäure aus Beispiel 365A wurden mit 127 mg (0.33 mmol) HATU und 0.24 ml (1.39 mmol) N,N-Diisopropylethylamin in 2 ml DMF vorgelegt und 10 min bei RT vorgerührt. Anschließend wurden 98.4 mg (0.42 mmol) *ent*-Benzyl-(1-amino-2-methylbutan-2-yl)carbamat aus Beispiel 274A zur Reaktionslösung gegeben und es wurde über Nacht bei RT gerührt. Die Reaktionslösung wurde mittels präparativer HPLC gereinigt (RP18 Säule, Laufmittel: Acetonitril/Wasser-Gradient unter Zusatz von 0.05% Ameisensäure). Es wurden 109 mg der Zielverbindung (68% d. Th.) erhalten.
LC-MS (Methode 2): Rₜ = 1.15 min
MS (ESpos): m/z = 579 (M+H)⁺
¹H-NMR (400 MHz, DMSO-d₆): δ = 0.79 - 0.88 (m, 6H), 1.20 (s, 3H), 1.55 - 1.70 (m, 3H), 1.73 - 1.86 (m, 1H), 2.29 (s, 3H), 2.83 (t, 2H), 3.45 - 3.58 (m, 2H), 5.00 (s, 2H), 5.29 (s, 2H), 6.91 (s, 1H), 7.03 - 7.10 (m, 1H), 7.20 - 7.28 (m, 2H), 7.28 - 7.38 (m, 5H), 7.55 - 7.64 (m, 1H), 7.79 (t, 1H), 8.37 (s, 1H).

### Beispiel 367A

### rac-ter-Butyl-{2-[({8-[(2,6-difluorbenzyl)oxy]-2,6-dimethylimidazo[1,2-a]pyridin-3-yl}carbonyl)amino]cyclobutyl}carbamat

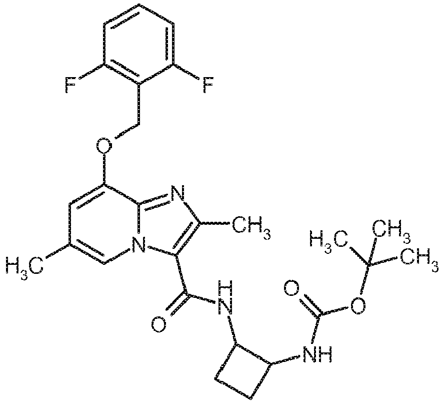

150 mg (0.45 mmol) 8-[(2,6-Difluorbenzyl)oxy]-2,6-dimethylimidazo[1,2-a]pyridin-3-carbonsäure aus Beispiel 21A wurden mit 206 mg (0.54 mmol) HATU und 0.4 ml (2.26 mmol) N,N-Diisopropylethylamin in 4.4 ml DMF vorgelegt, 10 min gerührt, anschließend bei RT mit 101 mg (0.54 mmol) *rac*-*tert*-Butyl-(2-aminocyclobutyl)carbamat versetzt und über Nacht bei RT gerührt. Das Reaktionsgemisch wurde mit Wasser versetzt, der entstandene Feststoff wurde ca. 30 min bei RT verrührt, anschließend abfiltriert und gut mit Wasser gewaschen. Es wurden 185 mg der Zielverbindung (82% d. Th.) erhalten.
LC-MS (Methode 2): Rₜ = 0.94 min
MS (ESpos): m/z = 501 (M+H)⁺
¹H-NMR (400 MHz, DMSO-d₆): δ = 1.37 (s, 9H), 1.48 -1.67 (m, 2H), 1.87 - 2.02 (m, 2H), 2.30 (s, 3H), 2.45 (s, 3H), 3.99 - 4.12 (m, 1H), 4.23 - 4.35 (m, 1H), 5.28 (s, 2H), 6.90 (s, 1H), 7.20 - 7.29 (m, 3H), 7.54 - 7.64 (m, 1H), 8.17 (d, 1H), 8.35 (s, 1H).

### Beispiel 368A

### ent-tert-Butyl-{2-[({8-[(2,6-difluorbenzyl)oxy]-2,6-dimethylimidazo[1,2-a]pyridin-3-yl}carbonyl)amino]cyclobutyl}carbamat (Enantiomer A)

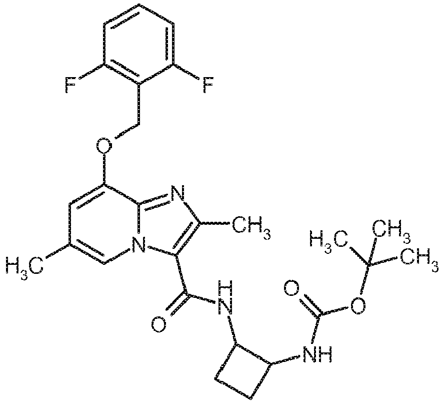

180 mg (0.37 mmol) *rac-tert*-Butyl-{2-[({8-[(2,6-difluorbenzyl)oxy]-2,6-dimethylimidazo[1,2-a]pyridin-3-yl}carbonyl)amino]cyclobutyl}carbamat aus Beispiel 367A wurden durch präparative Trennung an der chiralen Phase in die Enantiomere getrennt [Säule: SFC Chiralpak AZ-H, 5 µm, 250 x 20 mm, Eluent: 50% iso-Hexan, 50% Ethanol, Fluß: 50 ml/min; Temperatur: 20°C; Detektion: 220 nm].
Enantiomer A: Ausbeute: 77 mg (>99% ee)
Rt= 5.79 min [Daicel Chiralpak AZ-H, 5 µm, 250 x 4.6 mm; Eluent: 50% iso-Hexan, 50% Ethanol; Fluß: 1 ml/min; Detektion: 220 nm].

### Beispiel 369A

### ent-tert-Butyl-{2-[({8-[(2,6-difluorbenzyl)oxy]-2,6-dimethylimidazo[1,2-a]pyridin-3-yl}carbonyl)amino]cyclobutyl}carbamat (Enantiomer B)

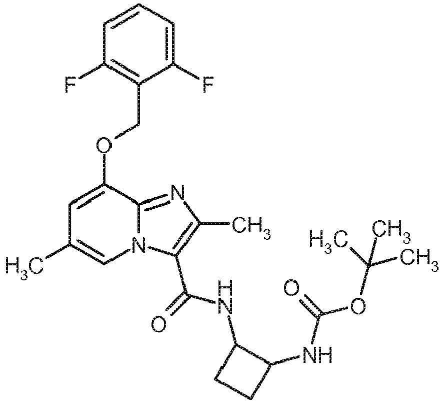

180 mg (0.37 mmol) *rac*-*tert*-Butyl-{2-[({8-[(2,6-difluorbenzyl)oxy]-2,6-dimethylimidazol[1,2-a]pyridin-3-yl}carbonyl)amino]cyclobutyl}carbamat aus Beispiel 367A wurden durch präparative Trennung an der chiralen Phase in die Enantiomere getrennt [Säule: SFC Chiralpak AZ-H, 5 µm, 250 x 20 mm, Eluent: 50% iso-Hexan, 50% Ethanol, Fluß: 50 ml/min; Temperatur: 20°C; Detektion: 220 nm].
Enantiomer B: Ausbeute: 67 mg (>99% ee)
Rₜ = 8.24 min [Daicel Chiralpak AZ-H, 5 µm, 250 x 4.6 mm; Eluent: 50% iso-Hexan, 50% Ethanol; Fluß: 1 ml/min; Detektion: 220 nm].

### Beispiel 370A

### ent-Benzyl-{1-[({8-[(2,6-difluorbenzyl)oxy]-2-ethyl-6-methylimidazo[1,2-a]pyridin-3-yl}carbonyl)amino]-2-methylpentan-2-yl}carbamat Trifluoracetat

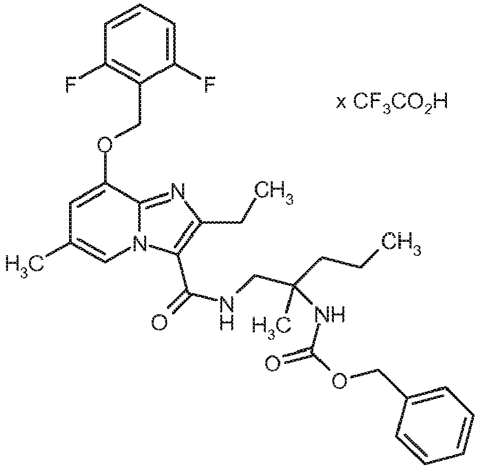

110 mg (0.32 mmol) 8-[(2,6-Difluorbenzyl)oxy]-2-ethyl-6-methylimidazo[1,2-a]pyridin-3-carbonsäure aus Beispiel 325A wurden mit 133 mg (0.35 mmol) HATU und 0.17 ml (0.95 mmol) N,N-Diisopropylethylamin in 2 ml DMF vorgelegt, 20 min gerührt, dann mit 106 mg (0.36 mmol, Reinheit 86%) *ent*-Benzyl-(1-amino-2-methylpentan-2-yl)carbamat aus Beispiel 288A versetzt und über Nacht bei RT gerührt. Die Reaktionslösung wurde mit etwas Wasser/TFA versetzt und mittels präparativer HPLC gereinigt (RP18 Säule, Laufmittel: Acetonitril/Wasser-Gradient unter Zusatz von 0.1% TFA). Es wurden 181 mg der Zielverbindung (82% d. Th.) erhalten.
LC-MS (Methode 2): Rₜ = 1.19 min
MS (ESpos): m/z = 579 (M-TFA+H)⁺
¹H-NMR (400 MHz, DMSO-d₆): δ = 0.86 (t, 3H), 1.17 - 1.24 (m, 6H), 1.25 - 1.35 (m, 2H), 1.44 - 1.57 (m, 1H), 1.69 - 1.81 (m, 1H), 2.37 (s, 3H), 2.91 (q, 2H), 3.50 - 3.60 (m, 2H), 4.97 - 5.02 (m, 2H), 5.38 (s, 2H), 7.09 (s, 1H), 7.22 - 7.39 (m, 8H), 7.56 - 7.66 (m, 1H), 8.06 - 8.31 (m, 1H), 8.42 (s, 1H).

### Beispiel 371A

### rac-tert-Butyl-4-[({8-[(2,6-difluorbenzyl)oxy]-2,6-dimethylimidazo[1,2-a]pyridin-3-yl}carbonyl)amino]hexahydrocyclopenta[b]pyrrol-1(2H)-carboxylat

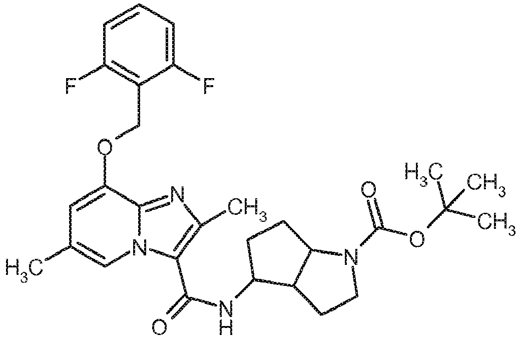

75 mg (0.23 mmol) 8-[(2,6-Difluorbenzyl)oxy]-2,6-dimethylimidazo[1,2-a]pyridin-3-carbonsäure aus Beispiel 21A wurden mit 94.4 mg (0.25 mmol) HATU und 0.12 ml (0.68 mmol) N,N-Diisopropylethylamin in 1.4 ml DMF vorgelegt, 20 min gerührt, anschließend mit 61 mg (0.27 mmol) *rac*-*tert*-Butyl-4-aminohexahydrocyclopenta[b]pyrrol-1(2H)-carboxylat (kommerziell erhätlich; außerdem beschrieben in WO 201056717 A1) versetzt und über Nacht bei RT gerührt. Die Reaktionslösung wurde mit Wasser versetzt, der ausgefallene Feststoff wurde 30 min bei RT verrührt, abfiltriert, mit Wasser gewaschen und im Hochvakuum getrocknet. Es wurden 116 mg der Zielverbindung (95% d. Th.) erhalten.
LC-MS (Methode 2): Rₜ = 1.02 min
MS (ESpos): m/z = 541 (M+H)⁺
¹H-NMR (400 MHz, DMSO-d₆): δ = 0.89 -1.08 (m, 3H), 1.40 (s, 9H), 1.58 -1.86 (m, 7H), 2.31 (s, 3H), 2.48 (s, 3H), 3.96 - 4.12 (m, 1H), 4.18 - 4.30 (m, 1H), 5.29 (s, 2H), 6.91 (s, 1H), 7.19 - 7.29 (m, 2H), 7.52 - 7.64 (m, 1H), 7.84 - 7.94 (m, 1H), 8.33 (s, 1H).

### Beispiel 372A

### rac-tert-Butyl-1-[({8-[(2,6-difluorbenzyl)oxy]-2,6-dimethylimidazo[1,2-a]pyridin-3-yl}carbonyl)amino]-3-azabicyclo[3.2.0]heptan-3-carboxylat

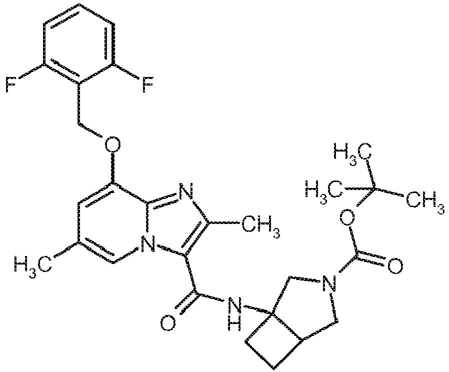

75 mg (0.23 mmol) 8-[(2,6-Difluorbenzyl)oxy]-2,6-dimethylimidazo[1,2-a]pyridin-3-carbonsäure aus Beispiel 21A wurden mit 94.4 mg (0.25 mmol) HATU und 0.20 ml (1.13 mmol) N,N-Diisopropylethylamin in 1.4 ml DMF vorgelegt, 20 min gerührt, anschließend mit 57.5 mg (0.27 mmol) *rac*-*tert*-Butyl-1-amino-3-azabicyclo[3.2.0]heptan-3-carboxylat (kommerziell erhältlich; CAS-Nr 1251009-41-2) versetzt und über Nacht bei RT gerührt. Die Reaktionslösung wurde mit Wasser versetzt, der ausgefallene Feststoff wurde 30 min bei RT verrührt, abfiltriert, mit Wasser gewaschen und im Hochvakuum getrocknet. Es wurden 97 mg der Zielverbindung (80% d. Th.) erhalten.
LC-MS (Methode 2): Rₜ = 1.05 min
MS (ESpos): m/z = 527 (M+H)⁺

### Beispiel 373A

### rac-tert-Butyl-3-[({8-[(2,6-difluorbenzyl)oxy]-2,6-dimethylimidazo[1,2-a]pyridin-3-yl}carbonyl)ammo]-4-fluorpyrrolidin-1-carboxylat Trifluoracetat

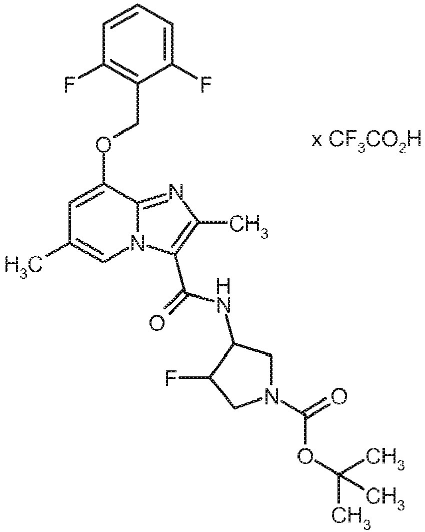

75 mg (0.23 mmol) 8-[(2,6-Difluorbenzyl)oxy]-2,6-dimethylimidazo[1,2-a]pyridin-3-carbonsäure aus Beispiel 21A wurden mit 112 mg (0.29 mmol) HATU und 0.32 ml (1.81 mmol) N,N-Diisopropylethylamin in 0.75 ml DMF vorgelegt, 10 min gerührt, anschließend bei RT mit 60 mg (0.29 mmol) *cis*-*rac*-*tert*-Butyl-3-amino-4-fluorpyrrolidin-1-carboxylat versetzt und 60 min bei RT gerührt. Die Reaktionslösung wurde mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereint, eingeengt und lyophylisiert. Es wurden 98 mg der Zielverbindung (68% d. Th.) erhalten.
LC-MS (Methode 2): Rₜ = 1.02 min
MS (ESpos): m/z = 519 (M-TFA+H)⁺
¹H-NMR (400 MHz, DMSO-d₆): δ = 2.41 (s, 3H), 2.53 (br. s., 3H), 3.27 - 3.37 (m, 1H), 3.55 - 3.62 (m, 4H), 4.59 - 4.77 (m, 1H), 5.17 - 5.35 (m, 1H), 5.39 (s, 2H), 7.21 - 7.30 (m, 2H), 7.32 - 7.49 (m, 1H), 7.57 - 7.67 (m,1H), 8.42 - 8.65 (m, 2H).

### Beispiel 374A

### Ethyl-8-[(2-fluor-6-methoxybenzyl)oxy]-2,6-dimethylimidazo[1,2-a]pyridin-3-carboxylat

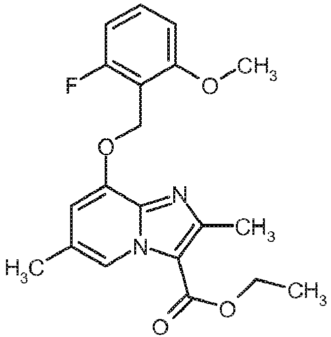

710 mg (3.03 mmol) Ethyl-8-hydroxy-2,6-dimethylimidazo[1,2-a]pyridin-3-carboxylat aus Beispiel 239A , 730 mg (3.33 mmol) 2-(Brommethyl)-1-fluor-3-methoxybenzol und 2.17 g (6.67 mmol) Cäsiumcarbonat wurden in 43 ml DMF für 30 min in einem vorgeheizten 60°C warmen Ölbad erhitzt. Das Reaktionsgemisch wurde auf Wasser gegossen, 60 min gerührt, der ausgefallene Feststoff abgesaugt, mit Wasser gewaschen und im Hochvakuum getrocknet. Es wurden 859 mg der Zielverbindung (72% d. Th., Reinheit ca.94%) erhalten.
LC-MS (Methode 2): Rₜ = 1.10 min
MS (ESpos): m/z = 373 (M+H)⁺

### Beispiel 375A

### 8-[(2-Fluor-6-methoxybenzyl)oxy]-2,6-dimethylimidazo[1,2-a]pyridin-3-carbonsäure

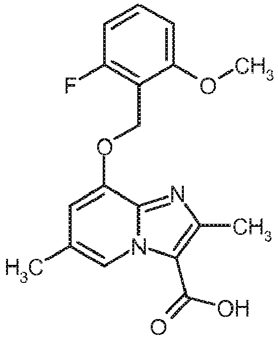

859 mg (2.17 mmol, 94%ig) Ethyl-8-[(2-fluor-6-methoxybenzyl)oxy]-2,6-dimethylimidazo[1,2-a]pyridin-3-carboxylat aus Beispiel 374A wurden in 46.8 ml THF/Methanol (5/1) gelöst, mit 10.8 ml (10.8 mmol) 1 N wässriger Lithiumhydroxidlösung versetzt und über Nacht bei RT gerührt. Die Reaktionslösung wurde mit 1 N wässriger Salzsäure angesäuert und das organische Lösungsmittel wurde abdestilliert. Der entstandene Niederschlag wurde abfiltriert, mit Wasser gewaschen und im Hochvakuum getrocknet. Es wurden 785 mg der Zielverbindung (98% d. Th., Reinheit ca. 94%) erhalten.
LC-MS (Methode 2): Rₜ = 0.77 min
MS (ESpos): m/z = 345 (M+H)⁺

### Beispiel 376A

### ent-Benzyl-{1-[({8-[(2,6-difluorbenzyl)oxy]imidazo[1,2-a]pyridin-3-yl}carbonyl)amino]-2-methylpentan-2-yl}carbamat Trifluoracetat

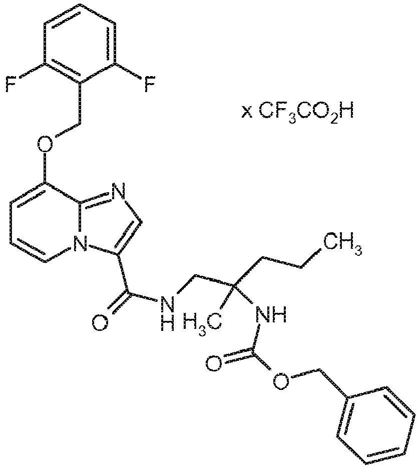

Zu 60 mg (0.20 mmol) 8-[(2,6-Difluorbenzyl)oxy]imidazo[1,2-a]pyridin-3-carbonsäure aus Beispiel 28A, 70 mg (0.22 mmol) TBTU und 0.11 ml (0.99 mmol) 4-Methylmorpholin in 0.66 ml DMF wurden 54 mg (0.22 mmol) *ent*-Benzyl-(1-amino-2-methylpentan-2-yl)carbamat aus Beispiel 289A gegeben und es wurde 1 h bei RT gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1 % TFA). Die Produktfraktionen wurden eingeengt und getrocknet. Es wurden 117 mg der Zielverbindung (91% d. Th.) erhalten.
LC-MS (Methode 2): Rₜ = 1.20 min
MS (ESpos): m/z = 537 (M+H)⁺

### Beispiel 377A

### Ethyl-8-[(2,6-difluor-3-methoxybenzyl)oxy]-2,6-dimethylimidazo[1,2-a]pyridin-3-carboxylat

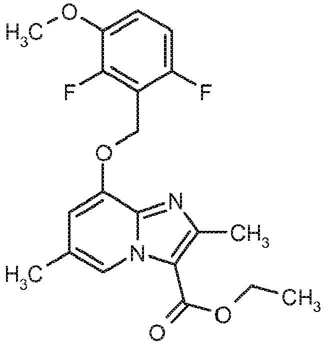

1.35 g (5.75 mmol) Ethyl-8-hydroxy-2,6-dimethylimidazo[1,2-a]pyridin-3-carboxylat aus Beispiel 239A und 4.12 g (12.66 mmol) Cäsiumcarbonat wurden in 82 ml DMF vorgelegt. Es wurde auf 60°C erhitzt, anschließend mit 1.50 g (6.33 mmol) 2-(Brommethyl)-1,3-difluor-4-methoxybenzol versetzt und 20 min bei 60°C gerührt. Das Reaktionsgemisch wurde auf ca. 500 ml Wasser gegossen und 30 min gerührt. Der entstandene Feststoff wurde abgesaugt, mit Wasser gewaschen und im Hochvakuum getrocknet. Es wurden 2.11 g der Titelverbindung (86 % d. Th., Reinheit 92%) erhalten.
LC-MS (Methode 2): Rₜ = 1.09 min
MS (ESpos): m/z = 391 (M+H)⁺
¹H-NMR (400 MHz, DMSO-d₆): δ [ppm] = 1.35 (t, 3H), 2.37 (s, 3H), 3.87 (s, 3H), 4.29 - 4.38 (m, 2H), 5.30 (s, 2H), 7.09 (s, 1H), 7.12 - 7.22 (m, 1H), 7.27 - 7.37 (m, 1H), 8.71 (s, 1H), [weiteres Signal unter Lösungsmittelpeak].

### Beispiel 378A

### 8-[(2,6-Difluor-3-methoxybenzyl)oxy]-2,6-dimethylimidazo[1,2-a]pyridin-3-carbonsäure

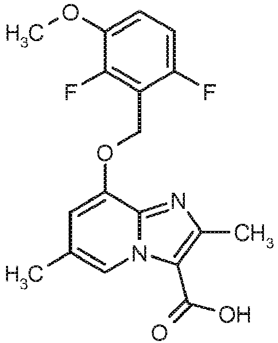

2.00 g (4.69 mmol) Ethyl-8-[(2,6-difluor-3-methoxybenzyl)oxy]-2,6-dimethylimidazo[1,2-a]pyridin-3-carboxylat aus Beispiel 377A wurden in 50 ml Dioxan suspendiert, mit 11.73 ml (23.46 mmol) 2 N wässriger Natriumhydroxidlösung versetzt und 5 h bei 90°C gerührt. Die Reaktionslösung wurde mit 1 N wässriger Salzsäure angesäuert und die wässrige Phase wurde dreimal mit Essigsäureethylester extrahiert. Die organische Phase wurde über Natriumsulfat getrocknet, filtriert und einrotiert. Hierbei erhielt man 790 mg der Titelverbindung. Die wässrige Phase wurde nochmals mit Essigsäureethylester für 1.5 h gerührt und die Phasen wurden getrennt. Die organische Phase wurde über Natriumsulfat getrocknet, filtriert und einrotiert. Hierbei erhielt man 70 mg der Titelverbindung. Die wässrige Phase wurde nochmals mit Dichlormethan für 2 h gerührt und die Phasen wurden getrennt. Die organische Phase wurde über Natriumsulfat getrocknet, filtriert und im Vakuum eingeengt. Hierbei erhielt man 60 mg der Titelverbindung. Die wässrige Phase wurde im Vakuum eingeengt und der Rückstand mittels präparativer HPLC gereinigt (RP18 Säule, Laufmittel: Acetonitril/Wasser-Gradient unter Zusatz von 0.1% TFA). Hierbei erhielt man 300 mg der Titelverbindung als Trifluoracetat-Salz. Es wurden insgesamt 920 mg der Titelverbindung (52 % d. Th.) erhalten (anteilig als Trifluoracetat-Salz).
LC-MS (Methode 2): Rₜ = 0.69 min
MS (ESpos): m/z = 363 (M+H)⁺
¹H-NMR (400 MHz, DMSO-d₆): δ [ppm] = 2.36 (s, 3H), 3.87 (s, 3H), 5.29 (s, 2H), 7.06 (s, 1H), 7.12 - 7.23 (m, 1H), 7.28 - 7.38 (m, 1H), 8.75 (s, 1H), 12.09 - 13.12 (br. s, 1H), [weiteres Signal unter Lösungsmittelpeak].

### Beispiel 379A

### 3-Cyclopropyl-2,6-difluorbenzaldehyd

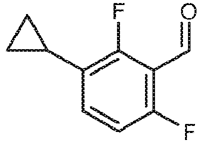

3.50 g (15.84 mmol) 3-Brom-2,6-difluorbenzaldehyd wurden in 87.5 ml Toluol gelöst. Eine Lösung von 3.36 g (31.67 mmol) Natriumcarbonat in 1.5 ml Wasser wurde hinzugegeben und das Gemisch wurde für 10 min bei RT gerührt. Anschließend wurden 2.04 g (23.75 mmol) Cyclopropylboronsäure und 366 mg (0.32 mmol) Tetrakis(triphenylphosphin)palladium(0) hinzugegeben und das Gemisch wurde über Nacht unter Rückfluss gerührt. Es wurden nochmals 0.68 g (7.92 mmol) Cyclopropylboronsäure, 0.34 g (3.17 mmol) Natriumcarbonat und 183 mg (0.16 mmol) Tetrakis(triphenylphosphin)palladium(0) hinzugegeben und das Gemisch wurde nochmals über Nacht unter Rückfluss gerührt. Das Reaktionsgemisch wurde mit Essigsäureethylester verdünnt und extrahiert. Die wässrige Phase wurde zweimal mit Essigsäureethylester gewaschen. Die vereinigten organischen Phasen wurden über Natriumsulfat getrocknet, filtriert und bei 35°C Badtemperatur im Vakuum eingeengt. Es wurden 3.50 g der Titelverbindung (92 % d. Th., Reinheit 76%) erhalten.
LC-MS (Methode 13): Rₜ = 2.11 min
MS (ESpos): m/z = 183 (M+H)⁺

### Beispiel 380A

### (3-Cyclopropyl-2,6-difluorphenyl)methanol

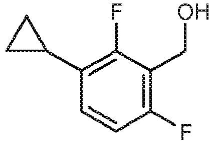

Unter Argon wurden 221 mg (5.84 mmol) Natriumborhydrid bei 0°C in 47 ml Tetrahydrofuran vorgelegt. Eine Lösung von 3.5 g (14.60 mmol) 3-Cyclopropyl-2,6-difluorbenzaldehyd aus Beispiel 379A in 189 ml Tetrahydrofuran wurden hinzugegeben. Anschließend wurden 14.8 ml Methanol bei 0°C zugetropft und 2 h bei Raumtemperatur gerührt. Die Reaktionslösung wurde auf ca. 88 ml Eiswasser gegeben, mit 2 N wässriger Schwefelsäure auf ca. pH = 1 gestellt und die Mischung dreimal mit Dichlormethan extrahiert. Die vereinten organischen Phasen wurden über Natriumsulfat getrocknet, filtriert und am Rotationsverdampfer bei 30°C Badtemperatur bis zur Trockne eingeengt. Der Rückstand wurde in wenig Dichlormethan/Methanol aufgenommen und mittels Kieselgelchromatographie gereinigt (Laufmittel: Cyclohexan/Essigsäureethylester-Gradient = 10/1 nach Cyclohexan/Essigester 5/1). Die Produktfraktionen vereinigt und bei 30°C Badtemperatur einrotiert. Es wurden 2.46 g der Titelverbindung (86 % d. Th., Reinheit 94%) erhalten.
LC-MS (Methode 13): Rₜ = 1.90 min
MS (ESpos): m/z = 167 (M-H₂O+H)⁺

### Beispiel 381A

### Ethyl-8-[(3-cyclopropyl-2,6-difluorbenzyl)oxy]-2,6-dimethylimidazo[1,2-a]pyridin-3-carboxylat Trifluoracetat

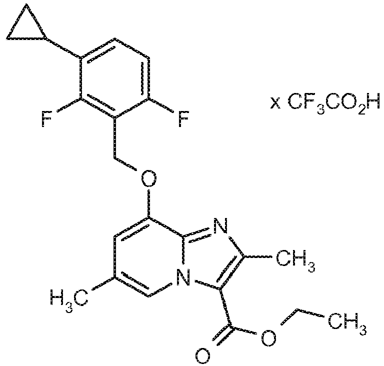

2.67 g (11.41 mmol) Ethyl-8-hydroxy-2,6-dimethylimidazo[1,2-a]pyridin-3-carboxylat aus Beispiel 239A wurden in 104 ml THF gelöst. 2.46 g (12.55 mmol) (3-Cyclopropyl-2,6-difluorphenyl)methanol aus Beispiel 380A und 6.29 g (23.97 mmol) Triphenylphosphin wurden hinzugegeben. Nach Zugabe von 4.75 ml (23.97 mmol) Azodicarbonsäurediisopropylester (DIAD) wurde das Reaktionsgemisch über Nacht bei RT gerührt. Das Gemisch wurde eingedampft und mittels Kieselgelchromatographie gereinigt (Laufmittel: Cyclohexan/Essigsäureethylester-Gradient = 10/1 nach 5/1). Die Produktfraktionen wurden eingedampft und nochmals mittels präparativer HPLC gereinigt (RP18 Säule, Laufmittel: Acetonitril/Wasser-Gradient unter Zusatz von 0.1% TFA). Es wurden 1.1 g der Titelverbindung (19 % d. Th.) erhalten.
LC-MS (Methode 2): Rₜ = 1.23 min
MS (ESpos): m/z = 401 (M-TFA+H)^{+ 1}H-NMR (400 MHz, DMSO-d₆): δ [ppm] = 0.70 - 0.78 (m, 2H), 0.95 - 1.03 (m, 2H), 1.36 (t, 3H), 2.00 - 2.13 (m, 1H), 2.40 (s, 3H), 4.33 - 4.40 (m, 2H), 5.32 (s, 2H), 7.08 - 7.28 (m, 3H), 8.75 (s, 1H), [weiteres Signal unter Lösungsmittelpeak].

### Beispiel 382A

### 8-[(3-Cyclopropyl-2,6-difluorbenzyl)oxy]-2,6-dimethylimidazo[1,2-a]pyridin-3-carbonsäure Trifluoracetat

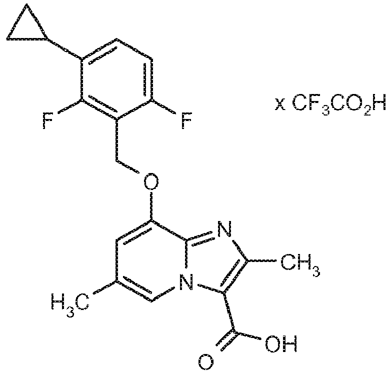

1.1 g (2.14 mmol) Ethyl-8-[(3-cyclopropyl-2,6-difluorbenzyl)oxy]-2,6-dimethylimidazo[1,2-a]pyridin-3-carboxylat Trifluoracetat aus Beispiel 381A wurden in 46 ml Dioxan suspendiert, mit 6.4 ml (12.8 mmol) 2 N wässriger Natriumhydroxidlösung versetzt und über Nacht bei 90°C gerührt. Das Gemisch wurde eingedampft und der Rückstand mit TFA/Wasser/Acetonitril versetzt. Der entstandene Feststoff wurde abfiltriert und mit wenig Wasser gewaschen. Das produkthaltige Filtrat wurde etwas eingeengt und mittels präparativer HPLC gereinigt (RP18 Säule, Laufmittel: Acetonitril/Wasser-Gradient unter Zusatz von 0.1% TFA). Die entsprechenden produkthaltigen Fraktionen wurden mit dem abfiltrierten Feststoff vereinigt und eingeengt. Es wurden 950 mg der Titelverbindung (91 % d. Th.) erhalten.
LC-MS (Methode 2): Rₜ = 0.87 min
MS (ESpos): m/z = 373 (M-TFA+H)⁺

### Beisipiel 383A

### ent-Benzyl-{1-[({8-[(3-cyclopropyl-2,6-difluorbenzyl)oxy]-2,6-dimethylimidazo[1,2-a]pyridin-3-yl}carbonyl)amino]-2-methylpentan-2-yl}carbamat Trifluoracetat

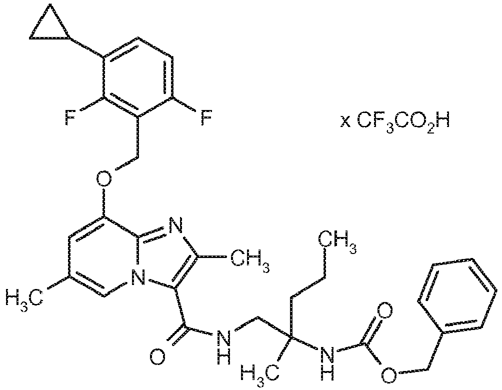

150 mg (0.31 mmol) 8-[(3-Cyclopropyl-2,6-difluorbenzyl)oxy]-2,6-dimethylimidazo[1,2-a]pyridin-3-carbonsäure Trifluoracetat aus Beispiel 382A wurden mit 129 mg (0.34 mmol) HATU und 0.22 ml (1.23 mmol) N,N-Diisopropylethylamin in 2 ml DMF vorgelegt, 20 min gerührt, anschließend bei RT mit 89 mg (0.36 mmol) *ent*-Benzyl-(1-amino-2-methylpentan-2-yl)carbamat (Enantiomer B) aus Beispiel 289A versetzt und 4.5 h bei RT gerührt. Die Reaktionslösung wurde mit etwas Wasser versetzt und der entstandene Feststoff wurde abfiltriert. Der Feststoff wurde in TFA/Wasser/Acetonitril gelöst und anschließend mittels präparativer HPLC gereinigt (RP18 Säule, Laufmittel: Acetonitril/Wasser-Gradient unter Zusatz von 0.1% TFA). Es wurden 159 mg der Titelverbindung (69 % d. Th.) erhalten.
LC-MS (Methode 2): Rₜ = 1.22 min
MS (ESpos): m/z = 605 (M-TFA+H)⁺

In Analogie zu Beispiel 383A wurden die in Tabelle 15A gezeigten Beispiele hergestellt, indem die entsprechende Carbonsäure mit den entsprechenden Aminen [*ent*-Benzyl-(1-amino-2-methylpentan-2-yl)carbamat (Enantiomer B) aus Beispiel 289A; *ent*-Benzyl-(1-amino-2-methylbutan-2-yl)carbamat (Enantiomer A) aus Beispiel 274A; *ent*-Benzyl-(1-amino-3-fluor-2-methylpropan-2-yl)carbamat (Enantiomer B) aus Beispiel 301A] unter den in der repräsentativen Arbeitsvorschrift 2 beschriebenen Reaktionsbedingungen umgesetzt wurde.

**Tabelle 15A:**

| **Beispiel** | **IUPAC-Name / Struktur (Ausbeute)** | **Analytische Daten** |
|---|---|---|
| **384A** | *ent*-Benzyl-{1-[({8-[(2,6-difluor-3-methoxybenzyl)oxy]-2,6-dimethylimidazo[1,2-a]pyridin-3-yl}carbonyl)amino]-2-methylbutan-2-yl}carbamat Trifluoracetat | LC-MS (Methode 2): Rₜ = 1.08 min |
| | | MS (ESpos): m/z = 581 (M-TFA+H)⁺ |
| | 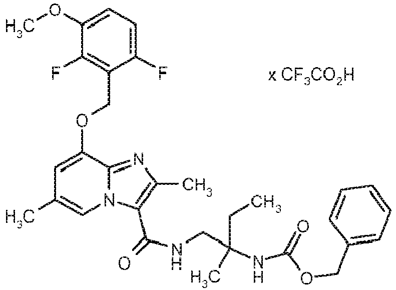 | |
| | (42% d. Th.) | |
| **385A** | *ent*-Benzyl-{1-[({8-[(2,6-difluor-3-methoxybenzyl)oxy]-2,6-dimethylimidazo[1,2-a]pyridin-3-yl}carbonyl)amino]-3-fluor-2-methylpropan-2-yl}carbamat Trifluoracetat | LC-MS (Methode 2): Rₜ =1.04 min |
| | | MS (ESpos): m/z = 585 (M-TFA+H)⁺ |
| | 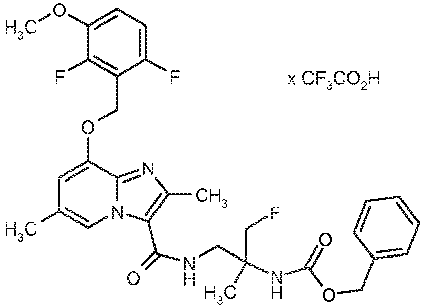 | |
| | (42% d. Th., Reinheit 94%) | |
| **386A** | *ent*-Benzyl-{1-[({8-[(2,6-difluor-3-methoxybenzyl)oxy]-2,6-dimethylimidazo[1,2-a]pyridin-3-yl}carbonyl)amino]-2-methylpentan-2-yl}carbamat Trifluoracetat | LC-MS (Methode 2): Rₜ =1.11 min |
| | | MS (ESpos): m/z = 595 (M-TFA+H)⁺ |
| | 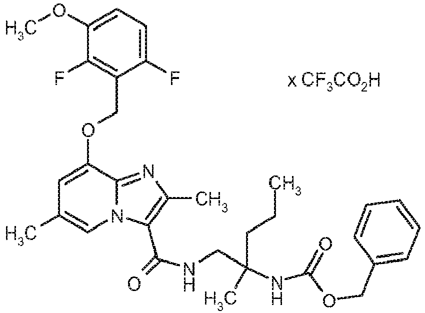 | |
| | (69% d. Th.) | |
| **387A** | *ent*-Benzyl-{1-[({8-[(3-cyclopropyl-2,6-difluorbenzyl)oxy]-2,6-dimethylimidazo[1,2-a]pyridin-3-yl}carbonyl)amino]-3-fluor-2-methylpropan-2-yl}carbamat Trifluoracetat | LC-MS (Methode 2): Rₜ = 1.14 min |
| | | MS (ESpos): m/z = 595 (M-TFA+H)⁺ |
| | 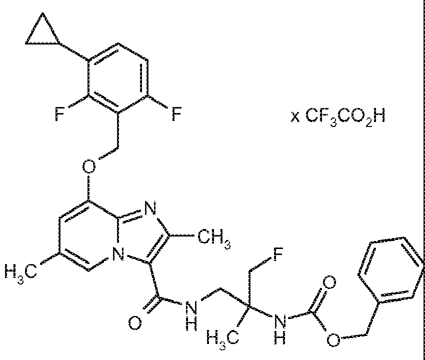 | |
| | (64% d. Th.) | |
| **388A** | *ent*-Benzyl-{1-[({8-[(3-cyclopropyl-2,6-difluorbenzyl)oxy]-2,6-dimethylimidazo[1,2-a]pyridin-3-yl}carbanyl)amino]-2-methylbutan-2-yl}carbamat Trifluoracetat | LC-MS (Methode 2): Rₜ = 1.17 min |
| | | MS (ESpos): m/z = 591 (M-TFA+H)⁺ |
| | 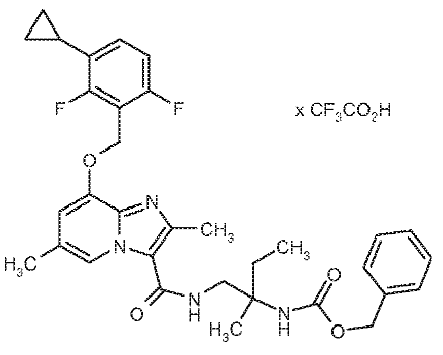 | |
| | (77% d. Th.) | |

### Beispiel 389A

### rac-tert.-Butyl-(2-cyanpentan-2-yl)carbamat

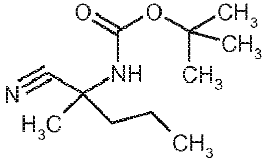

64.2 g (294.2 mmol) Di-tert.-butyldicarbonat wurden in einem Reaktionskolben vorgelegt und sehr langsam so mit 30.0 g (267.4 mmol) *rac*-2-Amino-2-methylpentanonitril (beschrieben in: Deng, S L. et al., Synthesis 2001, 2445-2449; Freifelder, M. et al., J. Am. Chem. Soc. 1960, 696-698) versetzt, dass die Innentemperatur bei 30°C nicht überstieg. Das Gemisch wurde über Nacht bei RT gerührt. Dann wurde mit Dichlormethan versetzt und zweimal mit 1 N wässriger Natronlauge gewaschen. Die organische Phase wurde über Natriumsulfat getrocknet, filtriert und bei 30°C Badtemperatur eingedampft. Es wurden 76.33 g (quantitative Ausbeute; tert.-Butanol ist im ¹H-NMR erkennbar) der Zielverbindung erhalten.
LC-MS (Methode 15): Rₜ = 2.39 min
MS (ESpos): m/z = 213 (M+H)⁺
¹H-NMR (400 MHz, DMSO-d₆): δ = 0.90 (t, 3H), 1.30 - 1.44 (m, 11H), 1.47 (s, 3H), 1.65 - 1.86 (m, 2H), 7.48 (br. s, 1H).

### Beispiel 390A

### rac-tert.-Butyl-(1-amino-2-methylpentan-2-yl)carbamat

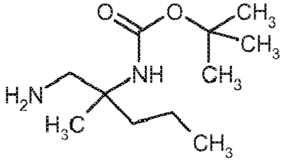

13.50 (47.13 mmol; Anteile von tert.-Butanol vorhanden) *rac*-tert.-Butyl-(2-cyanpentan-2-yl)carbamat aus Beispiel 389A wurden in 137 ml 7 N Ammoniak-Lösung in Methanol gelöst und unter Argon mit 14.6 g Raney-Nickel (50%ige wässrige Aufschlämmung) versetzt. Das Reaktionsgemisch wurde über Nacht im Autoklaven bei 20-30 bar hydriert. Die Reaktionsmischung wurde über Celite abfiltriert, mit Methanol gespült und eingeengt. Es wurden 18.50 g der Zielverbindung erhalten, welche ohne weitere Reinigung in die Folgestufe eingesetzt wurde.
LC-MS (Methode 15): Rₜ = 1.96 min
MS (ESpos): m/z = 217 (M+H)⁺

### Beispiel 391A

### rac-3-{3,4-Difluorphenoxy)-2-methylpropan-1,2-diamin

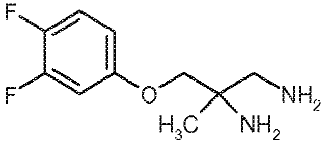

300 mg (1.41 mmol) *rac*-2-Amino-3-(3,4-difluorphenoxy)-2-methylpropanonitril wurden in 14.4 ml abs. THF vorgelegt und unter Argon bei 0°C mit 0.92 ml (0.92 mmol) einer 1 N Lithiumaluminiumhydrid-Lösung in Diethylether versetzt. Die Reaktionslösung wurde 30 min bei 0°C gerührt, dann ließ man langsam auf Raumtemperatur erwärmen und rührte über Nacht. Das Reaktionsgemisch wurde vorsichtig mit 140 µl Wasser, 140 µl 2 N wässriger Natriumhydroxidlösung und 280 µl Wasser versetzt, der Niederschlag wurde abfiltriert, mit THF und Methanol gewaschen, das Filtrat eingeengt und der Rückstand mittels KieselgelChromatographie gereinigt (Laufmittel: Dichlormethan/2N Ammoniak in Methanol = 20/1). Es wurden 87 mg der Zielverbindung (24% d. Th.; Reinheit ca. 84%) erhalten.
LC-MS (Methode 15): Rₜ = 1.73 min
MS (ESpos): m/z = 217 (M+H)⁺

### Beispiel 392A

### Ethyl-8-(benzyloxy)-2-methyl-6-vinylimidazo[1,2-a]pyridin-3-carboxylat

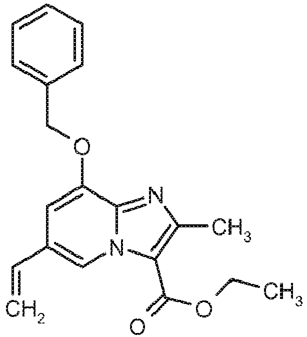

6.32 g (16.23 mmol) Ethyl-8-(benzyloxy)-6-brom-2-methylimidazo[1,2-a]pyridin-3-carboxylat aus Beispiel 237A, 6.52 g (48.69 mmol) Kaliumvinyltrifluoroborat, 8.21 g (81.15 mmol) Triethylamin und 2.68 g (3.28 mmol) 1,1'-Bis(diphenylphosphino)ferrocenpalladium(II)chlorid-Dichlormethan-Komplex wurden in 120 ml 2-Propanol vorgelegt und 1 Stunde bei 90°C gerührt. Das Reaktionsgemisch wurde mit Essigsäureethylester und Wasser versetzt, über Celite filtriert und mit Essigsäureethylester nachgewaschen. Die organische Phase wurde zweimal mit Wasser und einmal mit gesättigter, wässriger Natriumchlorid-Lösung gewaschen. Die organische Phase wurde über Natriumsulfat getrocknet, filtriert, eingeengt und im Hochvakuum getrocknet. Das isolierte Rohprodukt wurde direkt ohne weitere Reinigung weiter umgesetzt.
LC-MS (Methode 2): Rₜ = 1.17 min
MS (ESpos): m/z = 337 (M+H)⁺

### Beispiel 393A

### Ethyl-8-(benzyloxy)-6-formyl-2-methylimidazo[1,2-a]pyridin-3-carboxylat

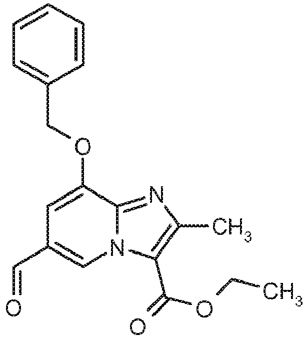

Das Rohprodukt Ethyl-8-(benzyloxy)-2-methyl-6-vinylimidazo[1,2-a]pyridin-3-carboxylat aus Beispiel 392A wurde in 200 ml Tetrahydrofuran/Wasser (1:1) vorgelegt, mit 10.42 g (1.64 mmol) Osmium(VIII)-oxid und 10.52 g (49.18 mmol) Natriumperiodat versetzt. Das Reaktionsgemisch wurde 1 Stunde bei Raumtemperatur gerührt. Dann wurde mit Essigsäureethylester versetzt, dreimal mit Wasser und einmal mit gesättigter, wässriger Natriumchlorid-Lösung gewaschen. Die organische Phase wurde über Natriumsulfat getrocknet, filtriert und eingeengt. Der Rückstand wurde mittels Kieselgel gereinigt (Laufmittel: Cyclohexan/Essigsäureethylester-Gradient; 80% Cyclohexan bis 10% Cyclohexan). Es wurden 4.05 g der Titelverbindung (74% d. Th., über zwei Stufen) erhalten.
LC-MS (Methode 2): Rₜ = 1.11 min
MS (ESpos): m/z = 339 (M+H)⁺
¹H-NMR (400 MHz, DMSO-d₆): δ [ppm] = 1.39 (t, 3H), 2.63 (s, 3H), 4.37 - 4.44 (m, 2H), 5.38 (s, 2H), 7.28 (d, 1H), 7.35 - 7.48 (m, 3H), 7.49 - 7.55 (m, 2H), 9.51 (s, 1H), 10.03 (s, 1H).

### Beispiel 394A

### Ethyl-8-(benzyloxy)-6-(hydroxymethyl)-2-methylimidazo[1,2-a]pyridin-3-carboxylat

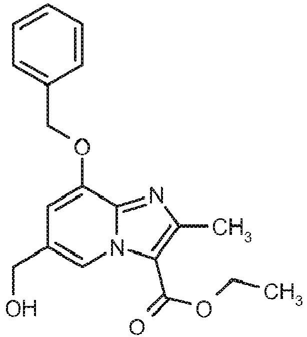

1.65 g (4.88 mmol) Ethyl-8-(benzyloxy)-6-formyl-2-methylimidazo[1,2-a]pyridin-3-carboxylat aus Beispiel 393A wurden unter Argon in 66 ml abs. Ethanol suspensiert. Es wurden 92 mg (2.44 mmol) Natriumborhydrid zu dem Reaktionsgemisch gegeben und 15 min bei Raumtemperatur gerührt. Die Reaktionslösung wurde vom Ethanol befreit und der Rückstand mit Wasser versetzt. Die wässrige Phase wurde dreimal mit Essigsäureethylester extrahiert. Die vereinigten organischen Phasen wurden mit gesättigter, wässriger Natriumchlorid-Lösung versetzt, über Celite abfiltriert und mit Essigsäureethylester nachgewaschen. Die beiden Phasen wurden voneinander getrennt. Die organische Phase wurde über Natriumsulfat getrocknet, filtriert, eingeengt und im Hochvakuum getrocknet. Es wurden 1.49 g der Titelverbindung (90% d. Th.) erhalten.
LC-MS (Methode 2): Rₜ = 0.87 min
MS (ESpos): m/z = 341 (M+H)⁺
¹H-NMR (400 MHz, DMSO-d₆): δ [ppm] = 1.36 (t, 3H), 4.32 - 4.39 (m, 2H), 4.55 (d, 2H), 5.29 (s, 2H), 5.45 (t,1H), 7.04 (s, 1H), 7.32 - 7.48 (m, 3H), 7.48 - 7.57 (m, 2H), 8.83 - 5.86 (m, 1H), [weiteres Signal unter Lösungsmittelpeak verborgen].

### Beispiel 395A

### Ethyl-8-hydroxy-6-(hydroxymethyl)-2-methylimidazo[1,2,a]pyridin-3-carboxylat

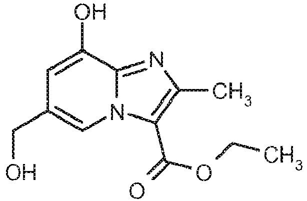

1.31g (3.85 mmol) Ethyl-8-(benzyloxy)-6-(hydroxymethyl)-2-methylimidazo[1,2-a]pyridin-3-carboxylat aus Beispiel 394A wurden in 99 ml Ethanol vorgelegt. Unter Argon wurden 820 mg (0.77 mmol) 10%iges Palladium auf Aktivkohle zu der Reaktionslösung gegeben und es wurde 3 Stunden unter Normaldruck hydriert. Das Reaktionsgemisch wurde über einen Millipore-Filter abfiltriert, mit Ethanol nachgewaschen und das Filtrat eingedampft. Es wurden 845 mg der Titelverbindung (82% d. Th., Reinheit 94%) erhalten.
LC-MS (Methode 2): Rₜ = 0.51 min
MS (ESpos): m/z = 251 (M+H)⁺

### Beispiel 396A

### Ethyl-8-[(2,6-difluorbenzyl)oxy]-6-(hydroxymethyl)-2-methylimidazo[1,2-a]pyridin-3-carboxylat

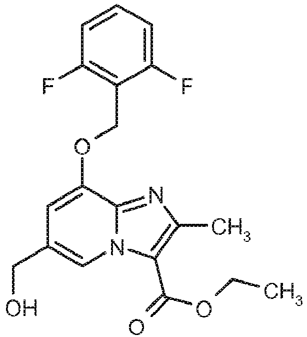

845 mg (3.38 mmol) Ethyl-8-hydroxy-6-(hydroxymethyl)-2-methylimidazo[1,2-a]pyridin-3-carboxylat aus Beispiel 395A wurden in 48.4 ml abs. DMF gelöst, mit 2.42 g (7.43 mmol) Cäsiumcarbonat und 699 mg (3.38 mmol) 2,6-Difluorbenzylbromid versetzt und 1 Stunde bei Raumtemperatur gerührt. Das Reaktionsgemisch wurde auf 500 ml Wasser gegossen und 30 min bei Raumtemperatur gerührt. Der entstandene Feststoff wurde abfiltriert und mit Wasser gewaschen. Es wurden 1.14 g der Titelverbindung (87% d. Th.) erhalten.
LC-MS (Methode 2): Rₜ = 0.87 min
MS (ESpos): m/z = 377 (M+H)⁺
¹H-NMR (400 MHz, DMSO-d₆): δ [ppm] = 1.36 (t, 3H), 4.32 - 4.40 (m, 2H), 4.58 (d, 2H), 5.31 (s, 2H), 5.47 (t,1H), 7.14 (s, 1H), 7.18 - 7.31 (m, 2H), 7.45 - 7.71 (m, 1H), 8.86 - 8.89 (m, 1H), [weiteres Signal unter Lösungsmittelpeak verborgen].

### Beispiel 397A

### 8-[(2,6-Difluorbenzyl)oxy]-6-(hydroxymethyl)-2-methylimidazo[1,2-a]pyridin-3-carbonsäure Trifluoracetat

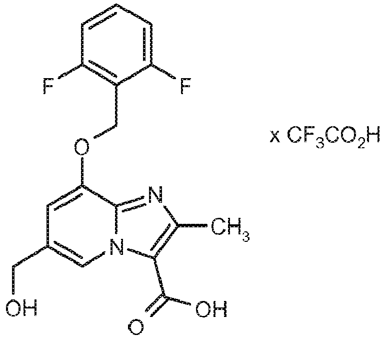

1.08 g (2.78 mmol) Ethyl-8-[(2,6-difluorbenzyl)oxy]-6-(hydroxymethyl)-2-methylimidazo[1,2-a]pyridin-3-carboxylat aus Beispiel 396A wurde in 60.3 ml Dioxan suspendiert und mit 8.35 ml (16.7 mmol) einer 2 N wässriger Natronlauge versetzt. Die Reaktionslösung wurde über Nacht bei 90°C gerührt. Die Reaktionslösung wurde mit 1 N wässriger Salzsäure angesäuert und anschließend wurde das Lösungsmittel abgedampft. Der Rückstand wurde mit Wasser verrührt und der Feststoff wurde abfiltriert. Die wässrige Phase wurde eingeengt und in Acetonitril/Wasser/TFA aufgenommen. Der entstandene produkthaltige Feststoff wurde abfiltriert. Hierbei wurden 1.19 g der Titelverbindung erhalten. Das Filtrat wurde mittels präparativer HPLC gereinigt (RP18 Säule; Laufmittel: Acetonitril/Wasser-Gradient unter Zusatz von 0.1% TFA). Hierbei wurden nochmals 0.08 g der Titelverbindung erhalten. In Summe wurden 1.27 g der Titelverbindung (96% d. Th.) erhalten.
LC-MS (Methode 2): Rₜ = 0.58 min
MS (ESpos): m/z = 349 (M-TFA+H)⁺

### Beispiel 398A

### ent-Benzyl-{1-[({8-[(2,6-difluorbenzyl)oxy]-6-(hydroxymethyl)-2-methylimidazo[1,2,a]pyridin-3-yl}carbonyl)amino]-2-methylpentan-2-yl}carbamat

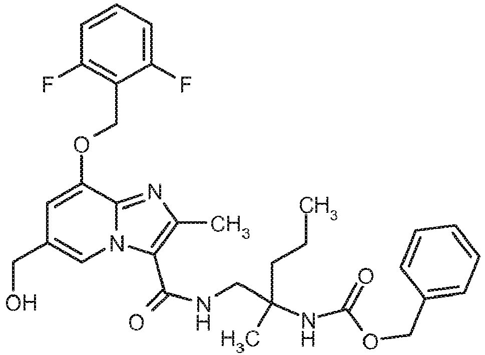

180 mg (0.38 mmol) 8-[(2,6-Difluorbenzyl)oxy]-6-(hydroxymethyl)-2-methylimidazo[1,2-a]pyridin-3-carbonsäure Trifluoracetat aus Beispiel 397A wurden mit 158 mg (0.42 mmol) HATU und 0.26 ml (1.51 mmol) N,N-DÜsopropylethylamin in 3.2 ml DMF vorgelegt, 20 min gerührt, anschließend bei Raumtemperatur mit 109 mg (0.43 mmol) *ent*-Benzyl-(1-amino-2-methylpentan-2-yl)carbamat (Enantiomer B) aus Beispiel 289A versetzt und 1 h bei Raumtemperatur gerührt. Die Reaktionslösung wurde mit Wasser versetzt, der Feststoff 30 min bei Raumtemperatur verrührt, abfiltriert und mit Wasser gewaschen. Es wurden 199 mg der Titelverbindung (91% d. Th.) erhalten.
LC-MS (Methode 2): Rₜ = 0.99 min
MS (ESpos): m/z = 581 (M+H)⁺

In Analogie zu Beispiel 398A wurden die in Tabelle 16A gezeigten Beispielverbindungen hergestellt, indem 8-[(2,6-Difluorbenzyl)oxy]-6-(hydroxymethyl)-2-methylimidazo[1,2-a]pyridin-3-carbonsäure Trifluoracetat aus Beispiel 397A mit den entsprechenden zuvor beschriebenen Aminen 274A und 301A unter den beschriebenen Bedingungen umgesetzt wurden:

**Tabelle 16A:**

| **Beispiel** | **IUPAC**-**Name / Struktur (Ausbeute)** | **Analytische Daten** |
|---|---|---|
| **399A** | *ent*-Benzyl-{1-[({8-[(2,6-difluorbenzyl)oxy]-6-(hydroxymethyl)-2-methylimidazo[1,2-a]pyridin-3-yl}carbonyl)amino]-2-methylbutan-2-yl}carbamat | LC-MS (Methode 2): Rₜ = 0.94 min |
| | | MS (ESpos): m/z = 567 (M+H)⁺ |
| | 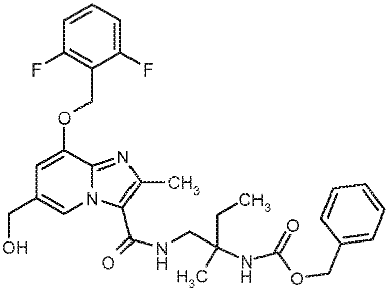 | |
| | (89% d. Th.) | |
| **400A** | *ent*-Benzyl-{1-[({8-[(2,6-difluorbenzyl)oxy]-6-(hydroxymethyl)-2-methylimidazo[1,2-a]pyridin-3-yl}carbonyl)amino]-3-fluor-2-methylpropan-2-yl}carbamat | LC-MS (Methode 2): Rₜ = 0.91 min |
| | | MS (ESpos): m/z = 571 (M+H)⁺ |
| | 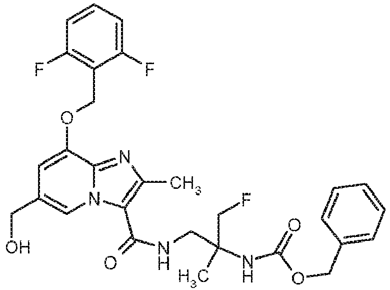 | |
| | (75% d. Th., Reinheit 91%) | |

### Beispiel 401A

### ent-Benzyl-{1-[({8-[(2,6-difluorbenzyl)oxy]-6-(fluormethyl)-2-methylimidazo[1,2,a]pyridin-3-yl}carbonyl)amino]-2-methylpentan-2-yl}carbamat Trifluoracetat

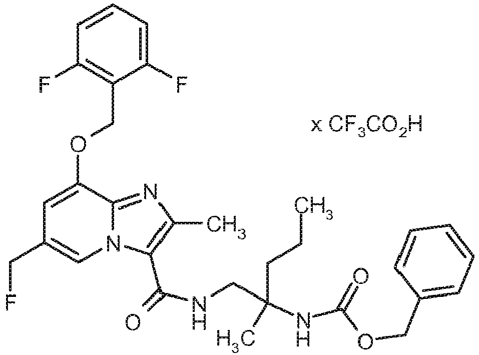

198 mg (0.34 mmol) *ent*-Benzyl-{1-[({8-[(2,6-difluorbenzyl)oxy]-6-(hydroxymethyl)-2-methylimidazo[1,2-a]pyridin-3-yl}carbonyl)amino]-2-methylpentan-2-yl}carbamat aus Beispiel 398A wurden in 3.4 ml Dichlormethan vorgelegt, bei -78°C mit 82.5 mg (0.51 mmol) Diethylaminoschwefeltrifluorid versetzt, 90 min bei -78°C und anschließend 30 min bei Raumtemperatur gerührt. Während der Reaktionszeit bei -78°C wurden 27.5 mg (0.17 mmol) Diethylaminoschwefeltrifluorid nachgegeben. Die Reaktionslösung wurde anschließend mit Dichlormethan verdünnt und zweimal mit gesättigter, wässriger Natriumhydrogencarbonat-Lösung und einmal mit gesättigter, wässriger Natriumchlorid-Lösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Das Rohprodukt wurde mittels präparativer HPLC gereinigt (RP18 Säule, Laufmittel: Acetonitril/Wasser-Gradient unter Zusatz von 0.1% TFA). Es wurden 191 mg der Titelverbindung (70% d. Th., Reinheit 87%) erhalten.
LC-MS (Methode 2): Rₜ = 1.19 min
MS (ESpos): m/z = 583 (M-TFA+H)⁺

In Analogie zu Beispiel 401A wurden die in Tabelle 17A gezeigten Beispielverbindungen hergestellt, indem die zuvor beschriebenen Alkohole mit Diethylaminoschwefeltrifluorid unter den beschriebenen Bedingungen umgesetzt wurden:

**Tabelle 17A:**

| **Beispiel** | **IUPAC-Name / Struktur (Ausbeute)** | **Analytische Daten** |
|---|---|---|
| **402A** | *ent*-Benzyl-{1-[({8-[(2,6-difluorbenzyl)oxy]-6-(fluormethyl)-2-methylimidazo[1,2,a]pyridin-3-yl)carbonyl)amino]-2-methylbutan-2-yl}carbamat Trifluoracetat | LC-MS (Methode 2): Rₜ = 1.13 min |
| | | MS (ESpos): m/z = 569 (M-TFA+H)⁺ |
| | 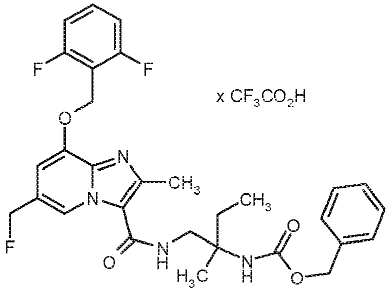 | |
| | (52% d. Th., Reinheit 70%) | |
| **403A** | *ent*-Benzyl-{1-[({8-[(2,6-difluorbenzyl)oxy]-6-(fluormethyl)-2-methylimidazo[1,2-a]pyridin-3-yl}carbonyl)amino]-3-fluor-2-methylpropan-2-yl}carbamat Trifluoracetat | LC-MS (Methode 2): Rₜ = 1.12 min |
| | | MS (ESpos): m/z = 573 (M-TFA+H)⁺ |
| | 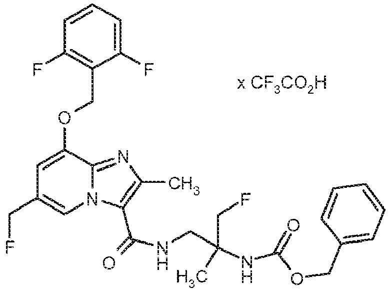 | |
| | (44% d. Th., Reinheit 73%) | |

### Beispiel 404A

### rac-2-Amino-5,5,5-trifluor-2-methylpentanonitril

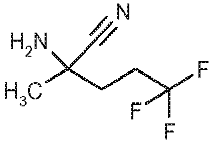

8.0 g (57.1 mmol) 5,5,5-Trifluorpentan-2-on [CAS Registry Nummer: 1341078-97-4; käuflich erhältlich oder das Methylketon kann nach literaturbekannten Methoden, welche dem Fachmann bekannt sind z. B. über a) zwei Stufen aus 4,4,4-Trifluorobutanal nach Y. Bai et al. Angewandte Chemie 2012, 51, 4112-4116; K. Hiroi et al. Synlett 2001, 263-265; K. Mikami et al. 1982 Chemistry Letters, 1349-1352; b) oder aus 4,4,4-Trifluorbutansäure nach A. A. Wube et al. Bioorganic and Medicinal Chemistry 2011, 19, 567-579; G. M. Rubottom et al. Journal of Organic Chemistry 1983, 48, 1550-1552; T. Chen et al. Journal of Organic Chemistry 1996, 61, 4716-4719, hergestellt werden. Die Isolierung des Produktes kann durch Destillation oder Chromatographie erfolgen] wurden in 47.8 ml 2 N Ammoniak in Methanol vorgelegt und bei Raumtemperatur mit 3.69 g (75.4 mmol) Natriumcyanid sowie 4.03 g (75.4 mmol) Ammoniumchlorid versetzt und 4 Stunden unter Rückfluss gerührt. Das Reaktionsgemisch wurde abgekühlt, mit Diethylether versetzt und der enthaltene Feststoff wurde abfiltriert. Das Lösungsmittel aus dem Filtrat wurde unter Normaldruck abdestilliert. Es wurden 8.7 g der Titelverbindung (92 % d. Th.) als Rückstand erhalten, der ohne weitere Reinigung in die Folgestufe eingesetzt wurde.
GC-MS (Methode 14): Rₜ = 1.90 min
MS (ESpos): m/z = 151 (M-CH₃)⁺

### Beispiel 405A

### rac-Benzyl-(2-cyan-5,5,5-trifluorpentan-2-yl)carbamat

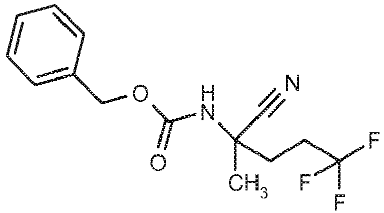

8.7 g (52.36 mmol) *rac*-2-Amino-5,5,5-trifluor-2-methylpentanonitril aus Beispiel 404A wurden in 128 ml Tetrahydrofuran/Wasser = 9/1 vorgelegt und mit 22.43 g (162.3 mmol) Kaliumcarbonat versetzt. Bei 0°C wurden langsam 8.93 g (52.36 mmol) Chlorameisensäurebenzylester zugetropft. Dann ließ man unter Rühren langsam auf Raumtemperatur erwärmen und rührte über Nacht bei Raumtemperatur. Das überstehende Lösungsmittel wurde abdekantiert, der Rückstand zweimal mit je 100 ml Tetrahydrofuran verrührt wonach das überstehende Lösungsmittel jeweils abdekantiert wurde. Die vereinigten organischen Phasen wurde eingeengt und das Rohprodukt wurde mittels Kieselgelchromatographie gereinigt (Laufmittel: Cyclohexan/Essigsäureethylester-Gradient 9/1 bis 4/1). Es wurden 11.14 g der Titelverbindung (68 % d. Th.) erhalten.
LC-MS (Methode 2): Rₜ = 1.01 min
MS (ESpos): m/z = 301 (M+H)⁺
¹H-NMR (400 MHz, DMSO-d₆): δ [ppm] = 1.58 (s, 3H), 2.08 - 2.21 (m, 2H), 2.24 - 2.52 (m, 2H), 5.09 (s, 2H), 7.29 - 7.41 (m, 5H), 8.17 (br. s, 1H).

### Beispiel 406A

### ent-Benzyl-(2-cyan-5,5,5-trifluorpentan-2-yl)carbamat (Enantiomer A)

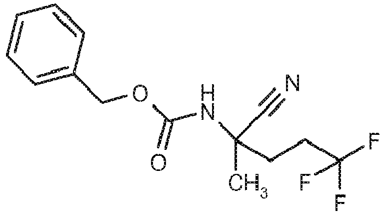

11.14 g *rac*-Benzyl-(2-cyan-5,5,5-trifluorpentan-2-yl)carbamat aus Beispiel 405A wurden durch präparative Trennung an der chiralen Phase in die Enantiomere getrennt [Säule: Daicel Chiralpak AZ-H, 5 µm, SFC, 250 x 50 mm, Eluent: 94% Kohlendioxid, 6% Methanol, Fluß: 200 ml/min, Temperatur: 38°C, Druck: 135 bar; Detektion: 210 nm].
Enantiomer A: 4.12 g (ca. 79% ee)
Rₜ = 1.60 min [SFC, Daicel Chiralpak AZ-H, 250 x 4.6 mm, 5 µm, Eluent: 90% Kohlendioxid, 10% Methanol, Fluß: 3 ml/min, Temperatur: 30°C, Detektion: 220 nm].
LC-MS (Methode 2): Rₜ = 1.01 min
MS (ESpos): m/z = 301 (M+H)⁺

### Beispiel 407A

### ent-Benzyl-(2-cyan-5,5,5-trifluorpentan-2-yl)carbamat (Enantiomer B)

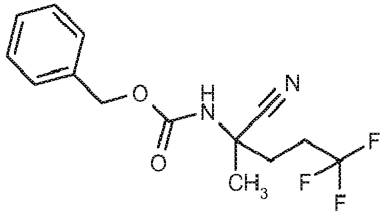

11.14 g *rac*-Benzyl-(2-cyan-5,5,5-trifluorpentan-2-yl)carbamat aus Beispiel 405A wurden durch präparative Trennung an der chiralen Phase in die Enantiomere getrennt [Säule: Daicel Chiralpak AZ-H, 5 µm, SFC, 250 x 50 mm, Eluent: 94% Kohlendioxid, 6% Methanol, Fluß: 200 ml/min, Temperatur: 38°C, Druck: 135 bar; Detektion: 210 nm].
Enantiomer B: 4.54 g (ca. 70% ee; Reinheit ca. 89%)
Rₜ = 1.91 min [SFC, Daicel Chiralpak AZ-H, 250 x 4.6 mm, 5 µm, Eluent: 90% Kohlendioxid, 10% Methanol, Fluß: 3 ml/min, Temperatur: 30°C, Detektion: 220 nm].
LC-MS (Methode 2): Rₜ = 1.01 min
MS (ESpos): m/z = 301 (M+H)⁺

### Beispiel 408A

### ent-Benzyl-(1-amino-5,5,5-trifluor-2-methylpentan-2-yl)carbamat (Enantiomer A)

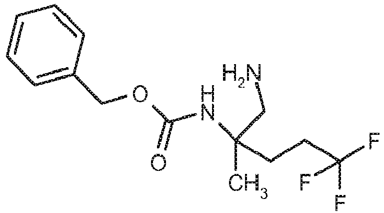

4.12 g (13.17 mmol) *ent*-Benzyl-(2-cyan-5,5,5-trifluorpentan-2-yl)carbamat (Enantiomer A) aus Beispiel 406A wurden in 39 ml 7 N Ammoniak-Lösung in Methanol gelöst und unter Argon mit 4 g Raney-Nickel (50%ige wässrige Aufschlämmung) versetzt. Das Reaktionsgemisch wurde über Nacht im Autoklaven bei 20-30 bar hydriert. Es wurden nochmals 1 g Raney-Nickel (50%ige wässrige Aufschlämmung) hinzugegeben und das Reaktionsgemisch wurde 5 h im Autoklaven bei 20-30 bar hydriert. Die Reaktionsmischung wurde über Kieselgur abfiltriert, mit Methanol gespült und eingeengt. Es wurden 3.35 g (56% d. Th.; Reinheit ca. 67%) der Zielverbindung erhalten, welche ohne weitere Reinigung in die Folgestufe eingesetzt wurde.
LC-MS (Methode 7): Rₜ = 1.68 min
MS (ESpos): m/z = 305 (M+H)⁺
¹H-NMR (400 MHz, DMSO-d₆): δ [ppm] = 1.13 (s, 3H), 1.40 (br. s, 2H), 1.70 -1.80 (m, 1H), 1.83 -1.95 (m, 1H), 2.08 - 2.2 (m, 2H), 4.98 (s, 2H), 6.85 (br. s, 1H), 7.28 - 7.41 (m, 5H).

### Beispiel 409A

### ent-Benzyl-(1-amino-5,5,5-trifluor-2-methylpentan-2-yl)carbamat (Enantiomer B)

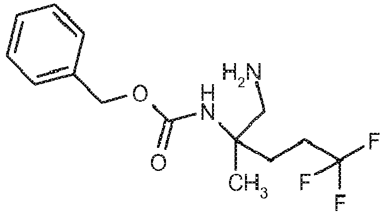

4.54 g (13.45 mmol; Reinheit ca. 89%) *ent*-Benzyl-(2-cyan-5,5,5-trifluorpentan-2-yl)carbamat (Enantiomer B) aus Beispiel 407A wurden in 39 ml 7 N Ammoniak-Lösung in Methanol gelöst und unter Argon mit 5 g Raney-Nickel (50%ige wässrige Aufschlämmung) versetzt. Das Reaktionsgemisch wurde 3 h im Autoklaven bei 20-30 bar hydriert. Die Reaktionsmischung wurde über Kieselgur abfiltriert, mit Methanol gespült und eingeengt. Es wurden 4.20 g (97% d. Th.; Reinheit ca. 95%) der Zielverbindung erhalten, welche ohne weitere Reinigung in die Folgestufe eingesetzt wurde.
LC-MS (Methode 15): Rₜ = 2.19 min
MS (ESpos): m/z = 305 (M+H)⁺
¹H-NMR (400 MHz, DMSO-d₆): δ [ppm] = 1.13 (s, 3H), 1.40 (br. s, 2H), 1.69 - 1.80 (m, 1H), 1.83 -1.96 (m, 1H), 2.07 - 2.22 (m, 2H), 4.98 (s, 2H), 6.85 (br. s, 1H), 7.27 - 7.40 (m, 5H).

### Beispiel 410A

### ent-Benzyl-{1-[({8-[(2,6-difluorbenzyl)oxy]-2,6-dimethylimidazo[1,2-a]pyridin-3-yl}carbonyl)amino]-5,5,5-trifluor-2-methylpentan-2-yl}carbamat (Enantiomer A)

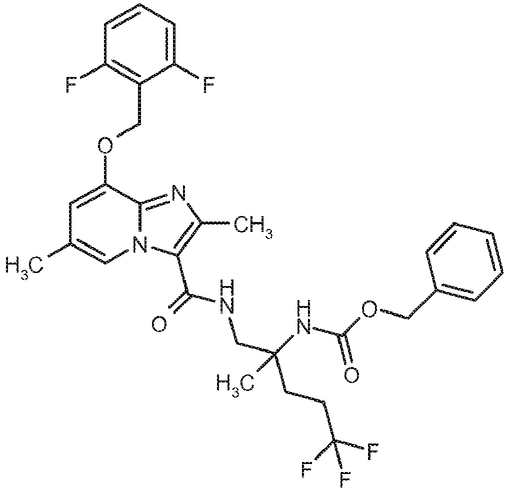

500 mg (1.51 mmol) 8-[(2,6-Difluorbenzyl)oxy]-2,6-dimethylimidazo[1,2-a]pyridin-3-carbonsäure aus Beispiel 21A, 629 mg (1.66 mmol) HATU und 583 mg (4.51 mmol) N,N-Diisopropylethylamin wurden in 9.6 ml DMF vorgelegt und 20 min bei RT gerührt. Anschließend wurden 957 mg (2.11 mmol; Reinheit ca. 67%) *ent*-Benzyl-(1-amino-5,5,5-trifluor-2-methylpentan-2-yl)carbamat (Enantiomer A) aus Beispiel 408A hinzugegeben und das Gemisch wurde über Nacht bei RT gerührt. Die Reaktionslösung wurde mit ca. 70 ml Wasser versetzt und 45 min bei Raumtemperatur verrührt. Der enthaltene Feststoff wurde abfiltriert, gut mit Wasser gewaschen und im Hochvakuum getrocknet. Es wurden 969 mg (99% d. Th.) der Zielverbindung erhalten, welche ohne weitere Reinigung in der Folgestufe eingesetzt wurde.
LC-MS (Methode 2): Rₜ = 1.11 min
MS (ESpos): m/z = 619 (M+H)⁺

### Beispiel 411A

### ent-Benzyl-{1-[({8-[(2,6-difluorbenzyl)oxy]-2,6-dimethylimidazo[1,2-a]pyridin-3-yl}carbonyl)amino]-5,5,5-trifluor-2-methylpentan-2-yl}carbamat (Enantiomer B)

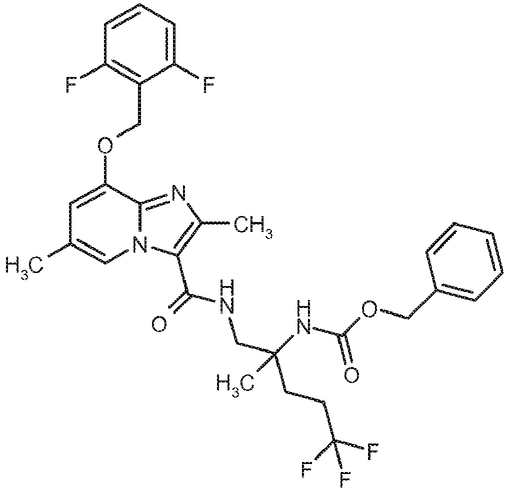

500 mg (1.51 mmol) 8-[(2,6-Difluorbenzyl)oxy]-2,6-dimethylimidazo[1,2-a]pyridin-3-carbonsäure aus Beispiel 21A, 629 mg (1.66 mmol) HATU und 583 mg (4.51 mmol) N,N-Diisopropylethylamin wurden in 9.6 ml DMF vorgelegt und 20 min bei RT gerührt. Anschließend wurden 675 mg (2.11 mmol; Reinheit ca. 95%) *ent*-Benzyl-(1-amino-5,5,5-trifluor-2-methylpentan-2-yl)carbamat (Enantiomer B) aus Beispiel 409A hinzugegeben und das Gemisch wurde über Nacht bei RT gerührt. Die Reaktionslösung wurde mit ca. 70 ml Wasser versetzt und 45 min bei Raumtemperatur verrührt. Der enthaltene Feststoff wurde abfiltriert, gut mit Wasser gewaschen und im Hochvakuum getrocknet. Es wurden 917 mg (98% d. Th.) der Titelverbindung erhalten, welche ohne weitere Reinigung in der Folgestufe eingesetzt wurde.
LC-MS (Methode 2): Rₜ = 1.14 min
MS (ESpos): m/z = 619 (M+H)⁺

### Beispiel 412A

### ent-Benzyl-{1-[({2,6-dimethyl-8-[(2,3,6-trifluorbenzyl)oxy]imidazo[1,2-a]pyridin-3-yl}carbonyl)amino]-5,5,5-trifluor-2-methylpentan-2-yl}carbamat(Enantiomer A)

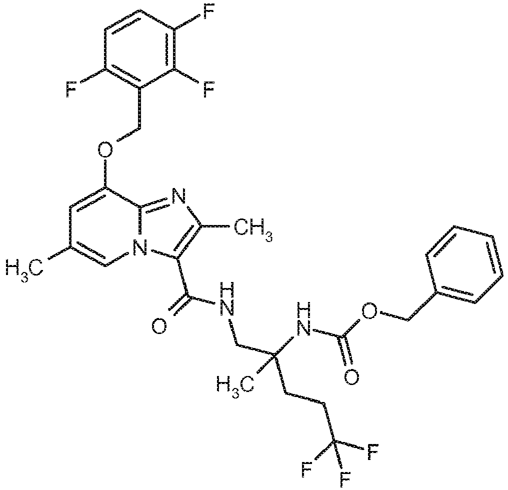

300 mg (0.86 mmol) 2,6-Dimethyl-8-[(2,3,6-trifluorbenzyl)oxy]imidazo[1,2-a]pyridin-3-carbonsäure aus Beispiel 265A, 358 mg (0.94 mmol) HATU und 332 mg (2.57 mmol) N,N-Diisopropylethylamin wurden in 5.5 ml DMF vorgelegt und 20 min bei RT gerührt. Anschließend wurden 545 mg (1.20 mmol; Reinheit ca. 67%) *ent*-Benzyl-(1-amino-5,5,5-trifluor-2-methylpentan-2-yl)carbamat (Enantiomer A) aus Beispiel 408A hinzugegeben und das Gemisch wurde 2 h bei RT gerührt. Die Reaktionslösung wurde mit ca. 50 ml Wasser versetzt und 45 min bei Raumtemperatur verrührt. Der enthaltene Feststoff wurde abfiltriert, gut mit Wasser gewaschen und im Hochvakuum getrocknet. Es wurden 553 mg (88% d. Th.; Reinheit 87%) der Titelverbindung erhalten, welche ohne weitere Reinigung in der Folgestufe eingesetzt wurde.
LC-MS (Methode 2): Rₜ = 1.13 min
MS (ESpos): m/z = 637 (M+H)⁺

### Beispiel 413A

### ent-Benzyl-{1-[({2,6-dimethyl-8-[(2,3,6-trifluorobenzyl)oxy]imidazo[1,2-a]pyridin-3-yl}carbonyl)amino]-5,5,5-trifluor-2-methylpentan-2-yl}carbamat (Enantiomer B)

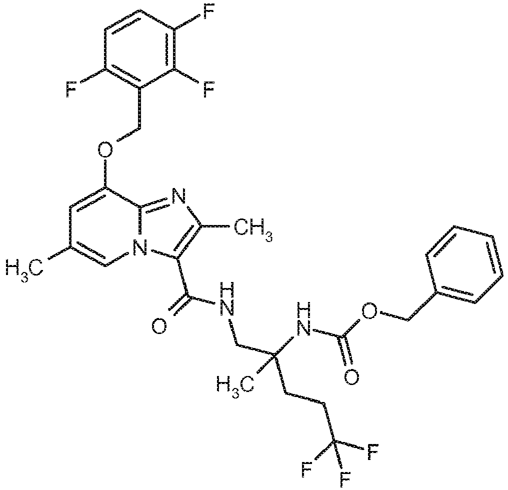

300 mg (0.86 mmol) 2,6-Dimethyl-8-[(2,3,6-trifluorbenzyl)oxy]imidazo[1,2-a]pyridin-3-carbonsäure aus Beispiel 265A, 358 mg (0.94 mmol) HATU und 332 mg (2.57 mmol) N,N-Diisopropylethylamin wurden in 5.5 ml DMF vorgelegt und 20 min bei RT gerührt. Anschließend wurden 384 mg (1.20 mmol; Reinheit ca. 95%) *ent*-Benzyl-(1-amino-5,5,5-trifluor-2-methylpentan-2-yl)carbamat (Enantiomer B) aus Beispiel 409A hinzugegeben und das Gemisch wurde 2 h bei RT gerührt. Die Reaktionslösung wurde mit ca. 50 ml Wasser versetzt und 45 min bei Raumtemperatur verrührt. Der enthaltene Feststoff wurde abfiltriert, gut mit Wasser gewaschen und im Hochvakuum getrocknet. Es wurden 540 mg (96% d. Th.) der Titelverbindung erhalten, welche ohne weitere Reinigung in der Folgestufe eingesetzt wurde.
LC-MS (Methode 2): Rₜ = 1.16 min
MS (ESpos): m/z = 637 (M+H)⁺

### Beispiel 414A

### rac-tert.-Butyl-{2-(3-acetamidophenyl)-2-[({8-[(2,6-diftuorbenzyl)oxy]-2,6-dimethylimidazo[1,2-a]pyridin-3-yl}carbonyl)amino]ethyl}carbamat Trifluoracetat

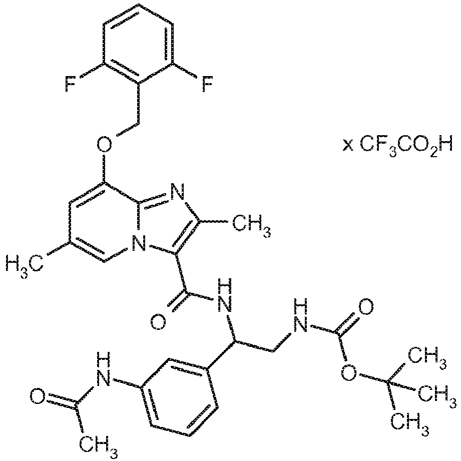

50 mg (0.15 mmol) 8-[(2,6-Difluorbenzyl)oxy]-2,6-dimethylimidazo[1,2-a]pyridin-3-carbonsäure aus Beispiel 21A, 63 mg (0.17 mmol) HATU und 58 mg (0.45 mmol) N,N-Diisopropylethylamin wurden in 0.5 ml DMF vorgelegt und 10 min bei RT gerührt. Anschließend wurden 51 mg (0.17 mmol) *tert*.-Butyl-[2-(3-acetamidophenyl)-2-aminoethyl]carbamat hinzugegeben und das Gemisch wurde 2 h bei RT gerührt. Die Reaktionsmischung wurde mit Acetonitril, TFA und Wasser versetzt und mittels präparativer HPLC gereinigt (RP-C18, Laufmittel: Acetonitril/Wasser-Gradient unter Zusatz von 0.1 % TFA). Es wurden 100 mg (92% d. Th.) der Titelverbindung erhalten.
LC-MS (Methode 2): Rₜ = 0.91 min
MS (ESpos): m/z = 608 (M-TFA+H)⁺

In Analogie zu Beispiel 414A wurden die in Tabelle 18A gezeigten Beispiele hergestellt, indem 8-[(2,6-Difluorbenzyl)oxy]-2,6-dimethylimidazo[1,2-a]pyridin-3-carbonsäure aus Beispiel 21A mit den entsprechenden Aminen unter den in der repräsentativen Arbeitsvorschrift 2 beschriebenen Reaktionsbedingungen mit HATU umgesetzt wurden.

**Tabelle 18A:**

| **Beispiel** | **IUPAC-Name / Struktur (Ausbeute)** | **Analytische Daten** |
|---|---|---|
| **415A** | *rac*-Ethyl-6-{2-[(tert-butoxycarbonyl)amino]-1-[({8-[(2,6-difluorbenzyl)oxy]-2,6-dimethylimidazo[1,2-a]pyridin-3-yl}carbonyl)amino]ethyl}pyridin-2-carboxylat Trifluoracetat | LC-MS (Methode 2): Rₜ = 1.02 min |
| | | MS (ESpos): m/z = 576 (M-TFA+H)⁺ |
| | 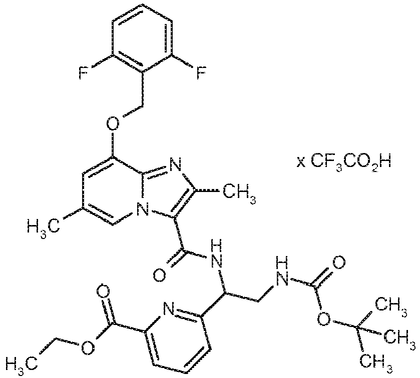 | |
| | (35% d. Th.; Reinheit 94%) | |
| **416A** | *rac-*tert-Butyl-{2-(4-cyanphenyl)-2-[({8-[(2,6-difluorbenzyl)oxy]-2,6-dimethylimidazo[1,2-a]pyridin-3-yl}carbonyl)amino]ethyl}carbamat Trifluoracetat | LC-MS (Methode 2): Rₜ = 1.04 min |
| | | MS (ESpos): m/z = 585 (M-TFA+H)⁺ |
| | 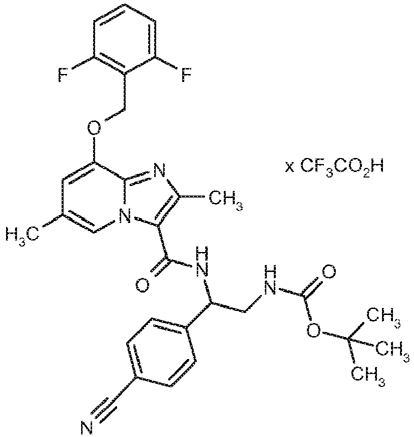 | |
| | (42% d. Th.) | |

### Beispiel 417A

### rac-tert-Butyl-3-{[({8-[(2,6-difluorbenzyl)oxy]-2,6dimethylimidazo[1,2-a]pyridin-3-yl}carbonyl)amino]methyl}thiomorpholin-4-carboxylat

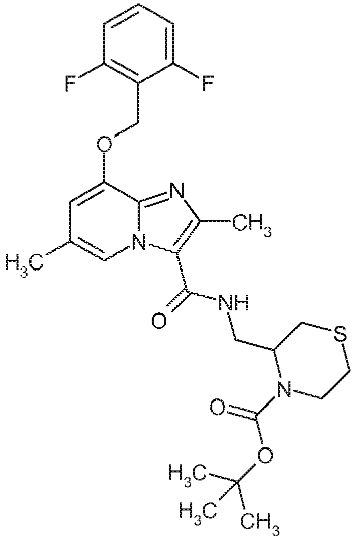

320 mg (0.717 mmol) *rac*-8-[(2,6-Difluorbenzyl)oxy]-2,6-dimethyl-N-(thiomorpholin-3-ylmethyl)imidazo[1,2-a]pyridin-3-carboxamid aus Beispiel 366, 217 mg (2.150 mmol) Triethylamin und 156 mg (0.717 mmol) Di-*tert*-butyldicarbonat wurden in 3.0 ml Dichlormethan vorgelegt und 1.5 h bei RT gerührt. Die Reaktionsmischung wurde mit Dichlormethan verdünnt, zweimal mit Wasser gewaschen, über Natriumsulfat getrocknet, am Rotationsverdampfer eingeengt und im Hochvakuum getrocknet. Die Titelverbindung, 292 mg (75% d. Th.), wurde ohne weitere Reinigung in die nächste Stufe eingesetzt.
LC-MS (Methode16): Rₜ = 1.26 min
MS (ESIpos): m/z = 547 (M+H)⁺

### Beispiel 418A

### rac-tert-Butyl-3-{[({8-[(2,6-difluorbenzyl)oxy]-2,6-dimethylimidazo[1,2-a]pyridin-3-yl}carbonyl)amino]methyl}thiomorpholin-4-carboxylat-1,1-dioxid Trifluoracetat

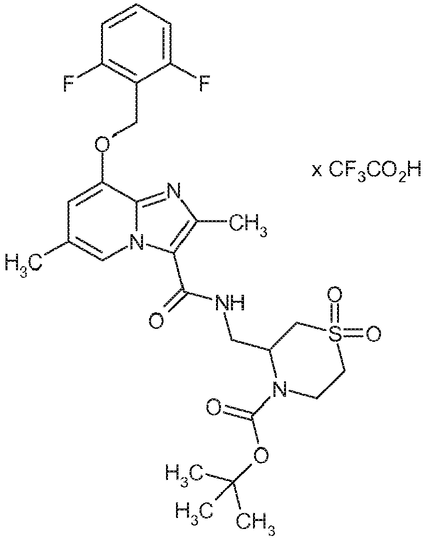

Eine Lösung von 150 mg (0.274 mmol) *rac-tert*-Butyl-3-{[({8-[(2,6-difluorbenzyl)oxy]-2,6-dimethylimidazo[1,2-a]pyridin-3-yl}carbonyl)amino]methyl}thiomorpholin-4-carboxylat aus Beispiel 417A in 3.3 ml Dichlormethan wurde mit 118 mg (0.686 mmol) 3-Chlorbenzolcarboperoxosäure bei 0°C versetzt und 70 min bei 0°C gerührt. Die Reaktionsmischung wurde mit Dichlormethan verdünnt, dreimal mit 1 N wässriger Natronlauge und einmal mit 10%-iger wässriger Natriumchlorid-Lösung gewaschen, über Natriumsulfat getrocknet und am Rotationsverdampfer eingeengt. Das Rohprodukt wurde mittels präparativer HPLC gereinigt (RP18 Säule, Laufmittel: Acetonitril/Wasser-Gradient unter Zusatz von 0.1% TFA). Es wurden 59 mg der Zielverbindung (31% d. Th.) erhalten.
LC-MS (Methode 16): Rₜ = 1.10 min
MS (ESIpos): m/z = 579 (M-TFA+H)⁺

### Beispiel 419A

### rac-Benzyl-(2-amino-2-cyanethyl)carbamat Trifluoracetat

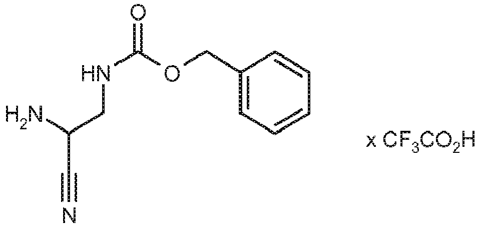

500 mg (3.16 mmol) *rac*-2,3-Diaminopropanonitril Dihydrochlorid wurden in 1.7 ml abs. Dichlormethan vorgelegt. Bei Raumtemperatur wurde mit 3.27 g (25.31 mmol) N,N-Diisopropylethylamin und 586.2 mg (3.16 mmol) Chlorameisensäurebenzylester versetzt und es wurde über Nacht bei Raumtemperatur gerührt. Die Reaktionslösung wurde mit TFA und Wasser versetzt und mittels präparativer HPLC gereinigt (RP18 Säule; Laufmittel: Acetonitril/Wasser-Gradient unter Zusatz von 0.1% TFA). Es wurden 125 mg der Titelverbindung (12% d. Th.) erhalten.
LC-MS (Methode 13): Rₜ = 1.31 min
MS (ESpos): m/z = 220 (M-TFA+H)⁺

### Beispiel 420A

### rac-Benzyl-{2-cyan-2-[({8-[(2,6-difluorbenzyl)oxy]-2,6-dimethylimidazo[1,2-a]pyridin-3-yl}carbonyl)amino]ethyl}carbamat Trifluoracetat

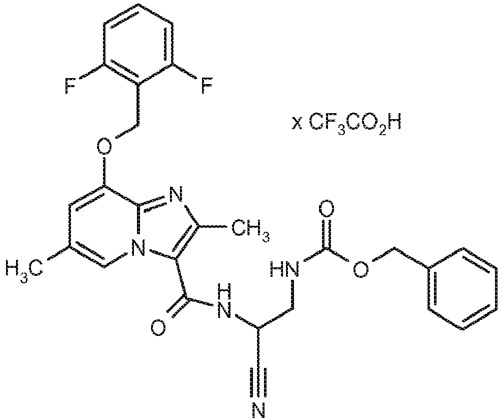

113 mg (0.34 mmol) 8-[(2,6-Difluorbenzyl)oxy]-2,6-dimethylimidazo[1,2-a]pyridin-3-carbonsäure aus Beispiel 21A wurden in 0.55 ml abs. DMF vorgelegt, mit 136 mg (0.36 mmol) HATU und 132 mg (1.02 mmol) N,N-Diisopropylethylamin versetzt, 20 min bei Raumtemperatur gerührt und anschließend auf 60°C erwärmt. 125 mg (0.38 mmol) *rac*-Benzyl-(2-amino-2-cyanethyl)carbamat Trifluoracetat aus Beispiel 419A wurden in 0.28 ml abs. DMF gelöst, mit 44 mg (0.34 mmol) N,N-Diisopropylethylamin versetzt, tropfenweise zu der auf 60°C erwärmten Reaktionslösung gegeben und es wurde 1 h bei 60°C gerührt. Die Reaktionslösung wurde mit Wasser/TFA/Acetonitril versetzt und mittels präparativer HPLC gereinigt (RP18 Säule, Laufmittel: Acetonitril/Wasser-Gradient unter Zusatz von 0.1% TFA). Es wurden 151 mg der Titelverbindung (68% d. Th.) erhalten.
LC-MS (Methode 2): Rₜ = 0.99 min
MS (ESpos): m/z = 534 (M-TFA+H)⁺

### Ausführungsbeispiele

### Beispiel 1

### N-(9-Cyclopropyl-9-azabicyclo[3.3.1]non-3-yl)-8-[(2,6-difluorbenzyl)oxy]-2-methylimidazo[1,2-a]pyridin-3-carboxamid

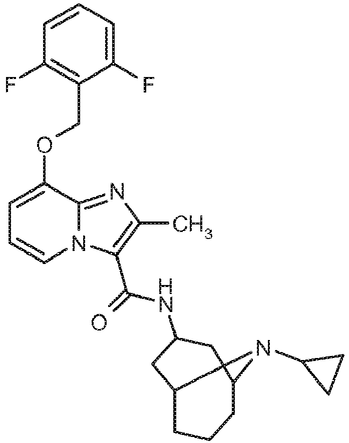

70 mg 8-[(2,6-Difluorbenzyl)oxy]-2-methylimidazo[1,2-a]pyridin-3-carbonsäure (0.22 mmol), 209 mg *O*-(7-Azabenzotriazol-1-yl)-*N,N,N',N'*-tetramethyluroniumhexafluorphosphat (HATU, 0.55 mmol) und 85 mg *N,N-*Diisopropylethylamin (0.66 mmol) wurden in 1.5 ml DMF vorgelegt, 15 min gerührt. Dann wurde mit 59 mg 9-Cyclopropyl-9-azabicyclo[3.3.1]nonan-3-amin (0.33 mmol) versetzt und über Nacht bei RT gerührt. Anschließend wurde nochmals über Nacht bei 60°C gerührt. Die Reaktionslösung wurde mit ca. 12 ml Wasser versetzt, der entstandene Niederschlag abfiltriert, mit Wasser gewaschen und getrocknet. Der erhaltene Rückstand wurde mittels Kieselgelchromatographie gereinigt (Laufmittel: Dichlormethan/Methanol 40:1). Es wurden 60 mg der Titelverbindung (53% d. Th., Reinheit 94%) erhalten.
LC-MS (Methode 2): Rₜ = 0.63 min
MS (ESpos): m/z = 481 (M+H)⁺
¹H-NMR (400 MHz, DMSO-d₆): δ = 0.24 (br s, 2H), 0.45 (d, 2H), 1.04 (d, 2H), 1.48-1.56 (m, 3H), 1.86-1.1.97 (m, 2H), 2.00-2.12 (m, 1H), 2.15-2.28 (m, 2H), 2.30-2.38 (m, 1H), 2.49 (s, 3H), 3.19 (d, 2H), 4.09-4.23 (m, 1H), 5.30 (s, 2H), 6.90 (t, 1H), 6.99 (d, 1H), 7.23 (t, 2H), 7.54-7.68 (m, 2H), 8.50 (d,1H).

### Beispiel 2

### rac-N-(1-Amino-4,4,4-trifluorbutan-2-yl)-8-[(2,6-difluorbenzyl)oxy]-2-methylimidazo[1,2-a]pyridin-3-carboxamid

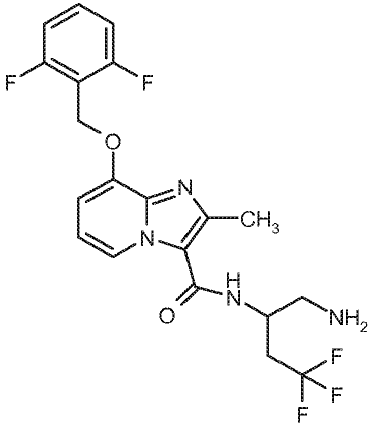

555 mg *rac-N-*(1-Azido-4,4,4-trifluorbutan-2-yl)-8-[(2,6-difluorbenzyl)oxy]-2-methylimidazo[1,2-a]pyridin-3-carboxamid (Beispiel 41A, 1.19 mmol) wurden in 74 ml Ethanol vorgelegt, mit 126 mg Palladium auf Aktivkohle (10%ig) versetzt und 60 min bei Wasserstoff-Normaldruck und RT hydriert. Die Reaktionslösung wurde filtriert und eingeengt. Das Rohprodukt wurde mittels präparativer HPLC gereinigt (RP18 Säule, Laufmittel: Acetonitril/Wasser-Gradient unter Zusatz von 0.1 % TFA). Die produkthaltigen Fraktionen wurden in Essigsäureethylester aufgenommen und mit gesättigter, wässriger Natriumhydrogencarbonat-Lösung gewaschen. Die organische Phase wurde über Natriumsulfat getrocknet, filtriert und eingeengt. Es wurden 473 mg der Titelverbindung (88% d. Th.) erhalten.
LC-MS (Methode 2): Rₜ = 0.65 min
MS (ESpos): m/z = 443 (M+H)⁺
¹H-NMR (400 MHz, DMSO-d₆): δ = 1.92 (br s, 2H), 2.48-2.60 (m, 3+1H), 2.62-2.80 (m, 3H), 4.18-4.30 (m, 1H), 5.30 (s, 2H), 6.93 (t, 1H), 7.00 (d, 1H), 7.22 (t, 2H), 7.59 (quint, 1H), 7.79 (br s, 1H), 8.53 (d, 1H).

### Beispiel 3

### ent-N-(1-Amino-4,4,4-trifluorbutan-2-yl)-8-[(2,6-difluorbenzyl)oxy]-2-methylimidazo[1,2-a]pyridin-3-carboxamid (Enantiomer B)

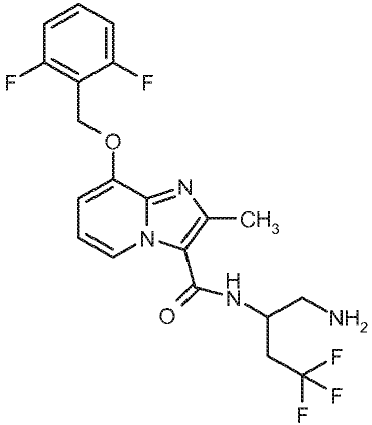

Durch präparative Trennung an chiraler Phase wurde Beispiel 2 in die Enantiomere getrennt [Säule: Daicel Chiralpak AD-H, 5 µm, 250 x 20 mm; Eluent: 25% iso-Hexan, 75% Ethanol + 0.2% Diethylamin; Fluß 15 ml/min; 45°C; Detektion: 220 nm]
EnantiomerB:
Ausbeute: 154 mg (99% Reinheit, >99% ee)
Rₜ = 16.57 min [Chiralpak AD-H, 5µm, 250 x 4.6 mm; Eluent: 25% iso-Hexan, 75% Ethanol + 0.2% Diethylamin; Fluss 1.0 ml/min; 40°C; Detektion: 220 nm].

In Analogie zu Beispiel 2 wurden die in Tabelle 1 gezeigten Beispiele hergestellt.

**Tabelle 1:**

| **Beispiel** | **IUPAC-Name / Struktur (Ausbeute)** | **Analytische Daten** |
|---|---|---|
| **4** | *rac-N-*(1-Amino-6,6,6-trifluorhexan-2-yl)-8-[(2,6-difluorbenzyl)oxy]-2-methylimidazo[1,2-a]pyridin-3-carboxamid | LC-MS (Methode 2): Rₜ = 1.03 min |
| | | MS (ESpos): m/z = 497 (M+H)⁺ |
| | 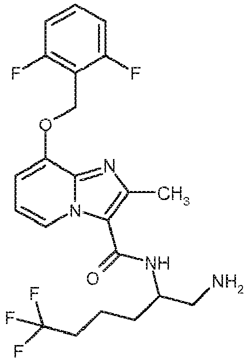 | ¹H-NMR (400 MHz, DMSO-d₆): δ = 1.49-1.72 (m, 4H), 2.18-2.41 (m, 2H), 2.50 (s, 3H), 2.69 (d, 2H), 3.87-3.98 (m, 1H), 5.30 (s, 2H), 6.92 (t, 1H), 7.00 (d, 1H), 7.22 (d, 2H), 7.56-7.68 (m, 2H), 8.52 (d,1H). |
| | (90% d. Th.) | |
| **5** | *rac-N-*[1-Amino-5,5,5-trifluor-4-{trifluormethyl)pentan-2-yl]-8-[(2,6-difluorbenzyl)oxy]-2-methylimidazo[1,2-a]pyridin-3-carboxamid-Trifluoracetat ^{a)} | LC-MS (Methode 7): Rₜ = 0.70 min |
| | | MS (ESpos): m/z = 525 (M-TFA+H)⁺ |
| | | ¹H-NMR (400 MHz, DMSO-d₆): δ = 2.12-2.28 (m, 2H), 2.60 (s, 3H), 2.99-3.10 (m, 2H), 3.14-3.25 (m, 1H), 3.95-4.10 (m, 1H), 4.37-4.49 (m, 1H), 5.35 (s, 2H), 7.05-7.12 (m, 1H), 7.15-7.28 (m, 3H), 7.60 (q, 1H), 7.69-7.82 (m, 1H), 7.97 (br s, 3H), 8.70 (d, 1H). |
| | 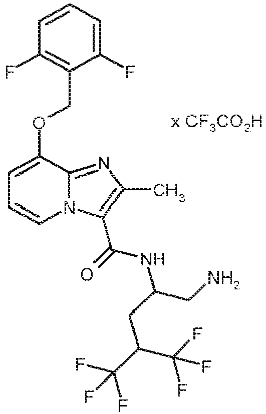 | |
| | (91 % d. Th.) | |

| | | |
|---|---|---|
| a) Es erfolgte nach der präparativen HPLC keine wässrige Aufarbeitung. | | |

### Beispiel 6

### ent-N-(1-Amino-6,6,6-trifluorhexan-2-yl)-8-[(2,6-difluorbenzyl)oxy]-2-methylimidazo[1,2-a]pyridin-3-carboxamid (Enantiomer B)

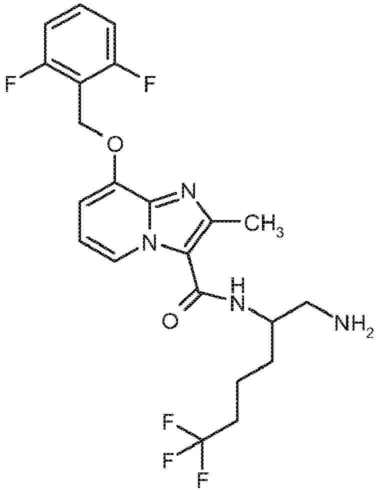

Durch präparative Trennung an chiraler Phase wurde Beispielverbindung 4 (388 mg) in die Enantiomere getrennt [Säule: Daicel Chiralpak AD-H, 5 µm, 250 x 20 mm; Eluent: 25% iso-Hexan, 75% Ethanol + 0.2% Dietylamin; Fluß 15 ml/min; 45°C; Detektion: 220 nm]
Enantiomer B:
Ausbeute: 136 mg (99% Reinheit, >99% ee)
Rₜ = 17.00 min [Chiralpak AD-H, 5µm, 250 x 4.6 mm; Eluent: 25% iso-Hexan, 75% Ethanol + 0.2% Diethylamin; Fluss 1.0 ml/min; 40°C; Detektion: 220 nm].

### Beispiel 7

### ent-N-[1-Amino-5,5,5-trifluor-4-(trifluormethyl)pentan-2-yl]-8-[(2,6-difluorbenzyl)oxy]-2-methylimidazo[1,2-a]pyridin-3-carboxamid (Enantiomer A)

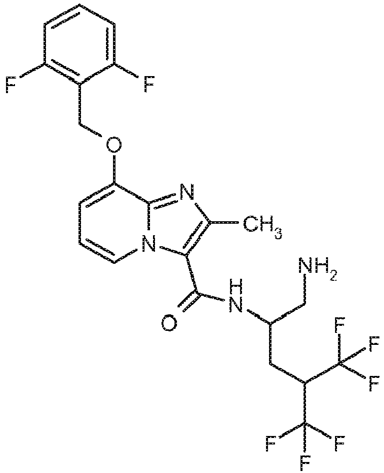

Durch präparative Trennung an chiraler Phase wurde Beispielverbindung 5 (140 mg) in die Enantiomere getrennt [Säule: Daicel Chiralpak OD-H, 5 µm, 250 x 20 mm; Eluent: 70% iso-Hexan, 30% Ethanol + 0.2% Dietylamin; Fluß 20 ml/min; 25°C; Detektion: 230 nm]
Enantiomer A:
Ausbeute: 31 mg (>99% ee)
Rt= 4.47 min [Daicel Chiralpak OD-H, 5µm, 250 x 4.6 mm; Eluent: 50% iso-Hexan, 50% Ethanol + 0.2% Diethylamin; Fluss 1.0 ml/min; Detektion: 250 nm].

### Beispiel 8

### N-[(2R)-1-Aminohexan-2-yl]-8-[(2,6-difluorbenzyl)oxy]-2-methylimidazo[1,2-a]pyridin-3-carboxamid

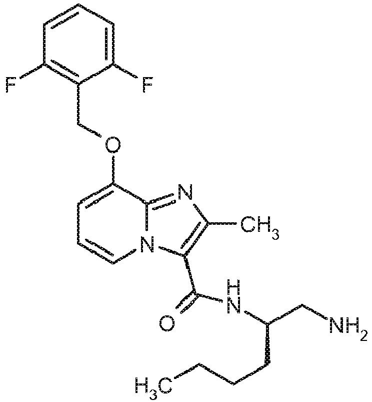

42 mg N[(2*R*)-1-Azidohexan-2-yl]-8-[(2,6-difluorbenzyl)oxy]-2-methylimidazo[1,2-a]pyridin-3-carboxamid (Beispiel 44A, 0.095 mmol) wurden in 0.78 ml THF/Wasser (10:1.5) vorgelegt, mit 28.3 mg Triphenylphosphin (0.108 mmol) versetzt und über Nacht bei RT gerührt. Das Reaktionsgemisch wurde eingeengt und der Rückstand mittels präparativer Dickschichtchromatographie (Laufmittel Essigsäureethylester:Dichlormethan:Diethylamin = 2:1:0.1) gereinigt. Die vereinten Produktfraktionen wurde mittels präparativer HPLC gereinigt (RP18 Säule, Laufmittel: Acetonitril/Wasser-Gradient unter Zusatz von 0.1% TFA). Die produkthaltigen Fraktionen wurden in Essigsäureethylester aufgenommen und mit gesättigter, wässriger Natriumhydrogencarbonat-Lösung gewaschen. Die organische Phase wurde über Natriumsulfat getrocknet, filtriert und eingeengt. Es wurden 17 mg der Titelverbindung (41% d. Th.) erhalten.
LC-MS (Methode 1): Rₜ = 0.88 min
MS (ESpos): m/z = 417 (M+H)⁺
¹H-NMR (400 MHz, DMSO-d₆): δ = 0.88 (t, 3H), 1.21-1.39 (m, 4H), 1.42-1.67 (m, 2H), 2.73 (d, 2H), 3.92-4.08 (m, 1H), 5.30 (s, 2H), 6.93 (t, 1H), 7.00 (d, 1H), 7.22 (t, 2H), 7.56-7.7.62 (m, 2H), 8.56 (d,1H).

### Beispiel 9

### rac-N-[2-Amino-1-(4-fluorphenyl)ethyl]-8-[(2,6-difluorbenzyl)oxy]-2-methylimidazo[1,2-a]pyridin-3-carboxamid

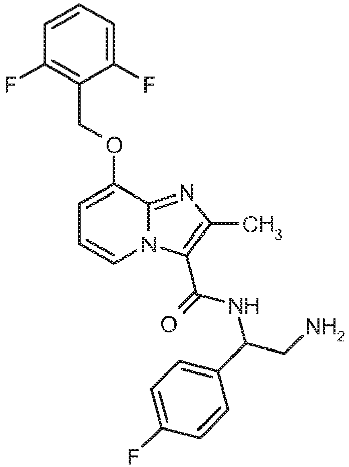

363 mg tert.-Butyl-{2-[({8-[(2,6-difluorbenzyl)oxy]-2-methylimidazo[1,2-a]pyridin-3-yl}-carbonyl)amino]-2-4-fluorphenyl)ethyl}carbamat (Beispiel 79A, 0.63 mmol) wurden mit 7 ml 2M Salzsäure in Diethylether (14 mmol) versetzt und 3 h bei RT gerührt. Der entstandene Niederschlag wurde abfiltriert, mit Diethylether gewaschen, in Dichlormethan und Methanol gelöst und mit gesättigter, wässriger Natriumhydrogencarbonat-Lösung gewaschen. Die organische Phase wurde über Natriumsulfat getrocknet, filtriert, das Filtrat eingeengt und im Hochvakuum getrocknet. Es wurden 275 mg der Titelverbindung (96% d. Th.) erhalten.
LC-MS (Methode 2): Rₜ = 0.68 min
MS (ESpos): m/z = 455 (M+H)⁺
¹H-NMR (400 MHz, DMSO-d₆): δ = 1.62 (br s, 2H), 2.60 (s, 3H), 2.85-2.96 (m, 2H), 4.98 (t, 1H), 5.31 (s, 2H), 6.90 (t, 1H), 6.99 (d, 1H), 7.18 (t, 2H), 7.22 (t, 2H), 7.41 (dd, 2H), 7.59 (quint, 1H), 8.20 (br s, 1H), 8.53 (d, 1H).

### Beispiel 10

### ent-N-[2-Amino-1-(4-fluorphenyl)ethyl]-8-[(2,6-difluorbenzyl)oxy]-2-methylimidazo[1,2-a]pyridin-3-carboxamid (Enantiomer B)

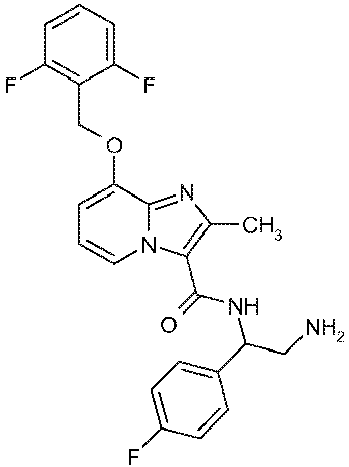

Durch präparative Trennung an chiraler Phase wurde Beispielverbindung 9 (242 mg) in die Enantiomere getrennt [Säule: Daicel Chiralpak AD-H, 5 µm, 250 x 20 mm, Eluent: 100% Ethanol + 0.2% Diethylamin, Fluß 15 ml/min; 45°C, Detektion: 220 nm].
Enantiomer B:
Ausbeute: 110 mg (99% Reinheit, ca. 99% ee)
Rₜ = 10.25 min [Chiralpak AD-H, 5µm, 250 x 4.6 mm; Eluent: 70% iso-Hexan, 30% Ethanol + 0.2% Diethylamin; Fluss 1.0 ml/min; 45°C; Detektion: 235 nm].

### Beispiel 11

### rac-N-[2-Amino-1-(3,4-difluorphenyl)ethyl]-8-[(2,6-difluorbenzyl)oxy]-2-methylimidazo[1,2-a]pyridin-3-carboxamid

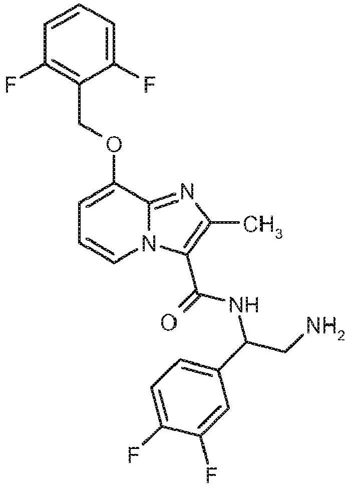

355 mg *rac*-tert.-Butyl-{2-[({8-[(2,6-difluorbenzyl)oxy]-2-methylimidazo[1,2-a]pyridin-3-yl}carbonyl)amino]-2-(3,4-difluorphenyl)ethyl}carbamat (Beispiel 77A, 0.62 mmol) wurden mit 3.1 ml 2 M Salzsäure in Diethylether (6.2 mmol) versetzt und 5.5 h bei RT gerührt. Der entstandene Niederschlag wurde abfiltriert, mit Diethylether gewaschen, in Dichlormethan suspendiert und mit gesättigter, wässriger Natriumhydrogencarbonat-Lösung gewaschen. Die organische Phase wurde über Natriumsulfat getrocknet, filtriert, das Filtrat eingeengt und im Hochvakuum getrocknet. Es wurden 279 mg der Titelverbindung (94% d. Th.) erhalten.
LC-MS (Methode 2): Rₜ = 0.77 min
MS (ESpos): m/z = 473 (M+H)⁺
¹H-NMR (400 MHz, DMSO-d₆): δ = 2.18 (br s, 2H), 2.60 (s, 3H), 2.87-2.97 (m, 2H), 4.99 (t, 1H), 5.31 (s, 2H), 6.91 (t, 1H), 7.01 (d, 1H), 7.19-7.28 (m, 3H), 7.36-7.51 (m, 2H), 7.59 (quint, 1H), 8.21 (br s, 1H), 8.54 (d,1H).

### Beispiel 12 (Enantiomer A)

### ent-N-[2-Amino-1-(3,4-difluorphenyl)ethyl]-8-[(2,6-difluorbenzyl)oxy]-2-methylimidazo[1,2-a]pyridin-3-carboxamid (Enantiomer A)

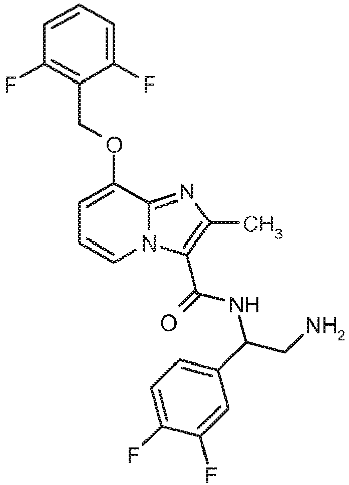

Durch präparative Trennung an chiraler Phase wurde Beispielverbindung 11 (250 mg) in die Enantiomere getrennt [Säule: Daicel Chiralpak AY-H, 5 µm, 250 x 20 mm, Eluent: 70% iso-Hexan, 30% Ethanol + 0.2% Diethylamin, Fluß 15 ml/min; 45°C, Detektion: 220 nm]. Die Produktfraktionen wurden eingeengt und in Wasser/Acetonitril aufgenommen und gefriergetrocknet. Anschließend wurde mittels präparativer HPLC gereinigt (RP18 Säule, Laufmittel: Acetonitril/Wasser-Gradient unter Zusatz von 0.1% TFA). Die vereinten Fraktionen wurden lyophilisiert, der Rückstand in Essigsäureethylester gelöst und mit wenig gesättigter, wässriger Natriumhydrogencarbonat-Lösung gewaschen. Die organische Phase wurde über Natriumsulfat getrocknet und filtriert. Das Filtrat wurde eingeengt und im Hochvakuum getrocknet.
Enantiomer A:
Ausbeute: 88 mg (99% Reinheit, ca 94% ee)
Rt= 7.55 min [Chiralpak AY-H, 5µm, 250 x 4.6 mm; Eluent: 70% iso-Hexan, 30% Ethanol + 0.2% Diethylamin; Fluss 1.0 ml/min; 45°C; Detektion: 235 nm].
LC-MS (Methode 2): Rₜ = 0.78 min
MS (ESpos): m/z = 473 (M+H)⁺
¹H-NMR (400 MHz, DMSO-d₆): δ = 2.59 (s, 3H), 2.88-3.01 (m, 2H), 5.02 (t, 1H), 5.31 (s, 2H), 6.91 (t, 1H), 7.01 (d, 1H), 7.19-7.29 (m, 3H), 7.36-7.52 (m, 2H), 7.59 (quint, 1H), 8.21 (br s, 1H), 8.54 (d, 1H).
Spezifischer Drehwert [a] (436 nm, 20.4°C) = +23.8° (c = 0.0053 g/ml, Acetonitril)

### Beispiel 13 (Enantiomer B)

### ent-N-[2-Amino-1-(3,4-difluorphenyl)ethyl]-8-[(2,6-difluorbenzyl)oxy]-2-methylimidazo[1,2-a]pyridin-3-carboxamid (Enantiomer B)

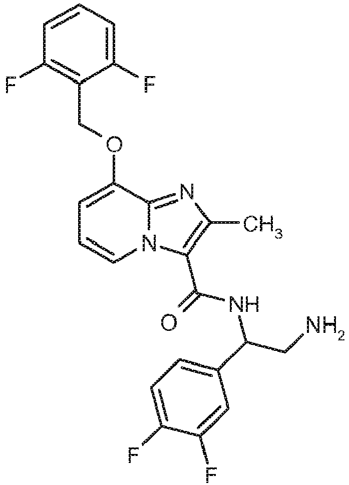

2.18 g *ent*-tert.-Butyl-{2-[({8-[(2,6-difluorbenzyl)oxy]-2-methylimidazo[1,2-a]pyridin-3-yl}carbonyl)amino]-2-(3,4-difluorphenyl)ethyl}carbamat (Beispiel 118A, 3.80 mmol) wurden mit 19 ml 2 M Salzsäure in Diethylether (38 mmol) versetzt und über Nacht bei RT gerührt. Das Reaktionsgemisch wurde mit Diethylether verdünnt. Der Niederschlag wurde abfiltriert und mit Essigsäureethylester und gesättigter, wässriger Natriumhydrogencarbonat-Lösung versetzt. Nach Phasentrennung wurde die wässrige Phase zweimal mit Essigsäureethylester extrahiert. Die vereinten organischen Phasen wurden über Natriumsulfat getrocknet, filtriert, das Filtrat eingeengt und lyophilisiert. Es wurden 1.85 g der Titelverbindung (quantitativ) erhalten.
LC-MS (Methode 2): Rₜ = 0.80 min
MS (ESpos): m/z = 473 (M+H)⁺
¹H-NMR (400 MHz, DMSO-d₆): δ = 1.55 (br s, 2H), 2.59 (s, 3H), 2.85-2.95 (m, 2H), 4.97 (t, 1H), 5.31 (s, 2H), 6.91 (t, 1H), 7.01 (d, 1H), 7.19-7.28 (m, 3H), 7.36-7.51 (m, 2H), 7.59 (quint, 1H), 8.21 (br s, 1H), 8.54 (d,1H).
Spezifischer Drehwert [a] (436 nm, 19.9°C) = -23.5° (c = 0.00505 g/ml, Acetonitril)

### Beispiel 14

### rac-N-[2-Amino-1-(4-chlorphenyl)ethyl]-8-[(2,6-difluorbenzyl)oxy]-2-methylimidazo[1,2-a]pyridin-3-carboxamid-Dihydrochlorid

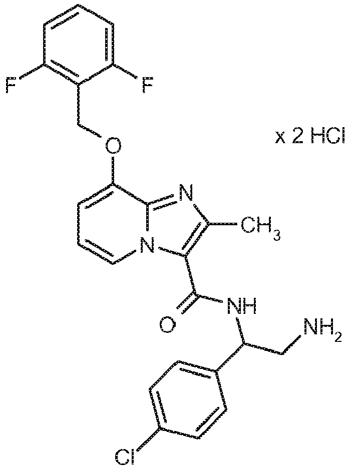

396 mg *rac*-tert.-Butyl-{2-(4-chlorphenyl)-2-[({8-[(2,6-difluorbenzyl)oxy]-2-methylimidazo[1,2-a]pyridin-3-yl}carbonyl)amino]ethyl}carbamat (Beispiel 78A, 0.69 mmol) wurden mit 3.5 ml (7.0 mmol) einer 2 M Lösung von Salzsäure in Diethylether versetzt und 5.5 h bei RT gerührt. Der entstandene Niederschlag wurde abfiltriert, mit Diethylether gewaschen und im Hochvakuum getrocknet. Es wurden 341 mg der Titelverbindung (90% d. Th.) erhalten.
LC-MS (Methode 2): Rₜ = 0.72 min
MS (ESpos): m/z = 471 (M-2HCl+H)⁺
¹H-NMR (400 MHz, DMSO-d₆): δ = 2.69 (s, 3H), 3.16-3.28 (m, 1H), 3.38-3.53 (m, 1H), 5.40-5.49 (m, 3H), 7.23 (t, 2H), 7.34 (br s, 1H), 7.41-7.67 (m, 6H), 8.20-8.43 (m, 3H), 8.65 (d, 1H), 9.39 (br s, 1H).

### Beispiel 15

### ent-N-[2-Amino-1-(4-chlorphenyl)ethyl]-8-[(2,6-difluorbenzyl)oxy]-2-methylimidazo[1,2-a]pyridin-3-carboxamid (Enantiomer B)

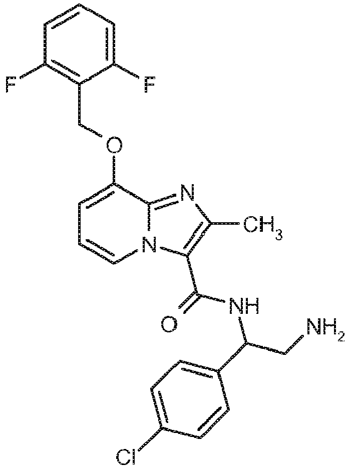

288 mg Beispielverbindung 14 wurden in Essigsäureethylester suspendiert und mit gesättigter, wässriger Natriumhydrogencarbonat-Lösung gewaschen. Die organische Phase wurde über Natriumsulfat getrocknet und filtriert. Das Filtrat wurde eingeengt und im Hochvakuum getrocknet. Durch präparative Trennung an chiraler Phase wurde das Rohprodukt (279 mg) in die Enantiomere getrennt [Säule: Daicel Chiralpak AD-H, 5 µm, 250 x 20 mm, Eluent: 30% iso-Hexan, 70% Ethanol + 0.2% Diethylamin, Fluß 15 ml/min; 45°C, Detektion: 220 nm]. Das Produkt wurde nochmals über Kieselgel gereinigt (Laufmittel: Dichlormethan nach Dichlormethan:Methanol = 10:1). Es erfolgte eine weitere Reinigung mittels präparativer HPLC (RP18 Säule, Laufmittel: Methanol/Wasser-Gradient unter Zusatz von 0.1% TFA). Die Produktfraktionen wurden in Essigsäureethylester gelöst und zweimal mit wenig gesättigter, wässriger Natriumhydrogencarbonat-Lösung gewaschen. Die organische Phase wurde über Natriumsulfat getrocknet, filtriert, das Filtrat eingeengt und über Nacht im Hochvakuum getrocknet.
EnantiomerB:
Ausbeute: 62 mg (99% Reinheit, 99% ee)
Rt= 8.77 min [Chiralpak AY-H, 5µm, 250 x 4.6 mm; Eluent: 60% iso-Hexan, 40% Ethanol + 0.2% Diethylamin; Fluss 1.0 ml/min; 35°C; Detektion: 235 nm].
¹H-NMR (400 MHz, DMSO-d₆): δ = 2.04 (br s, 2H), 2.60 (s, 3H), 2.85-2.95 (m, 2H), 4.98 (t, 1H), 5.30 (s, 2H), 6.90 (t, 1H), 7.02 (d, 1H), 7.22 (t, 2H), 7.38-7.44 (m, 4H), 7.59 (quint, 1H), 8.20 (br s, 1H), 8.53 (d, 1H).

### Beispiel 16

### rac-N-(2-Amino-1-phenylethyl)-8-[(2,6-difluorbenzyl)oxy]-2-methylimidazo[1,2-a]pyridin-3-carboxamid-Dihydrochlorid

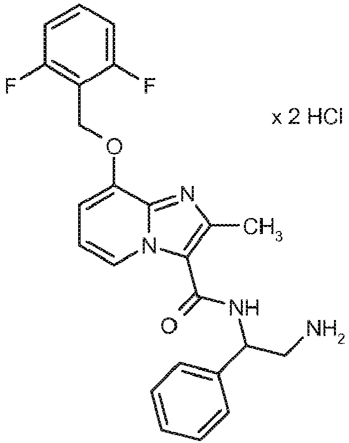

370 mg *rac*-tert.-Butyl-{2-[({8-[(2,6-difluorbenzyl)oxy]-2-methylimidazo[1,2-a]pyridin-3-yl}carbonyl)amino]-2phenylethyl}carbamat (Beispiel 80A, 0.69 mmol) wurden mit 15.75 ml einer 2 M Lösung von Salzsäure in Diethylether (31.5 mmol) versetzt und 3.5 h bei RT gerührt. Der entstandene Niederschlag wurde abfiltriert, mit Diethylether gewaschen und im Hochvakuum getrocknet. Es wurden 306 mg der Titelverbindung (85% d. Th., Reinheit 98%) erhalten.
LC-MS (Methode 2): Rₜ = 0.70 min
MS (ESpos): m/z = 437 (M-2HCl+H)⁺
¹H-NMR (400 MHz, DMSO-d₆): δ = 2.65 (s, 3H), 3.19-3.29 (m, 1H), 3.35-3.54 (m, 1H), 5.38-5.49 (m, 3H), 7.21-7.54 (m, 10H), 7.60 (quint, 1H), 8.12-8.32 (m, 2H), 8.66 (d, 1H), 9.09 (br s, 1H).

In Analogie zu Beispiel 16 wurde das in Tabelle 2 gezeigte Beispiel hergestellt.

**Tabelle 2:**

| **Beispiel** | **IUPAC-Name / Struktur (Ausbeute)** | **Analytische Daten** |
|---|---|---|
| **17** | *rac-N-*(1-Aminopropan-2-yl)-8-[(2,6-difluorbenzyl)oxy]-2-methylimidazo[1,2-a]pyridin-3-carboxamid-Dihydrochlorid | LC-MS (Methode 1): Rₜ = 0.69 min |
| | 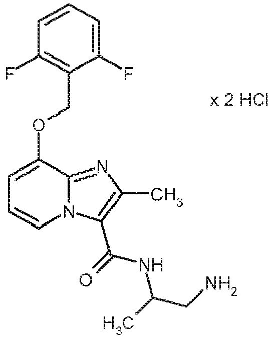 | MS (ESpos): m/z = 375 (M-HCl+H)⁺ |
| | | ¹H-NMR (400 MHz, DMSO-d₆): |
| | | δ = 1.28 (d, 3H), 2.64 (s, 3H), 2.96-3.10 (m, 2H), 4.32 (quintett, 1H), 5.44 (s, 2H), 7.22 (t, 2H), 7.33 (br s, 1H), 7.46-7.66 (m, 2H), 8.18 (br s, 3H), 8.58 (br s, 1H), 8.73 (d, 1H). |
| | (92% d. Th.) | |

### Beispiel 18

### ent-N-(2-Amino-1-phenylethyl)-8-[(2,6-difluorbenzyl)oxy]-2-methylimidazo[1,2-a]pyridin-3-carboxamid (Enantiomer B)

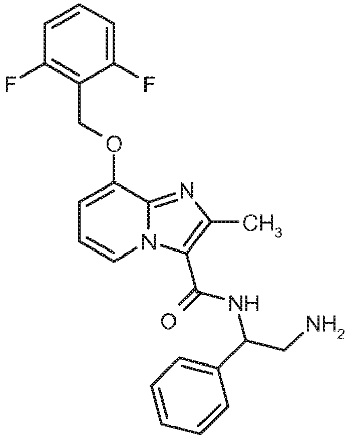

276 mg von Beispielverbindung 16 wurden in Essigsäureethylester/Dichlormethan (1:1) suspendiert und mit gesättigter, wässriger Natriumhydrogencarbonat-Lösung gewaschen. Die organische Phase wurde über Natriumsulfat getrocknet, filtriert und eingeengt. Durch präparative Trennung an chiraler Phase wurde das Rohprodukt (247 mg) in die Enantiomere getrennt [Säule: Daicel Chiralpak AD-H, 5 µm, 250 x 20 mm, Eluent: 30% iso-Hexan, 70% Ethanol + 0.2% Diethylamin, Fluß 15 ml/min; 45°C, Detektion: 220 nm]. Das erhaltene Produkt wurde nochmals über Kieselgel gereinigt (Laufmittel: Dichlormethan nach Dichlormethan:Methanol = 10:1). Es erfolgte eine weitere Reinigung mittels präparativer HPLC (RP18 Säule, Laufmittel: Methanol/Wasser-Gradient unter Zusatz von 0.1 % TFA). Die Produktfraktionen wurden eingeengt, in Essigsäureethylester gelöst und zweimal mit wenig gesättigter, wässriger Natriumhydrogencarbonat-Lösung gewaschen. Die organische Phasewurde über Natriumsulfat getrocknet, filtriert und eingeengt.
Enantiomer B:
Ausbeute: 78 mg (98% ee)
Rₜ = 11.00 min [Chiralpak AY-H, 5µm, 250 x 4.6 mm; Eluent: 60% iso-Hexan, 40% Ethanol + 0.2% Diethylamin; Fluss 1.0 ml/min; 35°C; Detektion: 235 nm].
¹H-NMR (400 MHz, DMSO-d₆): δ = 2.59 (s, 3H), 2.98-3.10 (m, 2H), 4.42 (br s, 2H), 5.09-5.18 (m, 1H), 5.30 (s, 2H), 6.90 (t, 1H), 7.01 (d, 1H), 7.19-7.30 (m, 3H), 7.32-7.44 (m, 4H), 7.59 (quint, 1H), 8.21 (d, 1H), 8.55 (d, 1H).

### Beispiel 19

### ent-N-(1-Aminopropan-2-yl)-8-[(2,6-difluorbenzyl)oxy]-2-methylimidazo[1,2-a]pyridin-3-carboxamid (Enantiomer A)

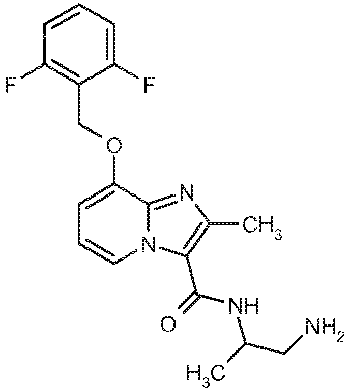

46 mg Beispielverbindung 17 wurden durch präparative Trennung an chiraler Phase in die Enantiomere getrennt [Säule: Daicel Chiralpak AY-H, 5 µm, 250 x 20 mm, Eluent: 70% iso-Hexan, 30% Ethanol + 0.2% Diethylamin, Fluß: 15 ml/min; 35°C, Detektion: 220 nm].

Enantiomer A wurde mittels präparativer HPLC (RP18 Säule, Laufmittel: Acetonitril/Wasser-Gradient unter Zusatz von 0.1% TFA) nachgereinigt. Die Produktfraktionen wurden eingeengt, in Dichlormethan gelöst und zweimal mit gesättigter, wässriger Natriumhydrogencarbonat-Lösung gewaschen. Die organische Phase wurde über Natriumsulfat getrocknet, filtriert und eingeengt.
Enantiomer A:
Ausbeute: 7.3 mg (99% ee)
Rt= 8.59 min [Chiralpak AY-H, 5µm, 250 x 4.6 mm; Eluent: 70% iso-Hexan, 30% Ethanol + 0.2% Diethylamin; Fluss 1.0 ml/min; 45°C; Detektion: 235 nm].
LC-MS (Methode 2): Rₜ = 0.56 min (Reinheit ca. 92%)
MS (ESpos): m/z = 375 (M+H)⁺

### Beispiel 20

### ent-N-(1-Aminopropan-2-yl)-8-[(2,6-difluorbenzyl)oxy]-2-methylimidazo[1,2-a]pyridin-3-carboxamid (Enantiomer B)

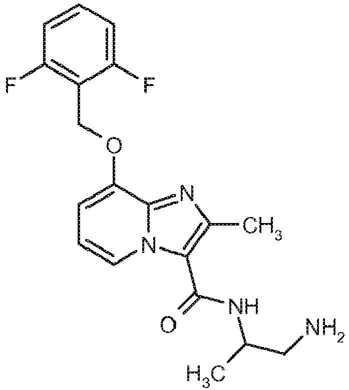

46 mg Beispielverbindung 17 wurden durch präparative Trennung an chiraler Phase in die Enantiomere getrennt [Säule: Daicel Chiralpak AY-H, 5 µm, 250 x 20 mm, Eluent: 70% iso-Hexan, 30% Ethanol + 0.2% Diethylamin, Fluß: 15 ml/min; 35°C, Detektion: 220 nm].
Enantiomer B:
Ausbeute: 13 mg (98.5% ee)
Rₜ = 10.57 min [Chiralpak AY-H, 5µm, 250 x 4.6 mm; Eluent: 70% iso-Hexan, 30% Ethanol + 0.2% Diethylamin; Fluss 1.0 ml/min; 45°C; Detektion: 235 nm].
LC-MS (Methode 2): Rₜ = 0.57 min (Reinheit ca. 92%)
MS (ESpos): m/z = 375 (M+H)⁺

### Beispiel 21

### ent-N-[2-Amino-1-(2,4-difluorphenyl)ethyl]-8-[(2,6difluorbenzyl)oxy]-2-methylimidazo[1,2-a]pyridin-3-carboxamid

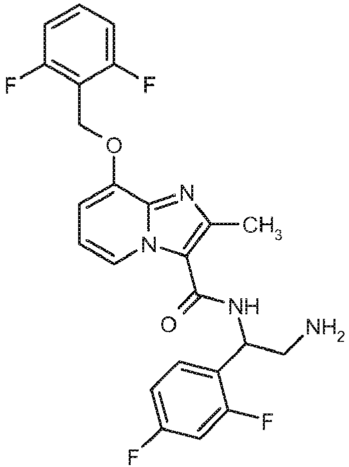

72 mg *ent-*tert*.-*Butyl*-*{2-[({8-[(2,6-difluorbenzyl)oxy]-2-methylimidazo[1,2-a]pyridin-3-yl}carbonyl)amino]-2-(2,4-difluorphenyl)ethyl}carbamat (Beispiel 81A, 0.13 mmol) wurden mit 0.63 ml 2 M Salzsäure in Diethylether (1.26 mmol) versetzt und über Nacht bei RT gerührt. Das Reaktionsgemisch wurde mit Diethylether verdünnt und der Niederschlag abfiltriert. Der Niederschlag wurde mit Essigsäureethylester und gesättigter, wässriger Natriumhydrogencarbonat-Lösung versetzt und die Phasen getrennt. Die wässrige Phase wurde zweimal mit Essigsäureethylester extrahiert und die vereinten organischen Phasen wurden über Natriumsulfat getrocknet, filtriert, eingeengt und lyophilisiert. Es wurden 51 mg der Titelverbindung (84% d. Th.) erhalten.
LC-MS (Methode 2): Rₜ = 0.78 min
MS (ESpos): m/z = 473 (M+H)⁺
¹H-NMR (400 MHz, DMSO-d₆): δ = 1.69 (br s, 2H), 2.59 (s, 3H), 2.82-2.92 (m, 2H), 5.20 (t, 1H), 5.30 (s, 2H), 6.90 (t, 1H), 7.02 (d, 1H), 7.05-7.11 (m, 1H), 7.18-7.28 (m, 3H), 7.50 (q, 1H), 7.59 (quint, 1H), 8.22 (br s, 1H), 8.53 (d, 1H).

In Analogie zu Beispiel 21 wurden die in Tabelle 3 gezeigten Beispiele hergestellt.

**Tabelle 3:**

| **Beispiel** | **IUPAC-Name / Struktur (Ausbeute)** | **Analytische Daten** |
|---|---|---|
| **22** | *ent*-*N*-(1-Aminopropan-2-yl)-6-chlor-8-[(2,6-difluorbenzyl)oxy]-2-methylimidazo[1,2-a]pyridin-3-carboxamid¹⁾ | LC-MS (Methode 2): Rₜ = 0.68 min |
| | | MS (ESpos): m/z = 409 (M+H)⁺ |
| | 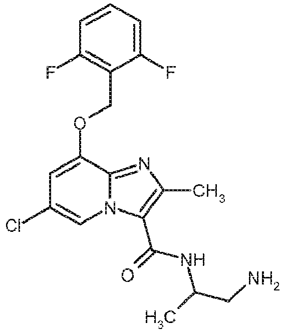 | ¹H-NMR (400 MHz, DMSO-d₆): δ = 1.24 (d, 3H), 1.63 (br s, 2H), 2.52 (s, 3H), 2.59-2.71 (m, 2H), 3.89-4.00 (m, 1H), 5.33 (s, 2H), 7.19 (s, 1H), 7.23 (t, 2H), 7.60 (quintett, 1H), 7.68 (br s, 1H), 8.70 (s, 1H). |
| | (88% d. Th.) | |
| **23** | *rac*-*N*-(2-Aminopropyl)-6-chlor-8-[(2,6-difluorbenzyl)oxy]-2-methylimidazo[1,2-a]pyridin-3-carboxamid²⁾ | LC-MS (Methode 2): Rₜ = 0.71 min |
| | | MS (ESpos): m/z = 409 (M+H)⁺ |
| | 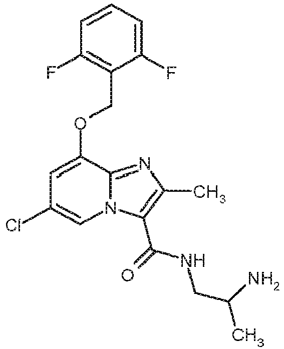 | ¹H-NMR (400 MHz, DMSO-d₆): δ = 1.01 (d, 3H), 1.70 (br s, 2H), 2.52 (s, 3H), 2.99 (sextett, 1H), 3.09-3.27 (m, 2H), 5.33 (s, 2H), 7.19 (s, 1H), 7.24 (t, 2H), 7.61 (quintett, 1H), 7.78 (br s, 1H), 8.76 (s, 1H). |
| | (78% d. Th., Reinheit ca. 90%) | |
| **24** | *N-*(1-Amino-2-methylpropan-2-yl)-6-chlor-8-[(2,6-difluorbenzyl)oxy]-2-methylimidazo[1,2-a]pyridin-3-carboxamid²⁾ | LC-MS (Methode 2): Rₜ = 0.73 min |
| | | MS (ESpos): m/z = 423 (M+H)⁺ |
| | 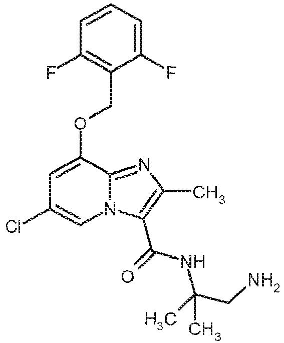 | ¹H-NMR (400 MHz, DMSO-d₆): δ = 1.33 (s, 6H), 1.89 (br s, 2H), 2.52 (s, 3H), 2.71 (s, 2H), 5.33 (s, 2H), 7.19 (s, 1H), 7.24 (t, 2H), 7.40 (s, 1H), 7.61 (quintett, 1H), 8.80 (s, 1H). |
| | (86% d. Th.) | |
| **25** | *rac*-*N*-(2-Aminopropyl)-8-[(2,6-difluorbenzyl)-oxy]-2,6-dimethylimidazo[1,2-a]pyridin-3-carboxamid ³⁾ | LC-MS (Methode 2): Rₜ = 0.55 min |
| | | MS (ESpos): m/z = 389 (M+H)⁺ |
| | 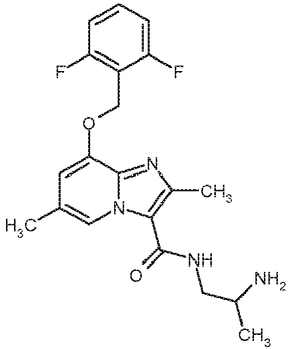 | ¹H-NMR (400 MHz, DMSO-d₆): δ = 1.01 (d, 3H), 1.52 (br s, 2H), 2.31 (s, 3H), 2.52 (s, 3H), 2.99 (sextett, 1H), 3.09-3.28 (m, 2H), 5.29 (s, 2H), 6.91 (s, 1H), 7.24 (t, 2H), 7.59 (quintett, 1H), 7.78 (br s, 1H), 8.45 (s, 1H). |
| | (73% d. Th., Reinheit ca. 90%) | |
| **26** | *N*-(1-Amino-2-methylpropan-2-yl)-8-[(2,6-difluorbaizyl)oxy]-2,6-dimethylimidazo[1,2-a]pyridin-3-carboxamid ³⁾ | LC-MS (Methode 2): Rₜ = 0.64 min |
| | | MS (ESpos): m/z = 403 (M+H)⁺ |
| | 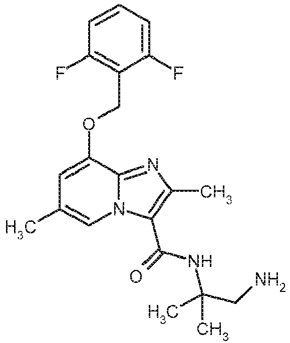 | ¹H-NMR (400 MHz, DMSO-d₆): δ = 1.32 (s, 6H), 1.70 (br s, 2H), 2.31 (s, 3H), 2.52 (s, 3H), 2.69 (s, 2H), 5.28 (s, 2H), 6.90 (s, 1H), 7.24 (t, 2H), 7.30 (s, 1H), 7.60 (quintett, 1H), 8.52 (s, 1H). |
| | (82% d. Th.) | |
| **27** | *N-*(9-Azabicyclo[3.3.1]non-3-yl)-8-[(2,6-difluorbenzyl)oxy]-2-methylimidazo[1,2-a]pyridin-3-carboramid ⁴⁾ | LC-MS (Methode 2): Rₜ = 0.62 min |
| | | MS (ESpos): m/z = 441 (M+H)⁺ |
| | 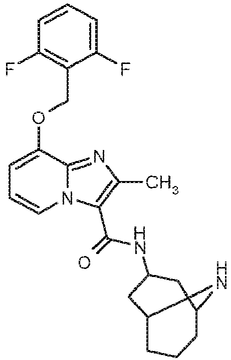 | ¹H-NMR (400 MHz, DMSO-d₆): δ = 1.63-1.90 (m, 8H), 1.94-2.03 (m, 2H), 2.48 (s, 3H), 3.28 (br s, 2H), 4.69-4.81 (m, 1H), 5.30 (s, 2H), 6.91 (t, 1H), 6.99 (d,1H), 7.24 (t, 2H), 7.59 (quintett, 1H), 7.78 (d, 1H), 8.51 (d, 1H). |
| | (81% d. Th.) | |
| **28** | *rac*-8-[(2,6-Difluorbenzyl)oxy]-N-(2,3-dihydro-1H-indol-3-yl)-2-methylimidazo[1,2-a]pyridin-3-carboxamid | LC-MS (Methode 2): Rₜ = 0.78 min |
| | | MS (ESpos): m/z = 435 (M+H)⁺ |
| | 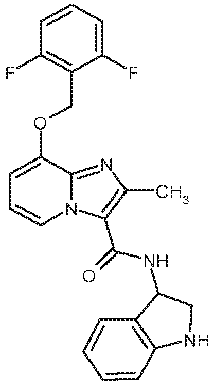 | ¹H-NMR (400 MHz, DMSO-d₆): δ = 2.50 (s, 3H), 3.31-3.40 (m,1H), 3.73-3.80 (m, 1H), 5.30 (s, 2H), 5.58-5.69 (m, 2H), 6.57-6.63 (m, 2H), 6.93 (t, 1H), 6.98-7.05 (m, 2H), 7.18-7.27 (m, 3H), 7.59 (quintett, 1H), 8.40 (d, 1H), 8.60 (d, 1H). |
| | (99% d. Th.) | |

| | | |
|---|---|---|
| ¹⁾ hergestellt aus Beispiel 119A, (Enantiomer B) ²⁾ Die Ausgangsverbindungen wurden in Diethylether gelöst, sodass eine 0.5 M Lösung entstand. ³⁾ Die Ausgangsverbindungen wurden in 1,4-Dioxan gelöst, sodass eine 0.2-0.5 M Lösung entstand. ⁴⁾ Die Reaktionszeit betrug 2.5 h. | | |

### Beispiel 29

### ent-N-(2-Aminopropyl)-6-chlor-8-[(2,6-difluorbenzyl)oxy]-2-methylimidazo[1,2-a]pyridin-3-carboxamid (Enantiomer A)

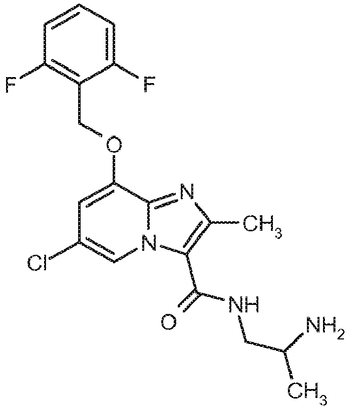

68 mg Beispiel 23 wurden durch zweifache präparative Trennung an chiraler Phase in die Enantiomere getrennt:
1) Säule: Daicel Chiralpak AD-H, 5 µm, 250 x 20 mm, Eluent: 60% iso-Hexan, 40% Ethanol + 0.2% Diethylamin, Fluß: 15 ml/min; 30°C, Detektion: 220 nm.
2) Säule: Daicel Chiralpak AD-H, 5 µm, 250 x 20 mm, Eluent: 60% iso-Hexan, 40% Iso-Propanol + 0.2% Diethylamin, Fluß: 15 ml/min; 30°C, Detektion: 220 nm.
Enantiomer A:
Ausbeute: 21 mg (99% ee)
Rt = 10.15 min [Chiralpak AY-H, 5µm, 250 x 4.6 mm; Eluent: 80% iso-Hexan, 20% Ethanol + 0.2% Diethylamin; Fluss 1.0 ml/min; 40°C; Detektion: 220 nm].

### Beispiel 30

### ent-N-(2-Aminopropyl)-6-chlor-8-[(2,6-difluorbenzyl)oxy]-2-methylimidazo[1,2-a]pyridin-3-carboxamid (Enantiomer B)

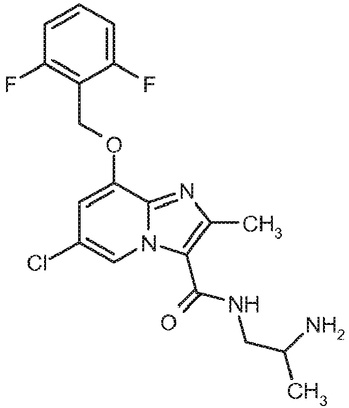

68 mg Beispiel 23 wurden durch zweifache präparative Trennung an chiraler Phase in die Enantiomere getrennt:
1) Säule: Daicel Chiralpak AD-H, 5 µm, 250 x 20 mm, Eluent: 60% iso-Hexan, 40% Ethanol + 0.2% Diethylamin, Fluß: 15 ml/min; 30°C, Detektion: 220 nm.
2) Säule: Daicel Chiralpak AD-H, 5 µm, 250 x 20 mm, Eluent: 60% iso-Hexan, 40% Iso-Propanol + 0.2% Diethylamin, Fluß: 15 ml/min; 30°C, Detektion: 220 nm.
Enantiomer B:
Ausbeute: 21 mg (95% ee)
Rₜ = 9.31 min [Chiralpak AY-H, 5µm, 250 x 4.6 mm; Eluent: 80% iso-Hexan, 20% Ethanol + 0.2% Diethylamin; Fluss 1.0 ml/min; 40°C; Detektion: 220 nm].

### Beispiel 31

### ent-N-(2-Aminopropyl)-8-[(2,6-difluorbenzyl)oxy]-2,6-dimethylimidazo[1,2-a]pyridin-3-carboxamid (Enantiomer A)

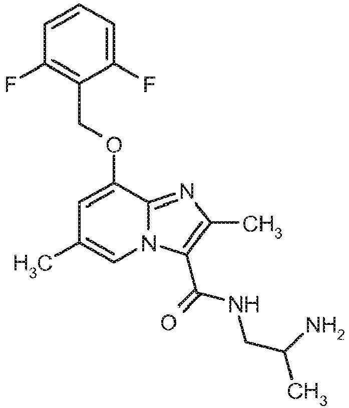

47 mg Beispiel 25 wurden durch zweifache präparative Trennung an chiraler Phase in die Enantiomere getrennt:
1) Säule: Daicel Chiralpak AD-H, 5 µm, 250 x 20 mm, Eluent: 50% iso-Hexan, 50% Ethanol + 0.2% Diethylamin, Fluß: 15 ml/min; 40°C, Detektion: 220 nm.
2) Säule: Daicel Chiralpak AD-H, 5 µm, 250 x 20 mm, Eluent: 50% iso-Hexan, 50% Iso-Propanol + 0.2% Diethylamin, Fluß: 15 ml/min; 40°C, Detektion: 220 nm.
Enantiomer A: Ausbeute: 13 mg (99% ee)
Rₜ = 7.00 min [Chiralpak AY-H, 5µm, 250 x 4.6 mm; Eluent: 70% iso-Hexan, 30% Ethanol + 0.2% Diethylamin; Fluss 1.0 ml/min; 40°C; Detektion: 220 nm].

### Beispiel 32

### ent-N-(2-Aminopropyl)-8-[(2,6-difluorbenzyl)oxy]-2,6-dimethylimidazo[1,2-a]pyridin-3-carboxamid (Enantiomer B)

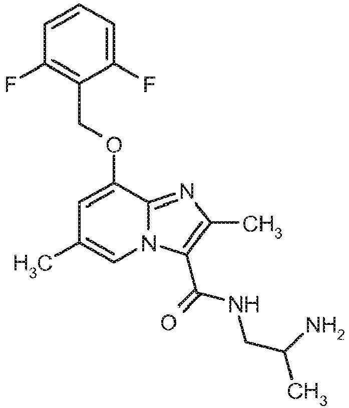

47 mg Beispiel 25 wurden durch zweifache präparative Trennung an chiraler Phase in die Enantiomere getrennt:
1) Säule: Daicel Chiralpak AD-H, 5 µm, 250 x 20 mm, Eluent: 50% iso-Hexan, 50% Ethanol + 0.2% Diethylamin, Fluß: 15 ml/min; 40°C, Detektion: 220 nm.
2) Säule: Daicel Chiralpak AD-H, 5 µm, 250 x 20 mm, Eluent: 50% iso-Hexan, 50% Iso-Propanol + 0.2% Diethylamin, Fluß: 15 ml/min; 40°C, Detektion: 220 nm.
Enantiomer B:
Ausbeute: 14 mg (97% ee)
Rₜ = 6.27 min [Chiralpak AY-H, 5µm, 250 x 4.6 mm; Eluent: 70% iso-Hexan, 30% Ethanol + 0.2% Diethylamin; Fluss 1.0 ml/min; 40°C; Detektion: 220 nm].

### Beispiel 33

### N-[2-(1-Aminocyclohexyl)ethyl]-6-chlor-8-[(2,6-difluorbenzyl)oxy]-2-methylimidazo[1,2-a]pyridin-3-carboxamid

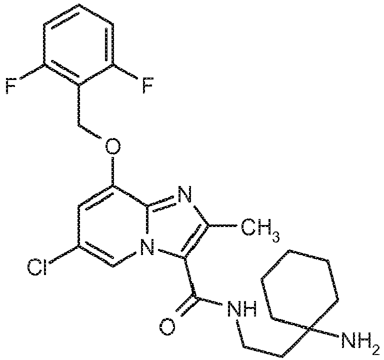

99 mg *rac*-tert.-Butyl-(1-{2-[({6-chlor-8-[(2,6-difluorbenzyl)oxy]-2-methylimidazo[1,2,a]pyridin-3-yl}carbonyl)amino]ethyl}cyclohexyl)carbamat-Trifluoracetat (Beispiel 93A, 0.14 mmol) wurden in 0.67 ml Diethylether suspendiert, mit 0.72 ml einer 2 M Lösung von Salzsäure in Diethylether (1.43 mmol) versetzt und über Nacht bei RT gerührt. Der Niederschlag wurde abfiltriert, mit Diethylether gewaschen und mit Essigsäureethylester und gesättigter, wässriger Natriumhydrogencarbonat-Lösung versetzt. Nach Phasentrennung wurde die wässrige Phase zweimal mit Essigsäureethylester extrahiert und die vereinten organischen Phasen über Natriumsulfat getrocknet. Dann wurde filtriert, das Filtrat eingeengt und lyophilisiert. Es wurden 61 mg der Titelverbindung (89% d. Th.) erhalten.
LC-MS (Methode 2): Rₜ = 0.80 min
MS (ESpos): m/z = 477 (M+H)⁺
¹H-NMR (400 MHz, DMSO-d₆): δ = 1.22-1.45 (m, 8H), 1.47-1.80 (m, 6H), 2.52 (s, 3H), 3.40 (t, 2H), 5.32 (s, 2H), 7.19 (s, 1H), 7.24 (t, 2H), 7.60 (quint, 1H), 8.55 (br s, 1H), 8.86 (s, 1H).

In Analogie zu Beispiel 33 wurden die in Tabelle 4 gezeigten Beispiele hergestellt, indem 10-20 Aquivalente einer 2 N Lösung von Salzsäure in Diethylether mit den entsprechenden N-Boc-Verbindungen unter den beschriebenen Reaktionsbedingungen umgesetzt wurden. Die Reaktionszeiten betrugen 3h - 5 d.

Gegebenenfalls erfolgten die Reinigungen mittels präparativer HPLC (RP18 Säule, Laufmittel: Acetonitril/Wasser-Gradient unter Zusatz von 0.1% Trifluoressigsäure) und/oder mittels Kieselgel-Chromatographie (Laufmittel-Gradient: Dichlormethan/Methanol). Gegebenenfalls wurden die produkthaltigen Fraktionen eingeengt, der Rückstand in Essigsäureethylester oder Dichlormethan/Methanol gelöst, mit wenig gesättigter, wässriger Natriumhydrogencarbonat-Lösung gewaschen. Anschließend wurden die organische Phase wurde über Natriumsulfat getrocknet, filtriert und das Filtrat eingeengt.

Gegebenenfalls wurde das Reaktionsgemisch vom Niederschlag abfiltriert, mit Diethylether gewaschen und am Hochvakuum getrocknet.

Gegebenenfalls wurde das Reaktionsgemisch mit Diethylether verdünnt, der Niederschlag abfiltriert und mit Essigsäureethylester oder Dichlormethan und gesättigter, wässriger Natriumhydrogencarbonat-Lösung versetzt. Die organische Phase wurde mit gesättigter, wässriger Natriumchlorid-Lösung gewaschen, über Natriumsulfat getrocknet, filtriert, das Filtrat eingeengt und im Hochvakuum getrocknet.

**Tabelle 4:**

| **Beispiel** | **IUPAC-Name / Struktur (Ausbeute)** | **Analytische Daten** |
|---|---|---|
| **34** | *rac*-*N*-(2-Aminopropyl)-8-[(2,6-difluorbenzyl)-oxy]-2-methylimidazo[1,2-a]pyridin-3-carboxamid-Dihydrochlorid | LC-MS (Methode 7): Rₜ = 0.48 min |
| | | MS (ESpos): m/z = 375 (M-2HCl+H)⁺ |
| | | ¹H-NMR (400 MHz, DMSO-d₆): |
| | 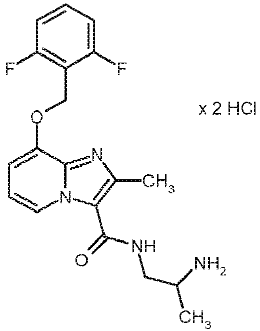 | δ = 1.24 (d, 3H), 2.62 (s, 3H), 3.31-3.56 (m, 3H), 5.32 (s, 2H), 7.21-7.32 (m, 3H), 7.45 (br s, 1H), 7.61 (quintett, 1H), 8.08 (br s, 3H), 8.54 (br s, 1H), 8.73 (d, 1H). |
| | (85% d. Th.) | |
| **35** | *ent-N-*[(2*R*)-2-Aminobutyl]-6-chlor-8-[(2,6-difluorbenzyl)oxy]-2-methylimidazo[1,2-a]pyridin-3-carboxamid | LC-MS (Methode 2): Rₜ = 0.72 min |
| | | MS (ESpos): m/z = 423 (M+H)⁺ |
| | | ¹H-NMR (400 MHz, DMSO-d₆): |
| | 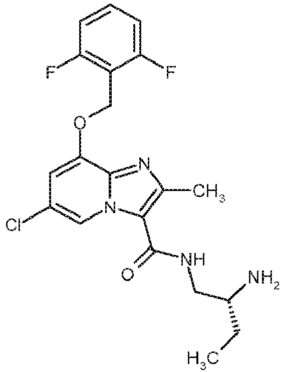 | δ = 0.90 (t, 3H), 1.18-1.30 (m,1H), 1.39-1.75 (m, 3H), 2.49-2.59 (m, 4H), 2.69-2.77 (m, 1H), 3.07-3.16 (m, 1H), 5.33 (s, 2H), 7.19 (s, 1H), 7.24 (t, 2H), 7.60 (quintett, 1H), 7.83 (br s, 1 H), 8.77 (s, 1H). |
| | (96% d. Th.) | |
| **36** | *N*-(2-Aminoethyl)-6-chlor-8-[(2,6-difluorbenzyl)-oxy]-2-methylimidazo[1,2-a]pyridin-3-carboxamid | LC-MS (Methode 2): Rₜ = 0.68 min |
| | | MS (ESpos): m/z = 395 (M+H)⁺ |
| | | ¹H-NMR (400 MHz, DMSO-d₆): |
| | 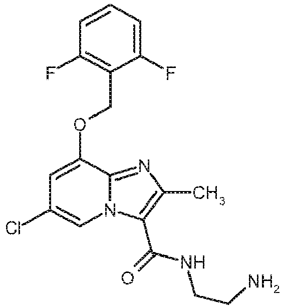 | δ = 2.50 (s, 3H), 2.73 (t, 2H), 3.32 (m, überdeckt vom Wasser-Signal, 2H), 5.33 (s, 2H), 7.19 (s, 1H), 7.24 (t, 2H), 7.60 (quintett, 1H), 7.90 (br s, 1H), 8.77 (s, 1H). |
| | (98% d. Th.) | |
| **37** | *N*-(3-Aminopropyl)-6-chlor-8-[(2,6-difluorbenzyl)oxyl-2-methylimidazo[1,2-a]pyridin-3-carboxamid | LC-MS (Methode 2): Rₜ = 0.68 min |
| | | MS (ESpos): m/z = 409 (M+H)⁺ |
| | | ¹H-NMR (400 MHz, DMSO-d₆): |
| | 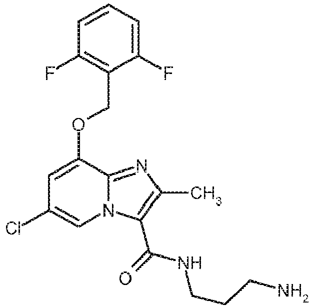 | δ = 1.62 (quintett, 2H), 2.52 (s, 3H), 2.63 (t, 2H), 3.32 (m, überdeckt vom Wasser-Signal, 2H), 5.32 (s, 2H), 7.19 (s, 1H), 7.24 (t, 2H), 7.61 (quintett, 1H), 8.10 (br s, 1H), 8.77 (s, 1H). |
| | (70% d. Th.) | |
| **38** | *N*-[(1-Aminocyclopropyl)methyl]-8-[(2,6-difluorbenzyl)oxy]-2-methylimidazo[1,2-a]pyridin-3-carboxamid | LC-MS (Methode 7): Rₜ = 0.49 min |
| | | MS (ESpos): m/z = 387 (M+H)⁺ |
| | | ¹H-NMR (400 MHz, DMSO-d₆): |
| | 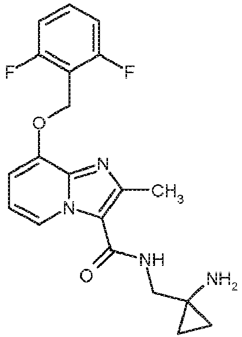 | δ = 0.42 (t, 2H), 0.54 (t, 2H), 1.94 (br s, 2H), 2.50 (s, 3H), 3.34 (d, 2H), 5.30 (s, 2H), 6.92 (t, 1H), 7.00 (d, 1H), 7.24 (t, 2H), 7.59 (quintett, 1H), 7.88 (t, 1H), 8.58 (d, 1H). |
| | (69% d. Th.) | |
| **39** | *N-*[(1Aminocyclopropyl)methyl]-6-chlor-8-[(2,6-difluorbenzyl)oxy]-2-methylimidazo[1,2-a]pyridin-3-carboxamid | LC-MS (Methode 2): Rₜ = 0.76 min |
| | | MS (ESpos): m/z = 421 (M+H)⁺ |
| | | ¹H-NMR (400 MHz, DMSO-d₆): |
| | 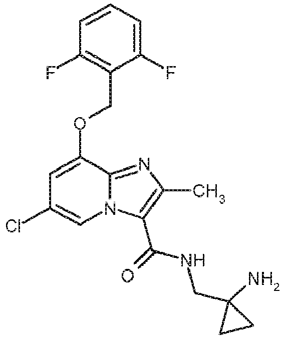 | δ = 0.44 (t, 2H), 0.55 (t, 2H), 1.98 (br s, 2H), 2.50 (s, 3H), 3.34 (d, 2H), 5.36 (s, 2H), 7.19 (s, 1H), 7.24 (t, 2H), 7.59 (quintett, 1H), 7.93 (t, 1H), 8.70 (s, 1H). |
| | (90% d. Th.) | |
| **40** | *N*-[(1-Aminocyclopropyl)methyl]-8-[(2,6-difluorbenzyl)oxy]-2,6-dimethylimidazo[1,2-a]pyridin-3-carboxamid | LC-MS (Methode 2): Rₜ = 0.60 min |
| | | MS (ESpos): m/z = 401 (M+H)⁺ |
| | | ¹H-NMR (400 MHz, DMSO-d₆): |
| | 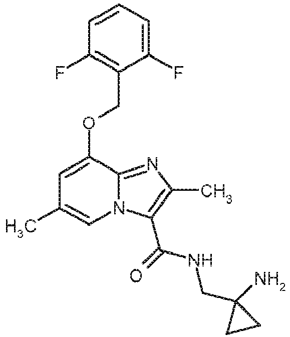 | δ = 0.44 (t, 2H), 0.54 (t, 2H), 1.98 (br s, 2H), 2.30 (s, 3H), 2.50 (s, 3H), 3.32 (d, überdeckt vom Wasser-Signal, 2H), 5.29 (s, 2H), 6.89 (s, 1H), 7.24 (t, 2H), 7.59 (quintett, 1H), 7.83 (t, 1H), 8.41 (s, 1H). |
| | (76% d. Th.) | |
| **41** | *N*-[(1-Aminocyclopentyl)methyl]-8-[(2,6-difluorbenzyl)oxy]-2-methylimidazo[1,2-a]pyridin-3-carboxamid | LC-MS (Methode 7): Rₜ = 0.54 min |
| | | MS (ESpos): m/z = 415 (M+H)⁺ |
| | | ¹H-NMR (400 MHz, DMSO-d₆): |
| | 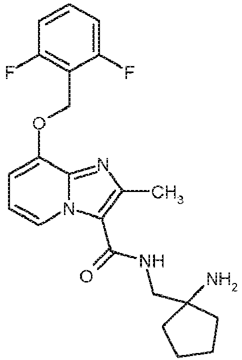 | δ = 1.32-1.44 (m, 2H), 1.50-1.61 (m, 4H), 1.65-2.10 (m, 4H), 2.50 (s, 3H), 3.32 (d, überdeckt vom Wasser-Signal, 2H), 5.30 (s, 2H), 6.92 (t, 1H), 7.01 (d, 1H), 7.23 (t, 2H), 7.59 (quintett, 1H), 7.70-7.80 (m, 1H), 8.62 (d, 1H). |
| | (77% d. Th.) | |
| **42** | *N*-[(1-Aminocyclopentyl)methyl]-6-chlor-8-[(2,6-difluorbenzyl)oxy]-2-methylimidazo[1,2-a]pyridin-3-carboxamid | LC-MS (Methode 7): Rₜ = 0.73 min |
| | | MS (ESpos): m/z = 449 (M+H)⁺ |
| | | ¹H-NMR (400 MHz, DMSO-d₆): |
| | 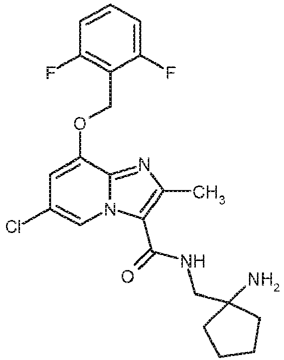 | δ = 1.32-1.45 (m, 2H), 1.50-1.80 (m, 8H), 2.50 (s, 3H), 3.32 (d, überdeckt vom Wasser-Signal, 2H), 5.33 (s, 2H), 7.19 (s, 1H), 7.24 (t, 2H), 7.60 (quintett, 1H), 7.69 (br s, 1H), 8.75 (s, 1H). |
| | (93% d. Th.) | |

### Beispiel 43

### rac-N-[2-Amino-1-{4-chlorphenyl)ethyl]-8-{cyclohexylmethoxy)-2-methylimidazo[1,2-a]pyridin-3-carboxamid

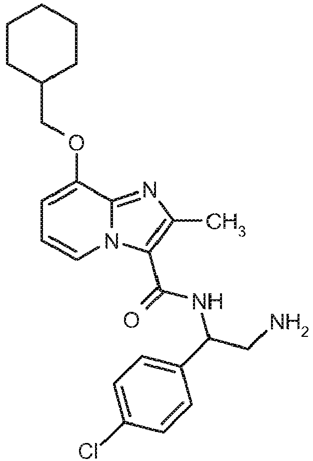

107 mg Beispiel 83A (0.16 mmol) wurden mit 2 ml 2 M Salzsäure in Diethylether (4.0 mmol) versetzt und über Nacht bei RT gerührt. Der Niederschlag wurde abfiltriert, mit Diethylether gewaschen, in Dichlormethan mit wenig Methanol gelöst, zweimal mit gesättigter, wässriger Natriumhydrogencarbonat-Lösung- und abschließend mit Wasser gewaschen. Die organische Phase wurde über Natriumsulfat getrocknet, filtriert, eingeengt und lyophilisiert. Es wurden 61 mg der Titelverbindung (85% d. Th) erhalten.
LC-MS (Methode 2): Rₜ = 0.80 min
MS (ESpos): m/z = 441 (M+H)⁺
¹H-NMR (400 MHz, DMSO-d₆): δ = 1.02-1.14 (m, 2H), 1.16-1.38 (m, 3H), 1.48-1.89 (m, 8H), 2.62 (s, 3H), 2.84-2.92 (m, 2H), 3.93 (d, 2H), 4.91-4.99 (m, 1H), 6.78 (d, 1H), 6.82 (t, 1H), 7.38-7.44 (m, 4H), 8.19 (br s, 1H), 8.46 (d, 1H).

### Beispiel 44

### rac-N-(2-Amino-1-phenylethyl)-8-(cyclohexylmethoxy)-2-methylimidazo[1,2-a]pyridin-3-carboxamid

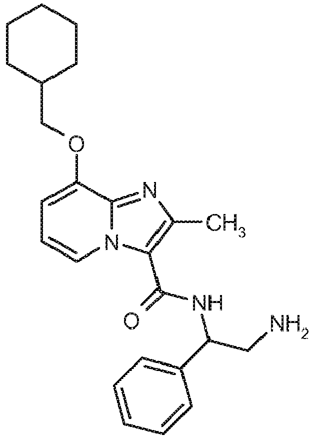

70 mg Beispiel 82A (0.1 mmol) wurden mit 2 ml 2 M Salzsäure in Diethylether (4.0 mmol) versetzt und über Nacht bei RT gerührt. Der Niederschlag wurde abfiltriert, mit Diethylether gewaschen, in einer Mischung aus Dichlormethan und wenig Methanol gelöst, zweimal mit gesättigter, wässriger Natriumhydrogencarbonat-Lösung- und abschließend mit Wasser gewaschen. Die organische Phase wurde über Natriumsulfat getrocknet, filtriert, eingeengt und lyophilisiert. Die Produktfraktion wurden mittels Dickschichtchromatographie gereinigt (Laufmittel: Toluol/Methanol = 10/1). Es wurden 31 mg der Titelverbindung (73% d. Th.) erhalten.
LC-MS (Methode 7): Rₜ = 0.68 min
MS (ESpos): m/z = 407 (M+H)⁺
¹H-NMR (400 MHz, DMSO-d₆): δ = 1.03-1.14 (m, 2H), 1.16-1.37 (m, 3H), 1.63-2.20 (m, 8H), 2.63 (s, 3H), 2.85-2.94 (m, 2H), 3.94 (d, 2H), 4.93-5.03 (m, 1H), 6.78 (d, 1H), 6.84 (t, 1H), 7.20-7.27 (m, 1H), 7.30-7.44 (m, 4H), 8.19 (br s, 1H), 8.48 (d, 1H).

### Beispiel 45

### ent-N-[2-Amino-1-(2,4-difluorphenyl)ethyl]-8-(cyclohexylmethoxy)-2-methylimidazo[1,2-a]pyridin-3-carboxamid

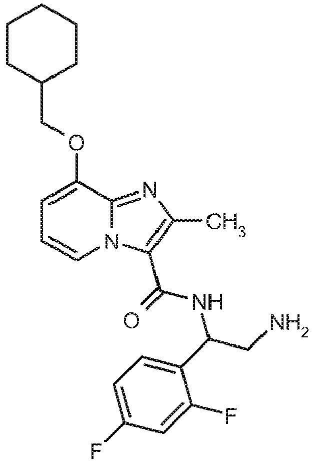

35 mg Beispiel 85A (0.05 mmol) wurden mit 0.27 ml 2M Salzsäure in Diethylether (0.53 mmol) versetzt und über Nacht bei RT gerührt. Der Niederschlag wurde abfiltriert, mit Diethylether gewaschen, in Essigsäureethylester gelöst und zweimal mit gesättigter, wässriger Natriumhydrogencarbonat-Lösung gewaschen. Die organische Phase wurde über Natriumsulfat getrocknet, filtriert und das Filtrat eingeengt und lyophilisiert. Es wurden 23 mg der Titelverbindung (93% d. Th.) erhalten.
LC-MS (Methode 7): Rₜ = 0.73 min
MS (ESpos): m/z = 443 (M+H)⁺
¹H-NMR (400 MHz, DMSO-d₆): δ = 1.02-1.13 (m, 2H), 1.14-1.36 (m, 3H), 1.63-1.99 (m, 8H), 2.62 (s, 3H), 2.83-2.94 (m, 2H), 3.95 (d, 2H), 5.18-5.23 (m, 1H), 6.78 (d, 1H), 6.83 (t, 1H), 7.07-7.12 (m, 1H), 7.18-7.26 (m,1H), 7.50 (q, 1H), 8.12-8.28 (m, 1H), 8.47 (d, 1H).

### Beispiel 46

### rac-N-[2-Amino-1-(3,4-difluorphenyl)ethyl]-8-(cyclohexylmethoxy)-2-methylimidazo[1,2-a]pyridin-3-carboxamid

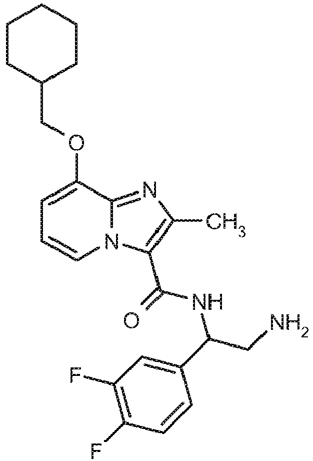

198 mg Beispiel 84A (0.30 mmol) wurden mit 1.5 ml 2 M Salzsäure in Diethylether (3.0 mmol) versetzt und über Nacht bei RT gerührt. Der Niederschlag wurde abfiltriert, gut mit Diethylether gewaschen, in Essigsäureethylester gelöst und zweimal mit gesättigter, wässriger Natriumhydrogencarbonat-Lösung gewaschen. Die organische Phase wurde über Natriumsulfat getrocknet, filtriert und das Filtrat eingeengt und lyophilisiert. Es wurden 119 mg der Titelverbindung (89% d. Th.) erhalten.
LC-MS (Methode 1): Rₜ = 1.04 min
MS (ESpos): m/z = 443 (M+H)⁺
¹H-NMR (400 MHz, DMSO-d₆): δ = 1.02-1.13 (m, 2H), 1.14-1.34 (m, 3H), 1.50-1.90 (m, 8H), 2.62 (s, 3H), 2.85-2.94 (m, 2H), 3.95 (d, 2H), 4.92-4.99 (m, 1H), 6.79 (d, 1H), 6.83 (t, 1H), 7.20-7.28 (m, 1H), 7.36-7.49 (m, 2H), 8.16 (br s, 1H), 8.48 (d, 1H).

### Beispiel 47

### ent-8-[(2,6-Difluorbenzyl)oxy]-N-(2,3-dihydro-1H-indol-3-yl)-2-methylimidazo[1,2-a]pyridin-3-carboxamid (Enantiomer B)

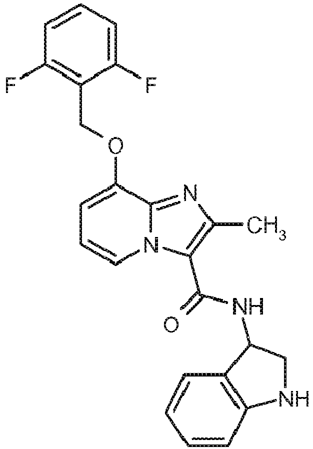

299 mg Beispiel 28 (69 mmol) wurden durch präparative Trennung an chiraler Phase in die Enantiomere getrennt [Säule: Daicel Chiralpak IA, 5 µm, 250 x 20 mm, Eluent: 40% Acetonitril, 60% Methanol, Fluß: 20 ml/min; 25°C, Detektion: 230 nm]. Die Produktfraktionen wurden in Acetonitril/Wasser gelöst und lyophilisiert.
EnantiomerB:
Ausbeute: 91 mg (99% ee)
Rₜ= 4.87 min [Chiralpak IA, 5µm, 250 x 4.6 mm; Eluent: 50% Acetonitril, 50% Methanol; Fluss 1.0 ml/min; 30°C; Detektion: 220 nm].

### Beispiel 48

### ent-N-(1-Aminopropan-2-yl)-8-[(2,6-difluorbenzyl)oxy]-2,6-dimethylimidazo[1,2-a]pyridin-3-carboxamid

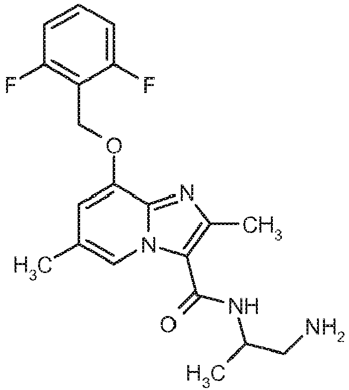

48 mg *ent-*tert.-Butyl-{2-[({8-[(2,6-difluorbenzyl)oxy]-2,6-dimethylimidazo[1,2-a]pyridin-3-yl}carbonyl)amino]propyl}carbamat (Beispiel 121A - Enantiomer A, 0.1 mmol) wurden in 2.4 ml Diethylether gelöst, mit 0.49 ml 2 M Salzsäure in Diethylether (0.98 mmol) versetzt und über Nacht bei RT gerührt. Die Reaktionslösung wurde nochmals mit 0.25 ml 2 M Salzsäure in Diethylether versetzt und über Nacht bei RT gerührt. Anschließend wurde eingeengt, der Rückstand in Dichlormethan gelöst und mit gesättigter, wässriger Natriumhydrogencarbonat-Lösung gewaschen. Die organische Phase wurde eingeengt. Es wurden 36 mg der Titelverbindung (92% d. Th.) erhalten.
LC-MS (Methode 1): Rₜ = 0.71 min
MS (ESpos): m/z = 389 (M+H)⁺
¹H-NMR (400 MHz, DMSO-d₆): δ = 1.28 (d, 3H), 2.38 (s, 3H), 2.59 (s, 3H), 2.94-3.07 (m, 2H), 4.30 (quintett, 1H), 5.37 (s, 2H), 7.05-7.40 (m, 3H), 7.61 (quintett, 1H), 8.09 (br s, 3H), 8.51 (s, 1H).

### Beispiel 49

### rac-6-Chlor-8-[(2,6-difluorbenzyl)oxy]-N-(2,3-dihydro-1H-indol-3-yl)-2-methylimidazo[1,2-a]pyridin-3-carboxamid

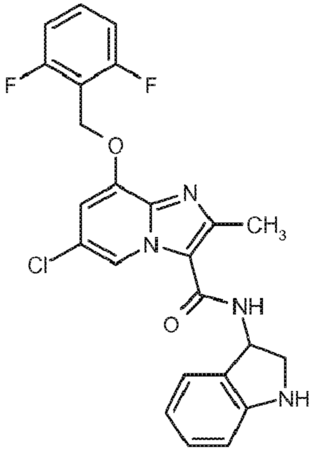

141 mg *rac*-*tert*.-Butyl-3-[({6-chlor-8-[(2,6-difluorbenzyl)oxy]-2-methylimidazo[1,2,a]pyridin-3-yl}carbonyl)amino]indolin-1-carboxylat (Beispiel 104A, 0.25 mmol) wurden in 1.2 ml Diethylether gelöst, mit 1.24 ml einer 2 M Lösung von Salzsäure in Diethylether (2.48 mmol) versetzt und über Nacht bei RT gerührt. Anschließend wurden 1.24 ml einer 2 M Lösung von Salzsäure in Diethylether (2.48 mmol) zugefügt und es wurde 3 d bei RT gerührt. Dann wurde portionsweise mit 1.24 ml einer 2 M Lösung von Salzsäure in Diethylether (2.48 mmol) versetzt und 4 h unter Rückfluss (dabei wurde Diethylether und Salzsäure in Diethylether portionsweise nachgegeben!) gerührt. Der Niederschlag wurde abfiltriert, gut mit Diethylether gewaschen und dann mit Essigsäureethylester und gesättigter, wässriger Natriumhydrogencarbonat-Lösung versetzt. Nach Phasentrennug wurde die wässrige Phase zweimal mit Essigsäureethylester extrahiert. Die vereinten organischen Phasen wurden über Natriumsulfat getrocknet, filtriert und das Filtrat eingeengt und lyophilisiert. Es wurden 93 mg der Titelverbindung (80% d. Th.) erhalten.
LC-MS (Methode 2): Rₜ = 1.02 min
MS (ESpos): m/z = 469 (M+H)⁺
¹H-NMR (400 MHz, DMSO-d₆): δ = 2.49 (s, 3H), 3.31-3.40 (m, 1H), 3.73-3.81 (m, 1H), 5.33 (s, 2H), 5.61 (q, 1H), 5.68 (s, 1H), 6.56-6.63 (m, 2H), 7.02 (t, 1H), 7.18-7.28 (m, 4H), 7.60 (quintett, 1H), 8.50 (d,1H), 8.71 (s, 1H).

### Beispiel 50

### rac-8-[(2,6-Difluorbenzyl)oxy]-2-methyl-N-(1,2,3,4-tetrahydrochinolin-4-yl)imidazo[1,2-a]pyridin-3-carboxamid

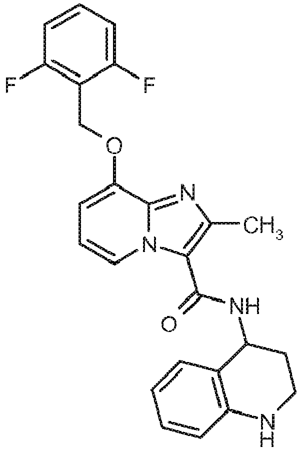

229 mg *rac*-*tert*.-Butyl-4-[({8-[(2,6-difluorbenzyl)oxy]-2-methylimidazo[1,2-a]pyridin-3-yl}carbonyl)amino]-3,4-dihydrochinolin-1(2H)-carboxylat (Beispiel 105A, 0.41 mmol) wurden in 10 ml Diethylether gelöst, mit 2.05 ml einer 2 M Lösung von Salzsäure in Diethylether (4.09 mmol) versetzt und über Nacht bei RT gerührt. Anschließend wurden nochmals 1.02 ml einer 2 M Lösung von Salzsäure in Diethylether zugegeben und über Nacht bei RT gerührt. Das Reaktionsgemisch wurde mit wenig Diethylether versetzt, der enthaltene Feststoff wurde abfiltriert und mit Diethylether gewaschen. Der Feststoff wurde in Dichlormethan mit wenig Methanol gelöst und mit gesättigter, wässriger Natriumhydrogencarbonat-Lösung gewaschen. Die organische Phase wurde eingeengt. Das Rohprodukt wurde mittels präparativer HPLC gereinigt (RP18 Säule, Laufmittel: Methanol/Wasser-Gradient unter Zusatz von 0.1% TFA). Die Produktfraktionen wurden eingeengt, in Dichlormethan aufgenommen und mit gesättigter, wässriger Natriumhydrogencarbonat-Lösung gewaschen. Die organische Phase wurde über Natriumsulfat getrocknet, filtriert und eingeengt. Es wurden 140 mg der Titelverbindung (75% d. Th.) erhalten.
LC-MS (Methode 2): Rₜ = 0.83 min
MS (ESpos): m/z = 449 (M+H)⁺
¹H-NMR (400 MHz, DMSO-d₆): δ = 1.92-2.05 (m, 2H), 2.50 (s, 3H), 3.20-3.29 (m, 2H), 5.20 (q, 1H), 5.31 (s, 2H), 5.87 (s, 1H), 6.46-6.54 (m, 2H), 6.89-6.94 (m, 2H), 7.00 (d, 1H), 7.10 (d, 1H), 7.23 (t, 2H), 7.59 (quintett, 1H), 8.27 (d, 1H), 8.58 (d, 1H).

### Beispiel 51

### ent-N-[2-Amino-1-(3,4-difluorphenyl)ethyl]-8-[(2,6-difluorbenzyl)oxy]-2,6-dimethylimidazo[1,2-a]pyridin-3-carboxamid

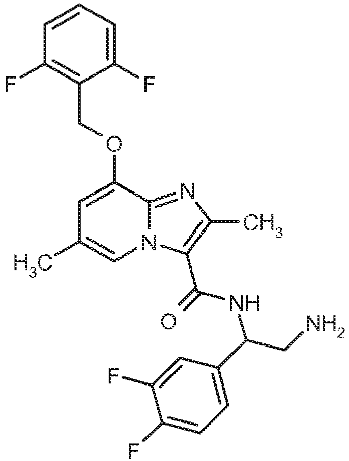

120 mg *ent*-*tert*.-Butyl-{2-[({8-[(2,6-difluorbenzyl)oxy]-2,6-dimethylimidazo[1,2-a]pyridin-3-yl}carbonyl)amino]-2-{3,4-difluorphenyl)ethyl}carbamat (Beispiel 107A, 0.20 mmol) wurden in 2 ml Diethylether gelöst, mit 1 ml einer 2 M Lösung von Salzsäure in Diethylether (2 mmol) versetzt und über Nacht bei RT gerührt. Anschließend wurde nochmals 1 ml einer 2 M Lösung von Salzsäure in Diethylether zugegeben und über Nacht bei RT gerührt. Das Reaktionsgemisch wurde abfiltriert und mit Diethylether gewaschen. Der Feststoff wurde in Dichlormethan mit wenig Methanol gelöst und mit gesättigter, wässriger Natriumhydrogencarbonat-Lösung gewaschen. Die organische Phase wurde eingeengt. Das Rohprodukt wurde mittels präparativer HPLC gereinigt (RP18 Säule, Laufmittel: Acetonitril/Wasser-Gradient unter Zusatz von 0.1% TFA). Die Produktfraktionen wurden eingeengt, in Dichlormethan aufgenommen und mit gesättigter, wässriger Natriumhydrogencarbonat-Lösung gewaschen. Die organische Phase wurde über Natriumsulfat getrocknet, filtriert und eingeengt. Es wurden 82 mg der Titelverbindung (84% d. Th.) erhalten.
LC-MS (Methode 2): Rₜ = 0.81 min
MS (ESpos): m/z = 487 (M+H)⁺
¹H-NMR (400 MHz, DMSO-d₆): δ = 2.29 (s, 3H), 2.52 (s, 3H), 2.88-3.00 (m, 2H), 4.93-5.05 (m, 1H), 5.29 (s, 2H), 6.90 (s, 1H), 7.19-7.28 (m, 3H), 7.36-7.49 (m, 2H), 7.59 (quintett, 1H), 8.17 (br s, 1H), 8.38 (s, 1H).

### Beispiel 52

### ent-8-[(2,6-Difluorbenzyl)oxy]-2-methyl-N-(1,2,3,4-tetrahydrochinolin-4-yl)imidazo[1,2-a]pyridin-3-carboxamid (Enantiomer B)

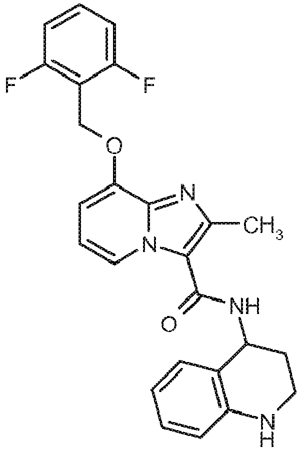

132 mg Beispiel 50 wurden durch präparative Trennung an chiraler Phase in die Enantiomere getrennt [Säule: Daicel Chiralpak AY-H, 5 µm, 250 x 20 mm, Eluent: 50% iso-Hexan, 50% Ethanol + 0.2% Diethylamin, Fluß: 15 ml/min; 45°C, Detektion: 220 nm].
EnantiomerB:
Ausbeute: 64 mg (99% ee)
Rt= 8.05 min [Daicel Chiralpak AY-H, 5µm, 250 x 4.6 mm; Eluent: 50% iso-Hexan, 50% Ethanol + 0.2% Diethylamin; Fluss 1.0 ml/min; 40°C; Detektion: 220 nm].

### Beispiel 53

### ent-N-[2-Amino-1-(3,4-difluorphenyl)ethyl]-8-[(2,6-difluorbenzyl)oxy]-6-fluor-2-methylimidazo[1,2-a]pyridin-3-carboxamid

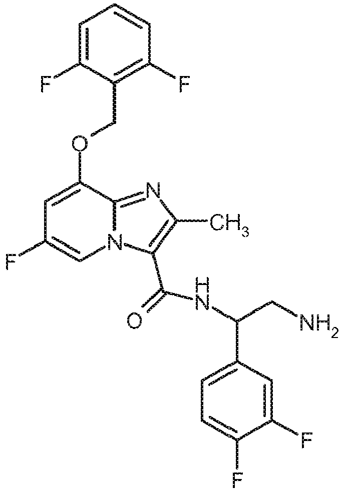

148 mg ent-tert.-Butyl-{2-[({8-[(2,6-difluorbenzyl)oxy]-6-fluor-2-methylimidazo[1,2-a]pyridin-3-yl)carbonyl)amino]-2-(3,4-difluorphenyl)ethyl)carbamat (Beispiel 106A; 0.251 mmol) wurden mit 12.5 ml einer 2 M Lösung von Salzsäure in Diethylether versetzt und über Nacht bei RT gerührt. Der Reaktionsniederschlag wurde abfiltriert, mit Diethylether nachgewaschen, in Essigsäureethylester aufgenommen und mit gesättigter, wässriger Natriumhydrogencarbonat-Lösung gewaschen. Die vereinigten organischen Phasen wurden über Magnesiumsulfat getrocknet und eingeengt. Der Rückstand wurde in Methanol gelöst und durch präparative HPLC (Methode 9) gereinigt. Es wurden 63 mg (51% d. Th.) der Titelverbindung erhalten.
LC-MS (Methode 2): Rₜ = 0.87 min
MS (ESpos): m/z = 491.1 (M+H)⁺
¹H-NMR (400 MHz, DMSO-d₆): δ = 1.61 (br. s, 2H), 2.58 (s, 3H), 3.38-3.42 (d, 2H), 4.91 (t, 1 H); 5.32 (s, 2 H); 7.20 - 7.26 (m, 4 H); 7.34 - 7.46 (m, 2 H); 7.59 (quint, 1H), 8.21 (br. s, 1H), 8.61 (d, 1H).

In Analogie zu Beispiel 53 wurden die in Tabelle 5 gezeigten Beispiele hergestellt.

**Tabelle 5:**

| **Beispiel** | **IUPAC-Name / Struktur (Ausbeute)** | **Analytische Daten** |
|---|---|---|
| **54** | *ent*-*N*-[2-Amino-1-(3,4-difluorphenyl)ethyl]-2-cyclopropyl-8-[(2,6-difluorbenzyl)oxy]imidazo[1,2-a]pyridin-3-carboxamid | ¹H-NMR (400 MHz, DMSO-d₆): |
| | | δ = 0.86 - 1.08 (m, 4H), 2.38 - 2.46 (m, 1 H); 3.12 - 3.26 (m, 2 H); 5.28 (m, 1 H); 5.30 (2,2H), 6.92 (t, 1 H); 7.02 (d, 1 H); 7.23 (t, 2 H); 7.29 - 7.34 (m, 1 H); 7.43 - 7.62 (m, 3 H); 8.53 (br. s, 1 H); 8.56 (d, 1 H). |
| | 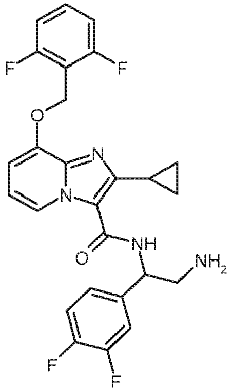 | |
| | (46% d. Th.) | |
| **55** | *ent-N-*[2-Amino-1-(3,4-difluorphenyl)ethyl]-8-[(2,6-difluorbenzyl)oxy]imidazo[1,2,a]pyridin-3-carboxamid-Dihydrochlorid | LC-MS (Methode 2): Rₜ = 0.84 min |
| | | MS (ESpos): m/z = 459.3 (M+H)⁺ |
| | | ¹H-NMR (400 MHz, DMSO-d₆): |
| | 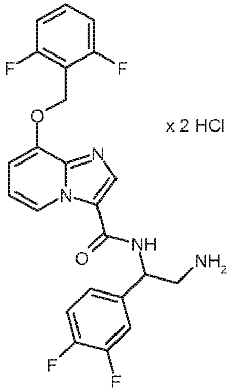 | δ = 3.29-3.35 (m, 2H), 5.38 (m, 1H), 5.41 (s, 2H), 7.20 - 7.38 (m, 4 H); 7.40-7.48 (m, 2H), 7.55 - 7.66 (m, 2H), 8.30 (br.s, 2 H); 8.90 (br. s, 1H), 9.12 (d,1H), 9.70 (br.s, 1 H). |
| | (95% d. Th.) | |
| **56** | *ent-N-(*1-Amino-3-ethoxypropan-2-yl)-8-[(2,6-difluorbenzyl)oxy]-2-methylimidazo[1,2-a]pyridin-3-carboxamid (Enantiomer A) | LC-MS (Methode 2): Rₜ = 0.59 min |
| | | MS (ESpos): m/z = 419.3 (M+H)⁺ |
| | | ¹H-NMR (400 MHz, DMSO-d₆): |
| | 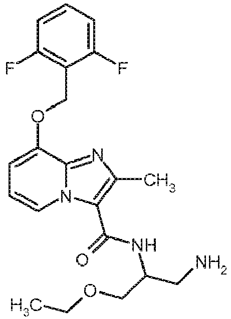 | δ = 1.10 (t, 3 H); 2.55 (s, 3H; verdeckt durch DMSO-Signal), 2.95 - 3.12 (m, 2 H); 3.47 - 3.58 (m, 4 H); 4.41 (m, 1 H); 5.32 (s, 2H), 6.95 - 7.10 (m, 2 H); 7.21 (t, 2 H); 7.59 (q, 1 H); 7.80 (br. s. 1 H); 7.91 (br. s., 2 H); 8.60 (d, 1 H). |
| | (40% d. Th.) | |
| **57** | *ent-N-(*1-Amino-3-ethoxypropan-2-yl)-6-chlor-8-[(2,6-difluorbenzyl)oxy]-2-methylimidazo[1,2-a]pyridin-3-carboxamid (Enantiomer B) | LC-MS (Methode 2): Rₜ = 0.73 min |
| | | MS (ESpos): m/z = 453.3 (M+H)⁺ |
| | | ¹H-NMR (400 MHz, DMSO-d₆): |
| | 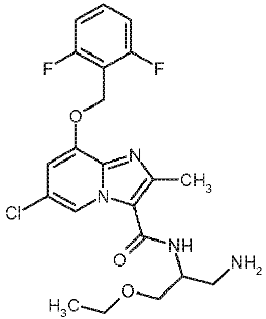 | δ = 1.10 (t, 3 H); 1.72 (br. s, 2H), 2.55 (s, 3H; überlagert durch DMSO-Signal), 2.71 (d, 2H), 3.48 - 3.53 (m, 4 H); 4.08 (m, 1H), 5.32 (s, 2H), 7.14 (s, 1 H); 7.21 (t, 2H), 7.59 (quint, 1H), 7.63 (br.s, 1 H); 8.66 (s, 1 H). |
| | (82% d. Th.) | |
| **58** | *rac-N-*[1-Amino-3-(4-fluorphenoxy)propan-2-yl]-8-[(2,6-difluorbenzyl)oxy]-2-methylimidazo[1,2-a]pyridin-3-carboxamid | LC-MS (Methode 2): Rₜ = 0.72 min |
| | | MS (ESpos): m/z = 485.3 (M+H)⁺ |
| | | ¹H-NMR (400 MHz, DMSO-d₆): |
| | 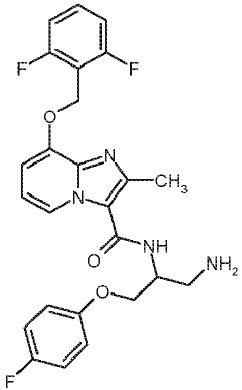 | δ = 2.55 (s, 3H; verdeckt durch DMSO-Signal), 2.85 - 2.90 (d, 2 H), 4.05 - 4.15 (m, 2 H), 4.31 (m, 1 H), 5.30 (s, 2H), 6.90 (t, 1 H), 6.95 - 7.00 (m, 3 H), 7.10 (t, 2 H), 7.21 (t, 2 H); 7.59 (q, 1 H), 7.80 (br. s. 1 H), 8.60 (d, 1 H). |
| | (68% d. Th.) | |
| **59** | *rac-N-*(1-Amino-3-phenoxypropan-2-yl)-8-[(2,6-difluorbenzyl)oxy]-2-methylimidazo[1,2-a]pyridin-3-carboxamid-Dihydrochlorid | LC-MS (Methode 2): Rₜ = 0.70 min |
| | | MS (ESpos): m/z = 467.3 (M+H)⁺ |
| | | ¹H-NMR (400 MHz, DMSO-d₆): |
| | 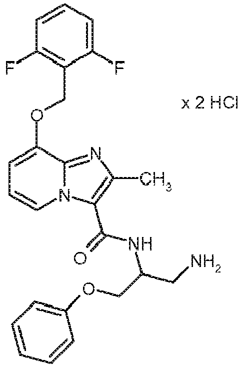 | δ = 2.60 (s, 3H), 3.10 - 3.30 (m, 2 H), 4.20 (d, 2 H), 4.65 (m, 1 H), 5.40 (s, 2H), 6.95 - 7.00 (m, 3 H), 7.20 (t, 2 H), 7.26 (m, 1 H), 7.28 (t, 2 H), 7.42 (br. s. 1 H), 7.59 (q, 1H), 8.10 (br. s, 3 H), 8.58 (br. s, 1 H), 8.70 (d, 1 H). |
| | (100% d. Th.) | |
| **60** | 8-[(2,6-Difluorbenzyl)oxy]-2-methyl-*N*-[(3*S*)-piperidin-3-yl]imidazo[1,2-a]pyridin-3-carboxamid-Dihydrochlorid | LC-MS (Methode 2): Rₜ = 0.53 min |
| | | MS (ESpos): m/z = 401.3 (M+H)⁺ |
| | | ¹H-NMR (400 MHz, DMSO-d₆): |
| | 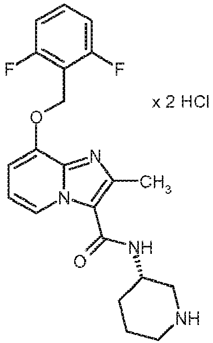 | δ = 1.63 -1.80 (m, 2 H); 1.90 - 2.00 (m, 2 H); 2.61 (s, 3H); 2.85 - 2.98 (m, 2 H); 3.20 - 3.25 (m, 2 H); 4.20 - 4.30 (m, 1 H); 5.40 (s, 2 H); 7.23 (t, 2H); 7.30 (br. s, 1 H); 7.56 (br. s, 1 H); 7.60 (quint, 1H), 8.65 (d, 1 H); 8.72 (br. s, 1 H); 9.02 (br. s, 1 H); 9.30 (br. *s,* 1 H). |
| | (99% d. Th.) | |

### Beispiel 61

### 8-[(2,6-Difluorbenzyl)oxy]-2-methyl-N-[(3S-pyrrolidin-3-yl]imidazo[1,2-a]pyridin-3-carboxamid

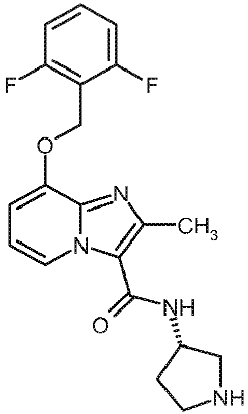

130 mg Benzyl-(3*S*)-3-[({8-[(2,6-difluorbenzyl)oxy]-2-methylimidazo[1,2,a]pyridin-3-yl}carbonyl)amino]pyrrolidin-1-carboxylat (Beispiel 115A; 0.250 mmol) wurden in 60 ml Ethanol vorgelegt, mit 17.5 mg 20%igem Palladium-(II)-hydroxid (0.025 mmol, 0.1 Äquivalente) versetzt und unter Wasserstoff-Normaldruck für zwei Stunden hydriert. Das Reaktionsgemisch wurde filtriert, eingeengt und der Rückstand per präparativer HPLC aufgereinigt. Es wurden 42 mg (44% d. Th.) der Titelverbindung erhalten.
LC-MS (Methode 2): Rₜ = 0.50 min
MS (ESpos): m/z = 387.2 (M+H)⁺
¹H-NMR (400 MHz, DMSO-d₆): δ = 1.60 - 1.70 (m, 1 H); 1.95 - 2.05 (m, 1 H); 2.55 (s, 3 H; überlagert durch DMSO-Signal); 2.61 - 2.80 (m, 2 H); 2.88 - 3.03 (m, 2 H); 4.28 - 4.37 (m, 1 H); 5.30 (s, 2 H); 6.90 (t, 1 H); 6.99 (d, 1 H); 7.21 (t, 2 H); 7.60 (q 1 H); 7.81 (d, 1 H); 8.51 (d, 1 H).

### Beispiel 62

### ent-N-[2-Amino-2-(3,4-difluorphenyl)ethyl]-8-[(2,6-difluorbenzyl)oxy]-2-methylimidazo[1,2-a]pyridin-3-carboxamid

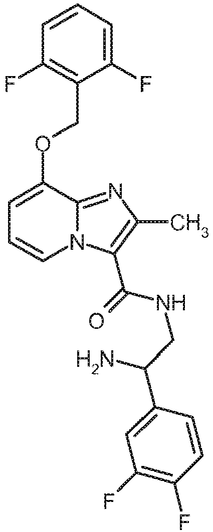

160 mg *ent*-Benzyl-{2-[({8-[(2,6-difluorbenzyl)oxy]-2-methylimidazo[1,2-a]pyridin-3-yl}carbonyl)amino]-1-(3,4-difluorphenyl)ethyl}carbamat (Intermediat 117A; 0.264 mmol) wurden in 150 ml Ethanol vorgelegt, mit 100 mg 10%igem Palladium auf Aktivkohle versetzt und unter Wasserstoff-Normaldruck für zwei Stunden hydriert. Das Reaktionsgemisch wurde filtriert und das Filtrat eingeengt. Es wurden 123 mg (99% d. Th.) der Titelverbindung erhalten.
LC-MS (Methode 2): Rₜ = 0.67 min
MS (ESpos): m/z = 373.3 (M+H)⁺
¹H-NMR (400 MHz, DMSO-d₆): δ = 2.33 (s, 3 H); 3.40 - 3.51 (m, 2 H); 4.08 (t, 1 H); 5.30 (s, 2 H); 6.88 (t, 1 H); 7.00 (d, 1 H); 7.19 - 7.24 (m, 3 H); 7.32 (q, 1 H); 7.48 (dd, 1 H); 7.56 (q, 1 H); 7.80 (t, 1 H); 8.51 (d, 1 H).

### Beispiel 63

### rac-N-(1-Amino-3-isopropoxypropan-2-yl)-8-[(2,6-difluorbenzyl)oxy]-2-methylimidazo[1,2-a]pyridin-3-carboxamid

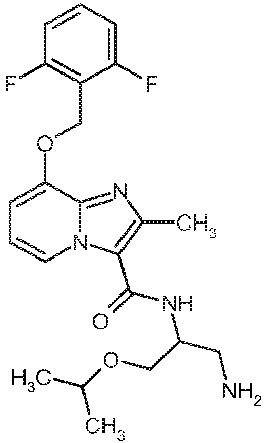

305 mg *rac*-Benzyl-{2-[({8-[(2,6difluorbenzyl)oxy]-2-methylimidazo[1,2-a]pyridin-3-yl}carbonyl)amino]-3-isopropoxypropyl}carbamat (Intermediat 116A; 0.54 mmol) wurden in 46 ml Ethanol vorgelegt, mit 44 mg 10%igem Palladium auf Aktivkohle versetzt und unter Wasserstoff-Normaldruck für 4 Stunden hydriert. Das Reaktionsgemisch wurde filtriert, eingeengt und der Rückstand per präparativer HPLC gereinigt (Säule: Macherey-Nagel VP 50/21 Nucleodur 100-5 C18 HTEC, 50x21 mm; Laufmittel: Gradient mit 0.1%iger wässriger Ammoniaklösung und Methanol). Es wurden 127 mg der Titelverbindung (54% d. Th.) erhalten.
LC-MS (Methode 2): Rₜ = 0.59 min
MS (ESpos): m/z = 433.2 (M+H)⁺
¹H-NMR (400 MHz, DMSO-d₆): δ = 1.10 (d, 6 H), 1.74 (br. s, 2 H), 2.54 (s, 3 H; überlagert durch DMSO-Signal), 2.72 (d, 2 H), 3.49 (d, 2 H), 3.58 (q, 1 H), 3.99 (m, 1 H), 5.30 (s, 2 H), 6.93 (t, 1 H), 7.00 (d, 1 H), 7.23 (t, 2 H), 7.53 (br. s, 1 H), 7.59 (q, 1 H), 8.60 (d, 1 H).

### Beispiel 64

### ent-8-[(2,6-Difluorbenzyl)oxy]-2-methyl-N-[(2R)-1-(morpholin-4-yl)hexan-2-yl]imidazo[1,2-a]pyridin-3-carboxamid

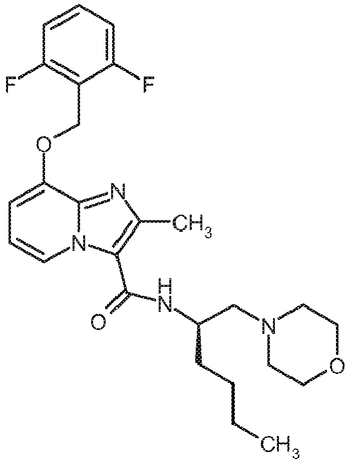

59 mg 8-[(2,6-Difluorbenzyl)oxy]-2-methyl-*N*-[(2*R*)-1-oxohexan-2-yl]imidazo[1,2-a]pyridin-3-carboxamid (0.12 mmol, 1 Äquivalent) wurden in 0.6 ml Dichlormethan suspendiert, mit 13.3 µL Morpholin (0.132 mmol, 1.1 Äquivalente) versetzt und für 3 h bei RT gerührt. Anschließend wurden 35.7 mg Natriumtriacetoxyborhydrid (0.168 mmol, 1.4 Äquivalente) zugesetzt und das Reaktionsgemisch über Nacht bei RT gerührt. Das Reaktionsgemisch wurde mit 1 N wässriger Natronlauge versetzt und dreimal mit Dichlormethan extrahiert. Die vereinigten organischen Phasen wurden mit gesättigter, wässriger Natriumchlorid-Lösung gewaschen, über Magnesiumsulfat getrocknet, filtriert und eingeengt. Der erhaltene Rückstand wurde in Methanol gelöst und durch präparative HPLC (Methode 9) aufgereinigt. Es wurden 22.4 mg (36% d. Th.) der gewünschten Verbindung als glasiger Feststoff eingeengt.
LC-MS (Methode 2): Rₜ = 0.81
MS (ESpos): m/z = 487.4 (M+H)⁺
¹H-NMR (400 MHz, DMSO-d₆): δ = 0.88 (t, 3H), 1.20 - 1.62 (m, 6H), 2.30 - 2.40 (m, 2 H), 2.54 (s + m, 3 + 4H, verdeckt durch DMSO-Signal), 3.50 - 3.60 (m, 4 H), 4.13 - 4.24 (m, 1H), 5.31 (s, 2H), 6.92 (t, 1H), 6.99 (d, 1H), 7.23 (t, 2H), 7.59 (q, 1H), 7.63 (d, 1 H), 8.50 (d, 1H).

In Analogie zu Beispiel 64 wurden die in Tabelle 6 gezeigten Beispiele hergestellt.

**Tabelle 6:**

| **Beispiel** | **IUPAC-Name / Struktur (Ausbeute)** | **Analytische Daten** |
|---|---|---|
| **65** | *ent*-8-[(2,6-Difluorbenzyl)oxyl-2-methyl-*N*-[(2*R*)-1-(piperidin-1-yl)hexan-2-yl]imidazo[1,2-a]pyridin-3-carboxamid | LC-MS (Methode 2): Rₜ = 0.53 min |
| | | MS (ESpos): m/z = 401.3 (M+H)⁺ |
| | | ¹H NMR (400 MHz, DMSO-d₆): |
| | 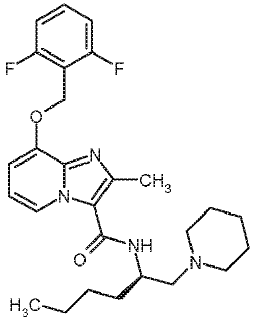 | δ = 0.88 (t, 3H), 1.20 - 1.62 (m, 12 H), 2.20 - 2.54 (m, 6 H), 2.54 (s, 3 H, verdeckt durch DMSO-Signal), 4.11 - 4.24 (m,1H), 5.30 (s, 2H), 6.91 (t, 1H), 6.98 (d, 1H), 7.21 (t, 2H), 7.57 (q, 1H), 7.61 (d, 1H), 8.51 (d, 1H). |
| | (17 % d. Th.) | |
| **66** | *ent*-8-[(2,6-Difluorbenzyl)oxy]-2-methyl-*N*-[(2*R*)-1-(pyrrolidin-1-yl)hexan-2-yl]imidazo[1,2-a]pyridin-3-carboxamid | LC-MS (Methode 5): Rₜ = 1.52 min |
| | | MS (ESpos): m/z = 471.3 (M+H)⁺ |
| | | ¹H-NMR (400 MHz, DMSO-d₆): |
| | 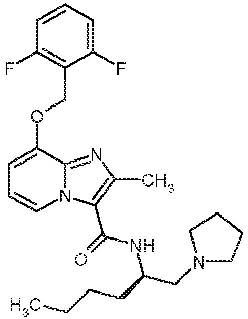 | δ = 0.88 (t, 3H), 1.20 - 1.72 (m, 10 H), 2.40 - 2.60 (m, 9 H: teilweise verdeckt durch DMSO-Signal), 4.06 - 4.18 (m, 1H), 5.30 (s, 2H), 6.90 (t, 1H), 6.98 (d, 1H), 7.21 (t, 2H), 7.58 (q, 1H);, 7.63 (d, 1 H), 8.50 (d, 1H). |
| | (31 % d. Th.) | |
| | Zugabe des Reduktionsmittels bereits nach 10 min. | |
| **67** | *ent*-8-[(2,6-Difluorbenzyl)oxy]-2-methyl-*N-*[(2*R*)-1-(4-methylpiperazin-1-yl)hexan-2-yl]imidazo[1,2-a]pyridin-3-carboxamid | LC-MS (Methode 2): Rt = 0.78 min |
| | | MS (ESpos): m/z = 500.4 (M+H)+ |
| | | ¹H-NMR (400 MHz, DMSO-d6): |
| | 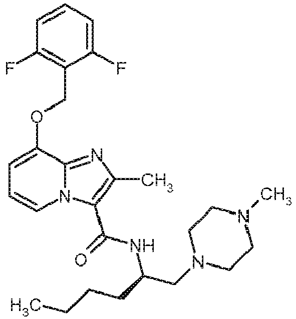 | δ = 0.90 (t, 3H), 1.35 - 1.62 (m, 6 H), 2.10 (s, 3 H), 2.20 - 2.55 (m, 10 H: teilweise verdeckt durch DMSO-Signal), 2.56 (s, 3 H), 4.10 - 4.21 (m, 1H), 5.30 (s, 2H), 6.90 (t, 1H), 6.98 (d, 1H), 7.21 (t, 2H), 7.58 (q, 1H), 7.63 (d, 1 H), 8.50 (d, 1H). |
| | (24 % d. Th.) | |
| | Verwendung von Dichlormethan als Lösungsmittel anstelle von Dimethylformamid | |
| **68** | *ent-*8-[(2,6-Difluorbenzyl)oxy]-*N*-[(2*R*)-1-(dimethylamino)hexan-2-yl]-2-methylimidazo[1,2-a]pyridin-3-carboxamid | LC-MS (Methode 2): Rₜ = 0.79 min |
| | | MS (ESpos): m/z = 445.4 (M+H)⁺ |
| | | ¹H-NMR (400 MHz, DMSO-d₆): |
| | 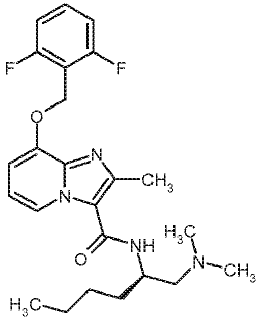 | δ = 0.88 (t, 3H), 1.25 - 1.65 (m, 6 H), 2.18 (s, 6 H), 2.20 (dd, 1 H), 2.40 (dd, 1 H), 2.56 (s, 3 H, verdeckt durch DMSO-Signal), 4.05 - 4.18 (m, 1H), 5.30 (s, 2H), 6.90 (t, 1H), 6.98 (d, 1H), 7.21 (t, 2H), 7.58 (q, 2H), 8.50 (d, 1H). |
| | (30 % d. Th.) | |
| | Verwendung von 1,2-Dichlorethan als Lösungsmittel anstelle von Dimethylformamid | |
| **69** | *ent*-*N*-[(2*R*)-1-(Diethylamino)hexan-2-yl]-8-[(2,6-difluorbenzyl)oxy]-2-methylimidazo[1,2-a]pyridin-3-carboxamid | LC-MS (Methode 2): Rₜ = 0.85 min |
| | | MS (ESpos): m/z = 473.4 (M+H)⁺ |
| | | ¹H-NMR (400 MHz, DMSO-d₆): |
| | 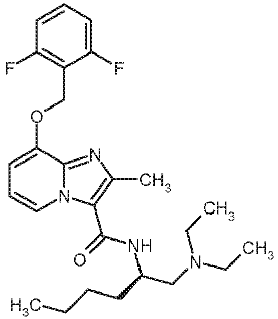 | δ = 0.88 (t, 3H), 0.95 (t, 6 H), 1.35 - 1.70 (m, 6 H), 2.40 (dd, 1 H), 2.49 - 2.53 (m, 5 H, überlagert durch DMSO-Signal), 2.56 (s, 3 H), 4.02 - 4.12 (m, 1H), 5.30 (s, 2H), 6.90 (t, 1H), 6.98 (d, 1H), 7.21 (t, 2H), 7.55 - 7.62 (m, 2H), 8.52 (d, 1H). |
| | (19 % d. Th.) | |
| **70** | ent-8-[(2,6-Difluorbenzyl)oxy]-*N*-{(2*R*)-1-[(2-methoxyethyl)amino]hexan-2-yl}-2-methylimidazo[1,2-a]pyridin-3-carboxamid | LC-MS (Methode 2): Rₜ = 0.81 min |
| | | MS (ESpos): m/z = 475.4 (M+H)⁺ |
| | | ¹H-NMR (400 MHz, DMSO-d₆): |
| | 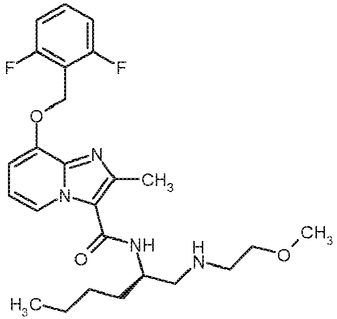 | δ = 0.87 (t, 3H), 1.25 -1.68 (m, 6 H), 2.56 (s, 3 H, überlagert durch DMSO-Signal), 2.60 - 2.78 (m, 4 H), 3.33 (t, 2 H), 4.02 -4.12 (m,1H), 5.30 (s, 2H), 6.90 (t, 1H), 6.98 (d, 1H), 7.21 (t, 2H), 7.55 - 7.62 (m, 2H), 8.51 (d, 1H). |
| | (35 % d. Th.) | |
| | Verwendung von Dichlormethan als Lösungsmittel anstelle von Dimethylformamid | |

### Beispiel 71

### 8-[(2,6-Difluorbenzyl)oxy]-N-(9-ethyl-9-azabicyclo[3.3.1]non-3-yl)-2-methylimidazo[1,2-a]pyridin-3-carboxamid

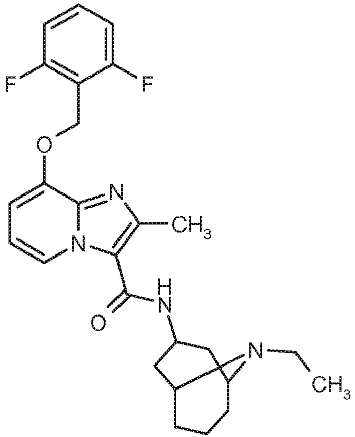

60 mg *N*-(9-Azabicyclo[3.3.1]non-3-yl)-8-[(2,6-difluorbenzyl)oxy]-2-methylimidazo[1,2-a]pyridin-3-carboxamid (Beispiel 27, 0.14 mmol) wurden in 1.4 ml abs. THF vorgelegt, mit 4.2 mg Natriumhydrid (Reinheit 95%, 0.16 mmol) versetzt und 15 min bei RT gerührt. Anschließend wurden 0.011 ml Iodethan (0.14 mmol) zugetropft und es wurde über Nacht bei RT gerührt. Das Reaktionsgemisch wurde mit Essigsäureethylester verdünnt und zweimal mit Wasser gewaschen. Die organische Phase wurde mit gesättigter, wässriger Natriumchlorid-Lösung gewaschen, über Natriumsulfat getrocknet und filtriert. Das Filtrat wurde eingeengt und getrocknet. Das Rohprodukt wurde mittels präparativer HPLC gereinigt (RP18 Säule, Laufmittel: Acetonitril/Wasser-Gradient unter Zusatz von 0.1 % TFA). Die produkthaltigen Fraktionen wurden eingeengt, der Rückstand in Essigsäureethylester aufgenommen und mit gesättigter, wässriger Natriumhydrogencarbonat-Lösung gewaschen. Die organische Phase wurde über Natriumsulfat getrocknet, filtriert und das Filtrat eingeengt und lyophilisiert. Es wurden 35 mg der Titelverbindung (55% d. Th.) erhalten.
LC-MS (Methode 2): Rₜ = 0.68 min
MS (ESpos): m/z = 469 (M+H)⁺
¹H-NMR (400 MHz, DMSO-d₆): δ = 0.97 (t, 3H), 1.50 (d, 2H), 1.60-1.97 (m, 8H), 2.50 (s, 3H), 2.69 (q, 2H), 2.96 (s, 2H), 4.63-4.79 (m, 1H), 5.30 (s, 2H), 6.91 (t, 1H), 6.99 (d, 1H), 7.23 (t, 2H), 7.59 (quintett, 1H), 7.71 (d, 1H), 8.52 (d, 1H).

### Beispiel 72

### N-(2-Amino-2-methylpropyl)-8-[(2,6-difluorbenzyl)oxy]-2-methylimidazo[1,2-a]pyridin-3-carboxamid

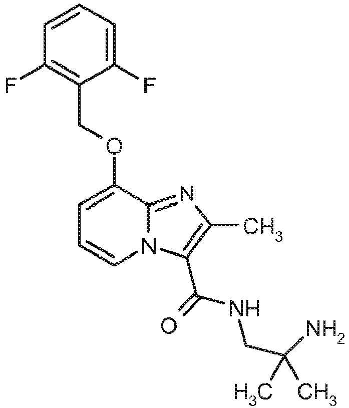

100 mg 8-[(2,6-Diftuorbenzyl)oxy]-2-methylimidazo[1,2-a]pyridin-3-carbonsäure (0.31 mmol), 151 mg (Benzotriazol-1-yloxy)bisdimethylaminomethyliumfluorborat (TBTU, 0.47 mmol) und 127 mg 4-Methylmorpholin (1.26 mmol) wurden in 2 ml DMF vorgelegt. Anschließend wurden 55 mg 2-Methylpropan-1,2-diamin (0.63 mmol) zugesetzt und über Nacht bei RT gerührt. Die Reaktionslösung wurde mittels präparativer HPLC gereinigt (RP18 Säule, Laufmittel: Acetonitril/Wasser-Gradient unter Zusatz von 0.1% TFA). Das Rohprodukt wurde in Essigsäureethylester aufgenommen und zweimal mit gesättigter, wässriger Natriumhydrogencarbonat-Lösung gewaschen. Die wässrige Phase wurde zweimal mit Essigsäureethylester extrahiert und die vereinten organischen Phasen über Natriumsulfat getrocknet. Dann wurde filtriert, das Filtrat eingeengt und lyophilisiert. Es wurden 80 mg der Titelverbindung (66% d. Th.) erhalten.
LC-MS (Methode 2): Rₜ = 0.55 min
MS (ESpos): m/z = 389 (M+H)⁺
¹H-NMR (400 MHz, DMSO-d₆): δ = 1.08 (s, 6H), 2.00 (br s, 2H), 2.52 (s, 3H), 3.22 (d, 2H), 5.30 (s, 2H), 6.91 (t, 1H), 7.01 (d, 1H), 7.23 (t, 2H), 7.59 (quintett, 1H), 7.74 (br s, 1H), 8.64 (d, 1H).

### Beispiel 73

### N-(2-Amino-2-methylpropyl)-6-chlor-8-[(2,6-difluorbenzyl)oxy]-2-methylimidazo[1,2,a]pyridin-3-carboxamid

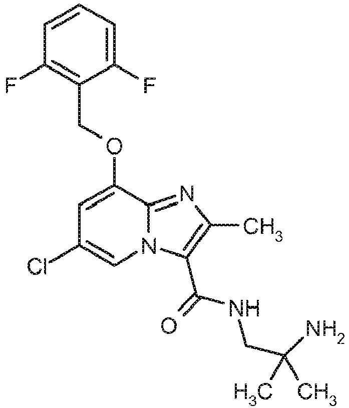

70 mg 6-Chlor-8-[(2,6-difluorbenzyl)oxy]-2-methylimidazo[1,2-a]pyridin-3-carbonsäure (0.20 mmol), 96 mg (Benzotriazol-1-yloxy)bisdimethylaminomethyliumfluorborat (TBTU, 0.30 mmol) und 80 mg 4-Methylmorpholin (0.79 mmol) wurden in 1 ml DMF vorgelegt. Anschließend wurden 35 mg 2-Methylpropan-1,2-diamin (0.40 mmol) zugesetzt und über Nacht bei RT gerührt. Die Reaktionslösung wurde mittels präparativer HPLC gereinigt (RP18 Säule, Laufmittel: Acetonitril/Wasser-Gradient unter Zusatz von 0.1% TFA). Das Produkt wurde in Essigsäureethylester aufgenommen und zweimal mit gesättigter, wässriger Natriumhydrogencarbonat-Lösung gewaschen. Die wässrige Phase wurde zweimal mit Essigsäureethylester extrahiert, die vereinten organischen Phasen über Natriumsulfat getrocknet und filtriert. Das Filtrat wurde eingeengt und lyophilisiert. Es wurden 38 mg der Titelverbindung (45% d. Th.) erhalten.
LC-MS (Methode 1): Rₜ = 0.88 min
MS (ESpos): m/z = 423 (M+H)⁺
¹H NMR (400 MHz, DMSO-d₆): δ = 1.05 (s, 6H), 2.04 (br s, 2H), 2.50 (s, 3H), 3.22 (d, 2H), 5.35 (s, 2H), 7.20 (d,1H), 7.24 (t, 2H), 7.61 (quintett, 1H), 7.80 (br s, 1H), 8.75 (d, 1H).

### Beispiel 74

### N-(2-Amino-2-methylpropyl)-8-[(2,6-difluorbenzyl)oxy]-2,6-dimethylimidazo[1,2-a]pyridin-3-carboxamid

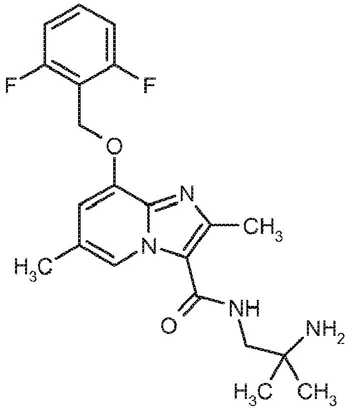

100 mg 8-[(2,6-Difluorbenzyl)oxy]-2,6-dimethylimidazo[1,2-a]pyridin-3-carbonsäure (0.30 mmol), 145 mg (Benzotriazol-1-yloxy)bisdimethylaminomethyliumfluorborat (TBTU, 0.45 mmol) und 122 mg 4-Methylmorpholin (1.20 mmol) wurden in 1.9 ml DMF vorgelegt. Anschließend wurden 53 mg 2-Methylpropan-1,2-diamin (0.60 mmol) zugesetzt und über Nacht bei RT gerührt. Die Reaktionslösung wurde mittels präparativer HPLC gereinigt (RP18 Säule, Laufmittel: Acetonitril/Wasser-Gradient unter Zusatz von 0.1% TFA). Die Produktfraktionen wurden in Dichlormethan aufgenommen und mit gesättigter, wässriger Natriumhydrogencarbonat-Lösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 94 mg der Titelverbindung (78% d. Th.) erhalten.
LC-MS (Methode 2): Rₜ = 0.61 min
MS (ESpos): m/z = 403 (M+H)⁺
¹H-NMR (400 MHz, DMSO-d₆): δ = 1.05 (s, 6H), 1.53 (br s, 2H), 2.31 (s, 3H), 2.52 (s, 3H), 3.20 (d, 2H), 5.29 (s, 2H), 6.91 (s, 1H), 7.24 (t, 2H), 7.59 (quintett, 1H), 7.64-7.72 (m,1H), 8.47 (s, 1H).

### Beispiel 75

### rac-8-[(2,6-Difluorbenzyl)oxy]-2-methyl-N-(1,2,3,4-tetrahydrochinolin-3-yl)imidazo[1,2-a]pyridin-3-carboxamid

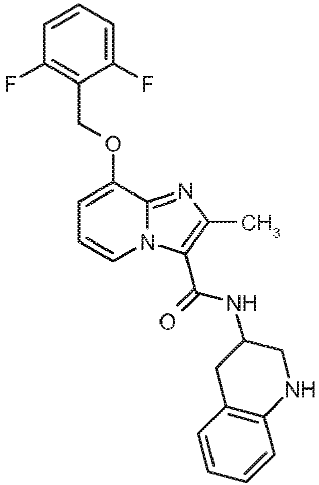

70 mg 8-[(2,6-Difluorbenzyl)oxy]-2-methylimidazo[1,2-a]pyridin-3-carbonsäure (0.22 mmol), 54 mg (Benzotriazol-1-yloxy)bisdimethylaminomethyliumfluorborat (TBTU, 0.26 mmol) und 133 mg 4-Methylmorpholin (1.32 mmol) wurden in 1.5 ml Dichlormethan vorgelegt. Nach 10 min bei RT wurden 53 mg 1,2,3,4-Tetrahydrochinolin-3-amin (0.24 mmol) zugesetzt und es wurde über Nacht bei RT gerührt. Die Reaktionslösung wurde mit ca. 5 ml Wasser versetzt, noch 30 min nachgerührt, der ausgefallene Niederschlag abfiltriert, gut mit Wasser gewaschen und im Hochvakuum getrocknet. Das Rohprodukt wurde mittels präparativer Dünnschichtchromatographie gereinigt (Dichlormethan/Methanol 20:1). Es wurden 52 mg der Titelverbindung (52% d. Th.) erhalten.
LC-MS (Methode 2): Rₜ = 0.88 min
MS (ESpos): m/z = 449 (M+H)⁺
¹H-NMR (400 MHz, DMSO-d₆): δ = 2.39 (s, 3H), 2.79-2.88 (m, 1H), 2.91-2.99 (m, 1H), 3.09-3.18 (m, 1H), 3.32-3.40 (m, 1H), 4.21-4.30 (m, 1H), 5.30 (s, 2H), 5.70 (s, 1H), 6.47 (t, 1H), 6.51 (d, 1H), 6.90 (d, 2H), 6.93 (t, 1H), 7.01 (d, 1H), 7.23 (t, 2H), 7.59 (quintett, 1H), 7.63 (d, 1H), 8.61 (d, 1H).

In Analogie zu Beispiel 75 wurden die in Tabelle 7 gezeigten Beispiele hergestellt, indem die entsprechende Carbonsäure mit den entsprechenden, kommerziell erhältlichen Aminen unter den in der repräsentativen Arbeitsvorschrift 2 beschriebenen Reaktionsbedingungen umgesetzt.

**Tabelle 7:**

| **Beispiel** | **IUPAC-Name / Struktur (Ausbeute)** | **Analytische Daten** |
|---|---|---|
| **76** | *rac*-8-[(2,6-Difluorbenzyl)oxy]-2,6-dimethyl-*N-*(1,2,3,4-tetrahydrochinolin-3-yl)imidazo[1,2-a]pyridin-3-carboxamid | LC-MS (Methode 2): Rₜ = 0.90 min |
| | | MS (ESpos): m/z = 463 (M+H)⁺ |
| | | ¹H-NMR (400 MHz, DMSO-d₆): |
| | 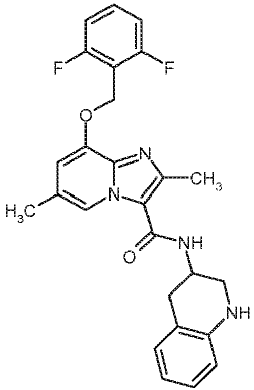 | δ = 2.32 (s, 3H), 2.38 (s, 3H), 2.79-2.88 (m, 1H), 2.91-2.99 (m, 1H), 3.09-3.18 (m,1H), 3.32-3.40 (m, 1H), 4.20-4.29 (m, 1H), 5.28 (s, 2H), 5.80 (s, 1H), 6.48 (t, 1H), 6.52 (d, 1H), 6.86-6.92 (m, 3H), 7.23 (t, 2H), 7.55-7.64 (m, 2H), 8.42 (s, 1H). |
| | (80% d. Th.) | |
| **77** | *rac*-8-[(2,6-Difluorbenzyl)oxy]-2,6-dimethyl-*N*-[1-(1-methylpiperidin-4-yl)ethyl]imidazo[1,2-a]pyridin-3-carboxamid | LC-MS (Methode 5): Rₜ = 1.28 min |
| | | MS (ESpos): m/z = 457 (M+H)⁺ |
| | | ¹H-NMR (400 MHz, DMSO-d₆): |
| | 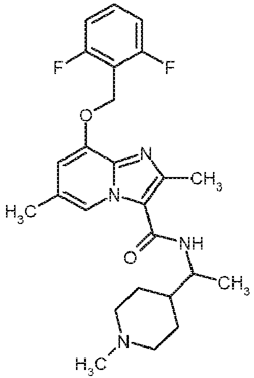 | δ = 1.14 (d, 3H), 1.21-1.48 (m, 3H), 1.73 (t, 2H), 2.00 (br s, 2H), 2.27 (br s, 3H), 2.30 (s, 3H), 2.48 (s, 3H), 2.85-2.98 (m, 2H), 3.91 (Sextett, 1H), 5.28 (s, 2H), 6.88 (s, 1H), 7.23 (t, 2H), 7.59 (quintett, 1H), 7.68 (d, 1H), 8.31 (s, 1H). |
| | (80% d. Th.) | |
| **78** | *rac*-6-Chlor-8-[(2,6-difluorbenzyl)oxy]-2-methyl-*N*-[1-(1-methylpiperidin-4-yl)ethyl]imidazo[1,2-a]pyridin-3-carboxamid | LC-MS (Methode 2): Rₜ = 0.70 min |
| | | MS (ESpos): m/z = 477 (M+H)⁺ |
| | | ¹H-NMR (400 MHz, DMSO-d₆): |
| | 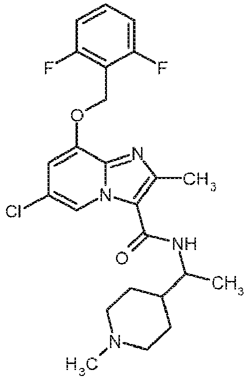 | δ =1.14 (d, 3H), 1.16-1.44 (m, 3H), 1.60-1.86 (m, 4H), 2.14 (s, 3H), 2.50 (s, 3H), 2.79 (d, 2H), 3.91 (Sextett, 1H), 5.34 (s, 2H), 7.18 (s, 1H), 7.22 (t, 2H), 7.60 (quintett, 1H), 7.69 (d, 1H), 8.61 (s, 1H). |
| | (98% d. Th.) | |
| **79** | 8-[(2,6-Difluorbenzyl)oxy]-*N*-(2-[(4-fluorphenyl)-amino]ethyl}-2-methylimidazo[1,2-a]pyridin-3-carboxamid | LC-MS (Methode 2): Rₜ = 0.88 min |
| | | MS (ESpos): m/z = 455 (M+H)⁺ |
| | | ¹H-NMR (400 MHz, DMSO-d₆): |
| | 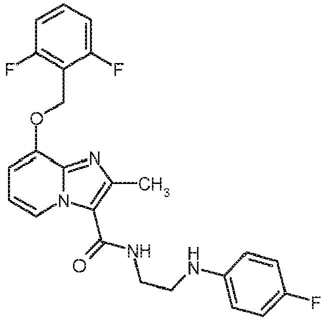 | δ = 2.50 (s, 3H), 3.20 (q, 2H), 3.47 (q, 2H), 5.30 (s, 2H), 5.69 (t, 1H), 6.60 (dd, 2H), 6.88-6.97 (m, 3H), 7.02 (d, 1H), 7.23 (t, 2H), 7.59 (quintett, 1H), 7.88 (t, 1H), 8.68 (d, 1H). |
| | (53% d. Th.) | |
| **80** | *rac*-8-[(2,6-Difluorbenzyl)oxy]-2-methyl-*N-*(1,2,3,4-tetrahydroisochinolin-1-ylmethyl)-imidazo[1,2-a]pyridin-3-carboxamid^{a)} | LC-MS (Methode 2): Rₜ = 0.70 min |
| | | MS (ESpos): m/z = 463 (M+H)⁺ |
| | | ¹H-NMR (400 MHz, DMSO-d₆): |
| | 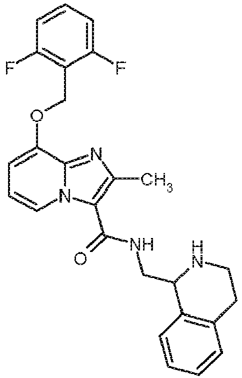 | δ = 2.43 (s, 3H), 2.67-2.75 (m, 2H), 2.84-2.91 (m, 1H), 3.06-3.16 (m, 1H), 3.555-3.70 (m, 2H), 4.12 (dd, 1H), 5.29 (s, 2H), 6.91 (t, 1H), 7.02 (d, 1H), 7.07-7.18 (m, 3H), 7.19-7.28 (m, 3H), 7.59 (quintett, 1H), 7.69 (t, 1H), 8.70 (d, 1H). |
| | (28% d. Th.) | |

| | | |
|---|---|---|
| ^{a)} Es erfolgte eine zweite chromatographische Trennung: [Säule: Sunfire C18, 5 µm, 250 x 20 mm, Eluent: 60% Wasser, 35% Methanol + 5% 1%ige wässrige TFA-Lsg., Fluß: 25 ml/min; 25°C, Detektion: 210 nm]. | | |

### Beispiel 81

### ent-8-[(2,6-Difluorbenzyl)oxy]-2-methyl-N-(1,2,3,4-tetrahydrochinolin-3-yl)imidazo[1,2-a]pyridin-3-carboxamid (Enantiomer A)

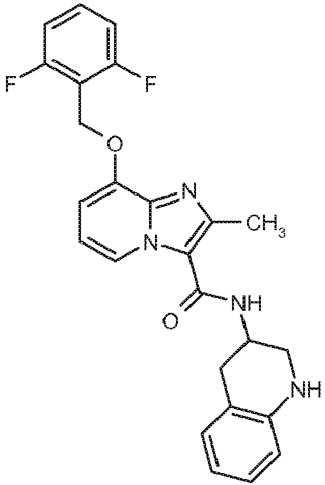

38 mg Beispiel 75 wurden durch präparative Trennung an chiraler Phase in die Enantiomere getrennt [Säule: Daicel Chiralpak AY-H, 5 µm, 250 x 20 mm, Eluent: 50% iso-Hexan, 50% Ethanol + 0.2% Diethylamin, Fluß: 15 ml/min; 40°C, Detektion: 220 nm].
Enantiomer A:
Ausbeute: 18 mg (99% ee)
Rt= 5.75 min [Daicel Chiralpak AY-H, 5µm, 250 x 4.6 mm; Eluent: 50% iso-Hexan, 50% Ethanol + 0.2% Diethylamin; Fluss 1.0 ml/min; 40°C; Detektion: 220 nm].

### Beispiel 82

### 8-[(2,6-Difluorbenzyl)oxy]-2-methyl-N-(2,2,6,6-tetramethylpiperidin-4-yl)imidazo[1,2-a]pyridin-3-carboxamid

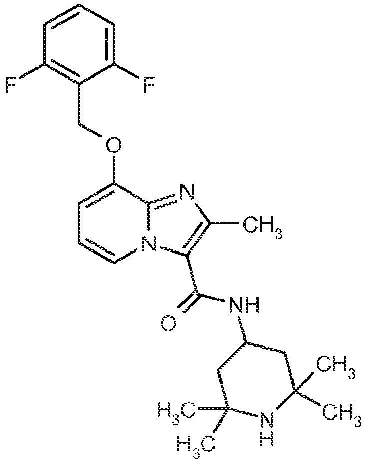

18.8 mg (0.12 mmol) 2,2,6,6-Tetramethylpiperidin-4-amin wurden vorgelegt und nacheinander mit 32 mg (0.10 mmol) 8-[(2,6-Difluorbenzyl)oxy]-2-methylimidazo[1,2-a]pyridin-3-carbonsäure in 0.4 ml DMF und 42 mg (0.13 mmol) (Benzotriazol-1-yloxy)bisdimethylaminomethyliumfluorborat (TBTU) in 0.2 ml DMF versetzt. Anschließend wurden 20 mg (0.20 mmol) 4-Methylmorpholin zugesetzt und die Reaktion wurde über Nacht bei RT gerührt. Dann wurde abfiltriert und das Filtrat per präparativer LC-MS (Methode 12) gereinigt. Die produkthaltigen Fraktionen wurden im Vakuum eingeengt. Es wurden 27 mg (59% d. Th.) Zielprodukt erhalten.
LC-MS (Methode 8): Rₜ = 0.69 min;
MS (ESIpos): m/z = 457 (M+H)⁺

In Analogie zu Beispiel 82 wurden die in Tabelle 8 aufgeführten Verbindungen hergestellt.

**Tabelle 8:**

| **Beispiel** | **IUPAC**-**Name / Struktur (Ausbeute)** | **Analytische Daten** |
|---|---|---|
| **83** | 8-[(2,6-Difluorbenzyl)oxy]-2-methyl-*N*-(1,2,2,6,6-pentamethylpiperidin-4-yl)imidazo[1,2-a]pyridin-3-carboxamid | LC-MS (Methode 8): Rₜ = 0.69 min |
| | | MS (ESpos): m/z = 471 (M+H)⁺ |
| | 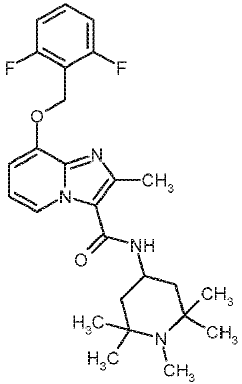 | |
| | (45% d. Th.) | |
| **84** | *rac*-8-[(2,6-Difluorbenzyl)oxy]-2-methyl-*N*-(1-methylpiperidin-3-yl)imidazo[1,2-a]pyridin-3-carboxamid | LC-MS (Methode 8): Rₜ = 0.64 min |
| | | MS (ESpos): m/z = 415 (M+H)⁺ |
| | 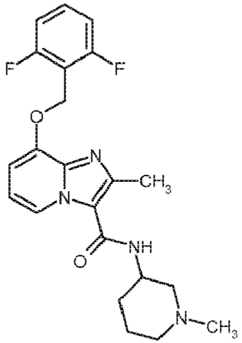 | |
| | (48% d. Th.) | |
| **85** | *rac-N*-(1-Benzylpyrrolidin-3-yl)-8-[(2,6-difluor-benzyl)-oxy]-2-methylimidazo[1,2-a]pyridin-3-carboxamid | LC-MS (Methode 8): Rₜ = 0.75 min |
| | | MS (ESpos): m/z = 477 (M+H)⁺ |
| | 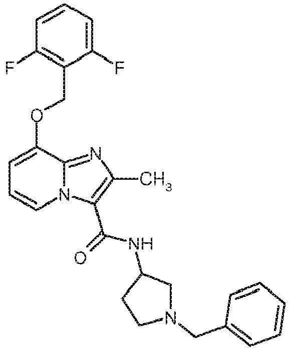 | |
| | (55% d. Th.) | |
| **86** | 8-[(2,6-Difluorbenzyl)oxy]-*N*-[2-(dimethyl-amino)ethyl]-2-methylimidazo[1,2,a]pyridin-3-carboxamid | LC-MS (Methode 8): Rₜ = 0.63 min |
| | | MS (ESpos): m/z = 389 (M+H)⁺ |
| | 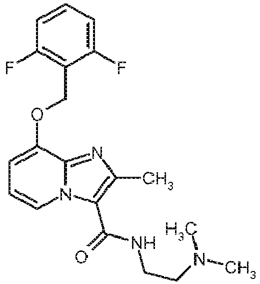 | |
| | (14% d. Th.) | |
| **87** | 8-[(2,6-Difluorbenzyl)oxy]-*N*-[3-(dimethyl-amino)propyl]-2-methylimidazo[1,2-a]pyridin-3-carboxamid | LC-MS (Methode 8): Rₜ = 0.63 min |
| | | MS (ESpos): m/z = 403 (M+H)⁺ |
| | 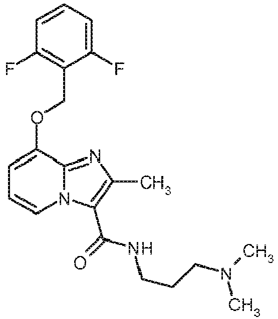 | |
| | (6% d. Th.) | |
| **88** | 8-[(2,6-Difluorbenzyl)oxy]-*N*-[2-(diisopropyl-amino)ethyl]-2-methylimidazo[1,2-a]pyridin-3-carboxamid | LC-MS (Methode 8): Rₜ = 0.75 min |
| | | MS (ESpos): m/z = 445 (M+H)⁺ |
| | 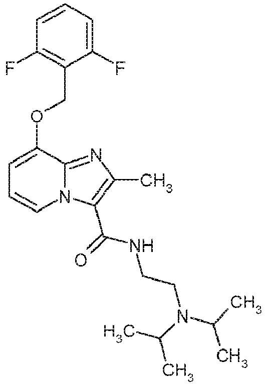 | |
| | (10% d. Th.) | |
| **89** | 8-[(2,6-Difluorbenzyl)oxy]-2-methyl-*N*-[2-(morpholin-4-yl)ethyl]imidazo[1,2-a]pyridin-3-carboxamid | LC-MS (Methode 8): Rₜ = 0.65 min |
| | | MS (ESpos): m/z = 431 (M+H)⁺ |
| | 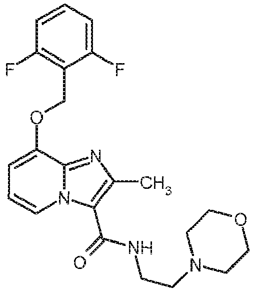 | |
| | (3% d. Th.) | |
| **90** | 8-[(2,6-Difluorbenzyl)oxy]-2-methyl-*N*-(9-methyl-9-azabicyclo[3.3.1]non-3-yl)imidazo[1,2-a]pyridin-3-carboxamid | LC-MS (Methode 8): Rₜ = 0.66 min |
| | | MS (ESpos): m/z = 455 (M+H)⁺ |
| | 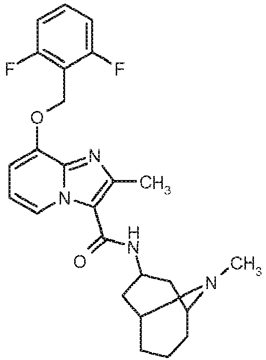 | |
| | (22% d. Th.) | |
| **91** | 8-[(2,6-Difluorbenzyl)oxy]-2-methyl-*N*-(8-methyl-8-azabicyclo[3.2.1]oct-3-yl)imidazo[1,2-a]pyridin-3-carboxamid | LC-MS (Methode 8): Rₜ = 0.65 min |
| | | MS (ESpos): m/z = 441 (M+H)⁺ |
| | 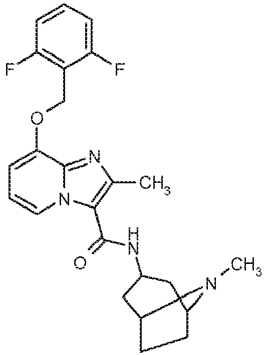 | |
| | (66% d. Th.) | |
| **92** | *N*-(1-Azabicyclo[2.2.2]oct-3-yl)-8-[(2,6-difluorbenzyl)oxy]-2-methylimidazo[1,2-a]pyridin-3-carboxamid | LC-MS (Methode 8): Rₜ = 0.65 min |
| | | MS (ESpos): m/z = 427 (M+H)⁺ |
| | 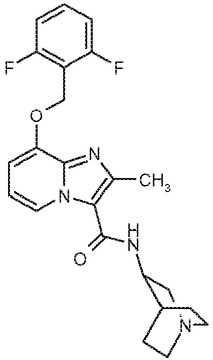 | |
| | (40% d. Th.) | |

### Beispiel 93

### ent-N-(1-Amino-3-isopropoxypropan-2-yl)-8-[(2,6-difluorbenzyl)oxy]-2-methylimidazo[1,2-a]pyridin-3-carboxamid (Enantiomer A)

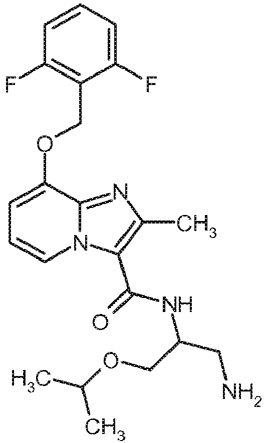

114 mg Beispiel 63 wurden in 2.5 ml Ethanol gelöst und an chiraler Phase in die Enantiomere getrennt [Säule: Daicel Chiralpak OD-H, 5 µm, 250 x 20 mm, Eluent: 50% iso-Hexan, 50% Ethanol, Fluß: 20 ml/min; 25°C, Detektion: 230 nm].
Ausbeute Enantiomer A: 44 mg (100% ee)
Enantiomer A: Rₜ = 4.33 min [Daicel Chiralpak OD-H, 5µm, 250 x 4.6 mm; Eluent: 50% iso-Hexan, 50% Ethanol; Fluss 1.0 ml/min; 40°C; Detektion: 220 nm].
¹H-NMR (400 MHz, DMSO-d₆): δ = 1.10 (d, 6 H), 1.68 (br. s, 2 H), 2.54 (s, 3 H; überlagert durch DMSO-Signal), 2.72 (d, 2 H), 3.49 (d, 2 H), 3.58 (q, 1 H), 3.99 (m, 1 H), 5.30 (s, 2 H), 6.91 (t, 1 H), 7.01 (d, 1 H), 7.23 (t, 2 H), 7.53 (br. s, 1 H), 7.58 (q, 1 H), 8.60 (d, 1 H).

### Beispiel 94

### 8-[(2,6-Difluorbenzyl)oxy]-2,6-dimethyl-N-{2-methyl-2-[(2,2,2-trifluorethyl)amino]propyl}imidazo[1,2-a]pyridin-3-carboxamid

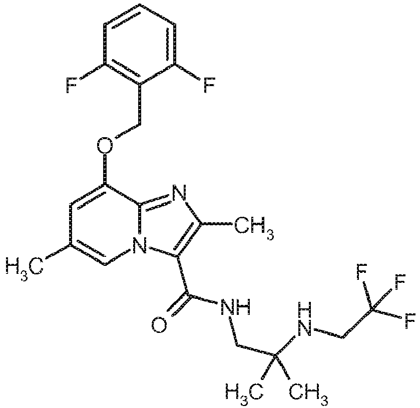

100 mg (0.25 mmol) *N*-2-Amino-2-methylpropyl)-8-[(2,6-difluorbenzyl)oxy]-2,6-dimethylimidazo[1,2,a]pyridin-3-carboxamid (Beispiel 74) wurden in 2 ml DMF vorgelegt, mit 0.041 ml (0.25 mmol) 2,2,2-Trifluorethyltrichlormethansulfonat, 69 mg (0.50 mmol) Kaliumcarbonat und 4.1 mg (0.025 mmol) Kaliumiodid versetzt und anschließend wurde das Gemisch über Nacht bei RT gerührt. Anschließend wurden mit 0.082 ml (0.50 mmol) 2,2,2-Trifluorethyltrichlormethansulfonat hinzugegeben und die Mischung wurde nochmals über Nacht bei RT gerührt. Es wurden erneut 0.123 ml (0.75 mmol) 2,2,2-Trifluorethyltrichlormethansulfonat hinzugegeben und die Mischung wurde nochmals über Nacht bei RT gerührt. Zur Aufarbeitung wurde 1 ml Wasser zugesetzt, über eine Extrelut-Kartusche filtriert, mit Dichlormethan eluiert und das Filtrat eingeengt. Das Rohprodukt wurde mittels mittels präparativer HPLC gereinigt (RP18 Säule, Laufmittel: Acetonitril/Wasser-Gradient unter Zusatz von 0.5% Ammoniumhydroxid). Die Produktfraktionen wurden vereint, eingedampft und im Hochvakuum getrocknet.
Ausbeute: 48 mg (38% d. Th.)
LC-MS (Methode 2): Rₜ = 0.96 min
MS (ESpos): m/z = 485.4 (M+H)⁺
¹H-NMR (400 MHz, DMSO-d₆): δ = 1.05 (s, 6H), 2.31 (s, 3H), 2.37 (t, 1H), 2.54 (s, 3H; verdeckt durch DMSO-Signal), 3.18-3.30 (m, 4H), 5.28 (s, 2H), 6.92 (s, 1H), 7.19-7.29 (m, 2H), 7.50 (t, 1H), 7.54-7.64 (m, 1H), 8.50 (s, 1H).

### Beispiel 95

### ent-N-(3-Amino-2-methylpropyl)-8-[(2,6-difluorbenzyl)oxy]-2-methylimidazo[1,2-a]pyridin-3-carboxamiddihydrochlorid (Enantiomer A)

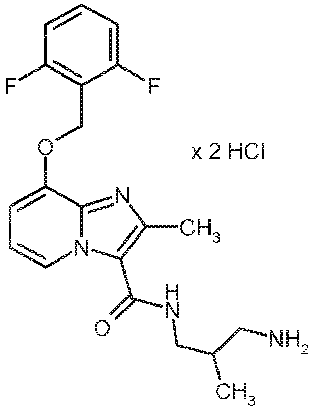

68 mg *ent*-tert.-Butyl-{3-[({8-[(2,6-difluorbenzyl)oxy]-2-methylimidazo[1,2-a]pyridin-3-yl}carbonyl)amino]-2-methylpropyl}carbamat (Beispiel 213A, 0.14 mmol, 1 Äquivalent) wurden in 2 ml 4 N Salzsäure in 1,4-Dioxan (1.4 mmol, 10 Äquivalente) gelöst und über Nacht bei RT gerührt. Dann wurde eingeengt und in Vakuum getrocknet. Es wurden 48 mg (75% d. Th.; Reinheit 100%) der Titelverbindung erhalten.
LC-MS (Methode 2): Rₜ = 0.56 min
MS (ESpos): m/z = 389.2 (M-2HCl+H)⁺
¹H-NMR (400 MHz, DMSO-d₆): δ = 0.99 (d, 3 H), 2.05 - 2.17 (m, 1 H), 2.62 (s, 3 H), 2.65 - 2.75 (m, 1 H), 2.83 - 2.93 (m, 1 H), 3.32 (t, 2 H), 5.43 (s, 2 H), 7.21 - 7.37 (m, 3 H), 7.47 (br. s, 1 H), 7.56 - 7.68 (m, 1 H), 7.98 (br. s, 3 H), 8.56 (br. s, 1 H), 8.68 (d, 1 H).

### Beispiel 96

### ent-N-(3-Amino-2-methylpropyl)-8-[(2,6-difluorbenzyl)oxy]-2-methylmidazo[1,2-a]pyridin-3-carboxamiddihydrochlorid (Enantiomer B)

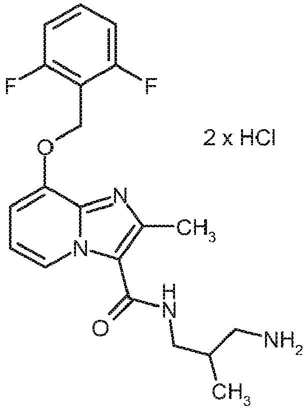

79 mg *ent*-tert.-Butyl-{3-[({8-[(2,6-difluorbenzyl)oxy]-2-methylimidazo[1,2-a]pyridin-3-yl)carbonyl)amino]-2-methylpropyl}carbamat (Beispiel 214A, 0.16 mmol, 1 Äquivalent) wurden in 2 ml 4 N Salzsäure in 1,4-Dioxan (1.6 mmol, 10 Äquivalente) gelöst und über Nacht bei RT gerührt. Dann wurde eingeengt und im Vakuum getrocknet. Es wurden 66 mg (88.5% d. Th.; Reinheit 100%) der Titelverbindung erhalten.
LC-MS (Methode 2): Rₜ = 0.55 min
MS (ESpos): m/z = 389.2 (M-2HCl+H)⁺
¹H-NMR (400 MHz, DMSO-d₆): δ = 0.99 (d, 3 H), 2.06 - 2.19 (m, 1 H), 2.63 (s, 3 H), 2.66 - 2.75 (m, 1 H), 2.83 - 2.94 (m, 1 H), 3.31 (t, 2 H), 5.43 (s, 2 H), 7.26 (t, 2 H), 7.36 (br. s, 1 H), 7.48 - 7.67 (m, 2 H), 8.03 (br. s, 2 H), 8.62 (br. s, 1 H), 8.71 (d, 1 H).

### Beispiel 97

### ent-N-[2-Amino-1-(3,4-difluorphenyl)ethyl]-8-{[(2,6-difluorphenyl)(²H₂)methyl]oxy}-2-methylimidazo[1,2-a]pyridin-3-carboxamid

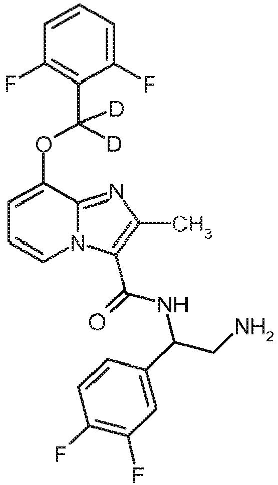

250 mg *ent*-tert.-Butyl-[2-(3,4-difluorphenyl)-2-{[(8-{[(2,6-difluorphenyl)(²H₂)methyl]oxy}-2-methylimidazo[1,2-a]pyridin-3-yl)carbonyl]amino}ethyl]carbamat (Beispiel 170A, 0.44 mmol, 1 Äquivalent) wurden bei RT in 4.4 ml 4 N Salzsäure in 1,4-Dioxan (17.4 mmol, 40 Äquivalente) gelöst und 3 h bei RT nachgerührt. Dann wurde eingeengt und der Rückstand mit Wasser und gesättigter, wässriger Ammoniak-Lösung versetzt und viermal mit Essigsäureethylester extrahiert. Die vereinigten organischen Phasen wurden mit gesättigter, wässriger Natriumchlorid-Lösung gewaschen, über Magnesiumsulfat getrocknet und eingeengt. Das so erhaltene Rohprodukt wurde auf Extrelut^{®} aufgezogen und über eine Kieselgelkartusche (Biotage SNAP Cartrige KP-Sil 10 g, Eluent: Dichlormethan/Methanol: 95:5 nach 0:100) gereinigt. Es wurden 198 mg (95% d. Th.; Reinheit 99%) der Titelverbindung erhalten.
LC-MS (Methode 7): Rₜ = 0.71 min
MS (ESpos): m/z = 475.1 (M+H)⁺
¹H-NMR (400 MHz, DMSO-d₆): δ = 1.70 (br. s, 2 H), 2.59 (s, 3 H), 2.86 - 2.94 (m, 2 H), 4.92 - 5.01 (m, 1 H), 6.91 (t, 1 H), 7.01 (d, 1 H), 7.20 - 7.28 (m, 3 H), 7.36 - 7.50 (m, 2 H), 7.54 - 7.63 (m, 1 H), 8.18 (br. s, 1 H), 8.53 (d, 1 H).

### Beispiel 98

### ent-N-[2-Amino-1-(3,4-difluorphenyl)ethyl]-8-[(2-fluorbenzyl)oxy]-2-methylimidazo[1,2-a]pyridin-3-carboxamid

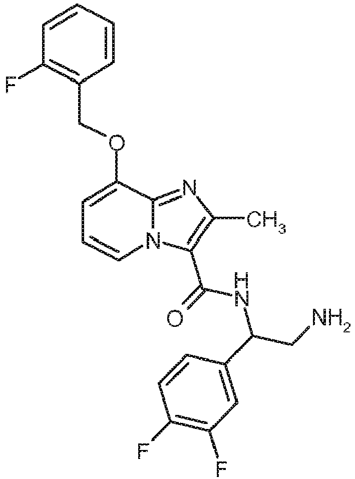

191 mg *ent*-tert.-Butyl-{2-(3,4-difluorphenyl)-2-[({8-[(2-fluorbenzyl)oxy]-2-methylimidazo[1,2-a]pyridin-3-yl}carbonyl)amino]ethyl}carbamat (Beispiel 197A, 0.3 mmol, 1 Äquivalent) wurden bei RT mit 0.8 ml 4 N Salzsäure in 1,4-Dioxan (3.1 mmol, 10 Äquivalente) versetzt. Es wurde über Nacht bei RT gerührt und dann eingeengt. Der Rückstand wurde in Essigsäureethylester aufgenommen und mit gesättigter, wässriger Natriumhydrogencarbonat-Lösung gewaschen. Die organische Phase wurde über Magnesiumsulfat getrocknet und eingeengt. Es wurden 101 mg (69% d. Th.; Reinheit 97%) der Titelverbindung erhalten.
LC-MS (Methode 7): Rₜ = 0.69 min
MS (ESpos): m/z = 455.1 (M+H)⁺
¹H-NMR (400 MHz, DMSO-d₆): δ = 1.70 (br. s, 2 H); 2.61 (s, 3 H), 2.83 - 2.93 (m, 2 H), 4.96 (t, 1 H), 5.33 (s, 2 H), 6.92 (t, 1 H), 6.99 (d, 1 H), 7.19 - 7.34 (m, 3 H), 7.35 - 7.53 (m, 3 H), 7.62 (t, 1 H), 8.20 (br. s, 1 H), 8.51 (d, 1 H).

In Analogie zu Beispiel 98 wurden die in Tabelle 9 aufgeführten Verbindungen aus den entprechenden Boc-geschützten Aminen hergestellt.

**Tabelle 9:**

| **Beispiel** | **IUPAC-Name / Struktur (Ausbeute)** | **Analytische Daten** |
|---|---|---|
| **99** | *ent*-*N*-[2-Amino-1-(3,4-difluorphenyl)ethyl]-8-[(2,3-difluorbenzyl)oxy]-2-methylimidazo[1,2-a]pyridin-3-carboxamid | LC-MS (Methode 7): Rₜ = 0.72 min |
| | | MS (ESpos): m/z = 473.1 (M+H)⁺ |
| | 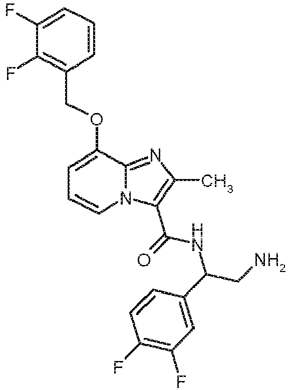 | ¹H-NMR (400 MHz, DMSO-d₆): δ = 1.70 (br. s, 2 H), 2.62 (s, 3 H), 2.85 - 2.93 (m, 2 H), 4.99 (t, 1 H), 5.39 (s, 2 H), 6.89 (t, 1 H), 6.97 (d, 1 H), 7.21 - 7.34 (m, 2 H), 7.36 - 7.56 (m, 4 H), 8.20 (br. s, 1 H), 8.52 (d, 1 H). |
| | (66% d. Th.; Reinheit 100%) | |
| **100** | *ent*-*N*-[2-Amino-1-(3,4-difluorphenyl)ethyl]-2-methyl-8-[(2,3,6-trifluorbenzyl)oxy]imidazo[1,2-a]pyridin-3-carboxamid | LC-MS (Methode 7): Rₜ = 0.72 min |
| | | MS (ESpos): m/z = 491.1 (M+H)⁺ |
| | 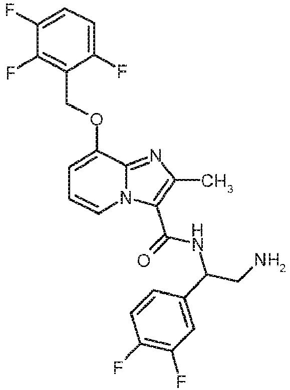 | ¹H-NMR (400 MHz, DMSO-d₆): δ = 1.70 (br. s, 2 H), 2.60 (s, 3 H), 2.85 - 2.92 (m, 2 H), 4.95 (t, 1 H), 5.36 (s, 2 H), 6.91 (t, 1 H), 7.01 (d, 1 H), 7.19 - 7.33 (m, 2 H), 7.35 - 7.51 (m, 2 H), 7.60 - 7.72 (m, 1 H), 8.20 (br. s, 1 H), 8.54 (d, 1 H). |
| | (33% d. Th.; Reinheit 96%) | |
| **101** | *ent*-*N*-[2-Amino-1-(3,4-difluorphenyl)ethyl]-2-methyl-8-[(2,4,6-trifluorbenzyl)oxy]imidazo[1,2-a]pyridin-3-carboxamid | LC-MS (Methode 7): Rₜ = 0.74 min |
| | | MS (ESpos): m/z = 491.1 (M+H)⁺ |
| | | ¹H-NMR (400 MHz. DMSO-d₆): |
| | 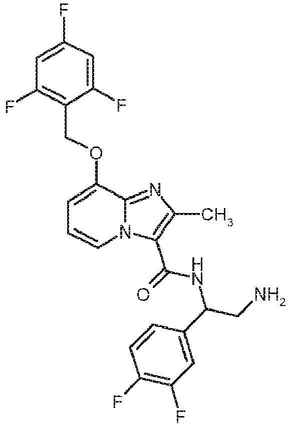 | δ = 1.70 (br. s, 2 H), 2.60 (s, 3 H), 2.88 (d, 2 H), 4.95 (t, 1 H), 5.28 (s, 2 H), 6.91 (t, 1 H), 7.00 (d, 1 H), 7.19 - 7.28 (m, 1 H), 7.30 - 7.50 (m, 4 H), 8.19 (br. s, 1 H), 8.54 (d, 1 H). |
| | (58% d. Th.; Reinheit 96%) | |
| **102** | *ent-N-*[2-Amino-1-(3,4-difluorphenyl)ethyl]-8-[(2-chlor-6-fluorbenzyl)oxy]-2-methylimidazo[1,2-a]pyridin-3-carboxamid | LC-MS (Methode 7): Rₜ = 0.76 min |
| | | MS (ESpos): m/z = 489.1 (M+H)⁺ |
| | 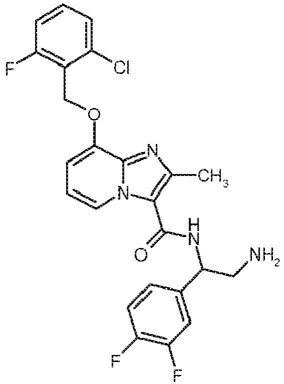 | ¹H-NMR (400 MHz, DMSO-d₆): δ = 1.70 (br. s, 2 H), 2.59 (s, 3 H), 2.84 - 2.93 (m, 2 H), 4.96 (t, 1 H), 5.34 (s, 2 H), 6.93 (t, 1 H), 7.03 (d, 1 H), 7.21-7.27(m, 1 H),7.33-7.51 (m, 4 H), 7.53 - 7.62 (m, 1 H), 8.18 (br. s, 1 H), 8.54 (d, 1 H). |
| | (61% d. Th.; Reinheit 95%) | |
| **103** | *N*-(3-Amino-2,2-dimethylpropyl)-8-[(2,6-difluorbenzyl)oxy]-2-methylimidazo[1,2-a]pyridin-3-carboaamid¹⁾ | LC-MS (Methode 7): Rₜ = 0.60 min MS (ESpos): m/z = 403.3 (M+H)^{+ 1}H-NMR (400 MHz, DMSO-d₆): δ = 0.87 (s, 6 H), 1.08 (br. s, 2 H), 2.41 (s, 2H), 3.21 (d, 2 H), 5.30 (s, 2 H), 6.93 (t, 1 H), 7.01 (d, 1 H), 7.23 (t, 2 H), 7.53 - 7.65 (m, 1 H), 8.21 (t, 1 H), 8.62 (d, 1 H). |
| | | |
| | | |
| | 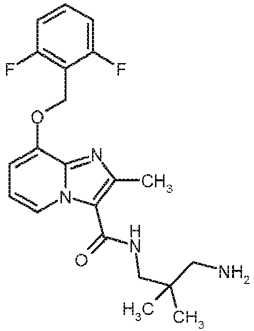 | |
| | (39% d. Th.; Reinheit 95%) | |

| | | |
|---|---|---|
| ¹⁾ Umsetzung ohne Zusatz von Lösungmittel, mit 20 Äquivalenten 4 M Salzsäure in 1,4-Dioxan. | | |

### Beispiel 104

### N-(3-Amino-3-methylbutyl)-6-chlor-8-[(2,6-difluorbenzyl)oxy]-2-methylimidazo[1,2-a]pyridin-3-carboxamid

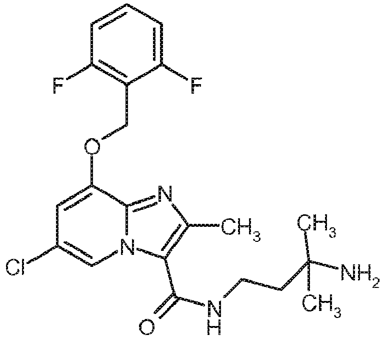

118 mg tert.-Butyl-{4-[({6-chlor-8-[(2,6-difluorbenzyl)oxy]-2-methylimidazo[1,2-a]pyridin-3-yl)carbonyl)amino]-2-methylbutan-2-yl}carbamat Trifluoracetat (Beispiel 179A, 0.22 mmol, 1 Äquivalent) wurden in 4 ml 2 N Salzsäure in Diethylether (8 mmol, 36 Äquivalente) vorgelegt und über Nacht bei RT gerührt. Der entstandene Niederschlag wurde abfiltiert, gut mit Diethylether gewaschen und dann in Essigsäureethylester gelöst. Die organische Phase wurde zweimal mit gesättigter, wässriger Natriumhydrogencarbonat-Lösung und mit Wasser gewaschen. Die wässrige Phase wurde zweimal mit Dichlorethan extrahiert und die vereinten organischen Phasen wurden über Natriumsulfat getrocknet, filtriert und eingeengt. Es wurden 65.5 mg (68% d. Th., Reinheit 99%) der Zielverbindung erhalten.
LC-MS (Methode 2): Rₜ = 0.75 min
MS (ESpos): m/z = 437.3 (M+H)⁺
¹H-NMR (400 MHz, DMSO-d₆): δ = 1.09 (s, 6 H), 1.59 (t, 2 H), 2.10 (br. s, 2 H), 3.40 (t, 2 H), 5.33 (s, 2 H), 7.20 (d, 1 H), 7.26 (t, 2 H), 7.55 - 7.65 (m, 1 H), 8.42 (br. s, 1 H), 8.84 (d, 1 H), [weiteres Signal unter den Lösungsmittel-Peaks verborgen].

In Analogie zu Beispiel 104 wurden die in Tabelle 10 aufgeführten Verbindungen aus den entprechenden Boc-geschützten Aminen hergestellt. Es wurden 10 - 40 Äquivalente 2 N Salzsäure verwendet. Zur Aufarbeitung der Reaktion wurde ein entstandener Feststoff in Essigsäureethylester oder Dichlormethan/Methanol gelöst und analog weiter bearbeitet.

**Tabelle 10:**

| **Beispiel** | **IUPAC-Name / Struktur (Ausbeute)** | **Analytische Daten** |
|---|---|---|
| **105** | *ent*-8-[(2,6-Difluorbenryl)oxy]-*N*-(6-fluor-1,2,3,4-tetrahydrochinolin-4-yl)-2-methylimidazo[1,2-a]pyridin-3-carboxamid (Enantiomer A) ¹⁾ | LC-MS (Methode 2): Rₜ = 0.86 min |
| | | MS (ESpos): m/z = 467.2 (M+H)⁺ |
| | | ¹H-NMR (400 MHz, DMSO-d₆): |
| | 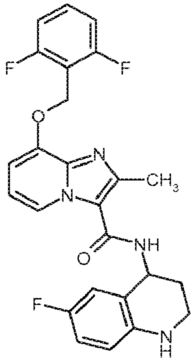 | δ = 1.94 - 2.03 (m, 2 H), 3.20 - 3.27 (m, 2 H), 5.14 - 5.23 (m, 1 H), 5.31 (s, 2 H), 5.82 (s, 1 H), 6.48 - 6.54 (m, 1 H), 6.81 (dt, 1 H), 6.88 - 6.94 (m, 1 H), 6.96 (d, 1 H), 7.02 (d, 1 H), 7.23 (t, 2 H), 7.54 - 7.64 (m, 1 H), 8.31 (d, 1 H), 8.58 (d, 1 H), [weiteres Signal unter DMSO-Peak verborgen]. |
| | (98% d. Th.; Reinheit 100%) | |
| **106** | *ent*-8-[(2,6-Difluorbenzyl)oxy]-*N*-(6-fluor-1,2,3,4-tetrahydrochinolin-4-yl)-2-methylimidazo[1,2-a]pyridin-3-carboxamid (Enantiomer B) ¹⁾ | LC-MS (Methode 2): Rₜ = 0.86 min |
| | | MS (ESpos): m/z = 467.2 (M+H)⁺ |
| | | ¹H-NMR (400 MHz, DMSO-d₆): |
| | 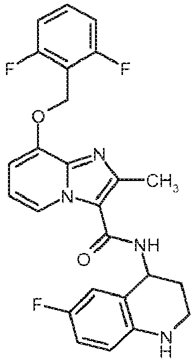 | δ = 1.93 - 2.05 (m, 2 H), 3.19 - 3.27 (m, 2 H), 5.14 - 5.23 (m, 1 H), 5.31 (s, 2 H), 5.83 (s, 1 H), 6.48 - 6.54 (m, 1 H), 6.81 (dt, 1 H), 6.88 - 6.94 (m, 1 H), 6.96 (d, 1 H), 7.02 (d, 1 H), 7.24 (t, 2 H), 7.54 - 7.65 (m, 1 H), 8.30 (d, 1 H), 8.58 (d, 1 H), [weiteres Signal unter DMSO-Peak verborgen]. |
| | (99% d. Th.; Reinheit 100%) | |
| **107** | *ent*-6-Chlor-8-[(2,6-difluorbenzyl)oxy]-2-methyl-*N*-(1,2,3,4-tetrahydroisochinolin-4-yl)imidazo[1,2-a]pyridin-3-carboxamid (Enantiomer A) ¹⁾ | LC-MS (Methode 2): Rₜ = 0.81 min |
| | | MS (ESpos): m/z = 483.2 (M+H)⁺ |
| | | ¹H-NMR (400 MHz, DMSO-d₆): |
| | 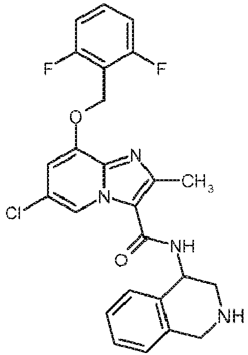 | δ = 2.46 (s, 3 H), 2.97 (dd, 1 H), 3.18 (dd, 1 H), 3.80 - 3.96 (m, 2 H), 5.13 - 5.21 (m, 1 H), 5.35 (s, 2 H), 7.05 - 7.11 (m, 1 H), 7.17 - 7.29 (m, 5 H), 7.36 - 7.41 (m, 1 H), 7.56 - 7.65 (m, 1 H), 8.26 (d, 1 H), 8.74 (d, 1 H). |
| | (92% d. Th.; Reinheit 100%) | |
| **108** | *ent*-6-Chlor-8-[(2,6-difluorbenzyl)oxy]-2-methyl-*N*-(1,2,3,4-tetrahydroisochinolin-4-yl)imidazo[1,2-a]pyridin-3-carboxamid (Enantiomer B) ¹⁾ | LC-MS (Methode 2): Rₜ = 0.81 min |
| | | MS (ESpos): m/z = 483.2 (M+H)⁺ |
| | | ¹H-NMR (400 MHz, DMSO-d₆): |
| | 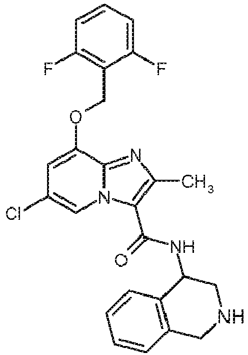 | δ = 2.46 (s, 3 H), 2.97 (dd, 1 H), 3.18 (dd, 1 H), 3.80 - 3.96 (m, 2 H), 5.13 - 5.22 (m, 1 H), 5.35 (s, 2 H), 7.05 - 7.11 (m, 1 H), 7.16 - 7.29 (m, 5 H), 7.36 - 7.41 (m, 1 H), 7.56 - 7.65 (m, 1 H), 8.26 (d, 1 H), 8.74 (d, 1 H). |
| | (95% d. Th.; Reinheit 100%) | |
| **109** | *ent*-8-[(2,6-Difluorbenzyl)oxy]-2-methyl-N-(1,2,3,4-tetrahydroisochinolin-4-yl)imidazo[1,2-a]pyridin-3-carboxamid (Enantiomer A) ²⁾ | LC-MS (Methode 7): Rₜ = 0.65 min |
| | | MS (ESpos): m/z = 449.2 (M+H)⁺ |
| | | ¹H-NMR (400 MHz, DMSO-d₆): |
| | 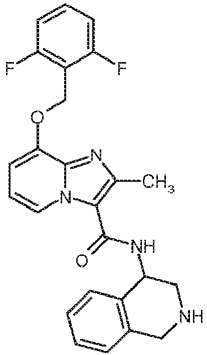 | δ = 2.47 (s, 3 H), 2.97 (dd, 1 H), 3.18 (dd, 1 H), 3.81 - 3.96 (m, 2 H), 5.14 - 5.22 (m, 1 H), 5.30 (s, 2 H), 6.96 (t, 1 H), 7.02 (d, 1 H), 7.06 - 7.11 (m, 1 H), 7.16 - 7.28 (m, 4 H), 7.35 - 7.41 (m, 1 H), 7.54 - 7.64 (m, 1 H), 8.17 (d, 1 H), 8.64 (d, 1 H), [weiteres Signal unter Lösungsmittel-Peaks verborgen]. |
| | (49% d. Th.; Reinheit 99%) | |
| **110** | *ent*-8-[(2,6-Difluorbenzyl)oxy]-2-methyl-N-(1,2,3,4-tetrahydroisochinolin-4-yl)imidazo[1,2-a]pyridin-3-carboxamid (Enantiomer B) ²⁾ | LC-MS (Methode 7): Rₜ = 0.69 min |
| | | MS (ESpos): m/z = 449.2 (M+H)⁺ |
| | | ¹H-NMR (400 MHz, DMSO-d₆): |
| | 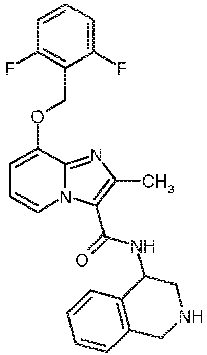 | δ = 2.47 (s, 3 H), 2.97 (dd, 1 H), 3.18 (dd, 1 H), 3.81 - 3.96 (m, 2 H), 5.15 - 5.22 (m, 1 H), 5.30 (s, 2 H), 6.96 (t, 1 H), 7.02 (d, 1 H), 7.06 - 7.11 (m, 1 H), 7.16 - 7.29 (m, 4 H), 7.35 - 7.40 (m, 1 H), 7.54 - 7.64 (m, 1 H), 8.17 (d, 1 H), 8.64 (d, 1 H), [weiteres Signal unter Lösungsmittel-Peaks verborgen]. |
| | (53% d. Th.; Reinheit 99%) | |
| **111** | *ent*-8-[(2,6-Difluorbenzyl)oxy]-2-methyl-*N*-[6-(trifluormethyl)-1,2,3,4-tetrahydrochinolin-4-yl]imidazo[1,2-a]pyridin-3-carboxamid (Enantiomer A) ¹⁾ | LC-MS (Methode 7): Rₜ =1.02 min |
| | | MS (ESpos): m/z = 517.1 (M+H)⁺ |
| | | ¹H-NMR (400 MHz, DMSO-d₆): |
| | 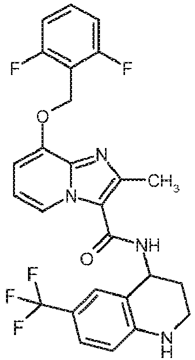 | δ = 1.91 - 2.09 (m, 2 H), 5.18 - 5.26 (m, 1 H), 5.32 (s, 2 H), 6.62 (d, 1 H), 6.70 (s, 1 H), 6.96 (t, 1 H), 7.02 (d, 1 H), 7.20 - 7.29 (m, 3 H), 7.37 (s, 1 H), 7.53 - 7.65 (m, 1 H), 8.35 (d, 1 H), 8.56 (d, 1 H), [weitere Signale unter Lösungsmittel-Peaks verborgen]. |
| | (64% d. Th.; Reinheit 97%) | |
| **112** | *ent*-8-[(2,6-Difluorbenzyl)oxy]-2-methyl-*N*-[6-(trifluormethyl)-1,2,3,4-tehahydrochinolin-4-yl]imidazo[1,2,a]pyridin-3-carboxamid (Enantiomer B) ¹⁾ | LC-MS (Methode 7): Rₜ = 1.02 min |
| | | MS (ESpos): m/z = 517.1 (M+H)⁺ |
| | | ¹H-NMR (400 MHz, DMSO-d₆): |
| | 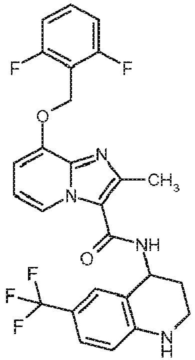 | δ = 1.91 - 2.08 (m, 2 H), 5.18 - 5.27 (m, 1 H), 5.32 (s, 2 H), 6.62 (d, 1 H), 6.70 (s, 1 H), 6.96 (t, 1 H), 7.02 (d, 1 H), 7.20 - 7.29 (m, 3 H), 7.37 (s, 1 H), 7.53 - 7.65 (m, 1 H), 8.35 (d, 1 H), 8.56 (d, 1 H), [weitere Signale unter Lösungsmittel-Peaks verborgen]. |
| | (78% d. Th.; Reinheit 98%) | |
| **113** | *N*-(2-Aminoethyl)-8-[(2,6-difluorbenzyl)oxy]-2,6-dimethylimidazo[1,2-a]pyridin-3-carboxamid ¹⁾ | LC-MS (Methode 7): Rₜ = 0.56 min |
| | | MS (ESpos): m/z = 375.2 (M+H)⁺ |
| | 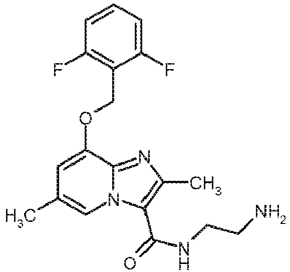 | ¹H-NMR (400 MHz, DMSO-d₆): |
| | | δ = 1.62 (br. s, 2 H), 2.31 (s, 3 H), 2.71 (t, 2 H), 5.27 (s, 2 H), 6.91 (s, 1 H), 7.24 (t, 2 H), 7.54 - 7.65 (m, 1 H), 7.72 - 7.81 (m, 1 H), 8.46 (s, 1 H), [weitere Signale unter Lösungsmittel-Peaks verborgen]. |
| | (74% d. Th.; Reinheit 98%) | |
| **114** | *N*-(3-Amino-3-methylbutyl)-8-[(2,6-difluorbenzyl)oxy]-2,6-dimethylimidazo[1,2-a]pyridin-3-carboxamid ¹⁾ | LC-MS (Methode 2): Rₜ = 0.62 min |
| | | MS (ESpos): m/z = 417.4 (M+H)⁺ |
| | | ¹H-NMR (400 MHz, DMSO-d₆): |
| | 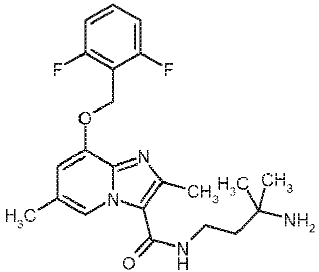 | δ = 1.08 (s, 6 H), 1.58 (t, 2 H), 1.78 (br. s, 2 H), 2.31 (s, 3 H), 3.36 - 3.43 (m, 2 H), 5.28 (s, 2 H), 6.91 (s, 1 H), 7.24 (t, 2 H), 7.54 - 7.64 (m, 1 H), 8.25 - 8.31 (m, 1 H), 8.53 (s, 1 H), [weiters Signal unter Lösungsmittel-Peak verborgen]. |
| | (85% d. Th.; Reinheit 95%) | |
| **115** | 8-[(2,6-Difluorbenzyl)oxy]-2,6-dimethyl-*N*-[(2*S*)-pyrrolidm-2-ylmethyl]imidazo[1,2-a]pyridin-3-carboxamid ¹⁾ | LC-MS (Methode 2): Rₜ = 0.63 min |
| | | MS (ESpos): m/z = 415.3 (M+H)⁺ |
| | | ¹H-NMR (400 MHz, DMSO-d₆): |
| | 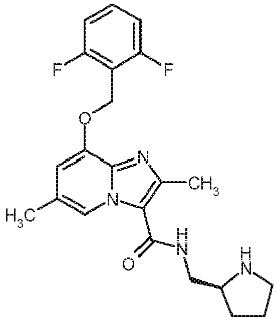 | δ = 1.34 - 1.45 (m, 1 H), 1.55 - 1.71 (m, 2 H), 1.72 -1.84 (m, 1 H), 2.31 (s, 3 H), 2.70 - 2.87 (m, 2 H), 3.17 - 3.28 (m, 3 H), 5.28 (s, 2 H), 6.92 (s, 1 H), 7.23 (t, 2 H), 7.54 - 7.64 (m, 1 H), 7.71 - 7.78 (m, 1 H), 8.46 (s, 1 H), [weiteres Signal unter Lösungsmittel-Peak verborgen]. |
| | (79% d. Th.; Reinheit 94%) | |
| **116** | 8-[(2,6-Difluorbenzyl)oxy]-2,6-dimethyl-*N*-[(2*R*)-pyirrolidin-2-ylmethyl]imidazo[1,2-a]pyridin-3-carboxamid ¹⁾ | LC-MS (Methode 2): Rₜ = 0.64 min |
| | | MS (ESpos): m/z = 415.3 (M+H)⁺ |
| | | ¹H-NMR (400 MHz, DMSO-d₆): |
| | 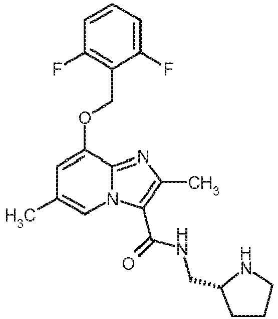 | δ = 1.34 - 1.45 (m, 1 H), 1.55 - 1.71 (m, 2 H), 1.72 -1.84 (m, 1 H), 2.31 (s, 3 H), 2.71 - 2.87 (m, 2 H), 3.17 - 3.28 (m, 3 H), 5.28 (s, 2 H), 6.92 (s, 1 H), 7.23 (t, 2 H), 7.54 - 7.64 (m, 1 H), 7.71 - 7.79 (m, 1 H), 8.46 (s, 1 H), [weiteres Signal unter Lösungsmittel-Peak verborgen]. |
| | (80% d. Th., Reinheit 98%) | |
| **117** | *N*-(3-Amino-3-methylbutyl)-8-[(2,6-difluorbenzyl)oxy]-2-methylimidazo[1,2-a]pyridin-3-carboxamid ³⁾ | LC-MS (Methode 2): Rₜ = 0.59 min |
| | | MS (ESpos): m/z = 403.3 (M+H)⁺ |
| | | ¹H-NMR (400 MHz, DMSO-d₆): |
| | 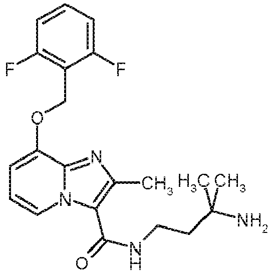 | δ = 1.08 (s, 6 H), 1.58 (t, 2 H), 1.79 (br. s, 2 H), 3.37 - 3.45 (m, 2 H), 5.30 (s, 2 H), 6.93 (t, 1 H), 7.01 (d, 1 H), 7.23 (t, 2 H), 7.54 - 7.64 (m, 1 H), 8.28 - 8.36 (m, 1 H), 8.70 (d, 1 H), [weiteres Signal unter Lösungsmittel-Peak verborgen]. |
| | (58% d. Th., Reinheit 99%) | |
| **118** | *rac*-6-Chlor-8-[(2,6-difluorbenzyl)oxy]-2-methyl--*N*-(piperidin-2-ylmethyl)imidazo[1,2-a]pyridin-3-carboxamid | LC-MS (Methode 7): Rₜ = 0.73 min |
| | | MS (ESpos): m/z = 449.1 (M+H)⁺ |
| | 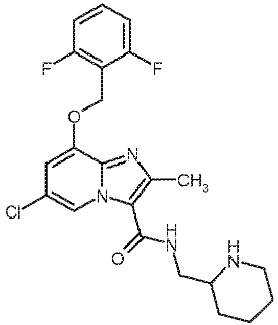 | ¹H-NMR (400 MHz, DMSO-d₆): |
| | | δ = 0.99 -1.13 (m, 1 H), 1.21 -1.37 (m, 2 H), 1.45 -1.53 (m, 1 H), 1.62 (d, 1 H), 1.74 (br. s., 1 H), 2.16 (br. s, 1 H), 2.59 - 2.70 (m, 1 H), 2.94 (d, 1 H), 3.17 - 3.27 (m, 2 H), 5.35 (s, 2 H), 7.18 (s, 1 H), 7.24 (t, 2 H), 7.54 - 7.65 (m, 1 H), 7.83 (t, 1 H), 8.76 (s, 1 H), [weitere Signale unter Lösungmittel-Peaks verborgen]. |
| | (92% d. Th.; Reinheit 98%) | |
| **119** | *N*-[(1-Aminocyclopentyl)methyl]-8-[(2,6-difluorbenzyl)oxy]-2,6-dimethylimidazo[1,2-a]pyridin-3-carboxamid | LC-MS (Methode 2): Rₜ = 0.58 min |
| | | MS (ESpos): m/z = 429.3 (M+H)⁺ |
| | | ¹H-NMR (400 MHz, DMSO-d₆): |
| | 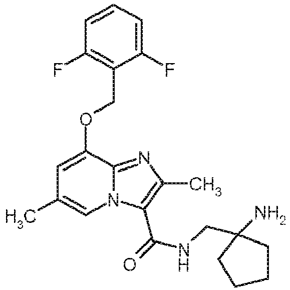 | δ = 1.31 - 1.42 (m, 2 H), 1.49 - 1.62 (m, 6 H), 1.66 -1.79 (m, 2 H), 2.31 (s, 3 H), 5.28 (s, 2 H), 6.91 (s, 1 H), 7.24 (t, 2 H), 7.53 - 7.64 (m, 1 H), 7.66 - 7.74 (m, 1 H), 8.46 (s, 1 H), [weitere Signale unter Lösungsmittel-Peaks verborgen]. |
| | (63% d. Th.; Reinheit 100%) | |

| | | |
|---|---|---|
| ¹⁾ Die Umsetzung erfolgte mit 10 Äquivalenten 2 M Salzsäure in Diethylether. ²⁾ Die Umsetzung erfolgte mit 56 Äquivalenten 2 M Salzsäure in Diethylether. ³⁾ Die Umsetzung erfolgte mit 31 Äquivalenten 2 M Salzsäure in Diethylether. | | |

### Beispiel 120

### N-[(3S)-3-Aminobutan-2-yl]-8-[(2,6-difluorbenzyl)oxy]-2-methylimidazo[1,2-a]pyridin-3-carboxamid

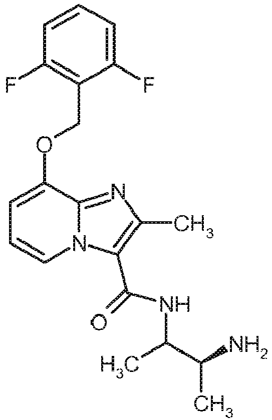

155 mg Benzyl-{(2*S*)-3-[({6-chlor-8-[(2,6-difluorbenzyl)oxy]-2-methylimidazo[1,2-a]pyridin-3-yl}carbonyl)amino]butan-2-yl}carbamat (Beispiel 191A, 0.28 mmol, 1 Äquivalent) wurden in 50 ml Ethanol gelöst und mit 40 mg Palladium auf Aktivkohle (5%ig) versetzt. Es wurde 3 h bei RT und Normaldruck hydriert. Nach beendeter Reaktionszeit wurde über Kieselgel filtriert, der Rückstand mit Ethanol gewaschen und die Mutterlauge im Vakuum eingeengt. Das erhaltene Rohprodukt wurde über präparative HPLC (Methode 9) gereinigt.
LC-MS (Methode 2): Rₜ = 0.55 min
MS (ESpos): m/z = 389.2 (M+H)⁺
¹H-NMR (400 MHz, DMSO-d₆): δ = 1.01 (d, 3 H), 1.15 (d, 3 H), 1.80 (br. s, 2 H), 2.50 (s, 3 H; überlagert durch DMSO-Signal), 2.82 - 2.96 (m, 1 H), 3.81 - 3.95 (m, 1 H), 5.30 (s, 2 H), 6.92 (t, 1 H), 7.01 (d, 1 H), 7.23 (t, 2 H), 7.47 - 7.54 (m, 1 H), 7.55 - 7.63 (m, 1 H), 8.64 (d, 1 H).

### Beispiel 121

### N-[(3R)-3-Aminobutan-2-yl]-8-[(2,6-difluorbenzyl)oxy]-2-methylimidazo[1,2-a]pyridin-3-carboxamid

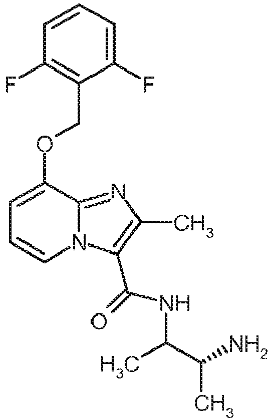

235 mg Benzyl-{(2*R*)-3-[({8-[(2,6-difluorbenzyl)oxy]-2-methylimidazo[1,2-a]pyridin-3-yl}carbonyl)amino]butan-2-yl}carbamat (Beispiel 184A, 0.45 mmol, 1 Äquivalent) wurden in 260 ml Ethanol gelöst und mit 63 mg Palladium auf Aktivkohle (10% ig, 0.45 mmol, 1 Äquivalent) versetzt. Es wurde 1.5 h bei RT unter Normaldruck hydriert und anschließend über Kieselgel filtriert und eingeengt. Den Rückstand löste man in Methanol und reinigte über präparative HPLC (Methode 9). Es wurden 96 mg (55% d. Th.) der Titelverbindung erhalten.
Diastereomerenverhältnis 3:1
Hauptdiastereomer:
LC-MS (Methode 2): Rₜ = 0.55 min
MS (ESpos): m/z = 389.2 (M+H)⁺
¹H-NMR (400 MHz, DMSO-d₆): δ = 1.01 (d, 3 H), 1.15 (d, 3 H), 1.65 (br. s, 2 H), 2,51 (s, 3 H; überlagert durch DMSO-Signal), 2.82 - 2.96 (m, 1 H), 3.81 - 3.95 (m, 1 H), 5.30 (s, 2 H), 6.92 (t, 1 H), 7.01 (d, 1 H), 7.23 (t, 2 H), 7.47 - 7.54 (m, 1 H), 7.55 - 7.63 (m, 1 H), 8.64 (d, 1 H).

### Beispiel 122

### ent-N-(1-Amino-3-methoxypropan-2-yl)-8-[(2,6-difluorbenzyl)oxy]-2-methylimidazo[1,2-a]pyridin-3-carboxamid Ditrifluoracetat (Enantiomer A)

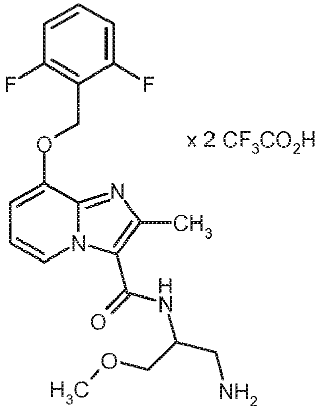

58 mg *ent*-Benzyl-{2-[({8-[(2,6difluorbenzyl)oxy]-2-methylimidazo[1,2-a]pyridin-3-yl}carbonyl)amino]-3-methoxypropyl}carbamat (Beispiel 210A, Enantiomer A, 0.1 mmol, 1 Äquivalent) wurden in 10 ml Ethanol gelöst und mit 10 mg Palladiumhydroxid auf Aktivkohle (10% ig) versetzt. Es wurde 3 h bei RT unter Normaldruck hydriert und anschließend über Kieselgel filtiert. Der Rückstand wurde mit Ethanol, Essigsäureethylester und Dichlormethan gewaschen und die organische Phase wurde eingeengt. Den Rückstand reinigte man über die präparativer HPLC (Säule: Sunfire C18, 5 µm, 250 x 20 mm, Fluß: 25 ml, Wellenlänge: 210 nm, Temperatur 40°C, Eluent: Acetonitril:Wasser + 1% TFA = 20:80). Es wurden 27 mg (43% d. Th., Reinheit 100%) der Titelverbindung erhalten.
LC-MS (Methode 2): Rₜ = 0.58 min
MS (ESpos): m/z = 405.3 (M-2TFA+H)⁺
¹H-NMR (400 MHz, DMSO-d₆): δ = 2.91 - 3.03 (m, 1 H), 3.05 - 3.16 (m, 1 H), 3.32 (s, 3 H), 3.45 - 3.54 (m, 2 H), 4.36 - 4.51 (m, 1 H), 5.33 (s, 2 H), 7.05 - 7.12 (m, 1 H), 7.15 - 7.20 (m, 1 H), 7.24 (t, 2 H), 7.53 - 7.65 (m, 1 H), 7.85 (br. s, 4 H), 8.62 (d, 1 H), [weitere Signale unter Lösungsmittel-Peaks verborgen].

### Beispiel 123

### ent-N-(1-Amino-3-methoxypropan-2-yl)-8-[(2,6-difluorbenzyl)oxy]-2-methylimidazo[1,2-a]pyridin-3-carboxamid Ditrifluoracetat (Enantiomer B)

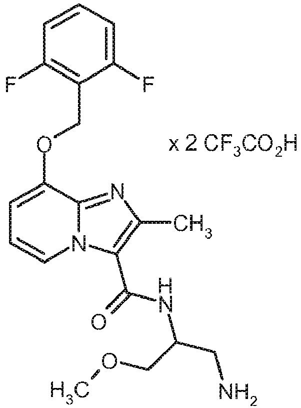

53 mg *ent*-Benzyl-{2-[({8-[(2,6-difluorbenzyl)oxy]-2-methylimidazo[1,2-a]pyridin-3-yl}carbonyl)amino]-3-methoxypropyl}carbamat (Beispiel 211A, Enantiomer B, 0.1 mmol, 1 Äquivalent) wurden in Analogie zu Beispiel 122 hydriert und aufgearbeitet. Es wurden 22 mg (36% d. Th.; Reinheit 100%) der Titelverbindung erhalten.
LC-MS (Methode 2): Rₜ = 0.58 min
MS (ESpos): m/z = 405.3 (M-2TFA+H)⁺
¹H-NMR (400 MHz, DMSO-d₆): δ = 2.91 - 3.03 (m, 1 H), 3.05 - 3.16 (m, 1 H), 3.32 (s, 3 H), 3.45 - 3.54 (m, 2 H), 4.36 - 4.51 (m, 1 H), 5.33 (s, 2 H), 7.05 - 7.12 (m, 1 H), 7.15 - 7.20 (m, 1 H), 7.24 (t, 2 H), 7.53 - 7.65 (m, 1 H), 7.85 (m, 4 H), 8.62 (d, 1 H), [weitere Signale unter Lösungsmittel-Peaks verborgen].

### Beispiel 124

### ent-N-[1-Amino-3-(3,4-difluorphenoxy)propan-2-yl]-8-[(2,6-difluorbenzyl)oxy]-2-methylimidazo[1,2,a]pyridin-3-carboxamid Ditrifluoracetat

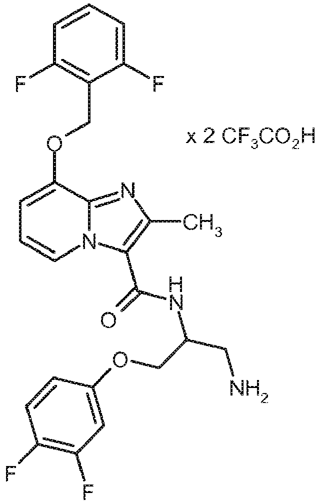

110 mg *ent*-Benzyl-{2-[({8-[(2,6-difluorbenzyl)oxy]-2-methylimidazo[1,2-a]pyridin-3-yl}carbonyl)amino]-3-{3,4-difluorphenoxy)propyl}carbamat (Beispiel 212A, Enantiomer B) wurden in Analogie zu Beispiel 122 hydriert und aufgearbeitet. Es wurden 69 mg (55% d. Th.; Reinheit 100%) der Titelverbindung erhalten.
LC-MS (Methode 2): Rₜ = 0.87 min
MS (ESpos): m/z = 503.2 (M-2TFA+H)⁺
¹H-NMR (400 MHz, DMSO-d₆): δ = 3.03 - 3.15 (m, 1 H), 3.16 - 3.27 (m, 1 H), 4.12 - 4.22 (m, 2 H), 4.58 - 4.70 (m, 1 H), 5.33 (s, 2 H), 6.80 - 6.87 (m, 1 H), 7.01 - 7.09 (m, 1 H), 7.10 - 7.19 (m, 2 H), 7.24 (t, 2 H), 7.34 - 7.44 (m, 1 H), 7.54 - 7.64 (m, 1 H), 7.88 - 8.05 (m, 4 H), 8.62 (d, 1 H), [weiteres Signal unter Lösungsmittel-Peaks verborgen].

### Beispiel 125

### rac-N-{3-Azabicyclo[4.1.0]hept-1-yl)-8-[(2,6-difluorbenzyl)oxy]-2-methylimidazo[1,2-a]pyridin-3-carboxamid

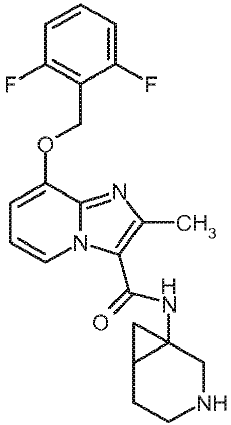

216 mg *rac*-Benzyl-1-[({8-[(2,6-difluoibenzyl)oxy]-2-methylimidazo[1,2-a]pyridin-3-yl}carbonyl)amino]-3-azabicyclo[4.1.0]heptan-3-carboxylat (Beispiel 176A, 0.4 mmol, 1 Äquivalent) wurden in Analogie zu Beispiel 121 umgesetzt [0.2 Äquivalente Palladium auf Aktivkohle (10% ig), Reaktionszeit: 16 h] und aufgearbeitet [Nach der Trennung per HPLC wurde der Rückstand in Essigsäureethylester aufgenommen und zweimal mit gesättigter, wässriger Natriumhydrogencarbonatlösung gewaschen. Die wässrige Phase wurde mit Essigsäureethylester extrahiert. Die vereinigten organischen Phasen wurden über Natriumsulfat getrocknet, filtriert und eingeengt]. Es wurden 123 mg (74% d. Th.; 98%) der Titelverbindung erhalten.
LC-MS (Methode 7): Rₜ = 0.56 min
MS (ESpos): m/z = 413.1 (M+H)⁺
¹H-NMR (400 MHz, DMSO-d₆): δ = 0.92 (t, 1 H), 0.96 -1.02 (m, 1 H), 1.22 -1.31 (m, 1 H), 1.57 - 1.68 (m, 1 H), 2.01 - 2.11 (m, 1 H), 2.45 (s, 3 H), 2.59 - 2.76 (m, 2 H), 3.19 - 3.29 (m, 2 H), 5.30 (s, 2 H), 5.79 (br. s, 1 H),6.94 (t, 1 H), 7.02 (d, 1 H), 7.23 (t, 2 H), 7.53 - 7.63 (m, 1 H), 8.24 (s, 1 H), 8.60 (d, 1 H).

### Beispiel 126

### ent-N-(2-Amino-4,4,4-trifluorbutyl)-8-[(2,6-difluorbenzyl)oxy]-2-methylimidazo[1,2-a]pyridin-3-carboxamid (Enantiomer A)

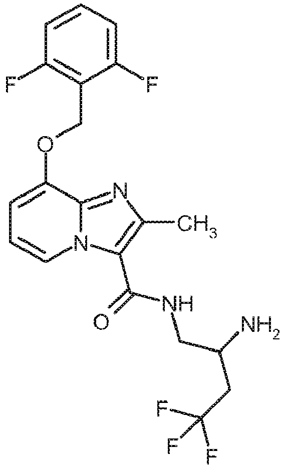

170 mg *ent*-Benzyl-{1-[({8-[(2,6-difluorbenzyl)oxy]-2-methylimidazo[1,2-a]pyridin-3-yl)carbonyl)amino]-4,4,4-trifluorbutan-2-yl)carbamat (Beispiel 215A, 0.30 mmol, 1 Äquivalent) wurden unter Argon in 3 ml Ethanol/Dichlorethan (2/1) vorgelegt, mit 31 mg Palladium auf Aktivkohle (10%ig) und 0.60 ml (5.90 mmol, 20 Äquivalente) Cyclohexen versetzt und über Nacht unter Rückfluss gerührt. Die Reaktionslösung wurde über einen Millipore^{®}-Filter filtriert, gut mit Ethanol/Dichlorethan (2/1) gewaschen, eingeengt und im Hochvakuum getrocknet. Es wurde mittels Kieselgel-Chromatographie gereinigt (Laufmittel: Dichlormethan/Methanol: 40/1 isokratisch). Es wurden 92 mg (67% d. Th.; 95%) der Titelverbindung erhalten.
LC-MS (Methode 7): Rₜ = 0.62 min
MS (ESpos): m/z = 443.2 (M+H)⁺
¹H-NMR (400 MHz, DMSO-d₆): δ = 1.85 (br. s, 2 H), 2.15 - 2.38 (m, 1 H), 2.40 - 2.47 (m, 1 H), 3.15 - 3.28 (m, 2 H), 5.30 (s, 2 H), 6.94 (t, 1 H), 7.01 (d, 1 H), 7.24 (t, 2 H), 7.54 - 7.64 (m, 1 H), 7.89 (t, 1 H), 8.64 (d, 1 H), [weitere Signale unter Lösungmittel-Peaks verborgen].

### Beispiel 127

### ent-N-(2-Amino-4,4,4-trifluorbutyl)-8-[(2,6-difluorbenzyl)oxy]-2-methylimidazo[1,2-a]pyridin-3-carboxamid (Enantiomer B)

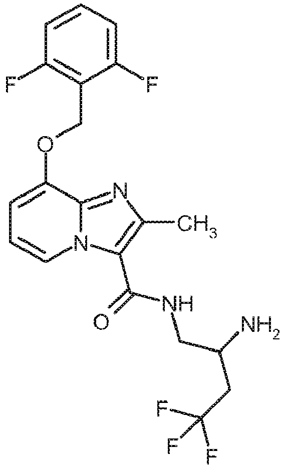

172 mg *ent*-Benzyl-{1-[({8-[(2,6-difluorbenzyl)oxy]-2-methylimidazo[1,2-a]pyridin-3-yl}carbonyl)amino]-4,4,4-trifluorbutan-2-yl}carbamat Trifluoracetat (Beispiel 216A, 0.30 mmol, 1 Äquivalent) wurden in Analogie zu Beispiel 126 umgesetzt und aufgearbeitet. Es wurden 66 mg (50% d. Th.; 95%) der Titelverbindung erhalten.
LC-MS (Methode 2): Rₜ = 0.60 min
MS (ESpos): m/z = 443.3 (M+H)⁺
¹H-NMR (400 MHz, DMSO-d₆): δ = 1.80 (br. s, 2 H), 2.15 - 2.37 (m, 1 H), 2.40 - 2.47 (m, 1 H), 3.15 - 3.28 (m, 2 H), 5.30 (s, 2 H), 6.94 (t, 1 H), 7.01 (d, 1 H), 7.24 (t, 2 H), 7.55 - 7.64 (m, 1 H), 7.89 (t, 1 H), 8.64 (d, 1 H), [weitere Signale unter Lösungmittel-Peaks verborgen].

### Beispiel 128

### ent-N-(2-Amino-4,4,4-trifluorbutyl)-8-[(2,6-difluorbenzyl)oxy]-2,6-dimethylimidazo[1,2-a]pyridin-3-carboxamid (Enantiomer A)

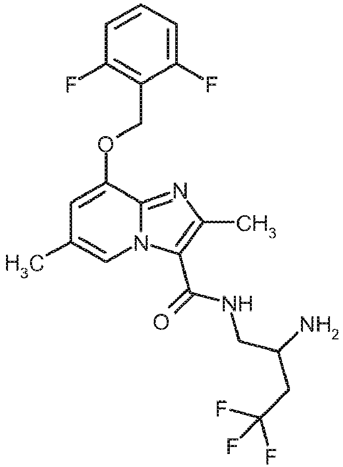

85 mg *ent-*Benzyl-{1-[({8-[(2,6difluorbenzyl)oxy]-2,6-dimethylimidazo[1,2-a]pyridin-3-yl}carbonyl)amino]-4,4,4-trifluorbutan-2-yl}carbamat (Beispiel 217A, 0.14 mmol, 1 Äquivalent) wurden in 2.1 ml eines 1:1:0.1-Gemisches aus Ethanol/Dichlormethan/Methanol vorgelegt und unter Zusatz von 0.3 ml Cyclohexen (2.88 mmol, 20 Äquivalente) und 15 mg 10%igem Palladium auf Aktivkohle (0.014 mmol, 0.1 Äquivalent) 3 h bei Rückfluss gerührt. Dann wurden 0.15 ml Cyclohexen (1.44 mmol, 10 Äquivalente) und 15 mg 10%iges Palladium auf Aktivkohle (0.014 mmol, 0.1 Äquivalent) nachgegeben und weitere 6 h bei Rückfluss gerührt. Die Reaktionslösung wurde über einen Millipore^{®}-Filter filtriert, gut mit Methanol gewaschen und eingeengt. Der Rückstand wurde über präparative HPLC (RP18 Säule, Laufmittel: Acetonitril/Wasser-Gradient unter Zusatz von 0.1% TFA) gereinigt. Das Rohprodukt wurde in Dichlormethan aufgenommen und zweimal mit gesättigter, wässriger Natriumhydrogencarbonat-Lösung gewaschen. Die wässrige Phase wurde zweimal mit Dichlormethan extrahiert und die vereinigten, organischen Phasen wurde über Natriumsulfat getrocknet, filtriert und eingeengt. Es wurden 52 mg (75% d. Th., Reinheit 95%) der Titelverbindung erhalten.
LC-MS (Methode 2): Rₜ = 0.69 min
MS (ESpos): m/z = 457.3 (M+H)⁺
¹H-NMR (400 MHz, DMSO-d₆): δ = 1.69 (br. s, 2 H), 2.16 - 2.30 (m, 1 H), 2.31 (s, 3 H), 2.36 - 2.46 (m, 1 H), 3.13 - 3.27 (m, 2 H), 5.27 (s, 2 H), 6.92 (s, 1 H), 7.23 (t, 2 H), 7.54 - 7.65 (m, 1 H), 7.81 - 7.88 (m, 1 H), 8.47 (s, 1 H), [weitere Signale unter Lösungmittel-Peaks verborgen].
Spezifischer Drehwert [a] (365 nm, 19.6°C) = +42.5° (c = 0.00445 g/ml, Acetonitril)

### Beispiel 129

### ent-N-(2-Amino-4,4,4-trifluorbutyl)-8-[(2,6-difluorbenzyl)oxy]-2,6-dimethylimidazo[1,2-a]pyridin-3-carboxamid (Enantiomer B)

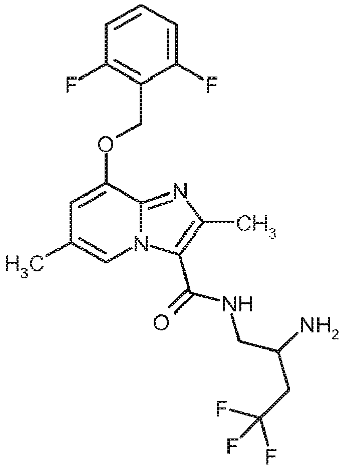

92 mg *ent*-Benzyl-{1-[({8-[(2,6difluorbenzyl)oxy]-2,6-dimethylimidazo[1,2-a]pyridin-3-yl}carbonyl)amino]-4,4,4-trifluorbutan-2-yl}carbamat (Beispiel 218A, 0.16 mmol, 1 Äquivalent) wurden in 2.1 ml eines 1:1:0.1 -Gemisches aus Ethanol/Dichlormethan/Methanol vorgelegt und unter Zusatz von 0.47 ml Cyclohexen (4.67 mmol, 30 Äquivalente) und 33 mg 10%igem Palladium auf Aktivkohle (0.03 mmol, 0.1 Äquivalent) 6 h bei Rückfluss gerührt. Die Reaktionslösung wurde über einen Millipore^{®}-Filter filtriert, gut mit Methanol gewaschen und eingeengt. Der Rückstand wurde über präparative HPLC (RP18 Säule, Laufmittel: Acetonitril/Wasser-Gradient unter Zusatz von 0.1% TFA) gereinigt. Das Rohprodukt wurde in Dichlormethan aufgenommen und zweimal mit gesättigter, wässriger Natriumhydrogencarbonat-Lösung gewaschen. Die wässrige Phase wurde zweimal mit Dichlormethan extrahiert. Die vereinigten organischen Phasen wurden über Natriumsulfat getrocknet, filtriert und eingeengt. Es wurden 46 mg (64% d. Th.) der Titelverbindung erhalten.
LC-MS (Methode 2): Rₜ = 0.70 min
MS (ESpos): m/z = 457.3 (M+H)⁺
¹H-NMR (400 MHz, DMSO-d₆): δ = 1.71 (br. s, 2 H), 2.16 - 2.30 (m, 1 H), 2.31 (s, 3 H), 2.36 - 2.46 (m, 1 H), 3.13 - 3.27 (m, 2 H), 5.28 (s, 2 H), 6.92 (s, 1 H), 7.23 (t, 2 H), 7.54 - 7.65 (m, 1 H), 7.81 - 7.88 (m, 1 H), 8.47 (s, 1 H), [weitere Signale unter Lösungmittel-Peaks verborgen].
Spezifischer Drehwert [a] (365 nm, 19.5°C) = -39.0° (c = 0.005 g/ml, Acetonitril)

In Analogie zu Beispiel 75 wurden die in Tabelle 11 gezeigten Beispiele hergestellt, indem die entsprechende Carbonsäure mit den entsprechenden, kommerziell erhältlichen Aminen unter den in der repräsentativen Arbeitsvorschrift 2 beschriebenen Reaktionsbedingungen umgesetzt wurde.

**Tabelle 11:**

| **Beispiel** | **IUPAC-Name / Struktur (Ausbeute)** | **Analytische Daten** |
|---|---|---|
| **130** | 8-[(2,6-Difluorbenryl)oxy]-N-[2-(isopropylamino)ethyl]-2-methylimidazo[1,2-a]pyridin-3-carboxamid | LC-MS (Methode 7): Rₜ = 0.53 min |
| | | MS (ESpos): m/z = 403.2 (M+H)⁺ |
| | | ¹H-NMR (400 MHz, DMSO-d₆): |
| | 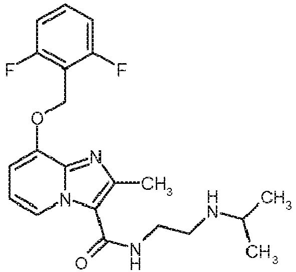 | δ = 0.98 (d, 6 H), 2.66 - 2.80 (m, 3 H), 5.30 (s, 2 H), 6.92 (t, 1 H), 7.02 (d, 1 H), 7.23 (t, 2 H), 7.54 - 7.64 (m, 1 H), 7.79 (t, 1 H), 8.64 (d, 1 H), [weitere Signale unter Lösungsmittel-Peaks verborgen]. |
| | (56% d. Th.; Reinheit 96%) | |
| **131** | 8-[(2,6-Difluorbenzyl)oxy]-*N*-[2-(1,1-dioxidothiomorpholin-4-yl)ethyl]-2-methylimidazo[1,2-a]pyridin-3-carboxamid | LC-MS (Methode 7): Rₜ = 0.65 min |
| | | MS (ESpos): m/z = 479.1 (M+H)⁺ |
| | | ¹H-NMR (400 MHz, DMSO-d₆): |
| | 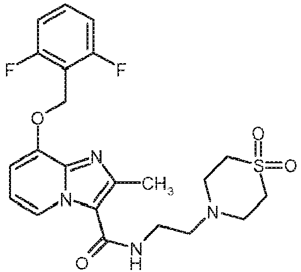 | δ = 2.53 (s, 3 H), 2.69 (t, 2 H), 2.94 - 3.00 (m, 4 H), 3.06 - 3.12 (m, 4 H), 3.39 - 3.46 (m, 2 H), 5.30 (s, 2 H), 6.93 (t, 1 H), 7.02 (d, 1 H), 7.23 (d, 2 H), 7.54 - 7.64 (m, 1 H), 7.74 (t, 1 H), 8.64 (d, 1 H). |
| | (84% d. Th.; Reinheit 99%) | |
| **132** | *rac*-8-[(2,6-Difluorbenzyl)oxy]-2-methyl-*N*-[2-(morpholin-4-yl)propyl]imidazo[1,2-a]pyridin-3-carboxamid | LC-MS (Methode 7): Rₜ = 0.53 min |
| | | MS (ESpos): m/z = 445.2 (M+H)⁺ |
| | | ¹H-NMR (400 MHz, DMSO-d₆): |
| | 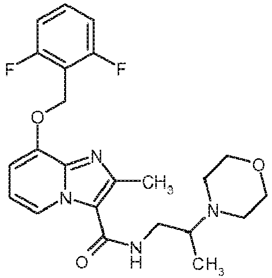 | δ = 0.96 (d, 2 H), 2.37 - 2.45 (m, 2 H), 2.58 (m, 5 H, verdeckt durch DMSO-Signal), 2.74 - 2.84 (m, 1 H), 3.52 - 3.63 (m, 4 H), 5.30 (s, 2 H), 6.94 (t, 1 H), 7.02 (d, 1 H), 7.23 (t, 2 H), 7.54 - 7.65 (m, 2 H), 8.71 (d, 1 H), [weitere Signale unter Lösungsmittel-Peaks verborgen]. |
| | (85% d. Th.; Reinheit 100%) | |
| **133** | 8-[(2,6-Difluoibenzyl)oxy]-2-methyl-*N*-[2-(4-methylpiperazin-1-yl)ethyl]imidazo[1,2-a]pyridin-3-carboxamid | LC-MS (Methode 2): Rₜ = 0.54 min |
| | | MS (ESpos): m/z = 444.2 (M+H)⁺ |
| | | ¹H-NMR (400 MHz, DMSO-d₆): |
| | 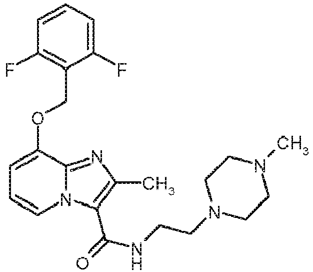 | δ = 2.15 (s, 3 H), 2.24 - 2.52 (m, 13 H; überlagert durch DMSO-Signal), 3.37 - 3.46 (m, 2 H), 5.29 (s, 2 H), 6.92 (t, 1 H), 7.02 (d, 1 H), 7.24 (t, 2 H), 7.53 - 7.64 (m, 1 H), 7.69 (t, 1 H), 8.66 (d, 1 H). |
| | (59% d. Th.; Reinheit 98%) | |
| **134** | *rac*-8-[(2,6-Difluorbenzyl)oxy]-2-methyl-*N*-[2-(morpholin-4-yl)-1-phenylethyl]imidazo[1,2-a]pyridin-3-carboxamid | LC-MS (Methode 7): Rₜ = 0.72 min |
| | | MS (ESpos): m/z = 507.2 (M+H)⁺ |
| | | ¹H-NMR (400 MHz, DMSO-d₆): |
| | 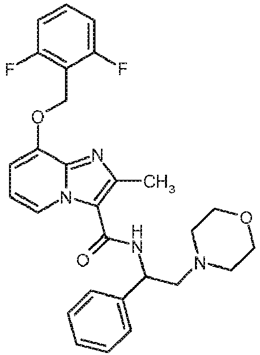 | δ = 2.34 - 2.44 (m, 2 H), 2.61 (s, 3 H), 2.75 - 2.84 (m, 1 H), 3.51 - 3.63 (m, 4 H), 5.13 - 5.24 (m, 1 H), 5.31 (s, 2 H), 6.91 (t, 1 H), 7.00 (d, 1 H), 7.19 - 7.29 (m, 3 H), 7.35 (t, 2 H), 7.44 (d, 2 H), 7.53 - 7.66 (m, 2 H), 8.27 (d, 1 H), 8.52 (d, 1 H), [weitere Signale unter Lösungsmittel-Peaks verborgen]. |
| | (71% d. Th.; Reinheit 95%) | |
| **135** | *rac*-8-[(2,6-Difluorbenzyl)oxy]-*N*-[2-(isopropylamino)ethyl]-2,6-dimethylimidazo[1,2-a]pyridin-3-carboxamid | LC-MS (Methode 7): Rₜ = 0.59 min |
| | | MS (ESpos): m/z = 417.2 (M+H)⁺ |
| | | ¹H-NMR (400 MHz, DMSO-d₆): |
| | 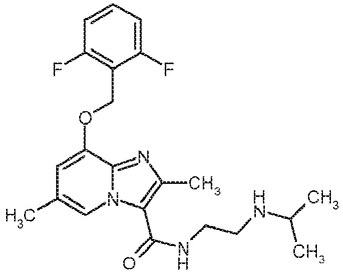 | δ = 0.98 (d, 6 H),1.68 (br. s, 1 H), 2.30 (s, 3 H), 2.66 - 2.80 (m, 3 H), 5.30 (s, 2 H), 6.91 (s, 1 H), 7.23 (t, 2 H), 7.54 - 7.64 (m, 1 H), 7.78 (t, 1 H), 8.48 (s, 1 H), [weitere Signale unter Lösungsmittel-Peaks verborgen]. |
| | (74% d. Th.; Reinheit: 100%) | |
| **136** | 8-[(2,6-Difluorbenzyl)oxy]-2,6-dimethyl-*N*-[2-(morpholin-4-yl)ethyl]imidazo[1,2-a]pyridin-3-carboxamid | LC-MS (Methode 7): Rₜ = 0.63 min |
| | | MS (ESpos): m/z = 445.2 (M+H)⁺ |
| | | ¹H-NMR (400 MHz, DMSO-d₆): |
| | 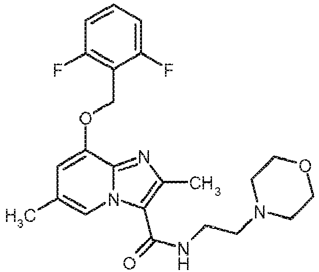 | δ = 2.30 (s, 3 H), 2.39 - 2.45 (m, 4 H), 3.39 - 3.46 (m, 2 H), 3.57 (br. s, 4 H), 5.28 (s, 2 H), 6.93 (s, 1 H), 7.22 (t, 2 H), 7.52 - 7.64 (m, 1 H), 7.70 (br. s, 1 H), 8.48 (br. s, 1 H), [weitere Signale unter Lösungsmittel-Peaks verborgen]. |
| | (59% d. Th.; Reinheit 97%) | |
| **137** | 8-[(2,6-Difluorbenzyl)oxy]-2,6-dimethyl-*N-*[2-(4-methylpiperazin-1-yl)ethyl]imidazo[1,2-a]pyridin-3-carboxamid | LC-MS (Methode 2): Rₜ = 0.57 min |
| | | MS (ESpos): m/z = 458.3 (M+H)⁺ |
| | | ¹H-NMR (400 MHz, DMSO-d₆): |
| | 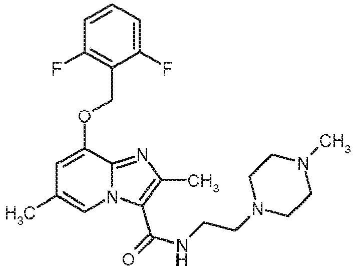 | δ = 2.31 (s, 3 H), 3.38 - 3.47 (m, 2 H), 5.28 (s, 2 H), 6.92 (s, 1 H), 7.25 (t, 3 H), 7.53 - 7.64 (m, 1 H), 7.68 (br. s, 1 H), 8.48 (s, 1 H), [weitere Signale unter Lösungsmittel-Peaks verborgen]. |
| | (71% d. Th.; Reinheit 97%) | |
| **138** | *rac*-8-[(2,6-Difluorbenzyl)oxy]-2,6-imethyl-*N-*[2-(morpholin-4-yl)propyl]imidazo[1,2-a]pyridin-3-carboxamid | LC-MS (Methode 7): Rₜ = 0.59 min |
| | | MS (ESpos): m/z = 459.2 (M+H)⁺ |
| | | ¹H-NMR (400 MHz, DMSO-d₆): |
| | 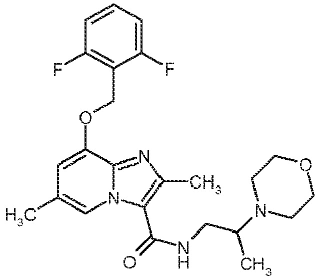 | δ = 0.97 (d, 3 H), 2.31 (s, 3 H), 2.38 - 2.46 (m, 2 H), 2.73 - 2.83 (m, 1 H), 3.50 - 3.64 (m, 4 H), 5.29 (s, 2 H), 6.93 (s, 1 H), 7.23 (t, 2 H), 7.54 - 7.66 (m, 2 H), 8.52 (s, 1 H), [weiteres Signal unter Lösungsmittel-Peak verborgen]. |
| | (59% d. Th.; Reinheit 98%) | |
| **139** | 8-[(2,6-Difluorbenzyl)oxy]-*N*-[2-(4-isopropylpiperazin-1-yl)ethyl]-2,6-dimethylimidazo[1,2-a]pyridin-3-carboxamid | LC-MS (Methode 2): Rₜ = 0.63 min |
| | | MS (ESpos): m/z = 486.3 (M+H)⁺ |
| | | ¹H-NMR (400 MHz, DMSO-d₆): |
| | 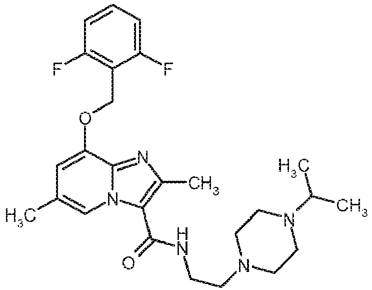 | δ = 0.95 (d, 6 H), 2.31 (s, 3 H), 2.38 - 2.45 (m, 6 H), 3.36 - 3.45 (m, 2 H), 5.28 (s, 2 H), 6.91 (s, 1 H), 7.24 (t, 2 H), 7.52 - 7.64 (m, 1 H), 7.67 (t, 1 H), 8.48 (s, 1 H), [weitere Signale unter Lösungsmittel-Peak verborgen]. |
| | (72% d. Th.; Reinheit 98%) | |

### Beispiel 140

### N-(2-Amino-2-methylpropyl)-8-[(2,6-difluorbenzyl)oxy]-6-fluor-2-methylimidazo[1,2,a]pyridin-3-carboxamid

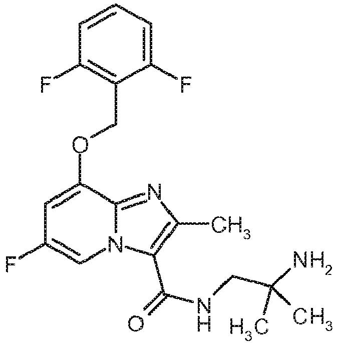

45 mg 8-[(2,6-Difluorbenzyl)oxy]-6-fluor-2-methylimidazo[1,2-a]pyridin-3-carbonsäure Beispiel 11A, 0.13 mmol, 1 Äquivalent), 65 mg (Benzotriazol-1-yloxy)bisdimethylamino-methyliumfluorborat (TBTU, 0.2 mmol, 1.5 Äquivalente) und 54 mg 4-Methylmorpholin (0.54 mmol, 4 Äquivalente) wurden in 0.9 ml DMF vorgelegt. Nach 10 min bei RT wurden 24 mg 1,2-Diamino-2-methylpropan (0.27 mmol, 2 Äquivalente) zugesetzt und es wurde über Nacht bei RT gerührt. Anschließend wurden nochmals 22 mg Äquivalente TBTU (0.07 mmol, 0.5 Äquivalente) und 12 mg 1,2-Diamino-2-methylpropan (0.13 mmol, 1 Äquivalent) hinzugegeben und die Reaktion wurde 2 h bei RT gerührt. Die Reaktionslösung wurde mit Wasser versetzt und über präparative HPLC (RP18 Säule, Laufmittel: Acetonitril/Wasser-Gradient unter Zusatz von 0.1% TFA) gereinigt. Der Rückstand wurde in Dichlormethan aufgenommen und zweimal mit gesättigter, wässriger Natriumhydrogencarbonat-Lösung gewaschen. Die wässrige Phase wurde zweimal mit Dichlormethan extrahiert und die vereinigten organischen Phasen wurde über Natriumsulfat getrocknet. Es wurde filtriert, eingeengt und das erhaltene Rohprodukt wurde über Dickschichtchromatographie gereinigt (Laufmittel: Dichlormethan/2 N Ammoniak in Methanol: 10:0.5). Es wurden 31 mg der Titelverbindung (55% d. Th.; Reinheit 98%) erhalten.
LC-MS (Methode 2): Rₜ = 0.64 min
MS (ESpos): m/z = 407.2 (M+H)⁺
¹H-NMR (400 MHz, DMSO-d₆): δ = 1.05 (s, 6 H), 1.52 (br. s, 2 H), 3.15 - 3.23 (m, 2 H), 5.32 (s, 2 H), 7.19 - 7.32 (m, 3 H), 7.55 - 7.66 (m, 1 H), 7.75 (br. s, 1 H), 8.69 - 8.76 (m, 1 H), [weiteres Signal unter Lösungsmittel-Peaks verborgen].

### Beispiel 141:

### N-(3-Amino-2,3-dimethylbutan-2-yl)-8-[(2,6-difluorbenzyl)oxy]-2-methylimidazo[1,2-a]pyridin-3-carboxamid

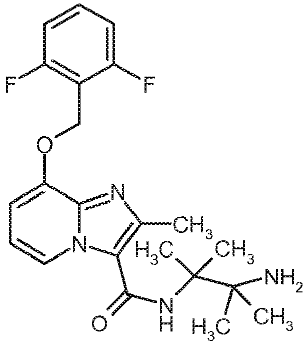

75 mg 8-[(2,6-Difluorbenzyl)oxy]-2-methylimidazo[1,2-a]pyridin-3-carbonsäure (Beispiel 3A, 0.24 mmol, 1 Äquivalent), 116 mg *O*-(7-Arabenzotriazol-1-yl)-*N,N,N'N*'-tetramethyluronium-hexafluorphosphat (HATU, 0.31 mmol, 1.3 Äquivalente) und 152 mg *N,N*-Diisopropylethylamin (0.21 ml, 1.18 mmol, 5 Äquivalente) wurden in 1.6 ml DMF gelöst, nach 10 min bei RT mit 111 mg 2,3-Dünethylbutan-2,3-diamindihydrochlorid (0.6 mmol, 2.5 Äquivalente) versetzt und über Nacht bei RT nachgerührt. Dann wurde per präparativer HPLC (Methode: RP18 Säule, Laufmittel: Acetonitril/Wasser-Gradient unter Zusatz von 0.1% TFA) gereinigt. Die erhaltene Produktfraktion wurde in Essigsäureethylester und gesättigter, wässriger Natriumhydrogencarbonat-Lösung gelöst und die wässrige Phase wurde zweimal mit Essigsäureethylester extrahiert. Die vereinigten organischen Phasen wurden über Natriumsulfat getrocknet, filtriert, eingeegnt und lyophilisiert. Es wurden 20 mg (20% d. Th.; Reinheit 99%) der Titelverbindung erhalten.
LC-MS (Methode 7): Rₜ = 0.57 min
MS (ESpos): m/z = 417.2 (M+H)⁺
¹H-NMR (400 MHz, DMSO-d₆): δ = 1.13 (s, 6 H), 1.42 (s, 6 H), 2.58 (s, 3 H), 5.30 (s, 2 H), 6.93 (t, 1 H), 7.02 (d, 1 H), 7.23 (t, 2 H), 7.53 - 7.65 (m, 1 H), 7.92 (s, 1 H), 8.89 (d, 1 H).

Die in Tabelle 12 gezeigten Beispiele wurden in Analogie zu Beispiel 141 hergestellt, indem die ensprechenden Carbonsäuren (Beispiel 3A, 16A und 21A) mit den entsprechenden - wie zuvor beschrieben hergestellten oder kommerziell erhältlichen - Aminen (1.05-2.5 Äquivalente) und *N*,*N-*Diisopropylethylamin (3-6 Äquivalente) unter den in der allgemeinen Arbeitsvorschrift 3 beschriebenen Reaktionsbedingungen umgesetzt wurden.

Beispielhafte Aufarbeitung der Reaktionsmischung: Die Reaktionslösung wurde mit Wasser versetzt, der entstandene Niederschlag noch 0.5-1.0 ausgeführt, abfiltriert, mit Wasser gewaschen und über Nacht im Hochvakuum getrocknet. Alternativ wurde der Niederschlag oder das Reaktionsrohgemisch direkt per präparativer HPLC (RP18 Säule, Laufmittel: Acetonitril/Wasser-Gradient unter Zusatz von 0.1 % TFA) weiter gereinigt und über Nacht im Hochvakuum getrocknet. Gegebenenfalls wurden die Produktfraktionen in Essigsäureethylester oder Dichlormethan aufgenommen und zweimal mit gesättigter, wässriger Natriumhydrogencarbonatlösung gewaschen. Die wässrige Phase wurde zweimal mit Essigsäureethylester oder Dichlormethan extrahiert und die vereinigten organischen Phasen wurden über Natriumsulfat getrocknet, filtriert und eingeengt.

**Tabelle 12:**

| **Beispiel** | **IUPAC-Name / Struktur (Ausbeute)** | **Analytische Daten** |
|---|---|---|
| **142** | *rac*-8-[(2,6-Difluorbenzyl)oxy]-*N*-[2-(dimethyl-amino)-3-methylbutyl]-2-methylimidaao[1,2-a]pyridin-3-carboxamid | LC-MS (Methode 2): Rₜ = 0.61 min |
| | | MS (ESpos): m/z = 431.3 (M+H)⁺ |
| | | ¹H-NMR (400 MHz, DMSO-d₆): |
| | 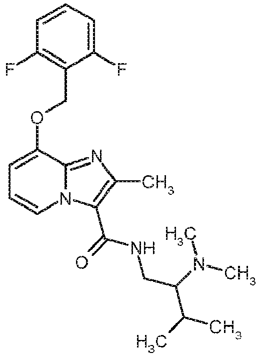 | δ = 0.95 (dd, 6 H),1.79 -1.89 (m, 1 H),2.31-2.41 (m, 7 H), 3.25 - 3.32 (m, 1 H), 3.39 - 3.48 (m, 1 H), 5.30 (s, 2 H), 6.93 (t, 1 H), 7.01 (d, 1 H), 7.24 (t, 2 H), 7.54 - 7.67 (m, 2 H), 8.71 (d, 1 H), [weiteres Signal unter Lösungsmittel-Peaks verborgen]. |
| | (84% d. Th.; Reinheit 96%) | |
| **143** | 8-[(2,6-Difluorbenzyl)oxy]-*N*-[2-(3,3-difluorpyrrolidin-1-yl)ethyl]-2-methylimidazo[1,2-a]pyridin-3-carboxamid | LC-MS (Methode 2): Rₜ = 0.65 min |
| | | MS (ESpos): m/z = 451.2 (M+H)⁺ |
| | | ¹H-NMR (400 MHz, DMSO-d₆): |
| | 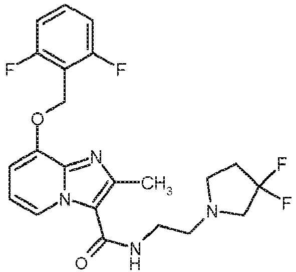 | δ = 2.16 - 2.31 (m, 2 H), 2.64 (t, 2 H), 2.77 (t, 2 H), 2.96 (t, 2 H), 3.38 - 3.46 (m, 2 H), 5.30 (s, 2 H), 6.93 (t, 1 H), 7.01 (d, 1 H), 7.24 (t, 2 H), 7.54 - 7.64 (m, 1 H), 7.82 (t, 1 H), 8.63 (d, 1 H), [weiteres Signal unter Lösungsmittel-Peaks verborgen]. |
| | (90% d. Th.; Reinheit 99%) | |
| **144** | 8-[(2,6-Difluorbenzyl)oxy]-*N*-{2-[(2-methoxyethyl)amino]ethyl}-2-methylimidaw[1,2-a]pyridin-3-carboxamid | LC-MS (Methode 2): Rₜ = 0.56 min |
| | | MS (ESpos): m/z = 419.2 (M+H)⁺ |
| | | ¹H-NMR (400 MHz, DMSO-d₆): |
| | 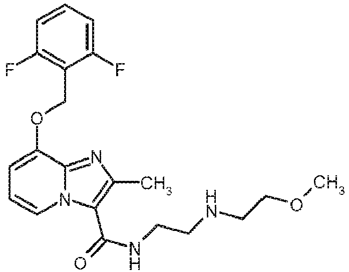 | δ = 1.82 (br. s, 1 H), 2.65 - 2.75 (m, 4 H), 3.23 (s, 3 H), 3.38 (m, 4 H), 5.28 (s, 2 H), 6.93 (t, 1 H), 7.01 (d, 1 H), 7.24 (t, 2 H), 7.54 - 7.64 (m, 1 H), 7.80 (t, 1 H), 8.63 (d, 1 H), [weiteres Signal unter Lösungsmittel-Peaks verborgen]. |
| | (60% d. Th.; Reinheit 99%) | |
| **145** | 8-[(2,6-Difluorbenzyl)oxy]-*N*-[2-dimethylamino)-2-methylpropyl]-2-methylimidazo[1,2-a]pyridin-3-carboxamid | LC-MS (Methode 2): Rₜ = 0.56 min |
| | | MS (ESpos): m/z = 417.2 (M+H)⁺ |
| | | ¹H-NMR (400 MHz, DMSO-d₆): |
| | 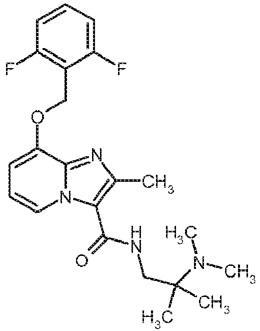 | δ = 1.01 (s, 6 H), 2.20 (s, 6 H), 2.56 (s, 3 H), 3.29 - 3.32 (m, 2 H; überlagert durch LösungsmittelSignal), 5.31 (s, 2 H), 6.94 (t, 1 H), 7.02 (d, 1 H), 7.24 (t, 2 H), 7.37 (t, 1 H), 7.54 - 7.65 (m, 1 H), 8.76 (d, 1 H). |
| | (90% d. Th.; Reinheit 99%) | |
| **146** | *N*-{2-[Cyclopropyl(2,2-difluorethyl)amino]ethyl}-8-[(2,6-difluorbenzyl)oxy]-2-methylimidazo[1,2-a]pyridin-3-carboxamid | LC-MS (Methode 2): Rₜ = 0.97 min |
| | | MS (ESpos): m/z = 465.3 (M+H)⁺ |
| | | ¹H-NMR (400 MHz, DMSO-d₆): |
| | 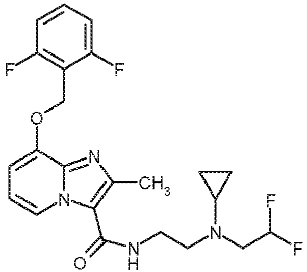 | δ = 0.35 - 0.42 (m, 2 H), 0.45 - 0.51 (m, 2 H), 2.05 - 2.14 (m, 1 H), 2.89 (t, 2 H), 3.03 (td, 2 H), 3.44 (dd, 2 H), 5.30 (s, 2 H), 6.13 (tt, 1 H), 6.92 (t, 1 H), 7.01 (d, 1 H), 7.24 (t, 2 H), 7.54 - 7.64 (m, 1 H), 7.67 (t, 1 H), 8.67 (d, 1 H), [weiteres Signal unter Lösungsmittel-Peaks verborgen]. |
| | (77% d. Th.; Reinheit: 97%) | |
| **147** | *rac-N*-(2-Amino-4,4,4-trifluorbutyl)-6-chlor-8-[(2,6-difluorbenzyl)oxy]-2-methylimidazo[1,2-a]pyridin-3-carboxamid | LC-MS (Methode 7): Rₜ = 0.75 min |
| | | MS (ESpos): m/z = 477.0 (M+H)⁺ |
| | | ¹H-NMR (400 MHz, DMSO-d₆): |
| | 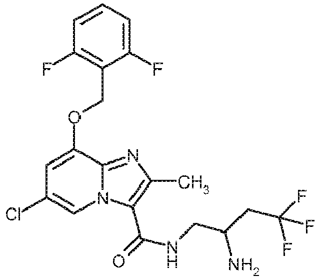 | δ =1.72 (br. s, 2 H), 2.18 - 2.32 (m, 1 H), 2.34 - 2.49 (m, 1 H), 3.16 - 3.28 (m, 2 H), 3.32 - 3.41 (m, 1 H), 5.32 (s, 2 H), 7.18 - 7.28 (m, 3 H), 7.55 - 7.65 (m, 1 H), 7.93 (t, 1 H), 8.64 (d, 1 H), [weiteres Signal unter Lösungsmittel-Peaks verborgen]. |
| | (63% d. Th.; Reinheit 99%) | |
| **148** | 6-Chlor-8-[(2,6-difluorbenzyl)oxy]-2-methyl-*N-*[2-methyl-2-(pyrrolidin-1-yl)propyl]imidazo[1,2-a]pyridin-3-carboxamid | LC-MS (Methode 7): Rₜ = 0.76 min |
| | | MS (ESpos): m/z = 477.1 (M+H)⁺ |
| | | ¹H-NMR (400 MHz, DMSO-d₆): |
| | 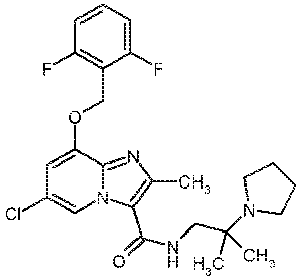 | δ = 1.04 (s, 6 H), 1.63 -1.72 (m, 4 H), 2.57 - 2.63 (m, 4 H), 3.28 - 3.31 (m, 2 H), 5.34 (s, 2 H), 7.18 - 7.29 (m, 3 H), 7.45 - 7.53 (m, 1 H), 7.56 - 7.66 (m, 1 H), 8.89 (d, 1 H), [weiteres Signal unter DMSO-Peak verborgen]. |
| | (64% d. Th.; Reinheit: 98%) | |
| **149** | 6-Chlor-8-[(2,6-difluorbenzyl)oxy]-*N*-[2-(isopropylamino)ethyl]-2-methylimidazo[1,2-a]pyridin-3-carboxamid | LC-MS (Methode 2): Rₜ = 0.73 min |
| | | MS (ESpos): m/z = 437.2 (M+H)⁺ |
| | | ¹H-NMR (400 MHz, DMSO-d₆): |
| | 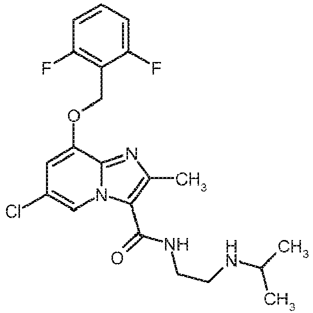 | δ = 0.98 (d, 6 H), 1.63 (br. s, 1 H), 2.66 - 2.79 (m, 3 H), 3.32 - 3.39 (m, 2 H, überlagert durch LösungsmittelSignal), 5.34 (s, 2 H), 7.19 (d, 1 H), 7.22 - 7.29 (m, 2 H), 7.56 - 7.65 (m, 1 H), 7.84 - 7.90 (m, 1 H), 8.77 (d, 1 H), [weiteres Signal unter DMSO-Peak verborgen]. |
| | (87% d. Th.; Reinheit 100%) | |
| **150** | 6-Chlor-*N*-{2-[cyclopropyl(2,2-difluorethyl)-amino]ethyl}-8-[(2,6-difluorbenzyl)oxy]-2-methylimidazo[1,2-a]pyridin-3-carboxamid | LC-MS (Methode 2): Rₜ = 1.22 min |
| | | MS (ESpos): m/z = 499.3 (M+H)⁺ |
| | 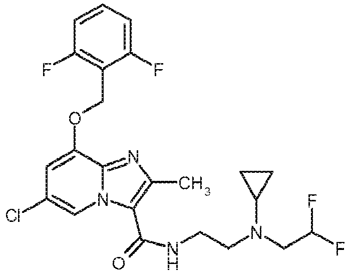 | ¹H-NMR (400 MHz, DMSO-d₆): |
| | | δ = 0.35 - 0.41 (m, 2 H), 0.46 - 0.52 (m, 2 H), 2.06 - 2.14 (m, 1 H), 2.48 (s, 3 H), 2.89 (t, 2 H), 3.02 (td, 2 H), 3.41 - 3.50 (m, 2 H), 5.34 (s, 2 H), 6.14 (tt, 1 H), 7.20 (d, 1 H), 7.26 (t, 2 H), 7.56 - 7.65 (m, 1 H), 7.74 (t, 1 H), 8.76 (d, 1 H). |
| | (78% d. Th.; Reinheit: 98%) | |
| **151** | 6-Chlor-8-[(2,6-difluorbenzyl)oxy]-*N-*[2-(4,4-difluorpiperidin-1-yl)ethyl]-2-methylimidazo[1,2-a]pyridin-3-carboxamid | LC-MS (Methode 2): Rₜ = 0.79 min |
| | | MS (ESpos): m/z = 499.2 (M+H)⁺ |
| | 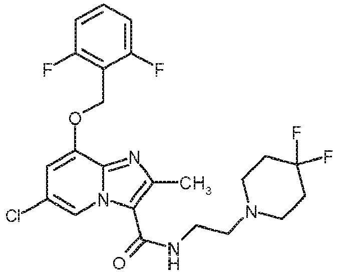 | ¹H-NMR (400 MHz, DMSO-d₆): |
| | | δ = 1.88 - 2.03 (m, 4 H), 3.39 - 3.47 (m, 2 H), 5.34 (s, 2 H), 7.19 (d, 1 H), 7.25 (t, 2 H), 7.55 - 7.65 (m, 1 H), 7.85 (t, 1 H), 8.76 (d, 1 H), [weitere Signale unter Lösungsmittel-Peaks verborgen]. |
| | (54% d. Th.; Reinheit: 97%) | |
| **152** | 6-Chlor-8-[(2,6-difluorbenzyl)oxy]-*N-*[2-(4-isopropylpiperazin-1-yl)ethyl]-2-methyl-imidazo[1,2-a]pyridin-3-carboxamid | LC-MS (Methode 2): Rₜ = 0.77 min |
| | | MS (ESpos): m/z = 506.4 (M+H)⁺ |
| | | ¹H-NMR (400 MHz, DMSO-d₆): |
| | 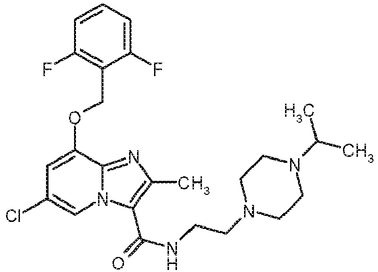 | δ = 0.94 (d, 6 H), 2.42 (br. s, 6 H), 3.37 - 3.46 (m, 2 H), 5.34 (s, 2 H), 7.18 (d, 1 H), 7.25 (t, 2 H), 7.55 - 7.66 (m, 1 H), 7.80 (t, 1 H), 8.75 (d, 1 H), [weitere Signale unter Lösungsmittel-Peaks verborgen]. |
| | (88% d. Th.; Reinheit 100%) | |
| **153** | 6-Chlor-8-[(2,6-difluorbenzyl)oxy]-*N*-{2-[(2-methoxyethyl)amino]ethyl}-2-methylimidazo[1,2-a]pyridin-3-carboxamid | LC-MS (Methode 2): Rₜ = 0.72 min |
| | | MS (ESpos): m/z = 453.2 (M+H)⁺ |
| | | ¹H-NMR (500 MHz, DMSO-d₆): |
| | 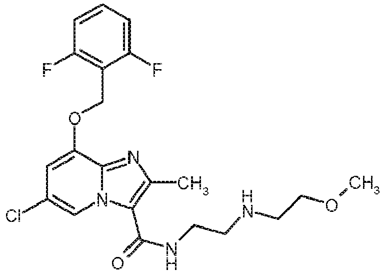 | δ = 2.66 - 2.76 (m, 4 H), 3.23 (s, 3 H), 3.36 - 3.41 (m, 4 H), 5.34 (s, 2 H), 7.19 (s, 1 H), 7.25 (t, 2 H), 7.56 - 7.66 (m, 1 H), 7.87 (t, 1 H), 8.76 (s, 1 H), [weitere Signale unter Lösungsmittel-Peaks verborgen]. |
| | (39% d. Th.; Reinheit 97%) | |
| **154** | 6-Chlor-8-[(2,6-difluorbenzyl)oxy]-2-methyl-N-[2-(4-methylpiperazin-1-yl)ethyl]imidazo[1,2-a]pyridin-3-carboxamid | LC-MS (Methode 2): Rₜ = 0.75 min |
| | | MS (ESpos): m/z = 478.4 (M+H)⁺ |
| | | ¹H-NMR (400 MHz, DMSO-d₆): |
| | 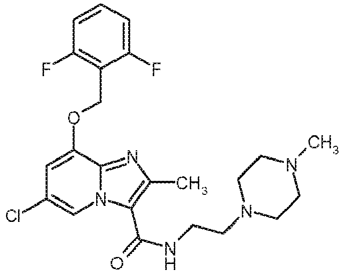 | δ = 2.14 (s, 3 H), 2.24 - 2.52 (m, 10 H, überlagert durch LösungsmittelSignal), 3.38 - 3.45 (m, 2 H), 5.34 (s, 2 H), 7.19 (d, 1 H), 7.26 (t, 2 H), 7.55 - 7.66 (m, 1 H), 7.79 (t, 1 H), 8.77 (d, 1 H), [weitere Signale unter Lösungsmittel-Peaks verborgen]. |
| | (81% d. Th.; Reinheit 100%) | |
| **155** | *N-*[(1-Aminocyclobutyl)methyl]-6-chlor-8-[(2,6-difluorbenzyl)oxy]-2-methylimidazo[1,2-a]pyridin-3-carboxamid Trifluoracetat | LC-MS (Methode 2): Rₜ = 0.77 min |
| | | MS (ESpos): m/z = 435.3 (M-TFA+H)⁺ |
| | | ¹H-NMR (500 MHz, DMSO-d₆): |
| | 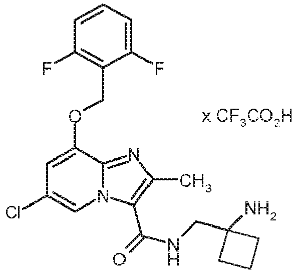 | δ = 1.79 - 1.97 (m, 2 H), 2.12 - 2.25 (m, 4 H), 2.56 (s, 3 H), 3.66 (d, 2 H), 5.36 (s, 2 H), 7.20 - 7.28 (m, 3 H), 7.57 - 7.65 (m, 1 H), 7.98 - 8.06 (m, 3 H), 8.21 (t, 1 H), 8.76 (s, 1 H). |
| | (27% d. Th.; Reinheit 95%) | |
| **156** | 6-Chlor-8-[(2,6-difluorbenzyl)oxy]-2-methyl-*N-*{2-[3-(pyrrolidin-1-yl)azetidin-1-yl]ethyl}-imidazo[1,2-a]pyridin-3-carboxamid | LC-MS (Methode 7): Rₜ = 0.62 min |
| | | MS (ESpos): m/z = 504.2 (M+H)⁺ |
| | | ¹H-NMR (400 MHz, DMSO-d₆): |
| | 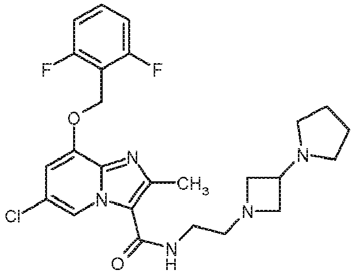 | δ = 1.63 -1.69 (m, 4 H), 2.32 - 2.36 (m, 4 H), 2.87 - 2.94 (m, 2 H), 2.97 - 3.05 (m, 1 H), 3.22 - 3.28 (m, 2 H), 3.40 (t, 2 H), 5.34 (s, 2 H), 7.19 (d, 1 H), 7.25 (t, 2 H), 7.56 - 7.65 (m, 1 H), 7.84 (t, 1 H), 8.77 (d, 1 H), [weitere Signale unter den Lösungsmittel-Peaks verborgen]. |
| | (35% d. Th.; Reinheit 98%) | |
| **157** | *rac-N*-(2-Amino-2-methylbutyl)-6-chlor-8-[(2,6-difluorbenzyl)oxy]-2-methylimidazo[1,2-a]pyridin-3-carboxamid | LC-MS (Methode 7): Rₜ = 0.69 min |
| | | MS (ESpos): m/z = 437.1 (M+H)⁺ |
| | | ¹H-NMR (400 MHz, DMSO-d₆): |
| | 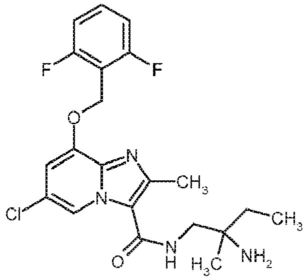 | δ = 0.86 (t, 3 H), 0.97 (s, 3 H), 1.30 - 1.38 (m, 2 H), 1.43 (br. s, 1 H), 3.15 - 3.27 (m, 2 H), 5.34 (s, 2 H), 7.19 (d, 1 H), 7.25 (t, 2 H), 7.55 - 7.65 (m, 1 H), 7.66 - 7.77 (m, 1 H), 8.77 (d, 1 H), [weiteres Signal unter den Lösungsmittel-Peaks verborgen]. |
| | (89% d. Th.; Reinheit 99%) | |
| **158** | 6-Chlor-8-[(2,6-difluorbenzyl)oxy]-*N*-{2-[(2*R*,6*S*)-2,6-dimethylmorpholin-4-yl]ethyl}-2-methylimidazo[1,2-a]pyridin-3-carboxamid | LC-MS (Methode 2): Rₜ = 0.82 min |
| | | MS (ESpos): m/z = 493.3 (M+H)⁺ |
| | | ¹H-NMR (400 MHz, DMSO-d₆): |
| | 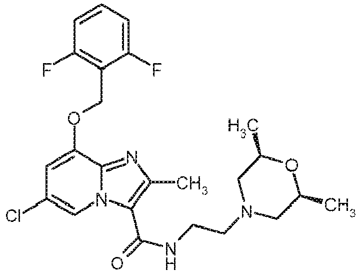 | δ = 1.05 (d, 6 H),1.67 (t, 2 H), 2.81 (d, 2 H), 3.38 - 3.46 (m, 2 H), 3.50 - 3.60 (m, 2 H), 5.34 (s, 2 H), 7.20 (d, 1 H), 7.25 (t, 2 H), 7.56 - 7.66 (m, 1 H), 7.81 (t, 1 H), 8.76 (d, 1 H), [weitere Signale unter den Lösungsmittel-Peaks verborgen]. |
| | (92% d. Th.; Reinheit 99%) | |
| **159** | *rac-N*-(2-Amino-3-methoxy-2-methylpropyl)-6-chlor-8-[(2,6-difluorbenzyl)oxy]-2-methylimidazo[1,2-a]pyridin-3-carboxamid | LC-MS (Methode 2): Rₜ = 0.73 min |
| | | MS (ESpos): m/z = 453.3 (M+H)⁺ |
| | | ¹H-NMR (400 MHz, DMSO-d₆): |
| | 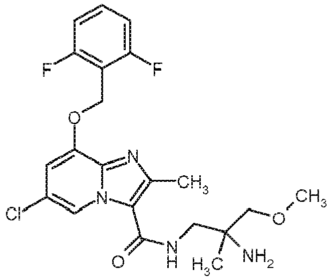 | δ = 1.00 (s, 3 H),1.72 (br. s, 2 H), 3.12 - 3.20 (m, 2 H), 3.22 - 3.31 (m, 5 H, überlagert mit Lösungsmittelsignal), 5.34 (s, 2 H), 7.20 (d, 1 H), 7.25 (t, 2 H), 7.56 - 7.71 (m, 2 H), 8.81 (d, 1 H), [weiteres Signal unter den Lösungsmittel-Peaks verborgen]. |
| | (88% d. Th.; Reinheit 100%) | |
| **160** | *N*-(3-Aminocyclohexyl)-8-[(2,6-difluorbenzyl)oxy]-2,6-dimethylimidazo[1,2-a]pyridin-3-carboxamid | LC-MS (Methode 2): Rₜ = 0.60 min |
| | | MS (ESpos): m/z = 429.3 (M+H)⁺ |
| | (Mischung von Stereoisomeren) | ¹H-NMR (400 MHz, DMSO-d₆): |
| | 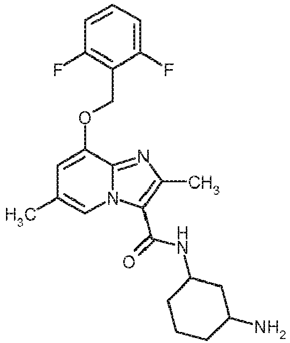 | δ = 0.89 -1.39 (m, 4 H), 1.42 -1.99 (m, 6 H), 2.30 (s, 3 H), 2.47 (s, 3 H), 2.62 - 2.72 und 3.06 - 3.11 (m, zusammen 1 H), 3.73 - 3.89 und 4.21 - 4.28 (m, zusammen 1 H), 5.28 (s, 2 H), 6.89 (s, 1 H), 7.23 (t, 2 H), 7.54 - 7.63 (m, 1 H), 7.79 und 7.92 (2 x d, zusammen 1 H), 8.85 und 8.89 (2 x s, zusammen 1 H). |
| | (58% d. Th.; Reinheit 98%) | |
| **161** | *rac-N*-(2-Amino-2-methylbutyl)-8-[(2,6-difluorbenzyl)oxy]-2,6-dimethylimidazo[1,2-a]pyridin-3-carboxamid | LC-MS (Methode 7): Rₜ = 0.56 min |
| | | MS (ESpos): m/z = 417.2 (M+H)⁺ |
| | | ¹H-NMR (400 MHz, DMSO-d₆): |
| | 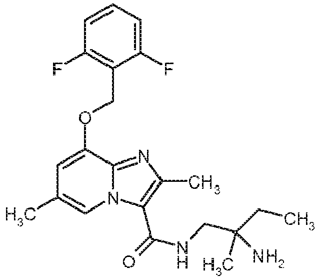 | δ = 0.86 (t, 3 H), 0.97 (s, 3 H),1.30 - 1.40 (m, 2 H), 1.45 (br. s, 2 H), 2.31 (s, 3 H), 2.53 (s, 3 H, überlagert mit Lösungsmittel-Peaks), 3.14 - 3.26 (m, 2 H), 5.29 (s, 2 H), 6.91 (s, 1 H), 7.20 - 7.28 (m, 2 H), 7.54 - 7.65 (m, 2 H), 8.49 (s, 1 H). |
| | (83% d. Th.; Reinheit 99%) | |
| **162** | 8-[(2,6-Difluorbaizyl)oxy]-2,6-dimethyl-*N*-{2-[3-(pyrrolidin-1-yl)azetidin-1-yl]ethyl}imidazo[1,2-a]pyridin-3-carboxamid | LC-MS (Methode 2): Rₜ = 0.58 min |
| | | MS (ESpos): m/z = 484.4 (M+H)⁺ |
| | | ¹H-NMR (400 MHz, DMSO-d₆): |
| | 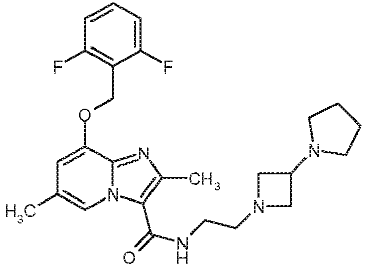 | δ =1.64 -1.69 (m, 4 H), 2.31 (s, 3 H), 2.32 - 2.39 (m, 4 H), 2.47 (s, 3 H), 2.56 - 2.62 (m, 2 H, überlagert mit Lösungsmittel-Peaks), 2.88 - 2.97 (m, 2 H), 2.98 - 3.07 (m, 1 H), 3.21 - 3.28 (m, 2 H), 3.38 - 3.45 (m, 2 H), 5.28 (s, 2 H), 6.91 (s, 1 H), 7.24 (t, 2 H), 7.54 - 7.64 (m, 1 H), 7.71 (t, 1 H), 8.47 (s, 1 H). |
| | (56% d. Th.; Reinheit 97%) | |
| **163** | 8-[(2,6-Difluorbenzyl)oxy]-*N*-[2-(4,4-difluor-piperidin-1-yl)ethyl]-2,6-dimethylimidazo[1,2-a]pyridin-3-carboxamid | LC-MS (Methode 2): Rₜ = 0.66 min |
| | | MS (ESpos): m/z = 479.3 (M+H)⁺ |
| | | ¹H-NMR (400 MHz, DMSO-d₆): |
| | 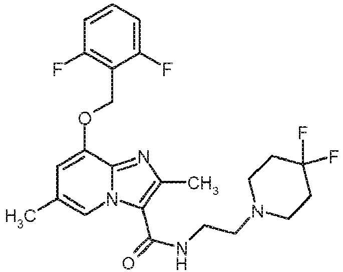 | δ = 1.88 - 2.02 (m, 4 H), 2.31 (s, 3 H), 2.56 - 2.60 (m, 4 H), 3.38 - 3.46 (m, 2 H), 5.27 (s, 2 H), 6.92 (s, 1 H), 7.24 (t, 2 H), 7.55 - 7.64 (m, 1 H), 7.71 (t, 1 H), 8.47 (s, 1 H), [weitere Signale unter Lösungsmittel-Peaks verborgen]. |
| | (56% d. Th.; Reinheit 98%) | |
| **164** | *N-*{2-[Cyclopropyl(2,2-difluorethyl)amino]ethyl}-8-[(2,6-difluorbenzyl)oxy]-2,6-dimethylimidazo[1,2-a]pyridin-3-carboxamid | LC-MS (Methode 2): Rₜ = 0.99 min |
| | | MS (ESpos): m/z = 479.3 (M+H)⁺ |
| | | ¹H-NMR (400 MHz, DMSO-d₆): |
| | 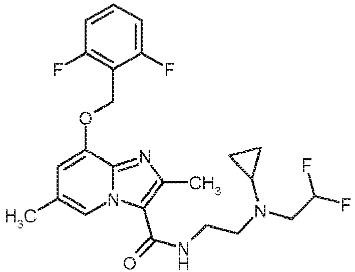 | δ = 0.35 - 0.42 (m, 2 H), 0.45 - 0.53 (m, 2 H), 2.06 - 2.13 (m, 1 H), 2.30 (s, 3 H), 2.46 (s, 3 H), 2.89 (t, 2 H), 3.03 (td, 2 H), 3.39 - 3.48 (m, 2 H), 5.27 (s, 2 H), 6.14 (tt, 1 H), 6.91 (s, 1 H), 7.24 (t, 2 H), 7.54 - 7.66 (m, 2 H), 8.48 (s, 1 H). |
| | (71% d. Th.; Reinheit 100%) | |
| **165** | *N*-[(1-Aminocyclobutyl)methyl]-8-[(2,6-difluorbenzyl)oxy]-2,6-dimethylimidazo[1,2-a]pyridin-3-carboxamid ¹⁾ | LC-MS (Methode 2): Rₜ = 0.63 min |
| | | MS (ESpos): m/z = 415.3 (M+H)⁺ |
| | | ¹H-NMR (400 MHz, DMSO-d₆): |
| | 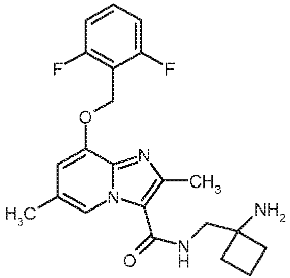 | δ = 1.56 -1.83 (m, 4 H), 1.92 - 2.03 (m, 2 H), 2.31 (s, 3 H), 3.39 (d, 2 H), 5.28 (s, 2 H), 6.92 (s, 1 H), 7.24 (t, 2 H), 7.53 - 7.65 (m, 2 H), 8.49 (s, 1 H), [weiteres Signal unter Lösungsmittel-Peaks verborgen]. |
| | (22% d. Th.; Reinheit 97%) | |
| **166** | *rac-N*-(2-Amino-3-methoxy-2-methylpropyl)-8-[(2,6-difluorbenzyl)oxy]-2,6-dimethylimidazo[1,2-a]pyridin-3-carboxamid | LC-MS (Methode 2): Rₜ = 0.62 min |
| | | MS (ESpos): m/z = 433.3 (M+H)⁺ |
| | | ¹H-NMR (400 MHz, DMSO-d₆): |
| | 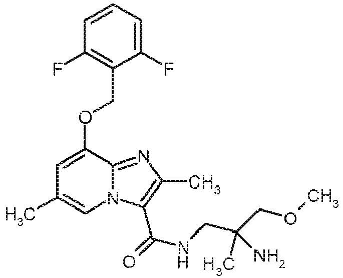 | δ = 1.00 (s, 3 H), 1.52 - 1.62 (br. s, 2 H), 2.31 (s, 3 H), 2.53 (s, 3 H), 3.123 - 3.19 (m, 2 H), 3.24 - 3.30 (m, 5 H), 5.28 (s, 2 H), 6.92 (s, 1 H), 7.24 (t, 2 H), 7.51 - 7.64 (m, 2 H), 8.52 (s, 1 H). |
| | (80% d. Th.; Reinheit 100%) | |
| **167** | 8-[(2,6-Difluorbenzyl)oxy]-*N*-{2-[(2*R*,6*S*)-2,6-dimethylmorpholin-4-yl]ethyl}-2,6-dimethyl-imidazo[1,2-a]pyridin-3-carboxamid | LC-MS (Methode 2): Rₜ = 0.71 min |
| | | MS (ESpos): m/z = 473.3 (M+H)⁺ |
| | | ¹H-NMR (400 MHz, DMSO-d₆): |
| | 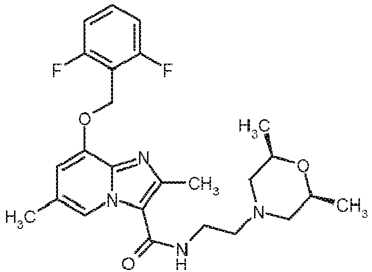 | δ = 1.05 (d, 6 H),1.67 (t, 2 H), 2.31 (s, 3 H), 2.80 (d, 2 H), 3.38 - 3.45 (m, 2 H), 3.49 - 3.60 (m, 2 H), 5.28 (s, 2 H), 6.91 (s, 1 H), 7.24 (t, 2 H), 7.54 - 7.64 (m, 1 H), 7.68 (t, 1 H), 8.48 (s, 1 H), [weitere Signale unter Lösungsmittel-Peaks verborgen]. |
| | (76% d. Th.; Reinheit 100%) | |

| | | |
|---|---|---|
| ¹⁾ Es erfolgte eine zusätzliche chromatographische Trennung: Säule: Sunfire C18, 5 µm, 250 x 20 mm, Eluent: 89% Methanol, 11% 1%ige TFA in Wasser, Fluß: 25 ml/min; 25°C, Detektion: 210 nm]. | | |

### Beispiel 168

### N-[2-(4-Cyclopropylpiperazin-1-yl)efhyl]-8-[(2,6-difluorbenzyl)oxy]-2-methylimidazo[1,2-a]pyridin-3-carboxamid

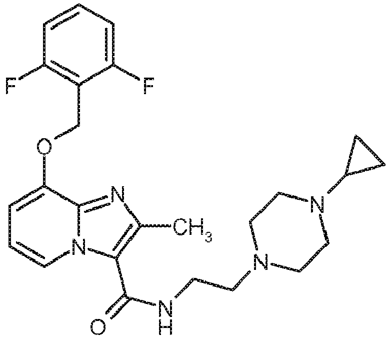

118 mg 8-[(2,6-Difluorbenzyl)oxy]-2-methyl-*N*-(2-oxoethyl)imidazo[1,2-a]pyridin-3-carboxamid (Beispiel 194A, 0.16 mmol, 1 Äquivalent) wurden in 0.8 ml trockenem Dichlorethan suspendiert, mit 23 mg 1-Cyclopropylpiperazin (0.18 mmol, 1.1 Äquivalente) versetzt und für 3 h bei RT gerührt. Anschließend wurden 52 mg Natriumtriacetoxyborhydrid (0.25 mmol, 1.5 Äquivalente) zugesetzt und es wurde über Nacht bei RT gerührt. Dann wurde mit 1 N wässriger Natronlauge versetzt und dreimal mit Dichlormethan extrahiert. Die vereinigten organischen Phasen wurden mit Wasser und gesättigter, wässriger Natriumchlorid-Lösung gewaschen, über Natriumsulfat getrocknet, filtriert und eingeengt. Der Rückstand wurde in Methanol gelöst und per präparativer HPLC (Methode: RP18 Säule, Laufmittel: Acetonitril/Wasser-Gradient unter Zusatz von 0.1% TFA) aufgereinigt. Die Produktfraktion wurde in Essigsäureethylester aufgenommen und zweimal mit gesättigter, wässriger Natriumhydrogen-carbonat-Lösung gewaschen. Die vereinigten organischen Phasen wurden über Natriumsulfat getrocknet, filtriert und eingeengt. Es wurden 33.5 mg (43% d. Th.) der Titelverbindung erhalten.
LC-MS (Methode 7): Rₜ = 0.60 min
MS (ESpos): m/z = 470.2 (M+H)⁺
¹H-NMR (400 MHz, DMSO-d₆): δ = 0.22 - 0.30 (m, 2 H), 0.34 - 0.43 (m, 2 H), 1.53 - 1.62 (m, 1 H), 2.30 - 2.44 (m, 3 H), 3.37 - 3.46 (m, 2 H), 5.30 (s, 2 H), 6.93 (t, 1 H), 7.01 (d, 1 H), 7.23 (t, 2 H), 7.55 - 7.64 (m, 1 H), 7.70 (t, 1 H), 8.67 (d, 1 H), [weitere Signale unter Lösungsmittel-Peaks verborgen].

In Analogie zu Beispiel 168 wurden die in Tabelle 13 gezeigten Beispiele aus den Aldehyden der Beispiele 194A,195A und 196A hergestellt.

**Tabelle 13:**

| **Beispiel** | **IUPAC-Name / Struktur (Ausbeute)** | **Analytische Daten** |
|---|---|---|
| **169** | 8-[(2,6-Difluorbenzyl)oxy]-*N-*{2-[(2,2-difluorethyl)amino]ethyl}-2-methylimidazo[1,2-a]pyridin-3-carboxamid | LC-MS (Methode 7): Rₜ = 0.57 min |
| | | MS (ESpos): m/z = 425.1 (M+H)⁺ |
| | 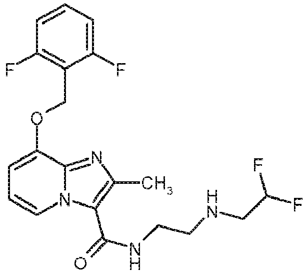 | ¹H-NMR (400 MHz, DMSO-d₆): δ = 2.78 (t, 2 H), 2.93 (dt, 2 H), 3.34 - 3.42 (m, 2 H), 5.30 (s, 2 H), 6.01 (tt, 1 H), 6.93 (t, 1 H), 7.01 (d, 1 H), 7.23 (t, 2 H), 7.55 - 7.64 (m, 1 H), 7.79 (t, 1 H), 8.64 (d, 1 H), [weiteres Signal unter Lösungsmittel-Peaks verborgen]. |
| | (51% d. Th.; Reinheit 93%) | |
| **170** | 8-[(2,6-Difluorbenzyl)oxy]-2-methyl-*N*-[(2*R*)-1-(morpholin-4-yl)propan-2-yl]imidazo[1,2-a]pyridin-3-carboxamid ¹⁾ | LC-MS (Methode 1): Rₜ = 0.60 min |
| | | MS (ESpos): m/z = 445.2 (M+H)⁺ |
| | 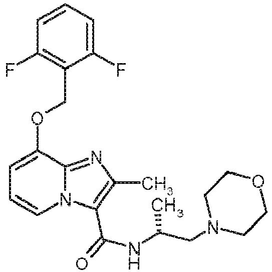 | ¹H-NMR (400 MHz, DMSO-d₆): δ = 1.17 (d, 3 H), 2.17 - 2.43 (m, 3 H), 3.46 - 3.62 (m, 4 H), 4.15 - 4.28 (m, 1 H), 5.29 (s, 2 H), 6.91 (t, 1 H), 6.99 (d, 1 H), 7.18 - 7.28 (m, 2 H), 7.53 - 7.63 (m, 1 H), 7.68 (d, 1 H), 8.56 (d, 1 H), [weitere Signale unter Lösungsmittel-Peaks verborgen]. |
| | (39% d. Th.; Reinheit 100%) | |
| **171** | 8-[(2,6-Difluorbenzyl)oxyl-2-methyl-*N*-[(2*S*)-1-(morpholin-4-yl)propan-2-yl]imidazo[1,2-a]pyridin-3-carboxamid ¹⁾ | LC-MS (Methode 7): Rₜ = 0.59 min |
| | | MS (ESpos): m/z = 445.2 (M+H)⁺ |
| | 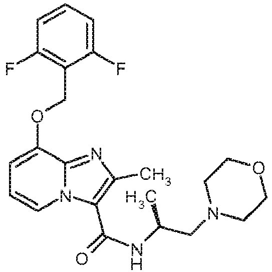 | ¹H-NMR (400 MHz, DMSO-d₆): δ = 1.17 (d, 3 H), 2.15 - 2.43 (m, 3 H), 3.47 - 3.62 (m, 4 H), 4.15 - 4.28 (m, 1 H), 5.30 (s, 2 H), 6.92 (t, 1 H), 6.99 (d, 1 H), 7.18 - 7.28 (m, 2 H), 7.53 - 7.63 (m, 1 H), 7.69 (d, 1 H), 8.56 (d, 1 H), [weitere Signale unter Lösungsmittel-Peaks verborgen]. |
| | (95% d. Th.; Reinheit 100%) | |

| | | |
|---|---|---|
| ¹⁾ Die in den Reaktionen entstandenen Minderenantiomere (teilweise Racemisierung unten diesen Bedingungen) wurden an chiraler Phase abgetrennt [Säule: Daicel Chiralpak AD-H, 5 µm, 250 x 20 mm, Eluent: 50% iso-Hexan, 50% Ethanol, 0.2% Diethylamin, Fluß: 20 ml/min; 25°C/40°C, Detektion: 210/220 nm]. | | |

### Beispiel 172

### 8-[(2,6-Difluorbenzyl)oxy]-2,6-dimethyl-N-[(2S)-piperidin-2-ylmethyl]imidazo[1,2-a]pyridin-3-carboxamid

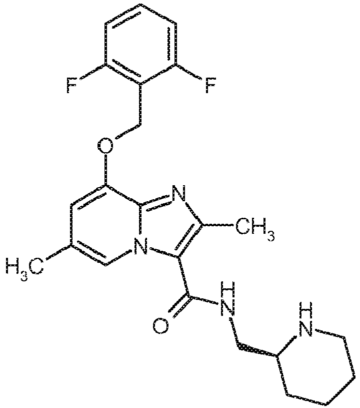

75 mg 8-[(2,6-Difluorbenzyl)oxy]-2,6-dimethylimidazol[1,2-a]pyridin-3-carbonsäure (Beispiel 21A, 0.23 mmol, 1 Äquivalent), 90 mg *O*-(7-Azabenzotriaml-1-yl)-*N,N,N'N'-*tetramethyluroniumhexafluorphosphat (HATU, 0.24 mmol, 1.05 Äquivalente) und 88 mg *N*,*N-*Diisopropylethylamin (0.12 ml, 0.68 mmol, 3 Äquivalente) in 1.4 ml DMF wurden bei RT mit 53 mg (0.25 mmol, 1.1 Äquivalente) tert.-Butyl-(2S)-2-(aminomethyl)peridin-1-carboxylat versetzt und über Nacht bei RT gerührt. Nach beendeter Reaktionszeit wurde per präparativer HPLC (Methode: RP18 Säule, Laufmittel: Acetonitril/Wasser-Gradient unter Zusatz von 0.1% TFA) gereinigt. Die erhaltene Produktfraktion wurde in Dichlormethan gelöst und mit gesättigter, wässriger Natriumhydrogencarbonat-Lösung gewaschen. Die wässrige Phase wurde zweimal mit Dichlormethan extrahiert und die vereinigten organischen Phasen wurden über Natriumsulfat getrocknet, filtriert, eingeengt und lyophilisiert. Es wurden 66 mg (64% d. Th.; Reinheit 95%) der Titelverbindung erhalten.
LC-MS (Methode 2): Rₜ = 0.65 min
MS (ESpos): m/z = 429.3 (M+H)⁺
¹H-NMR (400 MHz, DMSO-d₆): δ = 0.99 - 1.14 (m, 1 H), 1.21 - 1.36 (m, 2 H), 1.45 - 1.54 (m, 1 H), 1.59 -1.66 (m, 1 H), 1.70 - 1.77 (m, 1 H), 2.31 (s, 3 H), 2.60 - 2.69 (m, 1 H), 2.92 - 2.98 (m, 1 H), 3.16 - 3.29 (m, 2 H), 5.28 (s, 2 H), 6.91 (s, 1 H), 7.24 (t, 2 H), 7.55 - 7.64 (m, 1 H), 7.74 (t, 1 H), 8.46 (s, 1 H), [weiteres Signal unter Lösungsmittel-Peaks verborgen].

### Beispiel 173

### 8-[(2,6-Difluorbenzyl)oxy]-2,6-dimethyl-N-[(2R)-piperidin-2-ylmethyl]imidazo[1,2-a]pyridin-3-carboxamid

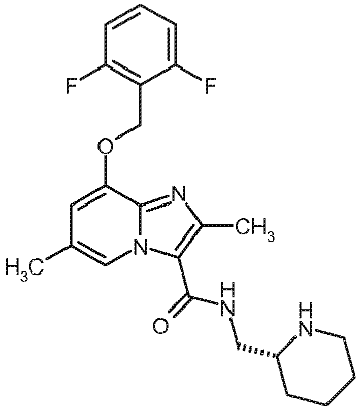

56 mg tert.-butyl-(2*R*)-2-{[({8-[(2,6-difluorbenzyl)oxy]-2,6-dimethylimidazo[1,2-a]pyridin-3-yl}carbonyl)amino]methyl}piperidin-1-carboxylat Trifluoracetat Beispiel 183A (0.09 mmol) wurden in 2 M Salzsäure in Diethylether vorgelegt und 5 h bei RT gerührt. Der entstandene Niederschlag wurde abfiltriert und mit Diethylether gewaschen, in Dichlormethan gelöst und mit gesättigter, wässriger Natriumhydrogencarbonat-Lösung gewaschen. Die wässrige Phase wurde zweimal mit Dichlormethan extrahiert und die vereinigten organischen Phasen wurden über Natriumsulfat getrocknet, filtriert, eingeengt und lyophilisiert. Es wurden 30 mg (78% d. Th.; Reinheit 95%) der Titelverbindung erhalten.
LC-MS (Methode 2): Rₜ = 0.64 min
MS (ESpos): m/z = 429.3 (M+H)⁺
¹H-NMR (400 MHz, DMSO-d₆): δ = 0.99 - 1.14 (m, 1 H), 1.21 - 1.36 (m, 2 H), 1.45 - 1.54 (m, 1 H), 1.59 -1.66 (m, 1 H), 1.71 - 1.77 (m, 1 H), 2.31 (s, 3 H), 2.60 - 2.69 (m, 1 H), 2.92 - 2.98 (m, 1 H), 3.16 - 3.29 (m, 2 H), 5.28 (s, 2 H), 6.90 (s, 1 H), 7.24 (t, 2 H), 7.55 - 7.64 (m, 1 H), 7.74 (t, 1 H), 8.46 (s, 1 H), [weiteres Signal unter Lösungsmittel-Peaks verborgen].

### Beispiel 174

### ent-N-[(3S)-3-Aminobutan-2-yl]-8-[(2,6-difluorbenzyl)oxy]-2-methylimidazo[1,2-a]pyridin-3-carboxamid (Diastereomer A)

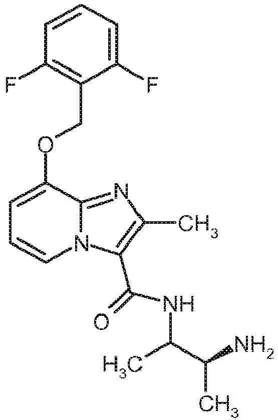

44.1 mg *N*-[(3*S*)-3-Aminobutan-2-yl]-8-[(2,6-difluorbenzyl)oxy]-2-methylimidazo[1,2-a]pyridin-3-carboxamid (Beispiel 120) wurden an chiraler Phase in die Diastereomere getrennt [Säule: Daicel Chiralpak AD-H, 5 µm, 250 x 20 mm, Eluent: 50% iso-Hexan, 50% Ethanol, 0.2% Diethylamin, Fluß: 20 ml/min; 25°C, Detektion: 230 nm].
Ausbeute Diastereomer A: 19.7 mg (100% ee)
Diastereomer A: Rₜ = 8.90 min [Daicel Chiralpak AD-H, 5 µm, 250 x 20 mm, Eluent: Eluent: 50% iso-Hexan, 50% Ethanol, 0.2% Diethylamin, Fluß: 1 ml/min; 25°C, Detektion: 230 nm].

### Beispiel 175

### ent-N-[(3S)-3-Aminobutan-2-yl]-8-[(2,6-difluorbenzyl)oxy]-2-methylimidazo[1,2-a]pyridin-3-carboxamid (Diastereomer B)

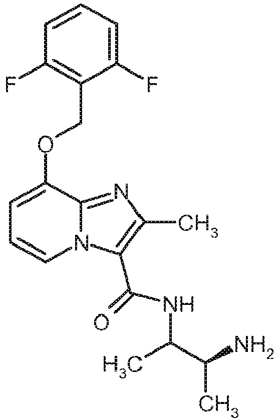

44.1 mg *rac-N-*[(3*S*)-3-Aminobutan-2-yl]-8-[(2,6-difluorbenzyl)oxy]-2-methylimidazo[1,2-a]pyridin-3-carboxamid (Beispiel 120) wurden an chiraler Phase in die Diastereomere getrennt [Säule: Daicel Chiralpak AD-H, 5 µm, 250 x 20 mm, Eluent: 50% iso-Hexan, 50% Ethanol, 0.2% Diethylamin, Fluß: 20 ml/min; 25°C, Detektion: 230 nm].
Ausbeute Diastereomer B: 2.3 mg (>90% ee)
Diastereomer A: Rₜ = 12.68 min [Daicel Chiralpak AD-H, 5 µm, 250 x 20 mm, Eluent: Eluent: 50% iso-Hexan, 50% Ethanol, 0.2% Diethylamin, Fluß: 1 ml/min; 25°C, Detektion: 230 nm].

### Beispiel 176

### ent-N-[(3R)-3-Aminobutan-2-yl]-8-[(2,6-difluorbenzyl)oxy]-2-methylimidazo[1,2-a]pyridin-3-carboxamid (Diastereomer A)

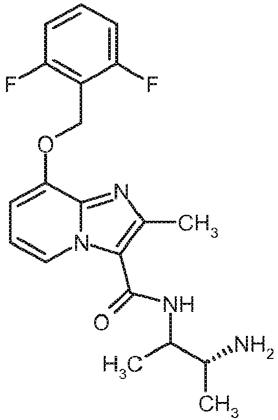

90 mg von *rac*-Benzyl-{(2*R*)-3-[({8-[(2,6-difluorbenzyl)oxy]-2-methylimidazo[1,2-a]pyhdin-3-yl}carbonyl)amino]butan-2-yl}carbamat (Beispiel 121) wurden an chiraler Phase in die Diastereomere getrennt [Säule: Daicel Chiralpak OD-H, 5 µm, 250 x 20 mm, Eluent: 50% iso-Hexan, 50% Iso-Propanol, 0.2% Diethylamin, Fluß: 20 ml/min; 25°C, Detektion: 230 nm].
Ausbeute Diastereomer A: 29.6 mg (100% ee)
Diastereomer A: Rₜ = 5.94 min [Daicel Chiralpak OD-H, 5 µm, 250 x 20 mm, Eluent: Eluent: 50% iso-Hexan, 50% Ethanol, 0.2% Diethylamin, Fluß: 1 ml/min; 25°C, Detektion: 230 nm].

### Beispiel 177

### ent-N-[(3R)-3-Aminobutan-2-yl]-8-[(2,6-difluorbenzyl)oxy]-2-methylimidazo[1,2-a]pyridin-3-carboxamid (Diastereomer B)

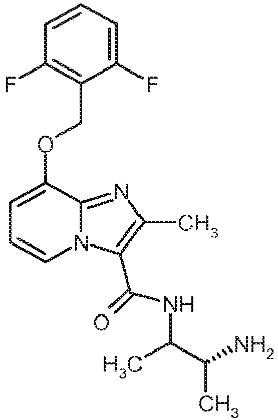

90 mg von *rac*-Benzyl-{(2*R*)-3-[({8-[(2,6-difluorbenzyl)oxy]-2-methylimidazo[1,2-a]pyridin-3-yl}carbonyl)amino]butan-2-yl}carbamat (Beispiel 121) wurden an chiraler Phase in die Diastereomere getrennt [Säule: Daicel Chiralpak OD-H, 5 µm, 250 x 20 mm, Eluent: 50% iso-Hexan, 50% Iso-Propanol, 0.2% Diethylamin, Fluß: 20 ml/min; 25°C, Detektion: 230 nm].
Ausbeute Diastereomer B: 4.9 mg (88% ee)
Diastereomer B: Rₜ = 9.79 min [Daicel Chiralpak OD-H, 5 µm, 250 x 20 mm, Eluent: Eluent: 50% iso-Hexan, 50% Ethanol, 0.2% Diethylamin, Fluß: 1 ml/min; 25°C, Detektion: 230 nm]

### Beispiel 178

### ent-N-[2-Amino-1-(3,4-difluorphenyl)ethyl]-8-(cyclohexylmethoxy)-2-methylimidazo[1,2-a]pyridin-3-carboxamid (Enantiomer A)

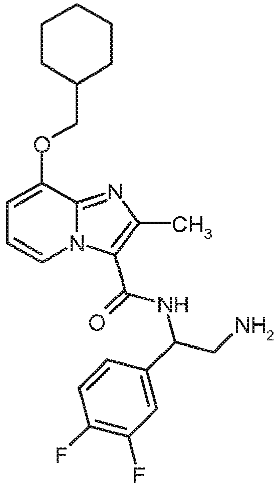

113 mg *rac-N-*[2-Amino-1-(3,4-difluorphenyl)ethyl]-8-cyclohexylmethoxy)-2-methylimidazo[1,2-a]pyridin-3-carboxamid (Beispiel 46) wurden an chiraler Phase in die Enantiomere getrennt [Säule: Daicel Chiralpak OD-H, 5 µm, 250 x 20 mm, Eluent: 50% iso-Hexan, 50% Ethanol, 0.2% Diethylamin, Fluß: 20 ml/min; 25°C, Detektion: 230 nm].
Ausbeute: Enantiomer A: 18.5 mg (89% ee)
Enantiomer A: Rₜ = 7.89 min [Daicel Chiralpak OD-H, 5 µm, 250 x 4.6 mm, Eluent: Eluent: 50% iso-Hexan, 50% Ethanol, 0.2% Diethylamin, Fluß: 1 ml/min; 40°C, Detektion: 220 nm].

### Beispiel 179

### ent-N-[2-Amino-1-(3,4-difluorphenyl)ethyl]-8-(cyclohexylmethoxy)-2-methylimidazo[1,2-a]pyridin-3-carboxamid (Enantiomer B)

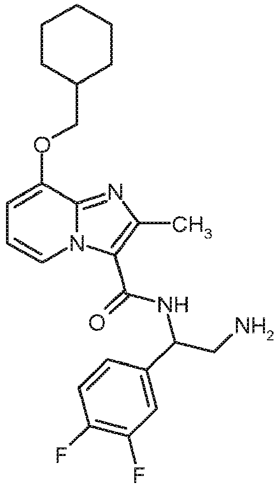

113 mg *rac*-*N-*[2-Amino-1-(3,4-difluorphenyl)ethyl]-8-(cyclohexymiethoxy)-2-methylimidazo[1,2-a]pyridin-3-carboxamid (Beispiel 46) wurden an chiraler Phase in die Enantiomere getrennt [Säule: Daicel Chiralpak OD-H, 5 µm, 250 x 20 mm, Eluent: 50% iso-Hexan, 50% Ethanol, 0.2% Diethylamin, Fluß: 20 ml/min; 25°C, Detektion: 230 nm].
Ausbeute: Enantiomer B: 28 mg (97% ee)
Enantiomer B: Rₜ = 12.84 min [Daicel Chiralpak OD-H, 5 µm, 250 x 4.6 mm, Eluent: Eluent: 50% iso-Hexan, 50% Ethanol, 0.2% Diethylamin, Fluß: 1 ml/min; 40°C, Detektion: 220 nm].

### Beispiel 180

### ent-8-[(2,6-Difluorbenzyl)oxy]-2-methyl-N-[2-(morpholin-4-yl)propyl]midazo[1,2-a]pyridin-3-carboxamid (Enantiomer A)

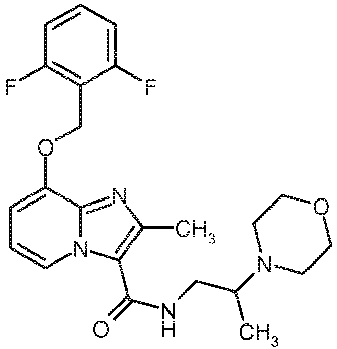

120 mg *rac*-8-[(2,6-Difluoibenzyl)oxy]-2-methyl-*N*-[2-(morpholin-4-yl)propyl]imidazo[1,2-a]pyridin-3-carboxamid (Beispiel 132) wurden an chiraler Phase in die Enantiomere getrennt [Säule: Daicel Chiralpak AD-H, 5 µm, 250 x 20 mm, Eluent: 50% iso-Hexan, 50% Iso-Propanol, 0.2% Diethylamin, Fluß: 20 ml/min; 40°C, Detektion: 210 nm].
Ausbeute: Enantiomer A: 45 mg (100% ee)
Enantiomer A: Rₜ = 8.99 min [Daicel Chiralpak AD-H, 5 µm, 250 x 4.6 mm, Eluent: Eluent: 50% iso-Hexan, 50% Iso-Propanol, 0.2% Diethylamin, Fluß: 1 ml/min; 40°C, Detektion: 220 nm].

### Beispiel 181

### ent-8-[(2,6-Difluorbenzyl)oxy]-2-methyl-N-[2-(morpholin-4-yl)propyl]imidazo[1,2-a]pyridin-3-carboxamid (Enantiomer B)

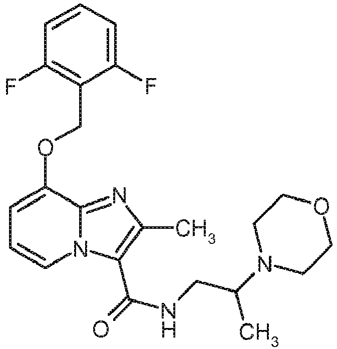

120 mg *rac*-8-[(2,6-Difluorbenzyl)oxy]-2-methyl-*N*-[2-(morpholin-4-yl)propyl]imidazo[1,2-a]pyridin-3-carboxamid (Beispiel 132) wurden an chiraler Phase in die Enantiomere getrennt [Säule: Daicel Chiralpak AD-H, 5 µm, 250 x 20 mm, Eluent: 50% iso-Hexan, 50% Iso-Propanol, 0.2% Diethylamin, Fluß: 20 ml/min; 40°C, Detektion: 210 nm].
Ausbeute: Enantiomer B: 47 mg (97% ee)
Enantiomer B: Rₜ = 11.00 min [Daicel Chiralpak AD-H, 5 µm, 250 x 4.6 mm, Eluent: Eluent: 50% iso-Hexan, 50% Iso-Propanol, 0.2% Diethylamin, Fluß: 1 ml/min; 40°C, Detektion: 220 nm].

### Beispiel 182

### ent-8-[(2,6-Difluorbenzyl)oxy]-2-methyl-N-[2-(morpholin-4-yl)-1-phenylethyl]imidazo[1,2-a]pyridin-3-carboxamid (Enantiomer A)

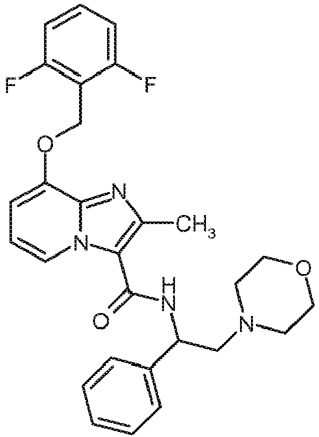

128 mg *rac*-8-[(2,6-Difluorbenzyl)oxy]-2-methyl-*N-*[2-(morpholin-4-yl)-1-phenylethyl]imidazo-[1,2-a]pyridin-3-carboxamid (Beispiel 134) wurden an chiraler Phase in die Enantiomere getrennt [Säule: Daicel Chiralpak AD-H, 5 µm, 250 x 20 mm, Eluent: 50% iso-Hexan, 50% Ethanol, 0.2% Diethylamin, Fluß: 20 ml/min; 25°C, Detektion: 230 nm].
Ausbeute: Enantiomer A: 27 mg (100% ee)
Enantiomer A: Rₜ = 8.96 min [Daicel Chiralpak AD-H, 5 µm, 250 x 4.6 mm, Eluent: 50% iso-Hexan, 50% Ethanol, 0.2% Diethylamin, Fluß: 1 ml/min; 40°C, Detektion: 220 nm].

### Beispiel 183

### ent-8-[(2,6-Difluorbenzyl)oxy]-2-methyl-N-[2-(morpholin-4-yl)-1-phenylethyl]imidazo[1,2-a]pyridin-3-carboxamid (Enantiomer B)

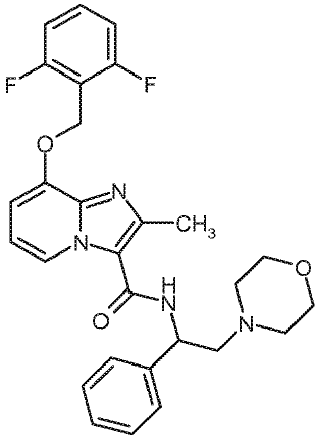

128 mg *rac*-8-[(2,6-Difluorbenzyl)oxy]-2-methyl-*N*-[2-(morpholin-4-yl)-1-phenylethyl]imidazo-[1,2-a]pyridin-3-carboxamid (Beispiel 134) wurden an chiraler Phase in die Enantiomere getrennt [Säule: Daicel Chiralpak AD-H, 5 µm, 250 x 20 mm, Eluent: 50% iso-Hexan, 50% Ethanol, 0.2% Diethylamin, Fluß: 20 ml/min; 25°C, Detektion: 230 nm].
Ausbeute: Enantiomer B: 23 mg (100% ee)
Enantiomer B: Rₜ = 13.66 min [Daicel Chiralpak AD-H, 5 µm, 250 x 4.6 mm, Eluent: 50% iso-Hexan, 50% Ethanol, 0.2% Diethylamin, Fluß: 1 ml/min; 40°C, Detektion: 220 nm].

### Beispiel 184

### ent-N-(3-Azabicyclo[4.1.0]hept-1-yl)-8-[(2,6-difluorbenzyl)oxy]-2-methylimidazo[1,2-a]pyridin-3-carboxamid (Enantiomer A)

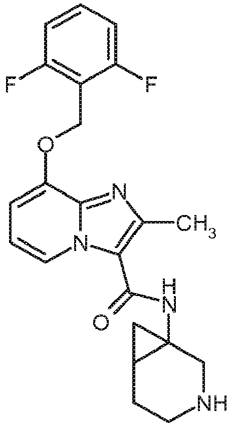

111 mg Beispiel 125 wurden an chiraler Phase in die Enantiomere getrennt [Säule: Daicel Chiralpak AD-H, 5 µm, 250 x 20 mm, Eluent: 50% iso-Hexan, 50% Iso-Propanol, 0.2% Diethylamin, Fluß: 20 ml/min; 40°C, Detektion: 210 nm].
Ausbeute: Enantiomer A: 32 mg (99% ee)
Enantiomer A: Rₜ = 8.04 min [Daicel Chiralpak AD-H, 5 µm, 250 x 4.6 mm, Eluent: 50% iso-Hexan, 50% Ethanol, 0.2% Diethylamin, Fluß: 1 ml/min; 40°C, Detektion: 220 nm].

### Beispiel 185

### ent-N-(3-Azabicyclo[4.1.0]hept-1-yl)-8-[(2,6-difluorbenzyl)oxy]-2-methylimidazo[1,2-a]pyridin-3-carboxamid (Enantiomer B)

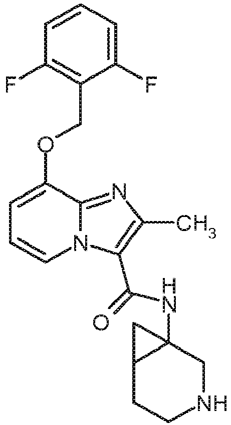

111 mg von Beispiel 125 wurden an chiraler Phase in die Enantiomere getrennt [Säule: Daicel Chiralpak AD-H, 5 µm, 250 x 20 mm, Eluent: 50% iso-Hexan, 50% Iso-Propanol, 0.2% Diethylamin, Fluß: 20 ml/min; 40°C, Detektion: 210 nm].
Ausbeute: Enantiomer B: 31 mg (100% ee)
Enantiomer B: Rₜ = 11.10 min [Daicel Chiralpak AD-H, 5 µm, 250 x 4.6 mm, Eluent: 50% iso-Hexan, 50% Ethanol, 0.2% Diethylamin, Fluß: 1 ml/min; 40°C, Detektion: 220 nm].

### Beispiel 186

### ent-8-[(2,6-Difluorbenzyl)oxy]-2,6-dimethyl-N-[2-(morpholin-4-yl)propyl]imidazo[1,2-a]pyridin-3-carboxamid (Enantiomer A)

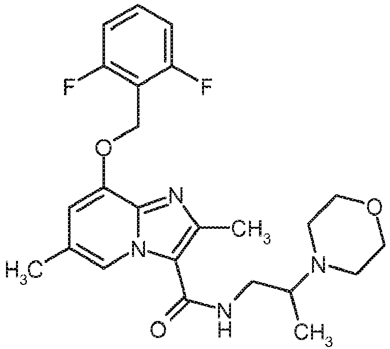

63 mg Beispiel 138 wurden an chiraler Phase in die Enantiomere getrennt [Säule: Daicel Chiralpak AD-H, 5 µm, 250 x 20 mm, Eluent: 50% iso-Hexan, 50% Iso-Propanol, 0.2% Diethylamin, Fluß: 20 ml/min; 40°C, Detektion: 210 nm].
Ausbeute: Enantiomer A: 21 mg (96% ee)
Enantiomer A: Rₜ = 8.73 min [Daicel Chiralpak AD-H, 5 µm, 250 x 4.6 mm, Eluent: 50% iso-Hexan, 50% Iso-Propanol + 0.2% Diethylamin, Fluß: 1 ml/min; 40°C, Detektion: 220 nm].

### Beispiel 187

### ent-8-[(2,6-Difluorbenzyl)oxy]-2,6-dimethyl-N-[2-(morpholin-4-yl)propyl]imidazo[1,2-a]pyridin-3-carboxamid (Enantiomer B)

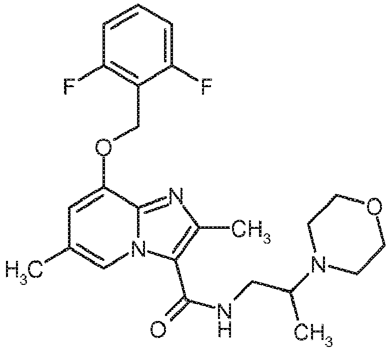

63 mg Beispiel 138 wurden an chiraler Phase in die Enantiomere getrennt [Säule: Daicel Chiralpak AD-H, 5 µm, 250 x 20 mm, Eluent: 50% iso-Hexan, 50% Iso-Propanol, 0.2% Diethylamin, Fluß: 20 ml/min; 40°C, Detektion: 210 nm].
Ausbeute: Enantiomer B: 25 mg (100% ee)
Enantiomer B: Rₜ = 9.70 min [Daicel Chiralpak AD-H, 5 µm, 250 x 4.6 mm, Eluent: 50% iso-Hexan, 50% Iso-Propanol + 0.2% Diethylamin, Fluß: 1 ml/min; 40°C, Detektion: 220 nm].

### Beispiel 188

### ent-8-[(2,6-Difluorbenzyl)oxy]-N-[2-(dimethylamino)-3-methylbutyl]-2-methylimidazo[1,2-a]pyridin-3-carboxamid (Enantiomer A)

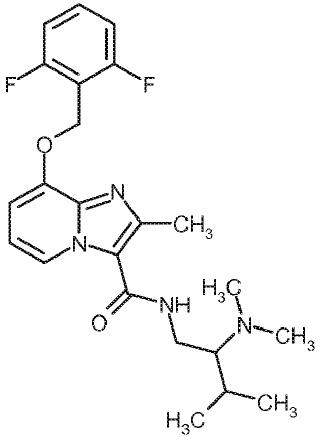

130 mg Beispiel 142 wurden an chiraler Phase in die Enantiomere getrennt [Säule: Daicel Chiralpak AD-H, 5 µm, 250 x 20 mm, Eluent: 50% iso-Hexan, 50% Ethanol + 0.2% Diethylamin, Fluß: 20 ml/min; 40°C, Detektion: 210 nm].
Ausbeute: Enantiomer A: 50 mg (99% ee)
Enantiomer A: Rₜ = 8.31 min [Säule: Daicel Chiralpak AD-H, 5 µm, 250 x 4.6 mm, Eluent: 50% iso-Hexan, 50% Ethanol + 0.2% Diethylamin, Fluß: 1 ml/min; 40°C, Detektion: 220 nm].

### Beispiel 189

### ent-8-[(2,6-Difluorbenzyl)oxy]-N-[2-(dimethylamino)-3-methylbutyl]-2-methylimidazo[1,2-a]pyridin-3-carboxamid (Enantiomer B)

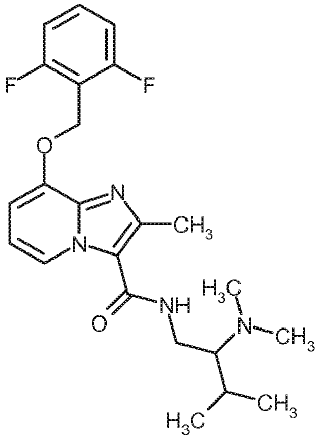

130 mg Beispiel 142 wurden an chiraler Phase in die Enantiomere getrennt [Säule: Daicel Chiralpak AD-H, 5 µm, 250 x 20 mm, Eluent: 50% iso-Hexan, 50% Ethanol + 0.2% Diethylamin, Fluß: 20 ml/min; 40°C, Detektion: 210 nm].
Ausbeute: Enantiomer B: 52 mg (96% ee)
Enantiomer B: Rₜ = 9.66 min [Säule: Daicel Chiralpak AD-H, 5 µm, 250 x 4.6 mm, Eluent: 50% iso-Hexan, 50% Ethanol + 0.2% Diethylamin, Fluß: 1 ml/min; 40°C, Detektion: 220 nm].

### Beispiel 190

### ent-6-Chlor-8-[(2,6-difluorbenzyl)oxy]-2-methyl-N-(piperidin-2-ylmethyl)imidazo[1,2-a]pyridin-3-carboxamid (Enantiomer A)

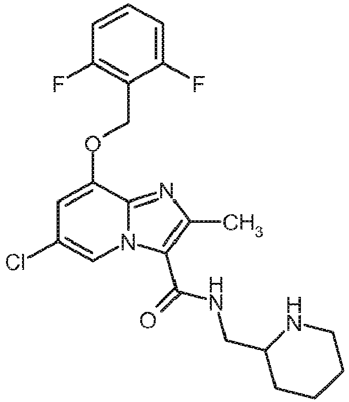

130 mg Beispiel 118 wurden an chiraler Phase in die Enantiomere getrennt [Säule: Daicel Chiralpak OZ-H, 5 µm, 250 x 20 mm, Eluent: 50% iso-Hexan, 50% Isopropanol + 0.2% Diethylamin, Fluß: 15 ml/min; 40°C, Detektion: 220 nm].
Ausbeute: Enantiomer A: 65 mg (99% ee)
Enantiomer A: Rₜ = 10.35 min [Säule: Daicel Chiralpak OZ-H, 5 µm, 250 x 4.6 mm, Eluent: 70% iso-Hexan, 30% Ethanol + 0.2% Diethylamin, Fluß: 1.0 ml/min; 40°C, Detektion: 220 nm].

### Beispiel 191

### ent-6-Chlor-8-[(2,6-difluorbenzyl)oxy]-2-methyl-N-(piperidin-2-ylmethyl)imidazo[1,2-a]pyridin-3-carboxamid (Enantiomer B)

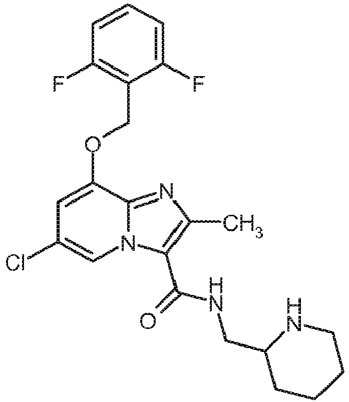

130 mg Beispiel 118 wurden an chiraler Phase in die Enantiomere getrennt [Säule: Daicel Chiralpak OZ-H, 5 µm, 250 x 20 mm, Eluent: 50% iso-Hexan, 50% Isopropanol + 0.2% Diethylamin, Fluß: 15 ml/min; 40°C, Detektion: 220 nm].
Ausbeute: Enantiomer B: 66 mg (98% ee)
Enantiomer B: Rₜ = 11.67 min [Säule: Daicel Chiralpak OZ-H, 5 µm, 250 x 4.6 mm, Eluent: 70% iso-Hexan, 30% Ethanol + 0.2% Diethylamin, Fluß: 1.0 ml/min; 40°C, Detektion: 220 nm].

### Beispiel 192

### ent-N-(2-Amino-4,4,4-trifluorbutyl)-6-chlor-8-[(2,6-difluorbenzyl)oxy]-2-methylimidazo[1,2-a]pyridin-3-carboxamid (Enantiomer A)

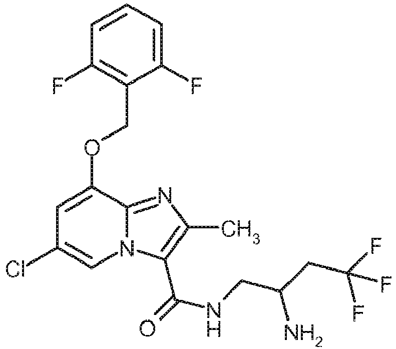

166 mg Beispiel 147 wurden an chiraler Phase in die Enantiomere getrennt [Säule: Daicel Chiralpak AD-H, 5 µm, 250 x 20 mm, Eluent: 50% iso-Hexan, 50% Isopropanol + 0.2% Diethylamin, Fluß: 12 ml/min; 25°C, Detektion: 230 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Es wurde eingefroren und lyophilisiert. Das Produkt wurde mittels Dickschichtchromatographie nachgereinigt (Laufmittel: Dichlormethan/Methanol 10:1).
Ausbeute Enantiomer A: 23 mg (99% ee)
Enantiomer A: Rₜ = 5.63 min [Säule: Daicel Chiralpak AD-H, 5 µm, 250 x 4.6 mm, Eluent: 50% iso-Hexan, 50% Isopropanol + 0.2% Diethylamin, Fluß: 1.0 ml/min; 40°C, Detektion: 220 nm].

### Beispiel 193

### ent-N-(2-Amino-4,4,4-trifluorbutyl)-6-chlor-8-[(2,6-difluorbenzyl)oxy]-2-methylimidazo[1,2-a]pyridin-3-carboxamid (Enantiomer B)

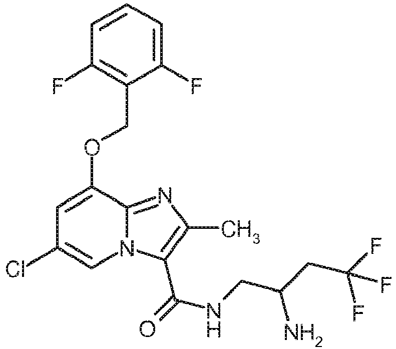

166 mg Beispiel 147 wurden an chiraler Phase in die Enantiomere getrennt [Säule: Daicel Chiralpak AD-H, 5 µm, 250 x 20 mm, Eluent: 50% iso-Hexan, 50% Isopropanol + 0.2% Diethylamin, Fluß: 12 ml/min; 25°C, Detektion: 230 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Es wurde eingefroren und lyophilisiert. Das Produkt wurde mittels Dickschichtchromatographie nachgereinigt (Laufmittel: Dichlormethan/Methanol 10:1).
Ausbeute: Enantiomer B: 22 mg (99% ee)
Enantiomer B: Rₜ = 6.13 min [Säule: Daicel Chiralpak AD-H, 5 µm, 250 x 4.6 mm, Eluent: 50% iso-Hexan, 50% Isopropanol + 0.2% Diethylamin, Fluß: 1.0 ml/min; 40°C, Detektion: 220 nm].

### Beispiel 194

### ent-N-(2-Amino-2-methylbutyl)-6-chlor-8-[(2,6-difluorbenzyl)oxy]-2-methylimidazo[1,2-a]pyridin-3-carboxamid (Enantiomer A)

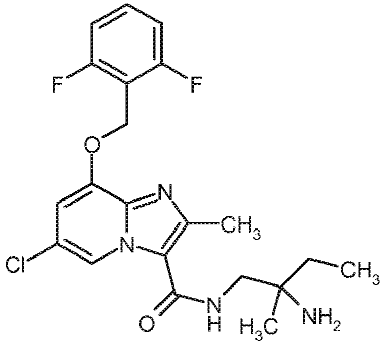

190 mg Beispiel 157 wurden an chiraler Phase in die Enantiomere getrennt [Säule: Daicel Chiralpak AZ-H, 5 µm, 250 x 20 mm, Eluent: 50% iso-Hexan, 50% Ethanol + 0.2% Diethylamin, Fluß: 15 ml/min; 35°C, Detektion: 220 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Es wurde eingefroren und lyophilisiert.
Ausbeute: Enantiomer A: 54 mg (98% ee)
Enantiomer A: Rₜ = 8.39 min [Säule: Daicel Chiralpak AZ-H, 5 µm, 250 x 4.6 mm, Eluent: 50% iso-Hexan, 50% Ethanol + 0.2% Diethylamin, Fluß: 1.0 ml/min; 35°C, Detektion: 250 nm].
Spezifischer Drehwert [a] (436 nm, 19.8°C) = -3.2° (c = 0.0044 g/ml, Acetonitril)

### Beispiel 195

### ent-N-(2-Amino-2-methylbutyl)-6-chlor-8-[(2,6-difluorbenzyl)oxy]-2-methylimidazo[1,2-a]pyridin-3-carboxamid (Enantiomer B)

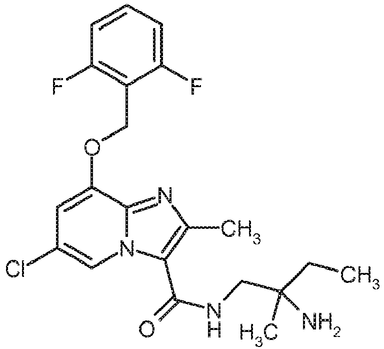

190 mg von Beispiel 157 wurden an chiraler Phase in die Enantiomere getrennt [Säule: Daicel Chiralpak AZ-H, 5 µm, 250 x 20 mm, Eluent: 50% iso-Hexan, 50% Ethanol + 0.2% Diethylamin, Fluß: 15 ml/min; 35°C, Detektion: 220 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Es wurde eingefroren und lyophilisiert.
Ausbeute: Enantiomer B: 71 mg (90% ee)
Enantiomer B: Rₜ = 9.04 min [Säule: Daicel Chiralpak AZ-H, 5 µm, 250 x 4.6 mm, Eluent: 50% iso-Hexan, 50% Ethanol + 0.2% Diethylamin, Fluß: 1.0 ml/min; 35°C, Detektion: 250 nm].

### Beispiel 196

### ent-N-(2-Amino-3-methoxy-2-methylpropyl)-6-chlor-8-[(2,6-difluorbenzyl)oxy]-2-methylimidazo[1,2,a]pyridin-3-carboxamid (Enantiomer A)

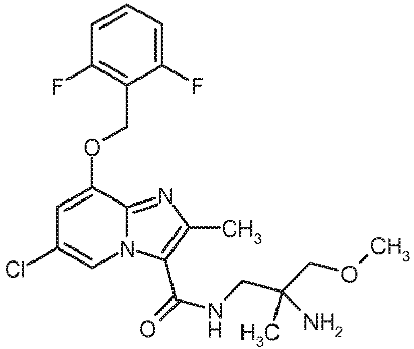

218 mg Beispiel 159 wurden an chiraler Phase in die Enantiomere getrennt [Säule: Daicel Chiralpak OZ-H, 5 µm, 250 x 20 mm, Eluent: 50% iso-Hexan, 50% Ethanol + 0.2% Diethylamin, Fluß: 15 ml/min; 40°C, Detektion: 220 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Es wurde eingefroren und lyophilisiert.
Ausbeute: Enantiomer A: 136 mg (99% ee)
Enantiomer A: Rₜ = 7.68 min [Säule: Daicel Chiralpak OZ-H, 5 µm, 250 x 4.6 mm, Eluent: 50% iso-Hexan, 50% Ethanol + 0.2% Diethylamin, Fluß: 1.0 ml/min; 40°C, Detektion: 270 nm].

### Beispiel 197

### ent-N-(2-Amino-3-methoxy-2-methylpropyl)-6-chlor-8-[(2,6-difluorbenzyl)oxy]-2-methylimidazo[1,2,a]pyridin-3-carboxamid (Enantiomer B)

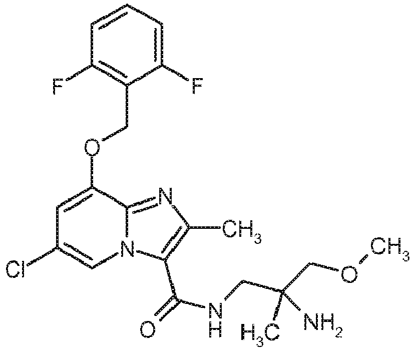

218 mg Beispiel 159 wurden an chiraler Phase in die Enantiomere getrennt [Säule: Daicel Chiralpak OZ-H, 5 µm, 250 x 20 mm, Eluent: 50% iso-Hexan, 50% Ethanol + 0.2% Diethylamin, Fluß: 15 ml/min; 40°C, Detektion: 220 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Es wurde eingefroren und lyophilisiert.
Ausbeute. Enantiomer B: 60 mg (98% ee)
Enantiomer B: Rₜ = 10.17 min [Säule: Daicel Chiralpak OZ-H, 5 µm, 250 x 4.6 mm, Eluent: 50% iso-Hexan, 50% Ethanol + 0.2% Diethylamin, Fluß: 1.0 ml/min; 40°C, Detektion: 270 nm].

### Beispiel 198

### ent-N-(2-Amino-3-methoxy-2-methylpropyl)-8-[(2,6-difluorbenzyl)oxy]-2,6-dimethylimidazo[1,2-a]pyridin-3-carboxamid (Enantiomer A)

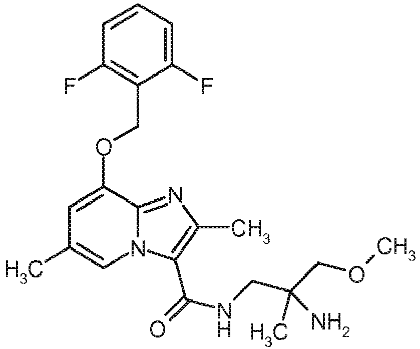

200 mg Beispiel 166 wurden an chiraler Phase in die Enantiomere getrennt [Säule: Daicel Chiralpak OZ-H, 5 µm, 250 x 20 mm, Eluent: 25% iso-Hexan, 75% Ethanol + 0.2% Diethylamin, Fluß: 12 ml/min; 45°C, Detektion: 220 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Es wurde eingefroren und lyophilisiert.
Ausbeute: Enantiomer A: 112 mg (99% ee)
Enantiomer A: Rₜ = 7.31 min [Säule: Daicel Chiralpak OZ-H, 5 µm, 250 x 4.6 mm, Eluent: 25% iso-Hexan, 75% Ethanol + 0.2% Diethylamin, Fluß: 1.0 ml/min; 45°C, Detektion: 235 nm].

### Beispiel 199

### ent-N-(2-Amino-3-methoxy-2-methylpropyl)-8-[(2,6-difluorbenzyl)xy]-2,6-dimethylimidazo[1,2-a]pyridin-3-carboxamid (Enantiomer B)

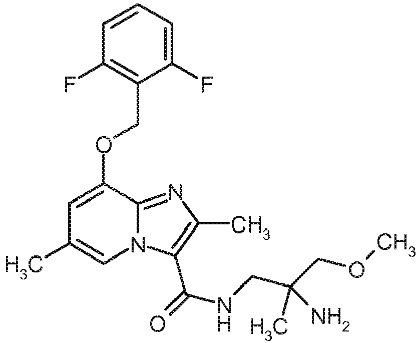

200 mg Beispiel 166 wurden an chiraler Phase in die Enantiomere getrennt [Säule: Daicel Chiralpak OZ-H, 5 µm, 250 x 20 mm, Eluent: 25% iso-Hexan, 75% Ethanol + 0.2% Diethylamin, Fluß: 12 ml/min; 45°C, Detektion: 220 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Es wurde eingefroren und lyophilisiert.
Ausbeute: Enantiomer B: 50 mg (98% ee)
Enantiomer B: Rₜ = 10.00 min [Säule: Daicel Chiralpak OZ-H, 5 µm, 250 x 4.6 mm, Eluent: 25% iso-Hexan, 75% Ethanol + 0.2% Diethylamin, Fluß: 1.0 ml/min; 45°C, Detektion: 235 nm].

### Beispiel 200

### ent-N-[(2S)-Amino-2-methylbutyl]-8-[(2,6-difluorbenzyl)oxy]-2,6-dimethylimidazo[1,2-a]pyridin-3-carboxamid (Enantiomer A)

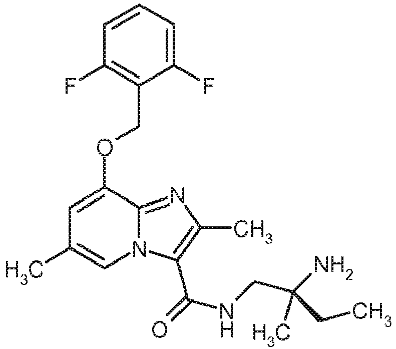

3.55 g (6.45 mmol) *ent*-Benzyl-{1-[({8-[(2,6-difluorbenzyl)oxy]-2,6-dimethylimidazo[1,2-a]pyridin-3-yl}carbonyl)amino]-2-methylbutan-2-yl}carbamat (Enantiomer A) Beispiel 276A wurden unter leichtem Erwärmen in 70 ml Ethanol gelöst und unter Argon mit 226 mg Palladium(II)hydroxid auf Aktivkohle (20%ig) versetzt. Das Reaktionsgemisch wurde 2 h unter Normaldruck bei RT hydriert. Das Reaktionsgemisch wurde über Celite abfiltriert, gut mit Ethanol gewaschen und das Filtrat wurde eingeengt. Das Rohprodukt wurde mittels Kieselgelchromatographie gereinigt (Laufmittel: Dichlormethan/2 N Ammoniak in Methanol = 10/0.5). Es wurden 2.27 g der Zielverbindung (85% d. Th.; Reinheit 99%) erhalten.
LC-MS (Methode 2): Rₜ = 0.62 min
MS (ESIpos): m/z = 417 (M+H)⁺
¹H-NMR (400 MHz, DMSO-d₆): δ = 0.87 (t, 3H), 0.97 (s, 3H), 1.31 - 1.39 (m, 2H), 1.43 (br. s, 2H), 2.31 (s, 3H), 2.53 (s, 3H, überlagert mit DMSO-Signal), 3.14 - 3.27 (m, 2H), 5.29 (s, 2H), 6.91 (s, 1H), 7.19 - 7.27 (m, 2H), 7.54 - 7.64 (m, 2H), 8.49 (s, 1H).
Spezifischer Drehwert [a] (365 nm, 20.0°C) = -6.6° (c = 0.0044 g/ml, Acetonitril)

Eine Einkristall-Röntgenstrukturanalyse bestätigte für dieses Enantiomer die S-Konfiguration.

### Beispiel 201

### ent-N-(2-Amino-2-methylbutyl)-8-[(2,6-difluorbenzyl)oxy]-2,6-dimethylimidazo[1,2-a]pyridin-3-carboxamid (Enantiomer B)

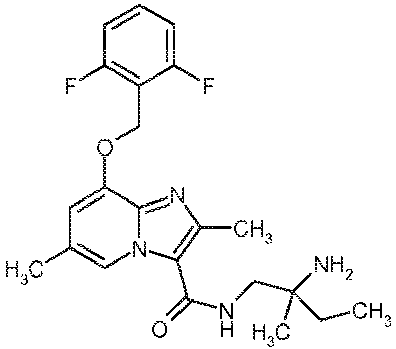

2.10 g (3.66 mmol) *ent*-Benzyl-{1-[({8-[(2,6-difluorbenzyl)oxy]-2,6-dimethylimidazo[1,2-a]pyridin-3-yl}carbonyl)amino]-2-methylbutan-2-yl}carbamat (Enantiomer B) Beispiel 277A wurden unter leichtem Erwärmen in 40 ml Ethanol gelöst und unter Argon mit 257 mg Palladium(II)hydroxid auf Aktivkohle (20%ig) versetzt. Das Reaktionsgemisch wurde 2 h unter Normaldruck bei RT hydriert. Das Reaktionsgemisch wurde über Celite abfiltriert, gut mit Ethanol gewaschen und das Filtrat wurde eingeengt. Das Rohprodukt wurde mittels Kieselgelchromatographie gereinigt (Laufmittel: Dichlormethan/2 N Ammoniak in Methanol = 10/0.5) gereinigt. Es wurden 1.26 g der Zielverbindung (82% d. Th.; Reinheit 99%) erhalten.
LC-MS (Methode 2): Rₜ = 0.66 min
MS (ESIpos): m/z = 417 (M+H)⁺
¹H-NMR (400 MHz, DMSO-d₆): δ = 0.87 (t, 3H), 0.98 (s, 3H), 1.31 -1.39 (m, 2H), 1.49 (br. s, 2H), 2.31 (s, 3H), 2.53 (s, 3H, überlagert mit DMSO-Signal), 3.14 - 3.27 (m, 2H), 5.29 (s, 2H), 6.91 (s, 1H), 7.19 - 7.27 (m, 2H), 7.54 - 7.64 (m, 2H), 8.49 (s, 1H).
Spezifischer Drehwert [a] (365 nm, 20.1°C) = +4.4° (c = 0.0044 g/ml, Acetonitril)

### Beispiel 202

### rac-N-(2-Amino-2,4-dimethylpentyl)-8-[(2,6-difluorbenzyl)oxy]-2-methylimidazo[1,2-a]pyridin-3-carboxamid

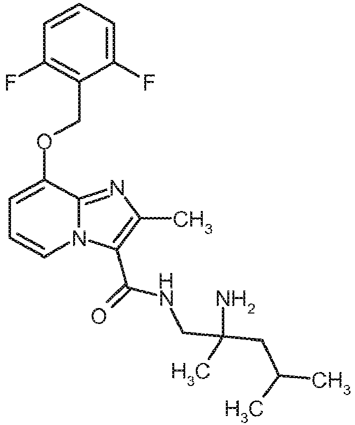

150 mg (0.47 mmol) 8-[(2,6-Difluorbenzyl)oxy]-2-methylimidazo[1,2-a]pyridin-3-carbonsäure Beispiel 3A wurde in DMF (1.7 ml) vorgelegt, mit 159 mg (0.50 mmol) TBTU und 0.16 ml (1.41 mmol) 4-Methylmorpholin versetzt. Anschließend wurden 68 mg (0.52 mmol) *rac*-2,4-Dimethylpentan-1,2-diamin zugegeben und die Reaktionsmischung über Nacht bei RT gerührt. Es wurden nochmals 6.1 mg (0.04 mmol) *rac*-2*,*4-Dimethylpentan-1,2-diamin zugegeben und die Reaktionsmischung 20 min bei RT gerührt. Das Gemisch wurde mit Wasser/TFA verdünnt und mittels präp. RP-HPLC (Acetonitril/Wasser-Gradient unter Zusatz von 0.1% TFA) gereinigt. Die Produktfraktion wurde eingeengt. Der Rückstand wurde in Dichlormethan und wenigen Tropfen Methanol aufgenommen, zweimal mit gesättigter, wässriger Natriumhydrogencabonat-Lösung gewaschen und die wässrige Phase wurde dreimal mit Dichlormethan extrahiert. Die organische Phase wurde über Natriumsulfat getrocknet, abfiltriert und eingeengt. Man erhielt 126 mg (61% d. Th., Reinheit 97%) der Zielverbindung.
LC-MS (Methode 2): Rₜ = 0.70 min
MS (ESIpos): m/z = 431 (M+H)⁺
¹H-NMR (400 MHz, DMSO-d₆): δ = 0.89 (d, 3H), 0.95 (d, 3H), 1.02 (s, 3H), 1.20 - 1.33 (m, 2H), 1.42 (br. s, 2H), 1.74 - 1.85 (m, 1H), 2.56 (s, 3H), 3.14 - 3.27 (m, 2H), 5.30 (s, 2H), 6.94 (t, 1H), 7.01 (d, 1H), 7.19 - 7.29 (m, 2H), 7.54 - 7.63 (m, 1H), 7.64 - 7.71 (m, 1H), 8.64 (d, 1H).

### Beispiel 203

### ent-N-(2-Amino-2,4-dimethylpentyl)-8-[(2,6-diftuorbenzyl)oxy]-2-methylimidazo[1,2-a]pyridin-3-carboxamid (EnantiomerA)

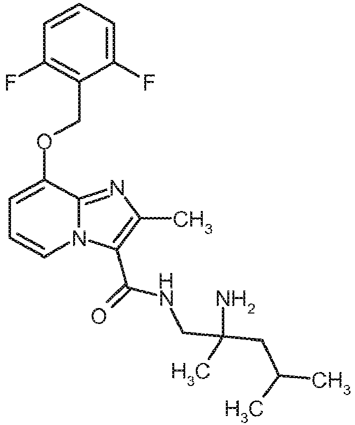

122 mg Beispiel 202 wurden durch präparative Trennung an chiraler Phase in die Enantiomere getrennt [Säule: Daicel Chiralpak AD-H, 5 µm, 250 x 20 mm, Eluent: 50% iso-Hexan, 50% Ethanol + 0.2% Diethylamin, Fluß: 20 ml/min; 40°C, Detektion: 210 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Die erhaltene Lösung wurde eingefroren und lyophilisiert.
Enantiomer A: Ausbeute: 28 mg (100% ee)
Rₜ = 8.71 min [Daicel Chiralpak AD-H, 5µm, 250 x 4.6 mm; Eluent: 50% iso-Hexan, 50% Ethanol + 0.2% Diethylamin; Fluss 1.0 ml/min; 40°C; Detektion: 220 nm].

### Beispiel 204

### ent-N-(2-Amino-2,4-dimethylpentyl)-8-[(2,6-difluorbenzyl)oxy]-2-methylimidazo[1,2-a]pyridin-3-carboxamid (EnantiomerB)

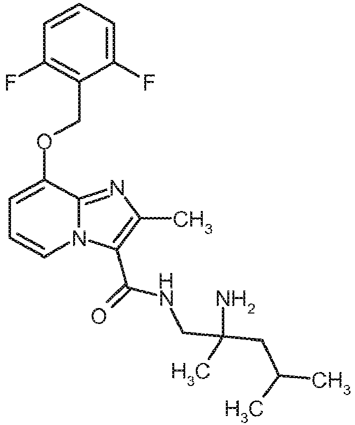

122 mg Beispiel 202 wurden durch präparative Trennung an chiraler Phase in die Enantiomere getrennt [Säule: Daicel Chiralpak AD-H, 5 µm, 250 x 20 mm, Eluent: 50% iso-Hexan, 50% Ethanol + 0.2% Diethylamin, Fluß: 20 ml/min; 40°C, Detektion: 210 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Die erhaltene Lösung wurde eingefroren und lyophilisiert.
Enantiomer B: Ausbeute: 68 mg (92% ee)
Rₜ = 9.79 min [Daicel Chiralpak AD-H, 5µm, 250 x 4.6 mm; Eluent: 50% iso-Hexan, 50% Ethanol + 0.2% Diethylamin; Fluss 1.0 ml/min; 40°C; Detektion: 220 nm].

### Beispiel 205

### rac-N-(2-Amino-3-isopropoxypropyl)-8-[(2,6-difluorbenzyl)oxy]-2-methylimidazo[1,2-a]pyridin-3-carboxamid

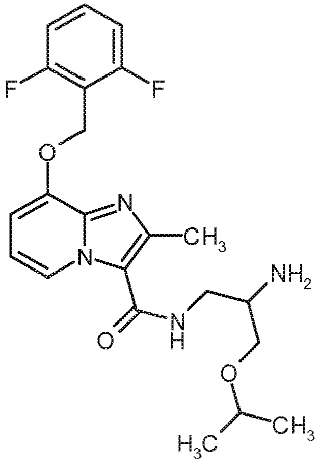

95 mg (0.30 mmol) 8-[(2,6-Difluorbenzyl)oxy]-2-methylimidazo[1,2-a]pyridin-3-carbonsäure Beispiel 3A in DMF (1.9 ml) wurden mit 125 mg (0.33 mmol) HATU und 0.16 ml (0.90 mmol) *N,N-*Diisopropylethylamin versetzt und 20 min bei RT gerührt. Anschließend wurden 145 mg (0.54 mmol, 76% rein) *rac*-3-Isopropoxypropan-1,2-diamindihydrochlorid Beispiel 224A in DMF (0.5 ml), die vorher mit 0.31 ml (1.8 mmol) *N,N-*Diisopropylethylamin versetzt und 10 min bei RT gerührt worden waren, bei -20°C zur Reaktionsmischung langsam zugetropft. Es wurde über Nacht bei RT gerührt. Dann wurde mit Wasser/TFA verdünnt und mittels präp. RP-HPLC (Acetonitril/Wasser-Gradient unter Zusatz von 0.1% TFA) gereinigt. Die Produktfraktion wurde eingeengt und anschließend mittels Dickichtchromatographie (Dichlormethan/Methanol: 10:1) nachgereinigt. Man erhielt 15 mg (12% d. Th.) der Zielverbindung.
LC-MS (Methode 2): Rₜ = 0.68 min.
MS (ESIpos): m/z = 433 (M+H)⁺
¹H-NMR (400 MHz, DMSO-d₆): δ = 1.10 (d, 6H), 1.24 (s, 2H), 2.57 (s, 3H; überlagert mit DMSO-Signal), 3.00 - 3.08 (m, 1H), 3.28 - 3.48 (m, 4H), 3.51 - 3.59 (m, 1H), 5.30 (s, 2H), 6.92 (t, 1H), 7.00 (d, 1H), 7.24 (t, 2H), 7.53 - 7.63 (m, 1H), 7.70 (t, 1H), 8.64 (d, 1H).

### Beispiel 206

### rac-N-(2-Amino-3-isopropoxypropyl)-8-[(2,6-difluorbenzyl)oxy]-2,6-dimethylimidazo[1,2-a]pyridin-3-carboxamid

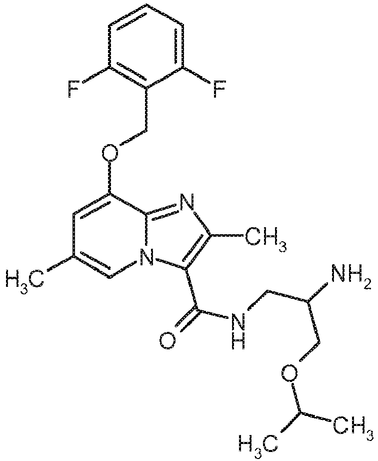

Darstellung und Reinigung der Titelverbindung erfolgte analog zu Beispiel 205. Ausgehend von 11.2 mg (0.03 mmol) 8-[(2,6-Difluorbenzyl)oxy]-2,6-dimethylimidazo[1,2-a]pyridin-3-carbonsäure Beispiel 21A und 15.0 mg (0.05 mmol, 76% rein) *rac*-3-Isopropoxypropan-1,2-diamindihydrochlorid Beispiel 224A erhielt man 1.6 mg (9.5% d. Th.) der Zielverbindung.
LC-MS (Methode 2): Rₜ = 0.65 min
MS (ESIpos): m/z = 447 (M+H)⁺
¹H-NMR (400 MHz, DMSO-d₆): δ =1.14 (d, 6H), 1.25 (s, 2H), 2.32 (s, 3H), 2.54 (s, 3H; überlagert mit DMSO-Signal), 3.35 - 3.42 (m, 1H), 3.44 - 3.66 (m, 5H), 5.31 (s, 2H), 6.94 (s, 1H), 7.24 (t, 2H), 7.56 - 7.66 (m, 1H), 7.80 (t, 1H), 8.53 (s, 1H).

### Beispiel 207

### rac-N-(2-Amino-3,3,3-trifluorpropyl)-8-[(2,6-difluorbenzyl)oxy]-2-methylimidazo[1,2-a]pyridin-3-carboxamid

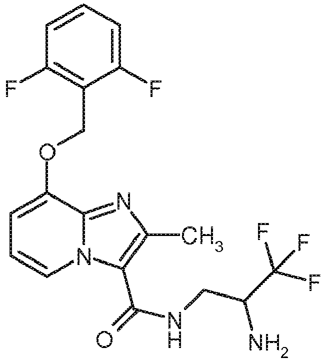

Darstellung und Reinigung der Titelverbindung erfolgte analog zu Beispiel 141. Ausgehend von 400 mg (1.26 mmol) 8-[(2,6-Difluorbenzyl)oxy]-2-methylimidazo[1,2-a]pyridin-3-carbonsäure Beispiel 3A und 278 mg (1.38 mmol) *rac*-1-Trifluormethyl)-ethylene-1,2-diamin-Dihydrochlorid erhielt man 340 mg (63% d. Th.) der Zielverbindung.
LC-MS (Methode 7): Rₜ = 0.70 min
MS (ESIpos): m/z = 429 (M+H)⁺.
¹H-NMR (400 MHz, DMSO-d₆): δ = 2.55 (s, 3H; überlagert mit DMSO-Signal), 3.49 - 3.62 (m, 1H), 3.67 - 3.77 (m, 1H), 3.89 - 4.10 (m, 1H), 5.31 (s, 2H), 6.99 (t, 1H), 7.07 (d, 1H), 7.19 - 7.29 (m, 2H), 7.54 - 7.65 (m, 1H), 7.96 (t, 1H), 8.71 (d, 1H).

### Beispiel 208

### ent-N-(2-Amino-3,3,3-trifluorpropyl)-8-[(2,6-difluorbenzyl)oxy]-2-methylimidazo[1,2-a]pyridin-3-carboxamid (Enantiomer A)

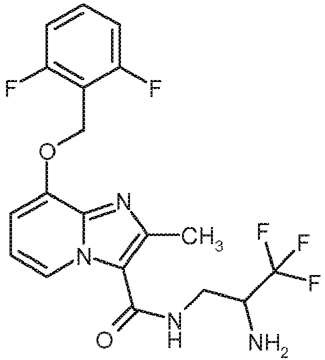

464 mg von Beispiel 207 wurde durch präparative Trennung an chiraler Phase in die Enantiomere getrennt [Säule: Daicel Chiralpak AD-H, 5 µm, 250 x 20 mm, Eluent: 50% iso-Hexan, 50% Isopropanol + 0.2% Diethylamin, Fluß: 20 ml/min; 40°C, Detektion: 210 nm].
Enantiomer A: Ausbeute: 120 mg (100% ee)
Rₜ = 8.87 min [Daicel Chiralpak AD-H, 5µm, 250 x 4.6 mm; Eluent: 50% iso-Hexan, 50% Isopropanol + 0.2% Diethylamin; Fluss 1.0 ml/min; 40°C; Detektion: 220 nm].

### Beispiel 209

### ent-N-(2-Amino-3,3,3-trifluorpropyl)-8-[(2,6-difluorbenzyl]oxy]-2-methylimidazo[1,2-a]pyridin-3-carboxamid (Enantiomer B)

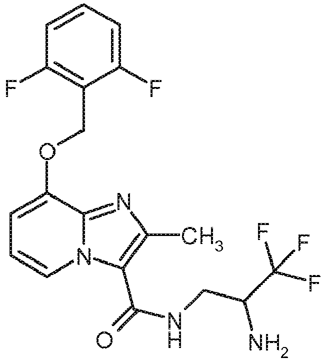

464 mg Beispiel 207 wurden durch präparative Trennung an chiraler Phase in die Enantiomere getrennt [Säule: Daicel Chiralpak AD-H, 5 µm, 250 x 20 mm, Eluent: 50% iso-Hexan, 50% Isopropanol + 0.2% Diethylamin, Fluß: 20 ml/min; 40°C, Detektion: 210 nm].
Enantiomer B: Ausbeute: 122 mg (92.6% ee)
Rₜ = 10.05 min [Daicel Chiralpak AD-H, 5µm, 250 x 4.6 mm; Eluent: 50% iso-Hexan, 50% Isopropanol + 0.2% Diethylamin; Fluss 1.0 ml/min; 40°C; Detektion: 220 nm].

### Beispiel 210

### ent-N-(1-amino-3-methoxypropan-2-yl)-8-[(2,6-difluorbenzyl)oxy]-2,6-dimethylimidazo[1,2-a]pyridin-3-carboxamid Trifluoracetat (Enantiomer B)

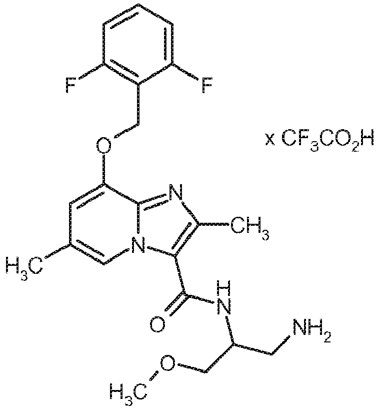

100 mg (0.18 mmol) *ent*-Benzyl-{2-[({8-[(2,6-difluorbenzyl)oxy]-2,6-dimethylimidazo[1,2-a]pyridin-3-yl}carbonyl)amino]-3-methoxypropyl}carbamat hergestellt nach der repräsentativen Arbeitsvorschrift 3 unter Verwendung der Beispiele 21A und 248A) wurde unter Argon in Ethanol (1.3 ml) vorgelegt und mit 19.2 mg (0.02 mmol, 10%ig) Palladium auf Aktivkohle und 0.37 ml (3.62 mmol) Cyclohexen versetzt. Das Reaktionsgemisch wurde über Nacht unter Rückfluss gerührt. Die auf RT abgekühlte Reaktionslösung wurde über Millipore®-Filter filtriert, mit Ethanol gewaschen und eingeengt. Der Rückstand wurde mittels präp. RP-HPLC (Acetonitril/Wasser-Gradient unter Zusatz von 0.1% TFA) gereinigt. Die Produktfraktion wurde eingeengt, danach in Dichlormethan aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, abfiltriert und eingeengt. Man erhielt 24 mg (22% d. Th., Reinheit 90%) der Titelverbindung.
LC-MS (Methode 7): Rₜ = 0.59 min
MS (ESIpos): m/z = 419 (M-TFA+H)⁺
¹H-NMR (400 MHz, DMSO-d₆): δ = 2.36 (s, 3H), 2.53 (s, 3H; überlagert mit DMSO-Signal), 2.93 - 3.04 (m, 1H), 3.06 - 3.17 (m, 1H), 3.32 (s, 3H), 3.47 - 3.54 (m, 2H), 4.37 - 4.48 (m, 1H), 5.34 (s, 2H), 7.10 - 7.19 (m, 1H), 7.21 - 7.39 (m, 2H), 7.61 (quintett, 1H), 7.83 - 8.00 (m, 2H), 8.46 (s, 1H).

### Beispiel 211

### ent-N-(2-Amino-3-methylbutyl)-6-chlor-8-[(2,6-difluorbenzyl)oxy]-2-methylimidazo[1,2-a]pyridin-3-carboxamid (Enantiomer A)

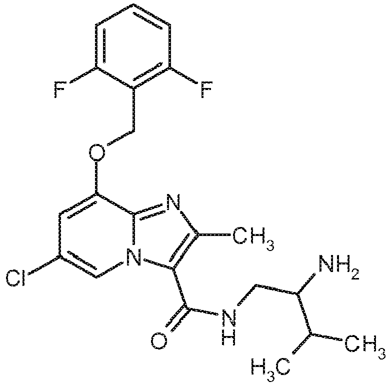

124 mg (0.23 mmol) *ent-*tert.-Butyl-{1-[({6-chlor-8-[(2,6-difluorbenzyl)oxy]-2-methylimidazo[1,2-a]pyridin-3-yl}carbonyl)amino]-3-methylbutan-2-yl}carbamat Beispiel 228A wurden in 1.16 ml (2.31 mmol) 2 N Chlorwasserstoff/Diethylether vorgelegt und 3.5 h bei RT gerührt. Anschließend wurde die Reaktionslösung eingeengt. Der Rückstand wurde mit Essigsäureethylester und gesättigter, wässriger Natriumhydrogencarbonat-Lösung versetzt. Nach Phasentrennung wurde die wässrige Phase zweimal mit Essigsäureethylester extrahiert und die vereinigten organischen Phasen wurden über Natriumsulfat getrocknet, filtriert und eingeengt. Man erhielt 89 mg (87% d. Th., Reinheit 99%) der Titelverbindung.
LC-MS (Methode 2): Rₜ = 0.73 min
MS (ESIpos): m/z = 437 (M+H)⁺
¹H-NMR (400 MHz, DMSO-d₆): δ = 0.88 (d, 3H), 0.92 (d, 3H), 1.57 - 1.68 (m, 1H), 1.86 - 2.24 (br. s, 2H), 2.53 (s, 3H; überlagert mit DMSO-Signal), 2.63 - 2.70 (m, 1H), 3.05 - 3.14 (m, 1H), 3.39 - 3.46 (m, 1H), 5.34 (s, 2H), 7.19 (d, 1H), 7.26 (t, 2H), 7.61 (quintett, 1H), 7.72 - 7.88 (br. s, 1H), 8.78 (d, 1H).

### Beispiel 212

### ent-N-(2-Amino-3-methylbutyl)-6-chlor-8-[(2,6-difluorbenzyl)oxy]-2-methylimidazo[1,2-a]pyridin-3-carboxamid (Enantiomer B)

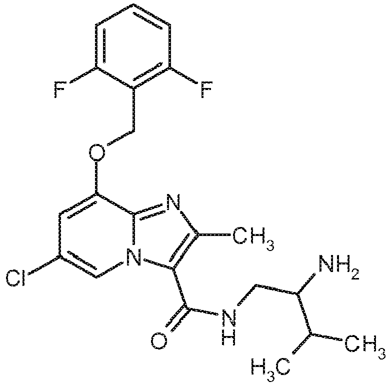

Darstellung und Reinigung (Verwendung von Dichlormethan in der Aufarbeitung) der Titelverbindung erfolgte analog zu Beispiel 211. Ausgehend von 122 mg (0.23 mmol) *ent-*tert*.-*Butyl-{1-[({6-chlor-8-[(2,6-difluorbenzyl)oxy]-2-methylimidazo[1,2-a]pyridin-3-yl}carbonyl)amino]-3-methylbutan-2-yl}carbamat (Enantiomer B) Beispiel 229A erhielt man 81 mg (81% d. Th., Reinheit 99%) der Titelverbindung.
LC-MS (Methode 2): Rₜ = 0.73 min
MS (ESIpos): m/z = 437 (M+H)⁺
¹H-NMR (400 MHz, DMSO-d₆): δ = 0.88 (d, 3H), 0.91 (d, 1H), 1.45 -1.78 (m, 3H), 2.52 (s, 3H; überlagert mit DMSO-Signal), 2.61 - 2.69 (m, 1H), 3.04 - 3.13 (m, 1H), 3.38 - 3.45 (m, 1H), 5.34 (s, 2H), 7.19 (d, 1H), 7.26 (t, 2H), 7.60 (quintett, 1H), 7.71 - 7.88 (br. s, 1H), 8.78 (d, 1H).

### Beispiel 213

### rac-N-(2-Amino-3-methoxy-2-methylpropyl)-8-[(2,6-difluorbenzyl)oxy]-2-methylimidazo[1,2-a]pyridin-3-carboxamid

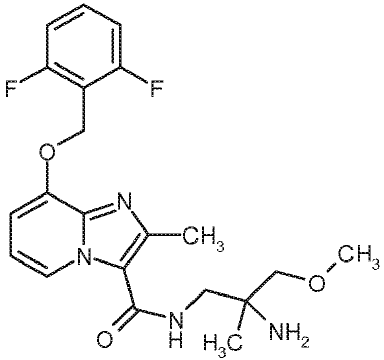

180 mg (0.57 mmol) 8-[(2,6-Difluorbenzyl)oxy]-2-methylimidazo[1,2-a]pyridin-3-carbonsäure Beispiel 3A, 237 mg (0.62 mmol) HATU und 0.30 ml (1.70 mmol) *N,N-*Diisopropylethylamin in 3.6 ml DMF vorgelegt wurden 20 min bei RT gerührt,. Anschließend wurde mit 74 mg (0.62 mmol) *rac*-3-Methoxy-2-methylpropan-1,2-diamin versetzt und das Reaktionsgemisch über Nacht bei RT gerührt. Die Reaktionslösung wurde mit Wasser/TFA verdünnt und mittels präparativer RP-HPLC (Acetonitril/Wasser-Gradient unter Zusatz von 0.1% TFA) gereinigt. Die Produktfraktion wurde eingeengt. Der Rückstand wurde in Dichlormethan aufgenommen und zweimal mit gesättigter, wässriger Natriumhydrogencarbonat-Lösung gewaschen. Die wässrige Phase wurde zweimal mit Dichlormethan extrahiert, die vereinigten organischen Phasen über Natriumsulfat getrocknet, filtriert und eingeengt. Man erhielt 136 mg (56% d. Th., Reinheit 98%) der Titelverbindung.
LC-MS (Methode 2): Rₜ = 0.56 min
MS (ESIpos): m/z = 419 (M+H)⁺
¹H-NMR (400 MHz, DMSO-d₆): δ = 1.00 (s, 3H), 1.53 (br. s, 2H), 2.56 (s, 3H; überlagert mit DMSO-Signal), 3.13 - 3.19 (m, 2H), 3.24 - 3.32 (m, 5H; überlagert mit Lösungsmittel-Signal), 5.30 (s, 2H), 6.94 (t, 1H), 7.02 (d, 1H), 7.19 - 7.29 (m, 2H), 7.59 (quintett, 2H), 8.69 (d, 1H).

### Beispiel 214

### ent-N-(2-Amino-3-methoxy-2-methylpropyl)-8-[(2,6-difluorbenzyl)oxy]-2-methylimidazo[1,2-a]pyridin-3-carboxamid (Enantiomer A)

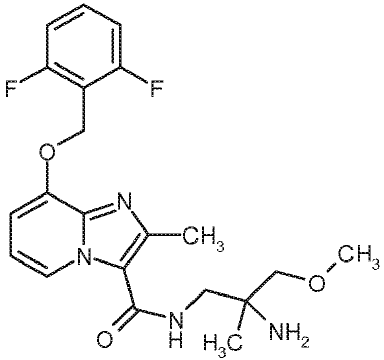

130 mg Beispiel 213 wurden durch präparative Trennung an chiraler Phase in die Enantiomere getrennt [Säule: Phenomenex Amylose II, 5 µm, 250 x 20 mm, Eluent: 50% iso-Hexan, 50% Ethanol + 0.2% Diethylamin, Fluß: 20 ml/min; 25°C, Detektion: 230 nm].
Enantiomer A: Ausbeute: 32.4 mg (100% ee)
Rₜ = 5.89 min [Daicel Chiralpak AY-H, 5µm, 250 x 4.6 mm; Eluent: 50% iso-Hexan, 50% Ethanol + 0.2% Diethylamin; Fluss 1.0 ml/min; 40°C; Detektion: 220 nm].

### Beispiel 215

### ent-N-(2-Amino-3-methoxy-2-methylpropyl)-8-[(2,6-difluorbenzyl)oxy]-2-methylimidazo[1,2-a]pyridin-3-carboxamid (Enantiomer B)

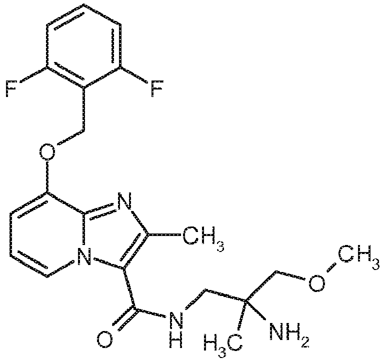

130 mg Beispiel 213 wurden durch präparative Trennung an chiraler Phase in die Enantiomere getrennt [Säule: Phenomenex Amylose II, 5 µm, 250 x 20 mm, Eluent: 50% iso-Hexan, 50% Ethanol + 0.2% Diethylamin, Fluß: 20 ml/min; 25°C, Detektion: 230 nm].
Enantiomer B: Ausbeute: 79.5 mg (100% ee)
Rₜ = 8.01 min [Daicel Chiralpak AY-H, 5µm, 250 x 4.6 mm; Eluent: 50% iso-Hexan, 50% Ethanol + 0.2% Diethylamin; Fluss 1.0 ml/min; 40°C; Detektion: 220 nm].

### Beispiel 216

### N-[(3-Aminooxetan-3-yl)methyl]-8-[(2,6-difluorbenzyl)oxy]-2,6-dimethylimidazo[1,2,a]pyridin-3-carboxamid

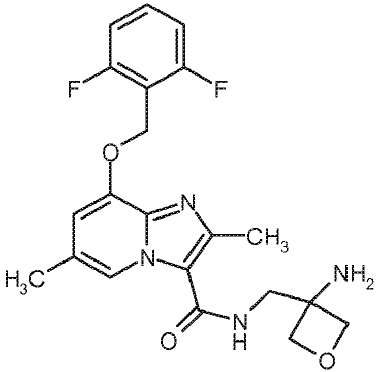

Darstellung und Reinigung der Titelverbindung erfolgte analog zu Beispiel 205. Ausgehend von 45 mg (0.14 mmol) 8-[(2,6-Difluorbenzyl)oxy]-2,6-dimethylimidazo[1,2-a]pyridin-3-carbonsäure Beispiel 21A und 95 mg (0.41 mmol, 75% rein) 3-(Aminomefhyl)oxetan-3-amindihydrochlorid Beispiel 226A. Nach einer zusätzlicher Reinigung [Säule: XBridge C18, 5 µm, 150 x 19 mm, Eluent: 32% Wasser, 60% Methanol + 8% 1%ige Ammoniak in Wasser, Fluß: 25 ml/min; 25°C, Detektion: 210 nm] erhielt man 21 mg (37% d. Th., Reinheit 100%) der Zielverbindung.
LC-MS (Methode 2): Rₜ = 0.63 min
MS (ESIpos): m/z = 417 (M+H)⁺
¹H-NMR (400 MHz, DMSO-d₆): δ = 2.30 (s, 3H), 3.52 - 3.62 (m, 2H), 4.29 (d, 2H), 4.43 (d, 2H), 5.29 (s, 2H), 6.92 (s, 1H), 7.19 - 7.28 (m, 2H), 7.54 - 7.65 (m, 1H), 7.88 - 7.98 (m, 1H), 8.44 (s, 1H), [zusätzliches Signal unter DMSO-Peak].

### Beispiel 217

### rac-N-(2-Amino-3-isopropoxypropyl)-6-chlor-8-[(2,6-difluorbenzyl)oxy]-2-methylimidazo[1,2-a]pyridin-3-carboxamid

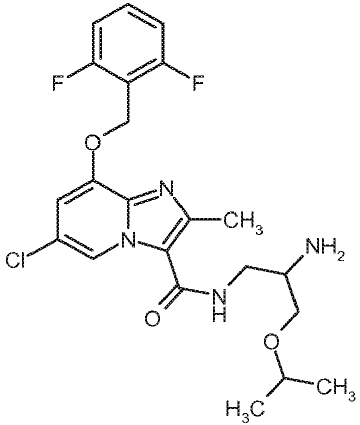

Darstellung und Reinigung der Titelverbindung erfolgte analog zu Beispiel 205. Ausgehend von 11.9 mg (0.03 mmol) 6-Chlor-8-[(2,6-difluorbenzyl)oxy]-2-methylimidazo[1,2-a]pyridin-3-carbonsäure Beispiel 16A und 15.0 mg (0.06 mmol, 76% rein) *rac*-3-Isopropoxypropan-1,2-diamindihydrochlorid Beispiel 224A erhielt man 1.9 mg (11% d. Th., Reinheit 90%) der Zielverbindung.
LC-MS (Methode 2): Rₜ = 0.76 min
MS (ESIpos): m/z = 467 (M+H)⁺
¹H-NMR (400 MHz, DMSO-d₆): δ = 1.14 (d, 6H), 1.23 - 1.28 (m, 2H), 2.55 (s, 3H; überlagert mit DMSO-Signal), 3.41 - 3.56 (m, 4H), 3.58 - 3.65 (m, 1H), 5.34 (s, 2H), 7.19 - 7.28 (m, 3H), 7.56 - 7.66 (m, 1H), 8.80 (d, 1H).

### Beispiel 218

### rac-N-(2-Amino-2,4-dimethylpentyl)-8-[(2,6-difluorbenzyl)oxy]-2,6-methylimidazo[1,2-a]pyridin-3-carboxamid

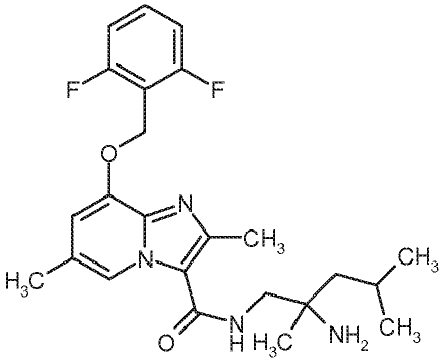

Darstellung und Reinigung der Titelverbindung erfolgte analog zu Beispiel 202. Ausgehend von 150 mg (0.45 mmol) 8-[(2,6-Difluorbenzyl)oxy]-2,6-dimethylimidazo[1,2-a]pyridin-3-carbonsäure Beispiel 21A und 65 mg (0.50 mmol) *rac*-2,4-Dimethylpentan-1,2-diamin erhielt man 155 mg (73% d. Th., Reinheit 95%) der Zielverbindung.
LC-MS (Methode 2): Rₜ = 0.66 min
MS (ESIpos): m/z = 445 (M+H)⁺
¹H-NMR (400 MHz, DMSO-d₆): δ = 0.89 - 0.96 (m, 6H), 1.02 (s, 3H), 1.21 - 1.34 (m, 2H), 1.67 (br. s, 2H), 1.74 - 1.85 (m, 1H), 2.31 (s, 3H), 2.53 (br. s, 3H; überlagert mit DMSO-Signal), 3.14 - 3.27 (m, 2H), 5.28 (s, 2H), 6.92 (s, 1H), 7.20 - 7.28 (m, 2H), 7.55 - 7.68 (m, 2H), 8.48 (s, 1H).

### Beispiel 219

### ent-N-(2-Amino-2,4-dimethylpentyl)-8-[(2,6-difluorbenzyl)oxy]-2,6-dimethylimidazo[1,2-a]pyridin-3-carboxamid (Enantiomer A)

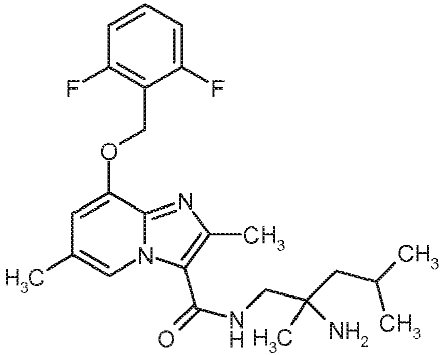

155 mg Beispiel 218 wurden durch präparative Trennung an chiraler Phase in die Enantiomere getrennt [Säule: Daicel Chiralpak AD-H, 5 µm, 250 x 20 mm, Eluent: 70% iso-Hexan, 30% Ethanol + 0.2% Diethylamin, Fluß: 20 ml/min; 40°C, Detektion: 210 nm].
Enantiomer A: Ausbeute: 37 mg (100% ee)
Rₜ = 11.40 min [Daicel Chiralpak AD-H, 5µm, 250 x 4.6 mm; Eluent: 70% iso-Hexan, 30% Ethanol + 0.2% Diethylamin; Fluss 1.0 ml/min; 40°C; Detektion: 220 nm].

### Beispiel 220

### ent-N-(2-Amino-2,4-dimethylpentyl)-8-[(2,6-difluorbenzyl)oxy]-2,6-dimethylimidazo[1,2-a]pyridin-3-carboxamid (Enantiomer B)

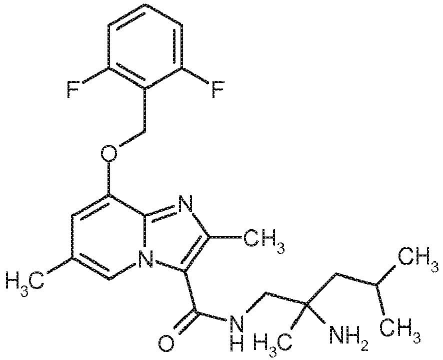

155 mg Beispiel 218 wurden durch präparative Trennung an chiraler Phase in die Enantiomere getrennt [Säule: Daicel Chiralpak AD-H, 5 µm, 250 x 20 mm, Eluent: 70% iso-Hexan, 30% Ethanol + 0.2% Diethylamin, Fluß: 20 ml/min; 40°C, Detektion: 210 nm].
Enantiomer B: Ausbeute: 79 mg (93% ee)
Rₜ = 12.56 min [Daicel Chiralpak AD-H, 5µm, 250 x 4.6 mm; Eluent: 70% iso-Hexan, 30% Ethanol + 0.2% Diethylamin; Fluss 1.0 ml/min; 40°C; Detektion: 220 nm].
Spezifischer Drehwert [a] (365 nm, 19.6°C) = +17.7° (c = 0.005 g/ml, Acetonitril)

### Beispiel 221

### N-(2-Amino-2-methylpropyl)-8-[(2,6-difluorbenzyl)oxy]-2,6-dimethylimidazo[1,2-a]pyridin-3-carboxamidhydrochlorid

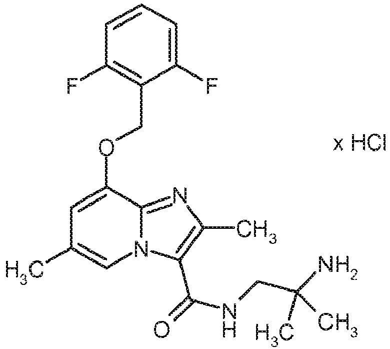

200 mg (0.50 mmol) *N*-(2-Amino-2-methylpropyl)-8-[(2,6-difluorbenzyl)oxy]-2,6-dimethylimidazo[1,2-a]pyridin-3-carboxamid Beispiel 74 wurde in 4 ml Diethylether vorgelegt, mit 0.26 ml (0.52 mmol) 2 N Salzsäure in Diethylether versetzt und die Reaktionsmischung 30 min bei RT gerührt. Anschließend wurde eingeengt und der Rückstand lyophilisiert. Man erhielt 217 mg (98.5% d. Th., Reinheit 99%) der Zielverbindung.
LC-MS (Methode 7): Rₜ = 0.57 min
MS (ESIpos): m/z = 403 (M-HCl+H)⁺
¹H-NMR (400 MHz, DMSO-d₆): δ = 1.24 (s, 6H), 2.32 (s, 3H), 2.55 (br. s, 3H; überlagert mit DMSO-Signal), 3.42 (d, 2H), 5.29 (s, 2H), 6.95 (s, 1H), 7.15 (br. s, 2H), 7.21 - 7.28 (m, 2H), 7.55 - 7.64 (m, 1H), 7.96 - 8.03 (m, 1H), 8.47 (m, 1H).

### Beispiel 222

### ent-8-[(2,6-Difluorbenzyl)oxy]-N-{1-(3,4-difluorphenyl)-2-[(2,2,2-trifluorethyl)amino]ethyl}-2-methylimidazo[1,2-a]pyridin-3-carboxamid (Enantiomer B)

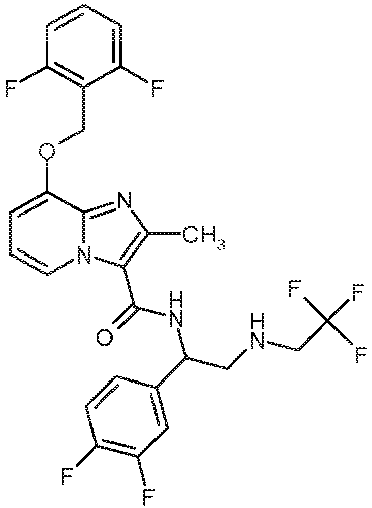

100 mg (0.21 mmol) *N*-[2-Amino-1-(3,4-difluorphenyl)ethyl]-8-[(2,6-difluorbenzyl)oxy]-2-methyl-imidazo[1,2-a]pyridin-3-carboxamid Beispiel 13 wurde in 2 ml DMF vorgelegt, mit 35 µl (0.21 mmol) 2,2,2-Trifluorethyltrichlormethansulfonat, 59 mg (0.42 mmol) Kalium-carbonat und 3.5 mg (0.02 mmol) Kaliumiodid versetzt und die Reaktionsmischung über Nacht bei RT gerührt. Die Reaktionslösung wurde mit 10 ml Wasser versetzt und zweimal mit 30 ml Essigsäureethylester extrahiert. Die vereinigten organischen Phasen wurden zweimal mit 20 ml Wasser und mit gesättigter, wässriger Natriumchlorid-Lösung gewaschen, über Magnesiumsulfat getrocknet, filtriert und eingeengt. Der Rückstand wurde mittels präparativer RP-HPLC (Acetonitril/Wasser mit 0.5%ige Ameisensäure-Gradient) gereinigt. Die Produktfraktionen wurden fast vollständigt eingeengt, mit 1 ml tert.-Butanol versetzt und über Nacht lyophilisiert. Man erhielt 18 mg (14% d. Th., Reinheit 91%) der Zielverbindung.
LC-MS (Methode 2): Rₜ = 1.05 min
MS (ESIpos): m/z = 555 (M+H)⁺
¹H-NMR (400 MHz, DMSO-d₆): δ = 2.57 (s, 3H), 2.89 - 3.08 (m, 2H), 5.09 - 5.18 (m, 1H), 5.30 (s, 2H), 6.91 (t, 1H), 7.02 (d, 1H), 7.18 - 7.33 (m, 3H), 7.36 - 7.46 (m, 1H), 7.47 - 7.56 (m, 1H), 7.57 - 7.64 (m, 1H), 8.17 (d, 1H), 8.53 (d, 1H), [weitere Signale unter Lösungsmittel-Peaks verborgen].

### Beispiel 223

### ent-N-(2-Amino-3-methylbutyl)-8-[(2,6-difluorbenzyl)oxy]-2,6-dimethylimidazo[1,2-a]pyridin-3-carboxamid (Enantiomer A)

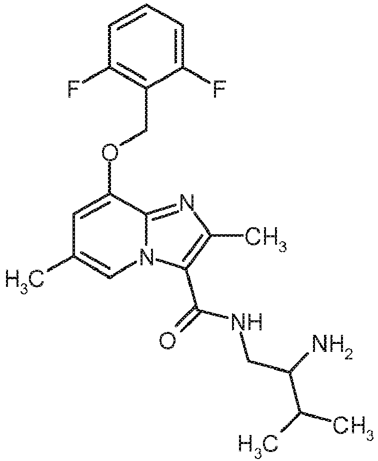

88 mg (0.17 mmol) *ent*-tert.-Butyl-{1-[({8-[(2,6-difluorbenzyl)oxy]-2,6-dimethylimidazo[1,2-a]pyridin-3-yl}carbonyl)amino]-3-methylbutan-2-yl}carbamat (Enantiomer A) Beispiel 231A in 0.85 ml 2 N Salzsäure/Diethylether wurden 4 h bei RT gerührt. Die Reaktionsmischung wurde eingeengt und der Rückstand in Dichlormethan und gesättigter, wässriger Natriumhydrogencarbonat-Lösung gelöst. Die wässrige Phase wurde zweimal mit Dichlormethan extrahiert, die vereinigten organischen Phasen über Natriumsulfat getrocknet, filtriert, eingeengt und lyophilisiert. Man erhielt 64 mg (88% d. Th., Reinheit 98%) der Zielverbindung.
LC-MS (Methode 7): Rₜ = 0.62 min
MS (ESIpos): m/z = 417 (M+H)⁺
¹H-NMR (400 MHz, DMSO-d₆): δ = 0.88 (d, 3H), 0.92 (d, 3H), 1.39 -1.56 (br. s, 2H), 1.56 -1.65 (m, 1H), 2.31 (s, 3H), 2.60 - 2.66 (m, 1H), 3.02 - 3.11 (m, 1H), 3.38 - 3.45 (m, 1H), 5.28 (s, 2H), 6.91 (s, 1H), 7.20 - 7.28 (m, 2H), 7.55 - 7.64 (m, 1H), 7.70 (t, 1H), 8.49 (s, 1H), [weiteres Signal unter DMSO-Peak].

### Beispiel 224

### ent-N-(2-Amino-3-methylbutyl)-8-[(2,6-difluorbenzyl)oxy]-2,6-dimethylimidazo[1,2-a]pyridin-3-carboxamid (Enantiomer B)

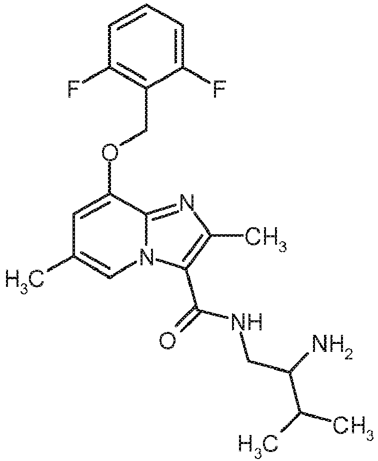

Darstellung und Reinigung der Titelverbindung erfolgte analog zu Beispiel 223. Ausgehend von 92 mg (0.18 mmol) *ent*-tert.-Butyl-{1-[({8-[(2,6-difluorbenzyl)oxy]-2,6-dimethylimidazo[1,2-a]pyridin-3-yl}carbonyl)amino]-3-methylbutan-2-yl}carbamat (Enantiomer B) Beispiel 232A erhielt man 67 mg (88% d. Th., Reinheit 98%) der Zielverbindung.
LC-MS (Methode 7): Rₜ = 0.64 min
MS (ESIpos): m/z = 417 (M+H)⁺
¹H-NMR (400 MHz, DMSO-d₆): δ = 0.89 (d, 3H), 0.92 (d, 3H), 1.19 -1.30 (br. s, 2H), 1.55 -1.69 (m, 2H), 2.31 (s, 3H), 2.60 - 2.68 (m, 1H), 3.02 - 3.11 (m, 1H), 3.39 - 3.46 (m, 1H), 5.28 (s, 2H), 6.91 (s, 1H), 7.20 - 7.28 (m, 2H), 7.55 - 7.64 (m, 1H), 7.67 - 7.73 (m, 1H), 8.48 (s, 1H), [weiteres Signal unter DMSO-Peak].

### Beispiel 225

### rac-N-(2-Amino-2,4-dimethylpentyl)-6-chlor-8-[(2,6-difluorbenzyl)oxy]-2-methylimidazo[1,2-a]pyridin-3-carboxamid

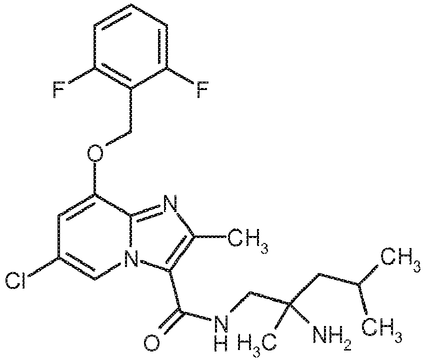

150 mg (0.43 mmol) 6-Chlor-8-[(2,6-difluorbenzyl)oxy]-2-methylimidazo[1,2-a]pyridin-3-carbonsäure Beispiel 16A, 143 mg (0.45 mmol) TBTU und 0.19 ml (1.70 mmol) 4-Methylmorpholin in DMF (1.5 ml) wurden mit 61 mg (0.47 mmol) *rac*-2,4-Dimethylpentan-1,2-diamin versetzt und die Reaktionsmischung wurde über Nacht bei RT gerührt. Das Gemisch wurde mit Wasser/TFA verdünnt und mittels präp. RP-HPLC (Acetonitril/Wasser-Gradient unter Zusatz von 0.1% TFA) gereinigt. Die Produktfraktion wurde eingeengt. Der Rückstand wurde mit gesättigter, wässriger Natriumhydrogencarbonat-Lösung versetzt und dreimal mit Dichlormethan extrahiert. Die vereinigten organischen Phasen wurden über Natriumsulfat getrocknet, abfiltriert und eingeengt. Man erhielt 162 mg (79% d. Th., Reinheit 96%) der Zielverbindung.
LC-MS (Methode 7): Rₜ = 0.75 min
MS (ESIpos): m/z = 465 (M+H)⁺
¹H-NMR (400 MHz, DMSO-d₆): δ = 0.91 (d, 3H), 0.94 (d, 3H), 1.02 (s, 3H), 1.20 - 1.33 (m, 2H), 1.35 - 1.55 (br. s, 2H), 1.73 - 1.85 (m, 1H), 2.55 (s, 3H; überlagert mit DMSO-Signal), 3.21 (q, 2H), 5.35 (s, 2H), 7.20 (d, 1H), 7.22 - 7.29 (m, 2H), 7.57 - 7.65 (m, 1H), 7.67 - 7.80 (br. s, 1H), 8.76 (d, 1H).

### Beispiel 226

### ent-N-(2-Amino-2,4-dimethylpentyl)-6-chlor-8-[(2,6-difluorbenzyl)oxy]-2-methylimidazo[1,2-a]pyridin-3-carboxamid (Enantiomer A)

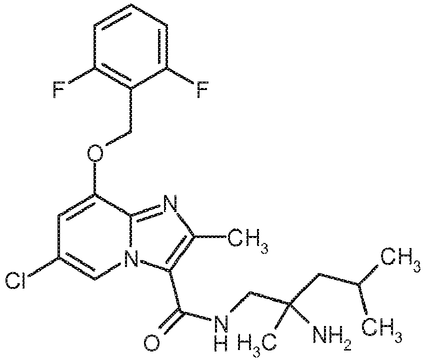

155 mg Beispiel 225 wurden durch präparative Trennung an chiraler Phase in die Enantiomere getrennt [Säule: Daicel Chiralpak AZ-H, 5 µm, 250 x 30 mm, Eluent: 50% iso-Hexan, 50% Ethanol + 0.2% Diethylamin, Fluß: 40 ml/min; 25°C, Detektion: 220 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Die erhaltene Lösung wurde eingefroren und lyophilisiert. Das Produkt wurde anschließend mittels Dickschichtchromatographie (Laufmittel: Dichlormethan/Methanol: 20/1) nachgereinigt.
Enantiomer A: Ausbeute: 18 mg (100% ee)
Rₜ = 8.53 min [Daicel Chiralpak AZ-H, 5µm, 250 x 4.6 mm; Eluent: 50% iso-Hexan, 50% Ethanol + 0.2% Diethylamin; Fluss 1.0 ml/min; 30°C; Detektion: 220 nm].

### Beispiel 227

### ent-N-(2-Amino-2,4-dimethylpentyl)-6-chlor-8-[(2,6-difluorbenzyl)oxy]-2-methylimidazo[1,2-a]pyridin-3-carboxamid (Enantiomer B)

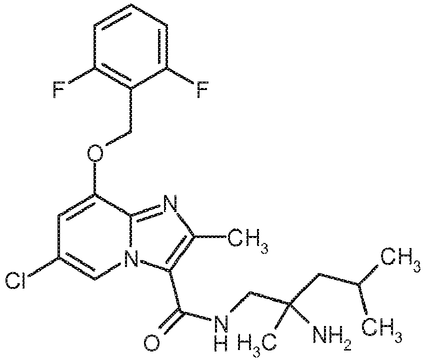

155 mg Beispiel 225 wurden durch präparative Trennung an chiraler Phase in die Enantiomere getrennt [Säule: Daicel Chiralpak AZ-H, 5 µm, 250 x 30 mm, Eluent: 50% iso-Hexan, 50% Ethanol + 0.2% Diethylamin, Fluß: 40 ml/min; 25°C, Detektion: 220 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Die erhaltene Lösung wurde eingefroren und lyophilisiert. Das Produkt wurde anschließend mittels Dickschichtchromatographie (Laufmittel: Dichlormethan/Methanol: 20/1) nachgereinigt.
Enantiomer B: Ausbeute: 31 mg (88% ee)
Rₜ = 10.41 min [Daicel Chiralpak AZ-H, 5µm, 250 x 4.6 mm; Eluent: 50% iso-Hexan, 50% Ethanol + 0.2% Diethylamin; Fluss 1.0 ml/min; 30°C; Detektion: 220 nm].

### Beispiel 228

### ent-N-(2-Amino-3-methylbutyl)-8-[(2,6-difluorbenzyl)oxy]-2-methylimidazo[1,2-a]pyridin-3-carboxamid (Enantiomer A)

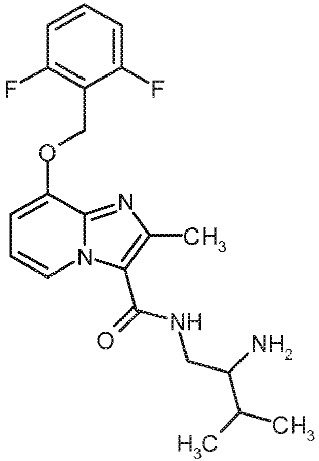

Darstellung und Reinigung der Titelverbindung erfolgte analog zu Beispiel 223. Ausgehend von 88 mg (0.18 mmol) *ent*-tert.-Butyl-{1-[({8-[(2,6-difluorbenzyl)oxy]-2-methylimidazo[1,2-a]pyridin-3-yl}carbonyl)amino]-3-methylbutan-2-yl}carbamat (Enantiomer A) Beispiel 234A erhielt man 64 mg (89% d. Th., Reinheit 98%) der Zielverbindung.
LC-MS (Methode 7): Rₜ = 0.61 min
MS (ESIpos): m/z = 403 (M+H)⁺
¹H-NMR (400 MHz, DMSO-d₆): δ = 0.90 (d, 3H), 0.94 (d, 3H), 1.19 -1.30 (br. s, 2H), 1.64 -1.75 (m, 1H), 2.72 - 2.78 (m, 1H), 3.12 - 3.21 (m, 1H), 3.41 - 3.49 (m, 1H), 5.30 (s, 2H), 6.94 (t, 1H), 7.01 (d, 1H), 7.20 - 7.28 (m, 2H), 7.59 (quintett, 1H), 7.74 - 7.79 (m, 1H), 8.67 (d, 1H), [weiteres Signal unter DMSO-Peak].

### Beispiel 229

### ent-N-(2-Amino-3-methylbutyl)-8-[(2,6-difluorbenzyl)oxy]-2-methylimidazo[1,2-a]pyridin-3-carboxamid (Enantiomer B)

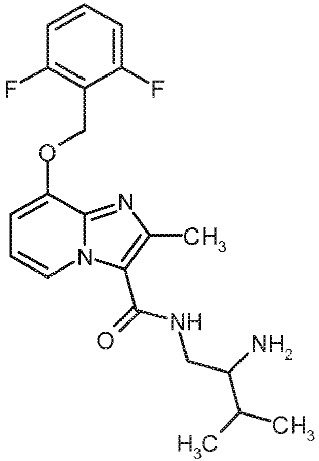

Darstellung und Reinigung der Titelverbindung erfolgte analog zu Beispiel 223. Ausgehend von 72 mg (0.14 mmol) *ent*-tert.-Butyl-{1-[({8-[(2,6-difluorbenzyl)oxy]-2-methylimidazo[1,2-a]pyridin-3-yl}carbonyl)amino]-3-methylbutan-2-yl}carbamat (Enantiomer B) Beispiel 235A erhielt man 52 mg (88% d. Th., Reinheit 98%) der Zielverbindung.
LC-MS (Methode 7): Rₜ = 0.63 min
MS (ESIpos): m/z = 403 (M+H)⁺
¹H-NMR (400 MHz, DMSO-d₆): δ = 0.88 - 0.97 (m, 6H), 1.19 - 1.29 (br. s, 2H), 1.64 - 1.76 (m, 1H), 2.73 - 2.80 (m, 1H), 3.14 - 3.23 (m, 1H), 3.41 - 3.49 (m, 1H), 5.30 (s, 2H), 6.94 (t, 1H), 7.02 (d, 1H), 7.19 - 7.28 (m, 2H), 7.59 (quintett, 1H), 7.73 - 7.82 (m, 1H), 8.67 (d, 1H), [weiteres Signal unter DMSO-Peak].

### Beispiel 230

### rac-N-(2-Amino-3,3,4,4-tetrafluorbutyl)-8-[(2,6-difluorbenzyl)oxy]-2-methylimidazo[1,2-a]pyridin-3-carboxamid

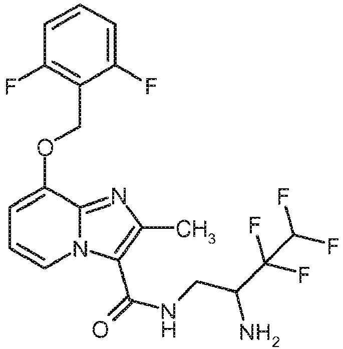

400 mg (1.26 mmol) 8-[(2,6-Difluorbenzyl)oxy]-2-methylimidazo[1,2-a]pyridin-3-carbonsäure Beispiel 3A, 526 mg (1.38 mmol) HATU und 1.1 ml (6.28 mmol) *N,N*-Diisopropylethylamin in DMF (8.0 ml) wurden 20 min bei RT gerührt. Dann wurden 322 mg (1.38 mmol) rac-1-(1,1,2,2-Tetrafluorethyl)-ethylen-1,2-diamin-dihydrochlorid zugegeben und die Reaktionsmischung über Nacht bei RT gerührt. Anschließend wurde direkt mittels präparativer RP-HPLC (Acetonitril/Wasser mit 0.05%ige Ameisensäure) gereinigt. Man erhielt 283 mg (49% d. Th., Reinheit 98%) der Zielverbindung.
LC-MS (Methode 2): Rₜ = 0.75 min
MS (ESIpos): m/z = 461 (M+H)⁺
¹H-NMR (400 MHz, DMSO-d₆): δ = 2.56 (s, 3H; überlagert durch DMSO-Signal), 3.37 - 3.47 (m, 1H), 3.52 - 3.67 (m, 1H), 3.67 - 3.76 (m, 1H), 5.31 (s, 2H), 6.68 (tt, 1H), 6.97 (t, 1H), 7.05 (d, 1H), 7.20 - 7.28 (m, 2H), 7.54 - 7.64 (m, 1H), 7.83 (t, 1H), 8.71 (d, 1H).

### Beispiel 231

### ent-N-(2-Amino-3,3,4,4-tetrafluorbutyl)-8-[(2,6-difluorbenzyl)oxy]-2-methylimidazo[1,2-a]pyridin-3-carboxamid (Enantiomer A)

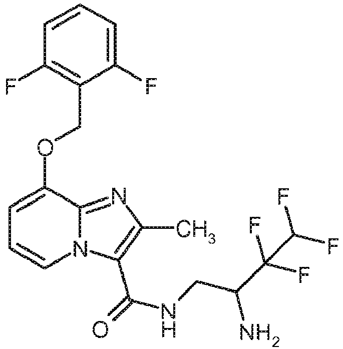

240 mg Beispiel 230 wurden durch präparative Trennung an chiraler Phase in die Enantiomere getrennt [Säule: Daicel Chiralcel OZ-H, 5 µm, 250 x 20 mm, Eluent: 50% iso-Hexan, 50% Ethanol + 0.2% Diethylamin, Fluß: 20 ml/min; 25°C, Detektion: 230 nm].
Enantiomer A: Ausbeute: 91.6 mg (100% ee)
Rₜ = 9.18 min [Daicel Chiralcel OZ-H, 5µm, 250 x 4.6 mm; Eluent: 50% iso-Hexan, 50% Ethanol + 0.2% Diethylamin; Fluss 1.0 ml/min; 30°C; Detektion: 220 nm].

Enantiomer A wurde mittels präparativer RP-HPLC (Acetonitril/Wasser mit 0.05%iger Ameisensäure) nachgereinigt. Die verunreinigte Produktfraktion wurde erneut gereinigt [Säule: Sunfire C18, 5 µm, 250 x 20 mm, Eluent: 55% Wasser, 45% Acetonitril, Fluß: 25 ml/min; 25°C, Detektion: 210 nm]. Man erhielt 48.5 mg (Reinheit 99%) der Zielverbindung.

### Beispiel 232

### rac-N-(2-Amino-2-methylpentyl)-8-[(2,6-difluorbenzyl)oxy]-2,6-dimethylimidazo[1,2-a]pyridin-3-carboxamid

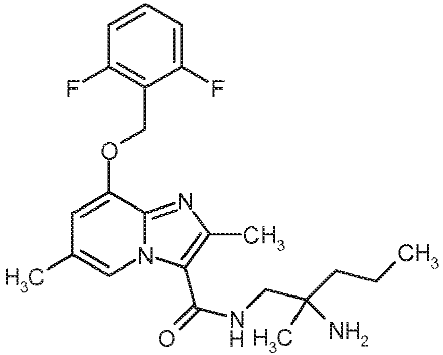

200 mg (0.60 mmol) 8-[(2,6-Difluorbenzyl)oxy]-2,6-dimethylimidazo[1,2-a]pyridin-3-carbonsäure Beispiel 21A, 203 mg (0.63 mmol) TBTU und 0.27 ml (2.41 mmol) 4-Methylmorpholin in DMF (2.1 ml) wurden mit 77 mg (0.66 mmol) *rac*-2-Methylpentan-1,2-diamin versetzt und die Reaktionsmischung über Nacht bei RT gerührt. Das Gemisch wurde mit Wasser/TFA verdünnt und mittels präparativer RP-HPLC (Acetonitril/Wasser-Gradient unter Zusatz von 0.1% TFA) gereinigt. Die Produktfraktion wurde eingeengt. Der Rückstand wurde mit gesättigter, wässriger Natriumhydrogencarbonat-Lösung versetzt und zweimal mit Dichlormethan extrahiert. Die vereinigten organischen Phasen wurden über Natriumsulfat getrocknet, abfiltriert und eingeengt. Man erhielt 199 mg (76% d. Th., Reinheit 99%) der Zielverbindung.
LC-MS (Methode 2): Rₜ = 0.67 min
MS (ESIpos): m/z = 431 (M+H)⁺
¹H-NMR (400 MHz, DMSO-d₆): δ = 0.87 (t, 3H), 0.99 (s, 3H), 1.22 - 1.42 (m, 4H), 1.43 - 1.67 (br. s, 2H), 2.31 (s, 3H), 2.53 (s, 3H; überlagert mit DMSO-Signal), 3.14 - 3.26 (m, 2H), 5.28 (s, 2H), 6.91 (s, 1H), 7.20 - 7.28 (m, 2H), 7.55 - 7.66 (m, 2H), 8.47 (s, 1H).

### Beispiel 233

### ent-N-(2-Amino-2-methylpentyl)-8-[(2,6-difluorbenzyl)oxy]-2,6-dimethylimidazo[1,2-alpyridin-3-carboxamid (Enantiomer A)

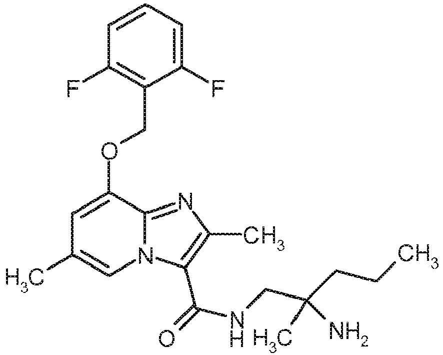

195 mg Beispiel 232 wurden durch präparative Trennung an chiraler Phase in die Enantiomere getrennt [Säule: Daicel Chiralpak AY-H, 5 µm, 250 x 20 mm, Eluent: 60% iso-Hexan, 40% Ethanol + 0.2% Diethylamin, Fluß: 20 ml/min; 25°C, Detektion: 220 nm]. Das Produkt wurde anschließend mittels präparativer RP-HPLC (Acetonitril/Wasser-Gradient unter Zusatz von 0.1% TFA) nachgereinigt. Die Produktfraktion wurde eingeengt. Der Rückstand wurde in Dichlormethan gelöst und zweimal mit gesättigter, wässriger Natriumhydrogencarbonat-Lösung gewaschen. Die vereinigten wässrigen Phasen wurden zweimal mit Dichlormethan extrahiert. Die organische Phase wurde über Natriumsulfat getrocknet, filtriert und eingeengt.
Enantiomer A: Ausbeute: 63 mg (99% ee)
Rₜ = 4.32 min [Daicel Chiralpak AY-H, 5µm, 250 x 4.6 mm; Eluent: 50% iso-Hexan, 50% Ethanol + 0.2% Diethylamin; Fluss 1.0 ml/min; 40°C; Detektion: 220 nm].
Spezifischer Drehwert [a] (365 nm, 19.9°C) = +31.8° (c = 0.005 g/ml, Acetonitril)

### Beispiel 234

### ent-N-(2-Amino-2-methylpentyl)-8-[(2,6-difluorbenzyl)oxy]-2,6-dimethylimidazo[1,2-a]pyridin-3-carboxamid (Enantiomer B)

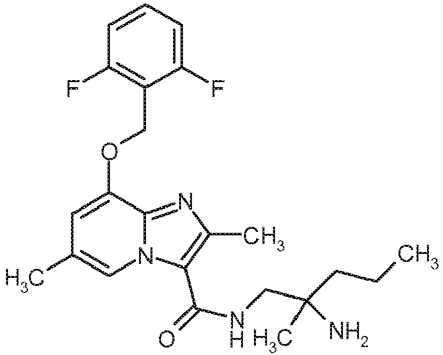

195 mg Beispiel 232 wurden durch präparative Trennung an chiraler Phase in die Enantiomere getrennt [Säule: Daicel Chiralpak AY-H, 5 µm, 250 x 20 mm, Eluent: 60% iso-Hexan, 40% Ethanol + 0.2% Diethylamin, Fluß: 20 ml/min; 25°C, Detektion: 220 nm]. Das Produkt wurde anschließend mittels präparativer RP-HPLC (Acetonitril/Wasser-Gradient unter Zusatz von 0.1% TFA) nachgereinigt. Die Produktfraktion wurde eingeengt. Der Rückstand wurde in Dichlormethan gelöst und zweimal mit gesättigter, wässriger Natriumhydrogencarbonat-Lösung gewaschen. Die vereinigten wässrigen Phasen wurden zweimal mit Dichlormethan extrahiert. Die organische Phase wurde über Natriumsulfat getrocknet, filtriert und eingeengt.
Enantiomer B: Ausbeute: 64 mg (89% ee)
Rₜ = 5.09 min [Daicel Chiralpak AY-H, 5µm, 250 x 4.6 mm; Eluent: 50% iso-Hexan, 50% Ethanol + 0.2% Diethylamin; Fluss 1.0 ml/min; 40°C; Detektion: 220 nm].

### Beispiel 235

### rac-8-[(2,6-Difluorbenzyl)oxyl-2,6-dimethyl-N-(1,2,3,4-tetrahydrochinolin-2-ylmethyl)imidazo[1,2-a]pyridin-3-carboxamid

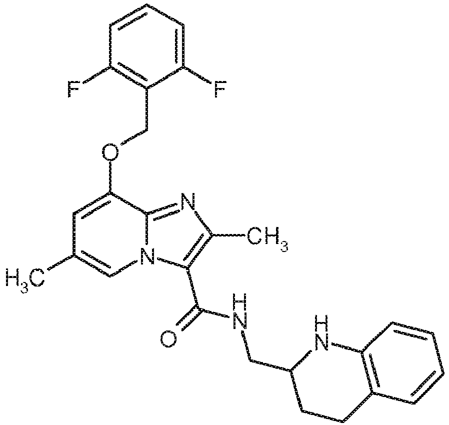

182 mg (0.32 mmol) *rac*-tert.-Butyl-2-{[({8-[(2,6-difluorbenzyl)oxy]-2,6-dimethylimidazo[l,2-a]pyridin-3-yl}carbonyl)amino]methyl}-3,4-dihydrochinolin-1(2H)-carboxylat Beispiel 240A wurde in 5 ml Diethylether und 1.58 ml (3.16 mmol) 2 N Salzsäure/Diethylether vorgelegt und über Nacht bei RT gerührt. Dann wurde mit 1 ml (2 mmol) 2 N Salzsäure/Diethylether versetzt und weiter über Nacht bei RT gerührt. Die Reaktionsmischung wurde abfiltriert und mit Diethylether gewaschen. Der Feststoff wurde in Dichlormethan und und sehr wenig Methanol aufgenommen und zweimal mit gesättigter, wässriger Natriumhydrogen-carbonat-Lösung gewaschen. Die wässrige Phase wurde zweimal mit Dichlormethan extrahiert und die vereinigten organischen Phasen über Natriumsulfat getrocknet. Es wurde filtriert, eingeengt und im Hochvakuum getrocknet. Man erhielt 140 mg (91% d. Th., Reinheit 98%) der Zielverbindung.
LC-MS (Methode 2): Rₜ = 1.01 min
MS (ESIpos): m/z = 477 (M+H)⁺
¹H-NMR (400 MHz, DMSO-d₆): δ = 1.55 -1.67 (m, 1H), 1.86 -1.96 (m, 1H), 2.31 (s, 3H), 2.69 (t, 2H), 3.36 - 3.50 (m, 3H), 5.28 (s, 2H), 5.72 (s, 1H), 6.43 (t, 1H), 6.48 (d, 1H), 6.82 - 6.89 (m, 2H), 6.92 (s, 1H), 7.19 - 7.28 (m, 2H), 7.54 - 7.64 (m, 1H), 7.91 (t, 1H), 8.46 (s, 1H), [weiteres Signal unter DMSO-Peak verborgen].

### Beispiel 236

### rac-N-(2-Amino-3,3,3-trifluor-2-methylpropyl)-6-chlor-8-[(2,6-difluorbenzyl)oxy]-2-methylimidazo[1,2,a]pyridin-3-carboxamid

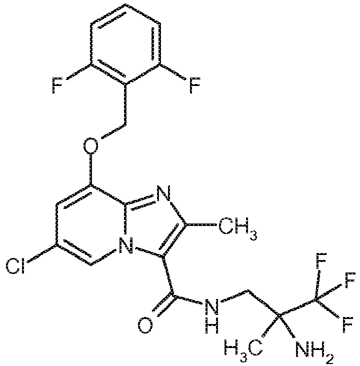

Darstellung und Reinigung der Titelverbindung erfolgte analog zu Beispiel 225. Ausgehend von 200 mg (0.57 mmol) 6-Chlor-8-[(2,6-difluorbenzyl)oxy]-2-methylimidazo[1,2-a]pyridin-3-carbonsäure Beispiel 16A und 111 mg (0.62 mmol) *rac*-3,3,3-Trifluor-2-methylpropan-1,2-diaminhydrochlorid Beispiel 246A erhielt man 211 mg (74% d. Th., Reinheit 95%) der Zielverbindung.
LC-MS (Methode 2): Rₜ = 0.98 min
MS (ESIpos): m/z = 477 (M+H)⁺
¹H-NMR (400 MHz, DMSO-d₆): δ = 1.23 (s, 3H), 2.55 (s, 3H; überlagert mit DMSO-Signal), 3.47 - 3.64 (m, 2H), 5.35 (s, 2H), 7.21 - 7.30 (m, 3H), 7.56 - 7.67 (m, 1H), 7.92 - 8.01 (m, 1H), 8.76 (d, 1H).

### Beispiel 237

### ent-N(2-Amino-3,3,3-trifluor-2-methylpropyl)-6-chlor-8-[(2,6-difluorbenzyl)oxy]-2-methylimidazo[1,2,a]pyridin-3-carboxamid (Enantiomer A)

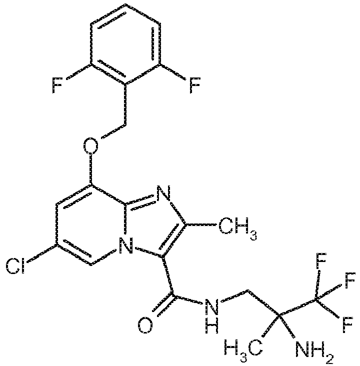

204 mg Beispiel 236 wurden durch präparative Trennung an chiraler Phase in die Enantiomere getrennt [Säule: Daicel Chiralpak OZ-H, 5 µm, 250 x 20 mm, Eluent: 50% iso-Hexan, 50% Isopropanol + 0.2% Diethylamin, Fluß: 17 ml/min; 40°C, Detektion: 210 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Die erhaltene Lösung wurde eingefroren und lyophilisiert.
Enantiomer A: Ausbeute: 50 mg (99% ee)
Rₜ = 9.71 min [Daicel Chiralpak OZ-H, 5 µm, 250 x 4.6 mm; Eluent: 50% iso-Hexan, 50% Isopropanol + 0.2% Diethylamin; Fluss 1.0 ml/min; 30°C; Detektion: 220 nm].

### Beispiel 238

### ent-N-(2-Amino-3,3,3-trifluor-2-methylpropyl)-6-chlor-8-[(2,6-difluorbenzyl)oxy]-2-methylimidazo[1,2-a]pyridin-3-carboxamid (Enantiomer B)

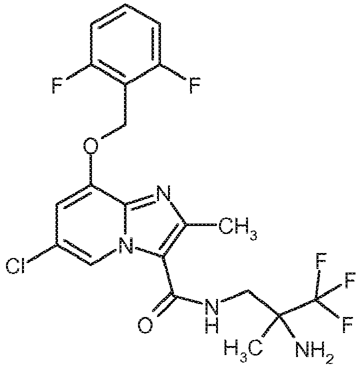

204 mg Beispiel 236 wurden durch präparative Trennung an chiraler Phase in die Enantiomere getrennt [Säule: Daicel Chiralpak OZ-H, 5 µm, 250 x 20 mm, Eluent: 50% iso-Hexan, 50% Isopropanol + 0.2% Diethylamin, Fluß: 17 ml/min; 40°C, Detektion: 210 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Die erhaltene Lösung wurde eingefroren und lyophilisiert.
Enantiomer B: Ausbeute: 69 mg (91% ee)
Rₜ = 11.21 min [Daicel Chiralpak OZ-H, 5 µm, 250 x 4.6 mm; Eluent: 50% iso-Hexan, 50% Isopropanol + 0.2% Diethylamin; Fluss 1.0 ml/min; 30°C; Detektion: 220 nm].

### Beispiel 239

### rac-N-(2-Amino-3,3,3-trifluor-2-methylpropyl)-8-[(2,6-difluorbenzyl)oxy]-2,6-dimethylimidazo[1,2-a]pyridin-3-carboxamid

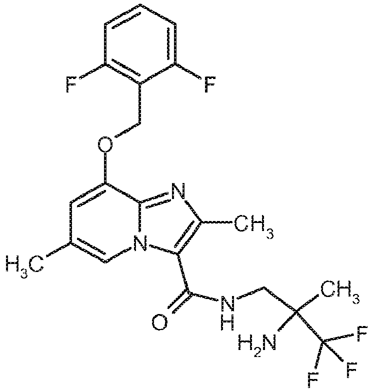

200 mg (0.60 mmol) 8-[(2,6-difluorbenzyl)oxy]-2,6-dimethylimidazo[1,2-a]pyridin-3-carbonsäure Beispiel 21A, 203 mg (0.63 mmol) TBTU und 0.27 ml (2.41 mmol) 4-Methylmorpholin in DMF (2.1 ml) wurden mit 118 mg (0.66 mmol) *rac-*3,3,3-Trifluor-2-methylpropan-1,2-diaminhydrochlorid Beispiel 246A versetzt und die Reaktionsmischung über Nacht bei RT gerührt. 50 mg (0.28 mmol) *rac*-3,3,3-Trifluor-2-methylpropan-1,2-diaminhydrochlorid Beispiel 246A wurde zum Reaktionsgemisch zugegeben und es wurde weiter über Nacht bei RT gerührt. Das Gemisch wurde mit Wasser/TFA verdünnt und mittels präparativer RP-HPLC (Acetonitril/Wasser-Gradient unter Zusatz von 0.1% TFA) gereinigt. Die Produktfraktion wurde eingeengt. Der Rückstand wurde mit gesättigter, wässriger Natriumhydrogencarbonat-Lösung versetzt und dreimal mit Dichlormethan extrahiert. Die vereinigten organischen Phasen wurden über Natriumsulfat getrocknet, abfiltriert und eingeengt. Man erhielt 219 mg (79% d. Th., Reinheit 99%) der Zielverbindung.
LC-MS (Methode 2): Rₜ = 0.79 min
MS (ESIpos): m/z = 457 (M+H)⁺
¹H-NMR (400 MHz, DMSO-d₆): δ = 1.19 (s, 3H), 2.31 (s, 3H), 2.53 (s, 3H; überlagert mit DMSO-Peak), 3.41 - 3.49 (m, 1H), 3.53 - 3.61 (m, 1H), 5.29 (s, 2H), 6.94 (s, 1H), 7.20 - 7.28 (m, 2H), 7.59 (quintett, 1H), 7.78 (t, 1H), 8.48 (s, 1H).

### Beispiel 240

### ent-N-(2-Amino-3,3,3-trifluor-2-methylpropyl)-8-[(2,6-difluorbenzyl)oxy]-2,6-dünethylimidazo[1,2-a]pyridin-3-carboxamid (Enantiomer A)

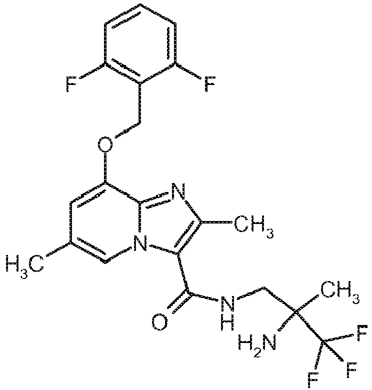

210 mg Beispiel 239 wurden durch präparative Trennung an chiraler Phase in die Enantiomere getrennt [Säule: Daicel Chiralcel OZ-H, 5 µm, 250 x 20 mm, Eluent: 25% iso-Hexan, 75% Isopropanol; Fluß: 20 ml/min; 23°C, Detektion: 210 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Die erhaltene Lösung wurde eingefroren und lyophilisiert.
Enantiomer A: Ausbeute: 63 mg (100% ee)
Rₜ = 6.53 min [Daicel Chiralcel OZ-H, 5 µm, 250 x 4.6 mm; Eluent: 50% iso-Hexan, 50% Ethanol + 0.2% Diethylamin; Fluss 1.0 ml/min; 30°C; Detektion: 220 nm].

### Beispiel 241

### ent-N-(2-Amino-3,3,3-trifluor-2-methylpropyl)-8-[(2,6-difluorbenzyl)oxy]-2,6-dimethylimidazo[1,2-a]pyndin-3-carboxamid (Enantiomer B)

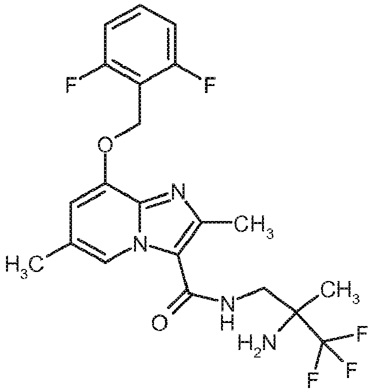

210 mg Beispiel 239 wurden durch präparative Trennung an chiraler Phase in die Enantiomere getrennt [Säule: Daicel Chiralcel OZ-H, 5 µm, 250 x 20 mm, Eluent: 25% iso-Hexan, 75% Isopropanol; Fluß: 20 ml/min; 23°C, Detektion: 210 nm]. Die Produktfraktionen wurden auf Trockeneis aufgefangen, vereint, am Rotationsverdampfer (30°C Badtemperatur) bis auf ca. 30 ml Restvolumen eingeengt und mit ca. 60 ml Wasser versetzt. Die erhaltene Lösung wurde eingefroren und lyophilisiert.
Enantiomer B: Ausbeute: 50 mg (92% ee)
Rₜ = 7.07 min [Daicel Chiralcel OZ-H, 5 µm, 250 x 4.6 mm; Eluent: 50% iso-Hexan, 50% Ethanol + 0.2% Diethylamin; Fluss 1.0 ml/min; 30°C; Detektion: 220 nm].

### Beispiel 242

### ent-N-(2-Amino-3-methoxypropyl)-8-[(2,6-difluorbenzyl)oxy]-2,6-dimethylünidazo[1,2-a]pyridin-3-carboxamid (Enantiomer A)

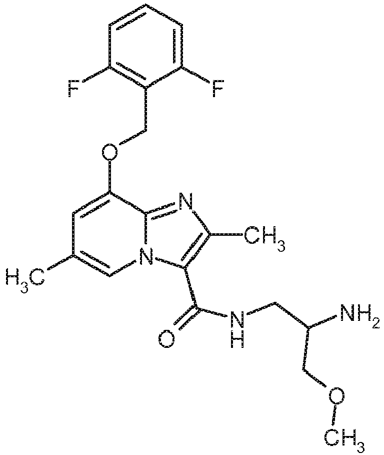

100 mg (0.29 mmol, 96% rein) 8-[(2,6-Difluorbenzyl)oxy]-2,6-dimethylimidazo[1,2-a]pyridin-3-carbonsäure Beispiel 21A wurden in DMF (1.84 ml) vorgelegt, mit 121 mg (0.32 mmol) HATU und 0.15 ml (0.87 mmol) *N,N* Diisopropylethylamin versetzt und 20 min bei RT gerührt. 72 mg (0.40 mmol) *ent*-3-Methoxypropan-1,2-diamindihydrochlorid (Enantiomer A) Beispiel 249A wurde in 0.48 ml DMF gelöst, mit 0.30 ml (1.73 mmol) *N,N-*Diisopropylethylamin versetzt und 10 min bei RT gerührt. Anschließend wurde diese Lösung bei -20°C zur vorher hergestellten Reaktionslösung gegeben und die Reaktionsmischung wurde über Nacht bei RT gerührt. Dann wurde mit Wasser/TFA verdünnt und mittels präparativer RP-HPLC (Acetonitril/Wasser-Gradient unter Zusatz von 0.1% TFA) gereinigt. Die Produktfraktion wurde eingeengt. Der Rückstand wurde mit Dichlormethan/Essigsäureethylester und gesättigter, wässriger Natriumhydrogen-carbonat-Lösung versetzt. Nach Phasentrennung wurde die wässrige Phase zweimal mit Dichlormethan extrahiert. Die vereinigten organischen Phasen wurden mit gesättigter, wässriger Natriumhydrogencarbonat-Lösung gewaschen, über Natriumsulfat getrocknet, abfiltriert und eingeengt. Man erhielt 77 mg (63% d. Th., Reinheit 99%) der Zielverbindung.
LC-MS (Methode 2): Rₜ = 0.67 min
MS (ESIpos): m/z = 419 (M+H)⁺
¹H-NMR (400 MHz, DMSO-d₆): δ = 2.31 (s, 3H), 3.09 - 3.16 (m, 1H), 3.19 - 3.27 (m, 2H), 3.28 (s, 3H), 3.38 - 3.44 (m, 2H; überlagert vom Wasser-Signal), 5.28 (s, 2H), 6.92 (s, 1H), 7.18 - 7.29 (m, 2H), 7.54 - 7.65 (m, 1H), 7.74 (t, 1H), 8.50 (s, 1H), [weiteres Signal unter DMSO-Peak].

### Beispiel 243

### ent-N-(2-Amino-3-methoxypropyl)-8-[(2,6-difluorbenzyl)oxy]-2,6-dimethylimidazo[1,2,-a]pyridin-3-carboxamid (Enantiomer B)

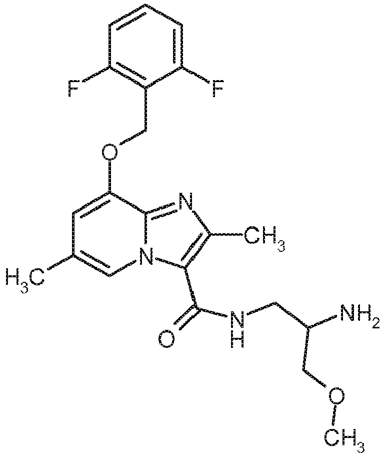

Darstellung und Reinigung der Titelverbindung erfolgte analog zu Beispiel 242. Ausgehend von 100 mg (0.29 mmol, 96% rein) 8-[(2,6-Difluorbenzyl)oxy]-2,6-dimethylimidazo[1,2-a]pyridin-3-carbonsäure Beispiel 21A und 72 mg (0.40 mmol) *ent*-3-Methoxypropan-1,2-diamindihydrochlorid (Enantiomer B) Beispiel 250A erhielt man 63 mg (51% d. Th., Reinheit 99%) der Zielverbindung.
LC-MS (Methode 2): Rₜ = 0.67 min
MS (ESIpos): m/z = 419 (M+H)⁺
¹H-NMR (400 MHz, DMSO-d₆): δ = 1.95 - 2.25 (br. s, 2H), 2.31 (s, 3H), 3.02 - 3.10 (m, 1H), 3.15 - 3.25 (m, 2H), 3.27 - 3.41 (m, 5H; überlagert mit Wasser-Signal), 5.28 (s, 2H), 6.92 (s, 1H), 7.19 - 7.28 (m, 2H), 7.55 - 7.64 (m, 1H), 7.72 (t, 1H), 8.49 (s, 1H).

### Beispiel 244

### rac-N-(2-Amino-2-methylpentyl)-6-chlor-8-[(2,6-difluorbenzyl)oxy]-2-methylimidazo[1,2-a]pyridin-3-carboxamid

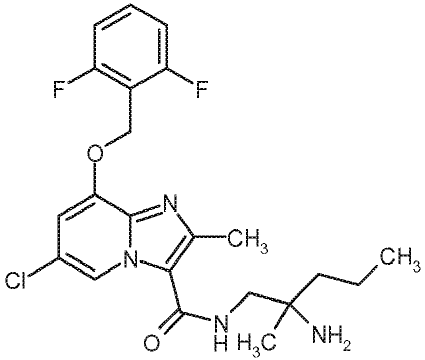

200 mg (0.57 mmol) 6-Chlor-8-[(2,6-difluorbenzyl)oxy]-2-methylimidazo[1,2-a]pyridin-3-carbonsäure Beispiel 16A wurden in DMF (2.0 ml) mit 191 mg (0.62 mmol) TBTU und 0.25 ml (2.27 mmol) 4-Methylmorpholin und 72 mg (0.62 mmol) *rac*-2-Methylpentan-1,2-diamin versetzt und die Reaktionsmischung über Nacht bei RT gerührt. Dann wurde mit 36 mg (0.31 mmol) *rac*-2-Methylpentan-1,2-diamin und 0.06 ml (0.57 mmol) 4-Methylmorpholin versetzt und weiter über Nacht bei RT gerührt. Das Gemisch wurde mit Wasser/TFA verdünnt und mittels präparativer RP-HPLC (Acetonitril/Wasser-Gradient unter Zusatz von 0.1% TFA) gereinigt. Die Produktfraktion wurde eingeengt. Der Rückstand wurde mit gesättigter, wässriger Natrium-hydrogencarbonat-Lösung versetzt und dreimal mit Dichlormethan extrahiert. Die vereinigten organischen Phasen wurden über Natriumsulfat getrocknet, abfiltriert und eingeengt. Der Rückstand wurde mittels Kieselgelchromatographie (Dichlormethan/Methanol: 10:1, anschließend Dichlormethan/2N Ammoniak in Methanol 20:1) gereinigt. Man erhielt 189 mg (73% d. Th., Reinheit 99%) der Zielverbindung.
LC-MS (Methode 2): Rₜ = 0.78 min
MS (ESIpos): m/z = 451 (M+H)⁺
¹H-NMR (400 MHz, DMSO-d₆): δ = 0.83 - 0.89 (m, 3H), 0.99 (s, 3H), 1.22 - 1.42 (m, 4H), 1.52 - 1.88 (br. s, 2H), 2.55 (s, 3H; überlagert mit DMSO-Peak), 3.15 - 3.26 (m, 2H), 5.35 (s, 2H), 7.20 (d, 1H), 7.22 - 7.29 (m, 2H), 7.56 - 7.65 (m, 1H), 7.67 - 7.82 (br. s, 1H), 8.76 (d, 1H).

### Beispiel 245

### ent-N-(2-Amino-2-methylpentyl)-6-hlor-8-[(2,6-difluorbenzyl)oxy]-2-methylimidazo[1,2-a]pyridin-3-carboxamid (Enantiomer A)

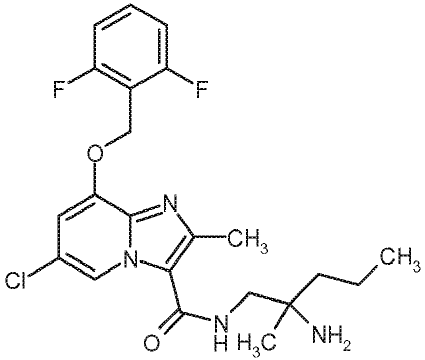

182 mg Beispiel 244 wurden durch präparative Trennung an chiraler Phase in die Enantiomere getrennt [Säule: Daicel Chiralpak AZ-H, 5 µm, 250 x 30 mm, Eluent: 50% iso-Hexan, 50% Ethanol + 0.2% Diethylamin, Fluß: 40 ml/min; 25°C, Detektion: 220 nm]. Die Produktfraktionen wurden auf Trockeneis aufgefangen, vereint und am Rotationsverdampfer (30°C Badtemperatur) eingeengt. Anschließend wurde nochmals mittels präparativer RP-HPLC (Acetonitril/Wasser-Gradient unter Zusatz von 0.1% TFA) gereinigt. Die Produktfraktion wurde eingeengt. Der Rückstand wurde in Dichlormethan gelöst und mit gesättigter, wässriger Natriumhydrogencarbonat-Lösung gewaschen. Die vereinigten wässrigen Phasen wurden dreimal mit Dichlormethan extrahiert. Die vereinten organischen Phasen wurden über Natriumsulfat getrocknet, filtriert und eingeengt.
Enantiomer A: Ausbeute: 30 mg (88% ee)
Rₜ = 7.90 min [Daicel Chiralpak AZ-H, 5 µm, 250 x 4.6 mm; Eluent: 50% iso-Hexan, 50% Ethanol + 0.2% Diethylamin; Fluss 1.0 ml/min; 35°C; Detektion: 220 nm].
Spezifischer Drehwert [a] (589 nm, 19.7°C) = -2.6° (c = 0.005 g/ml, Acetonitril)

### Beispiel 246

### ent-N-(2-Amino-2-methylpentyl)-6-chlor-8-[(2,6-difluorbenzyl)oxy]-2-methylimidazo[1,2-a]pyridin-3-carboxamid (Enantiomer B)

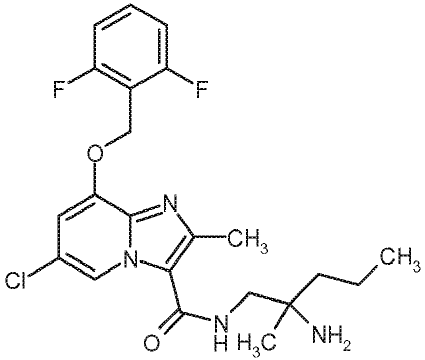

182 mg Beispiel 244 wurden durch präparative Trennung an chiraler Phase in die Enantiomere getrennt [Säule: Daicel Chiralpak AZ-H, 5 µm, 250 x 30 mm, Eluent: 50% iso-Hexan, 50% Ethanol + 0.2% Diethylamin, Fluß: 40 ml/min; 25°C, Detektion: 220 nm]. Die Produktfraktionen wurden auf Trockeneis aufgefangen, vereint und am Rotationsverdampfer (30°C Badtemperatur) eingeengt. Anschließend wurde nochmals mittels präparativer RP-HPLC (Acetonitril/Wasser-Gradient unter Zusatz von 0.1% TFA) gereinigt. Die Produktfraktion wurde eingeengt. Der Rückstand wurde in Dichlormethan gelöst und mit gesättigter, wässriger Natriumhydrogencarbonat-Lösung gewaschen. Die vereinigten wässrigen Phasen wurden dreimal mit Dichlormethan extrahiert. Die vereinten organischen Phasen wurden über Natriumsulfat getrocknet, filtriert und eingeengt.
Enantiomer B: Ausbeute: 24 mg (77% ee)
Rₜ = 9.07 min [Daicel Chiralpak AZ-H, 5 µm, 250 x 4.6 mm; Eluent: 50% iso-Hexan, 50% Ethanol + 0.2% Diethylamin; Fluss 1.0 ml/min; 35°C; Detektion: 220 nm].
Spezifischer Drehwert [a] (589 nm, 19.9°C) = +2.5° (c = 0.0048 g/ml, Acetonitril)

### Beispiel 247

### ent-N-(2-Amino-3-methoxypropyl)-8-[(2,6-difluorbenzyl)oxy]-2-methylimidazo[1,2-a]pyridin-3-carboxamid (Enantiomer A)

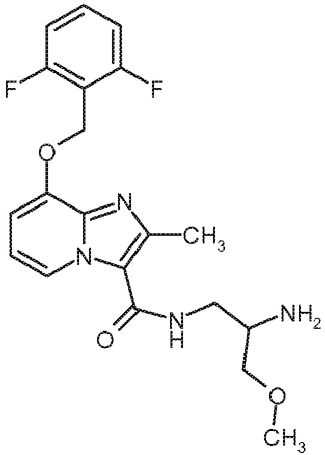

Darstellung und Reinigung der Titelverbindung erfolgte analog zu Beispiel 242. Ausgehend von 100 mg (0.31 mmol) 8-[(2,6-Difluorbenzyl)oxy]-2-methylimidazo[1,2-a]pyndin-3-carbonsäure Beispiel 3A und 78 mg (0.44 mmol) *ent*-3-Methoxypropan-1,2-diamindihydrochlorid (Enantiomer A) Beispiel 249 A erhielt man 76 mg (59% d. Th., Reinheit 98%) der Zielverbindung.
LC-MS (Methode 2): Rₜ = 0.62 min
MS (ESIpos): m/z = 405 (M+H)⁺
¹H-NMR (400 MHz, DMSO-d₆): δ = 1.57 - 1.87 (br. s, 2H), 2.53 (s, 3H; überlagert mit DMSO-Peak), 3.00 - 3.08 (m, 1H), 3.14 - 3.25 (m, 2H), 3.26 - 3.42 (m, 5H; überlagert mit Wasser-Signal), 5.30 (s, 2H), 6.93 (t, 1H), 7.01 (d, 1H), 7.19 - 7.28 (m, 2H), 7.54 - 7.64 (m, 1H), 7.75 (t, 1H), 8.65 (d, 1H).

### Beispiel 248

### ent-N-(2-Amino-3-methoxypropyl)-8-[(2,6-difluorbenzyl)oxy]-2-methylimidazo[1,2-a]pyridin-3-carboxamid (Enantiomer B)

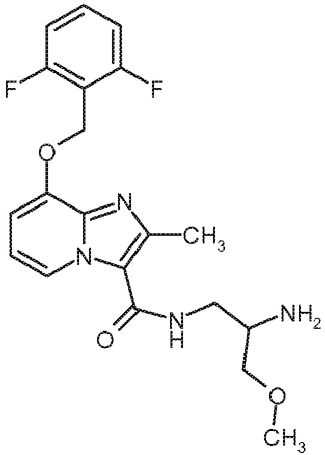

Darstellung und Reinigung der Titelverbindung erfolgte analog zu Beispiel 242. Ausgehend von 100 mg (0.31 mmol) 8-[(2,6-Difluorbenzyl)oxy]-2-methylimidazo[1,2-a]pyridin-3-carbonsäure Beispiel 3A und 78 mg (0.44 mmol) *ent*-3-Methoxypropan-1,2-diamindihydrochlorid (Enantiomer B) Beispiel 250A erhielt man 72 mg (56% d. Th., Reinheit 99%) der Zielverbindung.
LC-MS (Methode 2): Rₜ = 0.62 min
MS (ESIpos): m/z = 405 (M+H)⁺
¹H-NMR (400 MHz, DMSO-d₆): δ = 1.56 - 1.85 (br. s, 2H), 2.53 (s, 3H; überlagert mit DMSO-Peak), 3.00 - 3.07 (m, 1H), 3.14 - 3.26 (m, 2H), 3.26 - 3.42 (m, 5H; überlagert mit Wasser-Signal), 5.30 (s, 2H), 6.93 (t, 1H), 7.01 (d, 1H), 7.19 - 7.28 (m, 2H), 7.54 - 7.64 (m, 1H), 7.75 (t, 1H), 8.66 (d, 1H).

### Beispiel 249

### rac-N-(2-Amino-2,4-dimethylpentyl)-2,6-dimethyl-8-(3-methylbutoxy)imidazo[1,2-a]pyridin-3-carboxamid

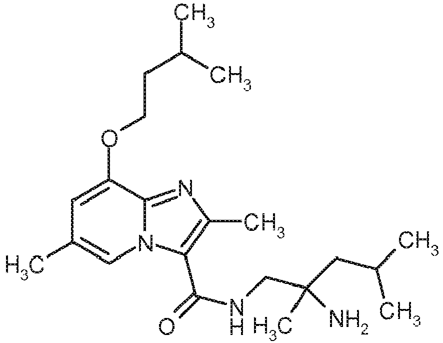

175 mg (0.63 mmol) 2,6-Dimethyl-8-(3-methylbutoxy)imidazo[1,2-a]pyridin-3-carbonsäure Beispiel 252A wurden in DMF (2.2 ml) vorgelegt, mit 214 mg (0.67 mmol) TBTU, 0.28 ml (2.53 mmol) 4-Methylmorpholin und 91 mg (0.70 mmol) *rac*-2,4-Dimethylpentan-1,2-diamin versetzt und die Reaktionsmischung über Nacht bei RT gerührt. Das Gemisch wurde mit Wasser/TFA verdünnt und mittels präparativer RP-HPLC (Acetonitril/Wasser-Gradient unter Zusatz von 0.1% TFA) gereinigt. Nach Einengen wurde der Rückstand mit gesättigter, wässriger Natriumhydrogen-carbonat-Lösung versetzt und dreimal mit Dichlormethan extrahiert. Die vereinigten organischen Phasen wurden über Natriumsulfat getrocknet, abfiltriert und eingeengt. Man erhielt 225 mg (90% d. Th., Reinheit 98%) der Zielverbindung.
LC-MS (Methode 7): Rₜ = 0.62 min
MS (ESIpos): m/z = 389 (M+H)⁺
¹H-NMR (400 MHz, DMSO-d₆): δ = 0.90 - 0.98 (m, 12H), 1.07 (s, 3H), 1.25 - 1.38 (m, 2H), 1.65 - 1.73 (m, 2H), 1.75 - 1.88 (m, 2H), 2.27 (s, 3H), 2.57 (s, 3H), 3.18 - 3.29 (m, 2H), 4.15 (t, 2H), 6.71 (s, 1H), 7.66 (t, 1H), 8.42 (s, 1H).

### Beispiel 250

### rac-8-[(2,6-Difluorbenzyl)oxy]-2-methyl-N-(1,2,3,4-tetrahydrochinolin-2-ylmethyl)imidazo[1,2-a]pyridin-3-carboxamid

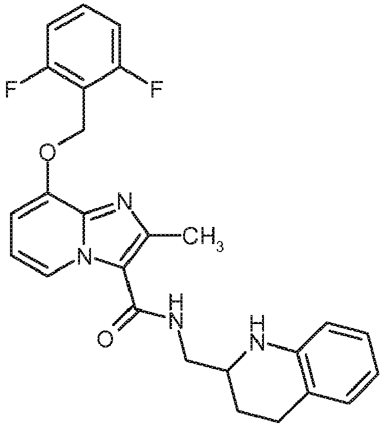

Darstellung und Reinigung der Titelverbindung erfolgte analog zu Beispiel 235. Ausgehend von 190 mg (0.34 mmol) *rac*-tert.-Butyl-2-{[({8-[(2,6-difluorbenzyl)oxy]-2-methylimidazo[1,2-a]pyridin-3-yl}carbonyl)amino]methyl}-3,4dihydrochinolin-1(2H)-carboxylat Beispiel 253A erhielt man 149 mg (93% d. Th., Reinheit 98%) der Zielverbindung.
LC-MS (Methode 2): Rₜ = 0.99 min
MS (ESIpos): m/z = 463 (M+H)⁺
¹H-NMR (400 MHz, DMSO-d₆): δ = 1.56 - 1.67 (m, 1H), 1.86 -1.95 (m, 1H), 2.66 - 2.73 (m, 2H), 3.37 - 3.50 (m, 3H), 5.31 (s, 2H), 5.70 - 5.77 (m, 1H), 6.43 (t, 1H), 6.48 (d, 1H), 6.82 - 6.89 (m, 2H), 6.95 (t, 1H), 7.03 (d, 1H), 7.20 - 7.28 (m, 2H), 7.54 - 7.64 (m, 1H), 7.96 (t, 1H), 8.64 (d, 1H), [weitere Signale unter Lösungsmittel-Peaks].

### Beispiel 251

### ent-N-[(1R,2R)-2-Aminocyclohexyl]-8-[(2,6-difluorbenzyl)oxy]-2,6-dimethylimidazo[1,2-a]pyridin-3-carboxamid

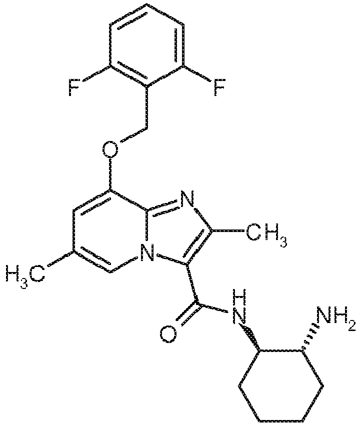

Darstellung und Reinigung der Titelverbindung erfolgte analog zu Beispiel 235. Ausgehend von 107 mg (0.2 mmol, 98% rein) *ent*-tert.-Butyl-{(1*R*,2*R*)-2-[({8-[(2,6-difluorbenzyl)oxy]-2,6-dimethylimidazo[1,2-a]pyridin-3-yl}carbonyl)amino]cyclohexyl}carbamat Beispiel 254A erhielt man 83 mg (97% d. Th., Reinheit 99%) der Zielverbindung.
LC-MS (Methode 7): Rₜ = 0.59 min
MS (ESIpos): m/z = 429 (M+H)⁺
¹H-NMR (400 MHz, DMSO-d₆): δ = 1.08 - 1.35 (m, 4H), 1.61 -1.72 (m, 2H), 1.81 -1.89 (m, 1H), 1.90 - 1.97 (m, 1H), 2.30 (s, 3H), 3.45 - 3.56 (m, 1H), 5.28 (s, 2H), 6.89 (s, 1H), 7.20 - 7.28 (m, 2H), 7.54 - 7.64 (m, 1H), 7.67 (d, 1H), 8.37 (s, 1H), [weitere Signale unter Lösungsmittel-Peaks].

### Beispiel 252

### N-(2-Amino-2-ethylbutyl)-8-[(2,6-difluorbenzyl)oxy]-2,6-dimethylimidazo[1,2-a]pyridin-3-carboxamid

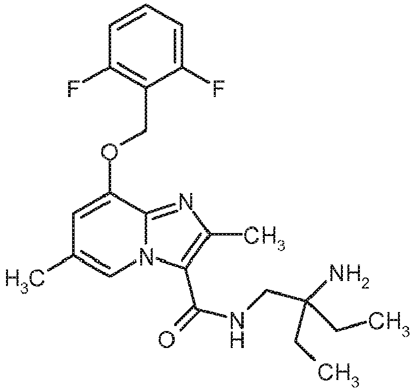

100 mg (0.30 mmol) 8-[(2,6-Difluorbenzyl)oxy]-2,6-dimethylimidazo[1,2-a]pyridin-3-carbonsäure Beispiel 21A, 145 mg (0.45 mmol) TBTU und 0.13 ml (1.20 mmol) 4-Methylmorpholin in DMF (1.9 ml) wurden mit 70 mg (0.60 mmol) 2-Ethylbutan-1,2-diamin versetzt und die Reaktionsmischung wurde über Nacht bei RT gerührt. Anschließend wurde mit Wasser/TFA verdünnt und mittels präparativer RP-HPLC (Acetonitril/Wasser-Gradient unter Zusatz von 0.1% TFA) gereinigt. Nach Einengen wurde der Rückstand in Dichlormethan aufgenommen und zweimal mit gesättigter, wässriger Natriumhydrogencarbonat-Lösung gewaschen. Die wässrige Phase wurde zweimal mit Dichlormethan extrahiert. Die vereinigten organischen Phasen wurden über Natriumsulfat getrocknet, abfiltriert, eingeengt und lyophilisiert. Man erhielt 110 mg (85% d. Th., Reinheit 100%) der Zielverbindung.
LC-MS (Methode 2): Rₜ = 0.60 min
MS (ESIpos): m/z = 431 (M+H)⁺
¹H-NMR (400 MHz, DMSO-d₆): δ = 0.82 (t, 6H), 1.22 - 1.49 (m, 6H), 2.31 (s, 3H), 2.52 (s, 3H; überlagert mit DMSO-Peak), 3.17 - 3.23 (m, 2H), 5.28 (s, 2H), 6.91 (s, 1H), 7.19 - 7.28 (m, 2H), 7.51 (t, 1H), 7.55 - 7.64 (m, 1H), 8.50 (s, 1H).

### Beispiel 253

### N-(2-Amino-2-ethylbutyl)-8-[(2,6-difluorbenzyl)oxy]-2-methylimidazo[1,2-a]pyridin-3-carboxamid

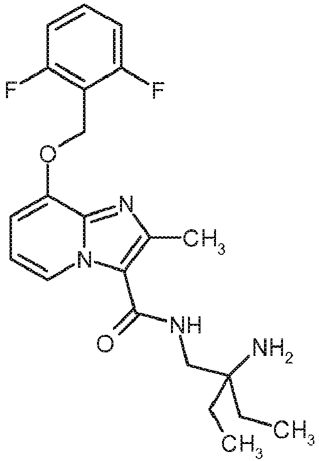

Darstellung und Reinigung der Titelverbindung erfolgte analog zu Beispiel 252. Ausgehend von 75 mg (0.24 mmol) 8-[(2,6-Difluorbenzyl)oxy]-2-methylimidazo[1,2-a]pyridin-3-carbonsäure Beispiel 3A und 41 mg (0.35 mmol) 2-Ethylbutan-1,2-diamin erhielt man 73 mg (74% d. Th., Reinheit 99%) der Zielverbindung.
LC-MS (Methode 2): Rₜ = 0.64 min
MS (ESIpos): m/z = 417 (M+H)⁺
¹H-NMR (400 MHz, DMSO-d₆): δ = 0.83 (t, 6H), 1.26 -1.43 (m, 4H), 1.89 (br. s, 2H), 2.56 (s, 3H; überlagert mit DMSO-Signal), 3.20 - 3.26 (m, 2H), 5.31 (s, 2H), 6.94 (t, 1H), 7.01 (d, 1H), 7.23 (t, 2H), 7.50 - 7.64 (m, 2H), 8.68 (d, 1H).

### Beispiel 254

### rac-N-(2-Amino-2-methylbutyl)-8-[(2,6-difluorbenzyl)oxy]-2-methylimidazo[1,2-a]pyridin-3-carboxamid

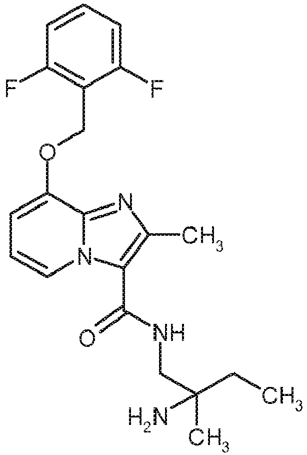

Darstellung und Reinigung der Titelverbindung erfolgte analog zu Beispiel 252. Ausgehend von 120 mg (0.38 mmol) 8-[(2,6-Difluorbenzyl)oxy]-2-methylimidazo[1,2-a]pyridin-3-carbonsäure Beispiel 3A und 58 mg (0.57 mmol) *rac*-2-Methylbutan-1,2-diamin erhielt man 98 mg (63% d. Th., Reinheit 98%) der Zielverbindung.
LC-MS (Methode 2): Rₜ = 0.58 min
MS (ESIpos): m/z = 403 (M+H)⁺
¹H-NMR (400 MHz, DMSO-d₆): δ = 0.86 (t, 3H), 0.97 (s, 3H), 1.28 - 1.39 (m, 2H), 1.40 - 1.49 (br s., 2H), 2.56 (s, 3H; überlagert mit DMSO-Signal), 3.14 - 3.27 (m, 2H), 5.30 (s, 2H), 6.94 (t, 1H), 7.01 (d, 1H), 7.19 - 7.27 (m, 2H), 7.54 - 7.69 (m, 2H), 8.65 (d, 1H).

### Beispiel 255

### N-[2-(tert.-Butylamino)ethyl]-8-[(2,6-difluorbenzyl)oxy]-2,6-dimethylimidazo[1,2-a]pyridin-3-carboxamid

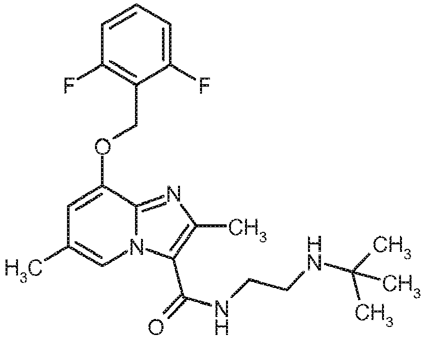

70 mg (0.21 mmol) 8-[(2,6-Difluorbenzyl)oxy]-2,6-dimefhylimidazo[1,2-a]pyridin-3-carbonsäure Beispiel 21A, 71 mg (0.22 mmol) TBTU und 128 mg (1.26 mmol) 4-Methylmorpholin wurden in DMF (0.74 ml) vorgelegt, mit 44 mg (0.23 mmol) *N-*tert.-Butylethan-1,2-diamindihydrochlorid versetzt und die Reaktionsmischung wurde über Nacht bei RT gerührt. Es wurden nochmals 22 mg (0.12 mmol) *N*-tert.-Butylethan-1,2-diamindihydrochlorid und 21 mg (0.21 mmol) 4-Methylmorpholin zugegeben und 1.5 h bei RT gerührt. Das Gemisch wurde mit einigen Tropfen Wasser/TFA verdünnt, mittels präparativer RP-HPLC (Acetonitril/Wasser unter zusatz von 0.1% TFA) gereinigt und die Produktfraktionen wurden eingeengt. Der Rückstand wurde mit gesättigter, wässriger Natriumhydrogencarbonat-Lösung versetzt und dreimal mit Dichlormethan extrahiert. Die vereinigten organischen Phasen wurden über Natriumsulfat getrocknet, abfiltriert und eingeengt. Man erhielt 78 mg (86% d. Th., Reinheit 96%) der Zielverbindung.
LC-MS (Methode 2): Rₜ = 0.66 min
MS (ESIpos): m/z = 431 (M+H)⁺
¹H-NMR (400 MHz, DMSO-d₆): δ = 1.18 (br. s, 9H), 2.32 (s, 3H), 2.52 (s, 3H, verdeckt durch DMSO-Signal), 2.70-3.12 (br. s, 2H), 3.38-3.62 (m, 2H), 5.29 (s, 2H), 6.92 (s, 1H), 7.20-7.27 (m, 2H), 7.59 (quintett, 1H), 7.74 (br. s, 1H), 8.34 (br. s, 1H), 8.53 (s, 1H).

### Beispiel 256

### ent-N-(2-Amino-2-methylpropyl)-8-[1-(2,6-difluorphenyl)ethoxy]-2,6-dimethylimidazo[1,2-a]pyridin-3-carboxamid (Enantiomer B)

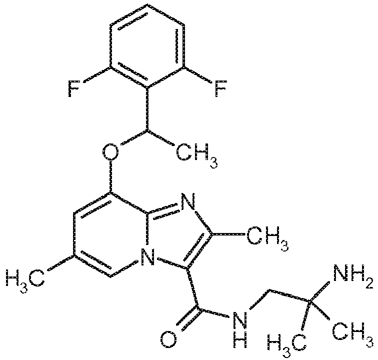

84 mg (0.24 mmol) *ent*-8-[1-(2,6-Difluorphenyl)ethoxy]-2,6-dimethylimidazo[1,2-a]pyridin-3-carbonsäure Beispiel 257A wurde in DMF (1.54 ml) vorgelegt, mit 117 mg (0.36 mmol) TBTU, 98 mg (0.97 mmol) 4-Methylmorpholin und 43 mg (0.49 mmol) 2-Methylpropan-1,2-diamin versetzt und die Reaktionsmischung über Nacht bei RT gerührt. Anschließend wurde mittels präparativer RP-HPLC (Acetonitril/Wasser-Gradient unter Zusatz von 0.1% TFA) gereinigt und die Produktfraktion eingeengt. Der Rückstand wurde in Dichlormethan aufgenommen und zweimal mit gesättigter, wässriger Natriumhydrogencarbonat-Lösung gewaschen. Die vereinigten organischen Phasen wurden über Natriumsulfat getrocknet, abfiltriert, und eingeengt. Der Rückstand wurde im Hochvakuum getrocknet und anschließend lyophilisiert. Man erhielt 85 mg (84% d. Th., Reinheit 99%) der Zielverbindung.
LC-MS (Methode 13): Rₜ = 1.48 min
MS (ESIpos): m/z = 417 (M+H)⁺
¹H-NMR (400 MHz, DMSO-d₆): δ = 1.05 (s, 6H), 1.61 - 1.74 (m, 2H), 1.78 (d, 3H), 2.18 (s, 3H), 2.57 (s, 3H), 3.19 (d, 2H), 6.20 (q, 1H), 6.56 (s, 1H), 7.06 - 7.14 (m, 2H), 7.37 - 7.46 (m, 1H), 7.69 (t, 1H), 8.40 (s, 1H).

### Beispiel 257

### N-(2-Amino-2-methylpentyl)-8-[1-(2,6-difluorphenyl)ethoxy]-2,6-dimethylimidazo[1,2-a]pyridin-3-carboxamid

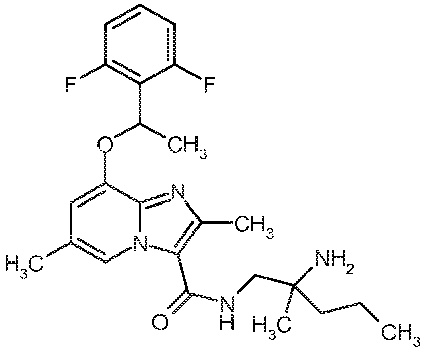

40 mg (0.12 mmol) *ent*-8-[1-(2,6-Difluorphenyl)ethoxy]-2,6-dimethylimidazo[1,2-a]pyridin-3-carbonsäure Beispiel 257A, 45 mg (0.14 mmol) TBTU und 47 mg (0.46 mmol) 4-Methylmorpholin in DMF (0.77 ml) wurden mit 16 mg (0.14 mmol) *rac*-2-Methylpentan-1,2-diamin versetzt und die Reaktionsmischung über Nacht bei RT gerührt. Das Gemisch wurde mit einigen Tropfen Wasser/TFA versetzt und mittels präparativer RP-HPLC (Acetonitril/Wasser-Gradient unter Zusatz von 0.1 % TFA) gereinigt. Die Produktfraktion wurde eingeengt. Der Rückstand wurde in Dichlormethan aufgenommen und mit gesättigter, wässriger Natriumhydrogencarbonat-Lösung gewaschen. Die wässrige Phase wurde dreimal mit Dichlormethan extrahiert und die vereinigten organischen Phasen wurden über Natriumsulfat getrocknet, abfiltriert, eingeengt, im Hochvakuum getrocknet und anschließend lyophilisiert. Man erhielt 27.4 mg (52% d. Th., Reinheit 97%) der Zielverbindung.
LC-MS (Methode 2): Rₜ = 0.71 min
MS (ESIpos): m/z = 445 (M+H)⁺
¹H-NMR (400 MHz, DMSO-d₆): δ = 0.87 (t, 3H), 0.98 (s, 3H), 1.22-1.39 (m, 4H), 1.48 (br. s, 2H), 1.78 (d, 3H), 2.19 (s, 3H), 2.57 (s, 3H), 3.13-3.26 (m, 2H), 6.20 (q, 1H), 6.56 (s, 1H), 7.06-7.14 (m, 2H), 7.37-7.44 (m, 1H), 7.58-7.64 (m, 1H), 8.41 (s, 1H).

### Beispiel 258

### rac-N-(2-Amino-2,4-dimethylpentyl)-2,6-dimethyl-8-[4,4,4-trifluor-3-(trifluormethyl)butoxy]-imidazo[1,2-a]pyridin-3-carboxamid

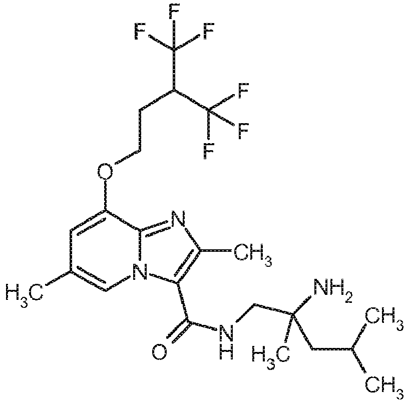

Zu 10 mg (0.08 mmol) *rac*-2,4-Dimethylpentan-1,2-diamin wurden 31 mg (0.08 mmol) 2,6-Dimethyl-8-[4,4,4-trifluor-3-(trifluormethyl)butoxy]imidazo[1,2-a]pyridin-3-carbon-säure Beispiel 259A, gelöst in 0.3 ml DMF, 40 mg (0.104 mmol) HATU, gelöst in 0.3 ml DMF, und anschließend 16 mg (0.16 mmol) 4-Methylmorpholin gegeben. Es wurde bei RT über Nacht geschüttelt. Die Zielverbindung wurde per präparativer HPLC (Methode 12) eingeengt. Es wurden 10 mg (25% d. Th.) erhalten.
LC-MS (Methode 8): Rₜ = 0.78 min
MS (ESpos): m/z = 497 (M+H)⁺

### Beispiel 259

### rac-N-(2-Amino-2-cyclopropylpropyl)-8-[(2,6-difluorbenzyl)oxy]-2,6-dimethylimidazo[1,2-a]pyridin-3-carboxamid

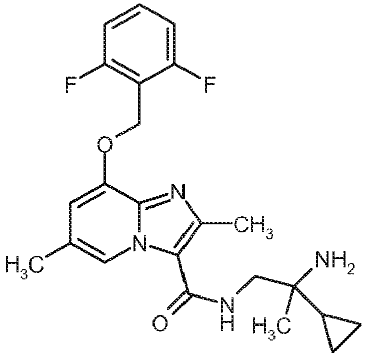

160 mg (0.48 mmol) 8-[(2,6-Difluorbenzyl)oxy]-2,6-dimethylimidazo[1,2-a]pyridin-3-carbonsäure Beispiel 21A, 162 mg (0.51 mmol) TBTU und 292 mg (2.89 mmol) 4-Methylmorpholin wurden in DMF (1.7 ml) vorgelegt und bei 0°C mit 99 mg (0.53 mmol) *rac*-2-Cylopropylpropan-1,2-diamindihydrochlorid versetzt. Die Reaktionsmischung wurde auf Raumtemperatur kommend über Nacht bei RT gerührt. Es wurden nochmals 50 mg (0.27 mmol) 2-Cyclopropylpropan-1,2-diamindihydrochlorid und 49 mg (0.48 mmol) 4-Methylmorpholin zugegeben und 1.5 h bei RT gerührt. Das Gemisch wurde mit einigen Tropfen Wasser/TFA verdünnt und mittels präp. RP-HPLC (Acetonitril/Wasser-Gradient unter Zusatz von 0.1% TFA) gereinigt. Die Produktfraktionen wurden eingeengt. Der Rückstand wurde mit gesättigter, wässriger Natriumhydrogencarbonat-Lösung versetzt und dreimal mit Dichlormethan extrahiert. Die vereinigten organischen Phasen wurden über Natriumsulfat getrocknet, abfiltriert und eingeengt. Man erhielt 156 mg (76% d. Th.) der Zielverbindung.
LC-MS (Methode 2): Rₜ = 0.63 min
MS (ESIpos): m/z = 429 (M+H)⁺
¹H-NMR (400 MHz, DMSO-d₆): δ = 0.18-0.38 (m, 4H),0.79-0.88 (m, 1H), 0.97 (s, 3H), 1.13-1.36 (m, 2H), 2.31 (s, 3H), 2.54 (s, 3H, verdeckt durch DMSO-Signal), 3.20-3.3.26 (m, 1H), 3.30-3.38 (m, 1H), 5.29 (s, 2H), 6.91 (s, 1H), 7.19-7.27 (m, 2H), 7.54-7.63 (m, 2H), 8.50 (s, 1H).

### Beispiel 260

### ent-N-(2-Amino-2-cyclopropylpropyl)-8-[(2,6-difluorbenzyl)oxy]-2,6-dimethylimidazo[1,2-a]pyridin-3-carboxamid (Enantiomer A)

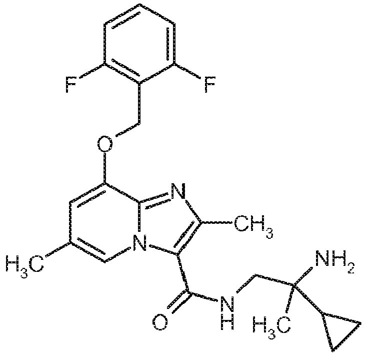

140 mg Beispiel 259 wurden durch präparative Trennung an chiraler Phase in die Enantiomere getrennt [Säule: Daicel Chiralpak AD-H, 5 µm, 250 x 20 mm, Eluent: 50% iso-Hexan, 50% Isopropanol + 0.2% Diethylamin, Fluß: 17 ml/min; 40°C, Detektion: 210 nm].
Enantiomer A: Ausbeute: 48 mg (100% ee)
Rt= 8.53 min [Daicel Chiralpak AD-H, 5µm, 250 x 4.6 mm; Eluent: 50% iso-Hexan, 50% Ethanol + 0.2% Diethylamin; Fluss 1.0 ml/min; 40°C; Detektion: 220 nm].
Spezifischer Drehwert [a] (365 nm, 20.5°C) = -8.4° (c = 0.005 g/ml, Acetonitril)

### Beispiel 261

### ent-N-(2-Amino-2-cyclopropylpropyl)-8-[(2,6-difluorbenzyl)oxy]-2,6-dimethylimidazo[1,2-a]pyridin-3-carboxamid (Enantiomer B)

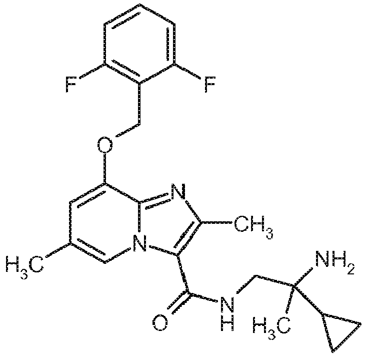

140 mg von Beispiel 259 wurden durch präparative Trennung an chiraler Phase in die Enantiomere getrennt [Säule: Daicel Chiralpak AD-H, 5 µm, 250 x 20 mm, Eluent: 50% iso-Hexan, 50% Isopropanol + 0.2% Diethylamin; Fluß: 17 ml/min; 40°C, Detektion: 210 nm].
Enantiomer B: Ausbeute: 38 mg (90% ee)
Rₜ = 9.12 min [Daicel Chiralpak AD-H, 5µm, 250 x 4.6 mm; Eluent: 50% iso-Hexan, 50% Ethanol + 0.2% Diethylamin; Fluss 1.0 ml/min; 40°C; Detektion: 220 nm].

### Beispiel 262

### rac-N-[2-Amino-4-(benzyloxy)-2-methylbutyl]-8-[(2,6-difluorbenzyl)oxy]-2,6-dimethylimidazo[1,2-a]pyridin-3-carboxamid

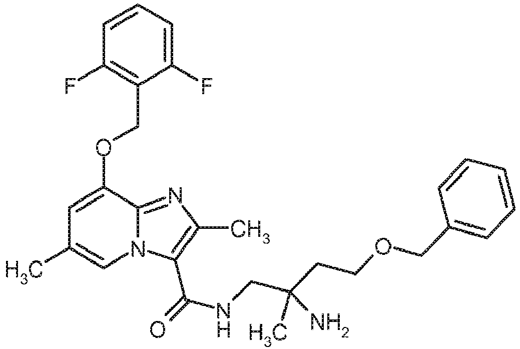

400 mg (1.2 mmol) 8-[(2,6-Difluorbenzyl)oxy]-2,6-dimethylimidazo[1,2-a]pyridin-3-carbonsäure Beispiel 21A wurden mit 503 mg (1.32 mmol) HATU und 1.05 ml (6.0 mmol) *N,N* Diisopropylethylamin in DMF (7.7 ml) versetzt und 20 min bei RT gerührt. 300 mg (1.38 mmol) *rac*-4-(Benzyloxy)-2-methylbutan-1,2-diamin Beispiel 261A wurden in 2 ml DMF gelöst und bei 0°C zur Reaktionslösung zugegeben. Es wurde 1 h bei 0°C gerührt und dann mit Wasser/TFA verdünnt und mittels präparativer RP-HPLC gereinigt (Acetonitril/Wasser-Gradient unter Zusatz von 0.1%ige TFA). Man erhielt 600 mg (88% d. Th., Reinheit 93%) der Zielverbindung.
LC-MS (Methode 2): Rₜ = 0.78 min
MS (ESIpos): m/z = 523 (M+H)⁺
¹H-NMR (400 MHz, DMSO-d₆): δ = 1.30 (s, 3H), 1.87-2.01 (m, 2H), 2.36 (s, 3H), 2.56 (s, 3H), 3.47 - 3.70 (m, 4H), 4.50 (s, 2H), 5.34 (s, 2H), 7.12 - 7.38 (m, 8H), 7.55 - 7.64 (m, 1H), 7.73 (br. s, 2H), 8.08 (br. s, 1H), 8.55 (s, 1H).

Die in Tabelle 14 gezeigten Beispiele wurden in Analogie zu Beispiel 262 hergestellt, indem die ensprechenden Carbonsäuren (Beispiel 3A und 21A) mit den entsprechenden wie zuvor beschrieben hergestellten oder kommerziell erhältlichen Aminen (1.05-2.5 Äquivalente) und *N,N* Diisopropylethylamin (3-6 Äquivalente) unter den in der allgemeinen Arbeitsvorschrift 3 beschriebenen Reaktionsbedingungen umgesetzt wurden.

Beispielhafte Aufarbeitung der Reaktionsmischung: Die Reaktionslösung wurde mit Wasser versetzt und dreimal mit Essigsäureethylester oder Dichlormethan extrahiert. Die vereinten organischen Phasen wurden über Natriumsulfat getrocknet, filtriert, eingeengt und mittels präparativer HPLC (RP18 Säule, Laufmittel: Acetonitril/Wasser-Gradient unter Zusatz von 0.1% TFA) gereinigt. Alternativ wurde der Niederschlag oder das Reaktionsgemisch direkt per präparativer HPLC (RP18 Säule, Laufmittel: Acetonitril/Wasser-Gradient unter Zusatz von 0.1% TFA) weiter aufgereinigt und über Nacht im Hochvakuum getrocknet. Gegebenenfalls wurden die Produktfraktionen in Essigsäureethylester oder Dichlormethan aufgenommen und zweimal mit gesättigter, wässriger Natriumhydrogencarbonatlösung gewaschen. Die wässrige Phase wurde zweimal mit Essigsäureethylester oder Dichlormethan extrahiert und die vereinigten organischen Phasen wurden über Natriumsulfat getrocknet, filtriert und eingeengt.

**Tabelle 14:**

| **Beispiel** | **IUPAC-Name / Struktur (Ausbeute)** | **Analytische Daten** |
|---|---|---|
| **263** | *rac-N*-[2-Amino-3-(benzyloxy)-2-methylpropyl]-8-[(2,6-difluorbenzyl)oxy]-2-methylimidazo[1,2-a]pyridin-3-carboxamid | LC-MS (Methode 2): Rₜ = 0.74 min |
| | | MS (ESpos): m/z = 495.3 (M+H)⁺ |
| | 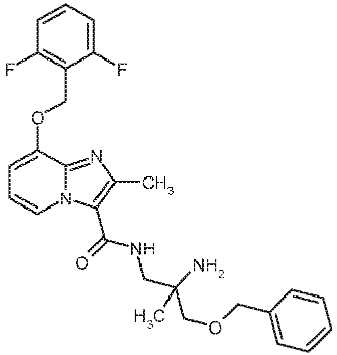 | ¹H-NMR (400 MHz, DMSO-d₆): δ = 1.03 (s, 3 H), 1.66 (br. s, 2 H), 2.49 (s, 3 H), 3.28 - 3.38 (m, 2 H; überlagert mit Wasser-Signal), 4.51 (s, 2 H), 5.30 (s, 2 H), 6.92 (t, 1 H), 7.01 (d, 1 H), 7.19 - 7.37 (m, 7 H), 7.50 - 7.67 (m, 2 H), 8.69 (d, 1 H). |
| | (81% d. Th.) | |
| **264** | *rac-N*-[2-Amino-4-(benzyloxy)-2-methylbutyl]-8-[(2,6-difluorbenzyl)oxy]-2-methylimidazo[1,2-a]pyridin-3-carboxamid | LC-MS (Methode 2): Rₜ = 0.77 min |
| | | MS (ESpos): m/z = 509.1 (M+H)⁺ |
| | 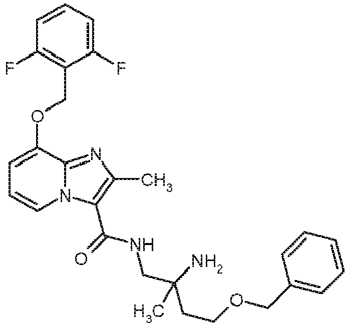 | ¹H-NMR (400 MHz, DMSO-d₆): δ = 1.11 (s, 3 H), 1.74 (t, 2 H), 2.51 (s, 3 H; überlagert mit DMSO-Peak), 3.28 - 3.38 (m, 2 H; überlagert mit Wasser-Signal), 3.59 - 3.67 (m, 2 H), 4.47 (s, 2 H), 5.31 (s, 2 H), 6.93 (t, 1 H), 7.02 (d, 1 H), 7.19 - 7.38 (m, 7 H), 7.54 - 7.71 (m, 2 H), 8.68 (d, 1 H). |
| | (81% d. Th.) | |
| **265** | *rac-N-*[2-Amino-3-(benzyloxy)-2-methylpropyl]-8-[(2,6-difluorbenzyl)oxy]-2,6-dimethylimidazo[1,2-a]pyridin-3-carboxamid | LC-MS (Methode 2): Rₜ = 0.80 min |
| | | MS (ESpos): m/z = 509.1 (M+H)⁺ |
| | 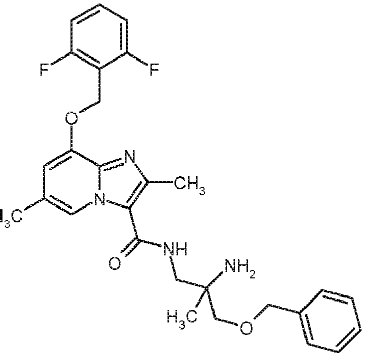 | ¹H-NMR (400 MHz, DMSO-d₆): δ = 1.05 (s, 3 H), 2.30 (s, 3 H), 2.49 (s, 3 H), 3.30 - 3.38 (m, 2 H; überlagert mit Wasser-Signal), 4.52 (s, 2 H), 5.30 (s, 2 H), 6.91 (s, 1 H). 7.19 - 7.38 (m, 8 H), 7.52 - 7.65 (m, 2 H), 8.51 (s, 1 H). |
| | (74% d. Th.) | |

### Beispiel 266

### ent-N-[2-Amino-4-(benzyloxy)-2-methylbutyl]-8-[(2,6-difluorbenzyl)oxy]-2-methylimidazo[1,2-a]pyridin-3-carboxamid (Enantiomer A)

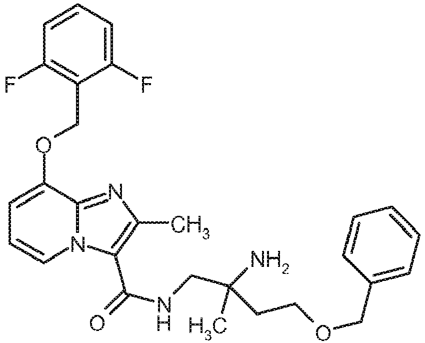

637 mg Beispiel 264 wurden durch präparative Trennung an chiraler Phase in die Enantiomere getrennt [Säule: Daicel Chiralcel OD-H, 5 µm, 250 x 20 mm, Eluent: 100% Ethanol + 0.2% Diethylamin, Fluß: 15 ml/min; 40°C, Detektion: 220 nm].
Enantiomer A: Ausbeute: 267 mg (99% ee)
Rₜ = 5.45 min [Daicel Chiralcel OD-H, 5 µm, 250 x 4.6 mm; Eluent: 100% Ethanol + 0.2% Diethylamin; Fluss 1.0 ml/min; 40°C; Detektion: 220 nm].

### Beispiel 267

### ent-N-[2-Amino-4-(benzyloxy)-2-methylbutyl]-8-[(2,6-difluorbenzyl)oxy]-2-methylimidazo[1,2-a]pyridin-3-carboxamid (Enantiomer B)

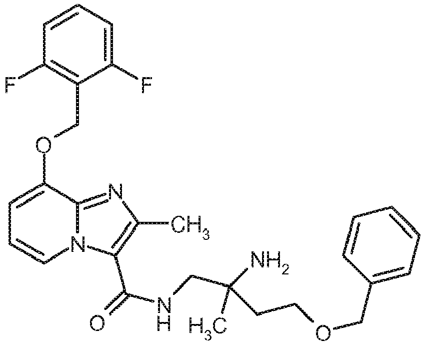

637 mg Beispiel 264 wurden durch präparative Trennung an chiraler Phase in die Enantiomere getrennt [Säule: Daicel Chiralcel OD-H, 5 µm, 250 x 20 mm, Eluent: 100% Ethanol + 0.2% Diethylamin, Fluß: 15 ml/min; 40°C, Detektion: 220 nm].
Enantiomer B: Ausbeute: 292 mg (99% ee)
Rt = 7.10 min [Daicel Chiralcel OD-H, 5 µm, 250 x 4.6 mm; Eluent: 100% Ethanol + 0.2% Diethylamin; Fluss 1.0 ml/min; 40°C; Detektion: 220 nm].

### Beispiel 268

### ent-N-(2-Amino-3-hydroxy-2-methylpropyl)-8-[(2,6-difluorbenzyl)oxy]-2,6-dimethylimidazo[1,2-a]pyridin-3-carboxamid (Enantiomer A)

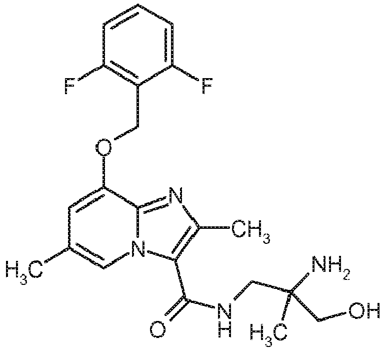

460 mg Beispiel 265 wurden durch präparative Trennung an chiraler Phase in die Enantiomere getrennt [Säule: Daicel Chiralcel OZ-H, 5 µm, 250 x 20 mm, Eluent: 100% Ethanol + 0.2% Diethylamin, Fluß: 15 ml/min; 40°C, Detektion: 220 nm]. Die Produktfraktionen wurden auf Trockeneis aufgefangen, vereint, am Rotationsverdampfer (30°C Badtemperatur) bis auf ca. 15 ml Restvolumen eingeengt und mit ca. 60 ml Wasser versetzt. Die erhaltene Lösung wurde eingefroren und lyophilisiert.
Ausbeute des Zwischenproduktes *ent-N*-[(2R)-2-Amino-3-(benzyloxy)-2-methylpropyl]-8-[(2,6-difluorbenzyl)oxy]-2,6-dimethylimidazo[1,2-a]pyridin-3-carboxamid (Enantiomer A):
183 mg (99% ee).
Rₜ = 6.61 min [Daicel Chiralcel OZ-H, 5 µm, 250 x 4.6 mm; Eluent: 100% Ethanol + 0.2% Diethylamin; Fluss 1.0 ml/min; 40°C; Detektion: 235 nm].

183 mg (0.36 mmol) *ent-N-*[(2R)-2-Amino-3-(benzyloxy)-2-methylpropyl]-8-[(2,6-difluorbenzyl)oxy]-2,6-dimethylimidazo[1,2-a]pyridin-3-carboxamid (Enantiomer A) wurden unter Argon in 3.7 ml Ethanol vorgelegt und mit 38.3 mg Palladium auf Aktivohle (10%ig) und 1.09 ml (10.8 mmol) Cyclohexen versetzt. Das Reaktionsgemisch wurde 6 h unter Rückfluss gerührt. Das Reaktionsgemisch wurde direkt auf Celite aufgezogen und mittels Kieselgelchromatographie gereinigt (Laufmittel: Dichlormethan/Methanol 10/1, Dichlormethan/2N Ammoniak in Methanol 10/1). Es wurden 80 mg der Zielverbindung (52% d. Th.; Reinheit 98%) erhalten.
LC-MS (Methode 2): Rₜ = 0.61 min
MS (ESIpos): m/z = 419 (M+H)⁺
¹H-NMR (400 MHz, DMSO-d₆): δ = 0.97 (s, 3H), 1.92 (br. s, 2H), 2.31 (s, 3H), 2.54 (s, 3H, verdeckt durch DMSO-Signal), 3.15 - 3.32 (m, 4H), 4.73 - 4.82 (m, 1H), 5.28 (s, 2H), 6.92 (s, 1 H), 7.22 (t, 2H), 7.53 - 7.66 (m, 2H), 8.51 (s, 1H).

### Beispiel 269

### ent-N-(2-Amino-3-hydroxy-2-methylpropyl)-8-[(2,6-difluorbenzyl)oxy]-2,6-dimethylimidazo[1,2-a]pyridin-3-carboxamid (Enantiomer B)

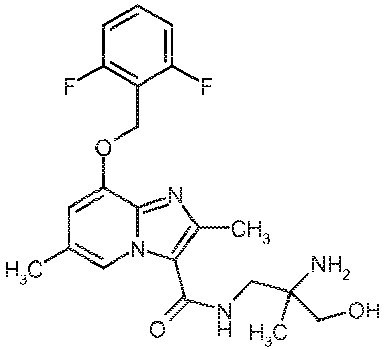

460 mg Beispiel 265 wurden durch präparative Trennung an chiraler Phase in die Enantiomere getrennt [Säule: Daicel Chiralcel OZ-H, 5 µm, 250 x 20 mm, Eluent: 100% Ethanol + 0.2% Diethylamin, Fluß: 15 ml/min; 40°C, Detektion: 220 nm]. Die Produktfraktionen wurden auf Trockeneis aufgefangen, vereint, am Rotationsverdampfer (30°C Badtemperatur) bis auf ca. 15 ml Restvolumen eingeengt und mit ca. 60 ml Wasser versetzt. Die erhaltene Lösung wurde eingefroren und lyophilisiert.
Ausbeute des Zwischenproduktes *ent-N*-[(2R)-2-Amino-3-(benzyloxy)-2-methylpropyl]-8-[(2,6-difluorbenzyl)oxy]-2,6-dimethylimidazo[1,2-a]pyridin-3-carboxamid (Enantiomer B):
189 mg (99% ee).
Rt = 8.47 min [Daicel Chiralcel OZ-H, 5 µm, 250 x 4.6 mm; Eluent: 100% Ethanol + 0.2% Diethylamin; Fluss 1.0 ml/min; 40°C; Detektion: 235 nm].

189 mg (0.37 mmol) *ent-N*-[(2R)-2-Amino-3-(benzyloxy)-2-methylpropyl]-8-[(2,6-difluorbenzyl)oxy]-2,6-dimethylimidazo[1,2-a]pyridin-3-carboxamid (Enantiomer B) wurden unter Argon in 3.8 ml Ethanol vorgelegt und mit 39.5 mg Palladium auf Aktivohle (10%ig) und 1.13 ml (11.2 mmol) Cyclohexen versetzt. Das Reaktionsgemisch wurde 12 h unter Rückfluss gerührt. Das Reaktionsgemisch wurde direkt auf Celite aufgezogen und mittels Kieselgelchromatographie gereinigt (Laufmittel: Dichlormethan/Methanol 10/1, Dichlormethan/2N Ammoniak in Methanol 10/1). Es wurden 85 mg der Zielverbindung (54% d. Th.; Reinheit 98%) erhalten.
LC-MS (Methode 2): Rₜ = 0.52 min
MS (ESIpos): m/z = 419 (M+H)⁺
¹H-NMR (400 MHz, DMSO-d₆): δ = 0.97 (s, 3H), 1.83 (br. s, 2H), 2.30 (s, 3H), 2.54 (s, 3H, verdeckt durch DMSO-Signal), 3.15 - 3.32 (m, 4H), 4.73 - 4.82 (m, 1H), 5.28 (s, 2H), 6.91 (s, 1H), 7.22 (t, 2H), 7.53 - 7.66 (m, 2H), 8.51 (s, 1H).

### Beispiel 270

### rac-N-(2-Amino-4-hydroxy-2-methylbutyl)-8-[(2,6-difluorbenzyl)oxy]-2,6-dimethylimidazo[1,2-a]pyridin-3-carboxamid

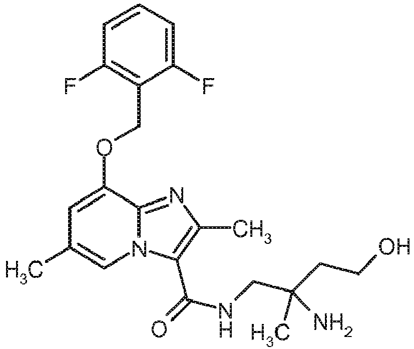

295 mg (0.52 mmol) *rac-N*-[2-Amino-4-(benzyloxy)-2-methylbutyl]-8-[(2,6-difluorbenzyl)oxy]-2,6-dimethylimidazo[1,2-a]pyridin-3-carboxamid Beispiel 262 wurden unter Argon in 5.4 ml Ethanol vorgelegt, mit 56 mg Pd/Kohle (10%ig) versetzt und 6.5 h unter Normaldruck bei Raumtemperatur hydriert. Die Reaktionsmischung wurde über Nacht unter Wasserstoff belassen. Das Reaktionsgemisch wurde auf Kieselgel gezogen, eingeengt und mittels Kieselgelchromatographie gereinigt (Laufmittel: Dichlormethan/2N Ammoniak in Methanol 10/1 isokratisch). Es wurden 95 mg der Zielverbindung (42% d. Th.) erhalten.
LC-MS (Methode 2): Rₜ = 0.70 min
MS (ESIpos): m/z = 433 (M+H)⁺
¹H-NMR (400 MHz, DMSO-d₆): δ = 1.07 (s, 3H), 1.49 - 1.68 (m, 2H), 2.31 (s, 3H), 2.54 (s, 3H, verdeckt durch DMSO-Signal), 3.26 (br. s, 2H), 3.51 - 3.68 (m, 2H), 5.28 (s, 2H), 6.91 (s, 1H), 7.22 (t, 2H), 7.53 - 7.70 (m, 2H), 8.50 (s, 1H).

### Beispiel 271

### ent-N-(2-Amino-4-hydroxy-2-methylbutyl)-8-[(2,6-difluorbenzyl)oxy]-2,6-dimethylimidazo[1,2-a]pyridin-3-carboxamid (Enantiomer A)

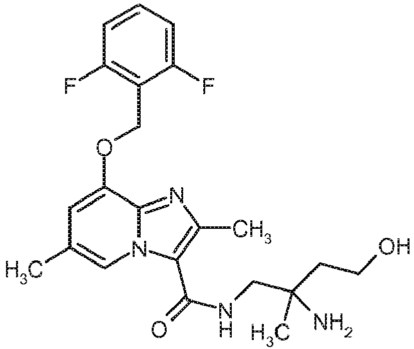

187 mg Beispiel 270 wurden durch präparative Trennung an chiraler Phase in die Enantiomere getrennt [Säule: Daicel Chiralcel OZ-H, 5 µm, 250 x 20 mm; Eluent: 50% iso-Hexan, 50% Ethanol + 0.2% Diethylamin; Fluß: 15 ml/min; 40°C; Detektion: 220 nm]. Die Produktfraktionen wurden auf Trockeneis aufgefangen, vereint, am Rotationsverdampfer (30°C Badtemperatur) bis auf ca. 15 ml Restvolumen eingeengt und mit ca. 60 ml Wasser versetzt. Die erhaltene Lösung wurde eingefroren und lyophilisiert.
Enantiomer A: Ausbeute: 63 mg (99% ee)
Rₜ = 5.57 min [Daicel Chiralcel OZ-H, 5 µm, 250 x 4.6 mm; Eluent: 100% Ethanol + 0.2% Diethylamin; Fluss 1.0 ml/min; 40°C; Detektion: 235 nm].
Spezifischer Drehwert [a] (365 nm, 20.2°C) = -8.3° (c = 0.005 g/ml, Acetonitril)

### Beisipiel 272

### ent- N-(2-Amino-4-hydroxy-2-methylbutyl)-8-[(2,6-difluorbenzyl)oxy]-2-methylimidazo[1,2-a]pyridin-3-carboxamid (Enantiomer A)

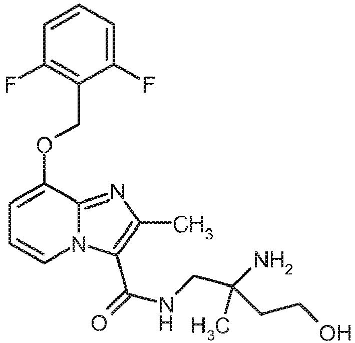

257 mg (0.51 mmol) *ent-N*-[2-Amino-4-(benzyloxy)-2-methylbutyl]-8-[(2,6-difluorbenzyl)oxy]-2-methylimidazo[1,2,a]pyridin-3-carboxamid (Enantiomer A) Beispiel 266 wurden unter Argon in 5.2 ml Ethanol vorgelegt und mit 54 mg Palladium auf Aktivkohle (10%ig) und 1.54 ml (15.2 mmol) Cyclohexen versetzt. Das Reaktionsgemisch wurde 6.5 h unter Rückfluss gerührt, dann auf Celite aufgezogen und mittels Kieselgelchromatographie gereinigt (Laufmittel: Dichlormethan/2N Ammoniak in Methanol 20/1). Es wurden 122 mg der Zielverbindung (56% d. Th.; Reinheit 98%) erhalten.
LC-MS (Methode 2): Rₜ = 0.60 min
MS (ESIpos): m/z = 419 (M+H)⁺
¹H-NMR (400 MHz, DMSO-d₆): δ = 1.03 (s, 3H), 1.46 - 1.62 (m, 2H), 1.71 (br. s, 2H), 2.55 (s, 3H, verdeckt durch DMSO-Signal), 3.19 - 3.28 (m, 2H), 3.52 - 3.68 (m, 2H), 4.75 (br. s, 1H), 5.30 (s, 2H), 6.92 (t, 1H), 7.00 (d, 1H), 7.22 (t, 2H), 7.59 (quintett, 1H), 7.63 - 7.73 (m, 1H), 8.67 (d, 1H).

### Beispiel 273

### rac-N-(2-Amino-3-hydroxy-2-methylpropyl)-8-[(2,6-difluorbenzyl)oxy]-2-methylimidazo[1,2-a]pyridin-3-carboxamid

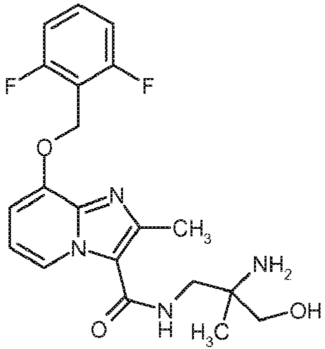

800 mg (1.57 mmol) *rac-N*-[2-Amino-3-(benzyloxy)-2-methylpropyl]-8-[(2,6-difluorbenzyl)oxy]-2-methylimidazo[1,2-a]pyridin-3-carboxamid Beispiel 263 wurden unter Argon in 16.2 ml Ethanol vorgelegt und mit 167 mg Palladium auf Aktivkohle (10%ig) und 4.77 ml (47.1 mmol) Cyclohexen versetzt. Das Reaktionsgemisch wurde 18 h unter Rückfluss gerührt und dann auf Celite aufgezogen und mittels Kieselgelchromatographie gereinigt (Laufmittel: Dichlormethan/Methanol 10/1, Dichlormethan/2N Ammoniak in Methanol 10/1). Es wurden 366 mg der Zielverbindung (56% d. Th.; Reinheit 97%) erhalten.
LC-MS (Methode 2): Rₜ = 0.54 min
MS (ESIpos): m/z = 405 (M+H)⁺
¹H-NMR (400 MHz, DMSO-d₆): δ = 0.96 (s, 3H), 1.79 (br. s, 2H), 2.56 (s, 3H, überlagert mit DMSO-Signal), 3.19 - 3.33 (m, 4H; überlagert mit Wasser-Signal), 4.74 - 4.80 (m, 1H), 5.30 (s, 2H), 6.92 (t, 1H), 7.01 (d, 1H), 7.22 (t, 2H), 7.55 - 7.69 (m, 2H), 8.69 (d, 1H).

### Beispiel 274

### ent-N-(2-Amino-2-methylbutyl)-2,6-dimethyl-8-[(2,3,6-trifluorbenzyl)oxy]imidazo[1,2-a]pyridin-3-carboxamid (Enantiomer A)

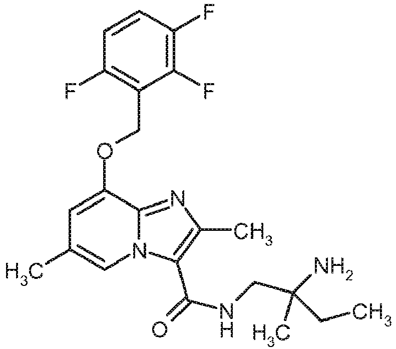

383 mg (0.56 mmol) *ent*-Benzyl-{1-[({2,6-dimethyl-8-[(2,3,6-trifluorbenzyl)oxy]imidazo[1,2-a]pyridin-3-yl}carbonyl)amino]-2-methylbutan-2-yl}carbamat-Trifluoracetat (Enantiomer A) Beispiel 278A wurden unter Argon in 5.75 ml Ethanol vorgelegt und mit 39 mg Palladium(II)hydroxid auf Aktivkohle (20%ig) versetzt. Das Reaktionsgemisch wurde 2 h unter Normaldruck bei RT hydriert, auf Celite aufgezogen und mittels Kieselgelchromatographie gereinigt (Laufmittel: Dichlormethan/2 N Ammoniak in Methanol = 40/1). Es wurden 208 mg der Zielverbindung (85% d. Th.) erhalten.
LC-MS (Methode 2): Rₜ = 0.62 min
MS (ESIpos): m/z = 435 (M+H)⁺
¹H-NMR (400 MHz, DMSO-d₆): δ = 0.86 (t, 3H), 0.97 (s, 3H), 1.31 - 1.39 (m, 2H), 1.42 (br. s, 2H), 2.31 (s, 3H), 2.53 (s, 3H, überlagert mit DMSO-Signal), 3.14 - 3.26 (m, 2H), 5.34 (s, 2H), 6.92 (s, 1H), 7.25 - 7.34 (m, 1H), 7.58 - 7.72 (m, 2H), 8.49 (s, 1H).

Eine Einkristall-Röntgenstrukturanalyse bestätigte für dieses Enantiomer die S-Konfiguration.

### Beispiel 275

### ent-N-(2-Amino-2-methylbutyl)-2,6-dimethyl-8-[(2,3,6-trifluorbenzyl)oxy]imidazo[1,2-a]pyridin-3-carboxamid (Enantiomer B)

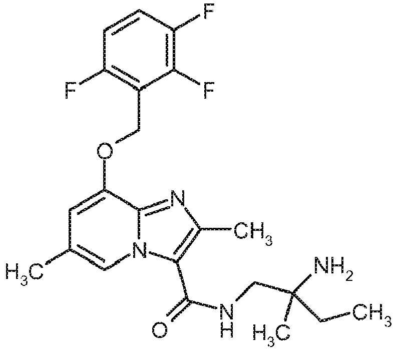

200 mg (0.29 mmol) *ent*-Benzyl-{1-[({2,6-dimethyl-8-[(2,3,6-trifluorbenzyl)oxy]imidazo[1,2-a]pyridin-3-yl}carbonyl)amino]-2-methylbutan-2-yl}carbamat-Trifluoracetat (Enantiomer B) Beispiel 279A wurden unter Argon in 3.0 ml Ethanol vorgelegt und mit 10.3 mg Palladium(II)hydroxid auf Aktivkohle (20%ig) versetzt. Das Reaktionsgemisch wurde 6 h unter Normaldruck bei RT hydriert. Es wurden nochmals 10.3 mg Palladium(II)hydroxid auf Aktivohle (20%ig) hinzugegeben und 1 h unter Normaldruck bei RT hydriert. Das Reaktionsgemisch wurde auf Celite aufgezogen und mittels Kieselgelchromatographie gereinigt (Laufmittel: Dichlormethan/2 N Ammoniak in Methanol = 40/1). Es wurden 92 mg der Zielverbindung (72% d. Th.) erhalten.
LC-MS (Methode 2): Rₜ = 0.66 min
MS (ESIpos): m/z = 435 (M+H)⁺
¹H-NMR (400 MHz, DMSO-d₆): δ = 0.87 (t, 3H), 0.98 (s, 3H), 1.32 - 1.41 (m, 2H), 1.81 (br. s, 2H), 2.31 (s, 3H), 2.54 (s, 3H, überlagert mit DMSO-Signal), 3.15 - 3.28 (m, 2H), 5.34 (s, 2H), 6.92 (s, 1H), 7.25 - 7.34 (m, 1H), 7.59 - 7.73 (m, 2H), 8.49 (s, 1H).

### Beispiel 276

### N-(2-Amino-2-methylpropyl)-2,6-dimethyl-8-[(2,3,6-trifluorbenzyl)oxy]imidazo[1,2,a]pyridin-3-carboxamid

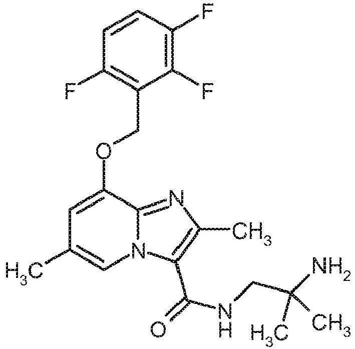

70 mg (0.20 mmol) 2,6-Dimethyl-8-[(2,3,6-trifluorbenzyl)oxy]imidazo[1,2-a]pyridin-3-carbonsäure Beispiel 265A wurden mit 84 mg (0.22 mmol) *O*-(7-Azabenzotriazol-1-yl)-*N*,*N*,*N*'*N*'-tetramethyluroniumhexafluorphosphat (HATU) und 77 mg (0.60 mmol) *N,N-*Diisopropylethylamin in 1.3 ml DMF gelöst und nach 20 min bei 0°C mit 19.4 mg (0.22 mmol) 2-Methylpropan-1,2-diamin versetzt. Es wurde 45 min bei 0°C gerührt und anschließend per präparativer HPLC (Methode: RP18 Säule, Laufmittel: Acetonitril/Wasser-Gradient unter Zusatz von 0.1% TFA) gereinigt. Die erhaltene Produktfraktion wurde in Dichlormethan aufgenommen und zweimal mit gesättigter, wässriger Natriumhydrogencarbonat-Lösung gewaschen. Die wässrige Phase wurde zweimal mit Dichlormethan extrahiert. Die vereinigten organischen Phasen wurden über Natriumsulfat getrocknet, filtiert, eingeengt und lyophilisiert. Es wurden 54 mg (64% d. Th.) der Titelverbindung erhalten.
LC-MS (Methode 2): Rₜ = 0.65 min
MS (ESpos): m/z = 421 (M+H)⁺
¹H-NMR (500 MHz, DMSO-d₆): δ = 1.05 (s, 6 H), 1.93 (br. s, 2 H), 2.30 (s, 3 H), 2.54 (s, 3 H; überlagert mit DMSO-Peak), 3.21 (d, 2 H), 5.32 (s, 2 H), 6.92 (s, 1 H), 7.27 - 7.32 (m, 1 H), 7.62 - 7.74 (m, 2 H), 8.48 (s, 1 H).

### Beispiel 277

### rac-N-(2-Amino-2-methylpentyl)-8-[(2,6-difluorbenzyl)oxy]-7-fluor-2-methylimidazo[1,2-a]pyridin-3-carboxamid

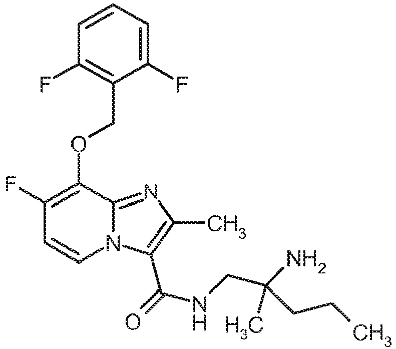

50 mg (0.15 mmol) 8-[(2,6-Difluorbenzyl)oxy]-7-fluor-2-methylimidazo[1,2-a]pyridin-3-carbonsäure Beispiel 270A, 62 mg (0.16 mmol) O-(7-Azabenzotriazol-1-yl)-N,N,N'N'-tetramethyluroniumhexafluorphosphat (HATU) und 58 mg (0.45 mmol) N,N-Diisopropylethylamin wurden in 1.0 ml DMF gelöst und nach 20 min bei 0°C mit 21.4 mg (0.18 mmol) 2-Methylpentan-1,2-diamin versetzt. Es wurde 1.5 h bei 0°C gerührt, mit Acetonitril/TFA versetzt und mittels präparative HPLC (Methode: RP18 Säule, Laufmittel: Acetonitril/Wasser-Gradient unter Zusatz von 0.1% TFA) gereinigt. Die erhaltene Produktfraktion wurde in Dichlormethan aufgenommen und mit gesättigter, wässriger Natriumhydrogencarbonat-Lösung gewaschen. Die wässrige Phase wurde zweimal mit Dichlormethan extrahiert. Die vereinigten organischen Phasen wurden über Natriumsulfat getrocknet, filtiert, eingeent und lyophilisiert. Es wurden 31 mg (45% d. Th.; Reinheit 94%) der Titelverbindung erhalten.
LC-MS (Methode 2): Rₜ = 0.77 min
MS (ESpos): m/z = 435 (M+H)⁺
¹H-NMR (400 MHz, DMSO-d₆): δ = 0.83 - 0.92 (m, 3 H), 1.02 (s, 3 H), 1.26 - 1.43 (m, 4 H), 1.87 (br. s, 2 H), 2.63 (s, 3 H), 3.15 - 3.30 (m, 2 H), 5.60 (s, 2 H), 6.97 - 7.06 (m, 1 H), 7.08 - 7.18 (m, 2 H), 7.51 (quintett, 1 H), 7.64 - 7.78 (m, 1 H), 8.77 (dd, 1 H).

### Beispiel 278

### ent-N-(2-Amino-2-methylpentyl)-8-[(2,6-difluorbenzyl)oxy]-6-methoxy-2-methylimidazo[1,2-a]pyridin-3-carboxamid (Enantiomer B)

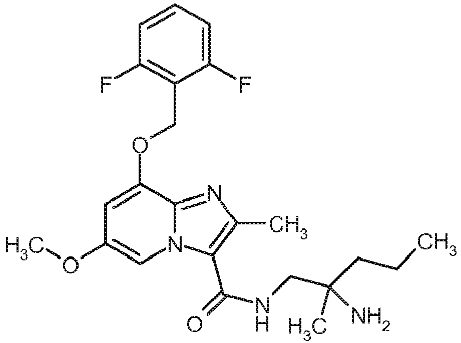

180 mg (0.26 mmol) *ent*-Benzyl-(1-[({8-[(2,6-difluorbenzyl)oxy]-6-methoxy-2-methylimidazo[1,2-a]pyridin-3-yl}carbonyl)amino]-2-methylpentan-2-yl}carbamat Trifluoracetat aus Beispiel 290A in 2.8 ml Ethanol wurden unter Argon mit 9 mg 20%igem Palladium(II)hydroxid auf Kohle versetzt und 2 h bei RT unter Normaldruck hydriert. Das Reaktionsgemisch wurde abfiltriert, mit Ethanol gewaschen und das Filtrat eingeengt. Der Rückstand wurde mittels Kieselgel-Chromatographie gereinigt (Laufmittel: Dichlormethan/2N Ammoniak in Methanol = 40/1). Die isolierte Produktfraktion wurde über präparative HPLC (RP18 Säule, Laufmittel: Acetonitril/Wasser-Gradient unter Zusatz von 0.1% TFA) getrennt. Die Produktfraktionen wurden am Rotationsverdampfer eingeengt. Der Rückstand wurde in Dichlormethan aufgenommen, zweimal mit gesättigter, wässriger Natriumhydrogencarbonat-Lösung gewaschen und die vereinigten wässrigen Phasen wurden zweimal mit Dichlormethan extrahiert. Die organische Phase wurde über Natriumsulfat getrocknet, abfiltriert, eingeengt und der Rückstand lyophilisiert. Es wurden 73 mg der Zielverbindung (63% d. Th.) erhalten.
LC-MS (Methode 2): Rₜ = 0.68 min
MS (ESpos): m/z = 447 (M+H)⁺
¹H-NMR (400 MHz, DMSO-d₆): δ = 0.87 (t, 3H), 0.99 (s, 3H), 1.23 - 1.41 (m, 4H), 2.53 (s, 3H), 3.14 - 3.26 (m, 2H), 3.79 (s, 3H), 5.30 (s, 2H), 6.87 (d, 1H), 7.24 (quin., 2H), 7.54 - 7.65 (m, 2H), 8.37 (d, 1H).

### Beispiel 279

### ent-N-(2-Amino-2-methylbutyl)-8-[(2,6-difluorbenzyl)oxy]-6-methoxy-2-methylimidazo[1,2-a]pyridin-3-carboxamid (Enantiomer A)

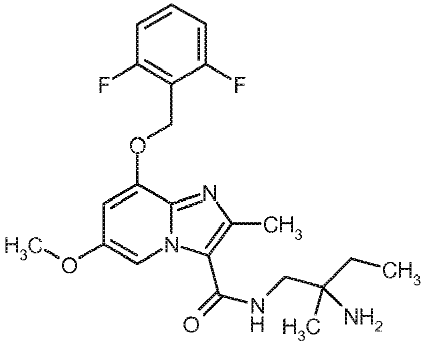

157 mg (0.23 mmol) *ent*-Benzyl-{1-[({8-[(2,6-difluorbenzyl)oxy]-6-methoxy-2-methylimidazo[1,2-a]pyridin-3-yl}carbonyl)amino]-2-methylbutan-2-yl)carbamat Trifluoracetat (Enantiomer A) aus Beispiel 291A in 2.5 ml Ethanol wurden unter Argon mit 8 mg 20%igem Palladium(II)hydroxid auf Kohle versetzt und 2 h bei RT unter Normaldruck hydriert. Das Reaktionsgemisch wurde über einen Milliporfilter abfiltriert, mit Ethanol gewaschen und das Filtrat eingeengt. Der Rückstand wurde mittels Kieselgel-Chromatographie gereinigt (Laufmittel: Dichlormethan/2N Ammoniak in Methanol = 40/1). Es wurden 90 mg der Zielverbindung (90% d. Th.) erhalten.
LC-MS (Methode 2): Rₜ = 0.63 min
MS (ESpos): m/z = 433 (M+H)⁺
¹H-NMR (400 MHz, DMSO-d₆): δ = 0.87 (t, 3H), 0.97 (s, 3H), 1.31-1.39 (m, 2H), 1.40 -1.49 (m, 2H), 2.53 (s, 3H), 3.14 - 3.27 (m, 2H), 3.79 (s, 3H), 5.30 (s, 2H), 6.87 (d, 1H), 7.24 (quin, 2H), 7.54 - 7.64 (m, 2H), 8.39 (d, 1H).

### Beispiel 280

### ent-N-(2-Amino-2-methylpentyl)-8-[(2,6-difluorbenzyl)oxy]-2-methyl-6-(morpholin-4-yl)imidazo[1,2-a]pyridin-3-carboxamid (Enantiomer B)

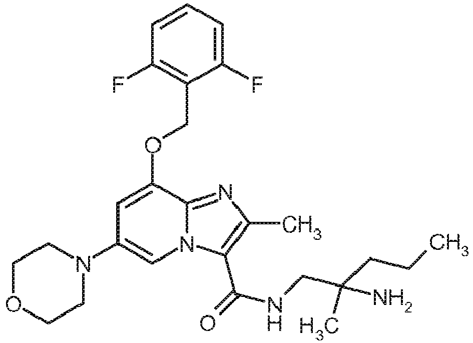

22 mg (0.04 mmol) ent-Benzyl-{1-[({8-[(2,6-difluorbenzyl)oxy]-2-methyl-6-(morpholin-4-yl)imidazo[1,2-a]pyridin-3-yl}carbonyl)amino]-2-methylpentan-2-yl}carbamat (Enantiomer B) aus Beispiel 293A in 0.38 ml Ethanol wurden unter Argon mit 1.2 mg 20%igem Palladium(II)hydroxid auf Kohle versetzt und 2 h bei RT unter Normaldruck hydriert. Das Reaktionsgemisch wurde über einen Milliporfilter filtriert, mit Ethanol gewaschen und das Filtrat eingeengt. Der Rückstand wurde mittels präparativer Dünnschicht-Chromatographie gereinigt (Laufmittel: Dichlormethan/2N Ammoniak in Methanol = 20/1). Es wurden 9 mg der Zielverbindung (48% d. Th.; Reinheit 92%) erhalten.
LC-MS (Methode 2): Rₜ = 0.68 min
MS (ESpos): m/z = 502 (M+H)⁺
¹H-NMR (400 MHz, DMSO-d₆): δ = 0.87 (t, 3H), 0.98 (s, 3H), 1.26 -1.36 (m, 2H), 1.38 -1.45 (m, 2H), 2.55 - 2.57 (s, 3H, verborgen unter Lösungsmittel-Peak), 3.05 (t, 4H), 3.16 - 3.22 (m, 2H), 3.77 (t, 4H), 5.31 (s, 2H), 6.99 - 7.05 (m, 1H), 7.20 - 7.28 (m, 2H), 7.54 - 7.64 (m, 2H), 8.20 (s, 1H).

### Beispiel 281

### ent-N-(2-Amino-2-methylpentyl)-6-cyclopropyl-8-[(2,6difluorbenzyl)oxy]-2-methylimidazo[1,2-a]pyridin-3-carboxamid (Enantiomer B)

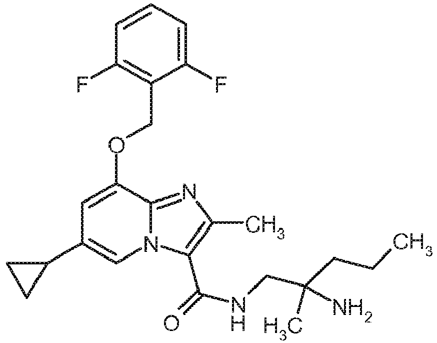

52 mg (0.07 mmol) *ent*-Benzyl-{1-[({6-cyclopropyl-8-[(2,6difluorbenzyl)oxy]-2-methylimidazo[1,2-a]pyridin-3-yl}carbonyl)amino]-2-methylpentan-2-yl}carbamat Trifluoracetat (Enantiomer B) aus Beispiel 294A in 0.76 ml Ethanol wurden unter Argon mit 7.8 mg 10%igem Palladium auf Kohle versetzt und 1 Stunde bei RT unter Normaldruck hydriert. Das Reaktionsgemisch wurde über einen Milliporfilter filtriert, mit Ethanol gewaschen und das Filtrat eingeengt. Der Rückstand wurde in Dichlormethan aufgenommen und zweimal mit gesättigter, wässriger Natriumhydrogen-carbonat-Lösung gewaschen. Die vereinigten wässrigen Phasen wurden zweimal mit Dichlormethan extrahiert. Die vereinigten organischen Phasen wurden über Natriumsulfat getrocknet, filtriert, das Filtrat eingeengt und im Hochvakuum getrocknet. Das Produkt wurde mittels Kieselgel-Chromatographie gereinigt (Laufmittel: Dichlormethan/2N Ammoniak in Methanol = 40/1). Es wurden 27 mg der Zielverbindung (76% d. Th.) erhalten.
LC-MS (Methode 2): Rₜ = 0.79 min
MS (ESpos): m/z = 457 (M+H)⁺
¹H-NMR (400 MHz, DMSO-d₆): δ = 0.72 - 0.77 (m, 2H), 0.87 (t, 3H), 0.90 - 0.97 (m, 2H), 0.99 (s, 3H), 1.22 - 1.42 (m, 4H), 1.43 - 1.51 (m, 2H), 1.94 - 2.03 (m, 1H), 2.52 (s, 3H), 3.13 - 3.26 (m, 2H), 5.31 (s, 2H), 6.68 (s, 1H), 7.23 (quin, 2H), 7.54 - 7.66 (m, 2H), 8.50 (s, 1 H).

### Beispiel 282

### rac-N-(2-Amino-3-fluor-2-methylpropyl)-8-[(2,6-difluorbenzyl)oxy]-2,6-dimethylimidazo[1,2-a]pyridin-3-carboxamid Formiat

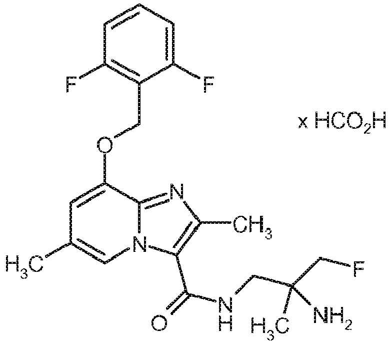

Unter Argon wurden 54 mg (0.09 mmol) *rac*-Benzyl-{1-[({8-[(2,6-difluorbenzyl)oxy]-2,6-dimethylimidazo[1,2-a]pyridin-3-yl)carbonyl)amino]-3-fluor-2-methylpropan-2-yl}carbamat aus Beispiel 302A in 5 ml Ethanol mit 1.4 mg (0.01 mmol, 20%ig) Palladium(II)hydroxid versetzt und das Reaktionsgemisch wurde über Nacht bei Normaldruck hydriert. Dann wurde über Celite filtriert, mit Ethanol gewaschen, das Filtrat eingeengt und über präparative HPLC gereinigt (RP18 Säule, Laufmittel: Acetonitril/Wasser-Gradient unter Zusatz von 0.1% TFA). Die Produktfraktionen wurden am Rotationsverdampfer eingedampft. Es wurden 18.4 mg der Zielverbindung (40% d. Th.) erhalten.
LC-MS (Methode 2): Rₜ = 0.66 min
MS (ESpos): m/z = 421 (M-HCO₂H+H)⁺
¹H-NMR (400 MHz, DMSO-d₆): δ = 1.08 (d, 3H), 2.31 (s, 3H), 2.53 (s, 3H), 3.28 - 3.43 (m, 2H), 4.17 (s, 1H), 4.29 (s, 1H), 5.29 (s, 2H), 6.93 (s, 1H), 7.24 (quin., 2H), 7.54 - 7.65 (m, 1H), 7.74 (t, 1H), 8.19 (s, 1H), 8.47 (s, 1H).

### Beispiel 283

### ent-N-(2-Amino-3-fluor-2-methylpropyl)-8-[(2,6-difluorbenzyl)oxy]-2,6-dimethylimidazo[1,2-a]pyridin-3-carboxamid (Enantiomer A)

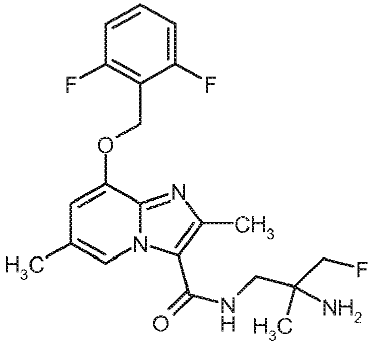

Unter Argon wurden 148 mg (0.21 mmol, Reinheit 95%) *ent*-Benzyl-{1-[({8-[(2,6-difluorbenzyl)oxy]-2,6-dimethylimidazo[1,2-a]pyridin-3-yl}carbonyl)amino]-3-fluor-2-methylpropan-2-yl}carbamat Trifluoracetat (Enantiomer A) aus Beispiel 303A in 9 ml Ethanol mit 14.8 mg (0.02 mmol) Palladium(II)hydroxid (20%ig) auf Aktivkohle versetzt und die Reaktionsmischung wurde 5.5 h bei Normaldruck hydriert. Dann wurde über einen Millipore-Filter filtriert, mit Ethanol gewaschen und das Filtrat anschließend eingeengt. Der Rückstand wurde mittels präparativer HPLC gereinigt (RP18 Säule, Laufmittel: Acetonitril/Wasser-Gradient unter Zusatz von 0.1% TFA). Die gesammelten Produktfraktionen wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, abfiltriert und eingeengt. Es wurden 61 mg der Zielverbindung (68% d. Th.) erhalten.
LC-MS (Methode 2): Rₜ = 0.61 min
MS (ESpos): m/z = 421 (M+H)⁺
¹H-NMR (400 MHz, DMSO-d₆): δ = 1.01 -1.06 (m, 3H), 1.67 (br. s, 2H), 2.31 (s, 3H), 2.53 (s, 3H; überlagert mit Lösungsmittel-Peak), 3.24 - 3.39 (m, 2H; überlagert mit Wasser-Peak), 4.07 - 4.15 (m, 1H), 4.19 - 4.27 (m, 1H), 5.29 (s, 2H), 6.92 (s, 1H), 7.20 - 7.28 (m, 2H), 7.54 - 7.64 (m, 1H), 7.68 (t, 1H), 8.47 (s, 1H).

### Beispiel 284

### ent-N-(2-Amino-3-fluor-2-methylpropyl)-8-[(2,6-difluorbenzyl)oxy]-2,6-dimethylimidazo[1,2-a]pyridin-3-carboxamid (Enantiomer B)

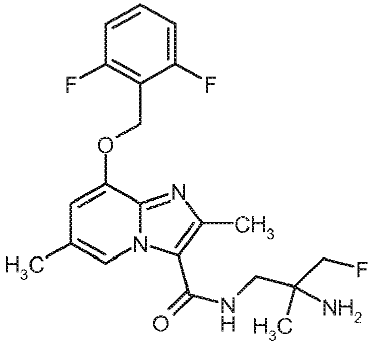

Unter Argon wurden 201 mg (0.29 mmol, 95%ig) *ent*-Benzyl-{1-[({8-[(2,6-difluorbenzyl)oxy]-2,6-dimethylimidazo[1,2-a]pyridin-3-yl}carbonyl)amino]-3-fluor-2-methylpropan-2-yl}carbamat Trifluoracetat (Enantiomer B) aus Beispiel 304A in 9 ml Ethanol mit 20 mg (0.03 mmol) Palladium(II)hydroxid (20%ig) auf Aktivkohle versetzt und die Reaktionsmischung wurde 4 h bei Normaldruck hydriert. Das Reaktionsgemisch wurde über einen Millipore-Filter filtriert, mit Ethanol gewaschen und das Filtrat anschließend eingeengt. Der Rückstand wurde in Acetonitril/ Wasser aufgenommen, mit TFA versetzt und zweimal mittels präparativer HPLC gereinigt (RP18 Säule, Laufmittel: Acetonitril/Wasser-Gradient unter Zusatz von 0.1% TFA). Die gesammelten Produktfraktionen wurden eingeengt. Der Rückstand wurde in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, abfiltriert, eingeengt und lyophilisiert. Es wurden 69 mg der Zielverbindung (59% d. Th.) erhalten.
LC-MS (Methode 2): Rₜ = 0.58 min
MS (ESpos): m/z = 421 (M+H)⁺
¹H-NMR (400 MHz, DMSO-d₆): δ = 1.01 - 1.07 (m, 3H), 1.64 (br. s, 2H), 2.31 (s, 3H), 2.53 (s, 3H; überlagert mit Lösungsmittel-Peak), 3.24 - 3.39 (m, 2H; überlagert mit Wasser-Peak), 4.08 - 4.15 (m, 1H), 4.20 - 4.27 (m, 1H), 5.29 (s, 2H), 6.92 (s, 1H), 7.20 - 7.28 (m, 2H), 7.55 - 7.64 (m, 1H), 7.66 (t, 1H), 8.47 (s, 1H).

### Beisipiel 285

### ent-N-(2-Amino-3-fluor-2-methylpropyl)-2,6-dimethyl-8-[(2,3,6-trifluorbenzyl)oxy]imidazo[1,2-a]pyridin-3-carboxamid (Enantiomer A)

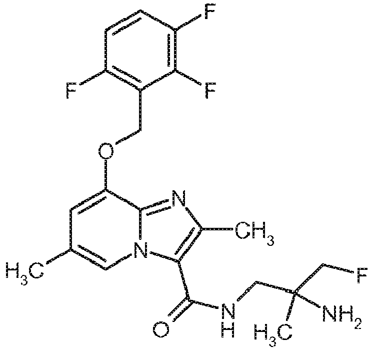

Unter Argon wurden 170 mg (0.21 mmol, 87%ig) *ent*-Benzyl-{1-[({2,6-dimethyl-8-[(2,3,6-trifluorbenzyl)oxy]imidazo[1,2-a]pyridin-3-yl}carbonyl)amino]-3-fluor-2-methylpropan-2-yl}carbamat Trifluoracetat (Enantiomer A) aus Beispiel 305A in 9 ml Ethanol mit 15 mg (0.02 mmol) Palladium(II)hydroxid (20%ig) auf Aktivkohle versetzt und die Reaktionsmischung wurde 5.5 Stunden bei Normaldruck hydriert. Dann wurde über einen Millipore-Filter filtriert, mit Ethanol gewaschen und das Filtrat anschließend eingeengt. Der Rückstand wurde mittels präparativer HPLC gereinigt (RP18 Säule, Laufmittel: Acetonitril/Wasser-Gradient unter Zusatz von 0.1% TFA). Die eingeengten Fraktionen wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 68 mg der Zielverbindung (71% d. Th.) erhalten.
LC-MS (Methode 2): Rₜ = 0.62 min
MS (ESpos): m/z = 439 (M+H)⁺
¹H-NMR (400 MHz, DMSO-d₆): δ = 1.00 -1.07 (m, 3H), 1.67 (br. s, 2H), 2.31 (s, 3H), 2.53 (s, 3H; überlagert mit Lösungsmittel-Peak), 3.24 - 3.39 (m, 2H; überlagert mit Wasser-Peak), 4.08 - 4.15 (m, 1H), 4.20 - 4.27 (m, 1H), 5.34 (s, 2H), 6.92 (s, 1H), 7.25 - 7.34 (m, 1H), 7.62 - 7.73 (m, 2H), 8.48 (s, 1H).

### Beispiel 286

### ent-N-(2-Amino-3-fluor-2-methylpropyl)-2,6-dimethyl-8-[(2,3,6-trifluorbenzyl)oxy]imidazo[1,2-a]pyridin-3-carboxamid (Enantiomer B)

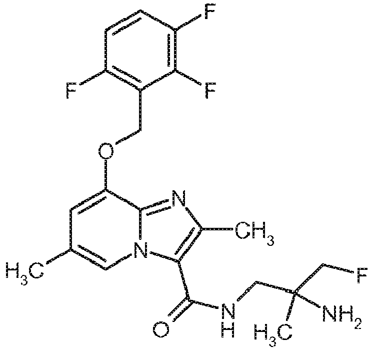

Unter Argon wurden 202 mg (0.28 mmol) *ent*-Benzyl-{1-[({2,6-dimethyl-8-[(2,3,6-trifluorbenzyl)oxy]imidazo[1,2-a]pyridin-3-yl}carbonyl)amino]-3-fluor-2-methylpropan-2-yl}carbamat Trifluoracetat (Enantiomer B) aus Beispiel 306A in 9.1 ml Ethanol mit 20 mg (0.03 mmol) Palladium(II)hydroxid (20%ig) auf Aktivkohle versetzt und die Reaktionsmischung wurde 2 h bei Normaldruck hydriert. Dann wurde über einen Millipore-Filter filtriert, das Filtrat eingeengt und im Hochvakuum getrocknet. Der Rückstand wurde in Acetonitril/Wasser aufgenommen, mit TFA versetzt und mittels präparativer HPLC gereinigt (RP18 Säule, Laufmittel: Acetonitril/Wasser-Gradient unter Zusatz von 0.1% TFA). Die gesammelten Produktfraktionen wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, abfiltriert, eingeengt und lyophilisiert. Es wurden 82 mg der Zielverbindung (66% d. Th.) erhalten.
LC-MS (Methode 2): Rₜ = 0.64 min
MS (ESpos): m/z = 439 (M+H)⁺
¹H-NMR (400 MHz, DMSO-d₆): δ = 1.01 -1.06 (m, 3H), 1.65 (br. s, 2H), 2.31 (s, 3H), 2.53 (s, 3H; überlagert mit Lösungsmittel-Peak), 3.24 - 3.39 (m, 2H, überlagert mit Wasser-Peak), 4.08 - 4.14 (m, 1H), 4.19 - 4.27 (m, 1H), 5.34 (s, 2H), 6.92 (s, 1H), 7.25 - 7.34 (m, 1H), 7.62 - 7.73 (m, 2H), 8.48 (s, 1H).

### Beisipiel 287

### ent-N-(2-Amino-2-methylbutyl)-8-[(2,6-difluorbenzyl)oxy]-6-(difluormethyl)-2-methylimidazo[1,2-a]pyridin-3-carboxamid Formiat (Enantiomer A)

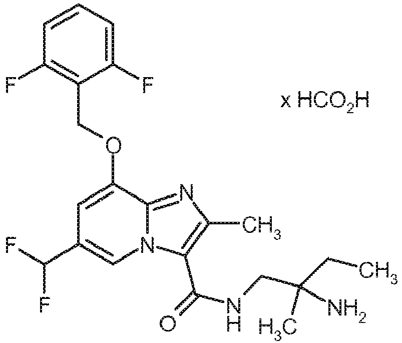

Unter Argon wurden 122 mg (0.21 mmol) *ent*-Benzyl-{1-[({8-[(2,6-difluorbenzyl)oxy]-6-(difluormethyl)-2-methylimidazo[1,2-a]pyridin-3-yl}carbonyl)amino]-2-methylbutan-2-yl}carbamat (Enantiomer A) aus Beispiel 311A in 2 ml DMF mit 17 mg 10%igem Palladium auf Kohle versetzt und 2 h unter Normaldruck bei RT hydriert. Das Reaktionsgemisch wurde über Celite filtriert, mit DMF gewaschen und eingeengt. Der Rückstand wurde in Dichlormethan gelöst und mittels Kieselgel-Chromatographie (Laufmittel: Dichlormethan/7 N Ammoniak in Methanol = 1/0 nach 30/1) gereinigt. Die Produktfraktionen wurden eingeengt. Der Rückstand wurde nochmals mittels präparativer HPLC gereinigt (RP18 Säule, Laufmittel: Acetonitril/Wasser-Gradient unter Zusatz von 0.05% Ameisensäure). Die Produktfraktionen wurden eingeengt und der Rückstand wurde im Hochvakuum getrocknet. Es wurden 47 mg der Zielverbindung (44% d. Th.) erhalten.
LC-MS (Methode 2): Rₜ = 0.75 min
MS (ESpos): m/z = 453 (M-HCO₂H+H)⁺
¹H-NMR (400 MHz, DMSO-d₆): δ = 0.91 (1, 3H), 1.12 (s, 3H), 1.43 - 1.60 (m, 2H), 2.58 (s, 3H), 3.30 - 3.46 (m, 2H; überlagert vom Wasser-Peak), 5.37 (s, 2H), 7.03 - 7.34 (m, 4H), 7.55 - 7.65 (m, 1H), 8.11 (br. s, 1H), 8.29 (s, 1H), 8.97 (s, 1H).

### Beispiel 288

### ent-N-(2-Amino-2-methylbutyl)-8-[(2,6-difluorbenzyl)oxy]-6-(difluormethyl)-2-methylimidazo[1,2-a]pyridin-3-carboxamid (Enantiomer A)

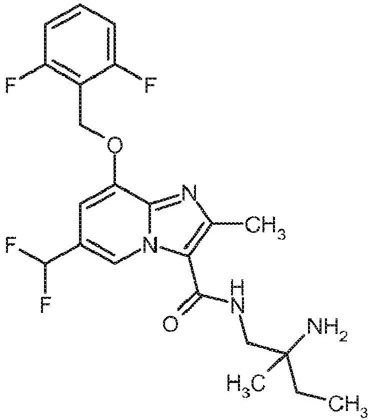

16.6 mg (0.03 mmol) *ent*-N-(2-Amino-2-methylbutyl)-8-[(2,6-difluorbenzyl)oxy]-6-(difluormethyl)-2-methylimidazo[1,2-a]pyridin-3-carboxamid Formiat aus Beispiel 287 wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden mit gesättigter, wässriger Natriumchloridlösung gewaschen, anschließend über Natriumsulfat getrocknet, abfiltriert und eingeengt. Es wurden 13 mg der Zielverbindung (85% d. Th.) erhalten.
LC-MS (Methode 2): Rₜ = 0.79 min
MS (ESpos): m/z = 453 (M+H)⁺
¹H-NMR (400 MHz, DMSO-d₆): δ = 0.87 (t, 3H), 0.98 (s, 3H), 1.29 - 1.43 (m, 2H), 1.58 (br. s, 2H), 2.57 (s, 3H), 3.22 (q, 2H), 5.37 (s, 2H), 7.02 - 7.33 (m, 4H), 7.55 - 7.65 (m, 1H), 7.79 (br. s, 1H), 8.99 (s, 1H).

### Beispiel 289

### ent-N-(2-Amino-2-methylpentyl)-8-[(2,6-difluorbenzyl)oxy]-6-(difluormethyl)-2-methylimidazo[1,2-a]pyridin-3-carboxamid Formiat (Enantiomer B)

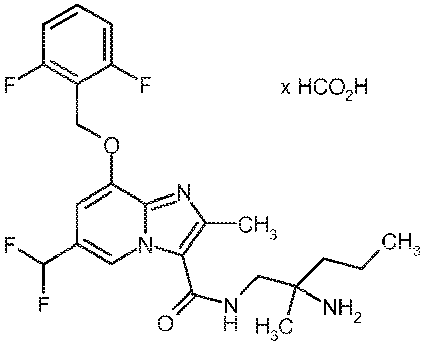

Unter Argon wurden 121 mg (0.20 mmol) *ent*-Benzyl-{1-[({8-[(2,6-difluorbenzyl)oxy]-6-(difluormethyl)-2-methylimidazo[1,2-a]pyridin-3-yl}carbonyl)amino]-2-methylpentan-2-yl}carbamat (Enantiomer B) aus Beispiel 312A in 2 ml DMF mit 16 mg 10%igem Palladium auf Kohle versetzt und 2 Stunden unter Normaldruck bei RT hydriert. Dann wurde über Celite filtriert, mit DMF nachgewaschen und eingeengt. Der Rückstand wurde in Dichlormethan gelöst und mittels Kieselgel-Chromatographie (Laufmittel: Dichlormethan/7 N Ammoniak in Methanol = 1/0 nach 30/1) gereinigt. Die Produktfraktionen wurden eingeengt. Der Rückstand wurde nochmals mittels präparativer HPLC gereinigt (RP18 Säule, Laufmittel: Acetonitril/Wasser-Gradient unter Zusatz von 0.05% Ameisensäure). Die Produktfraktionen wurden eingeengt und der Rückstand wurde im Hochvakuum getrocknet. Es wurden 49 mg der Zielverbindung (46% d. Th.) erhalten.
LC-MS (Methode 2): Rₜ = 0.77 min
MS (ESpos): m/z = 467 (M-HCO₂H+H)⁺
¹H-NMR (400 MHz, DMSO-d₆): δ = 0.89 (t, 3H), 1.13 (s, 3H), 1.29 - 1.55 (m, 4H), 2.58 (s, 3H), 3.30 - 3.45 (m, 2H; überlagert mit Wasser-Peak), 5.37 (s, 2H), 7.02 - 7.35 (m, 4H), 7.55 - 7.66 (m, 1H), 8.17 (br. s., 1H), 8.33 (s, 1H), 8.96 (s, 1H).

### Beispiel 290

### ent-N-(2-Amino-2-methylpentyl)-8-[(2,6-difluorbenzyl)oxy]-6-(difluormethyl)-2-methylimidazo[1,2-a]pyridin-3-carboxamid (Enantiomer B)

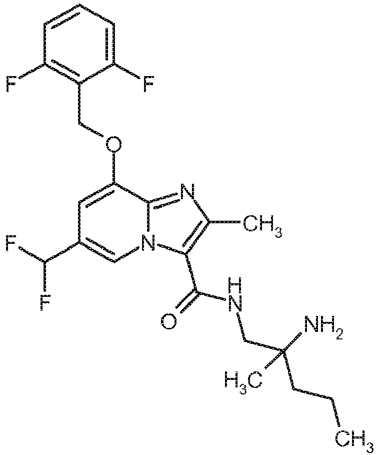

17 mg (0.03 mmol) *ent-*N-(2-Amino-2-methylpentyl)-8-[(2,6-difluorbenzyl)oxy]-6-(difluormethyl)-2-methylimidazo[1,2-a]pyridin-3-carboxamid Formiat aus Beispiel 289 wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden mit gesättigter, wässriger Natriumchloridlösung gewaschen, über Natriumsulfat getrocknet, abfiltriert und eingeengt. Es wurden 15 mg der Zielverbindung (96% d. Th.) erhalten.
LC-MS (Methode 2): Rₜ = 0.83 min
MS (ESpos): m/z = 467 (M+H)⁺
¹H-NMR (400 MHz, DMSO-d₆): δ = 0.87 (t, 3H), 0.99 (s, 3H), 1.25 - 1.41 (m, 4H), 1.51 (br. s, 2H), 2.57 (s, 3H), 3.22 (q, 2H), 5.37 (s, 2H), 7.02 - 7.33 (m, 4H), 7.55 - 7.65 (m, 1H), 7.80 (br. s, 1H), 8.98 (s, 1H).

### Beispiel 291

### ent-N-(2-Amino-3-fluor-2-methylpropyl)-8-[(2,6-difluorbenzyl)oxy]-6-(difluormethyl)-2-methylimidazo[1,2-a]pyridin-3-carboxamid (Enantiomer A)

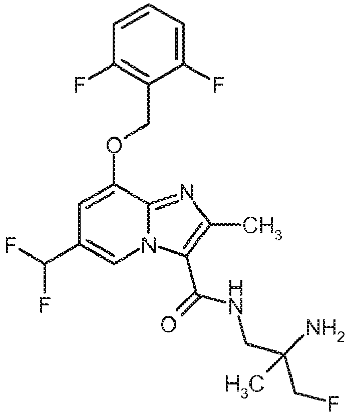

Unter Argon wurden 74.6 mg (0.11 mmol) *ent*-Benzyl-{1-[({8-[(2,6-difluorbenzyl)oxy]-6-(difluormethyl)-2-methylimidazo[1,2-a]pyridin-3-yl}carbonyl)amino]-3-fluor-2-methylpropan-2-yl}carbamat Trifluoroacetat (Enantiomer A) aus Beispiel 313A in 4.5 ml Ethanol mit 7.4 mg (0.01 mmol) Palladium(II)hydroxid (20%ig) auf Aktivkohle versetzt und das Gemisch 4 Stunden bei Normaldruck hydriert. Das Reaktionsgemisch wurde über einen Millipore-Filter filtriert, mit Ethanol gewaschen und das Filtrat anschließend eingeengt. Der Rückstand wurde mittels präparativer HPLC gereinigt (RP18 Säule, Laufmittel: Acetonitril/Wasser-Gradient unter Zusatz von 0.1% TFA). Die Produktfraktionen wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, abfiltriert und eingeengt. Es wurden 16 mg der Zielverbindung (31% d. Th.) erhalten.
LC-MS (Methode 2): Rₜ = 0.74 min
MS (ESpos): m/z = 457 (M+H)⁺
¹H-NMR (500 MHz, DMSO-d₆): δ = 1.02 - 1.06 (m, 3H), 1.68 (br. s, 2H), 2.57 (s, 3H), 3.30 - 3.40 (m, 2H, teilweise verdeckt durch Wasser-Peak), 4.10 - 4.16 (m, 1H), 4.20 - 4.26 (m, 1H), 5.37 (s, 2H), 7.05 - 7.31 (m, 4H), 7.56 - 7.63 (m, 1H), 7.84 (t, 1H), 8.98 (s, 1H).

### Beispiel 292

### ent-N-(2-Amino-3-fluor-2-methylpropyl)-8-[(2,6-difluorbenzyl)oxy]-6-(difluormethyl)-2-methylimidazo[1,2-a]pyridin-3-carboxamid (Enantiomer B)

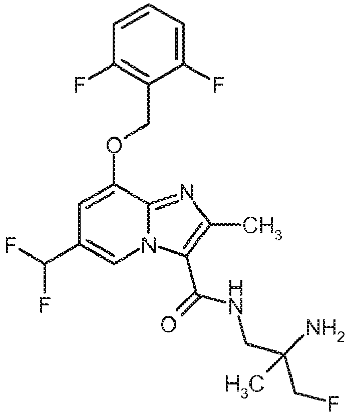

Unter Argon wurde 47 mg (0.06 mmol, 89%ig) *ent*-Benzyl-{1-[({8-[(2,6-difluorbenzyl)oxy]-6-(difluormethyl)-2-methylimidazo[1,2-a]pyridin-3-yl}carbonyl)amino]-3-fluor-2-methylpropan-2-yl}carbamat Trifluoroacetat (Enantiomer B) aus Beispiel 314A in 2 ml Ethanol mit 4.2 mg (0.01 mmol) Palladium(II)hydroxid (20%ig) auf Aktivkohle versetzt und es wurde 5 Stunden bei Normaldruck hydriert. Dann wurde über einen Millipore-Filter filtriert, mit Ethanol gewaschen und das Filtrat anschließend eingeengt. Der Rückstand wurde mittels präparativer HPLC gereinigt (RP18 Säule, Laufmittel: Acetonitril/Wasser-Gradient unter Zusatz von 0.1% TFA). Die Produktfraktionen wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Der Rückstand wurde in Dichlormethan und wenig Methanol aufgenommen und mittels Dickschichtchromatographie (Laufmittel: Dichlormethan/ 2 N Ammoniak in Methanol = 15:1) getrennt. Die Produktfraktionen wurden eingedampft und lyophilisiert. Es wurden 16 mg der Zielverbindung (56% d. Th.) erhalten.
LC-MS (Methode 2): Rₜ = 0.75 min
MS (ESpos): m/z = 457 (M+H)⁺
¹H-NMR (500 MHz, DMSO-d₆): δ = 1.02 - 1.06 (m, 3H), 1.71 (br. s, 2H), 2.57 (s, 3H), 3.30 - 3.40 (m, 2H, teilweise verdeckt durch Wasser-Peak), 4.10 - 4.16 (m, 1H), 4.20 - 4.26 (m, 1H), 5.37 (s, 2H), 7.05 - 7.31 (m, 4H), 7.56 - 7.63 (m, 1H), 7.84 (t, 1H), 8.98 (s, 1H).

### Beispiel 293

### rac-N-(2-Amino-2-methylpentyl)-2,6-dimethyl-8-[(2,3,6-trifluorbenzyl)oxy]imidazo[1,2-a]pyridin-3-carboxamid

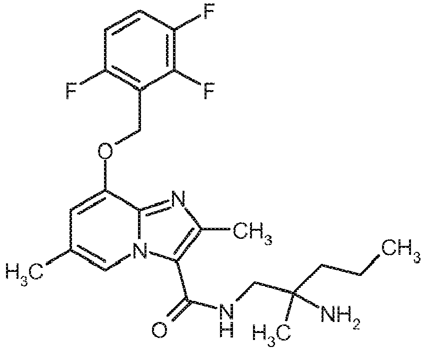

250 mg (0.71 mmol) 2,6-Dimethyl-8-[(2,3,6-trifluorbenzyl)oxylimidazo[1,2-a]pyridin-3-carbonsäure aus Beispiel 265A wurden mit 298 mg (0.79 mmol) HATU und 0.37 ml (2.14 mmol) N,N-Diisopropylethylamin 20 min in 4.8 ml DMF gerührt. Anschließend wurden bei 0°C 103 mg (0.86 mmol, Reinheit 97%) *rac*-2-Methylpentan-1,2-diamin zugegeben und für 30 min bei 0°C gerührt. Die Reaktionslösung wurde mit Acetonitril/TFA versetzt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan aufgenommen und einmal mit gesättigter, wässriger Natriumhydrogencarbonatlösung gewaschen. Die wässrige Phase wurde zweimal mit Dichlormethan gewaschen, die vereinigten organischen Phasen über Natriumsulfat getrocknet, filriert, das Filtrat eingeengt und der Rückstand lyophilisiert. Es wurden 222mg der Zielverbindung (69% d. Th.) erhalten.
LC-MS (Methode 2): Rₜ = 0.69 min
MS (ESpos): m/z = 449 (M+H)⁺
¹H-NMR (400 MHz, DMSO-d₆): 0.87 (t, 3H), 0.99 (s, 3H), 1.22 - 1.43 (m, 4H), 1.60 (br. s, 2H), 2.30 (s, 3H), 2.55 (s, 3H), 3.14 - 3.28 (m, 2H), 5.32 (s, 2H), 6.91 (s, 1H), 7.25 - 7.33 (m, 1H), 7.59 - 7.73 (m, 2H), 8.48 (s, 1H).

### Beispiel 294

### Methyl-N⁶-({8-[(2,6-difluorbenzyl)oxy]-2,6-dimethylimidazo[1,2-a]pyridin-3-yl)carbonyl)-L-lysinat

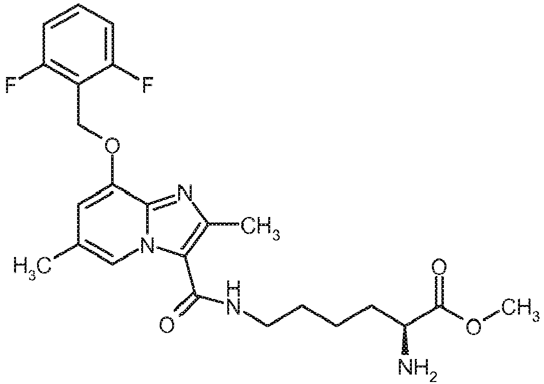

75 mg (0.11 mmol) Methyl-N²-(tert-butoxycarbonyl)-N⁶-({8-[(2,6-difluorbenzyl)oxy]-2,6-dimethyl-imidazo[1,2-a]pyridin-3-yl}carbonyl)-L-lysinat Trifluoracetat aus Beispiel 315A wurden in 0.54 ml Diethylether vorgelegt, mit 0.54 ml (1.08 mmol) 2N Salzsäure in Diethylether versetzt und über Nacht bei Raumtemperatur gerührt. Die Reaktionslösung wurde eingeengt, der Rückstand in Dichlormethan gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Das Rohprodukt wurde mittels Dickschichtchromatographie gereinigt (Laufmittel: Dichlormethan/2N Ammoniak in Methanol = 20/1). Es wurden 22 mg der Zielverbindung (42% d. Th.) erhalten.
LC-MS (Methode 2): Rₜ = 0.64 min
MS (ESpos): m/z = 475 (M+H)⁺
¹H-NMR (400 MHz, DMSO-d₆): δ = 1.31 - 1.42 (m, 2H), 1.43 -1.65 (m, 4H), 1.66 -1.81 (m, 2H), 2.30 (s, 3H), 2.47 (s, 3H), 3.25 - 3.29 (m, 2H), 3.60 (s, 3H), 5.28 (s, 2H), 6.87 - 6.91 (m, 1H), 7.19 - 7.27 (m, 2H), 7.54 - 7.64 (m, 1H), 7.82 (t, 1H), 8.42 (s, 1H).

### Beispiel 295

### ent-N-(2-Amino-2-methylpentyl)-2,6-dimethyl-8-[(2,3,6-trifluorbenzyl)oxy]imidazo[1,2-a]pyridin-3-carboxamid

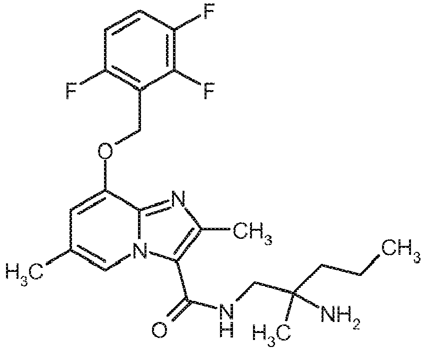

215 mg *rac*-N-(2-Amino-2-methylpentyl)-2,6-dimethyl-8-[(2,3,6-trifluorbenzyl)oxy]imidazo[1,2-a]pyridin-3-carboxamid aus Beispiel 293 wurden durch präparative Trennung an chiraler Phase in die Enantiomere getrennt [Säule: Daicel Chiralcel OZ-H, 5 µm, 250 x 20 mm, Eluent: 50% Iso-Hexan, 50% Ethanol + 0.2% Diethylamin, Fluß: 17 ml/min; Temperatur: 40°C, Detektion: 210 nm].
Enantiomer A: Ausbeute: 83 mg (>99% ee)
Rₜ = 11.59 min [Daicel Chiralcel OZ-H, 5 µm, 250 x 4.6 mm; Eluent: 50% iso-Hexan, 50% Ethanol + 0.2% Diethylamin; Fluss 1 ml/min; Temperatur: 40°C; Detektion: 235 nm].

### Beispiel 296

### rac-N-[2-Amino-3-(4-fluorphenyl)-2-methylpropyl]-8-[(2,6-difluorbenzyl)oxy]-2,6-dimethylimidazo[1,2-a]pyridin-3-carboxamid

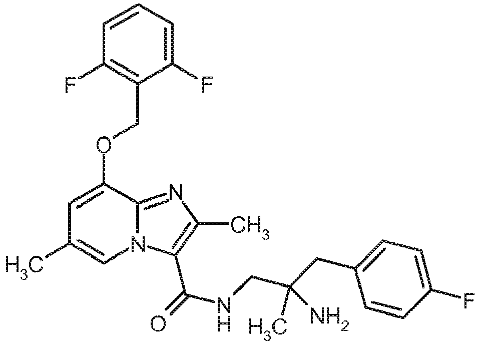

106 mg (0.32 mmol) 8-[(2,6-Difluorbenzyl)oxy]-2,6-dimethylimidazo[1,2-a]pyridin-3-carbonsäure aus Beispiel 21A wurden mit 134 mg (0.35 mmol) HATU und 0.28 ml (1.60 mmol) N,N-Diisopropylethylamin in 2 ml DMF vorgelegt und 20 min gerührt. Anschließend wurden bei 0°C 70 mg (0.38 mmol) *rac*-3-(4-Fluorphenyl)-2-methylpropan-1,2-diamin aus Beispiel 317A hinzugegeben und für 45 min bei 0°C gerührt. Die Reaktionslösung wurde mit Acetonitril/TFA versetzt und mittels präparativer HPLC gereinigt (RP18 Säule, Laufmittel: Acetonitril/Wasser-Gradient unter Zusatz von 0.1% TFA). Die Produktfraktionen wurden eingeengt, der Rückstand in Dichlormethan aufgenommen und zweimal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, das Filtrat eingeengt und lyophilisiert. Es wurden 121 mg der Zielverbindung (75% d. Th.) erhalten.
LC-MS (Methode 2): Rₜ = 0.71 min
MS (ESpos): m/z = 497 (M+H)⁺
¹H-NMR (400 MHz, DMSO-d₆): δ = 0.94 (s, 3H), 2.31 (s, 3H), 2.55 (br. s., 3H), 2.63 - 2.69 (m, 2H), 3.17 - 3.25 (m, 1H), 3.27 - 3.30 (m, 1H), 5.29 (s, 2H), 6.90 - 6.94 (m, 1H), 7.07 - 7.15 (m, 2H), 7.19 - 7.32 (m, 4H), 7.54 - 7.63 (m, 1H), 7.67 (t, 1H), 8.46 - 8.51 (m, 1H).

### Beispiel 297

### rac-N-[2-Amino-2-methyl-3-(pyridin-2-yl)propyl]-8-[(2,6-difluorbenzyl)oxy]-2,6-dimethylimidazo[1,2-a]pyridin-3-carboxamid

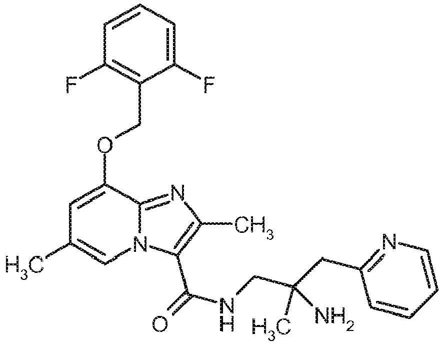

174 mg (0.52 mmol) 8-[(2,6-Difluorbenzyl)oxyl-2,6-dimethylimidazo[1,2-a]pyridin-3-carbonsäure aus Beispiel 21A wurden mit 219 mg (0.58 mmol) HATU und 0.46 ml (2.62 mmol) N,N-Diisopropylethylamin in 3.3 ml DMF vorgelegt und 20 min gerührt. Anschließend wurden bei 0°C 1.15 g (0.63 mmol, Annahme: Reinheit ca. 15%) *rac*-2-Methyl-3-(pyridin-2-yl)propan-1,2-diamin Trihydrochlorid aus Beispiel 319A hinzugegeben und über Nacht bei RT gerührt. Die Reaktionslösung wurde mit Acetonitril/TFA versetzt und mittels präparativer HPLC gereinigt (RP18 Säule, Laufmittel: Acetonitril/Wasser-Gradient unter Zusatz von 0.1% TFA). Die Produktfraktionen wurde eingeengt, der Rückstand in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen wurden über Natriumsulfat getrocknet, abfiltriert, das Filtrat eingeengt und lyophilisiert. Es wurden 106 mg der Zielverbindung (42% d. Th.) erhalten.
LC-MS (Methode 2): Rₜ = 0.70 min
MS (ESpos): m/z = 480 (M+H)⁺
¹H-NMR (400 MHz, DMSO-d₆): δ = 1.01 (s, 3H), 1.67 - 2.12 (br. s, 2H), 2.31 (s, 3H), 2.60 (s, 3H), 2.84 (s, 2H), 3.23 (d, 2H), 5.29 (s, 2H), 6.93 (s, 1H), 7.19 - 7.28 (m, 3H), 7.32 (d, 1 H), 7.54 - 7.64 (m, 1H), 7.69 - 7.79 (m, 2H), 8.51 (d, 1H), 8.57 (s, 1H).

### Beispiel 298

### ent-N-[2-Amino-2-methyl-3-{pyridin-2-yl)propyl]-8-[(2,6-difluorbenzyl)oxy]-2,6-dimethylimidazo[1,2-a]pyridin-3-carboxamid (Enantiomer A)

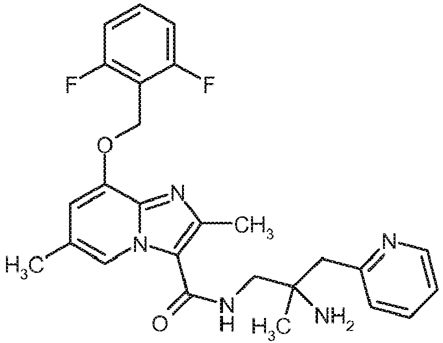

106 mg *rac*-N-[2-Amino-2-methyl-3-(pyridin-2-yl)propyl]-8-[(2,6-difluorbenzyl)oxy]-2,6-dimethylimidazo[1,2-a]pyridin-3-carboxamid aus Beispiel 297 wurden durch präparative Trennung an der chiralen Phase in die Enantiomere getrennt [Säule: Daicel Chiralpak ID, 5 µm, 250 x 20 mm, Eluent: 70% Methyl-*tert*.-butylether, 30% Acetonitril + 0.2%Diethylamin, Fluß: 15 ml/min; Temperatur: 40°C, Detektion: 220 nm].
Enantiomer A: Ausbeute: 27 mg (98% ee)
Rₜ = 11.80 min [Daicel Chiralcel ID, 5 µm, 250 x 4.6 mm; Eluent: 70% Methyl-*tert*.-butylether, 30% Acetonitril + 0.2%Diethylamin; Fluss 1 ml/min; Temperatur: 40°C; Detektion: 235 nm].

### Beispiel 299

### rac-8-[(2,6-Difluorbenzyl)oxy]-N-(3,3-difluorpiperidin-4-yl)-2,6-dimethylimidazo[1,2-a]pyridin-3-carboxamid

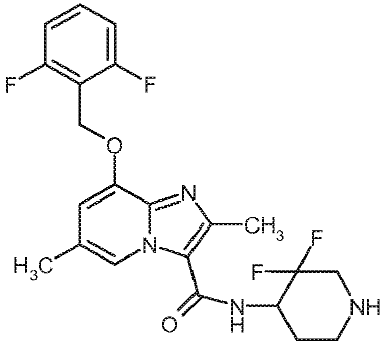

81 mg (0.15 mmol) *rac-tert*-Butyl-4-[({8-[(2,6-difluorbenzyl)oxy]-2,6-dimethylimidazo[1,2-alpyridin-3-yl}carbonyl)amino]-3,3-difluorpiperidin-1-carboxylat aus Beispiel 320A wurden in 0.8 ml Diethylether vorgelegt, mit 0.74 ml (1.47 mmol) 2 N Salzsäure in Diethylether versetzt und über Nacht bei Raumtemperatur gerührt. Dann wurde eingeengt, der Rückstand mit Dichlormethan und einem Tropfen Methanol versetzt und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 18 mg der Zielverbindung (26% d. Th.) erhalten.
LC-MS (Methode 2): Rₜ = 0.60 min
MS (ESpos): m/z = 451 (M+H)⁺
¹H-NMR (400 MHz, DMSO-d₆): δ = 1.18 -1.31 (m, 1H), 1.80 - 2.02 (m, 2H), 2.31 (s, 3H), 2.48 (s, 3H), 2.87 - 2.98 (m, 1H), 3.09 - 3.18 (m, 1H), 3.21 - 3.28 (m, 1H), 3.45 - 3.56 (m, 1H), 4.58 - 4.76 (m, 1H), 5.29 (s, 2H), 6.92 - 6.95 (m, 1H), 7.20 - 7.28 (m, 2H), 7.55 - 7.64 (m, 1H), 8.20 (d, 1H), 8.25 - 8.31 (m, 1H).

### Beispiel 300

### ent-N-(2-Amino-2-methylbutyl)-2,6-dimethyl-8-[(2,3,6-trifluorbenzyl)oxy]imidazo[1,2-a]pyridin-3-carboxamid (Enantiomer A) Hydrochlorid

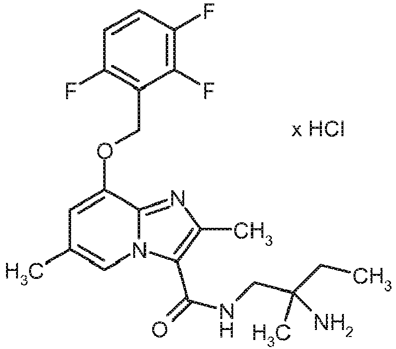

76 mg (0.18 mmol) *ent-*N-(2-Amino-2-methylbutyl)-2,6-dimethyl-8-[(2,3,6-trifluorbenzyl)oxy]imidazo[1,2,a]pyridin-3-carboxamid aus Beispiel 274 wurden in 1.4 ml Diethylether vorgelegt, mit 0.11 ml (0.21 mmol) 2 N Salzsäure in Diethylether versetzt und 30 min bei RT gerührt. Anschließend wurde das Lösungsmittel abdestilliert und der Rückstand im Hochvakuum getrocknet. Es wurden 86 mg der Zielverbindung (99% d. Th.) erhalten.
LC-MS (Methode 2): Rₜ = 0.62 min
MS (ESpos): m/z = 435 (M-HCl+H)⁺
¹H-NMR (400 MHz, DMSO-d₆): δ = 0.86 (t, 3H), 0.97 (s, 3H), 1.30 -1.40 (m, 2H), 1.41 -1.52 (m, 2H), 2.31 (s, 3H), 2.53 (s, 3H), 3.14 - 3.27 (m, 2H), 5.34 (s, 2H), 6.90 - 6.94 (m, 1H), 7.25 - 7.34 (m, 1H), 7.57 - 7.72 (m, 2H), 8.49 (s, 1H).

### Beispiel 301

### rac-N-(2-Amino-2-methylpentyl)-8-[(2,6-difluorbenzyl)oxy]-2-ethyl-6-methylimidazo[1,2-a]pyridin-3-carboxamid

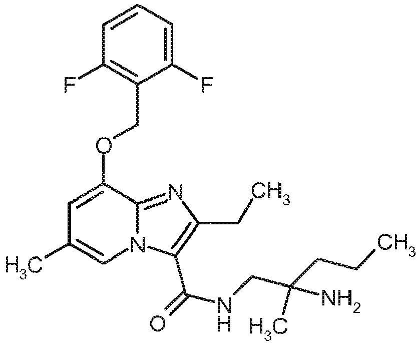

75 mg (0.22 mmol) 8-[(2,6-Difluorbenzyl)oxy]-2-ethyl-6-methylimidazo[1,2-a]pyridin-3-carbonsäure aus Beispiel 325A wurden mit 73 mg (0.23 mmol) (Benzotriazol-1-yloxy)bisdimethylamino-methyliumfluoroborat und 0.14 ml (1.30 mmol) 4-Methylmorpholin in 0.8 ml DMF vorgelegt, mit 45 mg (0.24 mmol) *rac*-2-Methylpentan-1,2-diamin Dihydrochlorid aus Beispiel 326A versetzt und über Nacht bei RT gerührt. Die Reaktionslösung wurde mit einigen Tropfen Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden eingeengt, der Rückstand in Dichlormethan gelöst und mit gesättigter, wässriger Natriumhydrogencarbonatlösung gewaschen. Die wässrige Phase wurde dreimal mit Dichlormethan extrahiert, die vereinigten organischen Phasen über Natriumsulfat getrocknet, filtriert, das Filtrat einegeengt und der Rückstand lyophilisiert. Es wurden 68 mg der Zielverbindung (69% d. Th.) erhalten.
LC-MS (Methode 2): Rₜ = 0.67 min
MS (ESpos): m/z = 445 (M+H)⁺
¹H-NMR (400 MHz, DMSO-d₆): δ = 0.83 - 0.91 (m, 3H), 1.04 (s, 3H), 1.22 (t, 3H), 1.28 -1.41 (m, 4H), 2.31 (s, 3H), 2.91 (q, 2H), 3.18 - 3.30 (m, 2H), 5.29 (s, 2H), 6.92 (s, 1H), 7.25 (quin, 2H), 7.55 - 7.65 (m, 1H), 7.69 - 7.79 (m, 1H), 8.41 (s,1H).

### Beispiel 302

### rac-8-[(2,6-Difluorbenzyl)oxy]-2,6-dimethyl-N-[2-(trifluormethyl)piperidin-4-yl]imidazo[1,2-a]pyridin-3-carboxamid

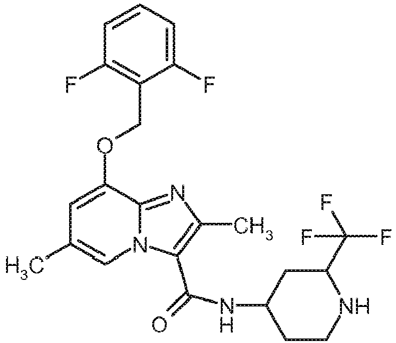

75 mg (0.23 mmol) 8-[(2,6-Difluorbenzyl)oxy]-2,6-dimethylimidazo[1,2-a]pyridin-3-carbonsäure aus Beispiel 21A wurden mit 94 mg (0.25 mmol) HATU und 0.24 ml (1.35 mmol) N,N-Diisopropylethylamin in 1.4 ml DMF vorgelegt und 20 min gerührt. Anschließend wurden 55 mg (0.27 mmol) *rac*-2-(Trifluormethyl)piperidin-amin Hydrochlorid aus Beispiel 327A hinzugegeben und über Nacht bei RT gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1% TFA). Die Produktfraktionen wurden im Vakuum eingeengt, anschließend in Dichlormethan aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert, das Filtrat eingeengt und der Rückstand lyophilisiert. Es wurden 65 mg der Zielverbindung (60% d. Th.) erhalten.
LC-MS (Methode 2): Rₜ = 0.69 min
MS (ESpos): m/z = 483 (M+H)⁺
¹H-NMR (400 MHz, DMSO-d₆): δ = 1.60 - 1.95 (m, 4H), 2.31 (s, 3H), 2.81 - 2.98 (m, 2H), 3.55 - 3.74 (m, 1H), 4.24 - 4.33 (m, 1H), 5.29 (s, 2H), 6.92 (s, 1H), 7.24 (quin, 2H), 7.55 - 7.64 (m, 1H), 7.89 (d,1H), 8.36 (s, 1H).

### Beispiel 303

### rac-N-(2-Amino-2-methylpentyl)-8-[(2,3-difluorbenzyl)oxy]-2,6-dimethylimidazo[1,2-a]pyridin-3-carboxamid

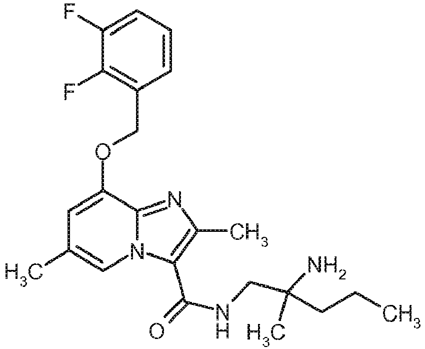

80 mg (0.19 mmol, Reinheit 95%) *rac*-tert-Butyl-(1-{[(8-hydroxy-2,6-dimethylimidazo[1,2-a]pyridin-3-yl)carbonyl]amino}-2-methylpentan-2-yl)carbamat aus Beispiel 330A, 43 mg (0.21 mmol) 2,3-Difluorbenzylbromid, 135 mg (0.41 mmol) Cäsiumcarbonat und 3.1 mg (0.02 mmol) Kaliumiodid wurden in 3.6 ml DMF vorgelegt und für 30 min in einem vorgeheizten 60°C warmen Ölbad erhitzt. Die Reaktionslösung wurde eingeengt und über Nacht im Hochvakuum getrocknet. Der Rückstand wurde in Diethylether aufgenommen, mit 0.94 ml (1.88 mmol) 2 N Salzsäure in Diethylether versetzt und über Nacht bei RT gerührt. Das Reaktionsgemisch wurde eingeengt und mittels präparativer HPLC gereinigt (RP18 Säule, Laufmittel:Acetonitril/Wasser-Gradient unter Zusatz von 0.1% TFA). Die Produktfraktionen wurden eingeengt, der Rückstand wurde in Dichlormethan aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, das Filtrat eingeengt und der Rückstand lyophilisiert. Es wurden 51 mg der Zielverbindung (61% d. Th.) erhalten.
LC-MS (Methode 2): Rₜ = 0.67 min
MS (ESpos): m/z = 431 (M+H)⁺

### Beispiel 304

### ent-N-(2-Amino-2-methylpentyl)-2,6-dimethyl-8-[(2,3,6-trifluorbenzyl)oxy]imidazo[1,2-a]pyridin-3-carboxamid

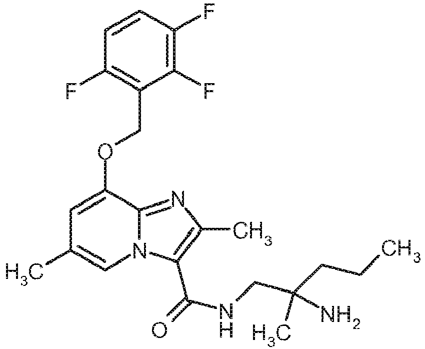

215 mg *rac-*N*-(2-*Amino-2-methylpentyl)-2,6-dimethyl-8-[(2,3,6-trifluorbenzyl)oxy]imidazo[1,2-a]pyridin-3-carboxamid aus Beispiel 293 wurden durch präparative Trennung an chiraler Phase in die Enantiomere getrennt [Säule: Daicel Chiralcel OZ-H, 5 µm, 250 x 20 mm, Eluent: 50% Iso-Hexan, 50% Ethanol + 0.2% Diethylamin, Fluß: 17 ml/min; Temperatur: 40°C, Detektion: 210 nm].
Enantiomer B: Ausbeute: 86 mg (97.5% ee)
Rₜ= 14.00 min [Daicel Chiralcel OZ-H, 5 µm, 250 x 4.6 mm; Eluent: 50% iso-Hexan, 50% Ethanol + 0.2% Diethylamin; Fluss 1 ml/min; Temperatur: 40°C; Detektion: 235 nm].

### Beispiel 305

### ent-N-(2-Amino-2-methylpentyl)-8-[(2,6-difluorbenzyl)oxy]-6-methyl-2-(trifluarmethyl)imidazo[1,2-a]pyridin-3-carboxamid

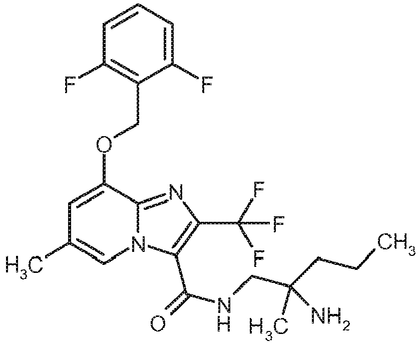

46 mg (0.06 mmol) *ent*-Benzyl-{1-[({8-[(2,6-difluorbenzyl)oxy]-methyl-2-(trifluormethyl)imidazo[1,2-a]pyridin-3-yl}carbonyl)amino]-2-methylpentan-2-yl}carbamat Trifluoracetat aus Beispiel 333A wurden in 0.7 ml Ethanol gelöst und unter Argon mit 4.4 mg (0.01 mmol) 20%-igem Palladium(II)hydroxid auf Kohle versetzt. Anschließend wurde das Reaktionsgemisch zwei Stunden bei RT unter Normaldruck hydriert. Das Reaktionsgemisch wurde auf Diatomeenerde gezogen und mittels Kieselgel-Chromatographie gereinigt (Laufmittel: Dichlormethan/2 N Ammoniak in Methanol = 50/1). Es wurden 30 mg der Zielverbindung (99% d. Th.) erhalten.
LC-MS (Methode 2): Rₜ = 0.91 min
MS (ESpos): m/z = 485 (M+H)⁺
¹H-NMR (400 MHz, DMSO-d₆): δ = 0.87 (t, 3H), 0.99 (s, 3H), 1.21 - 1.42 (m, 4H), 1.49 -1.89 (m, 2H), 2.34 (s, 3H), 3.23 (s, 2H), 5.33 (s, 2H), 7.08 (s, 1H), 7.20 - 7.30 (m, 2H), 7.56 - 7.66 (m, 1H), 8.02 (s,1H), 8.31-8.80 (m, 1H).

### Beispiel 306

### ent-N-(2-Amino-2-methylpentyl)-6-brom-8-[(2,6-difluorbenzyl)oxy]-2-methylimidazo[1,2-a]pyridin-3-carboxamid

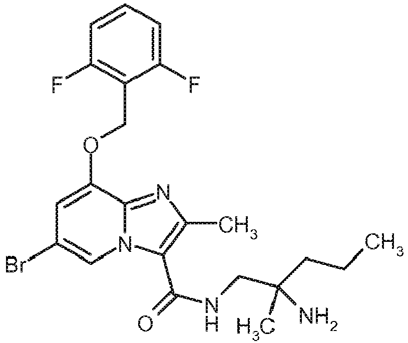

70 mg (0.09 mmol) *ent*-Benzyl-{1-[({6-brom-8-[(2,6-difluorbenzyl)oxy]-2-methylimidazo[1,2-a]pyridin-3-yl}carbonyl)amino]-2-methylpentan-2-yl}carbamat Trifluoracetat aus Beispiel 292A wurden unter Argon in 1.9 ml Dichlormethan vorgelegt und unter Argon bei 0°C mit 0.14 ml (0.14 mmol) 1 N Bortribromid in Dichlormethan versetzt. Es wurde 2.5 Stunden bei RT gerührt. Das Reaktionsgemisch wurde mit Wasser versetzt, eingeengt und der Rückstand mittels präparativer HPLC gereinigt (RP18 Säule, Laufmittel: Acetonitril/Wasser-Gradient unter Zusatz von 0.1% TFA). Das Produkt wurde in Dichlormethan gelöst und mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet und filtriert. Das Filtrat wurde eingeengt und der Rückstand mittels präparativer Dünnschichtchromatographie gereinigt (Laufmittel Dichlormethan/2 N Ammoniak in Methanol = 20/1). Die gesammelten Produktfraktionen wurden am Rotationsverdampfer eingeengt. Es wurden 5 mg der Zielverbindung (11% d. Th.) erhalten.
LC-MS (Methode 2): Rₜ = 0.84 min
MS (ESpos): m/z = 495 (M+H)⁺
¹H-NMR (400 MHz, DMSO-d₆): δ = 0.87 (t, 3H), 1.01 (s, 3H), 1.26 -1.42 (m, 4H), 1.44 -1.58 (m, 2H), 2.55 (s, 3H), 3.17 - 3.26 (m, 2H), 5.35 (s, 2H), 7.19 - 7.29 (m, 3H), 7.55 - 7.65 (m, 1H), 7.66 - 7.85 (m, 1H), 8.83 (d, 1H).

### Beispiel 307

### N-[(1-Aminocyclohexyl)methyl]-8-[(2,6-difluorbenzyl)oxy]-2,6-dimethylimidazo[1,2-a]pyridin-3-carboxamid

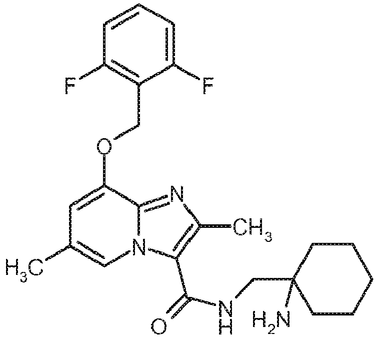

163 mg (0.25 mmol) *tert-*Butyl-(1*-*{[({8-[(2,6-difluorbenzyl)oxy]-2,6-dimethylimidazo[1,2-a]pyridin-3-yl}carbonyl)amino]methyl}cyclohexyl)carbamat Trifluoracetat aus Beispiel 334A wurden in 1.24 ml Diethylether vorgelegt, mit 1.24 ml (2.48 mmol) 2 N Salzsäure in Diethylether versetzt und 3 Stunden bei RT gerührt. Es wurden nochmals 0.5 ml (1 mmol) 2 N Salzsäure in Diethylether zugegeben und 4 Stunden bei RT gerührt. Das Reaktionsgemisch wurde eingeengt, der Rückstand in Dichlormethan aufgenommen und zweimal mit gesättigter, wässriger Natriumhydrogencarbonatlösung gewaschen. Die vereinten wässrigen Phasen wurden zweimal mit Dichlormethan gewaschen, die vereinten organischen Phasen wurden über Natriumsulfat getrocknet, filtriert und der Rückstand im Hochvakuum getrocknet. Es wurden 89 mg der Zielverbindung (81% d. Th.) erhalten.
LC-MS (Methode 2): Rₜ = 0.65 min
MS (ESpos): m/z = 443 (M+H)⁺
¹H-NMR (400 MHz, DMSO-d₆): δ = 1.19 - 1.48 (m, 8H), 1.49 -1.61 (m, 2H), 1.62 -1.77 (m, 2H), 2.31 (s, 3H), 2.53 (br. s., 3H), 3.24 (d, 2H), 5.28 (s, 2H), 6.92 (s, 1H), 7.18 - 7.29 (m, 2H), 7.54 - 7.66 (m, 2H), 8.49 (s, 1H).

### Beispiel 308

### N-(3-Aminocyclopentyl)-8-[(2,6-difluorbenzyl)oxy]-2,6-dimethylimidazo[1,2,a]pyridin-3-carboxamid (Stereoisomerengemisch)

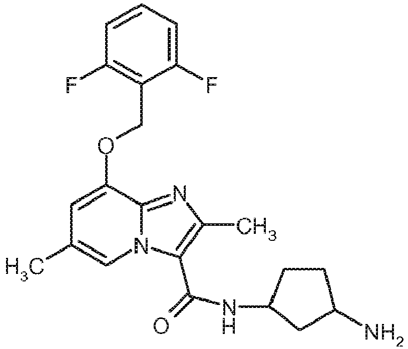

119 mg (0.23 mmol) *tert*-Butyl-{3-[({8-[(2,6-difluorbenzyl)oxy]-2,6-dimethylimidazo[1,2-a]pyridin-3-yl}carbonyl)amino]cyclopentyl}carbamat aus Beispiel 335A wurden in 1.2 ml Diethylether vorgelegt, mit 1.2 ml (2.31 mmol) 2 N Salzsäure in Diethylether versetzt und über Nacht bei RT gerührt. Das Reaktionsgemisch wurde eingeengt, der Rückstand mit Dichlormethan und einem Tropfen Methanol versetzt und zweimal mit gesättigter, wässriger Natriumhydrogencarbonatlösung gewaschen. Die vereinigten wässrigen Phasen wurden zweimal mit Dichlormethan extrahiert und die vereinigten organischen Phasen eingeengt. Es wurden 93 mg der Zielverbindung (92% d. Th.) erhalten.
LC-MS (Methode 2): Rₜ = 0.60 min
MS (ESpos): m/z = 415 (M+H)⁺
¹H-NMR (400 MHz, DMSO-de): δ = 1.37 - 1.50 (m, 2H), 1.67 -1.83 (m, 2H), 1.87 - 2.02 (m, 2H), 2.31 (s, 3H), 2.49 (s, 3H), 3.36 - 3.42 (m, 1H), 4.30 - 4.40 (m, 1H), 5.28 (s, 2H), 6.91 (s, 1H), 7.20 - 7.28 (m, 2H), 7.54 - 7.64 (m, 1H), 8.12 (d, 1H), 8.53 (s,1H).

### Beispiel 309

### ent-N-(3-Aminocyclopentyl)-8-[(2,6-difluorbenzyl)oxy]-2,6-dimethylimidazo[1,2-a]pyridin-3-carboxamid

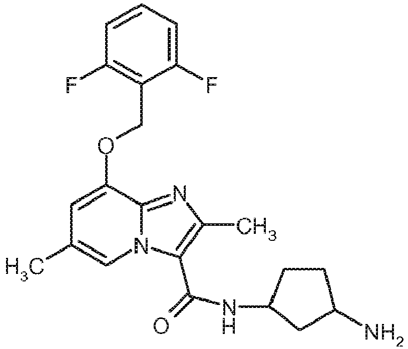

85 mg N-(3-Aminocyclopentyl)-8-[(2,6-difluorbenzyl)oxy]-2,6-dimethylimidazo[1,2-a]pyridin-3-carboxamid aus Beispiel 308 wurden durch präparative Trennung an chiraler Phase in die Stereoisomere getrennt [Säule: Daicel Chiralcel AY-H, 5 µm, 250 x 20 mm, Eluent: 50% Iso-Hexan, 50% Ethanol + 0.2% Diethylamin, Fluß: 15 ml/min; Temperatur: 25°C, Detektion: 220 nm]. Die Produktfraktion wurde nochmals mittels präparativer HPLC gereinigt (RP18 Säule, Laufmittel: Acetonitril/Wasser-Gradient unter Zusatz von 0.1% TFA). Die Produktfraktion wurde in Dichlormethan aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt.
Stereoisomer A: Ausbeute: 15.7 mg (>99% ee)
Rₜ= 4.82 min [Daicel Chiralcel AY-H, 5 µm, 250 x 4.6 mm; Eluent: 50% iso-Hexan, 50% Ethanol + 0.2% Diethylamin; Fluss 1 ml/min; Temperatur: 40°C; Detektion: 220 nm].

### Beispiel 310

### rac-N-[2-Amino-3-(1,3-benzothiazol-2-yl)-2-methylpropyl]-8-[(2,6-difluorbenzyl)oxy]-2,6-dimethylimidazo[1,2-a]pyridin-3-carboxamid

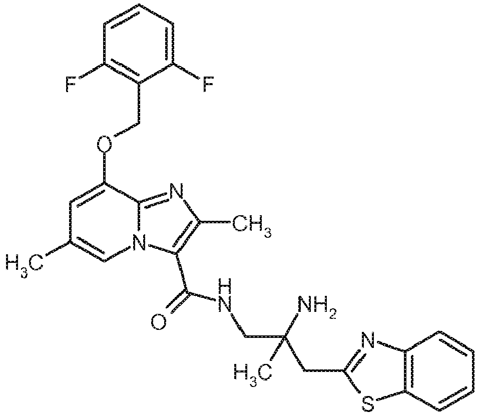

14 mg (0.04 mmol) 8-[(2,6-Difluorbenzyl)oxy]-2,6-dimethylimidazo[1,2-a]pyridin-3-carbonsäure aus Beispiel 21A wurden mit 18 mg (0.05 mmol) HATU und 0.04 ml (0.21 mmol) N,N-Diisopropylethylamin in 0.3 ml DMF vorgelegt, 20 min gerührt, anschließend bei 0°C mit 17 mg (0.07 mmol, Reinheit 94%) *rac*-3-(1,3-Benzothiazol-2-yl)-2-methylpropan-1,2-diamin aus Beispiel 337A versetzt und 60 min bei 0°C gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden im Vakuum eingeengt, der Rückstand dann in Dichlormethan gelöst, zweimal mit gesättigter, wässriger Natriumhydrogencarbonatlösung gewaschen und die vereinigten wässrigen Phasen wurden zweimal mit Dichlormethan extrahiert. Die vereinigten organischen Phasen wurden über Natriumsulfat getrocknet, abfiltriert und eingeengt. Es wurden 16 mg der Zielverbindung (68% d. Th.) erhalten.
LC-MS (Methode 2): Rₜ = 0.82 min
MS (ESpos): m/z = 536 (M+H)⁺
¹H-NMR (400 MHz, DMSO-d₆): δ = 1.11 (s, 3H), 1.18 - 1.29 (m, 2H), 2.31 (s, 3H), 2.58 (s, 3H), 3.16 - 3.27 (m, 2H), 3.38 - 3.45 (m, 2H), 5.29 (s, 2H), 6.90 - 6.95 (m, 1H), 7.20 - 7.29 (m, 2H), 7.37 - 7.43 (m, 1H), 7.44 - 7.50 (m, 1H), 7.54 - 7.64 (m, 1H), 7.78 (t, 1H), 7.95 (d, 1H), 8.05 (d, 1 H), 8.49 (s, 1H).

### Beispiel 311

### rac-N-(2-Amino-2-methylpentyl)-2,6-dimethyl-8-[(2,3,5,6-tetrafluorbenzyl)oxy]imidazo[1,2-a]pyridin-3-carboxamid

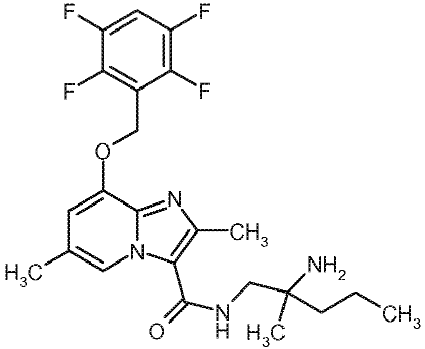

80 mg (0.19 mmol) *rac*-*tert*-Butyl-(1-{[(8-hydroxy-2,6-dimethylimidazo[1,2-a]pyridin-3-yl)carbonyl]amino}-2-methylpentan-2-yl)carbamat aus Beispiel 330A, 50 mg (0.21 mmol) 2,3,5,6-Tetrafluorbenzylbromid, 135 mg (0.41 mmol) Cäsiumcarbonat und 3 mg (0.02 mmol) Kaliumiodid wurden in 4 ml DMF vorgelegt und für 60 min in einem vorgeheizten 60°C warmen Ölbad erhitzt. Die Reaktionslösung wurde eingeengt und über Nacht im Hochvakuum getrocknet. Der Rückstand wurde in 1 ml Diethylether aufgenommen, mit 0.94 ml (1.88 mmol) 2 N Salzsäure in Diethylether versetzt und über Nacht bei RT gerührt. Das Reaktionsgemisch wurde eingeengt und mittels präparativer HPLC gereinigt (RP18 Säule, Laufmittel: Acetonitril/Wasser-Gradient unter Zusatz von 0.1% TFA). Die Produktfraktionen wurden eingeengt, der Rückstand in Dichlormethan aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert und eingeengt. Es wurden 60 mg der Zielverbindung (68% d. Th.) erhalten.
LC-MS (Methode 2): Rₜ = 0.73 min
MS (ESpos): m/z = 467 (M+H)⁺

### Beispiel 312

### rac-8-[(2,6-Difluorbenzyl)oxy]-N-(5,5-difluorpiperidin-3-yl)-2,6-dimethylimidazo[1,2-a]pyridin-3-carboxamid

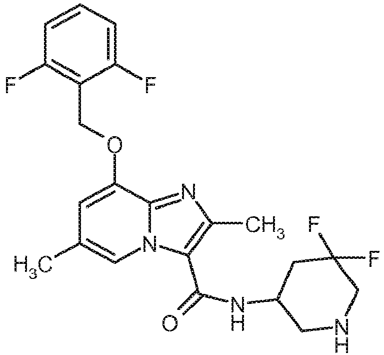

89 mg (0.16 mmol) *rac*-*tert*.-Butyl-5-[({8-[(2,6-difluorbenzyl)oxy]-2,6-dimethylimidazo[1,2-a]pyridin-3-yl}carbonyl)amino]-3,3-difluorpiperidin-1-carboxylat aus Beispiel 338A wurden in 0.8 ml Diethylether vorgelegt, mit 0.78 ml (1.55 mmol) 2 N Salzsäure in Diethylether versetzt und über Nacht bei RT gerührt. Das Reaktionsgemisch wurde eingeengt, der Rückstand mit Dichlormethan und einem Tropfen Methanol versetzt und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, abfiltriert und eingeengt. Es wurden 67 mg der Zielverbindung (91% d. Th.) erhalten.
LC-MS (Methode 2): Rₜ = 0.64 min
MS (ESpos): m/z = 451 (M+H)⁺
¹H-NMR (400 MHz, DMSO-d₆): δ = 1.22 - 1.28 (m, 1H), 1.99 - 2.18 (m, 1H), 2.31 (s, 3H), 2.38 - 2.45 (m, 1H), 2.48 (s, 3H), 2.57 - 2.64 (m, 1H), 2.82 - 2.98 (m, 1H), 3.01 - 3.18 (m, 2H), 4.12 - 4.23 (m, 1H), 5.28 (s, 2H), 6.91 - 6.95 (m, 1H), 7.20 - 7.28 (m, 2H), 7.54 - 7.65 (m, 1H), 7.77 (d, 1H), 8.42 (s, 1H).

### Beispiel 313

### 8-[(2,6-Difluorbenzyl)oxy]-2,6-dimethyl-N-[4-(trifluormethyl)pyrrolidin-3-yl]imidazo[1,2-a]pyridin-3-carboxamid (Mischung von Stereoisomeren)

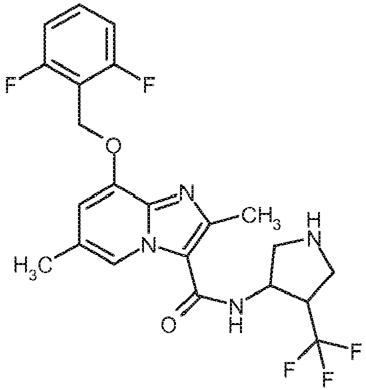

18 mg (0.03 mmol) *rac-tert*-Butyl-3-[({8-[(2,6-difluorbenzyl)oxy]-2,6-dimethylimidazo[1,2-a]pyridin-3-yl}carbonyl)amino]-4-(trifluormethyl)pyrrolidin-1-carboxylat Trifluoracetat (Mischung von Stereoisomeren) aus Beispiel 339A wurden in 0.1 ml Diethylether vorgelegt, mit 0.13 ml (0.26 mmol) 2 N Salzsäure in Diethylether versetzt und über Nacht bei RT gerührt. Das Reaktionsgemisch wurde eingeengt und der Rückstand zwischen Dichlorethan und gesättigter, wässriger Natrium-hydrogencarbonatlösung verteilt. Die wässrige Phase wurde zweimal mit Dichlormethan extrahiert, die vereinten organischen Phasen über Natriumsulfat getrocknet, abfiltriert und das Filtrat eingeengt. Der Rückstand wurde mittels präparativer Dünnschichtchromatographie (Laufmittel: Dichlormethan/Methanol = 20/1) gereinigt. Es wurden 5.8 mg der Zielverbindung (45% d. Th.) erhalten.
LC-MS (Methode 2): Rₜ = 0.74 min
MS (ESpos): m/z = 469 (M+H)⁺
¹H-NMR (400 MHz, DMSO-d₆): d [ppm] = 2.31 (s, 3H), 2.48 (s, 3H), 2.75 - 2.82 (m, 1H), 2.90 - 2.98 (m, 1H), 3.04 - 3.22 (m, 2H), 3.24 - 3.28 (m, 2H), 4.51 - 4.59 (m, 1H), 5.29 (s, 2H), 6.93 (s, 1H), 7.19 - 7.27 (m, 2H), 7.54 - 7.63 (m, 1H), 8.06 (d, 1H), 8.39 (s,1H).

### Beispiel 314

### 8-[(2,6-Difluorbenzyl)oxy]-2,6-dimethyl-N-[(7S,8aS)-2-methyloctahydropyrrolo[1,2-a]pyrazin-7-yl]imidazo[1,2-a]pyridin-3-carboxamid

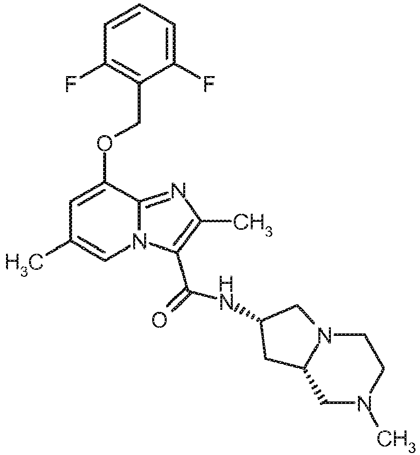

75 mg (0.23 mmol) 8-[(2,6-Difluorbenzyl)oxy]-2,6-dimethylimidazo[1,2-a]pyridin-3-carbonsäure aus Beispiel 21A wurden mit 111 mg (0.29 mmol) HATU und 0.20 ml (1.13 mmol) N,N-Diisopropylethylamin in 2 ml DMF vorgelegt, 10 min gerührt, anschließend bei RT mit 88 mg (0.29 mmol) (7S,8aS)-2-Methyloctahydropyrrolo[1,2-a]pyrazin-7-amintrihydrochloriddihydrat (kommerziell erhältlich, CAS Registry Nr: 1268326-45-9) versetzt und über Nacht bei RT gerührt. Die Reaktionslösung wurde mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereint, das Lösungsmittel eingeengt und lyophilisiert. Der Rückstand wurde in Dichlormethan aufgenommen und einmal mit gesättigter, wässriger Natriumhydrogencarbonatlösung gewaschen und die wässrige Phase zweimal mit Dichlormethan extrahiert. Die vereinten organischen Phasen wurden über Natriumsulfat getrocknet, filtriert, das Filtat eingeengt und lyophilisiert. Es wurden 81 mg der Zielverbindung (75% d. Th.) erhalten.
LC-MS (Methode 2): Rₜ = 0.62 min
MS (ESpos): m/z = 470 (M+H)⁺
¹H-NMR (400 MHz, DMSO-d₆): δ = 1.36 - 1.49 (m, 1H), 1.84 - 2.05 (m, 1H), 2.06 - 2.28 (m, 5H), 2.30 (s, 3H), 2.45 (s, 3H), 2.72 - 2.84 (m, 1H), 2.86 - 3.00 (m, 3H), 4.40 (quin, 1H), 5.28 (s, 2H), 6.90 (s,1H), 7.24 (quin, 2H), 7.54 - 7.64 (m, 1H), 8.06 (d, 1H), 8.35 (s, 1H).

### Beispiel 315

### ent-N-(1-Amino-2-methylbutan-2-yl)-2,6-dimethyl-8-[(2,3,6-trifluorbenzyl)oxy]imidazo[1,2-a]pyridin-3-carboxamid

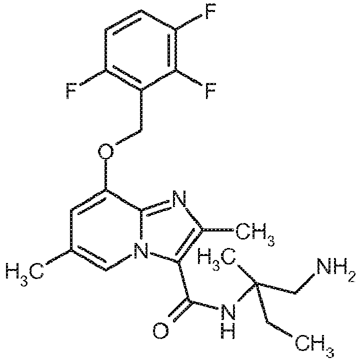

165 mg (0.24 mmol) Benzyl-{2-[({2,6-dimethyl-8-[(2,3,6-trifluorbenzyl)oxy]imidazo[1,2-a]pyridin-3-yl}carbonyl)amino]-2-methylbutyl}carbamat aus Beispiel 340A wurden in 2.5 ml Ethanol gelöst und unter Argon mit 17 mg (0.02 mmol) 20%-igem Palladium(II)hydroxid auf Kohle versetzt. Anschließend wurde 2.5 Stunden bei RT unter Normaldruck hydriert. Das Reaktionsgemisch wurde auf Celite aufgezogen und mittels Kieselgel-Chromatographie gereinigt (Laufmittel: Dichlormethan/2 N Ammoniak in Methanol = 40/1). Es wurden 90 mg der Zielverbindung (84% d. Th.) erhalten.
LC-MS (Methode 2): Rₜ = 0.68 min
MS (ESpos): m/z = 435 (M+H)⁺
¹H-NMR (500 MHz, DMSO-d₆): δ = 0.83 (t, 3H), 1.28 (s, 3H), 1.57 -1.72 (m, 1H), 1.73 -1.86 (m, 2H), 2.31 (s, 3H), 2.61 (d, 1H), 2.84 (d, 1H), 5.34 (s, 2H), 6.90 (d, 1H), 7.18 (s, 1H), 7.25 - 7.32 (m, 1H), 7.62 - 7.71 (m, 1H), 8.49 (s, 1H).

### Beispiel 316

### rac-N-(Azepan-3-yl)-8-[(2,6-difluorbenzyl)oxy]-2,6-dimethylimidazo[1,2-a]pyridin-3-carboxamid

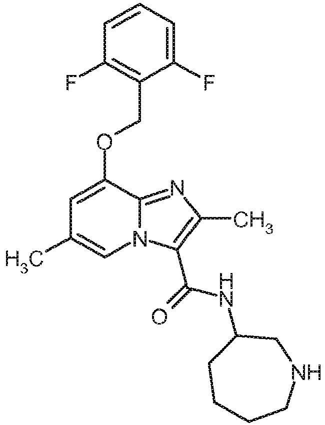

193 mg (0.30 mmol) *rac-tert*-Butyl-3-[({8-[(2,6-difluorbenzyl)oxy]-2,6-dimethylimidazo[1,2-a]pyridin-3-yl}carbonyl)amino]azepan-1-carboxylat Trifluoracetat aus Beispiel 341A wurden mit 1.5 ml (3.00 mmol) 2 N Chlorwasserstoff in Diethylether versetzt und über Nacht bei RT gerührt. Das Reaktionsgemisch wurde eingeengt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan gelöst und einmal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, das Filtrat eingeengt und lyophilisiert. Es wurden 114 mg der Zielverbindung (89% d. Th.) erhalten.
LC-MS (Methode 2): Rₜ = 0.63 min
MS (ESpos): m/z = 429 (M+H)⁺
¹H-NMR (400 MHz, DMSO-d₆): δ = 1.43 -1.74 (m, 5H), 1.78 - 1.88 (m, 1H), 2.31 (s, 3H), 2.48 (s, 3H), 2.66 - 2.73 (m, 1H), 2.77 (t, 2H), 2.92 - 2.99 (m, 1H), 3.98 - 4.08 (m, 1H), 5.28 (s, 2H), 6.90 (s, 1H), 7.20 - 7.28 (m, 2H), 7.55 - 7.68 (m, 2H), 8.45 (s, 1H).

### Beispiel 317

### rac-N-(2-Amino-2-methylpentyl)-8-[(2-fluor-3-methylbenzyl)oxy]-2,6-dimethylimidazo[1,2-a]pyridin-3-carboxamid

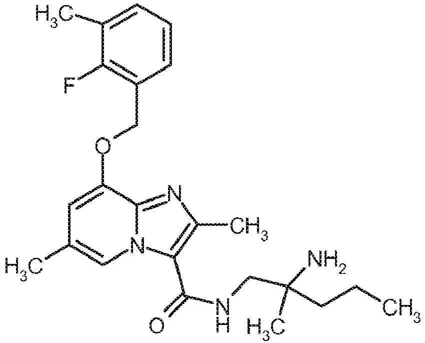

90 mg (0.21 mmol) *rac-tert*-Butyl-(1-{[(8-hydroxy-2,6-dimethylimidazo[1,2-a]pyridin-3-yl)carbonyl]amino}-2-methylpentan-2-yl)carbamat aus Beispiel 330A, 47 mg (0.23 mmol) 2-Fluoro-3-methylbenzylbromid, 152 mg (0.46 mmol) Cäsiumcarbonat und 3.5 mg (0.02 mmol) Kaliumiodid wurden in 4 ml DMF vorgelegt und für 30 min in einem vorgeheizten 60°C warmen Ölbad erhitzt. Die Reaktionslösung wurde eingeengt und über Nacht im Hochvakuum getrocknet. Der Rückstand wurde in 1 ml Diethylether aufgenommen, mit 1.06 ml (2.11 mmol) 2 N Salzsäure in Dimethylether versetzt und über Nacht bei RT gerührt. Das Reaktionsgemisch wurde eingeengt und mittels präparativer HPLC gereinigt (RP18 Säule, Laufmittel: Methanol/Wasser-Gradient unter Zusatz von 0.1% TFA). Die Produktfraktionen wurden eingeengt, der Rückstand in Dichlormethan aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert und eingeengt. Es wurden 48 mg der Zielverbindung (53% d. Th.) erhalten.
LC-MS (Methode 2): Rₜ = 0.72 min
MS (ESpos): m/z = 427 (M+H)⁺

### Beispiel 318

### ent-N-(2-Amino-2-methylpentyl)-8-[(2,6-difluorbenzyl)oxy]-2-methylimidazo[1,2-a]pyridin-3-carboxamid

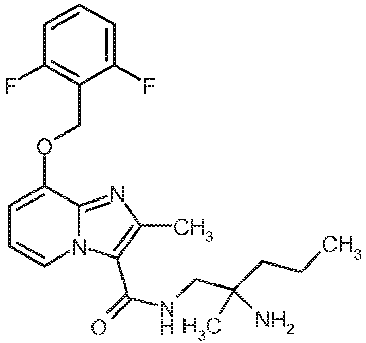

50 mg (0.07 mmol) *ent*-Benzyl-{1-[({6-brom-8-[(2,6-difluorbenzyl)oxy]-2-methylimidazo[1,2-a]pyridin-3-yl}carbonyl)amino]-2-methylpentan-2-yl}carbamat aus Beispiel 292A wurden unter Argon in 0.7 ml Ethanol vorgelegt, mit 7 mg (0.01 mmol, 10%ig) Palladium auf Kohle versetzt und 1 Stunde unter Normaldruck bei RT hydriert. Das Reaktionsgemisch wurde auf Celite überführt und mittels Kieselgelchromatographie gereinigt (Laufmittel Dichlormethan/2 N Ammoniak in Methanol = 60/1). Es wurden 27 mg der Zielverbindung (94% d. Th.) erhalten.
LC-MS (Methode 2): Rₜ = 0.61 min
MS (ESpos): m/z = 417 (M+H)⁺
¹H-NMR (400 MHz, DMSO-d₆): δ = 0.86 (t, 3H), 0.99 (s, 3H), 1.21-1.42 (m, 4H), 1.43 -1.72 (m, 2H), 2.56 (s, 3H), 3.14 - 3.27 (m, 2H), 5.31 (s, 2H), 6.94 (t, 1H), 7.00 (d, 1H), 7.20 - 7.28 (m, 2H), 7.55 - 7.63 (m, 1H), 7.64 - 7.69 (m, 1H), 8.64 (d,1H).

### Beispiel 319

### Methyl-3-[({8-[(2,6-difluorbenzyl)oxy]-2,6-dimethylimidazo[1,2-a]pyridin-3-yl}carbonyl)amino]-L-alaninat

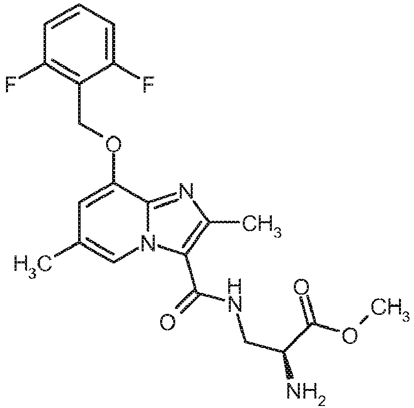

75 mg (0.14 mmol) Methyl-N-(tert-butoxycarbonyl)-3-[({8-[(2,6-difluorbenzyl)oxy]-2,6-dimethylimidazo[1,2-a]pyridin-3-yl}carbonyl)amino]-L-alaninat aus Beispiel 351A wurden in 0.7 ml Diethylether vorgelegt, mit 0.67 ml (1.35 mmol) 2 N Salzsäure in Diethylether versetzt und über Nacht bei RT gerührt. Die Reaktionslösung wurde eingeengt, der Rückstand in Dichlormethan gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, fitlriert und eingeengt. Es wurden 55 mg der Zielverbindung (92% d. Th.) erhalten.
LC-MS (Methode 2): Rₜ = 0.63 min
MS (ESpos): m/z = 433 (M+H)⁺
¹H-NMR (400 MHz, DMSO-d₆): δ = 1.84 - 2.03 (m, 2H), 2.31 (s, 3H), 2.48 (s, 3H), 3.46 - 3.51 (m, 2H), 3.56 - 3.61 (m, 1H), 3.63 (s, 3H), 5.28 (s, 2H), 6.89 - 6.94 (m, 1H), 7.19 - 7.27 (m, 2H), 7.53 - 7.63 (m, 1H), 7.81 (t, 1H), 8.41 - 8.46 (m, 1H).

### Beispiel 320

### rac-N-(2-Amino-1,2-dimethylcyclopropyl)-8-[(2,6-difluorbenzyl)oxy]-2,6-dimethylimidazo[1,2-a]pyridin-3-carboxamid (Racemat)

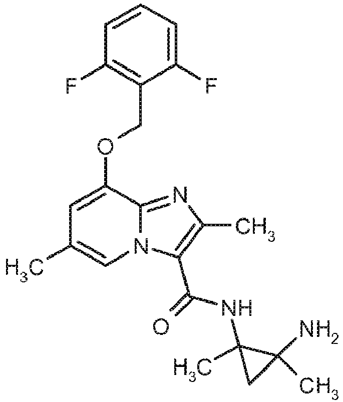

631 mg (2.41 mmol) *rac-*1,2-Dimethylcyclopropan-1,2-diamindihydrobromid (beschrieben in: W. v. d. Saal et al. Liebigs Annalen der Chemie 1994, 569-580) wurden mit 0.94 ml DMF und 1.26 ml (7.22 mmol) N,N-Diisopropylethylamin versetzt und auf 60°C erhitzt. Eine Lösung aus 200 mg (0.60 mmol) 8-[(2,6-Difluorbenzyl)oxy]-2,6-dimethylimidazo[1,2-a]pyridin-3-carbonsäure aus Beispiel 21A, 0.21 ml (1.20 mmol) N,N-Diisopmpylethylamin und 275 mg (0.72 mmol) HATU in 2.8 ml DMF, welche vorher 10 min bei RT gerührt wurden war, wurde zur Reaktionsmischung zugetropft und es wurde 30 min bei 60°C gerührt. Das Gemisch wurde mit Wasser und TFA versetzt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlorethan aufgenommen und zweimal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, das Filtrat eingeengt und der Rückstand eingeengt. Es wurden 175 mg der Zielverbindung (70% d. Th.) erhalten.
LC-MS (Methode 2): Rₜ = 0.58 min
MS (ESpos): m/z = 415 (M+H)⁺
¹H NMR (400 MHz, DMSO-*d₆*) δ = 0.54 (d, 1 H), 0.67 (d, 1 H), 1.32 (s, 3 H), 1.49 (s, 3 H), 1.79 (br. s, 2 H), 2.31 (s, 3 H), 5.29 (s, 2 H), 6.89 (s, 1 H), 7.23 (t, 2 H), 7.54 - 7.63 (m, 1 H), 7.85 (s, 1 H), 8.41 - 8.45 (m, 1 H), [weiteres Signal unter Lösungsmittelpeak verborgen].

### Beispiel 321

### rac-N-{2-Amino-2-methyl-3-[1-(5-methyl-1,2-oxazol-3-yl)-1H-1,2,4-triazol-3-yl]propyl}-8-[(2,6-difluorbenzyl)oxy]-2,6-dimethylimidazo[1,2-a]pyridin-3-carboxamid

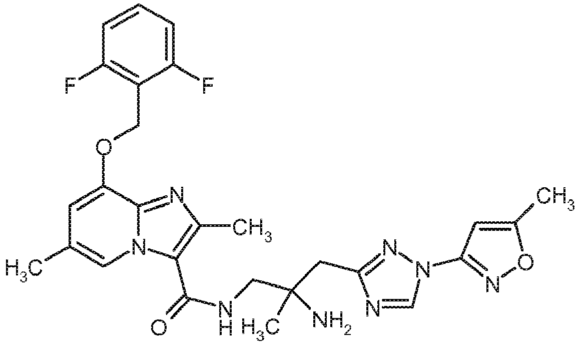

114 mg (0.34 mmol) 8-[(2,6-Difluorbenzyl)oxy]-2,6-dimethylünidazo[1,2-a]pyridin-3-carbonsäure aus Beispiel 21A wurden mit 143 mg (0.38 mmol) HATU und 0.18 ml (1.03 mmol) N,N-Diisopropylethylamin in 2.2 ml DMF vorgelegt, 20 min gerührt, anschließend bei 0°C mit 105 mg (0.44 mmol) *rac*-2-Methyl-3-[1-(5-methyl-1,2-oxazol-3-yl)-1H-1,2,4-triazol-3-yl]propan-1,2-diamin aus Beispiel 353A versetzt und 60 min bei 0°C gerührt. Das Reaktionsgemisch wurde mittels präparativer HPLC gereinigt (RP18 Säule, Laufmittel: Acetonitril/Wasser-Gradient unter Zusatz von 0.1% TFA). Die eingeengten Produktfraktionen wurden zwischen Dichlormethan und gesättigter, wässriger Natriumhydrogencarbonatlösung verteilt. Die wässrige Phase wurde noch zweimal mit Dichlorethan extrahiert, die vereinten organischen Phasen über Natriumsulfat getrocknet, filtriert, das Filtrat eingeengt und lyophilisiert. Es wurden 73 mg der Zielverbindung (38% d. Th.) erhalten.
LC-MS (Methode 2): Rₜ = 0.74 min
MS (ESpos): m/z = 551 (M+H)⁺
¹H-NMR (400 MHz, DMSO-d₆): δ = 1.11 (s, 3H), 1.32 - 1.46 (m, 1H), 2.30 (s, 3H), 2.49 (br. s., 3H), 2.57 (s, 3H), 2.89 (s, 2H), 3.36 - 3.43 (m, 1H), 5.29 (s, 2H), 6.73 - 6.78 (m, 1H), 6.92 (s, 1H), 7.19 - 7.29 (m, 2H), 7.53 - 7.67 (m, 2H), 8.52 (s, 1H), 9.21 (s, 1H).

### Beispiel 322

### ent-N-(2-Amino-2-methylpentyl)-2-cyclopropyl-8-[(2,6-difluorbenzyl)oxy]-6-methylimidazo[1,2-a]pyridin-3-carboxamid

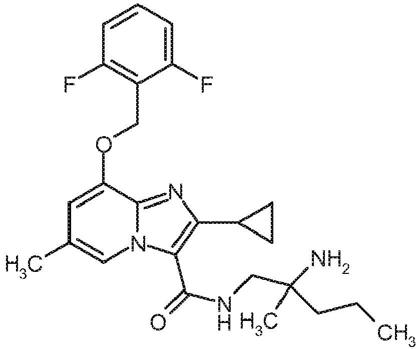

74 mg (0.10 mmol) *ent*-Benzyl-{1-[({2-cyclopropyl-8-[(2,6-difluorbenzyl)oxy]-6-methylimidazo[1,2-a]pyridin-3-yl}carbonyl)amino]-2-methylpentan-2-yl}carbamat Trifluoracetat aus Beispiel 357A wurdem unter Argon in 1 ml Ethanol vorgelegt, mit 11 mg (0.01 mmol) 10%-igenPalladium auf Kohle versetzt und 1 Stunde unter Normaldruck bei RT hydriert. Das Reaktionsgemisch wurde über einen Millipore-Filter filtriert, mit Ethanol gewaschen und eingeengt. Das Produkt wurde in Dichlormethan gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 41 mg der Zielverbindung (82% d. Th.) erhalten.
LC-MS (Methode 2): Rₜ = 0.78 min
MS (ESpos): m/z = 457 (M+H)⁺
¹H-NMR (400 MHz, DMSO-d₆): δ = 0.86 (t, 3H), 0.92 - 0.98 (m, 4H), 1.00 (s, 3H), 1.21 -1.42 (m, 4H), 1.54 - 2.00 (br. s, 2H), 2.25 - 2.34 (m, 4H), 3.15 - 3.29 (m, 2H), 5.28 (s, 2H), 6.92 (s, 1H), 7.19 - 7.28 (m, 2H), 7.54 - 7.64 (m, 1H), 7.75 (t,1H), 8.54 (s,1H).

### Beispiel 323

### ent-N-(2-Amino-2-methylpentyl)-8-[(2,6-difluorbenzyl)oxy]-6-ethyl-2-methylimidazo[1,2-a]pyridin-3-carboxamid

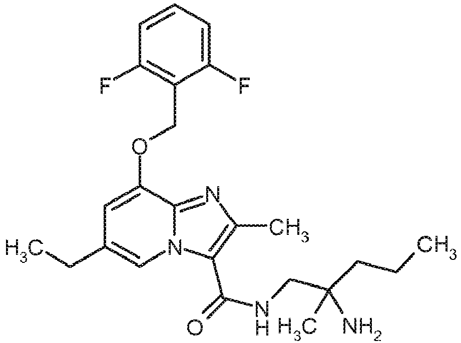

25 mg (0.04 mmol, Reinheit 76%) *ent-*Benzyl-{1-[({8-[(2,6-difluorbenzyloxy]-6-thinyl-2-methylimidazo[1,2-a]pyridin-3-yl}carbonyl)amino]-2-methylpentan-2-yl}carbamat aus Beispiel 359A wurden in 0.5 ml Ethanol gelöst und unter Argon mit 2.3 mg (0.002 mmol) 10%-igen Palladium auf Kohle versetzt. Anschließend wurde das Reaktionsgemisch 2 Stunden bei RT unter Normaldruck hydriert. Das Reaktionsgemisch wurde über einen Milliporfilter filtriert, mit Ethanol gewaschen, das Filtrat eingeengt und im Hochvakuum getrocknet. Der Rückstand wurde mittels präparativer Dünnschicht-Chromatographie gereinigt (Laufmittel: Dichlormethan/2 N Ammoniak in Methanol = 40/1). Es wurden 2.8 mg der Zielverbindung (13% d. Th., Reinheit 92%) erhalten.
LC-MS (Methode 2): Rₜ = 0.72 min
MS (ESpos): m/z = 445 (M+H)⁺
¹H-NMR (500 MHz, DMSO-d₆): δ = 1.04 (s, 3H), 1.23 (t, 6H), 1.30 -1.40 (m, 6H), 1.42 -1.57 (m, 2H), 2.54 (s, 3H), 2.60 - 2.66 (m, 2H), 5.32 (s, 2H), 6.94 - 6.96 (m, 1H), 7.20 - 7.26 (m, 2H), 7.55 - 7.62 (m, 1H), 7.63 - 7.72 (m, 1H), 8.48 - 8.51 (m, 1H).

### Beispiel 324

### N-[(1R,3S)-3-Amino-2,2,3-trimethylcyclopentyl]-8-[(2,6-difluorbenzyl)oxy]-2,6-dimethylimidazo[1,2,a]pyridin-3-carboxamid

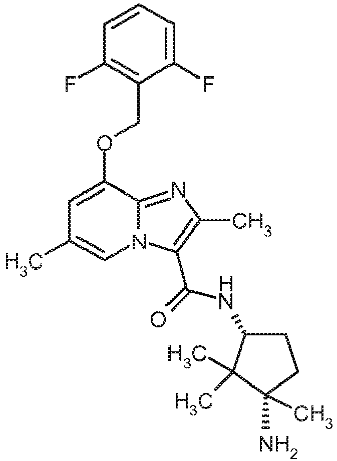

75 mg (0.23 mmol) 8-[(2,6-Difluorbenzyl)oxy]-2,6-dimethylimidazo[1,2-a]pyridin-3-carbonsäure aus Beispiel 21A wurden mit 112 mg (0.29 mmol) HATU und 0.20 ml (1.13 mmol) N,N-Diisopropylethylamin in 2.2 ml DMF vorgelegt, 10 min gerührt, anschließend bei RT mit 63 mg (0.29 mmol) (1*S*,3*R*)-1,2,2-Trimethylcyclopentan-1,3-diamin Dihydrochlorid versetzt und eine Stunde bei RT gerührt. Die Reaktionslösung wurde mit TFA versetzt und mittels präparativer HPLC gereinigt (RP18 Säule, Laufmittel: Acetonitril/Wasser-Gradient unter Zusatz von 0.1% TFA). Die Produktfraktionen wurden eingeengt und am Hochvakuum getrocknet. Der Rückstand wurde in Dichlormethan aufgenommen, einmal mit gesättigter, wässriger Natriumhydrogencarbonatlösung gewaschen und die wässrige Phase zweimal mit Dichlormethan extrahiert. Die vereinten organischen Phasen wurden über Natriumsulfat getrocknet, filtriert und das Filtrat eingeengt. Es wurden 90 mg der Zielverbindung (85% d. Th.) erhalten.
LC-MS (Methode 2): Rₜ = 0.62 min
MS (ESpos): m/z = 457 (M+H)⁺
¹H-NMR (400 MHz, DMSO-d₆): δ = 0.86 - 0.92 (m, 6H), 1.10 (s, 3H), 1.56 - 1.66 (m, 1H), 1.70 (t, 2H), 2.07 - 2.17 (m, 1H), 2.31 (s, 3H), 2.53 (s, 3H), 4.18 - 4.27 (m, 1H), 5.28 (s, 2H), 6.92 (s, 1H), 7.20 - 7.28 (m, 2H), 7.54 - 7.64 (m, 1H), 8.38 (d,1H), 8.65 (s, 1H).

### Beispiel 325

### ent-8-[(2,6-Difluorbenzyl)oxy]-N-[2-ethyl-6-(trifluormethyl)-1,2,3,4-tetrahydro-1,5-naphthyridin-4-yl]-2,6-dimethylimidazo[1,2-a]pyridin-3-carboxamid

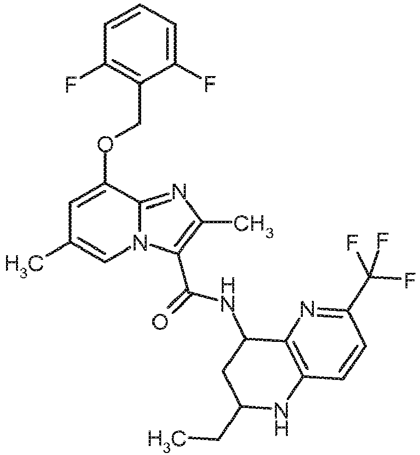

50 mg (0.15 mmol) 8-[(2,6-Difluorbenzyl)oxy]-2,6-dimethylimidazo[1,2-a]pyridin-3-carbonsäure aus Beispiel 21A wurden mit 69 mg (0.18 mmol) HATU und 0.13 ml (0.75 mmol) N,N-Diisopropylethylamin in 1.5 ml DMF vorgelegt, 10 min gerührt, anschließend bei RT mit 51mg (0.18 mmol) *ent*-2-Ethyl-6-(trifluormethyl)-1,2,3,4-tetrahydro-1,5-naphthyridin-4-amin Hydrochlorid aus Beispiel 361A versetzt und 2.5 Stunden bei RT gerührt. Das Reaktionsgemisch wurde mit Wasser versetzt, der entstandene Feststoff abfiltriert und am Hochvakuum getrocknet. Es wurden 66 mg der Zielverbindung (76% d. Th.) erhalten.
LC-MS (Methode 2): Rₜ = 1.12 min
MS (ESpos): m/z = 560 (M+H)⁺
¹H-NMR (400 MHz, DMSO-d₆): δ = 0.99 (t, 3H), 1.48 -1.72 (m, 3H), 2.32 (s, 3H), 2.36 - 2.44 (m, 1H), 2.57 (s, 3H), 3.49 - 3.59 (m, 1H), 5.20 - 5.27 (m, 1H), 5.29 (s, 2H), 6.70 (s, 1H), 6.93 (s, 1H), 7.02 (d, 1H), 7.20 - 7.29 (m, 2H), 7.43 (d, 1H), 7.54 - 7.65 (m, 1H), 8.09 (d, 1H), 8.50 (s, 1H).

### Beispiel 326

### ent-8-[(2,6-Difluorbenzyl)oxy]-2,6-dimethyl-N-[2-methyl-6-(trifluormethyl)-1,2,3,4-tetrahydro-1,5-naphthyridin-4-yl]imidazo[1,2-a]pyridin-3-carboxamid

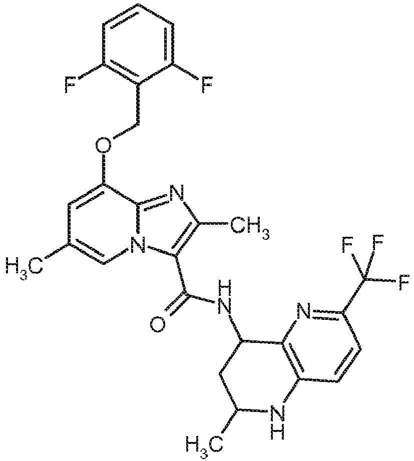

49 mg (0.15 mmol) 8-[(2,6-Difluorbenzyl)oxy]-2,6-dimethylimidazo[1,2-a]pyridin-3-carbonsäure aus Beispiel 21A wurden mit 67 mg (0.18 mmol) HATU und 0.13 ml (0.74 mmol) N,N-Diisopropylethylamin in 1.5 ml DMF vorgelegt, bei RT mit 47 mg (0.18 mmol) *ent*-2-Methyl-6-(trifluormethyl)-1,2,3,4-tetrahydro-1,5-naphthyridin-4-amin Hydrochlorid aus Beispiel 363A versetzt und 3 Stunden bei RT gerührt. Das Reaktionsgemisch wurde mit Wasser versetzt, der entstandene Feststoff abfiltriert und im Hochvakuum getrocknet. Es wurden 63 mg der Zielverbindung (74% d. Th.) erhalten.
LC-MS (Methode 2): Rₜ = 1.06 min
MS (ESpos): m/z = 546 (M+H)⁺
¹H-NMR (400 MHz, DMSO-d₆): δ = 0.91 - 0.97 (m, 1H), 1.23 (d, 3H),1.62 -1.73 (m,1H), 2.32 (s, 3H), 2.56 (br. s., 3H), 3.66 - 3.78 (m, 1H), 5.20 - 5.27 (m, 1H), 5.29 (s, 2H), 6.76 - 6.82 (m, 1H), 6.90 - 6.99 (m, 2H), 7.19 - 7.29 (m, 2H), 7.43 (d, 1H), 7.55 - 7.64 (m, 1H), 8.07 - 8.13 (m, 1H), 8.48 (s, 1H).

### Beispiel 327

### ent- N-(2-Amino-2-methylbutyl)-8-[(2,6-difluorbenzyl)oxy]-6-methyl-2-propylimidazo[1,2-a]pyridin-3-carboxamid

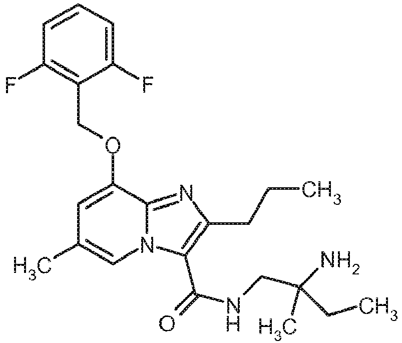

Unter Argon wurden 107 mg (0.18 mmol) *ent*-Benzyl-{1-[({8-[(2,6-difluorbenzyl)oxy]-6-methyl-2-propylimidazo[1,2-a]pyridin-3-yl}carbonyl)amino]-2-methylbutan-2-yl}carbamat aus Beispiel 366A in 3 ml DMF vorgelegt, mit 15 mg 10%-igem Palladium auf Aktiv-Kohle versetzt und 2 Stunden unter Normaldruck bei RT hydriert. Das Reaktionsgemisch wurde über Celite filtriert, gut mit DMF nachgewaschen und eingeengt. Der Rückstand wurde in Ethanol aufgenommen, erneut über Celite filtriert und eingeengt. Der Rückstand wurde in Dichlormethan gelöst und mittels Kieselgel-Chromatographie gereinigt (Laufmittel: Dichlormethan/7 N Ammoniak in Methanol: 1/0 nach 5/1). Es wurden 77 mg der Zielverbindung (93% d. Th.) erhalten.
LC-MS (Methode 2): Rₜ = 0.71 min
MS (ESpos): m/z = 445 (M+H)⁺
¹H-NMR (400 MHz, DMSO-d₆): δ = 0.82 - 0.93 (m, 6H), 1.00 (s, 3H), 1.31 - 1.45 (m, 2H), 1.60 - 1.72 (m, 2H), 2.30 (s, 3H), 2.86 (t, 2H), 3.15 - 3.28 (m, 2H), 5.29 (s, 2H), 6.91 (s, 1H), 7.24 (t, 2H), 7.55 - 7.64 (m, 1H), 7.70 (t,1H), 8.41 (s, 1H).

### Beispiel 328

### ent-N-(2-Aminocyclobutyl)-8-[(2,6-difluorbenzyl)oxy]-2,6-dimethylimidazo[1,2-a]pyridin-3-carboxamid (Enantiomer A)

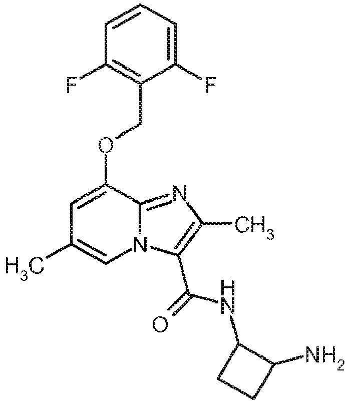

77 mg (0.15 mmol) *ent-tert*-Butyl-{2-[({8-[(2,6-difluorbenzyl)oxy]-2,6-dinethylimidazo[1,2-a]pyridin-3-yl}carbonyl)amino]cyclobutyl}carbamat (Entantiomer A) aus Beispiel 368A wurden in 0.8 ml Diethylether vorgelegt, mit 0.77 ml (1.54 mmol) 2 N Salzsäure in Diethylether versetzt und über Nacht bei RT gerührt. Die Reaktionslösung wurde eingeengt, der Rückstand in Dichlormethan aufgenommen und zweimal mit gesättigter, wässriger Natriumhydrogencarbonat-Lösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 56 mg der Zielverbindung (89% d. Th.) erhalten.
LC-MS (Methode 2): Rₜ = 0.60 min
MS (ESpos): m/z = 401 (M+H)⁺
¹H-NMR (400 MHz, DMSO-d₆): δ = 1.32 - 1.45 (m, 1H), 1.47 -1.59 (m, 1H), 1.92 - 2.03 (m, 2H), 2.31 (s, 3H), 2.49 (br. s., 3H), 3.25 - 3.30 (m, 1H), 4.00 - 4.11 (m, 1H), 5.28 (s, 2H), 6.91 (s, 1H), 7.20 - 7.28 (m, 2H), 7.55 - 7.64 (m, 1H), 8.00 (d, 1H), 8.41 (s, 1H).

### Beispiel 329

### ent-N-(2-Aminocyclobutyl)-8-[(2,6-difluorbenzyl)oxy]-2,6-dimethylimidazo[1,2-a]pyridin-3-carboxamid (Enantiomer B)

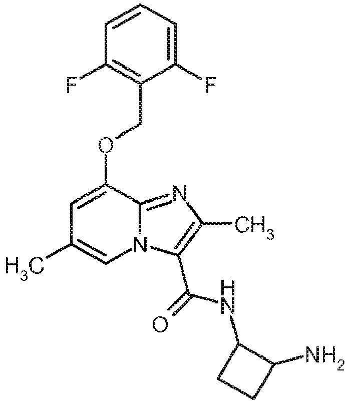

67 mg (0.13 mmol) *ent-tert*-Butyl-{2-[({8-[(2,6-difluorbenzyl)oxy]-2,6-dimethylimidazo[1,2-a]pyridin-3-yl}carbonyl)amino]cyclobutyl}carbamat (Enantiomer B) aus Beispiel 369A wurden in 0.7 ml Diethylether vorgelegt, mit 0.67 ml (1.34 mmol) 2 N Salzsäure in Diethylether versetzt und über Nacht bei RT gerührt. Die Reaktionslösung wurde eingeengt, der Rückstand in Dichlormethan aufgenommen und zweimal mit gesättigter, wässriger Natriumhydrogencarbonat-Lösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 51 mg der Zielverbindung (94% d. Th.) erhalten.
LC-MS (Methode 2): Rₜ = 0.60 min
MS (ESpos): m/z = 401 (M+H)⁺
¹H-NMR (400 MHz, DMSO-d₆): δ = 1.32 - 1.45 (m, 1H), 1.47 -1.59 (m, 1H), 1.92 - 2.03 (m, 2H), 2.31 (s, 3H), 2.49 (br. s., 3H), 3.25 - 3.30 (m, 1H), 4.00 - 4.11 (m, 1H), 5.28 (s, 2H), 6.91 (s, 1H), 7.20 - 7.28 (m, 2H), 7.55 - 7.64 (m, 1H), 8.00 (d, 1H), 8.41 (s, 1H).

### Beispiel 330

### rac-N-(2-Aminocyclopropyl)-8-[(2,6-difluorbenzyl)oxy]-2,6-dimethylimidazo[1,2-a]pyridin-3-carboxamid

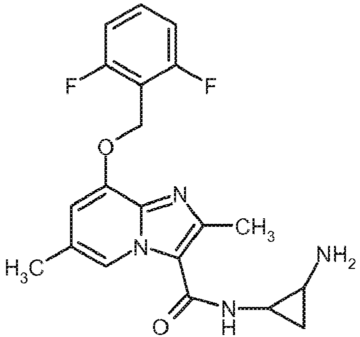

150 mg (0.45 mmol) 8-[(2,6-Difluorbenzyl)oxy]-2,6-dimethylimidazo[1,2-a]pyridin-3-carbonsäure aus Beispiel 21A wurden mit 206 mg (0.54 mmol) HATU und 0.47 ml (2.71 mmol) N,N-Diisopropylethylamin in 1.34 ml DMF vorgelegt und 10 min bei RT gerührt. Das Reaktionsgemisch wurde langsam zu 0.54 ml (1.13 mmol) *rac*-Cyclopropan-1,2-diamin Hydrochlorid in 0.45 ml DMF getropft und über Nacht bei RT gerührt. Die Reaktionslösung wurde mit TFA versetzt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt und der Rückstand wurde mittels Dickschichtchromatographie gereinigt (Laufmittel: Dichlormethan/2N Ammoniak in Methanol = 10/1). Es wurden 18 mg der Zielverbindung (10% d. Th.) erhalten.
LC-MS (Methode 2): Rₜ = 0.58 min
MS (ESpos): m/z = 387 (M+H)⁺
¹H-NMR (400 MHz, DMSO-d₆): δ = 0.66 - 0.72 (m, 1H), 0.73 - 0.79 (m, 1H), 1.85 -1.95 (m, 2H), 2.31 (s, 3H), 2.33 - 2.36 (m, 1H), 2.40 (s, 3H), 2.60 - 2.66 (m, 1H), 5.27 (s, 2H), 6.90 (s,1H), 7.19 - 7.28 (m, 2H), 7.54 - 7.64 (m, 1H), 7.82 (d, 1H), 8.40 (s,1H).

### Beispiel 331

### rac-N-(2-Amino-2-methylcyclobutyl)-8-[(2,6-difluorbenzyl)oxy]-2,6-dimethylimidazo[1,2-a]pyridin-3-carboxamid

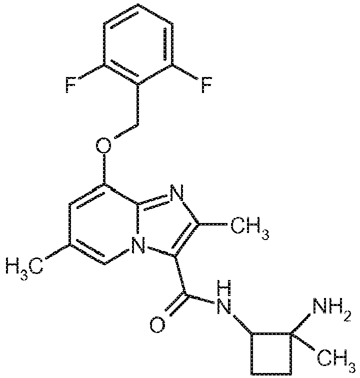

150 mg (0.45 mmol) 8-[(2,6-Difluorbenzyl)oxy]-2,6-dimethylimidazo[1,2-a]pyridin-3-carbonsäure aus Beispiel 21A wurden mit 206 mg (0.54 mmol) HATU und 0.47 ml (2.71 mmol) N,N-Diisopropylethylamin in 1.34 ml DMF vorgelegt und 10 min bei RT gerührt. Das Reaktionsgemisch wurde langsam zu 0.54 ml (1.81 mmol) *rac*-1-Methylcyclobutan-1,2-diamindihydrochlorid (beschrieben in: K.-H. Scholz; J. Hinz; H.-G. Heine; W. Hartmann, Liebigs Annalen der Chemie, 1981, 248-255; Duschinsky; Dolan Journal of the American Chemical Society, 1945, 67, 2079, 2082) in 0.9 ml DMF getropft und über Nacht bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die eingeengten Fraktionen wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 100 mg der Zielverbindung (52% d. Th.) erhalten.
LC-MS (Methode 2): Rₜ = 0.58 min
MS (ESpos): m/z = 415 (M+H)⁺
¹H-NMR (400 MHz, DMSO-d₆): δ = 1.12 (s, 3H), 1.57 - 1.73 (m, 3H), 1.88 - 1.97 (m, 1H), 1.98 - 2.07 (m, 2H), 2.31 (s, 3H), 2.51 (br. s., 3H), 4.20 (q, 1H), 5.28 (s, 2H), 6.90 (s, 1H), 7.19 - 7.28 (m, 2H), 7.54 - 7.64 (m,1H), 7.76 (d,1H), 8.38 (s, 1H).

### Beispiel 332

### ent- N-[2-Amino-2-methylcyclobutyl]-8-[(2,6-difluorbenzyl)oxy]-2,6-dimethylimidazo[1,2-a]pyridin-3-carboxamid (Enantiomer A)

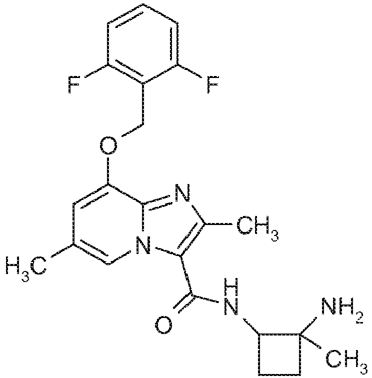

95 mg *rac*-N-[2-Amino-2-methylcyclobutyl]-8-[(2,6-difluorbenzyl)oxy]-2,6-dimethylimidazo-[1,2-a]pyridin-3-carboxamid (Beispiel 331) wurden an chiraler Phase in die Enantiomere getrennt [Säule: Daicel Chiralcel OZ-H, 5 µm, 250 x 20 mm, Eluent: 50% iso-Hexan, 50% Ethanol + 0.2% Diethylamin, Fluß 20 ml/min; Temperatur: 20°C, Detektion: 250 nm].
Enantiomer A: 29 mg (>99% ee)
Rₜ= 9.47 min [Daicel Chiralcel OZ-H, 5µm, 250 x 4.6 mm; Eluent: 50% iso-Hexan, 50% Ethanol + 0.2% Diethylamin; Fluss 1.0 ml/min; Temperatur: 30°C; Detektion: 220 nm].
LC-MS (Methode 2): Rₜ = 0.55 min
MS (ESpos): m/z = 415 (M+H)⁺
¹H NMR (400 MHz, DMSO-*d₆*) δ = 1.12 (s, 3 H), 1.55 - 1.75 (m, 3 H), 1.85 - 2.01 (m, 1 H), 2.31 (s, 3 H), 4.21 (q, 1 H), 5.28 (s, 2 H), 6.90 (s, 1 H), 7.24 (t, 2 H), 7.54 - 7.64 (m, 1 H), 7.77 (d, 1 H), 8.38 (s, 1H).

### Beispiel 333

### ent- N-[2-Amino-2-methylcyclobutyl]-8-[(2,6-difluorbenzyl)oxy]-2,6-dimethylimidazo[1,2-a]pyridin-3-carboxamid (Enantiomer B)

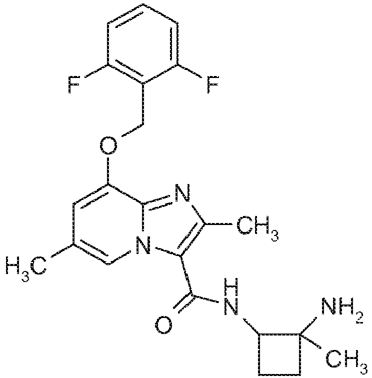

95 mg *rac*-N-[2-Amino-2-methylcyclobutyl]-8-[(2,6-difluorbenzyl)oxy]-2,6-dimethylimidazo[1,2-a]pyridin-3-carboxamid (Beispiel 331) wurden an chiraler Phase in die Enantiomere getrennt [Säule: Daicel Chiralcel OZ-H, 5 µm, 250 x 20 mm, Eluent: 50% iso-Hexan, 50% Ethanol + 0.2% Diethylamin, Fluß 20 ml/min; Temperatur: 20°C, Detektion: 250 nm].
Enantiomer B: 32 mg (Reinheit 90%, >83% ee)
Rₜ= 15.21 min [Daicel Chiralcel OZ-H, 5µm, 250 x 4.6 mm; Eluent: 50% iso-Hexan, 50% Ethanol + 0.2% Diethylamin; Fluss 1.0 ml/min; Temperatur: 30°C; Detektion: 220 nm].
LC-MS (Methode 2): Rₜ = 0.55 min
MS (ESpos): m/z = 415 (M+H)⁺
¹H NMR (400 MHz, DMSO-*d₆*) δ = 1.12 (s, 3 H), 1.55 - 1.75 (m, 3 H), 1.85 - 2.00 (m, 1 H), 2.31 (s, 3 H), 4.21 (q, 1 H), 5.28 (s, 2 H), 6.90 (s, 1 H), 7.24 (t, 2 H), 7.54 - 7.64 (m, 1 H), 7.77 (d, 1 H), 8.38 (s, 1 H).

### Beispiel 334

### N-(3-Aminocyclobutyl)-8-[(2,6-difluorbenzyl)oxy]-2,6-dimethylimidazo[1,2-a]pyridin-3-carboxamid (cis/trans-Gemisch)

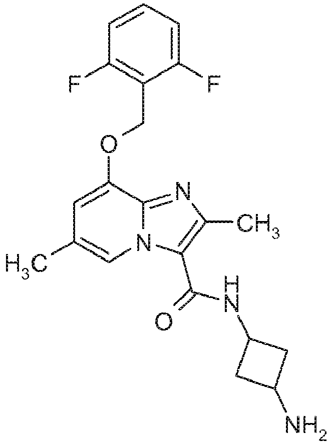

287 mg (1.81 mmol) Cyclobutan-1,3-diamindihydrochlorid (*cis*/*trans*-Gemisch)wurden mit 0.75 ml DMF, 0.94 ml (5.42 mmol) N,N-Diisopropylethylamin und 0.75 ml DMSO versetzt und eine Stunde bei RT gerührt. Die Suspension wurde auf 60°C erhitzt. 0.75 ml DMSO wurden hinzugefügt und es wurde anschließend mit einer Lösung aus 150 mg (0.45 mmol) 8-[(2,6-Difluorbenzyl)oxy]-2,6-dimethylimidazo[1,2-a]pyridin-3-carbonsäure aus Beispiel 21A, 0.16 ml (0.90 mmol) N,N-Diisopropylethylamin und 206 mg (0.54 mmol) HATU in 1.3 ml DMF, welche vorher 10 min bei RT gerührt wurden war, versetzt. Es wurde 30 min bei 60°C gerührt, dann mit Wasser und TFA versetzt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan aufgenommen und zweimal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filriert, das Filtrat eingeengt und der Rückstand eingeengt. Es wurden 110 mg der Zielverbindung (60% d. Th.) erhalten.
LC-MS (Methode 2): Rₜ = 0.60 min
MS (ESpos): m/z = 401(M+H)⁺
¹H NMR (400 MHz, DMSO-*d*₆) δ = 1.66 - 1.82 (m, 3 H), 1.94 - 2.06 und 2.19 - 2.27 (m, 1 H), 2.30 (s, 3 H), 2.47 (s, 3 H), 2.97 - 3.08 und 3.91 - 4.03 (m, 1 H), 5.28 (s, 2 H), 6.90 (s, 1 H), 7.24 (t, 2 H), 7.52 - 7.64 (m, 1 H), 7.92 und 8.08 (d und d, 1 H), 8.35 - 8.40 (m, 1 H).

### Beispiel 335

### rac-N-[2-Amino-1,2-dimethylcyclobutyl]-8-[(2,6-difluorbenzyl)oxy]-2,6-dimethylimidazo[1,2-a]pyridin-3-carboxamid

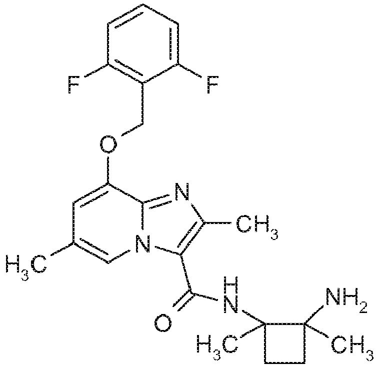

451 mg (2.41 mmol) *rac*-1,2-Dimethylcyclobutan-1,2-diamindihydrochlorid (beschrieben in: K.-H. Scholz; J. Hinz; H.-G. Heine; W. Hartmann, Liebigs Annalen der Chemie, 1981, 248-255; J. L. Gagnon; W. W. Zajac, Synthetic Commununications 1996, 26, 837-846) wurden mit 1 ml DMF, 1.26 ml (7.22 mmol) N,N-Diisopropylethylamin und 2 ml DMSO versetzt und bei 60°C gerührt. Es wurde eine Lösung aus 200 mg (0.60 mmol) 8-[(2,6-Difluorbenzyl)oxy]-2,6-dimethylimidazo[1,2-a]pyridin-3-carbonsäure aus Beispiel 21A, 0.21 ml (1.20 mmol) N,N-Diisopropylethylamin und 275 mg (0.72 mmol) HATU in 1.8 ml DMF, welche vorher 10 min bei RT gerührt wurden war, zugetropft. Die Lösung wurde 30 min bei 60°C gerührt, dann wurde mit Wasser und TFA versetzt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan aufgenommen und zweimal mit gesättigter, wässriger Natriumhydrogencarbonatlösung gewaschen. Die wässrige Phase wurde zweimal mit Dichlormethan extrahiert, die vereinigten organischen Phasen wurden über Natriumsulfat getrocknet, filriert, das Filtrat eingeengt und der Rückstand eingeengt. Es wurden 198 mg der Zielverbindung (77% d. Th.) erhalten.
LC-MS (Methode 2): Rₜ = 0.64 min
MS (ESpos): m/z = 429 (M+H)⁺
¹H NMR (400 MHz, DMSO-*d₆*) δ = 1.23 (s, 3 H), 1.45 (s, 3 H), 1.60 - 1.76 (m, 3 H), 1.93 - 2.04 (m, 1 H), 2.31 (s, 3 H), 2.47 (s, 3 H), 5.29 (s, 2 H), 6.88 (s, 1 H), 7.22 (t, 2 H), 7.54 - 7.63 (m, 1 H), 7.80 (s, 1 H), 8.36 (s, 1 H).

### Beispiel 336

### ent-N-[(2-Amino-1,2-dimethylcyclobutyl]-8-[(2,6-difluorbenzyl)oxy]-2,6-dimethylimidazo[1,2-a]pyridin-3-carboxamid (Enantiomer A)

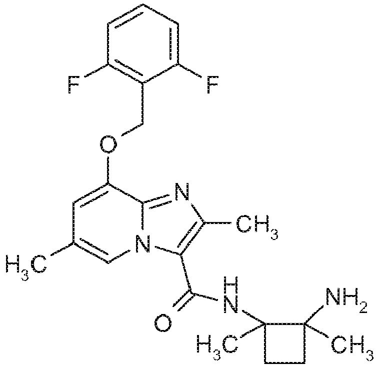

190 mg *rac*-N-[2-Amino-1,2-dimethylcyclobutyl]-8-[(2,6-difluorbenzyl)oxy]-2,6-dimethylimidazo[1,2-a]pyridin-3-carboxamid (Beispiel 335) wurden an chiraler Phase in die Enantiomere getrennt [Säule: Daicel Chiralcel OZ-H, 5 µm, 250 x 20 mm, Eluent: 45% Isopropanol, 50% iso-Hexan, 5%+ Isopropanol + 2% Dimethylamin, Fluß 20 ml/min; Temperatur: 25°C, Detektion: 210 nm].
Enantiomer A: 20 mg (>99% ee)
Rₜ = 6.26 min [Daicel Chiralcel OZ-H, 5µm, 250 x 4.6 mm; Eluent: 50% iso-Hexan, 50% Isopropanol + 0.2% Diethylamin; Fluss 1.0 ml/min; 30°C; Detektion: 220 nm].
LC-MS (Methode 2): Rₜ = 0.59 min
MS (ESpos): m/z = 429 (M+H)⁺
¹H NMR (500 MHz, DMSO-*d*₆) δ = 1.25 (s, 3 H), 1.48 (s, 3 H), 1.63 - 1.82 (m, 3 H), 1.93 - 2.04 (m, 1 H), 2.31 (s, 3 H), 2.49 (s, 3 H), 5.29 (s, 2 H), 6.89 (s, 1 H), 7.23 (t, 2 H), 7.54 - 7.63 (m, 1 H), 7.81 (s, 1 H), 8.37 (s, 1 H).

### Beispiel 337

### ent-N-[(2-Amino-1,2-dimethylcyclobutyl]-8-[(2,6-difluorbenzyl)oxy]-2,6-dimethylimidazo[1,2-a]pyridin-3-carboxamid (Enantiomer B)

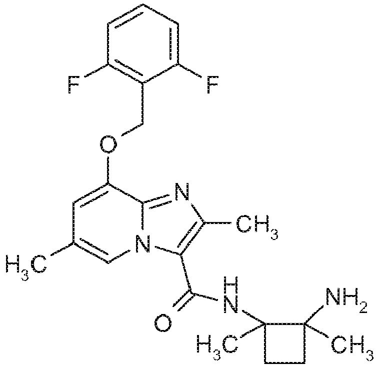

190 mg *rac-*N-[(1S,2S)-2-Amino-1,2-dimethylcyclobutyl]-8-[(2,6-difluorbenzyl)oxy]-2,6-dimethylimidazo[1,2-a]pyridin-3-carboxamid (Beispiel 335) wurden an chiraler Phase in die Enantiomere getrennt [Säule: Daicel Chiralcel OZ-H, 5 µm, 250 x 20 mm, Eluent: 45% Isopropanol, 50% iso-Hexan, 5%+ Isopropanol + 2% Dimethylamin, Fluß 20 ml/min; Temperatur: 25°C, Detektion: 210 nm].
Enantiomer B: 16 mg (>99% ee)
Rₜ = 13.44 min [Daicel Chiralcel OZ-H, 5µm, 250 x 4.6 mm; Eluent: 50% iso-Hexan, 50% Isopropanol + 0.2% Diethylamin; Fluss 1.0 ml/min; Temperatur: 30°C; Detektion: 220 nm].
LC-MS (Methode 2): Rₜ = 0.59 min
MS (ESpos): m/z = 429 (M+H)⁺
¹H NMR (500 MHz, DMSO-d₆) δ = 1.26 (s, 3 H), 1.48 (s, 3 H), 1.61 - 1.78 (m, 3 H), 1.93 - 2.04 (m, 1 H), 2.31 (s, 3 H), 2.49 (s, 3 H), 5.29 (s, 2 H), 6.89 (s, 1 H), 7.23 (t, 2 H), 7.54 - 7.63 (m, 1 H), 7.81 (s, 1 H), 8.36 (s, 1 H).

### Beispiel 338

### ent-N-(2-Amino-2-methylpentyl)-8-[(2,6-difluorbenzyl)oxy]-2-ethy1-6-methylimidazo[1,2-a]pyridin-3-carboxamid

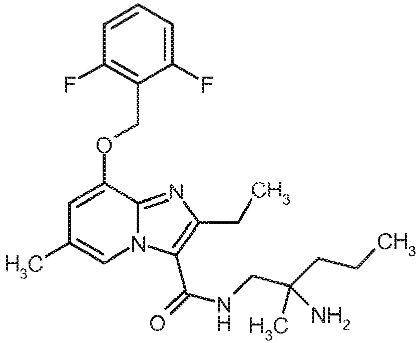

180 mg (0.26 mmol) *ent*-Benzyl-{1-[({8-[(2,6-difluorbenzyl)oxy]-2-ethyl-6-methylimidazo[12-a]pyridin-3-yl}carbonyl)amino]-2-methylpentan-2-yl}carbamat Trifluoracetat aus Beispiel 370A wurden in 2.8 ml Ethanol gelöst und unter Argon mit 18.2 mg (0.03 mmol) 20%-igem Palladium(II)hydroxid auf Kohle versetzt. Anschließend wurde die Reaktionsmischung zwei Stunden bei RT unter Normaldruck hydriert. Das Reaktionsgemisch wurde auf Diatomeenerde überführt und mittels Kieselgel-Chromatographie gereinigt (Laufmittel: Dichlormethan/2N Ammoniak in Methanol = 50/1). Es wurden 94 mg der Zielverbindung (81% d. Th.) erhalten.
LC-MS (Methode 2): Rₜ = 0.69 min
MS (ESpos): m/z = 445 (M+H)⁺
¹H-NMR (400 MHz, DMSO-d₆): δ = 0.87 (t, 3H), 0.99 (s, 3H), 1.22 (t, 3H), 1.26 - 1.40 (m, 4H), 1.41 - 1.54 (m, 2H), 2.30 (s, 3H), 2.91 (q, 2H), 3.14 - 3.25 (m, 2H), 5.29 (s, 2H), 6.91 (s, 1H), 7.20 - 7.28 (m, 2H), 7.55 - 7.71 (m, 2H), 8.41 (s,1H).

### Beispiel 339

### rac-8-[(2,6-Difluorbenzyl)oxy]-N-(6-methoxy-1,2,3,4-tetrahydrochinolin-4-yl)-2,6-dimethylimidazo[1,2-a]pyridin-3-carboxamid

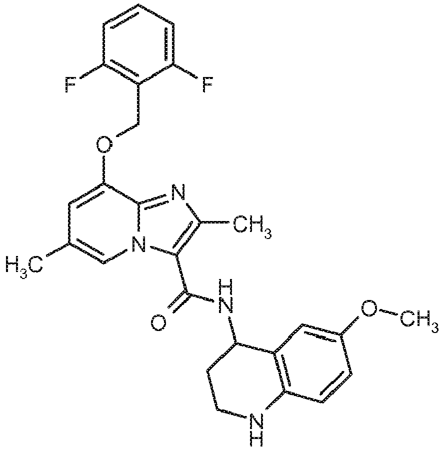

100 mg (0.30 mmol) 8-[(2,6-Difluorbenzyl)oxy]-2,6-dimethylimidazo[1,2-a]pyridin-3-carbonsäure aus Beispiel 21A, 126 mg (0.33 mmol) HATU und 117 mg (0.90 mmol) N,N-Diisopropylethylamin wurden in 1 ml DMF vorgelegt und 10 min bei RT gerührt. Anschließend wurden 62 mg (0.35 mmol) *rac*-6-Methoxy-1,2,3,4-tetrahydrochinolin-4-amin hinzugegeben und das Gemisch wurde 1 h bei RT gerührt. Die Reaktionsmischung wurde mit Acetonitril, TFA und Wasser versetzt und mittels präparativer HPLC gereinigt (RP-C18, Laufmittel: Acetonitril/Wasser-Gradient unter Zusatz von 0.1% TFA). Die produkthaltigen Fraktionen wurden eingeengt, der Rückstand in Dichlormethan aufgenommen und zweimal mit gesättigter, wässriger Natriumhydrogencarbonat-Lösung gewaschen. Die wässrige Phase wurde zweimal mit Dichlormethan extrahiert. Die vereinten organischen Phasen wurden über Natriumsulfat getrocknet, filtriert und das Filtrat eingeengt. Es wurden 129 mg (87% d. Th.) der Titelverbindung erhalten.
LC-MS (Methode 2): Rₜ = 0.82 min
MS (ESpos): m/z = 493 (M+H)⁺
¹H-NMR (400 MHz, DMSO-d₆): δ = 1.89 - 2.10 (m, 2H), 2.32 (s, 3H), 2.48 (s, 3H), 3.15 - 3.28 (m, 2H), 3.61 (s, 3H), 5.12 - 5.20 (m, 1H), 5.29 (s, 2H), 5.42 - 5.47 (m, 1H), 6.48 (d, 1H), 6.59 - 6.65 (m, 1H), 6.76 (d, 1H), 6.90 (s, 1H), 7.19 - 7.26 (m, 2H), 7.55 - 7.64 (m, 1H), 8.23 (d, 1H), 8.39 (s, 1H).

### Beispiel 340

### rac-N-(2-Amino-2-methylpentyl)-8-(benzyloxy)-2,6-dimethylimidazo[1,2-a]pyridin-3-carboxamid

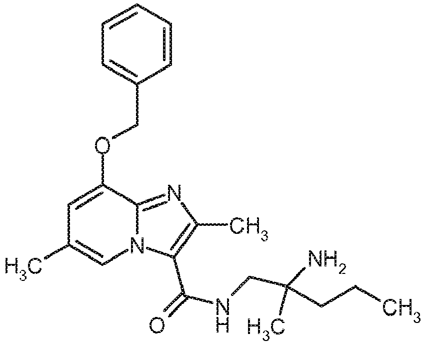

75 mg (0.15 mmol) *rac-tert*-Butyl-[1-({[8-(benzyloxy)-2,6-dimethylimidazo[1,2-a]pyridin-3-yl]carbonyl}amino)-2-methylpentan-2-yl]carbamat aus Beispiel 329A wurden in 0.74 ml Diethylether vorgelegt, mit 0.74 ml (1.47 mmol) 2 N Salzsäure in Diethylether versetzt und über Nacht bei RT gerührt. Das Reaktionsgemisch wurde mit 0.74 ml (1.47 mmol) 2 N Salzsäure in Diethylether versetzt und Nacht bei RT gerührt. Dann wurde eingeengt, der Rückstand in Dichlormethan gelöst und es wurde zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 57 mg der Zielverbindung (96% d. Th.) erhalten.
LC-MS (Methode 2): Rₜ = 0.67 min
MS (ESpos): m/z = 395 (M+H)⁺
¹H-NMR (400 MHz, DMSO-d₆): δ = 0.87 (t, 3H), 1.00 (s, 3H), 1.24 -1.43 (m, 4H), 1.52 -1.92 (m, 2H), 2.27 (s, 3H), 2.56 (s, 3H), 3.14 - 3.27 (m, 2H), 5.27 (s, 2H), 6.81 (s, 1H), 7.34 - 7.40 (m, 1H), 7.43 (t, 2H), 7.48 - 7.54 (m, 2H), 7.63 (t,1H), 8.45 (s, 1H).

### Beispiel 341

### rac-N-(3-Amino-2,2-dimethylcyclopropyl)-8-[(2,6-difluorbenzyl)oxy]-2,6-dimethylimidazo[1,2-a]pyridin-3-carboxamid (Racemat)

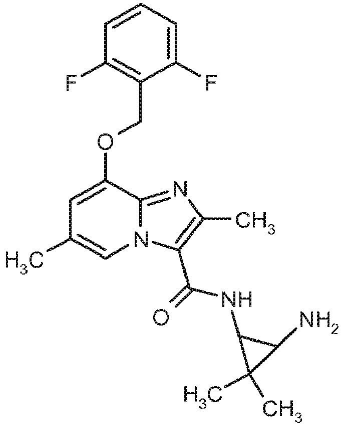

195 mg (1.13 mmol) *rac*-3,3-Dimethylcyclopropan-1,2-diamindihydrochlorid (beschrieben in: G.-Q. Feng et al. Tetrahedron: Asymmetry 2006,17,2775-2780) wurden mit 0.5 ml DMF und 0.39 ml (2.26 mmol) N,N-Diisopropylethylamin versetzt. Eine Lösung aus 150 mg (0.45 mmol) 8-[(2,6-Difluorbenzyl)oxy]-2,6-dimethylimidazo[1,2-a]pyridin-3-carbonsäure aus Beispiel 21A, 0.47 ml (2.71 mmol) N,N-Diisopropylethylamin und 206 mg (0.54 mmol) HATU in 1.34 ml DMF, welche vorher 10 min bei RT gerührt wurden war, wurde zur Reaktionsmischung zugetropft. Es wurde 30 min bei RT gerührt, dann mit Wasser und TFA versetzt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand wurde in Dichlormethan aufgenommen und zweimal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filriert, das Filtrat eingeengt und der Rückstand eingeengt. Es wurden 78 mg der Zielverbindung (40% d. Th.) erhalten.
LC-MS (Methode 2): Rₜ = 0.61 min
MS (ESpos): m/z = 415 (M+H)⁺
¹H NMR (500 MHz, DMSO-*d*₆) δ = 0.99 (s, 3 H), 1.14 (s, 3 H), 2.15 - 2.18 (m, 1 H), 2.28 -2.32 (m, 4 H), 2.44 (s, 3 H), 5.28 (s, 2 H), 6.89 (s, 1 H), 7.23 (t, 2 H), 7.54 - 7.63 (m, 1 H), 7.79 (s, 1 H), 8.39 - 8.43 (m, 1H).

### Beispiel 342

### rac-8-[(2,6-Difluorbenzyl)oxy]-2,6-dimethyl-N-(octahydrocyclopenta[b]pyrrol-4-yl)imidazo[1,2-a]pyridin-3-carboxamid

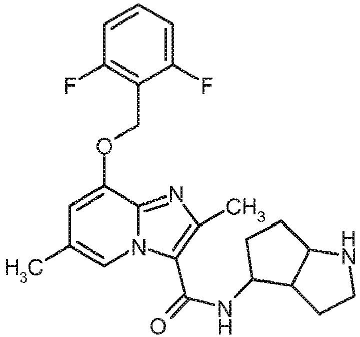

115 mg (0.21 mmol) *rac-tert*-Butyl-4-[({8-[(2,6-difluorbenzyl)oxy]-2,6-dimethylimidazo[1,2-a]pyridin-3-yl}carbonyl)amino]hexahydrocyclopenta[b]pyrrol-1(2H)-carboxylat aus Beispiel 371A wurden in 1 ml Diethylether vorgelegt, mit 1 ml (2.13 mmol) 2 N Salzsäure in Diethylether versetzt und 4 Stunden bei RT gerührt. Das Reaktionsgemisch wurde eingeengt, der Rückstand wurde mit Dichlormethan versetzt und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Der Rückstand wurde lyophilisiert. Es wurden 72 mg der Zielverbindung (75% d. Th.) erhalten.
LC-MS (Methode 2): Rₜ = 0.57 min
MS (ESpos): m/z = 441 (M+H)⁺
¹H-NMR (400 MHz, DMSO-d₆): δ = 1.47 -1.66 (m, 4H), 1.67 -1.79 (m, 2H), 2.31 (s, 3H), 2.48 (s, 3H), 2.57 - 2.65 (m, 1H), 2.68 - 2.79 (m, 1H), 2.84 - 2.92 (m, 1H), 3.58 (t, 1H), 4.12 - 4.24 (m, 1H), 5.28 (s, 2H), 6.90 (s, 1H), 7.18 - 7.29 (m, 2H), 7.53 - 7.66 (m, 1H), 7.84 (d, 1H), 8.37 (s, 1H).

### Beispiel 343

### rac-N-(2-Amino-2-methylpentyl)-8-[(2-chlorbenzyl)oxy]-2,6-dimethylimidazo[1,2-a]pyridin-3-carboxamid

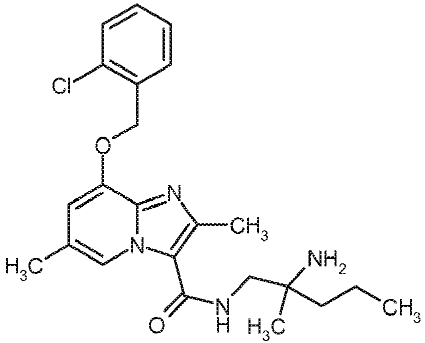

80 mg (0.19 mmol) *rac-tert*-Butyl-(1-{[(8-hydroxy-2,6-dimethylimidazo[1,2-a]pyridin-3-yl)carbonyl]amino}-2-methylpentan-2-yl)carbamat aus Beispiel 330A, 33 mg (0.21 mmol) 2-Chlorbenzylchlorid, 135 mg (0.41 mmol) Cäsiumcarbonat und 3.1 mg (0.02 mmol) Kaliumiodid wurden in 3.6 ml DMF vorgelegt und für 30 min in einem vorgeheizten 60°C warmen Ölbad erhitzt. Die Reaktionslösung wurde eingeengt und über Nacht im Hochvakuum getrocknet. Der Rückstand wurde in 1 ml Diethylether aufgenommen, mit 0.94 ml (1.88 mmol) 2 N Salzsäure in Diethylether versetzt und über Nacht bei RT gerührt. Das Reaktionsgemisch wurde eingeengt und mittels präparativer HPLC gereinigt (RP18 Säule, Laufmittel: Methanol/Wasser-Gradient unter Zusatz von 0.1% Ameisensäure). Die Produktfraktionen wurden vereinigt und eingeengt. Der Rückstand wurde in Dichlormethan aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 49 mg der Zielverbindung (61% d. Th.) erhalten.
LC-MS (Methode 2): Rₜ = 0.72 min
MS (ESpos): m/z = 429 (M+H)⁺

### Beispiel 344

### rac-N-[(4-Benzylmorpholin-2-yl)methyl]-8-[(2,6difluorbenzyl)oxy]-2,6-dimethylimidazo[1,2-a]pyridin-3-carboxamid

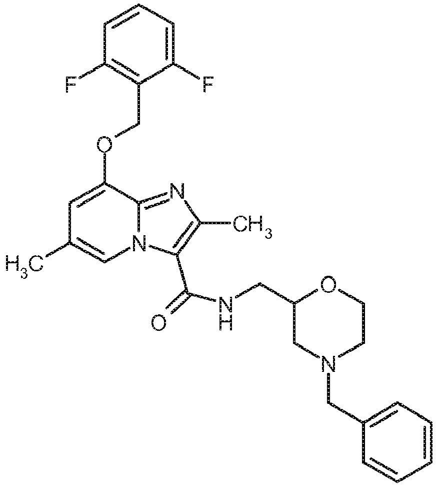

20 mg (0.06 mmol) 8-[(2,6-Difluorbenzyl)oxy]-2,6-dimethylimidazo[1,2-a]pyridin-3-carbonsäure aus Beispiel 21A wurden mit 25 mg (0.07 mmol) HATU und 0.05 ml (0.30 mmol) N,N-Diisopropylethylamin in 0.4 ml DMF vorgelegt und 20 min gerührt. Anschließend wurden bei 0°C 24.8 mg (0.12 mmol) *rac*-1-(4-Benzylmorpholin-2-yl)methanamin hinzugegeben und 60 min bei 0°C gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produkt Fraktionen wurden eingeengt, der Rückstand in Dichlormethan aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 31 mg der Zielverbindung (94% d. Th.) erhalten.
LC-MS (Methode 2): Rₜ = 1.94 min
MS (ESpos): m/z = 521 (M+H)⁺
¹H-NMR (400 MHz, DMSO-d₆): δ = 1.78 -1.88 (m, 1H), 2.05 - 2.15 (m, 1H), 2.30 (s, 3H), 2.37 (s, 3H), 2.60 - 2.67 (m, 1H), 2.74 - 2.80 (m, 1H), 3.25 - 3.31 (m, 2H), 3.42 - 3.46 (m, 1H), 3.47 - 3.57 (m, 2H), 3.58 - 3.66 (m, 1H), 3.78 - 3.85 (m, 1H), 5.28 (s, 2H), 6.91 (s, 1H), 7.20 - 7.27 (m, 3H), 7.28 - 7.34 (m, 4H), 7.54 - 7.64 (m, 1H), 7.87 (t, 1H), 8.37 (s,1H).

### Beispiel 345

### rac-8-[(2,6-Difluorbenzyl)oxy]-2,6-dimethyl-N-(morpholin-2-ylmethyl)imidazo[1,2-a]pyridin-3-carboxamid

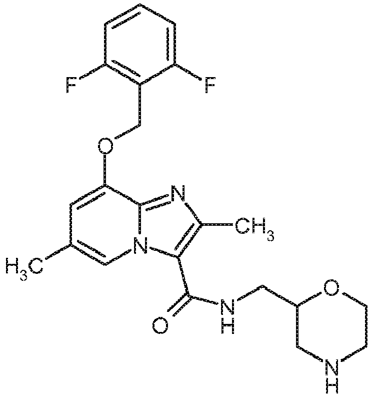

78 mg (0.15 mmol) *rac*-*N*-[(4-Benzylmorpholin-2-yl)methyl]-8-[(2,6-difluorbenzyl)oxy]-2,6-dimethylimidazo[1,2,a]pyridin-3-carboxamid aus Beispiel 344 wurde unter Argon in 1.6 ml Ethanol vorgelegt, mit 16 mg (0.02 mmol) 10%-igem Palladium auf Kohle versetzt und eine Stunde unter Normaldruck bei RT hydriert. Das Reaktionsgemisch wurde über einen Millipore-Filter filtriert, mit Ethanol nachgewaschen und im Vakuum eingeengt. Der Rückstand wurde in Dichlormethan gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 43 mg der Zielverbindung (63% d. Th.) erhalten.
LC-MS (Methode 2): Rₜ = 0.61 min
MS (ESpos): m/z = 431 (M+H)⁺
¹H-NMR (400 MHz, DMSO-d₆): δ = 2.31 (s, 3H), 2.41 - 2.46 (m, 1H), 2.48 (s, 3H), 2.62 - 2.78 (m, 3H), 2.84 - 2.94 (m, 1H), 3.28 - 3.32 (m, 2H), 3.43 - 3.51 (m, 1H), 3.53 - 3.61 (m, 1H), 3.74 - 3.81 (m, 1H), 5.28 (s, 2H), 6.92 (s, 1H), 7.20 - 7.28 (m, 2H), 7.54 - 7.64 (m, 1H), 7.88 (t, 1H), 8.42 (s, 1H).

### Beispiel 346

### rac-N-(3-Azabicyclo[3.2.0]hept-1-yl)-8-[(2,6-difluorbenzyl)oxy]-2,6-dimethylimidazo[1,2-a]pyridin-3-carboxamid

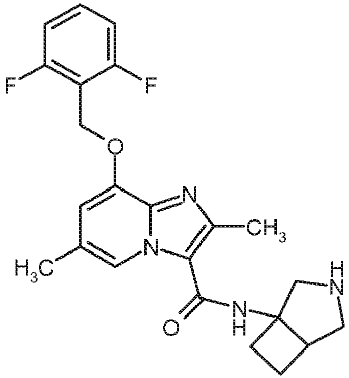

95 mg (0.18 mmol) *rac-tert*-Butyl-1-[({8-[(2,6-difluorbenzyl)oxy]-2,6-dimethylimidazo[1,2-a]pyridin-3-yl}carbonyl)amino]-3-azabicyclo[3.2.0]heptan-3-carboxylat aus Beispiel 372A wurden in 0.9 ml Diethylether vorgelegt, mit 0.88 ml (1.77 mmol) 2 N Salzsäure in Diethylether versetzt und über Nacht bei RT gerührt. Das Reaktiongemisch wurde eingeengt, der Rückstand wurde mit Dichlormethan und einem Tropfen Methanol versetzt und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 70 mg der Zielverbindung (90% d. Th.) erhalten.
LC-MS (Methode 2): Rₜ = 0.58 min
MS (ESpos): m/z = 427 (M+H)⁺
¹H-NMR (400 MHz, DMSO-d₆): δ = 1.38 - 1.48 (m, 1H), 2.03 - 2.15 (m, 2H), 2.21 - 2.29 (m, 1H), 2.31 (s, 3H), 2.48 (s, 3H), 2.65 - 2.74 (m, 2H), 2.83 - 2.95 (m, 2H), 3.11 - 3.18 (m, 1H), 5.28 (s, 2H), 6.90 (s, 1H), 7.20 - 7.28 (m, 2H), 7.54 - 7.64 (m, 1H), 8.08 (s, 1H), 8.42 (s, 1H).

### Beispiel 347

### rac-8-[(2,6-Difluorbenzyl)oxy]-N-(4-fluorpyrrolidin-3-yl)-2,6-dimethylimidazo[1,2-a]pyridin-3-carboxamid

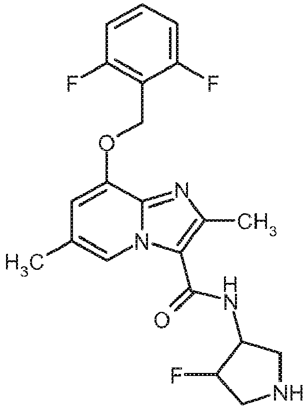

98 mg (0.15 mmol) *rac-tert*-Butyl-3-[({8-[(2,6-difluorbenzyl)oxy]-2,6-dimethylimidazo[1,2-a]pyridin-3-yl}carbonyl)amino]-4-fluorpyrrolidin-1-carboxylat Trifluoracetat aus Beispiel 373A wurden in 0.8 ml Diethylether vorgelegt, mit 0.77 ml (1.54 mmol) 2 N Salzsäure in Diethylether versetzt und über Nacht bei RT gerührt. Das Reaktionsgemisch wurde nochmals mit 0.77 ml (1.54 mmol) 2 N Salzsäure in Diethylether versetzt und über Nacht bei RT gerührt. Die Reaktionslösung wurde eingeengt, der Rückstand in Dichlormethan gelöst und zweimal mit gesättigter, wässriger Natriumhydrogen-carbonatlösung gewaschen. Die vereinigten wässrigen Phasen wurden zweimal mit Dichlormethan extrahiert. Die vereinigten organischen Phasen wurden über Natriumsulfat getrocknet, filtriert, eingeengt und lyophilisiert. Es wurden 64 mg der Zielverbindung (99% d. Th.) erhalten.
LC-MS (Methode 2): Rₜ = 0.63 min
MS (ESpos): m/z = 419 (M+H)⁺
¹H-NMR (400 MHz, DMSO-d₆): δ = 1.13 - 1.35 (m, 1H), 2.31 (s, 3H), 2.48 (s, 3H), 2.76 (t, 1H), 2.90 - 3.04 (m, 1H), 3.11 (dd, 1H), 3.23 (dd, 1H), 4.26 - 4.42 (m, 1H), 5.02 - 5.21 (m, 1H), 5.29 (s, 2H), 6.92 (s, 1H), 7.19 - 7.28 (m, 2H), 7.54 - 7.64 (m, 1H), 7.80 (d, 1H), 8.40 (s, 1H).

### Beispiel 348

### rac-N-(2-Amino-2-methylpentyl)-8-[(3-fluorbenzyl)oxy]-2,6-dimethylimidazo[1,2-a]pyridin-3-carboxamid

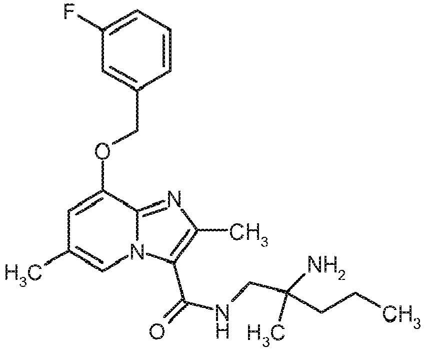

80 mg (0.19 mmol) *rac-tert-*Butyl-(1-{[(8-hydroxy-2,6-imethylimidazo[1,2-a]pyridin-3-yl)carbonyl]amino}-2-methylpentan-2-yl)carbamat aus Beispiel 330A, 39 mg (0.21 mmol) 3-Fluorbenzylbromid, 135 mg (0.41 mmol) Cäsiumcarbonat und 3.1 mg (0.02 mmol) Kaliumiodid wurden in 3.6 ml DMF vorgelegt und für 30 min in einem vorgeheizten 60°C warmen Ölbad erhitzt. Die Reaktionslösung wurde eingeengt und über Nacht im Hochvakuum getrocknet. Der Rückstand wurde in 0.9 ml Diethylether aufgenommen, mit 0.94 ml (1.88 mmol) 2 N Salzsäure in Diethylether versetzt und über Nacht bei RT gerührt. Das Reaktionsgemisch wurde eingeengt und mittels präparativer HPLC gereinigt (RP18 Säule, Laufmittel:Acetonitril/Wasser-Gradient unter Zusatz von 0.1% TFA). Die produkthaltigen Fraktionen wurden eingeengt, der Rückstand wurde in Dichlormethan aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, das Filtrat eingeengt und der Rückstand lyophilisiert. Es wurden 51 mg der Zielverbindung (64% d. Th.) erhalten.
LC-MS (Methode 2): Rₜ = 0.68 min
MS (ESpos): m/z = 413 (M+H)⁺

### Beispiel 349

### 8-[(2,6-Difluorbenzyl)oxy]-N-{2-[(2-hydroxyethyl)amino]-2-methylpropyl}-2,6-dimethylimidazo[1,2-a]pyridin-3-carboxamid

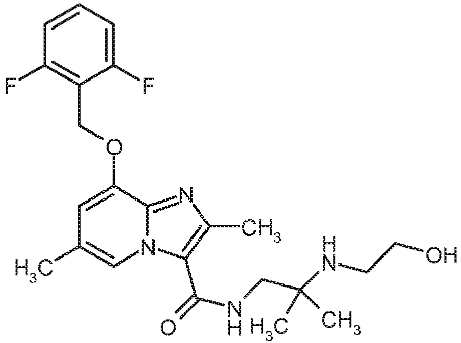

100 mg (0.25 mmol) N-(2-Amino-2-methylpropyl)-8-[(2,6-difluorbenzyl)oxy]-2,6-dimethylimidazo[1,2,a]pyridin-3-carboxamid aus Beispiel 74 wurden in 1 ml DMF vorgelegt und mit 0.023 ml (0.30 mmol) Iodethanol und 69 mg (0.50 mmol) Kaliumcarbonat versetzt. Das Reaktionsgemisch wurde über Nacht bei RT gerührt. 0.23 ml (3.0 mmol) Iodethanol und 69 mg (0.50 mmol) Kaliumcarbonat wurden zugegeben und die Mischung wurde über Nacht unter Rückfluss gerührt. Das Reaktionsgemisch wurde mit Wasser und Acetonitril verdünnt und mittels präparativer HPLC gereinigt (RP18 Säule, Laufmittel:Acetonitril/Wasser-Gradient unter Zusatz von 0.1% TFA). Die Produktfraktionen wurden eingeengt und im Hochvakuum getrocknet. Es wurden 5 mg der Zielverbindung (4% d. Th., Reinheit 90 %) erhalten.
LC-MS (Methode 2): Rₜ = 0.60 min
MS (ESpos): m/z = 447 (M+H)⁺
¹H-NMR (400 MHz, DMSO-d₆): δ = 1.05 (s, 6H), 1.54 (t, 1H), 2.31 (s, 3H), 3.24 (d, 2H), 3.28 - 3.31 (m, 1H), 3.42 (q, 2H), 4.48 (t, 1H), 5.28 (s, 2H), 6.92 (s, 1H), 7.20 - 7.28 (m, 2H), 7.50 (t, 1H), 7.54 - 7.64 (m, 1H), 8.52 - 8.56 (m, 1H).

### Beispiel 350

### rac-N-[2-Amino-3-(3,4-difluorphenoxy)-2-methylpropyl]-8-[(2,6-difluorbenzyl)oxy]-2,6-dimethylimidazo[1,2,e]pyridin-3-carboxamid

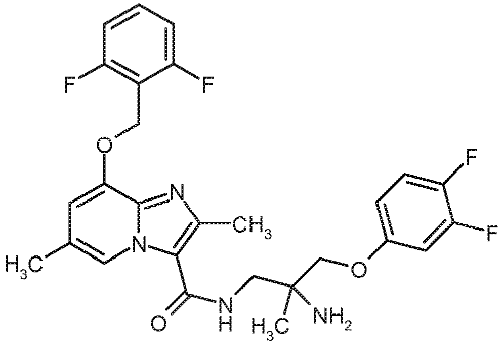

51 mg (0.15 mmol) 8-[(2,6-Difluorbenzyl)oxy]-2,6-dimethylimidazo[1,2-a]pyridin-3-carbonsäure aus Beispiel 21A wurden mit 64.5 mg (0.17 mmol) HATU und 0.13 ml (0.77 mmol) N,N-Diisopropylethylamin in 1 ml DMF vorgelegt und 20 min gerührt. Anschließend wurden bei 0°C 40 mg (0.19 mmol) *rac*-3-(3,4-Difluorphenoxy)-2-methylpropan-1,2-diamin aus Beispiel 391A hinzugegeben und 45 min bei 0°C gerührt. Die Reaktionslösung wurde mit Acetonitril und TFA versetzt und mittels präparativer HPLC gereinigt (RP18 Säule, Laufmittel: Acetonitril/Wasser-Gradient unter Zusatz von 0.1 % TFA). Die Produktfraktionen wurden eingeengt, der Rückstand in Dichlormethan aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filriert, das Filtrat eingeengt und lyophilisiert. Es wurden 62 mg der Zielverbindung (72% d. Th.) erhalten.
LC-MS (Methode 2): Rₜ = 0.80 min
MS (ESpos): m/z = 531 (M+H)⁺
¹H-NMR (400 MHz, DMSO-d₆): δ = 1.29 (s, 3H), 2.30 (s, 3H), 2.53 (br. s., 3H), 3.49 - 3.66 (m, 2H), 3.89 - 3.96 (m, 1H), 3.98 - 4.05 (m, 1H), 5.29 (s, 2H), 6.78 - 6.85 (m, 1H), 6.94 (s, 1H), 7.04 - 7.13 (m, 1H), 7.20 - 7.29 (m, 2H), 7.39 (q, 1H), 7.54 - 7.65 (m, 1H), 7.89 (t, 1H), 8.43 (s, 1H).

### Beispiel 351

### rac-N-(2-Amino-2-methylpentyl)-8-[(2-fluor-6-methoxybenzyl)oxy]-2,6-dimethylimidazo[1,2-a]pyridin-3-carboxamid

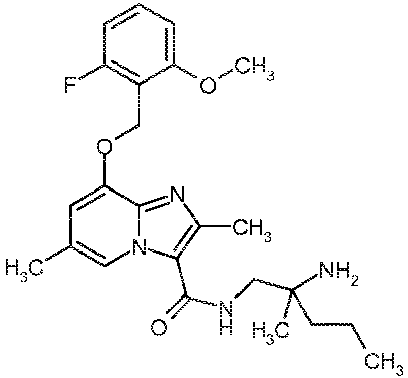

50 mg (0.14 mmol, Reinheit 94%) 8-[(2-Fluor-6-methoxybenzyl)oxy]-2,6-dimethylimidazo[1,2-a]pyridin-3-carbonsäure aus Beispiel 375A wurden mit 57 mg (0.15 mmol) HATU und 0.12 ml (0.68 mmol) N,N-Diisopropylethylamin in 0.9 ml DMF vorgelegt und 20 min bei RT gerührt. Dann wurden bei 0°C 28 mg (0.15 mmol) *rac*-2-Methylpentan-1,2-diamin Dihydrochlorid aus Beispiel 326A hinzugegeben und 30 min bei 0°C gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1% TFA). Die Produktfraktionen wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 45 mg der Zielverbindung (71% d. Th.) erhalten.
LC-MS (Methode 2): Rₜ = 0.69 min
MS (ESpos): m/z = 443 (M+H)⁺
¹H-NMR (400 MHz, DMSO-d₆): δ = 0.87 (t, 3H), 1.00 (s, 3H), 1.22 -1.44 (m, 4H), 1.72 - 2.11 (m, 2H), 2.31 (s, 3H), 2.52 (s, 3H), 3.15 - 3.27 (m, 2H), 3.85 (s, 3H), 5.18 (s, 2H), 6.87 - 6.95 (m, 2H), 6.99 (d, 1H), 7.44 - 7.53 (m, 1H), 7.62 (t, 1H), 8.44 (s, 1H).

### Beispiel 352

### N-(2-Amino-2-methylpropyl)-8-[(2-fluor-6-methoxybenzyl)oxy]-2,6-dimethylimidazo[1,2-a]pyridin-3-carboxamid

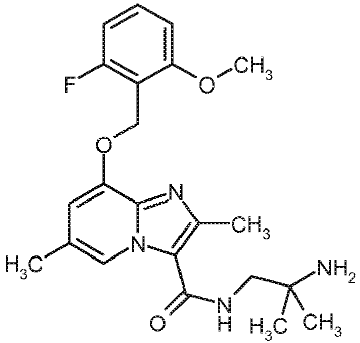

50 mg (0.14 mmol, Reinheit 94%) 8-[(2-Fluor-6-methoxybenzyl)oxy]-2,6-dimethylimidazo[1,2-a]pyridin-3-carbonsäure aus Beispiel 375A wurden mit 57 mg (0.15 mmol) HATU und 0.07 ml (0.41 mmol) N,N-Diisopropylethylamin in 0.9 ml DMF vorgelegt und 20 min bei RT gerührt. Dann wurden bei 0°C 0.02 ml (0.15 mmol) 1,2-Diamino-2-methylpropan hinzugegeben und 30 min bei 0°C gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1% TFA). Die Produktfraktionen wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 48 mg der Zielverbindung (83% d. Th.) erhalten.
LC-MS (Methode 2): Rₜ = 0.62 min
MS (ESpos): m/z = 415 (M+H)⁺
¹H-NMR (400 MHz, DMSO-d₆): δ = 1.05 (s, 6H), 1.47 - 1.78 (m, 2H), 2.31 (s, 3H), 2.52 (s, 3H), 3.20 (d, 2H), 3.85 (s, 3H), 5.18 (s, 2H), 6.86 - 6.95 (m, 2H), 6.99 (d, 1H), 7.49 (q, 1H), 7.65 (t, 1H), 8.44 (s, 1H).

### Beispiel 353

### N-(2-Amino-2-methylpentyl)-8-[(2-brombenzyl)oxy]-2,6-dimethylimidazo[1,2-a]pyridin-3-carboxamid

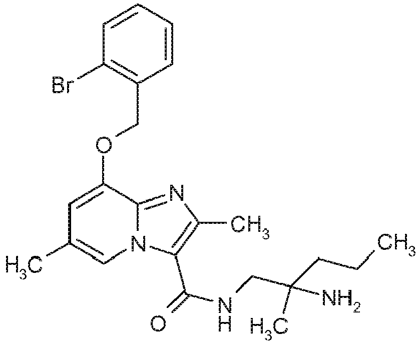

80 mg (0.19 mmol) *rac-tert-*Butyl*-*(1-{[(8-hydroxy-2,6-imethylimidazo[1,2-a]pyridin-3-yl)carbonyl]amino}-2-methylpentan-2-yl)carbamat 330A, 53 mg (0.21 mmol) 2-Brombenzylbromid, 135 mg (0.41 mmol) Cäsiumcarbonat und 3.1 mg (0.02 mmol) Kaliumiodid wurden in 3.6 ml DMF vorgelegt und für 30 min in einem vorgeheizten 60°C warmen Ölbad erhitzt. Die Reaktionslösung wurde eingeengt und über Nacht im Hochvakuum getrocknet. Der Rückstand wurde in Diethylether aufgenommen, mit 0.94 ml (1.88 mmol) 2 N Salzsäure in Diethylether versetzt und über Nacht bei RT gerührt. Das Reaktionsgemisch wurde eingeengt und mittels präparativer HPLC gereinigt (RP18 Säule, Laufmittel:Acetonitril/Wasser-Gradient unter Zusatz von 0.1% TFA). Die produkthaltigen Fraktionen wurden eingeengt, der Rückstand wurde in Dichlormethan aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, das Filtrat eingeengt und der Rückstand lyophilisiert. Es wurden 60 mg der Zielverbindung (67% d. Th.) erhalten.
LC-MS (Methode 2): Rₜ = 0.74 min
MS (ESpos): m/z = 473 (M+H)⁺

### Beispiel 354

### ent- N-(2-Amino-2-methylpentyl)-8-[(2,6-difluorbenzyl)oxy]imidazo[1,2-a]pyridin-3-carboxamid

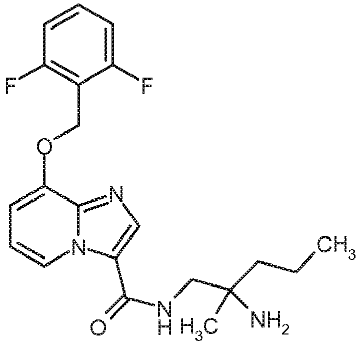

117 mg (0.18 mmol) *ent*-Benzyl-{1-[({8-[(2,6-difluorbenzyl)oxy]imida-zo[1,2-a]pyridin-3-yl}carbonyl)amino]-2-methylpentan-2-yl}carbamat Trifluoracetat aus Beispiel 376A wurden unter Argon in 1.9 ml Ethanol vorgelegt, mit 19.2 mg (0.02 mmol) 10%-igem Palladium auf Kohle versetzt und 1 Stunde unter Normaldruck bei RT hydriert. Das Reaktionsgemisch wurde auf Celite gezogen und mittels Kieselgelchmmatographie gereinigt (Laufmittel: Dichlormethan/2 N Ammoniak in Methanol = 40/1 bis 30/1). Es wurden 30 mg der Zielverbindung (41% d. Th.) erhalten.
LC-MS (Methode 2): Rₜ = 0.67 min
MS (ESpos): m/z = 403 (M+H)⁺
¹H NMR (400 MHz, DMSO-*d*₆) δ = 0.86 (t, 4 H), 0.95 (s, 3 H), 1.18 - 1.43 (m, 4 H), 1.44 - 1.56 (m, 1 H), 3.12 - 3.23 (m, 2 H), 5.34 (s, 2 H), 6.99 - 7.11 (m, 2 H), 7.24 (t, 2 H), 7.53 - 7.65 (m, 1 H), 8.17 - 8.26 (m, 1 H), 8.31 (s, 1 H), 9.08 (d, 1 H).

### Beispiel 355

### ent-N-(2-Amino-2-methylpentyl)-8-[(2,6-ifluorbenzyl)oxy]-6-thinyl-2-methylimidazo[1,2-a]pyridin-3-carboxamid Formiat

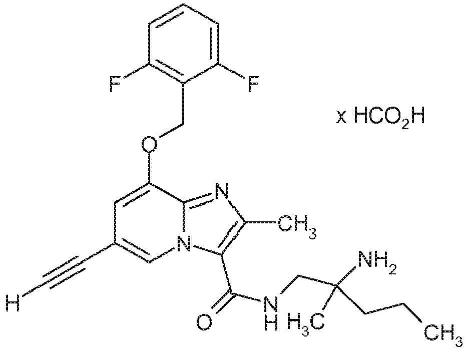

161 mg (0.28 mmol) *ent*-Benzyl-{1-[({8-[(2,6-difluorbenzyl)oxy]-6-ethinyl-2-methylimidazo[1,2-a]pyridin-3-yl}carbonyl)amino]-2-methylpentan-2-yl}carbamat aus Beispiel 359A und 0.2 ml Anisol wurden in 2.5 ml Dichlormethan bei 0°C vorgelegt und mit 2.5 ml einer 70%-igen Fluorwasserstoff-Lösung in Pyridin versetzt. Anschließend wurde 6 h bei RT gerührt und das Gemisch wurde bei -20°C über Nacht gelagert. Die Reaktionsmischung wurde mit 20 ml Dichlormethan verdünnt, bei 0°C zu 100 ml einer gesättigten, wässrigen Natriumhydrogencarbonatlösung getropft und 30 min bei 0°C gerührt. Die beiden Phasen wurden getrennt, die organische Phase zweimal mit gesättigter, wässriger Natriumhydrogencarbonatlösung und einmal mit Wasser gewaschen, anschließend über Natriumsulfat getrocknet, filtriert und eingeengt. Der Rückstand wurde mittels präparativer HPLC gereinigt (RP-C18, Laufmittel: Acetonitril/Wasser-Gradient unter Zusatz von 0.05% Ameisensäure). Es wurden 107 mg (78% d. Th.) der Titelverbindung erhalten.
LC-MS (Methode 2): Rₜ = 0.76 min
MS (ESpos): m/z = 441 (M-HCO₂H+H)⁺
¹H-NMR (400 MHz, DMSO-d₆): δ [ppm] = 0.86 - 0.93 (d, 3H), 1.14 (s, 3H), 1.30 - 1.55 (m, 4H), 2.56 (s, 3H), 3.30 - 3.45 (m, 2H), 4.34 (s, 1H), 5.33 (s, 2H), 7.08 (d, 1H), 7.20 - 7.29 (m, 2H), 7.55 - 7.65 (m, 1H), 8.14 (br. s, 1H), 8.32 (s, 1H), 8.79 (s, 1H).

### Beispiel 356

### ent-N-(2-Amino-2-methylpentyl)-8-[(2,6-difluorbenzyl)oxyl-6-ethinyl-2-methylimidazo[1,2-a]pyridin-3-carboxamid

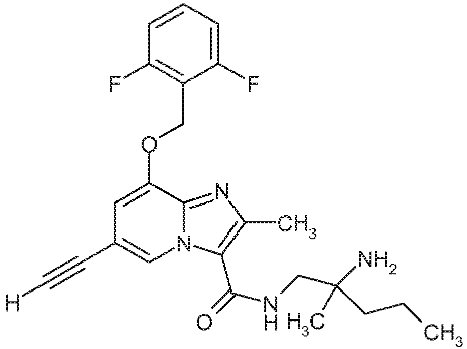

88 mg (0.18 mmol) *ent*-N-(2-Amino-2-methylpentyl)-8-[(2,6-difluorbenzyl)oxy]-ethinyl-2-methylimidazo[1,2-a]pyridin-3-carboxamid Formiat aus Beispiel 355 wurden in Dichlormethan gelöst und zweimal mit gesättigter, wässriger Natriumhydrogencarbonatlösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 79 mg der Titelverbindung (99% d. Th.) erhalten.
LC-MS (Methode 2): Rₜ = 0.78 min
MS (ESpos): m/z = 441 (M+H)⁺
¹H-NMR (400 MHz, DMSO-d₆): δ [ppm] = 0.82 - 0.90 (m, 3H), 1.00 (s, 3H), 1.25 - 1.41 (m, 4H), 1.68 - 2.15 (br. s, 2H), 3.17 - 3.32 (m, 2H), 4.32 (s, 1H), 5.33 (s, 2H), 7.07 (s, 1H), 7.20 - 7.28 (m, 2H), 7.55 - 7.66 (m, 1H), 7.76 (br. s, 1H), 8.80 (s, 1H), [weiteres Signal unter Lösungsmittelpeak].

### Beispiel 357

### ent-N-(2-Amino-2-methylbutyl)-8-[(2,6-difluorbenzyl)oxy]-2,6-dimethylimidazo[1,2-a]pyridin-3-carboxamid Hydrochlorid (Enantiomer A)

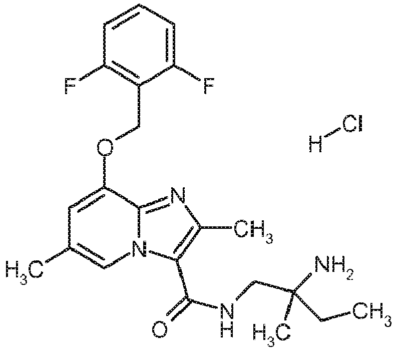

302 mg (0.71 mmol) *ent*-*N*-(2-Amino-2-methylbutyl)-8-[(2,6-difluorbenzyl)oxyl-2,6-dimethylimidazo[1,2-a]pyridin-3-carboxamid (Enantiomer A) aus Beispiel 200 wurden in 5.7 ml Diethylether vorgelegt, mit 0.43 ml (0.85 mmol) 2 N Salzsäure in Diethylether versetzt und 30 min bei Raumtemperatur gerührt. Anschließend wurde das Lösungsmittel eingeengt. Es wurden 326 mg der Zielverbindung (99% d. Th.) erhalten.
LC-MS (Methode 2): Rₜ = 0.64 min
MS (ESIpos): m/z = 417 (M-HCl+H)⁺
¹H-NMR (400 MHz, DMSO-d₆): δ = 0.95 (t, 3H), 1.24 (s, 3H), 1.55 - 1.73 (m, 2H), 2.34 (s, 3H), 2.57 (s, 3H), 3.41 - 3.56 (m, 2H), 5.32 (s, 2H), 7.06 (br. s, 1H), 7.19 - 7.30 (m, 2H), 7.54 - 7.66 (m, 1H), 7.87 (br. s, 3H), 8.11 (br. s, 1H), 8.50 (s, 1H).

### Beispiel 358

### N-(2-Ammo-3,3-dimethylcyclobutyl)-8-[(2,6-difluorbenzyl)oxy]-2,6-dimethylimidazo[1,2-a]pyridin-3-carboxamid Formiat (Isomerengemisch)

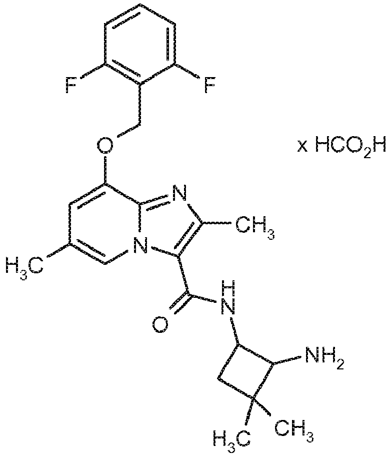

200 mg (0.60 mmol) 8-[(2,6-Difluorbenzyl)oxy]-2,6-dimethylimidazo[1,2-a]pyridin-3-carbonsäure (Beispiel 21A), 218 mg (0.57 mmol) HATU und 308 mg (2.39 mmol) N,N-Diisopropylethylamin wurden in 3.5 ml DMF vorgelegt und 10 min bei RT gerührt. Anschließend wurde die Reaktionsgemisch zu einer Mischung von 296 mg (1.50 mmol) 3,3-Dimethylcyclobutan-1,2-diamindihydrochlorid (Isomerengemisch; beschrieben in: K. Scholz et al. Liebigs Annalen der Chemie, 1981, (2), 248-55; D. Hossain et al. Synthetic Communications 2012 42, 1200-1210; M. Klapper et al. Angewandte Chemie 2003, 115, 4835-4690) und 467 mg (3.61 mmol) N,N-Diisopropylethylamin, gelöst in 0.7 ml DMF, getropft und 30 min bei RT gerührt. Die Reaktionsmischung wurde mittels präparativer HPLC gereinigt (RP-C18, Laufmittel: Acetonitril/Wasser-Gradient unter Zusatz von 0.05% Ameisensäure). Es wurden 131 mg (44% d. Th.) der Titelverbindung erhalten.
LC-MS (Methode 2): Rₜ = 0.62 min
MS (ESpos): m/z = 429 (M+H)⁺

### Beispiel 359

### ent-N-{2-Amino-2-methylpentyl)-8-[(3-cyclopropyl-2,6-difluorbenzyl)oxy]-2,6-dimethylimidazo[1,2-a]pyridin-3-carboxamid

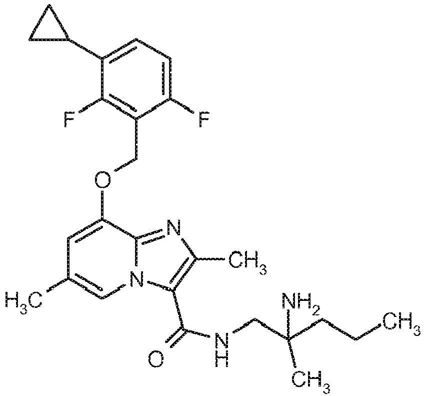

Eine Mischung von 159 mg (0.21 mmol) *ent*-Benzyl-{1-[({8-[(3-cyclopropyl-2,6-difluorbenzyl)oxy]-2,6-dimethylimidazo[1,2-a]pyridin-3-yl}carbonyl)amino]-2-methylpentan-2-yl}carbamat Trifluoracetat aus Beispiel 383A und 45 mg (0.04 mmol) 10 %-igem Palladiumhydroxid auf Kohle in 5.4 ml Ethanol wurde für 1 h bei RT und Normaldruck hydriert. Anschließend wurde über einen Millipore-Filter filtriert. Das Filtrat wurde eingeengt und der Rückstand mittels Dickschichtchromatographie gereinigt (Dichlormethan/Methanol = 10/1). Die Produktfraktion wurde in Dichlormethan aufgenommen und zweimal mit gesättigter, wässriger Natriumhydrogencarbonatlösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 75 mg der Titelverbindung (73 % d. Th.) erhalten.
LC-MS (Methode 2): Rₜ = 0.81 min
MS (ESpos): m/z = 471 (M+H)⁺
¹H-NMR (400 MHz, DMSO-d₆): δ [ppm] = 0.71 - 078 (m, 2H), 0.83 - 0.91 (m, 3H), 0.94 -1.04 (m, 5H), 1.26 - 1.41 (m, 4H), 2.00 - 2.09 (m, 1H), 2.31 (s, 3H), 3.16 - 3.28 (m, 2H), 5.27 (s, 2H), 6.92 (s, 1H), 7.07 - 7.22 (m, 2H), 7.61 - 7.69 (m, 1H), 8.47 (s, 1H), [weiteres Signal unter Lösungsmittelpeak].

In Analogie zu Beispiel 359 wurden die in Tabelle 15 gezeigten Beispiele hergestellt, indem die entsprechende Cbz-geschützten Amine mit Palladium/Kohle (10%ig; 0.1 - 0.3 Äquivalente) in Ethanol bei RT unter Normaldruck unter den beschriebenen Reaktionsbedingungen hydriert wurden. Die Reaktionszeiten lagen hierbei zwischen 1 h und 3 h.

**Tabelle 15:**

| **Beispiel** | **IUPAC-Name / Struktur (Ausbeute)** | **Analytische Daten** |
|---|---|---|
| **360** | *ent-*N*-*(2-Amino-2-methylbutyl)-8-[(2,6-difluor-3-methoxybenzyl)oxy]-2,6-dimethylimidazo[1,2-a]pyridin-3-carboxamid | LC-MS (Methode 2): Rₜ = 0.64 min |
| | | MS (ESpos): m/z = 447 (M+H)⁺ |
| | 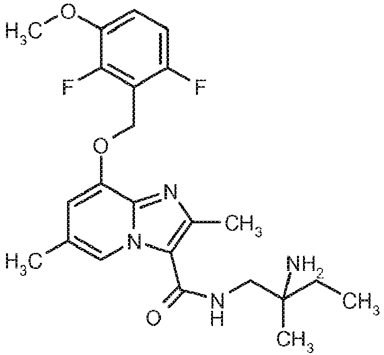 | ¹H NMR (400 MHz, DMSO-d₆) δ [ppm] = 0.87 (t, 3H), 0.98 (s, 3H), 1.30 - 1.40 (m, 2H), 1.41 - 1.69 (br. s, 2H), 2.30 (s, 3H), 3.14 - 3.26 (m, 2H), 3.87 (s, 3H), 5.28 (s, 2H), 6.91 (s, 1H), 7.14 - 7.21 (m, 1H), 7.28 - 7.37 (m, 1H), 7.57 - 7.64 (m, 1H), 8.48 (s, 1H) [weiteres Signal unter Lösungsmittelpeak]. |
| | (36% d. Th.) | |
| **361** | *ent*-N-(2-Amino-3-fluor-2-methylpropyl)-8-[(2,6-difluor-3-methoxybenzyl)oxy]-2,6-dimethylimidazo[1,2-a]pyridin-3-carboxamid | LC-MS (Methode 2): Rₜ = 0.62 min |
| | | MS (ESpos): m/z = 451 (M+H)⁺ |
| | 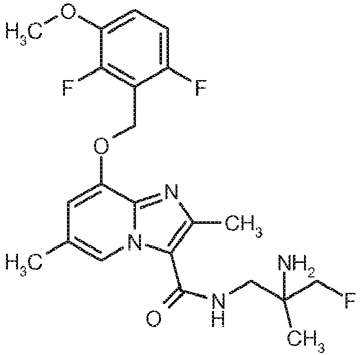 | ¹H-NMR (400 MHz, DMSO-d₆): δ [ppm] = 1.03 (d, 3H), 1.57 - 1.71 (br. s, 2H), 2.31 (s, 3H), 3.23 - 3.38 (m, 2H), 3.87 (s, 3H), 4.09 - 4.15 (m, 1H), 4.20 - 4.26 (s, 1H), 5.28 (s, 2H), 6.91 (s, 1H), 7.13 - 7.21 (m, 1H), 7.28 - 7.37 (m, 1H), 7.64 - 7.71 (m, 1H), 8.47 (s, 1H), [weiteres Signal unter Lösungsmittelpeak]. |
| | (77% d. Th.) | |
| **362** | N-(2-Amino-3-fluor-2-methylpropyl)-8-[(3-cyclopropyl-2,6-difluorbenzyl)oxy]-2,6-dimethylimidazo[1,2-a]pyridin-3-carboxamid | LC-MS (Methode 2): Rₜ = 0.75 min |
| | | MS (ESpos): m/z = 461 (M+H)⁺ |
| | 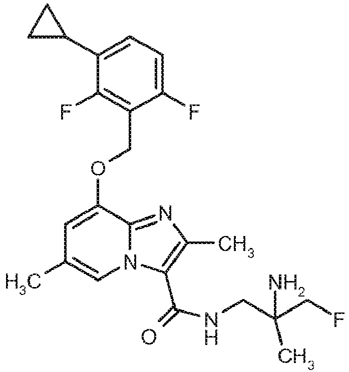 | ¹H-NMR (400 MHz, DMSO-d₆): δ [ppm] = 0.72 - 0.78 (m, 2H), 0.94 - 1.01 (m, 2H), 1.05 (d, 3H), 1.99 - 2.43 (br. s, 2H), 2.00 - 2.09 (m, 1H), 2.32 (s, 3H), 3.25 - 3.40 (m, 2H), 4.10 - 4.16 (m, 1H), 4.22 - 4.28 (m, 1H), 5.27 (s, 2H), 6.93 (s, 1H), 7.07 - 7.22 (m, 2H), 7.65 - 7.72 (m, 1H), 8.47 (s, 1H), [weiteres Signal unter Lösungsmittelpeak]. |
| | (72% d. Th.) | |
| **363** | *ent*-N-(2-Amino-2-methylbutyl)-8-[(3-cyclopropyl-2,6-difluorbenzyl)oxy]-2,6-dimethylimidazo[1,2-a]pyridin-3-carboxamid | LC-MS (Methode 2): Rₜ = 0.76 min |
| | | MS (ESpos): m/z = 457 (M+H)⁺ |
| | 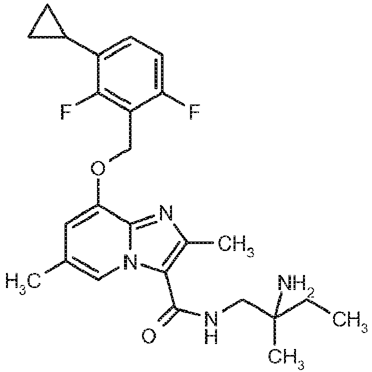 | ¹H-NMR (400 MHz, DMSO-d₆): δ [ppm] = 0.72 - 0.78 (m, 2H), 0.87 (t, 3H), 0.94 - 1.03 (m, 5H), 1.32 - 1.44 (m, 2H), 2.00 - 2.10 (m, 1H), 2.31 (s, 3H), 3.16 - 3.29 (m, 2H), 5.27 (s, 2H), 6.93 (s, 1H), 7.07 - 7.22 (m, 2H), 7.61 - 7.68 (m, 1H), 8.49 (s, 1H), [weiteres Signal unter Lösungsmittelpeak]. |
| | (62% d. Th.) | |

### Beispiel 364

### ent-N-(2-Amino-2-methylpentyl)-8-[(2,6-difluor-3-methoxybenzyl)oxy]-2,6-dimethylimidazo[1,2-a]pyridin-3-carboxamid

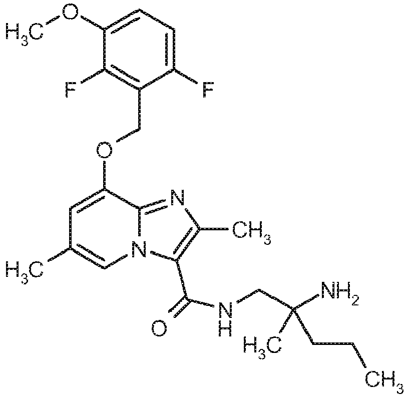

Eine Mischung von 205 mg (0.28 mmol) *ent*-Benzyl-{1-[({8-[(2,6-difluor-3-methoxybenzyl)oxy]-2,6-dimethylimidazo[1,2-a]pyridin-3-yl}carbonyl)amino]-2-methylpentan-2-yl}carbamat Trifluoracetat aus Beispiel 386A und 59 mg (0.06 mmol) 10 %-igem Palladiumhydroxid auf Kohle in 7.1 ml Ethanol wurde für 2 h bei RT und Normaldruck hydriert. Anschließend wurde über einen Millipore-Filter filtriert. Das Filtrat wurde einrotiert und der Rückstand wurde mittels Dickschichtchromatographie gereinigt (Dichlormethan/Methanol = 7.5/1). Die Produktfraktion wurde in Dichlormethan aufgenommen und zweimal mit gesättigter, wässriger Natriumhydrogencarbonatlösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 80 mg der Titelverbindung (63 % d. Th.) erhalten.
LC-MS (Methode 2): Rₜ = 0.69 min
MS (ESpos): m/z = 461 (M+H)⁺
¹H-NMR (400 MHz, DMSO-d₆): δ [ppm] = 0.87 (t, 3H), 0.99 (s, 3H), 1.24 - 1.40 (m, 4H), 1.50 - 1.90 (br. s, 2H), 2.30 (s, 3H), 3.14 - 3.26 (m, 2H), 3.87 (s, 3H), 5.28 (s, 2H), 6.91 (s, 1H), 7.13 - 7.21 (m, 1H), 7.28 - 7.37 (m, 1H), 7.58 - 7.67 (m, 1H), 8.47 (s, 1H) [weiteres Signal unter Lösungsmittelpeak].

### Beispiel 365

### N-(3-Aminoadamantan-1-yl)-8-[(2,6-difluorbenzyl)oxy]-2,6-dimethylimidazo[1,2-a]pyridin-3-carboxamid Formiat

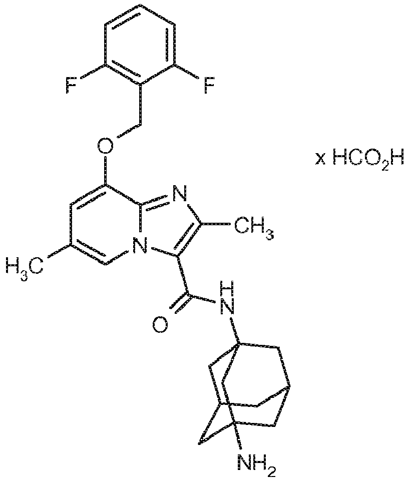

150 mg (0.45 mmol) 8-[(2,6-Difluorbenzyl)oxy]-2,6-dimethylimidazo[1,2-a]pyridin-3-carbonsäure Beispiel 21A, 206 mg (0.54 mmol) HATU und 117 mg (0.90 mmol) N,N-Diisopropylethylamin wurden in 1.3 ml DMF vorgelegt und 10 min bei RT gerührt. Anschließend wurde die Reaktionsgemisch zu einer Mischung von 324 mg (1.35 mmol) Adamantan-1,3-diamindihydrochlorid (G. Senchyk et al. Inorganica Chimica Acta, 2009, 362(12), 4439-4448) und 700 mg (5.42 mmol) N,N-Diisopropylethylamin in 0.6 ml DMF und 3.2 ml DMSO bei 60°C zugetropft und 1 h bei 60°C gerührt. Die Reaktionsmischung wurde abgekühlt und abfiltriert. Das Filtrat wurde mittels präparativer HPLC gereinigt (RP-C18, Laufmittel: Acetonitril/Wasser-Gradient unter Zusatz von 0.05% Ameisensäure). Es wurden 60 mg (25% d. Th.) der Titelverbindung erhalten.
LC-MS (Methode 2): Rₜ = 0.66 min
MS (ESpos): m/z = 481 (M+H)⁺

### Beispiel 366

### rac-8-[(2,6-Difluorbenzyl)oxy]-2,6-dimethyl-N-(thiomorpholin-3-ylmethyl)imidazo[1,2-a]pyridin-3-carboxamid

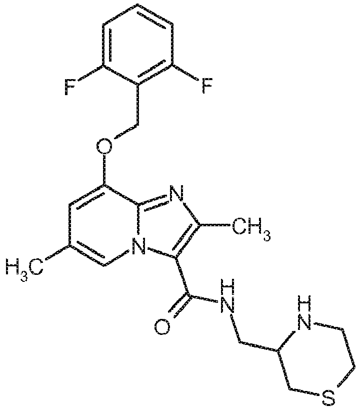

13 mg (0.1 mmol) 1-(Thiomorpholin-3-yl)methanamin (s. A. Eremeev et. Al. Zhurnal Organicheskoi Khimii, 21(10), 2239-41; 1985) wurden in einer 96er deep well Multititerplatte vorgelegt. Eine Lösung von 33 mg (0.1 mmol) 8-[(2,6-Difluorbenzyl)oxy]-2,6-dimethylimidazo[1,2,a]pyridin-3-carbonsäure aus Beispiel 21A in 0.3 ml DMF und eine Lösung von 45 mg (0.12 mmol) HATU in 0.3 ml DMF wurden nacheinander hinzugefügt. Nach Zugabe von 20 mg (0.2 mmol) 4-Methylmorpholin wurde das Gemisch bei RT über Nacht geschüttelt. Dann wurde filtriert und aus dem Filtrat die Zielverbindung per präparativer LC-MS (Methode 12) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 17.8 mg (39% d. Th.) erhalten.
LC-MS (Methode 8): Rₜ = 0.65 min
MS (ESpos): m/z = 447 (M+H)⁺

In Analogie zu Beispiel 366 wurden die in Tabelle 16 gezeigten Beispielverbindungen hergestellt, indem 8-[(2,6-Difluorbenzyl)oxy]-2,6-dimethylimidazo[1,2-a]pyridin-3-carbonsäure aus Beispiel 21A mit den entsprechenden, kommerziell erhältlichen oder zuvor beschriebenen Aminen unter den beschriebenen Bedingungen umgesetzt wurden:

**Tabelle 16:**

| **Beispiel** | **IUPAC-Name / Struktur (Ausbeute)** | **Analytische Daten** |
|---|---|---|
| **367** | *rac*-N-{2-Amino-2-[3-(2-hydroxyethoxy)phenyl]ethyl}-8-[(2,6-difluorbenzyl)oxy]-2,6-dimethylimidazo[1,2-a]pyridin-3-carboxamid | LC-MS (Methode 8): Rₜ = 0.65 min |
| | | MS (ESpos): m/z = 511 (M+H)⁺ |
| | 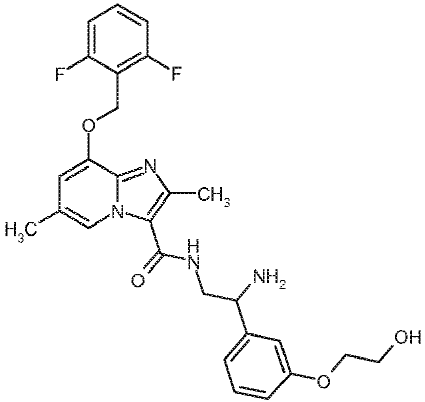 | |
| | (17% d. Th.; Reinheit 82%) | |
| **368** | *rac*-N-[2-Amino-3-(4-methoxyphenyl)-2-methylpropyl]-8-[(2,6-difluorbenzyl)oxy]-2,6-dimethylimidazo[1,2-alpyridin-3-carboxamid | LC-MS (Methode 8): Rₜ = 0.73 min |
| | | MS (ESpos): m/z = 509 (M+H)⁺ |
| | 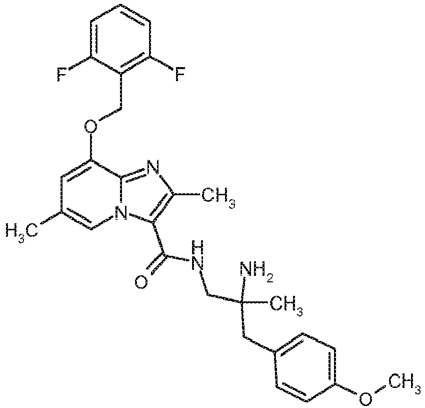 | |
| | (36% d. Th.; Reinheit 94%) | |
| **369** | *rac*-N-[2-Amino-2-(5-methyl-2-furyl)ethyl]-8-[(2,6-difluorbenzyl)oxy]-2,6-dimethylimidazo[1,2-alpyridin-3-carboxamid | LC-MS (Methode 8): Rₜ = 0.67 min |
| | | MS (ESpos): m/z = 455 (M+H)⁺ |
| | 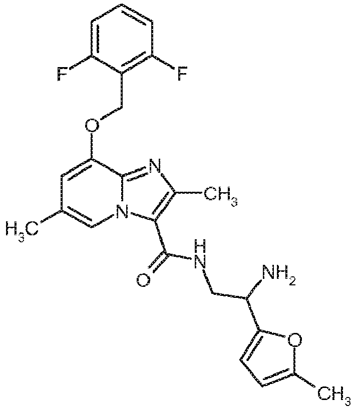 | |
| | (19% d. Th.; Reinheit 89%) | |
| **370** | *rac*-N-[2-Amino-2-(1-methyl-1H-pyrazol-4-yl)ethyl]-8-[(2,6-difluorbenzyl)oxy]-2,6-dimethylimidazo[1,2-alpyridin-3-carboxamid | LC-MS (Methode 8): Rₜ = 0.62 min |
| | | MS (ESpos): m/z = 455 (M+H)⁺ |
| | 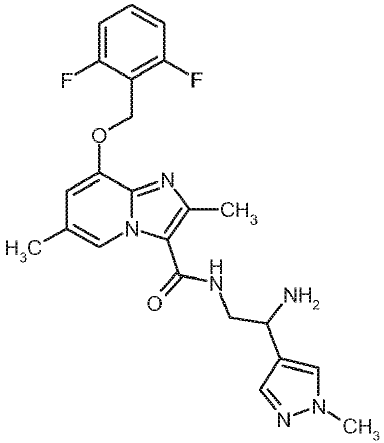 | |
| | (6% d. Th.; Reinheit 90%) | |
| **371** | *rac*-N-[2-Amino-2-(2,3-dihydro-1,4-benzodioxin-6-yl)ethyl]-8-[(2,6-difluorbenzyl)oxy]-2,6-dimethylimidazo[1,2-a]pyridin-3-carboxamid | LC-MS (Methode 8): Rₜ = 0.69 min |
| | | MS (ESpos): m/z = 509 (M+H)⁺ |
| | 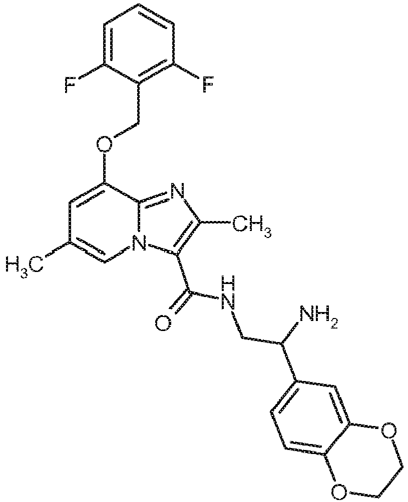 | |
| | (1% d. Th.; Reinheit 82%) | |
| **372** | *rac*-N-[2-Amino-2-(chinolin-6-yl)ethyl]-8-[(2,6-difluorbenzyl)oxy]-2,6-dimethylimidazo[1,2-a]pyridin-3-carboxamid | LC-MS (Methode 8): Rₜ = 0.66 min |
| | | MS (ESpos): m/z = 502 (M+H)⁺ |
| | 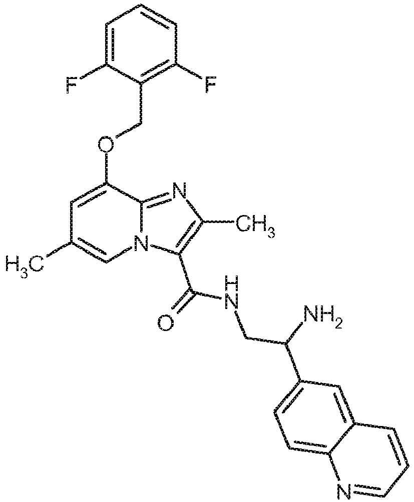 | |
| | (6% d. Th.; Reinheit 78%) | |
| **373** | *rac*-N-[2-Amino-2-(1-benzothiophen-3-yl)ethyl]-8-[(2,6-difluorbenzyl)oxy]-2,6-dimethylimidazo[1,2-a]pyridin-3-carboxamid | LC-MS (Methode 8): Rₜ = 0.74 min |
| | | MS (ESpos): m/z = 507 (M+H)⁺ |
| | 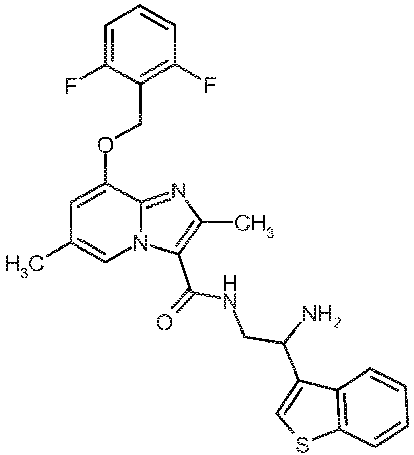 | |
| | (8% d. Th.; Reinheit 75%) | |
| **374** | *rac*-N-[2-Amino-2-(3,4,5-trifluorphenyl)ethyl]-8-[(2,6-difluorbenzyl)oxy]-2,6-dimethylimidazo[1,2-a]pyridin-3-carboxamid | LC-MS (Methode 8): Rₜ = 0. 73 min |
| | | MS (ESpos): m/z = 505 (M+H)⁺ |
| | 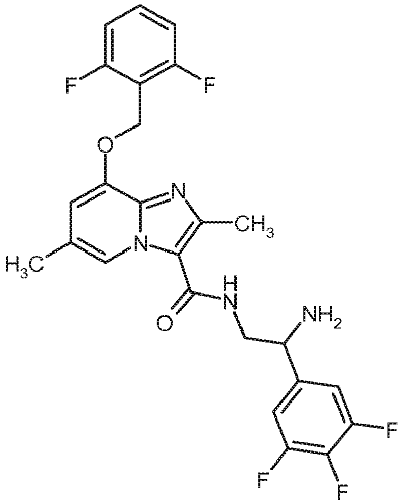 | |
| | (4% d. Th.; Reinheit 91%) | |
| **375** | *rac*-N-{2-Amino-2-[3-(difluormethoxy)phenyl]ethyl}-8-[(2,6-difluorbenzyl)oxy]-2,6-dimethylimidazo[1,2-a]pyridin-3-carboxamid | LC-MS (Methode 8): Rₜ = 0. 73 min |
| | | MS (ESpos): m/z = 517 (M+H)⁺ |
| | 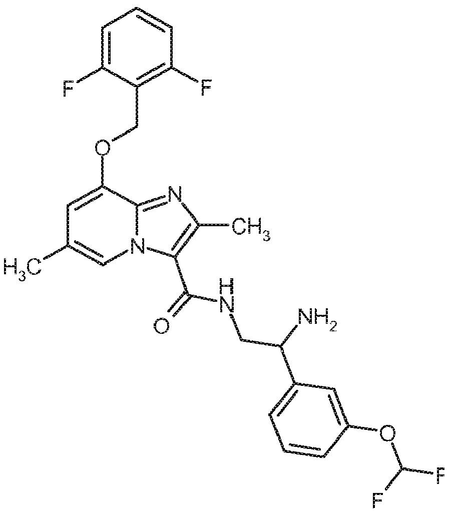 | |
| | (5% d. Th.; Reinheit 80%) | |
| **376** | *rac*-N-{2-Amino-2-[3-(trifluormethyl)phenyl]ethyl}-8-[(2,6-difluorbenzyl)oxy]-2,6-dimethylimidazo[1,2-a]pyridin-3-carboxamid | LC-MS (Methode 8): Rₜ = 0. 74 min |
| | | MS (ESpos): m/z = 519 (M+H)⁺ |
| | 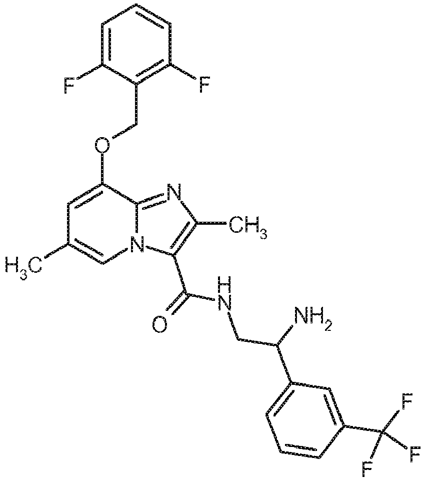 | |
| | (32% d. Th.) | |

### Beispiel 377

### rac-N-{2-Amino-2-[3-(trifluormethoxy)phenyl]ethyl}-8-[(2,6-difluorbenzyl)oxy]-2,6-dimethylimidazo[1,2-a]pyridin-3-carboxamid

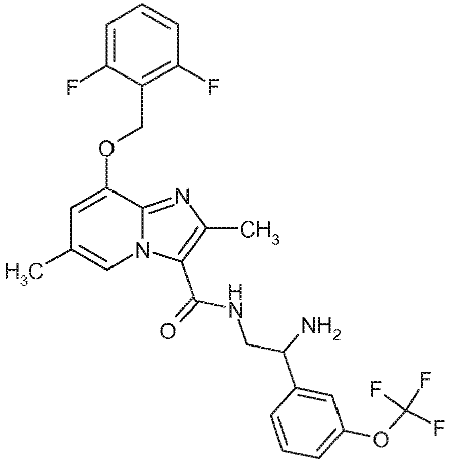

In einem Rundkolben wurden 100 mg (0.31 mmol) *tert*.-Butyl-{2-amino-2-[3-(trifluormethoxy)phenyl]ethyl}carbamat in 2 ml Dichlormethan gelöst, mit 2 ml (8 mmol) Chlorwasserstoff in Dioxan (4 M) versetzt und 4 Stunden bei RT gerührt. Anschließend wurde bis zur Trockne eingedampft und 30 mg des erhaltenen Feststoffes wurden auf eine 96er deep well Multititerplatte transferiert. Es wurde nacheinander mit einer Lösung von 33 mg (0.1 mmol) 8-[(2,6-Difluorbenzyl)oxy]-2,6-dimethylimidazo[1,2-a]pyridin-3-carbonsäure aus Beispiel 21A in 0.3 ml DMF und 49 mg (0.13 mmol) HATU in 0.3 ml DMF versetzt, dann wurden 20 mg (0.2 mmol) 4-Methylmorpholin hinzugefügt und es wurde bei RT über Nacht geschüttelt. Anschließend wurde das Reaktionsgemisch filtriert und aus dem Filtrat die Zielverbindung per präparativer LC-MS (Methode 12) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 10.3 mg (19% d. Th.) erhalten.
LC-MS (Methode 8): Rₜ = 0.75 min
MS (ESpos): m/z = 535 (M+H)⁺

In Analogie zu Beispiel 377 wurden die in Tabelle 17 gezeigten Beispielverbindungen hergestellt, indem 8-[(2,6-Difluorbenzyl)oxy]-2,6-dimethylimidazo[1,2-a]pyridin-3-carbonsäure aus Beispiel 21A mit den entsprechenden, kommerziell erhältlichen oder zuvor beschriebenen Aminen unter den beschriebenen Bedingungen umgesetzt wurden:

**Tabelle 17:**

| **Beispiel** | **IUPAC-Name / Struktur (Ausbeute)** | **Analytische Daten** |
|---|---|---|
| **378** | *rac*-N-[2-Amino-2-(3-methoxyphenyl)ethyl]-8-[(2,6-difluorbenzyl)oxy]-2,6-dimethylimidazo[1,2-a]pyridin-3-carboxamid | LC-MS (Methode 8): Rₜ = 0.69 min |
| | | MS (ESpos): m/z = 481 (M+H)⁺ |
| | 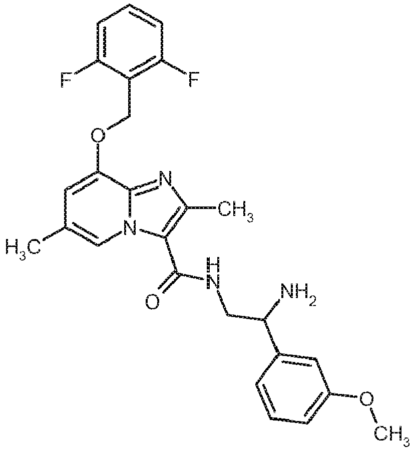 | |
| | (9% d. Th.; Reinheit 88%) | |

### Beispiel 379

### rac-N-[2-Amino-1-(2-naphthyl)ethyl]-8-[(2,6-difluorbenzyl)oxy]-2,6-dimethylimidazo[1,2-a]pyridin-3-carboxamid

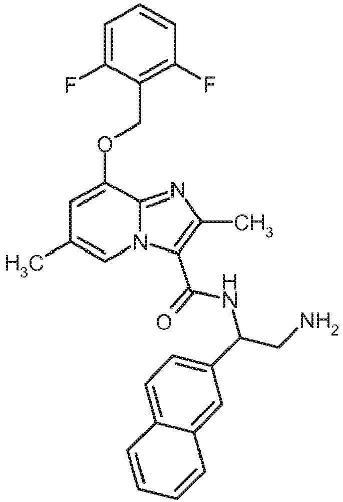

29 mg (0.1 mmol) *tert*-Butyl-[2-amino-2-(2-naphthyl)ethyl]carbamat wurden in einer 96er deep well Multititerplatte vorgelegt. Eine Lösung von 33 mg (0.1 mmol) 8-[(2,6-Difluorbenzyl)oxy]-2,6-dimethylimidazo[1,2,a]pyridin-3-carbonsäure aus Beispiel 21A in 0.3 ml DMF und eine Lösung von 45 mg (0.12 mmol) HATU in 0.3 ml DMF wurden nacheinander hinzugefügt. Nach Zugabe von 20 mg (0.2 mmol) 4-Methylmorpholin wurde das Gemisch bei RT über Nacht geschüttelt. Danach wurde das Lösemittel vollständig abgedampft, der Rückstand mit 0.6 ml TFA versetzt und bei RT über Nacht geschüttelt. Anschließend wurde eingedampft, der Rückstand erneut in DMF gelöst, filtriert und aus dem Filtrat die Zielverbindung per präparativer LC-MS (Methode 12) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 25.8 mg (49% d. Th.; Reinheit 94%) erhalten.
LC-MS (Methode 8): Rₜ = 0.76 min
MS (ESpos): m/z = 501 (M+H)⁺

In Analogie zu Beispiel 379 wurden die in Tabelle 18 gezeigten Beispielverbindungen hergestellt, indem 8-[(2,6-Difluorbenzyl)oxy]-2,6-dimethylimidazo[1,2-a]pyridin-3-carbonsäure aus Beispiel 21A mit den entsprechenden, kommerziell erhältlichen oder zuvor beschriebenen Aminen unter den beschriebenen Bedingungen umgesetzt wurden:

**Tabelle 18:**

| **Beispiel** | **IUPAC-Name / Struktur (Ausbeute)** | **Analytische Daten** |
|---|---|---|
| **380** | *rac*-N-{2-Amino-1-[3-(trifluormethoxy)phenyl]ethyl}-8-[(2,6-difluorbenzyl)oxy]-2,6-dimethylimidazo[1,2-a]pyridin-3-carboxamid | LC-MS (Methode 8): Rₜ = 0.78 min |
| | | MS (ESpos): m/z = 535 (M+H)⁺ |
| | 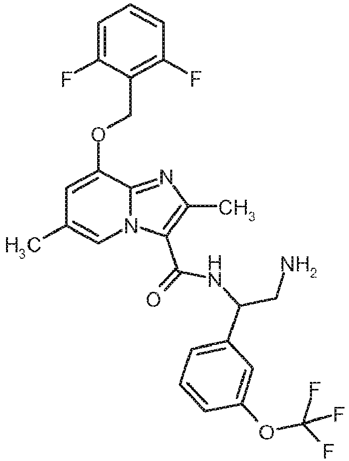 | |
| | (55% d. Th.; Reinheit 87%) | |
| **381** | *rac-*N-[2-Amino-1-(1,3-benzodioxol-5-yl)ethyl]-8-[(2,6-difluorbenzyl)oxy]-2,6-dimethylimidazo[1,2-a]pyridin-3-carboxamid | LC-MS (Methode 8): Rₜ = 0.70 min |
| | | MS (ESpos): m/z = 495 (M+H)⁺ |
| | 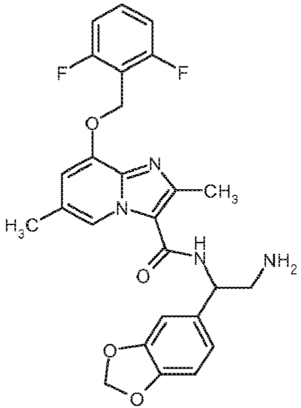 | |
| | (69% d. Th.; Reinheit 94%) | |
| **382** | *rac*-N-[2-Amino-1-(3-methoxyphenyl)ethyl]-8-[(2,6-difluorbenzyl)oxy]-2,6-dimethylimidazo[1,2-a]pyridin-3-carboxamid | LC-MS (Methode 8): Rₜ = 0.71 min |
| | | MS (ESpos): m/z = 481 (M+H)⁺ |
| | 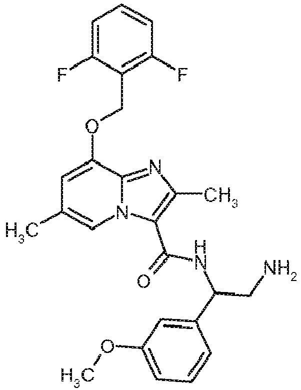 | |
| | (62% d. Th.; Reinheit 89%) | |
| **383** | *rac*-N-[2-Amino-1-(3-bromphenyl)ethyl]-8-[(2,6-difluorbenzyl)oxy]-2,6-dimethylimidazo[1,2-a]pyridin-3-carboxamid | LC-MS (Methode 8): Rₜ = 0.75 min |
| | | MS (ESpos): m/z = 529 (M+H)⁺ |
| | 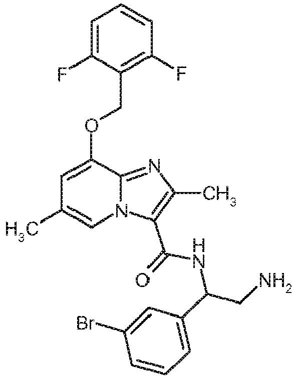 | |
| | (51% d. Th.; Reinheit 92%) | |
| **384** | *rac*-N-[2-Amino-1-(4-nitrophenyl)ethyl]-8-[(2,6-difluorbenzyl)oxy]-2,6-dimethylimidazo[1,2-a]pyridin-3-carboxamid | LC-MS (Methode 8): Rₜ = 0.73 min |
| | | MS (ESpos): m/z = 496 (M+H)⁺ |
| | 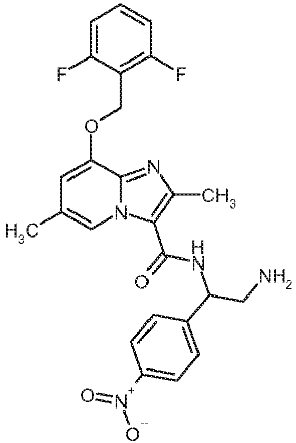 | |
| | (53% d. Th.; Reinheit 91%) | |

### Beispiel 385

### rac-N-[2-Amino-1-(3-ethenylphenyl)ethyl]-8-[(2,6-difluorbenzyl)oxy]-2,6-dimethylimidazo[1,2-a]pyridin-3-carboxamid

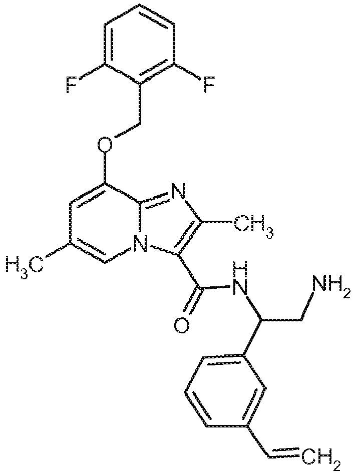

16 mg (0.1 mmol) 1-(3-Vinylphenyl)ethan-1,2-diamin wurden in einer 96er deep well Multititerplatte vorgelegt und nacheinander mit 0.2 ml Dichlormethan und einer Lösung von 22 mg (0.1 mmol) Di-*tert-*butylcarbonat in 0.2 ml Dichlormethan versetzt und 2 Stunden bei RT geschüttelt. In einem Kolben wurden 33 mg (0.1 mmol) 8-[(2,6-Difluorbenzyl)oxy]-2,6-dimethylimidazo[1,2-a]pyridin-3-carbonsäure aus Beispiel 21A und 49 mg (0.13 mmol) HATU in 0.4 ml DMF gelöst, mit 20 mg (0.2 mmol) 4-Methylmorpholin versetzt und 30 min bei RT gerührt. Anschließend wurde dieses Gemisch auf die Multititerplatte pipettiert und diese 48 Stunden bei RT geschüttelt. Das Lösemittel wurde vollständig abgedampft, der Rückstand mit 0.6 ml TFA versetzt und bei RT über Nacht geschüttelt. Anschließend wurde eingedampft, der Rückstand in DMF gelöst, filtriert und aus dem Filtrat die Zielverbindung per präparativer LC-MS (Methode 12) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 2.9 mg (6% d. Th.) erhalten.
LC-MS (Methode 8): Rₜ = 0.72 min
MS (ESpos): m/z = 477 (M+H)⁺

In Analogie zu Beispiel 385 wurden die in Tabelle 19 gezeigten Beispielverbindungen hergestellt, indem 8-[(2,6-Difluorbenzyl)oxy]-2,6-dimethylimidazo[1,2-a]pyridin-3-carbonsäure aus Beispiel 21A mit den entsprechenden, kommerziell erhältlichen oder zuvor beschriebenen Aminen unter den beschriebenen Bedingungen umgesetzt wurden:

**Tabelle 19:**

| **Beispiel** | **IUPAC-Name / Struktur (Ausbeute)** | **Analytische Daten** |
|---|---|---|
| **386** | *rac*-N-[1-Amino-3-(4-methoxyphenyl)-2-methylpropan-2-yl]-8-[(2,6-difluorbenzyl)oxy]-2,6-dimethylimidazo[1,2-a]pyridin-3-carboxamid | LC-MS (Methode 8): Rₜ = 0.75 min |
| | | MS (ESpos): m/z = 509 (M+H)⁺ |
| | 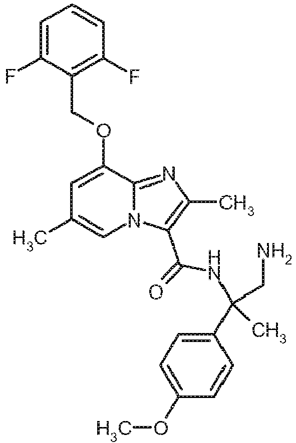 | |
| | (16% d. Th.) | |
| **387** | *rac*-N-[2-Amino-1-(1-methyl-1H-pyrazol-4-yl)ethyl]-8-[(2,6-difluorbenzyl)oxy]-2,6-dimethylimidazo[1,2-a]pyridin-3-carboxamid | LC-MS (Methode 8): Rₜ = 0.62 min |
| | | MS (ESpos): m/z = 455 (M+H)⁺ |
| | 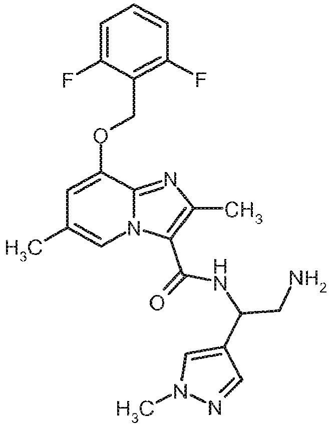 | |
| | (8% d. Th.; Reinheit 84%) | |
| **388** | *rac*-N-[2-Amino-1-(2,3-dihydro-1,4-benzodioxin-6-yl)ethyl]-8-[(2,6-difluorbenzyl)oxy]-2,6-dimethylimidazo[1,2-a]pyridin-3-carboxamid | LC-MS (Methode 8): Rₜ = 0.69 min |
| | | MS (ESpos): m/z = 509 (M+H)⁺ |
| | 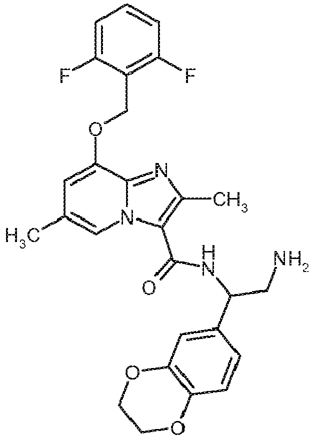 | |
| | (12%d. Th.) | |
| **389** | *rac*-N-[2-Amino-1-(1-benzothiophen-3-yl)ethyl]-8-[(2,6-difluorbenzyl)oxy]-2,6-dimethylimidazo[1,2-a]pyridin-3-carboxamid | LC-MS (Methode 8): Rₜ = 0.75 min |
| | | MS (ESpos): m/z = 507 (M+H)⁺ |
| | 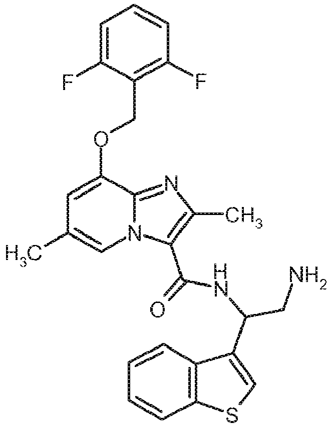 | |
| | (22% d. Th.) | |
| **390** | *rac*-N-[2-Amino-1-(3,4,5-trifluorphenyl)ethyl]-8-[(2,6-difluorbenzyl)oxy]-2,6-dimethylimidazo[1,2-a]pyridin-3-carboxamid | LC-MS (Methode 8): Rₜ = 0.73 min |
| | | MS (ESpos): m/z = 505 (M+H)⁺ |
| | 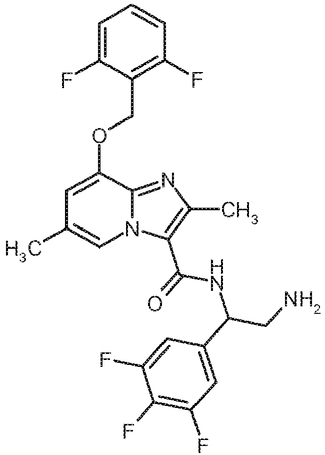 | |
| | (13% d. Th.) | |
| **391** | *rac*-N-[1-Amino-4-(methylsulfenyl)butan-2-yl]-8-[(2,6-difluorbenzyl)oxy]-2,6-dimethylimidazo[1,2-a]pyridin-3-carboxamid | LC-MS (Methode 8): Rₜ = 0.66 min |
| | | MS (ESpos): m/z = 449 (M+H)⁺ |
| | 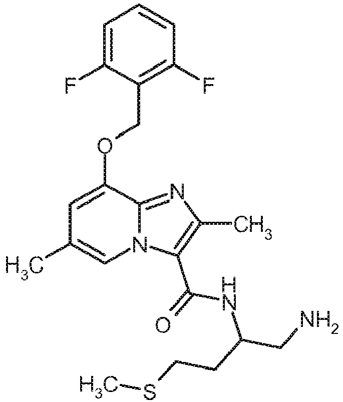 | |
| | (12% d. Th.; Reinheit 84%) | |
| **392** | *rac*-N-{2-Amino-1-[3-(difluormethoxy)phenyl]ethyl}-8-[(2,6-difluorbenzyl)oxy]-2,6-dimethylimidazo[1,2-a]pyridin-3-carboxamid | LC-MS (Methode 8): Rₜ = 0.72 min |
| | | MS (ESpos): m/z = 517 (M+H)⁺ |
| | 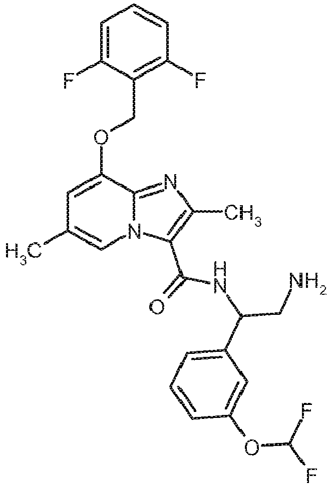 | |
| | (16% d. Th.) | |
| **393** | *rac*-N-{2-Amino-1-[3-{2-hydroxyethoxy)phenyl]ethyl}-8-[(2,6-difluorbenzyl)oxy]-2,6-dimethylimidazo[1,2-a]pyridin-3-carboxamid | LC-MS (Methode 8): Rₜ = 0.65 min |
| | | MS (ESpos): m/z = 511 (M+H)⁺ |
| | 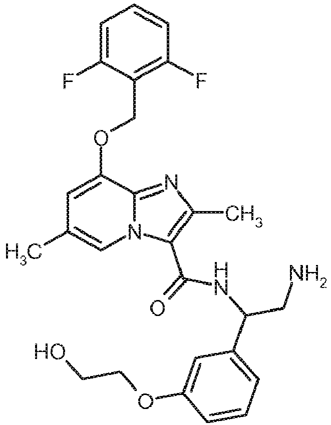 | |
| | (8% d. Th.; Reinheit 85%) | |
| **394** | *rac*-N-{2-Amino-1-[3-(trifluormethyl)phenyl]ethyl)-8-[(2,6-difluorbenzyl)oxy]-2,6-dimethylimidazo[1,2-a]pyridin-3-carboxamid | LC-MS (Methode 8): Rₜ = 0.77 min |
| | | MS (ESpos): m/z = 519 (M+H)⁺ |
| | 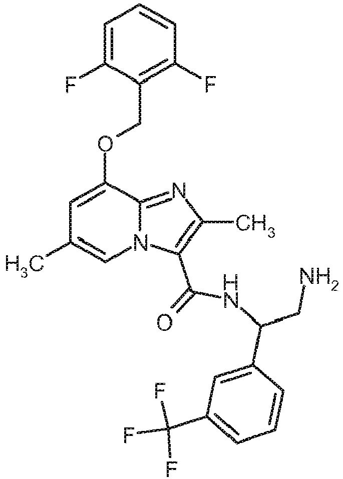 | |
| | (15% d. Th.) | |
| **395** | *rac*-N-[2-Amino-1-{chinolin-6-yl)ethyl]-8-[(2,6-difluorbenzyl)oxy]-2,6-dimethylimidazo[1,2-a]pyridin-3-carboxamid | LC-MS (Methode 8): Rₜ = 0.68 min |
| | | MS (ESpos): m/z = 502 (M+H)⁺ |
| | 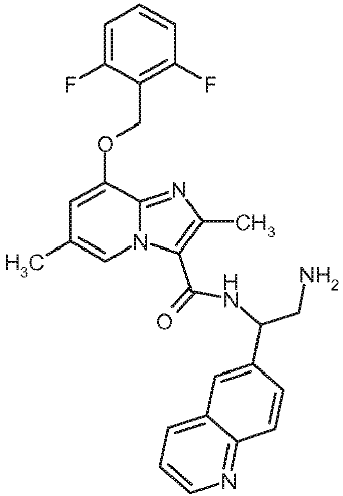 | |
| | (2% d. Th.) | |

### Beispiel 396

### rac-N-[1-(3-Acetamidophenyl)-2-aminoethyl]-8-[(2,6-difluorbenzyl)oxy]-2,6-dimethylimidazo[1,2-a]pyridin-3-carboxamid

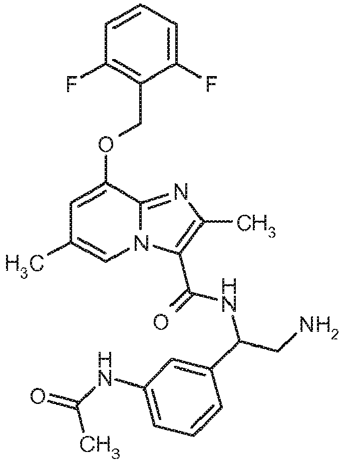

100 mg (0.14 mmol) *rac-tert*.-Butyl-{2-(3-acetamidophenyl)-2-[({8-[(2,6-difluorbenzyl)oxy]-2,6-dimethylimidazo[1,2,a]pyridin-3-yl}carbonyl)amino]ethyl}carbamat Trifluoracetat aus Beispiel 414A wurden in 0.6 ml Diethylether vorgelegt, mit 2.1 ml (4.2 mmol) 2 N Chlorwasserstoff in Diethylether versetzt und über Nacht bei RT gerührt. Das Reaktionsgemisch wurde eingeengt, der Rückstand wurde mit Dichlormethan versetzt und zweimal mit gesättigter, wässriger Natriumhydrogen-carbonat-Lösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 52 mg der Zielverbindung (72% d. Th.) erhalten.
LC-MS (Methode 2): Rₜ = 0.62 min
MS (ESpos): m/z = 508 (M+H)⁺
¹H-NMR (500 MHz, DMSO-d₆): δ [ppm] = 1.62 (br. s, 2H), 2.02 (s, 3H), 2.29 (s, 3H), 2.60 (s, 3H), 2.89 (d, 2H), 4.86 - 4.94 (m, 1H), 5.29 (s, 2H), 6.90 (s, 1H), 7.07 (d, 1H), 7.19 - 7.28 (m, 3H), 7.43 (d, 1H), 7.54 - 7.63 (m, 2H), 8.11 (br. s, 1H), 8.38 (s, 1H), 9.89 (s, 1H).

In Analogie zu Beispiel 396 wurden die in Tabelle 20 gezeigten Beispiele hergestellt, indem die zuvor beschriebenen Boc-geschützten Diamine unter den beschriebenen Reaktionsbedingungen mit Chlorwasserstofflösung umgesetzt wurden.

**Tabelle 20:**

| **Beispiel** | **IUPAC-Name / Struktur (Ausbeute)** | **Analytische Daten** |
|---|---|---|
| **397** | *rac*-Ethyl-6-{2-amino-1-[({8-[(2,6-difluorbenzyl)oxy]-2,6-dimethylimidazo[1,2-a]pyridin-3-yl}carbonyl)amino]ethyl}pyridin-2-carboxylat | LC-MS (Methode 2): Rₜ = 0.74 min |
| | | MS (ESpos): m/z = 524 (M+H)⁺ |
| | 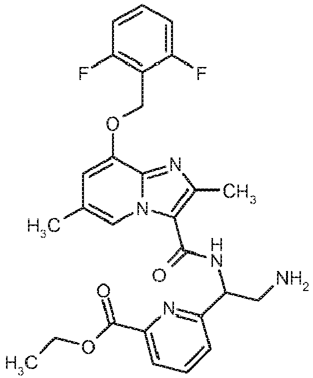 | ¹H-NMR (500 MHz, DMSO-d₆): δ [ppm] = 1.34 (t, 3H), 2.29 (s, 3H), 2.68 (s, 3H), 2.97 - 3.11 (m, 2H), 4.37 (q, 2H), 5.14 - 5.19 (m, 1H), 5.31 (s, 2H), 6.93 (s, 1H), 7.19 - 7.28 (m, 2H), 7.56 - 7.63 (m, 1H), 7.72 (d, 1H), 7.92 - 8.02 (m, 2H), 8.10 - 8.24 (m, 1H), 8.53 (s, 1H). |
| | (76% d. Th.) | |
| **398** | *rac*-N-[2-Ammo-1-(4-cyanphenyl)ethyl]-8-[(2,6-difluorbenzyl)oxy]-2,6-dimethylimidazo[1,2-a]pyridin-3-carboxamid | LC-MS (Methode 2): Rₜ = 0.72 min |
| | | MS (ESpos): m/z = 476 (M+H)⁺ |
| | 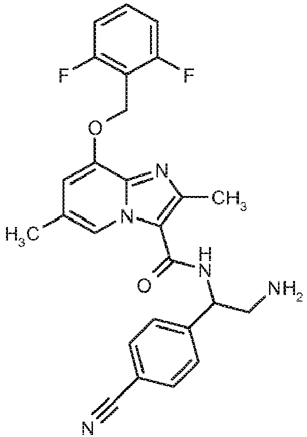 | ¹H-NMR (500 MHz, DMSO-d₆): δ [ppm] = 1.85 (br. s, 2H), 2.28 (s, 3H), 2.60 (s, 3H), 2.93 (d, 2H), 4.98 - 5.06 (m, 1H), 5.29 (s, 2H), 6.91 (s, 1H), 7.19 - 7.26 (m, 2H), 7.55 - 7.63 (m, 3H), 7.82 (d, 2H), 8.20 (br. s, 1H), 8.37 (s, 1H). |
| | (76% d. Th.) | |

### Beispiel 399

### rac-Methyl-3-{1-amino-2-[({8-[(2,6-difluorbenzyl)oxy]-2,6-dimethylimidazo[1,2-a]pyridin-3-yl}carbonyl)amino]ethyl}benzoat

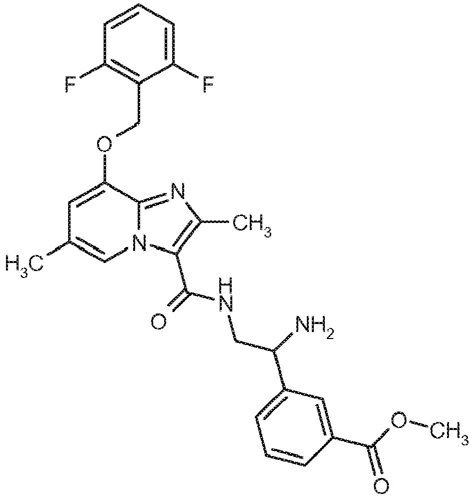

100 mg (0.30 mmol) 8-[(2,6-Difluorbenzyl)oxy]-2,6-dimethylimidazo[1,2-a]pyridin-3-carbonsäure aus Beispiel 21A, 126 mg (0.33 mmol) HATU und 117 mg (0.90 mmol) N,N-Diisopropylethylamin wurden in 1 ml DMF vorgelegt und 10 min bei RT gerührt. Anschließend wurden 67 mg (0.35 mmol) *rac-* Methyl-3-(1,2-diaminoethyl)benzoat hinzugegeben und das Gemisch wurde 2 h bei RT gerührt. Die Reaktionsmischung wurde mit Acetonitril, TFA und Wasser versetzt und mittels präparativer HPLC gereinigt (RP-C18, Laufmittel: Acetonitril/Wasser-Gradient unter Zusatz von 0.1% TFA). Die produkthaltigen Fraktionen wurden eingeengt, der Rückstand in Dichlormethan aufgenommen und zweimal mit gesättigter, wässriger Natriumhydrogencarbonat-Lösung gewaschen. Die vereinten wässrigen Phasen wurden zweimal mit Dichlormethan extrahiert. Die vereinten organischen Phasen wurden über Natriumsulfat getrocknet, filtriert und das Filtrat eingeengt. Es wurden 21 mg (13% d. Th.) der Titelverbindung erhalten.
LC-MS (Methode 2): Rₜ = 0.70 min
MS (ESpos): m/z = 509 (M+H)⁺
¹H-NMR (500 MHz, DMSO-d₆): δ = 2.11 (br. s, 2H), 2.28 (s, 3H), 2.35 (s, 3H), 3.38 - 3.46 (m, 1H), 3.48 - 3.56 (m, 1H), 3.72 (s, 3H), 4.18 (t, 1H), 5.29 (s, 2H), 6.88 (s, 1H), 7.19 - 7.26 (m, 2H), 7.48 (t, 1H), 7.55 - 7.64 (m, 1H), 7.70 (d, 1H), 7.78 (t, 1H), 7.83 (d, 1H), 8.03 (s, 1H), 8.28 (s, 1H).

### Beispiel 400

### rac-N-(2-Amino-2-cyanethyl)-8-[(2,6-difluorbenzyl)oxy]-2,6-dimethylimidazo[1,2-a]pyridin-3-carboxamid

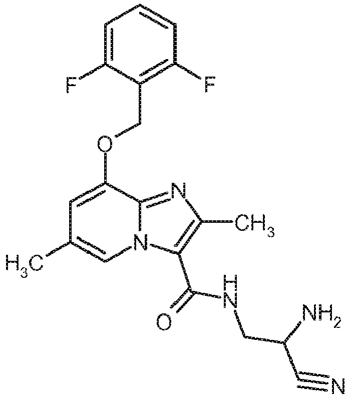

200 mg (0.60 mmol) 8-[(2,6-Difluorbenzyl)oxy]-2,6-dimethylimidazo[1,2-a]pyridin-3-carbonsäure aus Beispiel 21A wurden in 1.8 ml DMF vorgelegt, mit 252 mg (0.66 mmol) HATU und 311 mg (2.41 mmol) N,N-Diisopropylethylamin versetzt und 10 min bei Raumtemperatur gerührt. Das Reaktionsgemisch wurde auf 0°C abgekühlt. Langsam wurde eine Lösung von 109 mg (0.69 mmol) *rac*-2,3-Diaminopropanonitrildihydrochlorid (kommerziell erhältlich; s. a. A. H. Cook et al. Journal of the Chemical Society 1949, 3001) und 194 mg (1.51 mmol) N,N-Diisopropylethylamin in 0.67 ml DMF zugetropft und das Gemisch wurde 1 h bei 0°C gerührt. Die Reaktionslösung wurde mit TFA/Wasser und Acetonitril verdünnt und mittels präparativer HPLC gereinigt (RP18 Säule, Laufmittel: Acetonitril/Wasser-Gradient unter Zusatz von 0.1% TFA). Der eingeengte Rückstand wurde in Dichlormethan und wenig Methanol aufgenommen und zweimal mit gesättigter, wässriger Natriumhydrogencarbonat-Lösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 197 mg der Titelverbindung (79% d. Th.) erhalten.
LC-MS (Methode 2): Rₜ = 0.65 min
MS (ESpos): m/z = 400 (M+H)⁺
¹H-NMR (400 MHz, DMSO-d₆): δ [ppm] = 2.30 (s, 3H), 2.50 (s, 3H), 3.40 - 3.60 (m, 2H), 3.95 - 4.08 (m, 1H), 5.29 (s, 2H), 6.92 (s, 1H), 7.18 - 7.28 (m, 2H), 7.54 - 7.63 (m, 1H), 8.10 (t, 1H), 8.45 (s, 1H).

### Beispiel 401

### ent-N-(2-Amino-2-cyanethyl)-8-[(2,6-fluorbenzyl)oxy]-2,6-dimethylimidazo[1,2-a]pyridin-3-carboxamid (Enantiomer A)

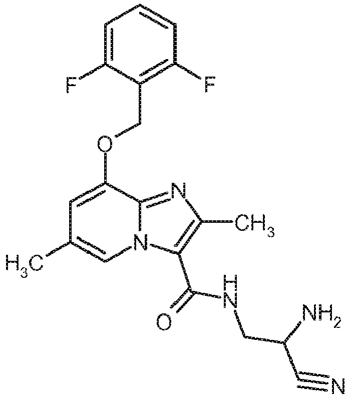

170 mg *rac*-N-(2-Amino-2-cyanethyl)-8-[(2,6-difluorbenzyl)oxy]-2,6-dimethylimidazo[1,2-a]pyridin-3-carboxamid aus Beispiel 400 wurden durch präparativer Trennung an der chiralen Phase in die Enantiomere getrennt [Säule: Daicel Chiralpak AD-H, 5 µm, SFC, 250 x 20 mm, Eluent: 70% Kohlendioxid, 30% Ethanol, Fluß: 80 ml/min, Temperatur: 40°C, Detektion: 210 nm].
Enantiomer A: 70 mg (>99% ee)
Rₜ = 7.67 min [SFC, Daicel Chiralpak AD-H, 250 x 4.6 mm, 5 µm, Eluent: 70% Kohlendioxid, 30% Ethanol, Fluß: 3 ml/min, Temperatur: 30°C, Detektion: 220 nm].

### Beispiel 402

### ent-N-(2-Amino-2-cyanethyl)-8-[(2,6-difluorbenzyl)oxy]-2,6-dimethylimidazo[1,2-a]pyridin-3-carboxamid (Enantiomer B)

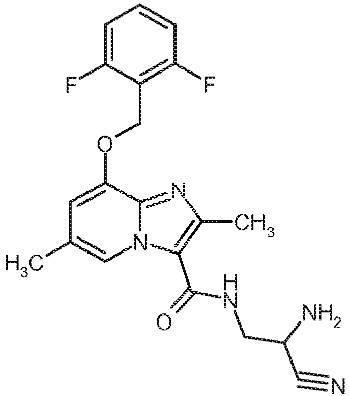

170 mg *rac*-N-(2-Amino-2-cyanethyl)-8-[(2,6-difluorbenzyl)oxy]-2,6-dimethylimidazo[1,2-a]pyridin-3-carboxamid aus Beispiel 400 wurden durch präparativer Trennung an der chiralen Phase in die Enantiomere getrennt [Säule: Daicel Chiralpak AD-H, 5 µm, SFC, 250 x 20 mm, Eluent: 70% Kohlendioxid, 30% Ethanol, Fluß: 80 ml/min, Temperatur: 40°C, Detektion: 210 nm].
Enantiomer B: 60 mg (>99% ee)
Rₜ = 12.45 min [SFC, Daicel Chiralpak AD-H, 250 x 4.6 mm, 5 µm, Eluent: 70% Kohlendioxid, 30% Ethanol, Fluß: 3 ml/min, Temperatur: 30°C, Detektion: 220 nm].

### Beispiel 403

### ent-N-(2-Amino-2-methylpentyl)-8-[(2,6-difluorbenzyl)oxy]-6-(fluormethyl)-2-methylimidazo[1,2-a]pyridin-3-carboxamid

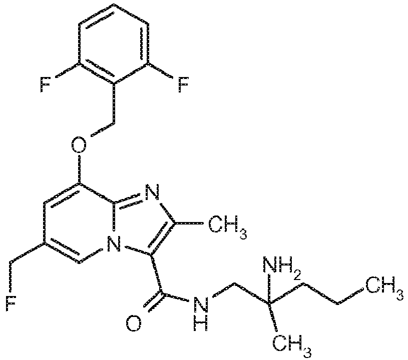

Eine Mischung von 171 mg (0.21 mmol, Reinheit 87%) *ent*-Benzyl-{1-[({8-[(2,6-difluorbenzyl)oxy]-6-(fluormethyl)-2-methylimidazo[1,2-a]pyridin-3-yl}carbonyl)amino]-2-methylpentan-2-yl}carbamat Trifluoracetat aus Beispiel 401A und 11.4 mg 10%igem Palladium auf Kohle (0.01 mmol) in 17 ml Ethanol wurde für 2 h bei Raumtemperatur und Normaldruck hydriert. Während der Reaktionszeit wurden nochmals 20 mg 10%iges Palladium auf Kohle (0.02 mmol) zu dem Reaktionsgemisch gegeben. Anschließend wurde über einen Filter abfiltriert, mit Ethanol gewaschen und das Filtrat eingeengt. Der Rückstand wurde in Dichlormethan aufgenommen und zweimal mit gesättigter, wässriger Natriumhydrogencarbonat-Lösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Das Rohprodukt wurde mittels Dickschichtchromatographie vorgereinigt (Laufmittel: Dichlormethan/Methanol 10/1). Das erhaltene vorgereinigte Produkt wurde an der chiralen Phase nochmals gereinigt [Säule: Daicel Chiralpak AY-H 5µm, 250 x 20 mm; Eluent: 70% iso-Hexan, 30% Isopropanol + 0.2% Diethylamin; Fluß: 20 ml/min; Temperatur 23°C; Detektion 220 nm]. Es wurden 13 mg der Titelverbindung (13 % d. Th.) erhalten.
LC-MS (Methode 2): Rₜ = 0.72 min
MS (ESpos): m/z = 449 (M+H)⁺
¹H-NMR (500 MHz, DMSO-d₆): δ [ppm] = 0.82 - 0.92 (m, 3H), 1.01 (s, 3H), 1.25 - 1.47 (m, 4H), 1.95 - 2.30 (br.s, 2H), 2.56 (s, 3H), 3.14 - 3.27 (m, 2H), 5.33 (s, 2H), 5.47 (d_{H-F}, 2H), 7.11 (s, 1H), 7.21 - 7.28 (m, 2H), 7.55 - 7.64 (m, 1H), 7.69 - 7.78 (m, 1H), 8.77 - 8.81 (m, 1H).

In Analogie zu Beispiel 403 wurden die in Tabelle 21 gezeigten Beispielverbindungen hergestellt, indem die zuvor beschriebenen Cbz-geschützten Amine aus Beispiel 402A und Beispiel 403A unter den beschriebenen Bedingungen hydriert wurden:

**Tabelle 21:**

| **Beispiel** | **IUPAC-Name / Struktur (Ausbeute)** | **Analytische Daten** |
|---|---|---|
| **404** | *ent-*N-(2-Amino-2-methylbutyl)-8-[(2,6-difluorbenzyl)oxy]-6-(fluormethyl)-2-methylimidazo[1,2-a]pyridin-3-carboxamid | LC-MS (Methode 2): Rₜ = 0.67 min |
| | | MS (ESpos): m/z = 435 (M+H)⁺ |
| | 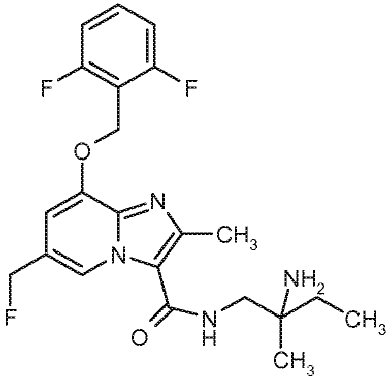 | ¹H NMR (500 MHz, DMSO-d₆) δ [ppm] = 0.87 (t, 3H), 0.99 (s, 3H), 1.32 - 1.45 (m, 2H), 2.56 (s, 3H), 3.16 - 3.28 (m, 2H), 5.33 (s, 2H), 5.47 (d_{H-F}, 2H), 7.11 (s, 1H), 7.21 - 7.28 (m, 2H), 7.55 - 7.64 (m, 1H), 7.67 - 7.76 (m, 1H), 8.77 - 8.81 (m, 1H). |
| | (13% d. Th.) | |
| **405** | *ent*-N-(2-Amino-3-fluor-2-methylpropyl)-8-[(2,6-difluorbenzyl)oxy]-6-(fluormethyl)-2-methylimidazo[1,2-a]pyridin-3-carboxamid | LC-MS (Methode 2): Rₜ = 0.65 min |
| | | MS (ESpos): m/z = 439 (M+H)⁺ |
| | 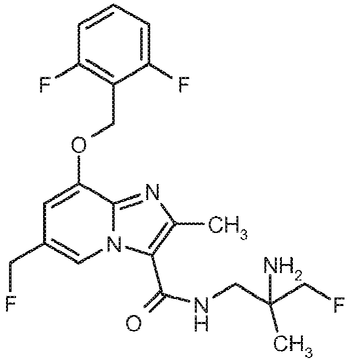 | ¹H-NMR (500 MHz, DMSO-d₆): 8 [ppm] = 1.01 - 1.07 (m, 3H), 1.70 (br. s, 2H), 2.56 (s, 3H), 3.30 - 3.40 (m, 2H), 4.10 - 4.16 (m, 1H), 4.20 - 4.26 (m, 1H), 5.33 (s, 2H), 5.47 (d_{H-F}, 2H), 7.11 (s, 1H), 7.21 - 7.28 (m, 2H), 7.55 - 7.64 (m, 1H), 7.74 - 7.81 (m, 1H), 8.76 - 8.80 (m, 1H). |
| | (36% d. Th.) | |

### Beispiel 406

### rac-N-[(4E/Z)-2-Aminohex-4-en-1-yl]-8-[(2,6-difluorbenzyl)oxy]-2,6-dimethylimidazo[1,2-a]pyridin-3-carboxamid

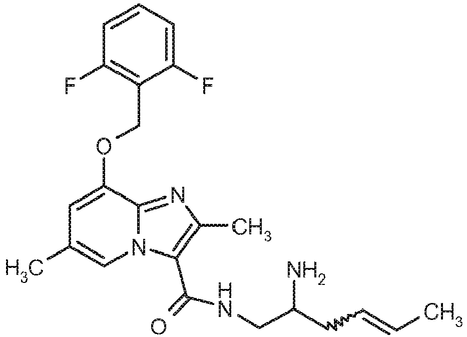

200 mg (0.60 mmol) 8-[(2,6-Difluorbenzyl)oxy]-2,6-dimethylimidazo[1,2-a]pyridin-3-carbonsäure aus Beispiel 21A wurden in 1.8 ml DMF vorgelegt, mit 252 mg (0.66 mmol) HATU und 467 mg (3.61 mmol) N,N-Diisopropylethylamin versetzt und 10 min bei Raumtemperatur gerührt. Das Reaktionsgemisch wurde auf 0°C abgekühlt. Langsam wurde eine Lösung von 130 mg (0.69 mmol) *rac*-(4*E*/*Z*)-Hex-4-en-1,2-diamindihydrochlorid und 194 mg (1.51 mmol) N,N-Diisopropylethylamin in 0.67 ml DMF zugetropft und das Gemisch wurde 1 h bei 0°C gerührt. Die Reaktionslösung wurde mit TFA/Wasser und Acetonitril verdünnt und mittels präparativer HPLC gereinigt (RP18 Säule, Laufmittel: Acetonitril/Wasser-Gradient unter Zusatz von 0.1% TFA). Der eingeengte Rückstand wurde in Dichlormethan aufgenommen und zweimal mit gesättigter, wässriger Natriumhydrogencarbonat-Lösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 114 mg der Titelverbindung (42% d. Th., Reinheit 94%) erhalten.
LC-MS (Methode 2): Rₜ = 0.65 min
MS (ESpos): m/z = 429 (M+H)⁺
¹H-NMR (500 MHz, DMSO-d₆): δ [ppm] = 1.56 - 1.67 (m, 3H), 1.94 - 2.08 (m, 1H), 2.09 - 2.23 (m, 1H), 2.31 (s, 3H), 2.50 (s, 3H), 2.83 - 2.94 (m, 1H), 3.07 - 3.18 (m, 1H), 3.29 - 3.41 (m, 1H), 5.29 (s, 2H), 5.42 - 5.58 (m, 2H), 6.91 (s, 1H), 7.19 - 7.27 (m, 2H), 7.54 - 7.63 (m, 1H), 7.68 - 7.78 (m, 1H), 8.47 (m, 1H).

### Beispiel 407

### rac-Ethyl-6-{1-amino-2-[({8-[(2,6-difluorbenzyl)oxy]-2,6-dimethylimidazo[1,2-a]pyridin-3-yl} carbonyl)amino]ethyl}pyridin-2-carboxylat

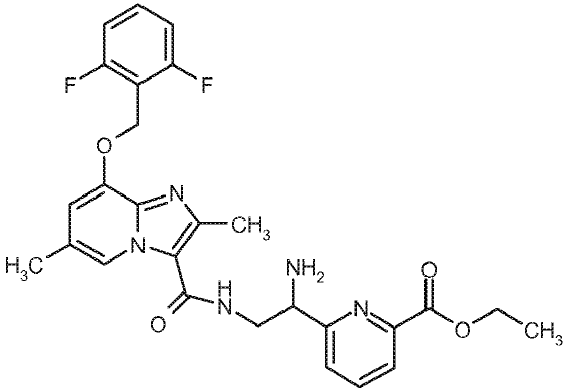

35 mg (0.11 mmol) 8-[(2,6-Difluorbenzyl)oxy]-2,6-dimethylimidazo[1,2-a]pyridin-3-carbonsäure aus Beispiel 21A wurden in 0.32 ml DMF vorgelegt, mit 45 mg (0.12 mmol) HATU und 55 mg (0.43 mmol) N,N-Diisopiopylethylamin versetzt und 10 min bei Raumtemperatur gerührt. Das Reaktionsgemisch wurde auf 0°C abgekühlt. Langsam wurde eine Lösung von 106 mg (0.13 mmol) *rac-* Ethyl-6-(1,2-diaminoethyl)pyridin-2-carboxylat-Dihydrochlorid (hergestellt aus Ethyl-6-{1-amino-2-[(tert-butoxycarbonyl)amino]ethyl}pyridin-2-carboxylat durch Behandlung mit 2 N Salzsäure in Diethylether und Eindampfen der Reaktionsmischung) und 50 mg (0.38 mmol) N,N-Diisopropylethylamin in 0.12 ml DMF zugetropft und das Gemisch wurde 1 h bei 0°C gerührt. Die Reaktionslösung wurde mit TFA/Wasser und Acetonitril verdünnt und mittels präparativer HPLC gereinigt (RP18 Säule, Laufmittel: Acetonitril/Wasser-Gradient unter Zusatz von 0.1% TFA). Die Produktfraktionen wurden eingeengt und der Rückstand wurde in Dichlormethan und wenig Methanol aufgenommen und zweimal mit gesättigter, wässriger Natriumhydrogencarbonat-Lösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Das Rohprodukt wurde nochmals mittels Dickschichtchromatographie gereinigt (Laufmittel: Dichlormethan/Methanol = 10/1). Es wurden 8.5 mg der Titelverbindung (9% d. Th., Reinheit 65%) erhalten.
LC-MS (Methode 2): Rₜ = 0.72 min
MS (ESpos): m/z = 524 (M+H)⁺

### Beispiel 408

### ent-N-(2-Amino-5,5,5-trifluor-2-methylpentyl)-8-[(2,6-difluorbenzyl)oxy]-2,6-dimethylimidazo[1,2-a]pyridin-3-carboxamid (Enantiomer A)

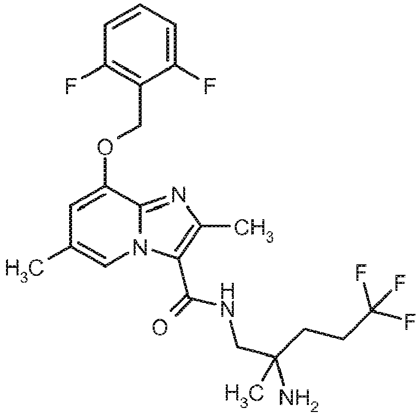

Eine Mischung von 1.02 g (1.56 mmol) *ent*-Benzyl-(1-[({8-[(2,6-difluorbenzyl)oxy]-2,6-dimethylimidazo[1,2-a]pyridin-3-yl}carbonyl)amino]-5,5,5-trifluor-2-methylpentan-2-yl)carbamat (Enantiomer A) aus Beispiel 410A und 332 mg 10%igem Palladium auf Aktivkohle in 40 ml Ethanol wurden 2 h bei Raumtemperatur und Normaldruck hydriert. Anschließend wurde über Celite abfiltriert, mit Ethanol gewaschen und das Filtrat eingeengt. Das Rohprodukt wurde mittels präparativer HPLC gereinigt (RP-C18, Laufmittel: Acetonitril/Wasser-Gradient unter Zusatz von 0.1% TFA). Die Produktfraktionen wurden in Dichlormethan aufgenommen und zweimal mit gesättigter, wässriger Natriumhydrogencarbonat-Lösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 601 mg der Titelverbindung (79% d. Th.) erhalten.
LC-MS (Methode 2): Rₜ = 0.73 min
MS (ESpos): m/z = 485 (M+H)⁺
¹H-NMR (500 MHz, DMSO-d₆): δ [ppm] = 1.02 (s, 3H), 1.48 -1.56 (m, 2H), 1.60 (br. s, 2H), 2.27 - 2.46 (m, 5H), 2.50 (s, 3H; überlagert vom Lösungsmittelpeak), 3.18 - 3.29 (m, 2H), 5.29 (s, 2H), 6.91 (s, 1H), 7.19 - 7.27 (m, 2H), 7.54 - 7.63 (m, 1H), 7.73 - 7.80 (m, 1H), 8.41 (s, 1H).
Enantiomer A: ca. 97% ee
Rₜ = 5.77 min [Daicel Chiralpak AZ-H, 5 µm, 250 x 4.6 mm; Eluent: 50% iso-Hexan, 50% Ethanol + 0.2% Diethylamin; Fluss 1.0 ml/min; Temperatur: 35°C; Detektion: 220 nm].

### Beispiel 409

### ent-N-(2-Amino-5,5,5-trifluor-2-methylpentyl)-8-[(2,6-difluorbenzyl)oxy]-2,6-dimethylimidazo[1,2-a]pyridin-3-carboxamid (Enantiomer B)

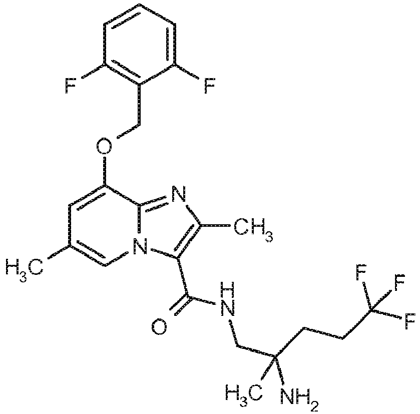

Eine Mischung von 965 mg (1.56 mmol) *ent*-Benzyl-{1-[({8-[(2,6-difluorbenzyl)oxy]-2,6-dimethylimidazo[1,2-a]pyridin-3-yl)carbonyl)amino]-5,5,5-trifluor-2-methylpentan-2-yl)carbamat (Enantiomer B) aus Beispiel 411A und 332 mg 10%igem Palladium auf Aktivkohle in 40 ml Ethanol wurden für 2 h bei Raumtemperatur und Normaldruck hydriert. Anschließend wurde über Celite abfiltriert, mit Ethanol gewaschen und das Filtrat eingeengt. Das Rohprodukt wurde mittels präparativer HPLC gereinigt (RP-C18, Laufmittel: Acetonitril/Wasser-Gradient unter Zusatz von 0.1% TFA). Die Produktfraktionen wurden in Dichlormethan aufgenommen und zweimal mit gesättigter, wässriger Natriumhydrogencarbonat-Lösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 586 mg der Titelverbindung (77% d. Th.) erhalten.
LC-MS (Methode 2): Rₜ = 0.72 min
MS (ESpos): m/z = 485 (M+H)⁺
¹H-NMR (500 MHz, DMSO-d₆): δ [ppm] = 1.02 (s, 3H), 1.48 - 1.56 (m, 2H), 1.59 (br. s, 2H), 2.27 - 2.46 (m, 5H), 2.50 (s, 3H; überlagert vom Lösungsmittelpeak), 3.18 - 3.29 (m, 2H), 5.29 (s, 2H), 6.91 (s, 1H), 7.19 - 7.27 (m, 2H), 7.54 - 7.63 (m, 1H), 7.73 - 7.80 (m, 1H), 8.41 (s, 1H).
Enantiomer B: ca. 78% ee
Rₜ = 5.02 min [Daicel Chiralpak AZ-H, 5 µm 250 x 4.6 mm; Eluent: 50% iso-Hexan, 50% Ethanol + 0.2% Diethylamin; Fluss 1.0 ml/min; Temperatur: 35°C; Detektion: 220 nm].

Die Titelverbindung wurde folgendermassen weiter aufgereinigt:
280 mg von Enantiomer B (ca. 78% ee) aus der oben beschriebenen Enantiomerentrennung wurden durch präparative Trennung an der chiralen Phase in die Enantiomere getrennt [Säule: Daicel Chiralpak IF, 5 µm, 250 x 20 mm, Eluent: 50% iso-Hexan, 50% Ethanol + 0.2% Diethylamin, Fluß: 50 ml/min, Temperatur: 23°C; Detektion: 220 nm]. Die Produktfraktionen wurden auf Trockeneis aufgefangen, eingedampft, mit Acetonitril/Wasser versetzt und lyophilisiert.
Enantiomer B: 158 mg (> 99% ee; Reinheit ca. 97%)
Rₜ = 5.05 min [Daicel Chiralpak AZ-H, 5 µm, 250 x 4.6 mm; Eluent: 50% iso-Hexan, 50% Ethanol + 0.2% Diethylamin; Fluss 1.0 ml/min; Temperatur: 35°C; Detektion: 220 nm].

### Beispiel 410

### ent-N-(2-Amino-5,5,5-trifluor-2-methylpentyl)-2,6-dimethyl-8-[(2,3,6-trifluorbenzyl)oxy]imidazo[1,2-a]pyridin-3-carboxamid (Enantiomer A)

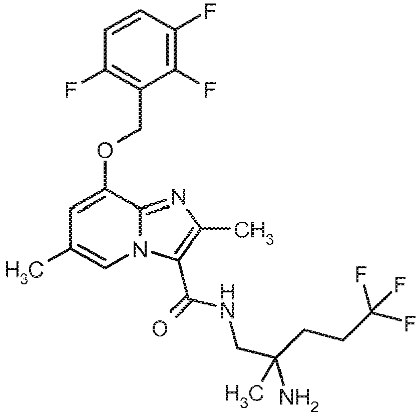

Eine Mischung von 550 mg (0.75 mmol, Reinheit 87%) *ent*-Benzyl-{1-[({2,6-dimethyl-8-[(2,3,6-trifluorbenzyl)oxy]imidazo[1,2-a]pyridin-3-yl}carbonyl)amino]-5,5,5-trifluor-2-methylpentan-2-yl}carbamat (Enantiomer A) aus Beispiel 412A und 160 mg 10%igem Palladium auf Aktivkohle in 19.4 ml Ethanol wurden für 2 h bei Raumtemperatur und Normaldruck hydriert. Anschließend wurde über Kieselgur abfiltriert, mit Ethanol gewaschen und das Filtrat eingeengt. Das Rohprodukt wurde mittels präparativer HPLC gereinigt (RP-C18, Laufmittel: Acetonitril/Wasser-Gradient unter Zusatz von 0.1% TFA). Die Produktfraktionen wurden in Dichlormethan aufgenommen und zweimal mit gesättigter, wässriger Natriumhydrogencarbonat-Lösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 262 mg der Titelverbindung (69% d. Th.) erhalten.
LC-MS (Methode 2): Rₜ = 0.74 min
MS (ESpos): m/z = 503 (M+H)⁺
¹H NMR (500 MHz, DMSO-d₆): 8 [ppm] = 1.03 (s, 3H), 1.49 -1.57 (m, 2H), 1.75 (br. s, 2H), 2.27 - 2.46 (m, 5H), 2.50 (s, 3H; überlagert vom Lösungsmittelpeak), 3.18 - 3.29 (m, 2H), 5.34 (s, 2H), 6.91 (s, 1H), 7.25 - 7.32 (m, 1H), 7.61 - 7.70 (m, 1H), 7.75 - 7.82 (m, 1H), 8.42 (s, 1H).
Enantiomer A: ca. 98% ee
Rₜ = 5.70 min [Daicel Chiralpak AZ-H, 5 µm, 250 x 4.6 mm; Eluent: 50% iso-Hexan, 50% Ethanol + 0.2% Diethylamin; Fluss 1.0 ml/min; Temperatur: 35°C; Detektion: 220 nm].

### Beispiel 411

### ent-N-(2-Amino-5,5,5-trifluor-2-methylpentyl)-2,6-dimethyl-8-[(2,3,6-trifluorbenzyl)oxy]imidazo[1,2-a]pyridin-3-carboxamid (Enantiomer B)

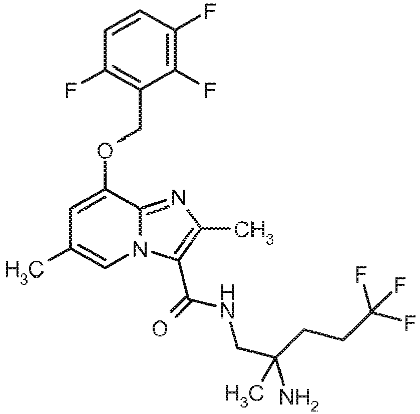

Eine Mischung von 535 mg (0.75 mmol) *ent*-Benzyl-{1-[({2,6-dimethyl-8-[(2,3,6-trifluorbenzyl)oxy]imidazo[1,2-a]pyridin-3-yl}carbonyl)amino]-5,5,5-trifluor-2-methylpentan-2-yl}carbamat (Enantiomer B) aus Beispiel 413A und 173 mg 10%igem Palladium auf Aktivkohle in 21 ml Ethanol wurden für 2 h bei Raumtemperatur und Normaldruck hydriert. Anschließend wurde über Kieselgur abfiltriert, mit Ethanol gewaschen und das Filtrat eingeengt. Das Rohprodukt wurde mittels präparativer HPLC gereinigt (RP-C18, Laufmittel: Acetonitril/Wasser-Gradient unter Zusatz von 0.1% TFA). Die Produktfraktionen wurden in Dichlormethan aufgenommen und zweimal mit gesättigter, wässriger Natriumhydrogencarbonat-Lösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 262 mg der Titelverbindung (68% d. Th.) erhalten.
LC-MS (Methode 2): Rₜ = 0.71 min
MS (ESpos): m/z = 503 (M+H)⁺
¹H-NMR (500 MHz, DMSO-d₆): δ [ppm] = 1.03 (s, 3H), 1.48 -1.56 (m, 2H), 1.60 (br. s, 2H), 2.27 - 2.46 (m, 5H), 2.50 (s, 3H; überlagert vom Lösungsmittelpeak), 3.18 - 3.29 (m, 2H), 5.34 (s, 2H), 6.91 (s, 1H), 7.25 - 7.32 (m, 1H), 7.61 - 7.70 (m, 1H), 7.74 - 7.81 (m, 1H), 8.42 (s, 1H).
Enantiomer B: ca. 78% ee
Rₜ = 4.90 min [Daicel Chiralpak AZ-H, 5 µm, 250 x 4.6 mm; Eluent: 50% iso-Hexan, 50% Ethanol + 0.2% Diethylamin; Fluss 1.0 ml/min; Temperatur: 35°C; Detektion: 220 nm].

Die Titelverbindung wurde folgendermassen weiter aufgereinigt:
140 mg von Enantiomer B (ca. 78% ee) aus der oben beschriebenen Enantiomerentrennung wurden durch präparative Trennung an der chiralen Phase in die Enantiomere getrennt [Säule: Daicel Chiralpak IF, 5 µm, 250 x 20 mm, Eluent: 50% iso-Hexan, 50% Ethanol + 0.2% Diethylamin, Fluß: 20 ml/min, Temperatur: 23°C; Detektion: 220 nm]. Die Produktfraktionen wurden auf Trockeneis aufgefangen, eingedampft, mit Acetonitril/Wasser versetzt und lyophilisiert.
Enantiomer B: 97 mg (> 99% ee; Reinheit ca. 98%)
Rt= 4.93 min [Daicel Chiralpak AZ-H, 5 µm, 250 x 4.6 mm; Eluent: 50% iso-Hexan, 50% Ethanol + 0.2% Diethylamin; Fluss 1.0 ml/min; Temperatur: 35°C; Detektion: 220 nm].

### Beispiel 412

### rac-N-[2-Amino-4-{methylsulfanyl)butyl]-8-[(2,6-difluorbenzyl)oxy]-2,6-dimethylimidazo[1,2-a]pyridin-3-carboxamid

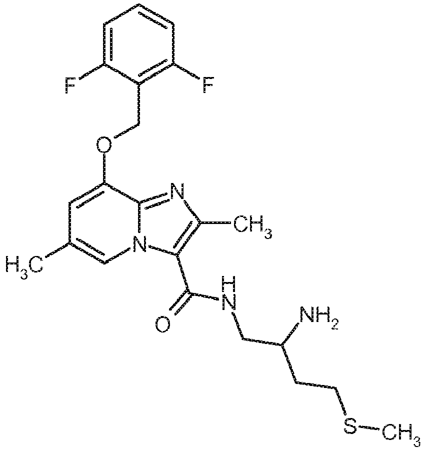

150 mg (0.45 mmol) 8-[(2,6-Difluorbenzyl)oxy]-2,6-dimethylimidazo[1,2-a]pyridin-3-carbonsäure aus Beispiel 21A, 189 mg (0.50 mmol) HATU und 175 mg (1.35 mmol) N,N-Diisopropylethylamin wurden in 2 ml DMF vorgelegt und 10 min bei RT gerührt. Anschließend wurde die Reaktionsgemisch zu einer Mischung von 112 mg (0.54 mmol) *rac-*4-(Methylsulfanyl)butan-1,2-diamindihydrochlorid und 175 mg (1.35 mmol) N,N-Diisopropylethylamin, gelöst in 0.26 ml DMF, bei 0°C getropft. Das Gemisch wurde 1 h bei 0°C und anschließend 30 min bei RT gerührt. Die Reaktionslösung wurde mit Wasser/TFA/Acetonitril versetzt und mittels präparativer HPLC gereinigt (RP-C18, Laufmittel: Acetonitril/Wasser-Gradient unter Zusatz von 0.1% TFA). Die Produktfraktionen wurden in Dichlormethan aufgenommen und zweimal mit gesättigter, wässriger Natriumhydrogencarbonat-Lösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 106 mg (51% d. Th.) der Titelverbindung erhalten.
LC-MS (Methode 2): Rₜ = 0.60 min
MS (ESpos): m/z = 449 (M+H)⁺
¹H-NMR (500 MHz, DMSO-d₆): δ = 1.42 -1.52 (m, 1H), 1.62 -1.93 (m, 3H), 2.07 (s, 3H), 2.30 (s, 3H), 2.49 - 2.68 (m, 5H; zum Teil überlagert mit Lösungsmittelpeak), 2.86 - 2.94 (m, 1H), 3.17 - 3.23 (m, 1H), 3.25 - 3.39 (m, 1H; überlagert mit Lösungsmittelpeak); 5.29 (s, 2H), 6.91 (s, 1H), 7.19 - 7.26 (m, 2H), 7.55 - 7.64 (m, 1H), 7.73 - 7.79 (m, 1H), 8.47 (s, 1H).

### Beispiel 413

### rac-N-[2-Amino-4-(methylsulfonyl)butyl]-8-[(2,6-difluorbenzyl)oxy]-2,6-dimethylimidazo[1,2-a]pyridin-3-carboxamid

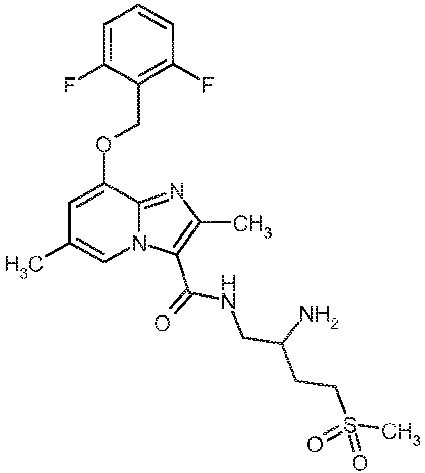

88 mg (0.20 mmol) *rac*-N-[2-Amino-4-(methylsulfanyl)butyl]-8-[(2,6-difluorbenzyl)oxy]-2,6-dimethylimidazo[1,2-a]pyridin-3-carboxamid aus Beispiel 412 wurden in 2 ml Dichlormethan vorgelegt, auf 0°C abgekühlt und bei dieser Temperatur portionsweise mit 3-Chlorbenzolcarboperoxosäure versetzt. Es wurde 30 min bei 0°C gerührt. Dann wurde mit Dichlormethan verdünnt und das Filtrat je einmal mit 1 N wässriger Natronlauge und Wasser gewaschen. Anschließend wurde über Natriumsulfat getrocknet, filtriert und im Vakuum eingeengt. Das Rohprodukt wurde mittels präparativer HPLC gereinigt (RP18 Säule, Laufmittel: Acetonitril/Wasser-Gradient unter Zusatz von 0.1% TFA). Die Produktfraktionen wurden eingeengt, der Rückstand in Dichlormethan aufgenommen und zweimal mit gesättigter, wässriger Natriumhydrogencarbonatlösung gewaschen. Die vereinten wässrigen Phasen wurden zweimal mit Dichlormethan extrahiert. Die vereinten organischen Phasen wurden über Natriumsulfat getrocknet, filtriert und einrotiert. Es wurden 53 mg (55% d. Th.; Reinheit 98%) der Titelverbindung erhalten.
LC-MS (Methode 2): Rₜ = 0.56 min
MS (ESpos): m/z = 481 (M+H)⁺
¹H-NMR (500 MHz, DMSO-d₆): δ = 1.57 -1.64 (m, 1H), 1.72 (br. s, 2H), 1.84 -1.93 (m, 1H), 2.30 (s, 3H), 2.50 (s, 3H; unter Lösungsmittelpeak), 2.87 - 3.00 (m, 4H), 3.14 - 3.32 (m, 4H; überlagert mit Lösungsmittelpeak), 5.29 (s, 2H), 6.91 (s, 1H), 7.19 - 7.26 (m, 2H), 7.55 - 7.64 (m, 1H), 7.81 (t, 1H), 8.47 (s, 1H).

### Beispiel 414

### rac-8-[(2,6-Difluorbenzyl)oxy]-N-[(1,1-dioxidothiomorpholin-3-yl)methyl]-2,6-dimethylimidazo[1,2-a]pyridin-3-carboxamid

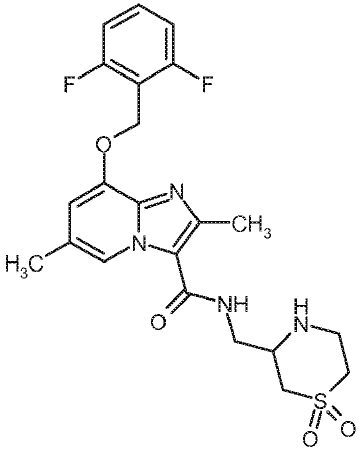

59 mg (0.09 mmol) *rac*-*tert*-Butyl-3-{[({8-[(2,6-difluorbenzyl)oxy]-2,6-dimethylimidazo[1,2-a]pyridin-3-yl}carbonyl)amino]methyl}thiomorpholin-4-carboxylat-1,1-dioxid Trifluoracetat aus Beispiel 418A wurden in 0.5 ml Diethylether gelöst und mit 1.28 ml (2.56 mmol) 2 N Salzsäure in Diethylether versetzt. Das Reaktionsgemisch wurde über Nacht bei Raumtemperatur gerührt. Dann wurde eingeengt und das Rohprodukt wurde mittels präparativer HPLC gereinigt (RP18 Säule, Laufmittel: Acetonitril/Wasser-Gradient unter Zusatz von 0.1% TFA). Die Produktfraktionen wurden eingeengt, der Rückstand in Dichlormethan und wenig Methanol aufgenommen und zweimal mit gesättigter, wässriger Natriumhydrogencarbonat-Lösung gewaschen. Die vereinten wässrigen Phasen wurden zweimal mit Dichlormethan extrahiert. Die vereinten organischen Phasen wurden über Natriumsulfat getrocknet, filtriert und eingeengt. Es wurden 30 mg der Zielverbindung (74% d. Th.) erhalten.
LC-MS (Methode 2): Rₜ = 0.62 min
MS (ESIpos): m/z = 479(M+H)⁺
¹H-NMR (500 MHz, DMSO-d₆): δ [ppm] = 2.31 (s, 3H), 2.47 - 2.54 (m, 4H; überlagert mit Lösungsmittelpeak), 2.76 - 2.83 (m, 1H), 2.85 - 3.05 (m, 3H), 3.08 - 3.23 (m, 2H), 3.30 - 3.47 (m, 3H; überlagert mit Lösungsmittelpeak), 5.29 (s, 2H), 6.92 (s, 1H), 7.18 - 7.25 (m, 2H), 7.54 - 7.63 (m, 1H), 7.80 (t, 1H), 8.48 (s, 1H).

### Beispiel 415

### rac-N-[2-Amino-2-(2-methyl-1,3-thiazol-4-yl)ethyl]-8-[(2,6difluorbenzyl)oxy]-2,6-dimethylimidazo[1,2-a]pyridin-3-carboxamid Triformiat

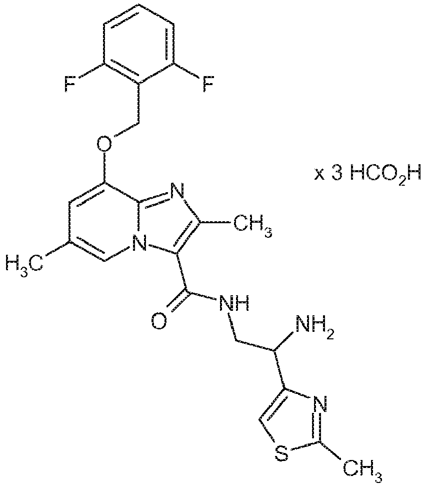

100 mg (0.30 mmol) 8-[(2,6-Difluorbenzyl)oxy]-2,6-dimethylimidazo[1,2-a]pyridin-3-carbonsäure aus Beispiel 21A, 137 mg (0.36 mmol) HATU und 121 mg (1.20 mmol) 4-Methylmorpholin wurden in 1.5 ml DMF vorgelegt und 60 min bei RT gerührt. Anschließend wurden 69 mg (0.30 mmol) *rac*-1-(2-Methyl-1,3-thiazol-4-yl)ethan-1,2-diamin-Dihydrochlorid zugegeben und über Nacht bei RT gerührt. Die Reaktionsmischung wurde mittels präparativer HPLC gereinigt (RP-C18, Laufmittel: Acetonitril/Wasser-Gradient unter Zusatz von 0.05% Ameisensäure). Es wurden 18 mg (10% d. Th.) der Titelverbindung erhalten.
LC-MS (Methode 2): Rₜ = 0.61 min
MS (ESIpos): m/z = 472 (M-3HCO₂H+H)⁺
¹H-NMR (400 MHz, DMSO-d₆): δ [ppm] = 2.35 (s, 3H), 2.40 (s, 3H), 2.70 (s, 3H), 3.71 - 3.88 (m, 2H), 4.60 - 4.68 (m, 1H), 5.32 (s, 2H), 7.13 (br.s, 1H), 7.21 - 7.28 (m, 2H), 7.56 - 7.65 (m, 1H), 7.65 (s, 1H), 8.06 (br.s, 1H), 8.44 - 8.55 (2 br. s, 4H).

### Beispiel 416

### rac-N-(2-Amino-1-cyanethyl)-8-[(2,6-difluorbenzyl)oxy]-2,6-dimethylimidazo[1,2-a]pyridin-3-carboxamid

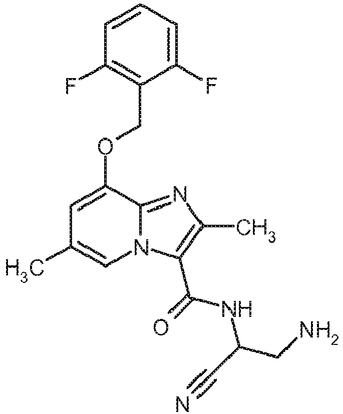

Eine Mischung aus 151 mg (0.23 mmol) *rac*-Benzyl-{2-cyan-2-[({8-[(2,6-difluorbenzyl)oxy]-2,6-dimethylimidazo[1,2-a]pyndin-3-yl}carbonyl)amino]ethyl}carbamat Trifluoracetat aus Beispiel 420A und 50 mg 10%igem Palladium auf Aktivkohle (0.05 mmol) in 6 ml Ethanol wurde für 2 h bei Raumtemperatur und Normaldruck hydriert. Das Reaktionsgemisch wurde über einen Filter abfiltriert, mit Ethanol gewaschen und das Filtrat eingeengt. Der Rückstand wurde in Dichlormethan aufgenommen und zweimal mit gesättigter, wässriger Natriumhydrogencarbonat-Lösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Das Rohprodukt wurde mittels Dickschichtchromatographie gereinigt (Laufmittel: Dichlormethan/Methanol = 10/1). Es wurden 4 mg der Titelverbindung (4% d. Th.) erhalten.
LC-MS (Methode 2): Rₜ = 0.62 min
MS (ESpos): m/z = 400 (M+H)⁺
¹H-NMR (500 MHz, DMSO-d₆): δ [ppm] = 2.33 (s, 3H), 2.93 - 3.10 (m, 2H), 4.82 - 4.82 (t, 1H), 5.30 (s, 2H), 6.98 (s, 1H), 7.20 - 7.26 (m, 2H), 7.51 - 7.66 (m, 1H), 8.47 (s, 1H), [weiteres Signal unter Lösungsmittelpeak].

### B. Bewertung der pharmakologischen Wirksamkeit

Es werden die folgenden Abkürzungen verwendet:
- ATP: Adenosintriphosphat
- Brij35: Polyoxyethylen(23)laurylether
- BSA: Rinderserumalbumin
- DTT: Dithiothreitol
- TEA: Triethanolamin

Die pharmakologische Wirkung der erfindungsgemäßen Verbindungen kann in folgenden Assays gezeigt werden:

### B-1. Vermessung von sGC Enzymaktivität mittels PPi Nachweis

Lösliche Guanylylcyclase (sGC) setzt unter Stimulation GTP zu cGMP und Pyrophosphat (PPi) um. PPi wird mit Hilfe des in WO 2008/061626 beschriebenen Verfahrens nachgewiesen. Das im Test entstehende Signal nimmt mit fortschreitender Umsetzung zu und dient als Maß für die sGC-Enzymaktivität. Mit Hilfe einer PPi Referenzkurve kann das Enzym in bekannter Weise charakterisiert werden, z.B. hinsichtlich Umsatzrate, Stimulierbarkeit oder Michaelis Konstante.

### Durchführung des Tests

Zur Durchführung des Tests wurden 29 µL Enzymlösung (0-10 nM lösliche Guanylylcyclase (hergestellt nach Hönicka et al., Journal of Molecular Medicine 77(1999)14-23), in 50 mM TEA, 2 mM Magnesiumchlorid, 0.1% BSA (FraktionV), 0.005% Brij 35, pH 7.5) in die Mikroplatte vorgelegt und 1 µL der Stimulatorlösung (0-10 µM 3-Morpholinosydnonimine, SIN-1, Merck in DMSO) hinzugegeben. Es wurde 10 min bei RT inkubiert. Anschließend wurden 20 µl Detektionsmix (1,2 nM Firefly Luciferase (*Photinus pyralis* Luziferase, Promega), 29 µM Dehydro-Luziferin (hergestellt nach Bitler & McElroy, Arch. Biochem. Biophys. 72 (1957) 358), 122 µM Luziferin (Promega), 153 µM ATP (Sigma) und 0,4 mM DTT (Sigma) in 50 mM TEA, 2 mM Magnesiumchlorid, 0.1% BSA (Fraktion V), 0.005% Brij 35, pH 7,5) zugegeben. Die Enzymreaktion wurde durch Zugabe von 20 µl Substratlösung (1.25 mM Guanosin-5'-triphosphat (Sigma) in 50 mM TEA, 2 mM Magnesiumchlorid, 0.1% BSA (Fraktion V), 0.005% Brij 35, pH 7.5) gestartet und kontinuierlich luminometrisch vermessen.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative MEC-Werte (MEC = minimal effektive Konzentration) für die erfindungsgemäßen Verbindungen sind in der nachstehenden Tabelle wiedergegeben (zum Teil als Mittelwerte aus Einzelbestimmungen):

**Tabelle A:**

| Beispiel | MEC [µM] | Beispiel | MEC [µM] |
|---|---|---|---|
| 1 | 0.10 | 219 | 0.30 |
| 2 | 0.65 | 220 | 0.10 |
| 3 | 0.30 | 221 | 0.30 |
| 4 | 0.30 | 222 | 0.30 |
| 5 | 1.00 | 223 | 0.30 |
| 6 | 0.23 | 224 | 0.30 |
| 7 | 1.00 | 225 | 0.30 |
| 8 | 0.10 | 226 | 0.10 |
| 9 | 0.30 | 227 | 0.30 |
| 10 | 0.30 | 228 | 1.00 |
| 11 | 0.30 | 229 | 1.00 |
| 12 | 1.00 | 230 | 1.00 |
| 13 | 0.23 | 231 | 0.30 |
| 14 | 1.00 | 232 | 0.10 |
| 15 | 0.53 | 233 | 0.10 |
| 16 | 0.30 | 234 | 0.03 |
| 17 | 1.00 | 235 | 0.30 |
| 18 | 0.55 | 236 | 0.30 |
| 19 | 1.66 | 237 | 0.30 |
| 20 | 1.00 | 238 | 1.00 |
| 21 | 0.30 | 239 | 0.30 |
| 22 | 0.30 | 240 | 0.30 |
| 23 | 0.30 | 241 | 0.30 |
| 24 | 0.65 | 242 | 0.30 |
| 25 | 1.00 | 243 | 1.00 |
| 26 | 0.30 | 244 | 0.30 |
| 27 | 1.00 | 245 | 0.10 |
| 28 | 0.30 | 246 | 0.30 |
| 29 | 0.20 | 247 | 3.00 |
| 30 | 0.30 | 248 | 1.00 |
| 31 | 0.30 | 249 | 1.00 |
| 32 | 0.30 | 250 | 1.00 |
| 33 | 0.30 | 251 | 3.00 |
| 34 | 1.00 | 252 | 0.10 |
| 35 | 1.00 | 253 | 0.10 |
| 36 | 1.00 | 254 | 0.30 |
| 37 | 1.00 | 255 | 0.30 |
| 38 | 0.40 | 256 | 3.00 |
| 39 | 0.10 | 257 | 1.00 |
| 40 | 0.10 | 258 | 1.00 |
| 41 | 0.30 | 259 | 0.03 |
| 42 | 1.00 | 260 | 0.10 |
| 43 | 1.00 | 261 | 0.10 |
| 44 | 0.30 | 262 | 0.10 |
| 45 | 1.00 | 263 | 1.00 |
| 46 | 1.00 | 264 | 0.03 |
| 47 | 0.30 | 265 | 0.30 |
| 48 | 1.00 | 266 | 0.03 |
| 49 | 0.30 | 267 | 0.10 |
| 50 | 0.03 | 268 | 0.55 |
| 51 | 0.20 | 269 | 0.65 |
| 52 | 0.03 | 270 | 1.00 |
| 53 | 0.30 | 271 | 0.30 |
| 54 | 1.00 | 272 | 5.5 |
| 55 | 2.00 | 273 | 1.00 |
| 56 | 1.00 | 274 | 0.10 |
| 57 | 0.30 | 275 | 0.10 |
| 58 | 1.00 | 276 | 0.10 |
| 59 | 3.00 | 277 | 1.00 |
| 60 | 0.30 | 278 | 0.3 |
| 61 | 3.00 | 279 | 0.3 |
| 62 | 1.00 | 280 | 0.65 |
| 63 | 0.30 | 281 | 0.3 |
| 64 | 0.30 | 282 | 0.3 |
| 65 | 1.00 | 283 | 0.3 |
| 66 | 1.00 | 284 | 0.1 |
| 67 | 0.30 | 285 | 0.3 |
| 68 | 0.30 | 286 | 0.1 |
| 69 | 1.00 | 287 | 0.3 |
| 70 | 1.00 | 288 | 0.3 |
| 71 | 0.30 | 289 | 0.3 |
| 72 | 0.30 | 290 | 0.3 |
| 73 | 0.30 | 291 | 1.0 |
| 74 | 0.20 | 292 | 0.03 |
| 75 | 1.00 | 293 | 0.03 |
| 76 | 0.30 | 294 | 0.065 |
| 77 | 1.00 | 295 | 0.10 |
| 78 | 1.00 | 296 | 0.10 |
| 79 | 1.00 | 297 | 0.10 |
| 80 | 1.00 | 298 | 0.10 |
| 81 | 1.00 | 299 | 0.10 |
| 82 | 0.20 | 300 | 0.10 |
| 83 | 0.10 | 301 | 0.10 |
| 84 | 1.00 | 302 | 0.10 |
| 85 | 1.00 | 303 | 0.20 |
| 86 | 2.00 | 304 | 0.10 |
| 87 | 1.00 | 305 | 0.10 |
| 88 | 1.00 | 306 | 0.10 |
| 89 | 0.65 | 307 | 0.65 |
| 90 | 0.10 | 308 | 0.20 |
| 91 | 1.00 | 309 | 0.30 |
| 92 | 0.30 | 310 | 0.30 |
| 93 | 0.30 | 311 | 0.30 |
| 94 | 0.30 | 312 | 0.30 |
| 95 | 1.00 | 313 | 0.30 |
| 96 | 1.00 | 314 | 0.30 |
| 97 | 0.10 | 315 | 0.30 |
| 98 | 1.00 | 316 | 0.30 |
| 99 | 1.00 | 317 | 0.30 |
| 100 | 0.30 | 318 | 0.30 |
| 101 | 1.00 | 319 | 0.30 |
| 102 | 1.00 | 320 | 0.30 |
| 103 | 3.00 | 321 | 0.30 |
| 104 | 0.30 | 322 | 0.30 |
| 105 | 0.30 | 323 | 0.30 |
| 106 | 0.03 | 324 | 1.00 |
| 107 | 0.30 | 325 | 0.30 |
| 108 | 1.00 | 326 | 0.30 |
| 109 | 1.00 | 327 | 1.00 |
| 110 | 0.30 | 328 | 3.00 |
| 111 | 1.00 | 329 | 0.30 |
| 112 | 0.10 | 330 | 0.30 |
| 113 | 0.65 | 331 | 0.30 |
| 114 | 0.30 | 332 | 1.00 |
| 115 | 1.00 | 333 | 1.00 |
| 116 | 3.00 | 334 | 1.00 |
| 117 | 1.00 | 335 | 0.30 |
| 118 | 1.00 | 336 | 0.30 |
| 119 | 0.10 | 337 | 0.55 |
| 120 | 0.30 | 338 | 0.10 |
| 121 | 0.30 | 340 | 1.00 |
| 122 | 1.00 | 341 | 0.30 |
| 123 | 3.00 | 342 | 1.00 |
| 124 | 1.00 | 343 | 1.00 |
| 125 | 1.00 | 344 | 1.00 |
| 126 | 0.30 | 345 | 1.00 |
| 127 | 1.00 | 346 | 1.00 |
| 128 | 0.10 | 347 | 1.00 |
| 129 | 0.10 | 348 | 1.00 |
| 130 | 1.00 | 349 | 1.00 |
| 131 | 3.00 | 350 | 1.00 |
| 132 | 1.00 | 351 | 3.00 |
| 133 | 3.00 | 352 | 3.00 |
| 134 | 3.00 | 353 | 3.00 |
| 135 | 1.00 | 354 | 3.00 |
| 136 | 0.10 | 355 | 0.30 |
| 137 | 1.00 | 356 | 0.10 |
| 138 | 0.30 | 357 | 0.03 |
| 139 | 1.00 | 358 | 0.30 |
| 140 | 1.00 | 359 | 3.00 |
| 141 | 3.00 | 360 | 1.00 |
| 142 | 1.00 | 361 | 1.00 |
| 143 | 0.30 | 362 | 1.00 |
| 144 | 3.00 | 363 | 3.00 |
| 145 | 3.00 | 364 | 0.30 |
| 146 | 1.00 | 365 | 1.00 |
| 147 | 0.30 | 366 | 0.10 |
| 148 | 1.00 | 367 | 3.00 |
| 149 | 1.00 | 368 | 0.10 |
| 150 | 0.10 | 369 | 0.30 |
| 151 | 0.10 | 370 | 3.00 |
| 152 | 1.00 | 371 | 3.00 |
| 153 | 1.00 | 372 | 3.00 |
| 154 | 1.00 | 373 | 3.00 |
| 155 | 0.30 | 374 | 1.00 |
| 156 | 3.00 | 375 | 3.00 |
| 157 | 0.30 | 376 | 3.00 |
| 158 | 0.30 | 377 | 3.00 |
| 159 | 0.30 | 378 | 1.00 |
| 160 | 1.00 | 379 | 1.00 |
| 161 | 0.10 | 380 | 1.00 |
| 162 | 3.00 | 381 | 0.30 |
| 163 | 0.30 | 382 | 0.30 |
| 164 | 0.10 | 383 | 0.30 |
| 165 | 0.10 | 384 | 0.30 |
| 166 | 0.30 | 385 | 1.00 |
| 167 | 1.00 | 386 | 0.65 |
| 168 | 1.00 | 387 | 10.0 |
| 169 | 1.65 | 388 | 0.30 |
| 170 | 3.00 | 389 | 1.00 |
| 171 | 1.00 | 390 | 0.30 |
| 172 | 0.30 | 391 | 0.10 |
| 173 | 0.30 | 392 | 1.00 |
| 174 | 0.30 | 393 | 1.00 |
| 175 | 1.00 | 394 | 0.30 |
| 176 | 1.00 | 395 | 1.00 |
| 177 | 1.00 | 396 | 3.00 |
| 178 | 0.30 | 397 | 1.00 |
| 179 | 1.00 | 398 | 0.30 |
| 180 | 1.00 | 399 | 1.00 |
| 181 | 1.00 | 400 | 0.65 |
| 182 | 3.00 | 401 | 1.00 |
| 183 | 1.00 | 402 | 0.30 |
| 184 | 1.00 | 403 | 0.10 |
| 185 | 1.00 | 404 | 0.30 |
| 186 | 0.30 | 405 | 0.30 |
| 187 | 0.30 | 406 | 0.10 |
| 188 | 1.00 | 408 | 0.10 |
| 189 | 3.00 | 409 | 0.03 |
| 190 | 1.00 | 410 | 0.10 |
| 191 | 1.00 | 411 | 0.03 |
| 192 | 0.30 | 412 | 0.10 |
| 193 | 1.00 | 413 | 5.5 |
| 194 | 0.10 | 414 | 0.30 |
| 195 | 0.30 | 415 | 5.5 |
| 196 | 1.00 | 416 | 1.00 |
| 197 | 1.00 | | |
| 198 | 0.30 | | |
| 199 | 0.30 | | |
| 200 | 0.04 | | |
| 201 | 0.10 | | |
| 202 | 0.10 | | |
| 203 | 0.10 | | |
| 204 | 1.00 | | |
| 205 | 0.30 | | |
| 206 | 0.30 | | |
| 207 | 1.00 | | |
| 208 | 0.30 | | |
| 209 | 0.30 | | |
| 210 | 0.30 | | |
| 211 | 0.30 | | |
| 212 | 1.00 | | |
| 213 | 1.00 | | |
| 214 | 0.30 | | |
| 215 | 1.00 | | |
| 216 | 1.00 | | |
| 217 | 1.00 | | |
| 218 | 0.10 | | |

### B-3. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1 %.

### B-4. Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht (Stasch et al. Br. J. Pharmacol. 2002; 135: 344-355).

### B-5. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
Implantierbare Sender (Physiotel® Telemetrietransmitter)
Empfänger (Physiotel® Receiver), die über einen Multiplexer (DSI Data Exchange Matrix) mit einem
Datenakquisitionscomputer verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5% iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen
Systolischer Blutdruck (SBP)
Diastolischer Blutdruck (DBP)
Arterieller Mitteldruck (MAP)
Herzfrequenz (HR)
Aktivität (ACT)

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor, APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur:

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787,1994.

### B-6. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wistar-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{½} (terminale Halbwertszeit), F (Bioverfügbarkeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

Tabelle B zeigt Daten repräsentativer Verbindungen der vorliegenden Erfindung nach intravenöser Gabe in Ratten:

**Tabelle B:**

| Beispiel | AUCₙₒᵣₘ [kg·h/L] | CL_{Blut} [L/h/kg] | t_{1/2} [h] | MRT [h] |
|---|---|---|---|---|
| 4 | 0.58 | 1.67 | 5.2 | 6.7 |
| 8 | 1.09 | 0.92 | 4.5 | 6.6 |
| 10 | 2.26 | 0.74 | 4.8 | 6.9 |
| 13 | 1.71 | 0.83 | 3.5 | 4.4 |
| 73 | 1.55 | 0.51 | 8.6 | 11.8 |
| 74 | 0.81 | 1.23 | 3.7 | 5.0 |
| 119 | 1.06 | 0.86 | 4.9 | 7.4 |
| 155 | 0.97 | 1.27 | 7.7 | 11.2 |
| 173 | 0.54 | 1.68 | 3.3 | 3.9 |
| 194 | 2.06 | 0.48 | 16.8 | 24.4 |
| 200 | 0.69 | 0.92 | 5.9 | 8.5 |
| 211 | 0.78 | 1.07 | 4.8 | 6.6 |
| 223 | 0.87 | 1.09 | 4.8 | 6.1 |
| 261 | 0.47 | 2.29 | 3.7 | 4.7 |
| 274 | 1.29 | 0.69 | 5.8 | 8.4 |
| 275 | 0.64 | 1.25 | 4.1 | 7.2 |
| 276 | 0.70 | 1.27 | 2.9 | 4.5 |
| 279 | 0.42 | 2.76 | 2.3 | 2.9 |
| 281 | 0.28 | 2.34 | 5.5 | 6.9 |
| 282 | 0.73 | 1.41 | 4.5 | 5.7 |
| 283 | 0.67 | 0.87 | 3.1 | 3.9 |
| 284 | 1.20 | 0.97 | 4.6 | 5.3 |
| 285 | 0.54 | 1.02 | 3.6 | 4.4 |
| 286 | 0.82 | 1.39 | 3.8 | 4.5 |
| 287 | 0.66 | 1.76 | 5.4 | 8.4 |
| 289 | 0.38 | 2.51 | 4.4 | 6.8 |
| 291 | 0.87 | 0.84 | 4.8 | 5.7 |
| 292 | 0.56 | 2.17 | 3.2 | 4.1 |
| 295 | 0.56 | 1.23 | 6.6 | 8.8 |
| 303 | 0.26 | 2.74 | 5.7 | 8.2 |
| 304 | 0.34 | 2.27 | 6.2 | 9.4 |
| 307 | 0.35 | 1.78 | 6.9 | 9.8 |
| 356 | 0.61 | 0.98 | 10.3 | 13.9 |
| 408 | 0.19 | 3.07 | 3.8 | 4.7 |
| 409 | 0.28 | 2.15 | 5.8 | 7.4 |
| 410 | 0.21 | 2.77 | 4.3 | 5.4 |
| 411 | 0.41 | 1.52 | 8.2 | 11.2 |

### B-7. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationeansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-8. Caco-2 Permeabilitäts-Test

Die Permeabilität einer Testsubstanz wurde mit Hilfe der Caco-2 Zelllinie, einem etablierten *in vitro* Modell für Permeabilitätsvorhersagen an der gastrointestinalen Barriere, bestimmt (Artursson, P. and Karlsson, J. (1991). Correlation between oral drug absorption in humans and apparent drug permeability coefficients in human intestinal epithelial (Caco-2) cells. Biochem. Biophys.175 (3), 880-885). Die Caco-2 Zellen (ACC No. 169, DSMZ, Deutsche Sammlung von Mikroorganismen und Zellkulturen, Braunschweig, Deutschland) wurden in 24-Well Platen mit Einsatz ausgesät und 14 bis 16 Tage kultiviert. Für die Permeabilitätsstudien wurde die Testsubstanz in DMSO gelöst und mit Transportpuffer (Hanks Buffered Salt Solution, Gibco/Invitrogen, mit 19.9 mM Glukose und 9.8 mM HEPES) auf die finale Testkonzentration verdünnt. Um die Permeabilität von apikal nach basolateral (PₐₚₚA-B) der Testsubstanz zu bestimmen, wurde die Lösung mit der Testsubstanz auf die apikale Seite des Caco-2 Zellmonolayers gegeben und Transportpuffer auf die basolaterale Seite. Um die Permeabilität von basolateral nach apikal (PₐₚₚB-A) der Testsubstanz zu bestimmen, wurde die Lösung mit der Testsubstanz auf die basolaterale Seite des Caco-2 Zellmonolayers gegeben und Transportpuffer auf die apikale Seite. Zu Beginn des Experiments wurden Proben aus dem jeweiligen Donor-Kompartiment genommen, um die Massenbilanz sicher zu stellen. Nach einer Inkubation von zwei Stunden bei 37° C wurden Proben aus beiden Kompartimenten genommen. Die Proben wurden mittels LC-MS/MS analysiert und die apparenten Permeabilitätskoeffizienten (Pₐₚₚ) berechnet. Die Permeabilität von Lucifer Yellow wurde für jeden Zellmonolayer bestimmt, um die Integrität der Zellschicht sicher zu stellen. Die Permeabilität von Atenolol (Marker für niedrige Permeabilität) und Sulfasalazin (Marker für aktive Exkretion) wurde in jedem Testlauf als Qualitätskontrolle mitbestimmt.

### B-9. hERG Kaliumstrom Assay.

Der sogenannte hERG (human ether-a-go-go related gene) Kaliumstrom trägt wesentlich zur Repolarisierung des humanen kardialen Aktionspotentials bei (Scheel et al., 2011). Eine Inhibition dieses Stroms durch Pharmaka kann in seltenen Fällen potentiell letale Herzrhythmusstörungen zur Folge haben, und wird deshalb frühzeitig während der Arzneimittelentwicklung untersucht.

Der hier verwendete funktionelle hERG Assay basiert auf einer recombinanten HEK293 ZellLinie, die das KCNH2(HERG)-Gen stabil exprimiert (Zhou et al., 1998). Diese Zellen werden mittels der "whole-cell voltage-clamp" Technik (Hamill et al., 1981) in einem automatisierten System (Patchliner™; Nanion, München, D) untersucht, welches die Membranspannung kontrolliert und den hERG Kalium-Strom bei Zimmertemperatur misst. Die PatchControlHT™ Software (Nanion) steuert Patchliner System, Datenerfassung und Datenanalyse. Die Spannungskontrolle erfolgt durch 2 EPC-10 quadro Verstärker unter Kontrolle der PatchMasterPro™ Software (beide: HEKA Elektronik, Lambrecht, D). NPC-16 Chips mit mittlerem Widerstand (∼2 MΩ; Nanion) dienen als planares Substrat für die Voltage-Clamp Experimente.

NPC-16 Chips werden mit intra- und extrazellulärer Lösung (vgl. Himmel, 2007) sowie mit Zellsuspension befüllt. Nach Bildung eines Giga-Ohm-Seals und Herstellen des Ganzzell-Modus (einschliesslich mehrerer automatisierter Qualitätskontrollschritte) wird die Zellmembran auf das Haltepotential -80 mV geklemmt. Das nachfolgende Spannungsklemm-Protokoll ändert die Kommandospannung auf +20 mV (Dauer 1000 ms), -120 mV (Dauer 500 ms), und zurück zum Haltepotential -80 mV; dies wird alle 12 s wiederholt. Nach einer initialen Stabilisierungsphase (ca 5-6 Minuten) wird Testsubstanzlösung in aufsteigenden Konzentrationen (z.B. 0.1, 1, und 10 µmol/L) zupipettiert (Exposition ca 5-6 Minuten pro Konzentration), gefolgt von mehreren Auswaschschritten.

Die Amplitude des einwärtsgerichteten "Tail"-Stroms, der durch eine Potentialänderung von +20 mV auf -120 mV erzeugt wird, dient zur Quantifizierung des hERG Kaliumstroms, und wird als Funktion der Zeit dargestellt (IgorPro™ Software). Die Stromamplitude am Ende verschiedener Zeitabschnitte (z.B. Stabilisierungsphase vor Testsubstanz, erste/zweite/dritte Konzentration Testsubstanz) dient zur Erstellung einer Konzentrations-Wirkungs-Kurve, aus der die halbmaximale Hemmkonzentration IC₅₀ der Testsubstanz errechnet wird.
Hamill OP, Marty A, Neher E, Sakmann B, Sigworth FJ. Improved patch-clamp techniques for high-resolution current recording from cells and cell-free membrane patches. Pfluegers Arch 1981; 391:85-100.
Himmel HM. Suitability of commonly used excipients for electrophysiological in-vitro safety pharmacology assessment of effects on hERG potassium current and on rabbit Purkinje fiber action potential. J Pharmacol Toxicol Methods 2007;56:145-158.
Scheel O, Himmel H, Rascher-Eggstein G, Knott T. Introduction of a modular automated voltage-clamp platform and its correlation with manual human ether-a-go-go related gene voltage-clamp data. Assay Drug Dev Technol 2011;9:600-607.
Zhou ZF, Gong Q, Ye B, Fan Z, Makielski JC, Robertson GA, January CT. Properties of hERG channels stably expressed in HEK293 cells studied at physiological temperature. Biophys J 1998;74:230-241.

### C. Ausführunesbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die erhaltene Lösung wird steril filtriert und in sterile und pyrogenfreie Injektionsbehältnisse abgefüllt.

## Patentansprüche

1. Verbindung der Formel (I) 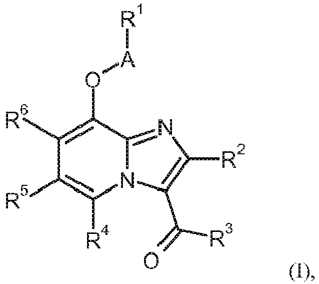 in welcher
A für CH₂, CD₂ oder CH(CH₃) steht,
R¹ für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₃-C₆)-Cycloalkyl, (C₁-C₄)-Alkoxy, Difluormethoxy und Trifluormethoxy substituiert sein kann,
R² für Wasserstoff, (C₁-C₄)-Alkyl, Cyclopropyl, Monofluormethyl, Difluormethyl oder Trifluormethyl steht,
R³ für eine Gruppe der Formel 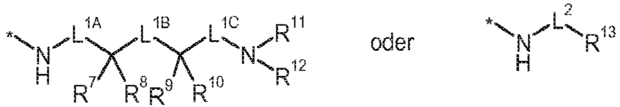 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy, substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃ , (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin das Phenyl an 2 benachbarten Kohlenstoffatomen mit einer Difluormethylendioxy-Brücke substituiert sein kann,
R⁸ für Wasserstoff oder (C₁-C₄)-Alkyl steht,
oder
R⁷ und R⁸ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für Wasserstoff, Cyano, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, Cyano, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5- oder 6-gliedriges Heteroaryl substituiert sein kann,
worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R¹⁰ für Wasserstoff oder (C₁-C₄)-Alkyl steht,
oder
R⁹ und R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können,
mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen 3- bis 7-gliedrigen Carbocyclus oder einen 4- bis 7-gliedrigen Heterocyclus bilden,
worin der 3- bis 7-gliedrigen Carbocyclus mit 1 oder 2 Substituenten unabhängig voneinander ausgewählt aus der Gruppe (C₁-C₄)-Alkyl, Fluor, Hydroxy und (C₁-C₄)-Alkoxy substituiert sein kann,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
R¹¹ für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
R¹² für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können, oder
R¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann, und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kein, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,
R⁴ für Wasserstoff steht,
R⁵ für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkenyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

2. Verbindung der Formel (I) nach Anspruch 1, in welcher
A für CH₂, CD₂ oder CH(CH₃) steht,
R¹ Phenyl steht,
wobei Phenyl mit 1 bis 3 Substituenten unabhängig voneinander ausgewählt aus der Gruppe Halogen, Cyano, Monofluormethyl, Difluormethyl, Trifluormethyl, (C₁-C₄)-Alkyl substituiert sein kann,
und
wobei Phenyl mit 1 bis 2 Substituenten ausgewählt aus der Gruppe (C₃-C₆)-Cycloalkyl, (C₁-C₄)-Alkoxy, Difluormethoxy oder Trifluormethoxy substituiert ist,
R² für Wasserstoff, (C₁-C₄)-Alkyl, Cyclopropyl, Monofluormethyl, Difluormethyl oder Trifluormethyl steht,
R³ für eine Gruppe der Formel 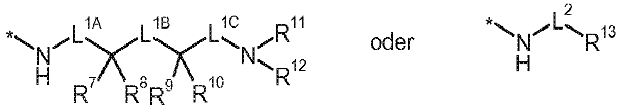 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy, substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃ , (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R⁸ für Wasserstoff oder (C₁-C₄)-Alkyl steht,
oder
R⁷ und R⁸ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für Wasserstoff, Cyano, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, Cyano, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5- oder 6-gliedriges Heteroaryl substituiert sein kann,
worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R¹⁰ für Wasserstoff oder (C₁-C₄)-Alkyl steht,
oder
R⁹ und R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können,
mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen 3- bis 7-gliedrigen Carbocyclus oder einen 4- bis 7-gliedrigen Heterocyclus bilden,
worin der 3- bis 7-gliedrigen Carbocyclus mit 1 oder 2 Substituenten unabhängig voneinander ausgewählt aus der Gruppe (C₁-C₄)-Alkyl, Fluor, Hydroxy und (C₁-C₄)-Alkoxy substituiert sein kann,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
R¹¹ für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
R¹² für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann,
und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kein, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,
R⁴ für Wasserstoff steht,
R⁵ für Wasserstoff, Halogen, Cyano, Monofluormethyl, Difluormethyl, Trifluormethyl, (C₁-C₄)-Alkyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

3. Verbindung der Formel (I) nach Anspruch 1, in welcher
A für CH₂, CD₂ oder CH(CH₃) steht,
R¹ für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, (C₃-C₆)-Cycloalkyl, Difluormethoxy und Trifluormethoxy substituiert sein kann,
R² für Wasserstoff, (C₁-C₄)-Alkyl, Cyclopropyl, Monofluormethyl, Difluormethyl oder Trifluormethyl steht,
R³ für eine Gruppe der Formel 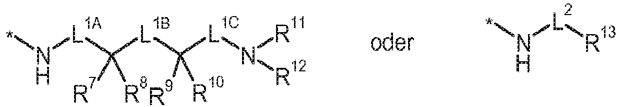 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy, substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃, (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R⁸ für Wasserstoff oder (C₁-C₄)-Alkyl steht,
oder
R⁷ und R⁸ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für Wasserstoff, Cyano, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, Cyano, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5- oder 6-gliedriges Heteroaryl substituiert sein kann,
worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R¹⁰ für Wasserstoff oder (C₁-C₄)-Alkyl steht,
oder
R⁹ und R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können,
mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen 3- bis 7-gliedrigen Carbocyclus oder einen 4- bis 7-gliedrigen Heterocyclus bilden,
worin der 3- bis 7-gliedrigen Carbocyclus mit 1 oder 2 Substituenten unabhängig voneinander ausgewählt aus der Gruppe (C₁-C₄)-Alkyl, Fluor, Hydroxy und (C₁-C₄)-Alkoxy substituiert sein kann,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
R¹¹ für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
R¹² für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann,
und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kann, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,
R⁴ für Wasserstoff steht,
R⁵ für Monofluormethyl, Difluormethyl, Trifluormethyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

4. Verbindung der Formel (I) nach Anspruch 1 in welcher
A für CH₂, CD₂ oder CH(CH₃) steht,
R¹ für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, (C₃-C₆)-Cycloalkyl, Difluormethoxy und Trifluormethoxy substituiert sein kann,
R² für Wasserstoff, (C₁-C₄)-Alkyl, Cyclopropyl, Monofluormethyl, Difluormethyl oder Trifluormethyl steht,
R³ für eine Gruppe der Formel 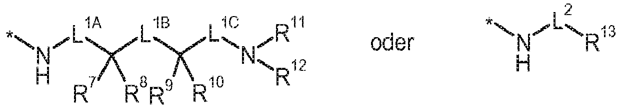 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für (C₁-C₆)-Alkyl, (C₂-C₆)-Alkinyl, Cyano oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy und Phenoxy substituiert ist,
worin Phenoxy mit 1 bis 3 Substituenten Halogen substituiert sein kann,
worin Phenyl mit 1 bis 2 Substituenten unabhängig voneinander ausgewählt aus der Gruppe Cyano, Nitro, Difluormethoxy, Trifluormethoxy, (C₁-C₄)-Alkoxy, -NH(CO)CH₃ und (C₁-C₄)-Alkenyl substituiert ist,
wobei (C₁-C₄)-Alkoxy mit Hydroxy substituiert ist,
R⁸ für Wasserstoff oder (C₁-C₄)-Alkyl steht,
R⁹ für Wasserstoff, Cyano, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, Cyano, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5- oder 6-gliedriges Heteroaryl substituiert sein kann,
worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycaibonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R¹⁰ für Wasserstoff oder (C₁-C₄)-Alkyl steht,
oder
R⁹ und R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können,
mit der Maßgabe, dass die Reste R⁷ und R⁹ nicht beide gleichzeitig für Phenyl stehen,
R¹¹ für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
R¹² für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann,
und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kann, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,
R⁴ für Wasserstoff steht,
R⁵ für Wasserstoff, Halogen, Cyano, Monofluormethyl, Difluormethyl, Trifluormethyl, (C₁-C₄)-Alkyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

5. Verbindung der Formel (I) nach Anspruch 1, in welcher
A für CH₂, CD₂ oder CH(CH₃) steht,
R¹ für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, (C₃-C₆)-Cycloalkyl, Difluormethoxy und Trifluormethoxy substituiert sein kann,
R² für Wasserstoff, (C₁-C₄)-Alkyl, Cyclopropyl, Monofluormethyl, Difluormethyl oder Trifluormethyl steht,
R³ für eine Gruppe der Formel 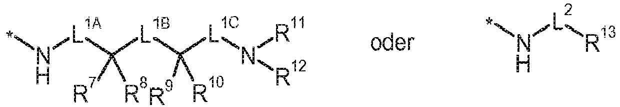 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁₋C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₁-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy, substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃, (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R⁸ für Wasserstoff oder (C₁-C₄)-Alkyl steht,
oder
R⁷ und R⁸ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für (C₁-C₆)-Alkyl, Cyano oder Phenyl steht,
worin (C₁-C₆)-Alkyl mit 1 bis 3 Substituenten unabhängig voneinander ausgewählt aus der Gruppe Cyano, Fluor, Difluormethyl, Trifluormethyl, Difluormethoxy, Trifluormethoxy, 5- oder 6-gliedriges Heteroaryl, Phenoxy und Benzyloxy substituiert ist,
worin Phenoxy mit 1 bis 3 Substituenten Halogen substituiert ist,
worin Benzyloxy mit 1 bis 3 Substituenten Halogen substituiert sein kann,
worin 5- oder 6-gliedriges Heteroaryl mit einem 5- oder 6-gliedriges Heteroaryl, substituiert ist,
worin 5- oder 6-gliedriges Heteroaryl seinerseits mit (C₁-C₄)-Alkyl substituiert sein kann,
worin Phenyl mit 1 bis 2 Substituenten unabhängig voneinander ausgewählt aus der Gruppe Cyano, Difluormethoxy, Trifluormethoxy und (C₁-C₄)-Alkoxy substituiert ist,
wobei (C₁-C₄)-Alkoxy mit Hydroxy substituiert ist,
R¹⁰ für Wasserstoff oder (C₁-C₄)-Alkyl steht,
R¹¹ für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
R¹² für Wasserstoff, (C₁₋C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann,
und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kann, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,
R⁴ für Wasserstoff steht,
R⁵ für Wasserstoff, Halogen, Cyano, Monofluormethyl, Difluormethyl, Trifluormethyl, (C₁-C₄)-Alkyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

6. Verbindung der Formel (I) nach Anspruch 1, in welcher
A für CH₂, CD₂ oder CH(CH₃) steht,
R¹ für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, (C₃-C₆)-Cycloalkyl, Difluormethoxy und Trifluormethoxy substituiert sein kann,
R² für Wasserstoff, (C₁-C₄)-Alkyl, Cyclopropyl, Monofluormethyl, Difluormethyl oder Trifluormethyl steht,
R³ für eine Gruppe der Formel 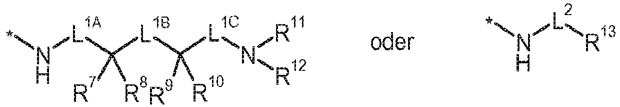 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy, substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃, (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R⁸ für Wasserstoff oder (C₁-C₄)-Alkyl steht,
oder
R⁷ und R⁸ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für Wasserstoff, Cyano, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, Cyano, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5- oder 6-gliedriges Heteroaryl substituiert sein kann,
worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁-C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R¹⁰ für Wasserstoff oder (C₁-C₄)-Alkyl steht,
oder
R⁹ und R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können,
mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen 3- bis 7-gliedrigen Carbocyclus oder einen 4- bis 7-gliedrigen Heterocyclus bilden,
worin der 3- bis 7-gliedrigen Carbocyclus mit 1 oder 2 Substituenten unabhängig voneinander ausgewählt aus der Gruppe (C₁-C₄)-Alkyl, Fluor, Hydroxy und (C₁-C₄)-Alkoxy substituiert sein kann,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
R¹¹ für (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert ist,
R¹² für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe (C₃-C₇)-Cycloalkyl und 4- bis 7-gliedriges Heterocyclyl substituiert ist,
und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kein, der seinerseits mit 1 oder 2 Substituenten ausgewählt aus Halogen, (C₁-C₄)-Alkyl, (C₁-C₄)-Alkoxy und Trifluormethyl substituiert sein kann,
oder
für Adamantyl steht,
R⁴ für Wasserstoff steht,
R⁵ für Wasserstoff, Halogen, Cyano, Monofluormethyl, Difluormethyl, Trifluormethyl, (C₁-C₄)-Alkyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

7. Verbindung der Formel (I) nach Anspruch 1, in welcher
A für CH₂, CD₂ oder CH(CH₃) steht,
R¹ für (C₄-C₆)-Alkyl, (C₃-C₇)-Cycloalkyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, (C₃-C₆)-Cycloalkyl, Difluormethoxy und Trifluormethoxy substituiert sein kann,
R² für Wasserstoff, (C₁-C₄)-Alkyl, Cyclopropyl, Monofluormethyl, Difluormethyl oder Trifluormethyl steht,
R³ für eine Gruppe der Formel 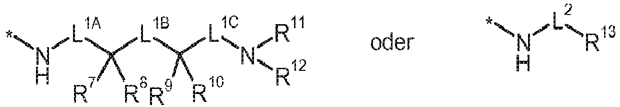 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, Cyano, 5- bis 10-gliedriges Heteroaryl, Naphthyl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy, substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Nitro, Difluormethyl, Trifluormethyl, Difluormethoxy, Trifluormethoxy, -NH(CO)CH₃, (C₁-C₄)-Alkyl, (C₁-C₄)-Cycloalkyl, (C₁-C₄)-Alkenyl, (C₁-C₄)-Alkyl-sulfonyl, (C₁-C₄)-Alkoxycarbonyl, und (C₁-C₄)-Alkoxy substituiert sein können,
wobei (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R⁸ für Wasserstoff oder (C₁-C₄)-Alkyl steht,
oder
R⁷ und R⁸ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für Wasserstoff, Cyano, (C₁-C₆)-Alkyl, (C₂-C₆)-Alkenyl, (C₂-C₆)-Alkinyl, (C₃-C₇)-Cycloalkyl, 5- bis 10-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, Cyano, (C₁-C₄)-Alkoxy, (C₁-C₄)-Alkoxycarbonyl, (C₁-C₄)-Alkylsulfonyl, 5- oder 6-gliedriges Heteroaryl, Phenyl, Phenoxy und Benzyloxy substituiert sein kann,
worin Phenyl, Phenoxy und Benzyloxy ihrerseits mit 1 bis 3 Substituenten Halogen oder (C₁-C₄)-Alkoxy substituiert sein können,
worin 5- oder 6-gliedriges Heteroaryl benzokondensiert oder mit einem 5- oder 6-gliedriges Heteroaryl substituiert sein kann,
worin 5- oder 6-gliedriges Heteroaryl mit (C₁-C₄)-Alkyl oder Trifluormethyl substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- bis 10-gliedriges Heteroaryl mit 1 bis 3 Substituenten unabhängig voneinander ausgewählt aus der Gruppe Halogen, Cyano, Difluormethyl, Trifluormethyl, Difluormethoxy, Trifluormethoxy, (C₁-C₄)-Alkyl, (C₁₋C₄)-Cycloalkyl, (C₁-C₄)-Alkoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin an 2 benachbarten Kohlenstoffatomen des Phenyls mit einer Difluormethylendioxy-Brücke substituiert sein kann,
R¹⁰ für Wasserstoff oder (C₁-C₄)-Alkyl steht,
oder
R⁹ und R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor, Benzyl und (C₁-C₄)-Alkyl substituiert sein können,
mit der Maßgabe, dass die Reste R⁷ und R⁹ nicht beide gleichzeitig für Phenyl stehen,
oder
R⁷ und R⁹ zusammen mit den Kohlenstoffatomen, an die sie gebunden sind, sowie der Gruppe L^{1B} einen 3- bis 7-gliedrigen Carbocyclus oder einen 4- bis 7-gliedrigen Heterocyclus bilden,
worin der 3- bis 7-gliedrigen Carbocyclus mit 1 oder 2 Substituenten unabhängig voneinander ausgewählt aus der Gruppe (C₁-C₄)-Alkyl, Fluor, Hydroxy und (C₁-C₄)-Alkoxy substituiert sein kann,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
R¹¹ für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
R¹² für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy, (C₁-C₄)-Alkoxy und 4- bis 7-gliedriges Heterocyclyl substituiert sein kann,
und
L² für eine Bindung oder (C₁-C₄)-Alkandiyl steht,
R¹³ für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe (C₃-C₇)-Cycloalkyl und Benzyl substituiert ist,
R⁴ für Wasserstoff steht,
R⁵ für Wasserstoff, Halogen, Cyano, Monofluormethyl, Difluormethyl, Trifluormethyl, (C₁-C₄)-Alkyl, (C₂-C₄)-Alkenyl, (C₂-C₄)-Alkinyl, (C₃-C₆)-Cycloalkyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

8. Verbindung der Formel (I) nach Anspruch 1, in welcher
A für CH₂ steht,
R¹ für Phenyl steht,
wobei Phenyl mit 2 bis 3 Fluor substituiert ist,
R² für Methyl steht,
R³ für eine Gruppe der Formel 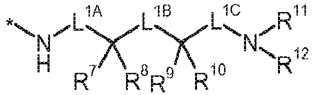 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff steht,
R⁸ für Wasserstoff steht,
R⁹ für Wasserstoff oder (C₁-C₄)-Alkyl steht,
R¹⁰ für Methyl oder Ethyl steht,
R¹¹ für Wasserstoff steht,
R¹² für Wasserstoff steht,
R⁴ für Wasserstoff steht,
R⁵ für Wasserstoff oder Methyl steht,
R⁶ für Wasserstoff steht,
sowie ihre *N*-Oxide, Salze, Solvate, Salze der *N*-Oxide und Solvate der *N*-Oxide und Salze.

9. Verbindung der Formel (I) nach Anspruch 1, in welcher
A für CH₂ steht,
R¹ für Phenyl steht,
wobei Phenyl mit 2 bis 3 Fluor substituiert ist,
R² für Methyl steht,
R³ für eine Gruppe der Formel 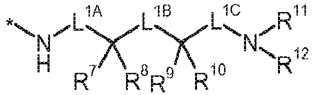 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff steht,
R⁸ für Wasserstoff steht,
R⁹ für (C₁-C₄)-Alkyl steht,
worin (C₁-C₄)-Alkyl bis zu fünffach mit Fluor substituiert ist,
R¹⁰ für Methyl oder Ethyl steht,
R¹¹ für Wasserstoff steht,
R¹² für Wasserstoff steht,
R⁴ für Wasserstoff steht,
R⁵ für Wasserstoff oder Methyl steht,
R⁶ für Wasserstoff steht,
sowie ihre *N*-Oxide, Salze, Solvate, Salze der *N*-Oxide und Solvate der *N*-Oxide und Salze.

10. Verfahren zur Herstellung von Verbindungen der Formel (I), wie in den Ansprüchen 1 bis 9 definiert, **dadurch gekennzeichnet, dass** man
[A] eine Verbindung der Formel (II) 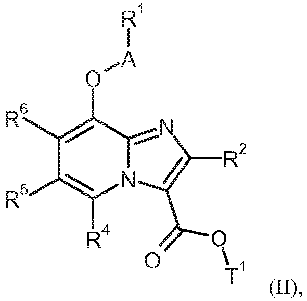 in welcher A, R¹, R², R⁴, R⁵ und R⁶ jeweils die oben angegebenen Bedeutungen haben und
T¹ für (C₁-C₄)-Alkyl oder Benzyl steht,
in einem inerten Lösungsmittel in Gegenwart einer geeigneten Base oder Säure zu einer Carbonsäure der Formel (III) 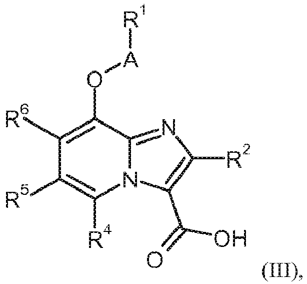 in welcher A, R¹, R², R⁴, R⁵ und R⁶ jeweils die oben angegebenen Bedeutungen haben,
umsetzt und diese in der Folge in einen inerten Lösungsmittel unter Amidkupplungsbedingungen mit einem Amin der Formel (IV-A) oder (IV-B) 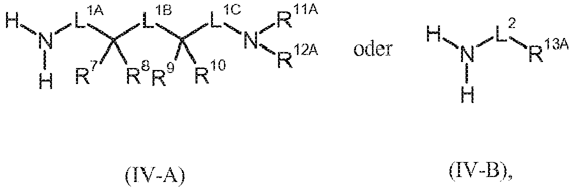 in welchem L^{1A}, L^{1B}, L^{1C}, L², R⁷, R⁸, R⁹, und R¹⁰ jeweils die oben angegebenen Bedeutungen haben
und
R^{11A}, R^{12A} und R^{13A} die oben für R¹¹, R¹² bzw. R¹³ angegebenen Bedeutungen haben oder für eine Amino-Schutzgruppe, wie beispielsweise tert.-Butoxycarbonyl, Benzyloxycarbonyl oder Benzyl stehen, umsetzt,
oder
[B] eine Verbindung der Formel (III-B) 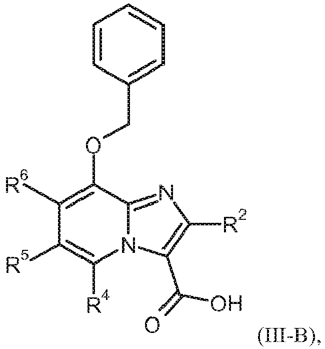 in welcher R², R⁴, R⁵ und R⁶ jeweils die oben angegebenen Bedeutungen haben,
in einem inerten Lösungsmittel unter Amidkupplungsbedingungen mit einem Amin der Formel (IV) zu einer Verbindung der Formel (I-A) und (I-B), 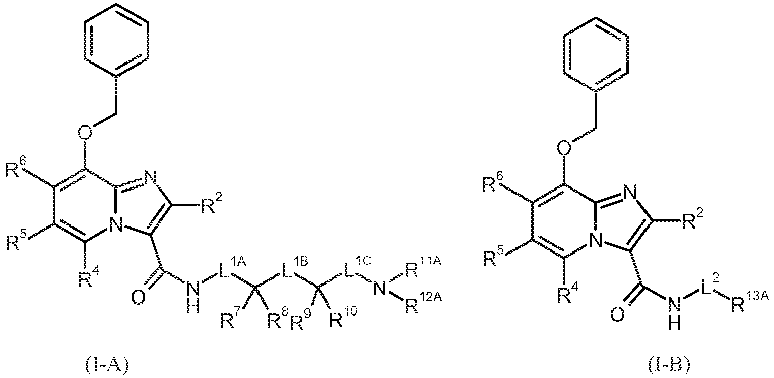 in welcher R², R⁴, R⁵, R⁶, L^{1A}, L^{1B}, L^{1C}, L², R⁷, R⁸, R⁹, R¹⁰, R^{11A}, R^{12A} und R^{13A} jeweils die oben angegebenen Bedeutungen haben,
umsetzt, von dieser im Folgenden nach den dem Fachmann bekannten Methoden die Benzylgruppe abspaltet und die resultierende Verbindung der Formel (V-A) oder (V-B) 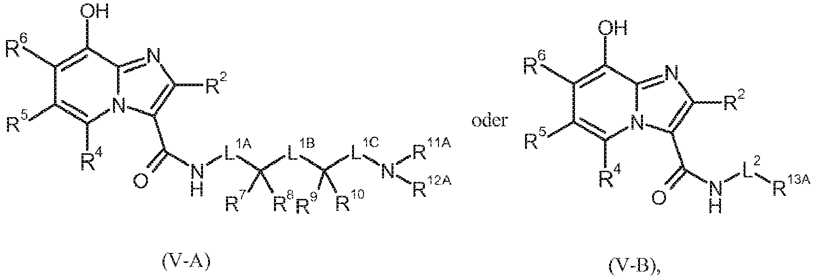 in welcher R², R⁴, R⁵, R⁶, L^{1A}, L^{1B}, L^{1C}, L², R⁷, R⁸, R⁹, R¹⁰, R^{11A}, R^{12A} und R^{13A} jeweils die oben angegebenen Bedeutungen haben,
in einem inerten Lösungsmittel in Gegenwart einer geeigneten Base mit einer Verbindung der Formel (VI) 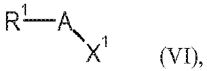 in welcher A und R¹ die oben angegebene Bedeutung hat und
X¹ für eine geeignete Abgangsgruppe, insbesondere Chlor, Brom, Iod, Mesylat, Triflat oder Tosylat, steht,
umsetzt,
anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

11. Verbindung der Formel (I), wie in einem der Ansprüche 1 bis 9 definiert, zur Behandlung und/oder Prophylaxe von Krankheiten.

12. Verwendung einer Verbindung der Formel (I), wie in einem der Ansprüche 1 bis 9 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

13. Arzneimittel enthaltend eine Verbindung der Formel (I), wie in einem der Ansprüche 1 bis 9 definiert, in Kombination mit einem inerten, nicht-toxischen, pharmazeutisch geeigneten Hilfsstoff.

14. Arzneimittel enthaltend eine Verbindung der Formel (I), wie in einem der Ansprüche 1 bis 9 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

15. Arzneimittel nach Anspruch 13 oder 14 zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

16. Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose bei Menschen und Tieren unter Verwendung einer wirksamen Menge mindestens einer Verbindung der Formel (I), wie in einem der Ansprüche 1 bis 9 definiert, oder eines Arzneimittels, wie in einem der Ansprüche 13 bis 15 definiert.
